(19) Europäisches Patentamt / European Patent Office / Office européen des brevets

(11) EP 4 385 985 A1

(12) **EUROPEAN PATENT APPLICATION**
published in accordance with Art. 153(4) EPC

(43) Date of publication:
**19.06.2024 Bulletin 2024/25**

(21) Application number: **22855498.6**

(22) Date of filing: **11.08.2022**

(51) International Patent Classification (IPC):
***C07D 401/14*** (2006.01) ***C07D 417/14*** (2006.01)
***C07D 471/04*** (2006.01) ***C07D 471/08*** (2006.01)
***C07D 471/10*** (2006.01) ***A61K 31/444*** (2006.01)
***A61K 31/496*** (2006.01) ***A61K 31/517*** (2006.01)
***A61P 35/00*** (2006.01)

(52) Cooperative Patent Classification (CPC):
**A61K 31/444; A61K 31/496; A61K 31/517; A61P 35/00; C07D 401/14; C07D 417/14; C07D 471/04; C07D 471/08; C07D 471/10**

(86) International application number:
**PCT/CN2022/111743**

(87) International publication number:
**WO 2023/016518 (16.02.2023 Gazette 2023/07)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:
**11.08.2021 CN 202110905755**
**06.09.2021 CN 202111023520**
**28.10.2021 CN 202111253799**
**31.12.2021 CN 202111649149**
**09.03.2022 CN 202210215583**
**27.05.2022 CN 202210585212**
**08.07.2022 CN 202210765950**

(71) Applicant: **Xizang Haisco Pharmaceutical Co., Ltd.**
**Lhoka, Tibet 856099 (CN)**

(72) Inventors:
- **ZHANG, Chen**
  **Chengdu City, Sichuan 611130 (CN)**
- **LIAO, Yuting**
  **Chengdu, Sichuan 611130 (CN)**
- **CHEN, Xiaogang**
  **Chengdu, Sichuan 611130 (CN)**
- **XU, Jinxiong**
  **Chengdu, Sichuan 611130 (CN)**
- **YU, Yan**
  **Chengdu, Sichuan 611130 (CN)**
- **TANG, Pingming**
  **Chengdu, Sichuan 611130 (CN)**
- **GAO, Qiu**
  **Chengdu, Sichuan 611130 (CN)**
- **ZHAO, Junbin**
  **Chengdu, Sichuan 611130 (CN)**
- **LI, Yupeng**
  **Chengdu, Sichuan 611130 (CN)**
- **CHENG, Xinfan**
  **Chengdu, Sichuan 611130 (CN)**
- **ZHU, Guozhi**
  **Chengdu, Sichuan 611130 (CN)**
- **YE, Fei**
  **Chengdu, Sichuan 611130 (CN)**
- **LI, Yao**
  **Chengdu, Sichuan 611130 (CN)**
- **NI, Jia**
  **Chengdu, Sichuan 611130 (CN)**
- **YAN, Pangke**
  **Chengdu, Sichuan 611130 (CN)**

(74) Representative: **Hoffmann Eitle**
**Patent- und Rechtsanwälte PartmbB**
**Arabellastraße 30**
**81925 München (DE)**

(54) **HETEROCYCLIC DERIVATIVE, AND COMPOSITION AND PHARMACEUTICAL USE THEREOF**

(57) Disclosed are a compound as represented by general formula (I) or a stereoisomer, deuterated compound, solvate, prodrug, metabolite, pharmaceutically acceptable salt or co-crystal thereof and an intermediate thereof; and the use thereof in AR-related diseases such as cancer. B-L-K (I)

EP 4 385 985 A1

## Description

Technical Field

The present invention relates to a compound of general formula (I) or a stereoisomer, deuterated compound, solvate, prodrug, metabolite, pharmaceutically acceptable salt or co-crystal thereof, and an intermediate thereof and a preparation method therefor, as well as the use thereof in AR-related diseases such as cancer diseases.

Background Art

The androgen receptor (AR), a nuclear hormone receptor that is structurally comprised of an N-terminal domain (NTD), a DNA-binding domain (DBD) and a ligand-binding domain (LTD), can regulate the expression of genes that induce prostate cancer, and thus, inhibition of the androgen receptor is an effective method for treating prostate cancer. Currently marketed androgen receptor inhibitors such as enzalutamide and bicalutamide mainly exert an inhibitory effect by interacting with the ligand-binding domain (LTD) of the androgen receptor. However, some patients will develop drug resistance caused by androgen receptor splice variants (AR-Vs) with the deletion of LTD fragment during treatment. Preclinical studies have shown that androgen receptor splice variants can accelerate the progression of enzalutamide-resistant prostate cancer, and therefore, how to solve the problem of drug resistance has become the focus of clinical medicine.

Small molecule degraders are drugs that utilize the body's ubiquitinproteasome system (UPS) for targeted degradation of target proteins. By virtue of unique catalytic mechanism, small molecule degraders can target targets with poor druggability and solve the problem of drug resistance. They are currently a hot topic in the field of drug research and development for tumors, autoimmune diseases, *etc.*

PROTAC (proteolysis targeting chimera) molecules are a class of bifunctional compounds that can simultaneously bind targeting proteins and E3 ubiquitin ligases. Such compounds can be recognized by proteasomes of cells, causing the degradation of targeting proteins, and can effectively reduce the content of targeting proteins in the cells. By introducing a ligand capable of binding to various targeted proteins into the PROTAC molecules, it is possible to apply the PROTAC technology to the treatment of various diseases, and this technology has attracted extensive attention in recent years.

Molecular glue is a type of small molecule that brings the target protein into contact with E3 ubiquitin ligase, inducing interaction between the two, thereby leading to the degradation of the target protein. From a functional perspective, molecular glue mainly fills the gap between the target protein and E3 ubiquitin ligase, enhancing the binding interface between the two and promoting a strong interaction between the two *(*Nat. Commun., 2022, 13, 815). Compared with conventional small molecule inhibitors, molecular glue has the advantages of catalytically driving target protein degradation and not requiring a binding pocket on the target protein, and has the potential to act on undruggable targets.

Therefore, it is necessary to develop a novel androgen receptor splice variant (AR-V, especially AR-V7 mutant) inhibitor and PROTAC for E3 ubiquitin ligase or other small molecule degrader drugs for the treatment of neoplastic diseases associated with androgen receptor splice mutants.

Summary of the Invention

The objective of the present invention is to provide a compound with novel structure, good efficacy, high bioavailability and higher safety that can inhibit and degrade AR or/and AR-Vs (especially AR-V7), for use in the treatment of AR-related diseases such as prostate cancer.

The present invention provides a compound or a stereoisomer, deuterated compound, solvate, prodrug, metabolite, pharmaceutically acceptable salt or co-crystal thereof, wherein the compound is selected from a compound as represented by general formula (I),

B-L-K (I)

;

in certain embodiments, L is selected from a bond or -$C_{1-50}$ hydrocarbyl-, wherein the hydrocarbyl has 0 to 20 methylene units optionally replaced by -Ak- or -Cy-;

in certain embodiments, L is selected from a bond or -$C_{1-20}$ hydrocarbyl-, wherein the hydrocarbyl has 0 to 20 methylene units optionally replaced by -Ak- or -Cy-;

in certain embodiments, L is selected from a bond or -$C_{1-10}$ hydrocarbyl-, wherein the hydrocarbyl has 0 to 10 (e.g., 0, 1, 2, 3, 4, 5, 6, 7, 8, 9 or 10) methylene units optionally replaced by -Ak- or -Cy-;

in certain embodiments, each -Ak- is independently selected from Ak1, Ak2, Ak3, Ak4 or Ak5;

in certain embodiments, each -Ak- is independently selected from $-(CH_2)_q-$, $-(CH_2)_q-O-$, $-O-(CH_2)_q-$, $-(CH_2)_q-NR^L-$, $-NR^L-(CH_2)_q-$, $-(CH_2)_q-NR^LC(=O)-$, $-NR^L(CH_2)_qC(=O)-$, $-(CH_2)_q-C(=O)NR^L-$, -C(=O)-, $-C(=O)-(CH_2)_q-NR^L-$, $-(C\equiv C)_q-$, -CH=CH-, $-Si(R^L)_2-$, $-Si(OH)(R^L)-$, $-Si(OH)_2-$, $-P(=O)(OR^L)-$, $-P(=O)(R^L)-$, -S-, -S(=O)-, $-S(=O)_2-$ or a bond, wherein the $-CH_2-$ or -CH=CH- is optionally substituted with 0 to 2 (e.g., 0, 1 or 2) substituents selected from H, halogen, OH, CN, $NH_2$, $C_{1-6}$ alkyl, $C_{1-6}$ alkoxy, halogen-substituted $C_{1-6}$ alkyl, hydroxyl-substituted $C_{1-6}$ alkyl or cyano-substituted $C_{1-6}$ alkyl;

in certain embodiments, each -Cy- is independently selected from Cy1, Cy2, Cy3, Cy4 or Cy5;

in certain embodiments, each -Cy- is independently selected from a bond, 4-to 8-membered mono-heterocyclic ring, 4- to 10-membered fused heterocyclic ring, 5- to 12-membered spiro-heterocyclic ring, 7- to 10-membered bridged-heterocyclic ring, 3- to 7-membered monocycloalkyl, 4- to 10-membered fused cycloalkyl, 5- to 12-membered spirocycloalkyl, 7- to 10-membered bridged cycloalkyl, 5- to 10-membered heteroaryl or 6- to 10-membered aryl, the aryl, heteroaryl, cycloalkyl, mono-heterocyclic ring, fused heterocyclic ring, spiro-heterocyclic ring or bridged-heterocyclic ring is optionally substituted with 0 to 4 (for example, 0, 1, 2, 3 or 4) substituents selected from H, F, Cl, Br, I, OH, COOH, CN, $NH_2$, =O, $C_{1-4}$ alkyl, halogen-substituted $C_{1-4}$ alkyl, hydroxyl-substituted $C_{1-4}$ alkyl or $C_{1-4}$ alkoxy, and the heteroaryl, mono-heterocyclic ring, fused heterocyclic ring, spiro-heterocyclic ring or bridged-heterocyclic ring contains 1 to 4 (for example, 1, 2, 3 or 4) heteroatoms selected from O, S or N, and is optionally substituted with 0, 1 or 2 =O when the heteroatom is selected from S;

in certain embodiments, each Cy1, Cy2, Cy3, Cy4 or Cy5 is independently selected from a bond, 4- to 7-membered mono-heterocyclic ring, 4- to 10-membered fused heterocyclic ring, 5- to 12-membered spiro-heterocyclic ring, 7- to 10-membered bridged-heterocyclic ring, 3- to 7-membered monocycloalkyl, 4-to 10-membered fused cycloalkyl, 5- to 12-membered spirocycloalkyl, 7- to 10-membered bridged cycloalkyl, 5- to 10-membered heteroaryl or 6- to 10-membered aryl, the aryl, heteroaryl, cycloalkyl, mono-heterocyclic ring, fused heterocyclic ring, spiro-heterocyclic ring or bridged-heterocyclic ring is optionally substituted with 0 to 4 (for example, 0, 1, 2, 3 or 4) substituents selected from H, F, Cl, Br, I, OH, COOH, CN, $NH_2$, =O, $C_{1-4}$ alkyl, halogen-substituted $C_{1-4}$ alkyl, hydroxyl-substituted $C_{1-4}$ alkyl or $C_{1-4}$ alkoxy, and the heteroaryl, mono-heterocyclic ring, fused heterocyclic ring, spiro-heterocyclic ring or bridged-heterocyclic ring contains 1 to 4 (for example, 1, 2, 3 or 4) heteroatoms selected from O, S or N, and is optionally substituted with 0, 1 or 2 =O when the heteroatom is selected from S;

in certain embodiments, each Cy1, Cy2, Cy3, Cy4 or Cy5 is independently selected from a bond, 4- to 7-membered nitrogen-containing mono-heterocyclic ring, 4- to 10-membered nitrogen-containing fused heterocyclic ring, 5- to 12-membered nitrogen-containing spiro-heterocyclic ring, 7- to 10-membered nitrogen-containing bridged-heterocyclic ring, 3- to 7-membered monocycloalkyl, 4- to 10-membered fused cycloalkyl, 5- to 12-membered spirocycloalkyl, 7- to 10-membered bridged cycloalky, 5- to 10-membered heteroaryl or 6- to 10-membered aryl, the mono-heterocyclic ring, fused heterocyclic ring, bridged-heterocyclic ring, spiro-heterocyclic ring, cycloalkyl, aryl or heteroaryl is optionally substituted with 0 to 4 substituents selected from H, F, Cl, Br, I, OH, COOH, CN, $NH_2$, =O, $C_{1-4}$ alkyl, halogen-substituted $C_{1-4}$ alkyl, hydroxyl-substituted $C_{1-4}$ alkyl or $C_{1-4}$ alkoxy, the mono-heterocyclic ring, fused heterocyclic ring, bridged-heterocyclic ring, spiro-heterocyclic ring or heteroaryl contains 1 to 4 heteroatoms selected from O, S or N, and is optionally substituted with 0, 1 or 2 =O when the heteroatom is selected from S;

in certain embodiments, L is selected from -Cy1-Ak1-Cy2-Ak2-Cy3-Ak3-Cy4-Ak4-Cy5-Ak5-, -Cy1-Cy2-Cy3-Cy4-Ak1-Ak2-Ak3-Ak4-Ak5-, -Cy1-Ak1-Cy2-Ak2-Cy3-Ak3-Cy4-Ak4-Ak5-, -Ak1-Cy1-Ak2-Cy2-Ak3-Cy3-Ak4-Cy4-Ak5-, -Cy1-Ak1-Cy2-Ak2-Cy3-Cy4-Ak3-Ak4-Ak5-, -Cy1-Ak1-Cy2-Ak2-Ak3-Cy3-Cy4-Ak4-Ak5-, -Cy1-Ak1-Ak2-Ak3-Ak4-Ak5-Cy2-Cy3-Cy4-, -Cy1-Cy2-Ak1-Ak2-Ak3-Ak4-Ak5-Cy3-Cy4-, -Cy1-Cy2-Cy3-Ak1-Ak2-Ak3-Ak4-Ak5-Cy4-, -Cy1-Cy2-Cy3-Cy4-Ak1-Ak2-Ak3-Ak4-Ak5-, -Cy1-Ak1-Cy2-Cy3-Cy4-Ak2-Ak3-Ak4-Ak5-, -Cy1-Cy2-Ak1-Cy3-Cy4-Ak2-Ak3-Ak4-Ak5-, -Cy1-Cy2-Cy3-Ak1-Cy4-Ak2-Ak3-Ak4-Ak5-, -Cy1-Ak1-Ak2-Cy2-Cy3-Cy4-Ak3-Ak4-Ak5-, -Cy1-Cy2-Ak1-Ak2-Cy3-Cy4-Ak3-Ak4-Ak5-, -Cy1-Cy2-Cy3-Ak1-Ak2-Cy4-Ak3-Ak4-Ak5-, -Cy1-Ak1-Ak2-Ak3-Cy2-Cy3-Cy4-Ak4-Ak5-, -Cy1-Cy2-Ak1-Ak2-Ak3-Cy3-Cy4-Ak4-Ak5-, -Cy1-Cy2-Cy3-Ak1-Ak2-Ak3-Cy4-Ak4-Ak5-, -Cy1-Ak1-Ak2-Ak3-Ak4-Cy2-Cy3-Cy4-Ak5-, -Cy1-Cy2-Ak1-Ak2-Ak3-Ak4-Cy3-Cy4-Ak5-, -Cy1-Cy2-Cy3-Ak1-Ak2-Ak3-Ak4-Cy4-Ak5-, -Ak1-Ak2-Ak3-Ak4-Ak5-Cy1-Cy2-Cy3-Cy4-, -Ak1-Cy1-Cy2-Cy3-Cy4-Ak2-Ak3-Ak4-Ak5-, -Ak1-Ak2-Cy1-Cy2-Cy3-Cy4-Ak3-Ak4-Ak5-, -Ak1-Ak2-Ak3-Cy1-Cy2-Cy3-Cy4-Ak4-Ak5-, -Ak1-Ak2-Ak3-Ak4-Cy1-Cy2-Cy3-Cy4-Ak5-, -Ak1-Cy1-Ak2-Ak3-Ak4-Ak5-Cy2-Cy3-Cy4-, -Ak1-Cy1-Cy2-Ak2-Ak3-Ak4-Ak5-Cy3-Cy4-, -Ak1-Cy1-Cy2-Cy3-Ak2-Ak3-Ak4-Ak5-Cy4-, -Ak1-Ak2-Cy1-Ak3-Ak4-Ak5-Cy2-Cy3-Cy4-, -Ak1-Ak2-Cy1-Cy2-Ak3-Ak4-Ak5-Cy3-Cy4-, -Ak1-Ak2-Cy1-Cy2-Cy3-Ak3-Ak4-Ak5-Cy4-, -Ak1-Ak2-Ak3-Cy1-Ak4-Ak5-Cy2-Cy3-Cy4-, -Ak1-Ak2-Ak3-Cy1-Cy2-Ak4-Ak5-Cy3-Cy4-, -Ak1-Ak2-Ak3-Cy1-Cy2-Cy3-Ak4-Ak5-Cy4-, -Ak1-Ak2-Ak3-Ak4-Cy1-Ak5-Cy2-Cy3-Cy4-, -Ak1-Ak2-Ak3-Ak4-Cy1-Cy2-Ak5-Cy3-Cy4-, or -Ak1-Ak2-Ak3-Ak4-Cy1-Cy2-Cy3-Ak5-Cy4-;

in certain embodiments, L is selected from a bond, -Ak1-, -Cy1-, -Cy1-Ak1-, -Cy1-Ak1-Ak2-, -Cy1-Ak1-Ak2-Ak3-, -Cy1-Ak1-Ak2-Ak3-Ak4-, -Cy1-Cy2-, -Cy1-Ak1-Cy2-, -Cy1-Cy2-Ak2-, -Cy1-Ak1-Cy2-Ak2-, -Cy1-Ak1-Cy2-Ak2-Ak3-, -Cy1-Ak1-Cy2-Ak2-Ak3-Ak4-, -Cy1-Cy2-Ak2-Ak3-, -Cy1-Cy2-Ak2-Ak3-Ak4-, -Cy1-Ak1-Cy2-Ak2-Ak3-Ak4-, -Cy1-Ak1-Ak2-Cy3-, -Cy1-Ak1-Ak2-Cy3-Ak3-, -Cy1-Cy2-Cy3-, -Cy1-Ak1-Cy2-Cy3-, -Cy1-Cy2-Ak2-Cy3-, -Cy1-Cy2-Cy3-Ak3-, -Cy1-Ak1-Cy2-Cy3-Ak3-, -Cy1-Cy2-Ak2-Cy3-Ak3-, -Cy1-Ak1-Cy2-Ak2-Cy3-, - Cy1-Ak1-Cy2-Ak2-Cy3-Ak3-, -Cy1-Cy2-Cy3-Ak3-Ak4-, -Cy1-Cy2-Cy3-Ak3-Cy4-, -Cy1-Cy2-Cy3-Cy4-, -Cy1-Ak1-Cy2-Cy3-Cy4-, -Cy1-Cy2-Ak2-Cy3-Cy4-, -Cy1-Cy2-Cy3-Ak3-Cy4-, -Cy1-Cy2-Cy3-Cy4-Ak4-, -Cy1-Ak1-Cy2-Ak2-Cy3-Ak3-Cy4-, -Cy1-Ak1-Cy2-Ak2-Cy3-Cy4-, -Ak1-Cy2-, -Ak1-Cy2-Cy3-, -Ak1-Ak2-Cy3-, -Ak1-Ak2-Cy3-Cy4-, -Ak1-Cy2-Ak2-Cy3-, -Ak1-Cy2-Cy3-Ak3-Cy4-, -Ak1-Cy2-Cy3-Cy4-Ak4-Cy5-, -Ak1-Cy2-Ak2-, -Ak1-Ak2-Ak3-Ak4-, -Ak1-Ak2-Ak3-, -Ak1-Ak2-, -Ak1-Ak2-Ak3-Ak4-Ak5-, -Cy1-Cy2-Cy3-Ak3-Ak4-Ak5-, - Cy1-Cy2-Ak2-Cy3-Ak3-Ak4-Ak5-, -Cy1-Ak1-Cy2-Ak2-Ak3-Ak4-Ak5-, -Cy1-Cy2-Cy3-Cy4-Ak4-Ak5-, -Cy1-Ak1-Ak2-Ak3-Ak4-Ak5-, -Ak1-Cy2-Ak2-Ak3-Ak4-Ak5-, -Ak1-Cy2-Ak2-Ak3-Ak4-, or -Ak1-Cy2-Ak2-Ak3-;

in certain embodiments, L is selected from a bond, -Ak1-Cy2-, -Ak1-Cy2-Cy3-, -Cy1-Ak1-, -Ak1-Cy2-Ak2-, -Cy1-Cy2-Cy3-, -Cy2-Cy3-, -Cy1-Ak1-Cy2-, - Cy1-Ak1-Cy2-Cy3-, -Cy1-Cy2-Ak2-Cy3-, -Ak1-, -Cy1-, -Ak1-Cy2-Ak2-Ak3-, - Ak1-Cy2-Ak2-Cy3-, -Ak1-Cy2-Ak2-Ak3-Ak4-, or -NHCO-$(CH_2)_{s1}$, $_{s1}$ is selected from 0, 1, 2, 3, 4, 5, 6 or 7;

in certain embodiments, L is selected from a bond or a group shown in Table B-1 or Table B-2, the left side of the group is connected to B;

in certain embodiments, L is selected from a bond or a group shown in Table B-2, the left side of the group is connected to B;

in certain embodiments, L is selected from

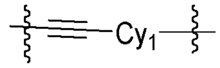

;

in certain embodiments, Cy1 is selected from 4- to 12-membered nitrogen-containing heterocycle, preferably 4-7-membered nitrogen-containing mono-heterocyclic ring, 4-10-membered nitrogen-containing fused heterocyclic ring, 5-12-membered nitrogen-containing spiro-heterocyclic ring, or 7- to 10-membered nitrogen-containing bridged-heterocyclic ring, the nitrogen-containing heterocycle contains 1 to 4 (for example 1, 2, 3 or 4) heteroatoms selected from O, S or N, and is optionally substituted with 0, 1 or 2 =O when the heteroatom is selected from S;

in certain embodiments, Cy1 is selected from 4- to 6-membered nitrogen-containing heterocycle;

in certain embodiments, L is selected from

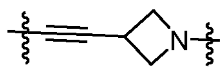

;

in certain embodiments, Ak1, Ak2, Ak3, Ak4, and Ak5 are each independently selected from $-(CH_2)_q-$, $-(CH_2)_q-O-$, $-O-(CH_2)_q-$, $-(CH_2)_q-NR^L-$, - $NR^L-(CH_2)_q-$, $-(CH_2)q-NR^LC(=O)-$, $-(CH_2)_q-C(=O)NR^L-$, -C(=O)-, $-C(=O)-(CH_2)_q-NR^L-$, -CH=CH-, $-(C\equiv C)_q-$ or a bond, the $-CH_2-$ or -CH=CH- is optionally substituted with 0 to 2 (for example 0, 1 or 2) substituents selected from H, halogen, OH, CN, $NH_2$, $C_{1-4}$ alkyl, $C_{1-4}$ alkoxy, halogen-substituted $C_{1-4}$ alkyl, hydroxyl-substituted $C_{1-4}$ alkyl, or cyano-substituted $C_{1-4}$ alkyl;

in certain embodiments, Ak1, Ak2, Ak3, Ak4, and Ak5 are each independently selected from $-(CH_2)_q-$, $-(CH_2)_q-O-$, $-O-(CH_2)_q-$, $-(CH_2)_q-NR^L-$, - $NR^L-(CH_2)_q-$, $-(CH_2)_q-NR^LC(=O)-$, $-(CH_2)_q-C(=O)NR^L-$, -C(=O)-, $-C(=O)-(CH_2)_q-NR^L-$, -CH=CH-, $-C\equiv C-$ or a bond, the $-CH_2-$ or -CH=CH- is optionally substituted with 1 to 2 substituents selected from F, Cl, Br, I, OH, CN, $NH_2$, $CF_3$, hydroxymethyl, methyl, ethyl, methoxy or ethoxy;

in certain embodiments, Ak1, Ak2, Ak3, Ak4, and Ak5 are each independently selected from -O-, $-OCH_2-$, $-CH_2O-$, $-OCH_2CH_2-$, $-CH_2CH_2O-$, - CH=CH-, -CH=C(CN)-, -CH=C(F)-, -C(CN)=CH-, -C(F)=CH-, $-C\equiv C-$, $-C(CH_3)_2-$, $-CH_2-$, $-CH_2CH_2-$, $-CH_2CH_2CH_2-$, $-N(CH_3)-$, -NH-, $-CH_2N(CH_3)-$, $-CH_2NH-$, - $NHCH_2-$, $-CH_2CH_2N(CH_3)-$, $-CH_2CH_2NH-$, $-NHCH_2CH_2-$, -C(=O)-, - $C(=O)CH_2NH-$, $-CH_2C(=O)NH-$, -C(=O)NH- or -NHC(=O)-;

in certain embodiments, Ak1, Ak2, Ak3, Ak4, and Ak5 are each independently selected from -C(=O)-, -O-, NH, -CH=CH-, -CH=C(CN)-, - CH=C(F)-, -C(CN)=CH-, -C(F)=CH-, $-C\equiv C-$, $-C(CH_3)_2-$, $-CH_2-$, $-CH_2CH_2-$, - $CH_2CH_2CH_2-$, or -NHCO-;

in certain embodiments, each $R^L$ is independently selected from H, $C_{1-6}$ alkyl, 3- to 7-membered heterocyclyl, 3- to 7-membered cycloalkyl, phenyl or 5- to 6-membered heteroaryl;

in certain embodiments, each $R^L$ is independently selected from H or $C_{1-6}$ alkyl;

in certain embodiments, each $R^L$ is independently selected from H or $C_{1-4}$ alkyl;

in certain embodiments, each $R^L$ is independently selected from H, methyl or ethyl;

in certain embodiments, each Cy1, Cy2, Cy3, Cy4 or Cy5 is independently selected from a bond or one of the following substituted or unsubstituted groups: cyclopropyl, cyclobutyl, cyclopentyl, cyclohexyl, azetidinyl, azacyclopentyl, azacyclohexenyl, piperidinyl, morpholinyl, piperazinyl, phenyl, cyclopropyl-fused-cyclopropyl, cyclopropyl-fused-cyclobutyl, cyclopropyl-fused-cyclopentyl, cyclopropyl-fused-cyclohexyl, cyclobutyl-fused-cyclobutyl, cyclobutyl-fused-cyclopentyl, cyclobutyl-fused-cyclohexyl, cyclopentyl-fused-cyclopentyl, cyclopentyl-fused-cyclohexyl, cyclohexyl-fused-cyclohexyl, cyclopropyl-spiro-cyclopropyl, cyclopropyl-spiro-cyclobutyl, cyclopropyl-spiro-cyclopentyl, cyclopropyl-spiro-cyclohexyl, cyclobutyl-spiro-cyclobutyl, cyclobutyl-spiro-cyclopentyl, cyclobutyl-spiro-cyclohexyl, cyclopentyl-spiro-cyclopentyl, cyclopentyl-spiro-cyclohexyl, cyclohexyl-spiro-cyclohexyl, cyclopropyl-fused-azetidinyl, cyclopropyl-fused-azacyclopentyl, cyclopropyl-fused-azacyclohexyl, cyclopropyl-fused-piperidine, cyclobutyl-fused-azetidinyl, cyclobutyl-fused-azacyclopentyl, cyclobutyl-fused-azacyclohexyl, cyclobutyl-fused-piperidine, cyclopentyl-fused-azetidinyl, cyclopentyl-fused-azacyclopentyl, cyclopentyl-fused-azacyclohexyl, cyclopentyl-fused-piperidinyl, cyclohexyl-fused-azetidinyl, cyclohexyl-fused-azacyclopentyl, cyclohexyl-fused-azacyclohexyl, cyclohexyl-fused-piperidinyl, azetidinyl-fused-azetidinyl, azetidinyl-fused-azacyclopentyl, azetidinyl-fused-azacyclohexyl, azetidinyl-fused-piperidinyl, azacyclopentyl-fused-azetidinyl, azacyclopentyl-fused-azacyclopentyl, azacyclopentyl-fused-azacyclohexyl, azacyclopentyl-fused-piperidinyl, azacyclohexyl-fused-azetidinyl, azacyclohexyl-fused-azacyclopentyl, azacyclohexyl-fused-azacyclohexyl, azacyclohexyl-fused-piperidinyl, cyclobutyl-spiro-azetidinyl, cyclobutyl-spiro-azacyclopentyl, cyclobutyl-spiro-azacyclohexyl, cyclopentyl-spiro-azetidinyl, cyclopentyl-spiro-azacyclopentyl, cyclopentyl-spiro-azacyclohexyl, cyclohexyl-spiro-azetidinyl, cyclohexyl-spiro-azacyclopentyl, cyclohexyl-spiro-azacyclohexyl, azetidinyl-spiro-azetidinyl, azetidinyl-spiro-azacyclopentyl, azetidinyl-spiro-azacyclohexyl, azacyclopentyl-spiro-azetidinyl, azacyclopentyl-spiro-azacyclopentyl, azacyclopentyl-spiro-azacyclohexyl, azacyclohexyl-spiro-azetidinyl, azacyclohexyl-spiro-azacyclopentyl, azacyclohexyl-spiro-azacyclohexyl,

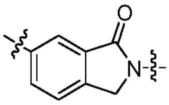, 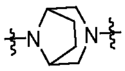, 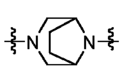, 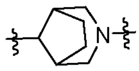, 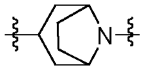,

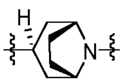, 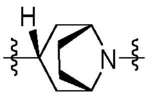, 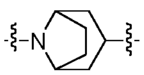, 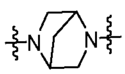, 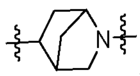, 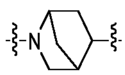, 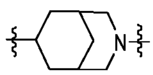,

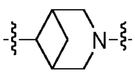, 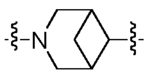, 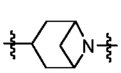, 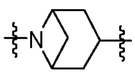, 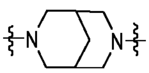, 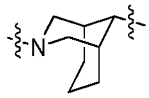, 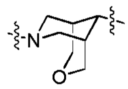,

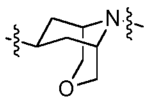, 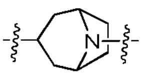, 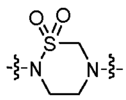, or 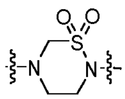,

and when substituted, is optionally further substituted with 0 to 4 (for example, 0, 1, 2, 3 or 4) substituents selected from H, F, Cl, Br, I, OH, $NH_2$, COOH, CN, =O, $C_{1-4}$ alkyl, halogen-substituted $C_{1-4}$ alkyl, hydroxyl-substituted $C_{1-4}$ alkyl or $C_{1-4}$ alkoxy;

in certain embodiments, each Cy1, Cy2, Cy3, Cy4 or Cy5 is independently selected from a bond or one of the following substituted or unsubstituted groups:

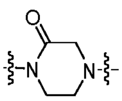, 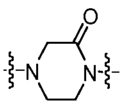, 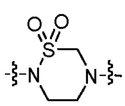, 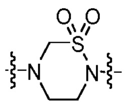, 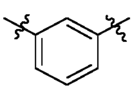, 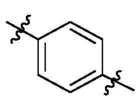, 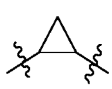,

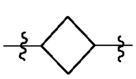, 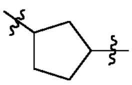, 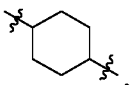, 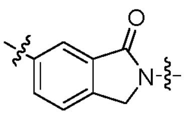, 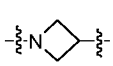, 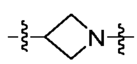, 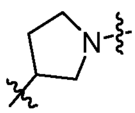

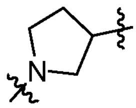, 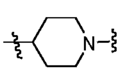, 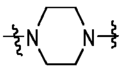, 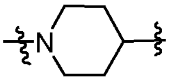, 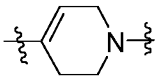, 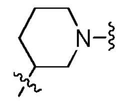, 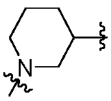,

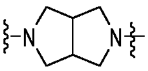, 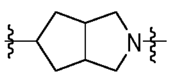, 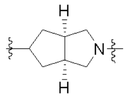, 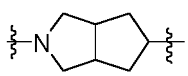, 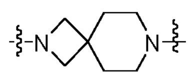,

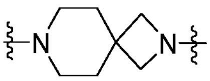, 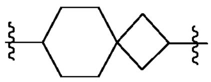, 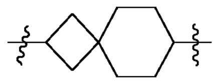, 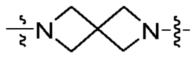,

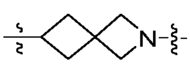, 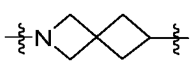, 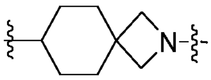, 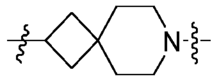,

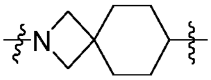, 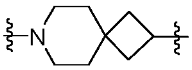, 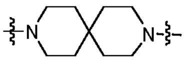, 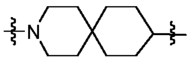, 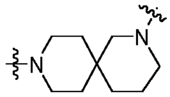,

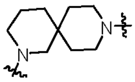, 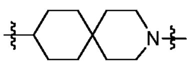, 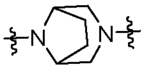, 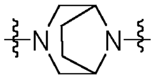, 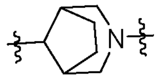, 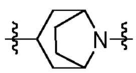,

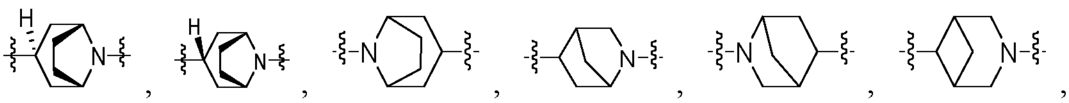

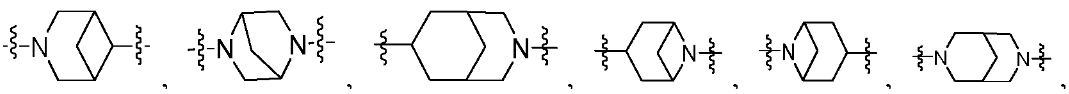

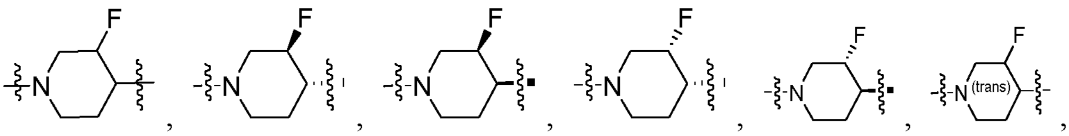

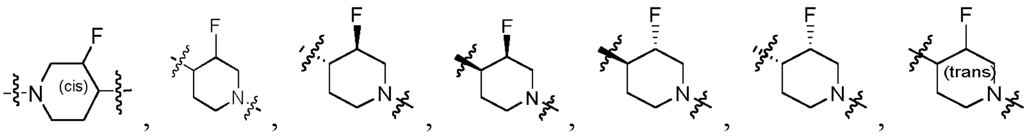 or

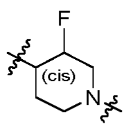,

when substituted, is optionally substituted with 0 to 4 (for example 0, 1, 2, 3 or 4) substituents selected from H, F, $CF_3$, OH, methyl, methoxy, =O, hydroxymethyl, COOH, CN or $NH_2$;

in certain embodiments, each Cy1, Cy2, or Cy3 is independently selected from one of the following substituted or unsubstituted groups:

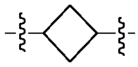,

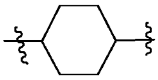, 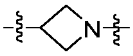, 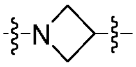, 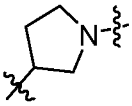, 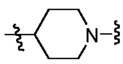, 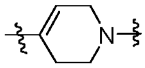, 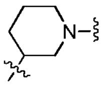,

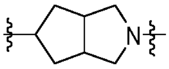, 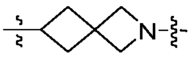, 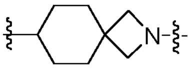, 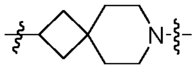, 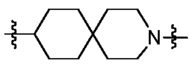,

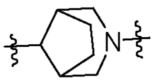, 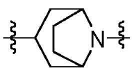, 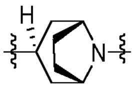, 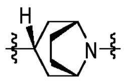, 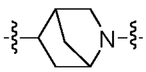, 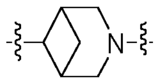,

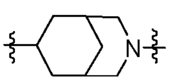, 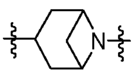, 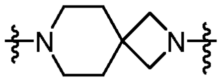, 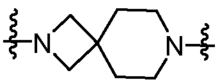, 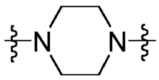,

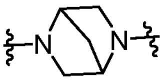, 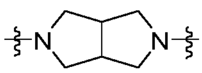, 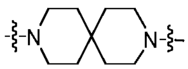, 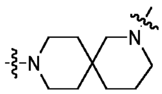, 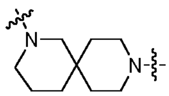,

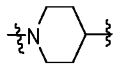, or 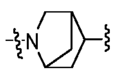,

and when substituted, is optionally substituted with 0 to 4 substituents selected from H, F, $CF_3$, OH, methyl, methoxy, =O, hydroxymethyl, COOH, CN or $NH_2$;
in certain embodiments, K is selected from

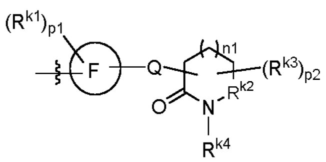,

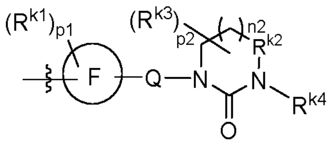, 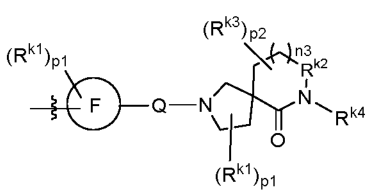, 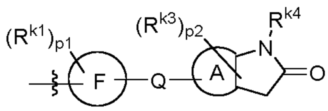,

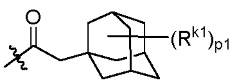, 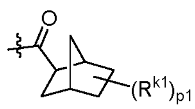, 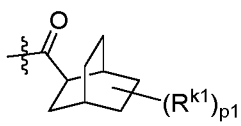, 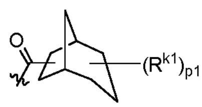,

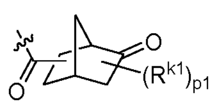 , 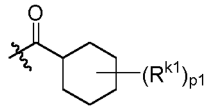 , 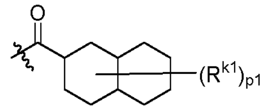 , 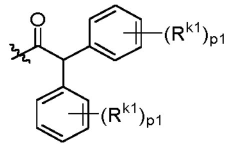 ,

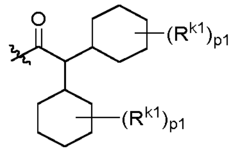 , 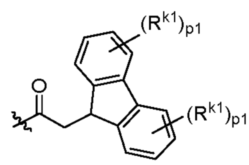 , 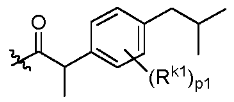 ,

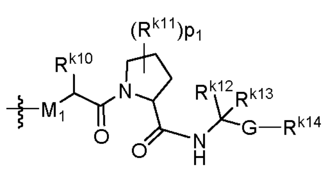 , 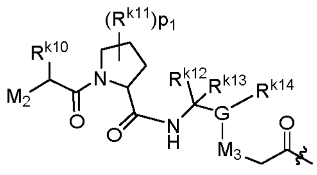 or 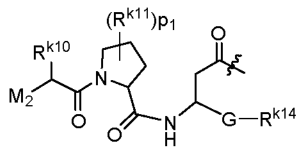 ;

in certain embodiments, K is selected from

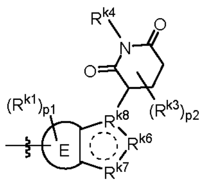 ,

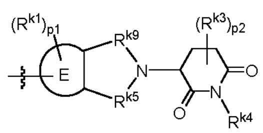 , 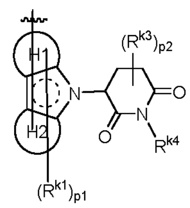 , 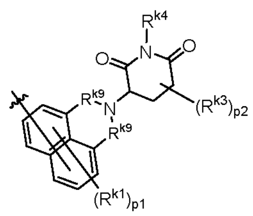 , 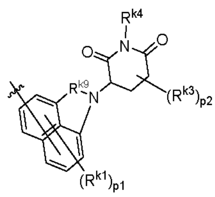 ,

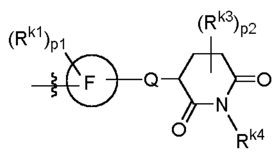 , 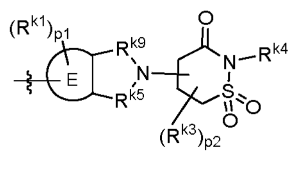 , 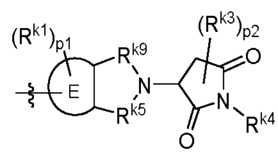 ,

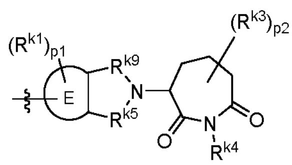 , 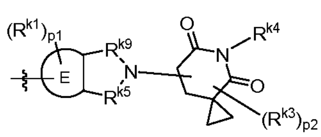 , 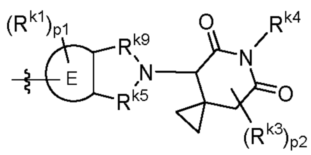 ,

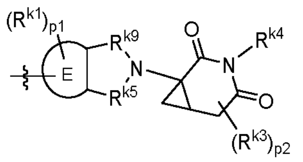 , 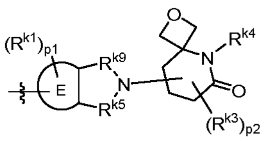 , 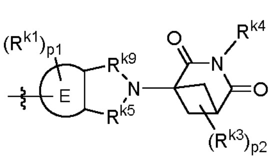 ,

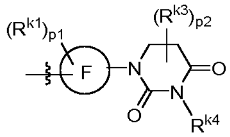 , 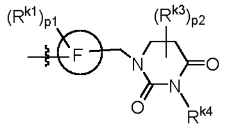 , 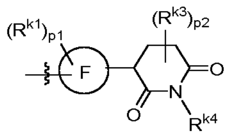 , 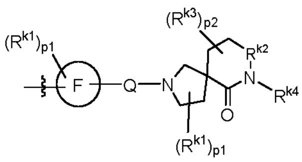 ,

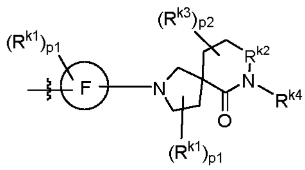 , 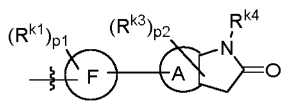 , 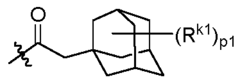 ,

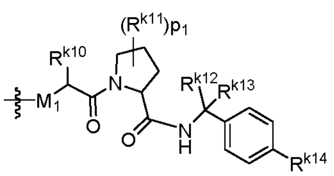 , 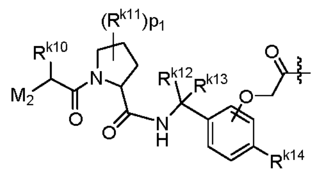 or 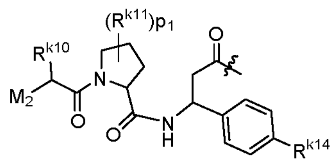 ,

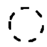

represents a ring selected from an aromatic ring or a non-aromatic ring;
in certain embodiments, K is selected from

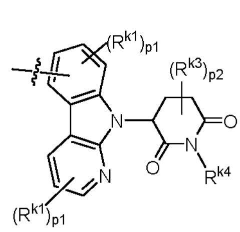 ,

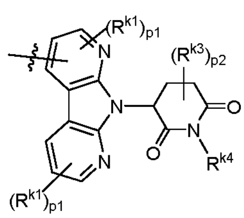 , 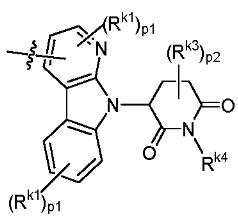 , 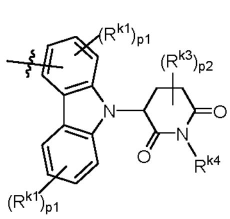 , 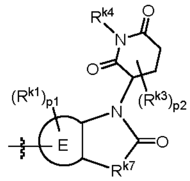 ,

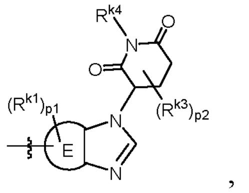 , 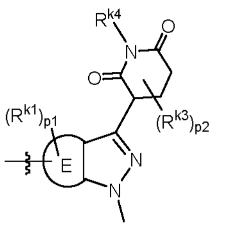 , 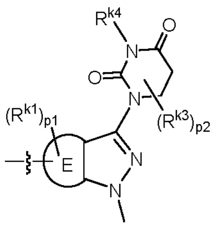 , 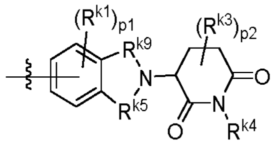 ,

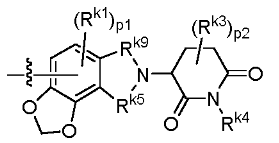 , 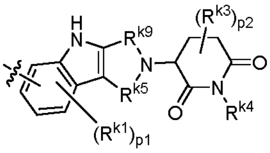 , 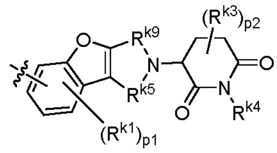

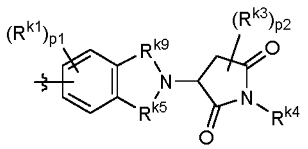 , 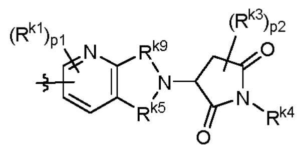 , 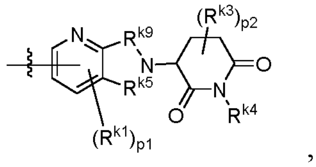 ,

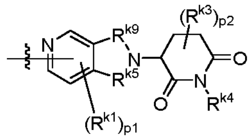 , 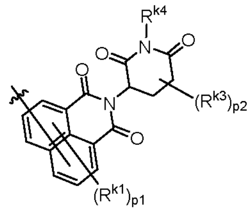 , 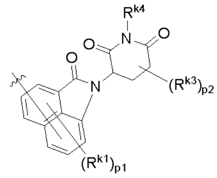 , 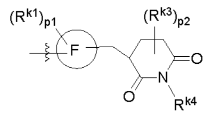 ,

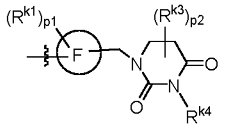 , 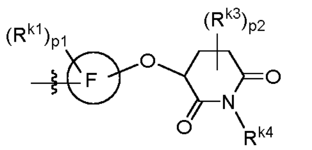 , 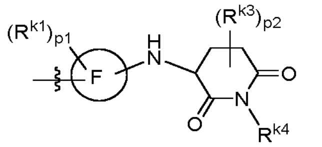 ,

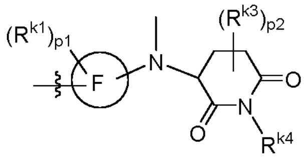 , 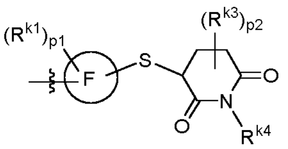 , 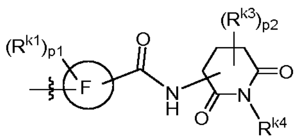 ,

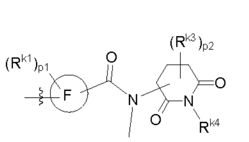 , 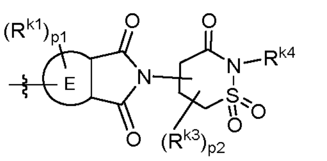 , 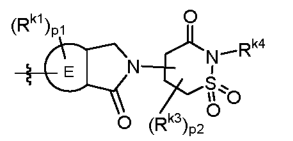 ,

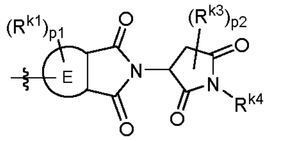 , 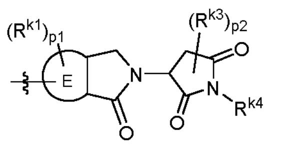 , 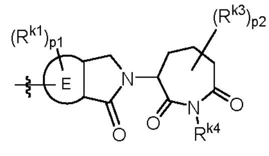 ,

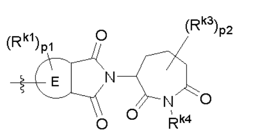, 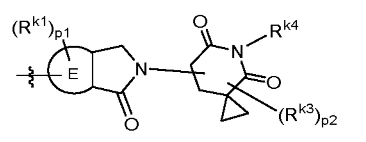, 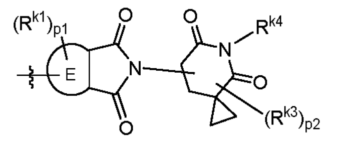,

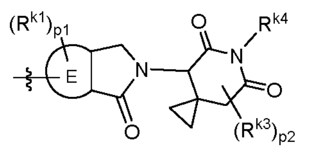, 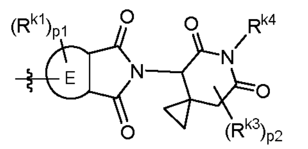, 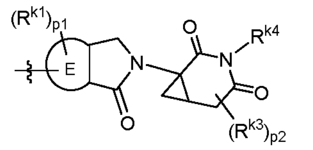,

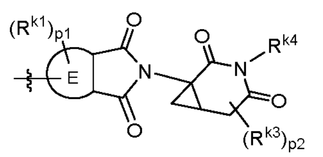, 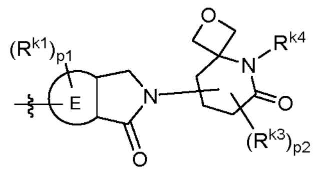, 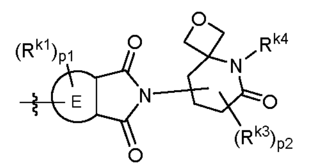,

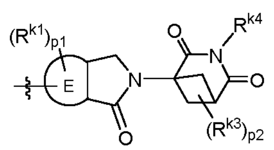, 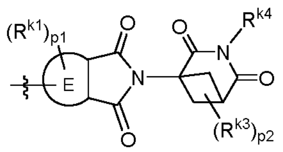, 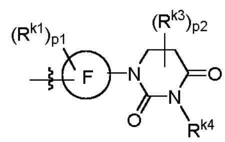, 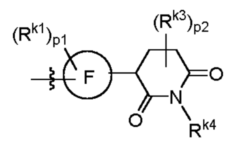,

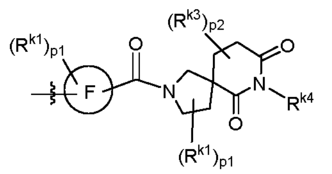, 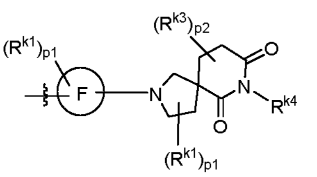, 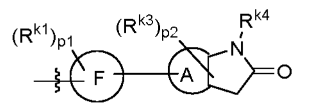,

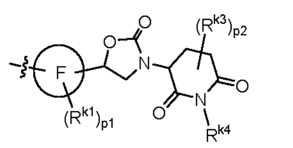, 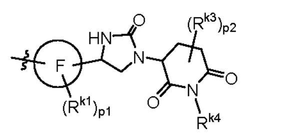, 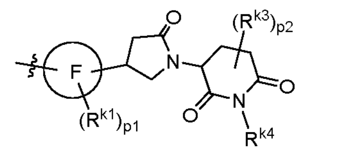,

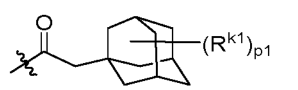, 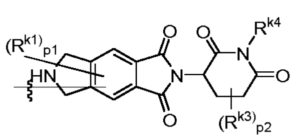, 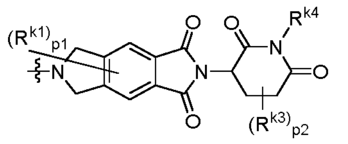,

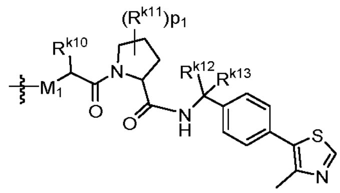, 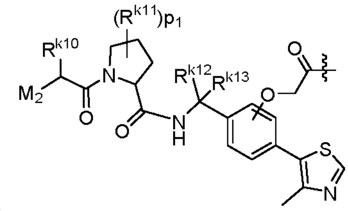, 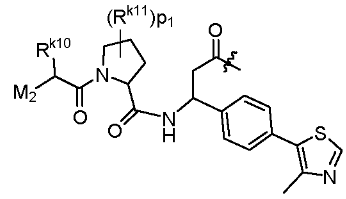,

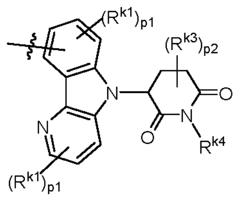 or 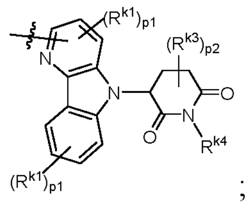 ;

in certain embodiments, each Q is independently selected from a bond, -O-, - S-, $-CH_2-$, $-NR^q-$, -C(=O)-, $-NR^qC(=O)-$, $-C(=O)NR^q-$ or 3- to 12-membered heterocyclyl, the heterocyclyl is optionally substituted with 0 to 4 (for example 0, 1, 2, 3, 4) substituents selected from H, F, Cl, Br, I, OH, =O, $NH_2$, CN, COOH, $CONH_2$, $C_{1-4}$ alkyl or $C_{1-4}$ alkoxy, the heterocyclyl contains 1 to 4 (for example, 1, 2, 3 or 4) heteroatoms selected from O, S or N;

in certain embodiments, each Q is independently selected from -O-, -S-, - $CH_2-$, $-NR^q-$, -C(=O)-, $-NR^qC(=O)-$, $-C(=O)NR^q-$ or 4- to 7-membered heterocyclyl, the heterocyclyl is optionally substituted with 0 to 4 (for example 0, 1, 2, 3, 4) substituents selected from H, F, Cl, Br, I, OH, =O, $NH_2$, CN, COOH, $CONH_2$, $C_{1-4}$ alkyl or $C_{1-4}$ alkoxy, the heterocyclyl contains 1 to 4 (for example, 1, 2, 3 or 4) heteroatoms selected from O, S or N;

in certain embodiments, $R^q$ is selected from H or $C_{1-6}$ alkyl;

in certain embodiments, $R^q$ is selected from H or $C_{1-4}$ alkyl;

in certain embodiments, $R^q$ is selected from H, methyl or ethyl;

in certain embodiments, each E is independently selected from $C_{3-10}$ carbocyclyl, $C_{6-10}$ aryl, 3- to 12-membered heterocyclyl or 5- to 12-membered heteroaryl, the heterocyclyl or heteroaryl contains 1 to 4 (for example, 1, 2, 3, or 4) heteroatoms selected from O, S or N;

in certain embodiments, each E is independently selected from $C_{3-8}$ carbocyclyl, phenyl, 4- to 7-membered heterocyclyl, 8- to 12-membered heterocyclyl, 7- to 12-membered heteroaryl or 5- to 6-membered heteroaryl, the heterocyclyl or heteroaryl contains 1 to 4 (for example, 1, 2, 3, or 4)heteroatoms selected from O, S or N;

in certain embodiments, each E is independently selected from phenyl, pyridyl, pyridazinyl, pyrazinyl, pyrimidinyl, pyrrolyl, pyrazolyl, imidazolyl, thiazolyl, furyl, thienyl, oxazolyl, indolinyl, isoindolinyl, 1,2,3,4-tetrahydroquinolinyl or 1,2,3,4-tetrahydroisoquinolinyl;

in certain embodiments, each E is independently selected from phenyl, pyridyl, pyridazinyl, pyrazinyl, pyrimidinyl, pyrrolyl, pyrazolyl, imidazolyl, thiazolyl, furyl, thienyl or oxazolyl;

in certain embodiments, each E is independently selected from phenyl, pyridyl, pyridazinyl, pyrazinyl or pyrimidinyl;

in certain embodiments, each E is independently selected from phenyl or pyridyl;

in certain embodiments, A is selected from $C_{3-10}$ carbocyclyl, $C_{6-10}$ aryl, 3- to 10-membered heterocyclyl or 5- to 10-membered heteroaryl, the heterocyclyl or heteroaryl contains 1 to 4 (for example, 1, 2, 3 or 4) heteroatoms selected from O, S or N;

in certain embodiments, each A, H1 or H2 is independently selected from $C_{3-8}$ carbocyclyl, phenyl, 4- to 7-membered heterocyclyl or 5- to 6-membered heteroaryl, the heterocyclyl or heteroaryl contains 1 to 4 (for example, 1, 2, 3 or 4) heteroatoms selected from O, S or N;

in certain embodiments, each A, H1 or H2 is independently selected from phenyl, pyridyl, pyridazinyl, pyrazinyl, pyrimidinyl, pyrrolyl, pyrazolyl, imidazolyl, thiazolyl, furyl, thienyl or oxazolyl;

in certain embodiments, each A, H1 or H2 is independently selected from phenyl or pyridyl;

in certain embodiments, each F is independently selected from $C_{3-20}$ carbocyclyl, $C_{6-20}$ aryl, 3- to 20-membered heterocyclyl or 5- to 20-membered heteroaryl, and the heterocyclyl or heteroaryl contains 1 to 4 (for example, 1, 2, 3 or 4) heteroatoms selected from O, S or N;

in certain embodiments, each F is independently selected from 3- to 7-membered monocycloalkyl, 4- to 10-membered fused cycloalkyl, 5- to 12-membered spirocycloalkyl, 5- to 10-membered bridged cycloalky, 4- to 7-membered mono-heterocyclic ring group, 4- to 10-membered fused heterocyclic ring group, 5- to 12-membered spiro-heterocyclic ring group, 5- to 10-membered bridged-heterocyclic ring group, $C_{6-14}$ aryl or 5- to 10-membered heteroaryl, the mono-heterocyclic ring group, fused heterocyclic ring group, spiro-heterocyclic ring group, bridged-heterocyclic ring group or heteroaryl contains 1 to 4 (for example, 1, 2, 3 or 4) heteroatoms selected from O, S or N;

in certain embodiments, each F is independently selected from a benzene ring or 5- to 6-membered heteroaromatic ring, the heteroaromatic ring contains 1 to 3 (for example, 1, 2, or 3) heteroatoms selected from O, S or N;

in certain embodiments, each F is independently selected from cyclopropyl, cyclobutyl, cyclopentyl, cyclohexyl, bicyclo[1.1.1]pentyl, 6,7-dihydro-5H-cyclopen[c]pyridyl, 2,3-dihydro-1H-indenyl, phenyl, naphthyl, anthracyl, phenanthrenyl, azetidinyl, azacyclopentyl, piperidinyl, morpholinyl, pyridyl, pyrimidinyl, pyridazinyl, pyrazinyl, triazinyl, pyrrolyl, pyrazolyl, imidazolyl, triazolyl, oxazolyl, furyl, thienyl, thiazolyl, 2-pyridone, benzoimidazolyl, benzopyrazolyl, benzothiazolyl, benzothienyl, benzofuryl, benzopyrrolyl, benzopyridyl, benzopyrazinyl, benzopyrimidinyl, benzopyridazinyl, benzotriazinyl, pyrrolopyrrolyl, pyrrolopyridyl, pyrrolopyrimidinyl, pyrrolopyridazinyl, pyrrolopyrazinyl, imidazopyrimidinyl, imidazopyridyl, imidazopyrazinyl, imidazopyridazinyl, pyrazolopyridyl, pyrazolopyrimidinyl, pyrazolopyridazinyl, pyrazolopyrazinyl, pyrimidopyridyl, pyrimidopyrazinyl, pyrimidopyridazinyl, pyrimidopyrimidinyl, pyridopyridyl, pyridopyrazinyl, pyridopyridazinyl, pyridazinopyridazinyl, pyridazinopyrazinyl or pyrazinopyrazinyl;
in certain embodiments, each $R^{k2}$ is independently selected from a bond, - C(=O)-, -S(=O)$_2$-, -S(=O)- or -C($R^{k3}$)$_2$-;
in certain embodiments, each $R^{k2}$ is independently selected from -C(=O)-, - S(=O)$_2$- or -C($R^{k3}$)$_2$-;
in certain embodiments, each $R^{k1}$ is independently selected from H, F, Cl, Br, I, OH, =O, $NH_2$, CN, COOH, $CONH_2$, $C_{1-6}$ alkyl or $C_{1-6}$ alkoxy, the alkyl or alkoxy is optionally substituted with 0 to 4 (for example, 0, 1, 2, 3, or 4) substituents selected from H, F, Cl, Br, I, OH, =O, $NH_2$, CN, COOH, $CONH_2$, $C_{1-4}$ alkyl or $C_{1-4}$ alkoxy;
in certain embodiments, each $R^{k3}$ is independently selected from H, F, Cl, Br, I, OH, =O, $NH_2$, CN, COOH, $CONH_2$, $C_{1-6}$ alkyl, $C_{1-6}$ alkoxy, $C_{3-8}$ cycloalkyl or 3-to 8-membered heterocyclyl, the alkyl, alkoxy, cycloalkyl or heterocyclyl is optionally substituted with 0 to 4 (for example, 0, 1, 2, 3, or 4) substituents selected from H, F, Cl, Br, I, OH, =O, $NH_2$, CN, COOH, $CONH_2$, $C_{1-4}$ alkyl or $C_{1-4}$ alkoxy, the heterocyclyl contains 1 to 4 (for example, 1, 2, 3, or 4) heteroatoms selected from O, S or N;
in certain embodiments, $R^{k1}$ and $R^{k3}$ are each independently selected from H, F, Cl, Br, I, OH, =O, $NH_2$, $CF_3$, CN, COOH, $CONH_2$, $C_{1-4}$ alkyl or $C_{1-4}$ alkoxy, the alkyl or alkoxy is optionally substituted with 0 to 4 (for example, 0, 1, 2, 3, or 4) substituents selected from H, F, Cl, Br, I, OH, or $NH_2$;
in certain embodiments, $R^{k1}$ and $R^{k3}$ are each independently selected from H, F, Cl, Br, I, OH, =O, $NH_2$, $CF_3$, CN, COOH, $CONH_2$, methyl, ethyl, isopropyl, methoxy, ethoxy or isopropoxy, the methyl, ethyl, isopropyl, methoxy, ethoxy or isopropoxy is optionally substituted with 0 to 4 (for example 0, 1, 2, 3 or 4) substituents selected from H, F, Cl, Br, I, OH, or $NH_2$;
in certain embodiments, two $R^{k3}$ together with the carbon atom or ring backbone to which they are directly attached form 3- to 8-membered carbocycle or 3- to 8-membered heterocycle, two $R^{k1}$ together with the carbon atom or ring backbone to which they are directly attached form 3- to 8-membered carbocycle or 3- to 8-membered heterocycle, the carbocycle or heterocycle is optionally substituted with 0 to 4 (for example, 0, 1, 2, 3, or 4) substituents selected from H, F, Cl, Br, I, OH, =O, $NH_2$, CN, COOH, $CONH_2$, $C_{1-4}$ alkyl or $C_{1-4}$ alkoxy, the heterocycle contains 1 to 4 (for example, 1, 2, 3, or 4) heteroatoms selected from O, S or N;
in certain embodiments, two $R^{k3}$ together with the carbon atom or ring backbone to which they are directly attached form 3- to 6-membered carbocycle or 3- to 7-membered heterocycle, two $R^{k1}$ together with the carbon atom or ring backbone to which they are directly attached form 3- to 6-membered carbocycle or 3- to 7-membered heterocycle, the carbocycle or heterocycle is optionally substituted with 0 to 4 (for example, 0, 1, 2, 3, or 4) substituents selected from H, F, Cl, Br, I, OH, =O, $NH_2$, CN, COOH, $CONH_2$, $C_{1-4}$ alkyl or $C_{1-4}$ alkoxy, the heterocycle contains 1 to 4 (for example, 1, 2, 3, or 4) heteroatoms selected from O, S or N;
in certain embodiments, each $R^{k4}$ is independently selected from H, OH, $NH_2$, CN, $CONH_2$, $C_{1-6}$ alkyl, $C_{3-8}$ cycloalkyl or 3- to 8-membered heterocyclyl, the alkyl, cycloalkyl or heterocyclyl is optionally substituted with 0 to 4 (for example, 0, 1, 2, 3, or 4) substituents selected from H, F, Cl, Br, I, OH, =O, $NH_2$, CN, COOH, $CONH_2$, $C_{1-4}$ alkyl or $C_{1-4}$ alkoxy, the heterocyclyl contains 1 to 4 (for example, 1, 2, 3, or 4) heteroatoms selected from O, S or N;
in certain embodiments, each $R^{k4}$ is independently selected from H, OH, $NH_2$, $CF_3$, CN or $C_{1-4}$ alkyl;
in certain embodiments, each $R^{k5}$ is independently selected from C(=O), $CH_2$, S(=O)$_2$,

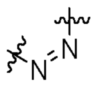, 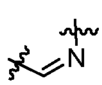, or 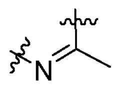;

in certain embodiments, each $R^{k5}$ is independently selected from C(=O), $CH_2$, S(=O)$_2$, or

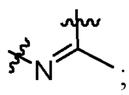;

in certain embodiments, each $R^{k6}$ is independently selected from C(=O), CH, S(=O), S(=O)$_2$, $CH_2$ or N;
in certain embodiments, each $R^{k7}$ is independently selected from C(=O), CH, N, $CH_2$, O, S, N($CH_3$) or NH;
in certain embodiments, each $R^{k7}$ is independently selected from $CH_2$, O, N($CH_3$) or NH;
in certain embodiments, each $R^{k8}$ is independently selected from C, N or CH;
in certain embodiments, each $R^{k9}$ is independently selected from C(=O), S(=O)$_2$, or $CH_2$;
it can be understood that the connection bonds between $R^{k6}$ and $R^{k7}$, and $R^{k7}$ and $R^{k8}$ can be selected from single bonds or double bonds according to whether they are aromatic;

in certain embodiments, $M_1$ is selected from a bond, -C(=O)NH-, -NHC(=O)-, $-CH_2-C(=O)NH-$, $-C(=O)CH_2NH-$, or 5- to 6-membered heteroaryl, the heteroaryl is optionally substituted with 0 to 4 (for example, 0, 1, 2, 3, or 4) substituents selected from H, F, Cl, Br, I, OH, =O, $CF_3$, $NH_2$, CN, $C_{1-4}$ alkyl, halogen-substituted $C_{1-4}$ alkyl, hydroxyl-substituted $C_{1-4}$ alkyl, or $C_{1-4}$ alkoxy, the heteroaryl contains 1 to 4 (for example, 1, 2, 3, or 4) heteroatoms selected from O, S or N;
in certain embodiments, M1 is selected from a bond, -C(=O)NH-, $-CH_2-C(=O)NH-$, $-C(=O)CH_2NH-$, pyrrolyl, pyrazolyl, imidazolyl, triazolyl, oxazolyl, isoxazolyl, furyl, thienyl, or thiazolyl;
in certain embodiments, $M_1$ is selected from a bond, $-CH_2-C(=O)NH-$ or - $C(=O)CH_2NH-$;
in certain embodiments, $M_2$ is selected from -NHC(=O)-$C_{1-6}$ alkyl, - NHC(=O)-$C_{3-6}$ cycloalkyl or 4- to 10-membered heterocyclyl, the alkyl, cycloalkyl or heterocyclyl is optionally substituted with 0 to 4 (for example, 0, 1, 2, 3, or 4) substituents selected from H, F, Cl, Br, I, =O, OH, $NH_2$, $C_{1-4}$ alkyl or $C_{1-4}$ alkoxy, the heterocyclyl contains 1 to 4 heteroatoms selected from O, S or N;
in certain embodiments, $M_2$ is selected from -NHC(=O)-$C_{1-4}$ alkyl, - NHC(=O)-$C_{3-6}$ cycloalkyl or 4- to 10-membered heterocyclyl, the alkyl, cycloalkyl or heterocyclyl is optionally substituted with 0 to 4 (for example, 0, 1, 2, 3, or 4) substituents selected from H, F, Cl, Br, I, =O, OH, $NH_2$, $C_{1-4}$ alkyl or $C_{1-4}$ alkoxy, the heterocyclyl contains 1 to 4 heteroatoms selected from O, S or N;
in certain embodiments, $M_2$ is selected from -NHC(=O)-$CH_3$, -NHC(=O)-cyclopropyl, -NHC(=O)-cyclobutyl, azetidinyl, azacyclopentyl, benzoazacyclopentyl, or benzoazacyclohexyl, the cyclopropyl, cyclobutyl, azetidinyl, azacyclopentyl, benzoazacyclopentyl or benzoazacyclohexyl is optionally substituted with 0 to 4 (for example, 0, 1, 2, 3, or 4) substituents selected from H, F, Cl, Br, I, =O, OH, $NH_2$, $C_{1-4}$ alkyl or $C_{1-4}$ alkoxy;
in certain embodiments, $M_3$ is selected from -NH- or -O-;
in certain embodiments, $R^{k10}$ is selected from $C_{1-6}$ alkyl, the alkyl is optionally substituted with 0 to 4 (for example, 0, 1, 2, 3, or 4) substituents selected from H, F, Cl, Br, I, =O, OH, $C_{1-6}$ alkyl or $C_{3-6}$ cycloalkyl;
in certain embodiments, $R^{k10}$ is selected from $C_{1-4}$ alkyl, the alkyl is optionally substituted with 0 to 4 (for example, 0, 1, 2, 3, or 4) substituents selected from H, F, Cl, Br, I, =O, OH, $C_{1-4}$ alkyl or $C_{3-6}$ cycloalkyl;
in certain embodiments, $R^{k10}$ is selected from methyl, ethyl, isopropyl, propyl, or tert-butyl, the methyl, ethyl, isopropyl, propyl, or tert-butyl is optionally substituted with 0 to 4 (for example, 0, 1, 2, 3, or 4) substituents selected from H, F, Cl, Br, I, =O, OH, $C_{1-4}$ alkyl or $C_{3-6}$ cycloalkyl;
in certain embodiments, G is selected from 6- to 10-membered aryl or 5- to 10-membered heteroaryl, the aryl or heteroaryl is optionally substituted with 0 to 4 (for example, 0, 1, 2, 3, or 4) substituents selected from H, F, Cl, Br, I, OH, =O, $CF_3$, CN, $C_{1-4}$ alkyl, halogen-substituted $C_{1-4}$ alkyl, hydroxyl-substituted $C_{1-4}$ alkyl, $C_{1-4}$ alkoxy or $C_{3-6}$ cycloalkyl, the heteroaryl contains 1 to 4 (for example, 1, 2, 3, or 4) heteroatoms selected from N, O and S;
in certain embodiments, each $R^{k11}$ is independently selected from H, F, Cl, Br, I, =O, OH, SH, $C_{1-6}$ alkyl, $C_{1-6}$ alkoxy or $C_{1-6}$ alkylthio or -O-C(=O)-$C_{1-6}$ alkyl, the alkyl, alkoxy or alkylthio is optionally substituted with 0 to 4 (for example, 0, 1, 2, 3, or 4) substituents selected from H, F, Cl, Br, I, OH, $C_{1-4}$ alkyl or $C_{1-4}$ alkoxy;
in certain embodiments, each $R^{k11}$ is independently selected from H, F, Cl, Br, I, =O, OH, SH, $C_{1-4}$ alkyl, $C_{1-4}$ alkoxy or $C_{1-4}$ alkylthio or -O-C(=O)-$C_{1-4}$ alkyl, the alkyl, alkoxy or alkylthio is optionally substituted with 0 to 4 (for example, 0, 1, 2, 3, or 4) substituents selected from H, F, Cl, Br, I, OH, $C_{1-4}$ alkyl or $C_{1-4}$ alkoxy;
in certain embodiments, each $R^{k11}$ is independently selected from H, F, Cl, Br, I, =O, OH, SH, methyl, ethyl, isopropyl, propyl, methoxy, ethoxy, propoxy, isopropoxy, methylthio, ethylthio, propylthio or -O-C(=O)-$CH_3$, the methyl, ethyl, isopropyl, propyl, methoxy, ethoxy, propoxy, isopropoxy, methylthio, ethylthio, or propylthio is optionally substituted with 0 to 4 (for example, 0, 1, 2, 3, or 4) substituents selected from H, F, Cl, Br, I, OH, $C_{1-4}$ alkyl or $C_{1-4}$ alkoxy;
in certain embodiments, $R^{k12}$ and $R^{k13}$ are each independently selected from H, $C_{1-6}$ alkyl or $C_{3-6}$ cycloalkyl, the alkyl or cycloalkyl is optionally substituted with 0 to 4 (for example, 0, 1, 2, 3, or 4) substituents selected from H, F, Cl, Br, I, =O, OH, $NH_2$, $C_{1-4}$ alkyl or $C_{1-4}$ alkoxy;
in certain embodiments, $R^{k12}$ and $R^{k13}$ are each independently selected from H, C1-4 alkyl or $C_{3-6}$ cycloalkyl, the alkyl or cycloalkyl is optionally substituted with 0 to 4 (for example, 0, 1, 2, 3, or 4) substituents selected from H, F, Cl, Br, I, =O, OH, $NH_2$, $C_{1-4}$ alkyl or $C_{1-4}$ alkoxy;
in certain embodiments, $R^{k12}$ and $R^{k13}$ are each independently selected from H, methyl, ethyl, isopropyl, propyl, cyclopropyl or cyclobutyl, the methyl, ethyl, isopropyl, propyl, cyclopropyl or cyclobutyl is optionally substituted with 0 to 4 (for example, 0, 1, 2, 3, or 4) substituents selected from H, F, Cl, Br, I, =O, OH, $NH_2$, $C_{1-4}$ alkyl or $C_{1-4}$ alkoxy;
in certain embodiments, $R^{k14}$ is selected from 5- to 6-membered heteroaryl, the heteroaryl is optionally substituted with 0 to 4 (for example, 0, 1, 2, 3, or 4) substituents selected from H, F, Cl, Br, I, OH, =O, $CF_3$, CN, $C_{1-4}$ alkyl, halogen-substituted $C_{1-4}$ alkyl, hydroxyl-substituted $C_{1-4}$ alkyl, $C_{1-4}$ alkoxy or $C_{3-6}$ cycloalkyl, the heteroaryl contains 1 to 4 (for example, 1, 2, 3, or 4) heteroatoms selected from N, O and S;
in certain embodiments, K is selected from one of the structural fragments shown in Table K-1;
in certain embodiments, K is selected from one of the structural fragments shown in Table K-2;
in certain embodiments, K is selected from one of the structural fragments shown in Table K-3;

in certain embodiments, B is selected from

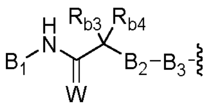
;

in certain embodiments, B is selected from

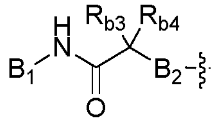
,
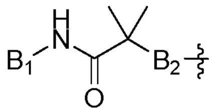
,

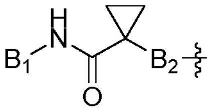
,
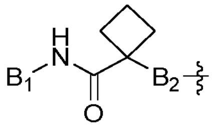
,
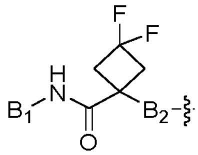
,
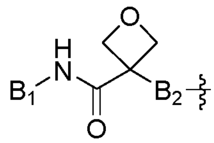
,

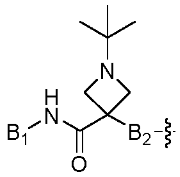
,
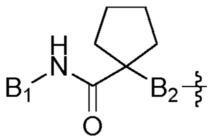
,
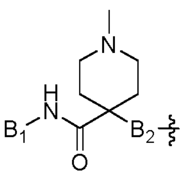
, or
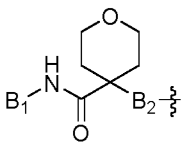
;

in certain embodiments, B is selected from

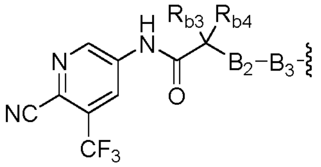

or

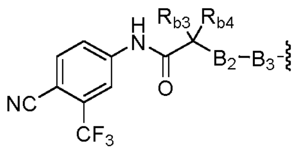
;

in certain embodiments, W is selected from O or S;

in certain embodiments, W is selected from O;

in certain embodiments, $B_1$ is selected from $C_{6-10}$ carbocyclyl or 5- to 10-membered heterocyclyl, the carbocyclyl or heterocyclyl is optionally substituted with 0 to 2 $R^{bb}$, the carbocyclyl or heterocyclyl is optionally substituted with 0 to 4 $R^{b1}$ (*i.e.*, the carbocyclyl or heterocyclyl is optionally substituted with 0 to 2 $R^{bb}$ and 0 to 4 $R^{b1}$), the heterocyclyl contains 1 to 4 heteroatoms selected from O, S or N;

in certain embodiments, $B_1$ is selected from $C_{6-10}$ carbocyclyl or 5- to 10-membered heterocyclyl, the carbocyclyl or heterocyclyl is optionally substituted with 0 to 4 $R^{b1}$, the heterocyclyl contains 1 to 4 heteroatoms selected from O, S or N;

in certain embodiments, $B_1$ is selected from $C_{6-10}$ aryl, 5- to 10-membered heteroaryl, $C_{6-10}$ cycloalkyl, $C_{6-10}$ car-

bocyclyl, or 5- to 10-membered heterocyclyl, the aryl, heteroaryl, cycloalkyl, carbocyclyl or heterocyclyl is optionally substituted with 1 or 2 $R^{bb}$, the aryl, heteroaryl, cycloalkyl, carbocyclyl or heterocyclyl is optionally substituted with 0 to 3 $R^{b1}$, the heteroaryl or heterocyclyl contains 1 to 4 heteroatoms selected from O, S or N;

in certain embodiments, $B_1$ is selected from $C_{6-10}$ aryl, 5- to 10-membered heteroaryl, $C_{6-10}$ cycloalkyl, $C_{6-10}$ carbocyclyl, or 5- to 10-membered heterocyclyl, the aryl, heteroaryl, cycloalkyl, carbocyclyl or heterocyclyl is optionally substituted with 1 or 2 $R^{bb}$, the aryl, heteroaryl, cycloalkyl, carbocyclyl or heterocyclyl is optionally substituted with 0 to 3 $R^{b1}$, the heteroaryl or heterocyclyl contains 1 to 4 heteroatoms selected from O, S or N, at least one of the 2 ortho-positions connecting B1 to the amide is not H;

in certain embodiments, at least 1 of the 2 ortho-positions connecting B1 to the amide is not H;

in certain embodiments, $B_1$ is selected from phenyl, naphthyl, thienyl, furyl, pyrrolyl, pyridyl, pyrimidinyl, pyrazinyl, pyridazinyl, quinolinyl, isoquinolinyl, quinazolinyl, 3,4-dihydro-1H-benzopyranyl, or 1,2,3,4-tetrahydroquinolinyl, the $B_1$ is optionally substituted with 0 to 4 $R^{b1}$;

in certain embodiments, $B_1$ is selected from benzofuryl, benzothienyl, benzopyrrolyl, benzoxazolyl, benzothiazolyl, benzoimidazolyl, benzopyrazolyl, azacyclopentyl, azacyclohexyl, oxacyclopentyl, oxacyclohexyl, adamantyl, bicyclo[2.2.2]octyl, or 1,2,3,4-tetrahydronaphthyl, the $B_1$ is optionally substituted with 0 to 4 $R^{b1}$;

in certain embodiments, $B_1$ is selected from phenyl, naphthyl, thienyl, furyl, pyrrolyl, or pyridyl, the $B_1$ is optionally substituted with 0 to 4 $R^{b1}$;

in certain embodiments, $B_1$ is selected from phenyl, naphthyl, thienyl, furyl, pyrrolyl, pyridyl, pyrimidinyl, pyrazinyl, pyridazinyl, quinolinyl, isoquinolinyl, quinazolinyl, benzofuryl, benzothienyl, benzopyrrolyl, benzoxazolyl, benzothiazolyl, benzoimidazolyl, benzopyrazolyl, azacyclopentyl, azacyclohexyl, oxacyclopentyl, oxacyclohexyl, adamantyl, bicyclo[2.2.2]octyl,

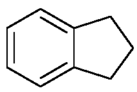,

or

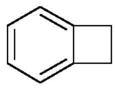,

the $B_1$ is optionally substituted with 1 or 2 $R^{bb}$, and the $B_1$ is optionally substituted with 0 to 3 $R^{b1}$;

in certain embodiments, $B_1$ is selected from phenyl, naphthyl, 1,2,3,4-tetrahydronaphthyl, pyridyl,

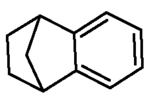, or 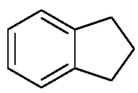,

the $B_1$ is optionally substituted with 1 or 2 $R^{bb}$, and the $B_1$ is optionally substituted with 0 to 3 $R^{b1}$;

in certain embodiments, $B_1$ is selected from

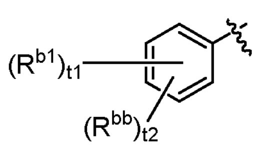,

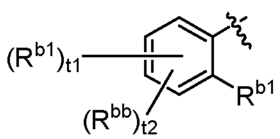, 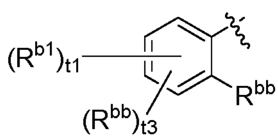, 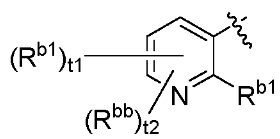,

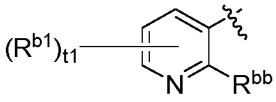, 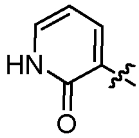, or 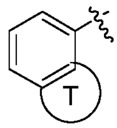,

ring T is selected from $C_{4-6}$ carbocycle or 4- to 6-membered heterocycle, the

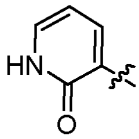 or 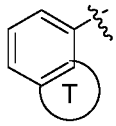

is optionally substituted with 1 or 2 $R^{bb}$ or optionally substituted with 1 to 3 $R^{b1}$, t1 is selected from 0, 1 or 2, t2 is selected from 0, 1 or 2, and t3 is selected from 0 or 1;

in certain embodiments, $B_1$ is selected from

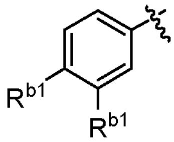, 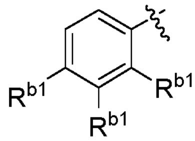,

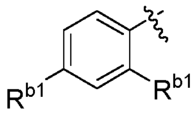, 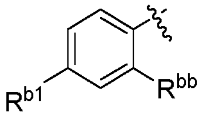, 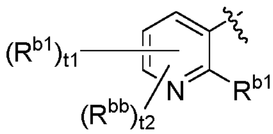, 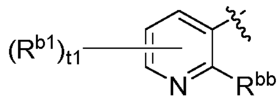,

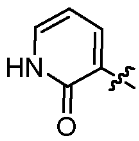, or 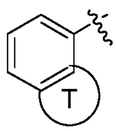

, ring T is selected from $C_{4-6}$ carbocycle or 4- to 6-membered heterocycle, the

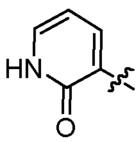 or 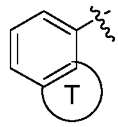

is optionally substituted with 1 or 2 $R^{bb}$ or optionally substituted with 1 to 3 $R^{b1}$, t1 is selected from 0, 1 or 2, t2 is selected from 0, 1 or 2, and t3 is selected from 0 or 1;

in certain embodiments, $B_1$ is selected from

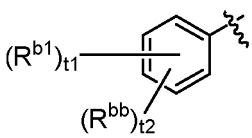,

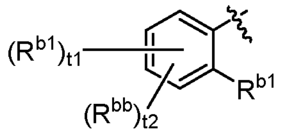, 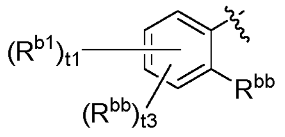, 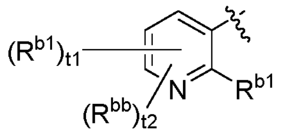,

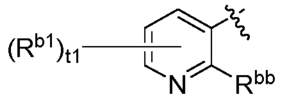, 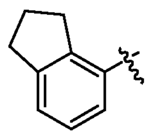, 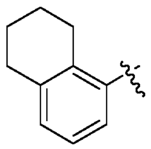, 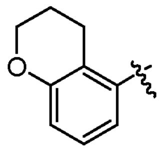, 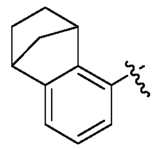,

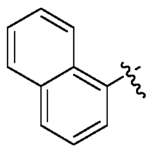, 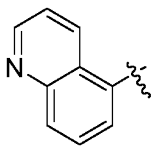, or 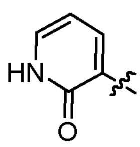, the 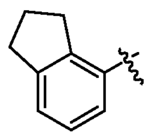, 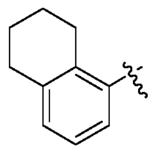, 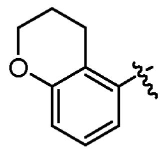,

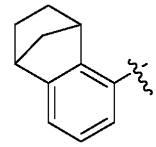, 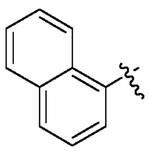, 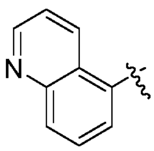, or 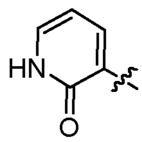

is optionally substituted with 1 or 2 $R^{bb}$ or optionally substituted with 1 to 3 $R^{b1}$, t1 is selected from 0, 1 or 2, t2 is selected from 0, 1 or 2, and t3 is selected from 0 or 1;
in certain embodiments, $B_1$ is selected from

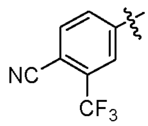, 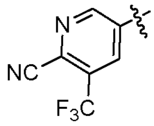,

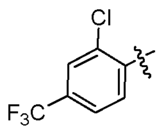, 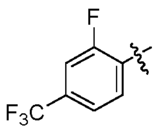, 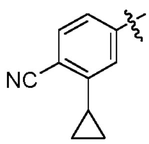, 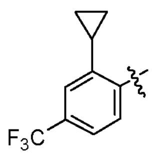, 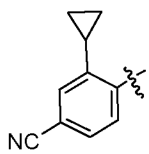, 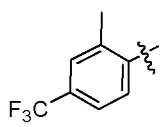,

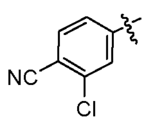, 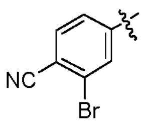, 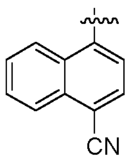, 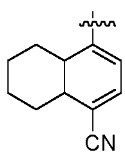, 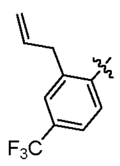, 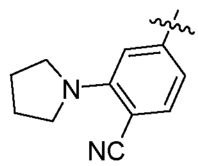,

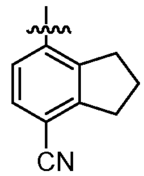, 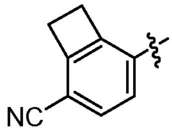, 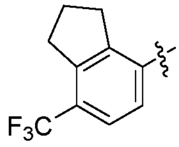, 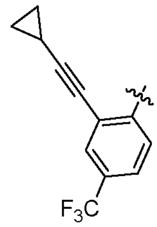, 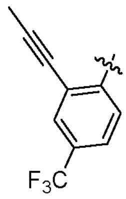, 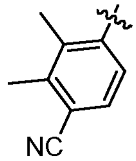,

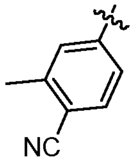, 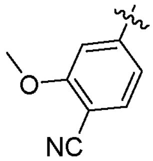, 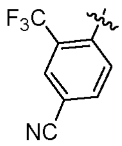, 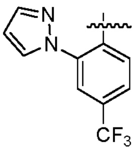, 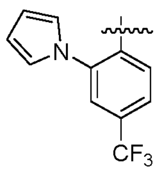, 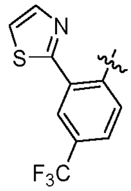, 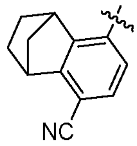

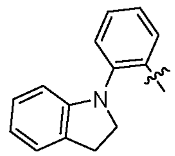, 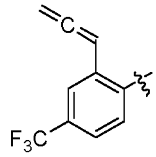, or 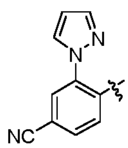;

in certain embodiments, $B_1$ is not

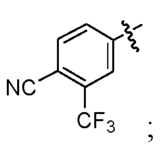;

in certain embodiments, $B_2$ is selected from -NHC(=O)- or 5- to 10-membered heterocyclyl, the heterocyclyl is optionally substituted with 0 to 4 $R^{b2}$, the heterocyclyl contains 1 to 4 heteroatoms selected from O, S or N;
in certain embodiments, $B_2$ is selected from -NHC(=O)- or unsaturated 5- to 10-membered heterocyclyl, the heterocyclyl is optionally substituted with 0 to 4 $R^{b2}$, the heterocyclyl contains 1 to 4 heteroatoms selected from O, S or N;
in certain embodiments, $B_2$ is selected from -NHC(=O)-, pyrazolyl, imidazolyl, triazolyl, thiazolyl, oxazolyl, benzopyrrolyl, benzoimidazolyl, pyrazolotetrahydropyrrolyl, or 3-pyridazinonyl, the pyrazolyl, imidazolyl, thiazolyl, oxazolyl, triazolyl, benzopyrrolyl, benzoimidazolyl, pyrazolotetrahydropyrrolyl, or 3-pyridazinonyl is optionally substituted with 0 to 4 $R^{b2}$;
in certain embodiments, $B_2$ is selected from substituted or unsubstituted benzopyrazolyl, 2-pyridonyl, 1,2,3,4-tetrahydroquinolinyl, 1,2,3,4-tetrahydroisoquinolinyl, azacyclohexyl,

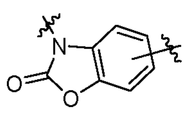, 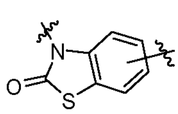,

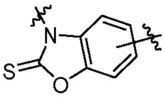, 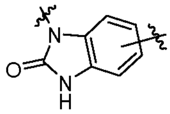, 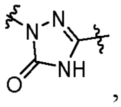, or 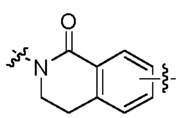,

and when substituted, is optionally substituted with 0 to 4 $R^{b2}$;
in certain embodiments, $B_2$ is selected from -NHC(=O)- or one of the following substituted or unsubstituted groups: pyrazolyl, imidazolyl, triazolyl, thiazolyl, oxazolyl, benzopyrrolyl, benzoimidazolyl, pyrazolotetrahydropyrrolyl, 3-pyridazinonyl, benzopyrazolyl, 2-pyridonyl, 1,2,3,4-tetrahydroquinolinyl, 1,2,3,4-tetrahydroisoquinolinyl, azacyclohexyl,

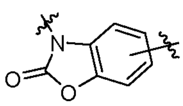, 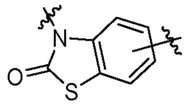,

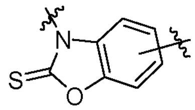, 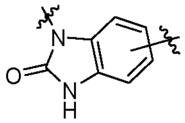, 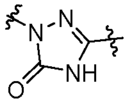, 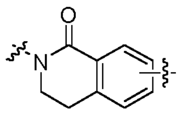, 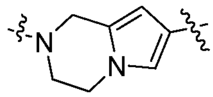,

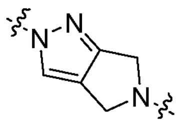, 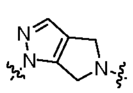, or 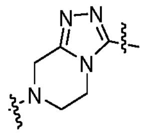,

and when substituted, is optionally substituted with 0 to 4 $R^{b2}$,
in certain embodiments, $B_2$ is selected from pyrazolyl, the pyrazolyl is optionally substituted with 1 to 2 $R^{b2}$;
in certain embodiments, $B_3$ is selected from a bond, phenyl or 5- to 6-membered heteroaryl, the phenyl and 5- to 6-membered heteroaryl are optionally substituted with 0 to 4 $R^{b2}$,
in certain embodiments, $B_3$ is selected from a bond or phenyl, the phenyl is optionally substituted with 0 to 4 $R^{b2}$;
in certain embodiments, $B_3$ is selected from a bond;
in certain embodiments, $B_1$ is selected from phenyl, naphthyl, 1,2,3,4-tetrahydronaphthyl,

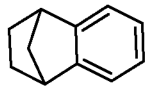, 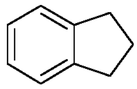

or 5- to 6-membered heteroaryl, $B_2$ is selected from 5- to 6-membered heteroaromatic ring containing nitrogen atoms, the $B_1$ is optionally substituted with 1 or 2 $R^{bb}$, the $B_1$ is optionally substituted with 0 to 3 $R^{b1}$, the $B_2$ is optionally substituted with 1 to 2 $R^{b2}$, $B_3$ is selected from a bond, and L is preferably -Ak1-Cy1-;
in certain embodiments, $B_1$ is selected from phenyl, naphthyl, 1,2,3,4-tetrahydronaphthyl,

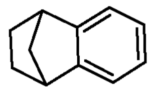, 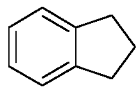

or 5- to 6-membered heteroaryl, $B_2$ is selected from pyrazolyl, $B_3$ is selected from a bond, $B_1$ is optionally substituted with 1 or 2 $R^{bb}$, the $B_1$ is optionally substituted with 0 to 3 $R^{b1}$, the $B_2$ is optional substituted with 1 to 2 $R^{b2}$, L is preferably

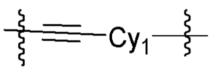,

Cy1 is selected from 4- to 10-membered nitrogen-containing heterocyclic ring, preferably 4- to 6-membered nitrogen-containing mono-heterocyclic ring (such as azetidinyl), the Cy1 is optionally substituted with 1 to 4 substituents selected from F, Cl, Br, I, OH, COOH, CN, $NH_2$, $C_{1-4}$ alkyl, halogen-substituted $C_{1-4}$ alkyl, hydroxyl-substituted $C_{1-4}$ alkyl or $C_{1-4}$ alkoxy, the nitrogen-containing heterocyclic ring contains 1 N;
in certain embodiments, $R^{bb}$ is selected from $-T-P(=O)_2(C_{1-4}\ alkyl)_2$, $-T-S(=O)_2C_{1-4}$ alkyl, $-T-P(=O)_2(C_{3-6}\ cycloalkyl)_2$, $-T-S(=O)_2C_{3-6}$ cycloalkyl, $-T-C(=O)NH_2$, $-T-C(=O)NH-C_{1-4}$ alkyl, $-T-C(=O)N(C_{1-4}\ alkyl)_2$, $-T-C_{1-4}$ alkyl, $-T-C_{2-6}$ alkenyl, $-T-C_{2-6}$ alkynyl, -T- $C_{3-12}$ carbocycle, or -T-3- to 12-membered heterocycle, the alkyl, alkenyl, alkynyl, carbocycle or heterocycle is optionally substituted with 0 to 4 substituents selected from H, F, Cl, Br, I, OH, $NH_2$, =O, halogen-substituted $C_{1-4}$ alkyl, cyano-substituted $C_{1-4}$ alkyl, hydroxyl-substituted $C_{1-4}$ alkyl, $C_{1-4}$ alkyl, $C_{1-4}$ alkoxy, $C_{3-6}$ cycloalkyl or 3- to 7-membered heterocycle, the heterocycle contains 1 to 4 heteroatoms selected from O, S or N;
in certain embodiments, $R^{bb}$ is selected from $-P(=O)_2(CH_3)_2$, $-S(=O)_2CH_3$, $-T-C(=O)NH_2$, $-T-C(=O)NHCH_3$, -T-

$C(=O)N(CH_3)_2$, -T-$C(=O)NHCH_2CH_3$, -T-$C(=O)N(CH_2CH_3)_2$, -T-$C_{3-6}$ monocyclic cycloalkyl, -T-phenyl, -$OCH_2CH_2OCH_3$, - $OCH_2CH_2OCH_2CH_3$, -T-5- to 6-membered heteroaryl, -T-4- to 6-membered monocyclic heterocycloalkyl, -T-6- to 10-membered spirocyclic heterocycloalkyl, - T-6- to 10-membered fused cyclic heterocycloalkyl, -T-benzopyrazolyl, -T-benzoimidazolyl, -T-benzopyrrolyl, -T-indolinyl, -T-vinyl, -T-propan-1,2-dien-1-yl, -T-ethynyl, -T-propynyl, or -T-propargyl, the $CH_2$, $CH_3$, cycloalkyl, phenyl, heterocycloalkyl, heteroaryl, benzopyrazolyl, benzoimidazolyl, benzopyrrolyl, indolinyl, vinyl, prop-1,2-dien-1-yl, ethynyl, propynyl, or propargyl is optionally substituted with 0 to 4 substituents selected from H, F, Cl, Br, I, OH, $NH_2$, =O, halogen-substituted $C_{1-4}$ alkyl, cyano-substituted $C_{1-4}$ alkyl, hydroxyl-substituted $C_{1-4}$ alkyl, $C_{1-4}$ alkyl, $C_{1-4}$ alkoxy, $C_{3-6}$ cycloalkyl or 3- to 7-membered heterocycloalkyl, the heterocycloalkyl contains 1 to 4 heteroatoms selected from O, S or N;

in certain embodiments, $R^{bb}$ is selected from -T-methyl or -T-ethyl, the methyl or ethyl is substituted with 1 to 4 substituents selected from F, Cl, Br, I, OH, $NH_2$, =O, halogen-substituted $C_{1-4}$ alkyl, cyano-substituted $C_{1-4}$ alkyl, hydroxyl-substituted $C_{1-4}$ alkyl, $C_{1-4}$ alkyl, $C_{1-4}$ alkoxy, $C_{3-6}$ cycloalkyl or 3- to 7-membered heterocycloalkyl, the heterocycloalkyl contains 1 to 4 heteroatoms selected from O, S or N;

in certain embodiments, $R^{bb}$ is selected from -$P(=O)_2(CH_3)_2$, -$S(=O)_2CH_3$, - $OCH_2CH_2OCH_3$, -$OCH_2C(=O)NH(CH_3)$, -T-$C(=O)NH_2$, -T-$C(=O)NHCH_3$, -T-$C(=O)N(CH_3)$, -T-cyclopropyl, -T-cyclobutyl, -T-cyclopentyl, -T-cyclohexyl, -T-azetidinyl, -T-azacyclopentyl, -T-azacyclohexyl, -T-oxetanyl, -T-oxacyclopentyl, - T-oxacyclohexyl, -T-phenyl, -T-pyridyl, -T-pyrazolyl, -T-pyrrolyl, -T-imidazolyl, - T-morpholinyl, -T-cyclopropyl-spiro-azacyclohexyl, -T-cyclopentyl-fused-azacyclopentyl, -T-benzopyrazolyl, -T-benzoimidazolyl, -T-benzopyrrolyl, -T-dihydroindolyl, -T-vinyl, -T-ethynyl, -T-propynyl, or -T-propargyl, the $CH_2$, $CH_3$, cyclopropyl, cyclobutyl, cyclopentyl, cyclohexyl, azetidinyl, azacyclopentyl, azacyclohexyl, oxetanyl, oxacyclopentyl, oxacyclohexyl, phenyl, pyridyl, pyrazolyl, pyrrolyl, imidazolyl, morpholinyl, cyclopropyl-spiro-azacyclohexyl, cyclopentyl-fused-azacyclopentyl, benzopyrazolyl, benzoimidazolyl, benzopyrrolyl, dihydroindolyl, vinyl, ethynyl, propynyl, or propargyl is optionally substituted with 0 to 4 substituents selected from H, F, Cl, Br, I, OH, =O, $CF_3$, methyl, ethyl, methoxy, ethoxy, or cyclopropyl;

in certain embodiments, $R^{bb}$ is selected from -$P(=O)_2(CH_3)_2$, -$S(=O)_2CH_3$, - $OCH_2CH_2OCH_3$, -T-$C(=O)NH_2$, -T-$C(=O)NHCH_3$, -T-$C(=O)N(CH_3)$, -T-cyclopropyl, -T-cyclobutyl, -T-cyclopentyl, -T-cyclohexyl, -T-azetidinyl, -T-azacyclopentyl, -T-azacyclohexyl, -T-oxetanyl, -T-oxacyclopentyl, -T-oxacyclohexyl, -T-phenyl, -T-pyridyl, -T-pyrrolyl, -T-thienyl, -T-furyl, -T-pyrazolyl, -T-pyrrolyl, -T-imidazolyl, -T-thiazolyl, -T-oxazolyl, -T-triazolyl, -T-pyridazinyl, -T-pyridazinone, -T-morpholinyl, -T-cyclopropyl-spiro-azacyclohexyl, -T-cyclopentyl-fused-azacyclopentyl, -T-benzopyrazolyl, -T-benzoimidazolyl, -T-benzopyrrolyl, -T-dihydroindolyl, -T-vinyl, -T-prop-1,2-dien-1-yl, -T-ethynyl, -T-propynyl, or -T-propargyl, the $CH_2$, $CH_3$, cyclopropyl, cyclobutyl, cyclopentyl, cyclohexyl, azetidinyl, azacyclopentyl, azacyclohexyl, oxetanyl, oxacyclopentyl, oxacyclohexyl, phenyl, pyridyl, pyrrolyl, thienyl, furyl, pyrazolyl, pyrrolyl, imidazolyl, thiazolyl, oxazolyl, triazolyl, pyridazinyl, pyridazinone, morpholinyl, cyclopropyl-spiro-azacyclohexyl, cyclopentyl-fused-azacyclopentyl, benzopyrazolyl, benzoimidazolyl, benzopyrrolyl, dihydroindolyl, vinyl, prop-1,2-dien-1-yl, ethynyl, propynyl, or propargyl is optionally substituted with 0 to 4 substituents selected from H, F, Cl, Br, I, OH, =O, $CF_3$, methyl, ethyl, methoxy, ethoxy, cyclopropyl, azetidinyl, azacyclopentyl, piperidinyl or piperazine;

in certain embodiments, $R^{bb}$ is selected from -T-methyl or -T-ethyl, the methyl or ethyl is substituted with 1 to 4 substituents selected from F, Cl, Br, I, OH, =O, $CF_3$, methyl, ethyl, methoxy, ethoxy, or cyclopropyl;

in certain embodiments, T is selected from a bond, -C(=O)-, -$P(=O)_2$-, - $S(=O)_2$-, -C(=O)NH-, -$P(=O)_2NH$-, -$S(=O)_2NH$-, -C(=O)N($C_{1-4}$ alkyl)-, - $P(=O)_2N$($C_{1-4}$ alkyl)-, -$S(=O)_2N$($C_{1-4}$ alkyl)-, -NHC(=0)-, -NHP$(=O)_2$-, - NHS$(=O)_2$-, -N($C_{1-4}$ alkyl)C(=O)-, -N($C_{1-4}$ alkyl)P$(=O)_2$-, -N($C_{1-4}$ alkyl)S$(=O)_2$-, O, S, NH, N($C_{1-4}$ alkyl), N($C_{3-6}$ cycloalkyl), N(3- to 10-membered heterocycle), $C_{1-4}$ alkylene, -O-$C_{1-4}$ alkylene-, -$C_{1-4}$ alkylene-O-, -S-$C_{1-4}$ alkylene-, -NH-$C_{1-4}$ alkylene-, -N($C_{1-4}$ alkyl)-$C_{1-4}$ alkylene-, or -O-$C_{1-4}$ alkylene-O-, the alkyl or alkylene is optionally substituted with 0 to 4 substituents selected from H, F, Cl, Br, I, OH, =O, $C_{1-4}$ alkyl, $C_{1-4}$ alkoxy, $C_{3-6}$ cycloalkyl or 3- to 7-membered heterocycle, the heterocycle contains 1 to 4 heteroatoms selected from O, S or N;

in certain embodiments, T is selected from a bond, -C(=O)-, O, S, NH, N($CH_3$), -$CH_2$-, -$CH_2CH_2$-, -$OCH_2O$-, -$OCH_2$-, -$CH_2O$-, -$OCH_2CH_2O$-, - $OCH_2CH_2$-, or -$CH_2CH_2O$-, the $CH_2$ or $CH_3$ is optionally substituted with 0 to 4 substituents selected from H, F, Cl, Br, I, OH, =O, $C_{1-4}$ alkyl, $C_{1-4}$ alkoxy, $C_{3-6}$ cycloalkyl or 3- to 7-membered heterocycle;

in certain embodiments, T is selected from a bond, -C(=O)-, O, S, NH, N($CH_3$), -$CH_2$-, -$CH_2CH_2$-, -$OCH_2O$-, -$OCH_2$-, -$CH_2O$-, -$OCH_2CH_2O$-, - $OCH_2CH_2$-, or -$CH_2CH_2O$-, the $CH_2$ or $CH_3$ is optionally substituted with 0 to 4 substituents selected from H, F, Cl, Br, I, OH, =O, methyl, ethyl, methoxy, ethoxy, or cyclopropyl;

in certain embodiments, 2 $R^{bb}$ together with the atom to which they are attached form $C_{4-12}$ carbocycle or 4- to 12-membered heterocycle (preferably $C_{6-12}$ carbocycle or 6- to 12-membered heterocycle), the carbocycle or heterocycle is optionally substituted with 0 to 4 substituents selected from H, F, Cl, Br, I, OH, $NH_2$, =O, halogen-substituted $C_{1-4}$ alkyl, cyano-substituted $C_{1-4}$ alkyl, hydroxyl-substituted $C_{1-4}$ alkyl, $C_{1-4}$ alkyl, $C_{1-4}$ alkoxy, $C_{3-6}$ cycloalkyl or 3- to 7-membered heterocycle, the heterocycle contains 1 to 4 heteroatoms selected from O, S or N;

in certain embodiments, 2 $R^{bb}$ together with the atom form to which they are attached non-aromatic $C_{4-12}$ carbocycle or 4- to 12-membered heterocycle (preferably $C_{6-12}$ carbocycle or 6- to 12-membered heterocycle), the carbocycle or heterocycle is optionally substituted with 0 to 4 substituents selected from H, F, Cl, Br, I, OH, $NH_2$, =O, halogen-

substituted $C_{1-4}$ alkyl, cyano-substituted $C_{1-4}$ alkyl, hydroxyl-substituted $C_{1-4}$ alkyl, $C_{1-4}$ alkyl, $C_{1-4}$ alkoxy, $C_{3-6}$ cycloalkyl or 3- to 7-membered heterocycle, the heterocycle contains 1 to 4 heteroatoms selected from O, S or N; in certain embodiments, 2 $R^{bb}$ together with the atom to which they are attached form a ring as shown below:

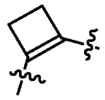, 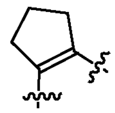, 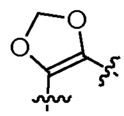, 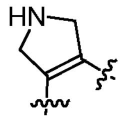,

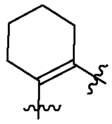, 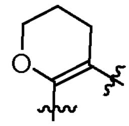, 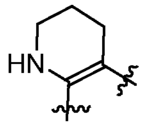, 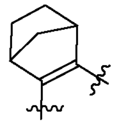, 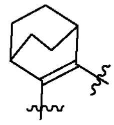, or 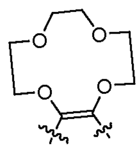,

the ring is optionally substituted with 1 to 4 substituents selected from F, Cl, Br, I, OH, $NH_2$, methyl, ethyl, or cyclopropyl;

in certain embodiments, $R^{b1}$ and $R^{b2}$ are each independently selected from H, F, Cl, Br, I, =O, OH, $NH_2$, CN, $NO_2$, $CF_3$, $-C(=O)NH_2$, -C(=O)NH-$C_{1-4}$ alkyl, - C(=O)N($C_{1-4}$ alkyl)$_2$, $C_{1-4}$ alkyl, $C_{1-4}$ alkoxy, or $C_{3-6}$ cycloalkyl, the alkyl, alkoxy or cycloalkyl is optionally substituted with 0 to 4 (for example, 0, 1, 2, 3 or 4) substituents selected from H, F, Cl, Br, I or OH;

in certain embodiments, $R^{b1}$ and $R^{b2}$ are each independently selected from H, F, Cl, Br, I, =O, OH, $NH_2$, CN, $NO_2$, $CF_3$, $-C(=O)NH_2$, -C(=O)NH- $CH_3$, - C(=O)N($CH_3$)$_2$, methyl, ethyl, propyl, isopropyl, methoxy, ethoxy, cyclopropyl, cyclobutyl, cyclopentyl or cyclohexyl, the methyl, ethyl, propyl, isopropyl, methoxy, ethoxy, cyclopropyl, cyclobutyl, cyclopentyl or cyclohexyl is optionally substituted with 0 to 4 (for example, 0, 1, 2, 3 or 4) substituents selected from H, F, Cl, Br, I or OH;

in certain embodiments, $R^{b3}$ and $R^{b4}$ are each independently selected from H or $C_{1-6}$ alkyl, the alkyl is optionally substituted with 0 to 4 (for example, 0, 1, 2, 3 or 4) substituents selected from H, F, Cl, Br, I, OH, $NH_2$, $C_{1-4}$ alkyl or $C_{1-4}$ alkoxy;

in certain embodiments, $R^{b3}$ and $R^{b4}$ are each independently selected from H, methyl, ethyl, isopropyl, or methoxymethyl;

in certain embodiments, $R^{b3}$ and $R^{b4}$ are each independently selected from H, methyl, ethyl, or methoxymethyl;

in certain embodiments, $R^{b3}$ and $R^{b4}$ cannot be both H;

in certain embodiments, $R^{b3}$ and $R^{b4}$ together with the carbon atom to which they are attached form $C_{3-6}$ cycloalkyl or 3- to 8-membered mono-heterocyclic ring, the cycloalkyl or mono-heterocyclic ring is optionally substituted with 0 to 4 (for example, 0, 1, 2, 3 or 4) substituents selected from H, F, Cl, Br, I, OH, $NH_2$, $C_{1-4}$ alkyl or $C_{1-4}$ alkoxy, the mono-heterocyclic ring contains 1 to 4 heteroatoms selected from O, S or N;

in certain embodiments, $R^{b3}$ and $R^{b4}$ together with the carbon atom to which they are attached form cyclopropyl, cyclobutyl, cyclopentyl, cyclohexyl, azetidinyl, azacyclopentyl, azacyclohexyl, oxetanyl, oxacyclopentyl, or oxacyclohexyl, the cyclopropyl, cyclobutyl, cyclopentyl, cyclohexyl, azetidinyl, azacyclopentyl, azacyclohexyl, oxetanyl, oxacyclopentyl, or oxacyclohexyl is optionally substituted with 0 to 4 (for example, 0, 1, 2, 3 or 4) substituents selected from H, F, Cl, Br, I, OH, $NH_2$, $C_{1-4}$ alkyl or $C_{1-4}$ alkoxy;

in certain embodiments, B is selected from one of the structural fragments shown in Table C-1, Table C-2, Table C-3, Table C-4, Table C-5, Table C-6, and Table C-7;

in certain embodiments, B is selected from one of the structural fragments shown in Table C-1, Table C-2, Table C-3, Table C-4, Table C-5, and Table C-6;

in certain embodiments, B is selected from one of the structural fragments shown in Table C-7;

in certain embodiments, B is selected from

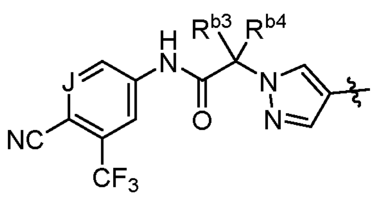

;

in certain embodiments, J is selected from N or CH;
in certain embodiments, J is selected from N;
in certain embodiments, L is selected from -Ak1-Cy2-, -Ak1-Cy2-Cy3-, - Ak1-Cy2-Ak2-, -Cy2-Cy3-, -Ak1-, or a bond;
in certain embodiments, $R^{b3}$ and $R^{b4}$ are each independently selected from methyl or ethyl;
in certain embodiments, $R^{b3}$ and $R^{b4}$ together with the carbon atom to which they are attached form cyclopropyl, cyclobutyl, cyclopentyl, cyclohexyl, azetidinyl, azacyclopentyl, azacyclohexyl, oxetanyl, oxacyclopentyl, or oxacyclohexyl, the cyclopropyl, cyclobutyl, cyclopentyl, cyclohexyl, azetidinyl, azacyclopentyl, azacyclohexyl, oxetanyl, oxacyclopentyl, or oxacyclohexyl is optionally substituted with 0 to 4 (for example, 0, 1, 2, 3 or 4) substituents selected from H, F, Cl, Br, I, OH, $NH_2$, $C_{1-4}$ alkyl or $C_{1-4}$ alkoxy;
in certain embodiments, Ak1 and Ak2 are each independently selected from - CH=CH-, -CH=C(CN)-, -CH=C(F)-, -C(CN)=CH-, -C(F)=CH-, -C≡C-, $-C(CH_3)_2-$, $-CH_2-$, $-CH_2CH_2-$, or $-CH_2CH_2CH_2-$;
in certain embodiments, each Cy1, Cy2, or Cy3 is independently selected from one of the following substituted or unsubstituted groups:

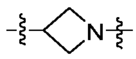,

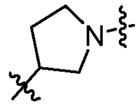, 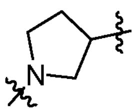, 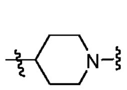, 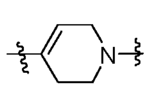, 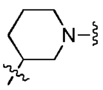, 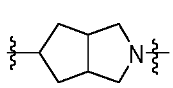,

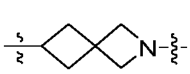, 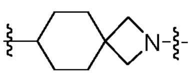, 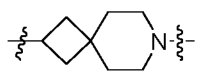, 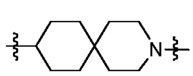, 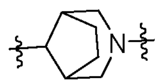,

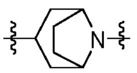, 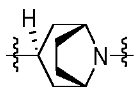, 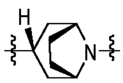, 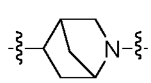, 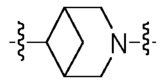, 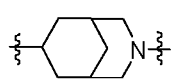,

or

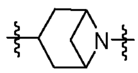,

and when substituted, is optionally substituted with 0 to 4 (for example, 0, 1, 2, 3 or 4) substituents selected from H, F, $CF_3$, OH, methyl, methoxy, =O, hydroxymethyl, COOH, CN or $NH_2$;
in certain embodiments, B is selected from

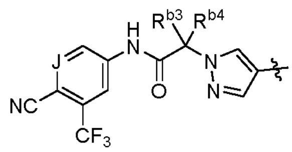;

J is selected from N or CH;
L is selected from -Ak1-Cy2-, -Ak1-Cy2-Cy3-, -Ak1-Cy2-Ak2-, -Cy2-Cy3-, - Ak1- or a bond;
$R^{b3}$ and $R^{b4}$ are each independently selected from methyl or ethyl;
alternatively, $R^{b3}$ and $R^{b4}$ together with the carbon atom to which they are attached form cyclopropyl, cyclobutyl, cyclopentyl, cyclohexyl, azetidinyl, azacyclopentyl, azacyclohexyl, oxetanyl, oxacyclopentyl, or oxacyclohexyl, the cyclopropyl, cyclobutyl, cyclopentyl, cyclohexyl, azetidinyl, azacyclopentyl, azacyclohexyl, oxetanyl, oxacyclopentyl, or oxacyclohexyl is optionally substituted with 0 to 4 (for example, 0, 1, 2, 3 or 4) substituents selected from H, F, Cl, Br, I, OH, $NH_2$, $C_{1-4}$ alkyl or $C_{1-4}$ alkoxy;
Ak1 and Ak1 are each independently selected from -CH=CH-, -CH=C(CN)-, -CH=C(F)-, -C(CN)=CH-, -C(F)=CH-,

-C≡C-, $-C(CH_3)_2-$, $-CH_2-$, $-CH_2CH_2-$, or - $CH_2CH_2CH_2-$;

each Cy1, Cy2, or Cy3 is independently selected from one of the following substituted or unsubstituted groups:

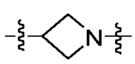, 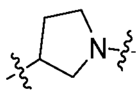, 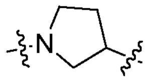, 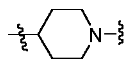

, 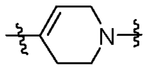, 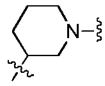, 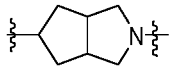, 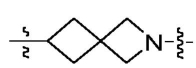, 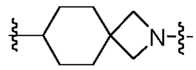,

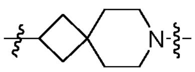, 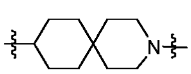, 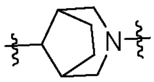, 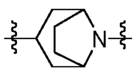, 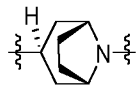, 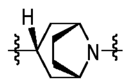,

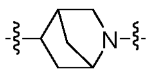, 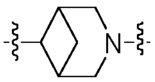, 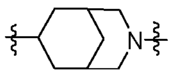, or 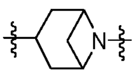,

and when substituted, is optionally substituted with 0 to 4 (for example, 0, 1, 2, 3 or 4) substituents selected from H, F, $CF_3$, OH, methyl, methoxy, =O, hydroxymethyl, COOH, CN or $NH_2$;

in certain embodiments, each q is independently selected from 0, 1, 2, 3, 4, 5 or 6;

in certain embodiments, each q is independently selected from 0, 1, 2, 3 or 4;

in certain embodiments, each q is independently selected from 0, 1 or 2;

in certain embodiments, n1, n2 and n3 are each independently selected from 0, 1, 2 or 3;

in certain embodiments, each p1 or p2 is independently selected from 0, 1, 2, 3, 4 or 5;

in certain embodiments, each p1 or p2 is independently selected from 0, 1 or 2;

in certain embodiments, the compound represented by general formula (I) is selected from one of the structures represented by (Ia) or (Ib);

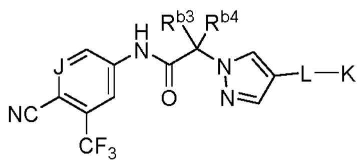

(Ia)

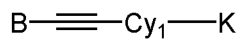

(Ib)

the definition of each group is the same as those in the above embodiment;

in certain embodiments, the compound represented by general formula (I) is selected from general formula (Id),

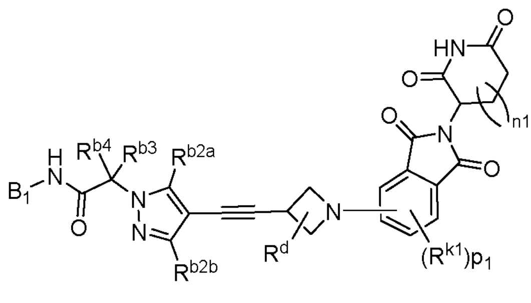

(Id)

$R^{b3}$ and $R^{b4}$ are each independently selected from H, methyl, ethyl, or isopropyl;

alternatively, $R^{b3}$ and $R^{b4}$ together with the carbon atom to which they are attached form cyclopropyl, cyclobutyl, cyclopentyl, or cyclohexyl, the cyclopropyl, cyclobutyl, cyclopentyl, or cyclohexyl is optionally substituted with 1 to 4 substituents selected from H, F, Cl, Br, I, OH, $NH_2$, $C_{1-4}$ alkyl or $C_{1-4}$ alkoxy;

$R^{b2a}$ and $R^{b2b}$ are each independently selected from H, F, Cl, Br, I, OH, $NH_2$, CN, $CF_3$, $C_{1-4}$ alkyl, $C_{1-4}$ alkoxy, or $C_{3-6}$ cycloalkyl, the alkyl, alkoxy or cycloalkyl is optionally substituted with 1 to 4 substituents selected from H, F, Cl, Br, I or OH;

$R^d$ is selected from H, F, Cl, Br, I, OH, COOH, CN, $NH_2$, $C_{1-4}$ alkyl, halogen-substituted $C_{1-4}$ alkyl, hydroxyl-substituted $C_{1-4}$ alkyl or $C_{1-4}$ alkoxy;

the other groups are the same as those in the above embodiment;

in some embodiments, 1 to 30 H contained in the deuterated compound are replaced by D;

optionally, the compound of general formula (I) is not a compound shown in Table A-1.

As a first embodiment of the present invention, provided is the above-mentioned compound shown in general formula (I) or the stereoisomer, deuterated compound, solvate, prodrug, metabolite, pharmaceutically acceptable salt or co-crystal thereof,

B-L-K (I)

;

L is selected from a bond or $-C_{1-50}$ hydrocarbyl-, wherein the hydrocarbyl has 0 to 20 methylene units optionally replaced by -Ak- or -Cy-;

each -Ak- is independently selected from $-(CH_2)_q-$, $-(CH_2)_q-O-$, $-O-(CH_2)_q-$, $-(CH_2)_q-NR^L-$, $-NR^L-(CH_2)_q-$, $-(CH_2)_q-NR^LC(=O)-$, $-NR^L(CH_2)_qC(=O)-$, $-(CH_2)_q-C(=O)NR^L-$, -C(=O)-, $-C(=O)-(CH_2)_q-NR^L-$, $-(C\equiv C)_q-$, -CH=CH-, $-Si(R^L)_2-$, $-Si(OH)(R^L)-$, $-Si(OH)_2-$, $-P(=O)(OR^L)-$, $-P(=O)(R^L)-$, -S-, -S(=O)-, $-S(=O)_2-$ or a bond, wherein the $-CH_2-$ or -CH=CH- is optionally substituted with 0 to 2 substituents selected from H, halogen, OH, CN, $NH_2$, $C_{1-6}$ alkyl, $C_{1-6}$ alkoxy, halogen-substituted $C_{1-6}$ alkyl, hydroxyl-substituted $C_{1-6}$ alkyl or cyano-substituted $C_{1-6}$ alkyl;

each q is independently selected from 0, 1, 2, 3, 4, 5 or 6;

each $R^L$ is independently selected from H, $C_{1-6}$ alkyl, 3- to 7-membered heterocyclyl, 3- to 7-membered cycloalkyl, phenyl or 5- to 6-membered heteroaryl;

each -Cy- is independently selected from a bond, 4- to 8-membered mono-heterocyclic ring group, 4- to 10-membered fused heterocyclic ring group, 5- to 12-membered spiro-heterocyclic ring group, 7- to 10-membered bridged-heterocyclic ring group, 3- to 7-membered monocycloalkyl, 4- to 10-membered fused cycloalkyl, 5- to 12-membered spirocycloalkyl, 7- to 10-membered bridged cycloalkyl, 5- to 10-membered heteroaryl or 6- to 10-membered aryl, the aryl, heteroaryl, cycloalkyl, mono-heterocyclic ring group, fused heterocyclic ring group, spiro-heterocyclic ring group or bridged-heterocyclic ring group is optionally substituted with 0 to 4 substituents selected from H, F, Cl, Br, I, OH, COOH, CN, $NH_2$, =O, $C_{1-4}$ alkyl, halogen-substituted $C_{1-4}$ alkyl, hydroxyl-substituted $C_{1-4}$ alkyl or $C_{1-4}$ alkoxy, and the heteroaryl, mono-heterocyclic ring group, fused heterocyclic ring group, spiro-heterocyclic ring group or bridged-heterocyclic ring group contains 1 to 4 (for example, 1, 2, 3 or 4) heteroatoms selected from O, S or N, and is optionally substituted with 0, 1 or 2 =O when the heteroatom is selected from S;

B is selected from

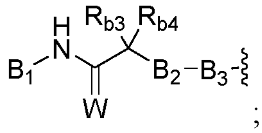

;

W is selected from O or S;

$B_1$ is selected from $C_{6-10}$ carbocyclyl or 5- to 10-membered heterocyclyl, the carbocyclyl or heterocyclyl is optionally substituted with 0 to 2 $R^{bb}$, the carbocyclyl or heterocyclyl is optionally substituted with 0 to 4 $R^{b1}$, the heterocyclyl contains 1 to 4 heteroatoms selected from O, S or N;

$B_2$ is selected from -NHC(=O)- or 5- to 10-membered heterocyclyl, the heterocyclyl is optionally substituted with 0 to 4 $R^{b2}$, the heterocyclyl contains 1 to 4 heteroatoms selected from O, S or N;

$B_3$ is selected from a bond, phenyl or 5- to 6-membered heteroaryl, the phenyl and 5- to 6-membered heteroaryl are optionally substituted with 0 to 4 $R^{b2}$;

$R^{b1}$ and $R^{b2}$ are each independently selected from H, F, Cl, Br, I, =O, OH, $NH_2$, CN, $NO_2$, $CF_3$, $-C(=O)NH_2$, $-C(=O)NH-C_{1-4}$ alkyl, $-C(=O)N(C_{1-4}\ alkyl)_2$, $C_{1-4}$ alkyl, $C_{1-4}$ alkoxy, or $C_{3-6}$ cycloalkyl, the alkyl, alkoxy or cycloalkyl is optionally

substituted with 0 to 4 substituents selected from H, F, Cl, Br, I or OH;

$R^{bb}$ is selected from -T-P(=O)$_2$(C$_{1-4}$ alkyl)$_2$, -T-S(=O)$_2$C$_{1-4}$ alkyl, -T-P(=O)$_2$(C$_{3-6}$ cycloalkyl)$_2$, -T-S(=O)$_2$C$_{3-6}$ cycloalkyl, -T-C(=O)NH$_2$, -T-C(=O)NH-C$_{1-4}$ alkyl, -T-C(=O)N(C$_{1-4}$ alkyl)$_2$, -T-C$_{1-4}$ alkyl, -T-C$_{2-6}$ alkenyl, -T-C$_{2-6}$ alkynyl, - T- C$_{3-12}$carbocycle, or -T-3- to 12-membered heterocycle, the alkyl, alkenyl, alkynyl, carbocycle or heterocycle is optionally substituted with 0 to 4 substituents selected from H, F, Cl, Br, I, OH, NH$_2$, =O, halogen-substituted C$_{1-4}$ alkyl, cyano-substituted C$_{1-4}$ alkyl, hydroxyl-substituted C$_{1-4}$ alkyl, C$_{1-4}$ alkyl, C$_{1-4}$ alkoxy, C$_{3-6}$ cycloalkyl or 3- to 7-membered heterocycle, the heterocycle contains 1 to 4 heteroatoms selected from O, S or N;

T is selected from a bond, -C(=O)-, -P(=O)$_2$-, -S(=O)$_2$-, -C(=O)NH-, - P(=O)$_2$NH-, -S(=O)$_2$NH-, -C(=O)N(C$_{1-4}$ alkyl)-, -P(=O)$_2$N(C$_{1-4}$ alkyl)-, - S(=O)$_2$N(C$_{1-4}$ alkyl)-, -NHC(=O)-, -NHP(=O)$_2$-, -NHS(=O)$_2$-, -N(C$_{1-4}$ alkyl)C(=O)-, -N(C$_{1-4}$ alkyl)P(=O)$_2$-, -N(C$_{1-4}$ alkyl)S(=O)$_2$-, O, S, NH, N(C$_{1-4}$ alkyl), N(C$_{3-6}$ cycloalkyl), N(3- to 10-membered heterocycle), C$_{1-4}$ alkylene, -O-C$_{1-4}$ alkylene-, -C$_{1-4}$ alkylene-O-, -S-C$_{1-4}$ alkylene-, -NH-C$_{1-4}$ alkylene-, -N(C$_{1-4}$ alkyl)-C$_{1-4}$ alkylene-, or -O-C$_{1-4}$ alkylene-O-, the alkyl or alkylene is optionally substituted with 0 to 4 substituents selected from H, F, Cl, Br, I, OH, =O, C$_{1-4}$ alkyl, C$_{1-4}$ alkoxy, C$_{3-6}$ cycloalkyl or 3- to 7-membered heterocycle, the heterocycle contains 1 to 4 heteroatoms selected from O, S or N;

alternatively, 2 $R^{bb}$ together with the atom to which they are attached form C$_{4-12}$ carbocycle or 4- to 12-membered heterocycle, the carbocycle or heterocycle is optionally substituted with 0 to 4 substituents selected from H, F, Cl, Br, I, OH, NH$_2$, =O, halogen-substituted C$_{1-4}$ alkyl, cyano-substituted C$_{1-4}$ alkyl, hydroxyl-substituted C$_{1-4}$ alkyl, C$_{1-4}$ alkyl, C$_{1-4}$ alkoxy, C$_{3-6}$ cycloalkyl or 3- to 7-membered heterocycle, the heterocycle contains 1 to 4 heteroatoms selected from O, S or N;

$R^{b3}$ and $R^{b4}$ are each independently selected from H or C$_{1-6}$ alkyl, the alkyl is optionally substituted with 0 to 4 substituents selected from H, F, Cl, Br, I, OH, NH$_2$, C$_{1-4}$ alkyl or C$_{1-4}$ alkoxy;

alternatively, $R^{b3}$ and $R^{b4}$ together with the carbon atom to which they are attached form C$_{3-6}$ cycloalkyl or 3- to 8-membered mono-heterocyclic ring, the cycloalkyl or mono-heterocyclic ring is optionally substituted with 0 to 4 substituents selected from H, F, Cl, Br, I, OH, NH$_2$, C$_{1-4}$ alkyl or C$_{1-4}$ alkoxy, the mono-heterocyclic ring contains 1 to 4 heteroatoms selected from O, S or N;

K is selected from

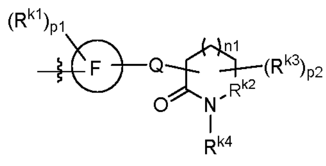 , 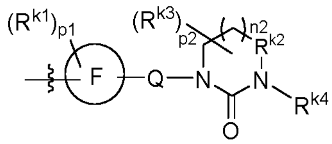 ,

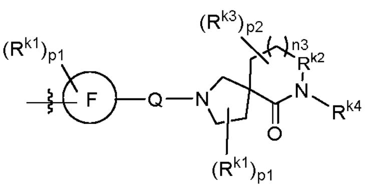 , 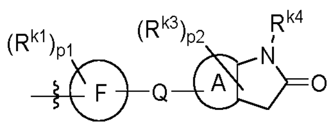 , 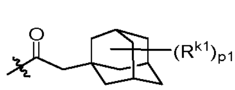 ,

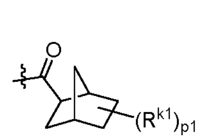 , 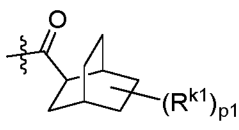 , 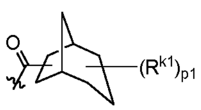 , 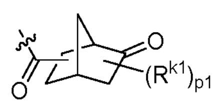 ,

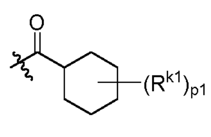 , 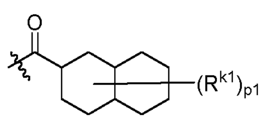 , 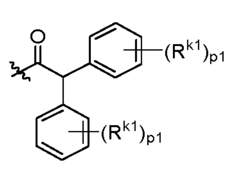 , 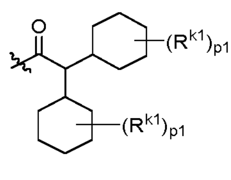 ,

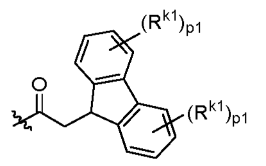, 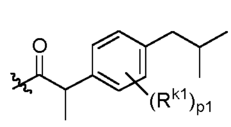, 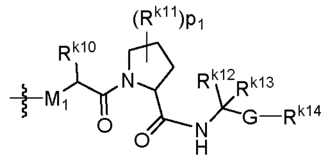,

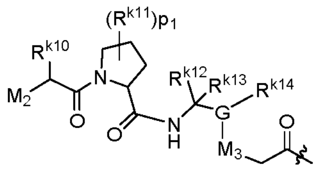 or 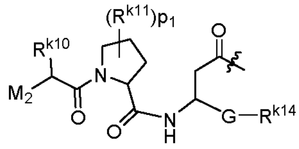;

each Q is independently selected from a bond, -O-, -S-, $-CH_2-$, $-NR^q-$, - C(=O)-, $-NR^qC(=O)-$, $-C(=O)NR^q-$ or 3- to 12-membered heterocyclyl, the heterocyclyl is optionally substituted with 0 to 4 substituents selected from H, F, Cl, Br, I, OH, =O, $NH_2$, CN, COOH, $CONH_2$, $C_{1-4}$ alkyl or $C_{1-4}$ alkoxy, the heterocyclyl contains 1 to 4 heteroatoms selected from O, S or N;

$R^q$ is selected from H or $C_{1-6}$ alkyl;

A is selected from $C_{3-10}$ carbocyclyl, $C_{6-10}$ aryl, 3- to 10-membered heterocyclyl or 5- to 10-membered heteroaryl, wherein the heterocycle or heteroaryl contains 1 to 4 heteroatoms selected from O, S or N;

each F is independently selected from $C_{3-20}$ carbocyclyl, $C_{6-20}$ aryl, 3- to 20-membered heterocyclyl or 5- to 20-membered heteroaryl, and the heterocyclyl or heteroaryl contains 1 to 4 heteroatoms selected from O, S or N;

each $R^{k2}$ is independently selected from a bond, -C(=O)-, $-S(=O)_2-$, -S(=O)- or $-C(R^{k3})_2-$;

each $R^{k1}$ is independently selected from H, F, Cl, Br, I, OH, =O, $NH_2$, CN, COOH, $CONH_2$, $C_{1-6}$ alkyl or $C_{1-6}$ alkoxy, the alkyl or alkoxy is optionally substituted with 0 to 4 substituents selected from H, F, Cl, Br, I, OH, =O, $NH_2$, CN, COOH, $CONH_2$, $C_{1-4}$ alkyl or $C_{1-4}$ alkoxy;

each $R^{k3}$ is independently selected from H, F, Cl, Br, I, OH, =O, $NH_2$, CN, COOH, $CONH_2$, $C_{1-6}$ alkyl, $C_{1-6}$ alkoxy, $C_{3-8}$ cycloalkyl or 3- to 8-membered heterocyclyl, the alkyl, alkoxy, cycloalkyl or heterocyclyl is optionally substituted with 0 to 4 substituents selected from H, F, Cl, Br, I, OH, =O, $NH_2$, CN, COOH, $CONH_2$, $C_{1-4}$ alkyl or $C_{1-4}$ alkoxy, the heterocyclyl contains 1 to 4 heteroatoms selected from O, S or N;

alternatively, two $R^{k3}$ together with the carbon atom or ring backbone to which they are directly attached form 3- to 8-membered carbocycle or 3- to 8-membered heterocycle, two $R^{k1}$ together with the carbon atom or ring backbone to which they are directly attached form 3- to 8-membered carbocycle or 3- to 8-membered heterocycle, the carbocycle or heterocycle is optionally substituted with 0 to 4 substituents selected from H, F, Cl, Br, I, OH, =O, $NH_2$, CN, COOH, $CONH_2$, $C_{1-4}$ alkyl or $C_{1-4}$ alkoxy, the heterocycle contains 1 to 4 heteroatoms selected from O, S or N;

each $R^{k4}$ is independently selected from H, OH, $NH_2$, CN, $CONH_2$, $C_{1-6}$ alkyl, $C_{3-8}$ cycloalkyl or 3- to 8-membered heterocyclyl, the alkyl, cycloalkyl or heterocyclyl is optionally substituted with 0 to 4 substituents selected from H, F, Cl, Br, I, OH, =O, $NH_2$, CN, COOH, $CONH_2$, $C_{1-4}$ alkyl or $C_{1-4}$ alkoxy, the heterocyclyl contains 1 to 4 heteroatoms selected from O, S or N;

$M_1$ is selected from a bond, -C(=O)NH-, -NHC(=O)-, $-CH_2-C(=O)NH-$, - $C(=O)CH_2NH-$, or 5- to 6-membered heteroaryl, the heteroaryl is optionally substituted with 0 to 4 substituents selected from H, F, Cl, Br, I, OH, =O, $CF_3$, $NH_2$, CN, $C_{1-4}$ alkyl, halogen-substituted $C_{1-4}$ alkyl, hydroxyl-substituted $C_{1-4}$ alkyl, or $C_{1-4}$ alkoxy, the heteroaryl contains 1 to 4 heteroatoms selected from O, S or N;

$M_2$ is selected from $-NHC(=O)-C_{1-6}$ alkyl, $-NHC(=O)-C_{3-6}$ cycloalkyl or 4- to 10-membered heterocyclyl, the alkyl, cycloalkyl or heterocyclyl is optionally substituted with 0 to 4 substituents selected from H, F, Cl, Br, I, =O, OH, $NH_2$, $C_{1-4}$ alkyl or $C_{1-4}$ alkoxy, the heterocyclyl contains 1 to 4 heteroatoms selected from O, S or N;

$M_3$ is selected from -NH- or -O-;

$R^{k10}$ is selected from $C_{1-6}$ alkyl, the alkyl is optionally substituted with 0 to 4 substituents selected from H, F, Cl, Br, I, =O, OH, $C_{1-6}$ alkyl or $C_{3-6}$ cycloalkyl;

each $R^{k11}$ is independently selected from H, F, Cl, Br, I, =O, OH, SH, $C_{1-6}$ alkyl, $C_{1-6}$ alkoxy or $C_{1-6}$ alkylthio or $-O-C(=O)-C_{1-6}$ alkyl, the alkyl, alkoxy or alkylthio is optionally substituted with 0 to 4 substituents selected from H, F, Cl, Br, I, OH, $C_{1-4}$ alkyl or $C_{1-4}$ alkoxy;

$R^{k12}$ and $R^{kl3}$ are each independently selected from H, $C_{1-6}$ alkyl or $C_{3-6}$ cycloalkyl, the alkyl or cycloalkyl is optionally substituted with 0 to 4 substituents selected from H, F, Cl, Br, I, =O, OH, $NH_2$, $C_{1-4}$ alkyl or $C_{1-4}$ alkoxy;

$R^{k14}$ is selected from 5- to 6-membered heteroaryl, the heteroaryl is optionally substituted with 0 to 4 substituents selected from H, F, Cl, Br, I, OH, =O, $CF_3$, CN, $C_{1-4}$ alkyl, halogen-substituted $C_{1-4}$ alkyl, hydroxyl-substituted $C_{1-4}$ alkyl, $C_{1-4}$ alkoxy or $C_{3-6}$ cycloalkyl, the heteroaryl contains 1 to 4 heteroatoms selected from N, O and S;

G is selected from 6- to 10-membered aryl or 5- to 10-membered heteroaryl, the aryl or heteroaryl is optionally substituted with 0 to 4 substituents selected from H, F, Cl, Br, I, OH, =O, $CF_3$, CN, $C_{1-4}$ alkyl, halogen-substituted $C_{1-4}$ alkyl, hydroxyl-substituted $C_{1-4}$ alkyl, $C_{1-4}$ alkoxy or $C_{3-6}$ cycloalkyl, the heteroaryl contains 1 to 4 heteroatoms selected from N, O and S;

n1, n2 and n3 are each independently selected from 0, 1, 2 or 3;

each p1 or p2 is independently selected from 0, 1, 2, 3, 4 or 5;

optionally, 1 to 30 H contained in the deuterated compound are replaced by D;

provided that the compound of general formula (I) is not a compound shown in Table A-1.

As a second embodiment of the present invention, provided is the above-mentioned compound shown in general formula (I) or the stereoisomer, deuterated compound, solvate, prodrug, metabolite, pharmaceutically acceptable salt or co-crystal thereof,

$B_1$ is selected from $C_{6-10}$ carbocyclyl or 5- to 10-membered heterocyclyl, the carbocyclyl or heterocyclyl is optionally substituted with 0 to 4 $R^{b1}$, the heterocyclyl contains 1 to 4 heteroatoms selected from O, S or N;

$B_2$ is selected from -NHC(=O)- or 5- to 10-membered heterocyclyl, the heterocyclyl is optionally substituted with 0 to 4 $R^{b2}$, the heterocyclyl contains 1 to 4 heteroatoms selected from O, S or N;

$B_3$ is selected from a bond, phenyl or 5- to 6-membered heteroaryl, the phenyl and 5- to 6-membered heteroaryl are optionally substituted with 0 to 4 $R^{b2}$;

$R^{b1}$ and $R^{b2}$ are each independently selected from H, F, Cl, Br, I, =O, OH, $NH_2$, CN, $NO_2$, $CF_3$, $-C(=O)NH_2$, -C(=O)NH-$C_{1-4}$ alkyl, -C(=O)N($C_{1-4}$ alkyl)$_2$, $C_{1-4}$ alkyl, $C_{1-4}$ alkoxy, or $C_{3-6}$ cycloalkyl, the alkyl, alkoxy or cycloalkyl is optionally substituted with 0 to 4 substituents selected from H, F, Cl, Br, I or OH;

$R^{b3}$ and $R^{b4}$ are each independently selected from H or $C_{1-6}$ alkyl, the alkyl is optionally substituted with 0 to 4 substituents selected from H, F, Cl, Br, I, OH, $NH_2$, $C_{1-4}$ alkyl or $C_{1-4}$ alkoxy;

alternatively, $R^{b3}$ and $R^{b4}$ together with the carbon atom to which they are attached form $C_{3-6}$ cycloalkyl or 3- to 8-membered mono-heterocyclic ring, the cycloalkyl or mono-heterocyclic ring is optionally substituted with 0 to 4 substituents selected from H, F, Cl, Br, I, OH, $NH_2$, $C_{1-4}$ alkyl or $C_{1-4}$ alkoxy, the mono-heterocyclic ring contains 1 to 4 heteroatoms selected from O, S or N;

the definitions of other groups are the same as those in the first embodiment of the present invention.

As a third embodiment of the present invention, provided is the above-mentioned compound shown in general formula (I) or the stereoisomer, deuterated compound, solvate, prodrug, metabolite, pharmaceutically acceptable salt or co-crystal thereof,

L is selected from -Cy1-Ak1-Cy2-Ak2-Cy3-Ak3-Cy4-Ak4-Cy5-Ak5-, -Cy1-Cy2-Cy3-Cy4-Ak1-Ak2-Ak3-Ak4-Ak5-, -Cy1-Ak1-Cy2-Ak2-Cy3-Ak3-Cy4-Ak4-Ak5-, -Ak1-Cy1-Ak2-Cy2-Ak3-Cy3-Ak4-Cy4-Ak5-, -Cy1-Ak1-Cy2-Ak2-Cy3-Cy4-Ak3-Ak4-Ak5-, -Cy1-Ak1-Cy2-Ak2-Ak3-Cy3-Cy4-Ak4-Ak5-, -Cy1-Ak1-Ak2-Ak3-Ak4-Ak5-Cy2-Cy3-Cy4-, -Cy1-Cy2-Ak1-Ak2-Ak3-Ak4-Ak5-Cy3-Cy4-, -Cy1-Cy2-Cy3-Ak1-Ak2-Ak3-Ak4-Ak5-Cy4-, -Cy1-Cy2-Cy3-Cy4-Ak1-Ak2-Ak3-Ak4-Ak5-, -Cy1-Ak1-Cy2-Cy3-Cy4-Ak2-Ak3-Ak4-Ak5-, -Cy1-Cy2-Ak1-Cy3-Cy4-Ak2-Ak3-Ak4-Ak5-, -Cy1-Cy2-Cy3-Ak1-Cy4-Ak2-Ak3-Ak4-Ak5-, - Cy1-Ak1-Ak2-Cy2-Cy3-Cy4-Ak3-Ak4-Ak5-, -Cy1-Cy2-Ak1-Ak2-Cy3-Cy4-Ak3-Ak4-Ak5-, -Cy1-Cy2-Cy3-Ak1-Ak2-Cy4-Ak3-Ak4-Ak5-, -Cy1-Ak1-Ak2-Ak3-Cy2-Cy3-Cy4-Ak4-Ak5-, -Cy1-Cy2-Ak1-Ak2-Ak3-Cy3-Cy4-Ak4-Ak5-, -Cy1-Cy2-Cy3 -Ak 1 -Ak2-Ak3 -Cy4-Ak4-Ak5 -, -Cy1-Ak1-Ak2-Ak3-Ak4-Cy2-Cy3-Cy4-Ak5-, -Cy1-Cy2-Ak1-Ak2-Ak3-Ak4-Cy3-Cy4-Ak5-, -Cy1-Cy2-Cy3-Ak1-Ak2-Ak3-Ak4-Cy4-Ak5-, -Ak1-Ak2-Ak3-Ak4-Ak5-Cy1-Cy2-Cy3-Cy4-, -Ak1-Cy1-Cy2-Cy3-Cy4-Ak2-Ak3-Ak4-Ak5-, -Ak1-Ak2-Cy1-Cy2-Cy3-Cy4-Ak3-Ak4-Ak5-, -Ak1-Ak2-Ak3-Cy1-Cy2-Cy3-Cy4-Ak4-Ak5-, -Ak1-Ak2-Ak3-Ak4-Cy1-Cy2-Cy3-Cy4-Ak5-, -Ak1-Cy1-Ak2-Ak3-Ak4-Ak5-Cy2-Cy3-Cy4-, -Ak1-Cy1-Cy2-Ak2-Ak3-Ak4-Ak5-Cy3-Cy4-, - Ak 1 -Cy 1 -Cy2-Cy 3 - Ak2-Ak3 - Ak4-Ak5 -Cy4-, - Ak1-Ak2-Cy1-Ak3-Ak4-Ak5-Cy2-Cy3-Cy4-, -Ak1-Ak2-Cy1-Cy2-Ak3-Ak4-Ak5 - Cy3-Cy4-, -Ak1-Ak2-Cy1-Cy2-Cy3-Ak3-Ak4-Ak5-Cy4-, -Ak1-Ak2-Ak3-Cy1-Ak4-Ak5-Cy2-Cy3-Cy4-, -Ak1-Ak2-Ak3-Cy1-Cy2-Ak4-Ak5-Cy3-Cy4-, -Ak1-Ak2-Ak3-Cy1-Cy2-Cy3-Ak4-Ak5-Cy4-, -Ak1-Ak2-Ak3-Ak4-Cy1-Ak5-Cy2-Cy3-Cy4-, -Ak1-Ak2-Ak3-Ak4-Cy1-Cy2-Ak5-Cy3-Cy4-, or -Ak1-Ak2-Ak3-Ak4-Cy1-Cy2-Cy3-Ak5-Cy4-;

Ak1, Ak2, Ak3, Ak4, and Ak5 are each independently selected from $-(CH_2)_q-$, $-(CH_2)_q$-O-, -O-$(CH_2)_q$-, $-(CH_2)_q-NR^L-$, $-NR^L-(CH_2)_q-$, -(CH$_2$)q-NR$^L$C(=O)-, - $(CH_2)_q$-C(=O)NR$^L$-, -C(=O)-, -C(=O)-$(CH_2)_q$-NR$^L$-, -CH=CH-, $-(C{\equiv}C)_q-$ or a bond, the $-CH_2-$ or -CH=CH- is optionally substituted with 0 to 2 substituents selected from H, halogen, OH, CN, $NH_2$, $C_{1-4}$ alkyl, $C_{1-4}$ alkoxy, halogen-substituted $C_{1-4}$ alkyl, hydroxyl-substituted $C_{1-4}$ alkyl, or cyano-substituted $C_{1-4}$ alkyl;

each Cy1, Cy2, Cy3, Cy4 or Cy5 is independently selected from a bond, 4- to 7-membered mono-heterocyclic ring group, 4- to 10-membered fused heterocyclic ring group, 5- to 12-membered spiro-heterocyclic ring group, 7- to 10-membered bridged-heterocyclic ring group, 3- to 7-membered monocycloalkyl, 4- to 10-membered fused cycloalkyl,

5- to 12-membered spirocycloalkyl, 7- to 10-membered bridged cycloalkyl, 5- to 10-membered heteroaryl or 6- to 10-membered aryl, the aryl, heteroaryl, cycloalkyl, mono-heterocyclic ring group, fused heterocyclic ring group, spiro-heterocyclic ring group or bridged-heterocyclic ring group is optionally substituted with 0 to 4 substituents selected from H, F, Cl, Br, I, OH, COOH, CN, $NH_2$, =O, $C_{1-4}$ alkyl, halogen-substituted $C_{1-4}$ alkyl, hydroxyl-substituted $C_{1-4}$ alkyl or $C_{1-4}$ alkoxy, and the heteroaryl, mono-heterocyclic ring group, fused heterocyclic ring group, spiro-heterocyclic ring group or bridged-heterocyclic ring group contains 1 to 4 heteroatoms selected from O, S or N, and is optionally substituted with 0, 1 or 2 =O when the heteroatom is selected from S;

each q is independently selected from 0, 1, 2, 3 or 4;

each $R^L$ is independently selected from H or $C_{1-6}$ alkyl;

the definitions of other groups are the same as those in the second embodiment of the present invention.

As a fourth embodiment of the present invention, provided is the above-mentioned compound shown in general formula (I) or the stereoisomer, deuterated compound, solvate, prodrug, metabolite, pharmaceutically acceptable salt or co-crystal thereof,

each Cy1, Cy2, Cy3, Cy4 or Cy5 is independently selected from a bond, 4- to 7-membered nitrogen-containing mono-heterocyclic ring group, 4- to 10-membered nitrogen-containing fused heterocyclic ring group, 5- to 12-membered nitrogen-containing spiro-heterocyclic ring group, 7- to 10-membered nitrogen-containing bridged-heterocyclic ring group, 3- to 7-membered monocycloalkyl, 4-to 10-membered fused cycloalkyl, 5- to 12-membered spirocycloalkyl, 7- to 10-membered bridged cycloalky, 5- to 10-membered heteroaryl or 6- to 10-membered aryl, the mono-heterocyclic ring group, fused heterocyclic ring group, bridged-heterocyclic ring group, spiro-heterocyclic ring group, cycloalkyl, aryl or heteroaryl is optionally substituted with 0 to 4 substituents selected from H, F, Cl, Br, I, OH, COOH, CN, $NH_2$, =O, $C_{1-4}$ alkyl, halogen-substituted $C_{1-4}$ alkyl, hydroxyl-substituted $C_{1-4}$ alkyl or $C_{1-4}$ alkoxy, the mono-heterocyclic ring group, fused heterocyclic ring group, bridged-heterocyclic ring group, spiro-heterocyclic ring group or heteroaryl contains 1 to 4 heteroatoms selected from O, S or N, and is optionally substituted with 0, 1 or 2 =O when the heteroatom is selected from S;

each $R^L$ is independently selected from H or $C_{1-4}$ alkyl;

K is selected from

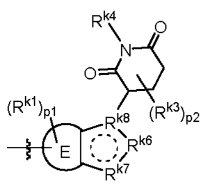 , 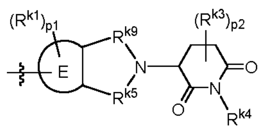 , 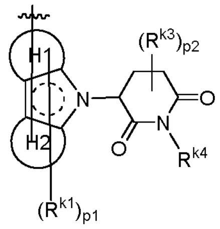 ,

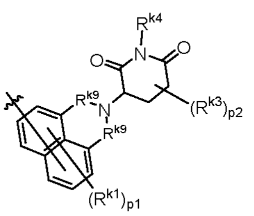 , 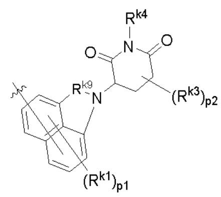 , 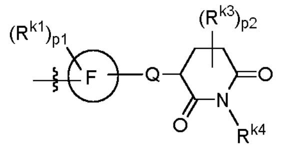 ,

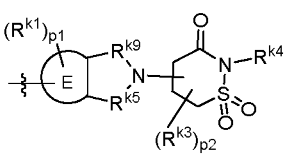 , 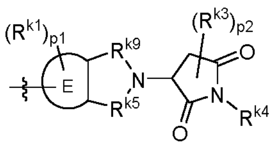 , 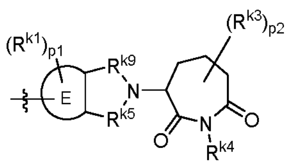 ,

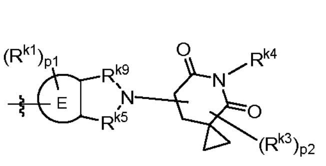, 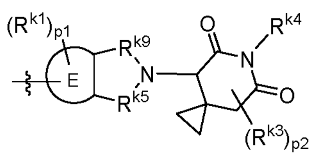, 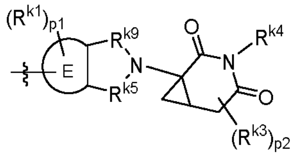,

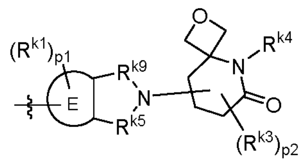, 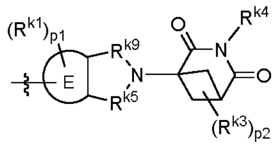, 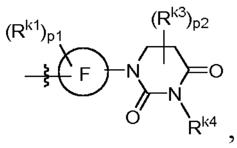, 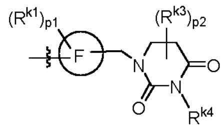

, 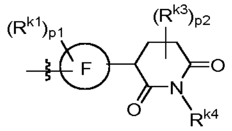, 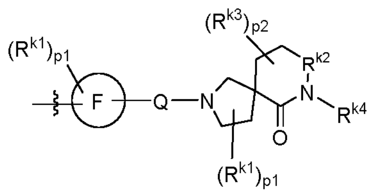, 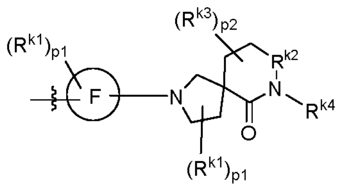,

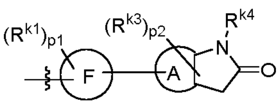, 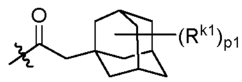, 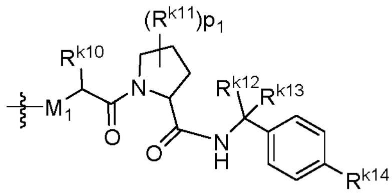,

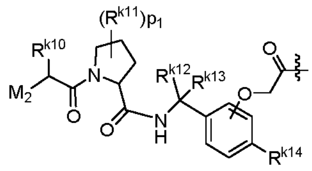 or 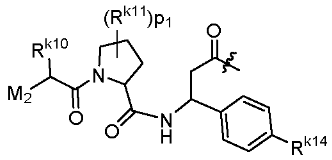;

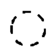

represents a ring selected from an aromatic ring or a non-aromatic ring;

$M_2$ is selected from -NHC(=O)-$C_{1-4}$ alkyl, -NHC(=O)-$C_{3-6}$ cycloalkyl or 4- to 10-membered heterocyclyl, the alkyl, cycloalkyl or heterocyclyl is optionally substituted with 0 to 4 substituents selected from H, F, Cl, Br, I, =O, OH, $NH_2$, $C_{1-4}$ alkyl or $C_{1-4}$ alkoxy, the heterocyclyl contains 1 to 4 heteroatoms selected from O, S or N;

$R^{k10}$ is selected from $C_{1-4}$ alkyl, the alkyl is optionally substituted with 0 to 4 substituents selected from H, F, Cl, Br, I, =O, OH, $C_{1-4}$ alkyl or $C_{3-6}$ cycloalkyl;

each $R^{k11}$ is independently selected from H, F, Cl, Br, I, =O, OH, SH, $C_{1-4}$ alkyl, $C_{1-4}$ alkoxy or $C_{1-4}$ alkylthio or -O-C(=O)-$C_{1-4}$ alkyl, the alkyl, alkoxy or alkylthio is optionally substituted with 0 to 4 substituents selected from H, F, Cl, Br, I, OH, $C_{1-4}$ alkyl or $C_{1-4}$ alkoxy;

$R^{k12}$ and $R^{k13}$ are each independently selected from H, $C_{1-4}$ alkyl or $C_{3-6}$ cycloalkyl, the alkyl or cycloalkyl is optionally substituted with 0 to 4 substituents selected from H, F, Cl, Br, I, =O, OH, $NH_2$, $C_{1-4}$ alkyl or $C_{1-4}$ alkoxy;

each Q is independently selected from -O-, -S-, -$CH_2$-, -$NR^q$-, -C(=O)-, -$NR^q$C(=O)-, -C(=O)$NR^q$- or 4- to 7-membered heterocyclyl, the heterocyclyl is optionally substituted with 0 to 4 substituents selected from H, F, Cl, Br, I, OH, =O, $NH_2$, CN, COOH, $CONH_2$, $C_{1-4}$ alkyl or $C_{1-4}$ alkoxy, the heterocyclyl contains 1 to 4 heteroatoms selected from O, S or N;

$R^q$ is selected from H or $C_{1-4}$ alkyl;

$R^{k1}$ and $R^{k3}$ are each independently selected from H, F, Cl, Br, I, OH, =O, $NH_2$, $CF_3$, CN, COOH, $CONH_2$, $C_{1-4}$ alkyl or $C_{1-4}$ alkoxy, the alkyl or alkoxy is optionally substituted with 0 to 4 substituents selected from H, F, Cl, Br, I, OH,

or $NH_2$;
alternatively, two $R^{k3}$ together with the carbon atom or ring backbone to which they are directly attached form 3- to 6-membered carbocycle or 3- to 7-membered heterocycle, two $R^{k1}$ together with the carbon atom or ring backbone to which they are directly attached form 3- to 6-membered carbocycle or 3- to 7-membered heterocycle, the carbocycle or heterocycle is optionally substituted with 0 to 4 substituents selected from H, F, Cl, Br, I, OH, =O, $NH_2$, CN, COOH, $CONH_2$, $C_{1-4}$ alkyl or $C_{1-4}$ alkoxy, the heterocycle contains 1 to 4 heteroatoms selected from O, S or N;
each $R^{k4}$ is independently selected from H, OH, $NH_2$, $CF_3$, CN or $C_{1-4}$ alkyl;
each $R^{k5}$ is independently selected from C(=O), $CH_2$, $S(=O)_2$,

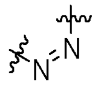,

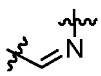, or 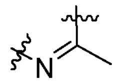;

each $R^{k6}$ is independently selected from C(=O), CH, S(=O), $SO_2$, $CH_2$ or N;
each $R^{k7}$ is independently selected from C(=O), CH, N, $CH_2$, O, S, $N(CH_3)$ or NH;
each $R^{k8}$ is independently selected from C, N or CH;
each $R^{k9}$ is independently selected from C(=O), $CH_2$ or $SO_2$;
each A, H1 or H2 is independently selected from $C_{3-8}$ carbocyclyl, phenyl, 4-to 7-membered heterocyclyl or 5- to 6-membered heteroaryl, the heterocyclyl or heteroaryl contains 1 to 4 heteroatoms selected from O, S or N;
each E is independently selected from $C_{3-8}$ carbocyclyl, phenyl, 4- to 7-membered heterocyclyl, 8- to 12-membered heterocyclyl, 7- to 12-membered heteroaryl or 5- to 6-membered heteroaryl, the heterocyclyl or heteroaryl contains 1 to 4 heteroatoms selected from O, S or N;
each F is independently selected from 3- to 7-membered monocycloalkyl, 4-to 10-membered fused cycloalkyl, 5- to 12-membered spirocycloalkyl, 5- to 10-membered bridged cycloalky, 4- to 7-membered mono-heterocyclic ring group, 4-to 10-membered fused heterocyclic ring group, 5- to 12-membered spiro-heterocyclic ring group, 5- to 10-membered bridged-heterocyclic ring group, $C_{6-14}$ aryl or 5- to 10-membered heteroaryl, the mono-heterocyclic ring group, fused heterocyclic ring group, spiro-heterocyclic ring group, bridged-heterocyclic ring group or heteroaryl contains 1 to 4 heteroatoms selected from O, S or N;
the definitions of other groups are the same as those in any one of the second and third embodiments of the present invention.

As a fifth embodiment of the present invention, provided is the above-mentioned compound shown in general formula (I) or the stereoisomer, deuterated compound, solvate, prodrug, metabolite, pharmaceutically acceptable salt or co-crystal thereof,

$R^L$ is selected from H, methyl or ethyl;
each q is independently selected from 0, 1 or 2;
each Cy1, Cy2, Cy3, Cy4 or Cy5 is independently selected from a bond or one of the following substituted or unsubstituted groups: cyclopropyl, cyclobutyl, cyclopentyl, cyclohexyl, azetidinyl, azacyclopentyl, azacyclohexenyl, piperidinyl, morpholinyl, piperazinyl, phenyl, cyclopropyl-fused-cyclopropyl, cyclopropyl-fused-cyclobutyl, cyclopropyl-fused-cyclopentyl, cyclopropyl-fused-cyclohexyl, cyclobutyl-fused-cyclobutyl, cyclobutyl-fused-cyclopentyl, cyclobutyl-fused-cyclohexyl, cyclopentyl-fused-cyclopentyl, cyclopentyl-fused-cyclohexyl, cyclohexyl-fused-cyclohexyl, cyclopropyl-spiro-cyclopropyl, cyclopropyl-spiro-cyclobutyl, cyclopropyl-spiro-cyclopentyl, cyclopropyl-spiro-cyclohexyl, cyclobutyl-spiro-cyclobutyl, cyclobutyl-spiro-cyclopentyl, cyclobutyl-spiro-cyclohexyl, cyclopentyl-spiro-cyclopentyl, cyclopentyl-spiro-cyclohexyl, cyclohexyl-spiro-cyclohexyl, cyclopropyl-fused-azetidinyl, cyclopropyl-fused-azacyclopentyl, cyclopropyl-fused-azacyclohexyl, cyclopropyl-fused-piperidinyl, cyclobutyl-fused-azetidinyl, cyclobutyl-fused-azacyclopentyl, cyclobutyl-fused-azacyclohexyl, cyclobutyl-fused-piperidinyl, cyclopentyl-fused-azetidinyl, cyclopentyl-fused-azacyclopentyl, cyclopentyl-fused-azacyclohexyl, cyclopentyl-fused-piperidinyl, cyclohexyl-fused-azetidinyl, cyclohexyl-fused-azacyclopentyl, cyclohexyl-fused-azacyclohexyl, cyclohexyl-fused-piperidinyl, azetidinyl-fused-azetidinyl, azetidinyl-fused-azacyclopentyl, azetidinyl-fused-azacyclohexyl, azetidinyl-fused-piperidinyl, azacyclopentyl-fused-azetidinyl, azacyclopentyl-fused-azacyclopentyl, azacyclopentyl-fused-azacyclohexyl, azacyclopentyl-fused-piperidinyl, azacyclohexyl-fused-azetidinyl, azacyclohexyl-fused-azacyclopentyl, azacyclohexyl-fused-azacyclohexyl, azacyclohexyl-fused-piperidinyl, cyclobutyl-spiro-azetidinyl, cyclobutyl-spiro-azacyclopentyl, cyclobutyl-spiro-azacyclohexyl, cyclopentyl-spiro-azetidinyl, cyclopentyl-spiro-azacyclopentyl, cyclopentyl-spiro-azacyclohexyl, cyclohexyl-spiro-azetidinyl, cyclohexyl-spiro-azacyclopentyl, cyclohexyl-spiro-azacyclohexyl, azetidinyl-spiro-azetidinyl, azetidinyl-spiro-azacyclopentyl, azetidinyl-spiro-azacyclohexyl, azacyclopentyl-spiro-azetidinyl, azacyclopentyl-spiro-azacyclopentyl, azacyclopentyl-spiro-azacyclohexyl, azacyclohexyl-spiro-azetidinyl, azacyclohexyl-spiro-azacyclopentyl, azacyclohexyl-spiro-azacyclohexyl,

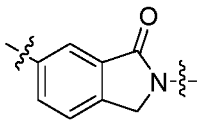, 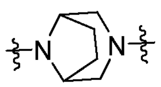, 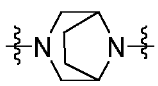,

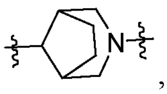, 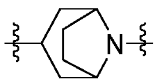, 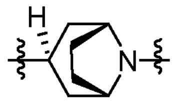, 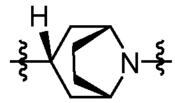, 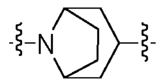, 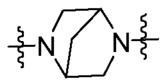,

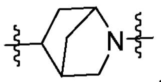, 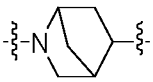, 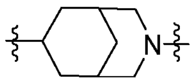, 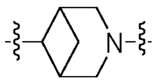, 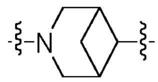, 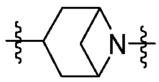,

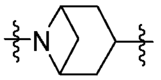, 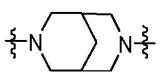, 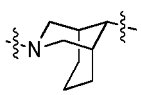, 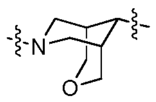, 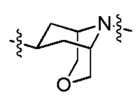, 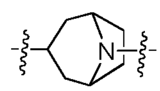,

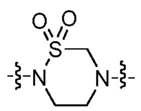, or 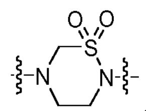,

and when substituted, is optionally substituted with 0 to 4 substituents selected from H, F, Cl, Br, I, OH, $NH_2$, COOH, CN, =O, $C_{1-4}$ alkyl, halogen-substituted $C_{1-4}$ alkyl, hydroxyl-substituted $C_{1-4}$ alkyl or $C_{1-4}$ alkoxy;

$B_1$ is selected from phenyl, naphthyl, thienyl, furyl, pyrrolyl, pyridyl, pyrimidinyl, pyrazinyl, pyridazinyl, quinolinyl, isoquinolinyl, quinazolinyl, 3,4-dihydro-1H-benzopyranyl, or 1,2,3,4-tetrahydroquinolinyl;

alternatively, $B_1$ is selected from benzofuryl, benzothienyl, benzopyrrolyl, benzoxazolyl, benzothiazolyl, benzoimidazolyl, benzopyrazolyl, azacyclopentyl, azacyclohexyl, oxacyclopentyl, oxacyclohexyl, adamantyl, bicyclo[2.2.2]octyl, or 1,2,3,4-tetrahydronaphthyl;

the $B_1$ is optionally substituted with 0 to 4 $R^{b1}$;

$B_2$ is selected from -NHC(=O)-, pyrazolyl, imidazolyl, triazolyl, thiazolyl, oxazolyl, benzopyrrolyl, benzoimidazolyl, pyrazolotetrahydropyrrolyl, or 3-pyridazinonyl, and the pyrazolyl, imidazolyl, thiazolyl, oxazolyl, triazolyl, benzopyrrolyl, benzoimidazolyl, pyrazolotetrahydropyrrolyl, or 3-pyridazinonyl is optionally substituted with 0 to 4 $R^{b2}$;

alternatively, $B_2$ is selected from substituted or unsubstituted benzopyrazolyl, 2-pyridonyl, 1,2,3,4-tetrahydroquinolinyl, 1,2,3,4-tetrahydroisoquinolinyl, azacyclohexyl,

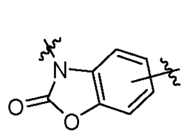, 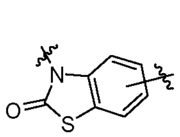, 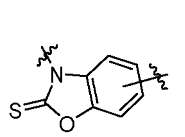, 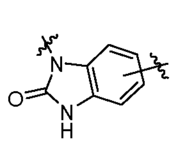,

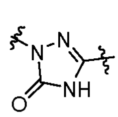 or 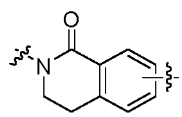,

and when substituted, is optionally substituted with 0 to $_4$ $R^{b2}$;

$B_3$ is selected from a bond or phenyl, the phenyl is optionally substituted with 0 to 4 $R^{b2}$,

$R^{b1}$ and $R^{b2}$ are each independently selected from H, F, Cl, Br, I, =O, OH, $NH_2$, CN, $NO_2$, $CF_3$, $-C(=O)NH_2$, $-C(=O)NH-CH_3$, $-C(=O)N(CH_3)_2$, methyl, ethyl, propyl, isopropyl, methoxy, ethoxy, cyclopropyl, cyclobutyl, cyclopentyl or cyclohexyl, the methyl, ethyl, propyl, isopropyl, methoxy, ethoxy, cyclopropyl, cyclobutyl, cyclopentyl or cyclohexyl is optionally substituted with 0 to 4 substituents selected from H, F, Cl, Br, I or OH;

$R^{b3}$ and $R^{b4}$ are each independently selected from H, methyl, ethyl, isopropyl, or methoxymethyl;

alternatively, $R^{b3}$ and $R^{b4}$ together with the carbon atom to which they are attached form cyclopropyl, cyclobutyl, cyclopentyl, cyclohexyl, azetidinyl, azacyclopentyl, azacyclohexyl, oxetanyl, oxacyclopentyl, or oxacyclohexyl, the cyclopropyl, cyclobutyl, cyclopentyl, cyclohexyl, azetidinyl, azacyclopentyl, azacyclohexyl, oxetanyl, oxacyclopentyl, or oxacyclohexyl is optionally substituted with 0 to 4 substituents selected from H, F, Cl, Br, I, OH, $NH_2$, $C_{1-4}$ alkyl or $C_{1-4}$ alkoxy;

K is selected from

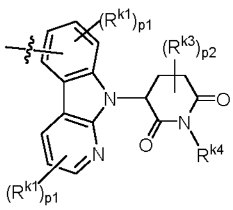,
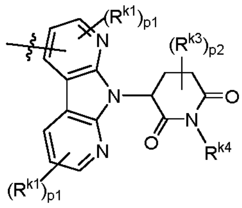,
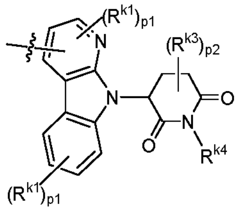,

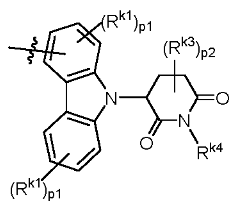,
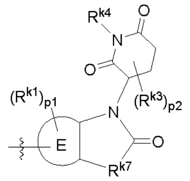,
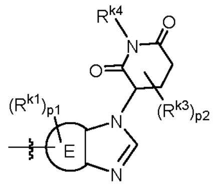,
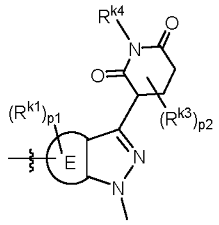,

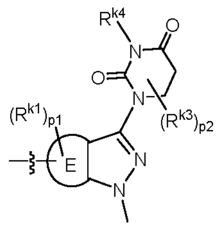,
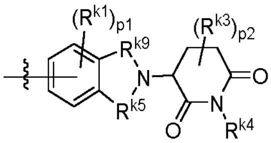,
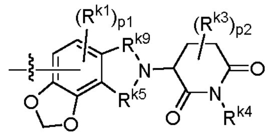,
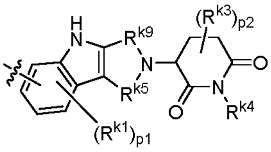,

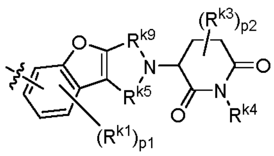,
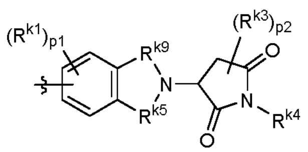,
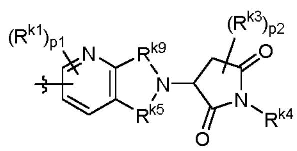,

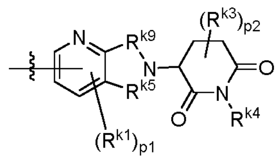,
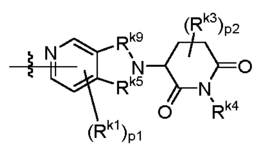,
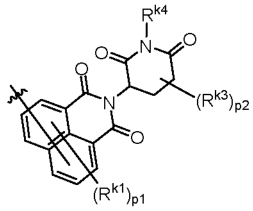,
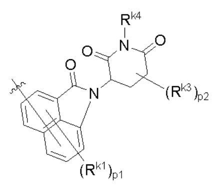,

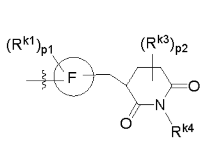, 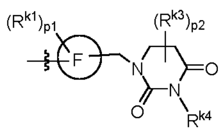, 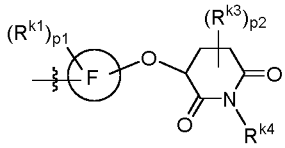, 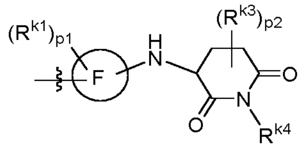,

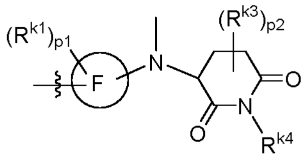, 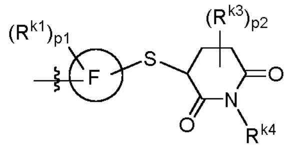, 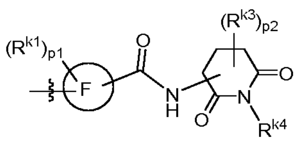,

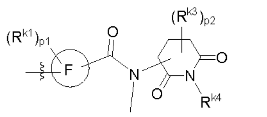, 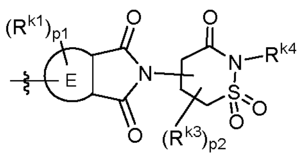, 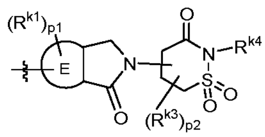,

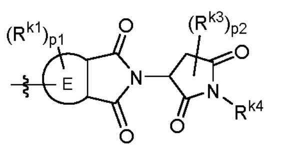, 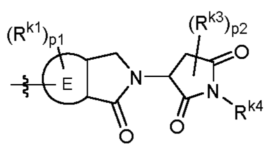, 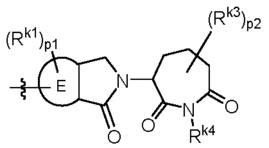,

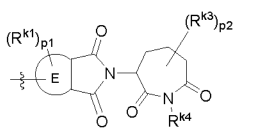, 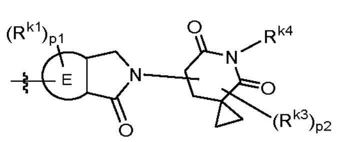, 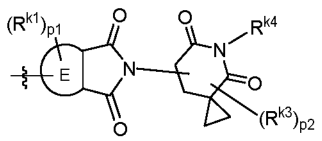,

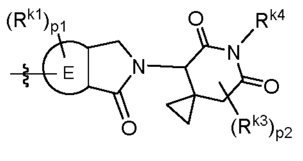, 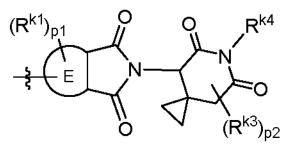, 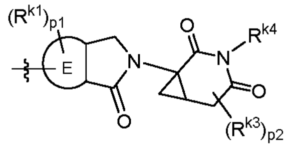,

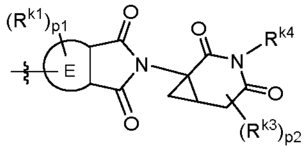, 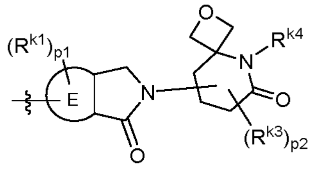, 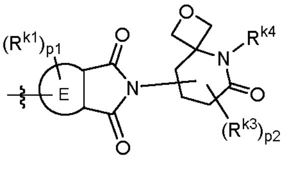,

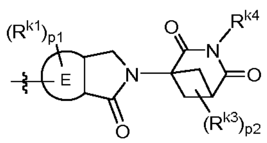, 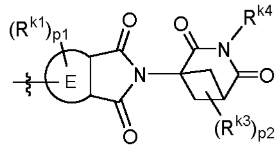, 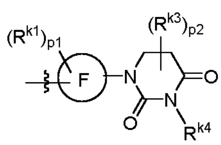, 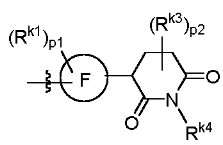,

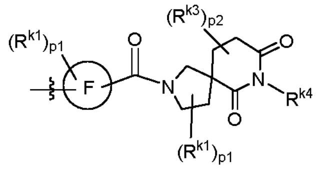, 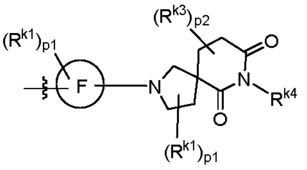, 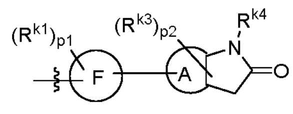,

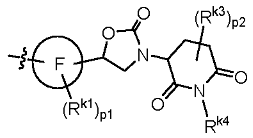, 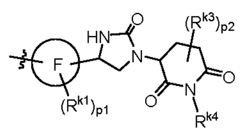, 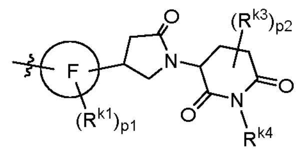,

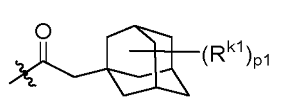, 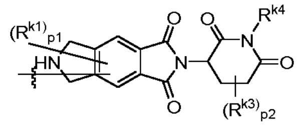, 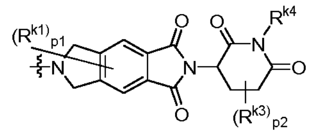,

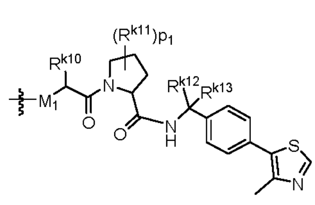, 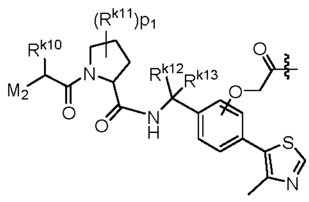, 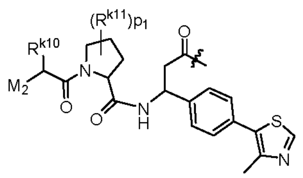,

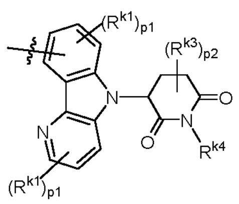 or 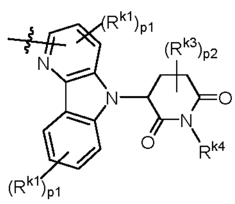;

M1 is selected from a bond, -C(=O)NH-, $-CH_2-C(=O)NH-$, $-C(=O)CH_2NH-$, pyrrolyl, pyrazolyl, imidazolyl, triazolyl, oxazolyl, isoxazolyl, furyl, thienyl, or thiazolyl;

$M_2$ is selected from $-NHC(=O)-CH_3$, -NHC(=O)-cyclopropyl, -NHC(=O)-cyclobutyl, azetidinyl, azacyclopentyl, benzoazacyclopentyl, or benzoazacyclohexyl, the cyclopropyl, cyclobutyl, azetidinyl, azacyclopentyl, benzoazacyclopentyl or benzoazacyclohexyl is optionally substituted with 0 to 4 substituents selected from H, F, Cl, Br, I, =O, OH, $NH_2$, $C_{1-4}$ alkyl or $C_{1-4}$ alkoxy;

$R^{k10}$ is selected from methyl, ethyl, isopropyl, propyl, or tert-butyl, the methyl, ethyl, isopropyl, propyl, or tert-butyl is optionally substituted with 0 to 4 substituents selected from H, F, Cl, Br, I, =O, OH, $C_{1-4}$ alkyl or $C_{3-6}$ cycloalkyl;

each $R^{k11}$ is independently selected from H, F, Cl, Br, I, =O, OH, SH, methyl, ethyl, isopropyl, propyl, methoxy, ethoxy, propoxy, isopropoxy, methylthio, ethylthio, propylthio or $-O-C(=O)-CH_3$, the methyl, ethyl, isopropyl, propyl, methoxy, ethoxy, propoxy, isopropoxy, methylthio, ethylthio, or propylthio is optionally substituted with 0 to 4 substituents selected from H, F, Cl, Br, I, OH, $C_{1-4}$ alkyl or $C_{1-4}$ alkoxy;

$R^{k12}$ and $R^{k13}$ are each independently selected from H, methyl, ethyl, isopropyl, propyl, cyclopropyl or cyclobutyl, the methyl, ethyl, isopropyl, propyl, cyclopropyl or cyclobutyl is optionally substituted with 0 to 4 substituents selected from H, F, Cl, Br, I, =O, OH, $NH_2$, $C_{1-4}$ alkyl or $C_{1-4}$ alkoxy;

each E is independently selected from phenyl, pyridyl, pyridazinyl, pyrazinyl, pyrimidinyl, pyrrolyl, pyrazolyl, imidazolyl, thiazolyl, furyl, thienyl or oxazolyl;

each A is independently selected from phenyl, pyridyl, pyridazinyl, pyrazinyl, pyrimidinyl, pyrrolyl, pyrazolyl, imidazolyl, thiazolyl, furyl, thienyl or oxazolyl;

each F is independently selected from cyclopropyl, cyclobutyl, cyclopentyl, cyclohexyl, bicyclo[1.1.1]pentyl, 6,7-dihydro-5H-cyclopen[c]pyridyl, 2,3-dihydro-1H-indenyl, phenyl, naphthyl, anthracyl, phenanthrenyl, azetidinyl, azacyclopentyl, piperidinyl, morpholinyl, pyridyl, pyrimidinyl, pyridazinyl, pyrazinyl, triazinyl, pyrrolyl, pyrazolyl, imidazolyl, triazolyl, oxazolyl, furyl, thienyl, thiazolyl, 2-pyridonyl, benzoimidazolyl, benzopyrazolyl, benzothiazolyl, benzothienyl, benzofuryl, benzopyrrolyl, benzopyridyl, benzopyrazinyl, benzopyrimidinyl, benzopyridazinyl, benzotriazinyl, pyrrolopyrrolyl, pyrrolopyridyl, pyrrolopyrimidinyl, pyrrolopyridazinyl, pyrrolopyrazinyl, imidazopyrimidinyl, imidazopyridyl, imidazopyrazinyl, imidazopyridazinyl, pyrazolopyridyl, pyrazolopyrimidinyl, pyrazolopyridazinyl, pyrazolopyrazinyl, pyrimidopyridyl, pyrimidopyrazinyl, pyrimidopyridazinyl, pyrimidopyrimidinyl, pyridopyridyl, pyridopyrazinyl, pyridopyridazinyl, pyridazinopyridazinyl, pyridazinopyrazinyl or pyrazinopyrazinyl;

each $R^{k7}$ is independently selected from $CH_2$, O, $N(CH_3)$ or NH;

each p1 or p2 is independently selected from 0, 1 or 2;

the definitions of other groups are the same as those in any one of the second, third and fourth embodiments of the present invention.

As a sixth embodiment of the present invention, provided is the above-mentioned compound shown in general formula (I) or the stereoisomer, deuterated compound, solvate, prodrug, metabolite, pharmaceutically acceptable salt or co-crystal thereof,

each Cy1, Cy2, Cy3, Cy4 or Cy5 is independently selected from a bond or one of the following substituted or unsubstituted groups:

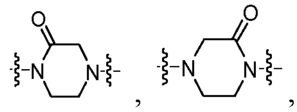 , ,

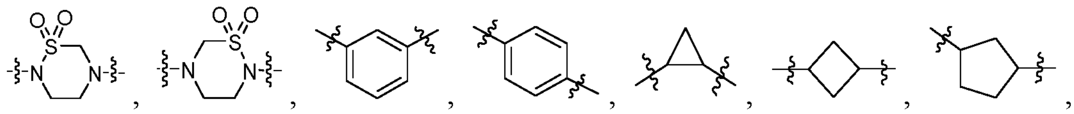 , , , , , , ,

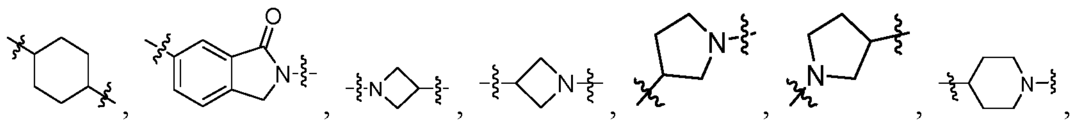 , , , , , , ,

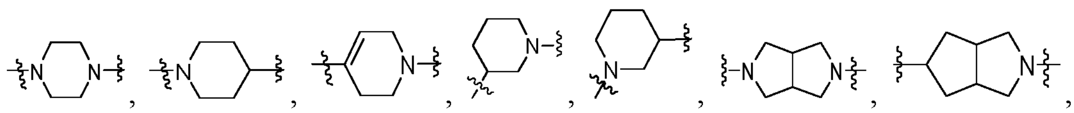 , , , , , , ,

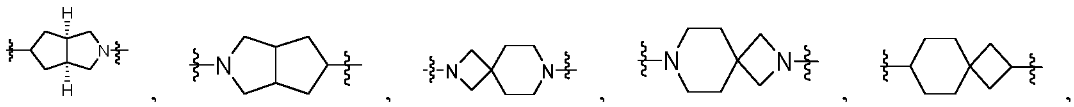 , , , , ,

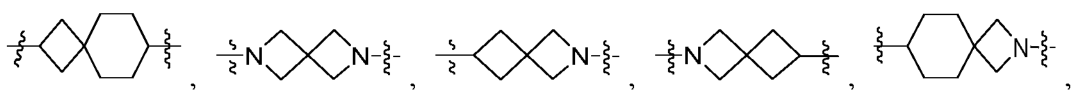 , , , , ,

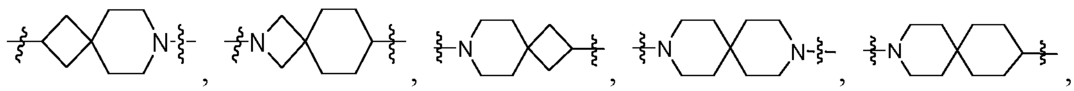 , , , , ,

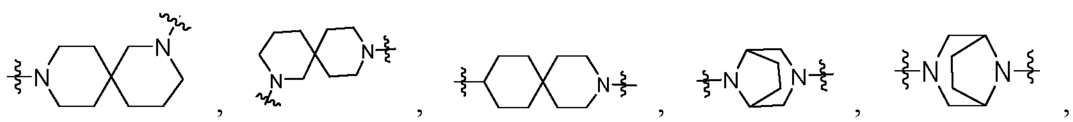 , , , , ,

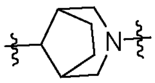, 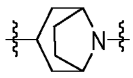, 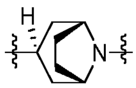, 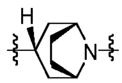, 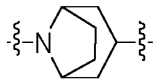, 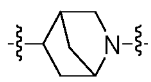,

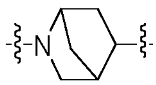, 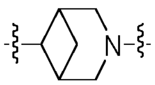, 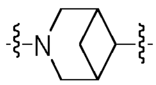, 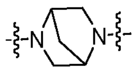, 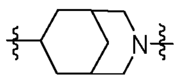, 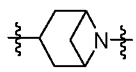,

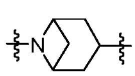, 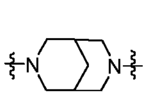, 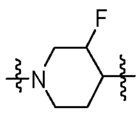, 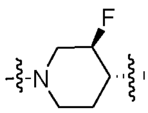, 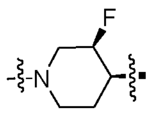, 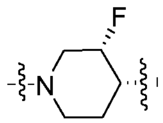,

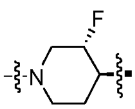, 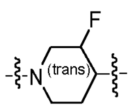, 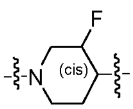, 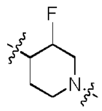, 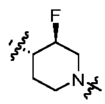, 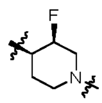, 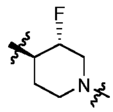, 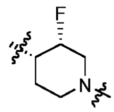,

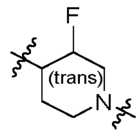 or 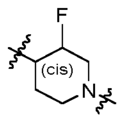;

, and when substituted, is optionally substituted with 0 to 4 substituents selected from H, F, $CF_3$, OH, methyl, methoxy, =O, hydroxymethyl, COOH, CN or $NH_2$;

B is selected from one of the following structures (Table C-1):

| | | | |
|---|---|---|---|
| 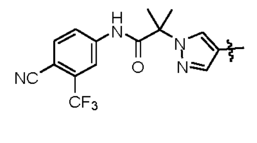 | 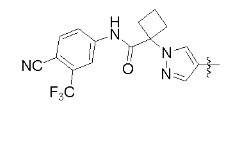 | 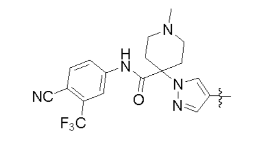 | 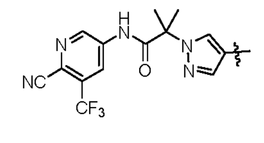 |
| 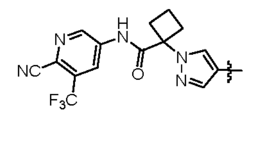 | 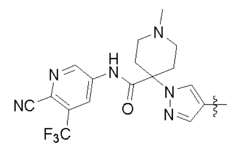 | 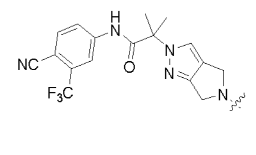 | 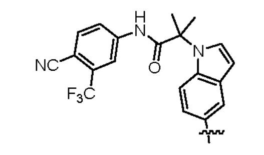 |

| | | | |
|---|---|---|---|
| 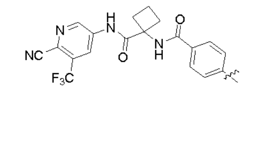 | 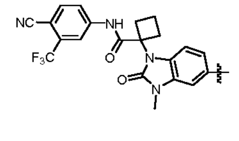 | 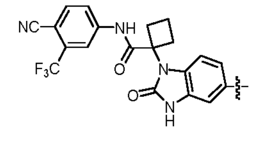 | 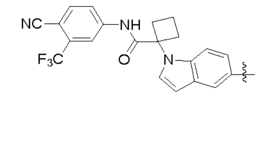 |
| 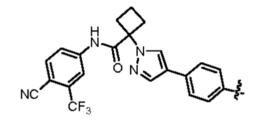 | 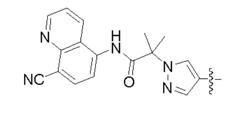 | 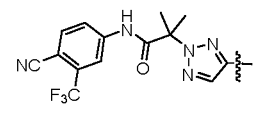 | 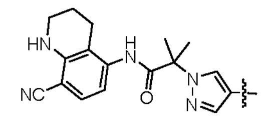 |
| 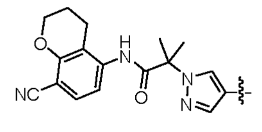 | 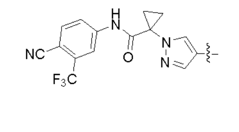 | 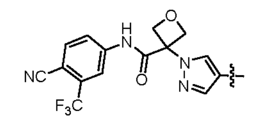 | 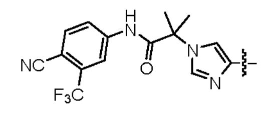 |
| 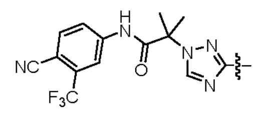 | 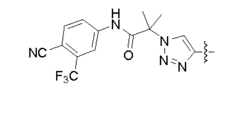 | 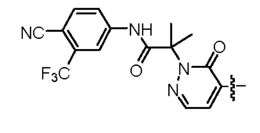 | 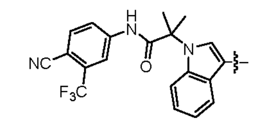 |
| 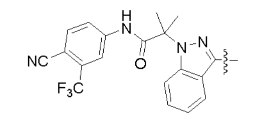 | 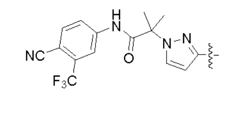 | 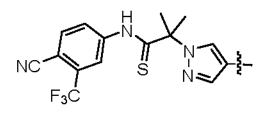 | |

alternatively, B is selected from one of the following structures (Table C-2):

| | | | |
|---|---|---|---|
| 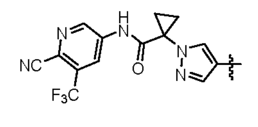 | 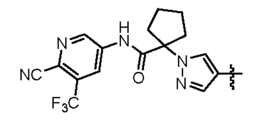 | 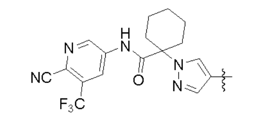 | 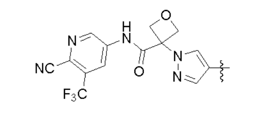 |
| 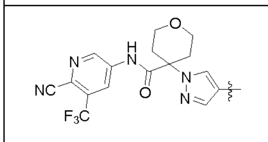 | 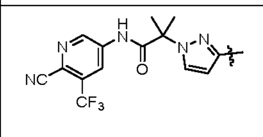 | 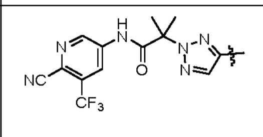 | 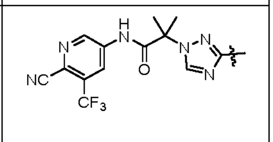 |
| 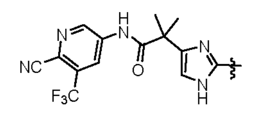 | 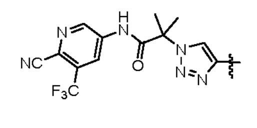 | 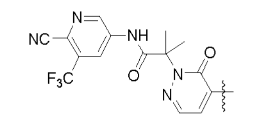 | 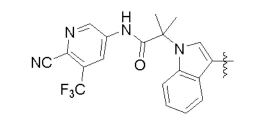 |
| 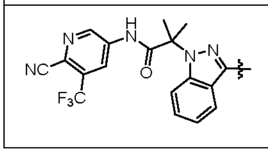 | 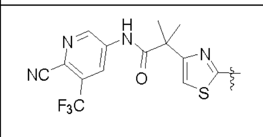 | 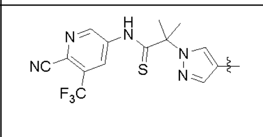 | 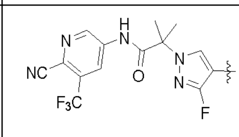 ; |

alternatively, B is selected from one of the following structures (Table C-3):

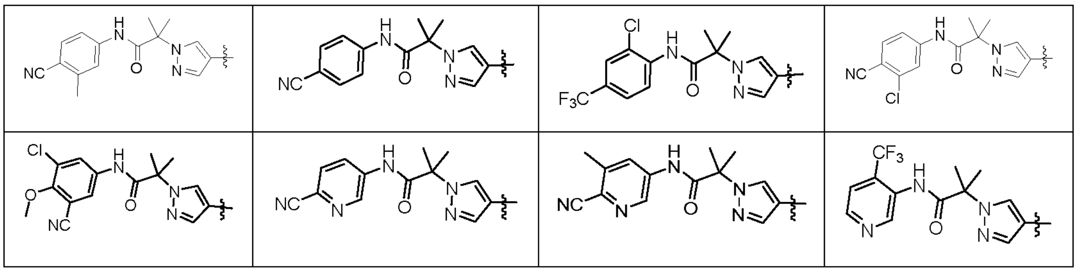

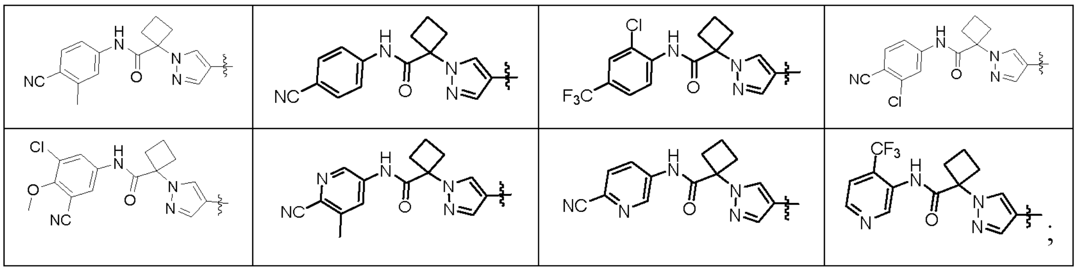
;

alternatively, B is selected from one of the following structures (Table C-4):

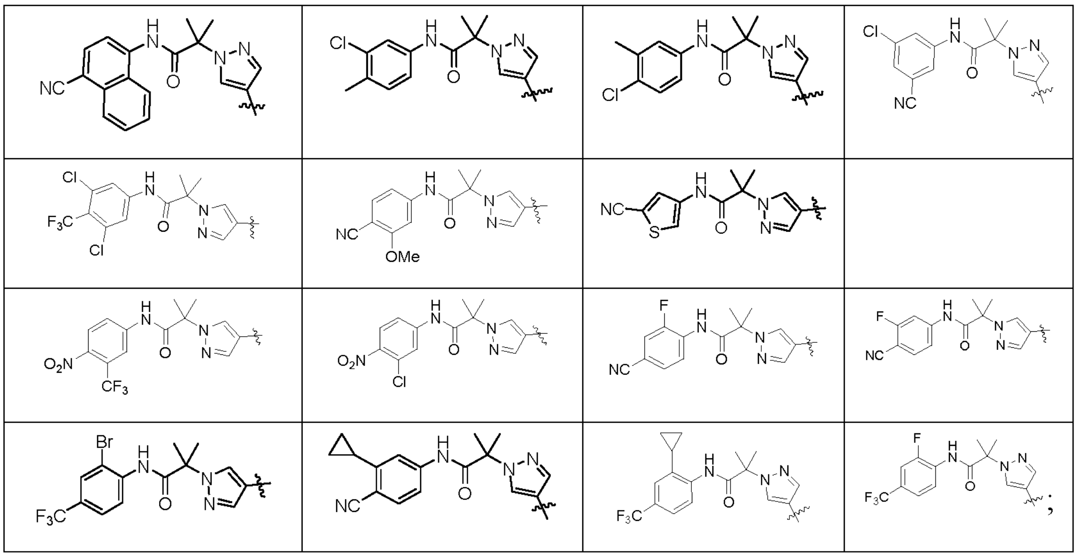
;

alternatively, B is selected from one of the following structures (Table C-5):

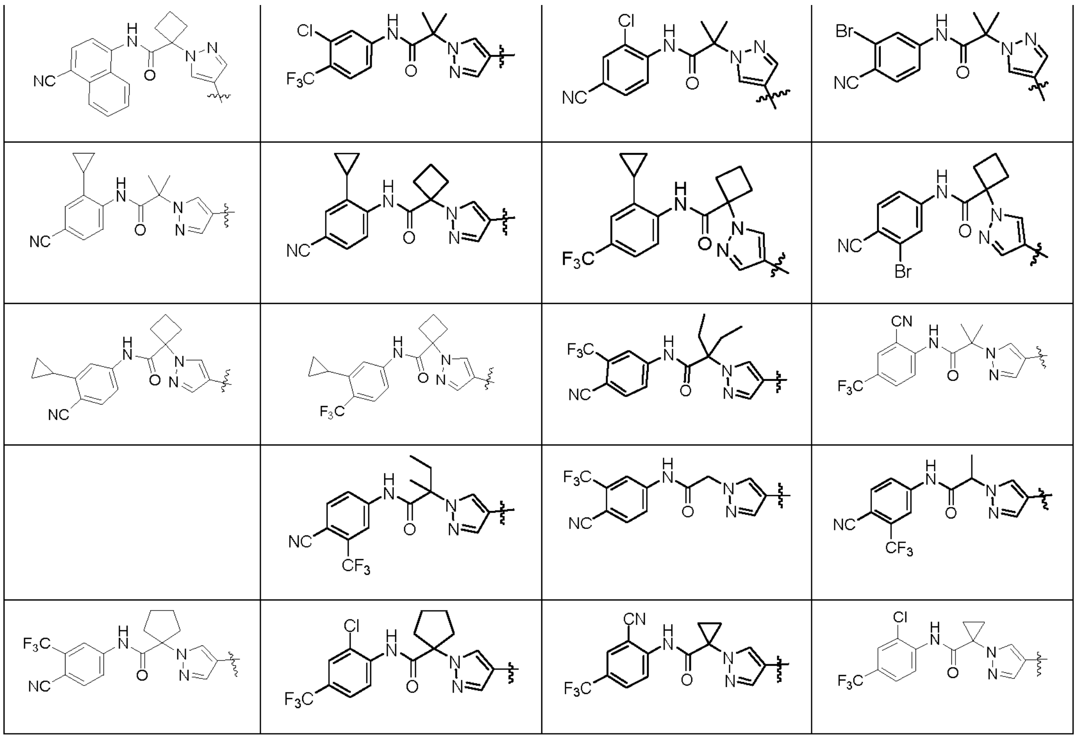

| | | | |
|---|---|---|---|
| 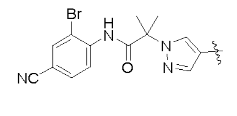 | 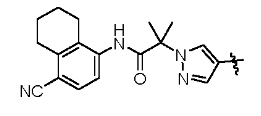 | 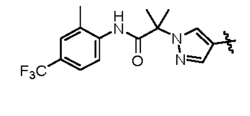 | 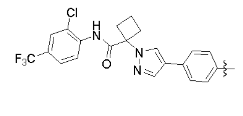 |
| 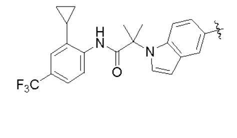 | 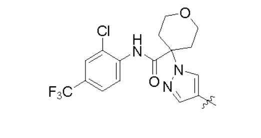 | 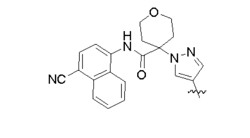 | 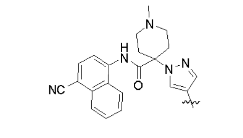 |
| 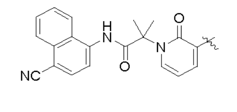 | 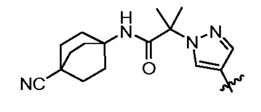 | 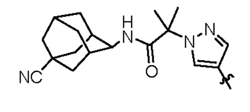 | |
| 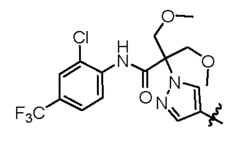 | 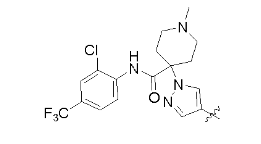 | 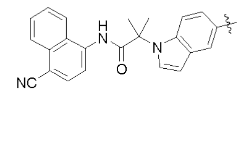 | 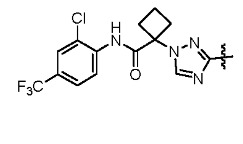 |
| 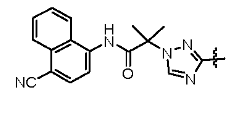 | 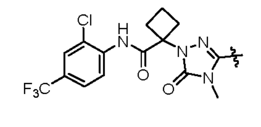 | 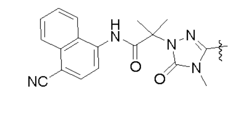 | 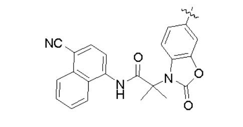 |
| 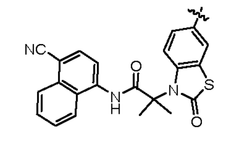 | 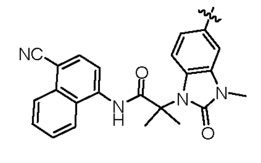 | 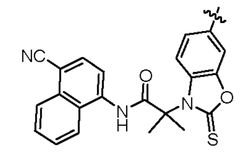 | 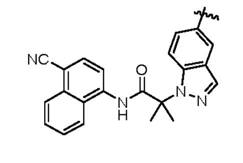 |
| 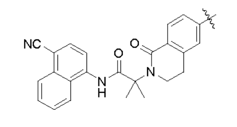 | 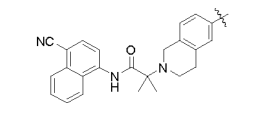 | 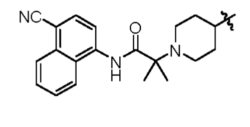 | 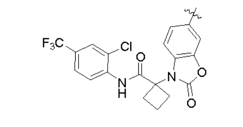 |
| 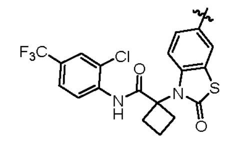 | 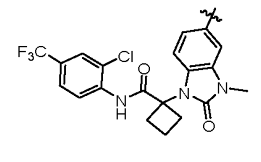 | 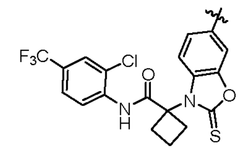 | 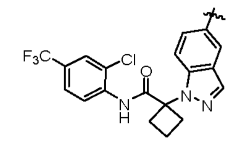 |
| 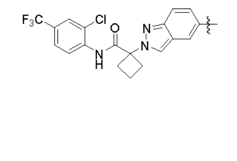 | 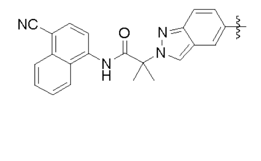 | 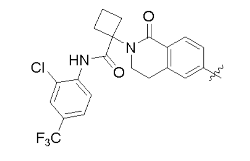 | 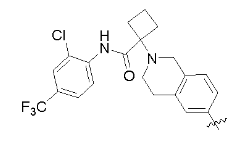 |
| 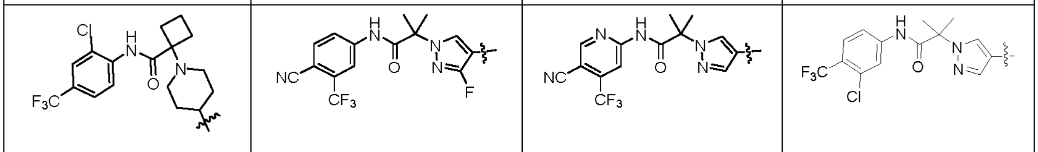 | | | |
| 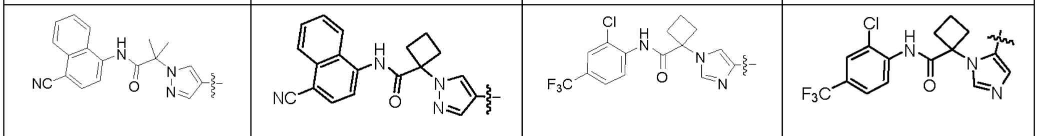 | | | |
| 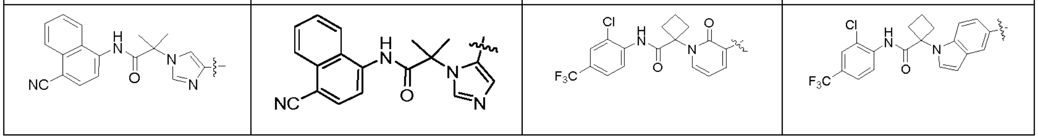 | | | |

| | | | |
|---|---|---|---|
| 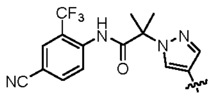 | 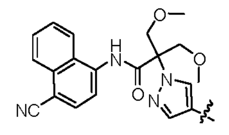 | 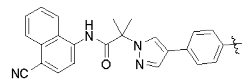 | ; |

alternatively, B is selected from one of the following structures (Table C-6):

| | | | |
|---|---|---|---|
| 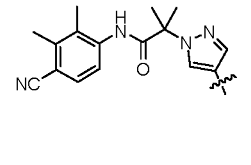 | 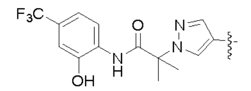 | 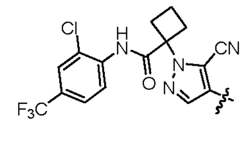 | 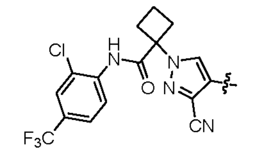 |
| 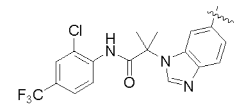 | 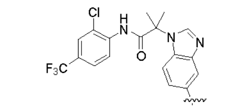 | 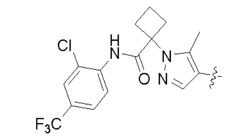 | 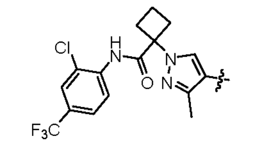 |
| 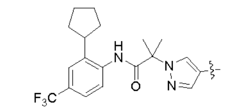 | 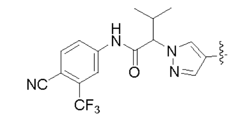 | 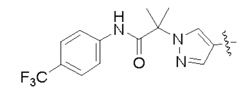 | 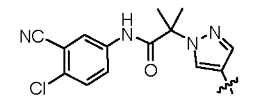 |
| 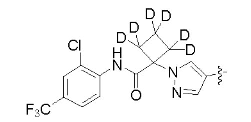 | 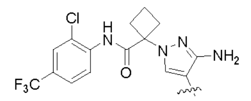 | 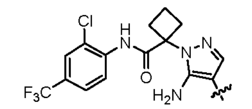 | 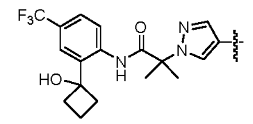 |
| 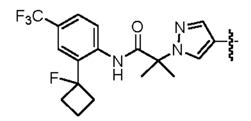 | 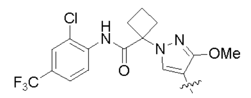 | 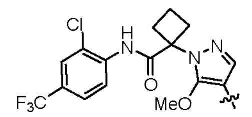 | 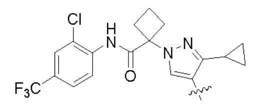 |
| 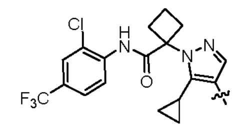 | 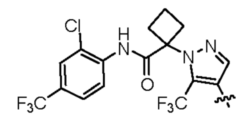 | 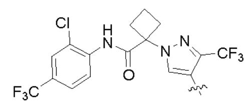 | 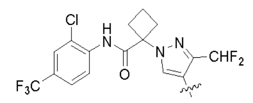 |
| 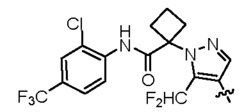 | 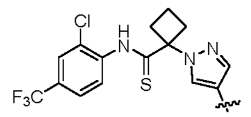 | 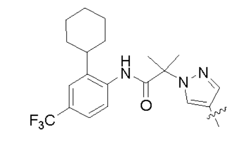 | 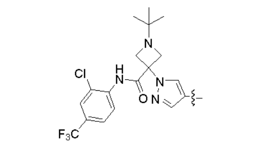 |
| 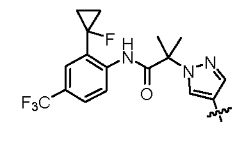 | 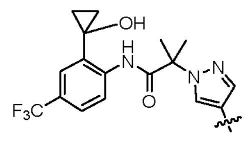 | 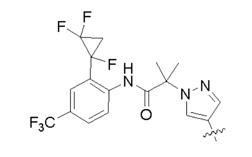 | 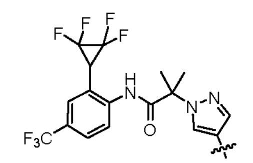 |
| 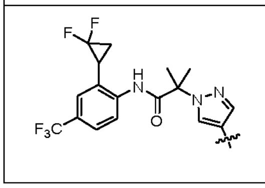 | 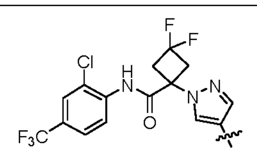 | 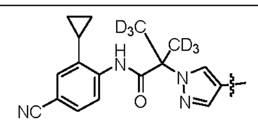 | 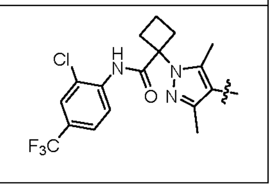 |

| | | | |
|---|---|---|---|
| 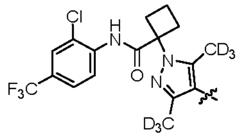 | 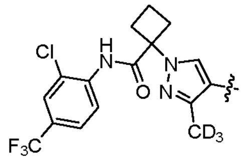 | 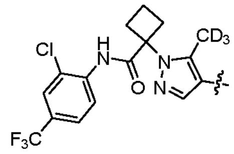 | 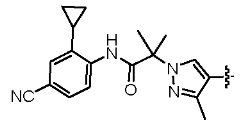 |
| 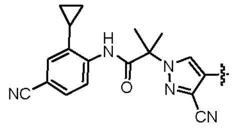 | 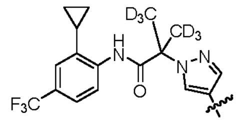 | 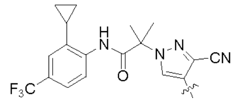 | 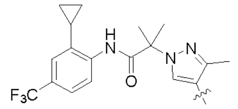 |
| 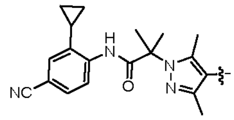 | 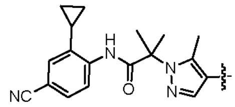 | | |

K is selected from one of the following structural fragments (Table K-1):

| | | | |
|---|---|---|---|
| 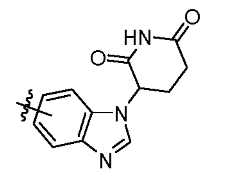 | 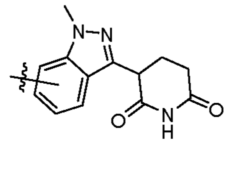 | 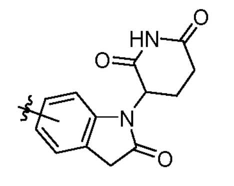 | 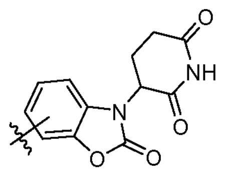 |
| 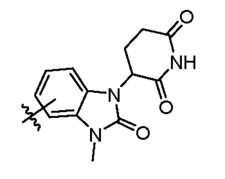 | 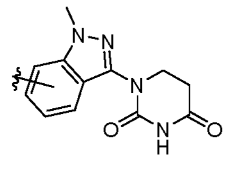 | 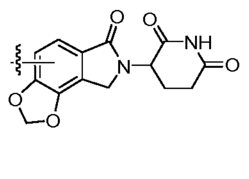 | 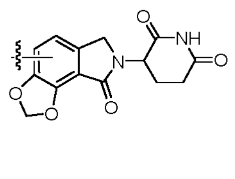 |
| 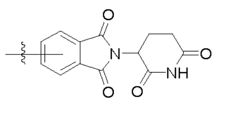 | 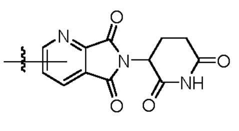 | 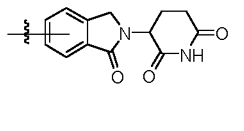 | 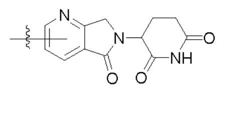 |
| 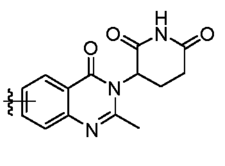 | 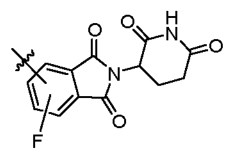 | 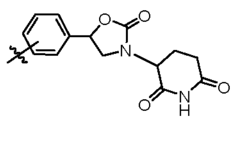 | 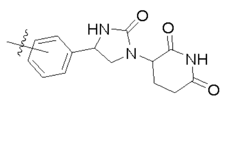 |
| 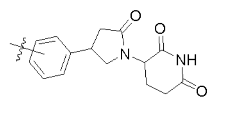 | 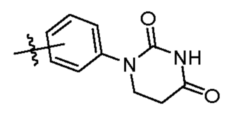 | 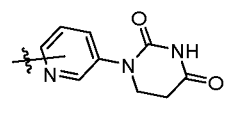 | 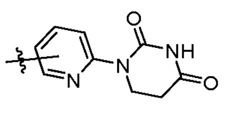 |
| 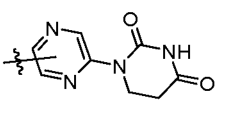 | 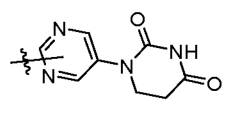 | 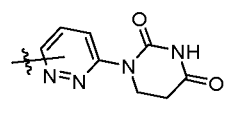 | 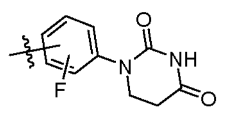 |
| 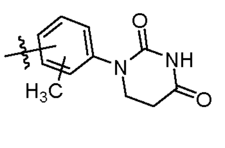 | 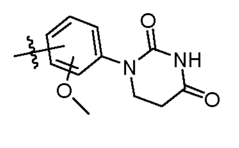 | 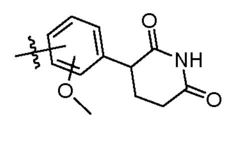 | 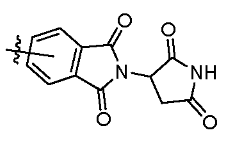 |
| 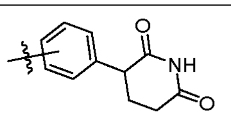 | 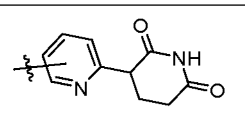 | 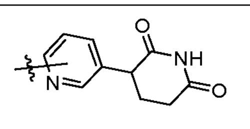 | 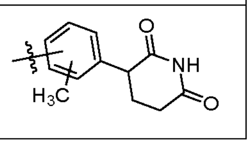 |

| | | | |
|---|---|---|---|
| 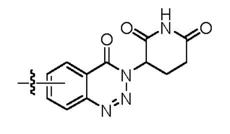 | 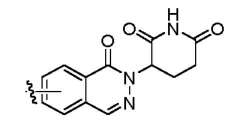 | 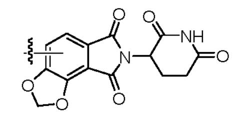 | 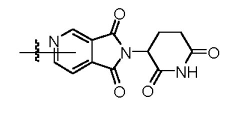 |
| 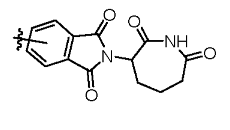 | 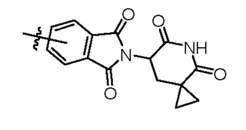 | 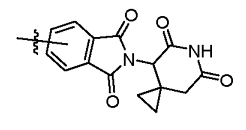 | 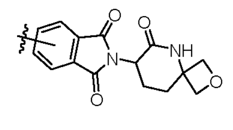 |
| 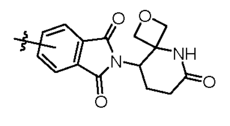 | 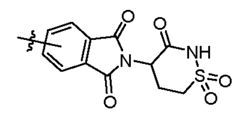 | 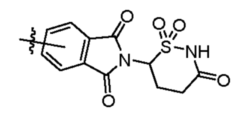 | 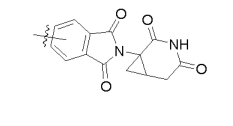 |
| 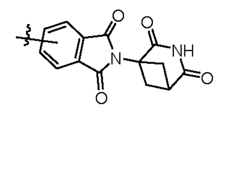 | 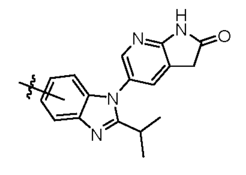 | 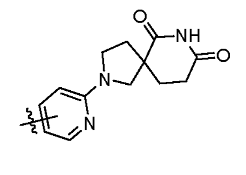 | 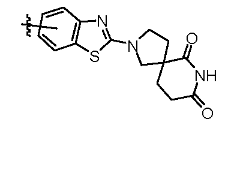 |
| 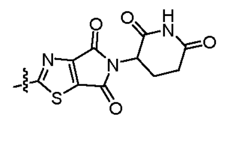 | 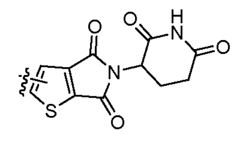 | | |
| 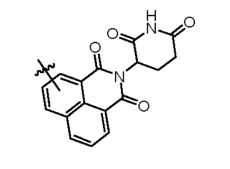 | 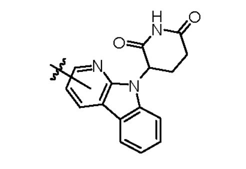 | 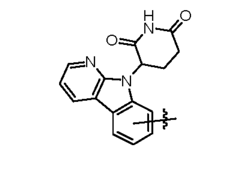 | 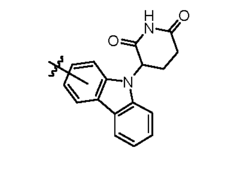 |
| 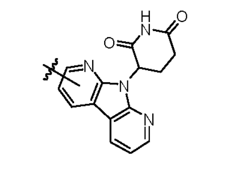 | 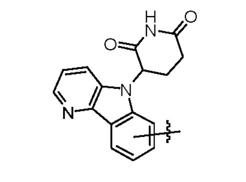 | 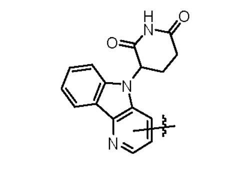 | 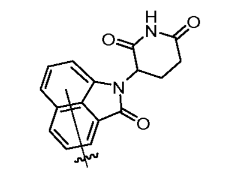 |
| 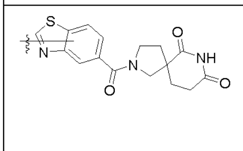 | 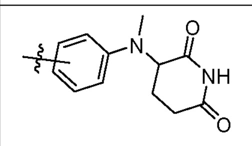 | 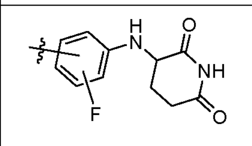 | 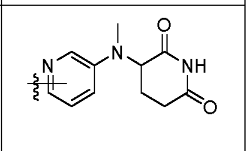 |
| 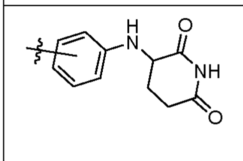 | 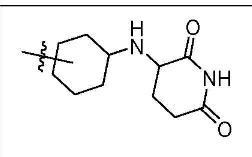 | 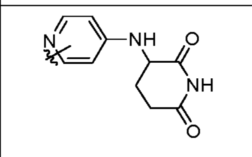 | 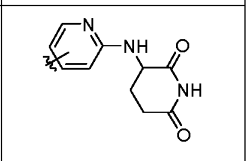 |
| 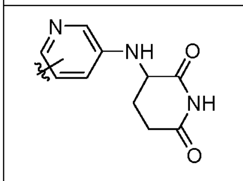 | 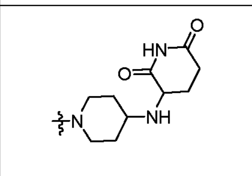 | 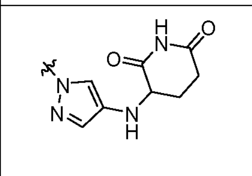 | 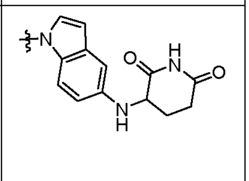 |
| 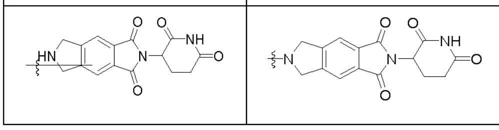 | | 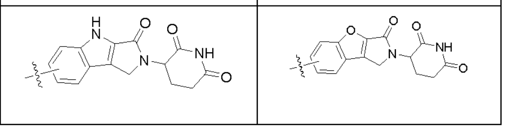 | |

| | | | |
|---|---|---|---|
| 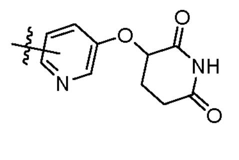 | 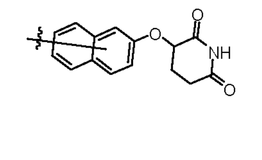 | 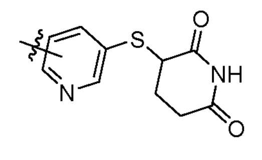 | 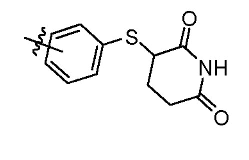 |
| 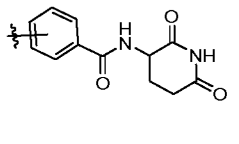 | 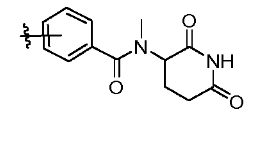 | 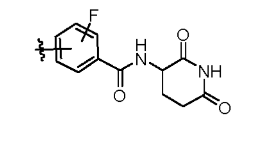 | 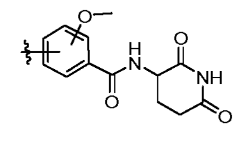 |
| 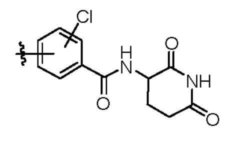 | 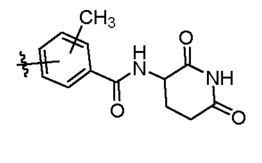 | 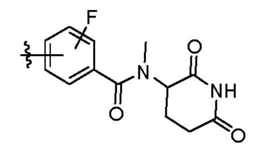 | 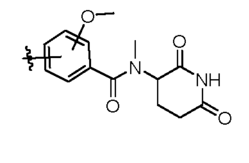 |
| 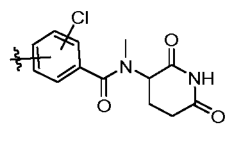 | 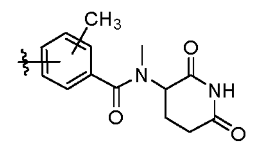 | 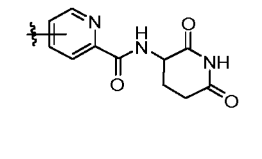 | 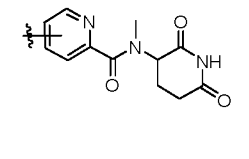 |
| 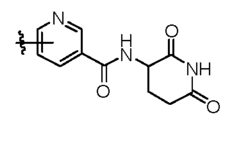 | 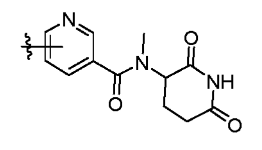 | 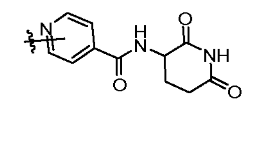 | 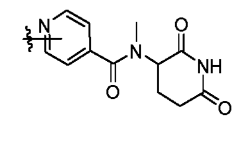 |
| 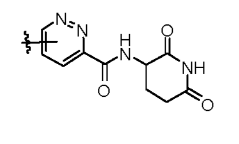 | 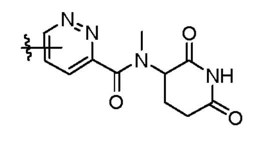 | 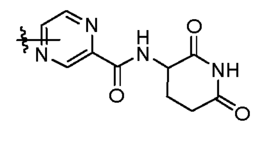 | 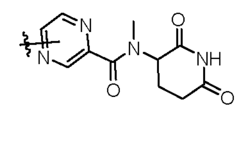 |
| 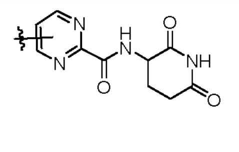 | 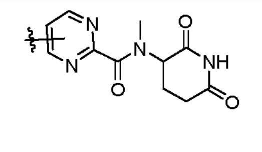 | 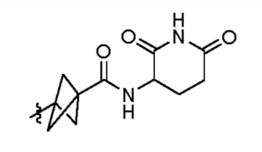 | 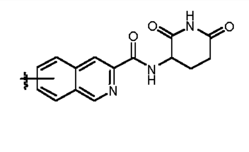 |
| 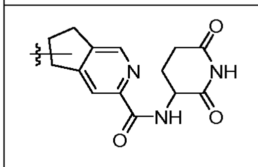 | 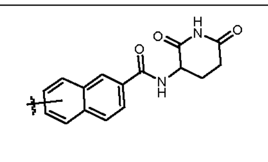 | 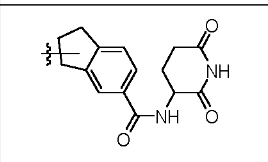 | 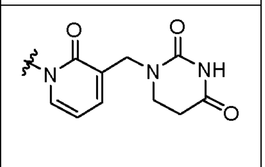 |
| 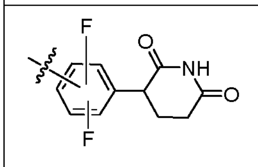 | | 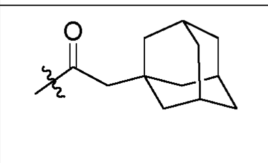 | |

| | | |
|---|---|---|
| 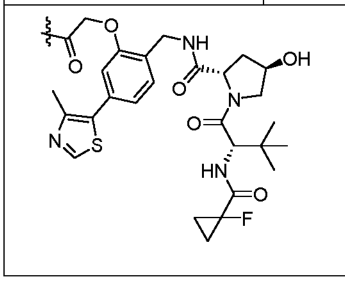 | 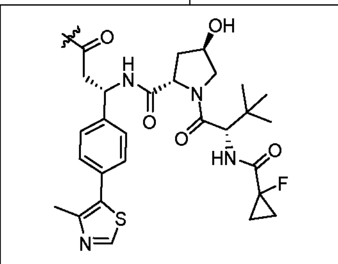 | 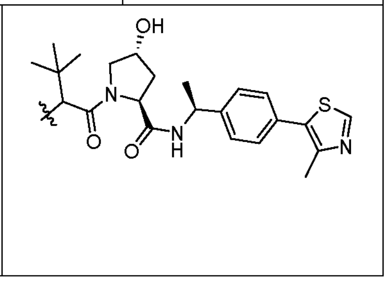 |

| | | |
|---|---|---|
| 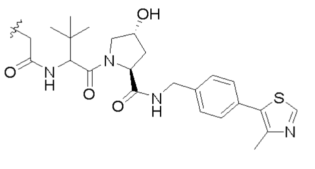 | 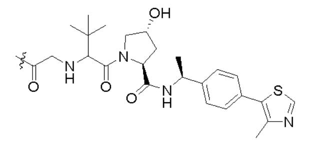 | 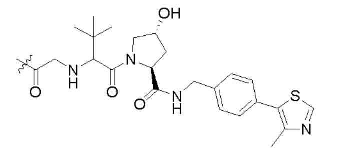 |
| 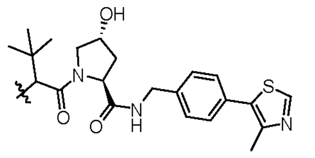 | 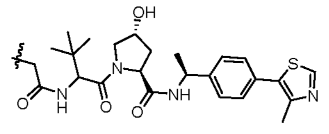 | 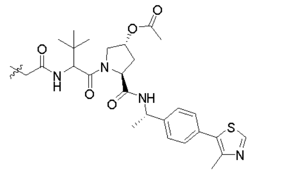 |
| 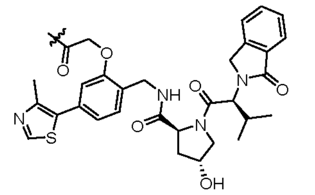 | 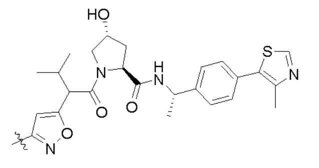 | 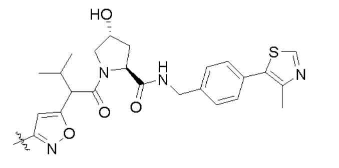 ; |

the definitions of other groups are the same as those in any one of the second, third, fourth or fifth embodiments of the present invention.

As a seventh embodiment of the present invention, provided is the above-mentioned compound shown in general formula (I) or the stereoisomer, deuterated compound, solvate, prodrug, metabolite, pharmaceutically acceptable salt or co-crystal thereof,

$B_1$ is selected from $C_{6-10}$ aryl, 5- to 10-membered heteroaryl, $C_{6-10}$ cycloalkyl, $C_{6-10}$ carbocyclyl, or 5- to 10-membered heterocyclyl, the aryl, heteroaryl, cycloalkyl, carbocyclyl or heterocyclyl is optionally substituted with 1 or 2 $R^{bb}$, the aryl, heteroaryl, cycloalkyl, carbocyclyl or heterocyclyl is optionally substituted with 0 to 3 $R^{b1}$, the heteroaryl or heterocyclyl contains 1 to 4 heteroatoms selected from O, S or N;

$R^{bb}$ is selected from -T-P(=O)$_2$(C$_{1-4}$ alkyl)$_2$, -T-S(=O)$_2$C$_{1-4}$ alkyl, -T-P(=O)$_2$(C$_{3-6}$ cycloalkyl)$_2$, -T-S(=O)$_2$C$_{3-6}$ cycloalkyl, -T-C(=O)NH$_2$, -T-C(=O)NH-C$_{1-4}$ alkyl, -T-C(=O)N(C$_{1-4}$ alkyl)$_2$, -T-C$_{1-4}$ alkyl, -T-C$_{2-6}$ alkenyl, -T-C$_{2-6}$ alkynyl, - T- C$_{3-12}$carbocyclyl, or -T-3- to 12-membered heterocyclyl, the alkyl, alkenyl, alkynyl, carbocyclyl or heterocyclyl is optionally substituted with 0 to 4 substituents selected from H, F, Cl, Br, I, OH, NH$_2$, =O, halogen-substituted C$_{1-4}$ alkyl, cyano-substituted C$_{1-4}$ alkyl, hydroxyl-substituted C$_{1-4}$ alkyl, C$_{1-4}$ alkyl, C$_{1-4}$ alkoxy, C$_{3-6}$ cycloalkyl or 3- to 7-membered heterocycle, the heterocycle contains 1 to 4 heteroatoms selected from O, S or N;

T is selected from a bond, -C(=O)-, -P(=O)$_2$-, -S(=O)$_2$-, -C(=O)NH-, - P(=O)$_2$NH-, -S(=O)$_2$NH-, -C(=O)N(C$_{1-4}$ alkyl)-, -P(=O)$_2$N(C$_{1-4}$ alkyl)-, - S(=O)$_2$N(C$_{1-4}$ alkyl)-, -NHC(=O)-, -NHP(=O)$_2$-, -NHS(=O)$_2$-, -N(C$_{1-4}$ alkyl)C(=O)-, -N(C$_{1-4}$ alkyl)P(=O)$_2$-, -N(C$_{1-4}$ alkyl)S(=O)$_2$-, O, S, NH, N(C$_{1-4}$ alkyl), N(C$_{3-6}$ cycloalkyl), N(3- to 10-membered heterocycle), C$_{1-4}$ alkylene, -O-C$_{1-4}$ alkylene-, -C$_{1-4}$ alkylene-O-, -S-C$_{1-4}$ alkylene-, -NH-C$_{1-4}$ alkylene-, -N(C$_{1-4}$ alkyl)-C$_{1-4}$ alkylene-, or -O-C$_{1-4}$ alkylene-O-, the alkyl or alkylene is optionally substituted with 0 to 4 substituents selected from H, F, Cl, Br, I, OH, =O, C$_{1-4}$ alkyl, C$_{1-4}$ alkoxy, C$_{3-6}$ cycloalkyl or 3- to 7-membered heterocycle, the heterocycle contains 1 to 4 heteroatoms selected from O, S or N;

alternatively, 2 $R^{bb}$ together with the atom to which they are attached form non-aromatic C$_{4-12}$ carbocycle or 4- to 12-membered heterocycle, the carbocycle or heterocycle is optionally substituted with 0 to 4 substituents selected from H, F, Cl, Br, I, OH, NH$_2$, =O, halogen-substituted C$_{1-4}$ alkyl, cyano-substituted C$_{1-4}$ alkyl, hydroxyl-substituted C$_{1-4}$ alkyl, C$_{1-4}$ alkyl, C$_{1-4}$ alkoxy, C$_{3-6}$ cycloalkyl or 3- to 7-membered heterocycle, the heterocycle contains 1 to 4 heteroatoms selected from O, S or N;

the definitions of other groups are the same as those in the first embodiment of the present invention.

As an eighth embodiment of the present invention, provided is the above-mentioned compound shown in general formula (I) or the stereoisomer, deuterated compound, solvate, prodrug, metabolite, pharmaceutically acceptable salt or co-crystal thereof,

the definition of L is the same as that in any one of the third, fourth, fifth or sixth embodiments of the present invention;

the definition of K is the same as that in any one of the third, fourth, fifth or sixth embodiments of the present invention; the definition of B is the same as those in the seventh embodiment of the present invention.

As a ninth embodiment of the present invention, provided is the above-mentioned compound shown in general formula (I) or the stereoisomer, deuterated compound, solvate, prodrug, metabolite, pharmaceutically acceptable salt or co-crystal thereof,

$B_1$ is selected from phenyl, naphthyl, thienyl, furyl, pyrrolyl, pyridyl, pyrimidinyl, pyrazinyl, pyridazinyl, quinolinyl, isoquinolinyl, quinazolinyl, benzofuryl, benzothienyl, benzopyrrolyl, benzoxazolyl, benzothiazolyl, benzoimidazolyl, benzopyrazolyl, azacyclopentyl, azacyclohexyl, oxacyclopentyl, oxacyclohexyl, adamantyl, bicyclo[2.2.2]octyl,

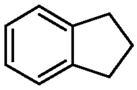 , or 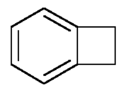 ,

the $B_1$ is optionally substituted with 1 or 2 $R^{bb}$, and the $B_1$ is optionally substituted with 0 to 3 $R^{b1}$;
$B_2$ is selected from -NHC(=O)- or one of the following substituted or unsubstituted groups: pyrazolyl, imidazolyl, triazolyl, thiazolyl, oxazolyl, benzopyrrolyl, benzoimidazolyl, pyrazolotetrahydropyrrolyl, 3-pyridazinonyl, benzopyrazolyl, 2-pyridonyl, 1,2,3,4-tetrahydroquinolinyl, 1,2,3,4-tetrahydroisoquinolinyl, azacyclohexyl,

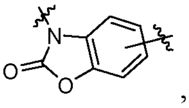 , 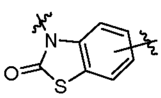 , 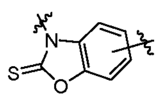 ,

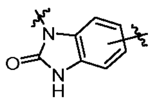 , 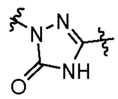 , 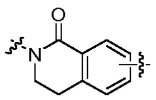 , 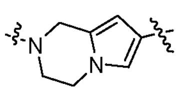 , 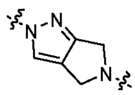 , 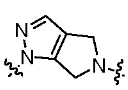 , or

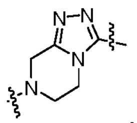 ,

, and when substituted, is optionally substituted with 0 to 4 $R^{b2}$,
$B_3$ is selected from a bond or phenyl, the phenyl is optionally substituted with 0 to 4 $R^{b2}$,
$R^{bb}$ is selected from $-P(=O)_2(CH_3)_2$, $-S(=O)_2CH_3$, $-T-C(=O)NH_2$, $-T-C(=O)NHCH_3$, $-T-C(=O)N(CH_3)_2$, $-T-C(=O)NHCH_2CH_3$, $-T-C(=O)N(CH_2CH_3)_2$, - T-$C_{3-6}$ monocyclic cycloalkyl, -T-phenyl, $-OCH_2CH_2OCH_3$, $-OCH_2CH_2OCH_2CH_3$, -T-5- to 6-membered heteroaryl, -T-4- to 6-membered monocyclic heterocycloalkyl, -T-6- to 10-membered spirocyclic heterocycloalkyl, -T-6- to 10-membered fused cyclic heterocycloalkyl, -T-benzopyrazolyl, -T-benzoimidazolyl, - T-benzopyrrolyl, -T-indolinyl, -T-vinyl, -T-propan-1,2-dien-1-yl, -T-ethynyl, -T-propynyl, or -T-propargyl, the $CH_2$, $CH_3$, cycloalkyl, phenyl, heterocycloalkyl, heteroaryl, benzopyrazolyl, benzoimidazolyl, benzopyrrolyl, indolinyl, vinyl, prop-1,2-dien-1-yl, ethynyl, propynyl, or propargyl is optionally substituted with 0 to 4 substituents selected from H, F, Cl, Br, I, OH, $NH_2$, =O, halogen-substituted $C_{1-4}$ alkyl, cyano-substituted $C_{1-4}$ alkyl, hydroxyl-substituted $C_{1-4}$ alkyl, $C_{1-4}$ alkyl, $C_{1-4}$ alkoxy, $C_{3-6}$ cycloalkyl or 3- to 7-membered heterocycloalkyl, the heterocycloalkyl contains 1 to 4 heteroatoms selected from O, S or N;
alternatively, $R^{bb}$ is selected from -T-methyl or -T-ethyl, the methyl or ethyl is substituted with 1 to 4 substituents selected from F, Cl, Br, I, OH, $NH_2$, =O, halogen-substituted $C_{1-4}$ alkyl, cyano-substituted $C_{1-4}$ alkyl, hydroxyl-substituted $C_{1-4}$ alkyl, $C_{1-4}$ alkyl, $C_{1-4}$ alkoxy, $C_{3-6}$ cycloalkyl or 3- to 7-membered heterocycloalkyl, the heterocycloalkyl contains 1 to 4 heteroatoms selected from O, S or N;
T is selected from a bond, -C(=O)-, O, S, NH, $N(CH_3)$, $-CH_2-$, $-CH_2CH_2-$, - $OCH_2O-$, $-OCH_2-$, $-CH_2O-$, $-OCH_2CH_2O-$, $-OCH_2CH_2-$, or $-CH_2CH_2O-$, the $CH_2$ or $CH_3$ is optionally substituted with 0 to 4 substituents selected from H, F, Cl, Br, I, OH, =O, $C_{1-4}$ alkyl, $C_{1-4}$ alkoxy, $C_{3-6}$ cycloalkyl or 3- to 7-membered heterocycle;
alternatively, 2 $R^{bb}$ together with the atom to which they are attached form non-aromatic $C_{4-12}$ carbocycle or 4- to 12-membered heterocycle, the carbocycle or heterocycle is optionally substituted with 0 to 4 substituents selected

from H, F, Cl, Br, I, OH, $NH_2$, =O, halogen-substituted $C_{1-4}$ alkyl, cyano-substituted $C_{1-4}$ alkyl, hydroxyl-substituted $C_{1-4}$ alkyl, $C_{1-4}$ alkyl, $C_{1-4}$ alkoxy, $C_{3-6}$ cycloalkyl or 3- to 7-membered heterocycle, the heterocycle contains 1 to 4 heteroatoms selected from O, S or N;

$R^{b1}$ and $R^{b2}$ are each independently selected from H, F, Cl, Br, I, =O, OH, $NH_2$, CN, $NO_2$, $CF_3$, -C(=O)$NH_2$, -C(=O)NH-$CH_3$, -C(=O)N$(CH_3)_2$, methyl, ethyl, propyl, isopropyl, methoxy, ethoxy, cyclopropyl, cyclobutyl, cyclopentyl or cyclohexyl, the methyl, ethyl, propyl, isopropyl, methoxy, ethoxy, cyclopropyl, cyclobutyl, cyclopentyl or cyclohexyl is optionally substituted with 0 to 4 substituents selected from H, F, Cl, Br, I or OH;

$R^{b3}$ and $R^{b4}$ are each independently selected from H, methyl, ethyl, isopropyl, or methoxymethyl;

alternatively, $R^{b3}$ and $R^{b4}$ together with the carbon atom to which they are attached form cyclopropyl, cyclobutyl, cyclopentyl, cyclohexyl, azetidinyl, azacyclopentyl, azacyclohexyl, oxetanyl, oxacyclopentyl, or oxacyclohexyl, the cyclopropyl, cyclobutyl, cyclopentyl, cyclohexyl, azetidinyl, azacyclopentyl, azacyclohexyl, oxetanyl, oxacyclopentyl, or oxacyclohexyl is optionally substituted with 0 to 4 substituents selected from H, F, Cl, Br, I, OH, $NH_2$, $C_{1-4}$ alkyl or $C_{1-4}$ alkoxy;

the definitions of other groups are the same as those in the eighth embodiment of the present invention.

As a tenth embodiment of the present invention, the compound represented by the above general formula (I) or a stereoisomer, deuterated compound, solvate, prodrug, metabolite, pharmaceutically acceptable salt or co-crystal thereof is provided,

$R^{bb}$ is selected from -P(=O)$_2$$(CH_3)_2$, -S(=O)$_2$$CH_3$, -$OCH_2CH_2OCH_3$, - $OCH_2$C(=O)NH($CH_3$), -T-C(=O)$NH_2$, -T-C(=O)$NHCH_3$, -T-C(=O)N($CH_3$), -T-cyclopropyl, -T-cyclobutyl, -T-cyclopentyl, -T-cyclohexyl, -T-azetidinyl, -T-azacyclopentyl, -T-azacyclohexyl, -T-oxetanyl, -T-oxacyclopentyl, -T-oxacyclohexyl, -T-phenyl, -T-pyridyl, -T-pyrrolyl, -T-thienyl, -T-furyl, -T-pyrazolyl, -T-pyrrolyl, -T-imidazolyl, -T-thiazolyl, -T-oxazolyl, -T-triazolyl, -T-pyridazinyl, -T-pyridazinonyl, -T-morpholinyl, -T-cyclopropyl-spiro-azacyclohexyl, -T-cyclopentyl-fused-azacyclopentyl, -T-benzopyrazolyl, -T-benzoimidazolyl, -T-benzopyrrolyl, -T-dihydroindolyl, -T-vinyl, -T-prop-1,2-dien-1-yl, -T-ethynyl, -T-propynyl, or -T-propargyl, and the $CH_2$, $CH_3$, cyclopropyl, cyclobutyl, cyclopentyl, cyclohexyl, azetidinyl, azacyclopentyl, azacyclohexyl, oxetanyl, oxacyclopentyl, oxacyclohexyl, phenyl, pyridyl, pyrrolyl, thienyl, furyl, pyrazolyl, pyrrolyl, imidazolyl, thiazolyl, oxazolyl, triazolyl, pyridazinyl, pyridazinone, morpholinyl, cyclopropyl-spiro-azacyclohexyl, cyclopentyl-fused-azacyclopentyl, benzopyrazolyl, benzoimidazolyl, benzopyrrolyl, dihydroindolyl, vinyl, prop-1,2-dien-1-yl, ethynyl, propynyl, or propargyl is optionally substituted with 0 to 4 substituents selected from H, F, Cl, Br, I, OH, =O, $CF_3$, methyl, ethyl, methoxy, ethoxy, cyclopropyl, azetidinyl, azacyclopentyl, piperidinyl or piperazinyl;

alternatively, $R^{bb}$ is selected from -T-methyl or -T-ethyl, the methyl or ethyl is substituted with 1 to 4 substituents selected from F, Cl, Br, I, OH, =O, $CF_3$, methyl, ethyl, methoxy, ethoxy, or cyclopropyl;

T is selected from a bond, -C(=O)-, O, S, NH, N($CH_3$), -$CH_2$-, -$CH_2CH_2$-, - $OCH_2O$-, -$OCH_2$-, -$CH_2O$-, -$OCH_2CH_2O$-, -$OCH_2CH_2$-, or -$CH_2CH_2O$-, the $CH_2$ or $CH_3$ is optionally substituted with 0 to 4 substituents selected from H, F, Cl, Br, I, OH, =O, methyl, ethyl, methoxy, ethoxy, or cyclopropyl;

alternatively, 2 $R^{bb}$ together with the atom to which they are attached form a ring as shown below:

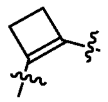, 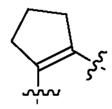, 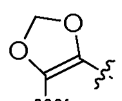, 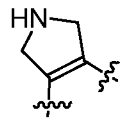, 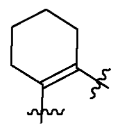, 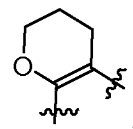,

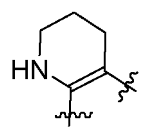, 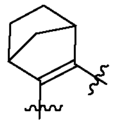, 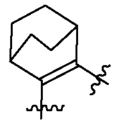, or 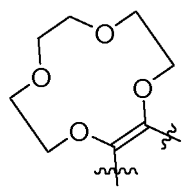,

the ring is optionally substituted with 1 to 4 substituents selected from F, Cl, Br, I, OH, $NH_2$, methyl, ethyl, or cyclopropyl;

the definitions of other groups are the same as those in the ninth embodiment of the present invention.

As an eleventh embodiment of the present invention, the compound represented by the above general formula (I) or a stereoisomer, deuterated compound, solvate, prodrug, metabolite, pharmaceutically acceptable salt or co-crystal

thereof is provided,

B is selected from one of the structures in Table C-1, Table C-2, Table C-3, Table C-4, Table C-5, or Table C-6, or one of the following structures (Table C-7):

| | | | |
|---|---|---|---|
| 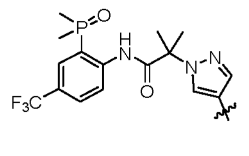 | 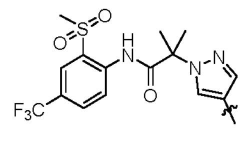 | 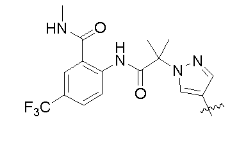 | 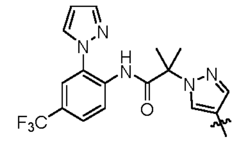 |
| 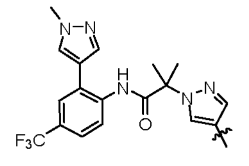 | 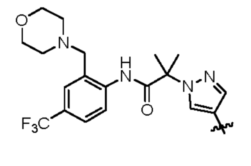 | 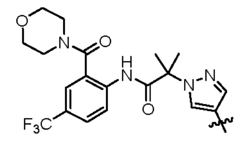 | 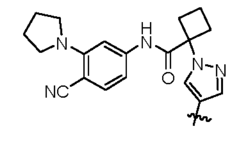 |
| 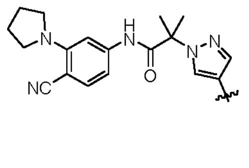 | 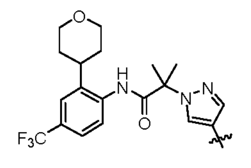 | 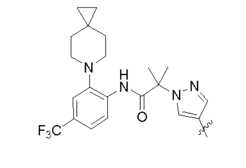 | 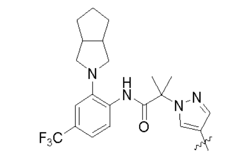 |
| 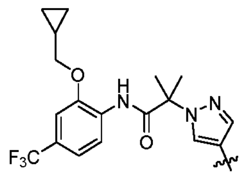 | 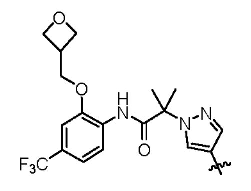 | 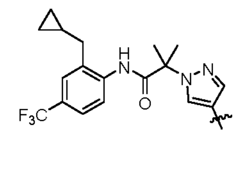 | 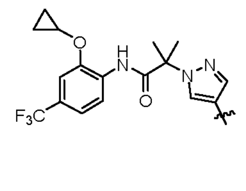 |
| 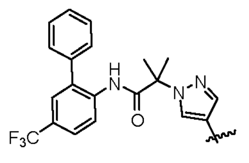 | 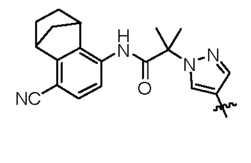 | 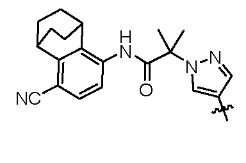 | 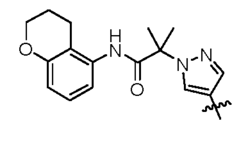 |

| | | | |
|---|---|---|---|
| 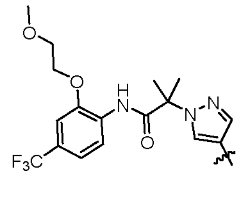 | 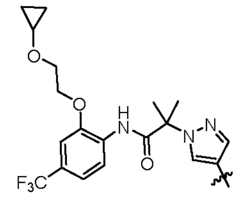 | 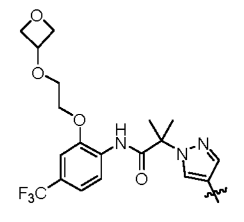 | 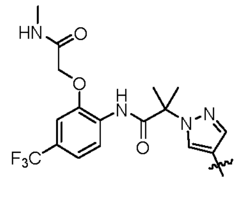 |
| 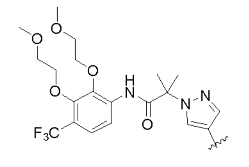 | 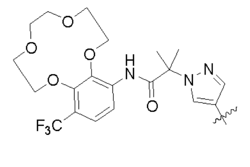 | 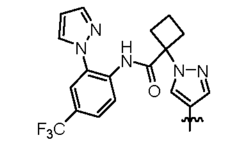 | 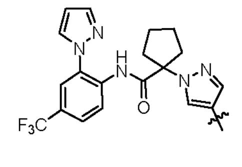 |
| 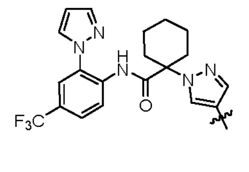 | 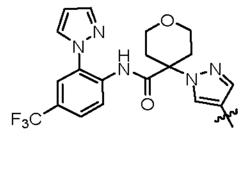 | 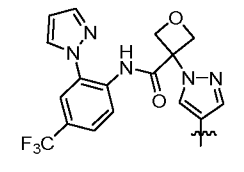 | 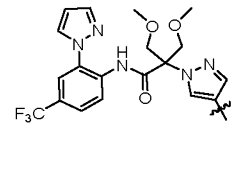 |
| 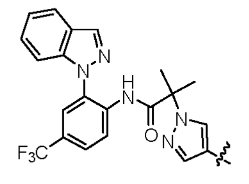 | 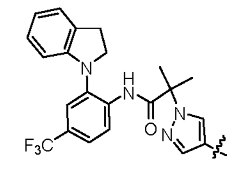 | 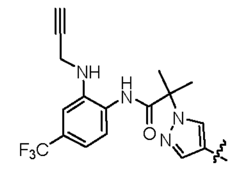 | 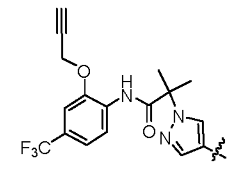 |
| 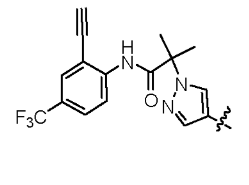 | 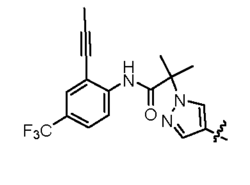 | 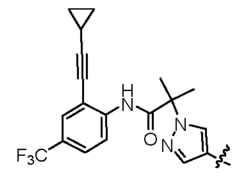 | 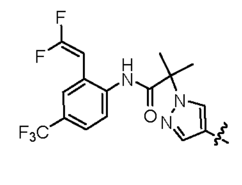 |
| 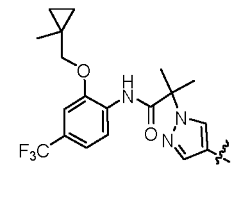 | 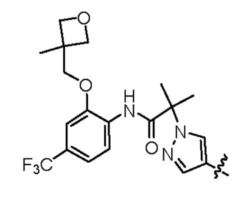 | 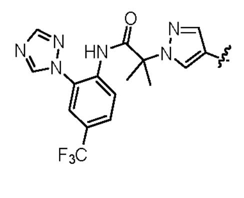 | 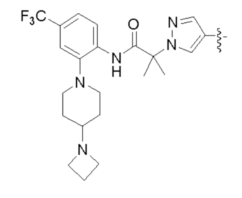 |
| 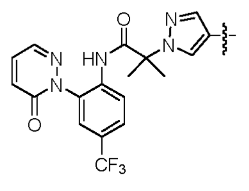 | 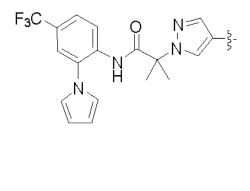 | 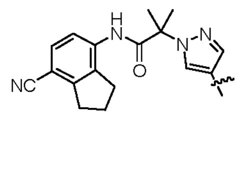 | 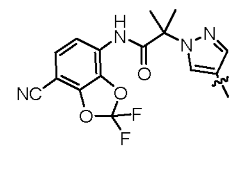 |
| 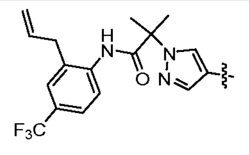 | 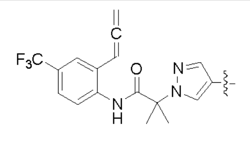 | 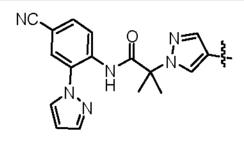 | 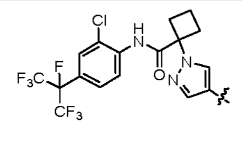 |
| 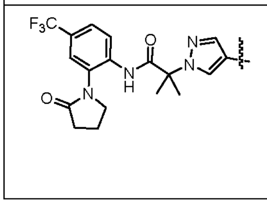 | 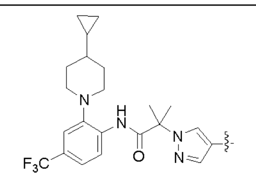 | 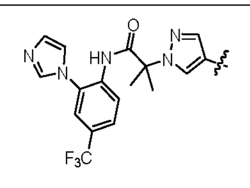 | 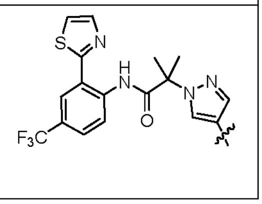 |

| | | | |
|---|---|---|---|
| 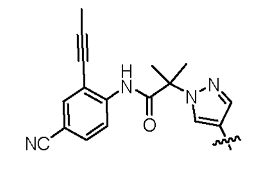 | 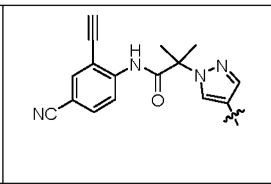 | 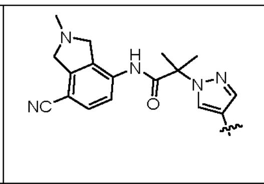 | 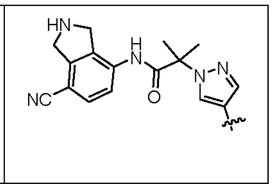 |
| 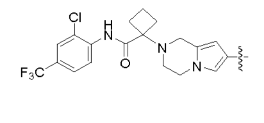 | 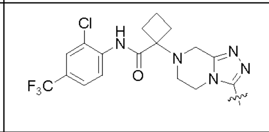 | 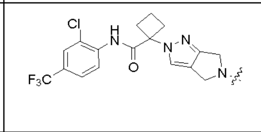 | 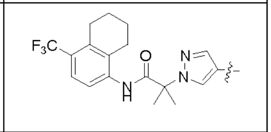 |
| 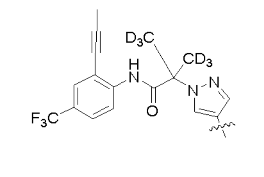 | 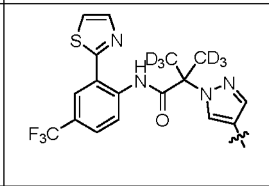 | 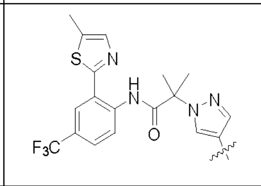 | 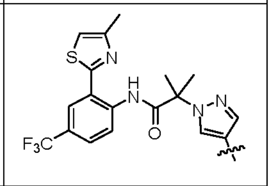 |
| 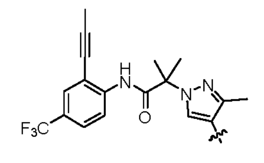 | 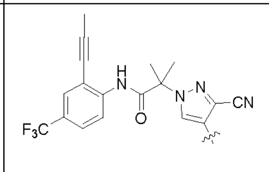 | 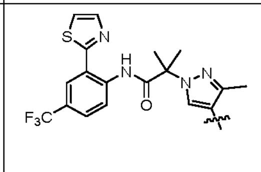 | 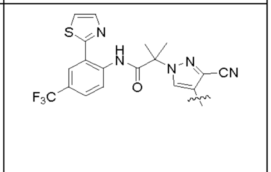 |
| 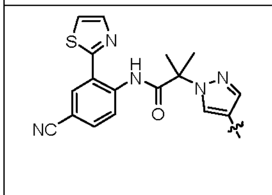 | 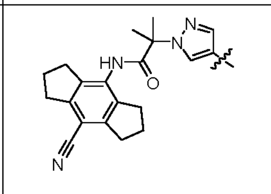 | 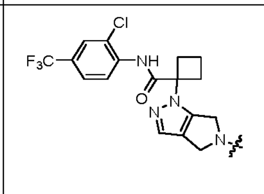 | 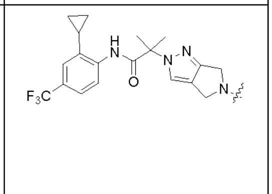 |
| 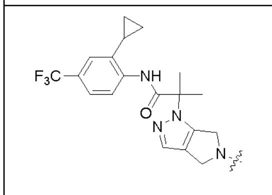 | 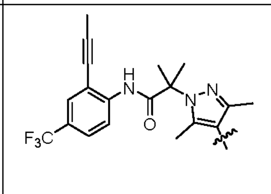 | 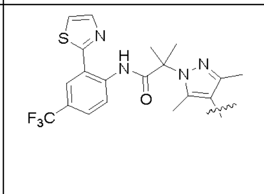 | 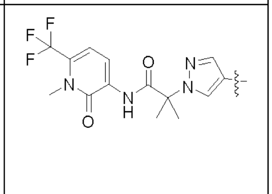 |
| 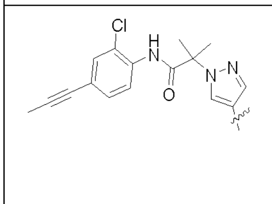 | 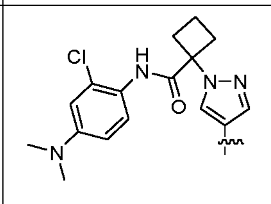 | 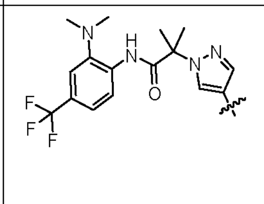 | 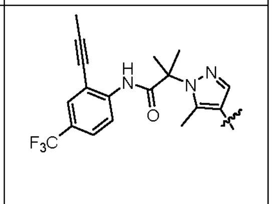 |
| 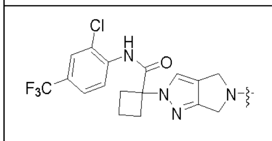 | 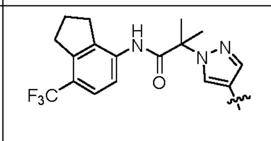 | 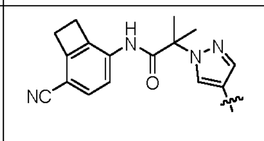 | 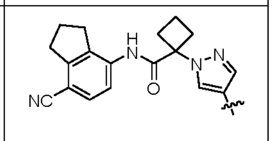 |
| 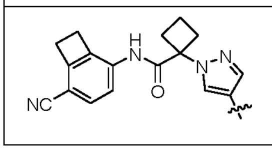 | | | |

the definition of K is the same as that in the sixth embodiment of the present invention;
the definition of L is the same as that in the tenth embodiment of the present invention.

As a twelfth embodiment of the present invention, the compound represented by the above general formula (I) or a stereoisomer, deuterated compound, solvate, prodrug, metabolite, pharmaceutically acceptable salt or co-crystal thereof is provided,

L is selected from a bond, -Ak1-, -Cy1-, -Cy1-Ak1-, -Cy1-Ak1-Ak2-, -Cyl-Ak1-Ak2-Ak3-, -Cy1-Ak1-Ak2-Ak3-Ak4-,

-Cy1-Cy2-, -Cy1-Ak1-Cy2-, -Cyl-Cy2-Ak2-, -Cy1-Ak1-Cy2-Ak2-, -Cy1-Ak1-Cy2-Ak2-Ak3-, -Cy1-Ak1-Cy2-Ak2-Ak3-Ak4-, -Cy1-Cy2-Ak2-Ak3-, -Cy1-Cy2-Ak2-Ak3-Ak4-, -Cy1-Ak1-Cy2-Ak2-Ak3-Ak4-, -Cy1-Ak1-Ak2-Cy3-, -Cy1-Ak1-Ak2-Cy3-Ak3-, -Cy1-Cy2-Cy3-, - Cy1-Ak1-Cy2-Cy3-, -Cy1-Cy2-Ak2-Cy3-, -Cy1-Cy2-Cy3-Ak3-, -Cy1-Ak1-Cy2-Cy3-Ak3-, -Cy1-Cy2-Ak2-Cy3-Ak3-, -Cy1-Ak1-Cy2-Ak2-Cy3-, -Cy1-Ak1-Cy2-Ak2-Cy3-Ak3-, -Cy1-Cy2-Cy3-Ak3-Ak4-, -Cy1-Cy2-Cy3-Ak3-Cy4-, -Cy1-Cy2-Cy3-Cy4-, -Cy1-Ak1-Cy2-Cy3-Cy4-, -Cy1-Cy2-Ak2-Cy3-Cy4-, -Cy1-Cy2-Cy3-Ak3-Cy4-, -Cy1-Cy2-Cy3-Cy4-Ak4-, -Cy1-Ak1-Cy2-Ak2-Cy3-Ak3-Cy4-, -Cyl-Ak1-Cy2-Ak2-Cy3-Cy4-, -Ak1-Cy2-, -Ak1-Cy2-Cy3-, -Ak1-Ak2-Cy3-, -Ak1-Ak2-Cy3-Cy4-, -Ak1-Cy2-Ak2-Cy3-, -Ak1-Cy2-Cy3-Ak3-Cy4-, -Ak1-Cy2-Cy3-Cy4-Ak4-Cy5-, -Ak1-Cy2-Ak2-, -Ak1-Ak2-Ak3-Ak4-, -Ak1-Ak2-Ak3-, -Ak1-Ak2-, -Ak1-Ak2-Ak3-Ak4-Ak5-, -Cy1-Cy2-Cy3-Ak3-Ak4-Ak5-, -Cy1-Cy2-Ak2-Cy3-Ak3-Ak4-Ak5-, -Cy1-Ak1-Cy2-Ak2-Ak3-Ak4-Ak5-, -Cy1-Cy2-Cy3-Cy4-Ak4-Ak5-, -Cy1-Ak1-Ak2-Ak3-Ak4-Ak5-, -Ak1-Cy2-Ak2-Ak3-Ak4-Ak5-, -Ak1-Cy2-Ak2-Ak3-Ak4-, or -Ak1-Cy2-Ak2-Ak3-;

Ak1, Ak2, Ak3, Ak4, and Ak5 are each independently selected from -O-, - $OCH_2$-, -$CH_2O$-, -$OCH_2CH_2$-, -$CH_2CH_2O$-, -CH=CH-, -CH=C(CN)-, -CH=C(F)-, - C(CN)=CH-, -C(F)=CH-, -C≡C-, -$C(CH_3)_2$-, -$CH_2$-, -$CH_2CH_2$-, -$CH_2CH_2CH_2$-, - $N(CH_3)$-, -NH-, -$CH_2N(CH_3)$-, -$CH_2NH$-, -$NHCH_2$-, -$CH_2CH_2N(CH_3)$-, - $CH_2CH_2NH$-, -$NHCH_2CH_2$-, -C(=O)-, -C(=O)$CH_2$NH-, -$CH_2$C(=O)NH-, - C(=O)NH- or -NHC(=O)-;

the definitions of other groups are the same as those in any one of the second, third, fourth, fifth, sixth, seventh, eighth, ninth, tenth and eleventh embodiments of the present invention.

As a thirteenth embodiment of the present invention, the compound represented by the above general formula (I) or a stereoisomer, deuterated compound, solvate, prodrug, metabolite, pharmaceutically acceptable salt or co-crystal thereof is provided,

L is selected from a bond or a group shown in Table B-1 or Table B-2, where the left side of the group is connected to B; the definitions of other groups are the same as those in any one of the second, third, fourth, fifth, sixth, seventh, eighth, ninth, tenth and eleventh embodiments of the present invention.

As a fourteenth embodiment of the present invention, the compound represented by the above general formula (I) or a stereoisomer, deuterated compound, solvate, prodrug, metabolite, pharmaceutically acceptable salt or co-crystal thereof is provided, the compound of general formula (I) is selected from the compound represented by general formula (Ia),

L is selected from a bond, -Ak1-Cy2-, -Ak1-Cy2-Cy3-, -Cy1-Ak1-, -Ak1-Cy2-Ak2-, -Cy1-Cy2-Cy3-, -Cy2-Cy3-, -Cy1-Ak1-Cy2-, -Cy1-Ak1-Cy2-Cy3-, - Cy1-Cy2-Ak2-Cy3-, -Ak1-, -Cy1-, -Ak1-Cy2-Ak2-Ak3-, -Ak1-Cy2-Ak2-Cy3-, - Ak1-Cy2-Ak2-Ak3-Ak4-, or -NHCO-$(CH2)_{s1}$;

$_{s1}$ is selected from 0, 1, 2, 3, 4, 5, 6 or 7;

Ak1, Ak2, Ak3, and Ak4 are each independently selected from -C(=O)-, -O-, NH, -CH=CH-, -CH=C(CN)-, -CH=C(F)-, -C(CN)=CH-, -C(F)=CH-, -C≡C-, - $C(CH_3)_2$-, -$CH_2$-, -$CH_2CH_2$-, -$CH_2CH_2CH_2$-, or -NHCO-;

each Cy1, Cy2, or Cy3 is independently selected from one of the following substituted or unsubstituted groups:

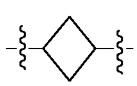 , 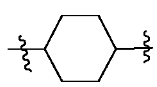 , 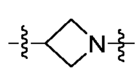 ,

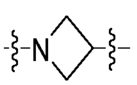 , 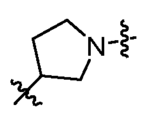 , 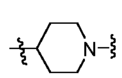 , 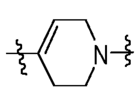 , 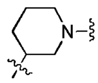 , 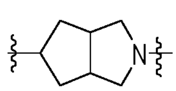 ,

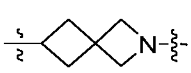 , 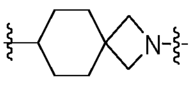 , 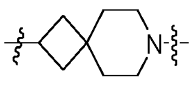 , 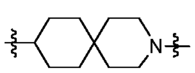 , 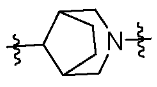 ,

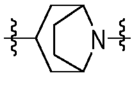 , 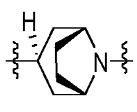 , 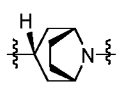 , 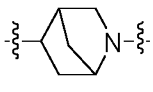 , 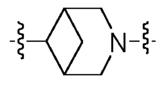 , 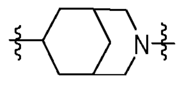 ,

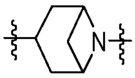, 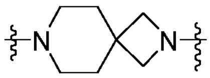, 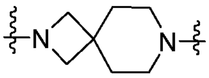, 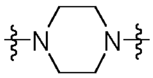, 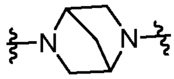,

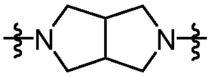, 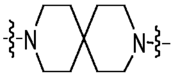, 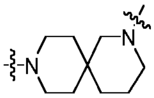, 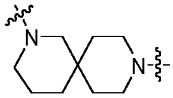, 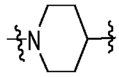, or 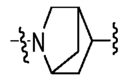,

and when substituted, is optionally substituted with 0 to 4 substituents selected from H, F, $CF_3$, OH, methyl, methoxy, =O, hydroxymethyl, COOH, CN or $NH_2$;

preferably, L is selected from a bond or one of the structural fragments in **Table B-2,** where the left side is connected to B;

the definitions of other groups are the same as those in any one of the second, third, fourth, fifth, sixth, seventh, eighth, ninth, tenth and eleventh embodiments of the present invention.

As a fifteenth embodiment of the present invention, the compound represented by the above general formula (I) or a stereoisomer, deuterated compound, solvate, prodrug, metabolite, pharmaceutically acceptable salt or co-crystal thereof is provided, the compound of general formula (I) is selected from the compound represented by general formula (Ia),

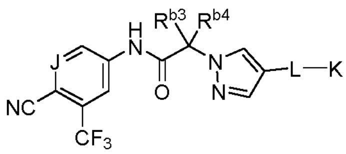

(Ia)

J is selected from N or CH, preferably J is N;

L is selected from -Ak1-Cy2-, -Ak1-Cy2-Cy3-, -Ak1-Cy2-Ak2-, -Cy2-Cy3-, - Ak1- or a bond;

$R^{b3}$ and $R^{b4}$ are each independently selected from methyl or ethyl;

alternatively, $R^{b3}$ and $R^{b4}$ together with the carbon atom to which they are attached form cyclopropyl, cyclobutyl, cyclopentyl, cyclohexyl, azetidinyl, azacyclopentyl, azacyclohexyl, oxetanyl, oxacyclopentyl, or oxacyclohexyl, the cyclopropyl, cyclobutyl, cyclopentyl, cyclohexyl, azetidinyl, azacyclopentyl, azacyclohexyl, oxetanyl, oxacyclopentyl, or oxacyclohexyl is optionally substituted with 0 to 4 substituents selected from H, F, Cl, Br, I, OH, $NH_2$, $C_{1-4}$ alkyl or $C_{1-4}$ alkoxy;

Ak1 and Ak2 are each independently selected from -CH=CH-, -CH=C(CN)-, -CH=C(F)-, -C(CN)=CH-, -C(F)=CH-, -C≡C-, $-C(CH_3)_2-$, $-CH_2-$, $-CH_2CH_2-$, or - $CH_2CH_2CH_2-$;

each Cy1, Cy2, or Cy3 is independently selected from one of the following substituted or unsubstituted groups:

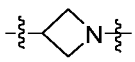, 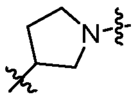, 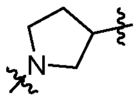, 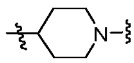,

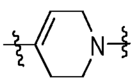, 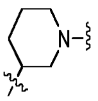, 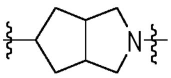, 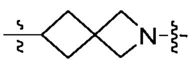, 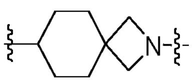,

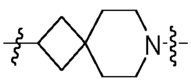, 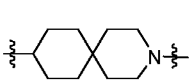, 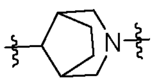, 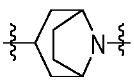, 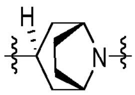, 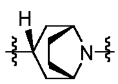,

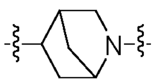, 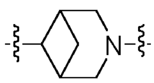, 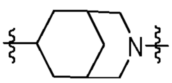, or 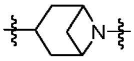,

and when substituted, is optionally substituted with 0 to 4 substituents selected from H, F, $CF_3$, OH, methyl, methoxy, =O, hydroxymethyl, COOH, CN or $NH_2$;
the definitions of other groups are the same as those in any one of the second, third, fourth, fifth, sixth and twelfth embodiments of the present invention.

As a sixteenth embodiment of the present invention, the compound represented by the above general formula (I) or general formula (Ia) or a stereoisomer, deuterated compound, solvate, prodrug, metabolite, pharmaceutically acceptable salt or co-crystal thereof is provided,

K is selected from one of the following structural fragments (Table K-2):

| | | | |
|---|---|---|---|
| 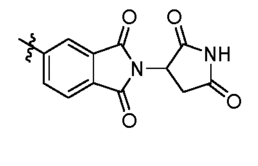 | 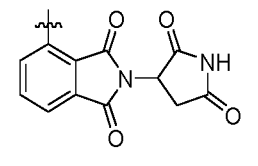 | 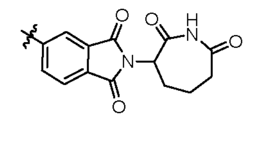 | 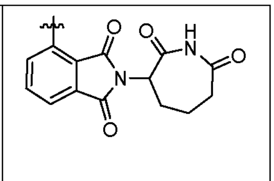 |
| 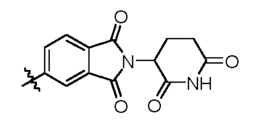 | 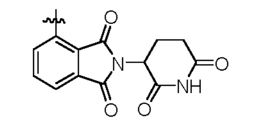 | 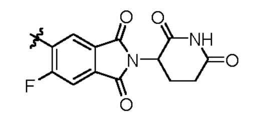 | 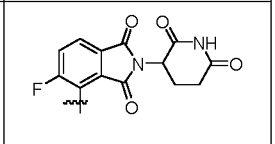 |
| 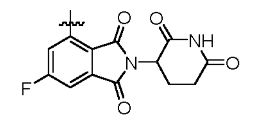 | 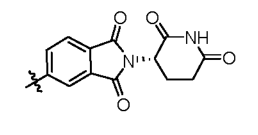 | 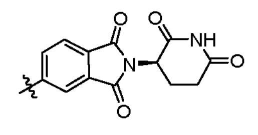 | 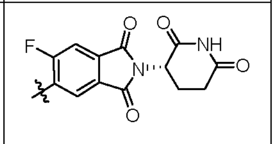 |
| 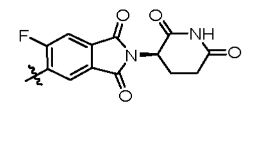 | 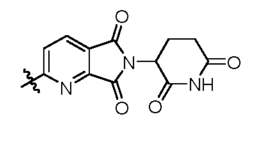 | 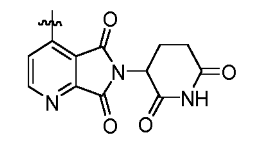 | 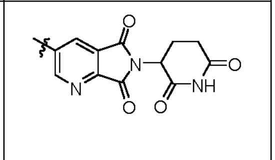 |
| 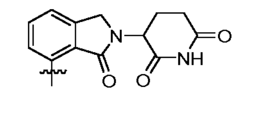 | 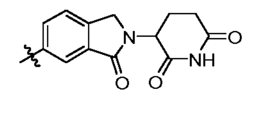 | 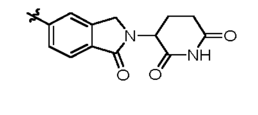 | 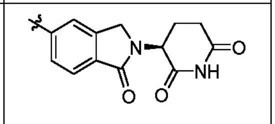 |
| 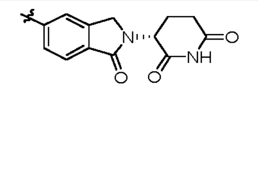 | 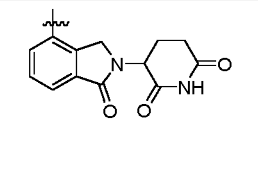 | 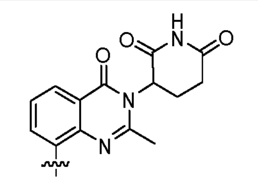 | 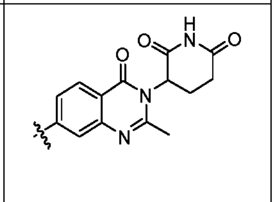 |
| 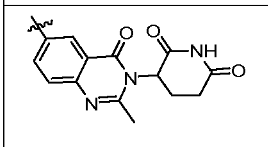 | 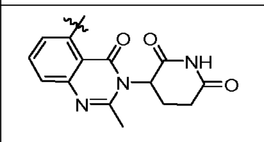 | 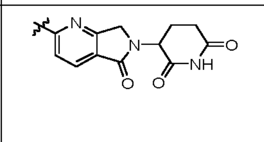 | 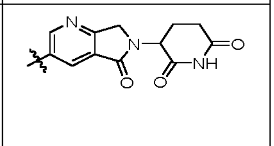 |
| 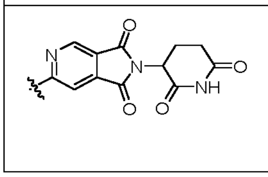 | 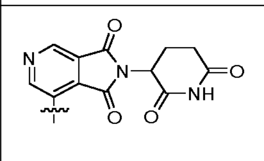 | 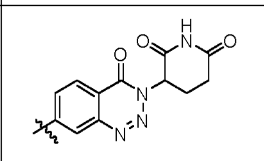 | 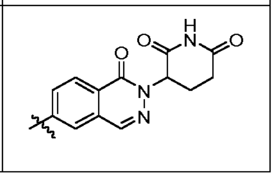 |

| | | | |
|---|---|---|---|
| 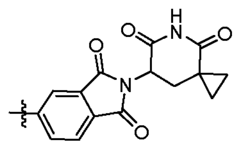 | 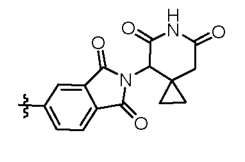 | 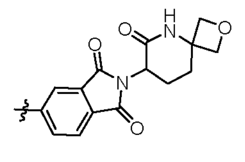 | 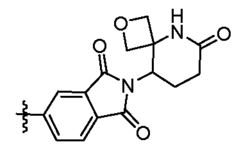 |
| 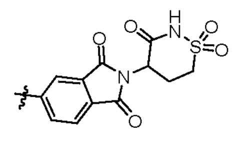 | 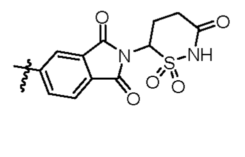 | 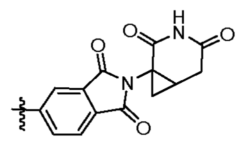 | 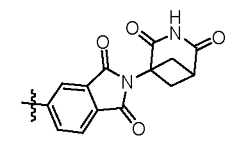 |
| 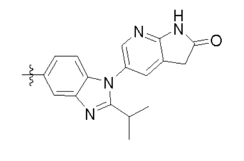 | 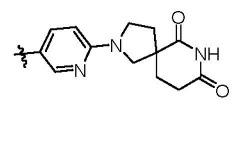 | 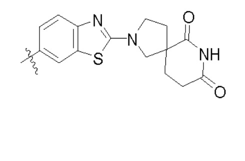 | 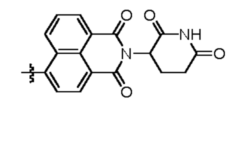 |
| 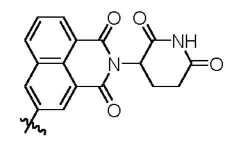 | 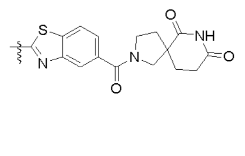 | | |
| 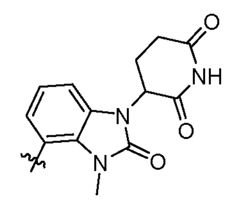 | 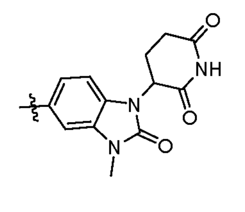 | 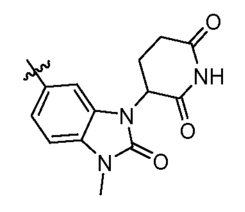 | 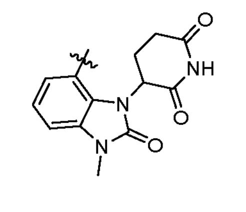 |
| 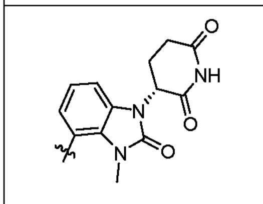 | 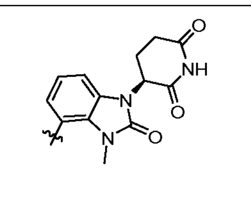 | 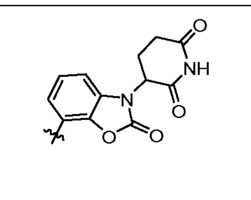 | 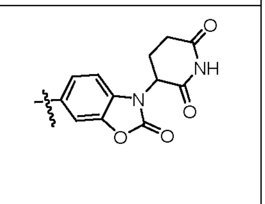 |
| 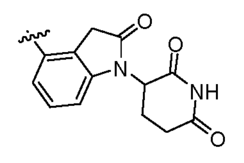 | 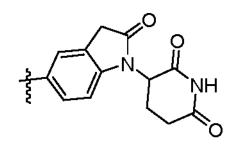 | 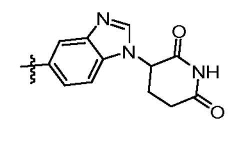 | 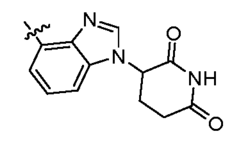 |
| 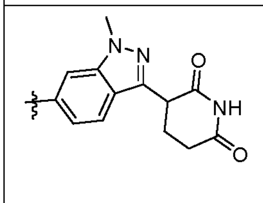 | 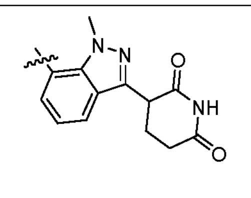 | 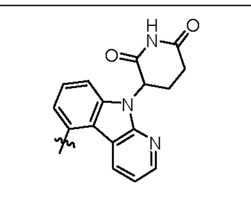 | 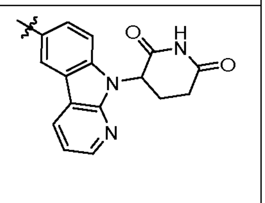 |
| 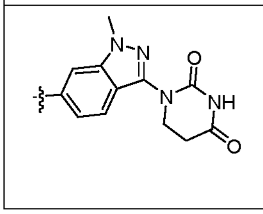 | 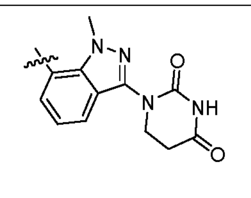 | 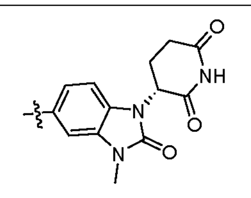 | 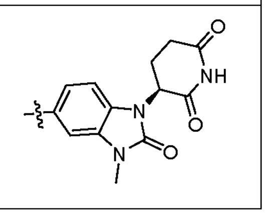 |

| | | | |
|---|---|---|---|
| 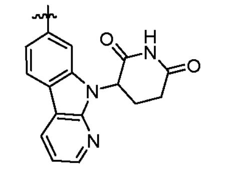 | 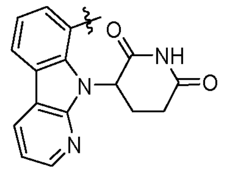 | 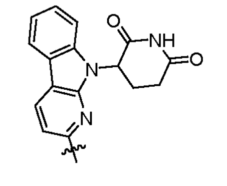 | 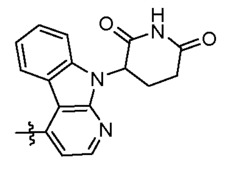 |
| 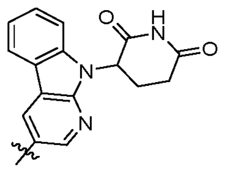 | 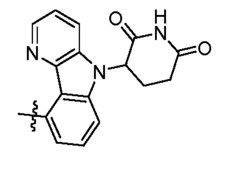 | 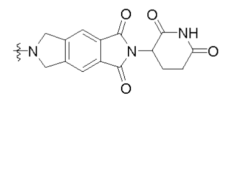 | 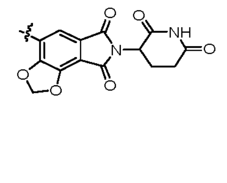 |
| 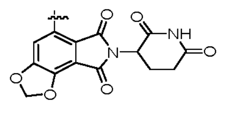 | 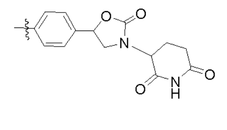 | 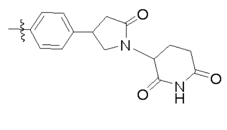 | 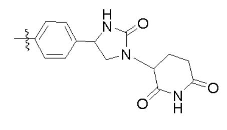 |
| 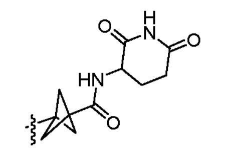 | 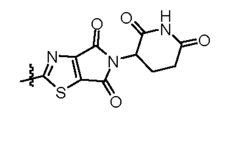 | 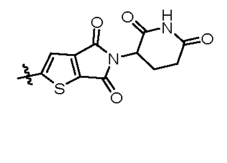 | 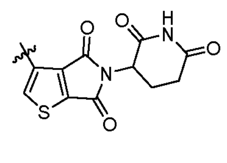 |
| 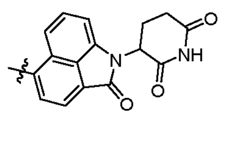 | 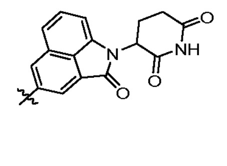 | 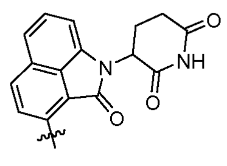 | 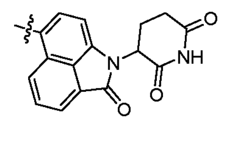 |
| 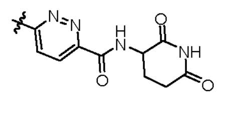 | 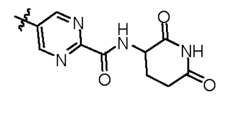 | 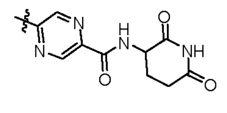 | 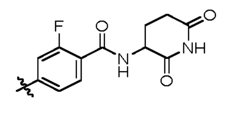 |
| 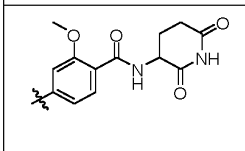 | 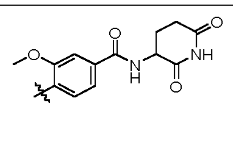 | 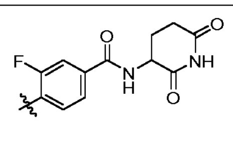 | 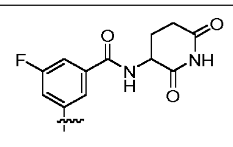 |
| 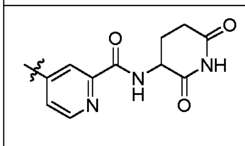 | 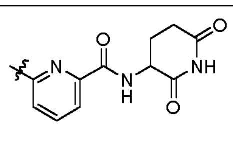 | 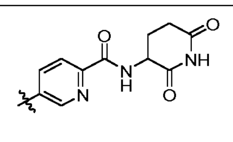 | 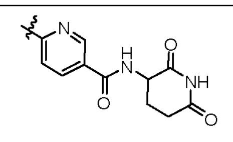 |
| 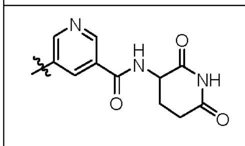 | 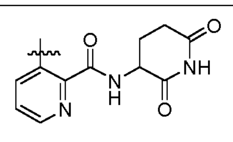 | 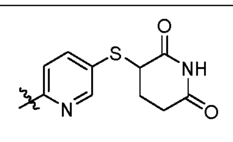 | 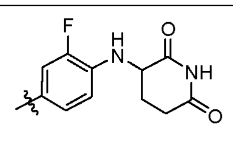 |
| 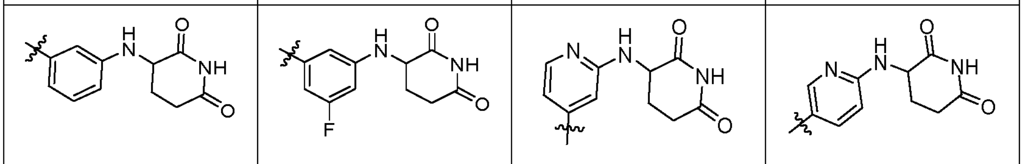 | | | |
| 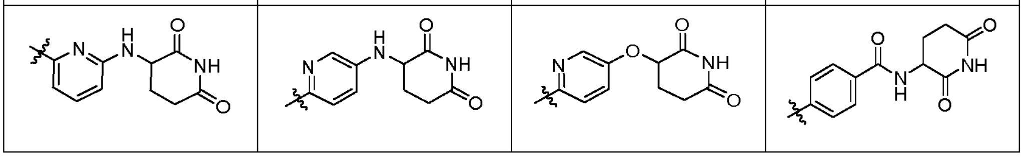 | | | |

| | | | |
|---|---|---|---|
| 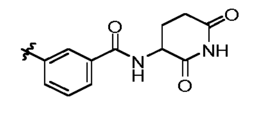 | 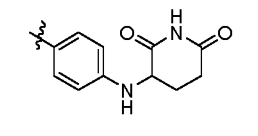 | 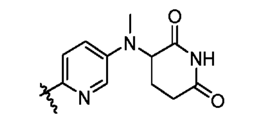 | 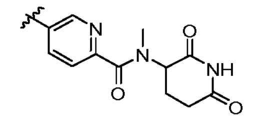 |
| 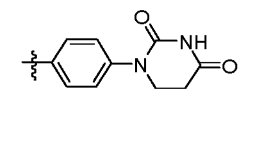 | 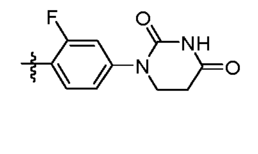 | 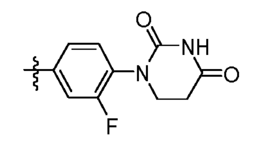 | 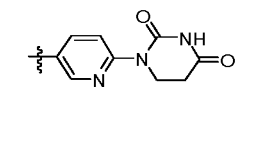 |
| 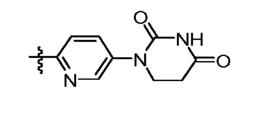 | 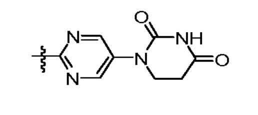 | 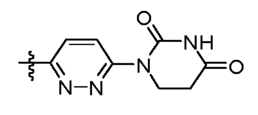 | 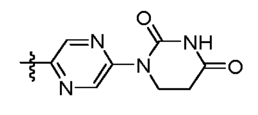 |
| 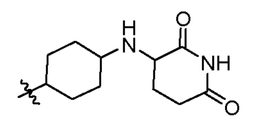 | 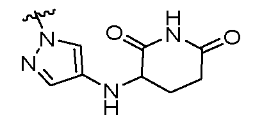 | 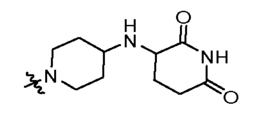 | 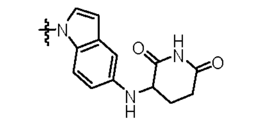 |
| 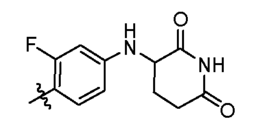 | 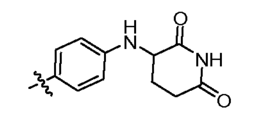 | 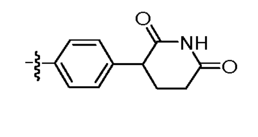 | 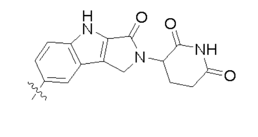 |
| 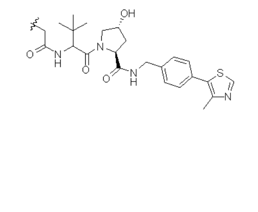 | 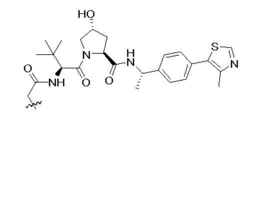 | 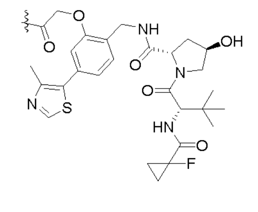 | 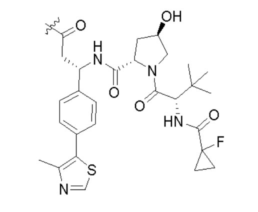 |
| 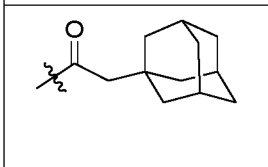 | 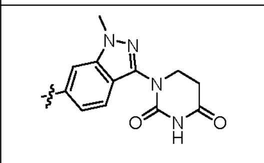 | 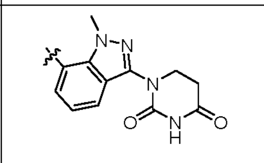 | 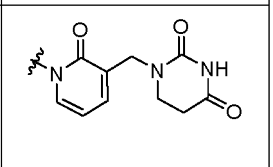 |
| 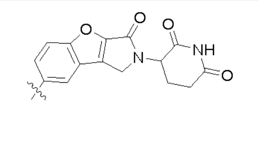 | 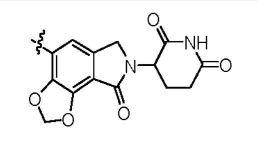 | 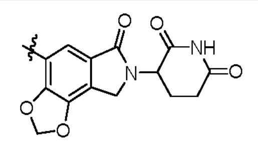 | 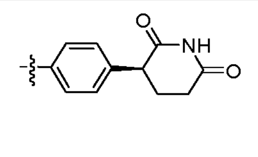 |
| 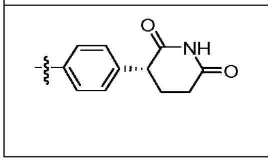 | 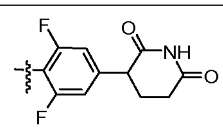 ; | | |

the definitions of other groups are the same as those in any one of the second, third, fourth, fifth, sixth, seventh, eighth, ninth, tenth, eleventh, twelfth, thirteenth, fourteenth or fifteenth embodiments of the present invention.

As a seventeenth embodiment of the present invention, the compound represented by the following general formula (Ib) or a stereoisomer, deuterated compound, solvate, prodrug, metabolite, pharmaceutically acceptable salt or co-crystal thereof is provided,

$$B-\equiv-Cy_1-K \quad (Ib)$$

The definitions of B, K, and Cy1 are the same as those in any one of the first, second, third, fourth, fifth, sixth,

seventh, eighth, ninth, tenth, eleventh, twelfth, thirteenth, fourteenth, fifteenth or sixteenth embodiments of the present invention.

As an eighteenth embodiment of the present invention, the compound shown in any of the aforementioned embodiments or a stereoisomer, deuterated compound, solvate, prodrug, metabolite, pharmaceutically acceptable salt or co-crystal thereof is provided,

K is selected from one of the structural fragments shown in Table K-3,

Table K-3

| | | | |
|---|---|---|---|
| 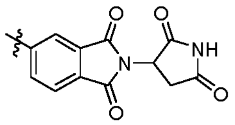 | 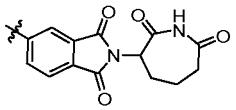 | | |
| 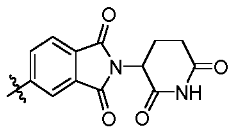 | 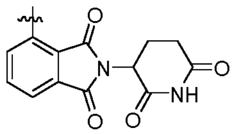 | 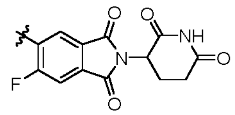 | 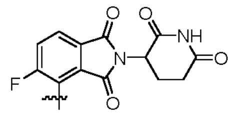 |
| 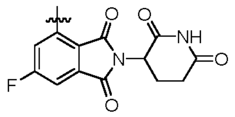 | 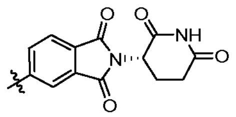 | 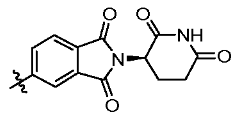 | 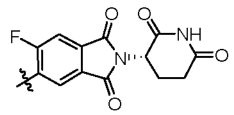 |
| 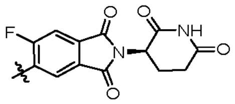 | 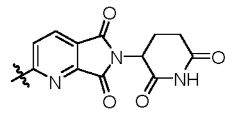 | 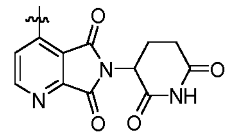 | 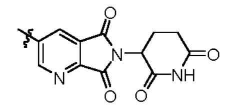 |
| 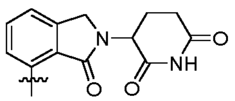 | 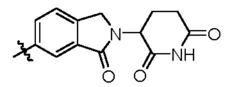 | 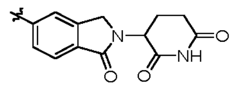 | 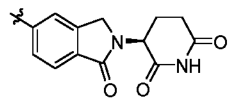 |
| 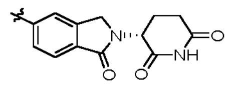 | 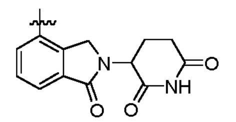 | 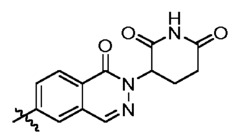 | 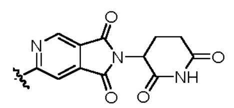 |
| 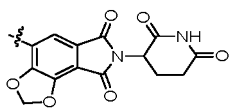 | 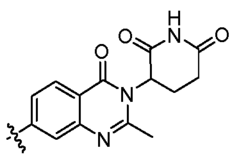 | 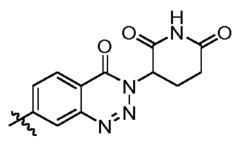 | |
| 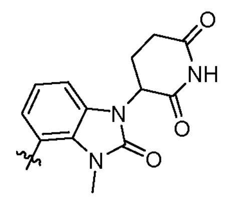 | 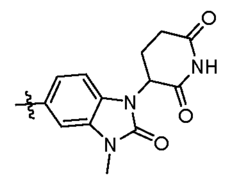 | 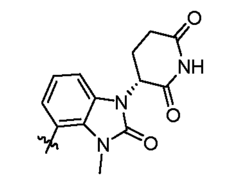 | 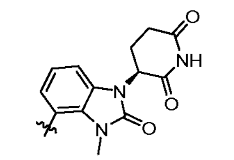 |

| | | | |
|---|---|---|---|
| 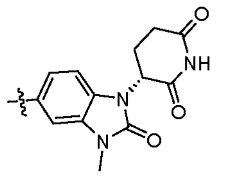 | 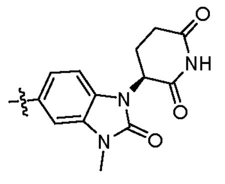 | 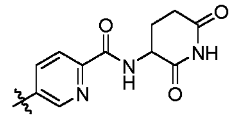 | 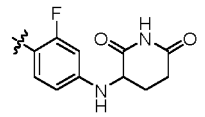 |
| 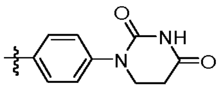 | 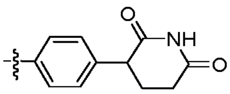 | 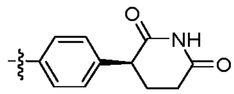 | 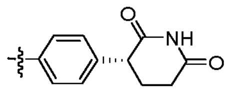 |

the definitions of other groups are the same as those in any one of the first, second, third, fourth, fifth, sixth, seventh, eighth, ninth, tenth, eleventh, twelfth, thirteenth, fourteenth, fifteenth, sixteenth or seventeenth embodiments of the present invention.

As a nineteenth embodiment of the present invention, the compound represented by the following general formula (Id) or a stereoisomer, deuterated compound, solvate, prodrug, metabolite, pharmaceutically acceptable salt or co-crystal thereof is provided,

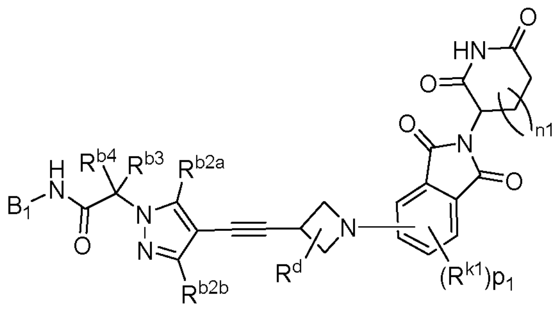

(Id)

$R^{b3}$ and $R^{b4}$ are each independently selected from H, methyl, ethyl, or isopropyl;

alternatively, $R^{b3}$ and $R^{b4}$ together with the carbon atom to which they are attached form cyclopropyl, cyclobutyl, cyclopentyl, or cyclohexyl, the cyclopropyl, cyclobutyl, cyclopentyl, or cyclohexyl is optionally substituted with 1 to 4 substituents selected from H, F, Cl, Br, I, OH, $NH_2$, $C_{1-4}$ alkyl or $C_{1-4}$ alkoxy;

$R^{b2a}$ and $R^{b2b}$ are each independently selected from H, F, Cl, Br, I, OH, $NH_2$, CN, $CF_3$, $C_{1-4}$ alkyl, $C_{1-4}$ alkoxy, or $C_{3-6}$ cycloalkyl, the alkyl, alkoxy or cycloalkyl is optionally substituted with 1 to 4 substituents selected from H, F, Cl, Br, I or OH;

$R^d$ is selected from H, F, Cl, Br, I, OH, COOH, CN, $NH_2$, $C_{1-4}$ alkyl, halogen-substituted $C_{1-4}$ alkyl, hydroxyl-substituted $C_{1-4}$ alkyl or $C_{1-4}$ alkoxy;

the compound of general formula (Id) is not a compound shown in Table A-1;

the definitions of other groups are the same as those in any one of the first, second, third, fourth, fifth, sixth, seventh, eighth, ninth, tenth, eleventh, twelfth, thirteenth, fourteenth, fifteenth, sixteenth, seventeenth or eighteen embodiments of the present invention.

The present invention relates to a compound as described below or a stereoisomer, deuterated compound, solvate, prodrug, metabolite, pharmaceutically acceptable salt or co-crystal thereof, wherein the compound is selected from one of the structures in **Table E-1.**

The present invention relates to a compound as described below or a stereoisomer, deuterated compound, solvate, prodrug, metabolite, pharmaceutically acceptable salt or co-crystal thereof, wherein the compound is selected from a compound shown in **Table E-2.**

The present invention relates to a compound as described below or a stereoisomer, deuterated compound, solvate, prodrug, metabolite, pharmaceutically acceptable salt or co-crystal thereof, wherein the compound is selected from a compound of example 1 to example 207.

Table E-1

| | |
|---|---|
| 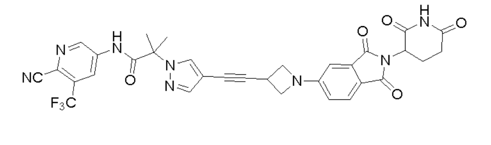 | 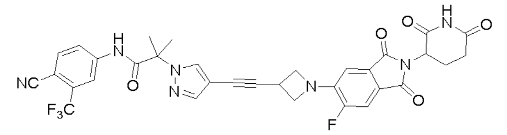 |
| 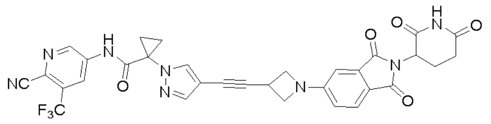 | 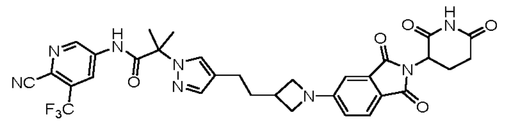 |
| 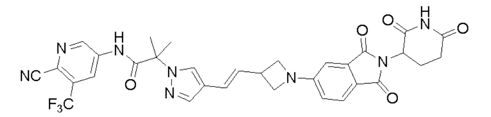 | 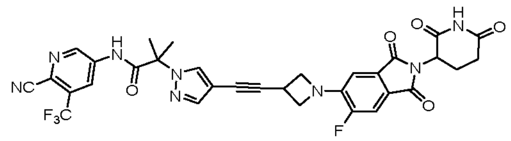 |
| 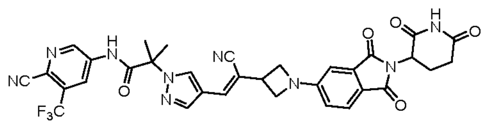 | 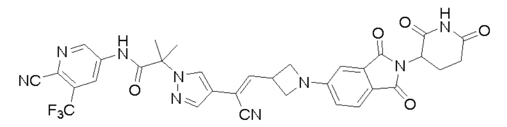 |
| 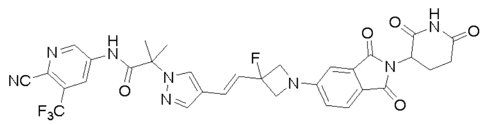 | 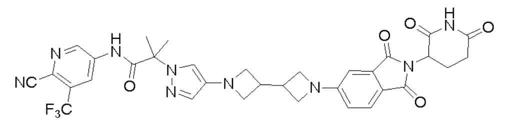 |

| | |
|---|---|
| 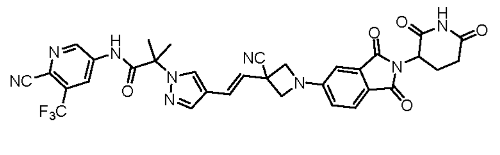 | 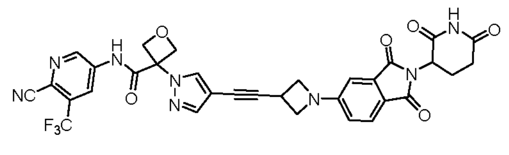 |
| 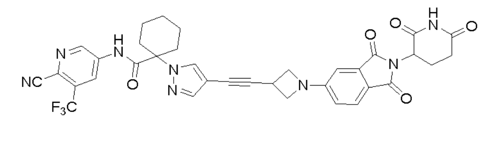 | 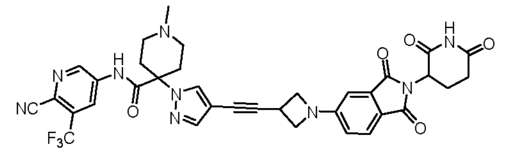 |
| 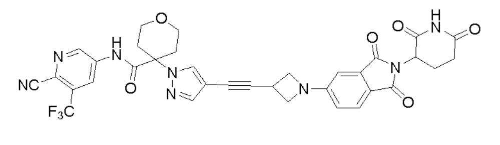 | 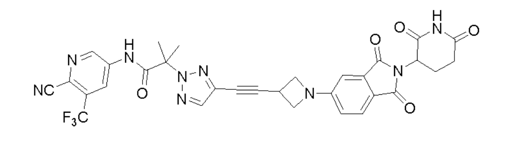 |
| 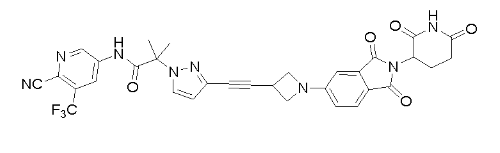 | 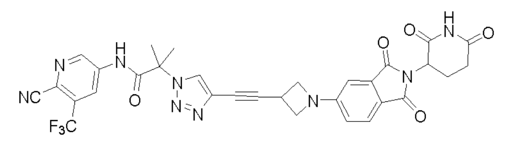 |
| 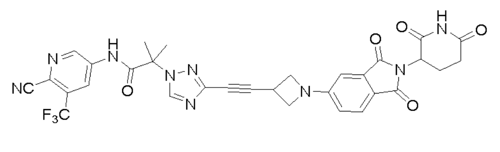 | 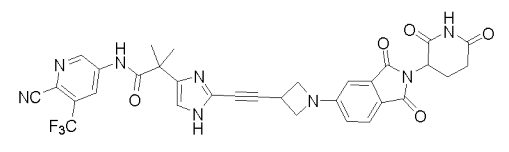 |
| 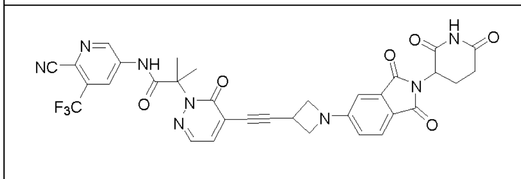 | 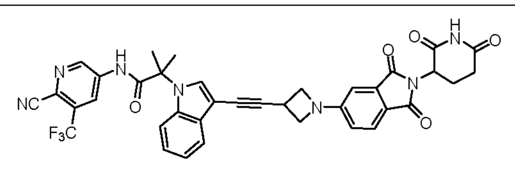 |
| 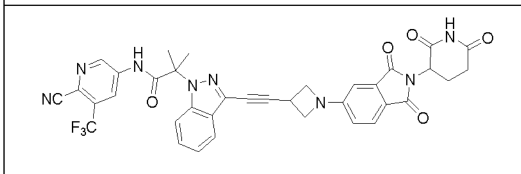 | 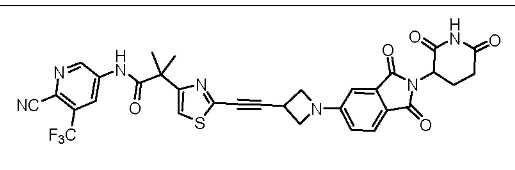 |
| 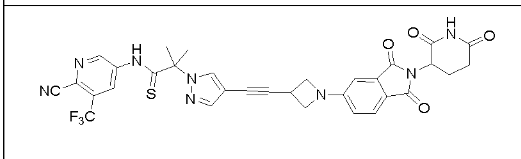 | 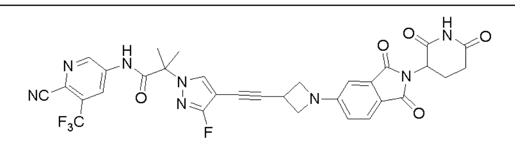 |
| 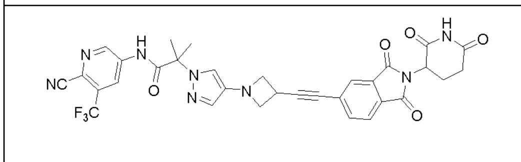 | 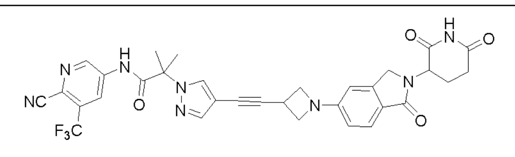 |
| 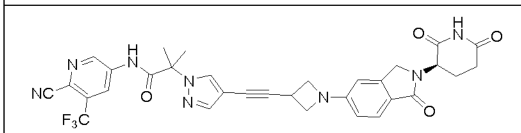 | 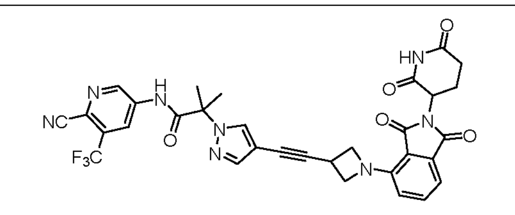 |

| | |
|---|---|
| 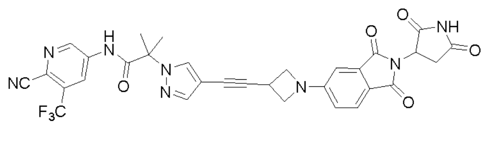 | 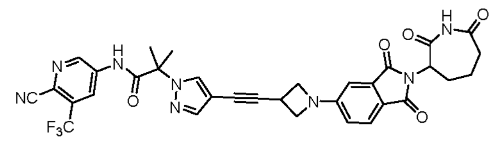 |
| 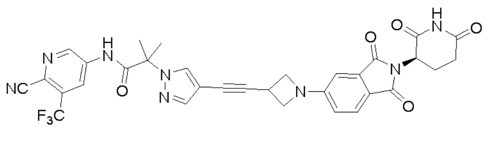 | 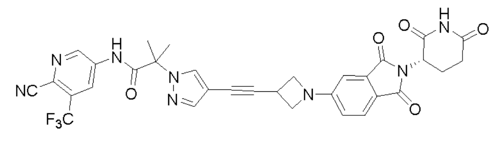 |
| 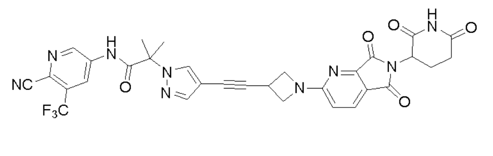 | 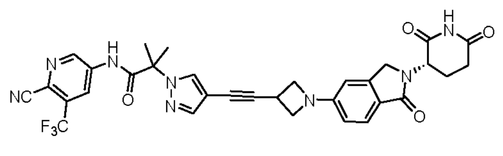 |
| 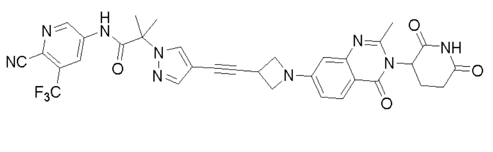 | 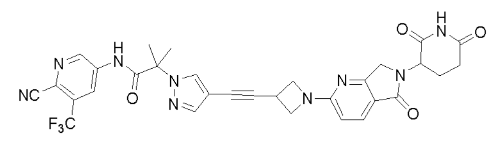 |
| 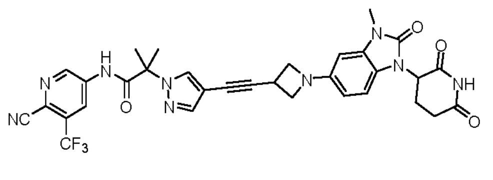 | 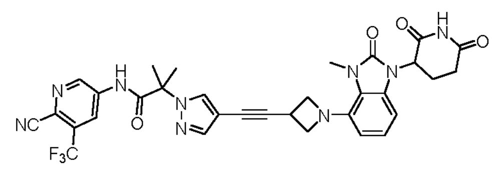 |
| 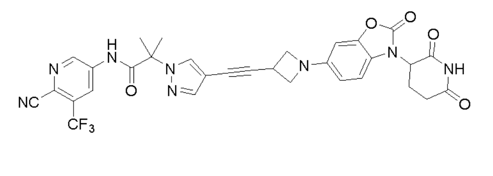 | 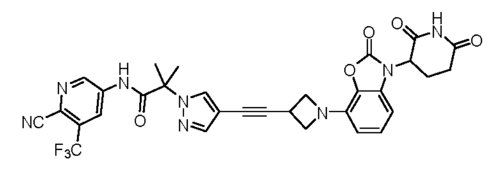 |
| 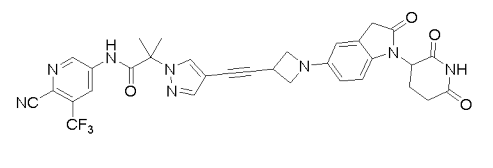 | 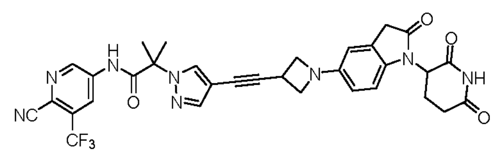 |
| 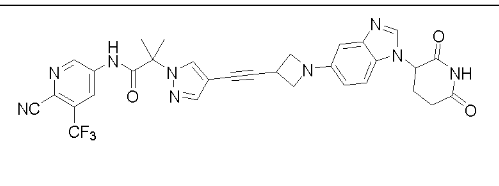 | 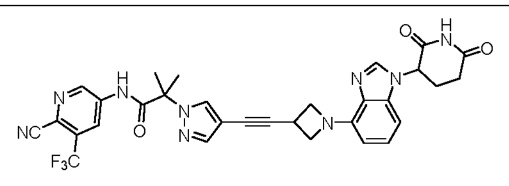 |
| 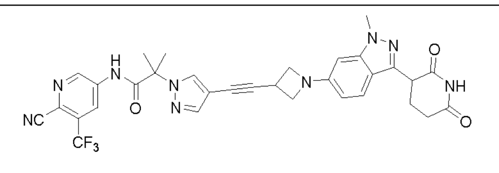 | 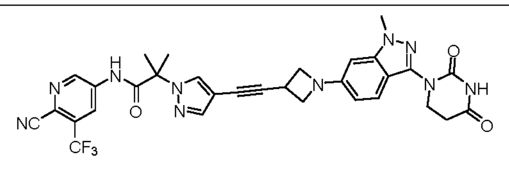 |
| 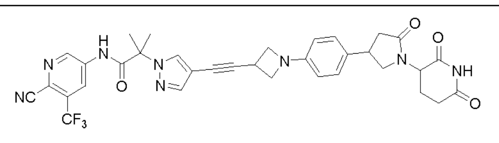 | 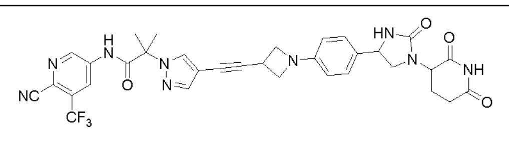 |

| | |
|---|---|
| 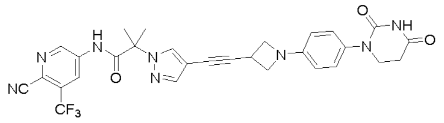 | 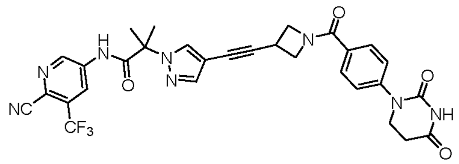 |
| 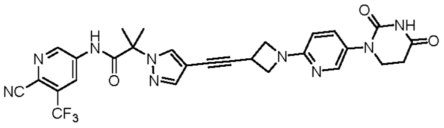 | 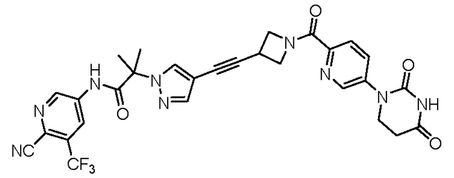 |
| 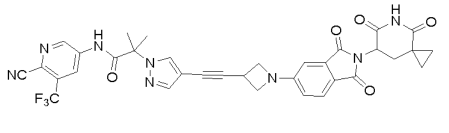 | 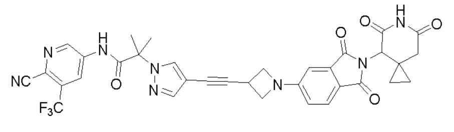 |
| 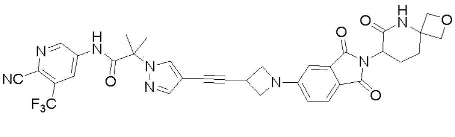 | 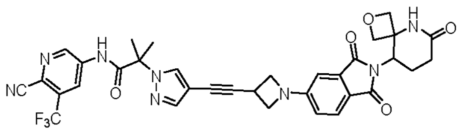 |
| 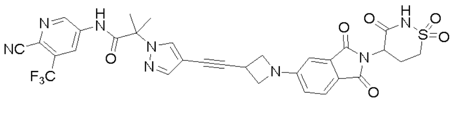 | 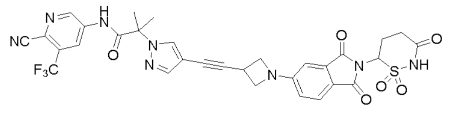 |
| 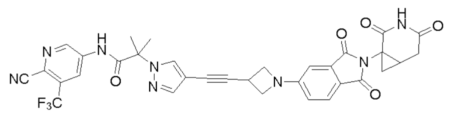 | 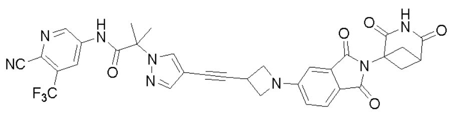 |
| 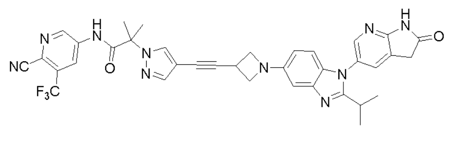 | 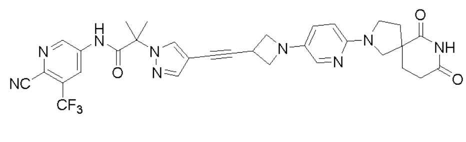 |
| 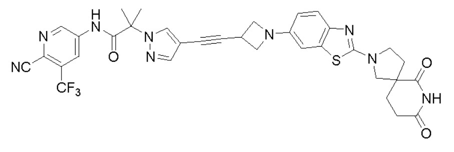 | 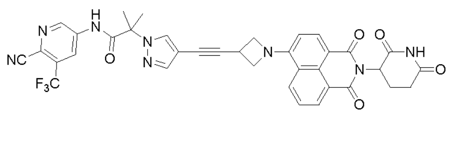 |
| 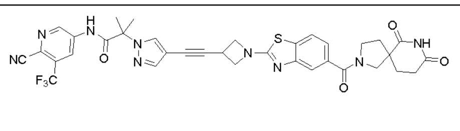 | 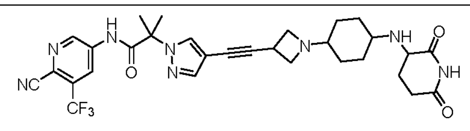 |
| 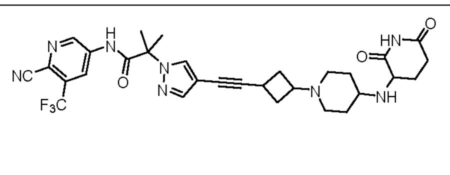 | 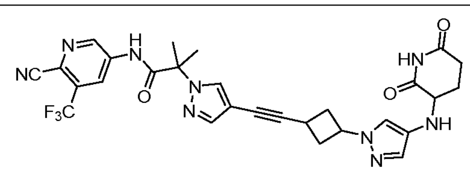 |
| 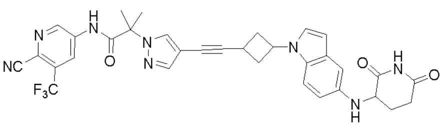 | |

| | |
|---|---|
| 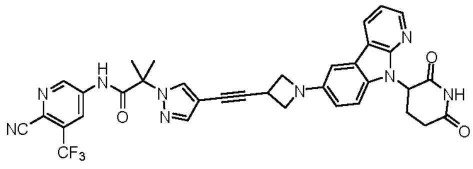 | 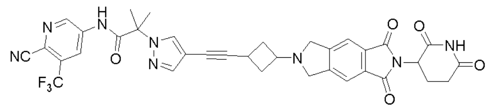 |
| 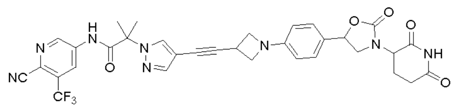 | 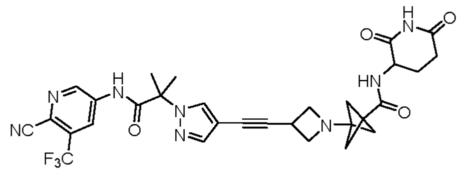 |
| 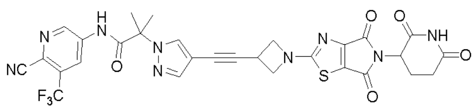 | 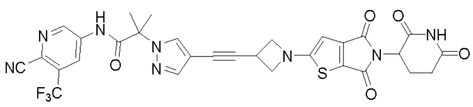 |
| 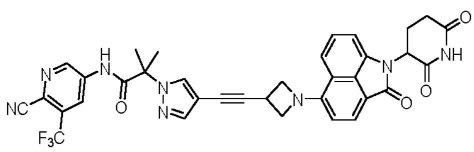 | 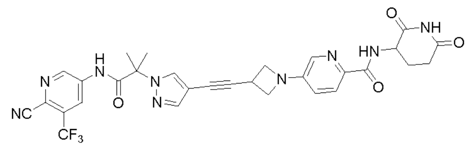 |
| 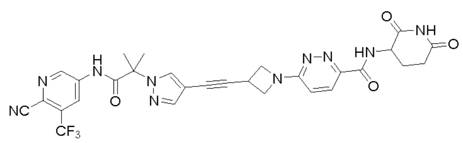 | 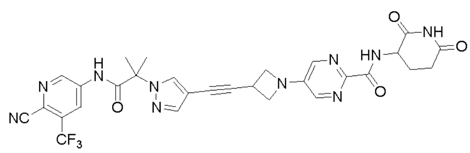 |
| 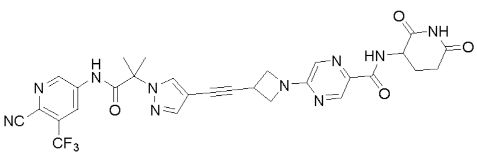 | 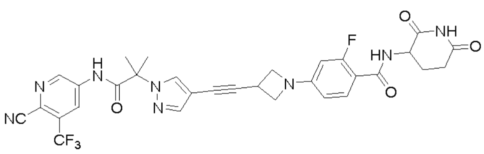 |
| 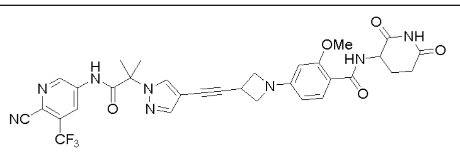 | 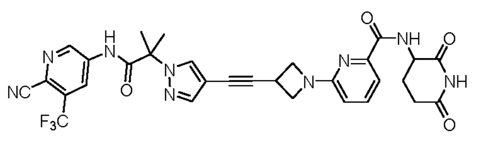 |
| 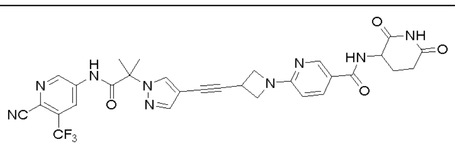 | 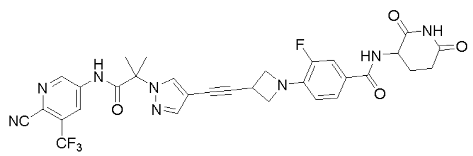 |
| 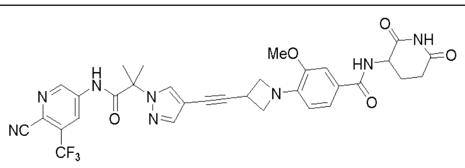 | 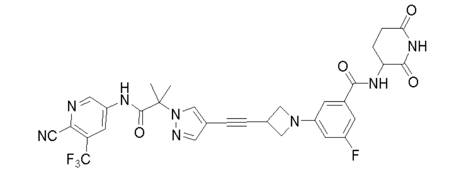 |
| 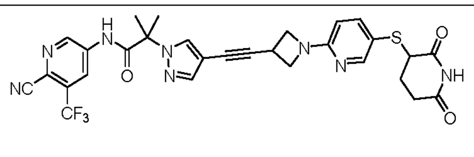 | 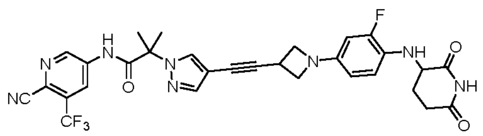 |

| | |
|---|---|
| 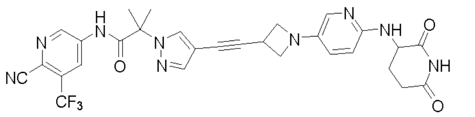 | 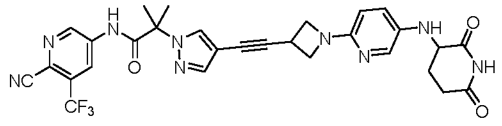 |
| 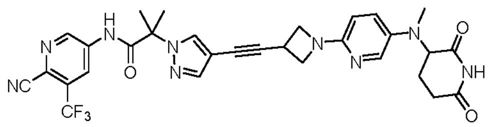 | 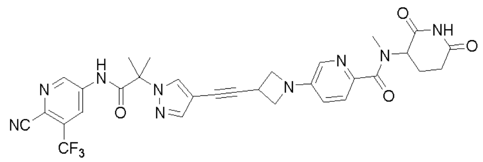 |
| 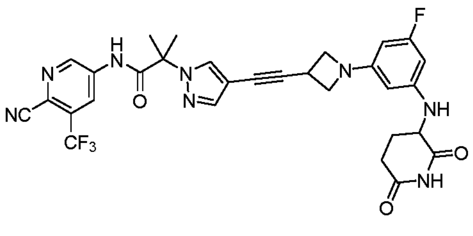 | 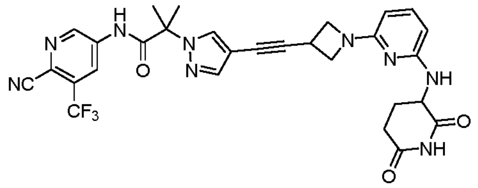 |
| 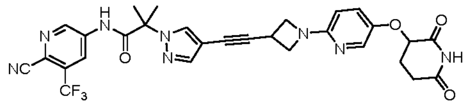 | 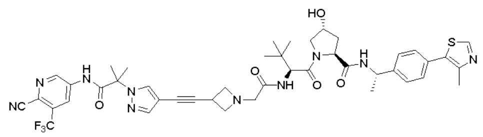 |
| 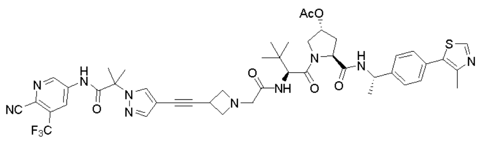 | 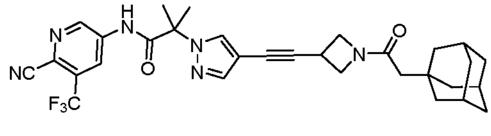 |
| 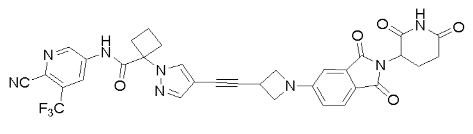 | 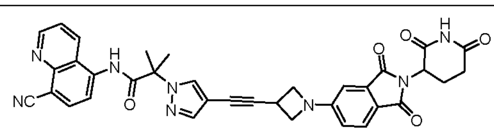 |
| 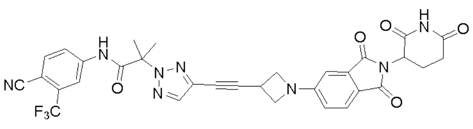 | |
| 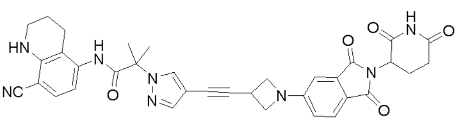 | 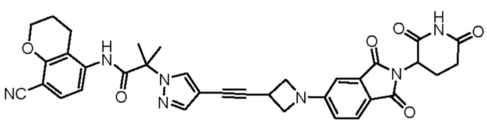 |
| 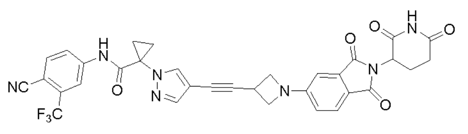 | 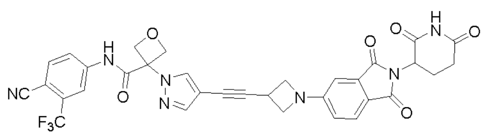 |
| 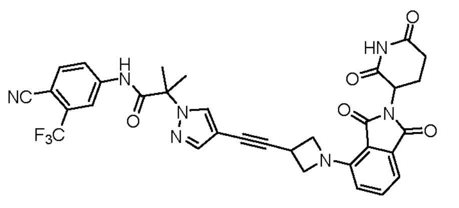 | 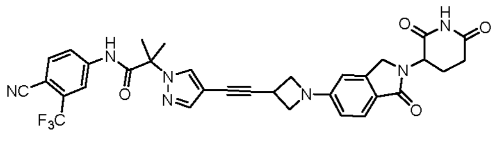 |

| | |
|---|---|
| 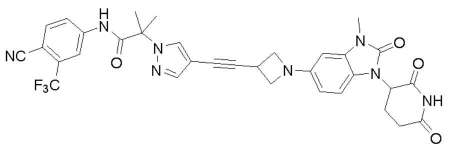 | 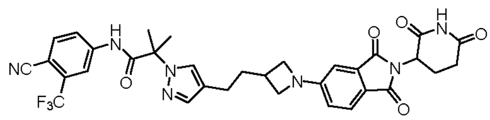 |
| 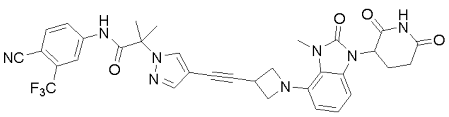 | 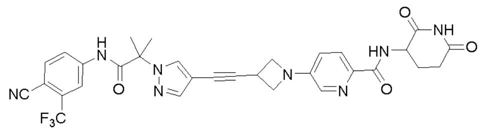 |
| 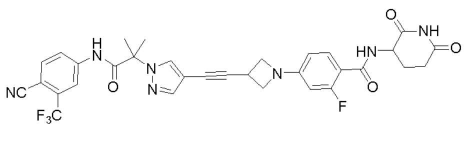 | 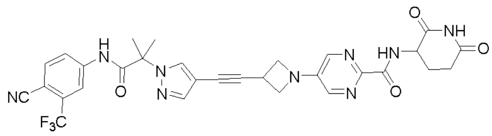 |
| 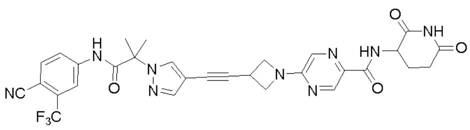 | 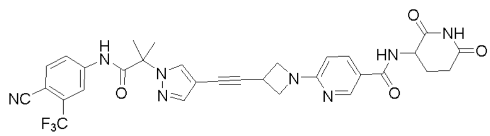 |
| 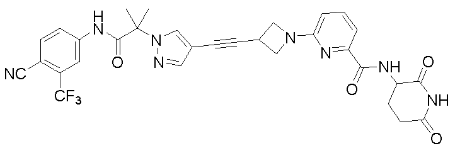 | 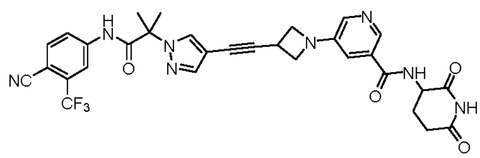 |
| 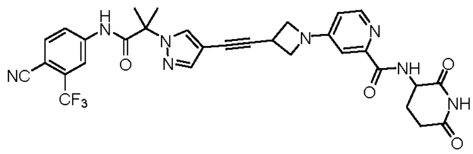 | 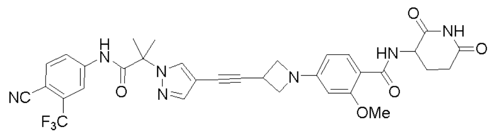 |
| 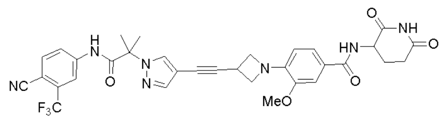 | 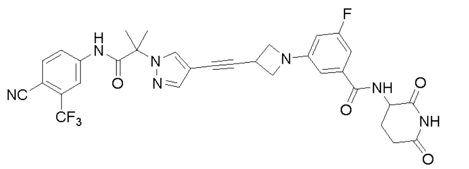 |
| 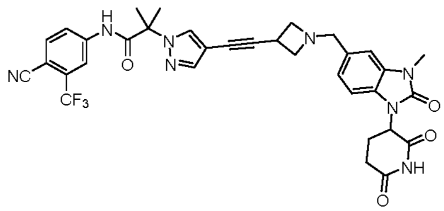 | 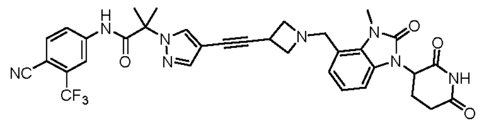 |
| 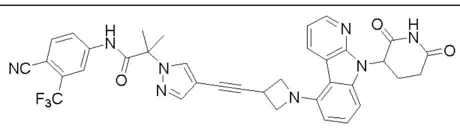 | 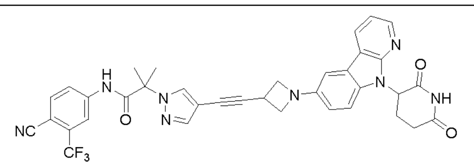 |
| 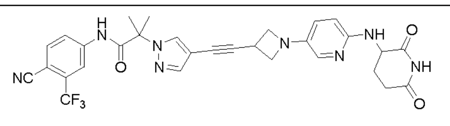 | 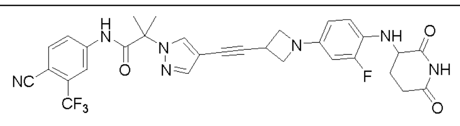 |

| | |
|---|---|
| 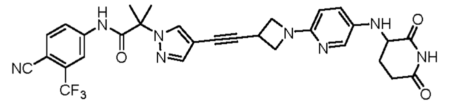 | 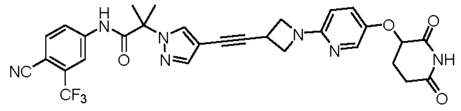 |
| 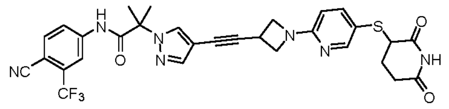 | 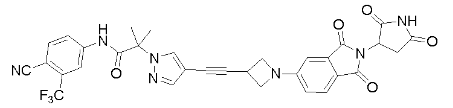 |
| 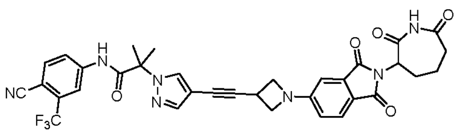 | 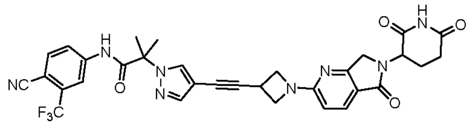 |
| 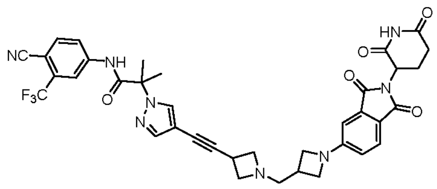 | 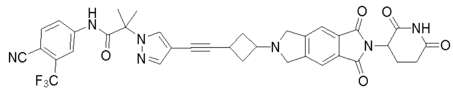 |
| 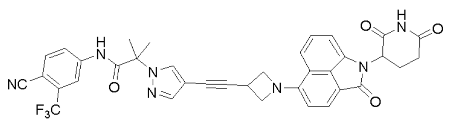 | 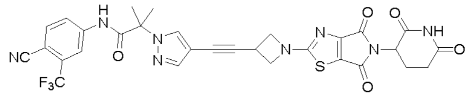 |
| 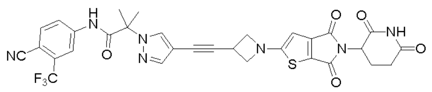 | |
| 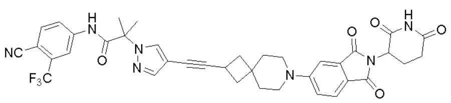 | 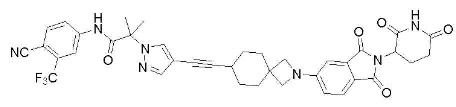 |
| 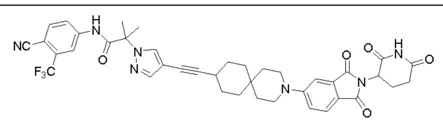 | 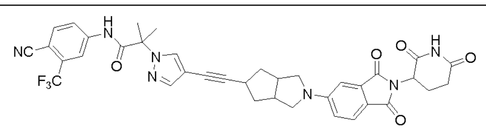 |
| 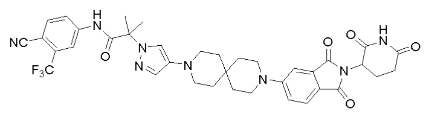 | 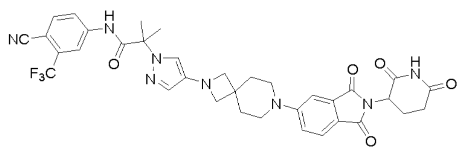 |
| 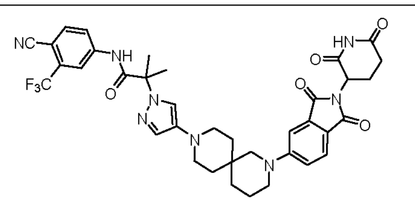 | 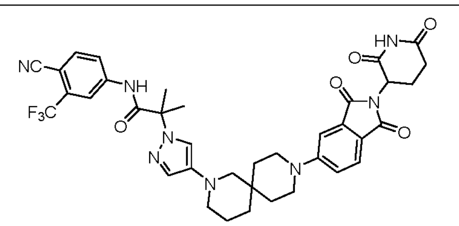 |
| 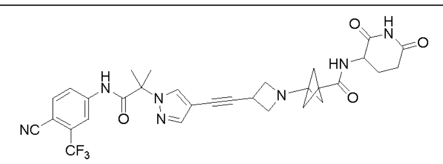 | 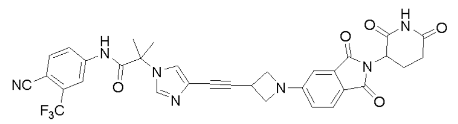 |

| | |
|---|---|
| 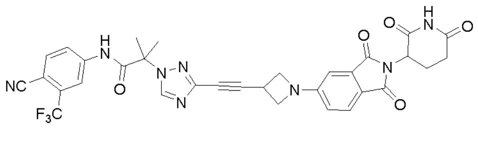 | 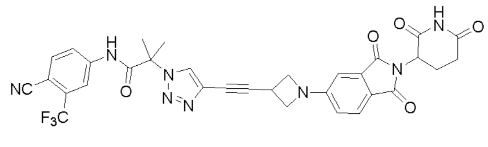 |
| 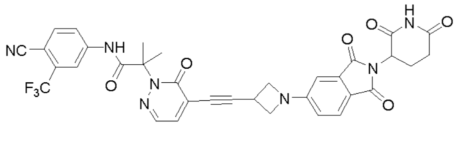 | 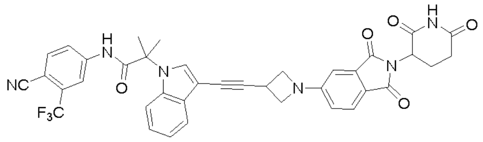 |
| 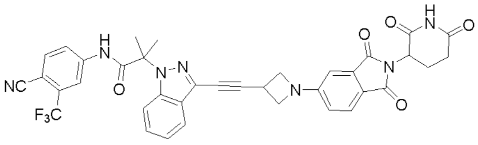 | 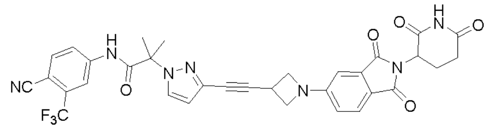 |
| 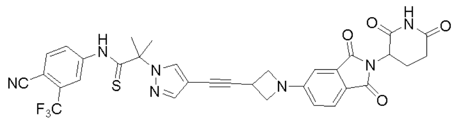 | 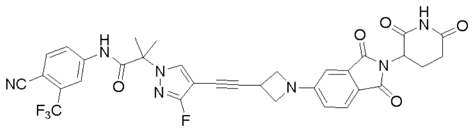 |
| 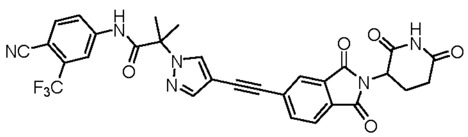 | 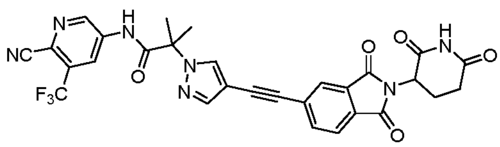 |
| 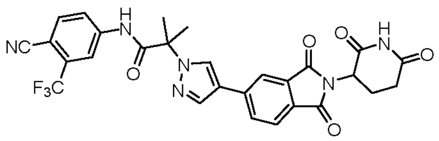 | 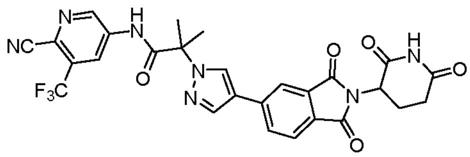 |
| 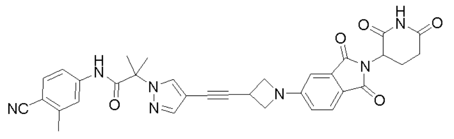 | 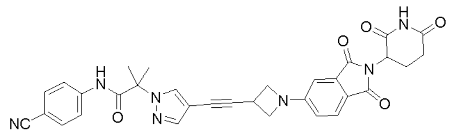 |
| 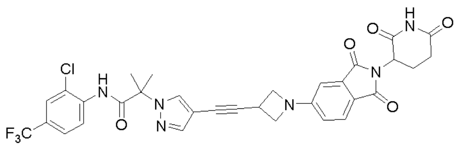 | 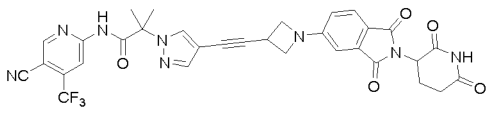 |
| 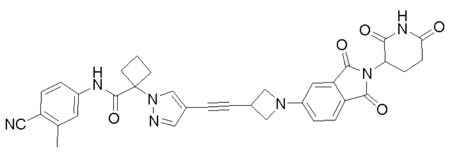 | 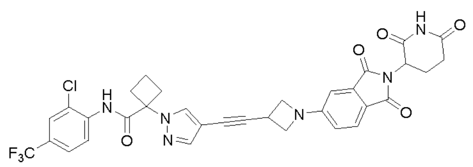 |
| 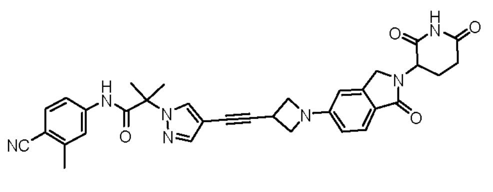 | 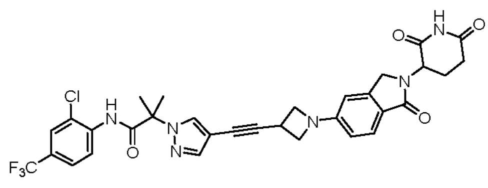 |

| | |
|---|---|
| 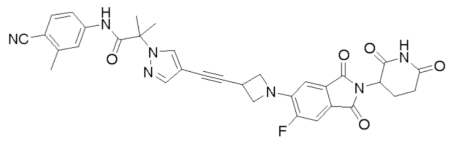 | 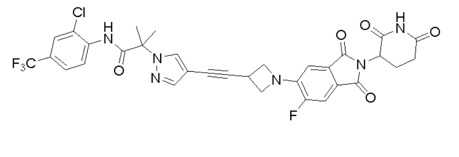 |
| 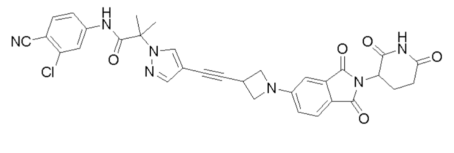 | 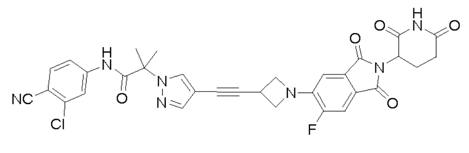 |
| 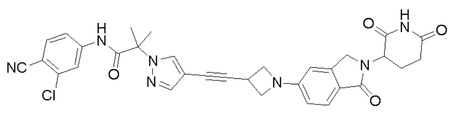 | 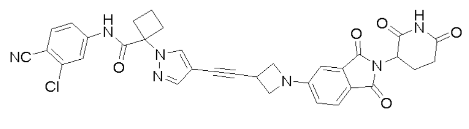 |
| 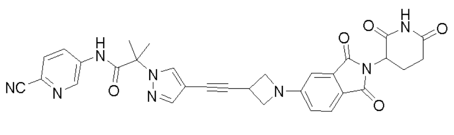 | 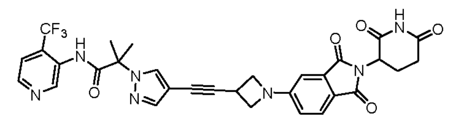 |
| 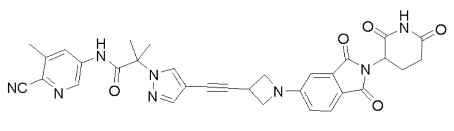 | 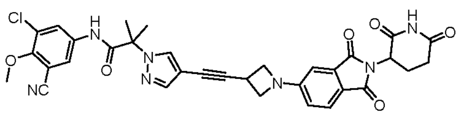 |
| 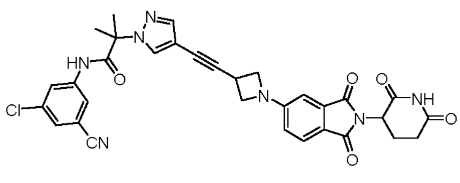 | 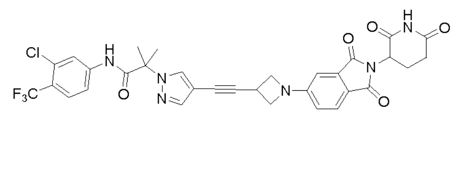 |
| 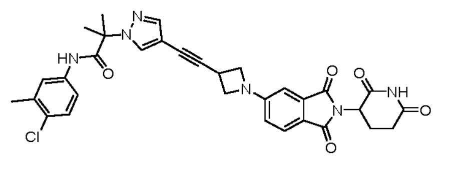 | 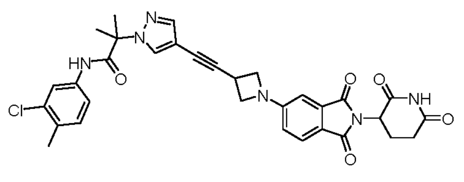 |
| 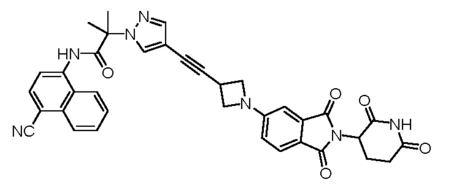 | 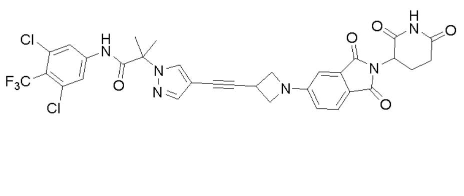 |
| 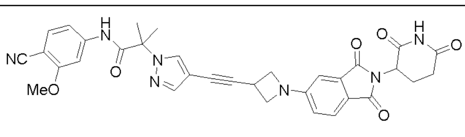 | 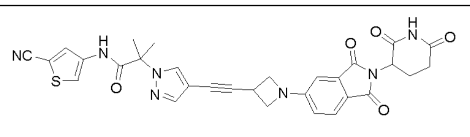 |
| 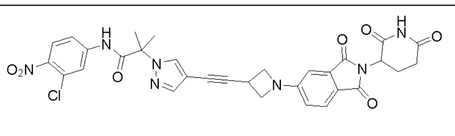 | 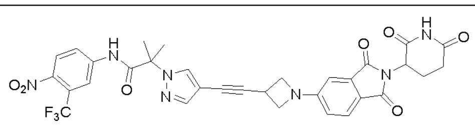 |
| 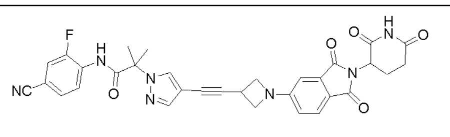 | 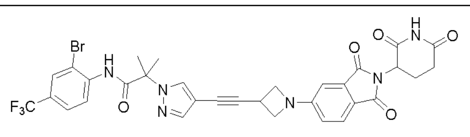 |

| | |
|---|---|
| 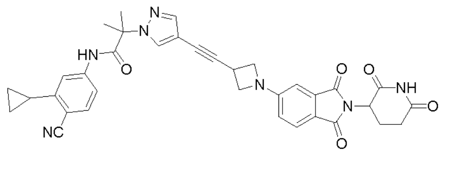 | 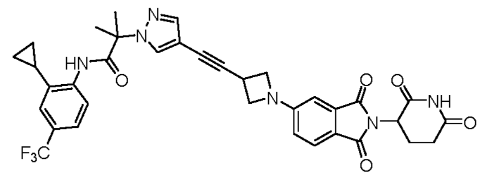 |
| 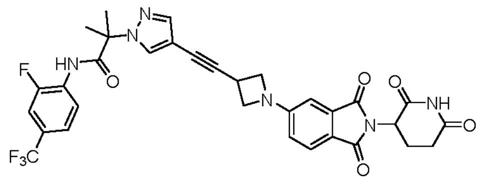 | 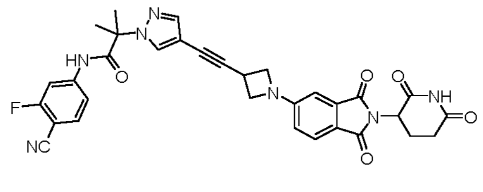 |
| 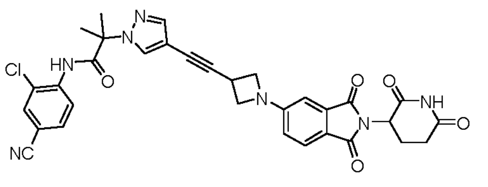 | 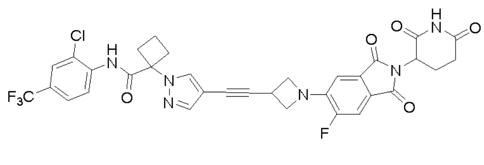 |
| 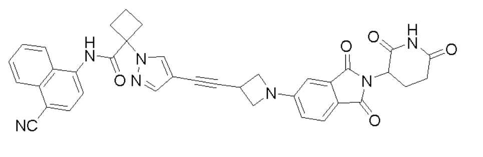 | 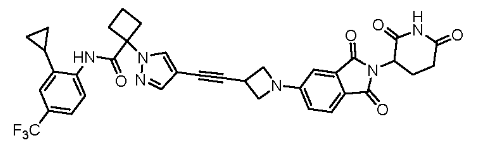 |
| 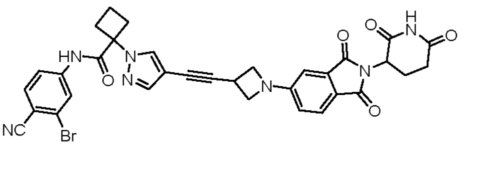 | 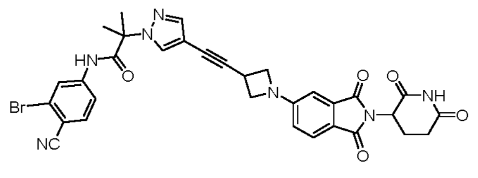 |
| 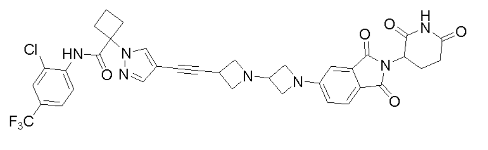 | 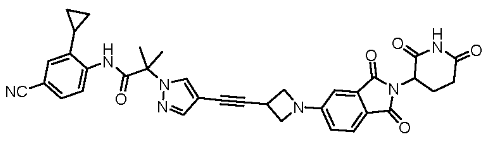 |
| 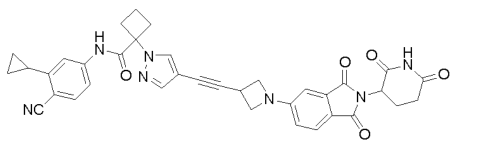 | 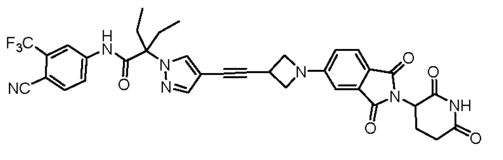 |
| 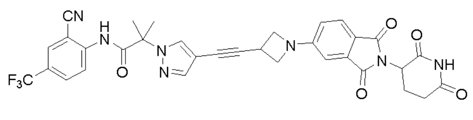 | |
| 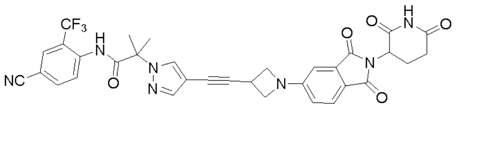 | 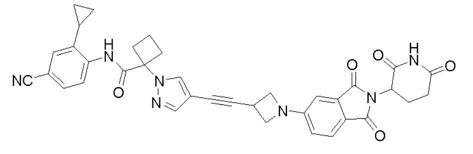 |
| 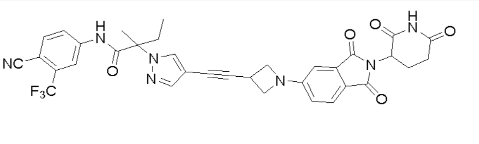 | 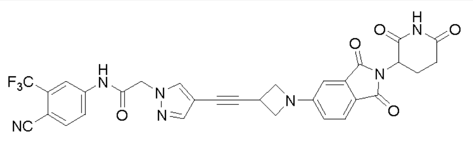 |

| | |
|---|---|
| 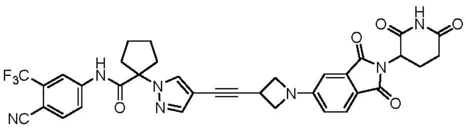 | 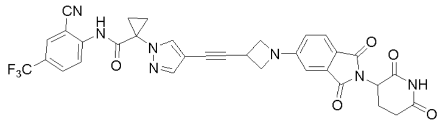 |
| 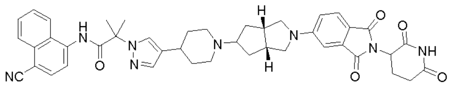 | 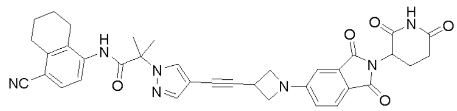 |
| 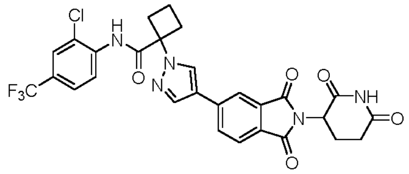 | 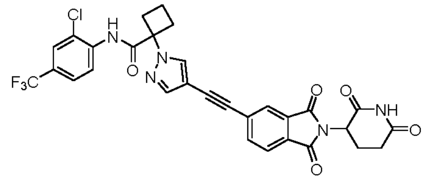 |
| 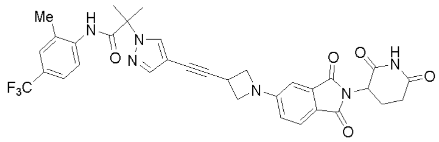 | 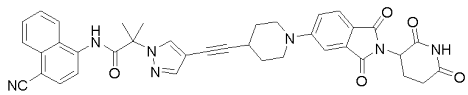 |
| 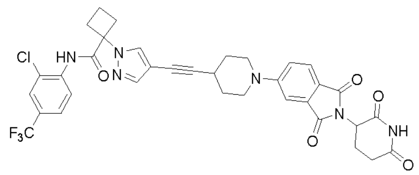 | 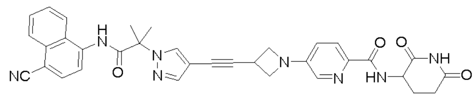 |
| 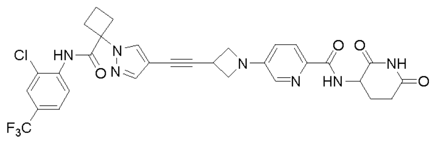 | 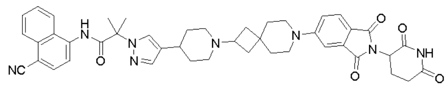 |
| 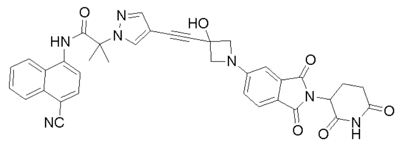 | 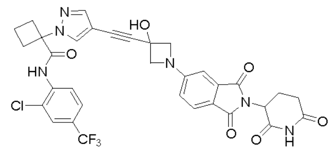 |
| 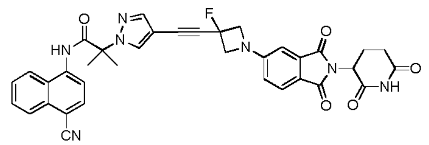 | 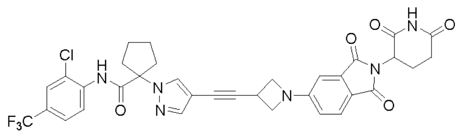 |
| 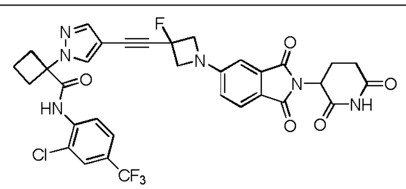 | 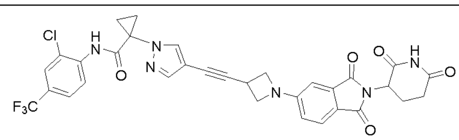 |
| 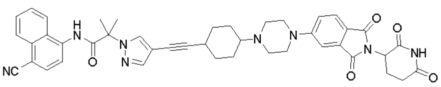 | 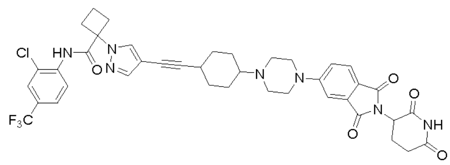 |

| | |
|---|---|
| 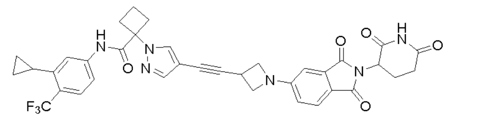 | 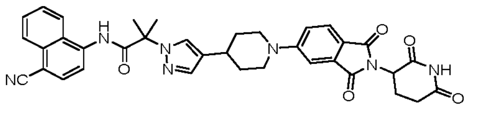 |
| 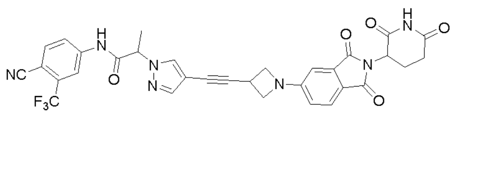 | 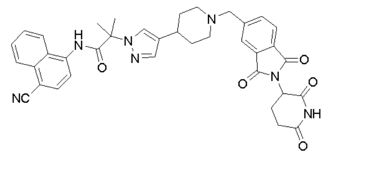 |
| 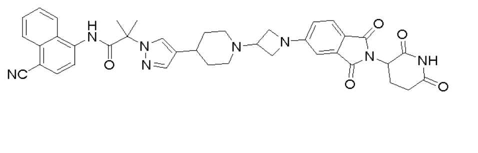 | 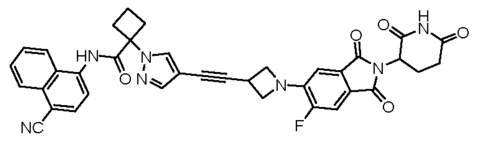 |
| 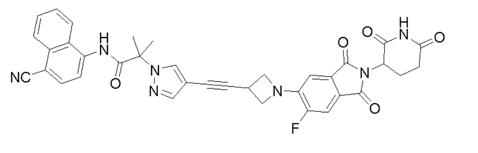 | 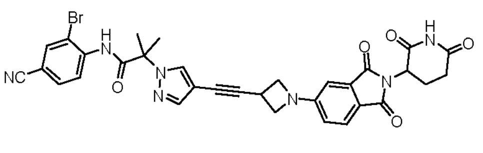 |
| 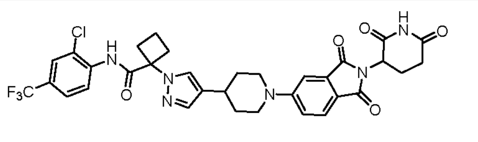 | 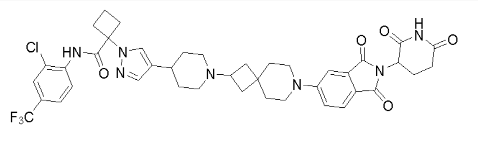 |
| 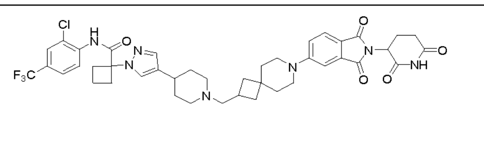 | 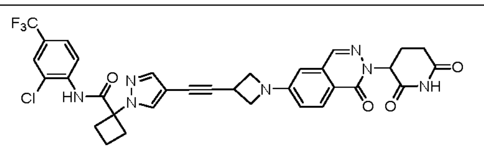 |
| 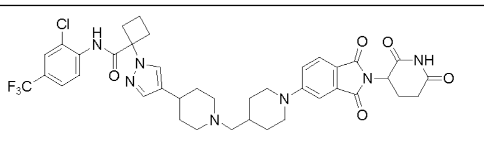 | 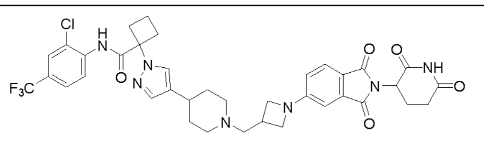 |
| 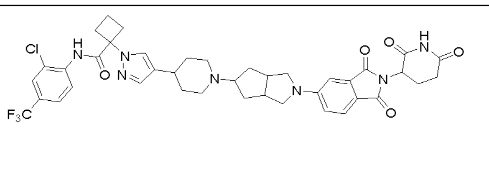 | 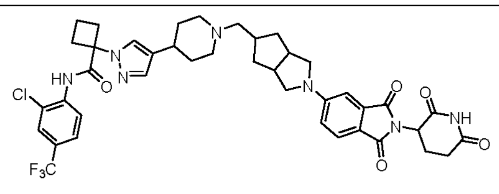 |
| 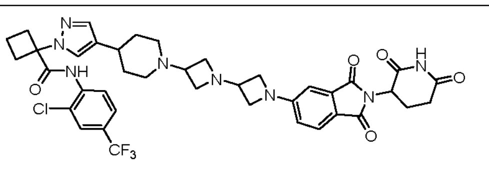 | 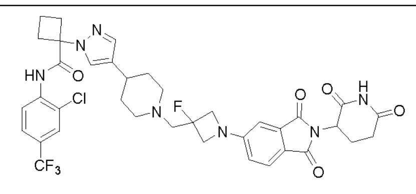 |
| 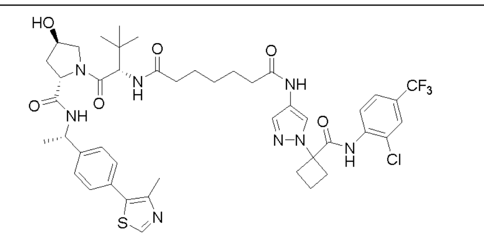 | 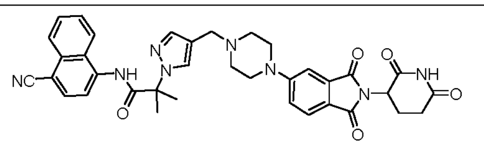 |

| | |
|---|---|
| 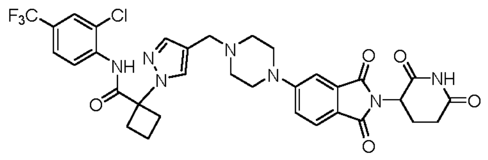 | 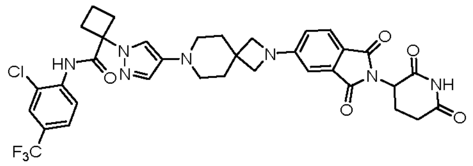 |
| 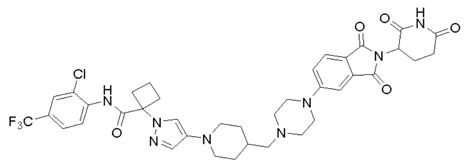 | 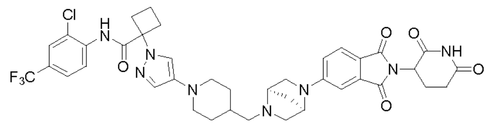 |
| 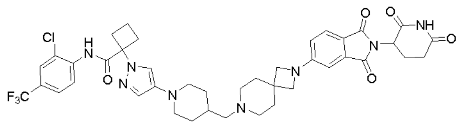 | 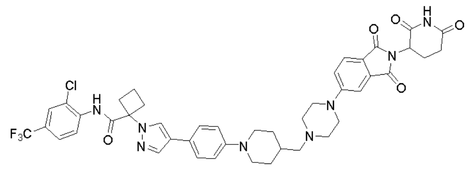 |
| 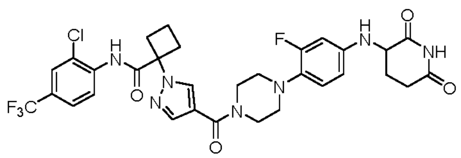 | 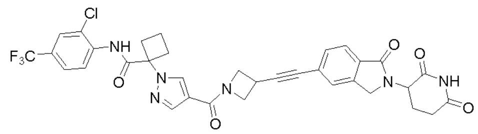 |
| 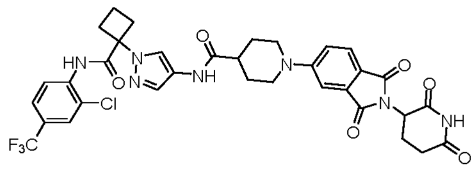 | 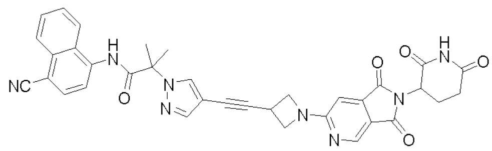 |
| 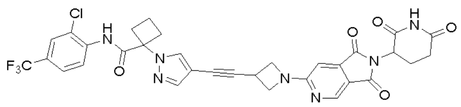 | 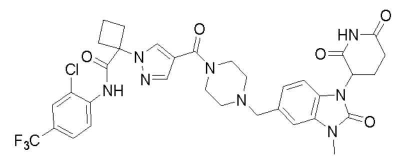 |
| 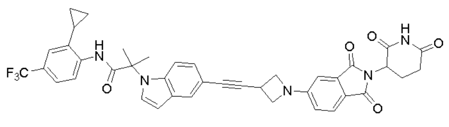 | 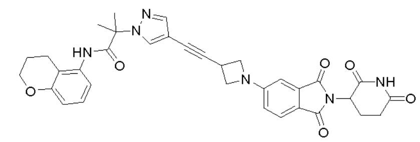 |
| 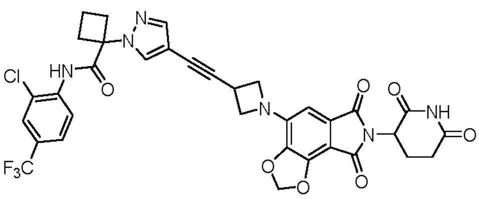 | 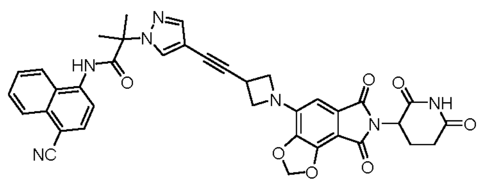 |
| 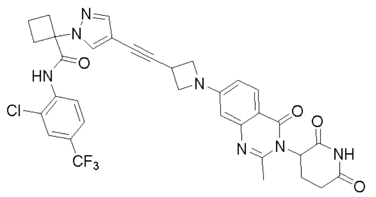 | 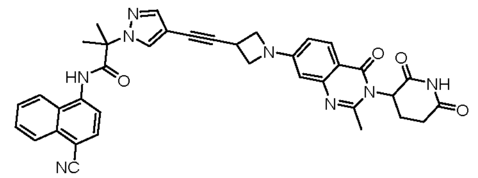 |

| | |
|---|---|
| 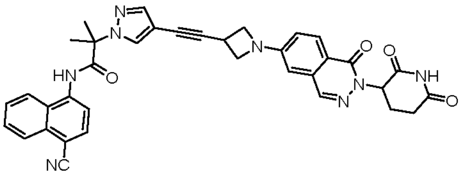 | 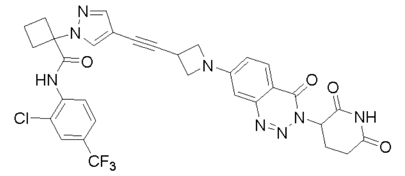 |
| 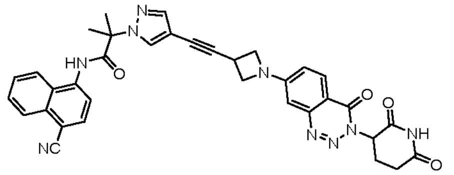 | 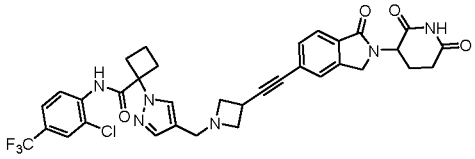 |
| 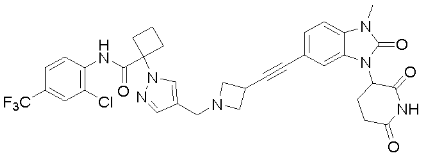 | 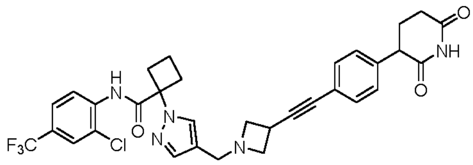 |
| 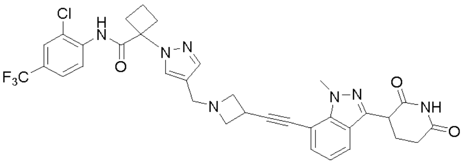 | 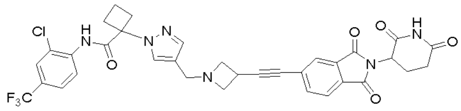 |
| 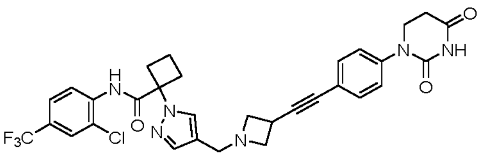 | 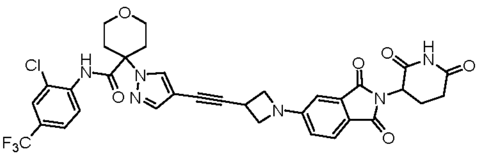 |
| 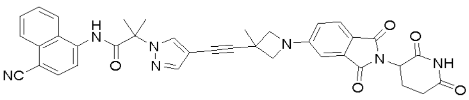 | 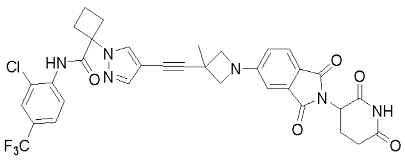 |
| 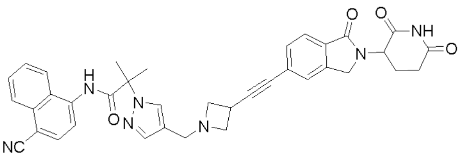 | 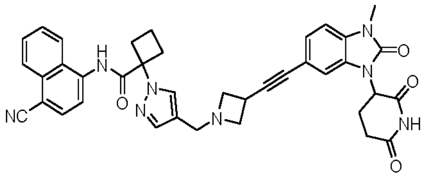 |
| 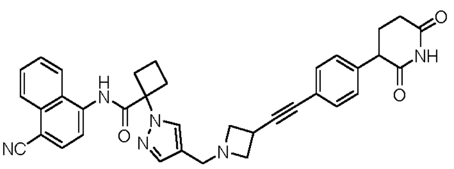 | 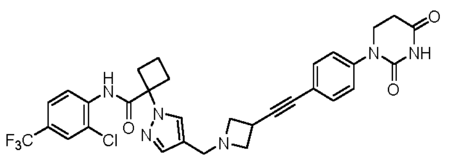 |
| 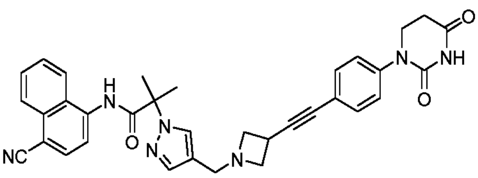 | 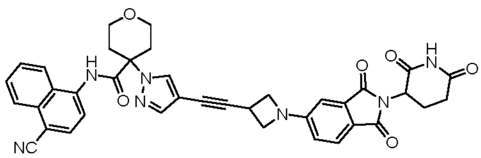 |

| | |
|---|---|
| 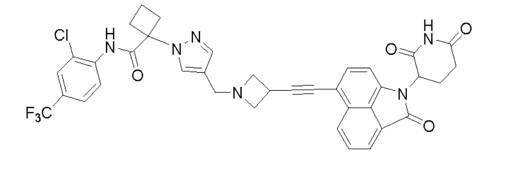 | 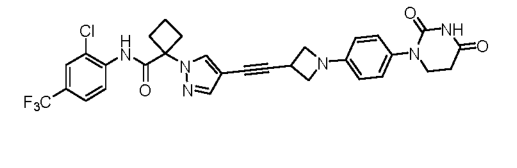 |
| 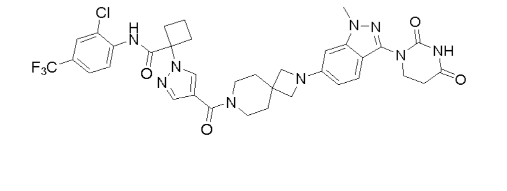 | 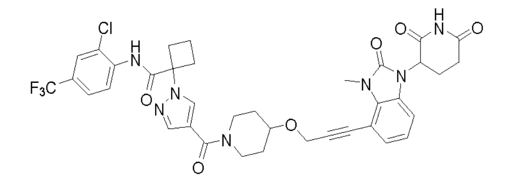 |
| 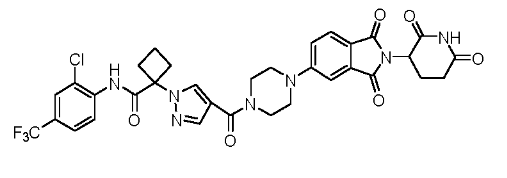 | 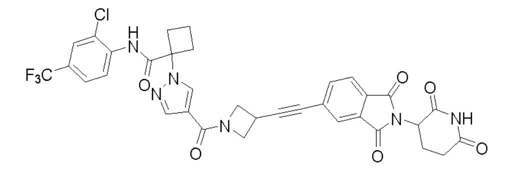 |
| 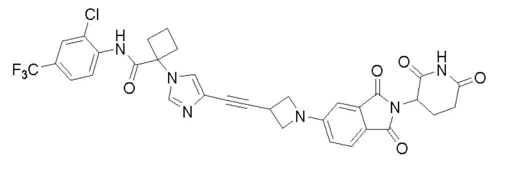 | 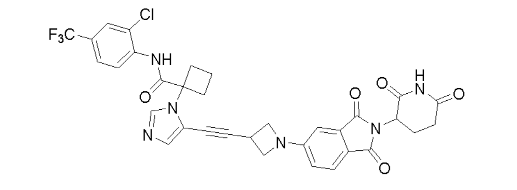 |
| 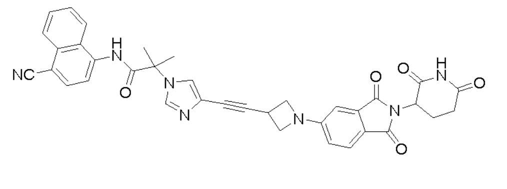 | 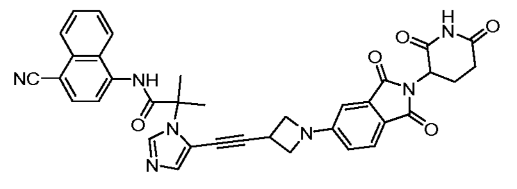 |
| 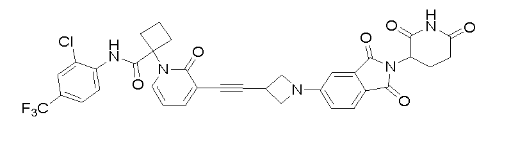 | 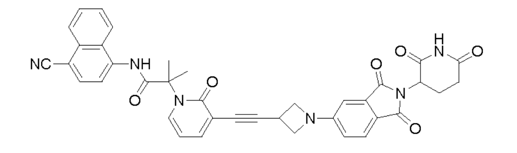 |
| 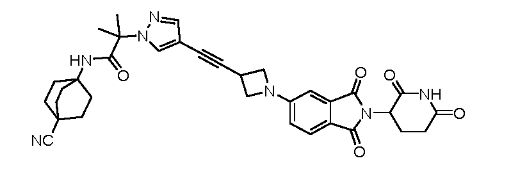 | 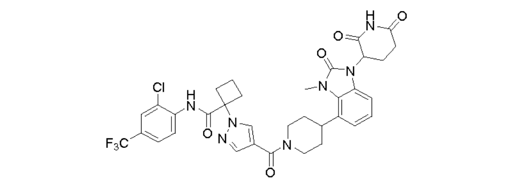 |
| 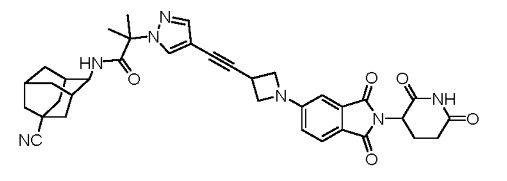 | 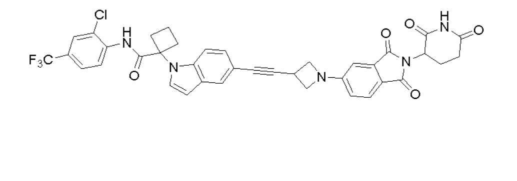 |
| 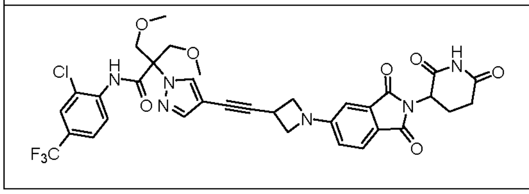 | 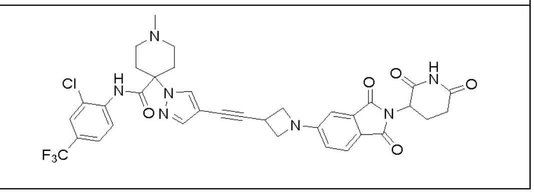 |

| | |
|---|---|
| 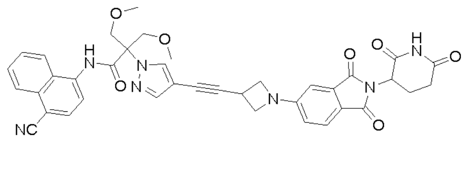 | 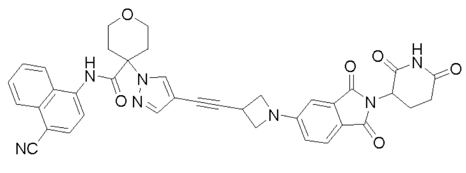 |
| 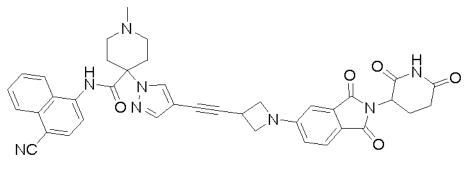 | 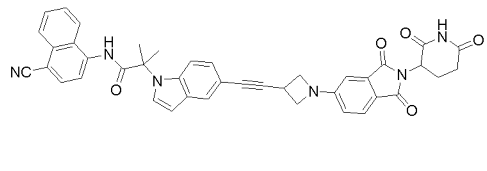 |
| 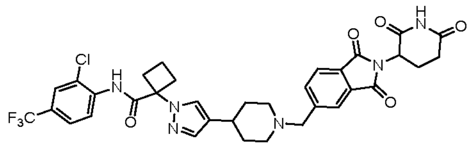 | 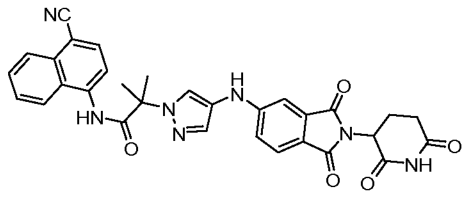 |
| 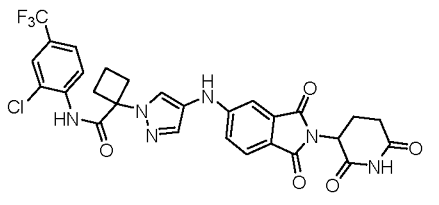 | 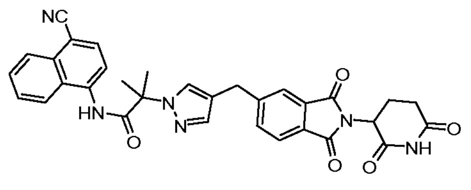 |
| 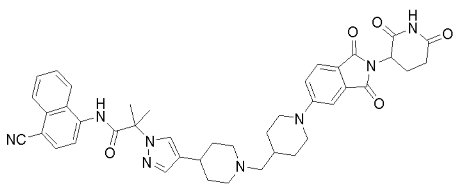 | 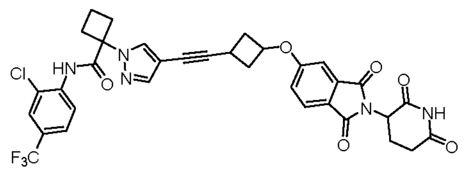 |
| 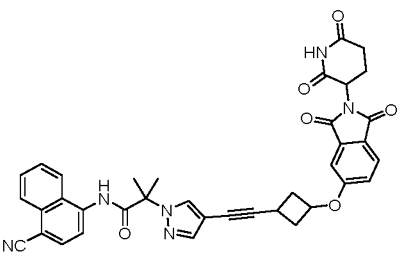 | 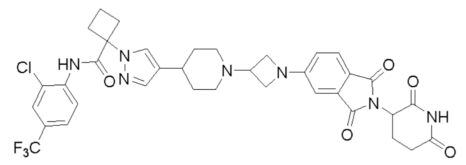 |
| 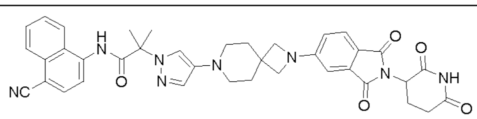 | 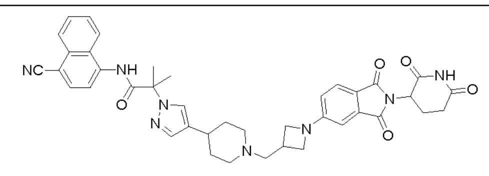 |
| 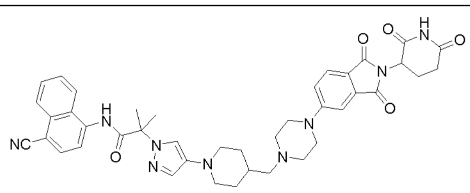 | 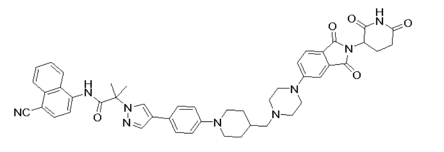 |

| | |
|---|---|
| 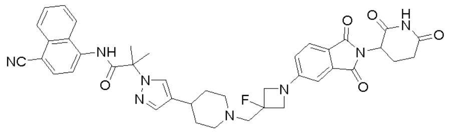 | 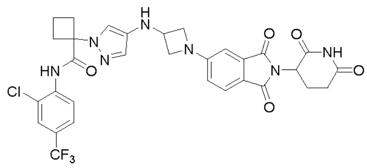 |
| 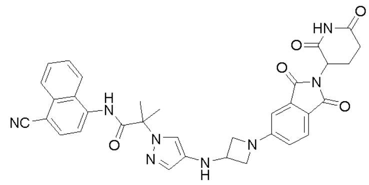 | 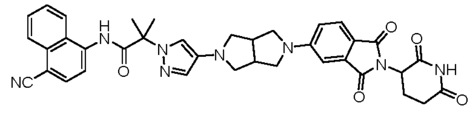 |
| 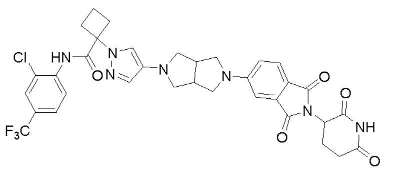 | 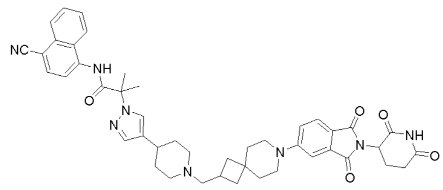 |
| 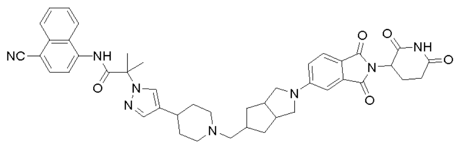 | 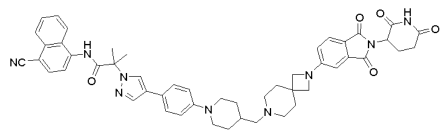 |
| 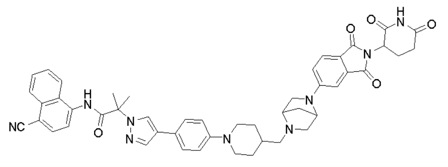 | 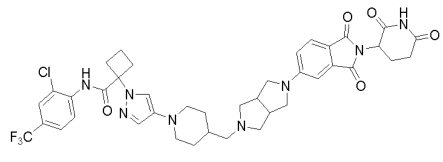 |
| 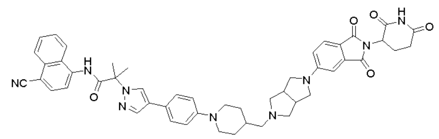 | 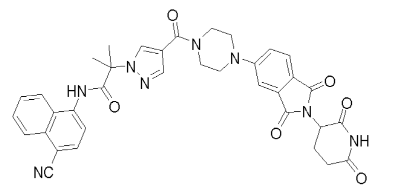 |
| 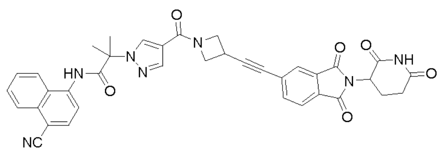 | 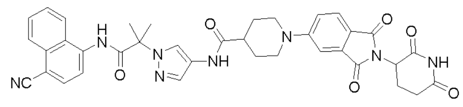 |
| 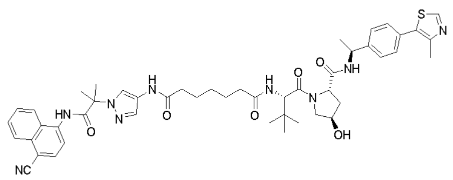 | 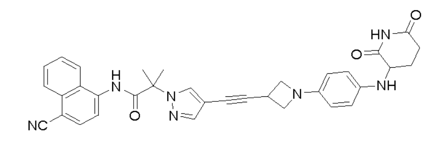 |
| 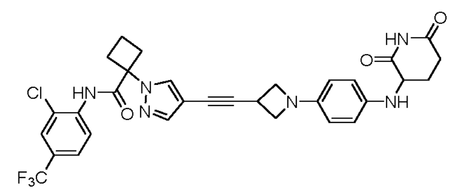 | 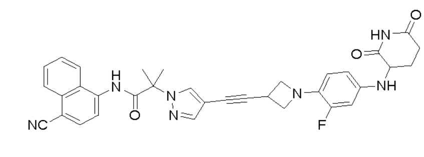 |

| | |
|---|---|
| 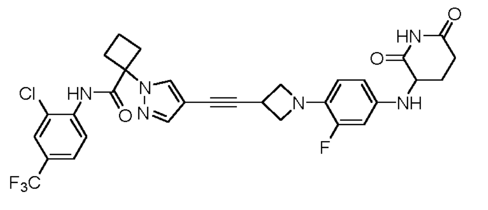 | 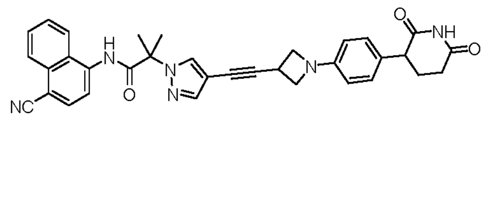 |
| 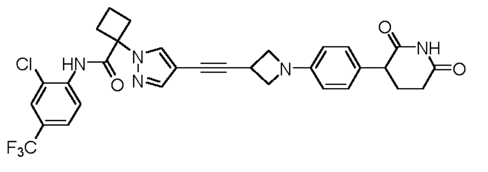 | 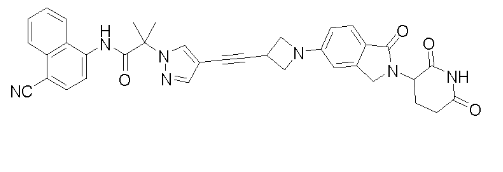 |
| 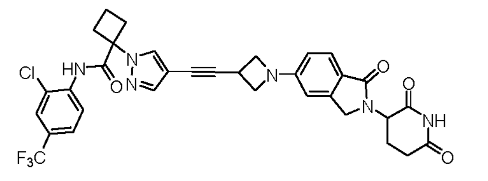 | 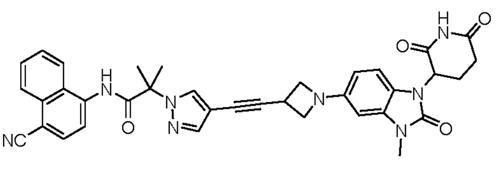 |
| 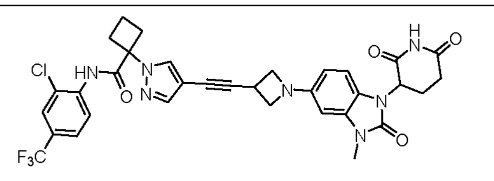 | 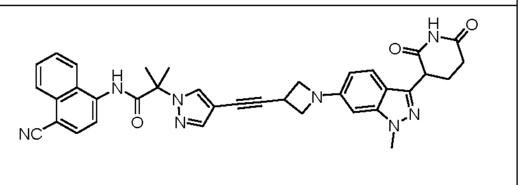 |
| 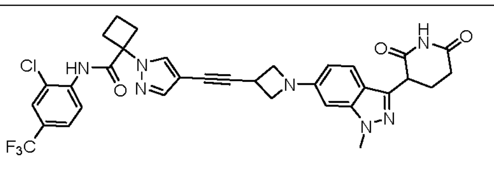 | 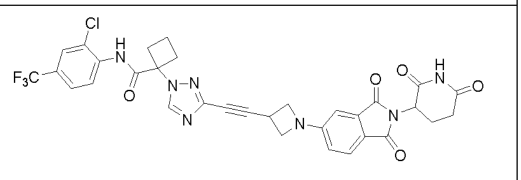 |
| 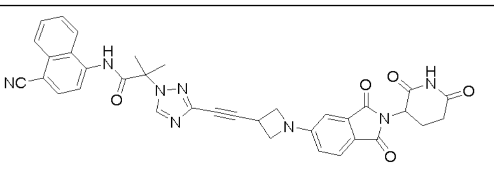 | 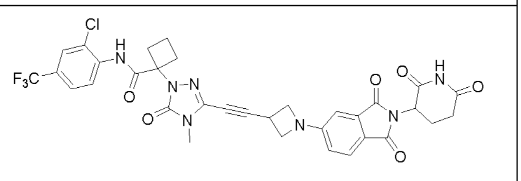 |
| 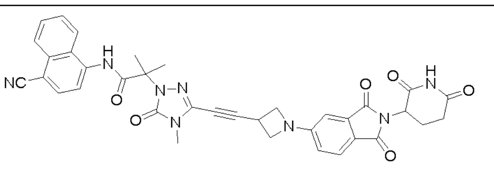 | 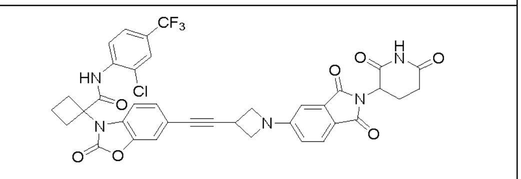 |
| 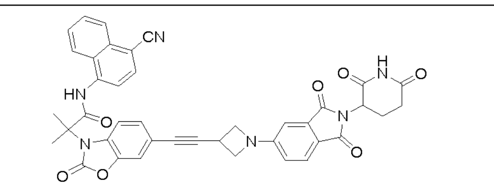 | 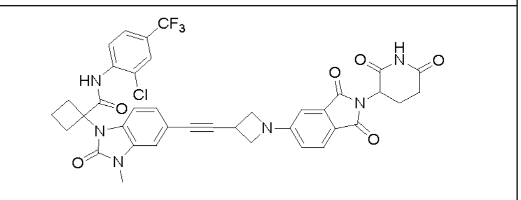 |
| 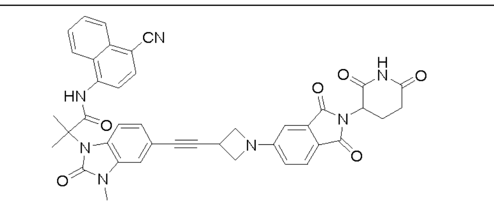 | 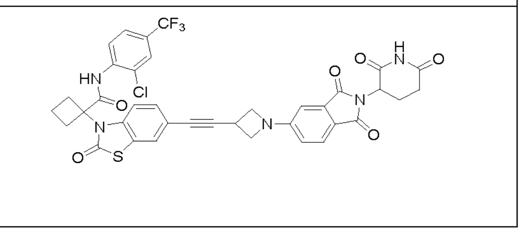 |

| | |
|---|---|
| 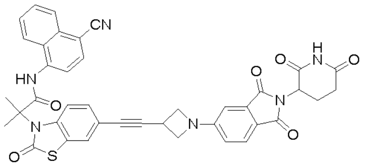 | 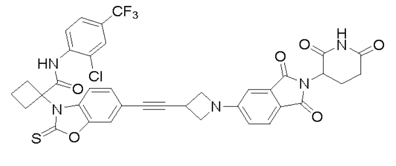 |
| 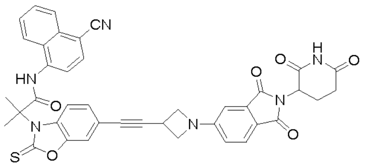 | 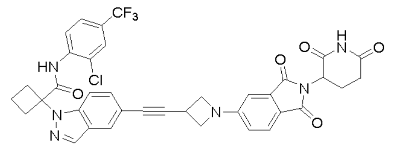 |
| 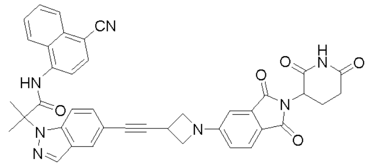 | 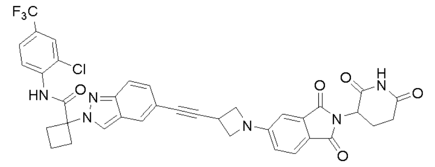 |
| 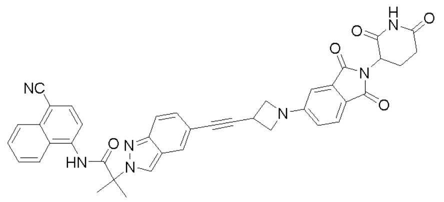 | 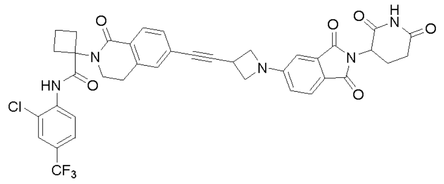 |
| 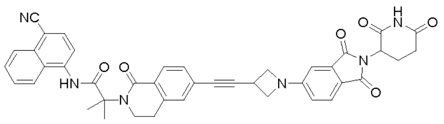 | 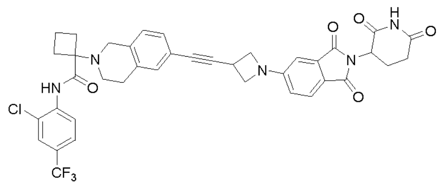 |
| 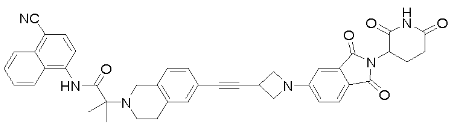 | 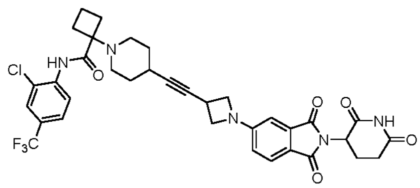 |
| 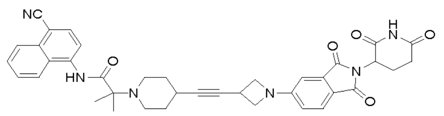 | 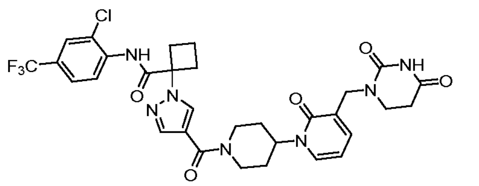 |
| 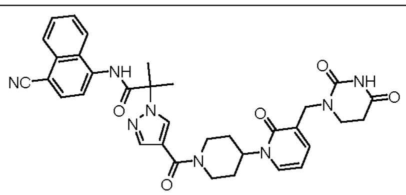 | 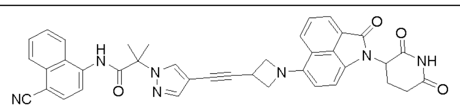 |
| 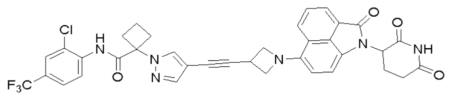 | 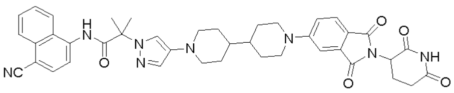 |

| | |
|---|---|
| 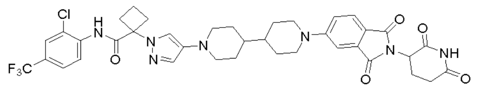 | 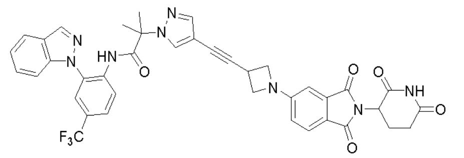 |
| 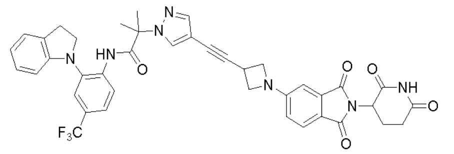 | 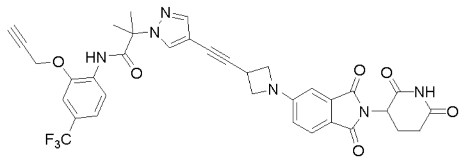 |
| 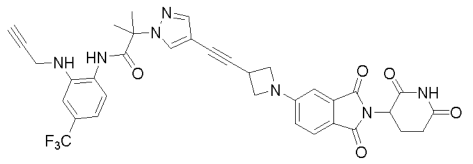 | 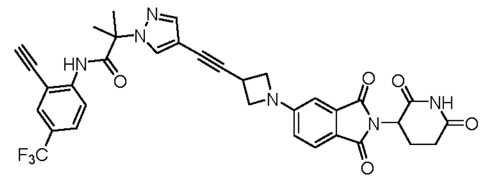 |
| 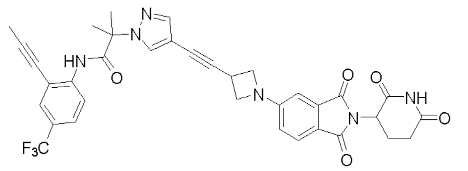 | 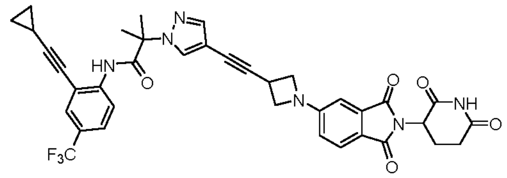 |
| 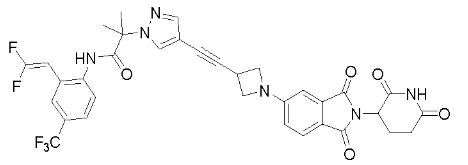 | 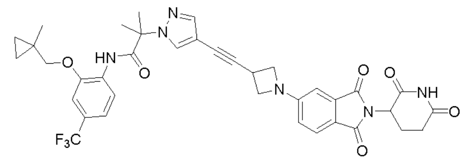 |
| 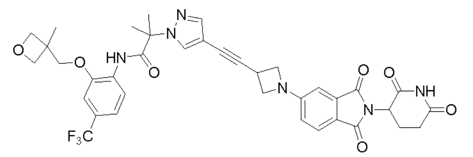 | 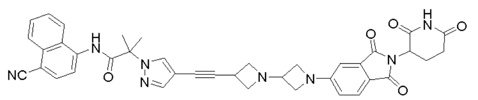 |
| 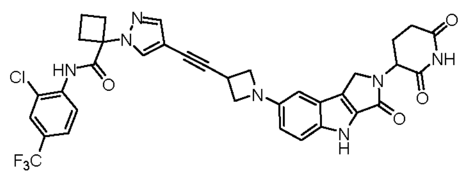 | 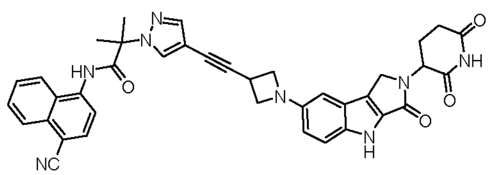 |
| 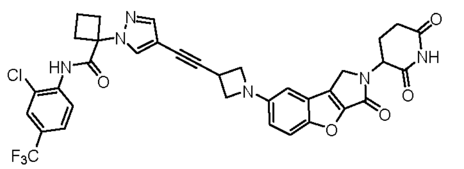 | 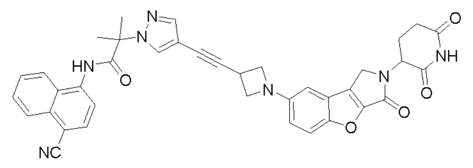 |
| 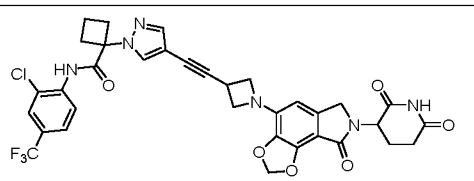 | 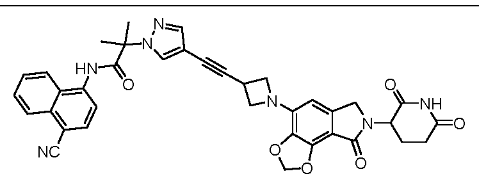 |

| | |
|---|---|
| 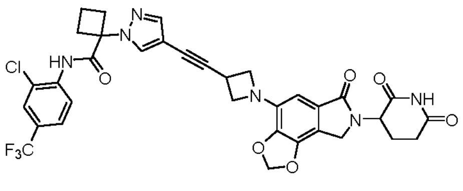 | 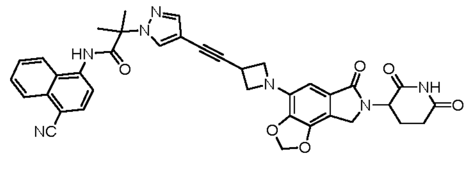 |
| 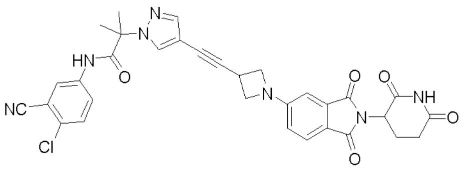 | 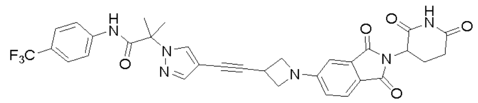 |
| 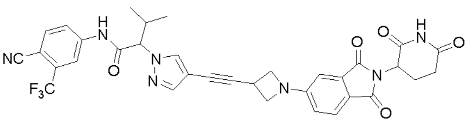 | 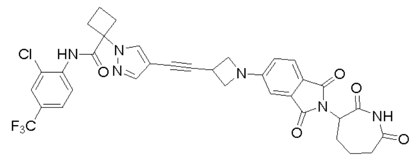 |
| 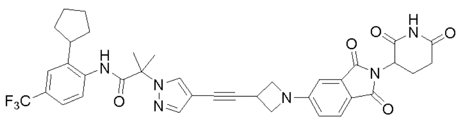 | 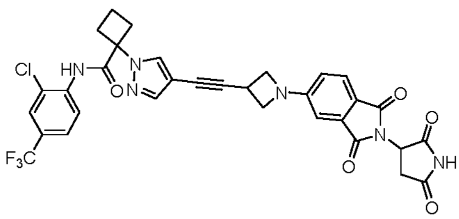 |
| 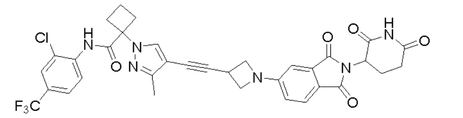 | 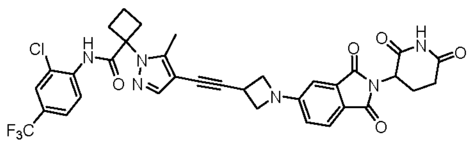 |
| 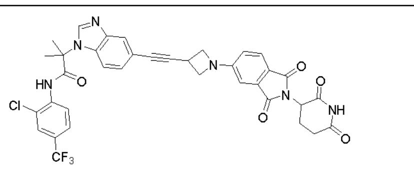 | 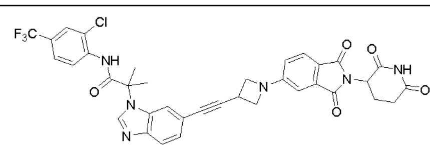 |
| 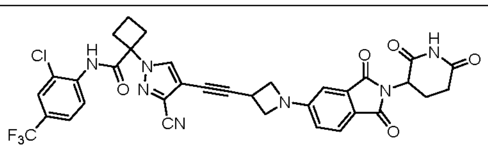 | 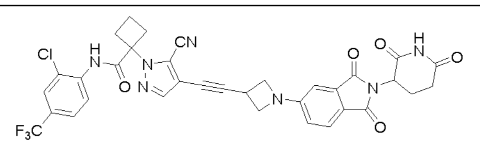 |
| 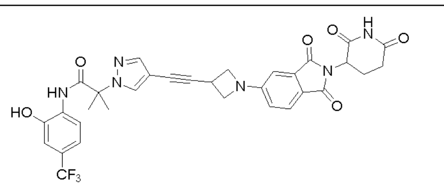 | 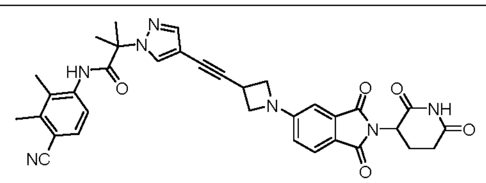 |
| 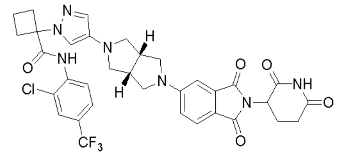 | 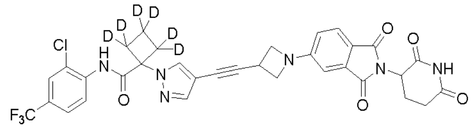 |

| | |
|---|---|
| 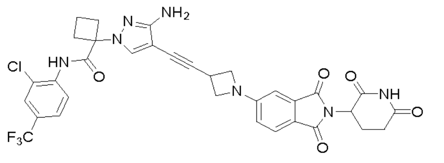 | 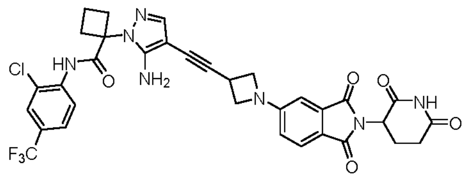 |
| 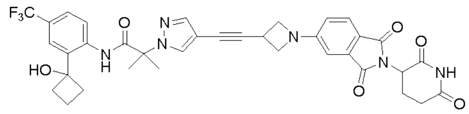 | 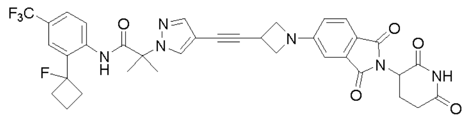 |
| 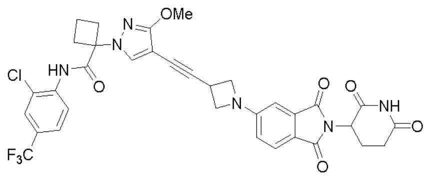 | 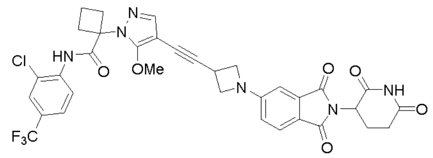 |
| 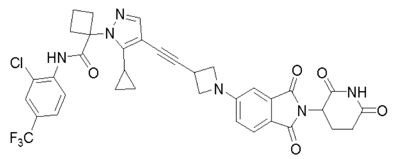 | 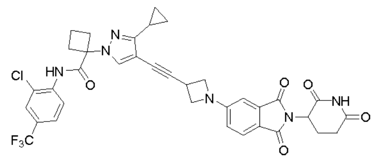 |
| 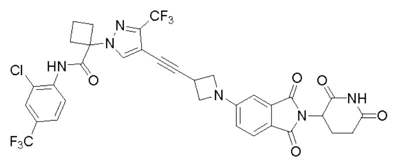 | 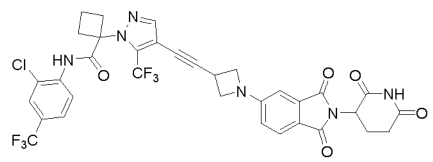 |
| 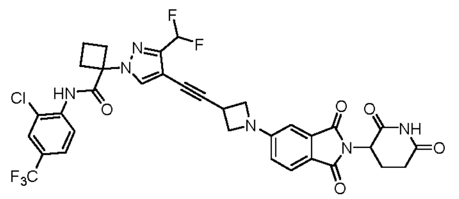 | 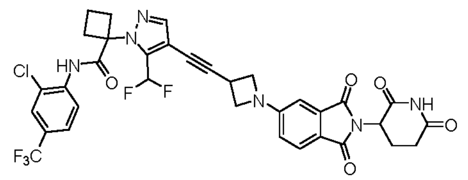 |
| 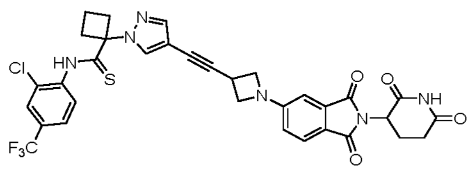 | 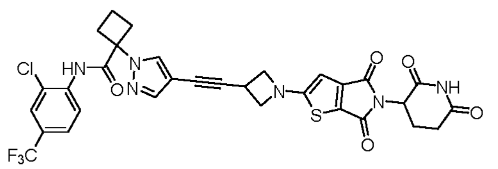 |
| 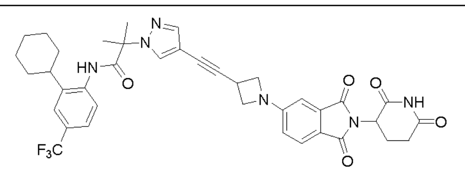 | 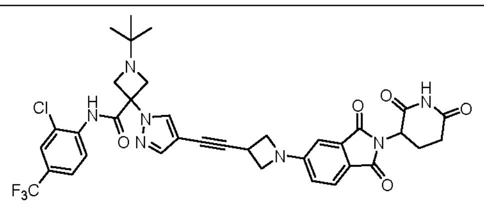 |
| 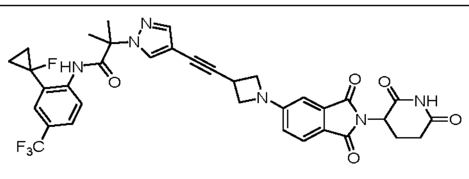 | 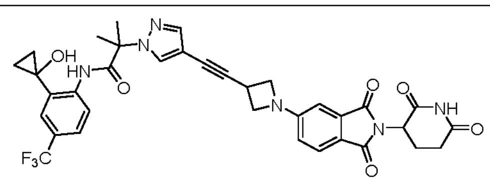 |

| | |
|---|---|
| 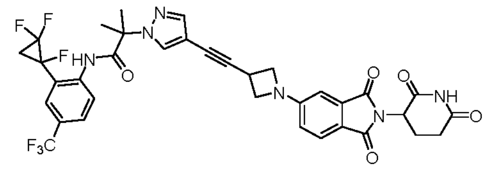 | 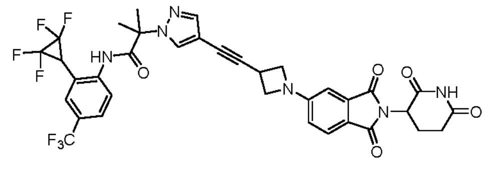 |
| 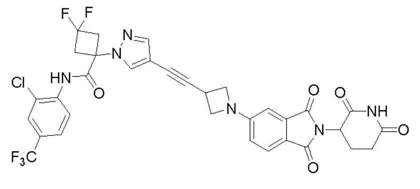 | 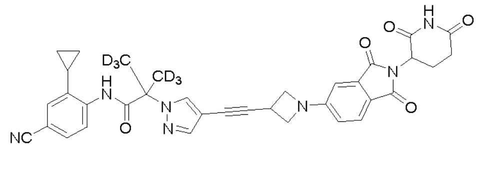 |
| 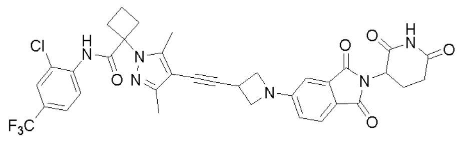 | 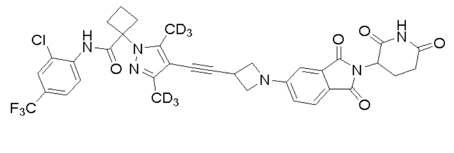 |
| 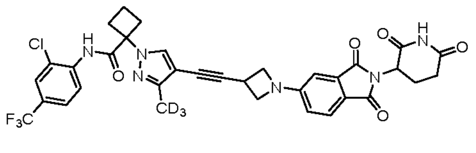 | 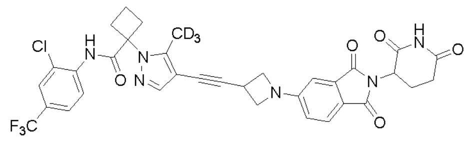 |
| 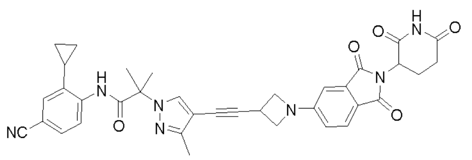 | 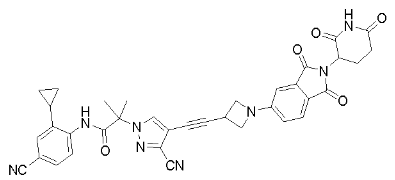 |
| 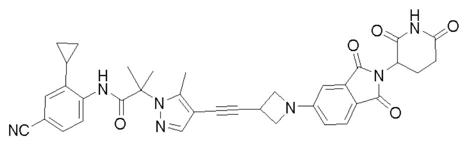 | 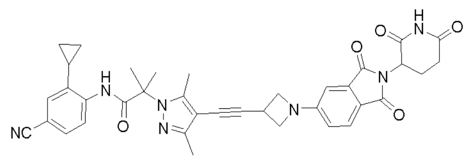 |

Table E-2

| | |
|---|---|
| 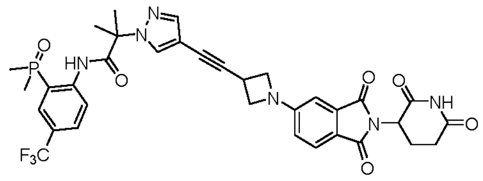 | 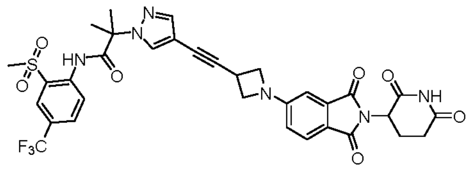 |
| 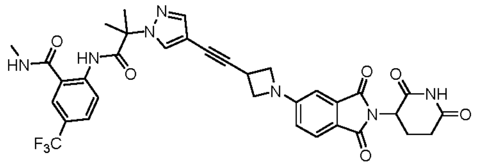 | 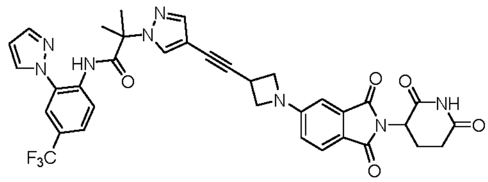 |
| 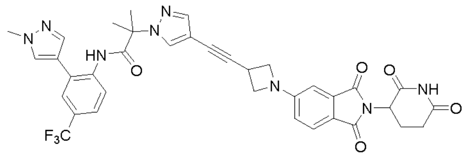 | 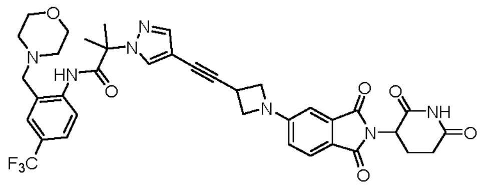 |

| | |
|---|---|
| 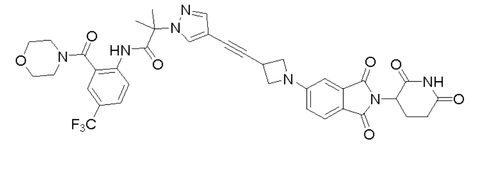 | 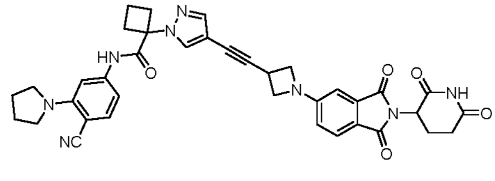 |
| 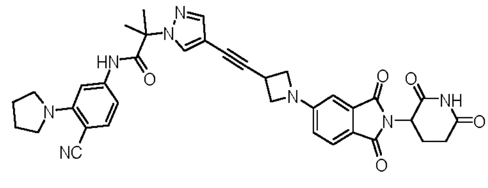 | 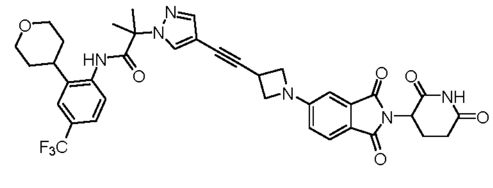 |
| 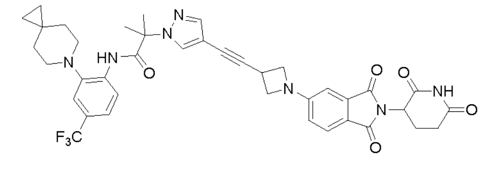 | 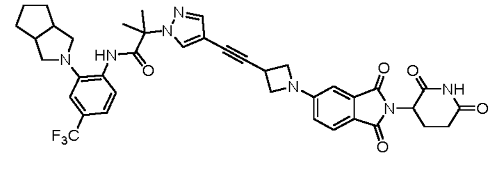 |
| 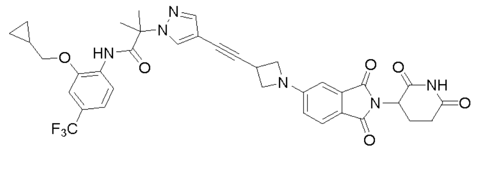 | 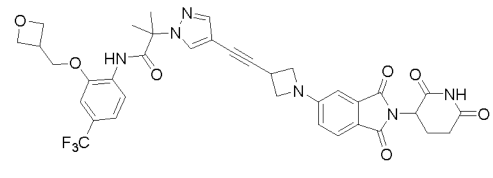 |
| 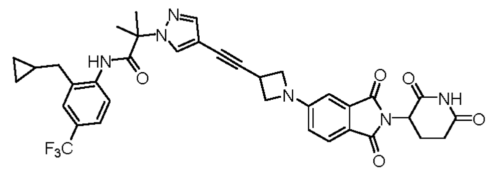 | 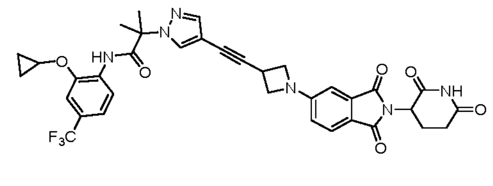 |
| 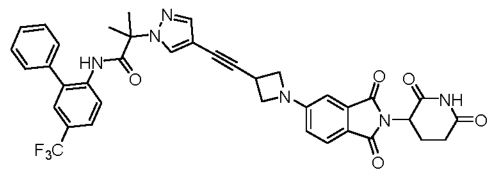 | 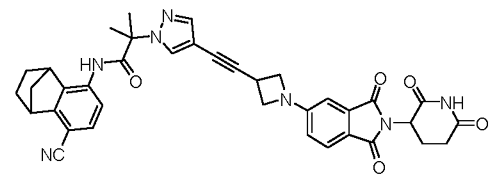 |
| 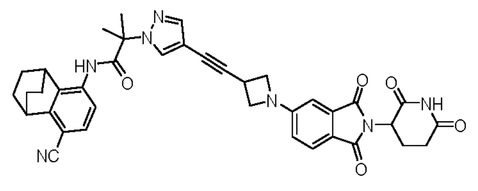 | 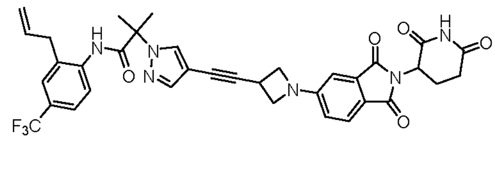 |
| 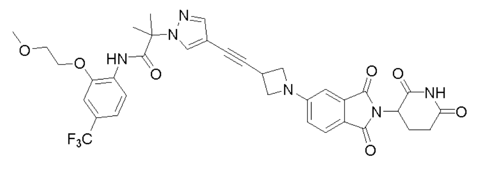 | 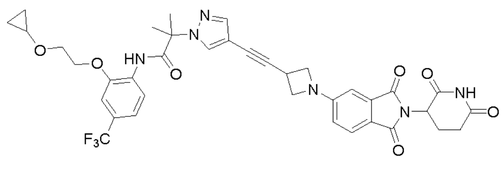 |
| 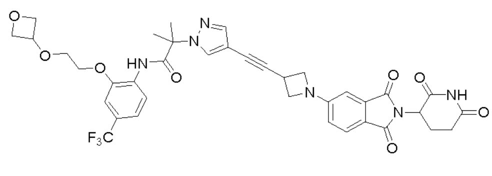 | 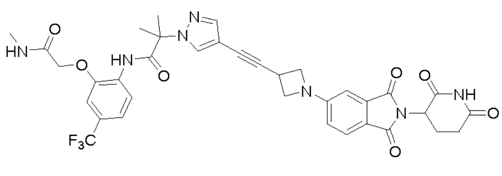 |

| | |
|---|---|
| 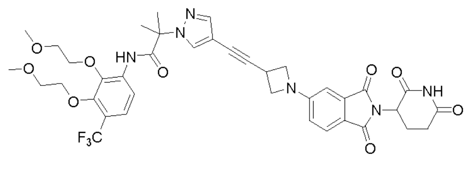 | 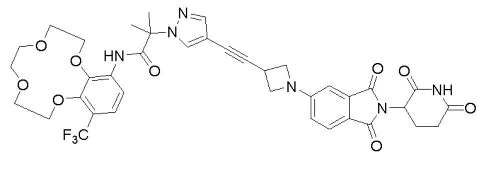 |
| 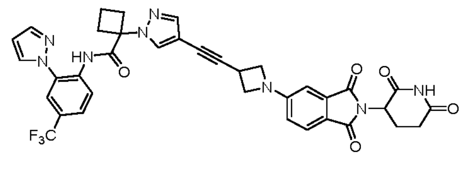 | 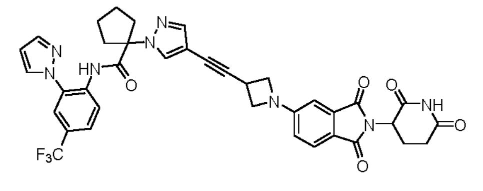 |
| 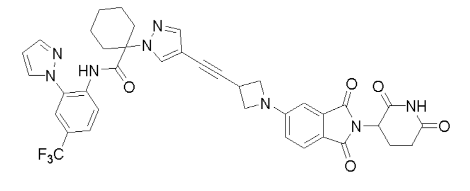 | 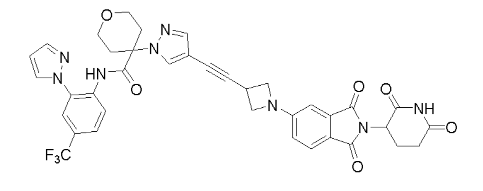 |
| 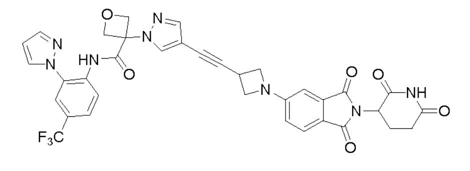 | 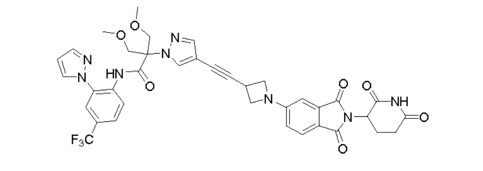 |
| 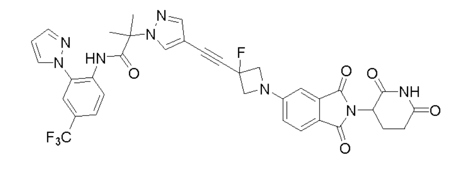 | 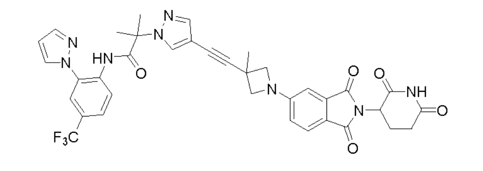 |
| 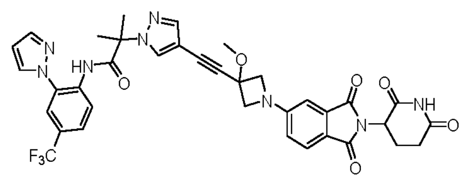 | 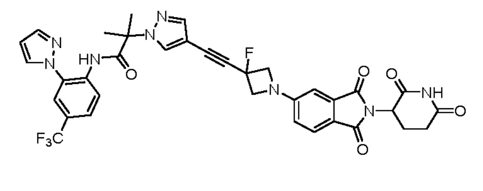 |
| 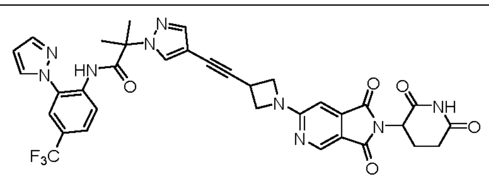 | 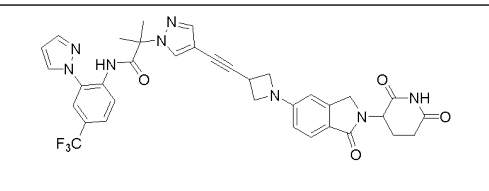 |
| 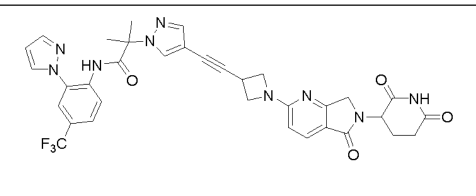 | 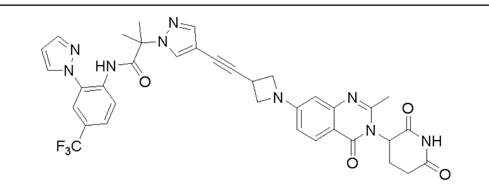 |
| 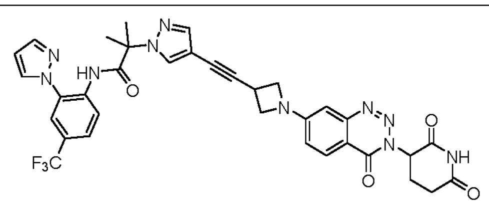 | 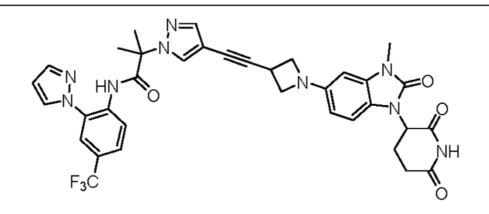 |

| | |
|---|---|
| 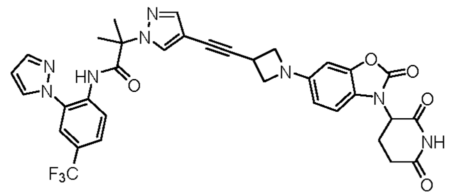 | 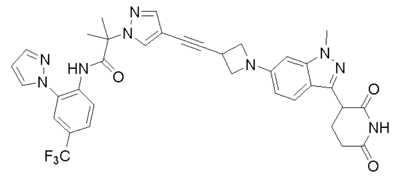 |
| 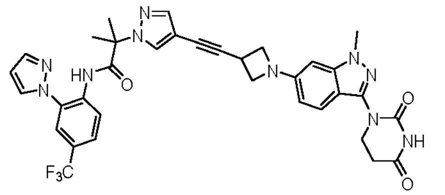 | 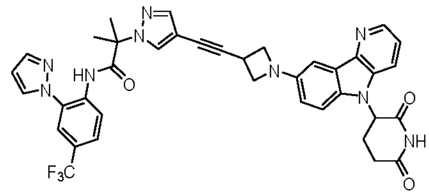 |
| 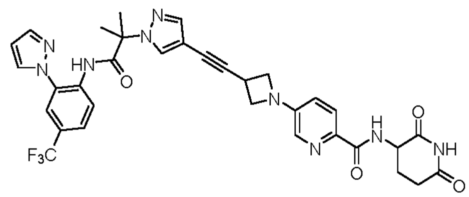 | 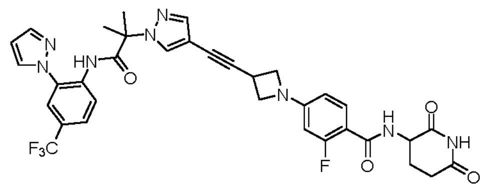 |
| 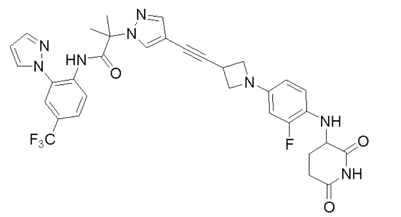 | 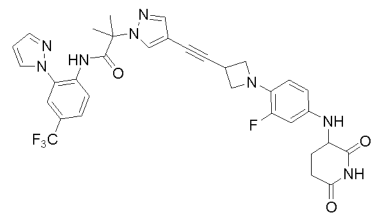 |
| 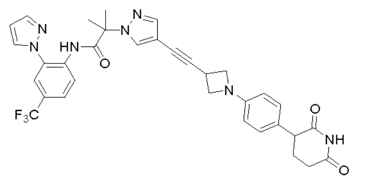 | 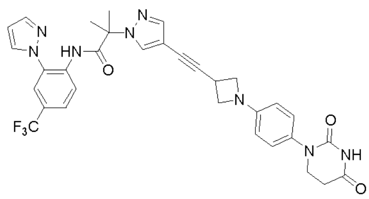 |
| 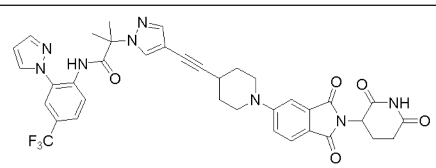 | 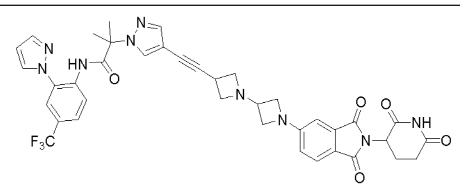 |
| 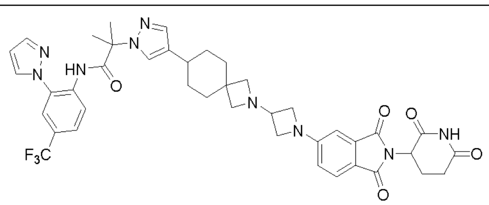 | 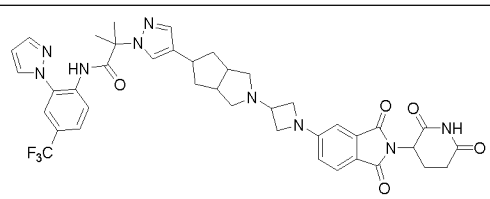 |
| 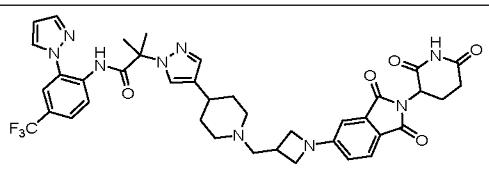 | 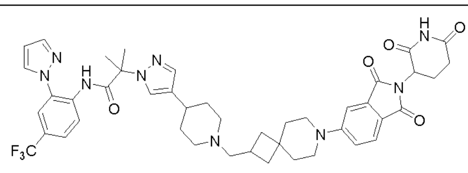 |

| | |
|---|---|
| 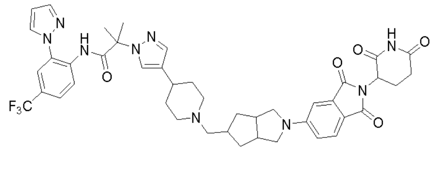 | 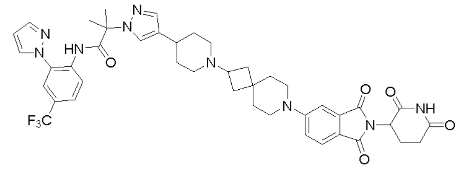 |
| 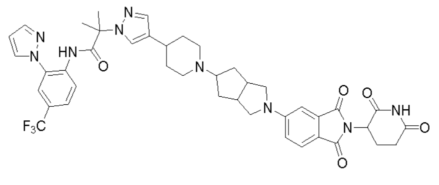 | 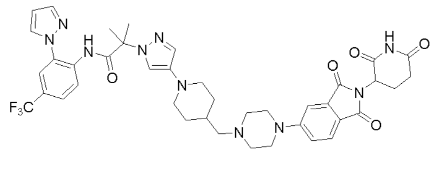 |
| 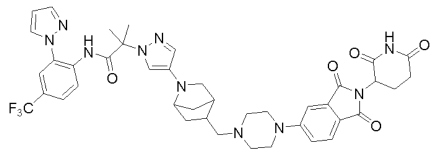 | 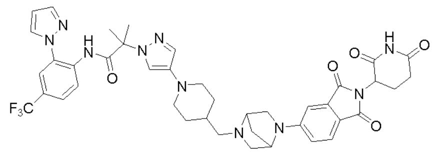 |
| 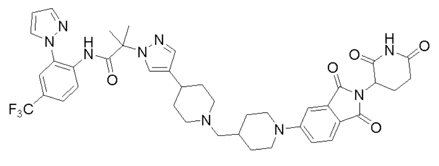 | 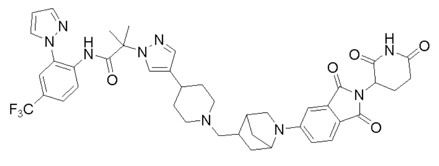 |
| 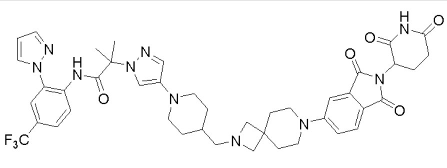 | 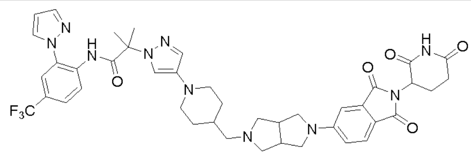 |
| 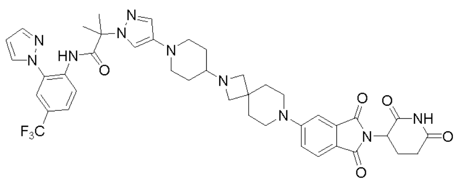 | 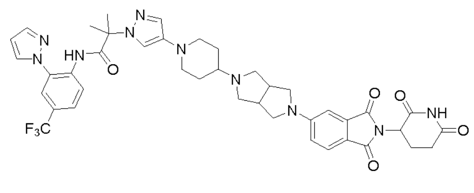 |
| 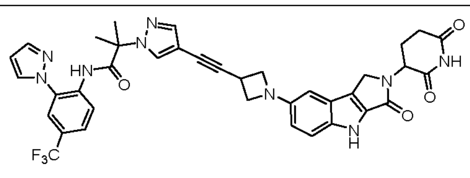 | 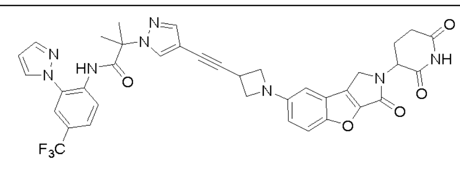 |
| 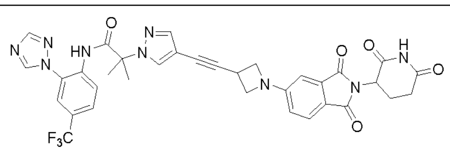 | 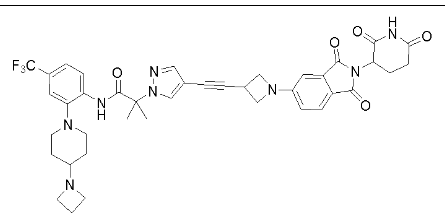 |
| 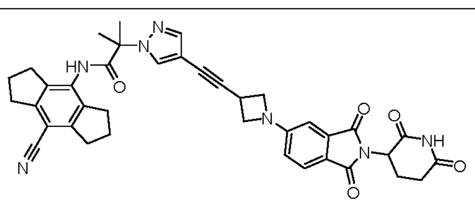 | 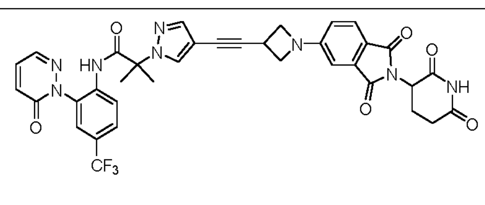 |

| | |
|---|---|
| 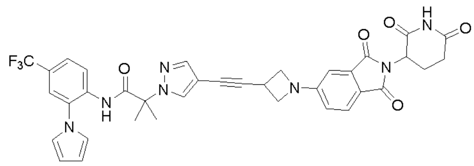 | 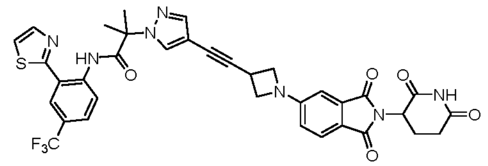 |
| 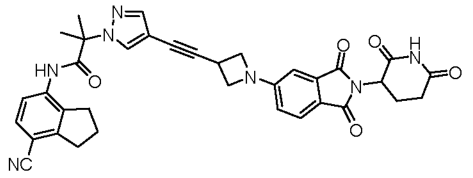 | 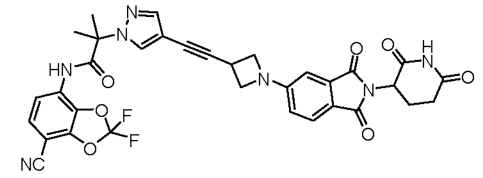 |
| 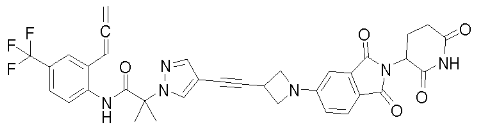 | 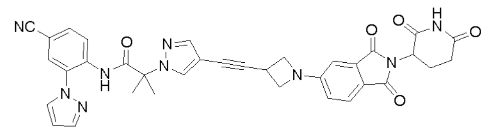 |
| 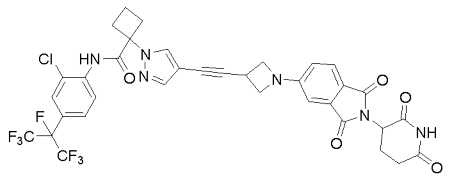 | 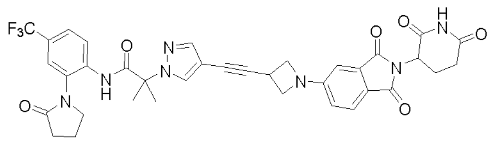 |
| 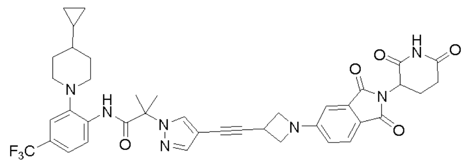 | 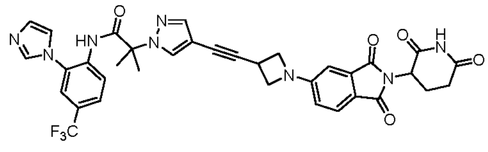 |
| 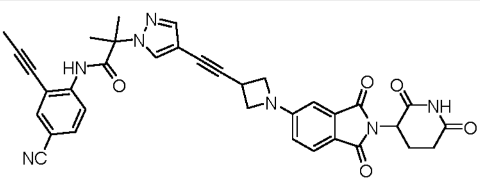 | 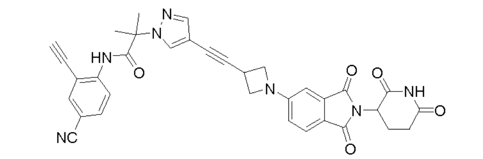 |
| 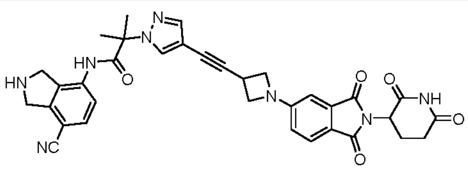 | 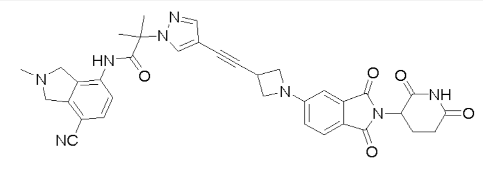 |
| 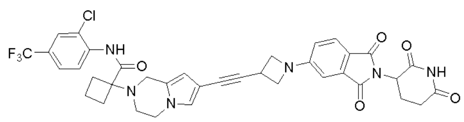 | 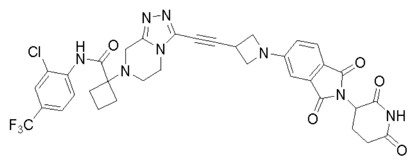 |
| 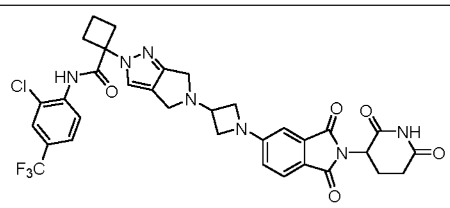 | 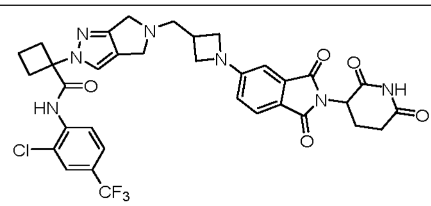 |

| | |
|---|---|
| 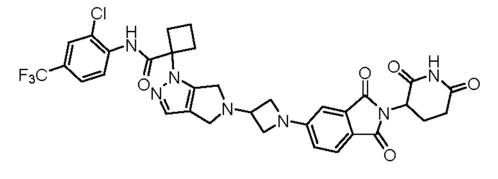 | 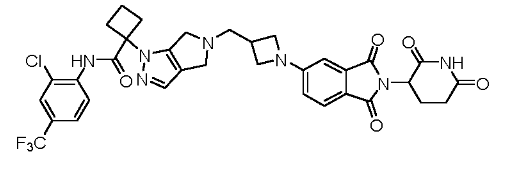 |
| 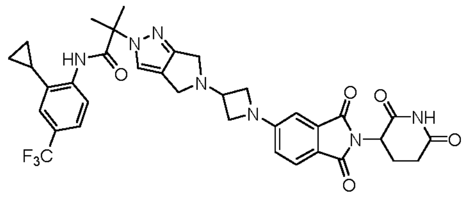 | 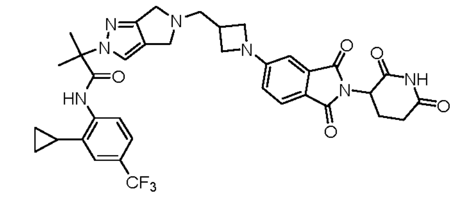 |
| 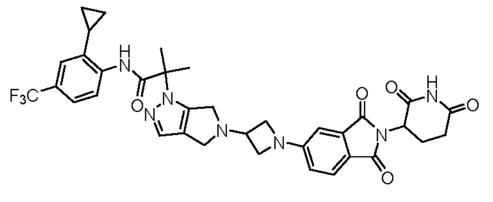 | 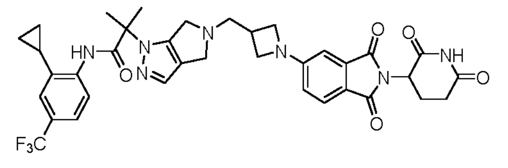 |
| 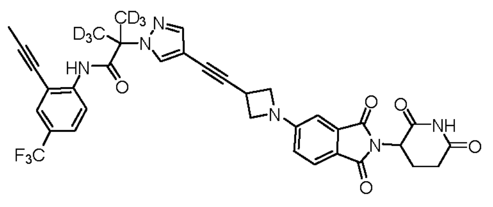 | 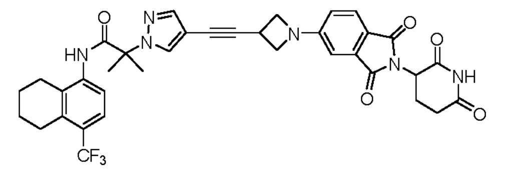 |
| 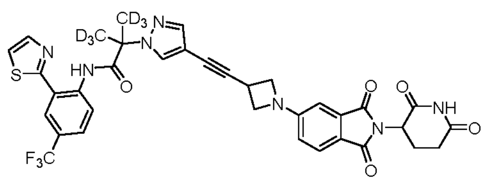 | 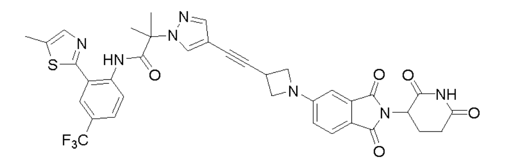 |
| 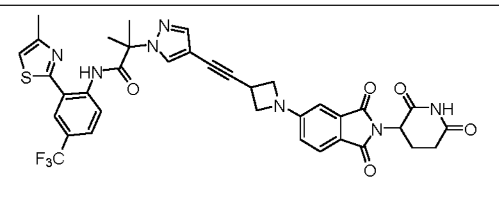 | 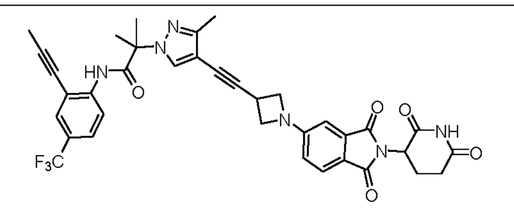 |
| 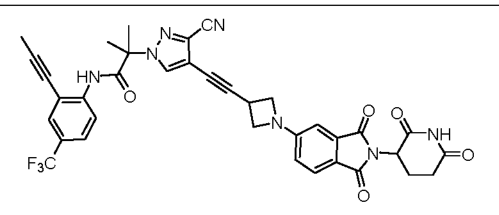 | 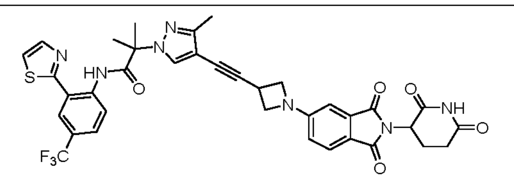 |
| 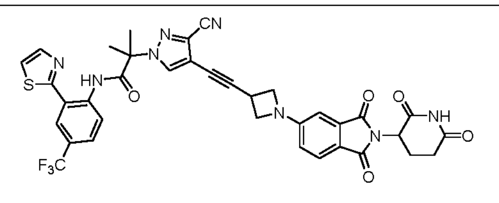 | 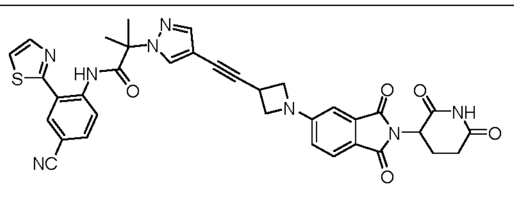 |

| | |
|---|---|
| 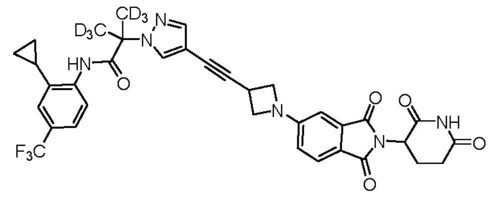 | 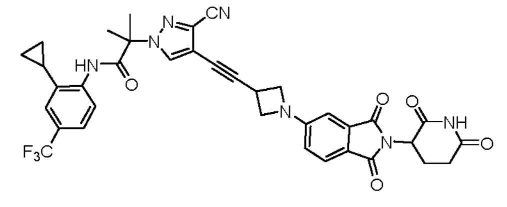 |
| 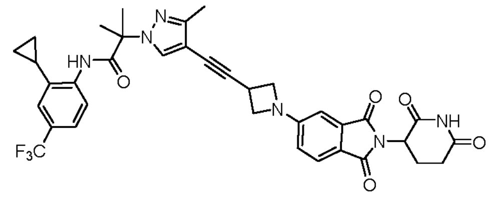 | 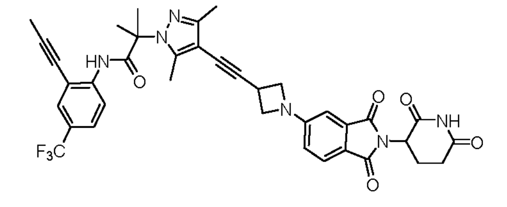 |
| 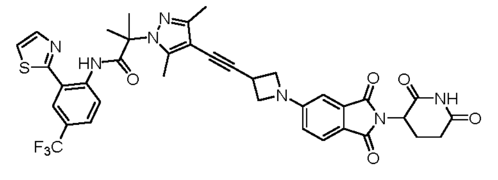 | 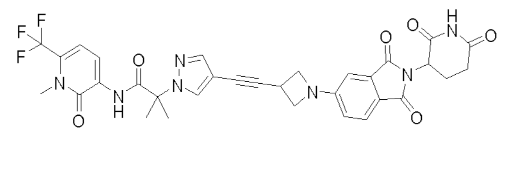 |
| 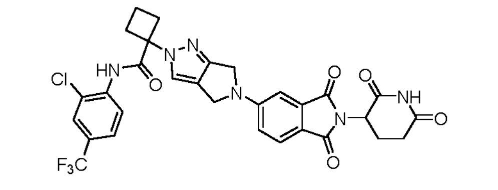 | 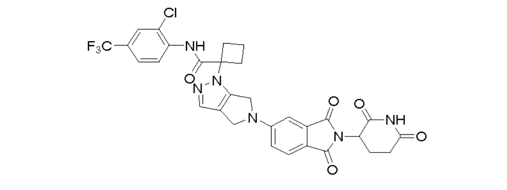 |
| 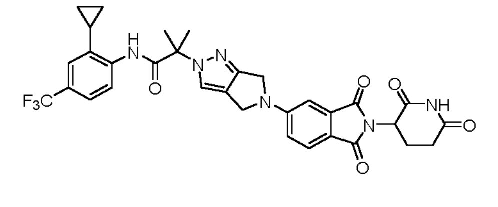 | 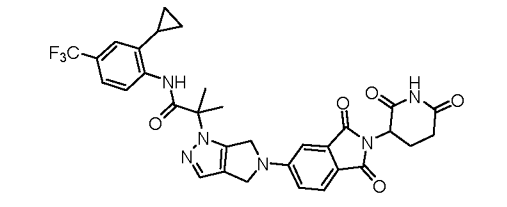 |
| 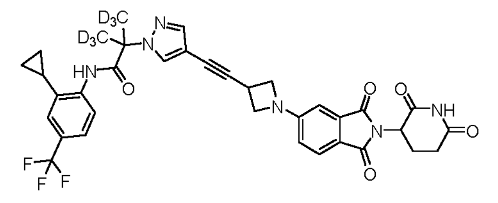 | 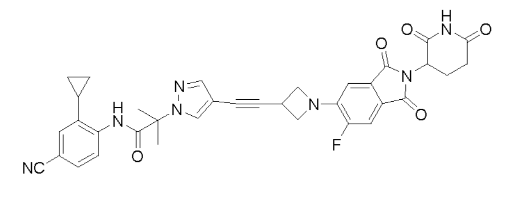 |
| 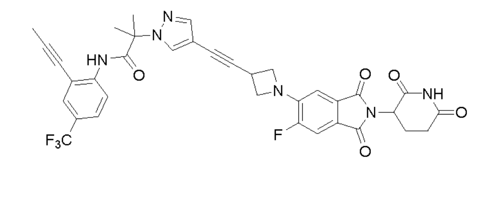 | 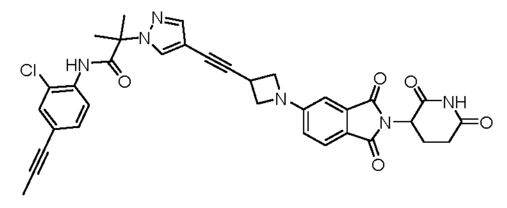 |
| 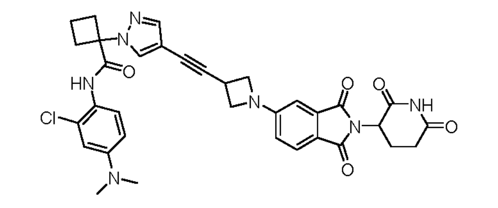 | 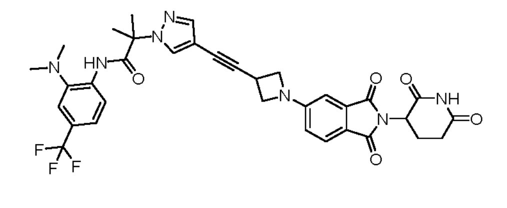 |

| | |
|---|---|
| 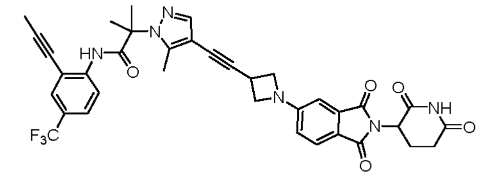 | 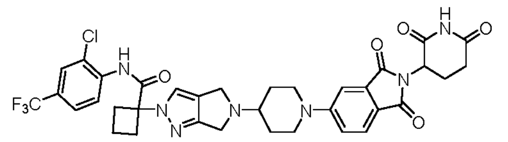 |
| 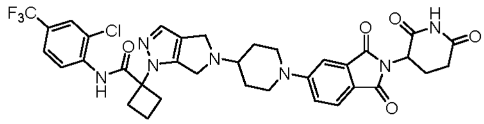 | |

Table A-1

| | |
|---|---|
| 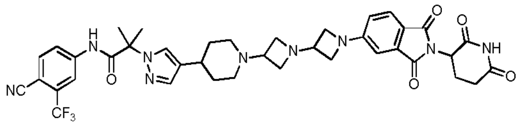 | 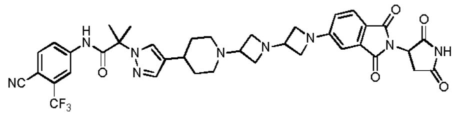 |
| 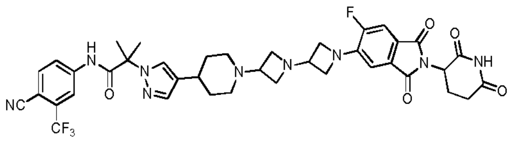 | 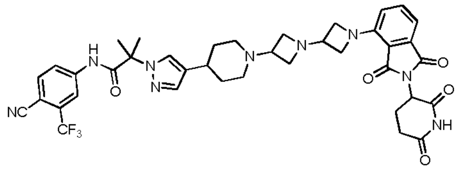 |
| 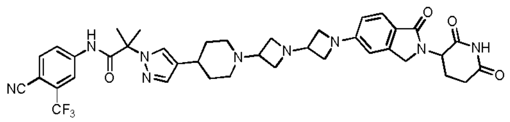 | 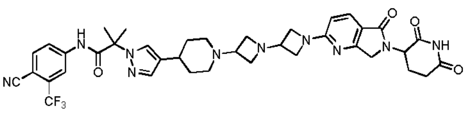 |
| 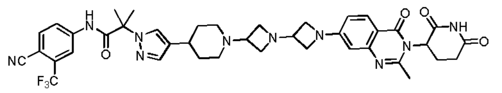 | 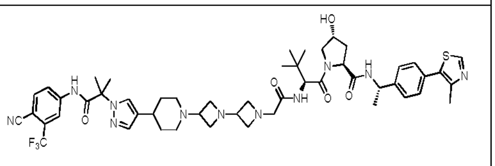 |
| 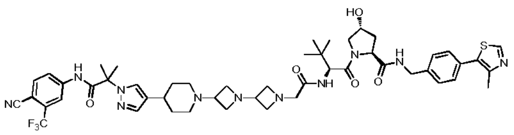 | 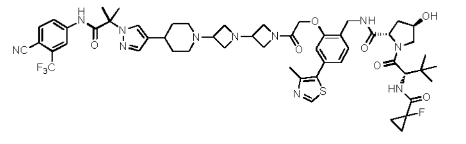 |
| 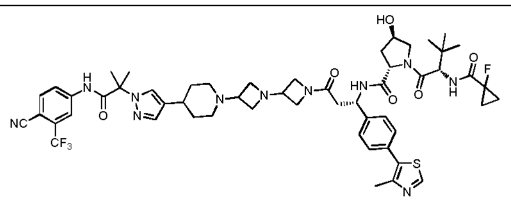 | 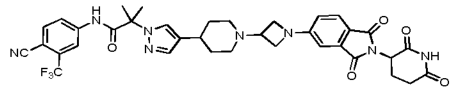 |
| 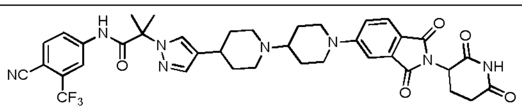 | 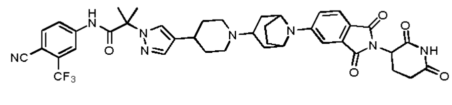 |
| 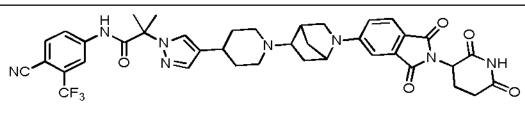 | 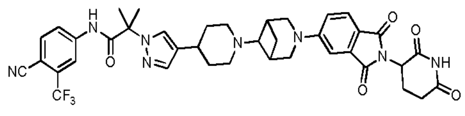 |

| | |
|---|---|
| 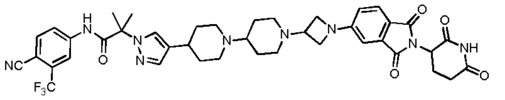 | 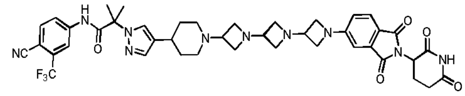 |
| 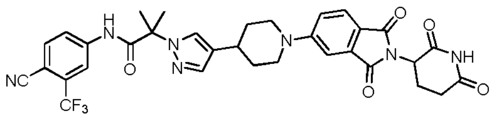 | 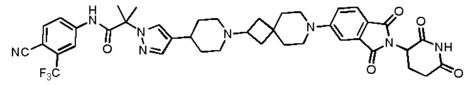 |
| 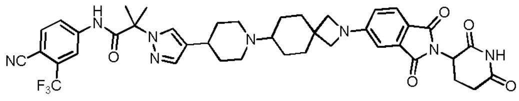 | 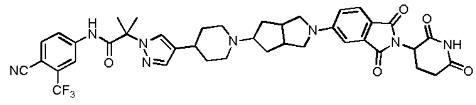 |
| 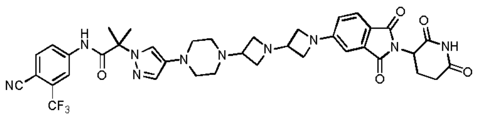 | 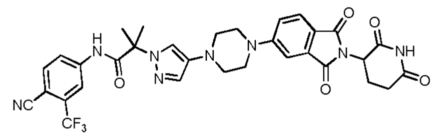 |
| 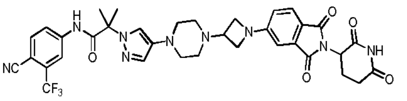 | 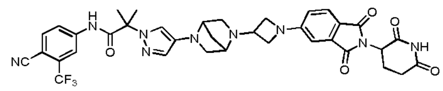 |
| 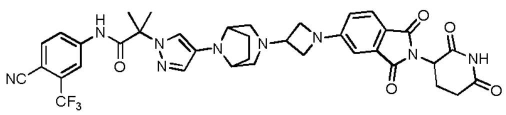 | 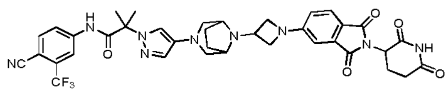 |
| 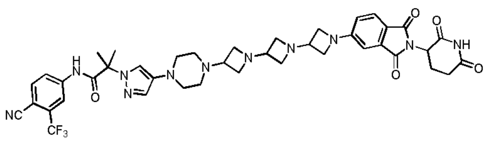 | 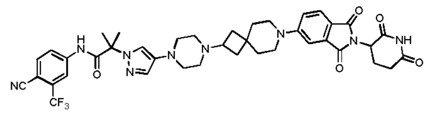 |
| 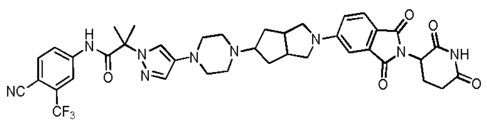 | 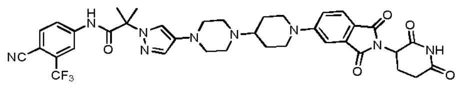 |
| 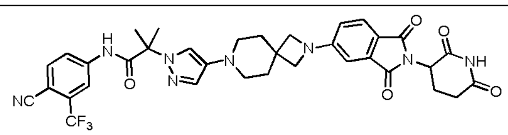 | 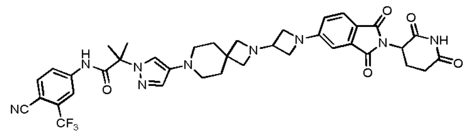 |
| 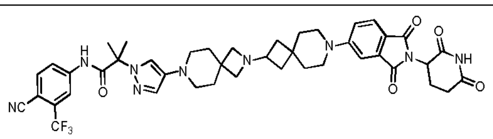 | 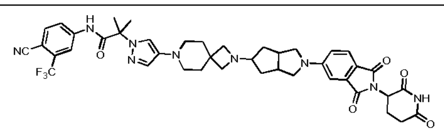 |
| 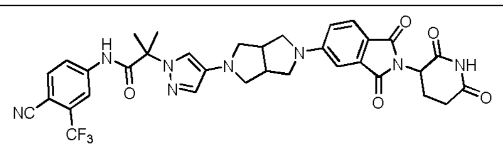 | 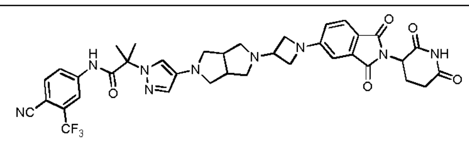 |
| 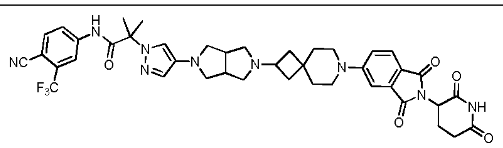 | 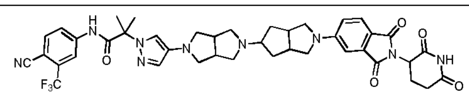 |

| | |
|---|---|
| 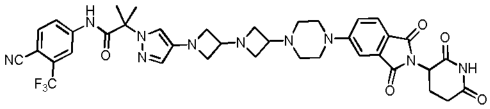 | 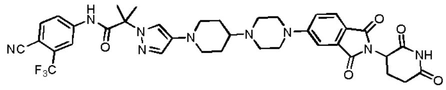 |
| 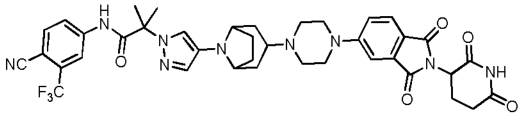 | 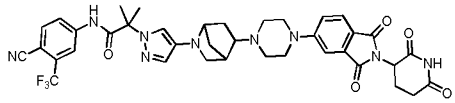 |
| 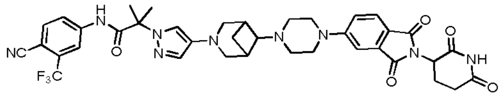 | 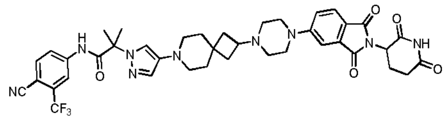 |
| 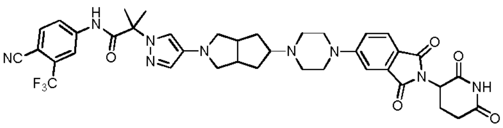 | 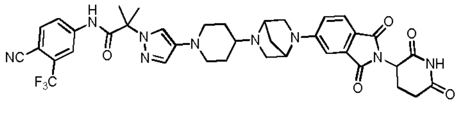 |
| 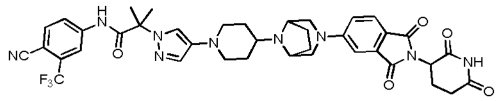 | 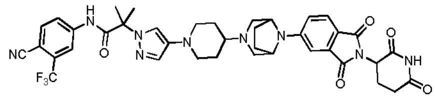 |
| 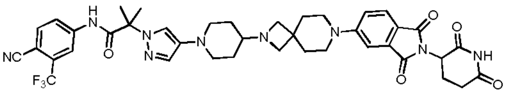 | 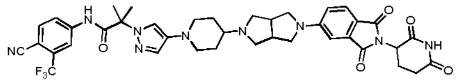 |
| 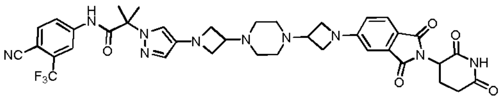 | 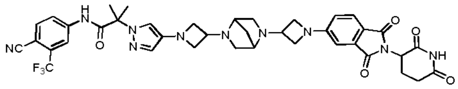 |
| 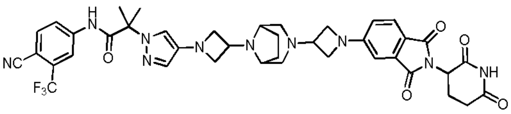 | 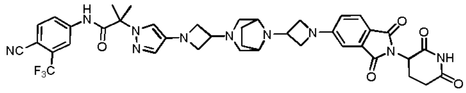 |
| 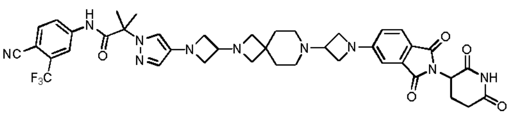 | 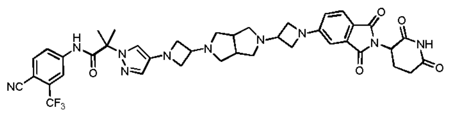 |
| 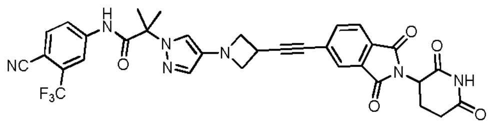 | 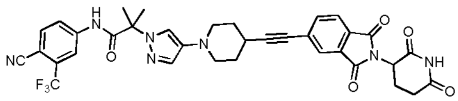 |
| 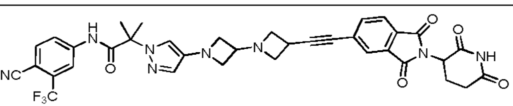 | 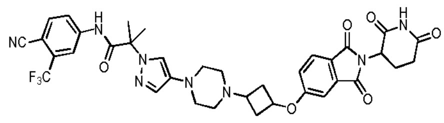 |
| 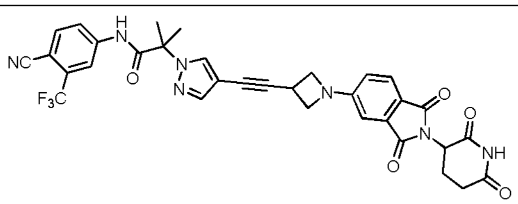 | 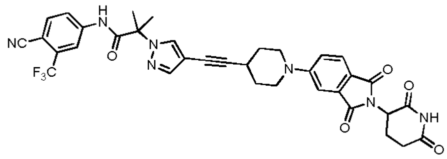 |

| | |
|---|---|
| 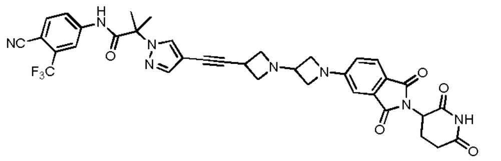 | 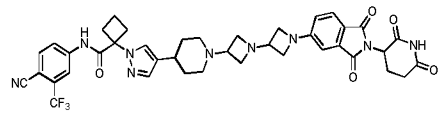 |
| 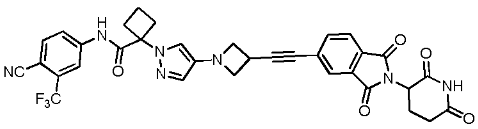 | 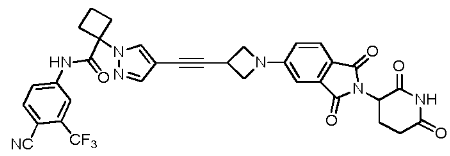 |
| 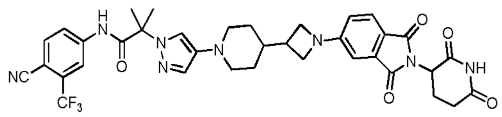 | 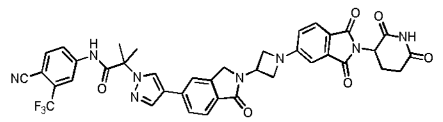 |
| 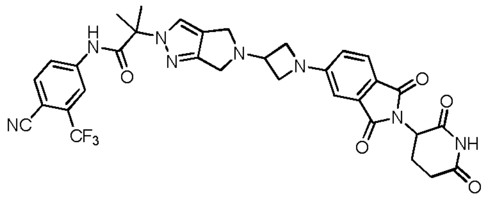 | 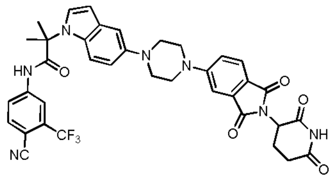 |
| 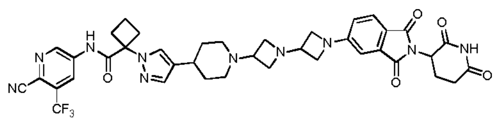 | 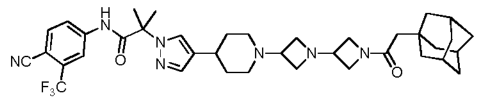 |
| 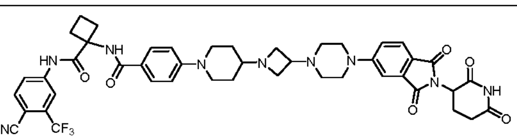 | 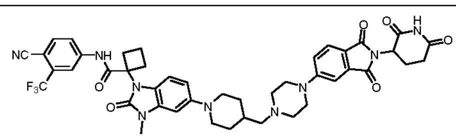 |
| 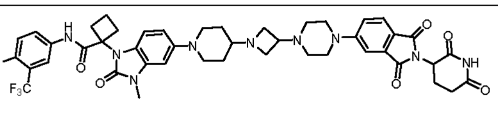 | 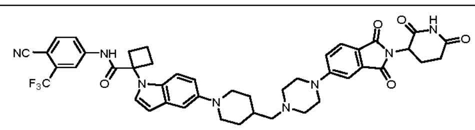 |
| 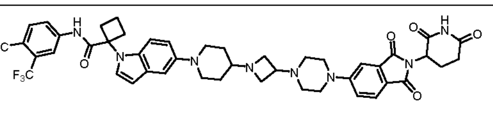 | 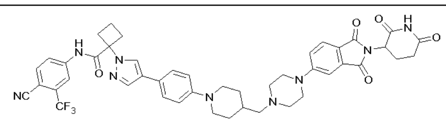 |
| 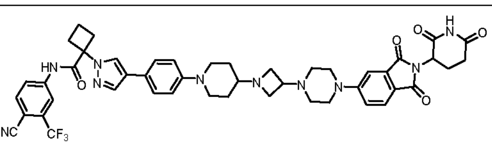 | 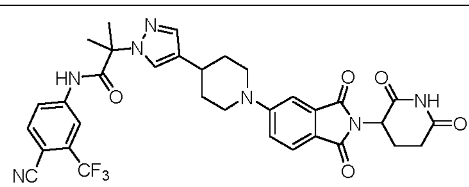 |
| 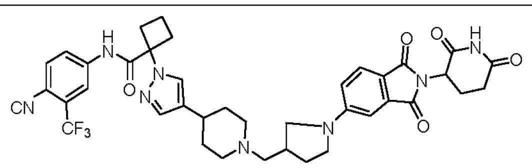 | 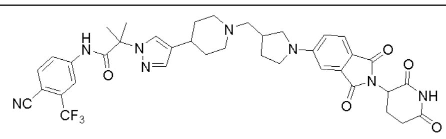 |

| | |
|---|---|
| 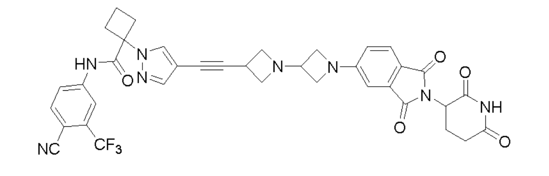 | 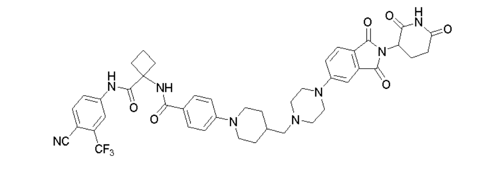 |

Table B-1 L group

| | | | |
|---|---|---|---|
| 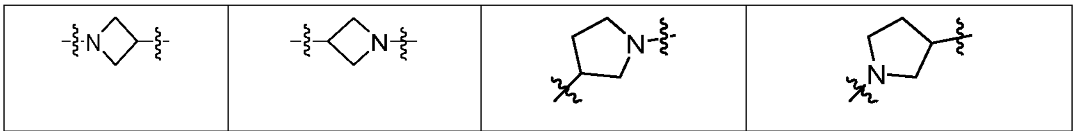 | | | |
| 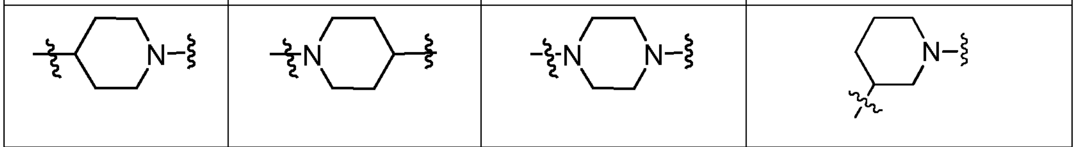 | | | |
| 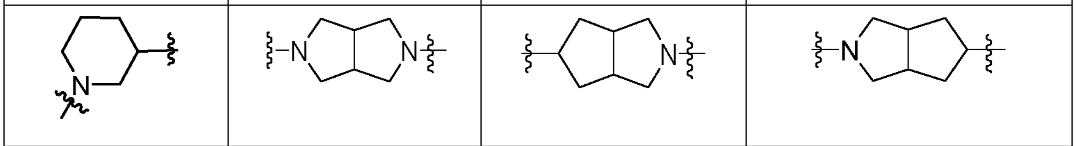 | | | |
| 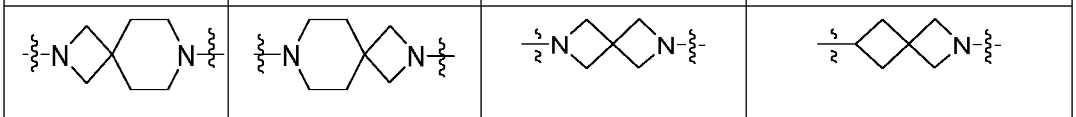 | | | |
| 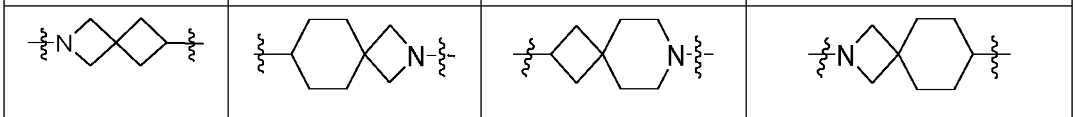 | | | |
| 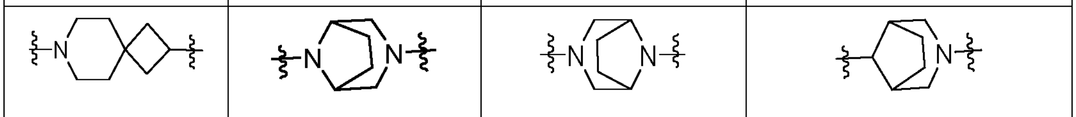 | | | |
| 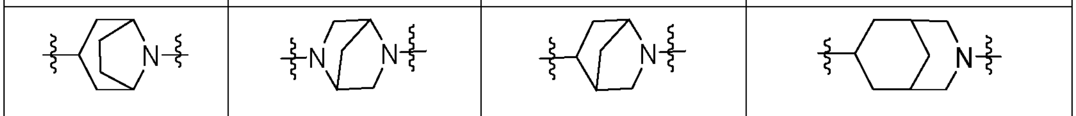 | | | |
| 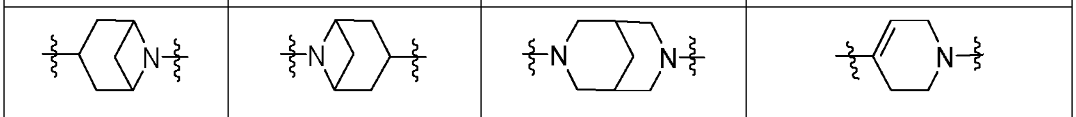 | | | |
| 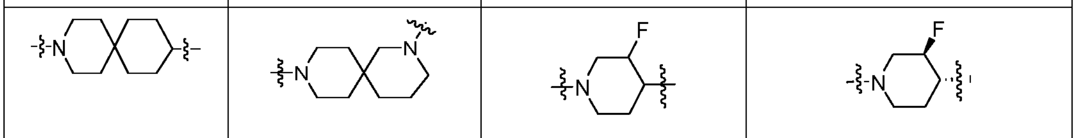 | | | |
| 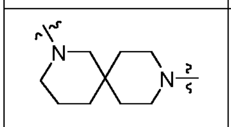 | | | |
| 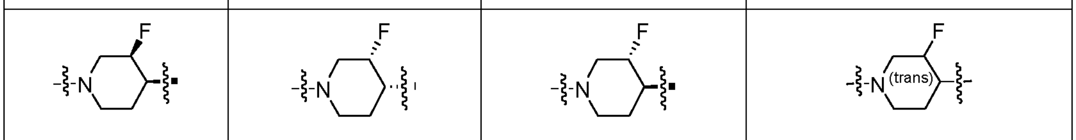 | | | |
| 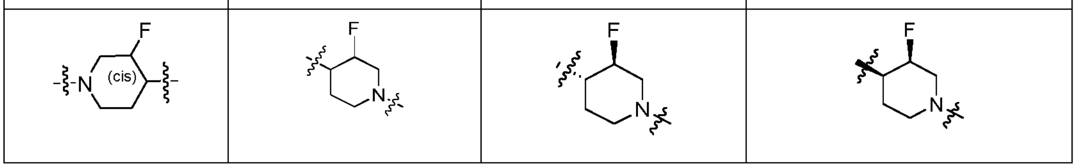 | | | |

| | | | |
|---|---|---|---|
| 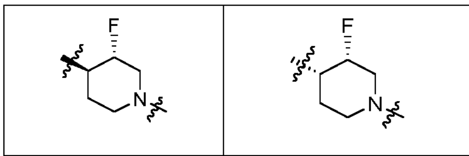 | | 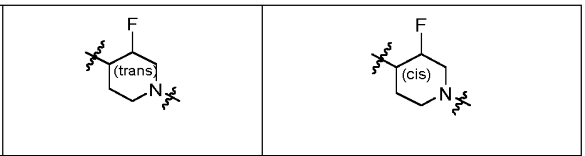 | |
| 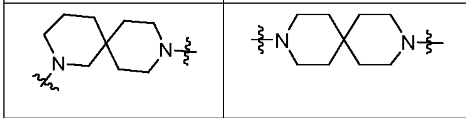 | | 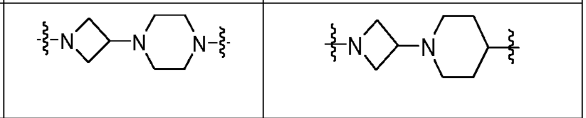 | |
| 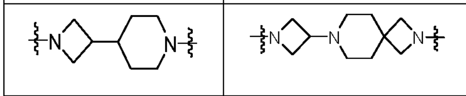 | | 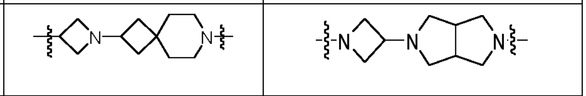 | |
| 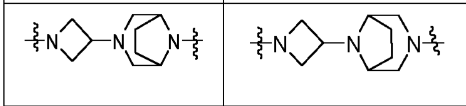 | | 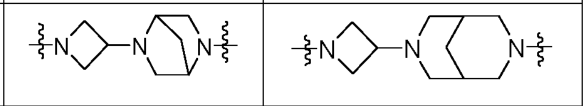 | |
| 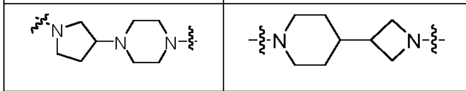 | | 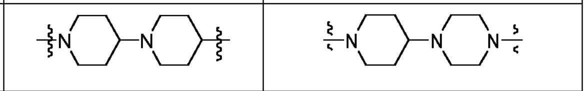 | |
| 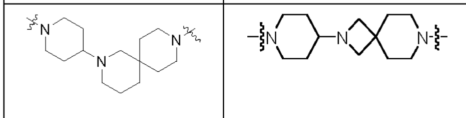 | | 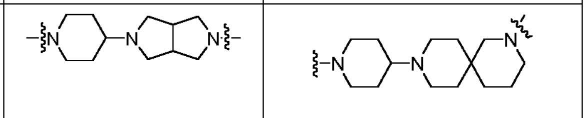 | |
| 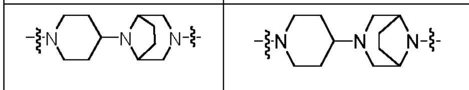 | | 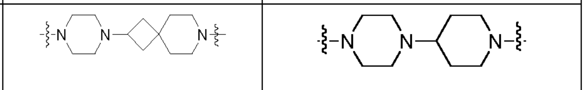 | |
| 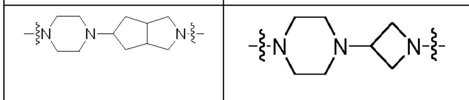 | | 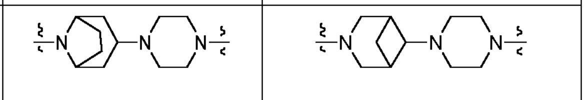 | |
| 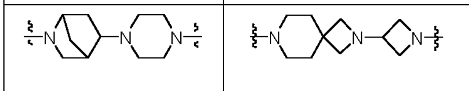 | | 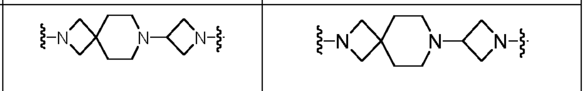 | |
| 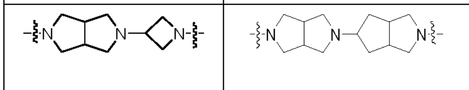 | | 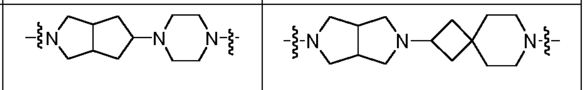 | |
| 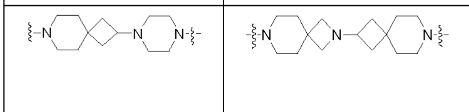 | | 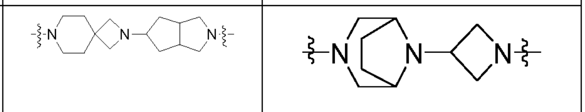 | |
| 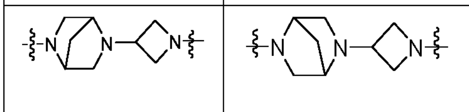 | | 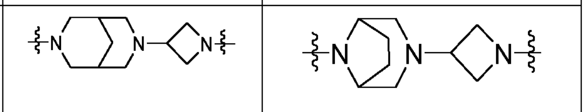 | |
| 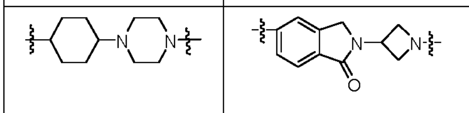 | | 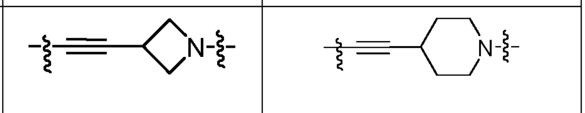 | |
| 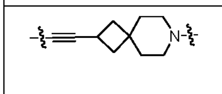 | | | 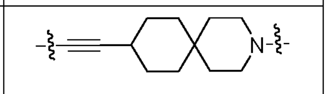 |
| 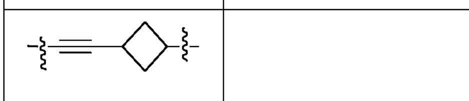 | | 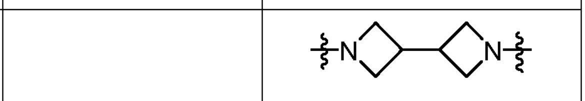 | |
| 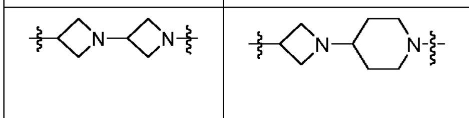 | | 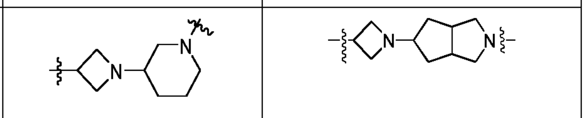 | |
| 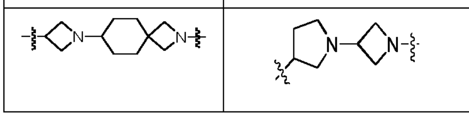 | | 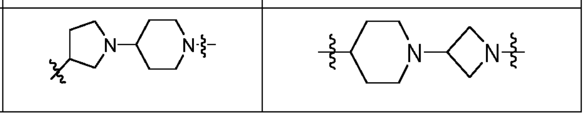 | |

| | | | |
|---|---|---|---|
| 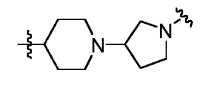 | 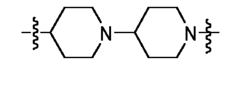 | 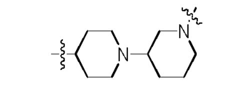 | 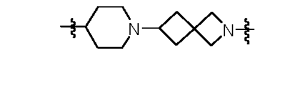 |
| 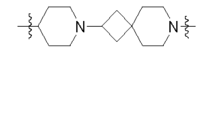 | 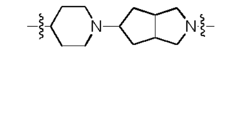 | 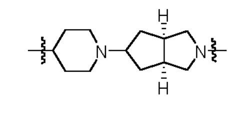 | 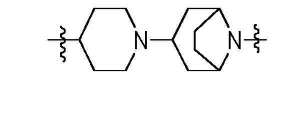 |
| 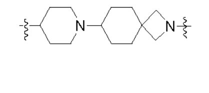 | 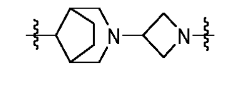 | 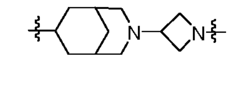 | 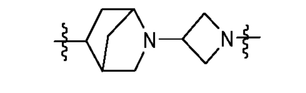 |
| 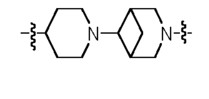 | 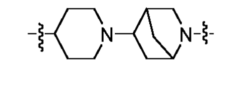 | 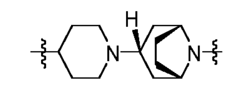 | 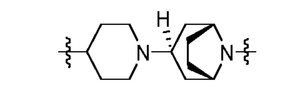 |
| 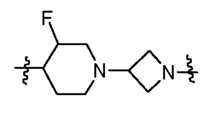 | 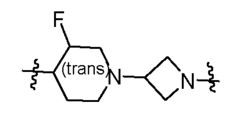 | 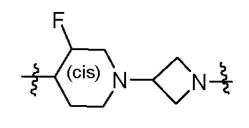 | 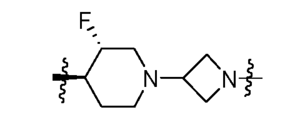 |
| 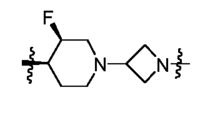 | 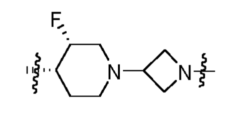 | 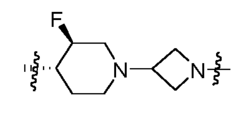 | 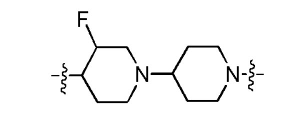 |
| 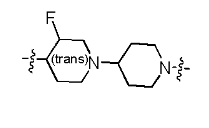 | 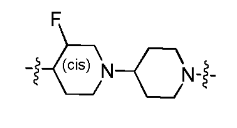 | 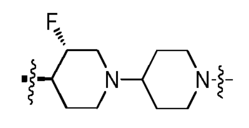 | 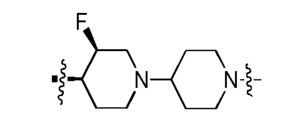 |
| 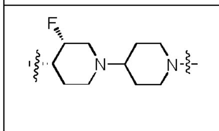 | 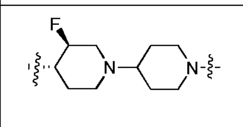 | 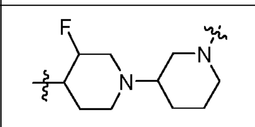 | 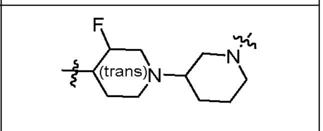 |
| 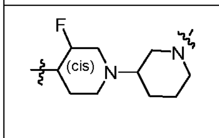 | 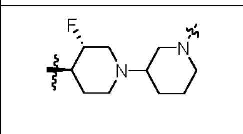 | 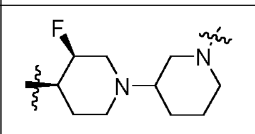 | 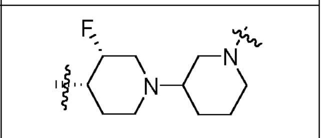 |
| 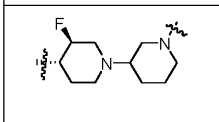 | 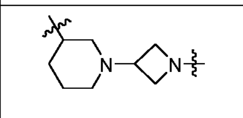 | 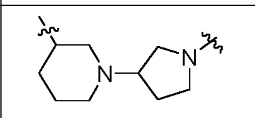 | 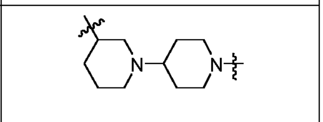 |
| | | | |
| | | | |
| | | | |
| | | | |

| | | |
|---|---|---|
| | | |

| | | |
|---|---|---|
| 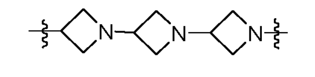 | 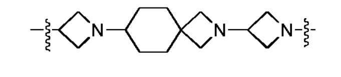 | 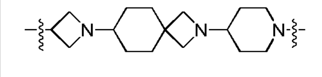 |
| 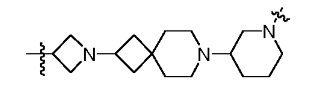 | 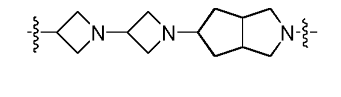 | 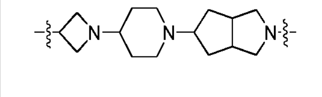 |
| 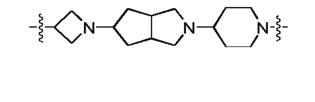 | 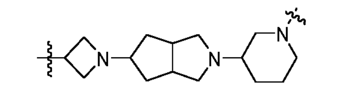 | 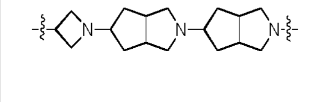 |
| 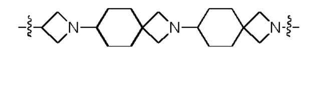 | 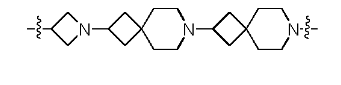 | 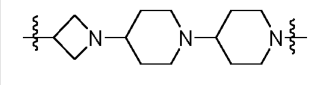 |
| 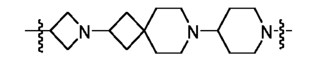 | 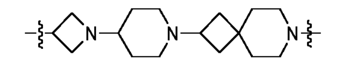 | 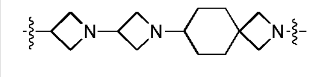 |
| 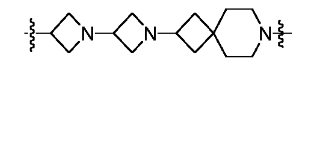 | 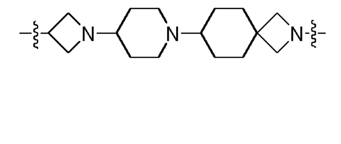 | 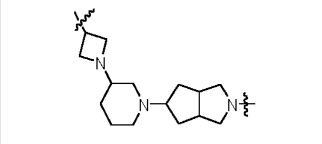 |
| 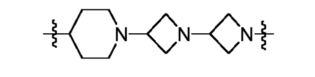 | 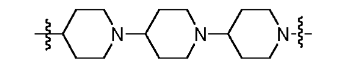 | 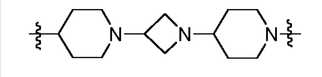 |
| 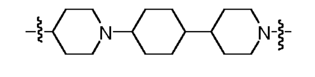 | 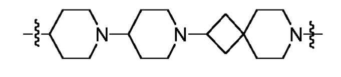 | 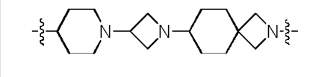 |
| 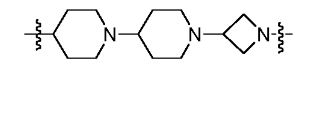 | 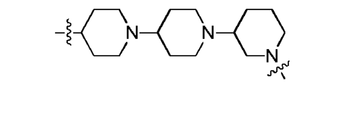 | 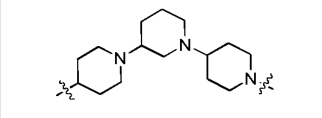 |
| 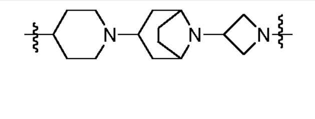 | 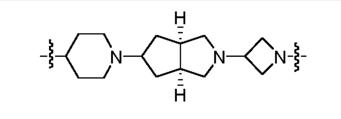 | 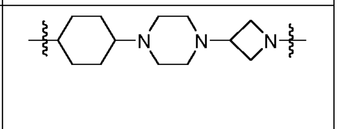 |
| 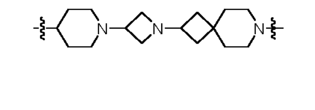 | 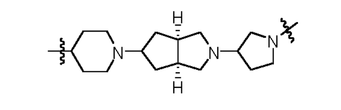 | 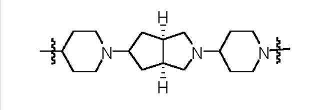 |
| 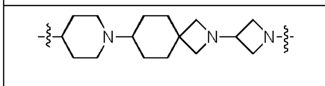 | 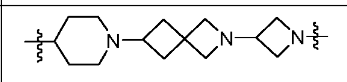 | 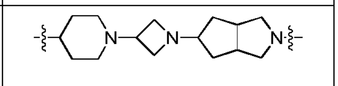 |
| 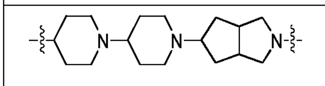 | 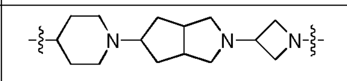 | 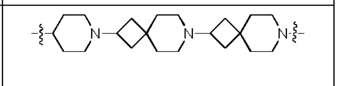 |
| 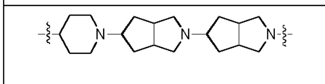 | | |
| | | |
| | | |
| | | |

| | | |
|---|---|---|
| 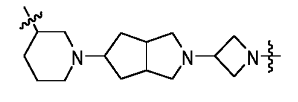 | 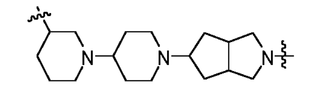 | 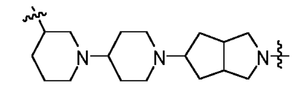 |
| 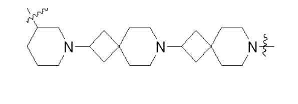 | 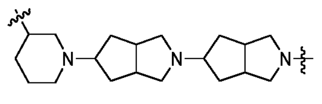 | 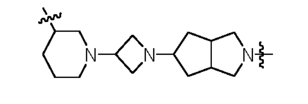 |
| 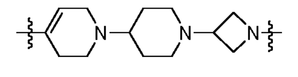 | 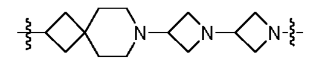 | 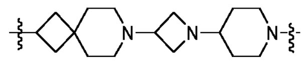 |
| 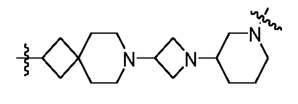 | 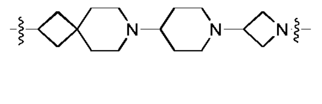 | |
| 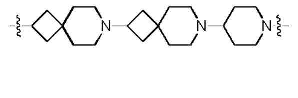 | 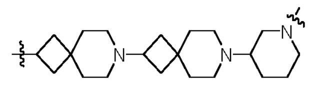 | 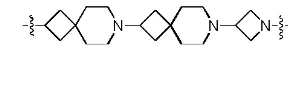 |
| 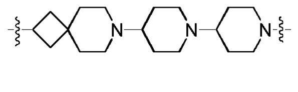 | 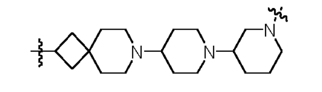 | 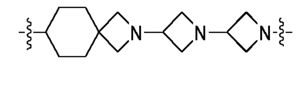 |
| 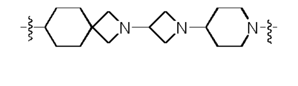 | 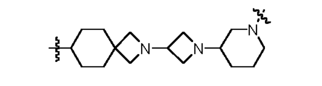 | 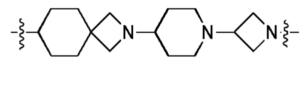 |
| 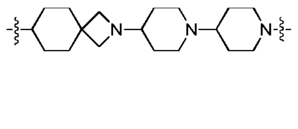 | 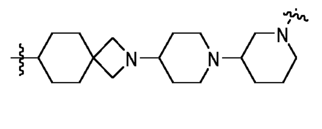 | 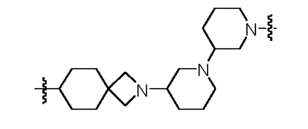 |
| 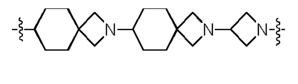 | 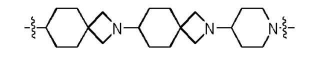 | 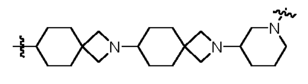 |
| 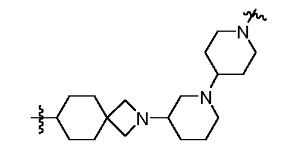 | 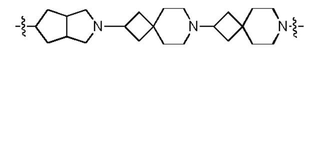 | |
| 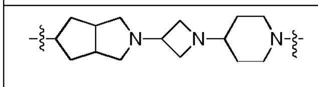 | 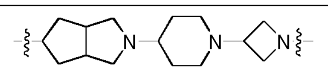 | 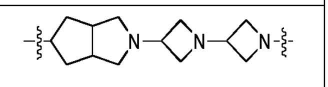 |
| 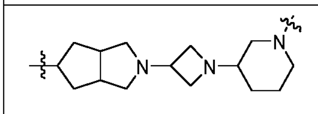 | 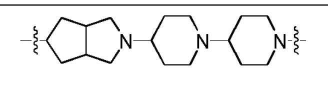 | 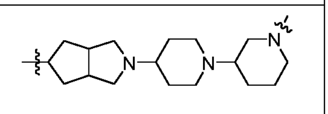 |
| 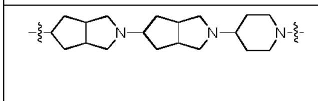 | 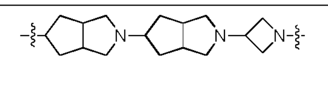 | 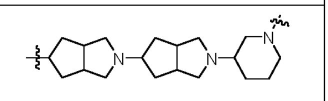 |
| 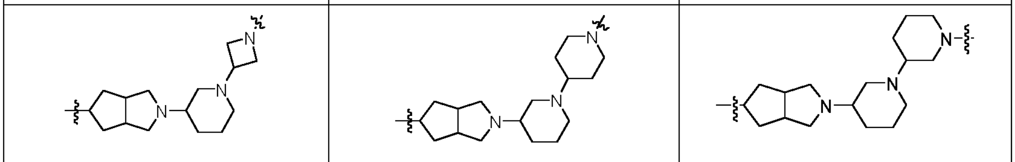 | | |
| 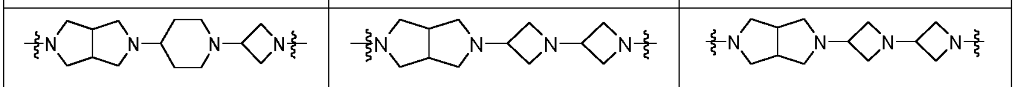 | | |
| 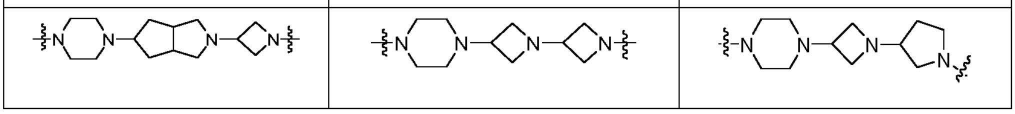 | | |

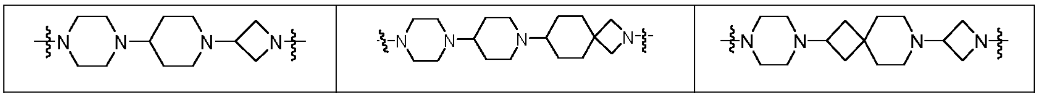
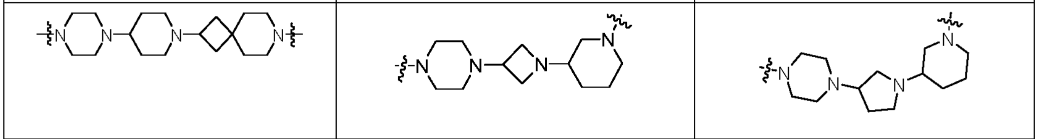
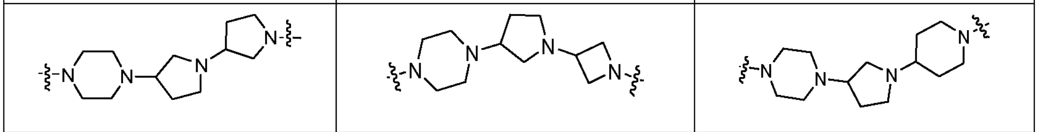
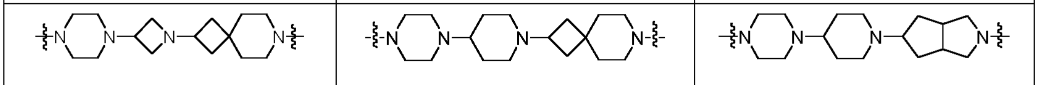
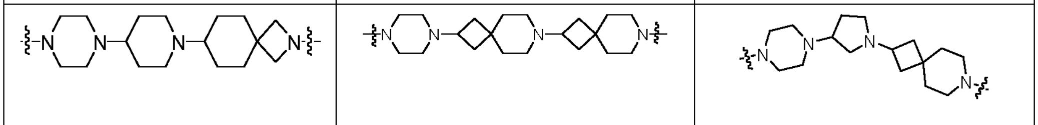
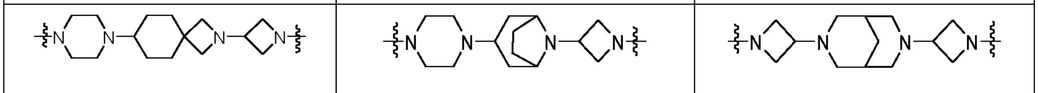
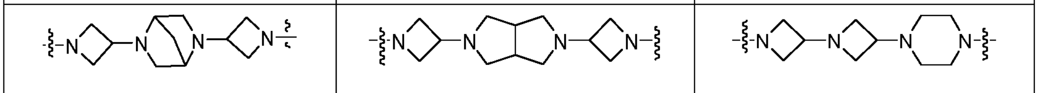
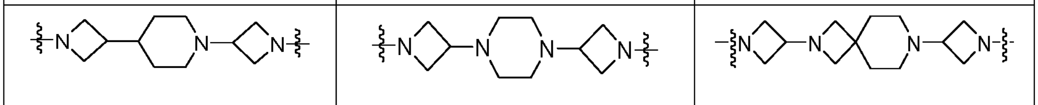
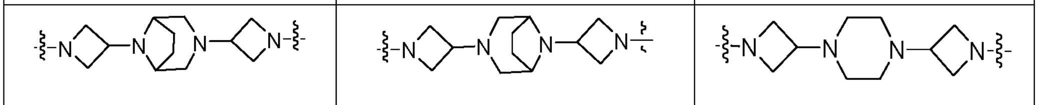
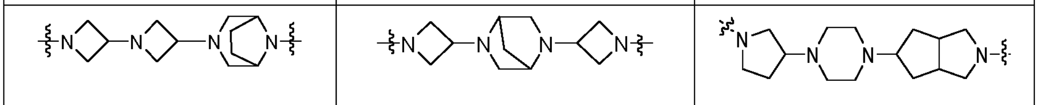
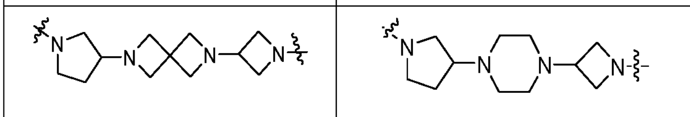
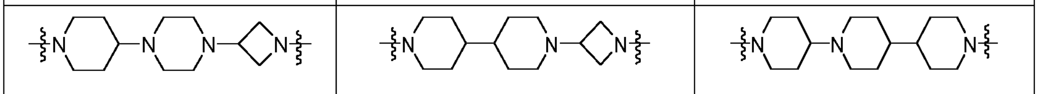
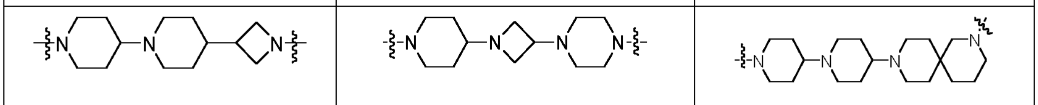
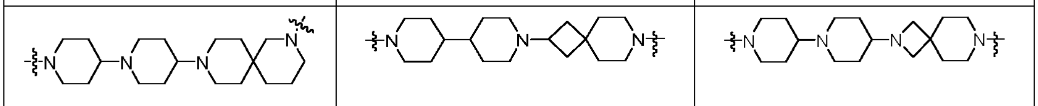
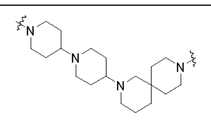
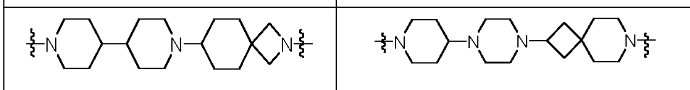
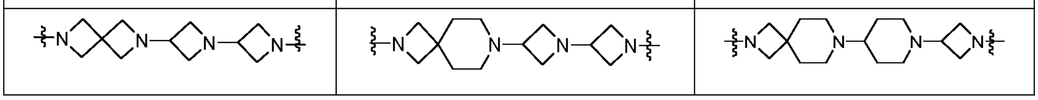

| | | |
|---|---|---|
| 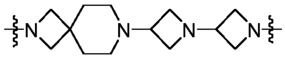 | 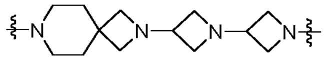 | 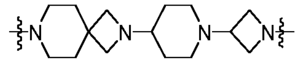 |
| 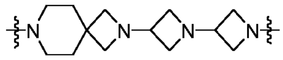 | 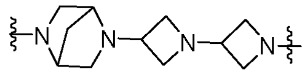 | 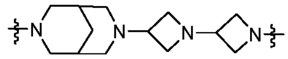 |
| 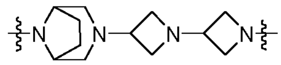 | 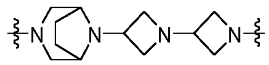 | 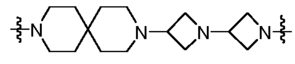 |
| 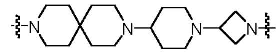 | 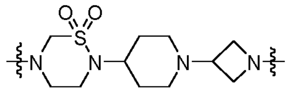 | 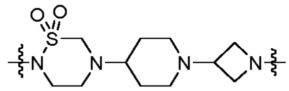 |
| 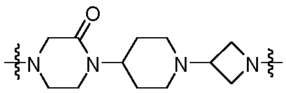 | 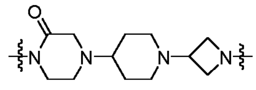 | |

| | |
|---|---|
| 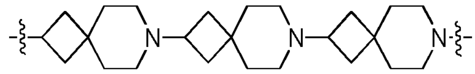 | 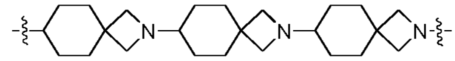 |
| 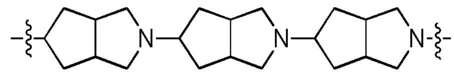 | 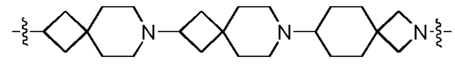 |
| 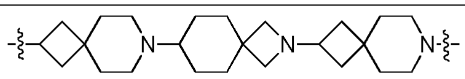 | 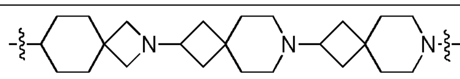 |
| 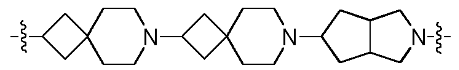 | 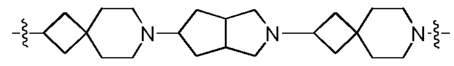 |

| | | | |
|---|---|---|---|
| 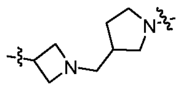 | 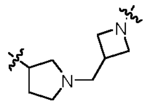 | 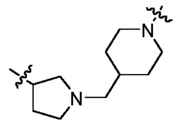 | |
| 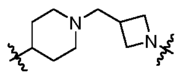 | 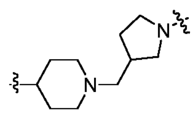 | 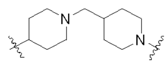 | 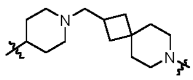 |
| 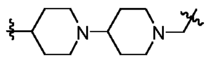 | 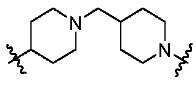 | 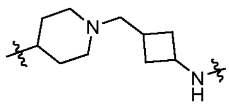 | 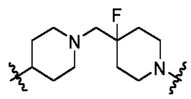 |
| 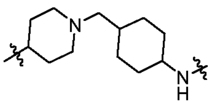 | 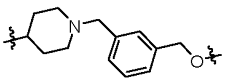 | 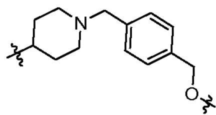 | 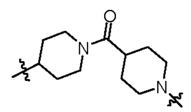 |
| 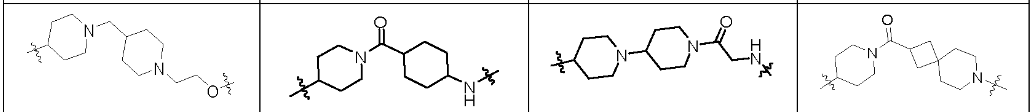 | | | |
| 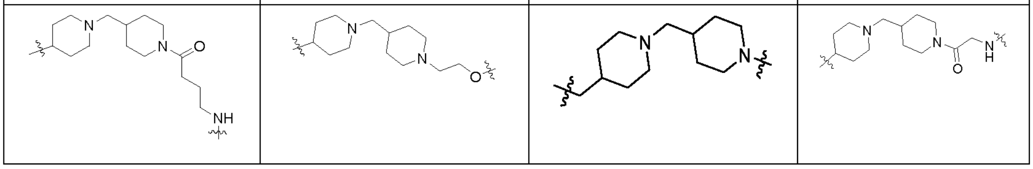 | | | |

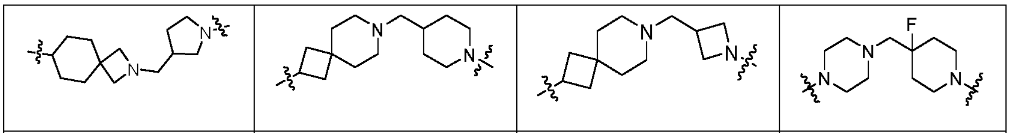

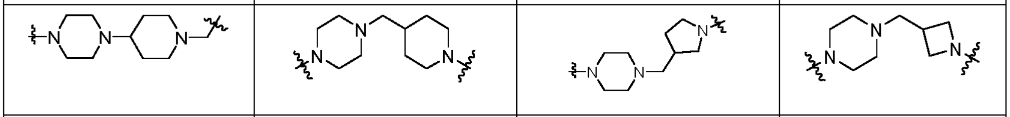

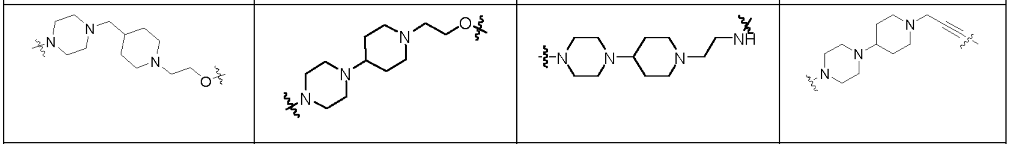

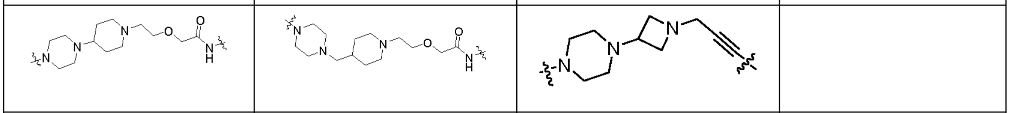

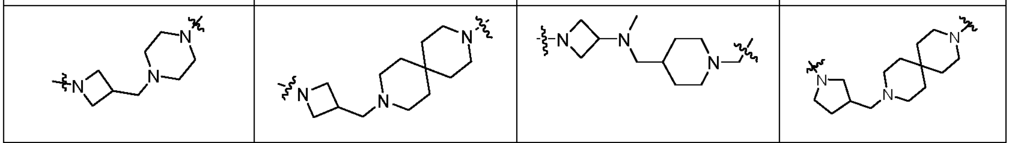

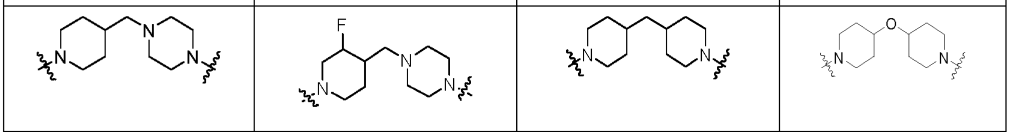

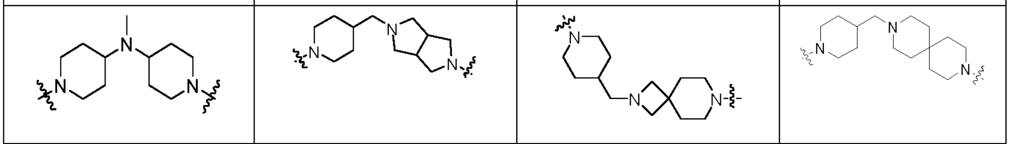

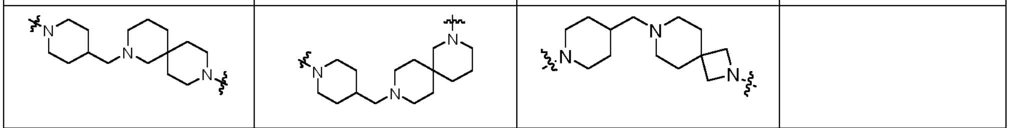

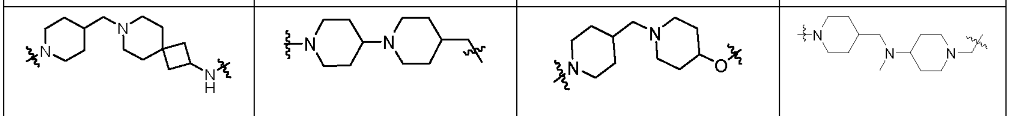

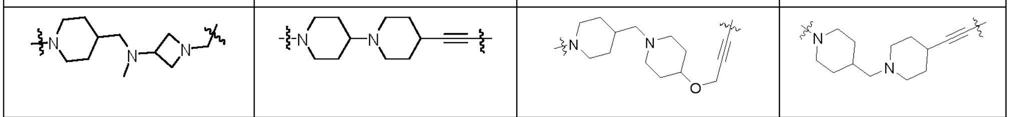

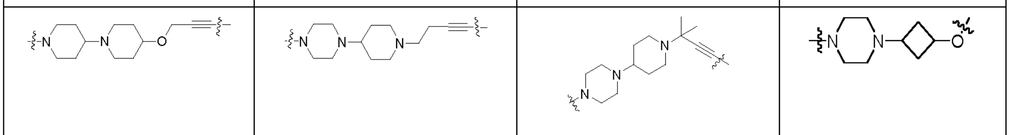

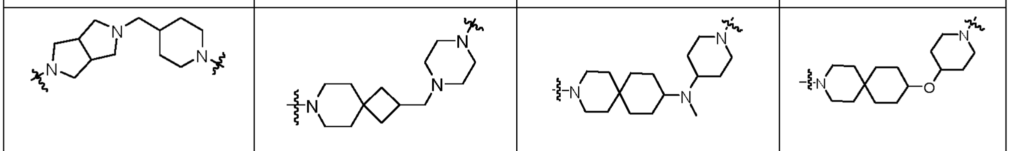

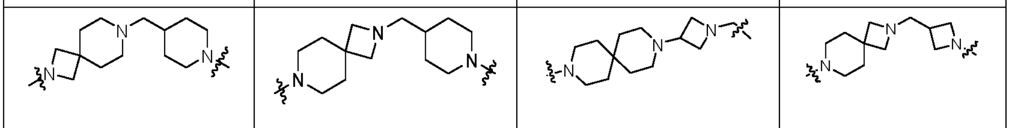

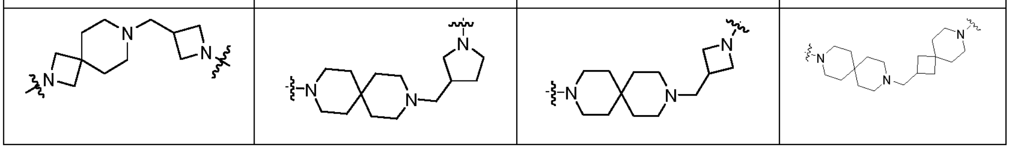

| | | | |
|---|---|---|---|
| 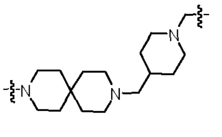 | 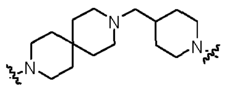 | 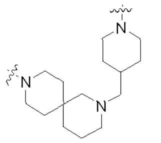 | 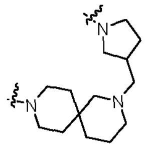 |
| 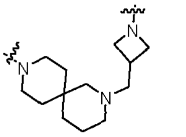 | 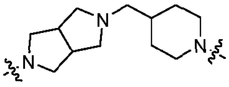 | | |
| | 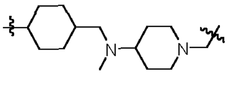 | 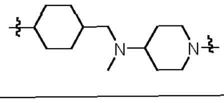 | 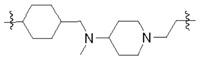 |
| 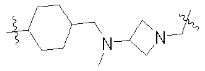 | 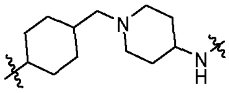 | 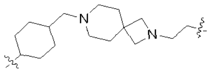 | 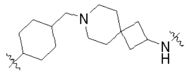 |
| 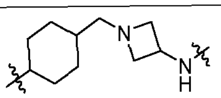 | 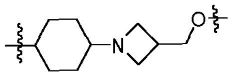 | 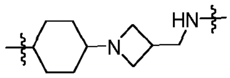 | 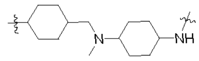 |
| 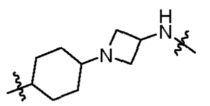 | 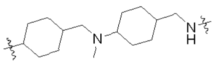 | 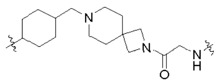 | 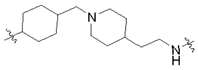 |
| 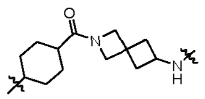 | 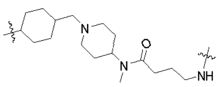 | 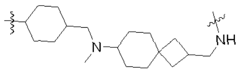 | 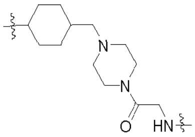 |
| 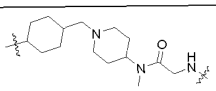 | 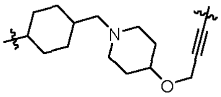 | 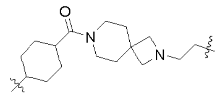 | |
| 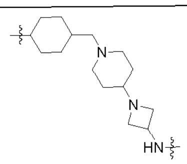 | 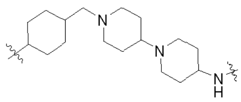 | 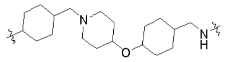 | |
| 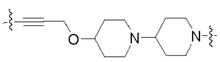 | 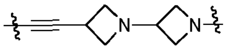 | 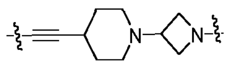 | 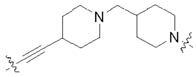 |
| 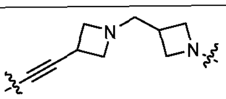 | 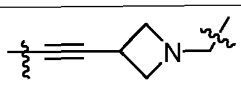 | | |
| 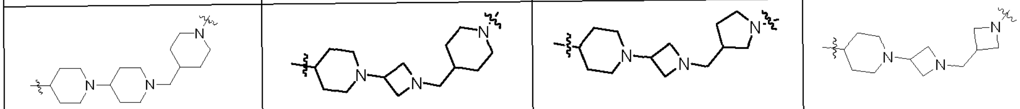 | | | |
| 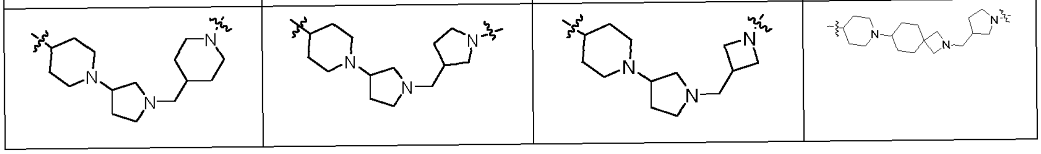 | | | |

| | | | |
|---|---|---|---|
| 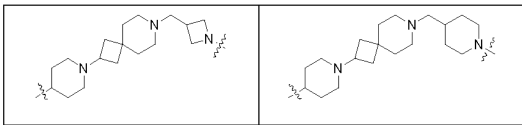 | | 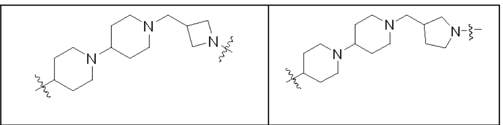 | |
| 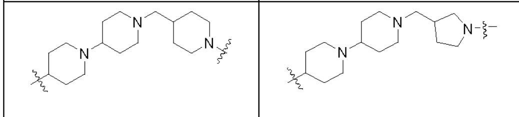 | | 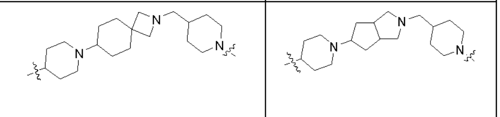 | |
| 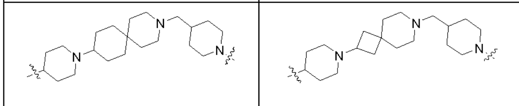 | | 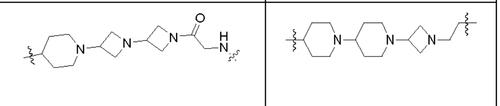 | |
| 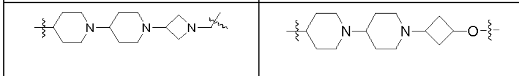 | | 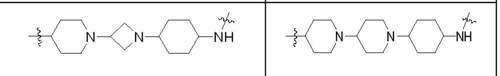 | |
| 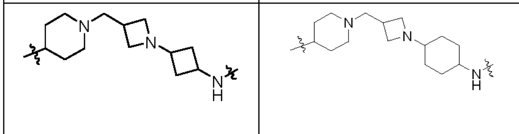 | | 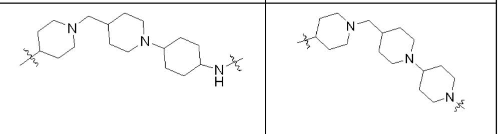 | |
| 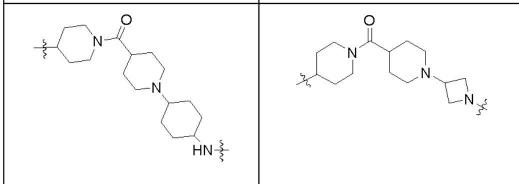 | | 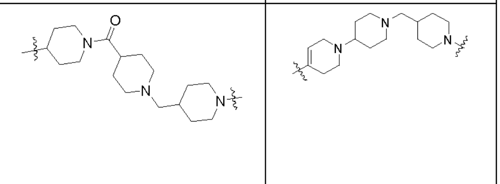 | |
| 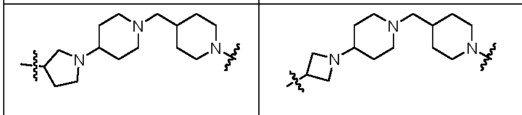 | | | |
| 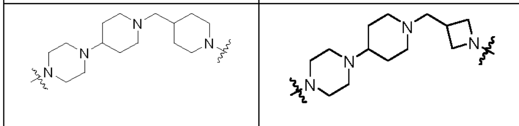 | | 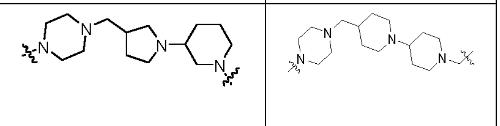 | |
| 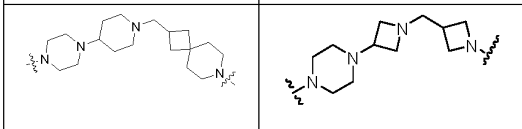 | | 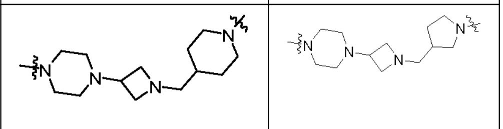 | |
| 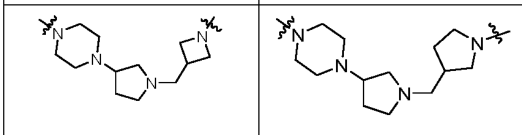 | | 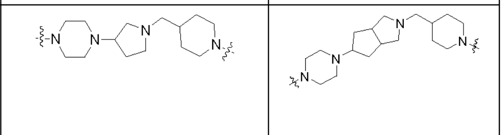 | |
| 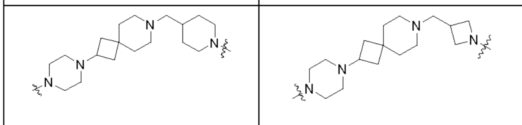 | | 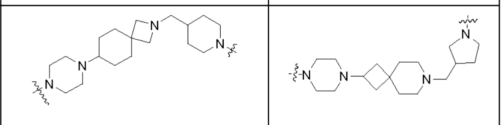 | |
| 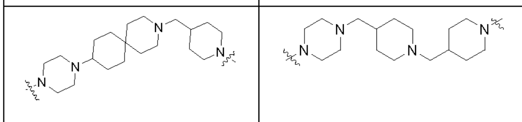 | | 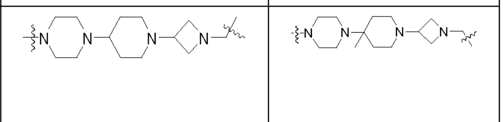 | |
| 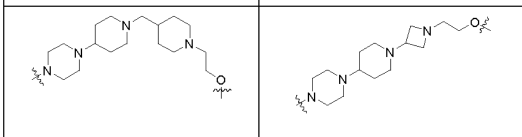 | | 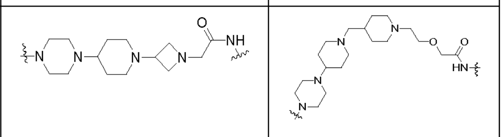 | |
| 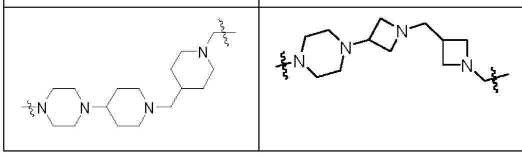 | | 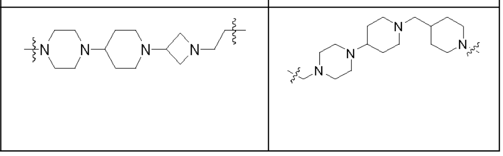 | |

| | | | |
|---|---|---|---|
| 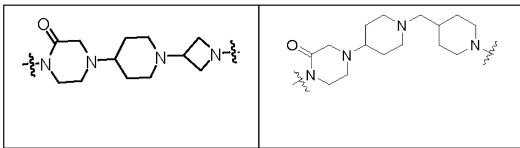 | | 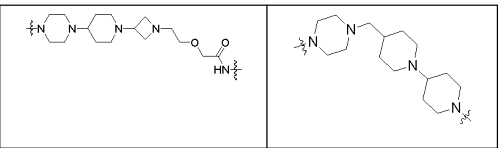 | |
| 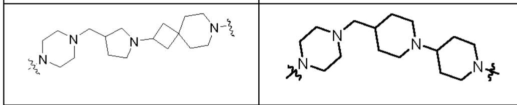 | | 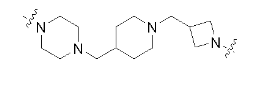 | |
| 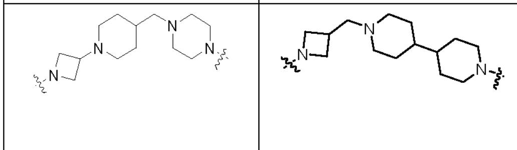 | | 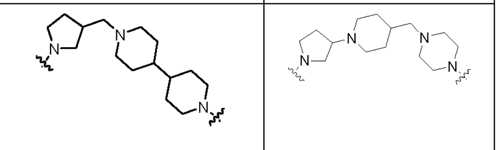 | |
| 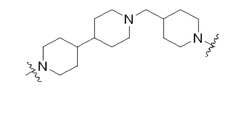 | | 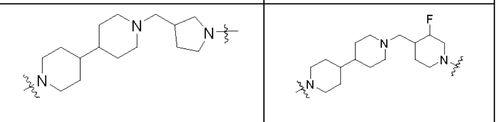 | |
| 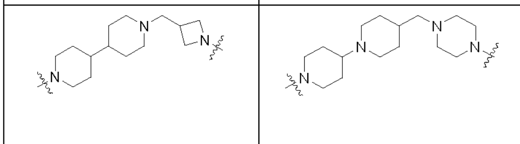 | | 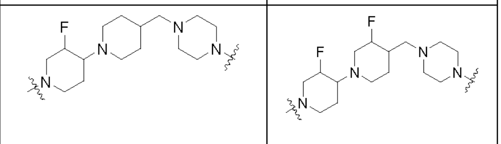 | |
| 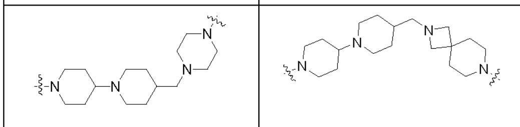 | | 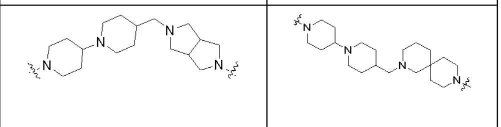 | |
| 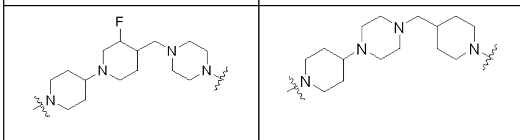 | | 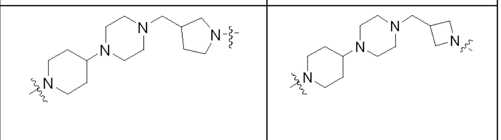 | |
| 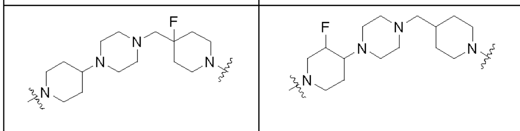 | | 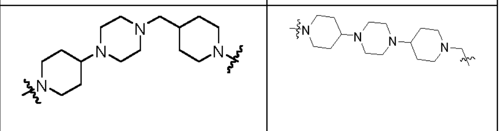 | |
| 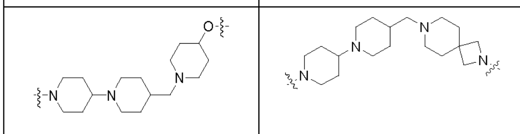 | | 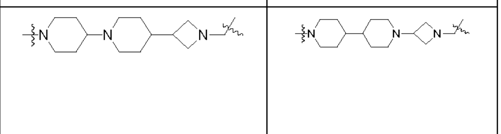 | |
| 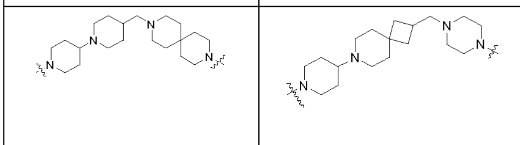 | | 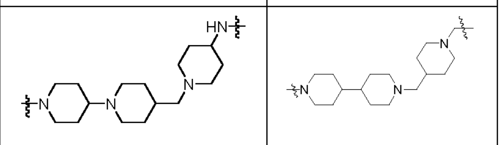 | |
| 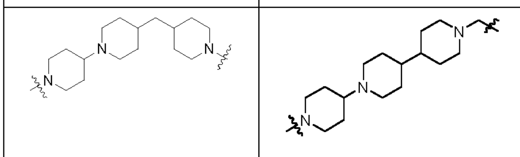 | | 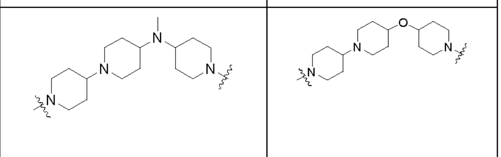 | |
| 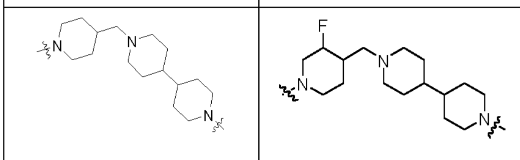 | | 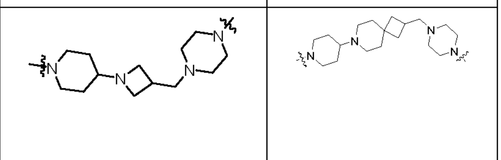 | |
| 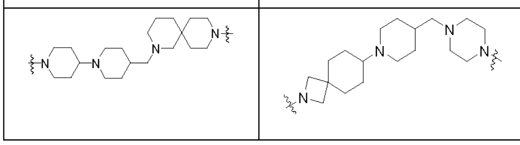 | | 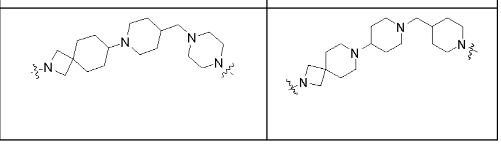 | |

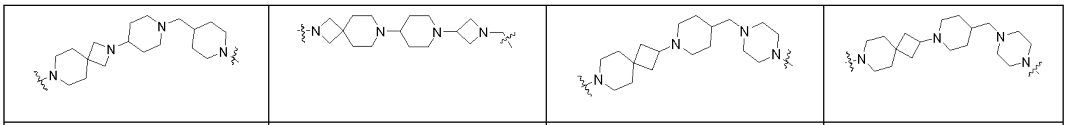
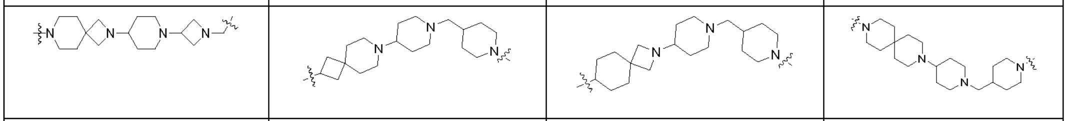
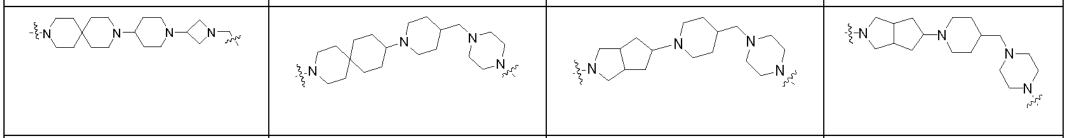
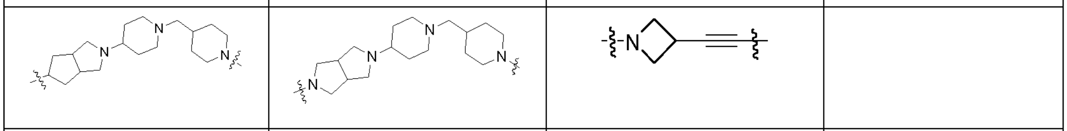
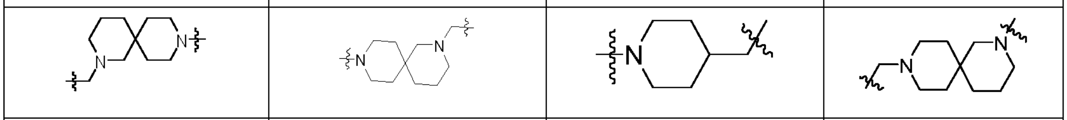
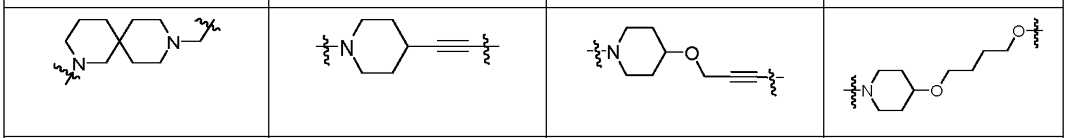
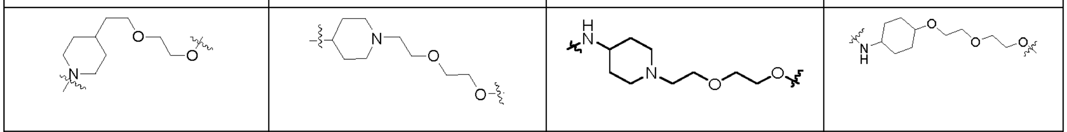
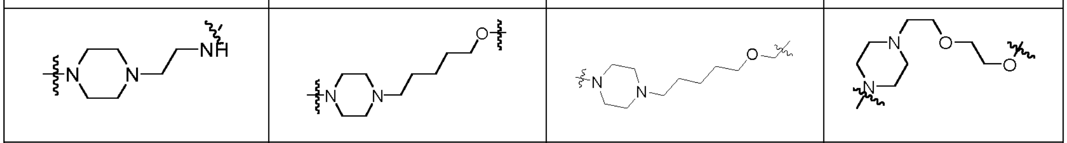
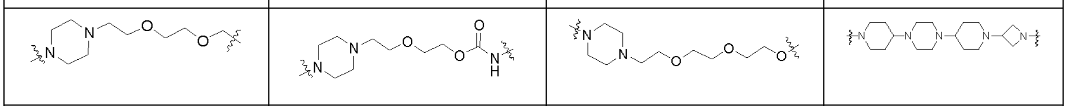
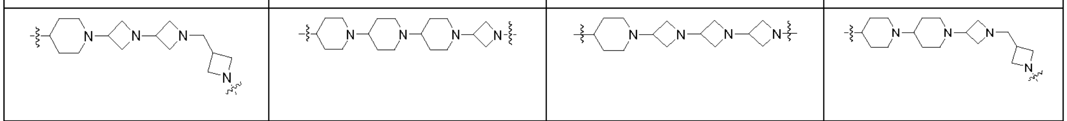
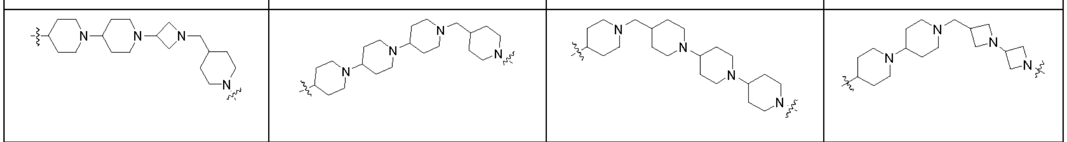
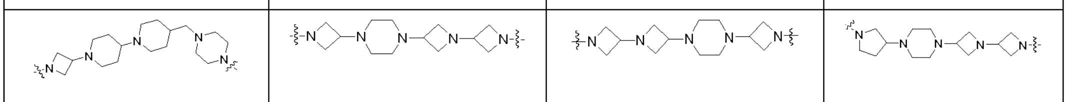
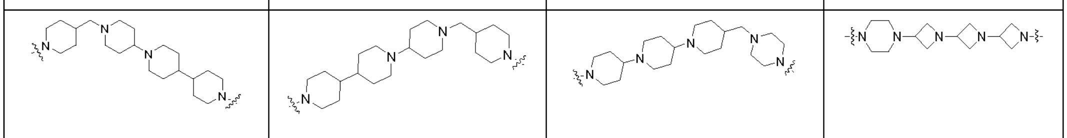
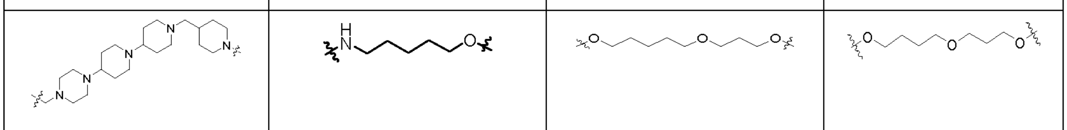
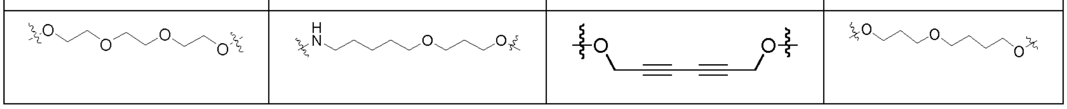

| | | | |
|---|---|---|---|
| 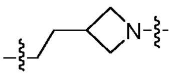 | 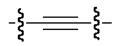 | 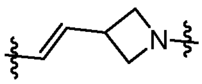 | 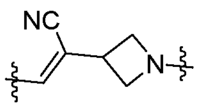 |
| 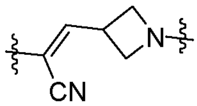 | 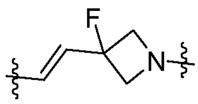 | 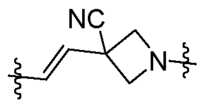 | 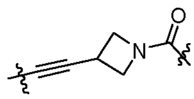 |

.

Table B-2 L group

| | | |
|---|---|---|
| 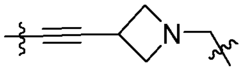 | 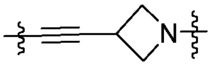 | 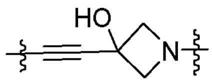 |
| 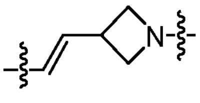 | 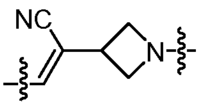 | 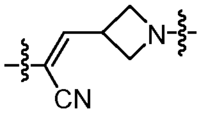 |
| 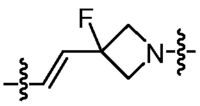 | 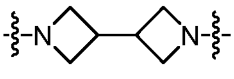 | 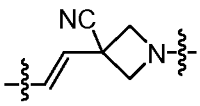 |
| 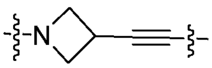 | 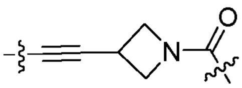 | 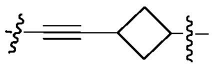 |
| 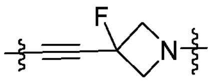 | 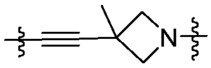 | 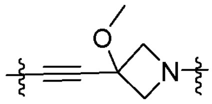 |
| 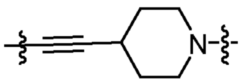 | 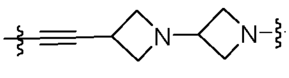 | 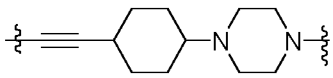 |
| 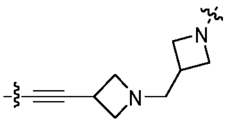 | 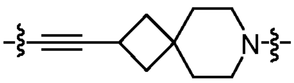 | 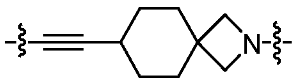 |
| 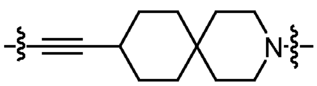 | 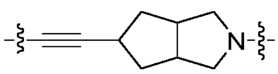 | 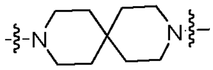 |
| 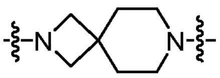 | 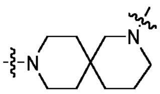 | 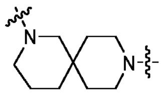 |
| 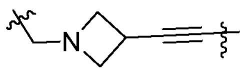 | 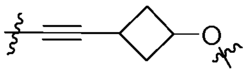 | 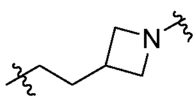 |
| 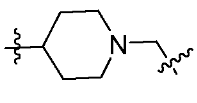 | 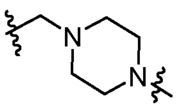 | |

| | | |
|---|---|---|
| 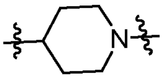 | 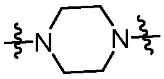 | 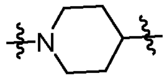 |
| 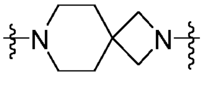 | 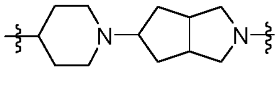 | 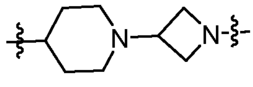 |
| 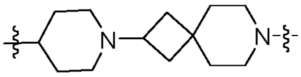 | 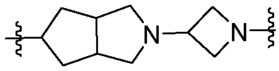 | 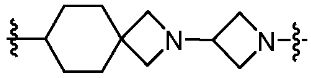 |
| | 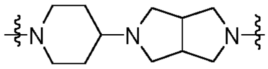 | 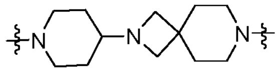 |
| 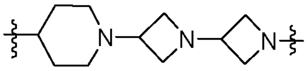 | 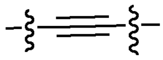 | -NH- |
| 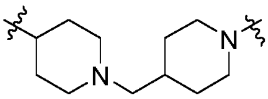 | 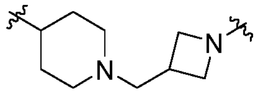 | 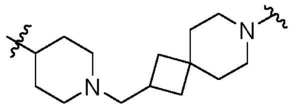 |
| 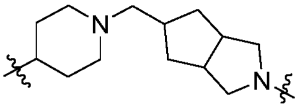 | 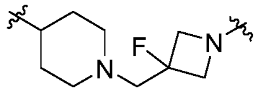 | 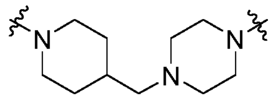 |
| 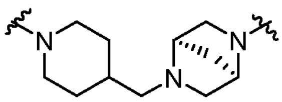 | 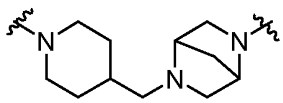 | 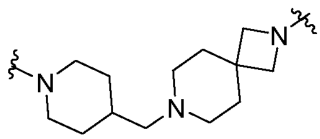 |
| 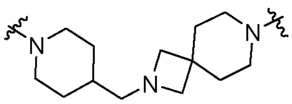 | 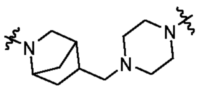 | 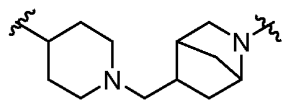 |
| 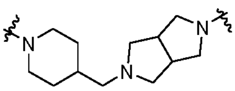 | 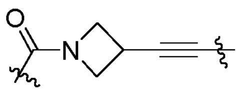 | 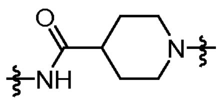 |
| 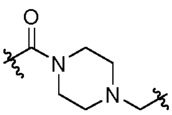 | 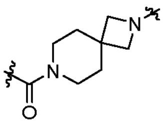 | 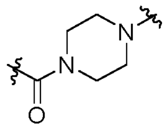 |
| 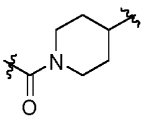 | 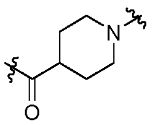 | 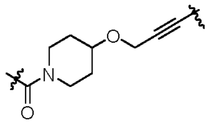 |
| 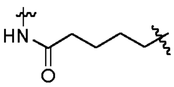 | 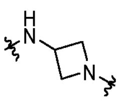 | $-CH_2-$ |
| 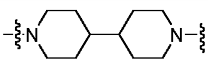 | 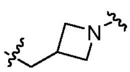 | 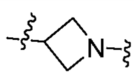 |

The present invention relates to a pharmaceutical composition, comprising the above-mentioned compound in the present invention or the stereoisomer, deuterated compound, solvate, prodrug, metabolite, pharmaceutically acceptable salt or co-crystal thereof, and a pharmaceutically acceptable carrier.

The present invention relates to a pharmaceutical composition, comprising a therapeutically effective amount of the above-mentioned compound in the present invention or the stereoisomer, deuterated compound, solvate, prodrug, metabolite, pharmaceutically acceptable salt or co-crystal thereof, and a pharmaceutically acceptable carrier.

In some embodiments, the pharmaceutical composition of the present invention can be in a unit preparation form (the amount of the active drug in the unit preparation is also referred to as the "preparation specification").

The term "effective amount" or "therapeutically effective amount" according to the present application refers to a sufficient amount of the compound disclosed in the present application that is administered to ameliorate, to some extent, one or more symptoms of a disease or condition being treated (for example, a disease related to the inhibition or degradation of AR or AR splice mutants, such as prostate cancer). In some embodiments, the outcome is the reduction and/or remission of signs, symptoms or causes of the disease, or any other desired change in the biological system. For example, an "effective amount" in terms of the therapeutic use is an amount of the composition comprising the compound disclosed in the present application that is required to provide clinically significant reduction of the symptoms of the disease. Examples of the therapeutically effective amount include, but are not limited to, 1-1000 mg, 1-900 mg, 1-800 mg, 1-700 mg, 1-600 mg, 2-600 mg, 3-600 mg, 4-600 mg, 5-600 mg, 6-600 mg, 10-600 mg, 20-600 mg, 25-600 mg, 30-600 mg, 40-600 mg, 50-600 mg, 60-600 mg, 70-600 mg, 75-600 mg, 80-600 mg, 90-600 mg, 100-600 mg, 200-600 mg, 1-500 mg, 2-500 mg, 3-500 mg, 4-500 mg, 5-500 mg, 6-500 mg, 10-500 mg, 20-500 mg, 25-500 mg, 30-500 mg, 40-500 mg, 50-500 mg, 60-500 mg, 70-500 mg, 75-500 mg, 80-500 mg, 90-500 mg, 100-500 mg, 125-500 mg, 150-500 mg, 200-500 mg, 250-500 mg, 300-500 mg, 400-500 mg, 5-400 mg, 10-400 mg, 20-400 mg, 25-400 mg, 30-400 mg, 40-400 mg, 50-400 mg, 60-400 mg, 70-400 mg, 75-400 mg, 80-400 mg, 90-400 mg, 100-400 mg, 125-400 mg, 150-400 mg, 200-400 mg, 250-400 mg, 300-400 mg, 1-300 mg, 2-300 mg, 5-300 mg, 10-300 mg, 20-300 mg, 25-300 mg, 30-300 mg, 40-300 mg, 50-300 mg, 60-300 mg, 70-300 mg, 75-300 mg, 80-300 mg, 90-300 mg, 100-300 mg, 125-300 mg, 150-300 mg, 200-300 mg, 250-300 mg, 1-200 mg, 2-200 mg, 5-200 mg, 10-200 mg, 20-200 mg, 25-200 mg, 30-200 mg, 40-200 mg, 50-200 mg, 60-200 mg, 70-200 mg, 75-200 mg, 80-200 mg, 90-200 mg, 100-200 mg, 125-200 mg, 150-200 mg, 80-1000 mg, or 80-800 mg.

In some embodiments, the pharmaceutical composition comprises the compound or the stereoisomer, deuterated compound, solvate, prodrug, metabolite, pharmaceutically acceptable salt or co-crystal thereof according to the present invention in an amount including but not limited to 1-1000 mg, 20-800 mg, 40-800 mg, 40-400 mg, 25-200 mg, 1 mg, 5 mg, 10 mg, 15 mg, 20 mg, 25 mg, 30 mg, 35 mg, 40 mg, 45 mg, 50 mg, 55 mg, 65 mg, 70 mg, 75 mg, 80 mg, 85 mg, 90 mg, 95 mg, 100 mg, 110 mg, 120 mg, 125 mg, 130 mg, 140 mg, 150 mg, 160 mg, 170 mg, 180 mg, 190 mg, 200 mg, 210 mg, 220 mg, 230 mg, 240 mg, 250 mg, 300 mg, 320 mg, 400 mg, 480 mg, 500 mg, 600 mg, 640 mg, or 840 mg.

A method for treating a disease in a mammal, the method comprises administering to a subject a therapeutically effective amount of the compound or the stereoisomer, deuterated compound, solvate, prodrug, metabolite, pharmaceutically acceptable salt or co-crystal thereof according to the present invention, wherein the therapeutically effective amount is preferably 1-1000 mg, and the disease is preferably a disease related to the inhibition or degradation of AR or AR splice mutants, such as prostate cancer.

A method for treating a disease in a mammal, the method comprises administrating a drug, that is, the compound or the stereoisomer, deuterated compound, solvate, prodrug, metabolite, pharmaceutically acceptable salt or co-crystal thereof according to the present invention to a subject at a daily dose of 1-1000 mg/day, wherein the daily dose can be a single dose or a divided dose. In some embodiments, the daily dose includes but is not limited to 10-1000 mg/day, 10-800 mg/day, 25-800 mg/day, 50-800 mg/day, 100-800 mg/day, 200-800 mg/day, 25-400 mg/day, 50-400 mg/day, 100-400 mg/day, and 200-400 mg/day. In some embodiments, the daily dose includes but is not limited to 10 mg/day, 20 mg/day, 25 mg/day, 50 mg/day, 80 mg/day, 100 mg/day, 125 mg/day, 150 mg/day, 160 mg/day, 200 mg/day, 300 mg/day, 320 mg/day, 400 mg/day, 480 mg/day, 600 mg/day, 640 mg/day, 800 mg/day, and 1000 mg/day.

The present invention relates to a kit, wherein the kit can comprise a composition in the form of a single dose or multiple doses and comprises the compound, or the stereoisomer, deuterated compound, solvate, prodrug, metabolite, pharmaceutically acceptable salt or co-crystal thereof according to the present invention, and the amount of the compound, or the stereoisomer, deuterated compound, solvate, prodrug, metabolite, pharmaceutically acceptable salt or co-crystal thereof according to the present invention is identical to the amount of same in the above-mentioned pharmaceutical composition.

The present invention relates to use of the above-mentioned compound or the stereoisomer, deuterated compound, solvate, prodrug, metabolite, pharmaceutically acceptable salt or co-crystal thereof, or the above-mentioned pharmaceutical composition according to the present invention in the preparation of a drug for the treatment of a disease related to the activity or expression level of AR or AR splice mutants.

The present invention relates to use of the above-mentioned compound or the stereoisomer, deuterated compound, solvate, prodrug, metabolite, pharmaceutically acceptable salt or co-crystal thereof, or the above-mentioned

pharmaceutical composition according to the present invention in the preparation of a drug for the treatment of a disease related to the inhibition or degradation of AR or AR splice mutants.

The present invention relates to the use of the above-mentioned compound or the stereoisomer, deuterated compound, solvate, prodrug, metabolite, pharmaceutically acceptable salt or co-crystal thereof, or the above-mentioned pharmaceutical composition according to the present invention, wherein the disease is selected from prostate cancer.

The amount of the compound, or the stereoisomer, deuterated compound, solvate, prodrug, metabolite, pharmaceutically acceptable salt or co-crystal thereof according to the present invention is calculated in the form of a free base in each case.

Unless stated to the contrary, the terms used in the description and claims have the following meanings.

The carbon, hydrogen, oxygen, sulfur, nitrogen or F, Cl, Br and I involved in the groups and compounds according to the present invention all include their isotopes, and the carbon, hydrogen, oxygen, sulfur or nitrogen involved in the groups and compounds according to the present invention is optionally replaced by one or more of their corresponding isotopes, where the isotopes of carbon include $^{12}C$, $^{13}C$ and $^{14}C$; the isotopes of hydrogen include protium (H), deuterium (D, also called heavy hydrogen), and tritium (T, also called superheavy hydrogen); the isotopes of oxygen include $^{16}O$, $^{17}O$ and $^{18}O$; the isotopes of sulfur include $^{32}S$, $^{33}S$, $^{34}S$ and $^{36}S$; the isotopes of nitrogen include $^{14}N$ and $^{15}N$; the isotopes of fluorine include $^{17}F$ and $^{19}F$; the isotopes of chlorine include $^{35}Cl$ and $^{37}Cl$; and the isotopes of bromine include $^{79}Br$ and $^{81}Br$.

"Halogen" refers to F, Cl, Br or I.

"Halogen-substituted" refers to F, Cl, Br or I substitution, including but not limited to a substitution with 1 to 10 substituents selected from F, Cl, Br or I, a substitution with 1 to 6 substituents selected from F, Cl, Br or I, or a substitution with 1 to 4 substituents selected from F, Cl, Br or I. "Halogen-substituted" is referred to simply as "halo".

"Alkyl" refers to a substituted or unsubstituted linear or branched saturated aliphatic hydrocarbyl group, including but not limited to an alkyl group of 1 to 20 carbon atoms, an alkyl group of 1 to 8 carbon atoms, an alkyl group of 1 to 6 carbon atoms, or an alkyl group of 1 to 4 carbon atoms. Non-limiting examples include methyl, ethyl, n-propyl, isopropyl, n-butyl, sec-butyl, neobutyl, tert-butyl, n-pentyl, isoamyl, neopentyl, n-hexyl and various branched isomers thereof. The definition of the "alkyl" herein is consistent with this definition. Alkyl can be monovalent, divalent, trivalent or tetravalent.

"Hydrocarbyl" refers to a substituted or unsubstituted linear or branched saturated or unsaturated group consisting of carbon and hydrogen atoms. Hydrocarbyl can be monovalent, divalent, trivalent or tetravalent.

"Heteroalkyl" refers to a substituted or unsubstituted alkyl group in which one or more (including but not limited to 2, 3, 4, 5 or 6) carbon atoms are replaced by heteroatoms (including but not limited to N, O or S). Non-limiting examples include $-X(CH_2)v-X(CH_2)v-X(CH_2)v-H$ (v is an integer from 1 to 5; each X is independently selected from a bond or a heteroatom, which includes but is not limited to N, O or S; at least one X is selected from a heteroatom; and N or S in the heteroatom can be oxidized to various oxidation states). Heteroalkyl can be monovalent, divalent, trivalent or tetravalent.

"Alkylene" refers to a substituted or unsubstituted linear or branched divalent saturated hydrocarbyl group, including $-(CH_2)_v-$ (v is an integer from 1 to 10), and examples of alkylene include, but are not limited to, methylene, ethylene, propylene, butylene, *etc.*

"Heteroalkylene" refers to a substituted or unsubstituted alkylene group in which one or more (including but not limited to 2, 3, 4, 5 or 6) carbon atoms are replaced by heteroatoms (including but not limited to N, O or S). Non-limiting examples include $-X(CH_2)v-X(CH_2)v-X(CH_2)v-$, wherein v is an integer from 1 to 5, each X is independently selected from a bond, N, O or S, and at least one X is selected from N, O or S.

"Cycloalkyl" refers to a substituted or unsubstituted saturated carbocyclic hydrocarbyl group, usually having from 3 to 10 carbon atoms, and non-limiting examples include cyclopropyl, cyclobutyl, cyclopentyl, cyclohexyl, cycloheptyl, etc. The "cycloalkyl" herein is as defined above. Cycloalkyl can be monovalent, divalent, trivalent or tetravalent.

"Heterocycloalkyl" refers to a substituted or unsubstituted saturated heteroatom-containing cyclic hydrocarbyl group, including but not limited to 3 to 10 atoms, 3 to 8 atoms, or 1 to 3 heteroatoms selected from N, O or S. N and S selectively substituted in the heterocycloalkyl ring can be oxidized to various oxidation states. Heterocycloalkyl can be connected to a heteroatom or a carbon atom; heterocycloalkyl can be connected to an aromatic ring or a non-aromatic ring; and heterocycloalkyl can be connected to a bridged ring or a spiro ring. Non-limiting examples include oxiranyl, azacyclopropyl, oxetanyl, azetidinyl, tetrahydrofuryl, tetrahydro-2*H*-pyranyl, dioxolanyl, dioxanyl, pyrrolidinyl, piperidinyl, imidazolidinyl, oxazolidinyl, oxazinanyl, morpholinyl, hexahydropyrimidinyl or piperazinyl. Heterocycloalkyl can be monovalent, divalent, trivalent or tetravalent.

"Alkenyl" refers to a substituted or unsubstituted linear or branched unsaturated hydrocarbyl group, having at least 1, usually 1, 2 or 3 carbon-carbon double bonds, with a main chain including but not limited to 2 to 10, 2 to 6, or 2 to 4 carbon atoms. Examples of alkenyl include, but are not limited to, ethenyl, allyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 2-methyl-1-butenyl, 2-methyl-3-butenyl, 1-hexenyl, 2-hexenyl, 3-hexenyl, 4-hexenyl, 5-hexenyl, 1-methyl-1-pentenyl, 2-methyl-1-pentenyl, 1-heptenyl, 2-heptenyl, 3-heptenyl, 4-heptenyl, 1-octenyl, 3-octenyl, 1-nonenyl, 3-nonenyl, 1-decenyl, 4-decenyl, 1,3-butadiene, 1,3-pentadiene, 1,4-pentadiene, 1,4-hexadiene, prop-1,2-dien-1-yl, *etc.* The definition of the "alkenyl" herein is consistent with this definition. Alkenyl can be monovalent, divalent, trivalent or tetravalent.

"Alkynyl" refers to a substituted or unsubstituted linear or branched monovalent unsaturated hydrocarbyl group, having at least 1, usually 1, 2 or 3 carbon-carbon triple bonds, with a main chain including but not limited to 2 to 10 carbon atoms, 2 to 6 carbon atoms, or 2 to 4 carbon atoms. Examples of alkynyl include, but are not limited to, ethynyl, propargyl, 1-propynyl, 2-propynyl, 1-butynyl, 2-butynyl, 3-butynyl, 1-pentynyl, 2-pentynyl, 3-pentynyl, 4-pentynyl, 1-methyl-1-butynyl, 2-methyl-1-butynyl, 2-methyl-3-butynyl, 1-hexynyl, 2-hexynyl, 3-hexynyl, 4-hexynyl, 5-hexynyl, 1-methyl-1-pentynyl, 2-methyl-1-pentynyl, 1-heptynyl, 2-heptynyl, 3-heptynyl, 4-heptynyl, 1-octynyl, 3-octynyl, 1-nonynyl, 3-nonynyl, 1-decynyl, 4-decynyl, etc. Alkynyl can be monovalent, divalent, trivalent or tetravalent.

"Alkoxy" refers to a substituted or unsubstituted -O-alkyl group. Non-limiting examples include methoxy, ethoxy, n-propoxy, isopropoxy, n-butoxy, sec-butoxy, tert-butoxy, n-pentoxy, n-hexyloxy, cyclopropoxy and cyclobutoxy.

"Carbocyclyl" or "carbocycle" refers to a substituted or unsubstituted saturated or unsaturated aromatic ring or non-aromatic ring, wherein the aromatic ring or non-aromatic ring can be a 3- to 8-membered monocyclic ring, a 4- to 12-membered bicyclic ring or a 10- to 15-membered tricyclic ring system. Carbocyclyl can be connected to an aromatic ring or a non-aromatic ring, wherein the aromatic ring or non-aromatic ring is optionally a monocyclic ring, a bridged ring or a spiro ring. Non-limiting examples include cyclopropane, cyclobutane, cyclopentane, cyclohexane, cycloheptane, 1-cyclopentyl-1-enyl, 1-cyclopentyl-2-enyl, 1-cyclopentyl-3-enyl, cyclohexyl, 1-cyclohexyl-2-enyl, 1-cyclohexyl-3-enyl, cyclohexenyl, a benzene ring, a naphthalene ring,

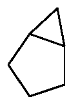,

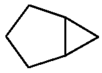, 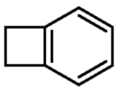, 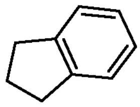 or 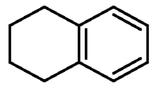.

"Carbocyclyl" or "carbocycle" can be monovalent, divalent, trivalent or tetravalent.

"Heterocyclyl" or "heterocycle" refers to a substituted or unsubstituted saturated or unsaturated aromatic ring or non-aromatic ring, wherein the aromatic ring or non-aromatic ring can be 3- to 8-membered monocyclic ring, 4- to 12-membered bicyclic ring or 10- to 15-membered tricyclic ring system, and contains one or more (including but not limited to 2, 3, 4 or 5) heteroatoms selected from N, O or S, and the selectively substituted N and S in the heterocyclyl ring can be oxidized to various oxidation states. Heterocyclyl can be connected to a heteroatom or a carbon atom; heterocyclyl can be connected to an aromatic ring or a non-aromatic ring; and heterocyclyl can be connected to a bridged ring or a spiro ring. Non-limiting examples include oxiranyl, azacyclopropyl, oxetanyl, azetidinyl, 1,3-dioxolanyl, 1,4-dioxolanyl, 1,3-dioxanyl, azacycloheptyl, pyridyl, furyl, thienyl, pyranyl, N-alkylpyrrolyl, pyrimidinyl, pyrazinyl, pyridazinyl, imidazolyl, piperidinyl, morpholinyl, thiomorpholinyl, 1,3-dithianyl, dihydrofuryl, dihydropyranyl, dithiolanyl, tetrahydrofuryl, tetrahydropyrrolyl, tetrahydroimidazolyl, tetrahydrothiazolyl, tetrahydropyranyl, benzoimidazolyl, benzopyridyl, pyrrolopyridinyl, benzodihydrofuryl, pyrrolyl, pyrazolyl, thiazolyl, oxazolyl, pyrazinyl, indazolyl, benzothienyl, benzofuryl, benzopyrrolyl, benzoimidazolyl, benzothiazolyl, benzoxazolyl, benzopyridyl, benzopyrimidinyl, benzopyrazinyl, piperazinyl, azabicyclo[3.2.1]octanyl, azabicyclo[5.2.0]nonanyl, oxatricyclo[5.3.1.1]dodecyl, azaadamantyl, oxaspiro[3.3]heptanyl,

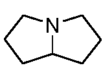,

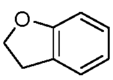, 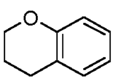, 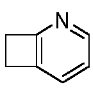, 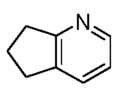, 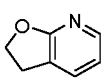, 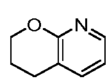, 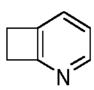, 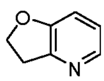, 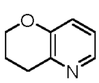

, 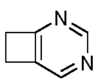, 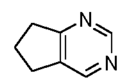, 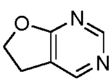 or 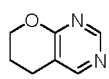.

"Heterocyclyl" or "heterocycle" can be monovalent, divalent, trivalent or tetravalent.

"Spiro ring" or "spiro ring group" refers to a polycyclic group that shares one atom (called a spiro atom) between substituted or unsubstituted monocyclic rings. The number of ring atoms in the spiro ring system includes but is not limited to 5 to 20, 6 to 14, 6 to 12, or 6 to 10, wherein one or more rings may contain 0 or more (including but not limited to 1, 2, 3 or 4) double bonds, and can optionally contain 0 to 5 heteroatoms selected from N, O or $S(=O)_n$.

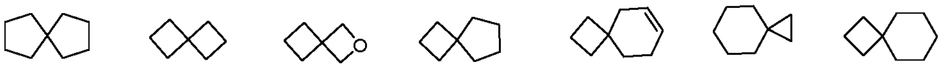

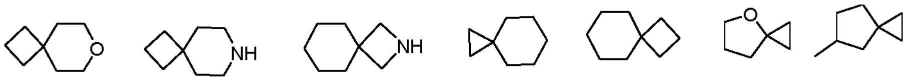

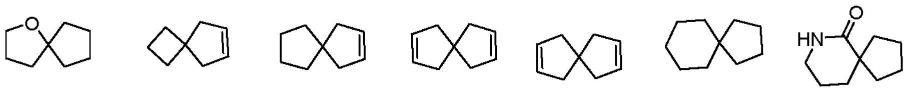

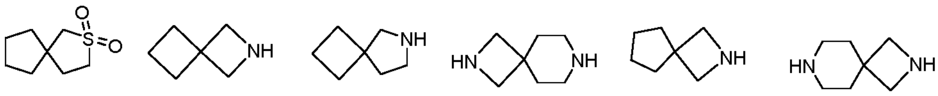 .

"Spiro ring" or "spiro ring group" can be monovalent, divalent, trivalent or tetravalent.

"Fused ring" or "fused ring group" refers to a polycyclic group in which each ring in the system shares an adjacent pair of atoms with other rings in the system, wherein one or more rings may contain 0 or more (including but not limited to 1, 2, 3 or 4) double bonds, and may be substituted or unsubstituted, and each ring in the fused ring system may contain 0 to 5 heteroatoms or groups containing heteroatoms (including but not limited to N, $S(=O)_n$ or O, wherein n is 0, 1 or 2). The number of ring atoms in the fused ring system includes but is not limited to 5 to 20, 5 to 14, 5 to 12, or 5 to 10. Non-limiting examples include:

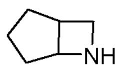 ,

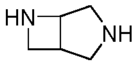 , 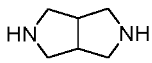 ,

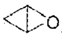 , 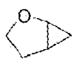 , 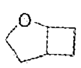 , 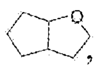 , 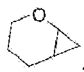 , 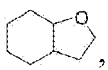 , 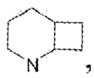 , 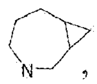 ,

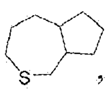 , 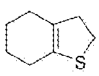 , 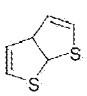 , 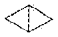 , 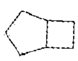 , 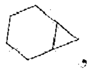 , 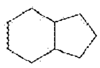 , 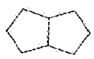 ,

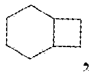 , 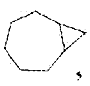 , 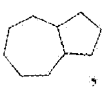 , 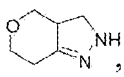 , 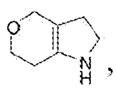 , 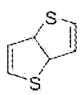 or 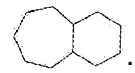 .

"Fused ring" or "fused ring group" can be monovalent, divalent, trivalent or tetravalent.

"Bridged ring" or "bridged ring group" refers to a substituted or unsubstituted polycyclic group containing any two atoms that are not directly connected, and may contain 0 or more double bonds. Any ring in the fused ring system may contain 0 to 5 groups selected from heteroatoms or groups containing heteroatoms (including but not limited to N, S(=O)n or O, wherein n is 0, 1 or 2). The number of ring atoms includes but is not limited to 5 to 20, 5 to 14, 5 to 12 or

5 to 10. Non-limiting examples include

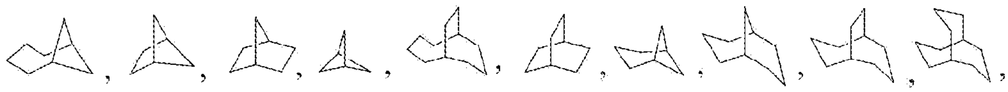

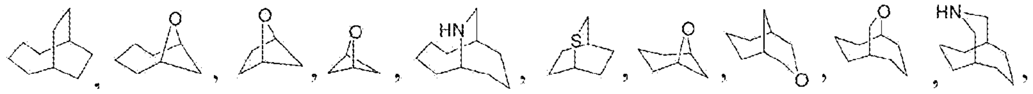

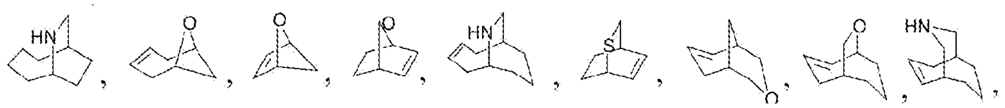

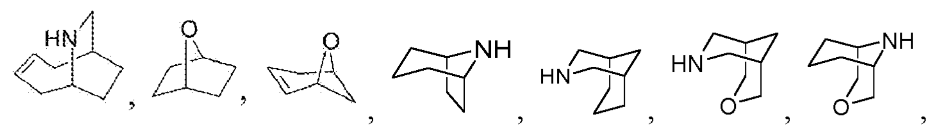

cubane or adamantane. "Bridged ring" or "bridged ring group" can be monovalent, divalent, trivalent or tetravalent.

"Carbospiro ring", "spiro ring carbocyclyl", "spirocarbocyclyl" or "carbospiro ring group" refers to a "spiro ring" with a ring system consisting only of carbon atoms. The definition of the "carbospiro ring", "spiro ring carbocyclyl", "spirocarbocyclyl" or "carbospiro ring group" herein is consistent with that of a spiro ring.

"Carbo-fused ring", "fused ring carbocyclyl", "fused carbocyclyl" or "carbo-fused ring group" refers to a "fused ring" with a ring system consisting only of carbon atoms. The definition of the "carbo-fused ring", "fused ring carbocyclyl", "fused carbocyclyl" or "carbo-fused ring group" herein is consistent with that of a fused ring.

"Carbo-bridged ring", "bridged ring carbocyclyl", "bridged carbocyclyl" or "carbo-bridged ring group" refers to a "bridged ring" with a ring system consisting only of carbon atoms. The definition of the "carbo-bridged ring", "bridged ring carbocyclyl", "bridged carbocyclyl" or "carbo-bridged ring group" herein is consistent with that of a bridged ring.

"Mono-heterocyclic ring", "monocyclic heterocyclyl" or "mono-heterocyclic ring group" refers to "heterocyclyl" or "heterocycle" with a monocyclic system. The definition of the "heterocyclyl", "monocyclic heterocyclyl" or "mono-heterocyclic ring group" herein is consistent with that of heterocycle.

"Fused heterocyclic ring", "fused heterocyclic ring group", "fused ring heterocyclyl" or "fused heterocyclic ring group" refers to a "fused ring" containing a heteroatom. The definition of the "fused heterocyclic ring", "fused heterocyclic ring group", "fused ring heterocyclyl" or "fused heterocyclic ring group" herein is consistent with that of a fused ring.

"Spiro-heterocyclic ring", "spiro-heterocyclic ring group", "spiro ring heterocyclyl" or "spiro-heterocyclic ring group" refers to a "spiro ring" containing a heteroatom. The definition of the "spiro-heterocyclic ring", "spiro-heterocyclic ring group", "spiro ring heterocyclyl" or "spiro-heterocyclic ring group" herein is consistent with that of a spiro ring.

"Bridged-heterocyclic ring", "bridged-heterocyclic ring group", "bridged ring heterocyclyl" or "bridged-heterocyclic ring group" refers to a "bridged ring" containing a heteroatom. The definition of the "bridged-heterocyclic ring", "bridged-heterocyclic ring group", "bridged ring heterocyclyl" or "bridged-heterocyclic ring group" herein is consistent with that of a bridged ring.

"Aryl" or "aromatic ring" refers to a substituted or unsubstituted aromatic hydrocarbyl group with a monocyclic ring or a fused ring, wherein the number of ring atoms in the aromatic ring includes but is not limited to 6 to 18, 6 to 12 or 6 to 10 carbon atoms. The aryl ring can be fused to a saturated or unsaturated carbocycle or heterocycle, wherein the ring connected to the parent structure is an aryl ring. Non-limiting examples include a benzene ring, a naphthalene ring, or

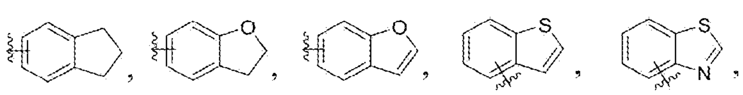

"Aryl" or "aromatic ring" can be monovalent, divalent, trivalent or tetravalent. When divalent, trivalent or tetravalent, the point of connection is on the aryl ring.

"Heteroaryl" or "heteroaromatic ring" refers to a substituted or unsubstituted aromatic hydrocarbyl group containing 1 to 5 heteroatoms or groups containing heteroatoms (including but not limited to N, O or S(=O)n, wherein n is 0, 1 or 2), wherein the number of ring atoms in the heteroaromatic ring includes but is not limited to 5-15, 5-10 or 5-6. Non-limiting examples of heteroaryl include, but are not limited to pyridyl, furyl, thienyl, pyridyl, pyranyl, N-alkylpyrrolyl, pyrimidinyl, pyrazinyl, pyridazinyl, imidazolyl, benzopyrazole, benzoimidazole, benzopyridine, pyrrolopyridine, etc. The heteroaryl ring may be fused to a saturated or unsaturated carbocycle or heterocycle, wherein the ring connected to the parent structure is an heteroaryl ring. Non-limiting examples include

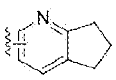, 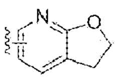, 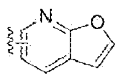, 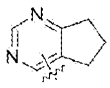, 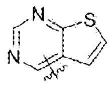, 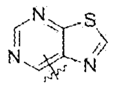, 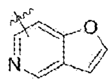

and

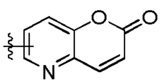.

The definition of the "heteroaryl" herein is consistent with this definition. Heteroaryl can be monovalent, divalent, trivalent or tetravalent. When divalent, trivalent or tetravalent, the point of connection is on the heteroaryl ring.

"5-membered ring fused 5-membered heteroaromatic ring" refers to a 5 fused 5-membered fused heteroaromatic ring, wherein at least one of the two fused rings contains at least one heteroatom (including but not limited to O, S or N), and the entire group is aromatic. Non-limiting examples include a pyrrolopyrrole ring, a pyrazolopyrrole ring, a pyrazolopyrazole ring, a pyrrolofuran ring, a pyrazolofuran ring, a pyrrolothiophene ring and a pyrazolothiophene ring.

"5 fused 6-membered heteroaromatic ring" refers to a 5 fused 6-membered fused heteroaromatic ring, wherein at least one of the two fused rings contains at least one heteroatom (including but not limited to O, S or N), and the entire group is aromatic. Non-limiting examples include a benzo 5-membered heteroaryl and 6-membered heteroaromatic ring fused 5-membered heteroaromatic ring.

"Substitution" or "substituted" refers to a substitution with 1 or more (including but not limited to 2, 3, 4 or 5) substituents including but not limited to H, F, Cl, Br, I, alkyl, cycloalkyl, alkoxy, haloalkyl, mercaptan, hydroxyl, nitro, mercapto, amino, cyano, isocyano, aryl, heteroaryl, heterocyclyl, bridged ring group, spiro ring group, fused ring group, hydroxyalkyl, =O, carbonyl, aldehyde, carboxylic acid, carboxylate, $-(CH_2)_m-C(=O)-R^a$, $-O-(CH_2)_m-C(=O)-R^a$, $-(CH_2)_m-C(=O)-NR^bR^c$, $-(CH_2)_mS(=O)_nR^a$, $-(CH_2)_m$-alkenyl $-R^a$, $OR^d$ or $-(CH_2)_m$-alkynyl - $R^a$ (wherein m and n are 0, 1 or 2), arylthio, thiocarbonyl, silyl, $-NR^bR^c$, etc., wherein $R^b$ and $R^c$ are independently selected from H, hydroxyl, amino, carbonyl, alkyl, alkoxy, cycloalkyl, heterocyclyl, aryl, heteroaryl, sulfonyl, or trifluoromethylsulfonyl. Alternatively, $R^b$ and $R^c$ may form a five- or six-membered cycloalkyl or heterocyclyl.

"Containing 1 to 5 heteroatoms selected from O, S or N" means containing 1, 2, 3, 4 or 5 heteroatoms selected from O, S or N.

"Substituted with 0 to X substituents selected from ..." means substituted with 0, 1, 2, 3 ... X substituents selected from ..., wherein X is selected from any integer between 1 and 10. For example, "substituted with 0 to 4 substituents selected from ..." means substituted with 0, 1, 2, 3 or 4 substituents selected from ... For example, "substituted with 0 to 5 substituents selected from ..." means substituted with 0, 1, 2, 3, 4 or 5 substituents selected from ... For example, "bridged-heterocyclic ring is optionally substituted with 0 to 4 substituents selected from H or F" means that the bridged-heterocyclic ring is optionally substituted with 0, 1, 2, 3 or 4 substituents selected from H or F.

An X- to Y-membered ring (X is selected from an integer less than Y and greater than or equal to 3, and Y is selected from any integer between 4 and 12) includes X+1-, X+2-, X+3-, X+4-, ..., Y-membered rings. Rings include heterocycle, carbocycle, an aromatic ring, aryl, heteroaryl, cycloalkyl, a mono-heterocyclic ring, a fused heterocyclic ring, a spiro-heterocyclic ring or a bridged-heterocyclic ring. For example, a "4- to 7-membered mono-heterocyclic ring" refers to a 4-, 5-, 6- or 7-membered mono-heterocyclic ring, and a "5- to 10-membered fused heterocyclic ring" refers to a 5-, 6-, 7-, 8-, 9- or 10-membered fused heterocyclic ring.

The term "optional" or "optionally" refers to that the events or circumstances subsequently described may but not necessarily occur, and the description includes the occasions where the events or circumstances occur or do not occur. For example, "alkyl optionally substituted with F" means that the alkyl may but not necessarily be substituted with F, and the description includes the case where the alkyl is substituted with F and the case where the alkyl is not substituted with F.

"Pharmaceutically acceptable salt" or "pharmaceutically acceptable salt thereof" refers to a salt of the compound of the present invention, which salt maintains the biological effectiveness and characteristics of a free acid or a free base, and is obtained by reacting the free acid with a non-toxic inorganic base or organic base, or reacting the free base with a non-toxic inorganic acid or organic acid.

"Pharmaceutical composition" refers to a mixture of one or more compounds of the present invention, or stereoisomers, tautomers, deuterated compounds, solvates, prodrugs, metabolites, pharmaceutically acceptable salts or co-crystals thereof and other chemical components, wherein "other chemical components" refer to pharmaceutically acceptable carriers, excipients and/or one or more other therapeutic agents.
The term "preparation specification" refers to the weight of the active drug contained in each vial, tablet or other unit preparation.
"Carrier" refers to a material that does not cause significant irritation to an organism and does not eliminate the biological activity and characteristics of a compound administered.
"Animal" is meant to include mammals, such as humans, companion animals, zoo animals, and domestic animals, preferably humans, horses, or dogs.
The term "stereoisomer" refers to an isomer produced as a result of different spatial arrangement of atoms in molecules, including cis-trans isomers, enantiomers and conformational isomers.
"Tautomer" refers to a functional group isomer produced by the rapid movement of an atom in two positions in a molecule, such as keto-enol isomerization and amide-imino alcohol isomerization.
"$IC_{50}$" refers to the concentration of a medicament or inhibitor required to inhibit half of a given biological process (or a component of the process such as an enzyme, a receptor and a cell).

Detailed Description of Embodiments

The technical solutions of the present invention will be described in detail below in conjunction with examples, but the protection scope of the present invention includes but is not limited thereto.
To achieve the objectives of the present invention, according to organic synthesis techniques known to those skilled in the art, and starting from commercially available chemicals and/or compounds described in chemical documents, the prepared compounds, "commercially available chemicals", for use in the reactions described herein are obtained from standard commercial sources, including Shanghai Aladdin Bio-Chem Technology Co., Ltd., Shanghai Macklin Biochemical Co., Ltd., Sigma-Aldrich, Alfa Aesar (China) Chemical Co., Ltd., Tokyo Chemical Industry (Shanghai) Co., Ltd., Energy Chemical Co., Ltd., Shanghai Titan Scientific Co., Ltd., Kelong Chemical Co., Ltd., J&K Scientific and the like.
The compounds used in the reactions described herein are prepared according to organic synthesis techniques known to those skilled in the art, and starting from commercially available chemicals and(or) compounds described in chemical documents. "Commercially available chemicals" are obtained from regular commercial sources, and suppliers include: Titan Technology Co., Ltd., Energy Chemical Co., Ltd., Shanghai Demo Co., Ltd., Chengdu Kelong Chemical Co., Ltd., Accela ChemBio Co., Ltd., PharmaBlock Sciences (Nanjing), Inc., WuXi Apptec Co., Ltd., J&K Scientific Co., Ltd., etc.
The structures of the compounds are determined by nuclear magnetic resonance (NMR) or (and) mass spectrometry (MS). The NMR shift (δ) is given in the unit of $10^{-6}$ (ppm). NMR is determined with (Bruker Avance III 400 and Bruker Avance 300) nuclear magnetic resonance instrument; the solvents for determination are deuterated dimethyl sulfoxide ($DMSO-d_6$), deuterated chloroform ($CDCl_3$) and deuterated methanol ($CD_3OD$); and the internal standard is tetramethylsilane (TMS);

MS is determined with Agilent 6120B (ESI) and Agilent 6120B (APCI);
HPLC is determined with Agilent 1260DAD high pressure liquid chromatograph (Zorbax SB-C18 100 × 4.6 mm, 3.5 μM);
Yantai Huanghai HSGF254 or Qingdao GF254 silica gel plate is used as a thin layer chromatography silica plate, and the silica gel plate for the thin layer chromatography (TLC) is of the specification of 0.15 mm-0.20 mm, and the specification when separating and purifying a product by thin layer chromatography is 0.4 mm - 0.5 mm;
and for the column chromatography, Yantai Huanghai silica gel of 200-300 mesh silica gel is generally used as a carrier. SEM:

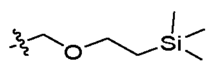
;

THP:

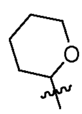
;

Ms:

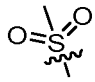

;

TBS:

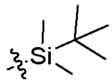

;

Cbz:

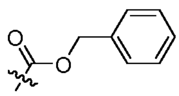

;

Bn: benzyl; Boc: tert-butoxycarbonyl; MTBE: methyl tert-butyl ether; DIPEA: N,N-diisopropylethylamine; DMAc: N,N-dimethylacetamide; DMSO: dimethyl sulfoxide; DCM: dichloromethane; NMP: N-methylpyrrolidone; DMF: N,N-dimethylformamide; TEA: triethylamine; DCE: dichloroethane; THF: tetrahydrofuran; LiHMDS: lithium hexamethyldisilazide; TBAF: tetrabutylammoniumfluoride;
HATU: CAS 148893-10-1; Pd(dppf)$Cl_2$·DCM: CAS 95464-05-4; $PdCl_2(PPh_3)_2$: CAS 13965-03-2; XPhos Pd G3: CAS 1445085-55-1; RuPhosPdG3: CAS 1445085-77-7; Xphos: CAS 564483-18-7; DAST: CAS: 38078-09-0; T3P: CAS 68957-94-8; TCFH: CAS 94790-35-9 2-(2,6-dioxopiperidin-3-yl)-5-fluoroisoindoline-1,3-dione:

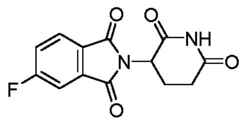

(see WO 2021194318 for the synthesis method); 2-(4-iodo-1H-pyrazol-1-yl)-2-methylpropionic acid:

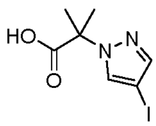

(see WO 2019074962 for the synthesis method)

Unless otherwise noted, the Sonogashira reaction involved in the present invention is performed under nitrogen atmosphere.

**Synthesis of intermediate 1:**

2-(2,6-dioxopiperidin-3-yl)-5-(3-ethynylazetidin-1-yl)isoindoline-1,3-dione

(**Intermediate 1**)

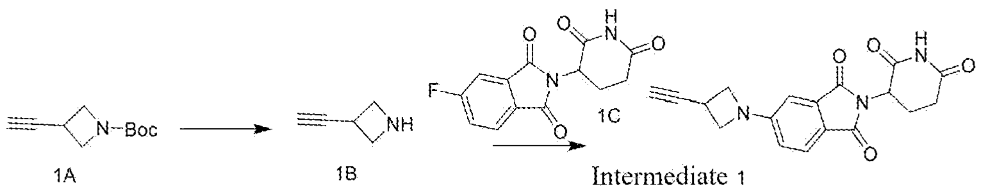

1A 1B 1C Intermediate 1

Step 1: 3-ethynylazetidine (1B) hydrochloride

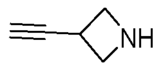

1A (90 g, 0.50 mol) was dissolved in 500 mL of 2 mol/L ethyl acetate hydrogen chloride solution and the mixture was reacted at room temperature for 5 h. The reaction system was concentrated under reduced pressure to afford the hydrochloride of crude 1B (59 g).

LCMS m/z = 82.3 $[M+1]^+$.

Step 2: 2-(2,6-dioxopiperidin-3-yl)-5-(3-ethynylazetidin-1-yl)isoindoline-1,3-dione (Intermediate 1)

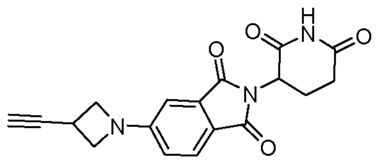

The hydrochloride of the crude 1B (59 g) was dissolved in 500 mL of DMSO, sodium bicarbonate (42 g, 0.50 mol) was added, the mixture was stirred at room temperature for 10 min, then 100 mL of DIPEA and 1C (165.6 g, 0.60 mol) were added, the mixture was reacted at 85°C for 5 h. The reaction liquid was cooled to room temperature, 5 L of water was added, the mixture was filtered, the solid was collected, washed with 500 mL of water, and the solid was dried by blowing to afford crude intermediate 1 (40 g).

**Synthesis of intermediate 2:**

2-(2,6-dioxopiperidin-3-yl)-5-(3-ethynylazetidin-1-yl)-6-fluoroisoindoline-1,3-dione (**Intermediate 2**)

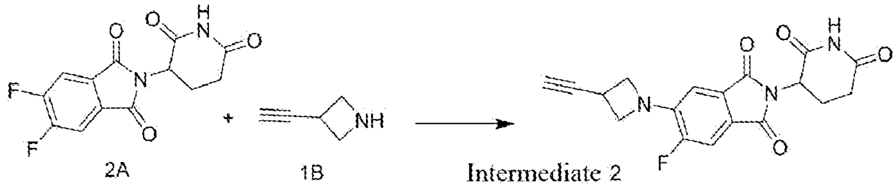

2A 1B Intermediate 2

The hydrochloride of the above crude 1B (0.72 g) was dissolved in 20 mL of DMSO, 2 mL of DIPEA and 2A (see WO 2020239103 for the synthesis method) (1.80 g, 6.12 mmol) were added, and the mixture was reacted at 80°C for 3 h. The reaction liquid was cooled to room temperature, 200 mL of water was added, the mixture was filtered, the solid was collected, washed with 50 mL of water, and the solid was dried by blowing to afford crude intermediate 2 (1.3 g).

**Example 1: Preparation of the trifluoroacetate of compound 1**

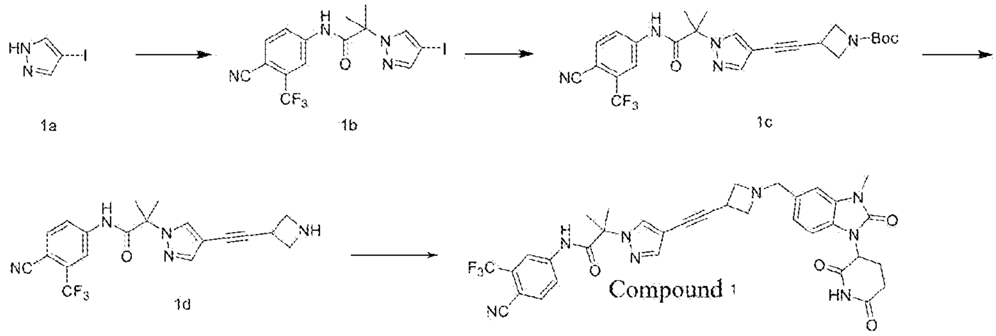

1a 1b 1c

1d Compound 1

Step 1: N-(4-cyano-3-(trifluoromethyl)phenyl)-2-(4-iodo-1H-pyrazol-1-yl)-2-methylpropanamide (1b)

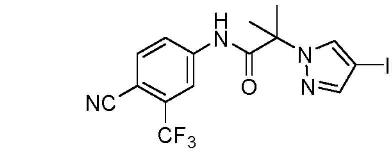

2-bromo-N-(4-cyano-3-(trifluoromethyl)phenyl)-2-methylpropanamide (see WO 2020063407 for the synthesis method) (1.72 g, 5.13 mmol) was dissolved in 35 mL of acetonitrile. 1a (1.0 g, 5.15 mmol) and cesium carbonate (3.36 g, 10.30 mmol) were added, and the mixture was stirred at 50°C for 3 h. The reaction liquid was cooled to room temperature. 50 mL of water was added, and the mixture was extracted with DCM (40 mL × 3). The organic phase was washed with 50 mL of water, dried over anhydrous sodium sulfate and concentrated under reduced pressure. The crude product was separated and purified with silica gel column chromatography (petroleum ether/ethyl acetate (v/v) = 4 : 1) to afford 1b (1.5 g, yield: 65%).

$^1$H NMR (400 MHz, DMSO-$d_6$) δ 10.06 (s, 1H), 8.26 (d, 1H), 8.18 (s, 1H), 8.15 (dd, 1H), 8.08 (d, 1H), 7.62 (s, 1H), 1.81 (s, 6H).

LCMS m/z = 449.0 $[M+1]^+$.

Step 2: tert-butyl 3-((1-(1-((4-cyano-3-(trifluoromethyl)phenyl)amino)-2-methyl-1- oxopropan-2-yl)- 1H-pyrazol-4-yl)ethynyl)azetidine-1-carboxylate (1c)

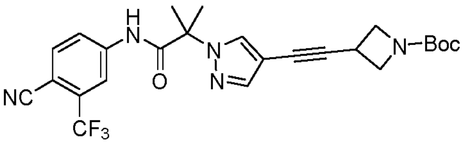

1b (224 mg, 0.50 mmol) was dissolved in DCM (10 mL). TEA (150 mg, 1.48 mmol) was added, $PdCl_2(PPh_3)_2$ (35 mg, 0.05 mmol) and CuI (10 mg, 0.05 mmol) were added in sequence under nitrogen atmosphere, and then a solution of tert-butyl 3-ethynylazetidine-1-carboxylate (136 mg, 0.75 mmol) in DCM (2 mL) was slowly added dropwise. After the addition was completed, the mixture was reacted at room temperature for 16 h. 15 mL of water and 10 mL of DCM were added to the reaction liquid, and the liquid separation was conducted. The organic layer was washed with 5 mL of saturated sodium chloride solution, dried over anhydrous sodium sulfate, and concentrated under reduced pressure. The residue was separated and purified with silica gel column chromatography (petroleum ether/ethyl acetate (v/v) = 4 : 1) to afford 1c (230 mg, yield: 92%).

Step 3: 2-(4-(azetidin-3-ylethynyl)-1H-pyrazol-1-yl)-N-(4-cyano-3-(trifluoromethyl)phenyl)-2-methylpropanamide (1d)

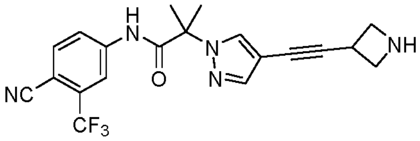

1c (0.23 g, 0.46 mmol) was dissolved in 10 mL of DCM, and 6 mL of trifluoroacetic acid was added. The mixture was stirred at room temperature for 3 h. After the reaction was completed, the system was directly concentrated under reduced pressure. The residue was dissolved in 20 mL of 4 mol/L aqueous NaOH solution and the mixture was extracted with DCM (40 mL × 3). The organic phase was washed with 50 mL of water, dried over anhydrous sodium sulfate, and concentrated under reduced pressure to afford crude 1d (0.18 g).
LCMS m/z = 402.1 $[M+1]^+$.

Step 4: N-(4-cyano-3-(trifluoromethyl)phenyl)-2-(4-((1-((1-(2,6-dioxopiperidin-3-yl)-3-methyl-2-oxo-2,3-dihydro-1H-benzo[d]imidazol-5-yl)methyl)azetidin-3-yl)ethynyl)-1H-pyrazol-1-yl)-2-methylpropanamide (Compound 1) trifluoroacetate

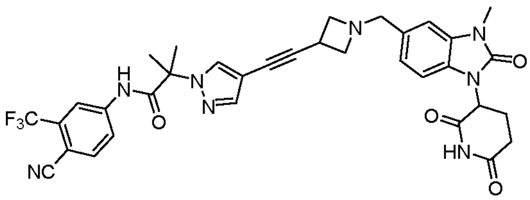

Crude 1d (85 mg) was dissolved in 4 mL of anhydrous THF, 1-(2,6-dioxopiperidin-3-yl)-3-methyl-2-oxo-2,3-dihydro-1H-benzo[d]imidazole-5-carbaldehyde (see WO 2020113233 for the synthesis method) (50 mg, 0.17 mmol) and 0.05 mL of acetic acid were added, the mixture was stirred at room temperature for 2 h, sodium triacetoxyborohydride (75 mg, 0.35 mmol) was added, and the stirring was continued at room temperature for 16 h. To the reaction liquid was added sodium bicarbonate (100 mg), and the mixture was stirred at room temperature for 15 min and then filtered by suction. The filtrate was concentrated under reduced pressure, and the crude product was subjected to Prep-HPLC (instrument and preparative column: using Glison GX-281 preparative liquid phase chromatographic instrument, preparative column model: Sunfire C18, 5 μm, inner diameter × length = 30 mm × 150 mm). Preparation method: the crude product was dissolved with methanol and dimethyl sulfoxide, and filtered with a 0.45 μm filter membrane, to prepare into a sample liquid. Mobile phase system: acetonitrile/water (containing 0.1% TFA). Gradient elution method: gradient elution of 5% to 60% acetonitrile (elution time 15 min), and lyophilization was performed to afford the trifluoroacetate of compound 1 (25 mg).
$^1$H NMR (400 MHz, DMSO-$d_6$) δ 11.10 (s, 1H), 10.11 - 10.03 (m, 1H), 8.29 - 8.06 (m, 4H), 7.70 - 7.63 (m, 1H), 7.36 - 7.10 (m, 3H), 5.40 (dd, 1H), 4.50 - 4.30 (m, 3H), 4.30 - 4.14 (m, 2H), 4.12 - 3.96 (m, 1H), 3.91 - 3.77 (m, 1H), 3.39 - 3.31 (m, 3H), 2.98 - 2.84 (m, 1H), 2.79 - 2.58 (m, 2H), 2.07 - 1.96 (m, 1H), 1.88 - 1.76 (m, 6H).
LCMS m/z = 673.3 $[M+1]^+$.

**Example 2: Preparation of the trifluoroacetate of compound 2**

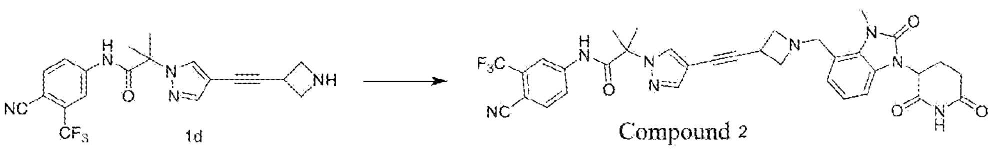

1d Compound 2

Crude 1d (85 mg) was dissolved in 4 mL of anhydrous THF, 1-(2,6-dioxopiperidin-3-yl)-3-methyl-2-oxo-2,3-dihydro-1H-benzo[d]imidazole-4-carbaldehyde (see WO 2020113233 for the synthesis method) (50 mg, 0.17 mmol) and 0.05 mL of acetic acid were added, the mixture was stirred at room temperature for 2 h, sodium triacetoxyborohydride (75 mg, 0.35 mmol) was added, and the stirring was continued at room temperature for 16 h. To the reaction liquid was added sodium bicarbonate (100 mg), and the mixture was stirred at room temperature for 15 min and then filtered by suction. The filtrate was concentrated under reduced pressure, and the crude product was subjected to Prep-HPLC (instrument and preparative column: using Glison GX-281 preparative liquid phase chromatographic instrument, preparative column model: Sunfire C18, 5 μm, inner diameter × length = 30 mm × 150 mm). Preparation method: the crude product was dissolved with methanol and dimethyl sulfoxide, and filtered with a 0.45 μm filter membrane, to prepare into a sample liquid. Mobile phase system: acetonitrile/water (containing 0.1% TFA). Gradient elution method: gradient elution of 5% to 60% acetonitrile (elution time 15 min), and lyophilization was performed to afford the trifluoroacetate of compound 2 (30 mg).

$^1$H NMR (400 MHz, DMSO-$d_6$) δ 11.10 (s, 1H), 10.07 (s, 1H), 8.30 - 8.04 (m, 4H), 7.67 (s, 1H), 7.32 - 7.00 (m, 3H), 5.48 - 5.36 (m, 1H), 4.92 - 4.58 (m, 2H), 4.58 - 4.40 (m, 1H), 4.40 - 4.09 (m, 2H), 4.09 - 3.92 (m, 1H), 3.92 - 3.69 (m, 1H), 3.59 (s, 3H), 2.98 - 2.83 (m, 1H), 2.80 - 2.57 (m, 2H), 2.06 - 1.70 (m, 7H).

LCMS m/z = 673.3 $[M+1]^+$.

**Example 3: Preparation of compound 3**

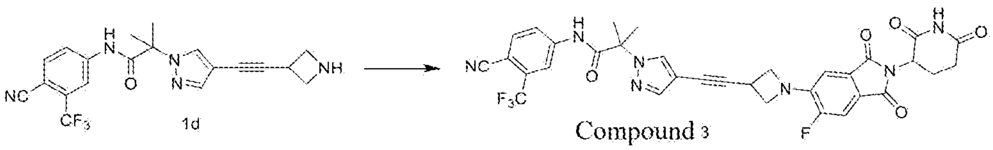

1d

Compound 3

The crude 1d (190 mg) was dissolved in 8 mL of DMSO, 0.5 mL of DIPEA and 2A (210 mg, 0.71 mmol) were added, and the mixture was reacted at 80°C for 5 h. The reaction liquid was cooled to room temperature, 50 mL of water was added, the mixture was filtered, and the solid was collected. The filter cake was washed with 20 mL of water, dissolved in 100 mL of DCM, the mixture was dried over anhydrous sodium sulfate, and concentrated under reduced pressure. The crude product was separated and purified with silica gel column chromatography (DCM/MeOH (v/v) = 10 : 1) to afford compound 3 (75 mg, two-step yield from compound 1c: 23%).

$^1$H NMR (400 MHz, $CDCl_3$) δ 9.49 (br.s, 1H), 8.08 (s, 1H), 7.93 (d, 1H), 7.85 - 7.70 (m, 4H), 7.39 (d, 1H), 6.84 (d, 1H), 4.96 - 4.87 (m, 1H), 4.54 - 4.43 (m, 2H), 4.24 - 4.14 (m, 2H), 3.85 - 3.72 (m, 1H), 2.96 - 2.64 (m, 3H), 2.18 - 2.06 (m, 1H), 1.92 (s, 6H).

LCMS m/z = 676.2 $[M+1]^+$.

**Example 4: Preparation of compound 4**

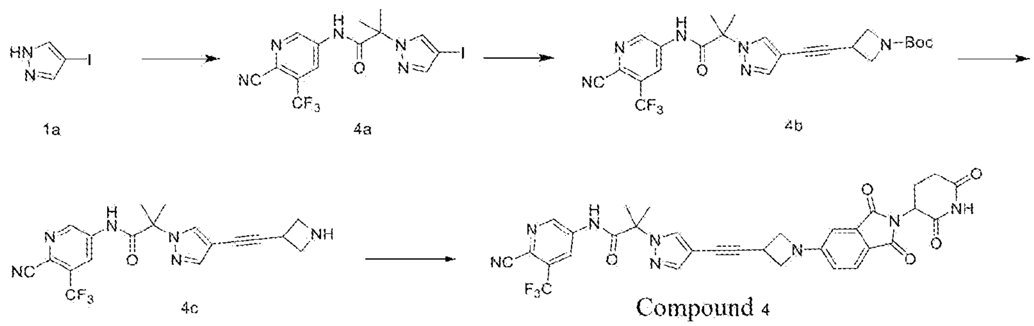

1a 4a 4b

4c Compound 4

Step 1: N-(6-cyano-5-(trifluoromethyl)pyridin-3-yl)-2-(4-iodo-1H-pyrazol-1-yl)-2-methylpropanamide (4a)

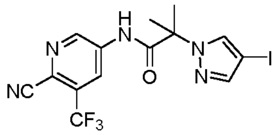

2-bromo-N-(6-cyano-5-(trifluoromethyl)pyridin-3-yl)-2-methylpropanamide (see WO 2020063407 for the synthesis method) (1.5 g, 4.47 mmol) was dissolved in 30.0 mL of acetonitrile. 1a (868.6 mg, 4.48 mmol) and cesium carbonate (4.36 g, 13.38 mmol) were added, and the mixture was stirred at 90°C for 4 h. The reaction liquid was cooled to room temperature, 60 mL of water was added, the mixture was extracted with ethyl acetate (40 mL × 3), the organic phase was washed with saturated aqueous sodium chloride solution (50 mL × 2), dried over anhydrous sodium sulfate, and concentrated under reduced pressure. The crude product was separated and purified with silica gel column chromatography (petroleum ether/ethyl acetate (v/v) = 5 : 1) to afford 4a (1.35 g, yield: 67%).

LCMS m/z = 450.0 $[M+1]^+$.

Step 2: tert-butyl 3-((1-(1-((6-cyano-5-(trifluoromethyl)pyridin-3-yl)amino)-2-methyl-1- oxopropan-2-yl)-1H-pyrazol-4-yl-4-yl)ethynyl)azetidine-1-carboxylate (4b)

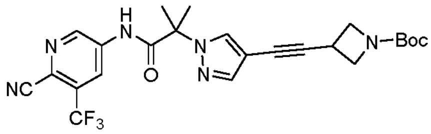

4a (1.0 g, 2.23 mmol) was dissolved in 25 mL of ultra-dry THF, tert-butyl 3-ethynylazetidine-1-carboxylate (606.35 mg, 3.35 mmol) was added, TEA (674.0 mg, 6.66 mmol) was added, nitrogen replacement was performed three times, and $PdCl_2(PPh_3)_2$ (312.6 mg, 0.45 mmol) and CuI (85.5 mg, 0.45 mmol) were added in sequence, then nitrogen replacement was performed three times, and the mixture was reacted at 60°C for 2.5 h. The reaction liquid was cooled to room temperature, 60 mL of water and 30 mL of ethyl acetate were added to the reaction liquid, the liquid separation was conducted, the aqueous phase was extracted with ethyl acetate (40 mL × 2), the organic phases were combined, the organic phase was washed with saturated aqueous sodium chloride solution (50 mL × 2), dried over anhydrous sodium sulfate, and concentrated under reduced pressure. The residue was separated and purified with silica gel column chromatography (petroleum ether/ethyl acetate (v/v) = 2 : 1-1.5 : 1) to afford 4b (850 mg, yield: 76%).

$^1H$ NMR (400 MHz, $CDCl_3$) δ 9.91 (s, 1H), 8.73 (d, 1H), 8.60 (d, 1H), 7.81 (s, 1H), 7.77 (s, 1H), 4.24 - 4.16 (m, 2H), 4.01 - 3.93 (m, 2H), 3.54 - 3.43 (m, 1H), 1.93 (s, 6H), 1.44 (s, 9H).

Step 3: 2-(4-(azetidin-3-ylethynyl)-1H-pyrazol-1-yl)-N-(6-cyano-5-(trifluoromethyl)pyridin-3-yl)-2-methylpropanamide (4c)

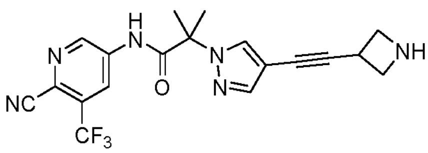

4b (250 mg, 0.498 mmol) was dissolved in 10 mL of DCM, and 4.0 mL of trifluoroacetic acid was added. The mixture was stirred at room temperature for 3 h. Saturated aqueous sodium bicarbonate solution was slowly added to the reaction liquid to adjust the pH to 9, and the liquid separation was conducted. The aqueous phase was extracted with DCM (20 mL × 2), and the organic phases were combined, washed with saturated aqueous sodium chloride solution (20 mL × 2), dried over anhydrous sodium sulfate, and concentrated under reduced pressure. The residue was separated and purified with silica gel column chromatography (DCM/MeOH (v/v) = 9 : 1) to afford 4c (90 mg, yield: 45%).

Step 4: N-(6-cyano-5-(trifluoromethyl)pyridin-3-yl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 4)

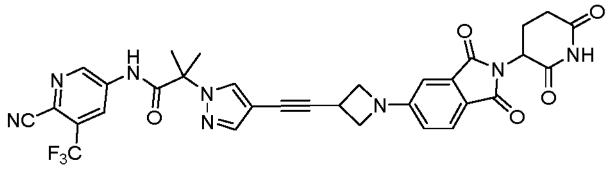

4c (70.0 mg, 0.174 mmol) was dissolved in 6.0 mL of DMSO, DIPEA (67.3 mg, 0.52 mmol) and 1C (57.6 mg, 0.209 mmol) were added, and the mixture was stirred at 90°C for 3 h. The reaction liquid was cooled to room temperature, 24 mL of water was added, the mixture was filtered, the solid was collected, and the solid was separated and purified with prep-TLC (developing solvent: ethyl acetate/petroleum ether (v/v) = 2 : 1) to afford compound 4 (37 mg, yield: 32%).

$^1$H NMR (400 MHz, $CDCl_3$) δ 9.91 (s, 1H), 8.72 (d, 1H), 8.60 (d, 1H), 7.93 (s, 1H), 7.83 (s, 1H), 7.79 (s, 1H), 7.67 (d, 1H), 6.81 (d, 1H), 6.56 (dd, 1H), 4.93 (dd, 1H), 4.41 - 4.32 (m, 2H), 4.10 - 4.02 (m, 2H), 3.86 - 3.75 (m, 1H), 2.95 - 2.66 (m, 3H), 2.18 - 2.08 (m, 1H), 1.93 (s, 6H).

LCMS m/z = 659.2 $[M+1]^+$.

**Example 5: Preparation of compound 5**

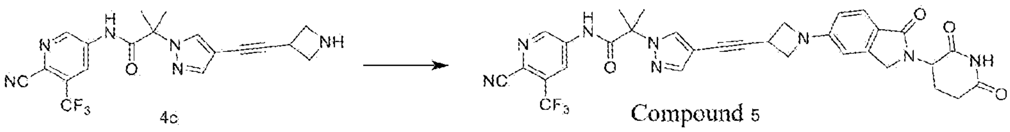
4c
Compound 5

4c (0.140 g, 0.348 mmol), 3-(5-bromo-1-oxoisoindolin-2-yl)piperidine-2,6-dione (0.150 g, 0.46 mmol), XPhos (0.017 g, 0.036 mmol) and cesium carbonate (0.342 g, 1.05 mmol) were suspended in 8 mL of 1,4-dioxane, and XPhos Pd G3 (0.030 g, 0.035 mmol) was added under nitrogen protection. After the addition, nitrogen replacement was performed three times, and the mixture was reacted at 95°C for 3 h. The reaction liquid was cooled to room temperature, 1 mol/L dilute hydrochloric acid was slowly added dropwise to adjust the pH to 7, DCM (30 mL) was added, the liquid separation was conducted, the organic layer was washed with 10 mL of saturated aqueous sodium chloride solution, dried over anhydrous sodium sulfate, and concentrated under reduced pressure. The crude product obtained was separated and purified with prep-TLC (developing solvent: ethyl acetate/petroleum ether = 4 : 1) to afford compound 5(0.030 g, yield: 13%).

$^1$H NMR (400 MHz, $CDCl_3$) δ 9.89 (s, 1H), 8.72 (d, 1H), 8.60 (d, 1H), 7.92 (s, 1H), 7.82 (s, 1H), 7.78 (s, 1H), 7.73 (d, 1H), 6.54 (d, 1H), 6.48 (s, 1H), 5.18 (dd, , 1H), 4.45 -4.20 (m, 4H), 4.03 - 3.95 (m, 2H), 3.84 - 3.74 (m, 1H), 2.96 - 2.75 (m, 2H), 2.38 - 2.26 (m, 1H), 2.24 - 2.15 (m, 1H), 1.93 (s, 6H).

LCMS m/z = 645.2 $[M+1]^+$.

**Example 6: Preparation of compound 6**

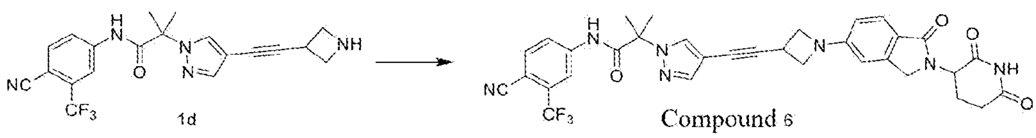
1d
Compound 6

Crude 1d (0.2 g), 3-(5-bromo-1-oxoisoindolin-2-yl)piperidine-2,6-dione (0.21 g, 0.64 mmol), XPhos (0.028 g, 0.059 mmol) and cesium carbonate (0.487 g, 1.49 mmol) were suspended in 8 mL of 1,4-dioxane, and XPhos Pd G3 (0.050 g, 0.06 mmol) was added under nitrogen protection. After the addition, nitrogen replacement was performed three

times, and the mixture was reacted at 95°C for 3 h. The reaction liquid was cooled to room temperature, 1 mol/L dilute hydrochloric acid was slowly added dropwise to adjust the pH to 7, DCM (30 mL) was added, the liquid separation was conducted, the organic layer was washed with 10 mL of saturated aqueous sodium chloride solution, dried over anhydrous sodium sulfate, and concentrated under reduced pressure. The crude product obtained was purified with prep-TLC (developing solvent: ethyl acetate/petroleum ether = 4 : 1) to afford compound 6 (0.087 g, two-step yield from compound 1c: 26%).

$^1H$ NMR (400 MHz, $CDCl_3$) δ 9.49 (s, 1H), 8.05 (s, 1H), 7.95 - 7.89 (m, 1H), 7.82 - 7.69 (m, 5H), 6.55 - 6.48 (m, 1H), 6.45 (s, 1H), 5.18 (dd, 1H), 4.39 (d, 1H), 4.34 - 4.27 (m, 2H), 4.24 (d, 1H), 4.02 - 3.94 (m, 2H), 3.83 - 3.72 (m, 1H), 2.96 - 2.75 (m, 2H), 2.39 - 2.25 (m, 1H), 2.24 - 2.14 (m, 1H), 1.92 (s, 6H).

LCMS m/z = 644.3 $[M+1]^+$.

**Example 7: Preparation of compound 7**

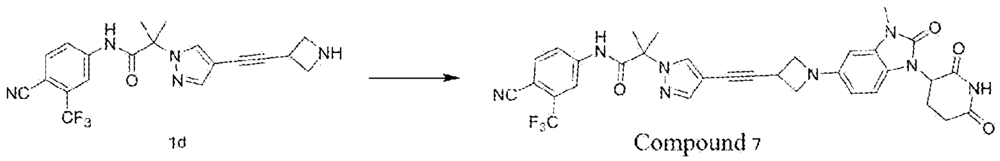

The above crude 1d (0.5 g), 3-(5-bromo-3-methyl-2-oxo-2,3-dihydro-1H-benzo[d]imidazol-1-yl)piperidine-2,6-dione (see WO 2020113233 for the synthesis method) (0.3 g, 0.89 mmol) and RuPhos (0.058 g, 0.124 mmol) were suspended in 10 mL of toluene, and RuPhosPdG3 (0.1 g, 0.12 mmol) was added under nitrogen protection, and after the addition was completed, nitrogen replacement was performed three times, a solution of 1 mol/L LiHMDS in THF (3.75 mL, 3.75 mmol) was added, nitrogen replacement was performed three times, and the mixture was reacted at 95°C for 2 h. The reaction liquid was cooled to room temperature, 1 mol/L dilute hydrochloric acid was slowly added dropwise to adjust the pH to 7, DCM (30 mL) was added, the liquid separation was conducted, the organic layer was washed with 10 mL of saturated sodium chloride solution, dried over anhydrous sodium sulfate, and concentrated under reduced pressure. The crude product obtained was purified with prep-TLC (developing solvent: ethyl acetate/petroleum ether (v/v) = 9 : 1) to afford compound 7 (0.017 g, two-step yield from compound 1c: 2%).

$^1H$ NMR (400 MHz, $CDCl_3$) δ 9.40 (s, 1H), 8.30 (s, 1H), 7.89 - 7.81 (m, 1H), 7.79 - 7.62 (m, 4H), 6.59 (d, 1H), 6.20 - 6.05 (m, 2H), 5.10 (dd, 1H), 4.20 - 4.10 (m, 2H), 3.85 - 3.73 (m, 2H), 3.72 - 3.58 (m, 1H), 3.31 (s, 3H), 2.90 - 2.52 (m, 3H), 2.21 - 2.07 (m, 1H), 1.84 (s, 6H).

LCMS m/z = 659.2 $[M+1]^+$.

**Example 8: Preparation of compound 8**

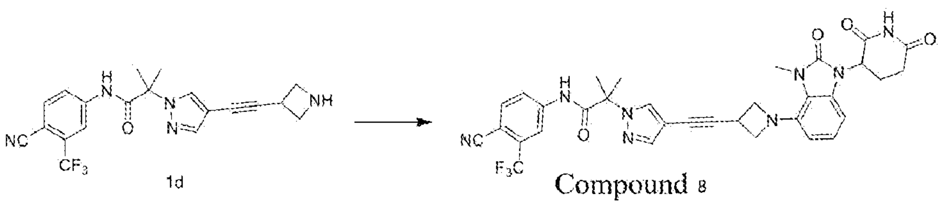

The above crude 1d (0.24 g), 3-(4-bromo-3-methyl-2-oxo-2,3-dihydro-1H-benzo[d]imidazol-1-yl)piperidine-2,6-dione (see WO 2020113233 for the synthesis method) (0.280 g, 0.83 mmol) and RuPhos (0.028 g, 0.060 mmol) were suspended in 10 mL of toluene, and RuPhosPdG3 (0.050 g, 0.06 mmol) was added under nitrogen protection, and after the addition was completed, nitrogen replacement was performed three times, a solution of 1 mol/L LiHMDS in THF (1.8 mL, 1.80 mmol) was added, nitrogen replacement was performed three times, and the mixture was reacted at 95°C for 2 h. The reaction liquid was cooled to room temperature, 1 mol/L hydrochloric acid was slowly added dropwise to adjust the pH to 7, DCM (30 mL) was added, the liquid separation was conducted, the organic layer was washed with 10 mL of saturated sodium chloride solution, dried over anhydrous sodium sulfate, and concentrated under reduced pressure. The crude product obtained was purified with prep-TLC (developing solvent: ethyl acetate/petroleum ether (v/v) = 9 : 1)

to afford compound 8 (0.028 g, two-step yield from compound 1c: 7%).

$^1$H NMR (400 MHz, $CDCl_3$) δ 9.50 (s, 1H), 8.26 (s, 1H), 7.98 - 7.90 (m, 1H), 7.86 - 7.70 (m, 4H), 7.01 (t, 1H), 6.76 (d, 1H), 6.51 (d, 1H), 5.23 - 5.13 (m, 1H), 4.16 - 4.04 (m, 2H), 3.96 - 3.84 (m, 2H), 3.75 (s, 3H), 3.71 - 3.59 (m, 1H), 2.99 - 2.65 (m, 3H), 2.26 - 2.14 (m, 1H), 1.92 (s, 6H).

LCMS m/z = 659.3 $[M+1]^+$.

**Example 9: Preparation of compound 9**

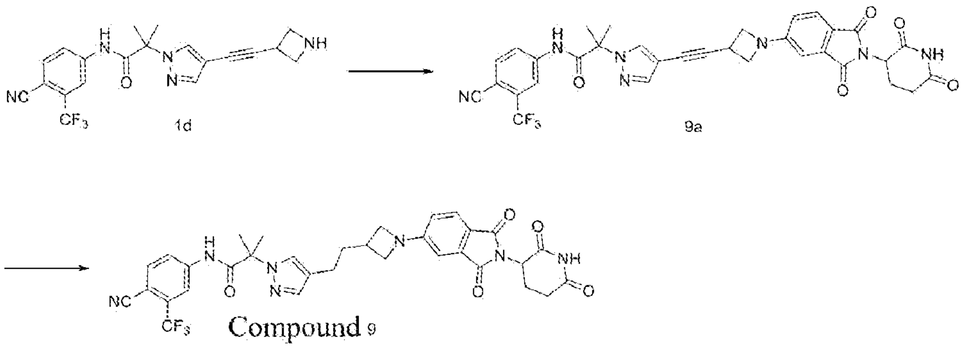
1d
9a
Compound 9

Step 1: N-(4-cyano-3-(trifluoromethyl)phenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (9a)

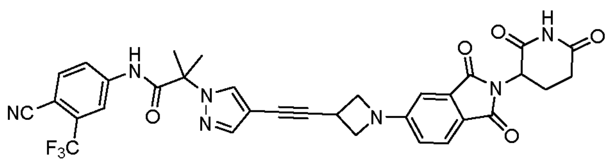

Crude 1d (0.17 g) was dissolved in 10 mL of DMSO, 0.5 mL of DIPEA and 1C (180 mg, 0.65 mmol) were added, and the mixture was stirred at 80°C for 5 h. The reaction liquid was cooled to room temperature, 20 mL of water was added, the mixture was filtered, and the solid was collected and washed with 20 mL of water, the solid was dissolved in 50 mL of DCM, the mixture was dried over anhydrous sodium sulfate, and concentrated under reduced pressure. The crude product was separated and purified with silica gel column chromatography (DCM/MeOH (v/v) = 15 : 1) to afford 9a (80 mg, two-step yield from compound 1c: 28%).

LCMS m/z = 658.2 $[M+1]^+$.

Step 2: N-(4-cyano-3-(trifluoromethyl)phenyl)-2-(4-(2-(1-(2-(2,6-dioxopiperidin-3-yl) 1,3-dioxo-2,3-dihydro-1H-isoindol-5-yl)azetidin-3-yl)ethyl)-1H-pyrazol-1-yl)-2-methylpropanamide (Compound 9)

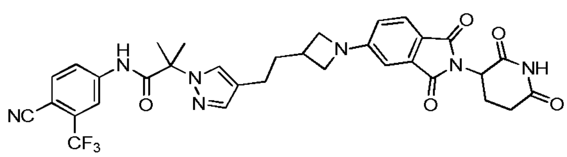

9a (0.070 g, 0.107 mmol) was dissolved in 3 mL of THF, 10% palladium on carbon (35 mg) was added, and the mixture was reacted at room temperature for 3 h under the atmosphere of hydrogen balloon. The reaction system was filtered by suction under reduced pressure, and the filtrate was concentrated under reduced pressure. The crude product was separated and purified with silica gel column chromatography (MeOH/DCM (v/v) = 1 : 19) to afford compound 9 (0.070 g, yield: 99%).

$^1H$ NMR (400 MHz, $CDCl_3$) δ 9.82 (s, 1H), 8.07 - 7.99 (m, 1H), 7.98 - 7.92 (m, 1H), 7.85 - 7.70 (m, 2H), 7.67 - 7.58 (m, 2H), 7.48 (s, 1H), 6.75 (d, 1H), 6.49 (dd, 1H), 4.93 (dd, 1H), 4.20 - 4.09 (m, 2H), 3.72 - 3.63 (m, 2H), 2.98 - 2.64 (m, 4H), 2.58 - 2.49 (m, 2H), 2.19 - 2.06 (m, 1H), 2.06 - 1.95 (m, 2H), 1.93 (s, 6H).

LCMS m/z = 662.3 $[M+1]^+$.

**Example 10: Preparation of compound 10**

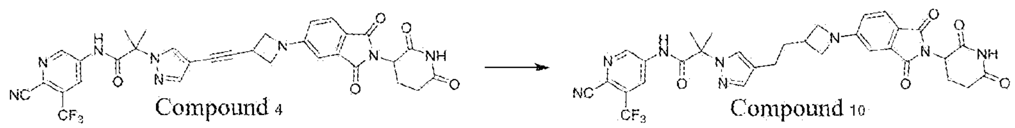

**Compound** 4 (0.1 g, 0.152 mmol) was dissolved in 3 mL of THF, 10% palladium on carbon (50 mg) was added, and the mixture was reacted at room temperature for 4 h under the atmosphere of hydrogen balloon. The reaction system was filtered by suction under reduced pressure, and the filtrate was concentrated under reduced pressure. The crude product was separated and purified with silica gel column chromatography (MeOH/DCM (v/v) = 1 : 19) to afford compound 10 (0.09 g, yield :89%).

$^1H$ NMR (400 MHz, $CDCl_3$) δ 10.25 (s, 1H), 8.73 (d, 1H), 8.64 (d, 1H), 8.03 (s, 1H), 7.68 - 7.58 (m, 2H), 7.49 (s, 1H), 6.76 (d, 1H), 6.50 (dd, 1H), 4.98 - 4.87 (m, 1H), 4.20 - 4.10 (m, 2H), 3.72 - 3.63 (m, 2H), 2.94 - 2.66 (m, 4H), 2.58 - 2.49 (m, 2H), 2.18 - 2.07 (m, 1H), 2.07 - 1.96 (m, 2H), 1.94 (s, 6H).

LCMS m/z = 663.3 $[M+1]^+$.

**Example 11: Preparation of compound 11**

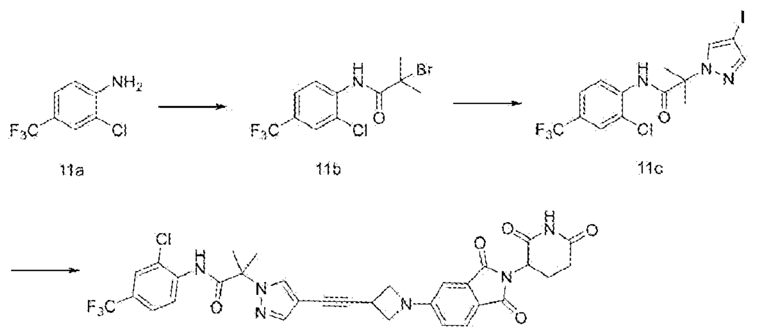

Compound 11

Step 1: 2-bromo-N-(2-chloro-4-(trifluoromethyl)phenyl)-2-methylpropanamide (11b)

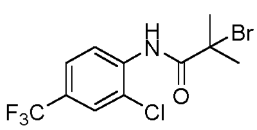

2-bromo-2-methylpropionic acid (2.00 g, 11.98 mmol) was added to a reaction flask, 20 mL of THF was added under nitrogen atmosphere, the mixture was cooled to 0°C, and thionyl chloride (1.10 mL, 15.16 mmol) was added. After the ice bath was removed, the mixture was slowly warmed to room temperature and the stirring was continued at room temperature for 2 h. The reaction system was cooled to 0°C, TEA (2.50 mL, 18.00 mmol) was slowly added dropwise, the mixture was stirred at 0°C for 20 min, then 10 mL of a solution of 11a (1.65 mL, 11.9 mmol) in THF was added at 0°C, and after the dropwise addition was completed, the mixture was reacted at 50°C for 3 h. The reaction system was cooled to 0°C, 10 mL of saturated sodium bicarbonate solution was added to quench the reaction, the mixture was

extracted with ethyl acetate (50 mL × 3), the organic phase was washed with 50 mL of saturated sodium chloride solution, dried over anhydrous sodium sulfate, and concentrated under reduced pressure. The crude product was recrystallized with petroleum ether to afford 11b (2.9 g, yield: 71%).

Step 2: N-(2-chloro-4-(trifluoromethyl)phenyl)-2-(4-iodo-1H-pyrazol-1-yl)-2-methylpropanamide (11c)

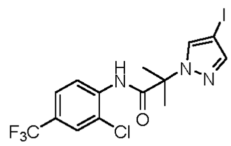

11b (2.90 g, 8.42 mmol), 4-iodo-1H-pyrazole (1.64 g, 8.45 mmol) and cesium carbonate (5.51 g, 16.91 mmol) were added to the reaction flask respectively, 20 mL of acetonitrile was added, and the mixture was reacted at 50°C for 3 h. The reaction system was cooled to room temperature and filtered by suction. The filtrate was concentrated under reduced pressure. The crude product was separated and purified with silica gel column chromatography (ethyl acetate/petroleum ether (v/v) = 1 : 5) to afford 11c (2.22 g, yield: 58%).

Step 3: N-(2-chloro-4-(trifluoromethyl)phenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 11)

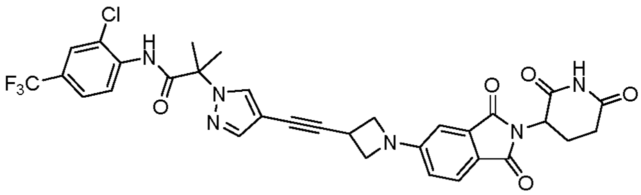

Under nitrogen atmosphere, 11c (270 mg, 0.59 mmol), intermediate 1 (200 mg), and $PdCl_2(PPh_3)_2$ (41.5 mg, 0.059 mmol) and CuI (22.6 mg, 0.12 mmol) were added to the reaction flask respectively, 10 mL of DMF and DIPEA (0.3 mL, 1.8 mmol) were added, and the mixture was reacted at 50°C for 2 h. The reaction liquid was cooled to room temperature, the reaction liquid was added dropwise into 20 mL of ice water, the mixture was filtered by suction, washed with 10 mL of water, the filter cake was collected, the filter cake was dissolved with 20 mL of DCM, the mixture was dried over anhydrous sodium sulfate, concentrated under reduced pressure and then the crude product was separated and purified with silica gel column chromatography (ethyl acetate/petroleum ether (v/v) = 1 : 1), and the obtained crude product was separated and purified with silica gel column chromatography (DCM/MeOH (v/v) = 20 : 1) to afford compound 11 (118 mg, yield from compound 11c: 30%).

$^1$H NMR (400 MHz, $CDCl_3$) δ 9.18 (s, 1H), 8.51 (d, 1H), 8.00 (s, 1H), 7.80 (s, 2H), 7.67 (d, 1H), 7.62 - 7.56 (m, 1H), 7.54 - 7.46 (m, 1H), 6.81 (d, 1H), 6.56 (dd, 1H), 5.00 - 4.88 (m, 1H), 4.43 - 4.27 (m, 2H), 4.14 - 4.00 (m, 2H), 3.89 - 3.74 (m, 1H), 2.96 - 2.63 (m, 3H), 2.20 - 2.06 (m, 1H), 1.95 (s, 6H).

LCMS m/z = 667.2 $[M+1]^+$.

Example 12: Preparation of compound 12

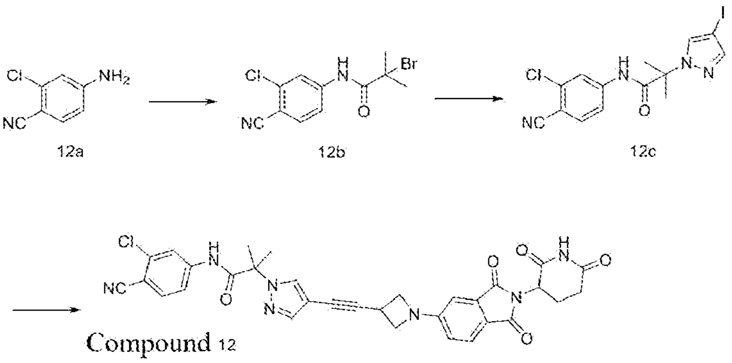

12a 12b 12c

Compound 12

Step 1: 2-bromo-N-(3-chloro-4-cyanophenyl)-2-methylpropanamide (12b)

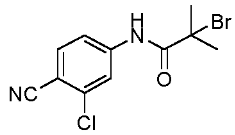

2-bromo-2-methylpropionic acid (2.00 g, 11.98 mmol) was added to a reaction flask, 20 mL of THF was added under nitrogen atmosphere, the mixture was cooled to 0°C, thionyl chloride (1.10 mL, 15.16 mmol) was added, the mixture was slowly warmed to room temperature and stirred for 2 h. The reaction system was cooled to 0°C, TEA (2.50 mL, 18.00 mmol) was slowly added dropwise, the stirring was continued at 0°C for 20 min, then a solution of 12a (1.65 g, 10.8 mmol) in THF (10 mL) was added dropwise at 0°C, and after the dropwise addition was completed, the mixture was reacted at 50°C for 3 h. The reaction system was cooled to 0°C, 10 mL of saturated sodium bicarbonate solution was added to quench the reaction, the mixture was extracted with ethyl acetate (50 mL × 3), the organic phase was washed with 50 mL of saturated sodium chloride solution, dried over anhydrous sodium sulfate, and concentrated under reduced pressure, and the crude product was slurried with 10 mL of a mixed solvent of ethyl acetate/petroleum ether (v/v) = 1 : 10 to afford crude 12b (3.3 g).

Step 2: N-(3-chloro-4-cyanophenyl)-2-(4-iodo-1H-pyrazol-1-yl)-2-methylpropanamide (12c)

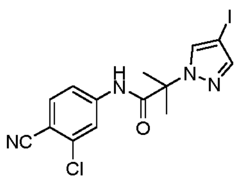

The above crude 12b (3.3 g), 4-iodo-1H-pyrazole (2.13 g, 10.98 mmol) and cesium carbonate (7.17 g, 22.00 mmol) were added to the reaction flask respectively, 20 mL of acetonitrile was added, and the mixture was reacted at 50°C for 3 h. The reaction system was cooled to room temperature and filtered by suction. The filtrate was concentrated under reduced pressure. The crude product was separated and purified by silica gel column chromatography (ethyl acetate/petroleum ether (v/v) = 1 : 5) to afford 12c (2.35 g, two-step yield from compound 12a: 52%).

Step 3: N-(3-chloro-4-cyanophenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 12)

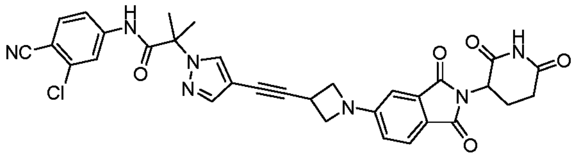

Under nitrogen atmosphere, 12c (244 mg, 0.59 mmol), intermediate 1 (200 mg), and $PdCl_2(PPh_3)_2$ (41.5 mg, 0.059 mmol) and CuI (22.6 mg, 0.12 mmol) were added to the reaction flask respectively, 10 mL of DMF and DIPEA (0.3 mL, 1.8 mmol) were added, and the mixture was reacted at 50°C for 2 h. The reaction liquid was cooled to room temperature, the reaction liquid was added dropwise into 20 mL of ice water, the mixture was filtered by suction, washed with 10 mL of water, the filter cake was collected, the filter cake was dissolved with 20 mL of DCM, the mixture was dried over anhydrous sodium sulfate, concentrated under reduced pressure and then the crude product was separated and purified with silica gel column chromatography (ethyl acetate/petroleum ether (v/v) = 1 : 1), and the obtained crude product was separated and purified with silica gel column chromatography (DCM/MeOH (v/v) = 20 : 1) to afford compound 12 (123 mg, yield from compound 12c: 33%).

$^1H$ NMR (400 MHz, $CDCl_3$) δ 9.23 (s, 1H), 8.36 - 8.20 (m, 1H), 7.85 - 7.73 (m, 3H), 7.66 (d, 1H), 7.54 (d, 1H), 7.38 (dd, 1H), 6.79 (s, 1H), 6.55 (dd, 1H), 4.98 - 4.88 (m, 1H), 4.41 - 4.31 (m, 2H), 4.12 - 4.00 (m, 2H), 3.88 - 3.74 (m, 1H), 2.96 - 2.64 (m, 3H), 2.18 - 2.06 (m, 1H), 1.91 (s, 6H).

LCMS m/z = 624.2 $[M+1]^+$.

**Example 13: Preparation of compound 13**

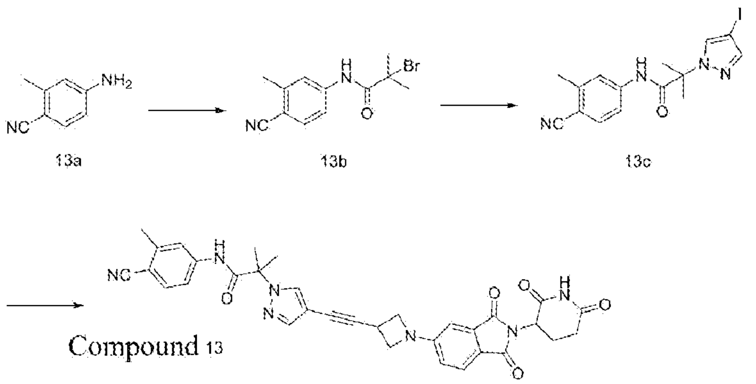


Step 1: 2-bromo-N-(4-cyano-3-methylphenyl)-2-methylpropanamide (13b)

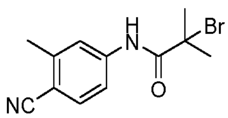

2-bromo-2-methylpropionic acid (1.00 g, 5.99 mmol) was added to a reaction flask, THF (10 mL) was added under nitrogen atmosphere, the mixture was cooled to 0°C, and thionyl chloride (0.55 mL, 7.58 mmol) was added. After the ice bath was removed, the mixture was slowly warmed to room temperature and the stirring was continued at room temperature for 2 h. The reaction system was cooled to 0°C, TEA (1.25 mL, 9.00 mmol) was slowly added dropwise, the mixture was stirred at 0°C for 20 min, then a solution of 13a (0.79 g, 6.0 mmol) in THF was added dropwise at 0°C, and after the dropwise addition was completed, the mixture was reacted at 50°C for 3 h. The reaction system was cooled to 0°C, 10 mL of saturated sodium bicarbonate solution was added to quench the reaction, the mixture was extracted with ethyl acetate (20 mL × 3), the organic phase was washed with 50 mL of saturated sodium chloride solution, dried over anhydrous sodium sulfate, and concentrated under reduced pressure, and the crude product was slurried with 10 mL of a mixed solvent of ethyl acetate/petroleum ether (v/v) = 1 : 10 to afford crude 13b (1.50 g).

Step 2: N-(4-cyano-3-methylphenyl)-2-(4-iodo-1H-pyrazol-1-yl)-2-methylpropanamide (13c)

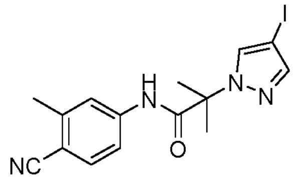

The above crude 13b (1.50 g), 4-iodo-1H-pyrazole (1.03 g, 5.31 mmol) and cesium carbonate (3.47 g, 10.65 mmol) were added to the reaction flask respectively, 10 mL of acetonitrile was added, and the mixture was reacted at 50°C for 3 h. The reaction system was cooled to room temperature and filtered by suction. The filtrate was concentrated under reduced pressure. The crude product was separated and purified by silica gel column chromatography (ethyl acetate/petroleum ether (v/v) = 1 : 5) to afford 13c (1.52 g, two-step yield from compound 13a: 64%).

Step 3: N-(4-cyano-3-methylphenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 13)

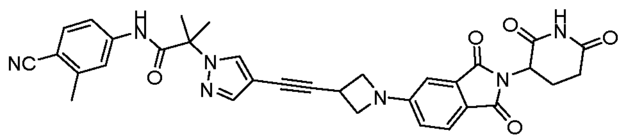

13c (0.2 g, 0.51 mmol), intermediate 1 (0.17 g), $PdCl_2(PPh_3)_2$ (50 mg, 0.071 mmol) and CuI (50 mg, 0.26 mmol) were added to 5 mL of DMF, 0.5 mL of DIPEA was added, and the mixture was reacted at 50°C for 2 h. The reaction liquid was cooled to room temperature, 20 mL of water was added, and the mixture was extracted with ethyl acetate (50 mL × 3), the organic phase was washed with 50 mL of saturated sodium chloride solution, dried over anhydrous sodium sulfate, concentrated under reduced pressure, and the crude product was separated and purified with silica gel column chromatography (DCM/MeOH (v/v) = 100 : 1-20 : 1) to afford compound 13 (0.05 g, yield from compound 13c: 16%).

$^1H$ NMR (400 MHz, $CDCl_3$) δ 8.95 (s, 1H), 7.92 (s, 1H), 7.79 (d, 2H), 7.67 (d, 1H), 7.51 (d, 1H), 7.46 - 7.42 (m, 1H), 7.34 (dd, 1H), 6.81 (d, 1H), 6.56 (dd, 1H), 4.98 - 4.88 (m, 1H), 4.40 - 4.32 (m, 2H), 4.10 - 4.02 (m, 2H), 3.86 - 3.74 (m, 1H), 2.95 - 2.61 (m, 3H), 2.50 (s, 3H), 2.18 - 2.08 (m, 1H), 1.91 (s, 6H).

LCMS m/z = 604.1 $[M+1]^+$.

**Example 14: Preparation of compound 14**

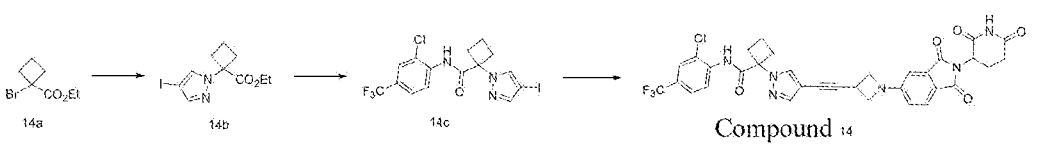

14a 14b 14c Compound 14

Step 1: ethyl 1-(4-iodo-1H-pyrazol-1-yl)cyclobutane-1-carboxylate (14b)

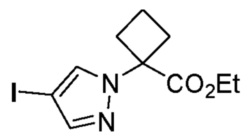

14a (4.1 g, 19.80 mmol) and 4-iodo-1H-pyrazole (4.22 g, 21.75 mmol) were dissolved in 40 mL of acetonitrile, cesium carbonate (9.68 g, 29.70 mmol) was added, and the mixture was reacted at 80°C for 3 h. The reaction liquid was cooled to room temperature, 30 mL of water and 100 mL of ethyl acetate were added, the liquid separation was conducted, the organic phase was washed with 30 mL of saturated sodium chloride solution, dried over anhydrous sodium sulfate, and concentrated under reduced pressure. The crude product was separated and purified with silica gel chromatography column (ethyl acetate/petroleum ether (v/v) = 1 : 9) to afford 14b (2.40 g, yield: 38%).

$^1H$ NMR (400 MHz, $CDCl_3$) δ 7.55 (s, 2H), 4.19 (q, 2H), 2.95 - 2.70 (m, 4H), 2.26 - 1.96 (m, 2H), 1.23 (t, 3H).

LCMS m/z = 321.1 $[M+1]^+$.

Step 2: N-(2-chloro-4-(trifluoromethyl)phenyl)-1-(4-iodo-1H-pyrazol-1-yl)cyclobutane-1-carboxamide (14c)

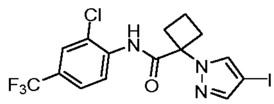

14b (2.30 g, 7.18 mmol) was dissolved in THF (20 mL), 4 mL of water and lithium hydroxide monohydrate (0.6 g, 14.3 mmol) were added, and the mixture was reacted at room temperature for 30 min. 1 mol/L hydrochloric acid was added dropwise to the reaction liquid to adjust the pH to 6, 50 mL of ethyl acetate was added, the liquid separation was conducted, the organic phase was washed with 20 mL of saturated sodium chloride solution, dried over anhydrous sodium sulfate, and concentrated under reduced pressure to afford crude 1 (2.0 g). Crude 1 (0.51 g) was dissolved in DCM (10 mL), 1-chloro-N,N,2-trimethylpropenylamine (0.35 g, 2.62 mmol) was slowly added dropwise, and the mixture was reacted at room temperature for 2 h. TEA (0.53 g, 5.24 mmol) and 2-chloro-4-(trifluoromethyl)aniline (0.34 g, 1.74 mmol) were added to the reaction liquid, and the mixture was reacted at room temperature for 3 h. 20 mL of saturated aqueous sodium bicarbonate solution was added to the reaction liquid, the mixture was extracted with 100 mL of ethyl acetate, the organic phase was washed with 50 mL of water, dried over anhydrous sodium sulfate, and concentrated under reduced pressure. The crude product was separated and purified with silica gel chromatography column (ethyl acetate/petroleum ether (v/v) = 1 : 3) to afford 14c (0.45 g, yield: 52%).

Step 3: N-(2-chloro-4-(trifluoromethyl)phenyl)-1-(4-((1-(2-(2,6-dioxopiperidin-3-yl)-1,3-dioxoisoindolin-5-yl)azetidin-3-yl)ethynyl)-1H-pyrazol-1-yl)cyclobutane-1-carboxamide (Compound 14)

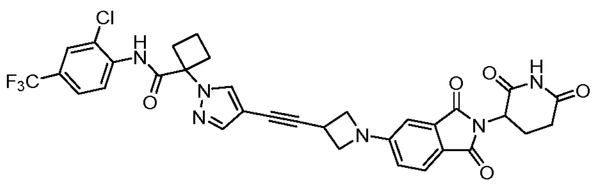

14c (0.24 g, 0.51 mmol) and intermediate 1 (0.22 g) were dissolved in 8 mL of ultra-dry DMF, DIPEA (0.25 g, 1.93 mmol) was added, nitrogen replacement was performed 3 times, $Pd(PPh_3)_2Cl_2$ (0.046 g, 0.066 mmol) and CuI (0.012 g, 0.063 mmol) were added, nitrogen replacement was performed 3 times, and the mixture was stirred at 50°C for 1 h. The reaction liquid was cooled to room temperature, 50 mL of water was added to the reaction liquid, the mixture was filtered by suction, washed with 10 mL of water, the filter cake was collected, the filter cake was dissolved with 20 mL of DCM, the mixture was dried over anhydrous sodium sulfate, and concentrated under reduced pressure and then the crude product was separated and purified with silica gel column chromatography (ethyl acetate/petroleum ether (v/v) = 1 : 3-1 : 1), and the obtained crude product was separated and purified with prep-TLC (ethyl acetate/petroleum ether (v/v) = 3 : 2) to afford compound 14 (80 mg, yield: 18%).

$^1H$ NMR (400 MHz, $CDCl_3$) δ 8.69 (s, 1H), 8.54 (d, 1H), 8.10 (s, 1H), 7.79 (s, 1H), 7.71 (s, 1H), 7.67 (d, 1H), 7.60 - 7.55 (m, 1H), 7.54 - 7.47 (m, 1H), 6.80 (d, 1H), 6.55 (dd, 1H), 4.98 - 4.89 (m, 1H), 4.42 - 4.30 (m, 2H), 4.12 - 4.02 (m, 2H), 3.87 - 3.74 (m, 1H), 3.14 - 2.99 (m, 2H), 2.96 - 2.65 (m, 5H), 2.30 - 2.04 (m, 3H).

LCMS m/z = 679.4 $[M+1]^+$.

**Example 15: Preparation of compound 15**

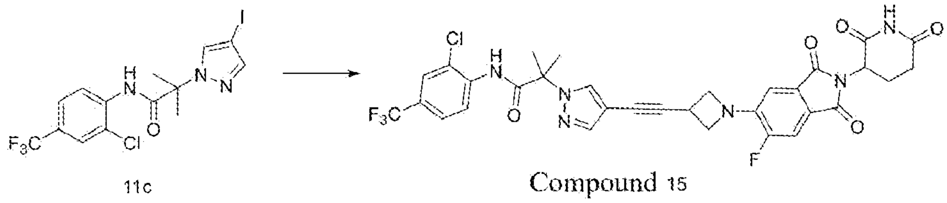

11c

Compound 15

Under nitrogen atmosphere, 11c (230 mg, 0.50 mmol), crude intermediate 2 (180 mg), and $PdCl_2(PPh_3)_2$ (36 mg, 0.051 mmol) and CuI (9.7 mg, 0.051 mmol) were added to the reaction flask respectively, 8 mL of DMF and DIPEA (0.2 g, 1.55 mmol) were added, and the mixture was reacted at 50°C for 1 h. The reaction liquid was cooled to room temperature, 50 mL of water was added to the reaction liquid, the mixture was filtered by suction, washed with 10 mL of water, the filter cake was collected, the filter cake was dissolved with 20 mL of DCM, the mixture was dried over anhydrous sodium sulfate, and concentrated under reduced pressure and then the crude product was separated and purified with silica gel column chromatography (ethyl acetate/petroleum ether (v/v) = 1 : 4-1 : 1), and the obtained crude product was separated and purified with prep-TLC (ethyl acetate/petroleum ether (v/v) = 3 : 2) to afford compound 15 (110 mg, yield: 32%).

$^1H$ NMR (400 MHz, $CDCl_3$) δ 9.18 (s, 1H), 8.51 (d, 1H), 7.95 (s, 1H), 7.80 (s, 2H), 7.62 - 7.57 (m, 1H), 7.54 - 7.46 (m, 1H), 7.39 (d, 1H), 6.84 (d, 1H), 4.98 - 4.87 (m, 1H), 4.54 - 4.41 (m, 2H), 4.25 - 4.15 (m, 2H), 3.85 - 3.73 (m, 1H), 2.96 - 2.66 (m, 3H), 2.17 - 2.08 (m, 1H), 1.95 (s, 6H).

LCMS m/z = 683.1 $[M-1]^-$.

**Example 16: Preparation of compound 16**

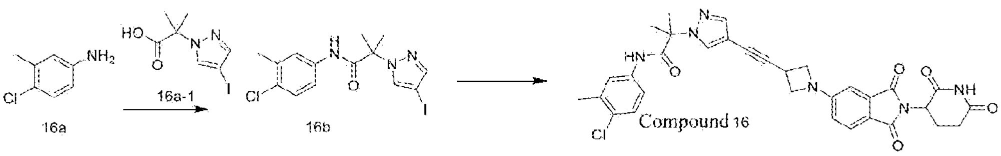


16a

16b

Compound 16

Step 1: N-(4-chloro-3-methylphenyl)-2-(4-iodo-1H-pyrazol-1-yl)-2-methylpropanamide (16b)

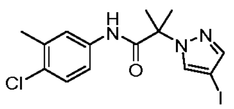

16a-1 (0.50 g, 1.79 mmol) was dissolved in 8 mL of THF, 1-chloro-N,N,2-trimethylpropenylamine (0.35 g, 2.62 mmol) was added, the mixture was stirred at room temperature for 2 h, and then 16a (0.25 g, 1.77 mmol) and TEA (0.54 g, 5.34 mmol) were added in sequence, and the mixture was reacted at room temperature for 12 h. 10 mL of ethyl acetate and 10 mL of water were added to the reaction liquid, the organic phase was separated, washed with 10 mL of saturated sodium chloride solution, dried over anhydrous sodium sulfate, and concentrated under reduced pressure. The crude product was separated and purified with silica gel chromatography column (petroleum ether/ethyl acetate (v/v) = 5 : 1) to afford 16b (0.34 g, yield: 47%).

LCMS m/z = 404.0 $[M+1]^+$.

Step 2: N-(4-chloro-3-methylphenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 16)

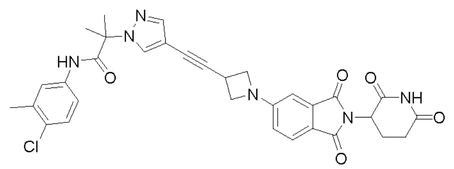

16b (0.34 g, 0.84 mmol) was dissolved in 5 mL of DMF, and intermediate 1 (0.28 g), TEA (0.25 g, 2.47 mmol), CuI (0.032 g, 0.17 mmol) and $PdCl_2(PPh_3)_2$ (0.059 g, 0.084 mmol) were added in sequence, and the mixture was reacted at 60°C for 1.5 h. The reaction liquid was cooled to room temperature, 30 mL of ethyl acetate and 30 mL of water were added, the organic phase was separated, the aqueous phase was extracted with ethyl acetate (30 mL × 3), the organic phases were combined, and the organic phase was washed with 50 mL of saturated sodium chloride solution, dried over anhydrous sodium sulfate, and concentrated under reduced pressure. The crude product was separated and purified with silica gel chromatography column (petroleum ether/ethyl acetate (v/v) = 1 : 1) to afford compound 16 (0.125 g, yield: 24%).

$^1H$ NMR (400 MHz, $CDCl_3$) δ 8.49 (s, 1H), 7.96 (s, 1H), 7.78 (s, 2H), 7.67 (d, 1H), 7.36 - 7.30 (m, 1H), 7.25 - 7.14 (m, 2H), 6.81 (d, 1H), 6.55 (dd, 1H), 4.98 - 4.86 (m, 1H), 4.44 - 4.30 (m, 2H), 4.15 - 4.00 (m, 2H), 3.88 - 3.74 (m, 1H), 2.95 - 2.64 (m, 3H), 2.32 (s, 3H), 2.18 - 2.08 (m, 1H), 1.91 (s, 6H).

LCMS m/z = 613.2 $[M+1]^+$.

**Example 17: Preparation of compound 17**

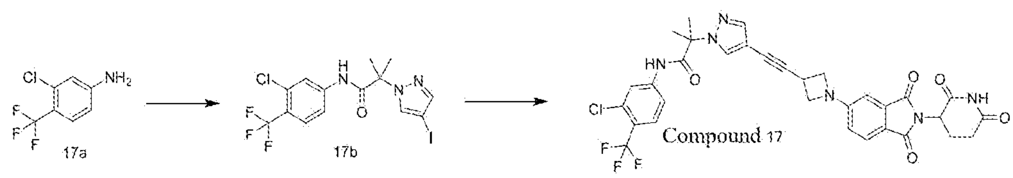

17a

17b

Compound 17

Step 1: N-(3-chloro-4-(trifluoromethyl)phenyl)-2-(4-iodo-1H-pyrazol-1-yl)-2-methylpropanamide (17b)

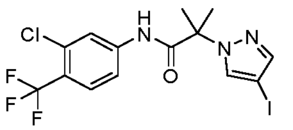

16a-1 (0.50 g, 1.79 mmol) was dissolved in THF (8 mL), 1-chloro-N,N,2-trimethylpropenylamine (0.35 g, 2.62 mmol) was added, the mixture was stirred at room temperature for 2 h, and then 17a (0.35 g, 1.79 mmol) and TEA (0.54 g, 5.34 mmol) were added in sequence, and the mixture was reacted at room temperature for 12 h. 10 mL of ethyl acetate and 10 mL of water were added to the reaction liquid, the organic phase was separated, washed with 10 mL of saturated sodium chloride solution, dried over anhydrous sodium sulfate, and concentrated under reduced pressure. The crude product was separated and purified with silica gel chromatography column (petroleum ether/ethyl acetate (v/v) = 5 : 1) to afford 17b (0.40 g, yield: 49%).

LCMS m/z = 458.0 $[M+1]^+$.

Step 2: N-(3-chloro-4-(trifluoromethyl)phenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 17)

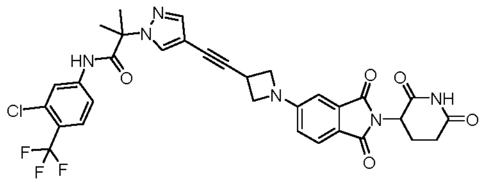

17b (0.28 g, 0.61 mmol) was dissolved in 5 mL of DMF, and intermediate 1 (0.21 g), TEA (0.19 g, 1.88 mmol), CuI (0.023 g, 0.12 mmol) and $PdCl_2(PPh_3)_2$ (0.043 g, 0.061 mmol) were added in sequence, and the mixture was reacted at 60°C for 1.5 h. The reaction liquid was cooled to room temperature, 30 mL of ethyl acetate and 30 mL of water were added, the organic phase was separated, the aqueous phase was extracted with ethyl acetate (30 mL × 3), the organic phases were combined, and the organic phase was washed with 50 mL of saturated sodium chloride solution, dried over anhydrous sodium sulfate, and concentrated under reduced pressure. The crude product was separated and purified with silica gel chromatography column (petroleum ether/ethyl acetate (v/v) = 1 : 1) to afford compound 17 (0.160 g, yield: 39%).

$^1H$ NMR (400 MHz, $CDCl_3$) δ 9.05 (s, 1H), 7.97 (s, 1H), 7.79 (d, 2H), 7.72 - 7.64 (m, 2H), 7.58 (d, 1H), 7.43 - 7.37 (m, 1H), 6.81 (d, 1H), 6.56 (dd, 1H), 4.98 - 4.88 (m, 1H), 4.40 - 4.31 (m, 2H), 4.10 - 4.03 (m, 2H), 3.87 - 3.75 (m, 1H), 2.96 - 2.64 (m, 3H), 2.18 - 2.07 (m, 1H), 1.91 (s, 6H).

LCMS m/z = 667.2 $[M+1]^+$.

**Example 18: Preparation of compound 18**

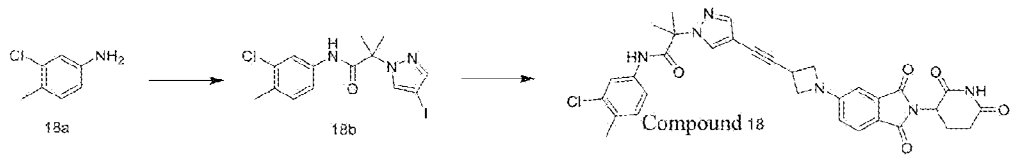


Step 1: N-(3-chloro-4-methylphenyl)-2-(4-iodo-1H-pyrazol-1-yl)-2-methylpropanamide (18b)

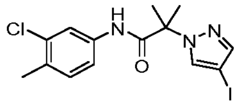

16a-1 (0.50 g, 1.79 mmol) was dissolved in THF (8 mL), 1-chloro-N,N,2-trimethylpropenylamine (0.35 g, 2.62 mmol) was added, the mixture was stirred at room temperature for 2 h, and then 18a (0.25 g, 1.77 mmol) and TEA (0.54 g, 5.34 mmol) were added in sequence, and the mixture was reacted at room temperature for 12 h. 10 mL of ethyl acetate was added to the reaction liquid to dilute the reaction liquid, 10 mL of water were added, the organic phase was separated, washed with 10 mL of saturated sodium chloride solution, dried over anhydrous sodium sulfate, and concentrated under reduced pressure. The crude product was separated and purified with silica gel chromatography column (petroleum ether/ethyl acetate (v/v) = 5 : 1) to afford 18b (0.30 g, yield: 42%).

LCMS m/z = 404.0 $[M+1]^+$.

Step 2: N-(3-chloro-4-methylphenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 18)

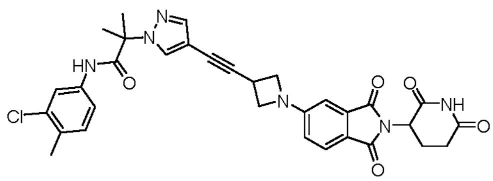

18b (0.30 g, 0.74 mmol) was dissolved in 5 mL of DMF, and intermediate 1 (0.25 g), TEA (0.22 g, 2.17 mmol), CuI (0.028 g, 0.15 mmol) and $PdCl_2(PPh_3)_2$ (0.052 g, 0.074 mmol) were added in sequence, and the mixture was reacted at 60°C for 1.5 h. The reaction liquid was cooled to room temperature, 30 mL of ethyl acetate and 30 mL of water were added, the organic phase was separated, the aqueous phase was extracted with ethyl acetate (30 mL × 3), the organic phases were combined, and the organic phase was washed with 50 mL of saturated sodium chloride solution, dried over anhydrous sodium sulfate, and concentrated under reduced pressure. The crude product was separated and purified with silica gel chromatography column (petroleum ether/ethyl acetate (v/v) = 1 : 1) to afford compound 18 (0.150 g, yield: 33%).

$^1H$ NMR (400 MHz, $CDCl_3$) δ 8.48 (s, 1H), 7.95 (s, 1H), 7.78 (s, 2H), 7.67 (d, 1H), 7.50 (d, 1H), 7.21 - 7.15 (m, 1H), 7.14 - 7.08 (m, 1H), 6.81 (d, 1H), 6.56 (dd, 1H), 4.98 - 4.87 (m, 1H), 4.42 - 4.30 (m, 2H), 4.12 - 4.02 (m, 2H), 3.88 - 3.75 (m, 1H), 2.95 - 2.64 (m, 3H), 2.30 (s, 3H), 2.18 - 1.08 (m, 1H), 1.91 (s, 6H).

LCMS m/z = 613.2 $[M+1]^+$.

**Example 19: Preparation of compound 19**

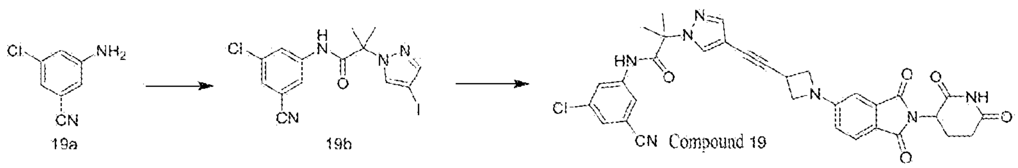


Step 1: N-(3-chloro-5-cyanophenyl)-2-(4-iodo-1H-pyrazol-1-yl)-2-methylpropanamide (19b)

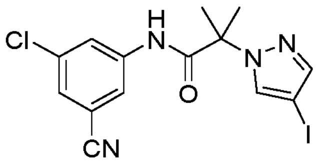

16a-1 (0.50 g, 1.79 mmol) was dissolved in THF (8 mL), 1-chloro-N,N,2-trimethylpropenylamine (0.35 g, 2.62 mmol) was added, the mixture was stirred at room temperature for 2 h, and then 19a (0.27 g, 1.77 mmol) and TEA (0.54 g, 5.34 mmol) were added in sequence, and the mixture was reacted at room temperature for 12 h. 10 mL of ethyl acetate and 10 mL of water were added to the reaction liquid, the organic phase was separated, washed with 10 mL of saturated sodium chloride solution, dried over anhydrous sodium sulfate, and concentrated under reduced pressure. The crude product was separated and purified with silica gel chromatography column (petroleum ether/ethyl acetate (v/v) = 5 : 1) to afford 19b (0.30 g, yield: 40%).

LCMS m/z = 414.9 $[M+1]^+$.

Step 2: N-(3-chloro-5-cyanophenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 19)

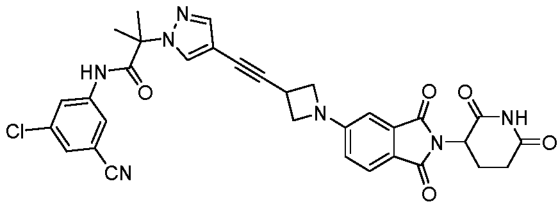

19b (0.30 g, 0.72 mmol) was dissolved in 5 mL of DMF, and intermediate 1 (0.24 g), TEA (0.22 g, 2.17 mmol), CuI (0.027 g, 0.14 mmol) and $PdCl_2(PPh_3)_2$ (0.051 g, 0.073 mmol) were added in sequence, and the mixture was reacted at 60°C for 1.5 h. The reaction liquid was cooled to room temperature, 30 mL of ethyl acetate and 30 mL of water were added, the organic phase was separated, the aqueous phase was extracted with ethyl acetate (30 mL × 3), the organic phases were combined, and the organic phase was washed with 50 mL of saturated sodium chloride solution, dried over anhydrous sodium sulfate, and concentrated under reduced pressure. The crude product was separated and purified with silica gel chromatography column (petroleum ether/ethyl acetate (v/v) = 1 : 1) to afford compound 19 (0.172 g, yield: 38%).

$^1H$ NMR (400 MHz, $CDCl_3$) δ 9.16 (s, 1H), 7.95 (s, 1H), 7.79 (d, 2H), 7.76 - 7.72 (m, 1H), 7.72 - 7.65 (m, 2H), 7.36 - 7.32 (m, 1H), 6.81 (d, 1H), 6.56 (dd, 1H), 4.99 - 4.88 (m, 1H), 4.43 - 4.30 (m, 2H), 4.15 - 4.00 (m, 2H), 3.88 - 3.75 (m, 1H), 2.96 - 2.64 (m, 3H), 2.18 - 2.08 (m, 1H), 1.91 (s, 6H).

LCMS m/z = 624.2 $[M+1]^+$.

**Example 20: Preparation of compound 20**

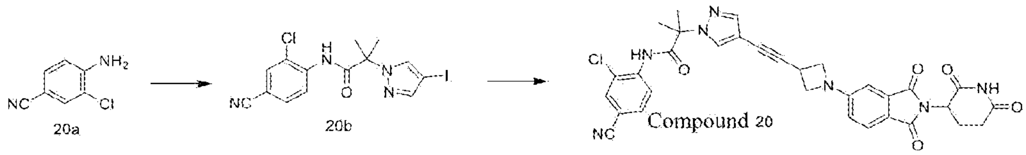


Step 1: N-(2-chloro-4-cyanophenyl)-2-(4-iodo-1H-pyrazol-1-yl)-2-methylpropanamide (20b)

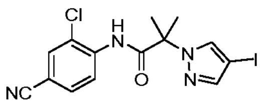

16a-1 (0.50 g, 1.79 mmol) was dissolved in 10 mL of DMF, and 20a (0.27 g, 1.77 mmol), TCFH (0.75 g, 2.67 mmol) and N-methylimidazole (0.44 g, 5.36 mmol) were added in sequence, the mixture was reacted at 80°C for 12 h. The reaction liquid was cooled to room temperature, 10 mL of ethyl acetate and 10 mL of water were added, the organic phase was separated, the aqueous phase was extracted with ethyl acetate (10 mL × 2), the organic phases were combined, washed with 10 mL of saturated sodium chloride solution, dried over anhydrous sodium sulfate, and concentrated under reduced pressure. The crude product was separated and purified with silica gel chromatography column (petroleum ether/ethyl acetate (v/v) = 5 : 1) to afford 20b (0.11 g, yield: 15%).

LCMS m/z = 415.0 $[M+1]^+$.

Step 2: N-(2-chloro-4-cyanophenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 20)

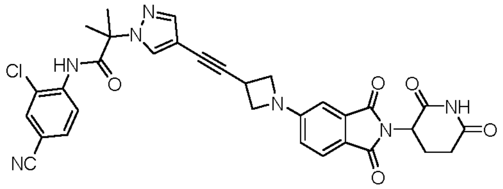

20b (0.11 g, 0.27 mmol) was dissolved in 5 mL of DMF, and intermediate 1 (0.091 g), TEA (0.082 g, 0.81 mmol), CuI (0.01 g, 0.053 mmol) and $PdCl_2(PPh_3)_2$ (0.019 g, 0.027 mmol) were added in sequence, and the mixture was reacted at 60°C for 2 h. The reaction liquid was cooled to room temperature, DCM (30 mL) was added, 30 mL of water was added, the organic phase was separated, the aqueous phase was extracted with DCM (30 mL × 3), the organic phases were combined, and the organic phase was washed with 50 mL of saturated sodium chloride solution, dried over anhydrous sodium sulfate, and concentrated under reduced pressure. The crude product was separated and purified with silica gel chromatography column (petroleum ether/ethyl acetate (v/v) = 1 : 1) to afford compound 20 (0.062 g, yield: 37%).

$^1H$ NMR (400 MHz, $CDCl_3$) δ 9.38 (s, 1H), 8.54 (d, 1H), 7.92 (s, 1H), 7.79 (d, 2H), 7.67 (d, 1H), 7.64 - 7.61 (m, 1H), 7.53 (dd, 1H), 6.83 - 6.79 (m, 1H), 6.56 (dd, 1H), 4.99 - 4.88 (m, 1H), 4.40 - 4.31 (m, 2H), 4.10 - 4.02 (m, 2H), 3.86 - 3.74 (m, 1H), 3.00 - 2.65 (m, 3H), 2.18 - 2.08 (m, 1H), 1.94 (s, 6H).

LCMS m/z = 624.2 $[M+1]^+$.

**Example 21: Preparation of compound 21**

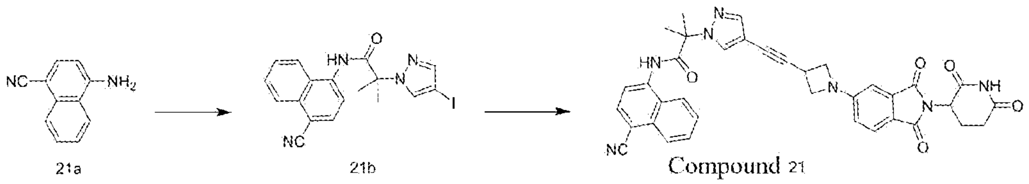


Step 1: N-(4-cyanonaphthalen-1-yl)-2-(4-iodo-1H-pyrazol-1-yl)-2-methylpropanamide (21b)

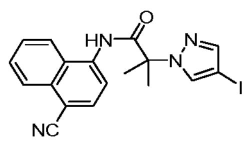

16a-1 (0.50 g, 1.79 mmol) was dissolved in THF (8 mL), 1-chloro-N,N,2-trimethylpropenylamine (0.35 g, 2.62 mmol) was added, the mixture was reacted at room temperature for 2 h, and then 21a (0.30 g, 1.78 mmol) and TEA (0.54 g, 5.34 mmol) were added in sequence, and the mixture was reacted at room temperature for 12 h. 10 mL of ethyl acetate and 10 mL of water were added to the reaction liquid, the organic phase was separated, washed with 10 mL of saturated sodium chloride solution, dried over anhydrous sodium sulfate, and concentrated under reduced pressure. The crude product was separated and purified with silica gel chromatography column (petroleum ether/ethyl acetate (v/v) = 5 : 1) to afford 21b (0.28 g, yield: 36%).

LCMS m/z = 431.0 $[M+1]^+$.

Step 2: N-(4-cyanonaphthalen-1-yl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 21)

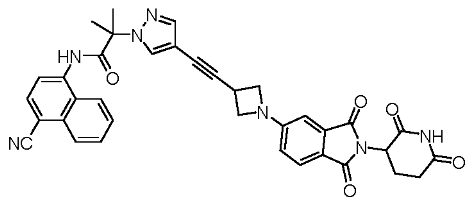

21b (0.28 g, 0.65 mmol) was dissolved in 5 mL of DMF, and intermediate 1 (0.22 g), TEA (0.2 g, 1.98 mmol), CuI (0.025 g, 0.13 mmol) and $PdCl_2(PPh_3)_2$ (0.046 g, 0.066 mmol) were added in sequence, and the mixture was reacted at 60°C for 2 h. The reaction liquid was cooled to room temperature, DCM (30 mL) was added, 30 mL of water was added, the organic phase was separated, the aqueous phase was extracted with DCM (30 mL × 3), the organic phases were combined, and the organic phase was washed with 50 mL of saturated sodium chloride solution, dried over anhydrous sodium sulfate, and concentrated under reduced pressure. The crude product was separated and purified with silica gel chromatography column (petroleum ether/ethyl acetate (v/v) = 1 : 1) to afford compound 21 (0.076 g, yield: 18%).

$^1H$ NMR (400 MHz, $CDCl_3$) δ 9.92 (s, 1H), 8.38 - 8.31 (m, 1H), 8.29 - 8.22 (m, 1H), 7.95 - 7.79 (m, 5H), 7.75 - 7.61 (m, 3H), 6.81 (d, 1H), 6.55 (dd, 1H), 4.98 - 4.88 (m, 1H), 4.42 - 4.30 (m, 2H), 4.12 - 4.01 (m, 2H), 3.87 - 3.75 (m, 1H), 2.95 - 2.63 (m, 3H), 2.18 - 2.08 (m, 1H), 2.01 (s, 6H).

LCMS m/z = 640.2 $[M+1]^+$.

**Example 22: Preparation of compound 22**

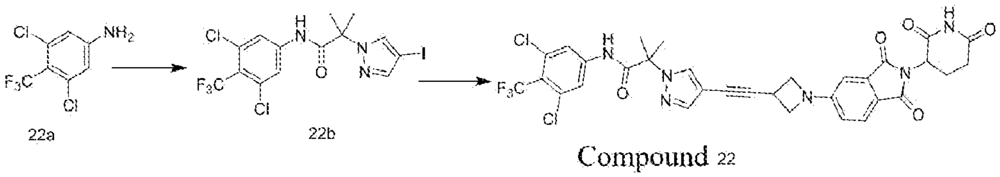

22a 22b Compound 22

Step 1: N-(3,5-dichloro-4-(trifluoromethyl)phenyl)-2-(4-iodo-1H-pyrazol-1-yl)-2-methylpropanamide (22b)

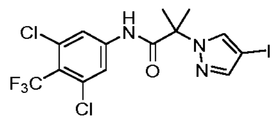

16a-1 (0.49 g, 1.75 mmol) was dissolved in 8 mL of DCM, 1-chloro-N,N,2-trimethylpropenylamine (0.35 g, 2.62 mmol) was added, the mixture was reacted at room temperature for 2 h, and then 22a (0.4 g, 1.74 mmol) and TEA (0.53 g, 5.24 mmol) were added in sequence, and the mixture was reacted at room temperature for 16 h. 10 mL of ethyl acetate and 10 mL of water were added to the reaction liquid, the organic phase was separated, washed with 10 mL of saturated sodium chloride solution, dried over anhydrous sodium sulfate, and concentrated under reduced pressure. The crude product was separated and purified with silica gel chromatography column (petroleum ether/ethyl acetate (v/v) = 5 : 1) to afford 22b (0.5 g, yield: 58%).

Step 2: N-(3,5-dichloro-4-(trifluoromethyl)phenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 22)

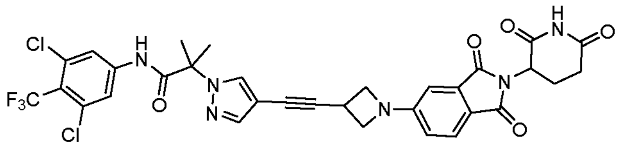

22b (0.25 g, 0.51 mmol) was dissolved in 8 mL of DMF, and intermediate 1 (0.17 g), DIPEA (0.2 g, 1.55 mmol), CuI (0.010 g, 0.053 mmol) and $PdCl_2(PPh_3)_2$ (0.036 g, 0.051 mmol) were added in sequence, and the mixture was reacted at 55°C for 1 h. The reaction liquid was cooled to room temperature, 50 mL of water was added, and the mixture was filtered by suction. The filter cake was washed with 10 mL of water. The solid was collected. The solid was dissolved in 20 mL of DCM, the mixture was dried over anhydrous sodium sulfate, and concentrated under reduced pressure. The crude product was separated and purified with silica gel chromatography column (petroleum ether/ethyl acetate (v/v) = 1 : 1) to afford compound 22 (0.050 g, yield: 14%).

$^1H$ NMR (400 MHz, $CDCl_3$) δ 9.19 (s, 1H), 7.96 (s, 1H), 7.84 - 7.74 (m, 2H), 7.67 (d, 1H), 7.58 (s, 2H), 6.81 (d, 1H), 6.56 (dd, 1H), 4.99 - 4.88 (m, 1H), 4.44 - 4.30 (m, 2H), 4.12 - 4.02 (m, 2H), 3.87 - 3.76 (m, 1H), 2.96 - 2.65 (m, 3H), 2.18 - 2.08 (m, 1H), 1.90 (s, 6H).

LCMS m/z = 701.0 $[M+1]^+$.

**Example 23: Preparation of compound 23**

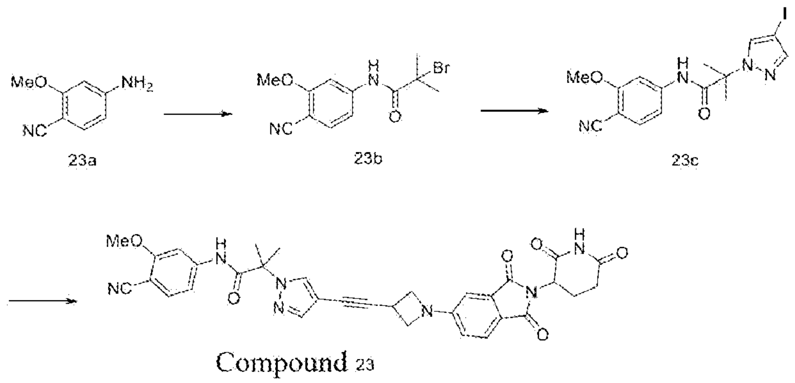

Step 1: 2-bromo-N-(4-cyano-3-methoxyphenyl)-2-methylpropanamide (23b)

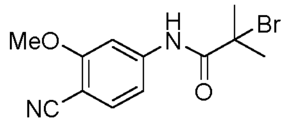

2-bromo-2-methylpropionic acid (1.00 g, 5.99 mmol) was added to a reaction flask, THF (10 mL) was added under nitrogen atmosphere, then the reaction system was cooled to 0°C under ice bath, and thionyl chloride (0.55 mL, 7.57 mmol) was added at 0°C. After the ice bath was removed, the mixture was slowly warmed to room temperature and the stirring was continued at room temperature for 2 h. The reaction system was cooled to 0°C under ice bath, TEA (1.25 mL, 8.99 mmol) was added dropwise, and the mixture was stirred at 0°C for 20 min. At this time, a large amount of insoluble matter was precipitated from the reaction system. A solution of 23a (0.89 g, 6.0 mmol) in THF (5 mL) was added dropwise into the above reaction system at 0°C and the mixture was reacted at 50°C for 3 h. The reaction system was cooled to 0°C under ice bath, saturated sodium bicarbonate solution (10 mL) was added to quench the reaction, the reaction liquid was extracted with ethyl acetate (20 × 3 mL), and the organic phase was washed with saturated sodium chloride solution (20 mL), dried over anhydrous sodium sulfate, concentrated under reduced pressure, and the crude product was slurried with a mixed solvent (ethyl acetate/petroleum ether (v/v) = 1 : 10) to afford 23b (1.58 g, yield: 89%).

Step 2: N-(4-cyano-3-methoxyphenyl)-2-(4-iodo-1H-pyrazol-1-yl)-2-methylpropanamide (23c)

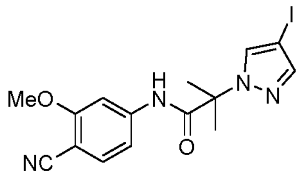

23b (1.58 g, 5.32 mmol), 4-iodo-1H-pyrazole (1.03 g, 5.31 mmol) and cesium carbonate (3.47 g, 10.65 mmol) were added to the reaction flask respectively, 10 mL of acetonitrile was added, and the mixture was reacted at 50°C for 3 h. The reaction system was cooled to room temperature and filtered by suction. The filtrate was concentrated under reduced pressure. The crude product was separated and purified by silica gel column chromatography (ethyl acetate/petroleum ether (v/v) = 1 : 5) to afford 23c (1.49 g, yield: 68%).

Step 3: N-(4-cyano-3-methoxyphenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 23)

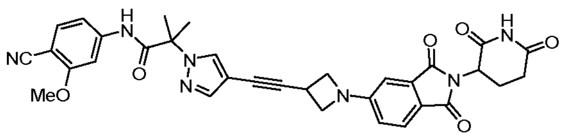

Under nitrogen atmosphere, 23c (240 mg, 0.59 mmol), intermediate 1 (200 mg), and $PdCl_2(PPh_3)_2$ (41.5 mg, 0.059 mmol) and CuI (22.6 mg, 0.12 mmol) were added to the reaction flask respectively, DMF (5 mL) and DIPEA (0.3 mL, 0.182 mmol) were added, and the mixture was reacted at 50°C for 2 h. The reaction system was cooled to room temperature, the reaction liquid was added dropwise into 20 mL of ice water, the mixture was filtered by suction, the filter cake was washed with 10 mL of water, the filter cake was collected, the filter cake was dissolved with 20 mL of DCM, the mixture was dried over anhydrous sodium sulfate, concentrated under reduced pressure and then the crude product was separated and purified with silica gel column chromatography (ethyl acetate/petroleum ether (v/v) = 1 : 1), and the obtained crude product was separated and purified with silica gel column chromatography (DCM/MeOH (v/v) = 20 : 1) to afford compound 23 (110 mg, yield: 30%).

$^1$H NMR (400 MHz, $CDCl_3$) $\delta$ 8.24 (s, 1H), 7.72 - 7.64 (m, 2H), 7.61 (d, 1H), 7.56 (d, 1H), 7.41 - 7.33 (m, 1H), 6.92 - 6.83 (m, 1H), 6.71 (dd, 1H), 5.13 - 4.98 (m, 1H), 4.49 - 4.30 (m, 2H), 4.07 - 3.94 (m, 2H), 3.94 - 3.80 (m, 4H), 2.96 - 2.80 (m, 1H), 2.66 - 2.51 (m, 2H), 2.10 - 1.93 (m, 1H), 1.80 (s, 6H).

LCMS m/z = 620.2 $[M+1]^+$.

**Example 24: Preparation of compound 24**

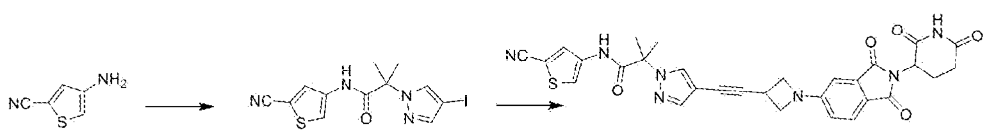

24a 24b Compound 24

Step 1: N-(5-cyanothiophen-3-yl)-2-(4-iodo-1H-pyrazol-1-yl)-2-methylpropanamide (24b)

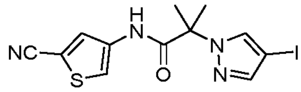

16a-1 (0.55 g, 1.96 mmol) was dissolved in 10 mL of DCM, 1-chloro-N,N,2-trimethylpropenylamine (0.36 g, 2.70 mmol) was added, the mixture was reacted at room temperature for 2 h, and then 24a (0.22 g, 1.77 mmol) and TEA (0.55 g, 5.44 mmol) were added in sequence, and the mixture was reacted at room temperature for 16 h. 10 mL of ethyl acetate and 10 mL of saturated aqueous sodium bicarbonate solution were added to the reaction liquid, the organic phase was separated, washed with 10 mL of saturated sodium chloride solution, dried over anhydrous sodium sulfate, and concentrated under reduced pressure. The crude product was separated and purified with silica gel chromatography column (petroleum ether/ethyl acetate (v/v) = 5 : 1) to afford 24b (0.52 g, yield: 76%).

Step 2: N-(5-cyanothiophen-3-yl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 24)

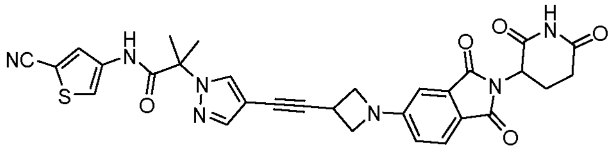

24b (0.19 g, 0.49 mmol) was dissolved in 8 mL of DMF, and intermediate 1 (0.19 g), DIPEA (0.19 g, 1.47 mmol), CuI (0.010 g, 0.053 mmol) and $PdCl_2(PPh_3)_2$ (0.035 g, 0.050 mmol) were added in sequence, and the mixture was reacted at 55°C for 1 h. The reaction liquid was cooled to room temperature, 50 mL of water was added, and the mixture was filtered by suction. The filter cake was washed with 10 mL of water. The solid was collected. The solid was dissolved in 20 mL of DCM, the mixture was dried over anhydrous sodium sulfate, and concentrated under reduced pressure. The crude product was separated and purified with silica gel chromatography column (petroleum ether/ethyl acetate (v/v) = 1 : 1) to afford compound 24 (0.070 g, yield: 24%).

$^1$H NMR (400 MHz, $CDCl_3$) δ 9.17 (s, 1H), 7.96 (s, 1H), 7.81 - 7.75 (m, 3H), 7.67 (d, 1H), 7.46 (d, 1H), 6.81 (d, 1H), 6.56 (dd, 1H), 4.98 - 4.88 (m, 1H), 4.42 - 4.32 (m, 2H), 4.12 - 4.02 (m, 2H), 3.88 - 3.75 (m, 1H), 2.99 - 2.65 (m, 3H), 2.20 - 2.08 (m, 1H), 1.90 (s, 6H).

LCMS m/z = 596.2 $[M+1]^+$.

**Example 25: Preparation of compound 25**

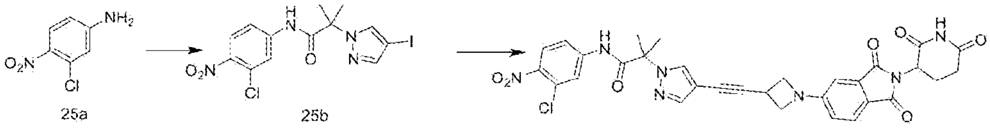


Step 1: N-(3-chloro-4-nitrophenyl)-2-(4-iodo-1H-pyrazol-1-yl)-2-methylpropanamide (25b)

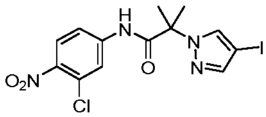

16a-1 (0.55 g, 1.96 mmol) was dissolved in 10 mL of DCM, 1-chloro-N,N,2-trimethylpropenylamine (0.36 g, 2.70 mmol) was added, the mixture was reacted at room temperature for 2 h, and then 3-chloro-4-nitroaniline (0.31 g, 1.80 mmol) and TEA (0.55 g, 5.44 mmol) were added in sequence, and the mixture was reacted at room temperature

for 16 h. 10 mL of ethyl acetate and 10 mL of saturated aqueous sodium bicarbonate solution were added to the reaction liquid, the organic phase was separated, washed with 10 mL of saturated sodium chloride solution, dried over anhydrous sodium sulfate, and concentrated under reduced pressure. The crude product was separated and purified with silica gel chromatography column (petroleum ether/ethyl acetate (v/v) = 5 : 1) to afford 25b (0.55 g, yield: 70%).

Step 2: N-(3-chloro-4-nitrophenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 25)

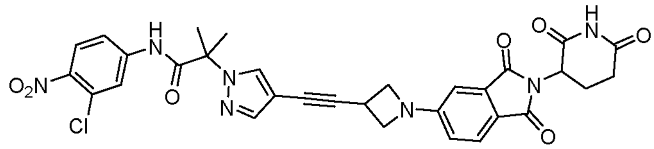

25b (0.217 g, 0.50 mmol) was dissolved in 8 mL of DMF, and intermediate 1 (0.19 g), DIPEA (0.19 g, 1.47 mmol), CuI (0.010 g, 0.053 mmol) and $PdCl_2(PPh_3)_2$ (0.035 g, 0.050 mmol) were added in sequence, and the mixture was reacted at 55°C for 1 h. The reaction liquid was cooled to room temperature, 50 mL of water was added, and the mixture was filtered by suction. The filter cake was washed with 10 mL of water. The solid was collected. The solid was dissolved in 20 mL of DCM, the mixture was dried over anhydrous sodium sulfate, and concentrated under reduced pressure. The crude product was separated and purified with silica gel chromatography column (petroleum ether/ethyl acetate (v/v) = 1 : 1) to afford compound 25 (0.080 g, yield: 25%).

$^1H$ NMR (400 MHz, $CDCl_3$) δ 9.32 (s, 1H), 8.05 (s, 1H), 7.91 (d, 1H), 7.84 - 7.75 (m, 3H), 7.67 (d, 1H), 7.44 (dd, 1H), 6.80 (d, 1H), 6.55 (dd, 1H), 4.99 - 4.88 (m, 1H), 4.43 - 4.30 (m, 2H), 4.13 - 4.00 (m, 2H), 3.88 - 3.75 (m, 1H), 2.95 - 2.65 (m, 3H), 2.18 - 2.08 (m, 1H), 1.92 (s, 6H).

LCMS m/z = 644.1 $[M+1]^+$.

**Example 26: Preparation of compound 26**

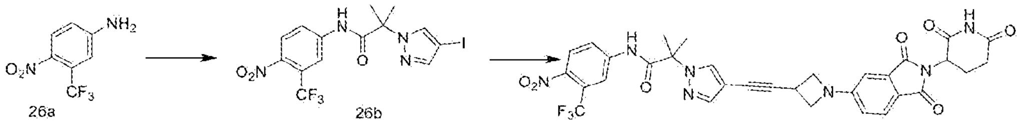

Compound 26

Step 1: 2-(4-iodo-1H-pyrazol-1-yl)-2-methyl-N-(4-nitro-3-(trifluoromethyl) phenyl)propanamide (26b)

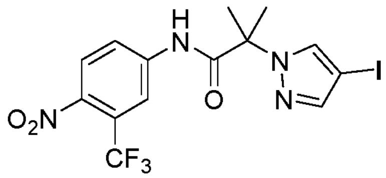

16a-1 (0.55 g, 1.97 mmol) was dissolved in 10 mL of DCM, 1-chloro-N,N,2-trimethylpropenylamine (0.36 g, 2.70 mmol) was added, the mixture was reacted at room temperature for 2 h, and then 26a (0.37 g, 1.80 mmol) and TEA (0.55 g, 5.44 mmol) were added in sequence, and the mixture was reacted at room temperature for 16 h. 10 mL of ethyl acetate and 10 mL of saturated aqueous sodium bicarbonate solution were added to the reaction liquid, the organic phase was separated, washed with 10 mL of saturated sodium chloride solution, dried over anhydrous sodium sulfate, and concentrated under reduced pressure. The crude product was separated and purified with silica gel chromatography column (petroleum ether/ethyl acetate (v/v) = 5 : 1) to afford 26b (0.5 g, yield: 59%).

Step 2: 2-(4-((1-(2-(2,6-dioxopiperidin-3-yl)-1,3-dioxoisoindolin-5-yl)azetidin-3-yl)ethynyl)-1H-pyrazol-1-yl)-2-methyl-N-(4-nitro-3-(trifluoromethyl)phenyl) propanamide (Compound 26)

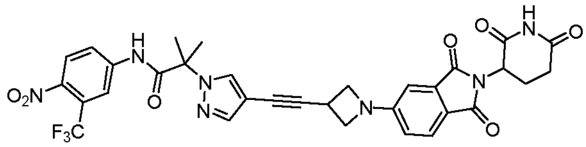

26b (0.23 g, 0.49 mmol) was dissolved in 8 mL of DMF, and intermediate 1 (0.19 g), DIPEA (0.19 g, 1.47 mmol), CuI (0.010 g, 0.0525 mmol) and $PdCl_2(PPh_3)_2$ (0.035 g, 0.050 mmol) were added in sequence, and the mixture was reacted at 55°C for 1 h. The reaction liquid was cooled to room temperature, 50 mL of water was added, and the mixture was filtered by suction. The filter cake was washed with 10 mL of water. The solid was dissolved in 20 mL of DCM, the mixture was dried over anhydrous sodium sulfate, and concentrated under reduced pressure. The crude product was separated and purified with silica gel chromatography column (petroleum ether/ethyl acetate (v/v) = 1 : 1) to afford compound 26 (0.060 g, yield: 18%).

$^1H$ NMR (400 MHz, $CDCl_3$) δ 9.53 (s, 1H), 8.11 (s, 1H), 8.00 - 7.74 (m, 5H), 7.66 (d, 1H), 6.86 - 6.76 (m, 1H), 6.60 - 6.50 (m, 1H), 4.99 - 4.88 (m, 1H), 4.45 - 4.27 (m, 2H), 4.15 - 3.98 (m, 2H), 3.88 - 3.72 (m, 1H), 2.98 - 2.64 (m, 3H), 2.20 - 2.07 (m, 1H), 1.93 (s, 6H).

LCMS m/z = 678.1 $[M+1]^+$.

**Example 27: Preparation of compound 27**

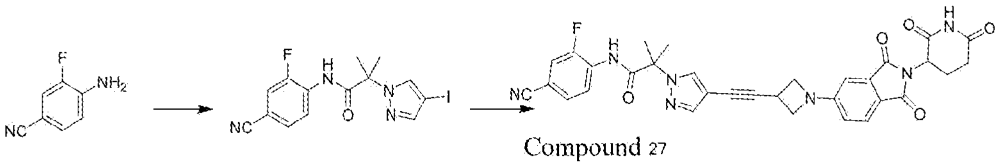

Compound 27

27a

27b

Step 1: N-(4-cyano-2-fluorophenyl)-2-(4-iodo-1H-pyrazol-1-yl)-2-methylpropanamide (27b)

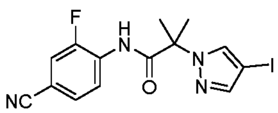

16a-1 (0.55 g, 1.97 mmol) was dissolved in 10 mL of DCM, 1-chloro-N,N,2-trimethylpropenylamine (0.36 g, 2.70 mmol) was added, the mixture was reacted at room temperature for 2 h, and then 27a (0.25 g, 1.80 mmol) and TEA (0.55 g, 5.44 mmol) were added in sequence, and the mixture was reacted at room temperature for 16 h. 10 mL of ethyl acetate and 10 mL of saturated aqueous sodium bicarbonate solution were added to the reaction liquid, the organic phase was separated, washed with 10 mL of saturated sodium chloride solution, dried over anhydrous sodium sulfate, and concentrated under reduced pressure. The crude product was separated and purified with silica gel chromatography column (petroleum ether/ethyl acetate (v/v) = 5 : 1) to afford 27b (0.48 g, yield: 67%).

Step 2: N-(4-cyano-2-fluorophenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 27)

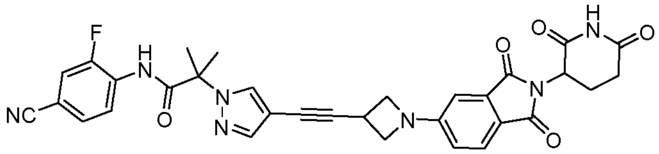

27b (0.20 g, 0.50 mmol) was dissolved in 8 mL of DMF, and intermediate 1 (0.19 g), DIPEA (0.19 g, 1.47 mmol), CuI (0.010 g, 0.0525 mmol) and $PdCl_2(PPh_3)_2$ (0.035 g, 0.050 mmol) were added in sequence, and the mixture was reacted at 55°C for 1 h. The reaction liquid was cooled to room temperature, 50 mL of water was added, and the mixture was filtered by suction. The filter cake was washed with 10 mL of water. The solid was dissolved in 20 mL of DCM, the mixture was dried over anhydrous sodium sulfate, and concentrated under reduced pressure. The crude product was separated and purified with silica gel chromatography column (petroleum ether/ethyl acetate (v/v) = 1 : 1) to afford compound 27 (0.080 g, yield: 26%).

$^1H$ NMR (400 MHz, $CDCl_3$) $\delta$ 9.44 (s, 1H), 8.48 (t, 1H), 7.97 (s, 1H), 7.79 (d, 2H), 7.67 (d, 1H), 7.43 (d, 1H), 7.39 - 7.29 (m, 1H), 6.85 - 6.75 (m, 1H), 6.62 - 6.50 (m, 1H), 5.00 - 4.88 (m, 1H), 4.45 - 4.28 (m, 2H), 4.12 - 4.00 (m, 2H), 3.88 - 3.72 (m, 1H), 2.98 - 2.60 (m, 3H), 2.22 - 2.06 (m, 1H), 1.93 (s, 6H).

LCMS m/z = 606.2 $[M-1]^+$.

**Example 28: Preparation of compound 28**

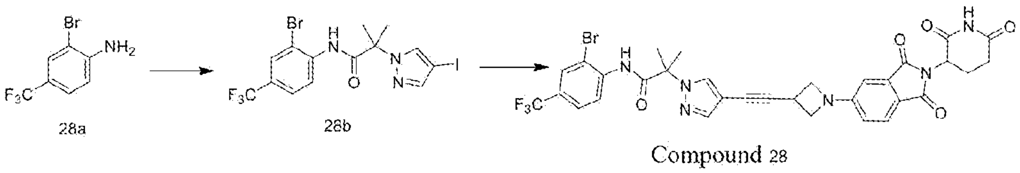

28a 28b Compound 28

Step 1: N-(2-bromo-4-(trifluoromethyl)phenyl)-2-(4-iodo-1H-pyrazol-1-yl)-2-methylpropanamide (28b)

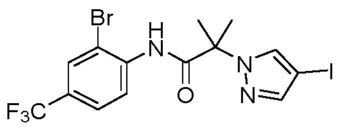

16a-1 (0.55 g, 1.97 mmol) was dissolved in 10 mL of DCM, 1-chloro-N,N,2-trimethylpropenylamine (0.36 g, 2.70 mmol) was added, the mixture was reacted at room temperature for 2 h, and then 28a (0.43 g, 1.79 mmol) and TEA (0.55 g, 5.44 mmol) were added in sequence, and the mixture was reacted at room temperature for 16 h. 10 mL of ethyl acetate and 10 mL of saturated aqueous sodium bicarbonate solution were added to the reaction liquid, the organic phase was separated, washed with 10 mL of saturated sodium chloride solution, dried over anhydrous sodium sulfate, and concentrated under reduced pressure. The crude product was separated and purified with silica gel chromatography column (petroleum ether/ethyl acetate (v/v) = 5 : 1) to afford 28b (0.32 g, yield: 36%).

Step 2: N-(2-bromo-4-(trifluoromethyl)phenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 28)

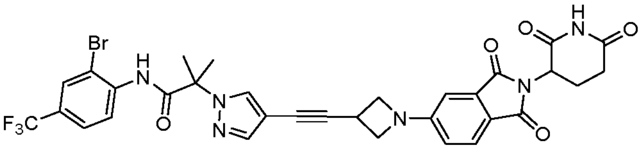

28b (0.20 g, 0.40 mmol) was dissolved in 8 mL of DMF, and intermediate 1 (0.15 g), TEA (0.12 g, 1.20 mmol),

CuI (0.008 g, 0.042 mmol) and $PdCl_2(PPh_3)_2$ (0.028 g, 0.04 mmol) were added in sequence, and the mixture was reacted at 55°C for 1 h. The reaction liquid was cooled to room temperature, 50 mL of water was added, and the mixture was filtered by suction. The filter cake was washed with 10 mL of water. The solid was dissolved in 20 mL of DCM, the mixture was dried over anhydrous sodium sulfate, and concentrated under reduced pressure. The crude product was separated and purified with silica gel chromatography column (petroleum ether/ethyl acetate (v/v) = 1 : 1) to afford compound 28 (0.10 g, yield: 35%).

$^1H$ NMR (400 MHz, $CDCl_3$) δ 8.98 (s, 1H), 8.47 (d, 1H), 8.01 (s, 1H), 7.85 - 7.62 (m, 4H), 7.58 - 7.49 (m, 1H), 6.85 - 6.76 (m, 1H), 6.60 - 6.50 (m, 1H), 4.98 - 4.88 (m, 1H), 4.43 - 4.30 (m, 2H), 4.17 - 4.00 (m, 2H), 3.89 - 3.75 (m, 1H), 3.00 - 2.64 (m, 3H), 2.20 - 2.07 (m, 1H), 1.95 (s, 6H).

LCMS m/z = 709.2 $[M-1]^+$.

**Example 29: Preparation of compound 29**

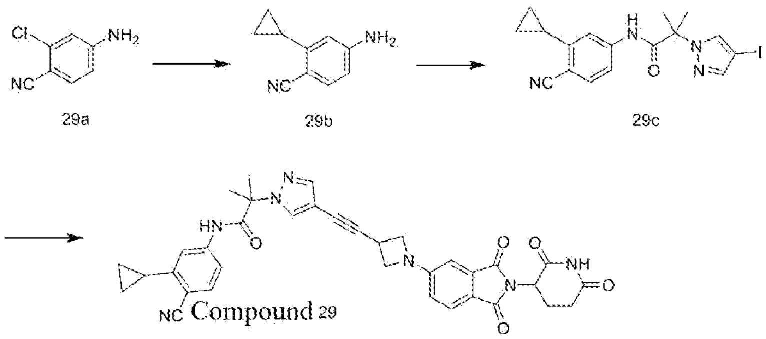

29a 29b 29c

Compound 29

Step 1: 4-amino-2-cyclopropylbenzonitrile (29b)

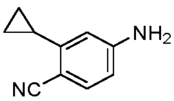

29a (1.00 g, 6.55 mmol) was dissolved in 15 mL of 1,4-dioxane and 1.5 mL of water, and cyclopropylboronic acid (0.84 g, 9.78 mmol), potassium phosphate heptahydrate (8.87 g, 26.2 mmol), tricyclohexylphosphine (0.73 g, 2.33 mmol) and palladium acetate (0.29 g, 1.29 mmol) were added in sequence, and the mixture was reacted at 90°C for 12 h. The reaction liquid was cooled to room temperature, 15 mL of ethyl acetate and 15 mL of water were added, the organic phase was separated, the aqueous phase was extracted with 10 mL of ethyl acetate twice, the organic phases were combined, washed with 10 mL of saturated sodium chloride solution, dried over anhydrous sodium sulfate, and concentrated under reduced pressure. The crude product was separated and purified with silica gel chromatography column (petroleum ether : ethyl acetate (v/v) = 5 : 1) to afford 29b (0.72 g, yield: 69%).

LCMS m/z= 159.1 $[M+1]^+$.

Step 2: N-(4-cyano-3-cyclopropylphenyl)-2-(4-iodo-1H-pyrazol-1-yl)-2-methylpropanamide (29c)

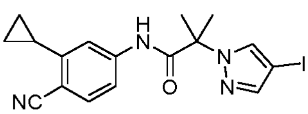

16a-1 (0.50 g, 1.79 mmol) was dissolved in THF (8 mL), 1-chloro-N,N,2-trimethylpropenylamine (0.36 g, 2.70 mmol) was added, the mixture was reacted at room temperature for 2 h, and then 29b (0.28 g, 1.77 mmol) and TEA (0.54 g, 5.34 mmol) were added in sequence, and the mixture was reacted at room temperature for 12 h. 10 mL of ethyl

acetate and 10 mL of water were added to the reaction liquid, the organic phase was separated, washed with 10 mL of saturated sodium chloride solution, dried over anhydrous sodium sulfate, and concentrated under reduced pressure. The crude product was separated and purified with silica gel chromatography column (petroleum ether : ethyl acetate (v/v) = 5 : 1) to afford 29c (0.27 g, yield: 36%).

LCMS m/z = 421.0 $[M+1]^+$.

Step 3: N-(4-cyano-3-cyclopropylphenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 29)

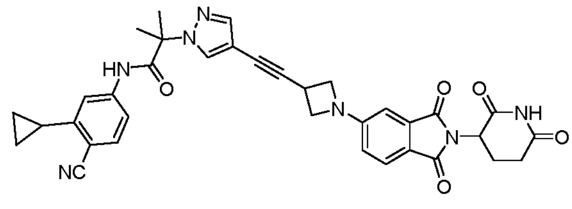

29c (0.26 g, 0.62 mmol) was dissolved in 5 mL of DMF, and intermediate 1 (0.23 g), TEA (0.19 g, 1.88 mmol), CuI (0.024 g, 0.126 mmol) and $PdCl_2(PPh_3)_2$ (0.044 g, 0.063 mmol) were added in sequence, and the mixture was reacted at 60°C for 1.5 h. The reaction liquid was cooled to room temperature, DCM (30 mL) and water (30 mL) were added, the organic phase was separated, the aqueous phase was extracted with DCM (30 mL × 3), the organic phases were combined, washed with 30 mL of saturated sodium chloride solution, dried over anhydrous sodium sulfate, and concentrated under reduced pressure. The crude product was separated and purified with silica gel chromatography column (petroleum ether : ethyl acetate (v/v) = 1 : 1) to afford compound 29 (24.8 mg, yield: 6%).

$^1H$ NMR (400 MHz, $CDCl_3$) δ 8.93 (s, 1H), 8.07 (s, 1H), 7.78 (d, 2H), 7.67 (d, 1H), 7.48 (d, 1H), 7.23 - 7.13 (m, 2H), 6.83 - 6.76 (m, 1H), 6.55 (dd, 1H), 4.99 - 4.88 (m, 1H), 4.43 - 4.30 (m, 2H), 4.13 - 4.00 (m, 2H), 3.89 - 3.73 (m, 1H), 3.00 - 2.60 (m, 3H), 2.30 - 2.05 (m, 2H), 1.90 (s, 6H), 1.18 - 1.06 (m, 2H), 0.84 - 0.74 (m, 2H).

LCMS m/z = 630.3 $[M+1]^+$

**Example 30: Preparation of compound 30**

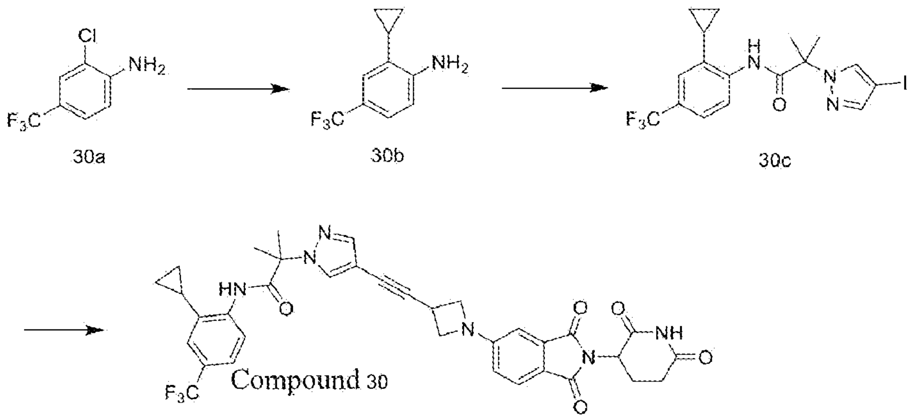

30a 30b 30c

Compound 30

Step 1: 2-cyclopropyl-4-(trifluoromethyl)aniline (30b)

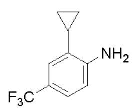

30a (2.00 g, 10.23 mmol) was dissolved in 30 mL of 1,4-dioxane and 3 mL of water, and cyclopropylboronic acid (1.32 g, 15.37 mmol) and potassium phosphate heptahydrate (13.85 g, 40.93 mmol), tricyclohexylphosphine (1.15 g, 3.67 mmol) and palladium acetate (0.46 g, 2.05 mmol) were added in sequence, and the mixture was reacted at 90°C for 12 h. The reaction liquid was cooled to room temperature, 15 mL of ethyl acetate and 15 mL of water were added, the organic phase was separated, the aqueous phase was extracted with 10 mL of ethyl acetate twice, the organic phases were combined, washed with 10 mL of saturated sodium chloride solution, dried over anhydrous sodium sulfate, and concentrated under reduced pressure. The crude product was separated and purified with silica gel chromatography column (petroleum ether : ethyl acetate (v/v) = 5 : 1) to afford 30b (2.00 g, yield: 97%).

Step 2: N-(2-cyclopropyl-4-(trifluoromethyl)phenyl)-2-(4-iodo-1H-pyrazol-1-yl)-2-methylpropanamide (30c)

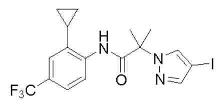

30c

16a-1 (0.50 g, 1.79 mmol) was dissolved in 5 mL of THF, 1-chloro-N,N,2-trimethylpropenylamine (0.36 g, 2.70 mmol) was added, the mixture was reacted at room temperature for 2 h, and then 30b (0.36 g, 1.79 mmol) and TEA (0.54 g, 5.34 mmol) were added in sequence, and the mixture was reacted at room temperature for 12 h. 10 mL of ethyl acetate and 10 mL of water were added to the reaction liquid, the organic phase was separated, washed with 10 mL of saturated sodium chloride solution, dried over anhydrous sodium sulfate, and concentrated under reduced pressure. The crude product was separated and purified with silica gel chromatography column (petroleum ether : ethyl acetate (v/v) = 5 : 1) to afford 30c (0.31 g, yield: 37%).

LCMS m/z = 464.0 $[M+1]^+$.

Step 3: N-(2-cyclopropyl-4-(trifluoromethyl)phenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 30)

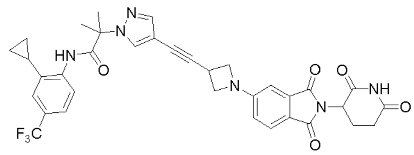

30c (0.31 g, 0.67 mmol) was dissolved in 5 mL of DMF, and intermediate 1 (0.25 g), TEA (0.20 g, 2.0 mmol), CuI (0.026 g, 0.14 mmol) and $PdCl_2(PPh_3)_2$ (0.047 g, 0.067 mmol) were added in sequence, and the mixture was reacted at 60°C for 2 h. The reaction liquid was cooled to room temperature, DCM (30 mL) was added, 30 mL of water was added, the organic phase was separated, the aqueous phase was extracted with DCM (30 mL × 3), the organic phases were combined, washed with 30 mL of saturated sodium chloride solution, dried over anhydrous sodium sulfate, and concentrated under reduced pressure. The crude product was separated and purified with silica gel chromatography column (petroleum ether : ethyl acetate (v/v) = 1 : 1) to afford compound 30 (0.186 g, yield: 41%).

$^1H$ NMR (400 MHz, $CDCl_3$) δ 8.91 (s, 1H), 8.33 (d, 1H), 7.95 (s, 1H), 7.81 (s, 1H), 7.72 (s, 1H), 7.70 - 7.64 (m, 1H), 7.49 - 7.41 (m, 1H), 7.36 - 7.30 (m, 1H), 6.83 - 6.77 (m, 1H), 6.55 (dd, 1H), 4.98 - 4.88 (m, 1H), 4.43 - 4.30 (m, 2H), 4.11 - 4.00 (m, 2H), 3.90 - 3.75 (m, 1H), 2.96 - 2.62 (m, 3H), 2.19 - 2.08 (m, 1H), 1.96 (s, 6H), 1.59 - 1.47 (m, 1H), 1.02 - 0.90 (m, 2H), 0.64 - 0.52 (m, 2H).

LCMS m/z = 673.2 $[M+1]^+$.

**Example 31: Preparation of compound 31**

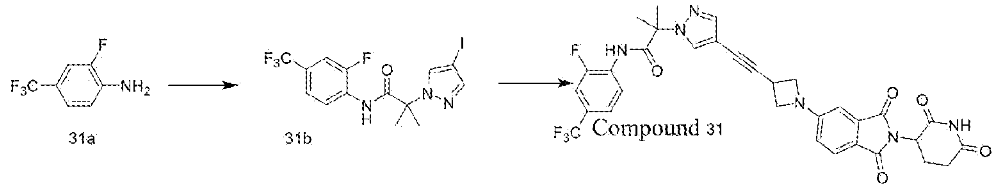

31a

31b

Compound 31

Step 1: N-(2-fluoro-4-(trifluoromethyl)phenyl)-2-(4-iodo-1H-pyrazol-1-yl)-2-methylpropanamide (31b)

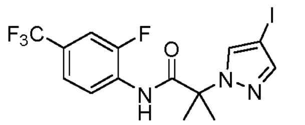

16a-1 (0.50 g, 1.79 mmol) was dissolved in 5 mL of THF, 1-chloro-N,N,2-trimethylpropenylamine (0.36 g, 2.70 mmol) was added, the mixture was reacted at room temperature for 2 h, and then 31a (0.32 g, 1.79 mmol) and TEA (0.54 g, 5.34 mmol) were added in sequence, and the mixture was reacted at room temperature for 12 h. 10 mL of ethyl acetate and 10 mL of water were added to the reaction liquid, the organic phase was separated, washed with 10 mL of saturated sodium chloride solution, dried over anhydrous sodium sulfate, and concentrated under reduced pressure. The crude product was separated and purified with silica gel chromatography column (petroleum ether : ethyl acetate (v/v) = 5 : 1) to afford 31b (0.27 g, yield: 34%).

LCMS m/z = 442.0 $[M+1]^+$.

Step 2: N-(2-fluoro-4-(trifluoromethyl)phenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 31)

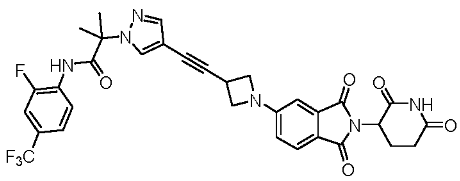

31b (0.27 g, 0.61 mmol) was dissolved in 5 mL of DMF, and intermediate 1 (0.23 g), TEA (0.19 g, 1.88 mmol), CuI (0.024 g, 0.126 mmol) and $PdCl_2(PPh_3)_2$ (0.044 g, 0.063 mmol) were added in sequence, and the mixture was reacted at 60°C for 2 h. The reaction liquid was cooled to room temperature, DCM (30 mL) was added, 30 mL of water was added, the organic phase was separated, the aqueous phase was extracted with DCM (30 mL × 3), the organic phases were combined, washed with 30 mL of saturated sodium chloride solution, dried over anhydrous sodium sulfate, and concentrated under reduced pressure. The crude product was separated and purified with silica gel chromatography column (petroleum ether : ethyl acetate (v/v) = 1 : 1) to afford compound 31 (30.9 mg, yield: 8%).

$^1H$ NMR (400 MHz, $CDCl_3$) δ 9.27 - 9.15 (m, 1H), 8.44 (t, 1H), 8.15 (s, 1H), 7.80 (d, 2H), 7.67 (d, 1H), 7.42 - 7.35 (m, 1H), 7.34 - 7.27 (m, 1H), 6.83 - 6.77 (m, 1H), 6.55 (dd, 1H), 5.00 - 4.86 (m, 1H), 4.43 - 4.30 (m, 2H), 4.13 - 4.00 (m, 2H), 3.87 - 3.72 (m, 1H), 2.98 - 2.60 (m, 3H), 2.20 - 2.05 (m, 1H), 1.94 (s, 6H).

LCMS m/z = 651.1 $[M+1]^+$.

**Example 32: Preparation of compound 32**

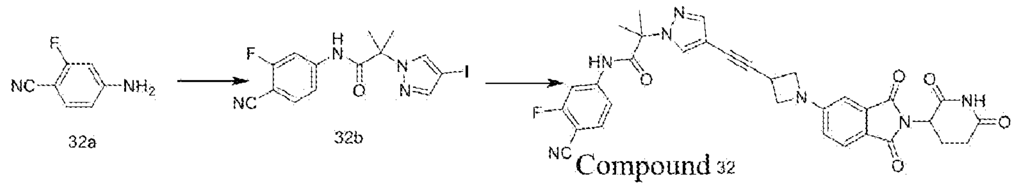

32a 32b Compound 32

Step 1: N-(4-cyano-3-fluorophenyl)-2-(4-iodo-1H-pyrazol-1-yl)-2-methylpropanamide (32b)

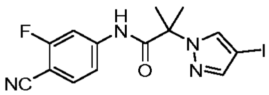

16a-1 (0.50 g, 1.79 mmol) was dissolved in 5 mL of THF, 1-chloro-N,N,2-trimethylpropenylamine (0.36 g, 2.70 mmol) was added, the mixture was reacted at room temperature for 2 h, and then 32a (0.24 g, 1.76 mmol) and TEA (0.54 g, 5.34 mmol) were added in sequence, and the mixture was reacted at room temperature for 12 h. 10 mL of ethyl acetate and 10 mL of water were added to the reaction liquid, the organic phase was separated, washed with 10 mL of saturated sodium chloride solution, dried over anhydrous sodium sulfate, and concentrated under reduced pressure. The crude product was separated and purified with silica gel chromatography column (petroleum ether : ethyl acetate (v/v) = 5 : 1) to afford 32b (0.11 g, yield: 16%).

LCMS m/z = 399.0 $[M+1]^+$.

Step 2: N-(4-cyano-3-fluorophenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 32)

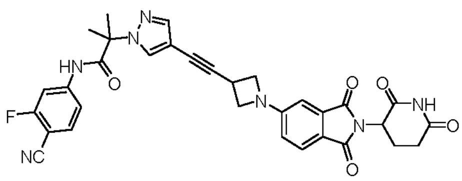

32b (0.11 g, 0.28 mmol) was dissolved in 5 mL of DMF, and intermediate 1 (0.10 g), TEA (0.085 g, 0.84 mmol), CuI (0.011 g, 0.058 mmol) and $PdCl_2(PPh_3)_2$ (0.02 g, 0.028 mmol) were added in sequence, and the mixture was reacted at 60°C for 2 h. The reaction liquid was cooled to room temperature, DCM (30 mL) was added, 30 mL of water was added, the organic phase was separated, the aqueous phase was extracted with DCM (30 mL $\times$ 3), the organic phases were combined, washed with 30 mL of saturated sodium chloride solution, dried over anhydrous sodium sulfate, and concentrated under reduced pressure. The crude product was separated and purified with silica gel chromatography column (petroleum ether : ethyl acetate (v/v) = 1 : 1) to afford compound 32 (20.2 mg, yield: 12%).

$^1H$ NMR (400 MHz, $CDCl_3$) $\delta$ 9.31 (s, 1H), 8.17 (s, 1H), 7.83 - 7.74 (m, 2H), 7.70 - 7.60 (m, 2H), 7.54 - 7.44 (m, 1H), 7.14 (dd, 1H), 6.83 - 6.77 (m, 1H), 6.55 (dd, 1H), 4.99 - 4.87 (m, 1H), 4.43 - 4.30 (m, 2H), 4.12 - 4.00 (m, 2H), 3.88 - 3.73 (m, 1H), 2.95 - 2.65 (m, 3H), 2.17 - 2.07 (m, 1H), 1.91 (s, 6H).

LCMS m/z = 608.1 $[M+1]^+$.

**Example 33: Preparation of compound 33**

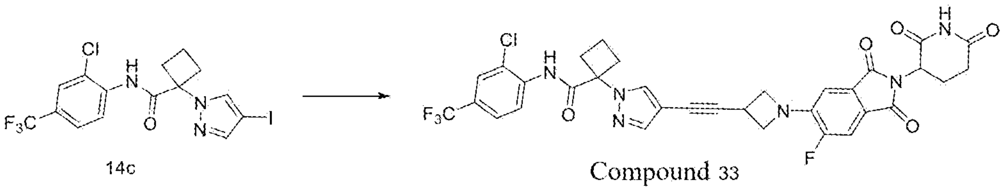

14c Compound 33

14c (15 g, 31.94 mmol) was dissolved in 100 mL of DMF, and the above crude intermediate 2 (12.48 g), TEA (9.70 g, 95.86 mmol), CuI (0.61 g, 3.20 mmol) and $PdCl_2(PPh_3)_2$ (2.24 g, 3.19 mmol) were added in sequence, nitrogen replacement was performed three times, and the mixture was reacted at 55°C for 1 h. The reaction liquid was cooled to room temperature, 800 mL of water was added, the mixture was filtered by suction, washed with 100 mL of water, the filter cake was collected, the filter cake was dissolved with 200 mL of DCM, the mixture was dried over anhydrous sodium sulfate, and concentrated under reduced pressure. The crude product was separated and purified with silica gel column chromatography (petroleum ether/ethyl acetate (v/v) = 1 : 1) to afford compound 33 (9.0 g, yield: 40%).

$^1H$ NMR (400 MHz, $CDCl_3$) δ 8.70 (s, 1H), 8.55 (d, 1H), 8.00 (s, 1H), 7.79 (s, 1H), 7.70 (s, 1H), 7.60 - 7.56 (m, 1H), 7.54 - 7.47 (m, 1H), 7.43 - 7.35 (m, 1H), 6.87 - 6.81 (m, 1H), 4.97 - 4.86 (m, 1H), 4.54 - 4.42 (m, 2H), 4.25 - 4.13 (m, 2H), 3.85 - 3.72 (m, 1H), 3.13 - 3.01 (m, 2H), 2.96 - 2.64 (m, 5H), 2.30 - 2.01 (m, 3H).

LCMS m/z = 695.1 $[M-1]^-$.

**Example 34: Preparation of compound 34**

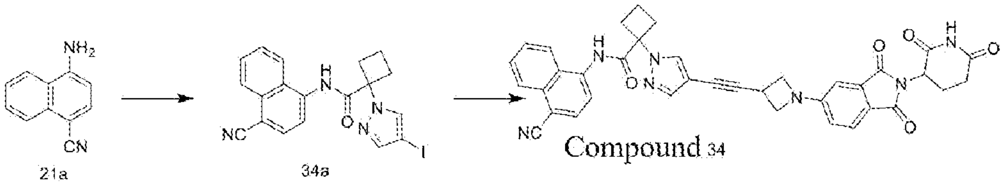

21a 34a Compound 34

Step 1: N-(4-cyanonaphthalen-1-yl)-1-(4-iodo-1H-pyrazol-1-yl)cyclobutane-1-carboxamide (34a)

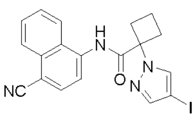

14b (11.5 g, 35.9 mmol) was dissolved in 100 mL of THF, 20 mL of water and lithium hydroxide monohydrate (3.0 g, 71.5 mmol) were added, and the mixture was reacted at room temperature for 30 min. 1 mol/L dilute hydrochloric acid was added dropwise to the reaction liquid to adjust the pH to 6, 250 mL of ethyl acetate was added, the liquid separation was conducted, the organic phase was washed with 100 mL of saturated sodium chloride solution, dried over anhydrous sodium sulfate, and concentrated under reduced pressure to afford a crude product (10.0 g). The above crude product (6.86 g) was dissolved in 60 mL of DCM, 1-chloro-N,N,2-trimethylpropenylamine (4.19 g, 31.37 mmol) was slowly added, and the mixture was reacted at room temperature for 2 h. 21a (3.3 g, 19.62 mmol) and TEA (5.94 g, 58.70 mmol) were added to the reaction liquid, and the mixture was reacted at room temperature for 16 h. 200 mL of DCM and 80 mL of saturated aqueous sodium bicarbonate solution were added to the reaction liquid, the organic phase was separated, the organic phase was washed with 80 mL of saturated sodium chloride solution, dried over anhydrous sodium sulfate, and concentrated under reduced pressure. The crude product was separated and purified with silica gel chromatography column (petroleum ether/ethyl acetate (v/v) = 5 : 1) to afford 34a (7.2 g, yield: 83%).

Step 2: N-(4-cyanonaphthalen-1-yl)-1-(4-((1-(2-(2,6-dioxopiperidin-3-yl)-1,3-dioxoisoindolin-5-yl)azetidin-3-yl)ethynyl)-1H-pyrazol-1-yl)cyclobutane-1-carboxamide (Compound 34)

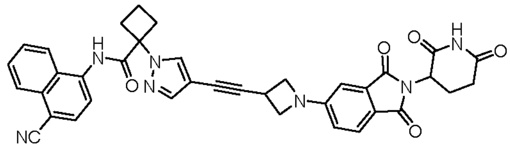

34a (2.20 g, 4.97 mmol) was dissolved in 35 mL of DMF, and intermediate 1 (2.01 g), TEA (1.51 g, 14.92 mmol), CuI (95 mg, 0.50 mmol) and $PdCl_2(PPh_3)_2$ (0.35 g, 0.50 mmol) were added in sequence, nitrogen replacement was performed three times, and the mixture was reacted at 55°C for 1 h. The reaction liquid was cooled to room temperature, 200 mL of water was added, the mixture was filtered by suction, washed with 50 mL of water, the filter cake was collected, the filter cake was dissolved with 150 mL of DCM, the mixture was dried over anhydrous sodium sulfate, and concentrated under reduced pressure. The crude product was separated and purified with silica gel chromatography column (petroleum ether/ethyl acetate (v/v) = 1 : 1) to afford compound 34 (1.5 g, yield: 46%).

$^1H$ NMR (400 MHz, $CDCl_3$) δ 9.27 (s, 1H), 8.43 - 8.34 (m, 1H), 8.25 (d, 1H), 8.00 (s, 1H), 7.94 - 7.86 (m, 2H), 7.78 (s, 1H), 7.76 - 7.60 (m, 4H), 6.82 - 6.77 (m, 1H), 6.54 (dd, 1H), 4.98 - 4.88 (m, 1H), 4.40 - 4.29 (m, 2H), 4.10 - 4.00 (m, 2H), 3.87 - 3.74 (m, 1H), 3.21 - 3.08 (m, 2H), 2.97 - 2.65 (m, 5H), 2.34 - 2.06 (m, 3H).

**Example 35: Preparation of compound 35**

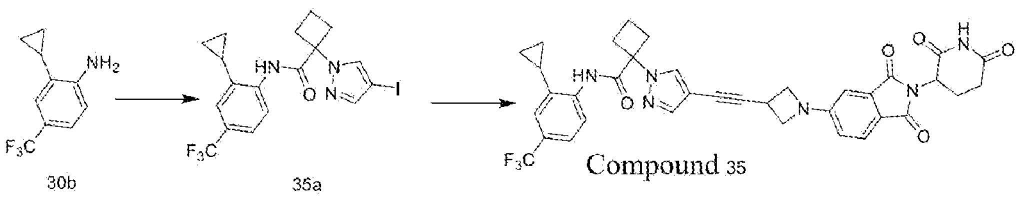

30b

35a

Compound 35

Step 1: N-(2-cyclopropyl-4-(trifluoromethyl)phenyl)-1-(4-iodo-1H-pyrazol-1-yl)cyclobutane-1-carboxamide (35a)

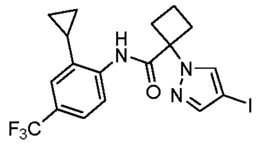

14b (11.5 g, 35.9 mmol) was dissolved in THF (100 mL), 20 mL of water and lithium hydroxide monohydrate (3.0 g, 71.5 mmol) were added, and the mixture was reacted at room temperature for 30 min. 1 mol/L dilute hydrochloric acid was added dropwise to the reaction liquid to adjust the pH to 6, 250 mL of ethyl acetate was added, the liquid separation was conducted, the organic phase was washed with 100 mL of saturated sodium chloride solution, dried over anhydrous sodium sulfate, and concentrated under reduced pressure to afford a crude product (10.0 g). The above crude product (2.09 g) was dissolved in 40 mL of DCM, 1-chloro-N,N,2-trimethylpropenylamine (1.27g, 9.51 mmol) was added, and the mixture was reacted at room temperature for 2 h. 30b (1.2 g, 5.96 mmol) and TEA (1.81 g, 17.89 mmol) were added to the reaction liquid, and the mixture was reacted at room temperature for 16 h. 100 mL of DCM was added to the reaction liquid, 60 mL of saturated aqueous sodium bicarbonate solution was added, the organic phase was separated, the organic phase was washed with 80 mL of saturated sodium chloride solution, dried over anhydrous sodium sulfate, and concentrated under reduced pressure. The crude product was separated and purified with silica gel chromatography column (petroleum ether/ethyl acetate (v/v) = 5 : 1) to afford 35a (2.3 g, yield: 81%).

Step 2: N-(2-cyclopropyl-4-(trifluoromethyl)phenyl)-1-(4-((1-(2-(2,6-dioxopiperidin-3-yl)-1,3-dioxoisoindolin-5-yl)azetidin-3-yl)ethynyl)-1H-pyrazol-1-yl)cyclobutane-1-carboxamide (Compound 35)

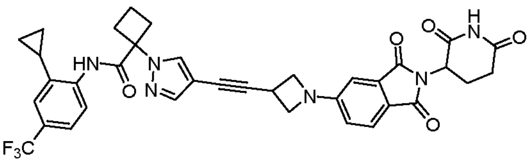

35a (2.3 g, 4.84 mmol) was dissolved in 35 mL of DMF, and intermediate 1 (1.96 g), TEA (1.47 g, 14.53 mmol), CuI (92 mg, 0.48 mmol) and $PdCl_2(PPh_3)_2$ (0.34 g, 0.48 mmol) were added in sequence, nitrogen replacement was performed three times, and the mixture was reacted at 55°C for 1 h. The reaction liquid was cooled to room temperature, 200 mL of water was added, the mixture was filtered by suction, washed with 50 mL of water, the filter cake was collected, the filter cake was dissolved with 150 mL of DCM, the mixture was dried over anhydrous sodium sulfate, and concentrated under reduced pressure. The crude product was separated and purified with silica gel chromatography column (petroleum ether/ethyl acetate (v/v) = 1 : 1) to afford compound 35 (1.5 g, yield: 45%).

$^1$H NMR (400 MHz, $CDCl_3$) δ 8.54 (s, 1H), 8.36 (d, 1H), 8.02 (s, 1H), 7.74 - 7.63 (m, 3H), 7.50 - 7.42 (m, 1H), 7.36 - 7.30 (m, 1H), 6.84 - 6.77 (m, 1H), 6.55 (dd, 1H), 4.99 - 4.88 (m, 1H), 4.42 - 4.30 (m, 2H), 4.12 - 4.00 (m, 2H), 3.86 - 3.75 (m, 1H), 3.16 - 3.02 (m, 2H), 2.95 - 2.65 (m, 5H), 2.32 - 1.99 (m, 3H), 1.54 - 1.41 (m, 1H), 1.00 - 0.90 (m, 2H), 0.62 - 0.51 (m, 2H).

LCMS m/z = 685.2 $[M+1]^+$.

**Example 36: Preparation of compound 36**

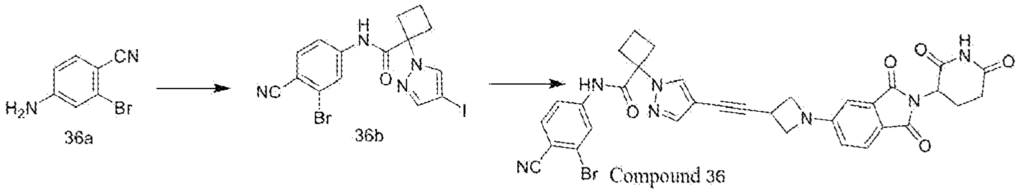

Step 1: N-(3-bromo-4-cyanophenyl)-1-(4-iodo-1H-pyrazol-1-yl)cyclobutane-1-carboxamide (36b)

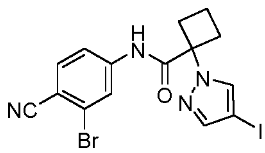

14b (11.5 g, 35.9 mmol) was dissolved in THF (100 mL), 20 mL of water and lithium hydroxide monohydrate (3.0 g, 71.5 mmol) were added, and the mixture was reacted at room temperature for 30 min. 1 mol/L dilute hydrochloric acid was added dropwise to the reaction liquid to adjust the pH to 6, 250 mL of ethyl acetate was added, the liquid separation was conducted, the organic phase was washed with 100 mL of saturated sodium chloride solution, dried over anhydrous sodium sulfate, and concentrated under reduced pressure to afford a crude product (10.0 g). The above crude product (2.97 g) was dissolved in 50 mL of DCM, 1-chloro-N,N,2-trimethylpropenylamine (1.81 g, 13.55 mmol) was added, and the mixture was reacted at room temperature for 2 h. TEA (2.57 g, 25.40 mmol) and 36a (1.67 g, 8.48 mmol) were added to the reaction liquid, and the mixture was reacted at room temperature for 18 h. 200 mL of DCM and 80 mL of saturated aqueous sodium bicarbonate solution were added to the reaction liquid, the organic phase was separated, the organic phase was washed with 100 mL of saturated sodium chloride solution, dried over anhydrous sodium sulfate, and concentrated under reduced pressure. The crude product was separated and purified with silica gel chromatography column (petroleum ether/ethyl acetate (v/v) = 5 : 1) to afford 36b (2.6 g, yield: 65%).

Step 2: N-(3-bromo-4-cyanophenyl)-1-(4-((1-(2-(2,6-dioxopiperidin-3-yl)-1,3-dioxoisoindolin-5-yl)azetidin-3-yl)ethynyl)-1H-pyrazol-1-yl)cyclobutane-1-carboxamide (Compound 36)

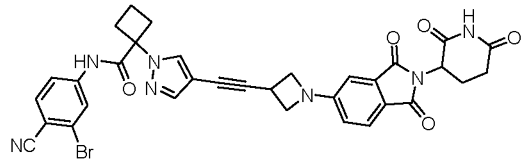

36b (2.35 g, 4.99 mmol) was dissolved in 40 mL of DMF, and intermediate 1 (1.85 g), TEA (1.51 g, 14.92 mmol), CuI (95 mg, 0.50 mmol) and $PdCl_2(PPh_3)_2$ (0.35 g, 0.50 mmol) were added in sequence, nitrogen replacement was performed three times, and the mixture was reacted at 55°C for 1 h. The reaction liquid was cooled to room temperature, 200 mL of water was added, the mixture was filtered by suction, washed with 50 mL of water, the filter cake was collected, the filter cake was dissolved with 150 mL of DCM, the mixture was dried over anhydrous sodium sulfate, and concentrated under reduced pressure. The crude product was separated and purified with silica gel chromatography column (petroleum ether/ethyl acetate (v/v) = 1 : 1) to afford compound 36 (1.2 g, yield: 35%).

$^1H$ NMR (400 MHz, $CDCl_3$) δ 8.61 (s, 1H), 8.28 (s, 1H), 7.93 (d, 1H), 7.78 (s, 1H), 7.72 - 7.63 (m, 2H), 7.55 - 7.49 (m, 1H), 7.48 - 7.41 (m, 1H), 6.79 (d, 1H), 6.55 (dd, 1H), 4.99 - 4.88 (m, 1H), 4.41 - 4.31 (m, 2H), 4.10 - 4.00 (m, 2H), 3.87 - 3.74 (m, 1H), 3.10 - 2.96 (m, 2H), 2.95 - 2.63 (m, 5H), 2.26 - 2.00 (m, 3H).

**Example 37: Preparation of compound 37**

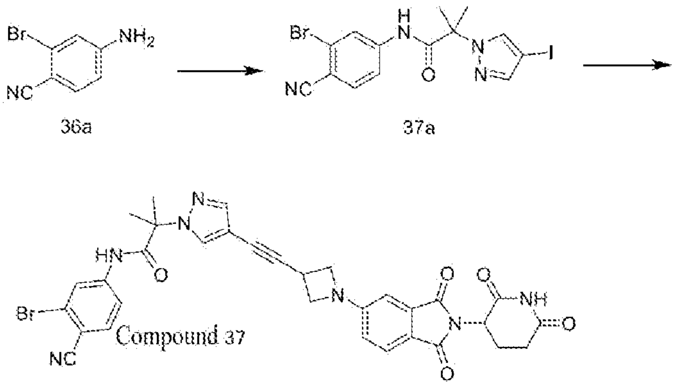
36a
37a
Compound 37

Step 1: N-(3-bromo-4-cyanophenyl)-2-(4-iodo-1H-pyrazol-1-yl)-2-methylpropanamide (37a)

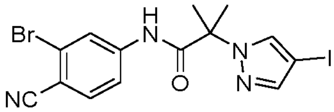

16a-1 (0.50 g, 1.79 mmol) was dissolved in THF (8 mL), 1-chloro-N,N,2-trimethylpropenylamine (0.36 g, 2.69 mmol) was added, the mixture was reacted at room temperature for 2 h, and then 36a (0.35 g, 1.78 mmol) and TEA (0.54 g, 5.34 mmol) were added in sequence, and the mixture was reacted at room temperature for 12 h. 10 mL of ethyl acetate and 10 mL of water were added to the reaction liquid, the organic phase was separated, washed with 10 mL of saturated sodium chloride solution, dried over anhydrous sodium sulfate, and concentrated under reduced pressure. The crude product was separated and purified with silica gel chromatography column (petroleum ether : ethyl acetate (v/v) = 5 : 1) to afford 37a (0.28 g, yield: 34%).

Step 2: N-(3-bromo-4-cyanophenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 37)

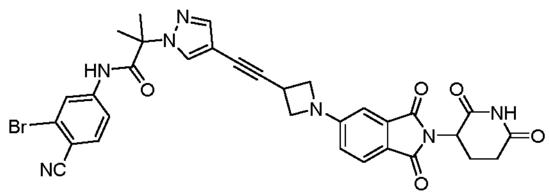

37a (0.28 g, 0.61 mmol) was dissolved in 5 mL of DMF, and intermediate 1 (0.25 g), TEA (0.19 g, 1.88 mmol), CuI (0.023 g, 0.12 mmol) and $PdCl_2(PPh_3)_2$ (0.043 g, 0.061 mmol) were added in sequence, and the mixture was reacted at 60°C for 2 h. The reaction liquid was cooled to room temperature, DCM (30 mL) was added, 30 mL of water was added, the organic phase was separated, the aqueous phase was extracted with DCM (30 mL × 3), the organic phases were combined, and the organic phase was washed with 50 mL of saturated sodium chloride solution, dried over anhydrous sodium sulfate, and concentrated under reduced pressure. The crude product was separated and purified with silica gel chromatography column (petroleum ether/ethyl acetate (v/v) = 1 : 1) to afford compound 37 (132 mg, yield: 32%).

$^1$H NMR (400 MHz, $CDCl_3$) δ 9.25 (s, 1H), 7.99 (s, 1H), 7.91 (d, 1H), 7.81 - 7.76 (m, 2H), 7.71 - 7.63 (m, 1H), 7.58 - 7.51 (m, 1H), 7.50 - 7.43 (m, 1H), 6.83 - 6.77 (m, 1H), 6.56 (dd, 1H), 4.98 - 4.89 (m, 1H), 4.41 - 4.31 (m, 2H), 4.12 - 4.01 (m, 2H), 3.88 - 3.74 (m, 1H), 2.96 - 2.64 (m, 3H), 2.18 - 2.08 (m, 1H), 1.91 (s, 6H).

LCMS m/z = 668.0 $[M+1]^+$.

**Example 38: Preparation of compound 38**

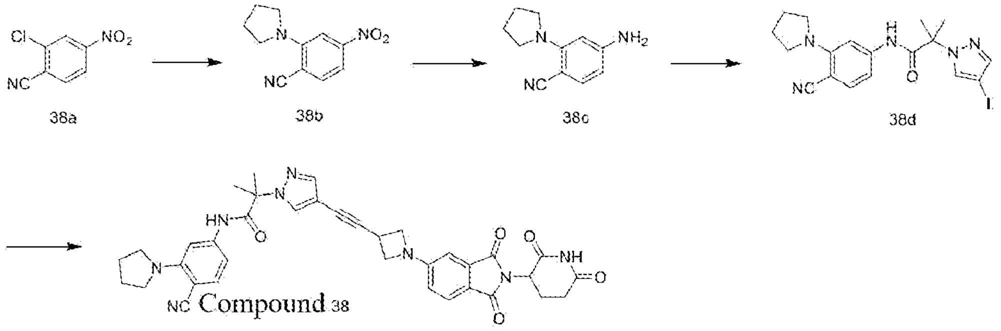
38a 38b 38c 38d
Compound 38

Step 1: 4-nitro-2-(pyrrolidin-1-yl)benzonitrile (38b)

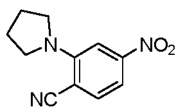

38a (2.00 g, 10.96 mmol) was dissolved in 11 mL of DMF, tetrahydropyrrole (1.17 g, 16.45 mmol) and potassium carbonate (3.78 g, 27.35 mmol) were added in sequence, and the mixture was reacted at 60°C for 3 h. The reaction liquid was cooled to room temperature, 25 mL of ethyl acetate and 10 mL of water were added, and the organic phase was separated. The organic phase was washed with 10 mL of saturated brine, dried over anhydrous sodium sulfate, and concentrated under reduced pressure. The crude product was separated and purified with silica gel chromatography column (petroleum ether : ethyl acetate (v/v) = 5 : 1) to afford 38b (0.45 g, yield: 19%).

LCMS m/z = 218.2 $[M+1]^+$.

Step 2: 4-amino-2-(pyrrolidin-1-yl)benzonitrile (38c)

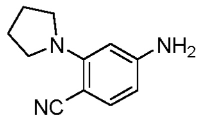

38b (0.45 g, 2.07 mmol) was dissolved in 10 mL of ethanol and 10 mL of water, zinc powder (0.68 g, 10.40 mmol) and ammonium chloride (1.11 g, 20.75 mmol) were added in sequence, and the mixture was reacted at 80°C for 4 h. After the reaction was completed, the reaction system was filtered through diatomaceous earth while it was hot. After the filtrate was cooled to room temperature, it was concentrated under reduced pressure. The residue was extracted with ethyl acetate (10 mL × 3). The organic phases were combined, and the organic phase was washed with 10 mL of saturated sodium chloride solution, dried over anhydrous sodium sulfate, and concentrated under reduced pressure to afford crude 38c (0.38 g).

Step 3: N-(4-cyano-3-(pyrrolidin-1-yl)phenyl)-2-(4-iodo-1H-pyrazol-1-yl)-2-methylpropanamide (38d)

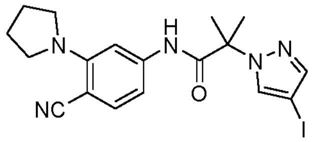

16a-1 (0.57 g, 2.04 mmol) was dissolved in THF (8 mL), 1-chloro-N,N,2-trimethylpropenylamine (0.41 g, 3.07 mmol) was added, the mixture was reacted at room temperature for 2 h, and then crude 38c (0.38 g) and TEA (0.62 g, 6.13 mmol) were added in sequence, and the mixture was reacted at room temperature for 12 h. 10 mL of ethyl acetate and 10 mL of water were added to the reaction liquid, the organic phase was separated, washed with 10 mL of saturated sodium chloride solution, dried over anhydrous sodium sulfate, and concentrated under reduced pressure. The crude product was separated and purified with silica gel chromatography column (petroleum ether : ethyl acetate (v/v) = 5 : 1) to afford 38d (0.44 g, yield: 48%).

LCMS m/z = 450.0 $[M+1]^+$.

Step 4: N-(4-cyano-3-(pyrrolidin-1-yl)phenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 38)

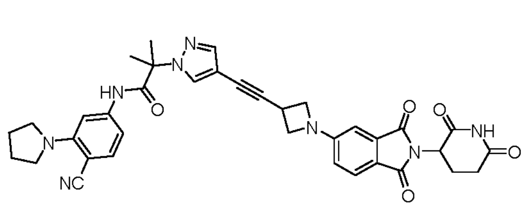

38d (0.44 g, 0.98 mmol) was dissolved in 5 mL of DMF, and intermediate 1 (0.40 g), TEA (0.30 g, 2.96 mmol), CuI (0.037 g, 0.19 mmol) and $PdCl_2(PPh_3)_2$ (0.069 g, 0.098 mmol) were added in sequence, and the mixture was reacted at 60°C for 2 h. The reaction liquid was cooled to room temperature, DCM (30 mL) was added, 30 mL of water was added, the organic phase was separated, the aqueous phase was extracted with DCM (30 mL × 3), the organic phases were combined, and the organic phase was washed with 50 mL of saturated sodium chloride solution, dried over anhydrous sodium sulfate, and concentrated under reduced pressure. The crude product was separated and purified with silica gel chromatography column (petroleum ether/ethyl acetate (v/v) = 1 : 1) to afford compound 38 (0.12 g, yield: 19%).

$^1H$ NMR (400 MHz, $CDCl_3$) δ 8.76 (s, 1H), 7.97 (s, 1H), 7.77 (d, 2H), 7.67 (d, 1H), 7.33 (d, 1H), 7.26 - 7.23 (m, 1H), 6.83 - 6.78 (m, 1H), 6.60 - 6.45 (m, 2H), 5.00 - 4.88 (m, 1H), 4.41 - 4.31 (m, 2H), 4.11 - 4.02 (m, 2H), 3.87 - 3.75

(m, 1H), 3.68 - 3.54 (m, 4H), 2.96 - 2.65 (m, 3H), 2.20 - 2.08 (m, 1H), 2.06 - 1.96 (m, 4H), 1.90 (s, 6H).
LCMS m/z = 659.3 [M+1]$^+$.

**Example 39: Preparation of compound 39**

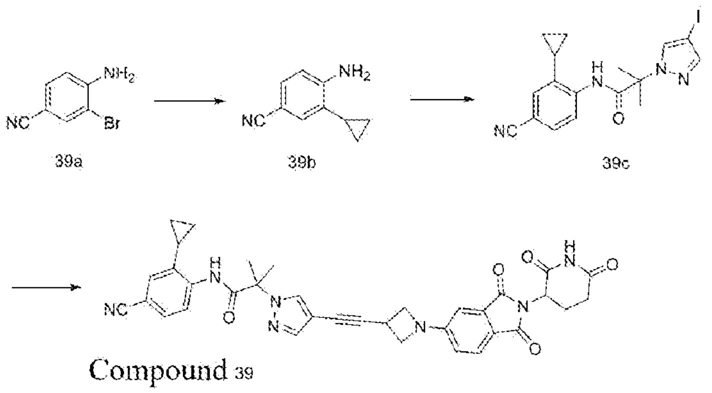

39a 39b 39c

Compound 39

Step 1: 4-amino-3-cyclopropylbenzonitrile (39b)

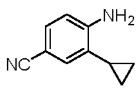

39a (2.00 g, 10.15 mmol), cyclopropylboronic acid (1.31 g, 15.25 mmol), anhydrous potassium phosphate (8.62 g, 40.61 mmol), and tricyclohexylphosphine (1.14 g, 3.63 mmol) and palladium acetate (0.46 g, 2.05 mmol) were added to a reaction flask in sequence, nitrogen replacement was performed three times, 1,4-dioxane (25 mL) and distilled water (2.5 mL) were added under nitrogen atmosphere, and the mixture was reacted at 90°C for 16 h. The reaction liquid was cooled to room temperature, and extracted by adding ethyl acetate (40 mL × 3). The organic phase was washed with 20 mL of saturated sodium chloride solution, dried over anhydrous sodium sulfate, and concentrated under reduced pressure. The crude product was separated and purified with silica gel chromatography column (ethyl acetate/petroleum ether (v/v) = 1 : 5) to afford 39b (1.32 g, yield: 82%).
LCMS m/z= 159.1 [M+1]$^+$.

Step 2: N-(4-cyano-2-cyclopropylphenyl)-2-(4-iodo-1H-pyrazol-1-yl)-2-methylpropanamide (39c)

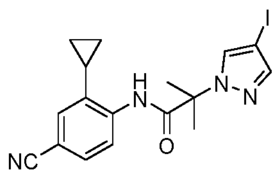

1-chloro-N,N,2-trimethylpropenylamine (0.72 g, 5.38 mmol), 16a-1 (1.00 g, 3.58 mmol) and DCM (20 mL) were added to a reaction flask respectively, and the mixture was stirred at room temperature for 1 h, then TEA (1.48 mL, 10.65 mmol) and 39b (0.56 g, 3.54 mmol) were added in sequence, and the mixture was reacted at room temperature for 1 h. The reaction liquid was concentrated under reduced pressure, and the crude product was separated and purified with silica gel chromatography column (ethyl acetate/petroleum ether (v/v) = 1 : 5) to afford 39c (1.05 g, yield: 71%).
LCMS m/z = 421.0 [M+1]$^+$.

Step 3: N-(4-cyano-2-cyclopropylphenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 39)

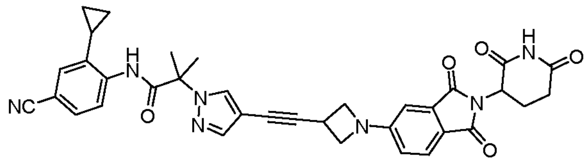

Under nitrogen atmosphere, 39c (0.50 g, 1.19 mmol), intermediate 1 (0.40 g), and $PdCl_2(PPh_3)_2$ (84 mg, 0.12 mmol) and CuI (45 mg, 0.24 mmol) were added to the reaction flask in sequence, DMF (10 mL) and DIPEA (0.59 mL, 3.39 mmol) were added, and the mixture was reacted at 50°C for 2 h. The reaction liquid was cooled to room temperature, the reaction liquid was added dropwise into 20 mL of ice water, the mixture was filtered by suction and washed with 10 mL of water, the filter cake was collected, the filter cake was dissolved with 20 mL of DCM, the mixture was dried over anhydrous sodium sulfate, concentrated under reduced pressure and then the crude product was separated and purified with silica gel column chromatography (ethyl acetate/petroleum ether (v/v) = 1 : 1), and the purified crude product was further separated and purified with silica gel column chromatography (ethyl acetate/petroleum ether (v/v) = 2 : 1) to afford compound 39 (210 mg, yield: 28%).

$^1H$ NMR (400 MHz, $CDCl_3$) $\delta$ 9.09 (s, 1H), 8.37 (d, 1H), 8.07 (s, 1H), 7.81 (s, 1H), 7.72 (s, 1H), 7.67 (d, 1H), 7.49 (dd, 1H), 7.40 - 7.35 (m, 1H), 6.81 (d, 1H), 6.55 (dd, 1H), 4.98 - 4.89 (m, 1H), 4.42 - 4.30 (m, 2H), 4.11 - 4.01 (m, 2H), 3.87 - 3.73 (m, 1H), 2.95 - 2.65 (m, 3H), 2.18 - 2.08 (m, 1H), 1.96 (s, 6H), 1.56 - 1.45 (m, 1H), 1.04 - 0.93 (m, 2H), 0.63 - 0.53 (m, 2H).

LCMS m/z = 630.3 $[M+1]^+$.

**Example 40: Preparation of compound 40**

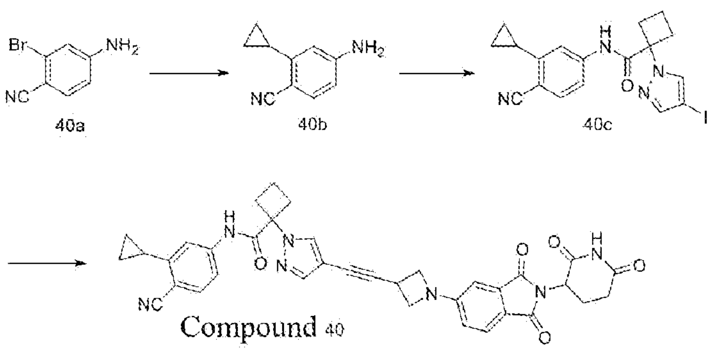

40a 40b 40c

Compound 40

Step 1: 4-amino-2-cyclopropylbenzonitrile (40b)

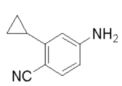

40a (2.00 g, 10.15 mmol), cyclopropylboronic acid (1.31 g, 15.25 mmol), anhydrous potassium phosphate (8.62 g, 40.61 mmol), and tricyclohexylphosphine (1.14 g, 3.63 mmol) and palladium acetate (0.46 g, 2.05 mmol) were added to a reaction flask in sequence, nitrogen replacement was performed three times, 1,4-dioxane (25 mL) and distilled water (2.5 mL) were added under nitrogen atmosphere, and the mixture was reacted at 90°C for 16 h. The reaction liquid was cooled to room temperature, and extracted with ethyl acetate (40 mL $\times$ 3). The organic phase was washed with 20 mL of saturated sodium chloride solution, dried over anhydrous sodium sulfate, and concentrated under reduced pressure.

The crude product was separated and purified with silica gel column chromatography (ethyl acetate/petroleum ether (v/v) = 1 : 5) to afford 40b (1.32 g, yield: 82%).

LCMS m/z = 159.1 $[M+1]^+$.

Step 2: N-(4-cyano-3-cyclopropylphenyl)-1-(4-iodo-1H-pyrazol-1-yl)cyclobutane-1-carboxamide (40c)

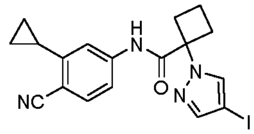

14b (11.5 g, 35.9 mmol) was dissolved in THF (100 mL), 20 mL of water was added and then lithium hydroxide monohydrate (3.0 g, 71.5 mmol) was added, and the mixture was reacted at room temperature for 30 min. 1 mol/L dilute hydrochloric acid was added dropwise to the reaction liquid to adjust the pH to 6, 250 mL of ethyl acetate was added, the liquid separation was conducted, the organic phase was washed with 100 mL of saturated sodium chloride solution, dried over anhydrous sodium sulfate, and concentrated under reduced pressure to afford a crude product (10.0 g). 1-chloro-N,N,2-trimethylpropenylamine (3.80 g, 28.45 mmol), the above crude product (5.54 g) and DCM (100 mL) were added in sequence to a reaction flask, and the mixture was stirred at room temperature for 1 h, then TEA (7.88 mL, 56.69 mmol) and 40b (3.00 g, 18.96 mmol) were added, and the mixture was reacted at room temperature for 1 h. The reaction liquid was concentrated under reduced pressure, and the crude product was separated and purified with silica gel column chromatography (ethyl acetate/petroleum ether (v/v) = 1 : 5) to afford 40c (4.80 g, yield: 59%).

LCMS m/z = 431.0 $[M-1]^-$.

Step 3: N-(4-cyano-3-cyclopropylphenyl)-1-(4-((1-(2-(2,6-dioxopiperidin-3-yl)-1,3-dioxoisoindolin-5-yl)azetidin-3-yl)ethynyl)-1H-pyrazol-1-yl)cyclobutane-1-carboxamide (Compound 40)

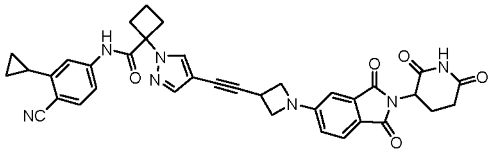

Under nitrogen atmosphere, 40c (3.00 g, 6.94 mmol), intermediate 1 (2.34 g), and $PdCl_2(PPh_3)_2$ (0.49 g, 0.70 mmol) and CuI (0.26 g, 1.37 mmol) were added to the reaction flask in sequence, 50 mL of DMF and DIPEA (3.45 mL, 20.87 mmol) were added, and the mixture was reacted at 50°C for 2 h. The reaction liquid was cooled to room temperature, the reaction liquid was added dropwise into 200 mL of ice water, the mixture was filtered by suction and washed with 50 mL of water, the filter cake was collected, the filter cake was dissolved with 300 mL of DCM, the mixture was dried over anhydrous sodium sulfate, concentrated under reduced pressure and then the crude product was separated and purified with silica gel column chromatography (ethyl acetate/petroleum ether (v/v) = 1 : 1), and the purified crude product was further separated and purified with silica gel column chromatography (ethyl acetate/petroleum ether (v/v) = 2 : 1) to afford compound 40 (1.45 g, yield: 33%).

$^1$H NMR (400 MHz, $CDCl_3$) δ 8.36 (s, 2H), 7.77 (s, 1H), 7.72 - 7.63 (m, 2H), 7.49 - 7.43 (m, 1H), 7.21 (dd, 1H), 7.18 - 7.13 (m, 1H), 6.83 - 6.76 (m, 1H), 6.55 (dd, 1H), 4.98 - 4.88 (m, 1H), 4.41 - 4.29 (m, 2H), 4.11 - 4.00 (m, 2H), 3.87 - 3.74 (m, 1H), 3.10 - 2.96 (m, 2H), 2.95 - 2.64 (m, 5H), 2.28 - 1.99 (m, 4H), 1.16 - 1.07 (m, 2H), 0.82 - 0.74 (m, 2H).

LCMS m/z = 642.3 $[M+1]^+$.

**Example 41: Preparation of compound 41**

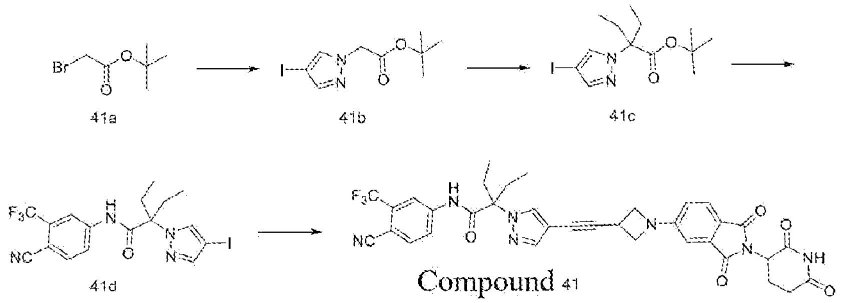
41a
41b
41c
41d
Compound 41

Step 1: tert-butyl 2-(4-iodo-1H-pyrazol-1-yl)acetate (41b)

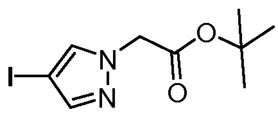

4-iodopyrazole (2 g, 10.31 mmol) was dissolved in 20 mL of acetonitrile, 41a (2.41 g, 12.36 mmol) and cesium carbonate (5.05 g, 15.50 mmol) were added in sequence, and the mixture was reacted at 70°C for 5 min. The reaction liquid was cooled to room temperature and filtered by suction. The filtrate was concentrated under reduced pressure. The crude product was separated and purified by silica gel chromatography column (ethyl acetate/petroleum ether (v/v) = 1 : 10-1 : 5) to afford 41b (3 g, yield: 94%).

Step 2: tert-butyl 2-ethyl-2-(4-iodo-1H-pyrazol-1-yl)butanoate (41c)

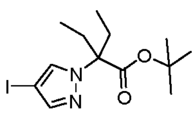

41b (2 g, 6.49 mmol) and ethyl iodide (4.05 g, 25.97 mmol) were dissolved in 20 mL of DMSO, 1.4 g of 60% NaH was slowly added under ice bath, and after the addition was completed, the mixture was reacted at room temperature for 16 h. 10 mL of saturated aqueous ammonium chloride solution was slowly added dropwise to the reaction liquid, 20 mL of water and 40 mL of ethyl acetate were added for extraction, the organic layer was washed with saturated sodium chloride solution (20 mL $\times$ 3), dried over anhydrous sodium sulfate, and concentrated under reduced pressure, and the crude product was separated and purified with silica gel chromatography column (ethyl acetate/petroleum ether (v/v) = 0 : 1-1 : 10) to afford 41c (1.6 g, yield: 68%).

$^1H$ NMR (400 MHz, $CDCl_3$) $\delta$ 7.71 (s, 1H), 7.54 (s, 1H), 2.26 - 2.14 (m, 4H), 1.44 (s, 9H), 0.76 (t, 6H).

Step 3: N-(4-cyano-3-(trifluoromethyl)phenyl)-2-ethyl-2-(4-iodo-1H-pyrazol-1-yl)butanamide (41d)

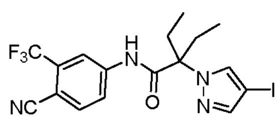

41c (1.6 g, 4.39 mmol) was dissolved in 10 mL of a solution of 4 mol/L 1,4-dioxane hydrogen chloride solution and the mixture was reacted at 60°C for 2 h. The reaction liquid was cooled to room temperature and concentrated under reduced pressure to afford a crude product (0.95 g). The above crude product (0.95 g) was dissolved in 20 mL of dry DCM, 1-chloro-N,N,2-trimethylpropenylamine (618 mg, 4.63 mmol) was added, the mixture was stirred at room temperature for 1 h, then TEA (933.24 mg, 9.22 mmol) and 4-amino-2-(trifluoromethyl)benzonitrile (515.6 mg, 2.77

mmol) were added, and the mixture was reacted at room temperature for 1 h. 0.2 mL of water was added to the reaction liquid, the mixture was concentrated under reduced pressure, and the crude product was separated and purified with silica gel chromatography column (petroleum ether/ethyl acetate (v/v) = 89 : 11) to afford 41d (270 mg, yield: 20%).

Step 4: N-(4-cyano-3-(trifluoromethyl)phenyl)-2-(4-((1-(2-(2,6-dioxopiperidin-3-yl)-1,3-dioxoisoindolin-5-yl)azetidin-3-yl)ethynyl)-1H-pyrazol-1-yl)-2-ethylbutanamide (Compound 41)

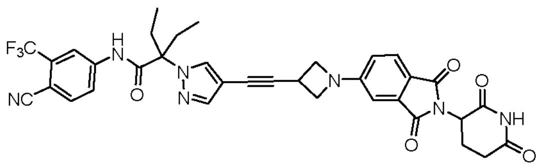

10 mL of dry DMF was added to 41d (270 mg, 0.57 mmol), intermediate 1 (230 mg) and TEA (180 mg, 1.78 mmol) were added in sequence, nitrogen replacement was performed three times, $PdCl_2(PPh_3)_2$ (40 mg, 0.057 mmol) and CuI (22 mg, 0.12 mmol) were added, nitrogen replacement was performed three times, and the mixture was reacted at 60°C for 2 h. The reaction liquid was cooled to room temperature, 100 mL of saturated ammonium chloride solution was slowly added, the mixture was filtered by suction, the filter cake was washed with 10 mL of water three times, the filter cake was dissolved in 200 mL of DCM, the mixture was dried over anhydrous sodium sulfate, and concentrated under reduced pressure, the crude product was separated and purified with silica gel chromatography column (petroleum ether/ethyl acetate (v/v) = 43 : 57) to afford compound 41 (170 mg, yield: 43%).

$^1H$ NMR (400 MHz, $CDCl_3$) δ 10.35 (s, 1H), 8.01 - 7.96 (m, 1H), 7.95 - 7.89 (m, 1H), 7.88 - 7.82 (m, 1H), 7.81 - 7.73 (m, 3H), 7.71 - 7.64 (m, 1H), 6.85 - 6.77 (m, 1H), 6.60 - 6.51 (m, 1H), 4.98 - 4.89 (m, 1H), 4.45 - 4.30 (m, 2H), 4.15 - 4.00 (m, 2H), 3.90 - 3.75 (m, 1H), 2.96 - 2.66 (m, 3H), 2.46 - 2.05 (m, 5H), 0.92 - 0.80 (m, 6H).

LCMS m/z = 686.2 $[M+1]^+$.

**Example 42: Preparation of compound 42**

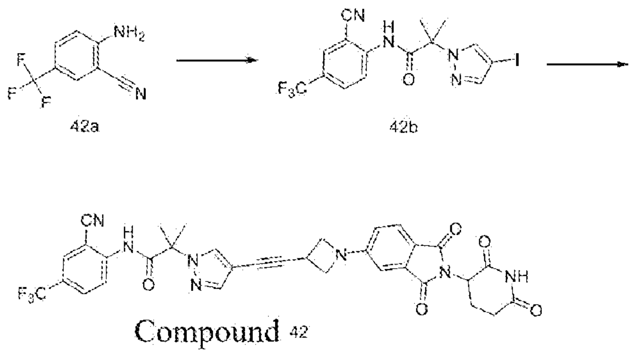

Compound 42

Step 1: N-(2-cyano-4-(trifluoromethyl)phenyl)-2-(4-iodo-1H-pyrazol-1-yl)-2-methylpropanamide (42b)

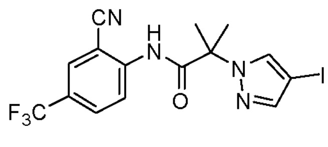

16a-1 (1.0 g, 3.58 mmol) was dissolved in 20 mL of dry DCM, 1-chloro-N,N,2-trimethylpropenylamine (718 mg, 5.37 mmol) was added, the mixture was reacted at room temperature for 1 h, then TEA (1.08 g, 10.67 mmol) and 42a

(600 mg, 3.22 mmol) were added, and the mixture was reacted at room temperature for 1 h. 0.2 mL of water was added to the reaction liquid, the mixture was concentrated under reduced pressure, and the crude product was separated and purified with silica gel column chromatography (petroleum ether/ethyl acetate (v/v) = 9 : 1) to afford 42b (670 mg, yield: 46%).

Step 2: N-(2-cyano-4-(trifluoromethyl)phenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 42)

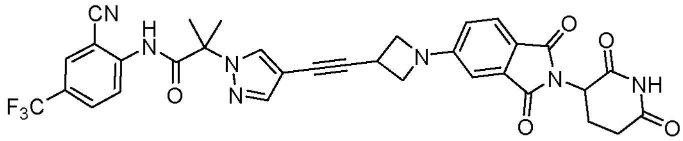

42b (200 mg, 0.45 mmol) was dissolved in 10 mL of dry DMF, intermediate 1 (180 mg) and TEA (140 mg, 1.38 mmol) were added, nitrogen replacement was performed three times, $PdCl_2(PPh_3)_2$ (32.0 mg, 0.045 mmol) and CuI (17 mg, 0.089 mmol) were added, nitrogen replacement was performed three times, and the mixture was reacted at 60°C for 2 h. The reaction liquid was cooled to room temperature, 90 mL of saturated ammonium chloride solution was slowly added, a yellow solid was precipitated, suction-filtration was performed, the filter cake was washed with 10 mL of water three times, the filter cake was dissolved in 100 mL of DCM, the mixture was dried over anhydrous sodium sulfate, and concentrated under reduced pressure, the crude product was separated and purified with silica gel column chromatography (petroleum ether/ethyl acetate (v/v) = 46 : 54) to afford compound 42 (120 mg, yield: 41%).

$^1H$ NMR (400 MHz, $CDCl_3$) δ 9.98 (s, 1H), 8.59 (d, 1H), 7.98 - 7.74 (m, 5H), 7.72 - 7.62 (m, 1H), 6.84 - 6.78 (m, 1H), 6.56 (dd, 1H), 4.98 - 4.89 (m, 1H), 4.43 - 4.30 (m, 2H), 4.14 - 4.01 (m, 2H), 3.88 - 3.75 (m, 1H), 2.95 - 2.65 (m, 3H), 2.19 - 2.08 (m, 1H), 1.95 (s, 6H).

LCMS m/z = 658.2 $[M+1]^+$.

**Example 43: Preparation of compound 43**

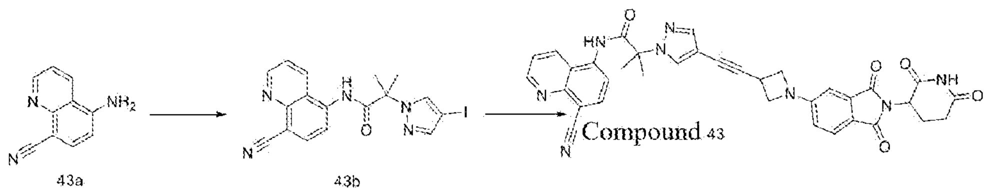

Step 1: N-(8-cyanoquinolin-5-yl)-2-(4-iodo-1H-pyrazol-1-yl)-2-methylpropanamide (43b)

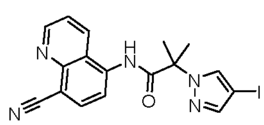

16a-1 (1.0 g, 3.58 mmol) was dissolved in 20 ml of dry DCM, 1-chloro-N,N,2-trimethylpropenylamine (718 mg, 5.37 mmol) was added, the mixture was reacted at room temperature for 1 h, then TEA (1.08 g, 10.67 mmol) and 43a (548 mg, 3.24 mmol) were added, and the mixture was reacted at room temperature for 1 h. 0.2 mL of water was added to the reaction liquid, the mixture was concentrated under reduced pressure, and the crude product was separated and purified with silica gel chromatography column (petroleum ether/ethyl acetate (v/v) = 9 : 1) to afford 43b (230 mg, yield: 16%).

Step 2: N-(8-cyanoquinolin-5-yl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 43)

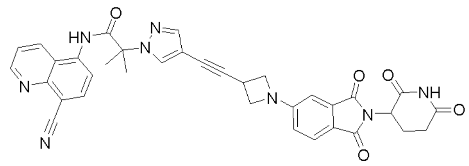

10 mL of dry DMF was added to 43b (230 mg, 0.53 mmol), intermediate 1 (215 mg) and TEA (160 mg, 1.58 mmol) were added, nitrogen replacement was performed three times, $PdCl_2(PPh_3)_2$ (38.7 mg, 0.055 mmol) and CuI (20.1 mg, 0.106 mmol) were added, nitrogen replacement was performed three times, and the mixture was reacted at 60°C for 2 h. The reaction liquid was cooled to room temperature, 100 mL of saturated ammonium chloride solution was slowly added, the mixture was filtered by suction, the filter cake was washed with 10 mL of water three times, the filter cake was dissolved in 200 mL of DCM, the mixture was dried over anhydrous sodium sulfate, and concentrated under reduced pressure, the crude product was separated and purified with silica gel column chromatography (petroleum ether/ethyl acetate (v/v) = 43 : 57) to afford compound 43 (51 mg, yield: 15%).

$^1H$ NMR (400 MHz, $CDCl_3$) $\delta$ 10.14 (s, 1H), 9.17 - 9.07 (m, 1H), 8.45 - 8.35 (m, 1H), 8.25 - 8.05 (m, 2H), 7.98 - 7.80 (m, 3H), 7.75 - 7.55 (m, 2H), 6.84 - 6.76 (m, 1H), 6.55 (dd, 1H), 4.98 - 4.88 (m, 1H), 4.43 - 4.30 (m, 2H), 4.14 - 3.97 (m, 2H), 3.87 - 3.75 (m, 1H), 3.00 - 2.66 (m, 3H), 2.20 - 2.08 (m, 1H), 2.00 (s, 6H).

LCMS m/z = 641.2 $[M+1]^+$.

**Example 44: Preparation of compound 44**

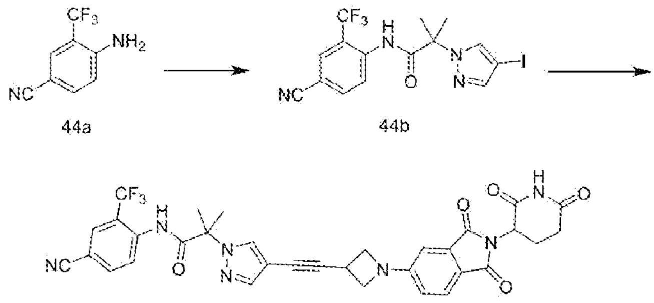

Compound 44

Step 1: N-(4-cyano-2-(trifluoromethyl)phenyl)-2-(4-iodo-1H-pyrazol-1-yl)-2-methylpropanamide (44b)

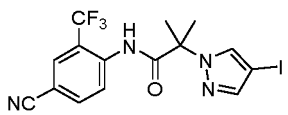

16a-1 (0.99 g, 3.54 mmol) was dissolved in 30 mL of DCM, 1-chloro-N,N,2-trimethylpropenylamine (0.65 g, 4.86 mmol) was added, the mixture was reacted at room temperature for 2 h, and then TEA (1.03 g, 10.18 mmol) and 44a (0.63 g, 3.38 mmol) were added in sequence, and the mixture was reacted at room temperature for 3 h. 100 mL of DCM and 50 mL of saturated aqueous sodium bicarbonate solution were added to the reaction liquid, the organic phase was separated, washed with 80 mL of saturated sodium chloride solution, dried over anhydrous sodium sulfate, and concentrated under reduced pressure. The crude product was separated and purified with silica gel chromatography column (petroleum ether/ethyl acetate (v/v) = 5 : 1) to afford 44b (0.38 g, yield: 25%).

Step 2: N-(4-cyano-2-(trifluoromethyl)phenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 44)

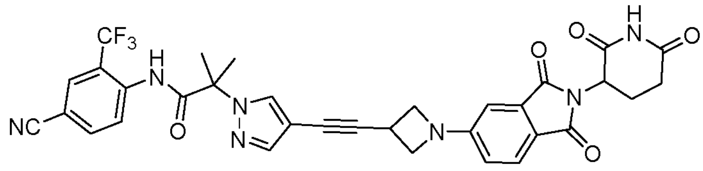

44b (0.21 g, 0.47 mmol) was dissolved in 6 mL of DMF, and intermediate 1 (0.19 g), TEA (0.15 g, 1.48 mmol), CuI (10 mg, 0.053 mmol) and $PdCl_2(PPh_3)_2$ (35 mg, 0.05 mmol) were added in sequence, and the mixture was reacted at 55°C for 1 h. The reaction liquid was cooled to room temperature, 50 mL of water was added, and the mixture was filtered by suction. The filter cake was washed with 10 mL of water. The filter cake was dissolved in 100 mL of DCM, the mixture was dried over anhydrous sodium sulfate, and concentrated under reduced pressure. The crude product was separated and purified with silica gel chromatography column (petroleum ether/ethyl acetate (v/v) = 1 : 1) to afford compound 44 (0.12 g, yield: 39%).

$^1H$ NMR (400 MHz, $CDCl_3$) $\delta$ 9.36 (s, 1H), 8.53 - 8.45 (m, 1H), 7.99 (s, 1H), 7.88 - 7.84 (m, 1H), 7.83 - 7.74 (m, 3H), 7.67 (d, 1H), 6.84 - 6.78 (m, 1H), 6.56 (dd, 1H), 4.98 - 4.89 (m, 1H), 4.42 - 4.32 (m, 2H), 4.12 - 4.02 (m, 2H), 3.88 - 3.75 (m, 1H), 2.97 - 2.62 (m, 3H), 2.20 - 2.06 (m, 1H), 1.93 (s, 6H).

LCMS m/z = 658.2 $[M+1]^+$.

**Example 45: Preparation of compound 45**

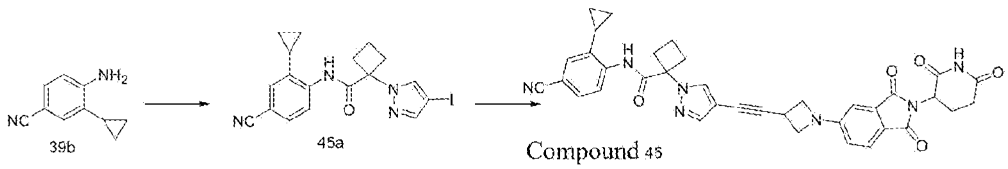

39b 45a Compound 45

Step 1: N-(4-cyano-2-cyclopropylphenyl)-1-(4-iodo-1H-pyrazol-1-yl)cyclobutane-1-carboxamide (45a)

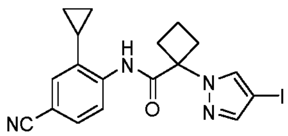

14b (11.5 g, 35.9 mmol) was dissolved in THF (100 mL), 20 mL of water and lithium hydroxide monohydrate (3.0 g, 71.5 mmol) were added, and the mixture was reacted at room temperature for 30 min. 1 mol/L dilute hydrochloric acid was added dropwise to the reaction liquid to adjust the pH to 6, 250 mL of ethyl acetate was added, the liquid separation was conducted, the organic phase was washed with 100 mL of saturated sodium chloride solution, dried over anhydrous sodium sulfate, and concentrated under reduced pressure to afford a crude product (10.0 g). 1-chloro-N,N,2-trimethylpropenylamine (3.80 g, 28.45 mmol), the above crude product (5.54 g) and DCM (100 mL) were added respectively to a reaction flask, and the mixture was stirred at room temperature for 1 h, then TEA (7.88 mL, 56.69 mmol) and 39b (3.00 g, 18.96 mmol) were added, and the mixture was reacted at room temperature for 1 h. The reaction liquid was concentrated under reduced pressure, and the crude product was separated and purified with silica gel column chromatography (ethyl acetate/petroleum ether (v/v) = 1 : 5) to afford 45a (6.05 g, yield: 74%).

LCMS m/z = 431.0 $[M-1]^-$.

Step 2: N-(4-cyano-2-cyclopropylphenyl)-1-(4-((1-(2-(2,6-dioxopiperidin-3-yl)-1,3-dioxoisoindolin-5-yl)azetidin-3-yl)ethynyl)-1H-pyrazol-1-yl)cyclobutane-1-carboxamide (Compound 45)

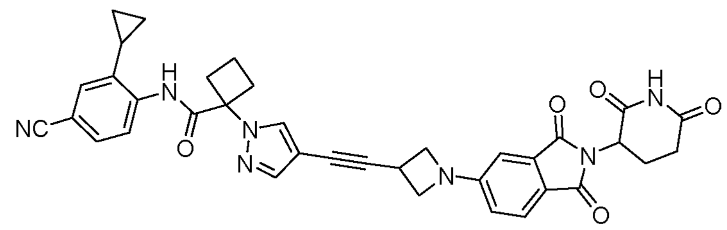

Under nitrogen atmosphere, 45a (3.00 g, 6.94 mmol), intermediate 1 (2.34 g), $PdCl_2(PPh_3)_2$ (0.49 g, 0.70 mmol) and CuI (0.26 g, 1.37 mmol) were added to the reaction flask in sequence, 50 mL of DMF and DIPEA (3.45 mL, 20.87 mmol) were added, and the mixture was reacted at 50°C for 2 h. The reaction liquid was cooled to room temperature, the reaction liquid was added dropwise into 200 mL of ice water, the mixture was filtered by suction and washed with 50 mL of water, the filter cake was collected, the filter cake was dissolved with 200 mL of DCM, the mixture was dried over anhydrous sodium sulfate, concentrated under reduced pressure and then the crude product was separated and purified with silica gel column chromatography (ethyl acetate/petroleum ether (v/v) = 1 : 1), and the purified crude product was further separated and purified with silica gel column chromatography (ethyl acetate/petroleum ether (v/v) = 1 : 1), and then the crude product was slurried with 36 mL of a mixed solvent (ethyl acetate/petroleum ether/dichloromethane (v/v) = 1 : 5 : 2.5), the mixture was filtered by suction to afford compound 45 (1.56 g, yield: 35%).

$^1H$ NMR (400 MHz, $CDCl_3$) δ 8.69 (s, 1H), 8.41 (d, 1H), 7.99 (s, 1H), 7.71 (s, 2H), 7.67 (d, 1H), 7.54 - 7.46 (m, 1H), 7.39 - 7.34 (m, 1H), 6.83 - 6.77 (m, 1H), 6.55 (dd, 1H), 5.00 - 4.88 (m, 1H), 4.43 - 4.29 (m, 2H), 4.10 - 4.00 (m, 2H), 3.87 - 3.73 (m, 1H), 3.15 - 3.00 (m, 2H), 2.96 - 2.65 (m, 5H), 2.32 - 2.00 (m, 3H), 1.53 - 1.38 (m, 1H), 1.05 - 0.90 (m, 2H), 0.60 - 0.49 (m, 2H).

LCMS m/z = 640.2 $[M-1]^-$.

**Example 46: Preparation of compound 46**

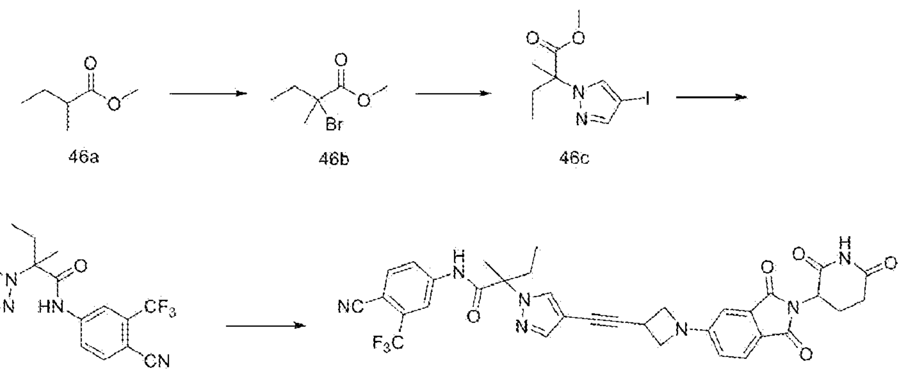

46a 46b 46c

46d Compound 46

Step 1: methyl 2-bromo-2-methylbutanoate (46b)

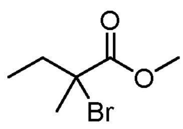

46a (3.0 g, 25.83 mmol) was dissolved in carbon tetrachloride (50 mL), 2,2-azobisisobutyronitrile (0.4 g, 2.44

mmol) and N-bromosuccinimide (4.83 g, 27.14 mmol) were added, and the mixture was reacted at 80°C for 3 h. The reaction liquid was cooled to room temperature, filtered by suction, and the filtrate was concentrated under reduced pressure. The crude product was separated and purified with silica gel chromatography column (petroleum ether/dichloromethane (v/v) = 100 : 1-2 : 1) to afford 46b (1.7 g, yield: 34%).

Step 2: methyl 2-(4-iodo-1H-pyrazol-1-yl)-2-methylbutanoate (46c)

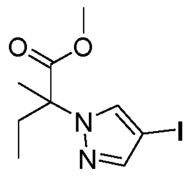

46b (1.4 g, 7.18 mmol) was dissolved in acetonitrile (30 mL), 4-iodo-1H-pyrazole (1.39 g, 7.17 mmol) and cesium carbonate (3.04 g, 9.33 mmol) were added in sequence, and the mixture was reacted at 80°C for 2 h. The reaction liquid was cooled to room temperature, filtered by suction, and the filtrate was concentrated under reduced pressure. The crude product was separated and purified with silica gel chromatography column (petroleum ether/ethyl acetate (v/v) = 100 : 1-10 : 1) to afford 46c (650 mg, yield: 29%).

LCMS m/z = 309.0 $[M+1]^+$.

Step 3: N-(4-cyano-3-(trifluoromethyl)phenyl)-2-(4-iodo-1H-pyrazol-1-yl)-2-methylbutanamide (46d)

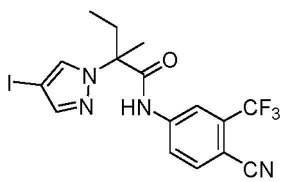

46c (650 mg, 2.11 mmol) was dissolved in a mixed solvent of tetrahydrofuran/methanol (12 mL/3 mL), 3 mL of an aqueous solution of lithium hydroxide monohydrate (0.89 g, 21.1 mmol) was added and the mixture was reacted at room temperature for 16 h. 2 mol/L aqueous hydrochloric acid solution was slowly added dropwise to the reaction system to adjust the pH to 4, 20 mL of ethyl acetate was added for extraction, the organic phase was further washed with 20 mL of saturated sodium chloride solution, dried over anhydrous sodium sulfate, and concentrated under reduced pressure to afford a crude product (600 mg). The above crude product (500 mg) was dissolved in 5 mL of pyridine, and 4-amino-2-(trifluoromethyl)benzonitrile (316 mg, 1.70 mmol) and a solution of T3P in 50% ethyl acetate (2.16 g) were added in sequence, the mixture was reacted at 30°C for 16 h. The reaction liquid was cooled to room temperature, 20 mL of ethyl acetate was added, the mixture was washed with 10 mL of saturated sodium bicarbonate solution and 10 mL of saturated sodium chloride solution, the organic phase was collected, dried over anhydrous sodium sulfate, and concentrated under reduced pressure. The crude product was separated and purified with silica gel chromatography column (petroleum ether/ethyl acetate (v/v) = 1 : 0-4 : 1) to afford 46d (220 mg, yield: 27%).

LCMS m/z = 463.0 $[M+1]^+$.

Step 4: N-(4-cyano-3-(trifluoromethyl)phenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylbutanamide (Compound 46)

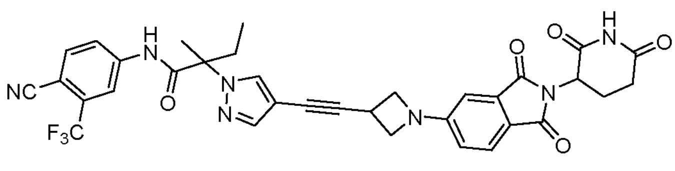

46d (200 mg, 0.43 mmol) was dissolved in 3 mL of DMF, and intermediate 1 (160 mg), CuI (8.2 mg, 0.043 mmol), $PdCl_2(PPh_3)_2$ (8.2 mg, 0.012 mmol) and TEA (0.18 mL, 1.29 mmol) were added in sequence, and the mixture was reacted at 55°C for 1 h under nitrogen protection. The reaction system was cooled to room temperature, 10 mL of water was added to the reaction liquid, the solid was precipitated, suction-filtration was performed, the filter cake was dissolved with 100 mL of DCM, the mixture was dried over anhydrous sodium sulfate, and concentrated under reduced pressure. The crude product was separated and purified with silica gel chromatography column (petroleum ether/ethyl acetate (v/v) = 10 : 1-1 : 2) to afford compound 46 (90 mg, yield: 31%).

$^1H$ NMR (400 MHz, $CDCl_3$) δ 9.87 (s, 1H), 7.99 - 7.88 (m, 2H), 7.86 - 7.62 (m, 5H), 6.84 - 6.77 (m, 1H), 6.56 (dd, 1H), 4.98 - 4.88 (m, 1H), 4.43 - 4.30 (m, 2H), 4.12 - 4.00 (m, 2H), 3.88 - 3.73 (m, 1H), 2.96 - 2.65 (m, 3H), 2.48 - 2.32 (m, 1H), 2.28 - 2.07 (m, 2H), 1.86 (s, 3H), 0.81 (t, 3H).

LCMS m/z = 672.2 $[M+1]^+$.

**Example 47: Preparation of compound 47**

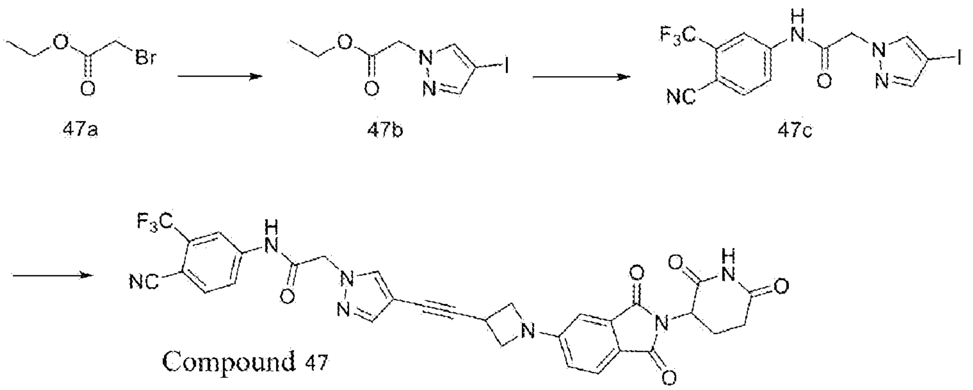


Step 1: ethyl 2-(4-iodo-1H-pyrazol-1-yl)acetate (47b)

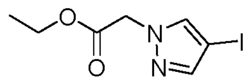

47a (0.80 g, 4.79 mmol) and 4-iodo-1H-pyrazole (1.02 g, 5.26 mmol) were dissolved in 20 mL of acetonitrile, cesium carbonate (2.34 g, 7.18 mmol) was added, and the mixture was reacted at 80°C for 3 h. The reaction liquid was cooled to room temperature, filtered through diatomaceous earth, and the filter cake was washed with 50 mL of ethyl acetate. The organic phase was collected and concentrated under reduced pressure. The crude product was separated and purified with silica gel chromatography column (petroleum ether/ethyl acetate (v/v) = 5 : 1) to afford 47b (1.11 g, yield: 83%).

LCMS m/z = 281.0 $[M+1]^+$.

Step 2: N-(4-cyano-3-(trifluoromethyl)phenyl)-2-(4-iodo-1H-pyrazol-1-yl)acetamide (47c)

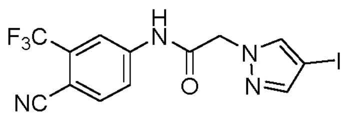

47b (1.11 g, 3.96 mmol) was dissolved in a mixed solvent of tetrahydrofuran (20 mL) and methanol (7 mL), 7 mL of an aqueous solution of lithium hydroxide monohydrate (0.33 g, 7.86 mmol) was added, and the mixture was reacted at 35°C for 16 h. The reaction liquid was cooled to room temperature, the pH was adjusted to 4 with 3 mol/L

hydrochloric acid solution, the mixture was extracted with 200 mL of ethyl acetate, the organic phase was washed with 50 mL of water and 50 mL of saturated sodium chloride solution, dried over anhydrous sodium sulfate and concentrated under reduced pressure to afford a crude product (0.87 g). The above crude product (0.40 g) and 4-amino-2-(trifluoromethyl)benzonitrile (0.30 g, 1.61 mmol) were dissolved in 10 mL of pyridine, a solution of T3P in 50% ethyl acetate (2.02 g) was added, and the mixture was reacted at 35°C for 16 h. The reaction liquid was cooled to room temperature and concentrated under reduced pressure, 10 mL of saturated sodium bicarbonate solution was added, 200 mL of ethyl acetate was added, the liquid separation was conducted, the organic phase was washed with water (50 mL × 3), and then washed with 50 mL of saturated sodium chloride solution, dried over anhydrous sodium sulfate, and concentrated under reduced pressure. The crude product was separated and purified with silica gel chromatography column (petroleum ether/ethyl acetate (v/v) = 3 : 1) to afford 47c (0.59 g, yield: 77%).

LCMS m/z = 421.0 $[M+1]^+$.

Step 3: N-(4-cyano-3-(trifluoromethyl)phenyl)-2-(4-((1-(2-(2,6-dioxopiperidin-3-yl)-1,3-dioxoisoindolin-5-yl)azetidin-3-yl)ethynyl)-1H-pyrazol-1-yl)acetamide (Compound 47)

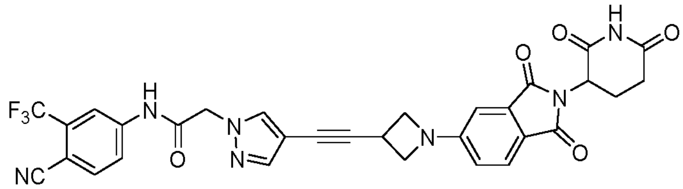

47c (0.15 g, 0.36 mmol) and intermediate 1 (0.13 g) were dissolved in 10 mL of DMF, and $PdCl_2(PPh_3)_2$ (0.03 g, 0.043 mmol), CuI (0.01 g, 0.053 mmol) and TEA (0.11 g, 1.09 mmol) were added in sequence, and the mixture was reacted at 55°C for 1 h under nitrogen protection. The reaction liquid was cooled to room temperature, 200 mL of ethyl acetate was added, the liquid separation was conducted, the organic phase was washed with water (50 mL × 3), then washed with 50 mL of saturated sodium chloride solution, dried over anhydrous sodium sulfate, and concentrated under reduced pressure, the crude product was separated and purified with silica gel chromatography column (petroleum ether/ethyl acetate (v/v) = 1 : 2) to afford compound 47 (0.09 g, yield: 40%).

$^1H$ NMR (400 MHz, $CDCl_3$) δ 9.23 (s, 1H), 8.07 (s, 1H), 7.95 - 7.72 (m, 4H), 7.72 - 7.61 (m, 2H), 6.80 (d, 1H), 6.56 (dd, 1H), 5.00 - 4.87 (m, 3H), 4.43 - 4.30 (m, 2H), 4.13 - 4.02 (m, 2H), 3.88 - 3.75 (m, 1H), 3.00 - 2.62 (m, 3H), 2.20 - 2.07 (m, 1H).

LCMS m/z = 630.2 $[M+1]^+$.

**Example 48: Preparation of compound 48**

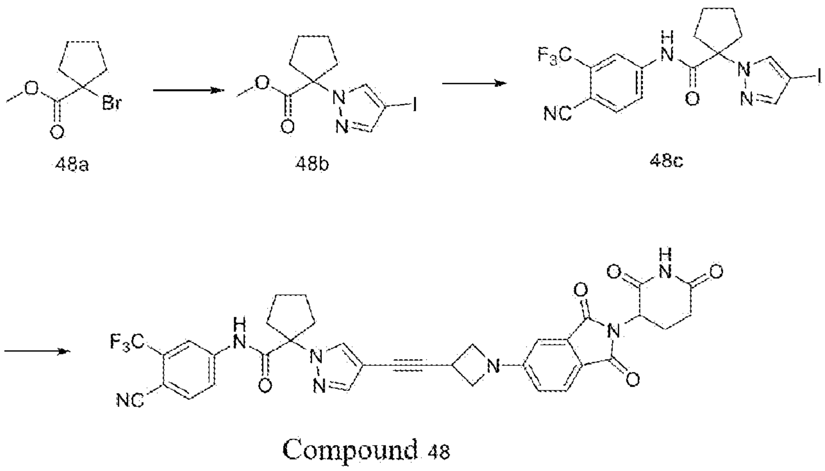

48a 48b 48c

Compound 48

Step 1: methyl 1-(4-iodo-1H-pyrazol-1-yl)cyclopentane-1-carboxylate (48b)

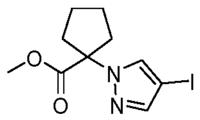

48a (0.70 g, 3.38 mmol) and 4-iodo-1H-pyrazole (0.72 g, 3.71 mmol) were dissolved in 20 mL of acetonitrile, cesium carbonate (1.65 g, 5.06 mmol) was added, and the mixture was reacted at 80°C for 3 h. The reaction liquid was cooled to room temperature, filtered through diatomaceous earth, and the filter cake was washed with 100 mL of ethyl acetate. The organic phase was collected and concentrated under reduced pressure. The crude product was separated and purified with silica gel column chromatography (petroleum ether/ethyl acetate (v/v) = 4 : 1) to afford 48b (0.85 g, yield: 79%).

LCMS m/z = 321.0 $[M+1]^+$.

Step 2: N-(4-cyano-3-(trifluoromethyl)phenyl)-1-(4-iodo-1H-pyrazol-1-yl)cyclopentane-1-carboxamide (48c)

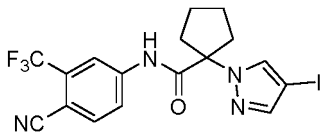

48b (0.95 g, 2.97 mmol) was dissolved in a mixed solvent of tetrahydrofuran (15 mL) and methanol (5 mL), 5 mL of an aqueous solution of lithium hydroxide monohydrate (0.25 g, 5.96 mmol) was added, and the mixture was stirred at 20°C for 16 h. The reaction system was adjusted to pH 4 with 3 mol/L aqueous hydrochloric acid solution, extracted with 200 mL of ethyl acetate, the organic phase was washed with 50 mL of water and 50 mL of saturated sodium chloride solution, dried over anhydrous sodium sulfate, and concentrated under reduced pressure to afford a crude product (0.9 g). The above crude product (0.45 g) and 4-amino-2-(trifluoromethyl)benzonitrile (0.27 g, 1.45 mmol) were dissolved in 5 mL of pyridine, a solution of T3P in 50% ethyl acetate (1.87 g) was added, and the mixture was reacted at 35°C for 16 h. The reaction liquid was cooled to room temperature and concentrated under reduced pressure, 10 mL of saturated aqueous sodium bicarbonate solution was added, 200 mL of ethyl acetate was added, the liquid separation was conducted, the organic phase was washed with water (50 mL × 3), and then washed with 50 mL of saturated sodium chloride solution, dried over anhydrous sodium sulfate, and concentrated under reduced pressure. The crude product was separated and purified with silica gel chromatography column (petroleum ether/ethyl acetate (v/v) = 1 : 1) to afford 48c (0.31 g, yield: 44%).

LCMS m/z = 475.0 $[M+1]^+$.

Step 3: N-(4-cyano-3-(trifluoromethyl)phenyl)-1-(4-((1-(2-(2,6-dioxopiperidin-3-yl)-1,3-dioxoisoindolin-5-yl)azetidin-3-yl)ethynyl)-1H-pyrazol-1-yl)cyclopentane-1-carboxamide (Compound 48)

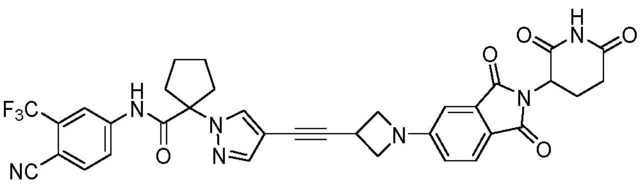

48c (0.15 g, 0.32 mmol) and intermediate 1 (0.12 g) were dissolved in 10 mL of DMF, and $PdCl_2(PPh_3)_2$ (0.02 g, 0.028 mmol), CuI (0.01 g, 0.053 mmol) and TEA (0.10 g, 0.99 mmol) were added in sequence, and the mixture was reacted at 55°C for 1 h under nitrogen protection. The reaction liquid was cooled to room temperature, 200 mL of ethyl acetate was added, the organic phase was washed with water (50 mL × 3), then washed with 50 mL of saturated sodium

chloride solution, dried over anhydrous sodium sulfate, and concentrated under reduced pressure, the crude product was separated and purified with silica gel chromatography column (petroleum ether/ethyl acetate (v/v) = 1 : 2) to afford compound 48 (0.07 g, yield: 32%).

$^1$H NMR (400 MHz, $CDCl_3$) $\delta$ 9.02 (s, 1H), 7.99 (s, 1H), 7.92 - 7.87 (m, 1H), 7.80 (s, 1H), 7.78 - 7.63 (m, 4H), 6.83 - 6.77 (m, 1H), 6.55 (dd, 1H), 5.00 - 4.88 (m, 1H), 4.42 - 4.29 (m, 2H), 4.12 - 4.00 (m, 2H), 3.88 - 3.72 (m, 1H), 2.96 - 2.48 (m, 7H), 2.20 - 1.88 (m, 3H), 1.81 - 1.65 (m, 2H).

LCMS m/z = 684.2 $[M+1]^+$.

**Example 49: Preparation of compound 49**

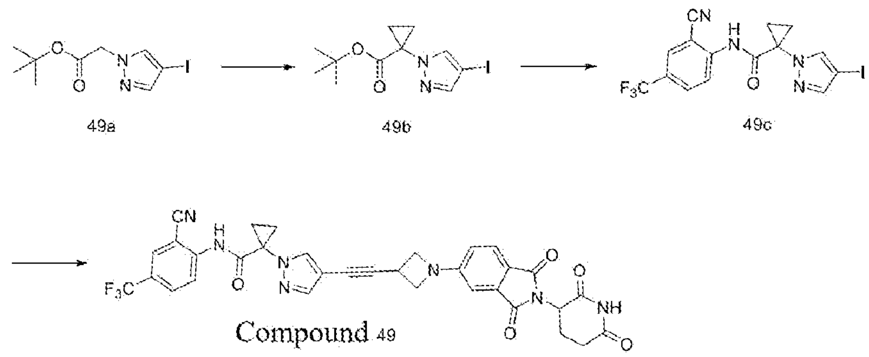

49a 49b 49c

Compound 49

Step 1: tert-butyl 1-(4-iodo-1H-pyrazol-1-yl)cyclopropane-1-carboxylate (49b)

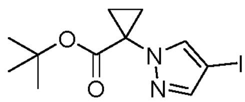

49a (3.00 g, 9.74 mmol) was dissolved in anhydrous THF (30 mL), the mixture was cooled to -78°C, a solution of 1 mol/L LiHMD in THF (10.71 mL, 10.71 mmol) was slowly added dropwise, and after the dropwise addition was completed, the reaction was continued at -78°C for 10 min, then 1,3,2-dioxatetrahydrothiophene 2,2-dioxide (CAS: 1072-53-3) (1.33 g, 10.72 mmol) was added, and then the mixture was warmed slowly to 0°C, and reacted at 0°C for 1 h. The reaction liquid was cooled to -78°C, a solution of 1 mol/L LiHMDS in THF (10.71 mL, 10.71 mmol) was slowly added dropwise, and after the dropwise addition was completed, the mixture was slowly warmed to room temperature until the reaction was completed. 100 mL of saturated ammonium chloride solution was added to the reaction liquid, 300 mL of ethyl acetate was added for extraction, the organic phase was washed with 50 mL of water and 50 mL of saturated sodium chloride solution, dried over anhydrous sodium sulfate, and concentrated under reduced pressure. The crude product was separated and purified with silica gel chromatography column (petroleum ether/ethyl acetate (v/v) = 4 : 1) to afford 49b (2.79 g, yield: 86%).

Step 2: N-(2-cyano-4-(trifluoromethyl)phenyl)-1-(4-iodo-1H-pyrazol-1-yl)cyclopropane-1-carboxamide (49c)

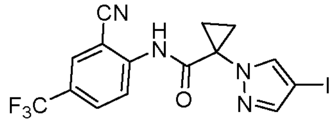

49b (1.00 g, 2.99 mmol) was dissolved in a solution of 4 mol/L 1,4-dioxane hydrogen chloride solution (15 mL) and the mixture was reacted at 60°C for 1 h. The reaction liquid was cooled to room temperature and concentrated under reduced pressure to afford a crude product (0.83 g). The above crude product (0.45 g) and 2-amino-5-(trifluoromethyl)benzonitrile (301.53 mg, 1.62 mmol) were dissolved in 10 mL of pyridine, a solution of T3P in 50% ethyl acetate (2.06 g) was added, the mixture was reacted at 35°C for 16 h. The reaction liquid was cooled to room temperature and concentrated under reduced pressure, 10 mL of saturated sodium bicarbonate solution was added, 200 mL of ethyl acetate was added, the liquid separation was conducted, the organic phase was washed with water (50 mL × 3), and then washed with 50 mL of saturated sodium chloride solution, dried over anhydrous sodium sulfate, and concentrated under reduced pressure. The crude product was separated and purified with silica gel chromatography column (petroleum ether/ethyl acetate (v/v) = 3 : 1) to afford 49c (0.498 g, yield: 69%).

LCMS m/z = 447.0 $[M+1]^+$.

Step 3: N-(2-cyano-4-(trifluoromethyl)phenyl)-1-(4-((1-(2-(2,6-dioxopiperidin-3-yl)-1,3-dioxoisoindolin-5-yl)azetidin-3-yl)ethynyl)-1H-pyrazol-1-yl)cyclopropane-1-carboxamide (Compound 49)

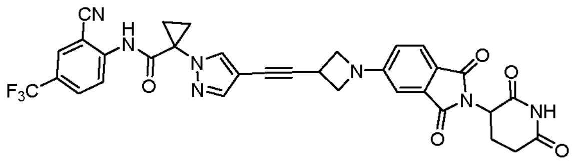

49c (200.0 mg, 0.45 mmol) was added into a 50 mL single-mouth bottle, then dry DMF (10 mL) was added, intermediate 1 (180 mg) and TEA (140 mg, 1.38 mmol) were added in sequence, nitrogen replacement was performed 3 times, $PdCl_2(PPh_3)_2$ (32 mg, 0.046 mmol) and CuI (17 mg, 0.089 mmol) were added, nitrogen replacement was performed three times, and the mixture was reacted at 60°C for 2 h. The reaction liquid was cooled to room temperature, saturated ammonium chloride solution (90 mL) was slowly added, a yellow solid was precipitated, suction-filtration was performed, the filter cake was washed with 10 mL of water three times, the filter cake was dissolved in 100 mL of DCM, the mixture was dried over anhydrous sodium sulfate, the crude product was separated and purified with silica gel chromatography column (petroleum ether/ethyl acetate (v/v) = 46 : 54) to afford compound 49 (127 mg, yield: 43%).

$^1H$ NMR (400 MHz, $CDCl_3$) δ 8.24 (s, 1H), 8.05 - 7.94 (m, 1H), 7.88 - 7.63 (m, 6H), 6.84 - 6.77 (m, 1H), 6.56 (dd, 1H), 4.99 - 4.88 (m, 1H), 4.43 - 4.31 (m, 2H), 4.13 - 4.00 (m, 2H), 3.88 - 3.75 (m, 1H), 2.98 - 2.63 (m, 3H), 2.20 - 2.07 (m, 1H), 2.00 - 1.90 (m, 2H), 1.64 - 1.53 (m, 2H).

LCMS m/z = 656.2 $[M+1]^+$.

**Example 50: Preparation of compound 50**

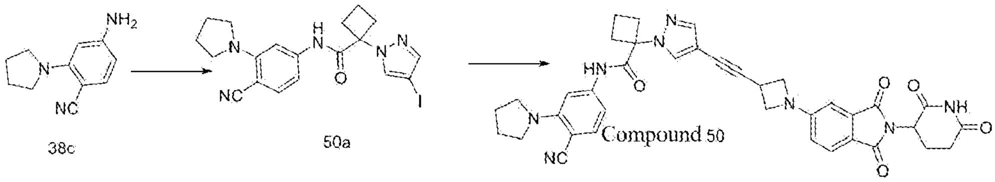
38c
50a
Compound 50

Step 1: N-(4-cyano-3-(pyrrolidin-1-yl)phenyl)-1-(4-iodo-1H-pyrazol-1-yl)cyclobutane-1-carboxamide (50a)

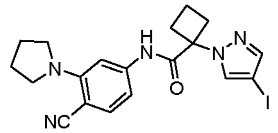

14b (11.5 g, 35.9 mmol) was dissolved in THF (100 mL), 20 mL of water and lithium hydroxide monohydrate (3.0 g, 71.5 mmol) were added, and the mixture was reacted at room temperature for 30 min. 1 mol/L dilute hydrochloric acid was added dropwise to the reaction liquid to adjust the pH to 6, 250 mL of ethyl acetate was added, the liquid

separation was conducted, the organic phase was washed with 100 mL of saturated sodium chloride solution, dried over anhydrous sodium sulfate, and concentrated under reduced pressure to afford a crude product (10.0 g). The above crude product (3.6 g) was added to a 100 mL single-mouth bottle, then 40 mL of dry DCM was added, 1-chloro-N,N,2-trimethylpropenylamine (2.48 g, 18.57 mmol) was added, and the mixture was stirred at room temperature for 1 h, then TEA (2.5 g, 24.7 mmol) was added, and crude 38 (2.1 g) was added, and the mixture was reacted at room temperature for 1.5 h. 30 mL of water was added to the reaction liquid, the mixture was extracted with DCM (40 mL × 2), the organic phase was washed with saturated brine (25 mL × 2), dried over anhydrous sodium sulfate, and concentrated under reduced pressure. The crude product was separated and purified with silica gel chromatography column (petroleum ether/ethyl acetate (v/v) = 47 : 53) to afford 50a (3.0 g, yield: 50%).

LCMS m/z = 462.1 $[M+1]^+$.

Step 2: N-(4-cyano-3-(pyrrolidin-1-yl)phenyl)-1-(4-((1-(2-(2,6-dioxopiperidin-3-yl)-1,3-dioxoisoindolin-5-yl)azetidin-3-yl)ethynyl)-1H-pyrazol-1-yl)cyclobutane-1-carboxamide (Compound 50)

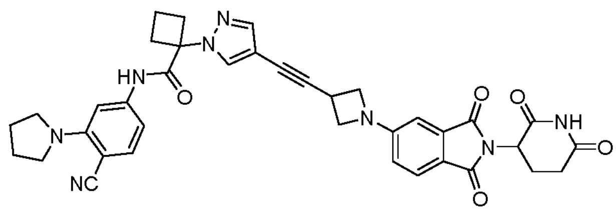

50a (3.0 g, 6.5 mmol) was added into a 100 mL single-mouth bottle, 30 mL of dry DMF was added, intermediate 1 (2.6 g) and TEA (1.97 g, 19.5 mmol) were added in sequence, nitrogen replacement was performed three times, and $PdCl_2(PPh_3)_2$ (25.6 mg, 0.036 mmol) and CuI (13.3 mg, 0.07 mmol) were added, nitrogen replacement was performed three times, and the mixture was reacted at 60°C for 2 h. The reaction liquid was cooled to room temperature, 100 mL of saturated ammonium chloride solution was slowly added, a yellow solid was precipitated, filtration was performed, the filter cake was washed with water (10 mL × 3), the filter cake was dissolved in 100 mL of DCM, the mixture was dried over anhydrous sodium sulfate and concentrated under reduced pressure, and the crude product was separated and purified with silica gel chromatography column (petroleum ether/ethyl acetate (v/v) = 47 : 53) to afford a crude product, which was subjected to Prep-HPLC (instrument and preparative column: using SHIMADZU LC-20AP & SHIMADZU SPD -20A preparative liquid phase chromatographic instrument, preparative column model: YMC Triart C18, 70 mm × 250 mm, 7 μm). Preparation method: the crude product was dissolved with methanol and dimethyl sulfoxide, and filtered with a 0.45 μm filter membrane, to prepare into a sample liquid. Mobile phase system: acetonitrile/water (containing 10 mmol/L $NH_4HCO_3$). Gradient elution method: gradient elution of 50% to 74% acetonitrile (elution time: 20 min), lyophilization was performed to afford compound 50 (185 mg, yield: 4%).

$^1H$ NMR (400 MHz, $CDCl_3$) δ 8.28 (s, 1H), 7.97 - 7.91 (m, 1H), 7.80 - 7.73 (m, 1H), 7.71 - 7.64 (m, 2H), 7.40 - 7.31 (m, 2H), 6.83 - 6.78 (m, 1H), 6.71 - 6.63 (m, 1H), 6.55 (dd, 1H), 4.98 - 4.88 (m, 1H), 4.42 - 4.30 (m, 2H), 4.12 - 4.01 (m, 2H), 3.86 - 3.71 (m, 1H), 3.70 - 3.58 (m, 4H), 3.12 - 2.96 (m, 2H), 2.96 - 2.64 (m, 5H), 2.26 - 1.95 (m, 7H).

LCMS m/z = 671.2 $[M+1]^+$.

**Example 51: Preparation of compound 51**

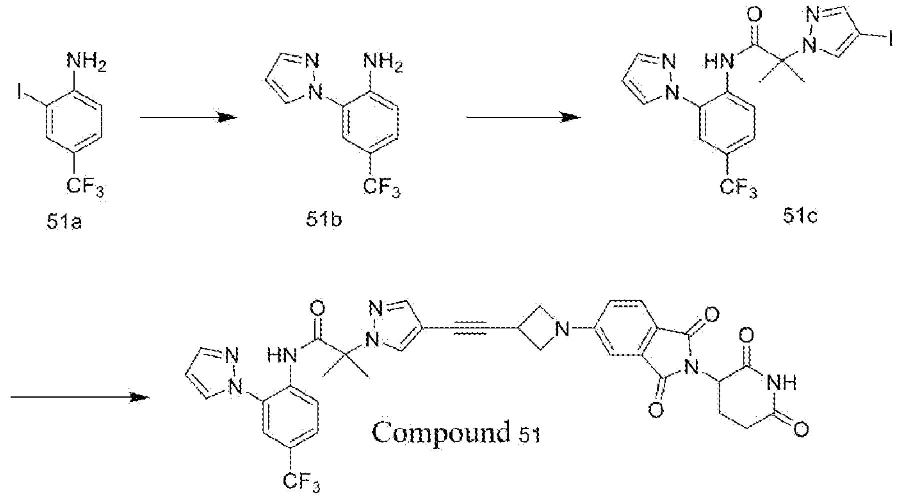
51a
51b
51c
Compound 51

Step 1: 2-(1H-pyrazol-1-yl)-4-(trifluoromethyl)aniline (51b)

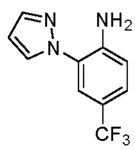

51a (500 mg, 1.74 mmol) was dissolved in 10 mL of toluene, and 1H-pyrazole (150 mg, 2.20 mmol), (1S,2S)-(+)-N,N'-dimethyl-1,2-cyclohexanediamine (50 mg, 0.35 mmol), CuI (3 mg, 0.016 mmol) and anhydrous potassium carbonate (490 mg, 3.55 mmol) were added in sequence, nitrogen replacement was performed three times, and the mixture was reacted at 100°C for 16 h. The reaction liquid was cooled to room temperature, 100 mL of saturated aqueous sodium chloride solution was added, the mixture was extracted with 100 mL of ethyl acetate, dried over anhydrous sodium sulfate, and concentrated under reduced pressure. The crude product was separated and purified with silica gel chromatography column (petroleum ether/ethyl acetate (v/v) = 4 : 1) to afford 51b (320 mg, yield: 81%).

Step 2: N-(2-(1H-pyrazol-1-yl)-4-(trifluoromethyl)phenyl)-2-(4-iodo-1H-pyrazol-1-yl)-2-methylpropanamide (51c)

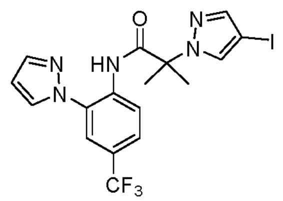

16a-1 (470 mg, 1.68 mmol) was dissolved in 5 mL of DCM, 1-chloro-N,N,2-trimethylpropenylamine (280 mg, 2.1 mmol) was added, the mixture was reacted at room temperature for 1 h, and then 51b (320 mg, 1.41 mmol) and TEA (210 mg, 2.08 mmol) were added, and the mixture was reacted at room temperature for 2 h. 50 mL of DCM was added to the reaction liquid, 50 mL of saturated aqueous sodium bicarbonate solution was added, the organic phase was separated, the organic phase was washed with 50 mL of saturated sodium chloride solution, dried over anhydrous sodium sulfate, and concentrated under reduced pressure. The crude product was separated and purified with silica gel chromatography column (petroleum ether/ethyl acetate (v/v) = 5 : 1) to afford 51c (560 mg, yield: 81%).

Step 3: N-(2-(1H-pyrazol-1-yl)-4-(trifluoromethyl)phenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 51)

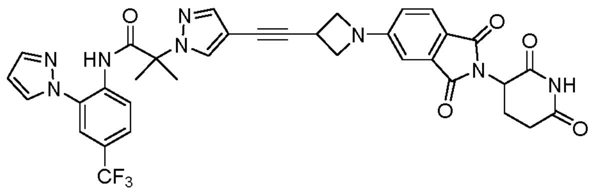

51c (300 mg, 0.61 mmol) was dissolved in 6 mL of DMF, and intermediate 1 (230 mg), TEA (180 mg, 1.78 mmol), CuI (12 mg, 0.063 mmol) and $PdCl_2(PPh_3)_2$ (43 mg, 0.061 mmol) were added in sequence, nitrogen replacement was performed three times, and the mixture was reacted at 55°C for 2 h. The reaction liquid was cooled to room temperature, 50 mL of water was added, the mixture was filtered by suction, washed with 50 mL of water, the filter cake was collected, the filter cake was dissolved with 150 mL of DCM, the mixture was dried over anhydrous sodium sulfate, and concentrated under reduced pressure. The crude product was separated and purified with silica gel chromatography column (petroleum ether/ethyl acetate (v/v) = 1 : 3) to afford compound 51 (30 mg, yield: 7%).

$^1$H NMR (400 MHz, $CDCl_3$) δ 10.53 (s, 1H), 8.69 (d, 1H), 8.08 (s, 1H), 7.83 - 7.73 (m, 2H), 7.71 - 7.62 (m, 2H), 7.61 - 7.47 (m, 3H), 6.86 - 6.78 (m, 1H), 6.57 (dd, 1H), 6.51 - 6.44 (m, 1H), 4.99 - 4.89 (m, 1H), 4.45 - 4.32 (m, 2H), 4.17 - 4.02 (m, 2H), 3.93 - 3.75 (m, 1H), 2.96 - 2.61 (m, 3H), 2.20 - 2.05 (m, 1H), 1.88 (s, 6H).

LCMS m/z = 699.2 $[M+1]^+$.

**Example 52: Preparation of compound 52**

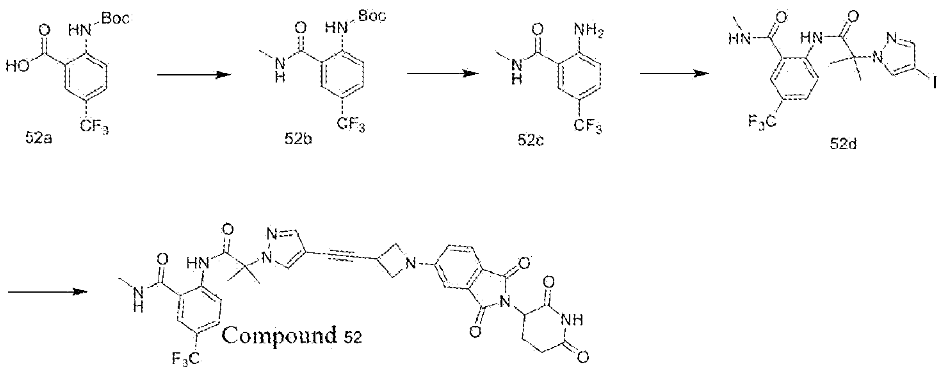

Step 1: tert-butyl (2-(methylcarbamoyl)-4-(trifluoromethyl)phenyl)carbamate (52b)

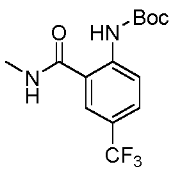

Methylamine hydrochloride (330 mg, 4.89 mmol) was dissolved in 6 mL of DCM, TEA (500 mg, 4.94 mmol) was added, the mixture was stirred at room temperature for 5 min, then 52a (300 mg, 0.983 mmol) and HATU (450 mg, 1.18 mmol) were added, the mixture was reacted at room temperature for 2 h. 50 mL of DCM was added to the reaction liquid, 30 mL of saturated aqueous sodium chloride solution was added, the organic phase was separated, dried over anhydrous sodium sulfate, and concentrated under reduced pressure. The crude product was separated and purified

with silica gel chromatography column (petroleum ether/ethyl acetate (v/v) = 9 : 1) to afford 52b (190 mg, yield: 61%).

Step 2: 2-amino-N-methyl-5-(trifluoromethyl)benzamide (52c)

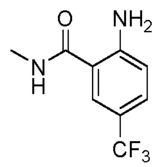

52b (190 mg, 0.597 mmol) was dissolved in 5 mL of DCM, and 3 mL of trifluoroacetic acid was added. The mixture was reacted at room temperature for 1 h. DCM (30 mL) was added to the reaction liquid, 30 mL of saturated aqueous sodium bicarbonate solution was added, the organic phase was separated, the organic phase was washed with 30 mL of saturated aqueous sodium chloride solution, dried over anhydrous sodium sulfate, and concentrated under reduced pressure to afford crude 52c (150 mg).

Step 3: 2-(2-(4-iodo-1H-pyrazol-1-yl)-2-methylpropanamido)-N-methyl-5-(trifluoromethyl)benzamide (52d)

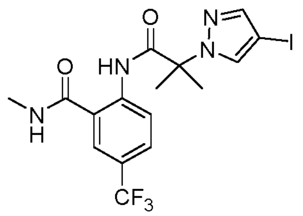

16a-1 (220 mg, 0.787 mmol) was dissolved in 5 mL of DCM, 1-chloro-N,N,2-trimethylpropenylamine (130 mg, 0.973 mmol) was added, the mixture was reacted at room temperature for 1 h, and then crude 52c (140 mg) and TEA (100 mg, 0.989 mmol) were added, and the mixture was reacted at room temperature for 2 h. DCM (30 mL) was added to the reaction liquid, 30 mL of saturated aqueous sodium bicarbonate solution was added, the organic phase was separated, the organic phase was washed with 30 mL of saturated sodium chloride solution, dried over anhydrous sodium sulfate, and concentrated under reduced pressure. The crude product was separated and purified with silica gel chromatography column (petroleum ether/ethyl acetate (v/v) = 5 : 1) to afford 52d (150 mg, yield: 40%).

Step 4: 2-(2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamido)-N-methyl-5-(trifluoromethyl)benzamide (Compound 52)

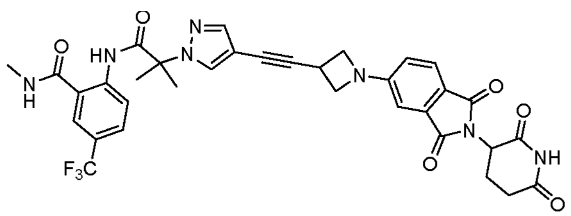

52d (150 mg, 0.31 mmol) was dissolved in 6 mL of DMF, and intermediate 1 (130 mg), TEA (38 mg, 0.376 mmol), CuI (3 mg, 0.0158 mmol) and $PdCl_2(PPh_3)_2$ (11 mg, 0.0157 mmol) were added in sequence, nitrogen replacement was performed three times, and the mixture was reacted at 55°C for 1 h. The reaction liquid was cooled to room temperature, 50 mL of water was added, the mixture was filtered by suction, washed with 50 mL of water, the filter cake was collected, the filter cake was dissolved with 150 mL of DCM, the mixture was dried over anhydrous sodium sulfate, and concentrated under reduced pressure. The crude product was separated and purified with silica gel chromatography

column (petroleum ether/ethyl acetate (v/v) = 1 : 1) to afford compound 52 (30 mg, yield: 14%).

$^1$H NMR (400 MHz, $CDCl_3$) δ 11.26 (s, 1H), 8.70 (d, 1H), 8.08 (s, 1H), 7.81 (s, 1H), 7.75 - 7.60 (m, 4H), 6.84 - 6.75 (m, 1H), 6.54 (dd, 1H), 6.40 - 6.25 (m, 1H), 4.99 - 4.88 (m, 1H), 4.41 - 4.30 (m, 2H), 4.12 - 4.00 (m, 2H), 3.87 - 3.73 (m, 1H), 2.95 (d, 3H), 2.93 - 2.66 (m, 3H), 2.17 - 2.08 (m, 1H), 1.94 (s, 6H).

LCMS m/z = 690.3 $[M+1]^+$.

**Example 53: Preparation of compound 53**

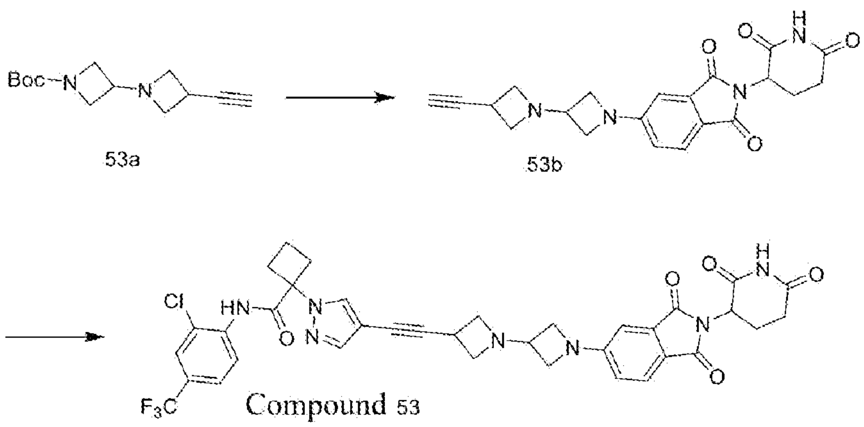

53a

53b

Compound 53

Step 1: 2-(2,6-dioxopiperidin-3-yl)-5-(3-ethynyl-[1,3'-biazetidin]-1'-yl)isoindoline-1,3-dione (53b)

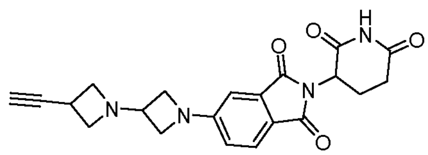

53a (see WO 2020211822 for the synthesis method) (4.0 g, 16.93 mmol) was dissolved in 80 mL of a solution of 3 mol/L ethyl acetate hydrogen chloride solution, and the mixture was reacted at 35°C for 3 h. The reaction liquid was cooled to room temperature and concentrated under reduced pressure to afford a crude product. The above crude product and 1C (4.68 g, 16.94 mmol) were dissolved in 60 mL of DMSO, the mixture was warmed to 95°C, TEA (8.47 g, 83.70 mmol) was slowly added dropwise at 95°C, and the mixture was reacted at 95°C for 3 h. The reaction liquid was cooled to room temperature, poured into 600 mL of water, the mixture was filtered, the filter cake was washed with 50 mL of water, and the filter cake was dried by blowing at 50°C for 18 h to afford crude 53b (4.5 g).

Step 2: N-(2-chloro-4-(trifluoromethyl)phenyl)-1-(4-((1'-(2-(2,6-dioxopiperidin-3-yl)-1,3-dioxoisoindolin-5-yl)-[1,3'-biazetidin]-3-yl)ethynyl)-1H-pyrazol-1-yl)cyclobutane-1-carboxamide (Compound 53)

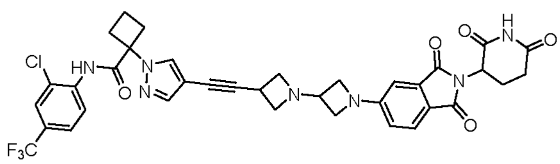

14c (150 mg, 0.32 mmol) was dissolved in 5 mL of DMF, and the above crude 53b (135 mg), TEA (97 mg, 0.96 mmol), CuI (6 mg, 0.032 mmol) and $PdCl_2(PPh_3)_2$ (22 mg, 0.031 mmol) were added in sequence, nitrogen replacement was performed three times, and the mixture was reacted at 55°C for 1.5 h. The reaction liquid was cooled to room

temperature, 40 mL of water was added, the solid was precipitated and filtered, the filter cake was washed with 20 mL of water. The filter cake was dissolved in 100 mL of DCM, the mixture was dried over anhydrous sodium sulfate, and concentrated under reduced pressure. The crude product was separated and purified with silica gel chromatography column (petroleum ether/ethyl acetate (v/v) = 1 : 1) to afford compound 53 (53 mg, yield: 23%).

$^1$H NMR (400 MHz, $CDCl_3$) δ 8.70 (s, 1H), 8.55 (d, 1H), 8.05 (s, 1H), 7.78 (s, 1H), 7.69 (s, 1H), 7.65 (d, 1H), 7.61 - 7.56 (m, 1H), 7.55 - 7.46 (m, 1H), 6.84 - 6.74 (m, 1H), 6.59 - 6.47 (m, 1H), 4.99 - 4.86 (m, 1H), 4.17 - 4.01 (m, 2H), 4.01 - 3.16 (m, 8H), 3.14 - 2.99 (m, 2H), 2.95 - 2.64 (m, 5H), 2.32 - 2.00 (m, 3H).

LCMS m/z = 734.2 $[M+1]^+$.

**Example 54: Preparation of compound 54**

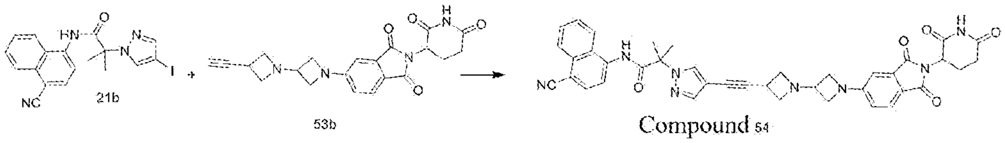

21b 53b Compound 54

21b (138 mg, 0.32 mmol) was dissolved in 5 mL of DMF, and the above crude 53b (135 mg), TEA (97 mg, 0.96 mmol), CuI (6 mg, 0.032 mmol) and $PdCl_2(PPh_3)_2$ (22 mg, 0.031 mmol) were added in sequence, nitrogen replacement was performed three times, and the mixture was reacted at 55°C for 1.5 h. The reaction liquid was cooled to room temperature, 40 mL of water was added, the solid was precipitated and filtered, the filter cake was washed with 20 mL of water. The filter cake was dissolved in 100 mL of DCM, the mixture was dried over anhydrous sodium sulfate, and concentrated under reduced pressure. The crude product was separated and purified with silica gel chromatography column (petroleum ether/ethyl acetate (v/v) = 1 : 1) to afford compound 54 (50 mg, yield: 22%).

$^1$H NMR (400 MHz, $CDCl_3$) δ 9.92 (s, 1H), 8.35 (d, 1H), 8.29 - 8.22 (m, 1H), 8.11 (s, 1H), 7.94 - 7.79 (m, 4H), 7.75 - 7.61 (m, 3H), 6.84 - 6.74 (m, 1H), 6.58 - 6.46 (m, 1H), 4.97 - 4.87 (m, 1H), 4.17 - 3.10 (m, 10H), 2.95 - 2.63 (m, 3H), 2.16 - 2.07 (m, 1H), 2.01 (s, 6H).

LCMS m/z = 695.3 $[M+1]^+$.

**Example 55: Preparation of compound 55**

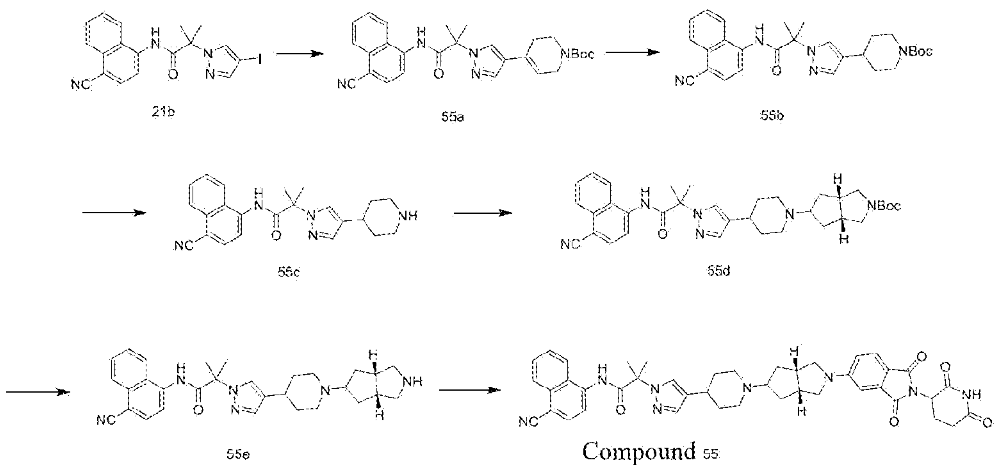

21b 55a 55b 55c 55d 55e Compound 55

Step 1: tert-butyl 4-(1-(1-((4-cyanonaphthalen-1-yl)amino)-2-methyl-1-oxopropan-2-yl)-1H-pyrazol-4-yl)-3,6-dihydropyridine-1(2H)-carboxylate (55a)

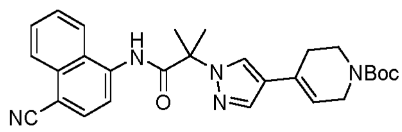

21b (2.4 g, 5.57 mmol) and tert-butyl 4-(4,4,5,5-tetramethyl-1,3,2-dioxaborolan-2-yl)-3,6-dihydropyridine-1(2H)-carboxylate (2.07 g, 6.69 mmol) were dissolved in a mixed solvent of 1,4-dioxane (30 mL) and water (5 mL), and Pd(dppf)$Cl_2$•DCM (0.46 g, 0.56 mmol) and potassium carbonate (1.54 g, 11.14 mmol) were added, nitrogen replacement was performed three times, and the mixture was reacted at 80°C for 4 h. The reaction liquid was cooled to room temperature and concentrated under reduced pressure, and the crude product was separated and purified with silica gel chromatography column (petroleum ether/ethyl acetate (v/v) = 1 : 0-3 : 7) to afford 55a (2.20 g, yield: 81%).
LCMS m/z = 486.3 $[M+1]^+$.

Step 2: tert-butyl 4-(1-(1-((4-cyanonaphthalen-1-yl)amino)-2-methyl-1-oxopropan-2-yl)- 1H-pyrazol-4-yl)piperidine-1-carboxylate (55b)

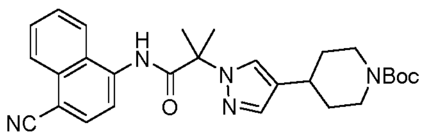

55a (2.20 g, 4.53 mmol) was dissolved in methanol (20 mL), 10% palladium on carbon (2.22 g) was added, hydrogen replacement was performed three times, and the mixture was stirred at 30°C for 2 h under hydrogen atmosphere. The reaction system was filtered by suction. The filtrate was concentrated under reduced pressure, and the crude product was separated and purified by silica gel column chromatography (petroleum ether/ethyl acetate (v/v) = 1 : 0-1 : 1) to afford 55b (1.10 g, yield: 50%).

Step 3: N-(4-cyanonaphthalen-1-yl)-2-methyl-2-(4-(piperidin-4-yl)-1H-pyrazol-1-yl)propanamide (55c) hydrochloride

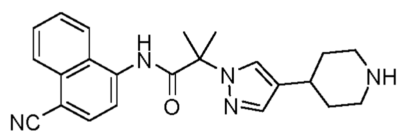

55b (1.0 g, 2.05 mmol) was dissolved in a solution of 4 mol/L 1,4-dioxane hydrogen chloride solution (20 mL), and the mixture was reacted at room temperature for 1 h. The reaction liquid was concentrated under reduced pressure to afford the hydrochloride of crude 55c (0.7 g).
LCMS m/z = 388.3 $[M+1]^+$.

Step 4: cis-tert-butyl 5-(4-(1-(1-((4-cyanonaphthalen-1-yl)amino)-2-methyl-1-oxopropan-2- yl)-1H-pyrazol-4-yl)piperidin-1-yl)hexahydrocyclopenta[c]pyrrole-2(1H)-carboxylate (55d)

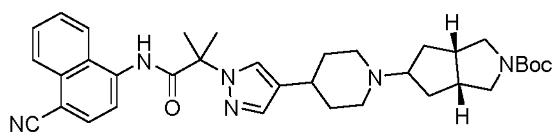

The hydrochloride of the above crude 55c (0.17 g) and tert-butyl cis-5-oxohexahydrocyclopenta[c]pyrrole-2(1H)-carboxylate (0.10 g, 0.44 mmol) were dissolved in DMAc (6 mL), acetic acid (0.1 mL) was added, the mixture was stirred at 50°C for 60 min, sodium triacetoxyborohydride (0.17 g, 0.80 mmol) was added at 50°C, and the mixture was stirred at 50°C for 16 h. The reaction liquid was cooled to room temperature, 30 mL of saturated aqueous sodium bicarbonate solution was added, the mixture was extracted with 30 mL of ethyl acetate, the organic phases were combined, concentrated under reduced pressure, and the crude product was separated and purified with silica gel chromatography column (DCM/MeOH (v/v) = 1 : 0-9 : 1) to afford 55d (0.1 g, two-step yield from compound 55b: 34%).

LCMS m/z = 597.3 $[M+1]^+$.

Step 5: cis-N-(4-cyanonaphthalen-1-yl)-2-methyl-2-(4-(1-(octahydrocyclopenta [c]pyrrol-5-yl)piperidin-4-yl)-1H-pyrazol-1-yl)propanamide (55e) hydrochloride

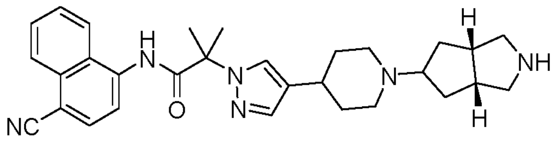

55d (0.1 g, 0.17 mmol) was dissolved in a solution of 4 mol/L 1,4-dioxane hydrogen chloride solution (20 mL) and the mixture was reacted at room temperature for 1 h. The reaction liquid was concentrated under reduced pressure to afford the hydrochloride of crude 55e (0.06 g).

LCMS m/z = 497.3 $[M+1]^+$.

Step 5: cis-N-(4-cyanonaphthalen-1-yl)-2-(4-(1-(2-(2-(2,6-dioxopiperidin-3-yl)-1,3-dioxoisoindolin-5-yl)octahydrocyclopenta[c]pyrrol-5-yl)piperidin-4-yl)-1H-pyrazol-1-yl)-2-methylpropanamide (Compound 55)

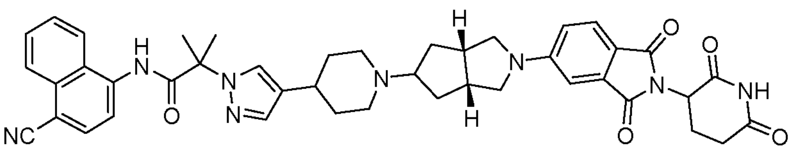

The hydrochloride of crude SSe (60 mg) was dissolved in DMSO (4 mL), 1C (46 mg, 0.17 mmol) and DIPEA (60 mg, 0.46 mmol) were added in sequence, and the mixture was reacted at 100°C for 2 h. The reaction liquid was cooled to room temperature, 20 mL of water was added, the solid was precipitated and filtered, the filter cake was collected and dried under vacuum, and the crude product was separated and purified with silica gel prep-TLC (DCM/MeOH (v/v) = 10 : 1) to afford compound 55 (15 mg, two-step yield from compound 55d: 12%).

$^1H$ NMR (400 MHz, DMSO-$d_6$) δ 11.05 (s, 1H), 9.65 (s, 1H), 8.20 - 8.06 (m, 2H), 7.96 - 7.58 (m, 7H), 6.98 - 6.89 (m, 1H), 6.85 (dd, 1H), 5.13 - 4.97 (m, 1H), 3.65 - 3.49 (m, 2H), 3.45 - 3.26 (m, 2H), 3.10 - 2.70 (m, 5H), 2.66 - 2.52 (m, 3H), 2.30 - 1.76 (m, 14H), 1.70 - 1.24 (m, 4H).

LCMS m/z = 753.3 $[M+1]^+$.

**Example 56: Preparation of compound 56**

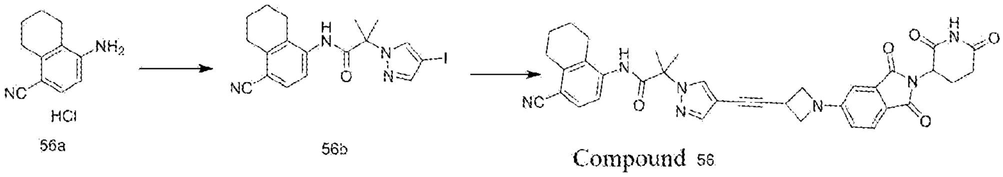

56a 56b Compound 56

Step 1: N-(4-cyano-5,6,7,8-tetrahydronaphthalen-1-yl)-2-(4-iodo-1H-pyrazol-1-yl)-2-methylpropanamide (56b)

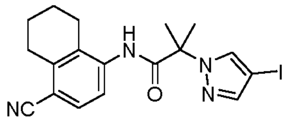

16a-1 (0.99 g, 3.54 mmol) was dissolved in 30 mL of DCM, 1-chloro-N,N,2-trimethylpropenylamine (0.71 g, 5.31 mmol) was added, the mixture was reacted at room temperature for 2 h, and then TEA (1.03 g, 10.18 mmol) and 56a (see WO 2003096980 for the synthesis method) (0.61 g, 2.92 mmol) were added in sequence, and the mixture was reacted at room temperature for 12 h. 100 mL of DCM and 50 mL of saturated aqueous sodium bicarbonate solution were added to the reaction liquid, the organic phase was separated, the organic phase was washed with 80 mL of saturated sodium chloride solution, dried over anhydrous sodium sulfate, and concentrated under reduced pressure. The crude product was separated and purified with silica gel chromatography column (petroleum ether/ethyl acetate (v/v) = 5 : 1) to afford 56b (0.8 g, yield: 63%).

Step 2: N-(4-cyano-5,6,7,8-tetrahydronaphthalen-1-yl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 56)

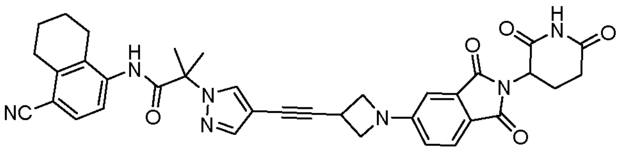

56b (0.8 g, 1.84 mmol) was dissolved in 6 mL of DMF, and intermediate 1 (0.62 g), TEA (1.11 g, 10.97 mmol), CuI (70 mg, 0.37 mmol) and $PdCl_2(PPh_3)_2$ (130 mg, 0.185 mmol) were added in sequence, and the mixture was reacted at 60°C for 3 h. The reaction liquid was cooled to room temperature, 50 mL of water was added, and the mixture was filtered by suction. The filter cake was washed with 10 mL of water. The filter cake was dissolved in 100 mL of DCM, the mixture was dried over anhydrous sodium sulfate, and concentrated under reduced pressure. The crude product was separated and purified with silica gel chromatography column (petroleum ether/ethyl acetate (v/v) = 1 : 1) to afford compound 56 (0.3 g, yield: 25%).

$^1H$ NMR (400 MHz, $CDCl_3$) δ 8.79 (s, 1H), 8.10 - 7.94 (m, 2H), 7.79 (d, 2H), 7.67 (d, 1H), 7.44 (d, 1H), 6.84 - 6.78 (m, 1H), 6.56 (dd, 1H), 4.98 - 4.89 (m, 1H), 4.42 - 4.30 (m, 2H), 4.12 - 4.02 (m, 2H), 3.87 - 3.76 (m, 1H), 2.98 - 2.65 (m, 5H), 2.43 - 2.32 (m, 2H), 2.20 - 2.07 (m, 1H), 2.00 - 1.73 (m, 10H).

LCMS m/z = 644.2 $[M+1]^+$.

**Example 57: Preparation of compound 57**

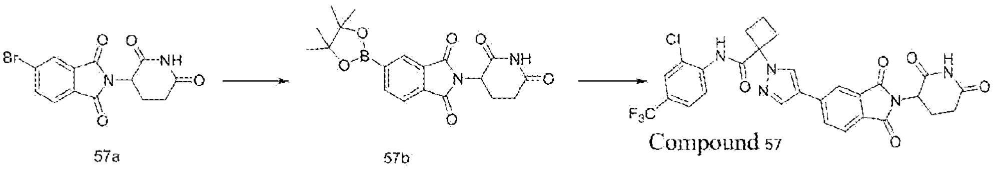

57a 57b Compound 57

Step 1: 2-(2,6-dioxopiperidin-3-yl)-5-(4,4,5,5-tetramethyl-1,3,2-dioxaborolan-2-yl)isoindoline-1,3-dione (57b)

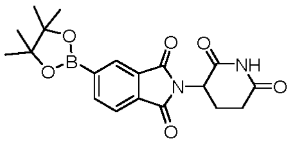

57a (see WO 2021091575 for the synthesis method) (1.00 g, 2.97 mmol), bis(pinacolato)diboron (1.13 g, 4.45 mmol), Pd(dppf)$Cl_2$•DCM (0.36 g, 0.441 mmol) and potassium acetate (0.88 g, 8.97 mmol) were added to a reaction flask respectively, nitrogen replacement was performed three times, 1,4-dioxane (20 mL) was added under nitrogen atmosphere, and the mixture was reacted at 90°C for 16 h. The reaction liquid was cooled to room temperature and filtered, water (20 mL) was added to the filtrate, the mixture was extracted with ethyl acetate (40 mL × 3), the organic phase was washed with 20 mL of saturated sodium chloride solution, dried over anhydrous sodium sulfate, and concentrated under reduced pressure, and the crude product was separated and purified with silica gel chromatography column (ethyl acetate/petroleum ether (v/v) = 1 : 1) to afford crude 57b (0.60 g).

LCMS m/z = 385.2 $[M+1]^+$.

Step 2: N-(2-chloro-4-(trifluoromethyl)phenyl)-1-(4-(2-(2,6-dioxopiperidin-3-yl)-1,3-dioxoisoindolin-5-yl)-1H-pyrazol-1-yl)cyclobutane-1-carboxamide (Compound 57)

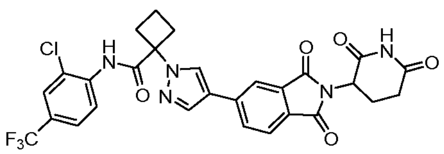

Under nitrogen atmosphere, crude 57b (600 mg), 14c (234 mg, 0.50 mmol), tetrakis triphenylphosphine palladium (120 mg, 0.104 mmol) and potassium carbonate (140 mg, 1.013 mmol) were added to a reaction flask respectively, 1,4-dioxane (10 mL) and water (1 mL) were added, and the mixture was reacted at 90°C for 16 h. The reaction system was cooled to room temperature, water (20 mL) was added, the mixture was extracted with ethyl acetate (40 mL × 3), the organic phase was washed with 20 mL of saturated sodium chloride solution, dried over anhydrous sodium sulfate, and concentrated under reduced pressure. The crude product was separated and purified with silica gel column chromatography (ethyl acetate/petroleum ether (v/v) = 1 : 1), and the obtained crude product was separated and purified with silica gel column chromatography (ethyl acetate/petroleum ether (v/v) = 1 : 1) to afford compound 57 (20 mg, yield: 7%).

$^1H$ NMR (400 MHz, $CDCl_3$) δ 8.81 (s, 1H), 8.58 (d, 1H), 8.12 (s, 1H), 8.07 (s, 1H), 7.99 (s, 2H), 7.92 - 7.83 (m, 2H), 7.62 - 7.56 (m, 1H), 7.55 - 7.48 (m, 1H), 5.05 - 4.95 (m, 1H), 3.22 - 3.06 (m, 2H), 3.00 - 2.66 (m, 5H), 2.37 - 1.96 (m, 3H).

LCMS m/z = 598.0 [M-1].

**Example 58: Preparation of compound 58**

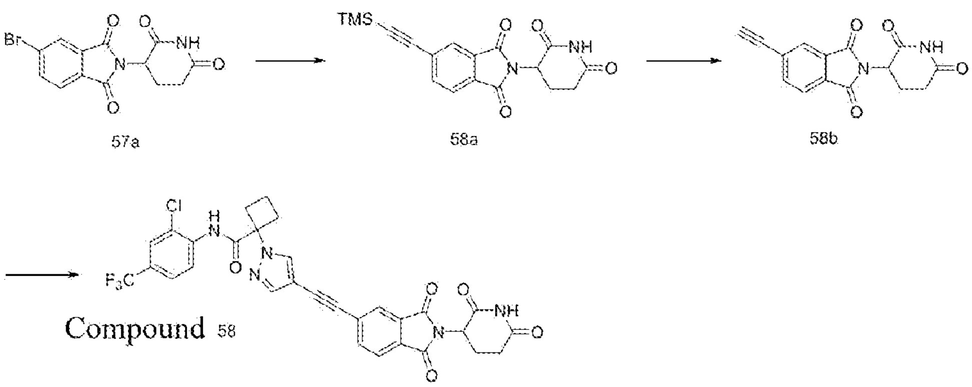

57a

58a

58b

Compound 58

Step 1: 2-(2,6-dioxopiperidin-3-yl)-5-((trimethylsilyl)ethynyl)isoindoline-1,3-dione (58a)

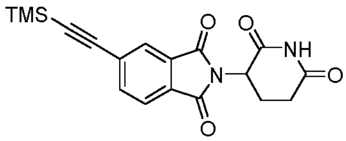

57a (see WO 2021091575 for the synthesis method) (2.00 g, 5.93 mmol), ethynyltrimethylsilane (0.76 g, 7.74 mmol), $PdCl_2(PPh_3)_2$ (0.42 g, 0.598 mmol) and CuI (0.23 g, 1.21 mmol) were added to a reaction flask respectively, nitrogen replacement was performed three times, THF (20 mL) and TEA (0.90 g, 8.89 mmol) were added under nitrogen atmosphere, and the mixture was reacted at 80°C for 16 h. The reaction system was cooled to room temperature and filtered, water (20 mL) was added to the filtrate, the mixture was extracted with ethyl acetate (40 mL × 3), the organic phase was washed with saturated sodium chloride solution (20 mL), dried over anhydrous sodium sulfate, and concentrated under reduced pressure, and the crude product was separated and purified with silica gel chromatography column (ethyl acetate/petroleum ether (v/v) = 1 : 1) to afford 58a (0.50 g, yield: 24%).
LCMS m/z = 355.1 $[M+1]^+$.

Step 2: 2-(2,6-dioxopiperidin-3-yl)-5-ethynylisoindoline-1,3-dione (58b)

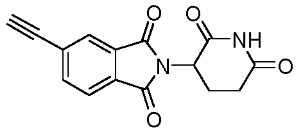

58a (0.50 g, 1.41 mmol), TBAF (0.74 g, 2.83 mmol) and THF (10 mL) were added to a reaction flask respectively, the mixture was stirred at room temperature for 1 h, then water (20 mL) was added, the mixture was extracted with ethyl acetate (40 mL × 3), the organic phase was washed with saturated sodium chloride solution (20 mL), dried over anhydrous sodium sulfate, and concentrated under reduced pressure, and the crude product was separated and purified with silica gel chromatography column (ethyl acetate/petroleum ether (v/v) = 1 : 1) to afford 58b (0.20 g, yield: 50%).
LCMS m/z = 283.1 $[M+1]^+$.

Step 3: N-(2-chloro-4-(trifluoromethyl)phenyl)-1-(4-((2-(2,6-dioxopiperidin-3-yl)-1,3-dioxoisoindolin-5-yl)ethynyl)-1H-pyrazol-1-yl)cyclobutane-1-carboxamide (Compound 58)

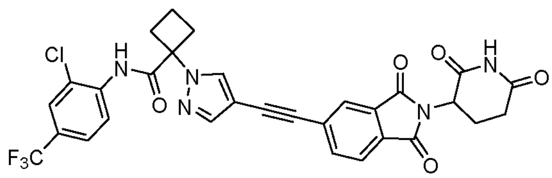

Under nitrogen atmosphere, 58b (200 mg, 0.709 mmol), 14c (120 mg, 0.256 mmol), $PdCl_2(PPh_3)_2$ (30 mg, 0.043 mmol) and CuI (16 mg, 0.084 mmol) were added to the reaction flask respectively, DMF (5 mL) and DIPEA (0.21 mL, 1.27 mmol) were added, and the mixture was reacted at 50°C for 2 h. The reaction system was cooled to room temperature, water (20 mL) was added, the mixture was extracted with ethyl acetate (40 mL × 3), the organic phase was washed with saturated sodium chloride solution (20 mL), dried over anhydrous sodium sulfate, and concentrated under reduced pressure. The crude product was separated and purified with silica gel column chromatography (ethyl acetate/petroleum ether (v/v) = 1 : 1), and the obtained crude product was separated and purified with silica gel column chromatography (ethyl acetate/petroleum ether (v/v) = 1 : 1) to afford compound 58 (25 mg, yield: 6%).
$^1H$ NMR (400 MHz, $CDCl_3$) δ 8.67 (s, 1H), 8.49 (d, 1H), 8.23 (s, 1H), 7.92 - 7.68 (m, 5H), 7.56 - 7.39 (m, 2H),

4.98 - 4.86 (m, 1H), 3.12 - 2.96 (m, 2H), 2.91 - 2.60 (m, 5H), 2.28 - 1.94 (m, 3H).

LCMS m/z = 622.0 $[M-1]^-$.

**Example 59: Preparation of compound 59**

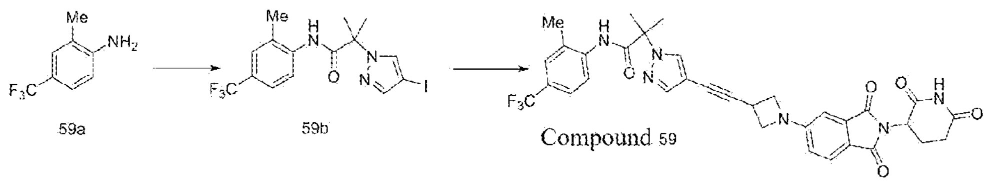


Step 1: 2-(4-iodo-1H-pyrazol-1-yl)-2-methyl-N-(2-methyl-4-(trifluoromethyl) phenyl)propanamide (59b)

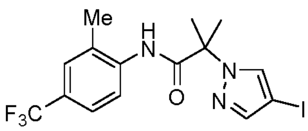

1-chloro-N,N,2-trimethylprop-1-en-1-amine (0.72 g, 5.39 mmol), 16a-1 (1.00 g, 3.576 mmol) and DCM (20 mL) were added to a reaction flask respectively, the mixture was reacted at room temperature for 1 h, then TEA (1.48 mL, 10.65 mmol) and 2-methyl-4-trifluoromethylaniline (0.63 g, 3.597 mmol) were added in sequence, and the mixture was reacted at room temperature for 1 h. The reaction liquid was concentrated under reduced pressure, and the crude product was separated and purified with silica gel chromatography column (ethyl acetate/petroleum ether (v/v) = 1 : 5) to afford 59b (0.18 g, yield: 12%).

LCMS m/z = 438.0 $[M+1]^+$.

Step 2: 2-(4-((1-(2-(2,6-dioxopiperidin-3-yl)-1,3-dioxoisoindolin-5-yl)azetidin-3-yl)ethynyl)-1H-pyrazol-1-yl)-2-methyl-N-(2-methyl-4-(trifluoromethyl)phenyl) propanamide (Compound 59)

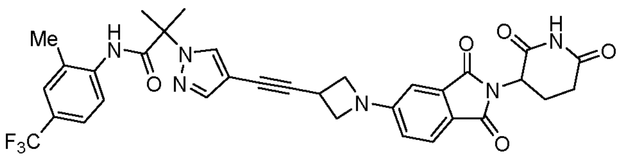

Under nitrogen atmosphere, 59b (180 mg, 0.412 mmol), intermediate 1 (140 mg), $PdCl_2(PPh_3)_2$ (29 mg, 0.041 mmol) and CuI (16 mg, 0.084 mmol) were added to a reaction flask respectively, then DMF (5 mL) and DIPEA (0.20 mL, 1.21 mmol) were added, and the mixture was reacted at 50°C for 2 h. The reaction system was cooled to room temperature and added dropwise into ice water (20 mL), the mixture was filtered, the filter cake was washed with water (10 mL), the filter cake was collected and dissolved in DCM (20 mL), the mixture was dried over anhydrous sodium sulfate, and concentrated under reduced pressure, then the crude product was separated and purified with silica gel column chromatography (ethyl acetate/petroleum ether (v/v) = 1 : 1), and the obtained crude product was separated and purified with silica gel column chromatography (ethyl acetate/petroleum ether (v/v) = 1 : 1) to afford compound 59 (50 mg, yield: 19%).

$^1H$ NMR (400 MHz, $CDCl_3$) δ 8.73 (s, 1H), 8.18 (d, 1H), 8.00 (s, 1H), 7.80 (d, 2H), 7.67 (d, 1H), 7.49 - 7.34 (m, 2H), 6.84 - 6.80 (m, 1H), 6.57 (dd, 1H), 4.98 - 4.88 (m, 1H), 4.42 - 4.31 (m, 2H), 4.12 - 4.01 (m, 2H), 3.87 - 3.76 (m, 1H), 2.95 - 2.65 (m, 3H), 2.20 - 2.08 (m, 4H), 1.95 (s, 6H).

LCMS m/z = 647.2 $[M+1]^+$.

**Example 60: Preparation of compound 60**

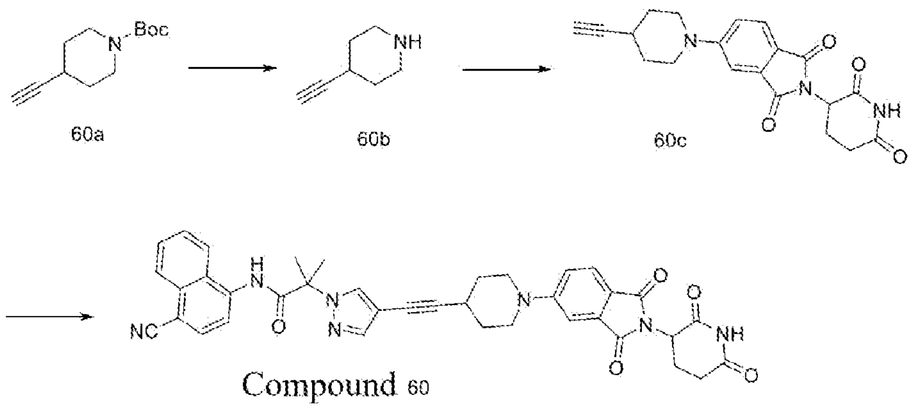

Compound 60

Step 1: 4-ethynylpiperidine (60b) hydrochloride

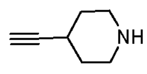

60a (3.0 g, 14.33 mmol) was dissolved in 15 mL of ethyl acetate, then a solution of 4.0 mol/L ethyl acetate hydrogen chloride solution (30.0 mL, 120.0 mmol) was added, and the mixture was stirred at 30°C for 3 h. The reaction system was concentrated under reduced pressure to afford the hydrochloride of crude 60b (1.8 g).

Step 2: 2-(2,6-dioxopiperidin-3-yl)-5-(4-ethynylpiperidin-1-yl)isoindoline-1,3-dione (60c)

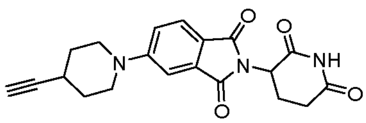

The hydrochloride of crude 60b (1.8 g) was added into a 250 mL single-mouth bottle, dry DMSO (30 mL) was added, then sodium bicarbonate (1.28 g, 15.24 mmol) was added, the mixture was stirred at room temperature for 10 min, then 1C (2.85 g, 10.33 mmol) and DIPEA (3.98 g, 30.79 mmol) were added, and the mixture was reacted at 90°C for 4 h. The reaction liquid was cooled to room temperature and slowly added to water (300 mL), a yellow solid was precipitated and filtered, and the filter cake was washed with 50 mL of water three times, and then the filter cake was dried by blowing at 50°C for 16 h to afford crude 60c (3.16 g).

Step 3: N-(4-cyanonaphthalen-1-yl)-2-(4-((1-(2-(2,6-dioxopiperidin-3-yl)-1,3-dioxoisoindolin-5-yl)piperidin-4-yl)ethynyl)-1H-pyrazol-1-yl)-2-methylpropanamide (Compound 60)

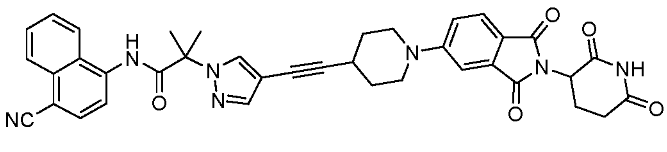

21b (150 mg, 0.349 mmol) was added into a 50 mL single-mouth bottle, dry DMF (10 mL) was added, crude

60c (153 mg) and TEA (106.1 mg, 1.05 mmol) were added, nitrogen replacement was performed three times, $PdCl_2(PPh_3)_2$ (25.6 mg, 0.036 mmol) and CuI (13.3 mg, 0.07 mmol) were added in sequence, nitrogen replacement was performed three times, and the mixture was reacted at 60°C for 2 h. The reaction system was cooled to room temperature, saturated ammonium chloride solution (100 mL) was slowly added, a yellow solid was precipitated, filtration was performed, the filter cake was washed with 20 mL of water three times, the filter cake was dissolved in 100 mL of DCM, the mixture was dried over anhydrous sodium sulfate and concentrated under reduced pressure, the crude product was separated and purified with silica gel chromatography column (petroleum ether/ethyl acetate (v/v) = 47 : 53) to afford compound 60 (120 mg, yield: 51%).

$^1H$ NMR (400 MHz, $CDCl_3$) δ 9.95 (s, 1H), 8.40 - 8.22 (m, 2H), 7.95 - 7.80 (m, 5H), 7.80 - 7.61 (m, 3H), 7.45 - 7.40 (m, 1H), 7.39 - 7.32 (m, 1H), 4.99 - 4.90 (m, 1H), 3.80 - 3.68 (m, 2H), 3.43 - 3.31 (m, 2H), 3.03 - 2.66 (m, 4H), 2.24 - 2.08 (m, 3H), 2.06 - 1.84 (m, 8H).

LCMS m/z = 668.2 $[M+1]^+$.

**Example 61: Preparation of compound 61**

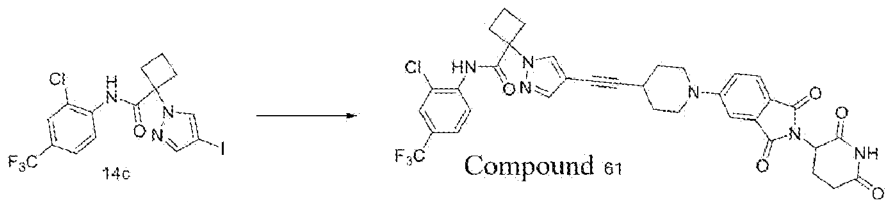

14c

Compound 61

14c (150 mg, 0.32 mmol) was added into a 50 mL single-mouth bottle, dry DMF (10 mL) was added, and crude 60c (144.5 mg) and TEA (118.1 mg, 1.17 mmol) were added. Nitrogen replacement was performed three times, and $PdCl_2(PPh_3)_2$ (24.1 mg, 0.034 mmol) and CuI (12.5 mg, 0.066 mmol) were added in sequence, nitrogen replacement was performed three times, and the mixture was reacted at 60°C for 2 h. The reaction system was cooled to room temperature, 100 mL of saturated ammonium chloride solution was slowly added, a yellow solid was precipitated, filtration was performed, the filter cake was washed with 20 mL of water three times, the filter cake was dissolved in 100 mL of DCM, the mixture was dried over anhydrous sodium sulfate and concentrated under reduced pressure, the crude product was separated and purified with silica gel chromatography column (petroleum ether/ethyl acetate (v/v) = 43 : 57) to afford compound 61 (110 mg, yield: 49%).

$^1H$ NMR (400 MHz, $CDCl_3$) δ 8.72 (s, 1H), 8.55 (d, 1H), 7.95 (s, 1H), 7.82 - 7.65 (m, 3H), 7.63 - 7.45 (m, 2H), 7.43 - 7.35 (m, 1H), 7.30 - 7.20 (m, 1H), 4.99 - 4.90 (m, 1H), 3.80 - 3.65 (m, 2H), 3.42 - 3.31 (m, 2H), 3.15 - 2.65 (m, 8H), 2.35 - 1.77 (m, 7H).

LCMS m/z = 707.2 $[M+1]^+$.

**Example 62: Preparation of compound 62**

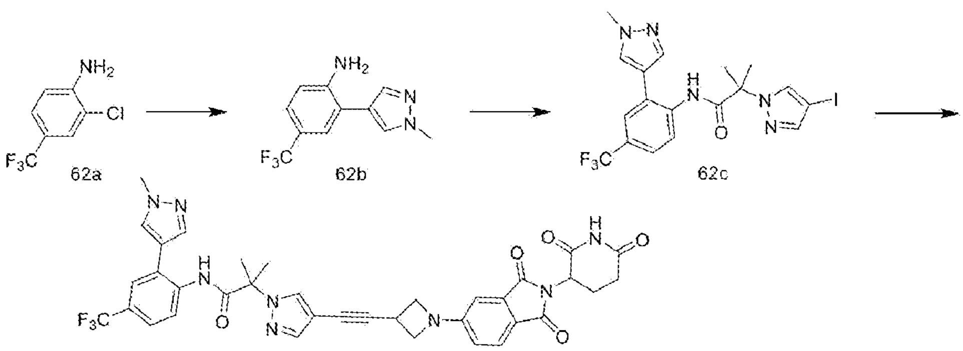

62a 62b 62c

Compound 62

Step 1: 2-(1-methyl-1H-pyrazol-4-yl)-4-(trifluoromethyl)aniline (62b)

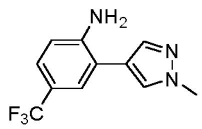

16 mL of 1,4-dioxane and 0.5 mL of water were added to 62a (0.75 g, 3.83 mmol), (1-methyl-1H-pyrazol-4-yl)boronic acid (0.73 g, 5.80 mmol) and potassium carbonate (1.13 g, 8.18 mmol), nitrogen replacement was performed three times, $Pd(dppf)Cl_2$ (0.3 g, 0.413 mmol) was added under nitrogen protection and the mixture was reacted at 100°C for 6 h. The reaction system was cooled to room temperature, 200 mL of water was added, the mixture was extracted with ethyl acetate (50 mL × 2), dried over anhydrous sodium sulfate, and concentrated under reduced pressure. The crude product was separated and purified with silica gel chromatography column (petroleum ether/ethyl acetate (v/v) =1 : 2) to afford 62b (0.25 g, yield: 27%).

LCMS m/z =242.1 $[M+1]^+$.

Step 2: 2-(4-iodo-1H-pyrazol-1-yl)-2-methyl-N-(2-(1-methyl-1H-pyrazol-4-yl)-4-(trifluoromethyl)phenyl)propanamide (62c)

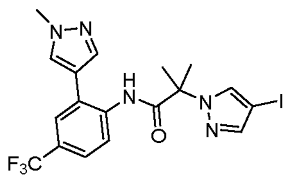

16a-1 (260 mg, 0.93 mmol) was dissolved in 10 mL of DCM, 1-chloro-N,N,2-trimethylpropenylamine (170 mg, 1.27 mmol) was added, the mixture was reacted at room temperature for 2 h, and then TEA (0.25 g, 2.47 mmol) and 62b (200 mg, 0.83 mmol) were added in sequence, and the mixture was reacted at room temperature for 3 h. 80 mL of DCM and 30 mL of aqueous sodium bicarbonate solution were added to the reaction liquid, the organic phase was separated, washed with 50 mL of saturated sodium chloride solution, dried over anhydrous sodium sulfate, and concentrated under reduced pressure. The crude product was separated and purified with silica gel chromatography column (petroleum ether/ethyl acetate (v/v) = 3 : 1) to afford 62c (250 mg, yield: 60%).

Step 3: 2-(4-((1-(2-(2,6-dioxopiperidin-3-yl)-1,3-dioxoisoindolin-5-yl)azetidin-3-yl)ethynyl)-1H-pyrazol-1-yl)-2-methyl-N-(2-(1-methyl-1H-pyrazol-4-yl)-4-(trifluoromethyl)phenyl)propanamide (Compound 62)

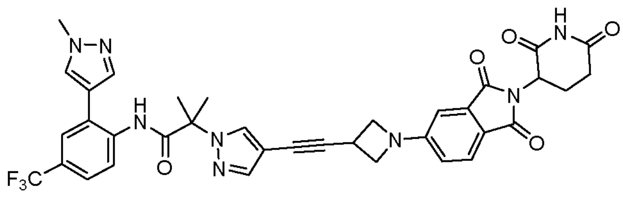

62c (200 mg, 0.40 mmol) was dissolved in 5 mL of DMF, and intermediate 1 (150 mg), TEA (0.12 g, 1.19 mmol), CuI (8 mg, 0.042 mmol) and $PdCl_2(PPh_3)_2$ (28 mg, 0.04 mmol) were added in sequence, nitrogen replacement was performed three times, and the mixture was reacted at 55°C for 1.5 h. The reaction liquid was cooled to room temperature, 40 mL of water was added, the solid was precipitated, suction-filtration was performed, the filter cake was washed with 20 mL of water. The filter cake was dissolved in 100 mL of DCM, the mixture was dried over anhydrous sodium sulfate, and concentrated under reduced pressure. The crude product was separated and purified with silica gel chromatography

column (petroleum ether/ethyl acetate (v/v) = 1 : 1) to afford compound 62 (30 mg, yield: 11%).

$^1$H NMR (400 MHz, $CDCl_3$) δ 8.55 (s, 1H), 8.42 (d, 1H), 8.05 (s, 1H), 7.73 - 7.64 (m, 2H), 7.58 - 7.50 (m, 1H), 7.49 - 7.39 (m, 3H), 7.29 (s, 1H), 6.81 (d, 1H), 6.56 (dd, 1H), 4.98 - 4.89 (m, 1H), 4.42 - 4.30 (m, 2H), 4.10 - 3.97 (m, 5H), 3.88 - 3.73 (m, 1H), 2.99 - 2.64 (m, 3H), 2.20 - 2.06 (m, 1H), 1.87 (s, 6H).

LCMS m/z = 713.0 [M+1] $^+$.

**Example 63: Preparation of compound 63**

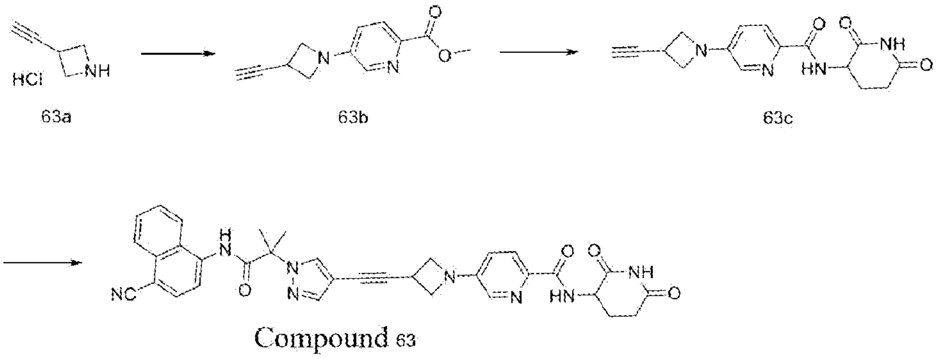


Step 1: methyl 5-(3-ethynylazetidin-1-yl)picolinate (63b)

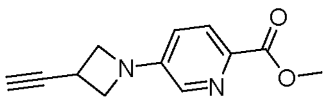

63a (1.0 g, 8.50 mmol) was added to 10 mL of DMSO, methyl 5-fluoropyridine-2-carboxylate (1.45 g, 9.35 mmol) and TEA (2.58 g, 25.5 mmol) were added in sequence, and the mixture was reacted at 90°C for 4 h. The reaction system was cooled to room temperature, 20 mL of water was added, the mixture was extracted with ethyl acetate (40 mL × 2), the organic phases were combined, dried over anhydrous sodium sulfate, and concentrated under reduced pressure, and the crude product was separated and purified with silica gel chromatography column (DCM/MeOH (v/v) = 10 : 1) to afford 63b (1.05 g, yield: 57%).

LCMS m/z = 217.1 [M+1]$^+$.

Step 2: N-(2,6-dioxopiperidin-3-yl)-5-(3-ethynylazetidin-1-yl)picolinamide (63c)

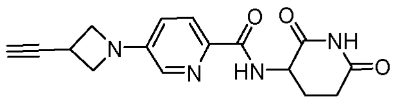

63b (0.58 g, 2.68 mmol) was added to 2.5 mL of a mixed solvent of tetrahydrofuran/water (v/v) = 4 : 1, anhydrous lithium hydroxide (0.13 g, 5.43 mmol) was added, and the mixture was stirred at 30°C for 3 h. The reaction liquid was adjusted to pH 6 with 2 mol/L hydrochloric acid and concentrated under reduced pressure, and the residue was freeze-dried to afford a crude product (0.55 g). The crude product (0.55 g) was added to 8 mL of DMF, 3-aminopiperidine-2,6-dione hydrochloride (0.54 g, 3.28 mmol), TEA (0.82 g, 8.10 mmol) and HATU (1.55 g, 4.08 mmol) were added in sequence, and the mixture was reacted at room temperature for 19 h. 20 mL of water was added to the reaction system, the mixture was extracted with ethyl acetate (30 mL × 2), the organic phases were combined and dried over anhydrous sodium sulfate, and the crude product was separated and purified with silica gel chromatography column (DCM/MeOH (v/v) = 10 : 1) to afford 63c (0.32 g, yield: 38%).

LCMS m/z = 313.1 $[M+1]^+$.

Step 3: 5-(3-((1-(1-((4-cyanonaphthalen-1-yl)amino)-2-methyl-1-oxopropan-2-yl)-1H-pyrazol-4-yl)ethynyl)azetidin-1-yl)-N-(2,6-dioxopiperidin-3-yl)picolinamide (Compound 63)

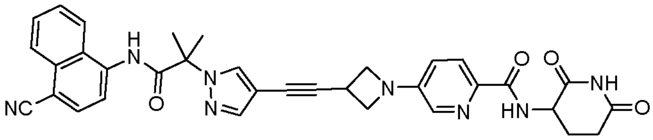

63c (0.15 g, 0.48 mmol) was added to 8 mL of DCM, 21b (0.54 g, 1.26 mmol) and DIPEA (0.19 g, 1.47 mmol) were added in sequence, nitrogen replacement was performed three times, the mixture was stirred at room temperature for 5 min, CuI (9.3 mg, 0.049 mmol) and $PdCl_2(PPh_3)_2$ (34 mg, 0.048 mmol) were added, and the mixture was stirred at 30°C for 2 h. 20 mL of water was added to the reaction system, the mixture was extracted with 30 mL of DCM twice, the organic phases were combined, dried over anhydrous sodium sulfate, and concentrated under reduced pressure, and the crude product was separated and purified with silica gel chromatography column (DCM/MeOH (v/v) = 10 : 1) to afford compound 63 (98 mg, yield: 33%).

$^1H$ NMR (400 MHz, $CDCl_3$) δ 9.91 (s, 1H), 8.43 (d, 1H), 8.34 (d, 1H), 8.30 - 8.20 (m, 2H), 8.01 (d, 1H), 7.95 - 7.62 (m, 7H), 6.77 (dd, 1H), 4.84 - 4.73 (m, 1H), 4.40 - 4.28 (m, 2H), 4.08 - 3.98 (m, 2H), 3.86 - 3.76 (m, 1H), 2.90 - 2.70 (m, 2H), 2.66 - 2.53 (m, 1H), 2.08 - 1.93 (m, 7H).

LCMS m/z = 615.3 $[M+1]^+$.

**Example 64: Preparation of compound 64**

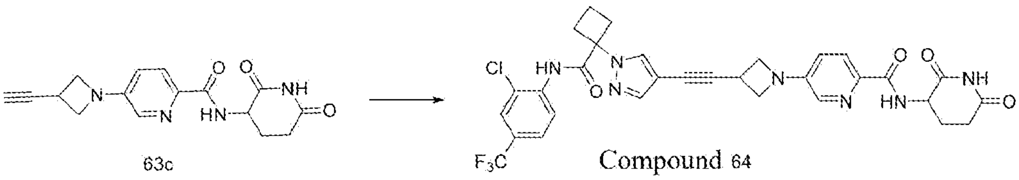

63c

Compound 64

63c (0.2 g, 0.64 mmol) was added to 8 mL of DCM, 14c (0.33 g, 0.70 mmol) and DIPEA (0.25 g, 1.93 mmol) were added in sequence, nitrogen replacement was performed three times, the mixture was stirred at room temperature for 5 min, CuI (12 mg, 0.063 mmol) and $PdCl_2(PPh_3)_2$ (45 mg, 0.064 mmol) were added, and the mixture was stirred at 30°C for 2 h. 20 mL of water was added to the reaction system, the mixture was extracted with 30 mL of DCM twice, the organic phases were combined, dried over anhydrous sodium sulfate, and concentrated under reduced pressure, and the crude product was separated and purified with silica gel chromatography column (DCM/MeOH (v/v) = 10 : 1) to afford compound 64 (0.16 g, yield: 38%).

$^1H$ NMR (400 MHz, $CDCl_3$) δ 8.69 (s, 1H), 8.59 - 8.47 (m, 2H), 8.42 (d, 1H), 8.01 (d, 1H), 7.82 - 7.74 (m, 2H), 7.71 (s, 1H), 7.61 - 7.47 (m, 2H), 6.77 (dd, 1H), 4.86 - 4.74 (m, 1H), 4.40 - 4.26 (m, 2H), 4.09 - 3.97 (m, 2H), 3.87 - 3.75 (m, 1H), 3.14 - 3.00 (m, 2H), 2.90 - 2.70 (m, 4H), 2.64 - 2.50 (m, 1H), 2.32 - 1.94 (m, 3H).

LCMS m/z = 654.1 $[M+1]^+$.

**Example 65: Preparation of compound 65**

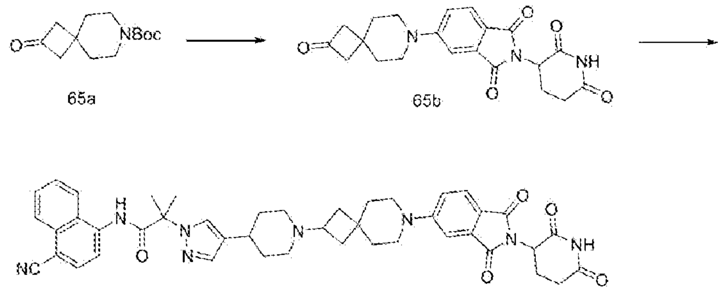
65a
65b

Compound 65

Step 1: 2-(2,6-dioxopiperidin-3-yl)-5-(2-oxo-7-azaspiro[3.5]nonan-7-yl)isoindoline-1,3-dione (65a)

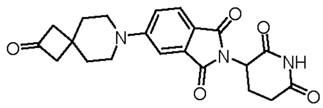

65a (300 mg, 1.25 mmol) was dissolved in methanol (4 mL), a solution of 4 mol/L 1,4-dioxane hydrogen chloride solution (5 mL) was added, and the mixture was reacted at room temperature for 1 h. The reaction liquid was concentrated under reduced pressure, the residue was dissolved in DMF (10 mL), DIPEA (645 mg, 4.99 mmol) and 1C (522 mg, 1.89 mmol) were added, and the mixture was reacted at 80°C for 4 h. The reaction liquid was cooled to room temperature, 20 mL of water and 20 mL of ethyl acetate were added, the organic phase was separated, the organic phase was concentrated under reduced pressure, and the residue was separated and purified with silica gel column chromatography (DCM/MeOH (v/v) = 100 : 1-20 : 1) to afford 65a (300 mg, yield: 61%).

LCMS m/z = 396.1 $[M+1]^+$.

Step 2: N-(4-cyanonaphthalen-1-yl)-2-(4-(1-(7-(2-(2,6-dioxopiperidin-3-yl)-1,3-dioxoisoindolin-5-yl)-7-azaspiro[3.5]nonan-2-yl)piperidin-4-yl)-1H-pyrazol-1-yl)-2-methylpropanamide (Compound 65)

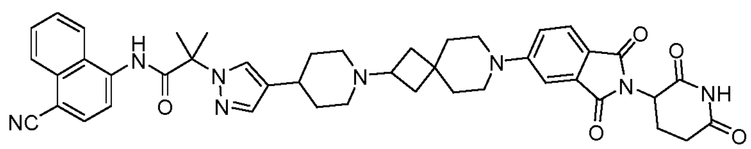

55a (200 mg, 0.41 mmol) was dissolved in methanol (4 mL), a solution of 4 mol/L 1,4-dioxane hydrogen chloride solution (5 mL) was added, and the mixture was reacted at room temperature for 1 h. The reaction liquid was concentrated under reduced pressure. 10 mL of DCM was added to the residue. 3 mL of TEA was added. The mixture was stirred at room temperature for 1 min, then concentrated under reduced pressure. The residue was dissolved in DMAc (6 mL). 65a (245 mg, 0.62 mmol) was added. The mixture was stirred at room temperature for 30 min, then sodium triacetoxyborohydride (131 mg, 0.62 mmol) was added, and the mixture was reacted at room temperature for 16 h. 20 mL of saturated aqueous sodium bicarbonate solution and 20 mL of ethyl acetate were added to the reaction liquid, the organic phase was separated, the organic phase was concentrated under reduced pressure, and the residue was separated and purified with silica gel chromatography column (DCM/MeOH (v/v) = 50 : 1-20 : 1) to afford compound 65 (180 mg, yield: 57%).

$^1$H NMR (400 MHz, DMSO-$d_6$) δ 11.07 (s, 1H), 9.68 (s, 1H), 8.24 - 8.06 (m, 2H), 8.00 - 7.56 (m, 7H), 7.40 - 7.16 (m, 2H), 5.14 - 5.01 (m, 1H), 3.58 - 3.20 (m, 6H), 3.05 - 2.79 (m, 2H), 2.69 - 2.50 (m, 2H), 2.25 - 1.80 (m, 12H), 1.75 - 1.43 (m, 6H), 1.37 - 1.00 (m, 4H).

LCMS m/z = 767.4 $[M+1]^+$.

**Example 66: Preparation of compound 66**

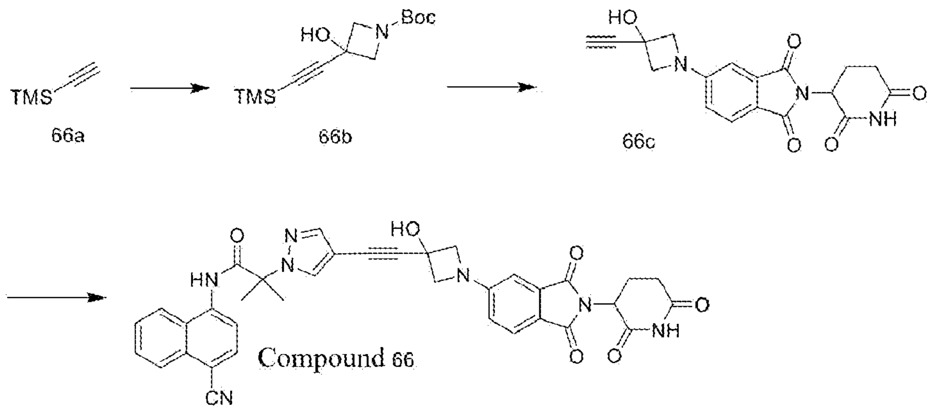

66a

66b

66c

Compound 66

Step 1: tert-butyl 3-hydroxy-3-(2-(trimethylsilyl)ethynyl)azetidine-1-carboxylate (66b)

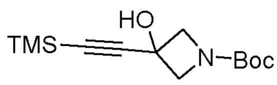

66a (4.0 g, 40.72 mmol) was dissolved in 50 mL of dry THF, nitrogen replacement was performed three times, the mixture was cooled to -78°C, and a solution of 1.6 mol/L n-butyllithium in n-hexane (28 mL, 44.8 mmol) was slowly added dropwise. The mixture was stirred at -78°C for 30 min, and then a solution of tert-butyl 3-oxoazetidine-1-carboxylate (7.67 g, 44.8 mmol) in THF (50 mL) was slowly added dropwise. After the dropwise addition was completed, the reaction mixture was slowly warmed to -20°C and stirred for 2 h, 20 mL of saturated aqueous ammonium chloride solution was slowly added dropwise at - 20°C to quench the reaction, and the mixture was warmed to room temperature. 100 mL of ethyl acetate was added for extraction, and the organic phase was washed with 30 mL of saturated aqueous sodium chloride solution, dried over anhydrous sodium sulfate, and concentrated under reduced pressure. The resulting crude product was separated and purified with silica gel chromatographic column (ethyl acetate/petroleum ether (v/v) = 1 : 9) to afford 66b (8.6 g, yield: 78%).

Step 2: 2-(2,6-dioxopiperidin-3-yl)-5-(3-ethynyl-3-hydroxyazetidin-1-yl)-2,3-dihydro-1H-isoindole-1,3-dione (66c)

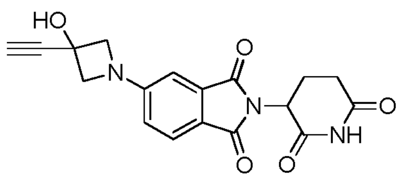

66b (1.0 g, 3.71 mmol) was dissolved in DCM (10 mL), 3 mL of trifluoroacetic acid was added, and the mixture was reacted at room temperature for 40 min. The reaction liquid was concentrated under reduced pressure to afford a crude product (1.05 g). The above crude product (1.05 g) was dissolved in 10 mL of dry dimethyl sulfoxide, 1C (1.03 g, 3.73 mmol) was added, the mixture was heated to 100°C under nitrogen protection, and DIPEA (1.44 g, 11.14 mmol) was slowly added dropwise at 100°C, after the dropwise addition was completed, the reaction was continued at 100°C for 3 h. The reaction liquid was cooled to room temperature and slowly poured into 100 mL of water, the mixture was stirred for 10 min, then filtered by suction, the filter cake was washed with 20 mL of water, the filter cake was dissolved with 30 mL of ethyl acetate, and the mixture was washed with saturated aqueous sodium chloride solution (10 mL × 2), dried over anhydrous sodium sulfate, and concentrated under reduced pressure, and the crude product was separated and purified with silica gel chromatography column (ethyl acetate/petroleum ether (v/v) = 3 : 7-4 : 1) to obtain 66c (0.5

g, yield: 38%).

LCMS m/z = 354.1 $[M+1]^+$.

Step 3: N-(4-cyanonaphthalen-1-yl)-2-(4-(2-(1-(2-(2,6-dioxopiperidin-3-yl)-1,3-dioxo-2,3-dihydro-1H-isoindol-5-yl)-3-hydroxyazetidin-3-yl)ethynyl)-1H-pyrazol-1-yl)-2-methylpropanamide (Compound 66)

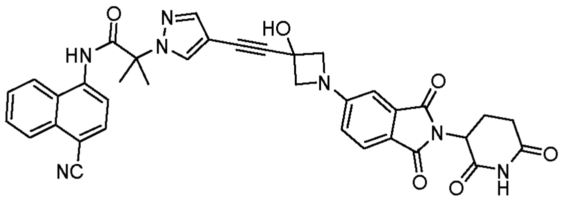

66c (0.18 g, 0.51 mmol) and 21b (0.22 g, 0.51 mmol) were dissolved in 5 mL of dry DMF, TEA (0.15 g, 1.48 mmol) was added, nitrogen replacement was performed three times, CuI (0.015 g, 0.079 mmol) and $PdCl_2(PPh_3)_2$ (0.054 g, 0.077 mmol) were added, and the mixture was reacted at 50°C for 2 h. The reaction liquid was cooled to room temperature, 10 mL of water was added, the mixture was stirred for 5 min and filtered by suction, the filter cake was washed with 5 mL of water, the filter cake was dissolved with 100 mL of DCM, the mixture was dried over anhydrous sodium sulfate, and concentrated under reduced pressure, and the crude product obtained was separated and purified with silica gel chromatography column (ethyl acetate/petroleum ether (v/v) = 3 : 2) to afford compound 66 (0.05 g, yield: 15%).

$^1H$ NMR (400 MHz, $CDCl_3$) δ 9.89 (s, 1H), 8.37 - 8.30 (m, 1H), 8.29 - 8.22 (m, 1H), 8.03 - 7.77 (m, 5H), 7.75 - 7.60 (m, 3H), 6.85 - 6.78 (m, 1H), 6.57 (dd, 1H), 4.97 - 4.88 (m, 1H), 4.44 - 4.34 (m, 2H), 4.25 - 4.15 (m, 2H), 2.96 - 2.64 (m, 3H), 2.17 - 2.07 (m, 1H), 2.02 (s, 6H).

LCMS m/z = 654.2 $[M-1]^-$.

**Example 67: Preparation of compound 67**

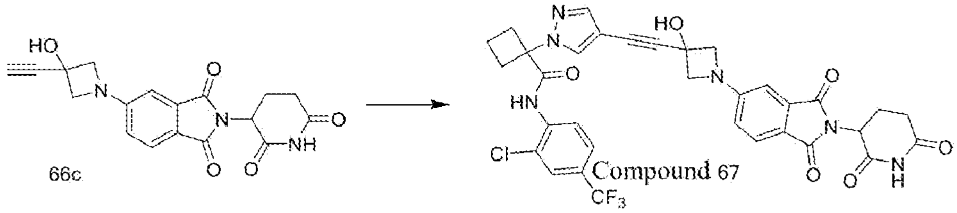


66c (0.19 g, 0.538 mmol) and 14c (0.26 g, 0.55 mmol) were dissolved in 5 mL of dry DMF, TEA (0.17 g, 1.68 mmol) was added, nitrogen replacement was performed three times, CuI (0.016 g, 0.084 mmol) and $PdCl_2(PPh_3)_2$ (0.058 g, 0.083 mmol) were added, and the mixture was reacted at 50°C for 2 h. The reaction liquid was cooled to room temperature, 10 mL of water was added, the mixture was stirred for 5 min and filtered by suction, the filter cake was washed with 5 mL of water, the filter cake was dissolved with 100 mL of DCM, the mixture was dried over anhydrous sodium sulfate, and concentrated under reduced pressure, and the crude product obtained was separated and purified with silica gel chromatography column (ethyl acetate/petroleum ether (v/v) = 3 : 2) to afford compound 67 (0.02 g, yield: 5%).

$^1H$ NMR (400 MHz, $CDCl_3$) δ 8.69 (s, 1H), 8.54 (d, 1H), 8.16 (s, 1H), 7.86 - 7.73 (m, 2H), 7.70 - 7.46 (m, 3H), 6.81 (d, 1H), 6.56 (dd, 1H), 4.98 - 4.88 (m, 1H), 4.44 - 4.32 (m, 2H), 4.25 - 4.14 (m, 2H), 3.15 - 2.63 (m, 7H), 2.31 - 1.97 (m, 3H).

LCMS m/z = 695.2 $[M+1]^+$.

**Example 68: Preparation of compound 68**

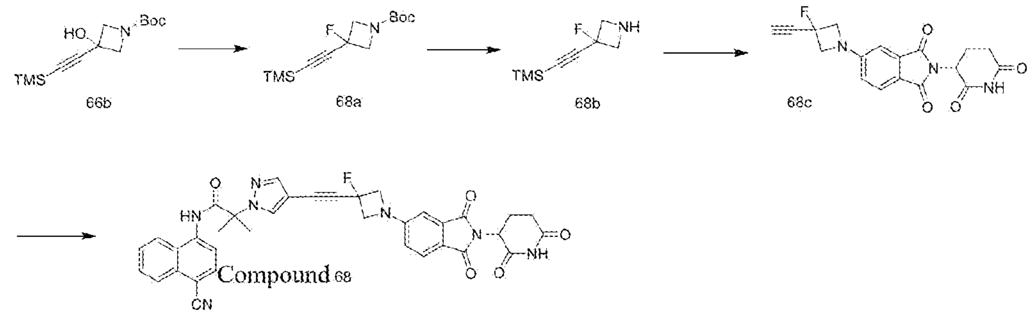


Step 1: tert-butyl 3-fluoro-3-(2-(trimethylsilyl)ethynyl)azetidine-1-carboxylate (68a)

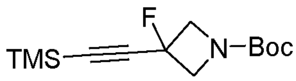

66b (3.0 g, 11.14 mmol) was dissolved in 30 mL of dry DCM, the mixture was cooled to -78°C under nitrogen protection, DAST (3.59 g, 22.27 mmol) was slowly added dropwise, and after the dropwise addition was completed, the mixture was warmed to room temperature for 2 h. The reaction liquid was cooled to 0°C, saturated aqueous sodium bicarbonate solution was slowly added dropwise to adjust the pH to 9, 20 mL of DCM was added for extraction, the organic layer was washed with 20 mL of saturated aqueous sodium chloride solution, dried over anhydrous sodium sulfate, and concentrated under reduced pressure. The crude product was separated and purified with silica gel chromatography column (ethyl acetate/petroleum ether (v/v) = 0 : 1-3 : 17) to afford 68a (2.25 g, yield: 74%).

$^1$H NMR (400 MHz, $CDCl_3$) δ 4.30 - 4.12 (m, 4H), 1.45 (s, 9H), 0.21 (s, 9H).

Step 2: 3-fluoro-3-(2-(trimethylsilyl)ethynyl)azetidine (68b) trifluoroacetate

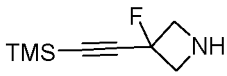

68a (0.5 g, 1.84 mmol) was dissolved in 5 mL of DCM, 3 mL of trifluoroacetic acid was added, and the mixture was reacted at room temperature for 40 min. The reaction liquid was concentrated under reduced pressure to afford the trifluoroacetate of crude 68b (0.525 g).

LCMS m/z = 172.1 $[M+1]^+$.

Step 3: 2-(2,6-dioxopiperidin-3-yl)-5-(3-ethynyl-3-fluoroazetidin-1-yl)-2,3-dihydro-1H-isoindole-1,3-dione (68c)

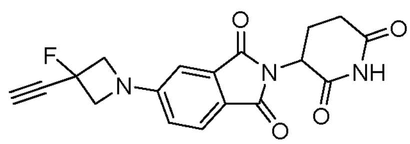

The trifluoroacetate of crude 68b (0.525 g) was dissolved in 10 mL of dry DMSO, 1C (0.52 g, 1.88 mmol) was added, and after the addition was completed, the mixture was heated to 100°C under nitrogen protection, DIPEA (0.73 g, 5.65 mmol) was slowly added dropwise at 100°C, and after the addition was completed, the reaction was continued at 100°C for 3 h. The reaction liquid was cooled to room temperature and slowly poured into 100 mL of water, the mixture was stirred for 10 min and filtered, the filter cake was washed with 20 mL of water, the filter cake was collected, the filter cake was dissolved with 30 mL of ethyl acetate, and the mixture was washed with saturated aqueous sodium chloride solution (10 mL × 2), dried over anhydrous sodium sulfate, and concentrated under reduced pressure, and the crude

product was separated and purified with silica gel chromatography column (ethyl acetate/petroleum ether (v/v) = 3 : 7-3 : 2) to obtain 68c (0.23 g, yield: 34%).

LCMS m/z = 356.1 $[M+1]^+$.

Step 4: N-(4-cyanonaphthalen-1-yl)-2-(4-(2-(1-(2-(2,6-dioxopiperidin-3-yl)-1,3-dioxo-2,3-dihydro-1H-isoindol-5-yl)-3-fluoroazetidin-3-yl)ethynyl)-1H-pyrazol-1-yl)-2-methylpropanamide (Compound 68)

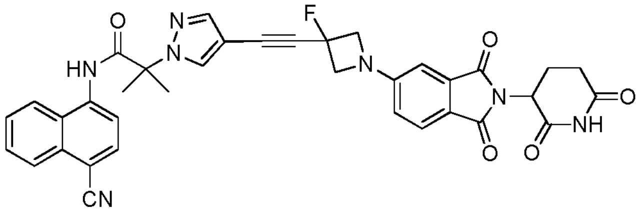

68c (0.21 g, 0.59 mmol) and 21b (0.26 g, 0.60 mmol) were dissolved in 5 mL of dry DMF, TEA (0.18 g, 1.78 mmol) was added, nitrogen replacement was performed three times, CuI (0.017 g, 0.089 mmol) and $PdCl_2(PPh_3)_2$ (0.063 g, 0.09 mmol) were added under nitrogen protection, and the mixture was reacted at 50°C for 2 h. The reaction liquid was cooled to room temperature, 10 mL of water was added, the mixture was stirred for 5 min and filtered by suction, the filter cake was washed with 5 mL of water, the filter cake was collected, the filter cake was dissolved with 100 mL of DCM, the mixture was dried over anhydrous sodium sulfate, concentrated under reduced pressure, and separated and purified with silica gel chromatography column (ethyl acetate/petroleum ether (v/v) = 3 : 2) to afford compound 68 (0.02 g, yield: 5%).

$^1$H NMR (400 MHz, $CDCl_3$) δ 9.87 (s, 1H), 8.34 (d, 1H), 8.29 - 8.23 (m, 1H), 8.03 - 7.94 (m, 3H), 7.90 (d, 1H), 7.85 - 7.78 (m, 1H), 7.75 - 7.63 (m, 3H), 6.84 (d, 1H), 6.60 (dd, 1H), 4.98 - 4.89 (m, 1H), 4.45 (s, 2H), 4.41 (s, 2H), 2.95 - 2.65 (m, 3H), 2.18 - 2.08 (m, 1H), 2.03 (s, 6H).

LCMS m/z = 658.2 $[M+1]^+$.

**Example 69: Preparation of compound 69**

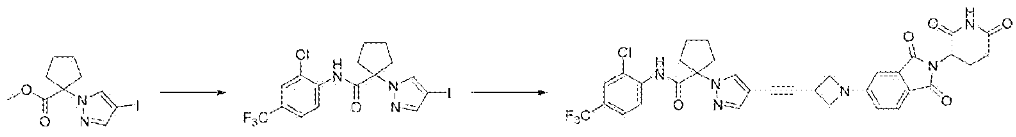

48b 69a Compound 69

Step 1: N-(2-chloro-4-(trifluoromethyl)phenyl)-1-(4-iodo-1H-pyrazol-1-yl)cyclopentane-1-carboxamide (69a)

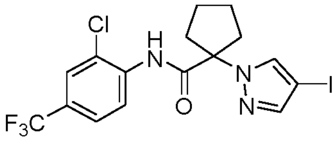

48b (0.95 g, 2.97 mmol) was dissolved in a mixed solvent of THF (15 mL) and methanol (5 mL), 5 mL of an aqueous solution of lithium hydroxide monohydrate (0.25 g, 5.96 mmol) was added, and the mixture was stirred at 20°C for 16 h. The reaction system was adjusted to pH 4 with 3 mol/L aqueous hydrochloric acid solution, extracted with 200 mL of ethyl acetate, the organic phase was washed with 50 mL of water and 50 mL of saturated sodium chloride solution, dried over anhydrous sodium sulfate, and concentrated under reduced pressure to afford a crude product (0.9 g). The above crude product (0.45 g) and 11a (0.29 g, 1.48 mmol) were dissolved in pyridine (10 mL), a solution of T3P in 50% ethyl acetate (1.87 g) was added, and the mixture was reacted at 35°C for 16 h. The reaction liquid was cooled to room

temperature and concentrated under reduced pressure, 10 mL of saturated aqueous sodium bicarbonate solution was added, 200 mL of ethyl acetate was added, the liquid separation was conducted, the organic phase was washed with water (50 mL × 3), and then washed with 50 mL of saturated sodium chloride solution, dried over anhydrous sodium sulfate, and concentrated under reduced pressure. The crude product was separated and purified with silica gel chromatography column (petroleum ether/ethyl acetate (v/v) = 1 : 1) to afford 69a (0.23 g, yield: 32%).

LCMS m/z = 484.0 $[M+1]^+$.

Step 2: N-(2-chloro-4-(trifluoromethyl)phenyl)-1-(4-((1-(2-(2,6-dioxopiperidin-3-yl)-1,3-dioxoisoindolin-5-yl)azetidin-3-yl)ethynyl)-1H-pyrazol-1-yl)cyclopentane-1-carboxamide (Compound 69)

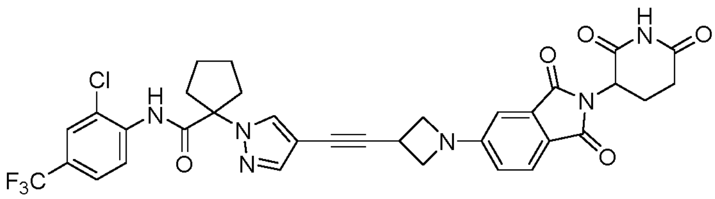

69a (0.15 g, 0.31 mmol) and intermediate 1 (0.12 g) were dissolved in DMF (10 mL), and $PdCl_2(PPh_3)_2$ (0.02 g, 0.028 mmol), CuI (0.01 g, 0.053mmol) and TEA (0.09 g, 0.89 mmol) were added in sequence, and the mixture was reacted at 55°C for 1 h under nitrogen protection. The reaction liquid was cooled to room temperature, 200 mL of ethyl acetate was added, the organic phase was washed with water (50 mL × 3), then washed with 50 mL of saturated sodium chloride solution, dried over anhydrous sodium sulfate, and concentrated under reduced pressure, the crude product was separated and purified with silica gel chromatography column (petroleum ether/ethyl acetate (v/v) = 1 : 2) to afford compound 69 (0.075 g, yield: 35%).

$^1H$ NMR (400 MHz, $CDCl_3$) δ 8.82 (s, 1H), 8.50 (d, 1H), 7.92 (s, 1H), 7.81 - 7.73 (m, 2H), 7.67 (d, 1H), 7.61 - 7.56 (m, 1H), 7.53 - 7.45 (m, 1H), 6.81 (d, 1H), 6.55 (dd, 1H), 4.99 - 4.88 (m, 1H), 4.40 - 4.30 (m, 2H), 4.13 - 4.00 (m, 2H), 3.87 - 3.75 (m, 1H), 2.96 - 2.56 (m, 7H), 2.18 - 2.07 (m, 1H), 2.04 - 1.90 (m, 2H), 1.81 - 1.65 (m, 2H).

LCMS m/z = 693.1 $[M+1]^+$.

**Example 70: Preparation of compound 70**

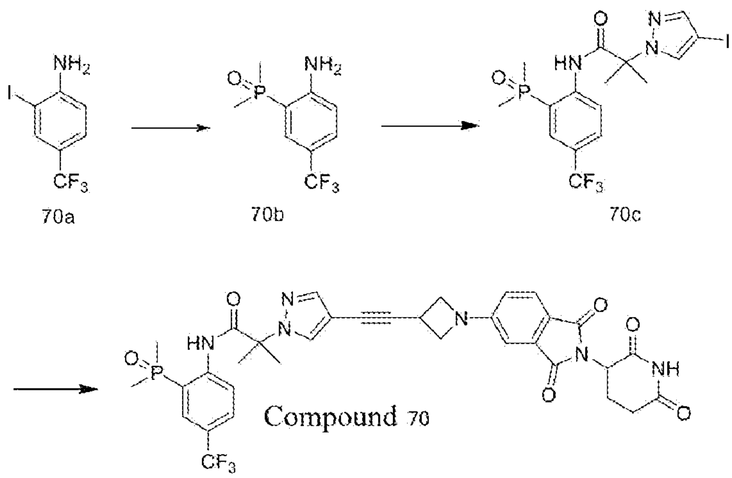

70a 70b 70c

Compound 70

Step 1: (2-amino-5-(trifluoromethyl)phenyl)dimethylphosphine oxide (70b)

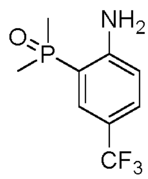

70a (500 mg, 1.74 mmol) was dissolved in 10 mL of 1,4-dioxane, and dimethylphosphine oxide (163 mg, 2.09 mmol), $Pd_2(dba)_3$ (40 mg, 0.0437 mmol), Xantphos (50.4 mg, 0.087 mmol) and TEA (212 mg, 2.1 mmol) were added in sequence, nitrogen replacement was performed three times, and the mixture was reacted at 30°C for 6 h. The reaction liquid was cooled to room temperature, 50 mL of saturated aqueous sodium chloride solution was added, the mixture was extracted with 50 mL of ethyl acetate, dried over anhydrous sodium sulfate, and concentrated under reduced pressure. The crude product was separated and purified with silica gel chromatography column (petroleum ether/ethyl acetate (v/v) = 1 : 2) to afford 70b (400 mg, yield: 97%).

Step 2: N-(2-(dimethylphosphoryl)-4-(trifluoromethyl)phenyl)-2-(4-iodo-1H-pyrazol-1-yl)-2-methylpropanamide (70c)

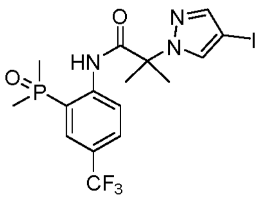

16a-1 (708 mg, 2.53 mmol) was dissolved in 8 mL of DCM, 1-chloro-N,N,2-trimethylpropenylamine (421 mg, 3.15 mmol) was added, the mixture was reacted at room temperature for 1 h, and then 70b (500 mg, 2.1 mmol) and TEA (318 mg, 3.14 mmol) were added, and the mixture was reacted at room temperature for 2 h. 50 mL of DCM and 50 mL of saturated aqueous sodium bicarbonate solution were added to the reaction liquid, the organic phase was separated, washed with 50 mL of saturated sodium chloride solution, dried over anhydrous sodium sulfate, and concentrated under reduced pressure. The crude product was separated and purified with silica gel chromatography column (petroleum ether/ethyl acetate (v/v) = 1 : 1) to afford 70c (600 mg, yield: 57%).

Step 3: N-(2-(dimethylphosphoryl)-4-(trifluoromethyl)phenyl)-2-(4-((1-(2-(2,6-dioxopiperidin-3 -yl)-1,3 -dioxoisoindolin-5 -yl)azetidin-3 -yl)ethynyl)- 1H-pyrazol-1-yl)-2-methylpropanamide (Compound 70)

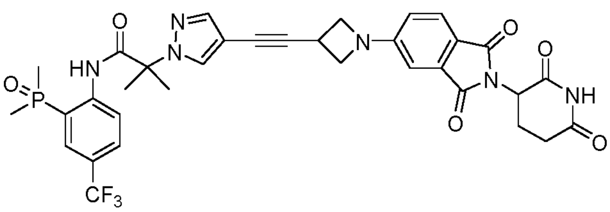

70c (0.8 g, 1.60 mmol) was dissolved in 6 mL of DMF, and intermediate 1 (0.62 g), TEA (1.11 g, 10.97 mmol), CuI (70 mg, 0.37 mmol) and $PdCl_2(PPh_3)_2$ (130 mg, 0.185 mmol) were added in sequence, and the mixture was reacted at 60°C for 3 h. The reaction liquid was cooled to room temperature, 50 mL of water was added, the solid was precipitated, suction-filtration was performed, the filter cake was washed with 10 mL of water. The filter cake was dissolved in 100 mL of DCM, the mixture was dried over anhydrous sodium sulfate, and concentrated under reduced pressure. The crude product was separated and purified with silica gel chromatography column (DCM/MeOH (v/v) = 100 : 1-10 : 1) to afford compound 70 (0.3 g, yield: 26%).

$^1H$ NMR (400 MHz, $CDCl_3$) $\delta$ 11.53 (s, 1H), 8.75 (dd, 1H), 8.04 (s, 1H), 7.80 (s, 1H), 7.76 - 7.60 (m, 3H), 7.43 - 7.34 (m, 1H), 6.85 - 6.75 (m, 1H), 6.55 (dd, 1H), 4.98 - 4.88 (m, 1H), 4.40 - 4.29 (m, 2H), 4.12 - 4.00 (m, 2H), 3.87 - 3.75 (m, 1H), 3.00 - 2.64 (m, 3H), 2.20 - 2.05 (m, 1H), 1.92 (s, 6H), 1.79 (s, 3H), 1.76 (s, 3H).

LCMS m/z = 709.2 [M+1] +.

**Example 71: Preparation of compound 71**

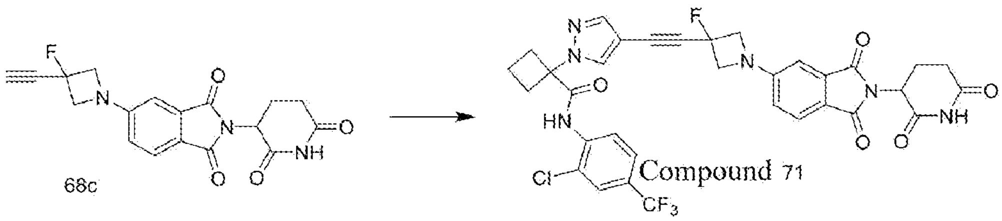

68c

Compound 71

68c (0.38 g, 1.07 mmol) and 14c (0.5 g, 1.06 mmol) were dissolved in 10 mL of dry DMF, TEA (0.32 g, 3.16 mmol) was added, nitrogen replacement was performed three times, CuI (0.03 g, 0.16 mmol) and $PdCl_2(PPh_3)_2$ (0.11 g, 0.16 mmol) were added under nitrogen protection, and the mixture was reacted at 50°C for 2 h. The reaction liquid was cooled to room temperature, 10 mL of water was added, the mixture was stirred for 5 min and filtered by suction, the filter cake was washed with 5 mL of water, the filter cake was collected, the filter cake was dissolved with 100 mL of DCM, the mixture was dried over anhydrous sodium sulfate, concentrated under reduced pressure, and the crude product was separated and purified with silica gel chromatography column (ethyl acetate/petroleum ether (v/v) = 3 : 2) to afford compound 71 (0.18 g, yield: 24%).

$^1H$ NMR (400 MHz, $CDCl_3$) δ 8.70 (s, 1H), 8.54 (d, 1H), 8.02 (s, 1H), 7.86 (s, 1H), 7.80 (s, 1H), 7.70 (d, 1H), 7.61 - 7.57 (m, 1H), 7.54 - 7.48 (m, 1H), 6.85 (d, 1H), 6.60 (dd, 1H), 4.98 - 4.89 (m, 1H), 4.45 (s, 2H), 4.40 (s, 2H), 3.15 - 3.02 (m, 2H), 2.96 - 2.66 (m, 5H), 2.32 - 2.03 (m, 3H).

LCMS m/z = 696.9 [M+1]+.

**Example 72: Preparation of compound 72**

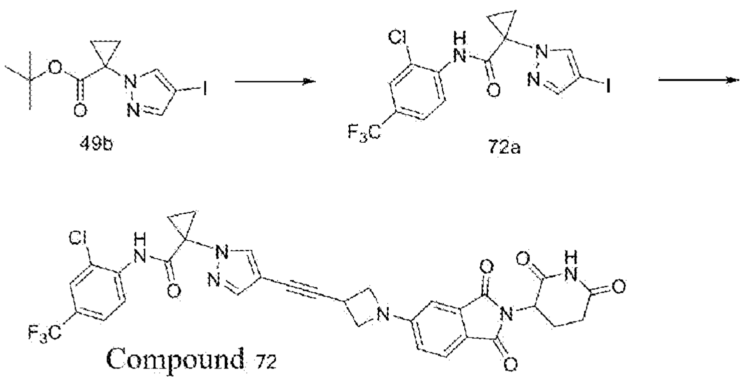

49b

72a

Compound 72

Step 1: N-(2-chloro-4-(trifluoromethyl)phenyl)-1-(4-iodo-1H-pyrazol-1-yl)cyclopropane-1-carboxamide (72a)

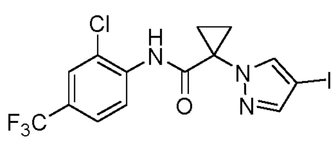

49b (1.00 g, 2.99 mmol) was dissolved in a solution of 4 mol/L 1,4-dioxane hydrogen chloride solution (15 mL) and the mixture was reacted at 60°C for 1 h. The reaction liquid was cooled to room temperature and concentrated

under reduced pressure to afford a crude product (0.83 g). The above crude product (0.45 g) and 11a (0.32 g, 1.636 mmol) were dissolved in pyridine (10 mL), a solution of T3P in 50% ethyl acetate (2.06 g) was added, and the mixture was reacted at 35°C for 16 h. The reaction liquid was cooled to room temperature and concentrated under reduced pressure, 10 mL of saturated aqueous sodium bicarbonate solution was added, 200 mL of ethyl acetate was added, the liquid separation was conducted, the organic phase was washed with water (50 mL × 3), and then washed with 50 mL of saturated sodium chloride solution, dried over anhydrous sodium sulfate, and concentrated under reduced pressure. The crude product was separated and purified with silica gel chromatography column (petroleum ether/ethyl acetate (v/v) = 3 : 1) to afford 72a (0.29 g, yield: 39%).

LCMS m/z =456.0 $[M+1]^+$.

Step 2: N-(2-chloro-4-(trifluoromethyl)phenyl)-1-(4-((1-(2-(2,6-dioxopiperidin-3-yl)-1,3-dioxoisoindolin-5-yl)azetidin-3-yl)ethynyl)-1H-pyrazol-1-yl)cyclopropane-1-carboxamide (Compound 72)

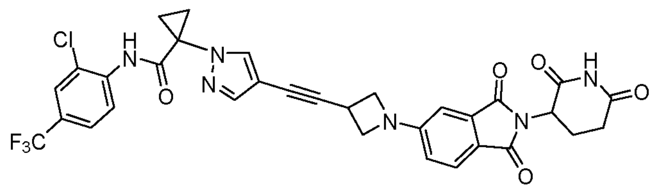

Under nitrogen atmosphere, 72a (270 mg, 0.593 mmol), intermediate 1 (200 mg), $PdCl_2(PPh_3)_2$ (41 mg, 0.058 mmol) and CuI (22 mg, 0.12 mmol) were added to the reaction flask in sequence, 5 mL of DMF and DIPEA (0.30 mL, 1.82 mmol) were added, and the mixture was reacted at 50°C for 2 h. The reaction liquid was cooled to room temperature and added dropwise into 20 mL of ice water, the mixture was filtered by suction, the filter cake was washed with 10 mL of water, the filter cake was collected, the filter cake was dissolved with 20 mL of DCM, the mixture was dried over anhydrous sodium sulfate, concentrated under reduced pressure and then the crude product was separated and purified with silica gel column chromatography (ethyl acetate/petroleum ether (v/v) = 1 : 1), and the purified crude product was further separated and purified with silica gel column chromatography (ethyl acetate/petroleum ether (v/v) = 1 : 1) to afford compound 72 (60 mg, yield: 15%).

$^1H$ NMR (400 MHz, $CDCl_3$) δ 8.50 (d, 1H), 8.34 (s, 1H), 7.97 (s, 1H), 7.79 (s, 1H), 7.73 (s, 1H), 7.67 (d, 1H), 7.61 - 7.55 (m, 1H), 7.53 - 7.46 (m, 1H), 6.84 - 6.78 (m, 1H), 6.56 (dd, 1H), 4.98 - 4.88 (m, 1H), 4.41 - 4.32 (m, 2H), 4.12 - 4.03 (m, 2H), 3.87 - 3.75 (m, 1H), 2.96 - 2.64 (m, 3H), 2.18 - 2.07 (m, 1H), 1.99 - 1.92 (m, 2H), 1.65 - 1.55 (m, 2H).

LCMS m/z = 665.2 $[M+1]^+$.

**Example 73:**

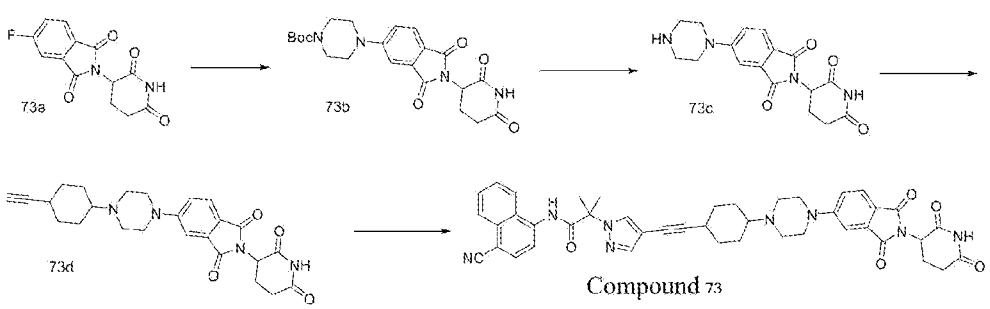

Step 1: tert-butyl 4-(2-(2,6-dioxopiperidin-3-yl)-1,3-dioxoisoindolin-5-yl)piperazine-1-carboxylate (73b)

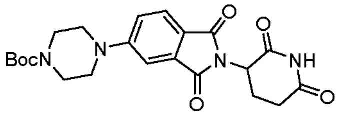

73a (3 g, 10.87 mmol) was dissolved in 30 mL of DMSO, tert-butyl piperazine-1-carboxylate (2.43 g, 13.05 mmol) and DIPEA (4.2g, 32.5 mmol) were added, and the mixture was reacted at 100°C for 3 h. The reaction liquid was cooled to room temperature and slowly added to 300 mL of water, and a yellow solid was precipitated and filtered, the filter cake was washed with 30 mL of water three times, the filter cake was collected, and the filter cake was dried by blowing at 50°C to afford crude 73b (4.5 g).

LCMS m/z = 443.2 $[M+1]^+$.

Step 2: 2-(2,6-dioxopiperidin-3-yl)-5-(piperazin-1-yl)isoindoline-1,3-dione (73c) hydrochloride

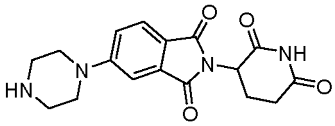

10 mL of ethyl acetate and 20 mL of a solution of 4 mol/L ethyl acetate hydrogen chloride solution were added to crude 73b (2 g), and the mixture was reacted at room temperature for 3 h. The reaction liquid was concentrated under reduced pressure to afford the hydrochloride of crude 73c (1.6 g).

LCMS m/z = 343.2 $[M+1]^+$.

Step 3: 2-(2,6-dioxopiperidin-3-yl)-5-(4-(4-ethynylcyclohexyl)piperazin-1-yl)isoindoline-1,3-dione (73d)

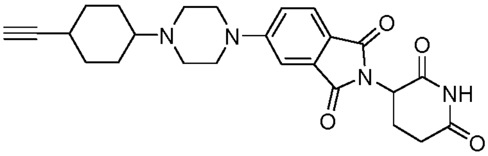

The hydrochloride of crude 73c (1.2 g) was added into a 100 mL single-mouth bottle, 20 mL of dry DCE was added, sodium bicarbonate (266 mg, 3.17 mmol) was added, the mixture was stirred at room temperature for 20 min, then 4-ethynylcyclohexan-1-one (464 mg, 3.80 mmol) and 0.1 mL of acetic acid were added, and the mixture was reacted at room temperature for 1.5 h, then sodium triacetoxyborohydride (1.3 g, 6.13 mmol) was added, and the mixture was reacted at room temperature for 16 h. 50 mL of saturated sodium bicarbonate solution was added to the reaction liquid, 50 mL of water was added, the mixture was extracted with ethyl acetate (30 mL × 3), the organic phase was washed with saturated sodium chloride solution (20 mL × 2), dried over anhydrous sodium sulfate, and concentrated under reduced pressure, the crude product was separated and purified with silica gel chromatography column (petroleum ether/ethyl acetate (v/v) = 23 : 77) to afford 73d (670 mg, three-step yield from compound 73a: 41%).

LCMS m/z = 449.2 $[M+1]^+$.

Step 4: N-(4-cyanonaphthalen-1-yl)-2-(4-((4-(4-(2-(2,6-dioxopiperidin-3-yl)-1,3-dioxoisoindolin-5-yl)piperazin-1-yl)cyclohexyl)ethynyl)-1H-pyrazol-1-yl)-2-methylpropanamide (Compound 73)

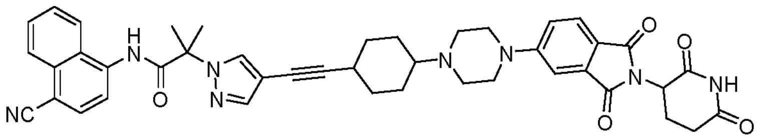

73d (120 mg, 0.27 mmol) was added into a 50 mL single-mouth bottle, 10 mL of dry DMF was added, 21b (96 mg, 0.223 mmol) and TEA (82 mg, 0.81 mmol) were added, nitrogen replacement was performed three times, and $PdCl_2(PPh_3)_2$ (19 mg, 0.027 mmol) and CuI (10 mg, 0.053 mmol) were added, nitrogen replacement was performed three times, and the mixture was reacted at 60°C for 2 h. The reaction system was cooled to room temperature, 100 mL of saturated ammonium chloride solution was slowly added, a yellow solid was precipitated, filtration was performed, the filter cake was washed with 10 mL of water three times, the filter cake was dissolved in DCM (30 mL), the mixture was dried over anhydrous sodium sulfate and concentrated under reduced pressure, the crude product was separated and purified with silica gel column chromatography (petroleum ether/ethyl acetate (v/v) = 13 : 87) to afford compound 73 (110 mg, yield: 66%).

$^1H$ NMR (400 MHz, $CDCl_3$) δ 10.06 - 9.87 (m, 1H), 8.42 - 8.20 (m, 2H), 8.05 - 7.55 (m, 8H), 7.34 - 7.24 (m, 1H), 7.14 - 6.97 (m, 1H), 4.99 - 4.88 (m, 1H), 4.20 - 3.25 (m, 4H), 3.15 - 2.60 (m, 7H), 2.40 - 1.88 (m, 11H), 1.88 - 1.46 (m, 6H).

LCMS m/z = 751.3 $[M+1]^+$.

**Example 74: Preparation of compound 74**

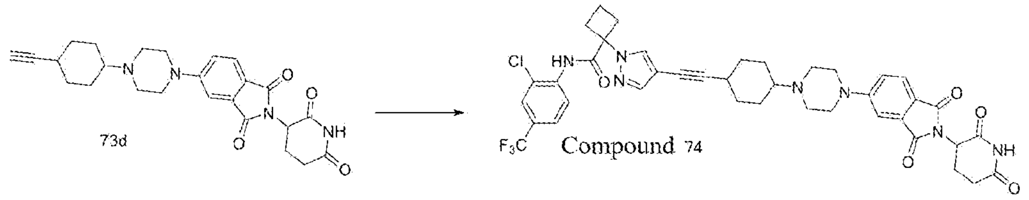

73d

Compound 74

73d (120 mg, 0.27 mmol) was added into a 50 mL single-mouth bottle, 10 mL of dry DMF was added, 14c (124 mg, 0.264 mmol) and TEA (82 mg, 0.81 mmol) were added, nitrogen replacement was performed three times, and $PdCl_2(PPh_3)_2$ (19 mg, 0.027 mmol) and CuI (10 mg, 0.053 mmol) were added, nitrogen replacement was performed three times, and the mixture was reacted at 60°C for 2 h. The reaction system was cooled to room temperature, 100 mL of saturated ammonium chloride solution was slowly added, a yellow solid was precipitated, filtration was performed, the filter cake was washed with 10 mL of water three times, the filter cake was dissolved in 50 mL of DCM, the mixture was dried over anhydrous sodium sulfate and concentrated under reduced pressure, the crude product was separated and purified with silica gel column chromatography (petroleum ether/ethyl acetate (v/v) = 11 : 89) to afford compound 74 (90 mg, yield: 43%).

$^1H$ NMR (400 MHz, $CDCl_3$) δ 8.85 - 8.67 (m, 1H), 8.60 - 8.50 (m, 1H), 7.95 (s, 1H), 7.80 - 7.35 (m, 5H), 7.33 - 7.26 (m, 1H), 7.12 - 7.00 (m, 1H), 4.99 - 4.89 (m, 1H), 4.25 - 3.25 (m, 4H), 3.20 - 2.60 (m, 11H), 2.50 - 1.86 (m, 7H), 1.86 - 1.43 (m, 4H), 1.39 - 1.21 (m, 2H).

LCMS m/z = 790.2 $[M+1]^+$.

**Example 75: Preparation of compound 75**

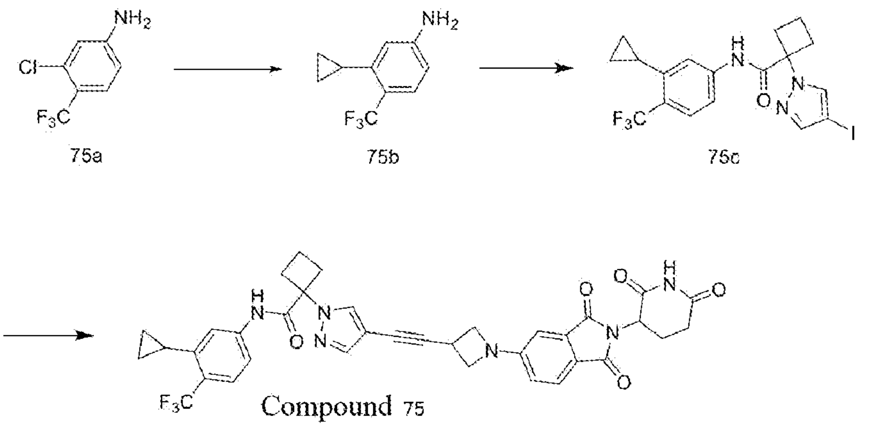

75a 75b 75c

Compound 75

Step 1: 3-cyclopropyl-4-(trifluoromethyl)aniline (75b)

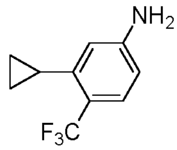

Cyclopropylboronic acid (32.95 g, 383.6 mmol) and 75a (50 g, 255.7 mmol) were added to a mixed solvent of 500 mL of dioxane and 50 mL of water with anhydrous potassium phosphate (217.11 g, 1022.8 mmol) dissolved therein, nitrogen replacement was performed three times, tricyclohexylphosphine (28.68 g, 102.27 mmol) and palladium acetate (11.48 g, 51.13 mmol) were added under nitrogen protection, and the mixture was reacted at 90°C for 16 h. The reaction liquid was cooled to room temperature, 15 mL of ethyl acetate and 15 mL of water were added, the liquid separation was conducted, the aqueous phase was extracted with ethyl acetate (10 mL × 2), the organic phases were combined, washed with 20 mL of saturated sodium chloride solution, dried over anhydrous sodium sulfate, and concentrated under reduced pressure. The crude product was separated and purified with silica gel chromatography column (petroleum ether/ethyl acetate (v/v) = 5 : 1) to afford 75b (4 g, yield: 8%).

Step 2: N-(3-cyclopropyl-4-(trifluoromethyl)phenyl)-1-(4-iodo-1H-pyrazol-1-yl)cyclobutane-1-carboxamide (75c)

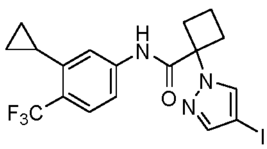

14b (11.5 g, 35.9 mmol) was dissolved in THF (100 mL), 20 mL of water and lithium hydroxide monohydrate (3.0 g, 71.5 mmol) were added, and the mixture was reacted at room temperature for 30 min. 1 mol/L dilute hydrochloric acid was added dropwise to the reaction liquid to adjust the pH to 6, 250 mL of ethyl acetate was added, the liquid separation was conducted, the organic phase was washed with 100 mL of saturated sodium chloride solution, dried over anhydrous sodium sulfate, and concentrated under reduced pressure to afford a crude product (10.0 g). The above crude product (4.13 g) was dissolved in 60 mL of DCM, 1-chloro-N,N,2-trimethylpropenylamine (2.52g, 18.87 mmol) was slowly added, and the mixture was reacted at room temperature for 2 h. 75b (2.37 g, 11.78 mmol) and TEA (3.58 g, 35.38 mmol) were added to the reaction liquid, and the mixture was reacted at room temperature for 16 h. 200 mL of DCM and 150 mL of saturated aqueous sodium bicarbonate solution were added to the reaction liquid, the organic phase was separated, washed with 100 mL of saturated sodium chloride solution, dried over anhydrous sodium sulfate, and concentrated under reduced pressure. The crude product was separated and purified with silica gel chromatography column (petroleum ether/ethyl acetate (v/v) = 5 : 1) to afford 75c (5.2 g, yield: 93%).

Step 3: N-(3-cyclopropyl-4-(trifluoromethyl)phenyl)-1-(4-((1-(2-(2,6-dioxopiperidin-3-yl)-1,3-dioxoisoindolin-5-yl)azetidin-3-yl)ethynyl)-1H-pyrazol-1-yl)cyclobutane-1-carboxamide (Compound 75)

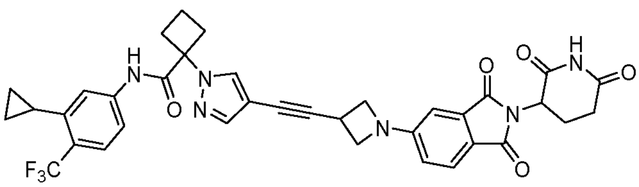

75c (2.6 g, 5.47 mmol) was dissolved in 30 mL of DMF, and intermediate 1 (2.03 g), TEA (1.66 g, 16.40 mmol), CuI (100 mg, 0.525 mmol) and $PdCl_2(PPh_3)_2$ (0.38 g, 0.54 mmol) were added in sequence, and the mixture was reacted at 55°C for 1 h. The reaction liquid was cooled to room temperature, 200 mL of water was added, the solid was precipitated,

suction-filtration was performed, the filter cake was washed with 50 mL of water. The filter cake was dissolved in 200 mL of DCM, the mixture was dried over anhydrous sodium sulfate, and concentrated under reduced pressure. The crude product was separated and purified with silica gel chromatography column (petroleum ether/ethyl acetate (v/v) = 1 : 1) to afford compound 75 (2.0 g, yield: 53%).

$^1$H NMR (400 MHz, $CDCl_3$) δ 8.25 - 8.12 (m, 2H), 7.77 (s, 1H), 7.72 - 7.62 (m, 2H), 7.53 - 7.46 (m, 1H), 7.27 - 7.21 (m, 1H), 7.20 - 7.14 (m, 1H), 6.83 - 6.77 (m, 1H), 6.55 (dd, 1H), 4.98 - 4.88 (m, 1H), 4.42 - 4.28 (m, 2H), 4.18 - 4.00 (m, 2H), 3.87 - 3.74 (m, 1H), 3.13 - 2.62 (m, 7H), 2.28 - 1.97 (m, 4H), 1.06 - 0.96 (m, 2H), 0.80 - 0.70 (m, 2H).

LCMS m/z = 685.2 $[M+1]^+$.

**Example 76: Preparation of compound 76**

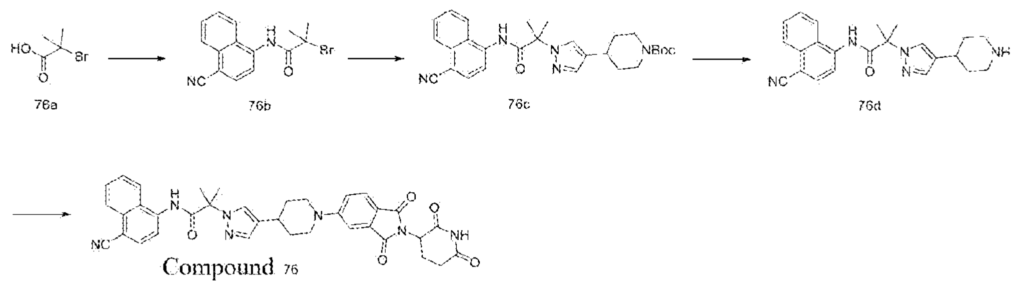

76a 76b 76c 76d

Compound 76

Step 1: 2-bromo-N-(4-cyanonaphthalen-1-yl)-2-methylpropanamide (76b)

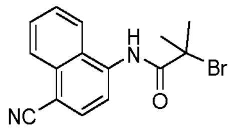

76a (2.0 g, 11.98 mmol) was dissolved in 20 mL of dry DCM, 1-chloro-N,N,2-trimethylpropenylamine (2.38 g, 17.79 mmol) was added, the mixture was reacted at room temperature for 1 h, then TEA (3.63 g, 35.87 mmol) and 4-amino-1-naphthalenenitrile (21a) (1.8 g, 10.7 mmol) were added, and the mixture was reacted at room temperature for 1 h. 0.5 mL of water was added to the reaction liquid, the mixture was concentrated under reduced pressure, and the crude product was separated and purified with silica gel chromatography column (petroleum ether/ethyl acetate (v/v) = 76 : 24) to afford 76b (2.7 g, yield: 80%).

LCMS m/z = 317.2 $[M+1]^+$.

Step 2: tert-butyl 4-(1-(1-((4-cyanonaphthalen-1-yl)amino)-2-methyl-1-oxopropan-2-yl)-1H- pyrazol-4-yl)piperidine-1-carboxylate (76c)

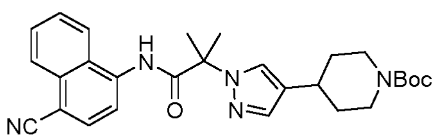

76b (500 mg, 1.58 mmol) was added into a 100 mL single-mouth bottle, 12 mL of acetonitrile and tert-butyl 4-(1H-pyrazol-4-yl)piperidine-1-carboxylate (396.19 mg, 1.58 mmol) and cesium carbonate (1.03 g, 3.16 mmol) were added, and the mixture was reacted at 80°C for 4 h. The reaction liquid was cooled to room temperature, filtered by suction, and the filtrate was concentrated under reduced pressure. The crude product was separated and purified with silica gel chromatography column (petroleum ether/ethyl acetate (v/v) = 83 : 17) to afford 76c (620 mg, yield: 80%).

LCMS m/z = 488.2 $[M+1]^+$.

Step 3: N-(4-cyanonaphthalen-1-yl)-2-methyl-2-(4-(piperidin-4-yl)-1H-pyrazol-1-yl)propanamide (76d) hydrochloride

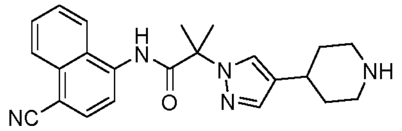

76c (620 mg, 1.27 mmol) was added into a 100 mL single-mouth bottle, 6 mL of ethyl acetate and a solution of 4 mol/L ethyl acetate hydrogen chloride solution (12 mL, 48 mmol) were added, and the mixture was reacted at room temperature for 2 h. The reaction liquid was concentrated under reduced pressure to afford the hydrochloride of crude 76d (490 mg).

Step 4: N-(4-cyanonaphthalen-1-yl)-2-(4-(1-(2-(2,6-dioxopiperidin-3-yl)-1,3-dioxoisoindolin-5-yl)piperidin-4-yl)-1H-pyrazol-1-yl)-2-methylpropanamide (Compound 76)

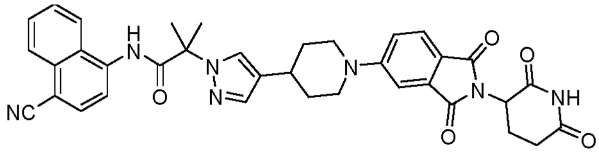

The hydrochloride of crude 76d (60 mg) was added into a 25 mL single-mouth bottle, then 6 mL of dry DMSO and sodium bicarbonate (61 mg, 0.73 mmol) were added, the mixture was stirred at room temperature for 20 min, then 1C (46.91 mg, 0.17 mmol) and DIPEA (54.9 mg, 0.425 mmol) were added, and the mixture was reacted at 60°C for 3 h. The reaction liquid was cooled to room temperature, 80 mL of water was added, a yellow solid was precipitated and filtered by suction, the filter cake was washed with 4 mL of water three times, the filter cake was dissolved in 50 mL of DCM, the mixture was dried over anhydrous sodium sulfate and concentrated under reduced pressure, the crude product was separated and purified with silica gel chromatography column (petroleum ether/ethyl acetate (v/v) = 31 : 69) to afford compound 76 (41 mg, yield: 37%).

$^1$H NMR (400 MHz, $CDCl_3$) $\delta$ 10.12 - 9.98 (m, 1H), 8.43 - 8.35 (m, 1H), 8.29 - 8.22 (m, 1H), 7.98 - 7.48 (m, 10H), 5.01 - 4.91 (m, 1H), 4.03 - 3.91 (m, 2H), 3.27 - 3.15 (m, 2H), 2.98 - 2.65 (m, 4H), 2.20 - 1.97 (m, 11H).

LCMS m/z = 644.3 [M+1]$^+$.

**Example 77: Preparation of compound 77**

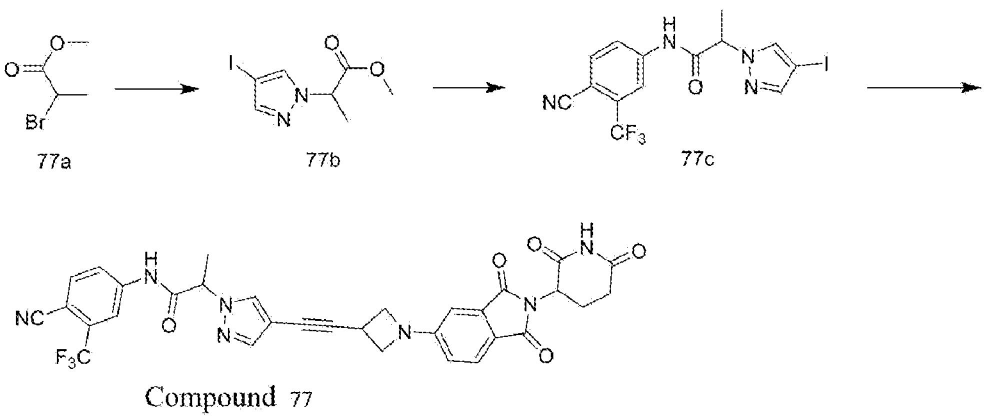
77a 77b 77c

Compound 77

Step 1: methyl 2-(4-iodo-1H-pyrazol-1-yl)propanoate (77b)

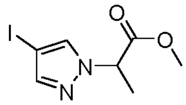

77a (5.0 g, 29.94 mmol) and 4-iodo-1H-pyrazole (6.38 g, 32.89 mmol) were dissolved in 80 mL of acetonitrile, cesium carbonate (14.62 g, 44.87 mmol) was added, and the mixture was reacted at 80°C for 3 h. The reaction liquid was cooled to room temperature and concentrated under reduced pressure, and the crude product was separated and purified with silica gel chromatography column (petroleum ether/ethyl acetate (v/v) = 10 : 1) to afford 77b (7.0 g, yield: 83%).

Step 2: N-(4-cyano-3-(trifluoromethyl)phenyl)-2-(4-iodo-1H-pyrazol-1-yl)propanamide (77c)

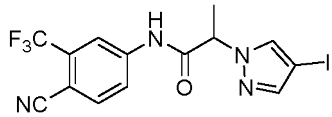

77b (7.0 g, 24.99 mmol) was dissolved in 40 mL of THF and 10 mL of water, lithium hydroxide monohydrate (1.05 g, 25.00 mmol) was added, the mixture was reacted at room temperature for 0.5 h, 50 mL of water was added, and the mixture was extracted with ethyl acetate (30 mL × 2), the aqueous phase was adjusted to pH 4 with concentrated hydrochloric acid, and the mixture was extracted with ethyl acetate (50 mL × 2), dried over anhydrous sodium sulfate, and concentrated under reduced pressure, the residue was slurried by adding 60 mL of petroleum ether, the mixture was filtered, and the filter cake was collected to afford a crude product (6.0 g). The above crude product (0.50 g) was dissolved in THF (8 mL), 1-chloro-N,N,2-trimethylpropenylamine (0.38 g, 2.84 mmol) was added, the mixture was reacted at room temperature for 2 h, then 4-aminoamine-2-(trifluoromethyl)benzonitrile (0.35 g, 1.88 mmol) and TEA (0.57 g, 5.63 mmol) were added in sequence, and the mixture was reacted at room temperature for 12 h. 10 mL of ethyl acetate and 10 mL of water were added to the reaction liquid, the organic phase was separated, washed with 10 mL of saturated sodium chloride solution, dried over anhydrous sodium sulfate, and concentrated under reduced pressure. The crude product was separated and purified with silica gel chromatography column (petroleum ether/ethyl acetate (v/v) = 3 : 1) to afford 77c (0.44 g, yield: 54%).

Step 3: N-(4-cyano-3-(trifluoromethyl)phenyl)-2-(4-((1-(2-(2,6-dioxopiperidin-3-yl)-1,3-dioxoisoindolin-5-yl)azetidin-3-yl)ethynyl)-1H-pyrazol-1-yl)propanamide (Compound 77)

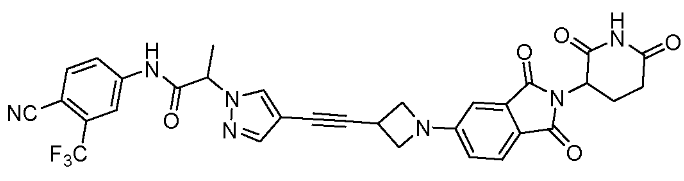

77c (0.22 g, 0.507 mmol) was dissolved in 6 mL of DMF, and intermediate 1 (0.19 g), TEA (0.15 g, 1.50 mmol), CuI (10 mg, 0.05 mmol) and $PdCl_2(PPh_3)_2$ (35 mg, 0.05 mmol) were added in sequence, nitrogen replacement was performed three times, and the mixture was reacted at 55°C for 1.5 h. The reaction liquid was cooled to room temperature, 50 mL of water was added, the solid was precipitated, suction-filtration was performed, the filter cake was washed with 10 mL of water. The filter cake was dissolved in 100 mL of DCM, the mixture was dried over anhydrous sodium sulfate, and concentrated under reduced pressure. The crude product was separated and purified with silica gel chromatography column (petroleum ether/ethyl acetate (v/v) = 1 : 1) to afford compound 77 (0.08 g, yield: 25%).

$^1H$ NMR (400 MHz, $CDCl_3$) δ 9.43 (s, 1H), 8.61 (s, 1H), 8.00 - 7.92 (m, 1H), 7.88 - 7.61 (m, 5H), 6.84 - 6.73

(m, 1H), 6.55 (dd, 1H), 5.11 - 4.88 (m, 2H), 4.44 - 4.30 (m, 2H), 4.13 - 4.00 (m, 2H), 3.88 - 3.73 (m, 1H), 2.97 - 2.65 (m, 3H), 2.19 - 2.08 (m, 1H), 1.85 (d, 3H).

LCMS m/z = 644.2 $[M+1]^+$.

## Example 78: Preparation of compound 78

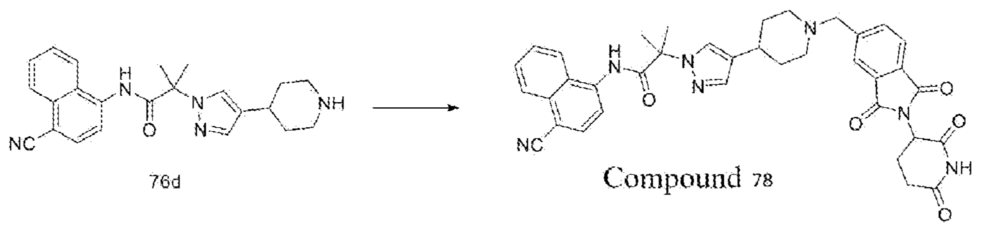

76d

Compound 78

The hydrochloride of crude 76d (60 mg) was added into a 25 mL single-mouth bottle, then 6 mL of dry DMSO and sodium bicarbonate (61 mg, 0.73 mmol) were added, the mixture was stirred at room temperature for 20 min, and 5-(bromomethyl)-2-(2,6-dioxopiperidin-3-yl)isoindoline-1,3-dione (59.64 mg, 0.170 mmol) (see WO 2021077010 for the synthesis method) and DIPEA (54.9 mg, 0.425 mmol) were added, and the mixture was reacted at 90°C for 3 h. The reaction liquid was cooled to room temperature, 80 mL of water was slowly added, a solid was precipitated and filtered by suction, the filter cake was washed with 4 mL of water three times, the filter cake was dissolved in 50 mL of DCM, the mixture was dried over anhydrous sodium sulfate and concentrated under reduced pressure, the crude product was separated and purified with silica gel chromatography column (petroleum ether/ethyl acetate (v/v) = 21 : 79) to afford compound 78 (11 mg, yield: 10%).

$^1$H NMR (400 MHz, $CDCl_3$) δ 10.20 - 9.85 (m, 1H), 8.45 - 8.32 (m, 1H), 8.30 - 8.20 (m, 1H), 8.10 - 7.50 (m, 10H), 5.05 - 4.93 (m, 1H), 3.74 - 3.46 (m, 2H), 3.03 - 2.42 (m, 8H), 2.25 - 1.85 (m, 10H), 1.83 - 1.62 (m, 1H).

LCMS m/z = 658.2 $[M+1]^+$.

## Example 79: Preparation of compound 79

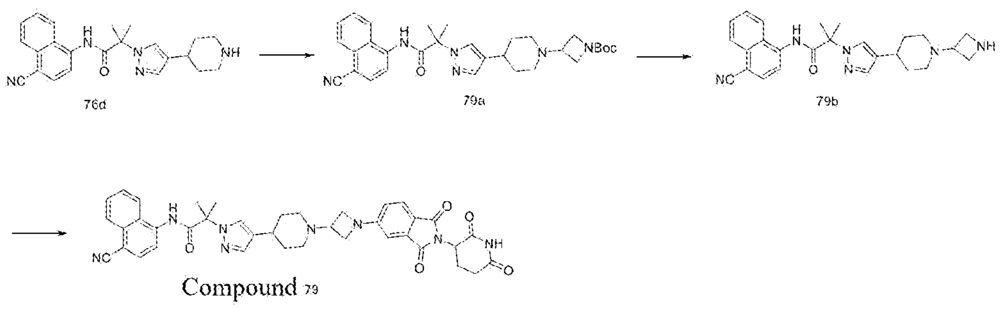

76d

79a

79b

Compound 79

Step 1: tert-butyl 3-(4-(1-(1-((4-cyanonaphthalen-1-yl)amino)-2-methyl-1-oxopropan-2-yl)-1H-pyrazol-4-yl)piperidin-1-yl)azetidine-1-carboxylate (79a)

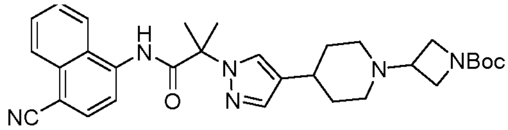

The hydrochloride of crude 76d (200 mg) was dissolved in 15 mL of dry 1,2-dichloroethane, sodium bicarbonate (48.6 mg, 0.579 mmol) was added, the mixture was reacted at room temperature for 20 min, then tert-butyl 3-oxoazetidine-1-carboxylate (161.53 mg, 0.945 mmol) was added, 0.06 mL of glacial acetic acid was added, the mixture was stirred at room temperature for 1.2 h, then sodium triacetoxyborohydride (399.2 mg, 1.88 mmol) was added, and the mixture was reacted at room temperature for 16 h. The reaction liquid was washed with saturated aqueous sodium bicarbonate solution (30 mL × 2), and the organic phase was further washed with saturated sodium chloride solution (20 mL × 2), dried over anhydrous sodium sulfate, and concentrated under reduced pressure, the crude product was separated and purified with silica gel chromatography column (petroleum ether/ethyl acetate (v/v) = 56 : 44) to afford 79a (130 mg, two-step yield from compound 76c: 46%).

LCMS m/z= 543.3 $[M+1]^+$.

Step 2: 2-(4-(1-(azetidin-3-yl)piperidin-4-yl)-1H-pyrazol-1-yl)-N-(4-cyanonaphthalen-1-yl)-2-methylpropanamide (79b) hydrochloride

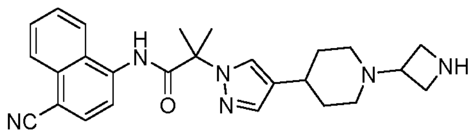

79a (130 mg, 0.239 mmol) was added into a 50 mL single-mouth bottle, 6 mL of ethyl acetate and a solution of 4 mol/L ethyl acetate hydrogen chloride solution (12 mL, 48 mmol) were added, and the mixture was reacted at room temperature for 2 h. The reaction liquid was concentrated under reduced pressure to afford the hydrochloride of crude 79b (80 mg).

LCMS m/z = 443.2 $[M+1]^+$.

Step 3: N-(4-cyanonaphthalen-1-yl)-2-(4-(1-(1-(2-(2,6-dioxopiperidin-3-yl)-1,3-dioxoisoindolin-5-yl)azetidin-3-yl)piperidin-4-yl)-1H-pyrazol-1-yl)-2-methylpropanamide (Compound 79)

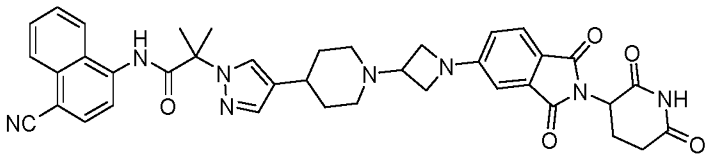

The hydrochloride of crude 79b (80 mg) was added into a 25 mL single-mouth bottle, 8 mL of dry DMSO and sodium bicarbonate (17.2 mg, 0.205 mmol) were added, the mixture was stirred at room temperature for 20 min, then 1C (55.36 mg, 0.20 mmol) and DIPEA (64.6 mg, 0.50 mmol) were added, and the mixture was reacted at 90°C for 3 h. The reaction liquid was cooled to room temperature, 80 mL of water was slowly added, a yellow solid was precipitated and filtered by suction, the filter cake was washed with 4 mL of water three times, the filter cake was dissolved in 50 mL of DCM, the mixture was dried over anhydrous sodium sulfate and concentrated under reduced pressure, the crude product was separated and purified with silica gel chromatography column (petroleum ether/ethyl acetate (v/v) = 51 : 49) to afford compound 79 (61 mg, two-step yield from compound 79a: 37%).

$^1H$ NMR (400 MHz, $CDCl_3$) δ 10.00 (s, 1H), 8.38 (d, 1H), 8.29 - 8.21 (m, 1H), 8.08 - 7.47 (m, 8H), 6.90 - 6.72 (m, 1H), 6.65 - 6.44 (m, 1H), 4.98 - 4.87 (m, 1H), 4.82 - 3.20 (m, 6H), 3.19 - 2.30 (m, 7H), 2.25 - 1.90 (m, 11H).

LCMS m/z = 699.0 $[M+1]^+$.

**Example 80: Preparation of compound 80**

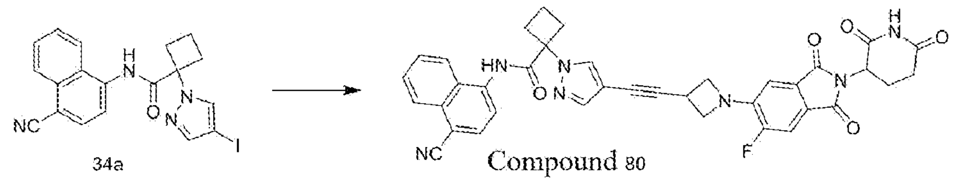

34a

Compound 80

34a (2.2 g, 4.97 mmol) was dissolved in 30 mL of DMF, and crude intermediate 2 (2.12 g), TEA (1.51 g, 14.92 mmol), CuI (95 mg, 0.50 mmol) and $PdCl_2(PPh_3)_2$ (350 mg, 0.50 mmol) were added in sequence, nitrogen replacement was performed three times, and the mixture was reacted at 55°C for 1.5 h. The reaction liquid was cooled to room temperature, 250 mL of water was added, the solid was precipitated, filtration was performed, the filter cake was washed with 50 mL of water. The filter cake was dissolved in 200 mL of DCM, the mixture was dried over anhydrous sodium sulfate, and concentrated under reduced pressure. The crude product was separated and purified with silica gel chromatography column (petroleum ether/ethyl acetate (v/v) = 1 : 1) to afford compound 80 (2.0 g, yield: 60%).

$^1H$ NMR (400 MHz, $CDCl_3$) δ 9.27 (s, 1H), 8.42 - 8.35 (m, 1H), 8.28 - 8.21 (m, 1H), 8.06 (s, 1H), 7.94 - 7.85 (m, 2H), 7.82 - 7.59 (m, 4H), 7.38 (d, 1H), 6.83 (d, 1H), 4.96 - 4.87 (m, 1H), 4.55 - 4.40 (m, 2H), 4.24 - 4.12 (m, 2H), 3.84 - 3.73 (m, 1H), 3.21 - 3.08 (m, 2H), 2.96 - 2.64 (m, 5H), 2.35 - 2.05 (m, 3H).

LCMS m/z = 670.1 $[M+1]^+$.

**Example 81: Preparation of compound 81**

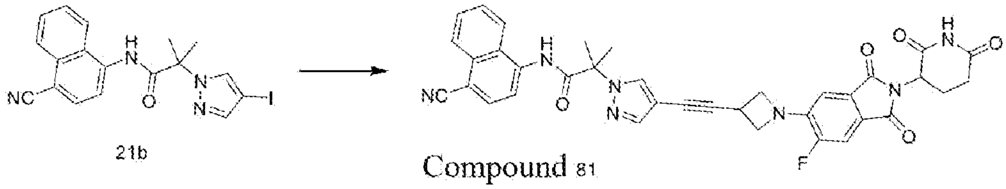

21b

Compound 81

21b (1.2 g, 2.79 mmol) was dissolved in 25 mL of DMF, and crude **intermediate 2** (1.19 g), TEA (0.85 g, 8.40 mmol), CuI (53 mg, 0.28 mmol) and $PdCl_2(PPh_3)_2$ (200 mg, 0.28 mmol) were added in sequence, nitrogen replacement was performed three times, and the mixture was reacted at 55°C for 1.5 h. The reaction liquid was cooled to room temperature, 150 mL of water was added, the solid was precipitated, filtration was performed, the filter cake was washed with 50 mL of water. The filter cake was dissolved in 200 mL of DCM, the mixture was dried over anhydrous sodium sulfate, and concentrated under reduced pressure. The crude product was separated and purified with silica gel chromatography column (petroleum ether/ethyl acetate (v/v) = 1 : 1) to afford compound 81 (1.5 g, yield: 82%).

$^1H$ NMR (400 MHz, $CDCl_3$) δ 9.92 (s, 1H), 8.37 - 8.32 (m, 1H), 8.28 - 8.22 (m, 1H), 8.06 (s, 1H), 7.95 - 7.79 (m, 4H), 7.75 - 7.62 (m, 2H), 7.42 - 7.34 (m, 1H), 6.84 (d, 1H), 4.96 - 4.87 (m, 1H), 4.52 - 4.43 (m, 2H), 4.26 - 4.13 (m, 2H), 3.86 - 3.71 (m, 1H), 2.94 - 2.66 (m, 3H), 2.18 - 2.08 (m, 1H), 2.01 (s, 6H).

LCMS m/z = 656.2 $[M-1]^-$.

**Example 82: Preparation of compound 82**

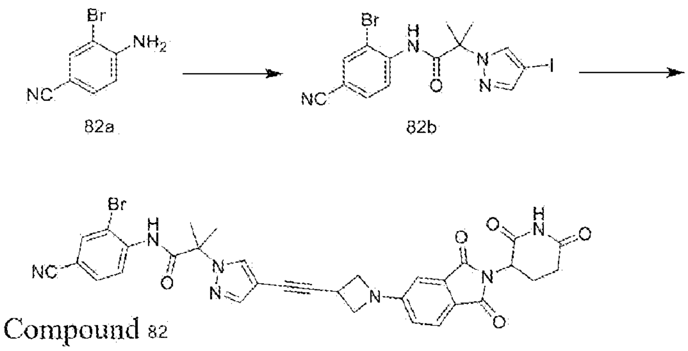

82a

82b

Compound 82

Step 1: N-(2-bromo-4-cyanophenyl)-2-(4-iodo-1H-pyrazol-1-yl)-2-methylpropanamide (82b)

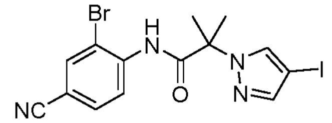

16a-1 (1.0 g, 3.57 mmol) was dissolved in 10 mL of DCM, 1-chloro-N,N,2-trimethylpropenylamine (0.65 g, 4.86 mmol) was added, the mixture was reacted at room temperature for 2 h, and then TEA (0.98 g, 9.68 mmol) and 82a (0.64 g, 3.25 mmol) were added in sequence, and the mixture was reacted at room temperature for 3 h. 80 mL of DCM was added to the reaction liquid, 30 mL of saturated aqueous sodium bicarbonate solution was added, the organic phase was separated, washed with 50 mL of saturated sodium chloride solution, dried over anhydrous sodium sulfate, and concentrated under reduced pressure. The crude product was separated and purified with silica gel chromatography column (petroleum ether/ethyl acetate (v/v) = 3 : 1) to afford 82b (1.2 g, yield: 80%).

Step 2: N-(2-bromo-4-cyanophenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 82)

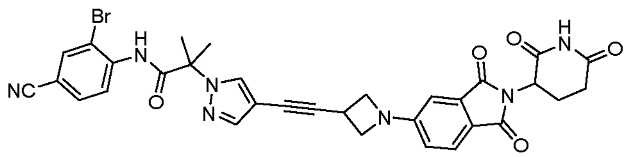

82b (230 mg, 0.50 mmol) was dissolved in 6 mL of DMF, and intermediate 1 (190 mg), TEA (0.15 g, 1.48 mmol), CuI (10 mg, 0.05 mmol) and $PdCl_2(PPh_3)_2$ (35 mg, 0.05 mmol) were added in sequence, nitrogen replacement was performed three times, and the mixture was reacted at 55°C for 1.5 h. The reaction liquid was cooled to room temperature, 50 mL of water was added, the solid was precipitated, suction-filtration was performed, the filter cake was washed with 10 mL of water. The filter cake was dissolved in 100 mL of DCM, the mixture was dried over anhydrous sodium sulfate, and concentrated under reduced pressure. The crude product was separated and purified with silica gel chromatography column (petroleum ether/ethyl acetate (v/v) = 1 : 1) to afford compound 82 (120 mg, yield: 36%).

$^1H$ NMR (400 MHz, $CDCl_3$) δ 9.16 (s, 1H), 8.51 (d, 1H), 8.00 (s, 1H), 7.83 - 7.77 (m, 3H), 7.67 (d, 1H), 7.57 (dd, 1H), 6.81 (d, 1H), 6.56 (dd, 1H), 4.98 - 4.89 (m, 1H), 4.40 - 4.32 (m, 2H), 4.10 - 4.03 (m, 2H), 3.87 - 3.76 (m, 1H), 2.96 - 2.66 (m, 3H), 2.17 - 2.08 (m, 1H), 1.95 (s, 6H).

LCMS m/z = 668.0 $[M+1]^+$.

**Example 83: Preparation of compound 83**

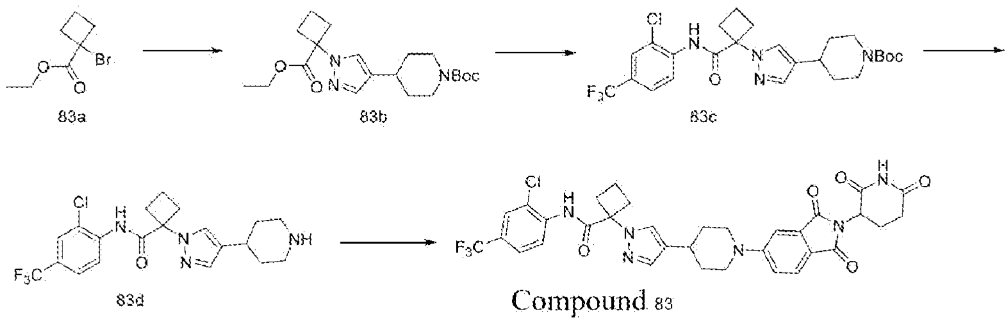

83a 83b 83c

83d Compound 83

Step 1: tert-butyl 4-(1-(1-(ethoxycarbonyl)cyclobutyl)-1H-pyrazol-4-yl)piperidine-1-carboxylate (83b)

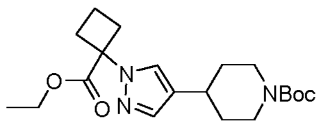

83a (800 mg, 3.86 mmol) was dissolved in 20 mL of dry acetonitrile, tert-butyl 4-(1H-pyrazol-4-yl)piperidine-1-carboxylate (971 mg, 3.86 mmol) and cesium carbonate (2.5 g, 7.67 mmol) were added, and the mixture was reacted at 80°C for 3 h. The reaction liquid was cooled to room temperature, filtered by suction, and the filtrate was concentrated under reduced pressure. The crude product was separated and purified with silica gel chromatography column (petroleum ether/ethyl acetate (v/v) = 78 : 22) to afford 83b (610 mg, yield: 42%).

LCMS m/z = 378.2 $[M+1]^+$.

Step 2: tert-butyl 4-(1-(1-((2-chloro-4-(trifluoromethyl)phenyl)carbamoyl) cyclobutyl)-1H-pyrazol-4-yl)piperidine-1-carboxylate (83c)

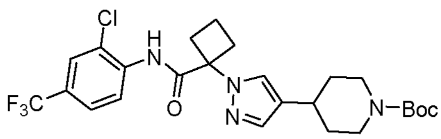

83b (610 mg, 1.62 mmol) was added into a 100 mL single-mouth bottle, 12 mL of THF and 4 mL of water were added, anhydrous lithium hydroxide (45.5 mg, 1.90 mmol) was added, and the mixture was reacted at room temperature for 1.5 h. The pH of the reaction liquid was adjusted to 5 with 1 mol/L dilute hydrochloric acid, the mixture was extracted with ethyl acetate (50 mL × 3), the organic phase was washed with saturated sodium chloride solution (30 mL × 2), dried over anhydrous sodium sulfate, and concentrated under reduced pressure, and the crude product was separated and purified with silica gel chromatography column (petroleum ether/ethyl acetate (v/v) = 46 : 54) to afford a crude product (460 mg). The crude product (460 mg) was added to a 50 mL single-mouth bottle, 12 mL of dry DCM was added, 1-chloro-N,N,2-trimethylpropenylamine (263.85 mg, 1.97 mmol) was added, and the mixture was reacted at room temperature for 1 h, then TEA (399.64 mg, 3.95 mmol) and 11a (231.71 mg, 1.18 mmol) were added, and the mixture was reacted at room temperature for 1.5 h. The reaction liquid was concentrated under reduced pressure, and the crude product was separated and purified with silica gel chromatography column (petroleum ether/ethyl acetate (v/v) = 67 : 33) to afford 83c (90 mg, yield: 14%).

LCMS m/z = 527.2 $[M+1]^+$.

Step 3: N-(2-chloro-4-(trifluoromethyl)phenyl)-1-(4-(piperidin-4-yl)-1H-pyrazol-1-yl)cyclobutane-1-carboxamide (83d) hydrochloride

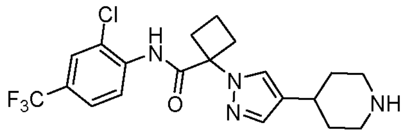

83c (90 mg, 0.17 mmol) was added into a 50 mL single-mouth bottle, 3 mL of ethyl acetate and a solution of 4 mol/L ethyl acetate hydrogen chloride solution (6 mL, 24.0 mmol) were added, and the mixture was reacted at room temperature for 1.5 h. The reaction liquid was concentrated under reduced pressure to afford the hydrochloride of crude 83d (60 mg).

LCMS m/z = 427.2 $[M+1]^+$.

Step 4: N-(2-chloro-4-(trifluoromethyl)phenyl)-1-(4-(1-(2-(2,6-dioxopiperidin-3-yl)-1,3-dioxoisoindolin-5-yl)piperidin-4-yl)-1H-pyrazol-1-yl)cyclobutane-1-carboxamide (Compound 83)

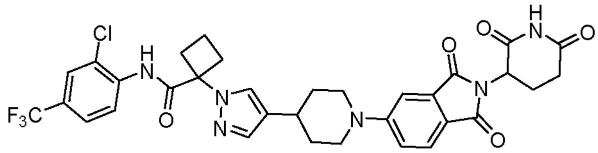

The hydrochloride of crude 83d (60 mg) was added into a 25 mL single-mouth bottle, 6 mL of dry DMSO and sodium bicarbonate (55.4 mg, 0.66 mmol) were added, the mixture was stirred at room temperature for 20 min, then 1C (42.92 mg, 0.156 mmol) and DIPEA (49.9 mg, 0.386 mmol) were added, and the mixture was reacted at 60°C for 3 h. The reaction liquid was cooled to room temperature, 80 mL of water was slowly added, a yellow solid was precipitated and filtered by suction, the filter cake was washed with 4 mL of water three times, the filter cake was dissolved in 20 mL of DCM, the mixture was dried over anhydrous sodium sulfate and concentrated under reduced pressure, the crude product was separated and purified with silica gel chromatography column (petroleum ether/ethyl acetate (v/v) = 26 : 74) to afford compound 83 (53 mg, two-step yield from compound 83c: 46%).

$^1H$ NMR (400 MHz, $CDCl_3$) δ 9.82 (s, 1H), 8.59 (d, 1H), 7.95 (s, 1H), 7.77 - 7.70 (m, 1H), 7.67 - 7.59 (m, 1H), 7.56 - 7.46 (m, 2H), 7.44 - 7.38 (m, 1H), 7.36 - 7.26 (m, 2H), 5.00 - 4.90 (m, 1H), 4.06 - 3.92 (m, 2H), 3.23 - 3.07 (m, 2H), 2.98 - 2.65 (m, 5H), 2.55 -2.00 (m, 8H), 1.95 - 1.75 (m, 2H).

LCMS m/z = 683.0 $[M+1]^+$.

**Example 84: Preparation of compound 84**

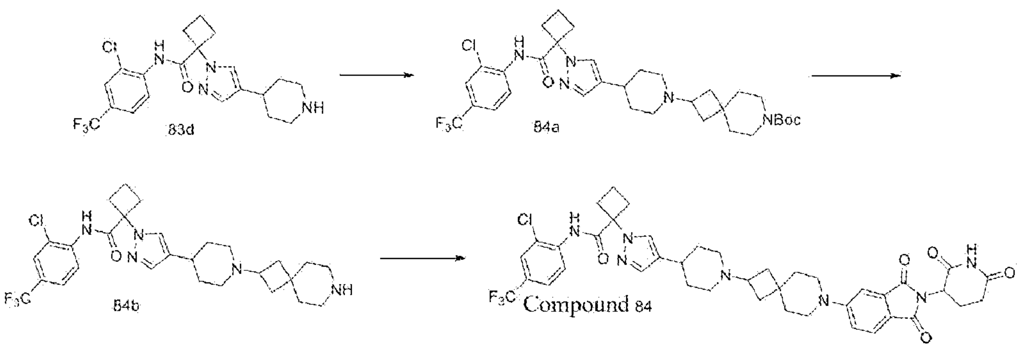

Step 1: tert-butyl 2-(4-(1-(1-((2-chloro-4-(trifluoromethyl)phenyl)carbamoyl) cyclobutyl)-1H-pyrazol-4-yl)piperidin-1-yl)-7-azaspiro[3. 5]nonane-7-carboxylate (84a)

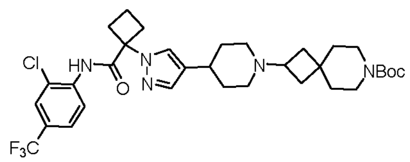

The hydrochloride of crude 83d (200 mg) was dissolved in 2 mL of DCE, sodium bicarbonate (48.6 mg, 0.579 mmol) was added, the mixture was reacted at room temperature for 20 min, then glacial acetic acid (6 mg, 0.1 mmol) and tert-butyl 2-oxo-7-azaspiro[3.5]nonane-7-carboxylate (130 mg, 0.54 mmol) were added, the mixture was stirred at room temperature for 1 h, then sodium triacetoxyborohydride (300 mg, 1.42 mmol) was added, and the mixture was reacted at room temperature for 5 h. 20 mL of saturated aqueous sodium bicarbonate solution was slowly added to the reaction liquid, the mixture was extracted with DCM (40 mL × 3), the organic phase was washed with 50 mL of water, dried over anhydrous sodium sulfate, and concentrated under reduced pressure. The crude product was separated and purified with silica gel column chromatography (DCM/MeOH (v/v) = 20 : 1) to afford 84a (0.25 g, two-step yield from compound 83c: 68%).

LCMS m/z = 650.3 $[M+1]^+$.

Step 2: 1-(4-(1-(7-azaspiro[3.5]nonan-2-yl)piperidin-4-yl)-1H-pyrazol-1-yl)-N-(2-chloro-4-(trifluoromethyl)phenyl)cyclobutane-1-carboxamide (84b)

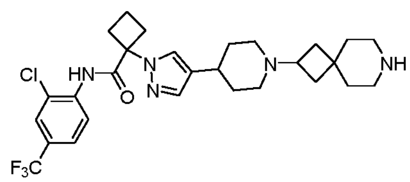

84a (0.2 g, 0.31 mmol) was dissolved in 1 mL of DCM, and 0.5 mL of trifluoroacetic acid was added. The mixture was reacted at room temperature for 1 h. The reaction system was concentrated under reduced pressure. The residue was dissolved in 30 mL of 4 mol/L aqueous NaOH solution and the mixture was extracted with DCM (40 mL × 3). The organic phase was washed with 50 mL of water, dried over anhydrous sodium sulfate, and concentrated under reduced pressure to afford crude 84b (0.15 g).

LCMS m/z= 550.3 $[M+1]^+$.

Step 3: N-(2-chloro-4-(trifluoromethyl)phenyl)-1-(4-(1-(7-(2-(2,6-dioxopiperidin-3-yl)-1,3-dioxoisoindolin-5-yl)-7-azaspiro[3.5]nonan-2-yl)piperidin-4-yl)-1H-pyrazol-1-yl)cyclobutane-1-carboxamide (Compound 84)

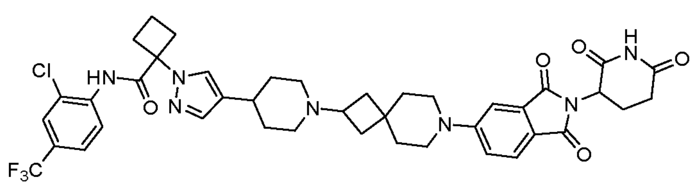

Crude 84b (200 mg) was dissolved in 2 mL of DMSO, 0.2 mL of DIPEA, sodium bicarbonate (61 mg, 0.73 mmol) and 1C (119 mg, 0.43 mmol) were added in sequence, and the mixture was stirred at 80°C for 5 h. The reaction liquid was cooled to room temperature, 20 mL of water was added, the mixture was filtered, and the filter cake was collected. The filter cake was washed with 10 mL of water, dissolved in 50 mL of DCM, dried over anhydrous sodium sulfate, and concentrated under reduced pressure. The crude product was separated and purified with silica gel column chromatography (DCM/MeOH (v/v) = 15 : 1) to afford compound 84 (200 mg, two-step yield from compound 84a: 60%).

$^1$H NMR (400 MHz, $CDCl_3$) δ 8.65 - 8.45 (m, 2H), 8.15 - 7.95 (m, 1H), 7.72 - 7.32 (m, 5H), 7.30 - 7.25 (m, 1H), 7.10 - 6.98 (m, 1H), 4.99 - 4.87 (m, 1H), 3.50 - 3.25 (m, 4H), 3.13 - 2.65 (m, 9H), 2.62 - 2.44 (m, 1H), 2.44 - 2.00 (m, 6H), 2.00 - 1.59 (m, 12H).

LCMS m/z= 806.3 [M+1]+.

**Example 85: Preparation of compound 85**

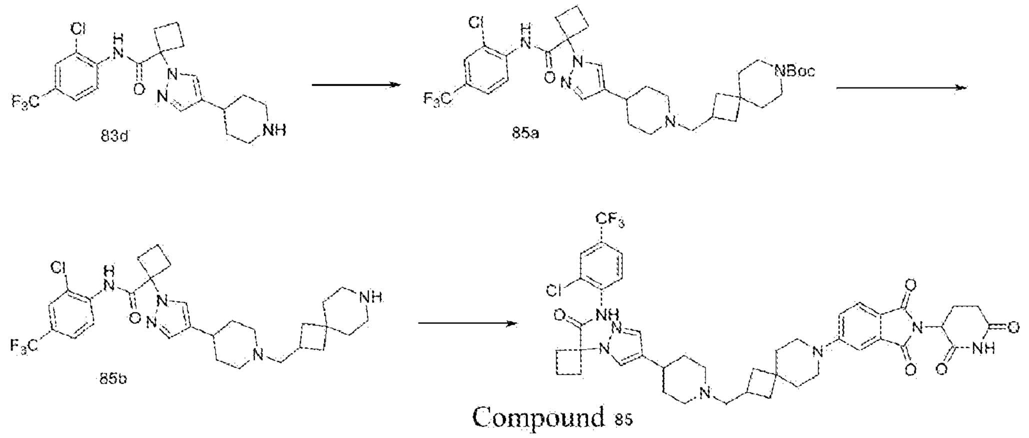


Step 1: tert-butyl 2-((4-(1-(1-((2-chloro-4-(trifluoromethyl)phenyl)carbamoyl) cyclobutyl)-1H-pyrazol-4-yl)piperidin-1-yl)methyl)-7-azaspiro[3.5]nonane-7-carboxylate (85a)

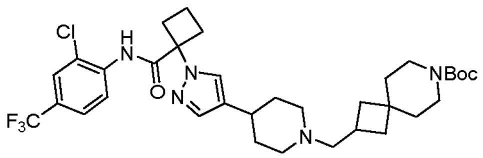

The hydrochloride of crude 83d (200 mg) was dissolved in 2 mL of DCE, sodium bicarbonate (48.6 mg, 0.579 mmol) was added, the mixture was reacted at room temperature for 20 min, then glacial acetic acid (6 mg, 0.1 mmol) and tert-butyl 2-formyl-7-azaspiro[3.5]nonane-7-carboxylate (see WO 2021023105 for the synthesis method) (86 mg, 0.34 mmol) were added, the mixture was stirred at room temperature for 1 h, then sodium triacetoxyborohydride (300 mg, 1.42 mmol) was added, and the mixture was stirred at room temperature for 5 h. 20 mL of saturated aqueous sodium bicarbonate solution was slowly added to the reaction liquid, the mixture was extracted with DCM (40 mL × 3), the organic phase was washed with 50 mL of water, dried over anhydrous sodium sulfate, and concentrated under reduced pressure. The crude product was separated and purified with silica gel column chromatography (DCM/MeOH (v/v) = 20 : 1) to afford 85a (0.25 g, two-step yield from compound 83c: 66%).

LCMS m/z = 664.3 [M+1]+.

Step 2: 1-(4-(1-((7-azaspiro[3.5]nonan-2-yl)methyl)piperidin-4-yl)-1H-pyrazol-1-yl)-N-(2-chloro-4-(trifluoromethyl)phenyl)cyclobutane-1-carboxamide (85b)

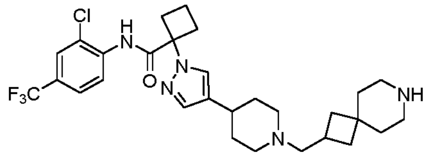

85a (0.2 g, 0.30 mmol) was dissolved in 1 mL of DCM, and 0.5 mL of trifluoroacetic acid was added. The mixture was stirred at room temperature for 2 h. The reaction system was concentrated under reduced pressure. The residue was dissolved in 30 mL of 4 mol/L aqueous NaOH solution and the mixture was extracted with DCM (40 mL × 3). The

organic phase was washed with 50 mL of water, dried over anhydrous sodium sulfate, and concentrated under reduced pressure to afford crude 85b (0.15 g).

LCMS m/z = 564.2 $[M+1]^+$.

Step 3: N-(2-chloro-4-(trifluoromethyl)phenyl)-1-(4-(1-((7-(2-(2,6-dioxopiperidin-3-yl)-1,3-dioxoisoindolin-5-yl)-7-azaspiro[3.5]nonan-2-yl)methyl) piperidin-4-yl)-1H-pyrazol-1-yl)cyclobutane-1-carboxamide (Compound 85)

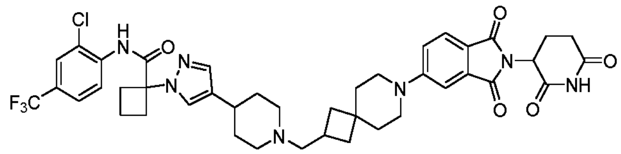

Crude 85b (200 mg) was dissolved in 2 mL of DMSO, 0.2 mL of DIPEA, sodium bicarbonate (59 mg, 0.70 mmol) and 1C (116 mg, 0.42 mmol) were added in sequence, and the mixture was stirred at 80°C for 5 h. The reaction liquid was cooled to room temperature, 20 mL of water was added, the mixture was filtered, and the filter cake was collected. The filter cake was washed with 10 mL of water, the filter cake was dissolved in 50 mL of DCM, dried over anhydrous sodium sulfate, and concentrated under reduced pressure. The crude product was separated and purified with silica gel column chromatography (DCM/MeOH (v/v) = 15 : 1) to afford compound 85 (200 mg, two-step yield from compound 85a: 61%).

$^1$H NMR (400 MHz, $CDCl_3$) δ 8.61 - 8.52 (m, 2H), 8.28 - 8.14 (m, 1H), 7.70 - 7.45 (m, 4H), 7.37 (s, 1H), 7.29 - 7.24 (m, 1H), 7.07 - 6.99 (m, 1H), 4.98 - 4.88 (m, 1H), 3.47 - 3.26 (m, 4H), 3.14 - 2.65 (m, 9H), 2.61 - 2.39 (m, 3H), 2.34 - 1.97 (m, 7H), 1.97 - 1.82 (m, 2H), 1.82 - 1.44 (m, 9H).

LCMS m/z = 820.3 $[M+1]^+$.

**Example 86: Preparation of compound 86**

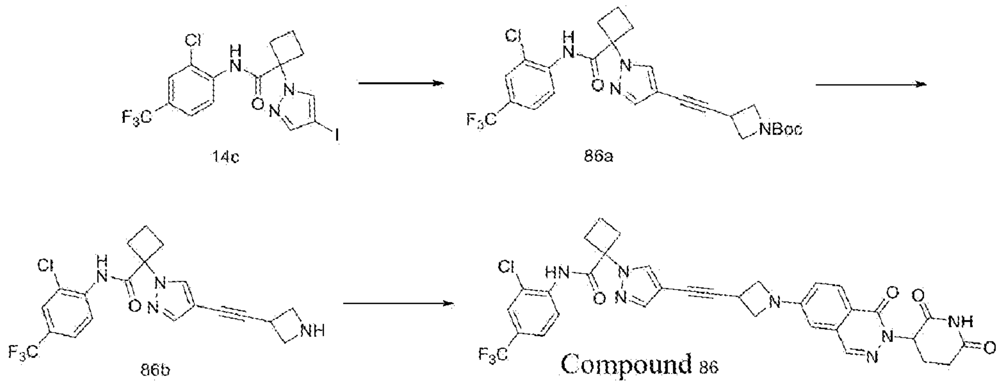

14c

86a

86b

Compound 86

Step 1: tert-butyl 3-((1-(1-((2-chloro-4-(trifluoromethyl)phenyl)carbamoyl) cyclobutyl)-1H- pyrazol-4-yl)ethynyl)azetidine-1-carboxylate (86a)

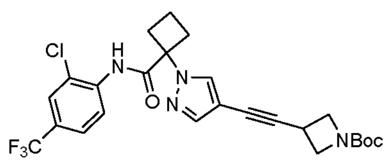

14c (1.0 g, 2.13 mmol) was dissolved in 10 mL of DMF, and tert-butyl 3-ethynylazetidine-1-carboxylate (460 mg, 2.54 mmol), CuI (41.0 mg, 0.22 mmol), DIPEA (550 mg, 4.26 mmol) and $PdCl_2(PPh_3)_2$ (150 mg, 0.214 mmol) were

added in sequence, nitrogen replacement was performed three times, and the mixture was reacted at 50°C for 3 h. The reaction liquid was cooled to room temperature, 200 mL of ethyl acetate and 100 mL of saturated aqueous sodium bicarbonate solution were added for extraction, the aqueous phase was extracted with 100 mL of ethyl acetate, the organic phases were combined, and the organic phase was washed with 100 mL of saturated sodium chloride solution, dried over anhydrous sodium sulfate, and concentrated under reduced pressure. The crude product was separated and purified with silica gel chromatography column (petroleum ether/ethyl acetate (v/v) = 5 : 1) to afford 86a (1.1 g, yield: 99%).

LCMS m/z= 521.0 $[M-1]^+$.

Step 2: 1-(4-(azetidin-3-ylethynyl)-1H-pyrazol-1-yl)-N-(2-chloro-4-(trifluoromethyl)phenyl)cyclobutane-1-carboxamide (86b)

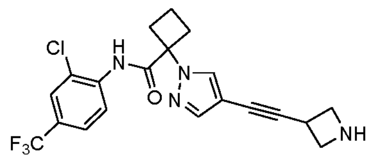

86a (100 mg, 0.19 mmol) was dissolved in DCM (2 mL), and 1 mL of trifluoroacetic acid was added. The mixture was reacted at room temperature for 2 h. The reaction system was concentrated under reduced pressure. The residue was dissolved in 30 mL of 4 mol/L aqueous NaOH solution and the mixture was extracted with DCM (40 mL × 3). The organic phase was washed with 50 mL of water, dried over anhydrous sodium sulfate, and concentrated under reduced pressure to afford crude 86b (70 mg).

LCMS m/z = 423.1 $[M+1]^+$.

Step 3: N-(2-chloro-4-(trifluoromethyl)phenyl)-1-(4-((1-(2-(2,6-dioxopiperidin-3-yl)-1-oxo-1,2-dihydrophthalazin-6-yl)azetidin-3-yl)ethynyl)-1H-pyrazol-1-yl)cyclobutane-1-carboxamide (Compound 86)

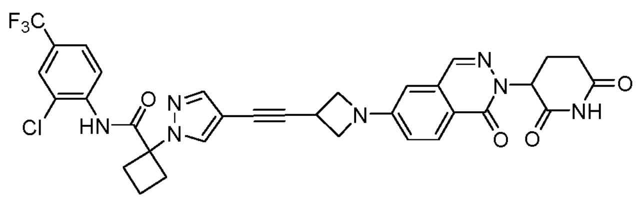

Crude 86b (70 mg) was dissolved in 2 mL of DMSO, 140 μL of DIPEA, sodium bicarbonate (29 mg, 0.35 mmol) and 3-(6-fluoro-1-oxophthalazin-2(1H)-yl)piperidine-2,6-dione (see WO 2021249534 for the synthesis method) (56 mg, 0.20 mmol) were added, and the mixture was stirred at 130°C for 12 h. The reaction liquid was cooled to room temperature, 20 mL of water was added, the mixture was filtered, and the filter cake was collected. The filter cake was washed with 10 mL of water, the filter cake was dissolved in 50 mL of DCM, dried over anhydrous sodium sulfate, and concentrated under reduced pressure. The crude product was separated and purified with silica gel column chromatography (DCM/MeOH (v/v) = 15 : 1) to afford compound 86 (100 mg, two-step yield from compound 86a: 78%).

$^1H$ NMR (400 MHz, $CDCl_3$) δ 8.69 (s, 1H), 8.54 (d, 1H), 8.23 (d, 1H), 8.01 (s, 1H), 7.79 (s, 1H), 7.70 (s, 1H), 7.60 - 7.46 (m, 2H), 6.80 (dd, 1H), 6.46 - 6.40 (m, 1H), 5.88 - 5.77 (m, 1H), 4.42 - 4.30 (m, 2H), 4.13 - 4.00 (m, 2H), 3.88 - 3.74 (m, 1H), 3.14 - 2.99 (m, 2H), 2.96 - 2.64 (m, 5H), 2.34 - 2.00 (m, 3H).

LCMS m/z = 678.2 $[M+1]^+$.

**Example 87: Preparation of compound 87**

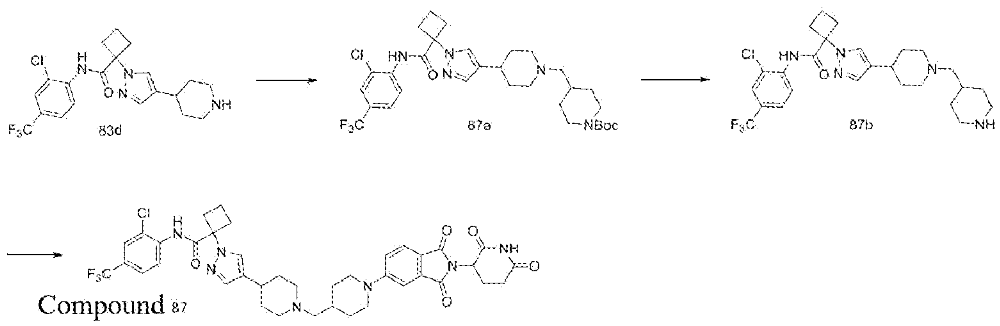

83d 87a 87b

Compound 87

Step 1: tert-butyl 4-((4-(1-(1-((2-chloro-4-(trifluoromethyl)phenyl)carbamoyl) cyclobutyl)-1H-pyrazol-4-yl)piperidin-1-yl)methyl)piperidine-1-carboxylate (87a)

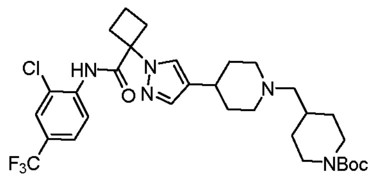

The hydrochloride of crude 83d (120 mg) was dissolved in 5 mL of DCE, sodium bicarbonate (29.16 mg, 0.347 mmol) was added, the mixture was reacted at room temperature for 20 min, and then tert-butyl 4-formylpiperidine-1-carboxylate (66 mg, 0.31 mmol), 100 mg of molecular sieve and 0.1 mL of acetic acid were added in sequence, the mixture was stirred at room temperature for 2 h, then sodium triacetoxyborohydride (180 mg, 0.85 mmol) was added, and the mixture was reacted at room temperature for 16 h. 20 mL of saturated aqueous sodium bicarbonate solution was slowly added to the reaction liquid, the mixture was extracted with DCM (40 mL × 3), the organic phase was washed with 50 mL of water, dried over anhydrous sodium sulfate, and concentrated under reduced pressure. The crude product was separated and purified with silica gel column chromatography (DCM/MeOH (v/v) = 20 : 1) to afford 87a (170 mg, two-step yield from compound 83c: 80%).

LCMS m/z = 624.3 $[M+1]^+$.

Step 2: N-(2-chloro-4-(trifluoromethyl)phenyl)-1-(4-(1-(piperidin-4-ylmethyl) piperidin-4-yl)-1H-pyrazol-1-yl)cyclobutane-1-carboxamide (87b)

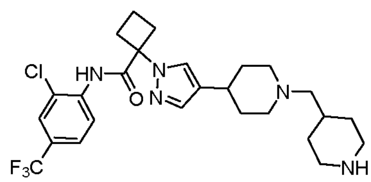

87a (0.17 g, 0.27 mmol) was dissolved in DCM (2 mL), and 1 mL of trifluoroacetic acid was added. The mixture was reacted at room temperature for 3 h. The reaction system was concentrated under reduced pressure, and the residue was dissolved in 30 mL of saturated aqueous sodium carbonate solution and the mixture was extracted with DCM (40 mL × 3). The organic phase was washed with 50 mL of water, dried over anhydrous sodium sulfate, and concentrated under reduced pressure to afford crude 87b (0.14 g).

LCMS m/z = 524.3 $[M+1]^+$.

Step 3: N-(2-chloro-4-(trifluoromethyl)phenyl)-1-(4-(1-((1-(2-(2,6-dioxopiperidin-3-yl)-1,3-dioxoisoindolin-5-yl)piperidin-4-yl)methyl)piperidin-4-yl)-1H-pyrazol-1-yl) cyclobutane-1-carboxamide (Compound 87)

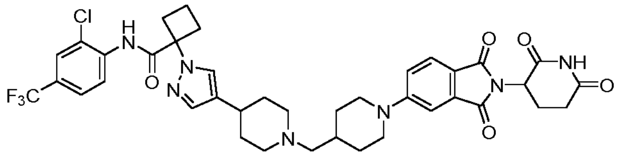

Crude 87b (0.14 g) was dissolved in 4 mL of DMSO, 0.1 mL of DIPEA, sodium bicarbonate (45 mg, 0.54 mmol) and 1C (89 mg, 0.32 mmol) were added in sequence, and the mixture was stirred at 80°C for 4 h. The reaction liquid was cooled to room temperature, 20 mL of water was added, the mixture was filtered, and the filter cake was collected. The filter cake was washed with 10 mL of water, the filter cake was dissolved in 50 mL of DCM, dried over anhydrous sodium sulfate, and concentrated under reduced pressure. The crude product was separated and purified with silica gel column chromatography (DCM/MeOH (v/v) = 15 : 1) to afford compound 87 (140 mg, two-step yield from compound 87a: 66%).

$^1$H NMR (400 MHz, $CDCl_3$) δ 8.66 - 8.50 (m, 2H), 8.01 (s, 1H), 7.73 - 7.32 (m, 5H), 7.30 - 7.25 (m, 1H), 7.08 - 7.01 (m, 1H), 4.99 - 4.88 (m, 1H), 4.02 - 3.86 (m, 2H), 3.15 - 2.64 (m, 12H), 2.62 - 2.40 (m, 1H), 2.34 - 1.20 (m, 15H).

LCMS m/z = 780.3 $[M+1]^+$.

**Example 88: Preparation of compound 88**

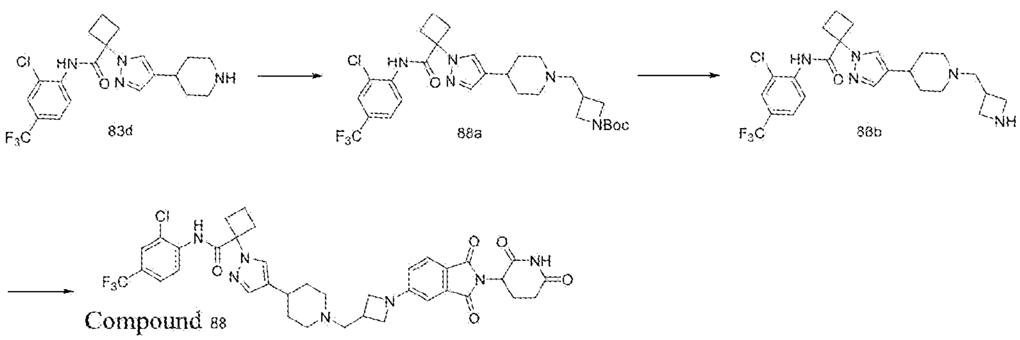

83d

88a

88b

Compound 88

Step 1: tert-butyl 3-((4-(1-(1-((2-chloro-4-(trifluoromethyl)phenyl)carbamoyl) cyclobutyl)-1H-pyrazol-4-yl)piperidin-1-yl)methyl)azetidine-1-carboxylate (88a)

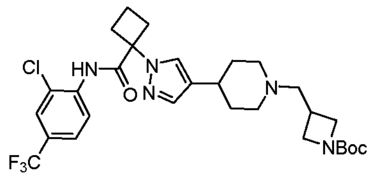

The hydrochloride of crude 83d (120 mg) was dissolved in 5 mL of DCE, sodium bicarbonate (29.16 mg, 0.347 mmol) was added, the mixture was reacted at room temperature for 20 min, and then tert-butyl 3-formylazetidine-1-carboxylate (57 mg, 0.31 mmol), 100 mg of molecular sieve and 0.1 mL of acetic acid were added in sequence, the mixture was stirred at room temperature for 2 h, then sodium triacetoxyborohydride (180 mg, 0.85 mmol) was added, and the mixture was reacted at room temperature for 16 h. 20 mL of saturated aqueous sodium bicarbonate solution was slowly added to the reaction liquid, the mixture was extracted with DCM (40 mL × 3), the organic phase was washed with 50 mL of water, dried over anhydrous sodium sulfate, and concentrated under reduced pressure. The crude product was separated and purified with silica gel column chromatography (DCM/MeOH (v/v) = 20 : 1) to afford 88a (166 mg, two-step yield from compound 83c: 82%).

LCMS m/z= 596.3 $[M+1]^+$.

Step 2: 1-(4-(1-(azetidin-3-ylmethyl)piperidin-4-yl)-1H-pyrazol-1-yl)-N-(2-chloro-4-(trifluoromethyl)phenyl)cyclobutane-1-carboxamide (88b)

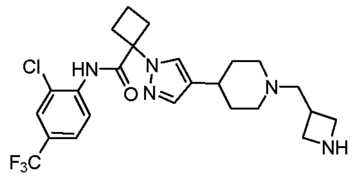

88a (0.166 g, 0.278 mmol) was dissolved in DCM (2 mL), and 1 mL of trifluoroacetic acid was added. The mixture was reacted at room temperature for 3 h. The reaction system was concentrated under reduced pressure, and the residue was dissolved in 30 mL of saturated aqueous sodium carbonate solution and the mixture was extracted with DCM (40 mL × 3). The organic phase was washed with 50 mL of water, dried over anhydrous sodium sulfate, and concentrated under reduced pressure to afford crude 88b (0.14 g).
LCMS m/z = 496.3 $[M+1]^+$.

Step 3: N-(2-chloro-4-(trifluoromethyl)phenyl)-1-(4-(1-((1-(2-(2,6-dioxopiperidin-3-yl)-1,3-dioxoisoindolin-5-yl)azetidin-3-yl)methyl)piperidin-4-yl)-1H-pyrazol-1-yl)cyclobutane-1-carboxamide (Compound 88)

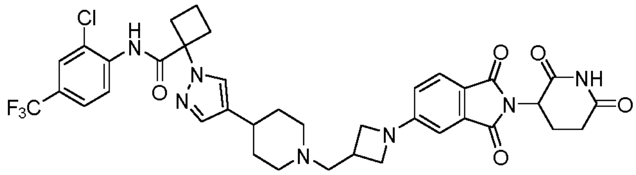

Crude 88b (0.14 g) was dissolved in 4 mL of DMSO, 0.1 mL of DIPEA, sodium bicarbonate (47 mg, 0.56 mmol) and 1C (93 mg, 0.34 mmol) were added in sequence, and the mixture was stirred at 80°C for 4 h. The reaction liquid was cooled to room temperature, 20 mL of water was added, the mixture was filtered, and the filter cake was collected. The filter cake was washed with 10 mL of water, the filter cake was dissolved in 50 mL of DCM, dried over anhydrous sodium sulfate, and concentrated under reduced pressure. The crude product was separated and purified with silica gel column chromatography (DCM/MeOH (v/v) = 15 : 1) to afford compound 88 (80 mg, two-step yield from compound 88a: 38%).
$^1H$ NMR (400 MHz, $CDCl_3$) δ 8.64 - 8.52 (m, 2H), 8.20 (s, 1H), 7.69 - 7.46 (m, 4H), 7.39 (s, 1H), 6.83 - 6.78 (m, 1H), 6.55 - 6.46 (m, 1H), 4.98 - 4.87 (m, 1H), 4.29 - 4.05 (m, 2H), 3.85 - 3.60 (m, 2H), 3.15 - 2.40 (m, 13H), 2.35 - 1.82 (m, 7H), 1.78 - 1.56 (m, 2H).
LCMS m/z = 752.3 $[M+1]^+$.

**Example 89: Preparation of compound 89**

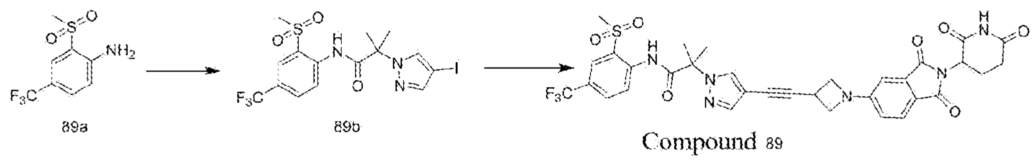

89a

89b

Compound 89

Step 1: 2-(4-iodo-1H-pyrazol-1-yl)-2-methyl-N-(2-(methylsulfonyl)-4-(trifluoromethyl)phenyl)propanamide (89b)

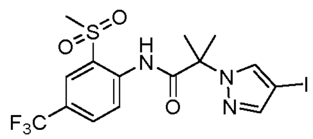

16a-1 (0.25 g, 0.89 mmol) was dissolved in 5 mL of DCM, 1-chloro-N,N,2-trimethylpropenylamine (0.18 g, 1.35 mmol) was added, the mixture was reacted at room temperature for 2 h, and then TEA (0.27 g, 2.67 mmol) and 89a (0.21 g, 0.88 mmol) (see WO 201875937 for the synthesis method) were added in sequence, and the mixture was reacted at room temperature for 12 h. 100 mL of DCM and 50 mL of saturated aqueous sodium bicarbonate solution were added to the reaction liquid, the organic phase was separated, washed with 80 mL of saturated sodium chloride solution, dried over anhydrous sodium sulfate, and concentrated under reduced pressure. The crude product was separated and purified with silica gel chromatography column (petroleum ether/ethyl acetate (v/v) = 5 : 1) to afford 89b (0.2 g, yield: 45%).

Step 2: 2-(4-((1-(2-(2,6-dioxopiperidin-3-yl)-1,3-dioxoisoindolin-5-yl)azetidin-3-yl)ethynyl)-1H-pyrazol-1-yl)-2-methyl-N-(2-(methylsulfonyl)-4-(trifluoromethyl) phenyl)propanamide (Compound 89)

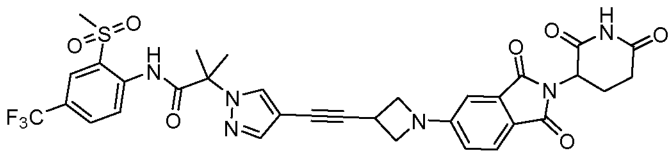

89b (0.2 g, 0.4 mmol) was dissolved in 5 mL of DMF, and intermediate 1 (0.13g), TEA (0.24 g, 2.37 mmol), CuI (15 mg, 0.08 mmol) and $PdCl_2(PPh_3)_2$ (28 mg, 0.04 mmol) were added in sequence, and the mixture was reacted at 60°C for 3 h. The reaction liquid was cooled to room temperature, 50 mL of water was added, the solid was precipitated, suction-filtration was performed, the filter cake was washed with 10 mL of water. The filter cake was dissolved in 100 mL of DCM, the mixture was dried over anhydrous sodium sulfate, and concentrated under reduced pressure. The crude product was separated and purified with silica gel chromatography column (petroleum ether/ethyl acetate (v/v) = 1 : 1) to afford compound 89 (0.02 g, yield: 7%).

$^1$HNMR (400 MHz, $CDCl_3$) δ 9.64 (s, 1H), 8.63 (d, 1H), 8.18 - 8.11 (m, 1H), 8.04 (br.s, 1H), 7.86 (dd, 1H), 7.82 - 7.78 (m, 1H), 7.74 - 7.69 (m, 1H), 7.67 (d, 1H), 6.84 - 6.79 (m, 1H), 6.56 (dd, 1H), 4.98 - 4.88 (m, 1H), 4.44 - 4.27 (m, 2H), 4.15 - 4.00 (m, 2H), 3.88 - 3.75 (m, 1H), 3.00 - 2.65 (m, 6H), 2.21 - 2.07 (m, 1H), 1.93 (s, 6H).

LCMS m/z = 711.2 $[M+1]^+$.

**Example 90: Preparation of compound 90**

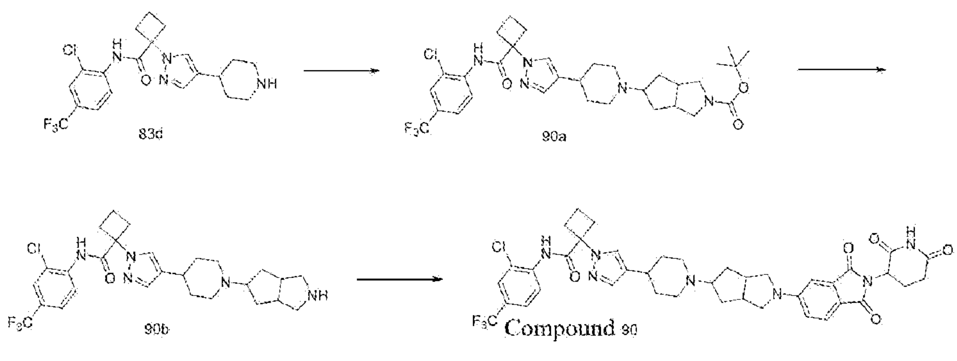


Step 1: tert-butyl 5-(4-(1-(1-((2-chloro-4-(trifluoromethyl)phenyl)carbamoyl) cyclobutyl)-1H- pyrazol-4-yl)piperidin-1-yl)hexahydrocyclopenta[c]pyrrole-2(1H)-carboxylate (90a)

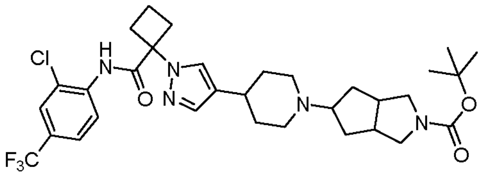

The hydrochloride of crude 83d (0.1 g) was dissolved in 10 mL of DMAc, sodium bicarbonate (24.3 mg, 0.289 mmol) was added, the mixture was reacted at room temperature for 20 min, then tert-butyl 5-oxohexahydrocyclopenta[c]pyrrole-2(1H)-carboxylate (0.078 g, 0.35 mmol), 0.1 mL of glacial acetic acid and 400 mg of anhydrous magnesium sulfate were added, the mixture was stirred at room temperature for 2 h, then sodium triacetoxyborohydride (0.146 g, 0.69 mmol) was added, and the mixture was reacted at room temperature for 2 h. 50 mL of saturated aqueous sodium bicarbonate solution was added to the reaction liquid, the mixture was extracted with ethyl acetate (50 mL × 3), dried over anhydrous sodium sulfate, and concentrated under reduced pressure. The crude product was separated and purified with silica gel column chromatography (DCM/MeOH (v/v) = 20 : 1) to afford 90a (0.11 g, two-step yield from compound 83c: 61%).

LCMS m/z = 636.3 $[M+1]^+$.

Step 2: N-(2-chloro-4-(trifluoromethyl)phenyl)-1-(4-(1-(octahydrocyclopenta [c]pyrrol-5-yl)piperidin-4-yl)-1H-pyrazol-1-yl)cyclobutane-1-carboxamide (90b)

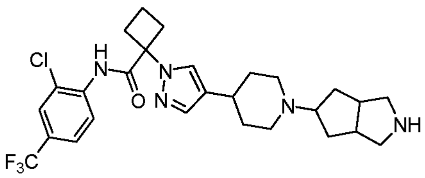

90a (0.11 g, 0.17 mmol) was dissolved in 2 mL of acetonitrile, 2 mL of a solution of 4 mol/L 1,4-dioxane hydrogen chloride solution was added, and the mixture was reacted at room temperature for 1 h. The reaction liquid was concentrated under reduced pressure to afford the hydrochloride of crude 90b (0.098 g).

LCMS m/z= 536.3 $[M+1]^+$.

Step 3: N-(2-chloro-4-(trifluoromethyl)phenyl)-1-(4-(1-(2-(2-(2,6-dioxopiperidin-3-yl)-1,3-dioxoisoindolin-5-yl)octahydrocyclopenta[c]pyrrol-5-yl)piperidin-4-yl)-1H-pyrazol-1-yl)cyclobutane-1-carboxamide (Compound 90)

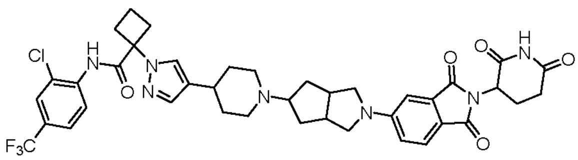

The hydrochloride of crude 90b (0.09 g) was dissolved in 5 mL of DMSO, 1C (0.088 g, 0.32 mmol), DIPEA (0.1 g, 0.8 mmol) and sodium bicarbonate (0.067 g, 0.8 mmol) were added in sequence, and nitrogen replacement was performed three times, the mixture was reacted at 80°C for 3 h. The reaction liquid was cooled to room temperature, 20 mL of water was added, and the mixture was extracted with ethyl acetate (50 mL × 3), the organic phase was dried over anhydrous sodium sulfate, concentrated under reduced pressure, and the crude product was separated and purified with silica gel column chromatography (DCM/MeOH (v/v) = 15 : 1) to afford compound 90 (30 mg, two-step yield from compound 90a: 24%).

$^1$H NMR (400 MHz, $CDCl_3$) δ 8.63 - 8.51 (m, 2H), 8.09 (s, 1H), 7.70 - 7.44 (m, 4H), 7.43 - 7.32 (m, 1H), 7.00 - 6.91 (m, 1H), 6.69 (dd, 1H), 5.00 - 4.87 (m, 1H), 3.70 - 3.30 (m, 4H), 3.25 - 2.96 (m, 4H), 2.94 - 2.65 (m, 8H), 2.64 - 2.44 (m, 1H), 2.40 - 1.55 (m, 13H).

LCMS m/z = 792.3 $[M+1]^+$.

**Example 91: Preparation of compound 91**

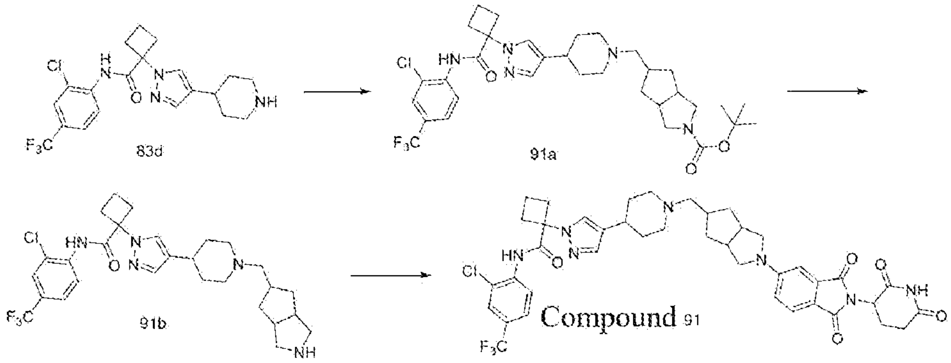

83d, 91a, 91b, Compound 91

Step 1: tert-butyl 5-((4-(1-(1-((2-chloro-4-(trifluoromethyl)phenyl)carbamoyl) cyclobutyl)-1H-pyrazol-4-yl)piperidin-1-yl)methyl)hexahydrocyclopenta[c]pyrrole -2(1H)-carboxylate (91a)

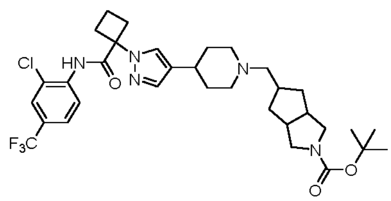

The hydrochloride of crude 83d (0.1 g) was dissolved in 10 mL of DMAc, sodium bicarbonate (24.3 mg, 0.289 mmol) was added, the mixture was reacted at room temperature for 20 min, then tert-butyl 5-formylhexahydrocyclopenta [c]pyrrole-2(1H)-carboxylate (0.083 g, 0.35 mmol) (see WO 2020224656 for the synthesis method), 0.1 mL of glacial acetic acid and 400 mg of anhydrous magnesium sulfate were added, the mixture was stirred at room temperature for 2 h, then sodium triacetoxyborohydride (0.146 g, 0.69 mmol) was added, and the mixture was reacted at room temperature for 2 h. 50 mL of saturated aqueous sodium bicarbonate solution was added to the reaction liquid, the mixture was extracted with ethyl acetate (50 mL × 3), dried over anhydrous sodium sulfate, and concentrated under reduced pressure. The crude product was separated and purified with silica gel column chromatography (DCM/MeOH (v/v) = 20 : 1) to afford 91a (0.1 g, two-step yield from compound 83c: 54%).

LCMS m/z = 650.3 $[M+1]^+$.

Step 2: N-(2-chloro-4-(trifluoromethyl)phenyl)-1-(4-(1-((octahydrocyclopenta [c]pyrrol-5-yl)methyl)piperidin-4-yl)-1H-pyrazol-1-yl)cyclobutane-1-carboxamide (91b) hydrochloride

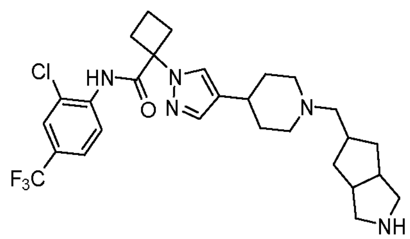

91a (0.1 g, 0.154 mmol) was dissolved in 2 mL of acetonitrile, 2 mL of a solution of 4 mol/L 1,4-dioxane hydrogen chloride solution was added, and the mixture was reacted at room temperature for 1 h. The reaction liquid was concentrated under reduced pressure to afford the hydrochloride of crude 91b (0.08 g).

LCMS m/z = 550.3 $[M+1]^+$.

Step 3: N-(2-chloro-4-(trifluoromethyl)phenyl)-1-(4-(1-((2-(2-(2,6-dioxopiperidin -3-yl)-1,3-dioxoisoindolin-5-yl)octahydrocyclopenta[c]pyrrol-5-yl)methyl)piperidin-4-yl)-1H-pyrazol-1-yl)cyclobutane-1-carboxamide (Compound 91)

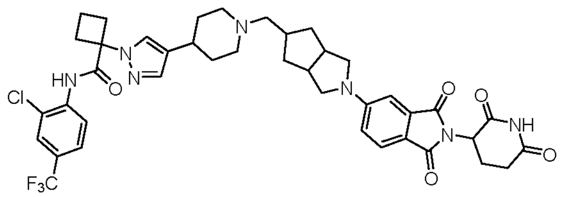

The hydrochloride of crude 91b (0.080 g) was dissolved in 5 mL of DMSO, 1C (0.083 g, 0.30 mmol), DIPEA (0.097 g, 0.75 mmol) and sodium bicarbonate (0.063 g, 0.75 mmol) were added in sequence, and nitrogen replacement was performed three times, the mixture was reacted at 80°C for 3 h. The reaction liquid was cooled to room temperature, 20 mL of water was added, and the mixture was extracted with ethyl acetate (50 mL × 3), the organic phase was dried over anhydrous sodium sulfate, concentrated under reduced pressure, and the crude product was separated and purified with silica gel column chromatography (DCM/MeOH (v/v) = 15 : 1) to afford compound 91 (25 mg, two-step yield from compound 91a: 20%).

$^1H$ NMR (400 MHz, $CDCl_3$) δ 8.64 - 8.48 (m, 2H), 8.04 (s, 1H), 7.70 - 7.44 (m, 4H), 7.42 - 7.32 (m, 1H), 6.99 - 6.92 (m, 1H), 6.69 (dd, 1H), 4.98 - 4.88 (m, 1H), 3.68 - 3.50 (m, 2H), 3.36 - 3.15 (m, 2H), 3.12 - 2.64 (m, 12H), 2.60 - 1.60 (m, 16H).

LCMS m/z= 806.3 $[M+1]^+$.

**Example 92: Preparation of compound 92**

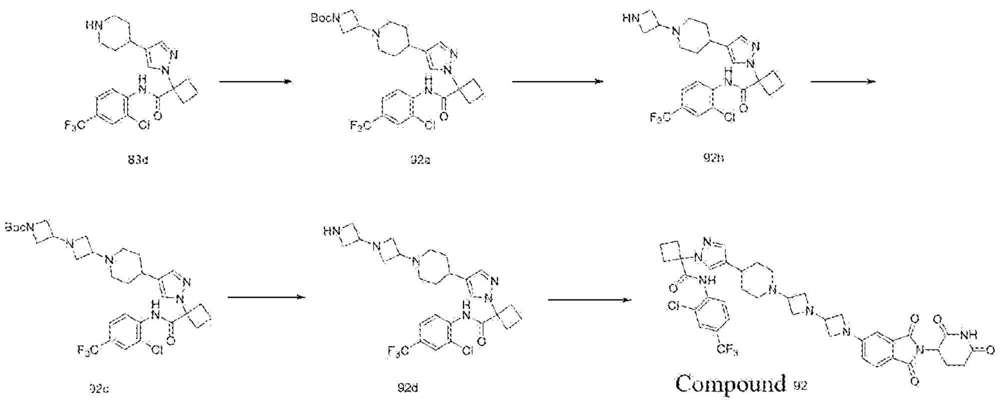

83d 92a 92b

92c 92d Compound 92

Step 1: tert-butyl 3-(4-(1-(1-((2-chloro-4-(trifluoromethyl)phenyl)carbamoyl) cyclobutyl)-1H-pyrazol-4-yl)piperidin-1-yl)azetidine-1-carboxylate (92a)

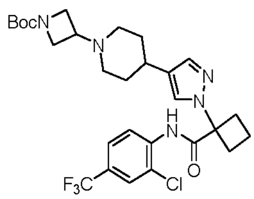

The hydrochloride of crude 83d (300 mg) was dissolved in 15 mL of DMAc, sodium bicarbonate (73 mg, 0.869 mmol) was added, the mixture was reacted at room temperature for 20 min, and then tert-butyl 3-oxoazetidine-1-carboxylate (240 mg, 1.40 mmol) and 0.1 mL of acetic acid were added, the mixture was stirred at room temperature for 1 h, then sodium triacetoxyborohydride (443 mg, 2.09 mmol) was added, and the mixture was reacted at room temperature for 1 h. 20 mL of saturated aqueous sodium bicarbonate solution was slowly added to the reaction liquid, the mixture was extracted with ethyl acetate (40 mL × 3), the organic phase was washed with 50 mL of water, dried over anhydrous sodium sulfate, and concentrated under reduced pressure. The crude product was separated and purified with silica gel column chromatography (DCM/MeOH (v/v) = 20 : 1) to afford 92a (0.30 g, two-step yield from compound 83c: 61%).

LCMS m/z= 582.3 $[M+1]^+$.

Step 2: 1-(4-(1-(azetidin-3-yl)piperidin-4-yl)-1H-pyrazol-1-yl)-N-(2-chloro-4-(trifluoromethyl)phenyl)cyclobutane-1-carboxamide (92b)

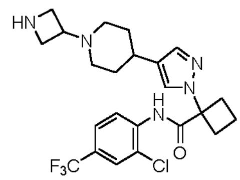

92a (0.3 g, 0.515 mmol) was dissolved in 10 mL of DCM, and 6 mL of trifluoroacetic acid was added. The mixture was reacted at room temperature for 3 h. The reaction system was concentrated under reduced pressure. The residue was dissolved in 30 mL of 4 mol/L aqueous NaOH solution and the mixture was extracted with DCM (40 mL × 3). The organic phase was washed with 50 mL of water, dried over anhydrous sodium sulfate, and concentrated under reduced pressure to afford crude 92b (0.20 g).

LCMS m/z = 482.3 $[M+1]^+$.

Step 3: tert-butyl 3-(4-(1-(1-((2-chloro-4-(trifluoromethyl)phenyl)carbamoyl) cyclobutyl)-1H-pyrazol-4-yl)piperidin-1-yl)-[1,3'-biazetidine]-1'-carboxylate (92c)

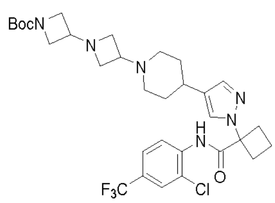

Crude 92b (200 mg) was dissolved in 15 mL of DMAc, tert-butyl 3-oxoazetidine-1-carboxylate (140 mg, 0.82 mmol) and 0.1 mL of acetic acid were added, the mixture was stirred at room temperature for 1 h, then sodium triacetoxyborohydride (260 mg, 1.23 mmol) was added, and the mixture was reacted at room temperature for 1 h. 20 mL of saturated aqueous sodium bicarbonate solution was slowly added to the reaction liquid, the mixture was extracted with ethyl acetate (40 mL × 3), the organic phase was washed with 50 mL of water, dried over anhydrous sodium sulfate, and concentrated under reduced pressure. The crude product was separated and purified with silica gel column chromatography (DCM/MeOH (v/v) = 20 : 1) to afford 92c (0.20 g, two-step yield from compound 92a: 61%).

LCMS m/z = 637.3 $[M+1]^+$.

Step 4: 1-(4-(1-([1,3'-biazetidin]-3-yl)piperidin-4-yl)-1H-pyrazol-1-yl)-N-(2-chloro-4-(trifluoromethyl)phenyl)cyclobutane-1-carboxamide (92d)

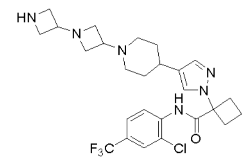

92c (0.2 g, 0.314 mmol) was dissolved in 10 mL of DCM, and 6 mL of trifluoroacetic acid was added. The mixture was reacted at room temperature for 3 h. The reaction system was concentrated under reduced pressure. The residue was dissolved in 30 mL of 4 mol/L aqueous NaOH solution and the mixture was extracted with DCM (40 mL × 3). The organic phase was washed with 50 mL of water, dried over anhydrous sodium sulfate, and concentrated under reduced pressure to afford crude 92d (0.15 g).

LCMS m/z= 537.3 $[M+1]^+$.

Step 5: N-(2-chloro-4-(trifluoromethyl)phenyl)-1-(4-(1-(1'-(2-(2,6-dioxopiperidin-3-yl)-1,3-dioxoisoindolin-5-yl)-[1,3'-biazetidin]-3-yl)piperidin-4-yl)-1H-pyrazol-1-yl)cyclobutane-1-carboxamide (Compound 92)

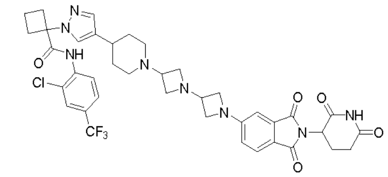

Crude 92d (100 mg) was dissolved in 10 mL of DMSO, DIPEA (74 mg, 0.57 mmol) and 1C (79 mg, 0.29 mmol) were added, and the mixture was stirred at 80°C for 5 h. The reaction liquid was cooled to room temperature, 20 mL of water was added, the mixture was filtered, and the filter cake was collected. The filter cake was washed with 10 mL of water, the filter cake was dissolved in 50 mL of DCM, dried over anhydrous sodium sulfate, and concentrated under reduced pressure. The crude product was separated and purified with silica gel column chromatography (DCM/MeOH (v/v) = 15 : 1) to afford compound 92 (60 mg, two-step yield from compound 92c: 36%).

$^1$H NMR (400 MHz, $CDCl_3$) δ 8.75 - 8.45 (m, 2H), 8.08 (s, 1H), 7.70 - 7.30 (m, 5H), 6.80 - 6.74 (m, 1H), 6.62 - 6.45 (m, 1H), 4.98 - 4.85 (m, 1H), 4.20 - 3.40 (m, 8H), 3.15 - 2.63 (m, 11H), 2.60 - 2.42 (m, 1H), 2.34 - 1.55 (m, 9H).

LCMS m/z = 793.3 $[M+1]^+$.

**Example 93: Preparation of compound 93**

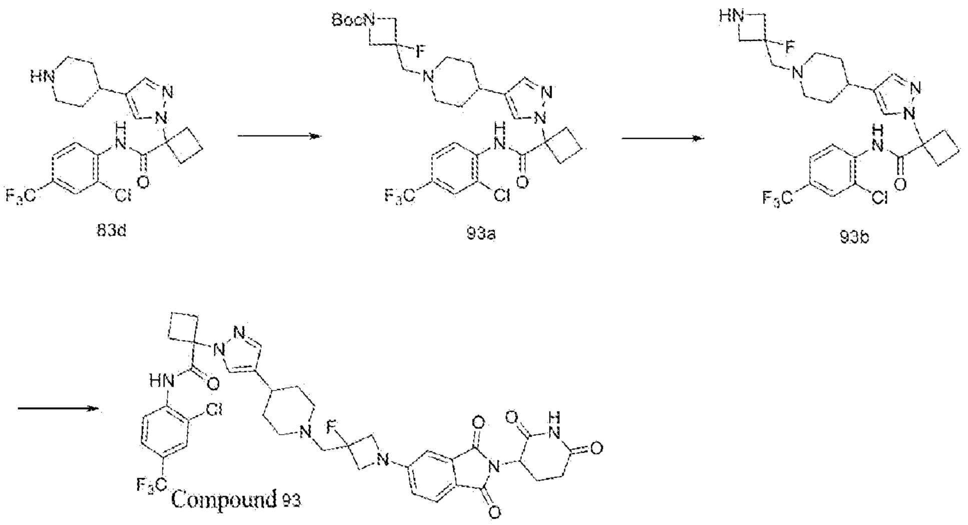

83d 93a 93b

Compound 93

Step 1: tert-butyl 3-((4-(1-(1-((2-chloro-4-(trifluoromethyl)phenyl)carbamoyl) cyclobutyl)-1H-pyrazol-4-yl)piperidin-1-yl)methyl)-3-fluoroazetidine-1-carboxylate (93a)

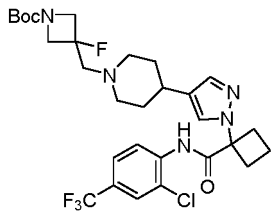

The hydrochloride of crude 83d (100 mg) was dissolved in 15 mL of DMAc, sodium bicarbonate (24.3 mg, 0.289 mmol) was added, the mixture was reacted at room temperature for 20 min, and then tert-butyl 3-fluoro-3-formylazetidine-1-carboxylate (300 mg, 1.476 mmol) (see WO 2018089355 for the synthesis method) and 0.1 mL of acetic acid were added, the mixture was stirred at room temperature for 1.0 h, then sodium triacetoxyborohydride (150 mg, 0.708 mmol) was added, and the mixture was reacted at room temperature for 1 h. 20 mL of saturated aqueous sodium bicarbonate solution was slowly added to the reaction liquid, the mixture was extracted with ethyl acetate (40 mL × 3), the organic phase was washed with 50 mL of water, dried over anhydrous sodium sulfate, and concentrated under reduced pressure. The crude product was separated and purified with silica gel column chromatography (DCM/MeOH (v/v) = 20 : 1) to afford 93a (0.10 g, two-step yield from compound 83c: 57%).

LCMS m/z = 614.2 $[M+1]^+$.

Step 2: N-(2-chloro-4-(trifluoromethyl)phenyl)-1-(4-(1-((3-fluoroazetidin-3-yl)methyl)piperidin-4-yl)-1H-pyrazol-1-yl)cyclobutane-1-carboxamide (93b)

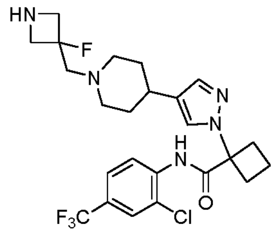

93a (0.1 g, 0.163 mmol) was dissolved in 10 mL of DCM, and 6 mL of trifluoroacetic acid was added. The mixture was reacted at room temperature for 3 h. The reaction system was concentrated under reduced pressure. The residue was dissolved in 30 mL of 4 mol/L aqueous NaOH solution and the mixture was extracted with DCM (40 mL × 3). The organic phase was washed with 50 mL of water, dried over anhydrous sodium sulfate, and concentrated under reduced pressure to afford crude 93b (0.07 g).

LCMS m/z= 514.2 $[M+1]^+$.

Step 3: N-(2-chloro-4-(trifluoromethyl)phenyl)-1-(4-(1-((1-(2-(2,6-dioxopiperidin-3-yl)-1,3-dioxoisoindolin-5-yl)-3-fluoroazetidin-3-yl)methyl) piperidin-4-yl)-1H-pyrazol-1-yl)cyclobutane-1-carboxamide (Compound 93)

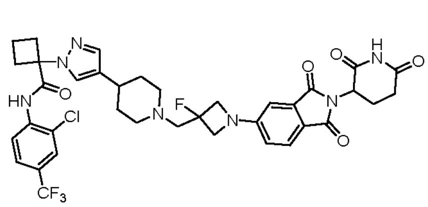

Crude 93b (70 mg) was dissolved in 10 mL of DMSO, DIPEA (54 mg, 0.42 mmol) and 1C (58 mg, 0.21 mmol)

were added, and the mixture was stirred at 80°C for 5 h. The reaction liquid was cooled to room temperature, 20 mL of water was added, the mixture was filtered, and the filter cake was collected. The filter cake was washed with 10 mL of water, the filter cake was dissolved in 50 mL of DCM, dried over anhydrous sodium sulfate, and concentrated under reduced pressure. The crude product was separated and purified with silica gel column chromatography (DCM/MeOH (v/v) = 15 : 1) to afford compound 93 (10 mg, two-step yield from compound 93a: 8%).

$^1$H NMR (400 MHz, $CDCl_3$) δ 8.70 - 8.50 (m, 2H), 7.99 (s, 1H), 7.74 - 7.32 (m, 5H), 6.90 - 6.80 (m, 1H), 6.70 - 6.50 (m, 1H), 4.99 - 4.87 (m, 1H), 4.45 - 3.95 (m, 4H), 3.18 - 2.63 (m, 11H), 2.60 - 2.43 (m, 1H), 2.43 - 2.00 (m, 5H), 2.00 - 1.58 (m, 4H).

LCMS m/z = 770.2 [M+1]$^+$.

**Example 94: Preparation of compound 94**

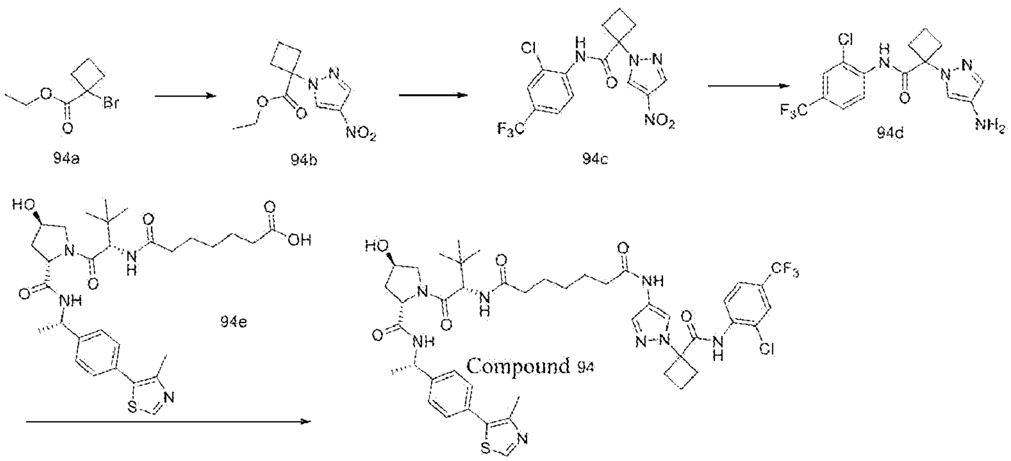

94a 94b 94c 94d 94e Compound 94

Step 1: ethyl 1-(4-nitro-1H-pyrazol-1-yl)cyclobutane-1-carboxylate (94b)

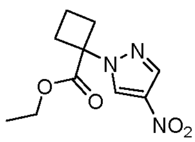

94a (12.6 g, 60.85 mmol) was added to 200 mL of acetonitrile, 4-nitro-1H-pyrazole (6.8 g, 60.13 mmol) and cesium carbonate (39 g, 119.7 mmol) were added, and the mixture was reacted at 80°C for 3 h. The reaction liquid was cooled to room temperature, concentrated under reduced pressure, and washed with 200 mL of ethyl acetate and 200 mL of water, the organic phase was separated, dried over anhydrous sodium sulfate and concentrated under reduced pressure, and the crude product was separated and purified with silica gel chromatography column (petroleum ether/ethyl acetate (v/v) = 4 : 1) to afford 94b (2.8 g, yield: 20%).

LCMS m/z = 240.1 [M+1]$^+$.

Step 2: N-(2-chloro-4-(trifluoromethyl)phenyl)-1-(4-nitro-1H-pyrazol-1-yl)cyclobutane-1-carboxamide (94c)

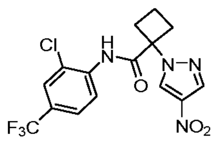

94b (2.8 g, 11.7 mmol) was added to 30 mL of methanol, 10 mL of water and lithium hydroxide monohydrate (1.68 g, 40.04 mmol) were added, and the mixture was reacted at room temperature for 3 h. The reaction liquid was concentrated under reduced pressure, the pH was adjusted to 5 with 2 mol/L hydrochloric acid, 50 mL of DCM was added for extraction, the organic phase was separated, dried over anhydrous sodium sulfate, and concentrated under reduced pressure to afford a crude product (2.4 g). The above crude product (2.4 g) was added to 100 mL of DCM, 1-chloro-N,N,2-trimethylpropenylamine (2.0 g, 15.0 mmol) and TEA (3.5 g, 34.6 mmol) were added, the mixture was stirred at room temperature for 1 h, then 11a (2.0 g, 10.23 mmol) was added and the mixture was reacted at room temperature for 16 h. The reaction liquid was concentrated under reduced pressure, and the crude product was separated and purified with silica gel chromatography column (petroleum ether/ethyl acetate (v/v) = 4 : 1) to afford 94c (0.8 g, yield: 20%).
LCMS m/z = 389.0 [M +1]$^+$.

Step 3: 1-(4-amino-1H-pyrazol-1-yl)-N-(2-chloro-4-(trifluoromethyl)phenyl) cyclobutane-1-carboxamide (94d)

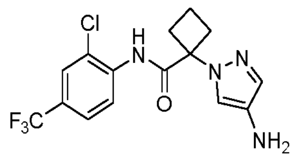

94c (0.3 g, 0.77 mmol) was dissolved in 9 mL of ethanol and 3 mL of water, reduced iron powder (260 mg, 4.64 mmol) and ammonium chloride (410 mg, 7.66 mmol) were added, and the mixture was reacted at 70°C for 2 h. The reaction liquid was cooled to room temperature and concentrated under reduced pressure, and the crude product was separated and purified with silica gel column chromatography (DCM/MeOH (v/v) = 20 : 1) to afford 94d (0.2 g, yield: 72%).
LCMS m/z = 359.1 [M+1]$^+$.

Step 4: N1-(1-(1-((2-chloro-4-(trifluoromethyl)phenyl)carbamoyl)cyclobutyl)-1H-pyrazol-4-yl)-N7-((S)-1-((2S,4R)-4-hydroxy-2-(((S)-1-(4-(4-methylthiazol-5-yl)phenyl)ethyl)carbamoyl)pyrrolidin-1-yl)-3,3-dimethyl-1-oxobutan-2-yl)heptanediamide (Compound 94)

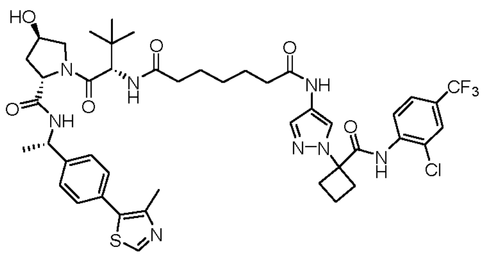

94d (0.1 g, 0.278 mmol) was dissolved in 10 mL of DMF, 94e (0.16 g, 0.273 mmol) (see WO 2021066873 for the synthesis method), DIPEA (72 mg, 0.56 mmol) and HATU (0.16 g, 0.42 mmol) were added, and the mixture was reacted at room temperature for 2 h. 10 mL of water was added to the reaction liquid, the mixture was extracted with ethyl acetate (40 mL × 3), the organic phase was washed with 50 mL of water, dried over anhydrous sodium sulfate, and concentrated under reduced pressure. The crude product was separated and purified with silica gel column chromatography (DCM/MeOH (v/v) = 10 : 1) to afford compound 94 (0.02 g, yield: 8%).
$^1$H NMR (400 MHz, DMSO-$d_6$) δ 10.1 - 9.98 (m, 1H), 9.04 - 8.84 (m, 2H), 8.40 - 8.22 (m, 2H), 8.15 - 8.08 (m, 1H), 7.95 - 7.85 (m, 1H), 7.80 - 7.65 (m, 3H), 7.48 - 7.30 (m, 4H), 5.13 - 5.05 (m, 1H), 4.99 - 4.85 (m, 1H), 4.57 - 4.36 (m, 2H), 4.34 - 4.20 (m, 1H), 3.65 - 3.54 (m, 2H), 3.00 - 2.83 (m, 2H), 2.83 - 2.63 (m, 2H), 2.48 - 2.42 (m, 3H), 2.30 - 1.90 (m, 7H), 1.85 - 1.72 (m, 1H), 1.65 - 1.18 (m, 9H), 0.98 - 0.86 (m, 9H).
LCMS m/z =927.3 [M+1]$^+$.

**Example 95: Preparation of compound 95**

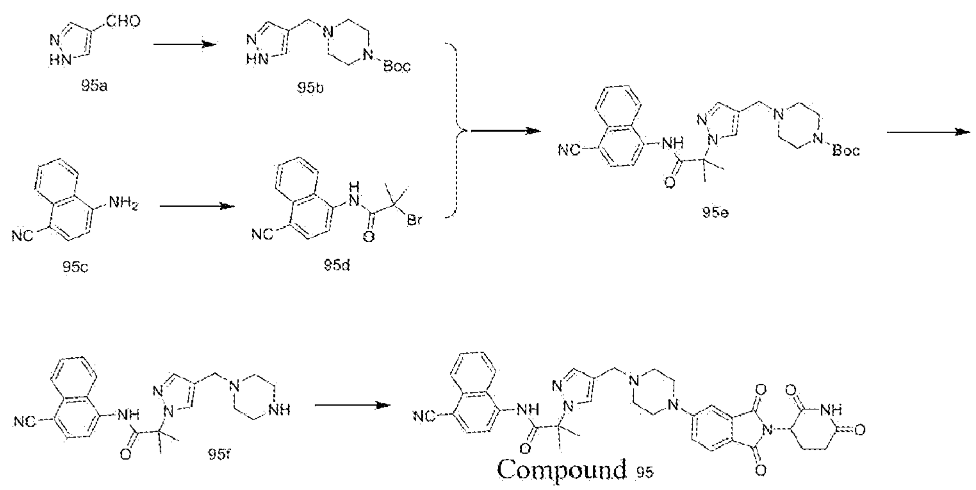

95a 95b 95c 95d 95e 95f Compound 95

Step 1: tert-butyl 4-((1H-pyrazol-4-yl)methyl)piperazine-1-carboxylate (95b)

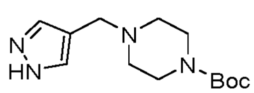

95a (1.5 g, 15.61 mmol) was dissolved in 30 ml of THF, tert-butyl piperazine-1-carboxylate (3.21 g, 17.23 mmol) and 1.8 mL of acetic acid were added, the mixture was stirred at room temperature for 40 min, then sodium triacetoxyborohydride (6.64 g, 31.33 mmol) was added, and the mixture was reacted at room temperature for 16 h. 120 mL of saturated aqueous sodium bicarbonate solution was slowly added to the reaction liquid, the mixture was extracted with DCM (80 mL × 3), the organic phase was washed with 50 mL of water, dried over anhydrous sodium sulfate, and concentrated under reduced pressure. The crude product was separated and purified with silica gel column chromatography (DCM/MeOH (v/v) = 15 : 1) to afford 95b (4.1 g, yield: 99%).

LCMS m/z = 267.3 $[M+1]^+$.

Step 2: 2-bromo-N-(4-cyanonaphthalen-1-yl)-2-methylpropanamide (95d)

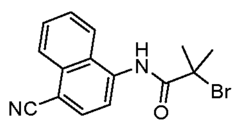

2-bromo-2-methylpropionic acid (2.38 g, 14.25 mmol) was dissolved in 60 mL of DCM, 1-chloro-N,N,2-trimethylpropenylamine (2.54 g, 19.05 mmol) was added, the mixture was stirred at room temperature for 2 h, and then 95d (2.0 g, 11.89 mmol) and TEA (3.61 g, 35.68 mmol) were added in sequence, and the mixture was reacted at room temperature for 16 h. 80 mL of ethyl acetate and 80 mL of aqueous sodium bicarbonate solution were added to the reaction liquid, the organic phase was separated, washed with 100 mL of saturated sodium chloride solution, dried over anhydrous sodium sulfate, and concentrated under reduced pressure. The crude product was separated and purified with silica gel chromatography column (petroleum ether/ethyl acetate (v/v) = 3 : 1) to afford 95d (1.4 g, yield: 37%).

LCMS m/z = 317.0 $[M+1]^+$.

Step 3: tert-butyl 4-((1-(1-((4-cyanonaphthalen-1-yl)amino)-2-methyl-1-oxopropan-2-yl)-1H- pyrazol-4-yl)methyl)piperazine-1-carboxylate (95e)

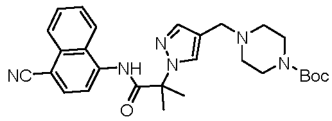

95d (0.32 g, 1.008 mmol) and 95b (0.32 g, 1.20 mmol) were dissolved in 30 mL of acetonitrile, cesium carbonate (0.66 g, 2.03 mmol) was added, and the mixture was reacted at 50°C for 3 h. The reaction liquid was cooled to room temperature and filtered, and the filtrate was concentrated under reduced pressure. The crude product was separated and purified with silica gel chromatography column (DCM/MeOH (v/v) = 15 : 1) to afford 95e (0.1 g, yield: 20%).
LCMS m/z= 503.2 $[M+1]^+$.

Step 4: N-(4-cyanonaphthalen-1-yl)-2-methyl-2-(4-(piperazin-1-ylmethyl)-1H-pyrazol-1-yl)propanamide (95f) hydrochloride

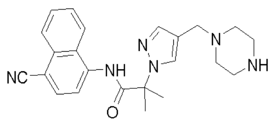

95e (0.1 g, 0.20 mmol) was dissolved in 2 mL of ethyl acetate and 2 mL of methanol, 10 mL of a solution of 4 mol/L ethyl acetate hydrogen chloride solution was added, and the mixture was reacted at room temperature for 2 h. The reaction system was concentrated under reduced pressure to afford the hydrochloride of crude 95f (0.11 g).
LCMS m/z = 403.3 $[M+1]^+$.

Step 5: N-(4-cyanonaphthalen-1-yl)-2-(4-((4-(2-(2,6-dioxopiperidin-3-yl)-1,3-dioxoisoindolin-5-yl)piperazin-1-yl)methyl)-1H-pyrazol-1-yl)-2-methylpropanamide (Compound 95)

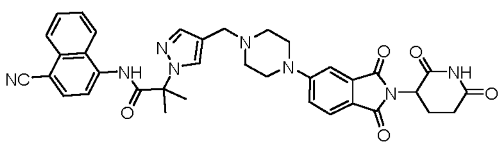

The hydrochloride of the crude 95f (0.11 g) was dissolved in 5 mL of DMSO, sodium bicarbonate (48 mg, 0.57 mmol) was added, the mixture was stirred at room temperature for 10 min, then 0.15 mL of TEA and 1C (0.10 g, 0.36 mmol) were added, the mixture was stirred at 80°C for 5 h. The reaction liquid was cooled to room temperature, 20 mL of water was added, the mixture was filtered, and the filter cake was collected. The filter cake was washed with 10 mL of water, the filter cake was dissolved in 50 mL of DCM, dried over anhydrous sodium sulfate, and concentrated under reduced pressure. The crude product was separated and purified with silica gel column chromatography (DCM/MeOH (v/v) = 15 : 1) to afford compound 95 (50 mg, two-step yield from compound 95e: 38%).
$^1H$ NMR (400 MHz, $CDCl_3$) δ 10.14 (s, 1H), 8.44 - 8.32 (m, 1H), 8.30 - 8.22 (m, 1H), 8.09 (s, 1H), 7.95 - 7.81 (m, 3H), 7.76 - 7.60 (m, 4H), 7.27 - 7.22 (m, 1H), 7.02 (dd, 1H), 5.00 - 4.86 (m, 1H), 3.70 - 3.30 (m, 6H), 2.95 - 2.52 (m, 7H), 2.18 - 2.08 (m, 1H), 2.03 (s, 6H).
LCMS m/z = 659.3 $[M+1]^+$.

**Example 96: Preparation of compound 96**

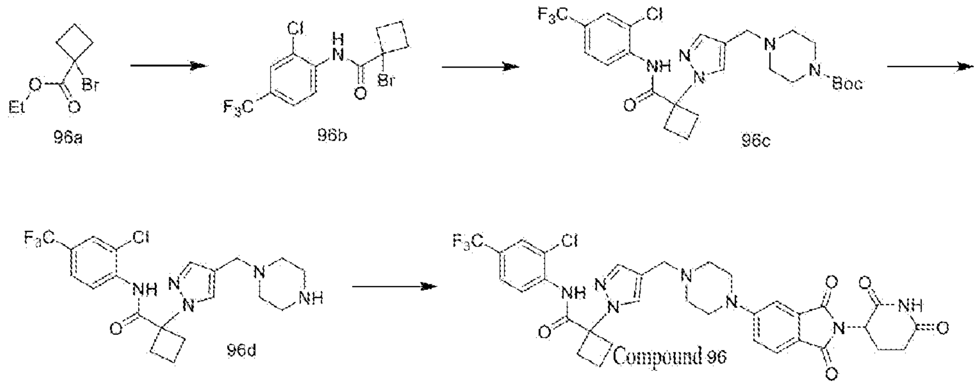

96a

96b

96c

96d

Compound 96

Step 1: 1-bromo-N-(2-chloro-4-(trifluoromethyl)phenyl)cyclobutane-1-carboxamide (96b)

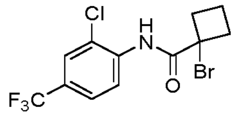

Ethyl 1-bromocyclobutane-1-carboxylate (5.00 g, 24.15 mmol), 2 mol/L aqueous sodium hydroxide solution (25 mL) and 25 mL of ethanol were added to a reaction flask, and the mixture was stirred at room temperature for 2 h. The reaction liquid was cooled to 0°C, 1 mol/L hydrochloric acid was added to adjust the pH to 2, the mixture was extracted with ethyl acetate (50 mL × 3), the organic phase was washed with 20 mL of saturated sodium chloride solution, dried over anhydrous sodium sulfate and concentrated under reduced pressure to afford a crude product (4.6 g). 1-chloro-N,N,2-trimethylpropenylamine (5.14 g, 38.55 mmol) and DCM (100 mL) were added to the above crude product (4.6 g), the mixture was stirred at room temperature for 1 h, then TEA (10.67 mL, 76.55 mmol) and 2-chloro-4-trifluoromethylaniline (5.02 g, 25.67 mmol) were added at room temperature for 16 h. The reaction liquid was concentrated under reduced pressure, and the crude product was separated and purified with silica gel chromatography column (ethyl acetate/petroleum ether (v/v) = 1 : 5) to afford 96b (6.05 g, yield: 70%).

Step 2: tert-butyl 4-((1-(1-((2-chloro-4-(trifluoromethyl)phenyl)carbamoyl) cyclobutyl)-1H- pyrazol-4-yl)methyl)piperazine-1-carboxylate (96c)

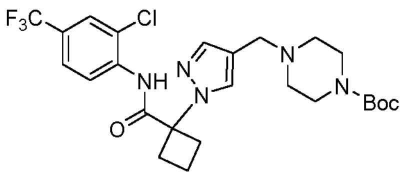

96b (0.36 g, 1.01 mmol) and 95b (0.32 g, 1.20 mmol) were dissolved in 30 mL of acetonitrile, cesium carbonate (0.66 g, 2.03 mmol) was added, and the mixture was reacted at 50°C for 3 h. The reaction liquid was cooled to room temperature and filtered, and the filtrate was concentrated under reduced pressure. The crude product was separated and purified with silica gel chromatography column (DCM/MeOH (v/v) = 15 : 1) to afford 96c (0.25 g, yield: 46%).

LCMS m/z = 542.2 $[M+1]^+$.

Step 3: N-(2-chloro-4-(trifluoromethyl)phenyl)-1-(4-(piperazin-1-ylmethyl)-1H-pyrazol-1-yl)cyclobutane-1-carboxamide (96d) hydrochloride

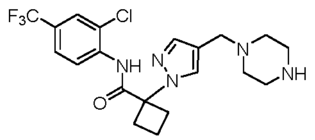

96c (0.125 g, 0.23 mmol) was dissolved in 2 mL of ethyl acetate and 2 mL of methanol, 10 mL of a solution of 4 mol/L ethyl acetate hydrogen chloride solution was added, and the mixture was reacted at room temperature for 2 h. The reaction system was concentrated under reduced pressure to afford the hydrochloride of crude 96d (0.11 g).
LCMS m/z = 442.1 $[M+1]^+$.

Step 4: N-(2-chloro-4-(trifluoromethyl)phenyl)-1-(4-((4-(2-(2,6-dioxopiperidin-3-yl)-1,3-dioxoisoindolin-5-yl)piperazin-1-yl)methyl)-1H-pyrazol-1-yl)cyclobutane-1-carboxamide (Compound 96)

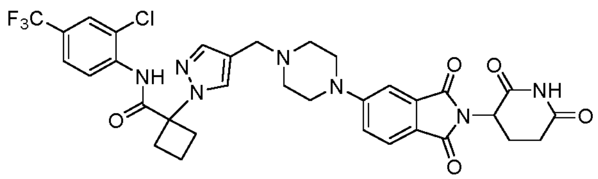

The hydrochloride of the crude 96d (0.1 g) was dissolved in 5 mL of DMSO, sodium bicarbonate (53 mg, 0.63 mmol) was added, the mixture was stirred at room temperature for 10 min, then 0.15 mL of TEA and 1C (0.12 g, 0.435 mmol) were added, the mixture was stirred at 80°C for 5 h. The reaction liquid was cooled to room temperature, 20 mL of water was added, the mixture was filtered, and the filter cake was collected and washed with 10 mL of water, the filter cake was dissolved in 50 mL of DCM, dried over anhydrous sodium sulfate, and concentrated under reduced pressure. The crude product was separated and purified with silica gel column chromatography (DCM/MeOH (v/v) = 15 : 1) to afford compound 96 (50 mg, two-step yield from compound 96c: 34%).
$^1H$ NMR (400 MHz, $CDCl_3$) δ 8.72 (s, 1H), 8.58 (d, 1H), 8.09 (s, 1H), 7.76 - 7.46 (m, 5H), 7.28 (d, 1H), 7.05 (dd, 1H), 5.00 - 4.87 (m, 1H), 3.70 - 3.32 (m, 6H), 3.17 - 3.00 (m, 2H), 2.97 - 2.47 (m, 9H), 2.37 - 2.02 (m, 3H).
LCMS m/z = 698.2 $[M+1]^+$.

**Example 97: Preparation of compound 97**

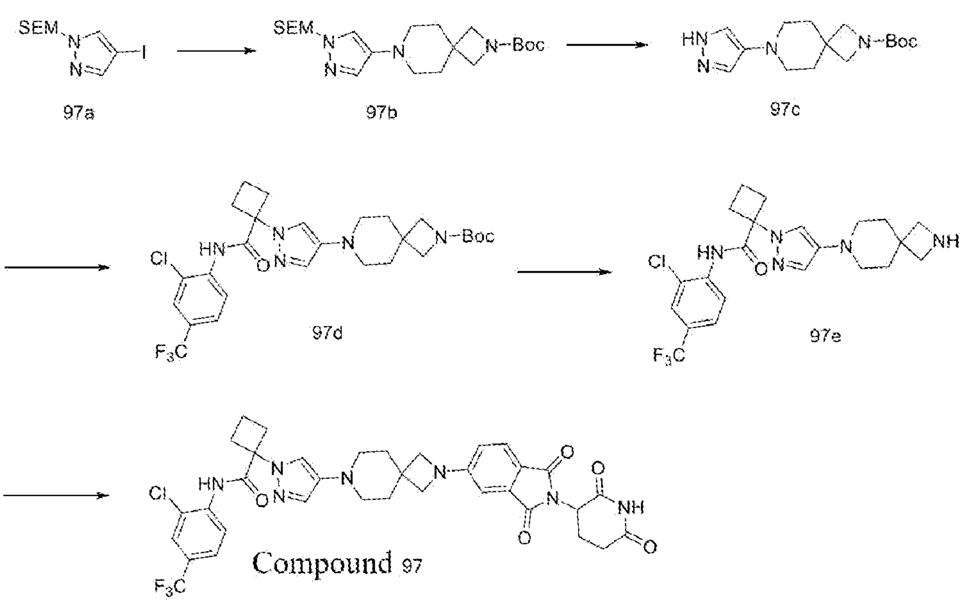

97a 97b 97c 97d 97e Compound 97

Step 1: tert-butyl 7-(1-((2-(trimethylsilyl)ethoxy)methyl)-1H-pyrazol-4-yl)-2,7-diazaspiro [3.5]nonane-2-carboxylate (97b)

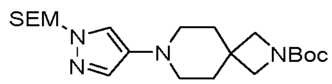

97a (see WO 2018114786 for the synthesis method) (350 mg, 1.079 mmol) and tert-butyl 2,7-diazaspiro[3.5]nonane-2-carboxylate (300 mg, 1.326 mmol) were dissolved in 3 mL of DMSO, CuI (30 mg, 0.16 mmol), L-proline (37 mg, 0.32 mmol) and potassium carbonate (370 mg, 2.68 mmol) were added in sequence under nitrogen protection, and the mixture was reacted at 100°C for 16 h. The reaction liquid was cooled to room temperature, 50 mL of water and 100 mL of ethyl acetate were added, the liquid separation was conducted, the organic phase was washed with 100 mL of saturated sodium chloride solution, dried over anhydrous sodium sulfate, and concentrated under reduced pressure. The crude product was separated and purified with silica gel chromatography column (petroleum ether/ethyl acetate (v/v) = 5 : 1-2 : 1) to afford 97b (380 mg, yield: 83%).

LCMS m/z = 423.3 $[M+1]^+$.

Step 2: tert-butyl 7-(1H-pyrazol-4-yl)-2,7-diazaspiro[3.5]nonane-2-carboxylate (97c)

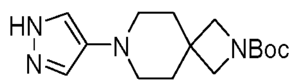

97b (330 mg, 0.78 mmol) was dissolved in 2 mL of THF, 2 mL of a solution of 1 mol/L TBAF in THF was added, and the mixture was reacted at 70°C for 3 h. The reaction liquid was cooled to room temperature, 100 mL of ethyl acetate and 20 mL of water were added, the liquid separation was conducted, the organic phase was washed with 50 mL of saturated sodium chloride solution, dried over anhydrous sodium sulfate, concentrated under reduced pressure, and the crude product was separated and purified with silica gel column chromatography (DCM/MeOH (v/v) = 20 : 1-10 : 1) to afford crude 97c (250 mg).

LCMS m/z = 293.3 $[M+1]^+$.

Step 3: tert-butyl 7-(1-(1-((2-chloro-4-(trifluoromethyl)phenyl)carbamoyl) cyclobutyl)-1H- pyrazol-4-yl)-2,7-diazaspiro[3.5]nonane-2-carboxylate (97d)

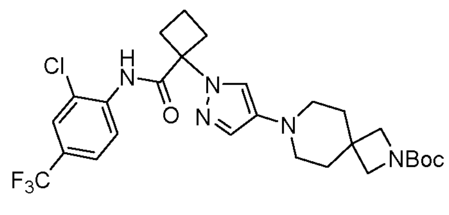

Crude 97c (200 mg) and 96b (360 mg, 1.01 mmol) were dissolved in 5 mL of acetonitrile, cesium carbonate (440 mg, 1.35 mmol) was added, and the mixture was reacted at 50°C for 5 h. The reaction liquid was cooled to room temperature, 50 mL of water and 100 mL of ethyl acetate were added, the liquid separation was conducted, the organic phase was washed with 100 mL of saturated sodium chloride solution, dried over anhydrous sodium sulfate, and concentrated under reduced pressure. The crude product was separated and purified with silica gel chromatography column (DCM/MeOH (v/v) = 20 : 1-5 : 1) to afford 97d (110 mg, yield: 19%).

LCMS m/z= 568.3 $[M+1]^+$.

Step 4: 1-(4-(2,7-diazaspiro[3.5]nonan-7-yl)-1H-pyrazol-1-yl)-N-(2-chloro-4-(trifluoromethyl)phenyl)cyclobutane-1-carboxamide (97e) trifluoroacetate

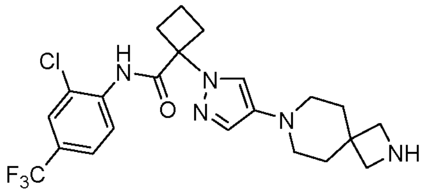

97d (110 mg, 0.19 mmol) was dissolved in DCM (2 mL), 1.5 mL of trifluoroacetic acid was added, and the mixture was reacted at room temperature for 2 h. The reaction system was concentrated under reduced pressure to afford the trifluoroacetate of crude 97e (160 mg).

LCMS m/z = 468.1 $[M+1]^+$.

Step 5: N-(2-chloro-4-(trifluoromethyl)phenyl)-1-(4-(2-(2-(2,6-dioxopiperidin-3-yl)-1,3-dioxoisoindolin-5-yl)-2,7-diazaspiro[3.5]nonan-7-yl)-1H-pyrazol-1-yl)cyclobutane-1-carboxamide (Compound 97) trifluoroacetate

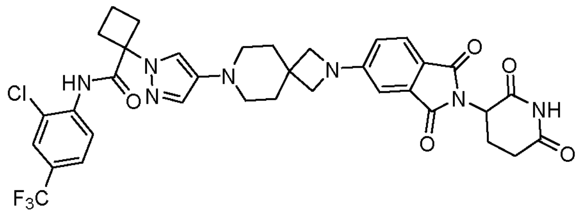

The trifluoroacetate of crude 97e (160 mg) and 1C (80 mg, 0.29 mmol) were dissolved in 1 mL of DMSO, DIPEA (0.16 mL, 0.92 mmol) and sodium bicarbonate (48 mg, 0.571 mmol) were added in sequence, the mixture was reacted at 80°C for 6 h. The reaction liquid was cooled to room temperature, 50 mL of water and 100 mL of ethyl acetate were added, the liquid separation was conducted, the organic phase was washed with 100 mL of saturated sodium chloride solution, dried over anhydrous sodium sulfate, and concentrated under reduced pressure. The crude product was purified with Prep-HPLC (instrument and preparative column: using WATERS 2767 preparative liquid phase chromatographic instrument, preparative column model: Xselect C18, 5 μm, inner diameter × length = 19 mm × 150 mm). Preparation method: the crude product was dissolved with methanol and dimethyl sulfoxide, and filtered with a 0.45 μm filter membrane, to prepare into a sample liquid. Mobile phase system: acetonitrile/water (containing 0.1% TFA). Gradient elution method: gradient elution of 5% to 50% acetonitrile (elution time 15 min), and lyophilization was performed to afford the trifluoroacetate of compound 97 (30 mg).

$^1H$ NMR (400 MHz, DMSO-$d_6$) δ 11.05 (s, 1H), 8.70 (s, 1H), 8.38 (d, 1H), 7.96 - 7.88 (m, 1H), 7.79 - 7.57 (m, 4H), 6.84 - 6.74 (m, 1H), 6.66 (dd, 1H), 5.13 - 4.98 (m, 1H), 3.81 (s, 4H), 3.05 - 2.66 (m, 9H), 2.65 - 2.44 (m, 2H), 2.14 - 1.78 (m, 7H).

LCMS m/z = 724.2 $[M+1]^+$.

**Example 98: Preparation of compound 98**

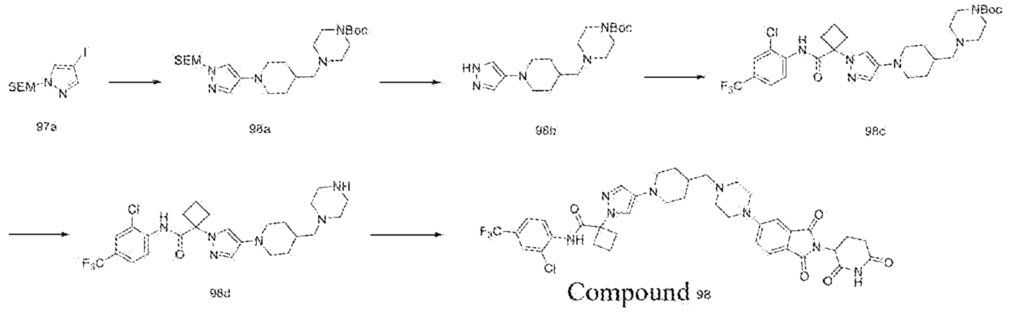

97a 98a 98b 98c 98d Compound 98

Step 1: tert-butyl 4-((1-(1-((2-(trimethylsilyl)ethoxy)methyl)-1H-pyrazol-4-yl)piperidin-4-yl)methyl)piperazine-1-carboxylate (98a)

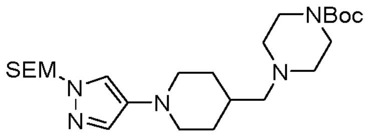

97a (see WO 2018114786 for the synthesis method) (1.0 g, 3.08 mmol) and tert-butyl 4-(piperidin-4-ylmethyl)piperazine-1-carboxylate (see WO 2020201080 for the synthesis method) (1.0 g, 3.53 mmol) were dissolved in 20 mL of DMSO, CuI (58 mg, 0.305 mmol), L-proline (71 mg, 0.62 mmol) and potassium carbonate (850 mg, 6.15 mmol) were added in sequence under nitrogen protection, and the mixture was reacted at 100°C for 16 h. The reaction liquid was cooled to room temperature, 100 mL of water and 200 mL of ethyl acetate were added, the liquid separation was conducted, the organic phase was washed with 100 mL of saturated sodium chloride solution, dried over anhydrous sodium sulfate, and concentrated under reduced pressure. The crude product was separated and purified with silica gel chromatography column (petroleum ether/ethyl acetate (v/v) = 6 : 1-2 : 1) to afford 98a (626 mg, yield: 42%).
LCMS m/z = 480.3 $[M+1]^+$.

Step 2: tert-butyl 4-((1-(1H-pyrazol-4-yl)piperidin-4-yl)methyl)piperazine-1-carboxylate (98b)

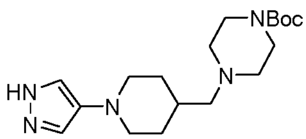

98a (600 mg, 1.25 mmol) was dissolved in 3 mL of THF, 3.5 mL of a solution of 1 mol/L TBAF in THF was added, and the mixture was reacted at 70°C for 3 h. The reaction liquid was cooled to room temperature, 100 mL of ethyl acetate and 40 mL of water were added, the liquid separation was conducted, the organic phase was washed with 50 mL of saturated sodium chloride solution, dried over anhydrous sodium sulfate, concentrated under reduced pressure, and the crude product was separated and purified with silica gel column chromatography (DCM/MeOH (v/v) = 20 : 1-10 : 1) to afford crude 98b (600 mg).
LCMS m/z = 350.3 $[M+1]^+$.

Step 3: tert-butyl 4-((1-(1-(1-((2-chloro-4-(trifluoromethyl)phenyl)carbamoyl) cyclobutyl)-1H- pyrazol-4-yl)piperidin-4-yl)methyl)piperazine-1-carboxylate (98c)

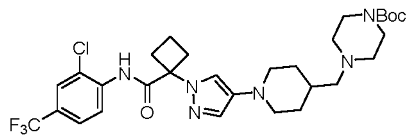

Crude 96b (760 mg, 2.13 mmol) was dissolved in 10 mL of acetonitrile, cesium carbonate (930 mg, 2.85 mmol) was added, and the mixture was reacted at 50°C for 5 h. The reaction liquid was cooled to room temperature, 50 mL of water and 100 mL of ethyl acetate were added, the liquid separation was conducted, the organic phase was washed with 100 mL of saturated brine, dried over anhydrous sodium sulfate, and concentrated under reduced pressure. The crude product was separated and purified with silica gel chromatography column (DCM/MeOH (v/v) = 20 : 1-5 : 1) to afford 98c (280 mg, yield: 21%).

LCMS m/z = 625.2 $[M+1]^+$.

Step 4: N-(2-chloro-4-(trifluoromethyl)phenyl)-1-(4-(4-(piperazin-1-ylmethyl) piperidin-1-yl)-1H-pyrazol-1-yl)cyclobutane-1-carboxamide (98d) trifluoroacetate

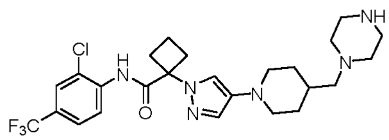

98c (280 mg, 0.45 mmol) was dissolved in DCM (2 mL), 3 mL of trifluoroacetic acid was added, and the mixture was reacted at room temperature for 2 h. The reaction system was concentrated under reduced pressure to afford the trifluoroacetate of crude 98d (500 mg).

LCMS m/z = 525.2 $[M+1]^+$.

Step 5: N-(2-chloro-4-(trifluoromethyl)phenyl)-1-(4-(4-((4-(2-(2,6-dioxopiperi din-3 -yl)-1,3 -dioxoisoindolin-5 -yl)piperazin-1 -yl)methyl)piperidin-1 - yl)-1H-pyrazol-1-yl)cyclobutane-1-carboxamide (Compound 98)

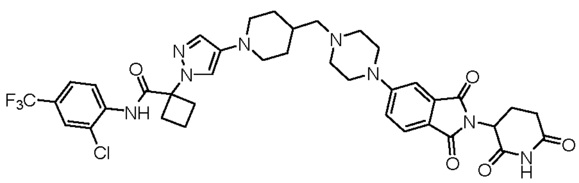

The trifluoroacetate of crude 98d (500 mg) and 1C (190 mg, 0.69 mmol) were dissolved in 3 mL of DMSO, DIPEA (0.37 mL, 2.12 mmol) and sodium bicarbonate (110 mg, 1.31 mmol) were added in sequence, the mixture was reacted at 80°C for 6 h. The reaction liquid was cooled to room temperature, 50 mL of water and 100 mL of ethyl acetate were added, the liquid separation was conducted, the organic phase was washed with 100 mL of saturated sodium chloride solution, dried over anhydrous sodium sulfate, and concentrated under reduced pressure. The crude product was purified with Prep-HPLC (instrument and preparative column: using WATERS 2767 preparative liquid phase chromatographic instrument, preparative column model: Xselect C18, 5 μm, inner diameter × length = 19 mm × 150 mm). Preparation method: the crude product was dissolved with methanol and dimethyl sulfoxide, and filtered with a 0.45 μm filter membrane, to prepare into a sample liquid. Mobile phase system: acetonitrile/water (containing 0.1% ammonium acetate). Gradient elution method: gradient elution of 5% to 50% acetonitrile (elution time: 15 min), lyophilization was performed to afford compound 98 (150 mg, two-step yield from compound 98c: 43%).

$^1H$ NMR (400 MHz, DMSO-$d_6$) δ 11.08 (br.s, 1H), 8.64 (br.s, 1H), 8.40 (d, 1H), 7.93 - 7.85 (m, 1H), 7.80 - 7.64 (m, 2H), 7.58 (s, 1H), 7.53 (s, 1H), 7.37 - 7.30 (m, 1H), 7.29 - 7.20 (m, 1H), 5.12 - 5.01 (m, 1H), 3.52 - 3.32 (m, 6H), 2.98 - 2.81 (m, 3H), 2.81 - 2.67 (m, 2H), 2.65 - 2.40 (m, 8H), 2.27 - 2.15 (m, 2H), 2.13 - 1.93 (m, 3H), 1.85 - 1.52 (m, 3H), 1.32 - 1.15 (m, 2H).

LCMS m/z= 781.2 $[M+1]^+$.

**Example 99: Preparation of the trifluoroacetate of compound 99**

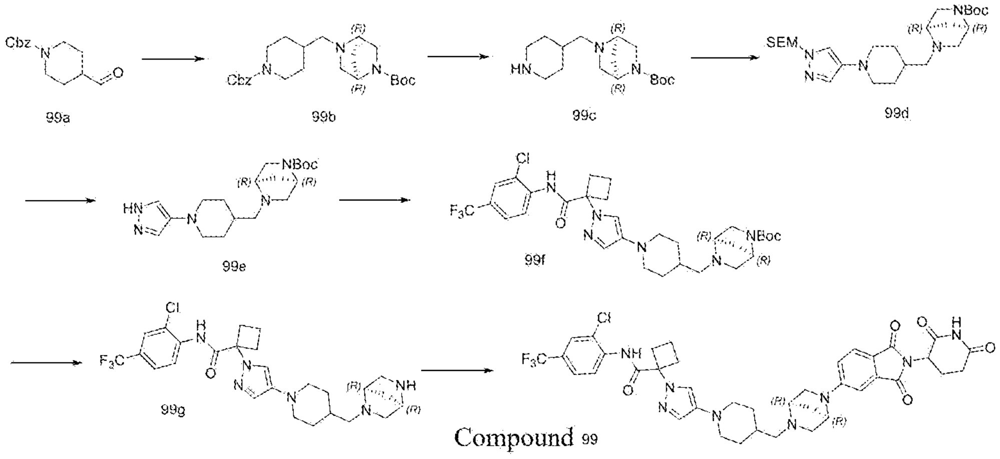

Step 1: tert-butyl (1R,4R)-5-((1-((benzyloxy)carbonyl)piperidin-4-yl)methyl)-2,5-diazabicyclo [2.2.1]heptane-2-carboxylate (99b)

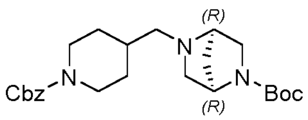

99a (1.00 g, 4.04 mmol) and tert-butyl (1R,4R)-2,5-diazabicyclo[2.2.1]heptane-2-carboxylate (0.80 g, 4.03 mmol) were dissolved in DCM (30 mL), glacial acetic acid (0.49 g, 8.167 mmol) was added dropwise at room temperature, then sodium triacetoxyborohydride (1.71 g, 8.07 mmol) was added, and the mixture was reacted at room temperature for 1 h. The pH of the reaction liquid was adjusted to 10 with 1 mol/L aqueous sodium hydroxide solution, the organic phase was separated, the aqueous phase was extracted with DCM (50 mL × 3), the organic phases were combined, dried over anhydrous sodium sulfate, and concentrated under reduced pressure. The crude product was separated and purified with silica gel chromatography column (pure ethyl acetate) to afford 99b (1.58 g, yield: 91%).

LCMS m/z =430.3 $[M+1]^+$.

Step 2: tert-butyl (1R,4R)-5-(piperidin-4-ylmethyl)-2,5-diazabicyclo[2.2.1] heptane-2-carboxylate (99c)

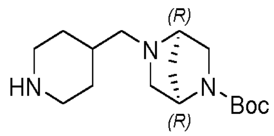

99b (1.58 g, 3.68 mmol) was dissolved in 30 mL of ethyl acetate, 10% palladium on carbon (0.79 g) was added, hydrogen replacement was performed three times, and the mixture was reacted at room temperature for 16 h under the atmosphere of hydrogen balloon. The reaction liquid was filtered through diatomaceous earth pad, the filter cake was washed with 20 mL of ethyl acetate, the filtrate was combined, and the filtrate was concentrated under reduced pressure to afford crude 99c (1.00 g).

LCMS m/z = 296.3 $[M+1]^+$.

Step 3: tert-butyl (1R,4R)-5-((1-(1-((2-(trimethylsilyl)ethoxy)methyl)-1H-pyrazol-4-yl) piperidin-4-yl)methyl)-2,5-diazabicyclo[2.2.1]heptane-2-carboxylate (99d)

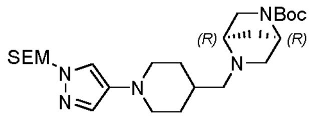

4-iodo-1-((2-(trimethylsilyl)ethoxy)methyl)-1H-pyrazole (525 mg, 1.62 mmol) and crude 99c (575 mg) were dissolved in 10 mL of DMSO, CuI (30 mg, 0.16 mmol), L-proline (37 mg, 0.32 mmol) and potassium carbonate (447 mg, 3.23 mmol) were added in sequence under nitrogen protection, and the mixture was reacted at 100°C for 16 h. The reaction liquid was cooled to room temperature, 100 mL of water was added, the mixture was extracted with ethyl acetate (100 mL × 3), the organic phases were combined, the organic phase was washed with 100 mL of saturated sodium chloride solution, dried over anhydrous sodium sulfate, and concentrated under reduced pressure. The crude product was separated and purified with silica gel chromatography column (MeOH/DCM (v/v) = 0 : 1-1 : 4) to afford 99d (613 mg, yield: 77%).

LCMS m/z = 492.4 $[M+1]^+$.

Step 4: tert-butyl (1R,4R)-5-((1-(1H-pyrazol-4-yl)piperidin-4-yl)methyl)-2,5-diazabicyclo[2.2.1] heptane-2-carboxylate (99e)

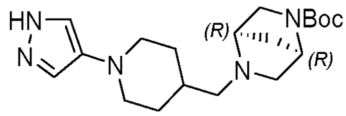

99d (548 mg, 1.11 mmol) was dissolved in 20 mL of THF, 11 mL of a solution of 1 mol/L TBAF in THF was added, and the mixture was reacted at 70°C for 3 h. The reaction liquid was cooled to room temperature and concentrated under reduced pressure, 100 mL of water was added, the mixture was extracted with ethyl acetate (20 mL × 3), the organic phases were combined, the organic phase was washed with 50 mL of saturated sodium chloride solution, dried over anhydrous sodium sulfate and concentrated under reduced pressure to afford crude 99e (398 mg).

LCMS m/z= 362.3 $[M+1]^+$.

Step 5: tert-butyl (1R,4R)-5-((1-(1-(1-((2-chloro-4-(trifluoromethyl)phenyl) carbamoyl)cyclobutyl)-1H-pyrazol-4-yl)piperidin-4-yl)methyl)-2,5-diazabicyclo [2.2.1]heptane-2-carboxylate (99f)

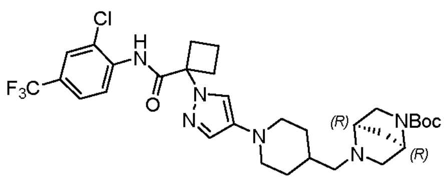

Crude 99e (384 mg) and 96b (567 mg, 1.59 mmol) were dissolved in 10 mL of acetonitrile, cesium carbonate (690 mg, 2.12 mmol) was added, and the mixture was reacted at 50°C for 3 h. The reaction liquid was cooled to room temperature and concentrated under reduced pressure, 50 mL of water was added to the residue, the mixture was extracted with ethyl acetate (100 mL × 3), the organic phases were combined, the organic phase was washed with 100 mL of saturated sodium chloride solution, dried over anhydrous sodium sulfate, and concentrated under reduced pressure. The crude product was separated and purified with silica gel chromatography column (MeOH/DCM (v/v) = 0 : 1-3 : 17) to afford 99f (154 mg, yield: 15%).

LCMS m/z = 637.3 $[M+1]^+$.

Step 6: 1-(4-(4-(((1R,4R)-2,5-diazabicyclo[22.1]heptan-2-yl)methyl)piperidin-1-yl)-1H-pyrazol-1-yl)-N-(2-chloro-4-(trifluoromethyl)phenyl)cyclobutane-1-carboxamide (99g) hydrochloride

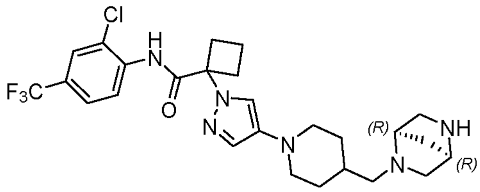

99f (133 mg, 0.21 mmol) was dissolved in 5 mL of DCM, 2 mL of a solution of 4 mol/L 1,4-dioxane hydrogen chloride solution was added, and the mixture was reacted at room temperature for 1 h. The reaction liquid was concentrated under reduced pressure to afford the hydrochloride of crude 99g (212 mg).

LCMS m/z= 537.3 $[M+1]^+$.

Step 7: N-(2-chloro-4-(trifluoromethyl)phenyl)-1-(4-(4-(((1R,4R)-5-(2-(2,6-dioxopiperidin-3-yl)-1,3-dioxoisoindolin-5-yl)-2,5-diazabicyclo[2.2.1]heptan-2-yl)methyl)piperidin-1-yl)-1H-pyrazol-1-yl)cyclobutane-1-carboxamide (Compound 99) trifluoroacetate

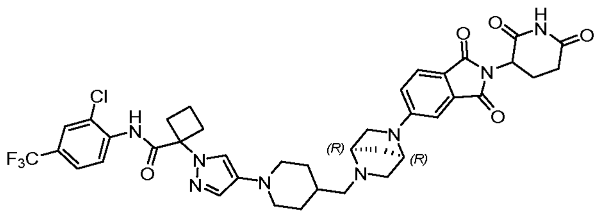

The hydrochloride of crude 99g (212 mg) and 1C (160 mg, 0.58 mmol) were dissolved in 5 mL of DMSO, DIPEA (250 mg, 1.93 mmol) and sodium bicarbonate (200 mg, 2.38 mmol) were added in sequence, and the mixture was reacted at 80°C for 6 h. The reaction liquid was cooled to room temperature, 50 mL of water was added, the mixture was extracted with ethyl acetate (100 mL × 3), the organic phases were combined, the organic phase was washed with 100 mL of saturated sodium chloride solution, dried over anhydrous sodium sulfate, and concentrated under reduced pressure. The crude product was subjected to Prep-HPLC (instrument and preparative column: using Glison GX-281 preparative liquid phase chromatographic instrument, preparative column model: Sunfire C18, 5 μm, inner diameter × length = 30 mm × 150 mm). Preparation method: the crude product was dissolved with methanol and dimethyl sulfoxide, and filtered with a 0.45 μm filter membrane, to prepare into a sample liquid. Mobile phase system: acetonitrile/water (containing 0.1% TFA). Gradient elution method: gradient elution of 5% to 60% acetonitrile (elution time 15 min), and lyophilization was performed to afford the trifluoroacetate of compound 99 (63 mg).

$^1H$ NMR (400 MHz, $CDCl_3$) δ 9.25 - 8.25 (m, 3H), 7.86 - 7.42 (m, 5H), 7.10 - 6.88 (m, 1H), 6.84 - 6.66 (m, 1H), 5.05 - 4.86 (m, 1H), 4.75 - 4.40 (m, 2H), 3.95 - 3.65 (m, 2H), 3.63 - 3.45 (m, 2H), 3.20 - 2.65 (m, 12H), 2.55 - 1.65 (m, 11H).

LCMS m/z= 793.3 $[M+1]^+$.

**Example 100: Preparation of the trifluoroacetate of compound 100**

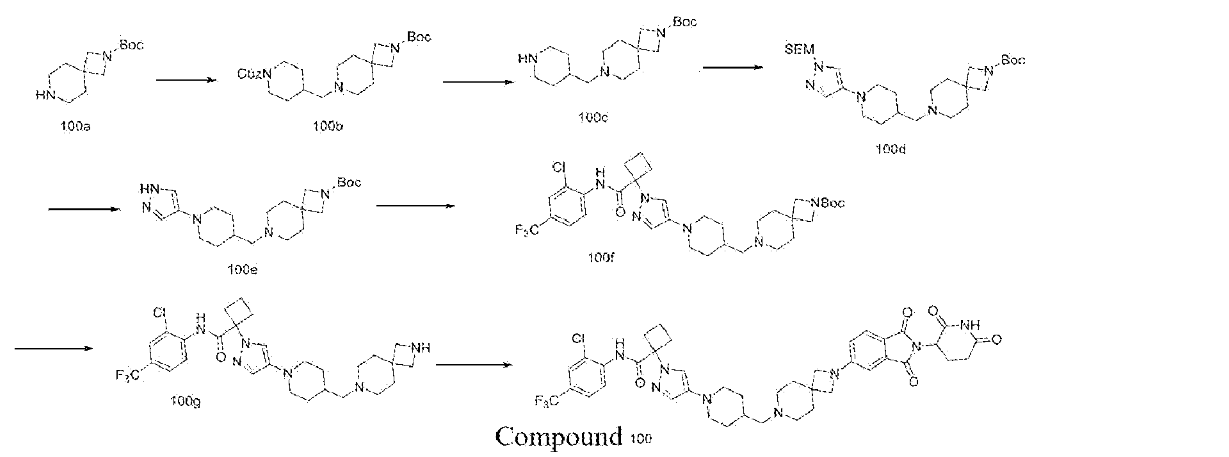

Compound 100

Step 1: tert-butyl 7-((1-((benzyloxy)carbonyl)piperidin-4-yl)methyl)-2,7-diazaspiro[3.5] nonane-2-carboxylate (100b)

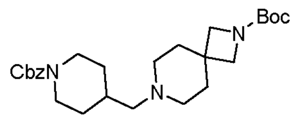

Benzyl 4-formylpiperidine-1-carboxylate (2.0 g, 8.09 mmol) and 100a (1.8 g, 7.95 mmol) were dissolved in 20 mL of anhydrous THF, sodium triacetoxyborohydride (2.57 g, 12.13 mmol) was added in batches under stirring at room temperature and the mixture was reacted at room temperature for 2 h. 10 mL of ice water was added to the reaction liquid, the mixture was concentrated under reduced pressure, 50 mL of water was added, the mixture was extracted with ethyl acetate (30 mL × 3), the organic phases were combined, the organic phase was washed with 50 mL of saturated sodium chloride solution, dried over anhydrous sodium sulfate, and concentrated under reduced pressure. The crude product was separated and purified with silica gel chromatography column (ethyl acetate/petroleum ether (v/v) = 1 : 4-7 : 3) to afford 100b (910 mg, yield: 25%).

LCMS m/z = 458.3 $[M+1]^+$.

Step 2: tert-butyl 7-(piperidin-4-ylmethyl)-2,7-diazaspiro[3.5]nonane-2-carboxylate (100c)

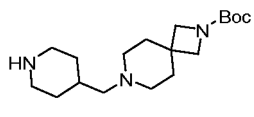

100b (890 mg, 1.94 mmol) was dissolved in 30 mL of methanol, 44.5 mg of 10% palladium on carbon was added, hydrogen replacement was performed three times, and the mixture was reacted at room temperature under hydrogen atmosphere for 16 h. The reaction liquid was filtered, and the filtrate was concentrated under reduced pressure to afford crude 100c (584 mg).

LCMS m/z= 324.3 $[M+1]^+$.

Step 3: tert-butyl 7-((1-(1-((2-(trimethylsilyl)ethoxy)methyl)-1H-pyrazol-4-yl)piperidin-4-yl) methyl)-2,7-diazaspiro[3.5]nonane-2-carboxylate (100d)

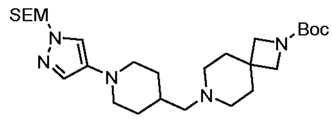

4-iodo-1-((2-(trimethylsilyl)ethoxy)methyl)-1H-pyrazole (see WO 2018114786 for the synthesis method) (454 mg, 1.40 mmol) and crude 100c (544 mg) were dissolved in 10 mL of DMSO, CuI (27 mg, 0.142 mmol), L-proline (32 mg, 0.28 mmol) and potassium carbonate (387 mg, 2.80 mmol) were added in sequence under nitrogen protection, and the mixture was reacted at 100°C for 16 h. The reaction liquid was cooled to room temperature, 100 mL of water was added, the mixture was extracted with 200 mL of ethyl acetate, the organic phases were combined, the organic phase was washed with 100 mL of saturated sodium chloride solution, dried over anhydrous sodium sulfate, and concentrated under reduced pressure. The crude product was separated and purified with silica gel chromatography column (MeOH/DCM (v/v) = 0 : 1-1 : 4) to afford 100d (145 mg, yield: 20%).

LCMS m/z= 520.3 $[M+1]^+$.

Step 4: tert-butyl 7-((1-(1H-pyrazol-4-yl)piperidin-4-yl)methyl)-2,7-diazaspiro[3.5] nonane-2-carboxylate (100e)

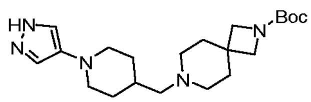

100d (145 mg, 0.28 mmol) was dissolved in 6 mL of THF, 2.8 mL of a solution of 1 mol/L TBAF in THF was added, and the mixture was reacted at 70°C for 2 h. The reaction liquid was cooled to room temperature and concentrated under reduced pressure, 20 mL of saturated sodium chloride solution was added, the mixture was extracted with DCM/MeOH (v/v) = 10 : 1 (20 mL × 2), the organic phases were combined, dried over anhydrous sodium sulfate, and concentrated under reduced pressure to afford crude 100e (1.27 g).

LCMS m/z = 390.3 $[M+1]^+$.

Step 5: tert-butyl 7-((1-(1-(1-((2-chloro-4-(trifluoromethyl)phenyl)carbamoyl) cyclobutyl)-1H-pyrazol-4-yl)piperidin-4-yl)methyl)-2,7-diazaspiro[3.5]nonane-2-carboxylate (100f)

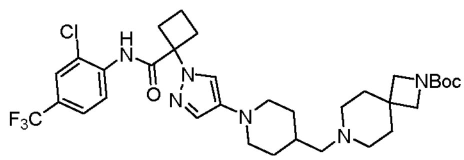

Crude 100e (1.27 g) and 96b (1.74 g, 4.88 mmol) were dissolved in 5 mL of acetonitrile, cesium carbonate (2.12 g, 6.51 mmol) was added, and the mixture was reacted at 50°C for 5 h. The reaction liquid was cooled to room temperature, 50 mL of water was added, the mixture was extracted with ethyl acetate (100 mL × 3), the organic phases were combined, the organic phase was washed with 100 mL of saturated sodium chloride solution, dried over anhydrous sodium sulfate, and concentrated under reduced pressure. The crude product was separated and purified with silica gel chromatography column (MeOH/DCM (v/v) = 0 : 1-3 : 17) to afford 100f (32 mg, yield: 1%).

LCMS m/z = 665.2 $[M+1]^+$.

Step 6: 1-(4-(4-((2,7-diazaspiro[3.5]nonan-7-yl)methyl)piperidin-1-yl)-1H-pyrazol-1-yl)-N-(2-chloro-4-(trifluoromethyl)phenyl)cyclobutane-1-carboxamide (100g) hydrochloride

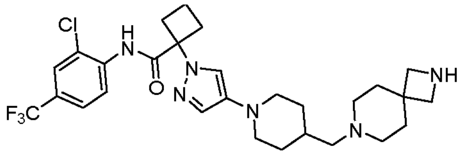

100f (32 mg, 0.048 mmol) was dissolved in DCM (2 mL), 1 mL of a solution of 4 mol/L 1,4-dioxane hydrogen chloride solution was added, and the mixture was reacted at room temperature for 1 h. The reaction liquid was concentrated under reduced pressure to afford the hydrochloride of crude 100g (36 mg).

LCMS m/z = 565.3 [M+1] $^+$.

Step 7: N-(2-chloro-4-(trifluoromethyl)phenyl)-1 -(4-(4-((2-(2-(2,6-dioxopiperidin -3-yl)-1,3-dioxoisoindolin-5-yl)-2,7-diazaspiro[3.5]nonan-7-yl)methyl)piperidin-1-yl)-1H-pyrazol-1-yl)cyclobutane-1-carboxamide (Compound 100) trifluoroacetate

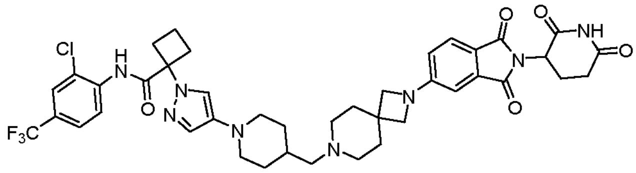

The hydrochloride of crude 100g (36 mg) and 1C (26 mg, 0.094 mmol) were dissolved in 3 mL of DMSO, DIPEA (41 mg, 0.32 mmol) and sodium bicarbonate (32 mg, 0.38 mmol) were added in sequence, and the mixture was reacted at 80°C for 6 h. The reaction liquid was cooled to room temperature, 50 mL of water was added, the mixture was extracted with 100 mL of ethyl acetate, the organic phases were combined, the organic phase was washed with 100 mL of saturated sodium chloride solution, dried over anhydrous sodium sulfate, and concentrated under reduced pressure. The crude product was subjected to Prep-HPLC (instrument and preparative column: using Glison GX-281 preparative liquid phase chromatographic instrument, preparative column model: Sunfire C18, 5 μm, inner diameter × length = 30 mm × 150 mm). Preparation method: the crude product was dissolved with methanol and dimethyl sulfoxide, and filtered with a 0.45 μm filter membrane, to prepare into a sample liquid. Mobile phase system: acetonitrile/water (containing 0.1% TFA). Gradient elution method: gradient elution of 5% to 60% acetonitrile (elution time 15 min), and lyophilization was performed to afford the trifluoroacetate of compound 100 (3.5 mg).

$^1$H NMR (400 MHz, $CDCl_3$) δ 8.55 - 8.40 (m, 2H), 7.94 (s, 1H), 7.72 - 7.40 (m, 5H), 6.80 - 6.68 (m, 1H), 6.54 - 6.44 (m, 1H), 4.95 - 4.80 (m, 1H), 3.78 (s, 4H), 3.72 - 3.44 (m, 4H), 3.10 - 2.57 (m, 13H), 2.55 - 1.80 (m, 12H).

LCMS m/z= 821.2 [M+1] $^+$.

**Example 101: Preparation of the trifluoroacetate of compound 101**

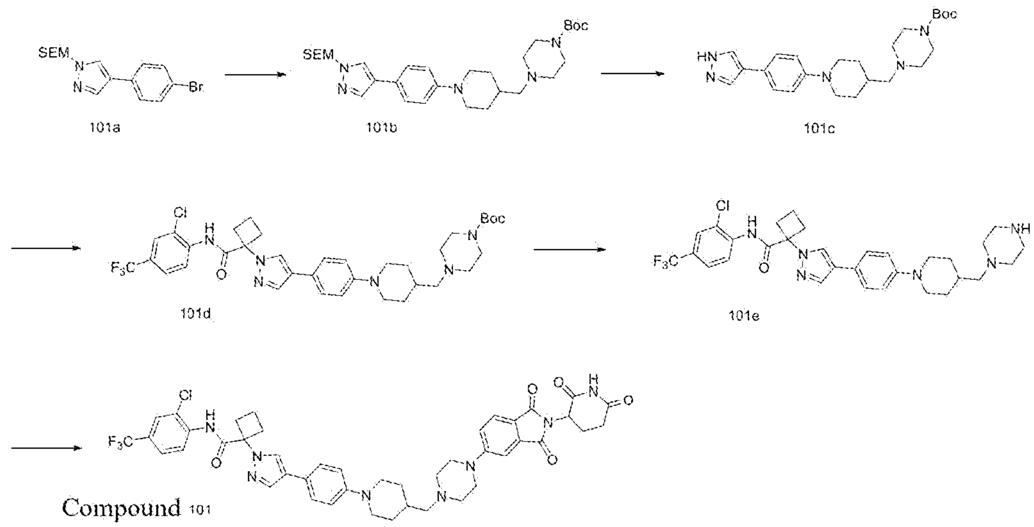

101a 101b 101c 101d 101e Compound 101

Step 1: tert-butyl 4-((1-(4-(1-((2-(trimethylsilyl)ethoxy)methyl)-1H-pyrazol-4-yl)phenyl) piperidin-4-yl)methyl)piperazine-1-carboxylate (101b)

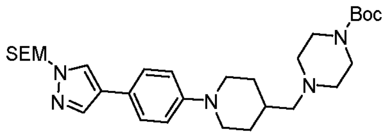

101a (335 mg, 0.95 mmol) (see WO 2011029027 for the synthesis method) and tert-butyl 4-(piperidin-4-ylmethyl)piperazine-1-carboxylate (296 mg, 1.04 mmol) were dissolved in 10 mL of toluene, $Pd_2(dba)_3$ (87 mg, 0.095 mmol), dave-phos (CAS: 213697-53-1) (37 mg, 0.094 mmol) and t-BuONa (228 mg, 2.37 mmol) were added in sequence, nitrogen replacement was performed three times, and the mixture was reacted at 100°C for 4 h. The reaction liquid was cooled to room temperature and concentrated under reduced pressure, 100 mL of water was added, the mixture was extracted with ethyl acetate (50 mL × 3), the organic phases were combined, the organic phase was washed with 100 mL of saturated sodium chloride solution, dried over anhydrous sodium sulfate, and concentrated under reduced pressure. The crude product was separated and purified with silica gel chromatography column (ethyl acetate/petroleum ether (v/v) = 1 : 4-3 : 2) to afford 101b (270 mg, yield: 47%).

LCMS m/z= 556.3 $[M+1]^+$.

Step 2: tert-butyl 4-((1-(4-(1H-pyrazol-4-yl)phenyl)piperidin-4-yl)methyl) piperazine-1-carboxylate (101c)

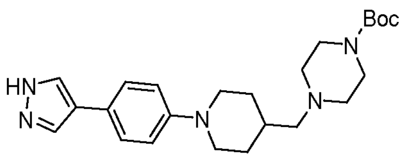

101b (262 mg, 0.47 mmol) was dissolved in THF (10 mL), 4.7 mL of a solution of 1 mol/L TBAF in THF was added, and the mixture was reacted at 70°C for 2 h. The reaction liquid was cooled to room temperature and concentrated under reduced pressure, 100 mL of water was added, the mixture was extracted with ethyl acetate (20 mL × 3), the organic phases were combined, the organic phase was washed with 50 mL of saturated sodium chloride solution, dried over anhydrous sodium sulfate and concentrated under reduced pressure to afford crude 101c (343 mg).

LCMS m/z= 426.3 $[M+1]^+$.

Step 3: tert-butyl 4-((1-(4-(1-(1-((2-chloro-4-(trifluoromethyl)phenyl) carbamoyl)cyclobutyl)-1H- pyrazol-4-yl)phenyl)piperidin-4-yl)methyl)piperazine-1-carboxylate (101d)

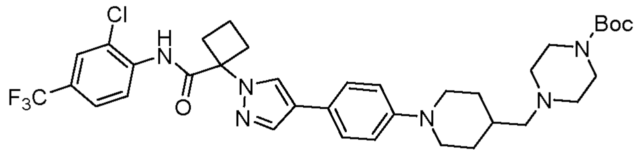

Crude 101c (333 mg) and 96b (417 mg, 1.17 mmol) were dissolved in 10 mL of acetonitrile, cesium carbonate (508 mg, 1.56 mmol) was added, and the mixture was reacted at 50°C for 5 h. The reaction liquid was cooled to room temperature, 50 mL of water was added, the mixture was extracted with ethyl acetate (100 mL × 3), the organic phases were combined, the organic phase was washed with 100 mL of saturated sodium chloride solution, dried over anhydrous sodium sulfate, and concentrated under reduced pressure. The crude product was separated and purified with silica gel chromatography column (MeOH/DCM (v/v) = 0 : 1-1 : 9) to afford 101d (89 mg, yield: 11%).
LCMS m/z= 701.2 [M+1] $^+$.

Step 4: N-(2-chloro-4-(trifluoromethyl)phenyl)-1-(4-(4-(4-(piperazin-1-ylmethyl)piperidin-1-yl)phenyl)-1H-pyrazol-1-yl)cyclobutane-1-carboxamide (101e) hydrochloride

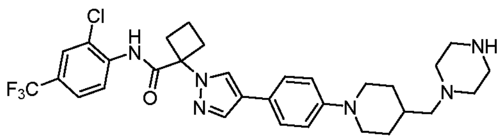

101d (78 mg, 0.11 mmol) was dissolved in DCM (2 mL), 1 mL of a solution of 4 mol/L 1,4-dioxane hydrogen chloride solution was added, and the mixture was reacted at room temperature for 1 h. The reaction liquid was concentrated under reduced pressure to afford the hydrochloride of crude 101e (77 mg).
LCMS m/z= 601.3 [M+1] $^+$.

Step 5: N-(2-chloro-4-(trifluoromethyl)phenyl)-1-(4-(4-(4-((4-(2-(2,6-dioxopiperidin-3 -yl)-1,3 -dioxoisoindolin-5 -yl)piperazin-1 -yl)methyl)piperidin-1 - yl)phenyl)-1H-pyrazol-1-yl)cyclobutane-1-carboxamide (Compound 101) trifluoroacetate

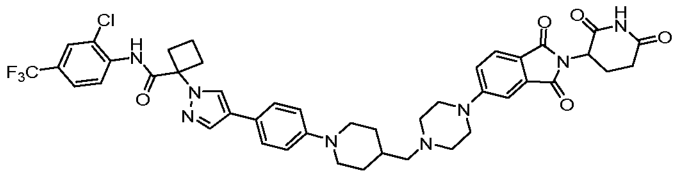

The hydrochloride of crude 101e (77 mg) and 1C (54 mg, 0.2 mmol) were dissolved in 5 ml of DMSO, DIPEA (84 mg, 0.65 mmol) and sodium bicarbonate (65 mg, 0.77 mmol) were added in sequence, and the mixture was reacted at 80°C for 6 h. The reaction liquid was cooled to room temperature, 50 mL of water was added, the mixture was extracted with ethyl acetate (100 mL × 3), the organic phases were combined, the organic phase was washed with 100 mL of saturated sodium chloride solution, dried over anhydrous sodium sulfate, and concentrated under reduced pressure. The crude product was subjected to Prep-HPLC (instrument and preparative column: using Glison GX-281 preparative liquid phase chromatographic instrument, preparative column model: Sunfire C18, 5 μm, inner diameter × length = 30 mm × 150 mm). Preparation method: the crude product was dissolved with methanol and dimethyl sulfoxide, and filtered with a 0.45 μm filter membrane, to prepare into a sample liquid. Mobile phase system: acetonitrile/water (containing 0.1% TFA). Gradient elution method: gradient elution of 5% to 60% acetonitrile (elution time 15 min), and lyophilization was performed to afford the trifluoroacetate of compound 101 (5.2 mg).

$^1$H NMR (400 MHz, $CDCl_3$) $\delta$ 8.76 (s, 1H), 8.56 (d, 1H), 8.05 - 7.26 (m, 11H), 7.18 - 7.02 (m, 1H), 5.07 - 4.85 (m, 1H), 4.00 - 2.60 (m, 23H), 2.54 - 2.34 (m, 1H), 2.32 - 2.00 (m, 5H).

LCMS m/z= 857.2 [M+1] $^+$.

**Example 102: Preparation of compound 102**

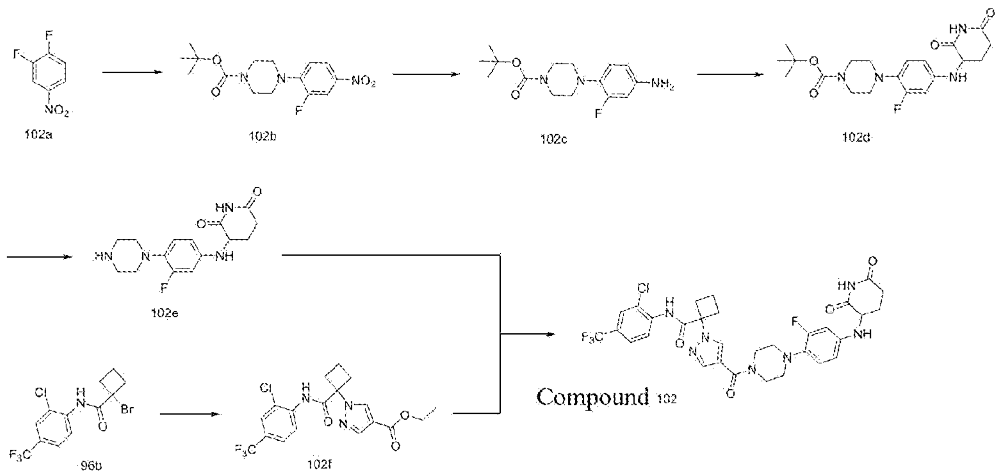


Step 1: tert-butyl 4-(2-fluoro-4-nitrophenyl)piperazine-1-carboxylate (102b)

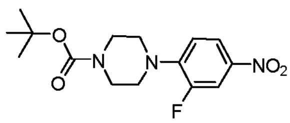

102a (1.0 g, 6.29 mmol) was dissolved in 20 mL of DMF, tert-butyl piperazine-1-carboxylate (1.16 g, 6.23 mmol) and cesium carbonate (3.05 g, 9.36 mmol) were added, and the mixture was reacted at 50°C under nitrogen protection for 16 h. The reaction system was cooled to room temperature and filtered, and the filtrate was poured into 50 mL of water. A large amount of yellow solid was precipitated, filtration was performed, and the filter cake was collected and dried to afford crude 102b (1.3 g).

Step 2: tert-butyl 4-(4-amino-2-fluorophenyl)piperazine-1-carboxylate (102c)

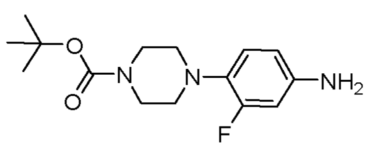

Crude 102b (1.3 g) was dissolved in 30 mL of ethyl acetate, 0.5 g of 10% palladium on carbon was added, and the mixture was reacted at room temperature for 3 h under the atmosphere of hydrogen balloon. The reaction system was filtered through diatomaceous earth pad, and the filtrate was concentrated under reduced pressure to afford crude 102c (1.1 g).

LCMS m/z = 296.1 [M+1]$^+$.

Step 3: tert-butyl 4-(4-((2,6-dioxopiperidin-3-yl)amino)-2-fluorophenyl) piperazine-1-carboxylate (102d)

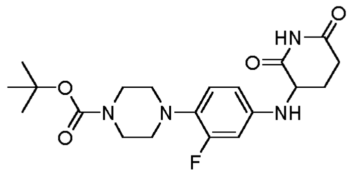

Crude 102c (1.1 g) was dissolved in 10 mL of DMF, sodium bicarbonate (1.88 g, 22.38 mmol) was added, 3-bromopiperidine-2,6-dione (2.15 g, 11.20 mmol) was added, and the mixture was reacted at 90°C for 16 h. The reaction liquid was cooled to room temperature and concentrated under reduced pressure, and the crude product was separated and purified with silica gel chromatography column (MeOH/DCM (v/v) = 3 : 97) to afford 102d (0.4 g, three-step yield from compound 102a: 16%).

LCMS m/z = 407.2 $[M+1]^+$.

Step 4: 3-((3-fluoro-4-(piperazin-1-yl)phenyl)amino)piperidine-2,6-dione (102e)

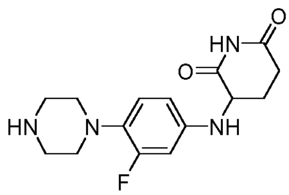

102d (0.3 g, 0.74 mmol) was dissolved in 2 mL of acetonitrile, 2 mL of a solution of 4 mol/L 1,4-dioxane hydrogen chloride solution was added, and the mixture was reacted at room temperature for 1 h. The reaction liquid was concentrated under reduced pressure, and the pH was adjusted to 9 with saturated aqueous sodium bicarbonate solution. The aqueous phase was separated and purified with reverse phase column chromatography (acetonitrile/water (v/v) = 0 : 1-3 : 7) to afford 102e (0.1 g, yield: 44%).

LCMS m/z = 307.2 $[M+1]^+$.

Step 5: ethyl 1-(1-((2-chloro-4-(trifluoromethyl)phenyl)carbamoyl) cyclobutyl)-1H-pyrazole-4- carboxylate (102f)

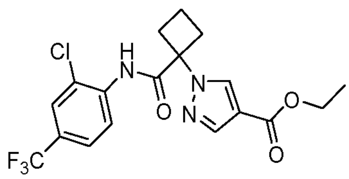

96b (2 g, 5.61 mmol), ethyl 1H-pyrazole-4-carboxylate (0.79 g, 5.64 mmol) and cesium carbonate (3.66 g, 11.23 mmol) were added to 20 mL of acetonitrile and the mixture was reacted at 50°C for 16 h. The reaction system was cooled to room temperature and filtered, and the filtrate was concentrated under reduced pressure. The crude product was separated and purified with silica gel chromatography column (petroleum ether/ethyl acetate (v/v) = 5 : 1) to afford 102f (1.4 g, yield: 60%).

Step 6: N-(2-chloro-4-(trifluoromethyl)phenyl)-1-(4-(4-(4-((2,6-dioxopiperidin-3-yl)amino)-2-fluorophenyl)piperazine-1-carbonyl)-1H-pyrazol-1-yl)cyclobutane-1-carboxamide (Compound 102)

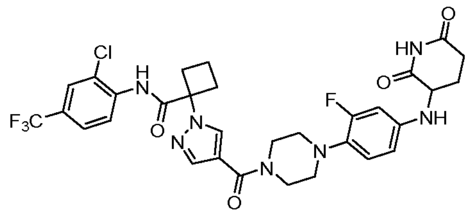

102f (1.0 g, 2.41 mmol) was dissolved in acetonitrile/water ((v/v) = 4 : 1) (20 mL), lithium hydroxide monohydrate (0.3 g, 7.15 mmol) was added, and the mixture was reacted at room temperature for 16 h. The reaction liquid was added to 50 mL of water, the mixture was extracted with 50 mL of ethyl acetate, the pH of the organic phase was adjusted to 6 with 1 mol/L hydrochloric acid, the mixture was extracted with 50 mL of ethyl acetate, the organic phase was dried over anhydrous sodium sulfate, and concentrated under reduced pressure to afford a crude product (0.1 g). The above crude product (0.03 g) and 102e (0.028 g, 0.091 mmol) were dissolved in 2 mL of DMF, N-methylimidazole (0.032 g, 0.39 mmol) and TCFH (0.065 g, 0.23 mmol) were added, and the mixture was reacted at room temperature for 2 h. 10 mL of water was added to the reaction system, the mixture was extracted with 20 mL of ethyl acetate, dried over anhydrous sodium sulfate, and concentrated under reduced pressure, and the crude product was separated and purified with silica gel chromatography column (DCM/MeOH (v/v) = 20 : 1) to afford compound 102 (12 mg, yield: 20%).

$^1$H NMR (400 MHz, $CDCl_3$) $\delta$ 8.82 (s, 1H), 8.56 (d, 1H), 8.00 - 7.82 (m, 3H), 7.64 - 7.46 (m, 2H), 7.14 - 6.85 (m, 1H), 6.53 - 6.32 (m, 2H), 4.95 - 4.35 (m, 1H), 4.08 - 3.78 (m, 5H), 3.20 - 2.66 (m, 10H), 2.60 - 2.45 (m, 1H), 2.34 - 2.02 (m, 2H), 2.00 - 1.82 (m, 1H).

LCMS m/z = 676.2 [M+1]$^+$.

**Example 103: Preparation of compound 103**

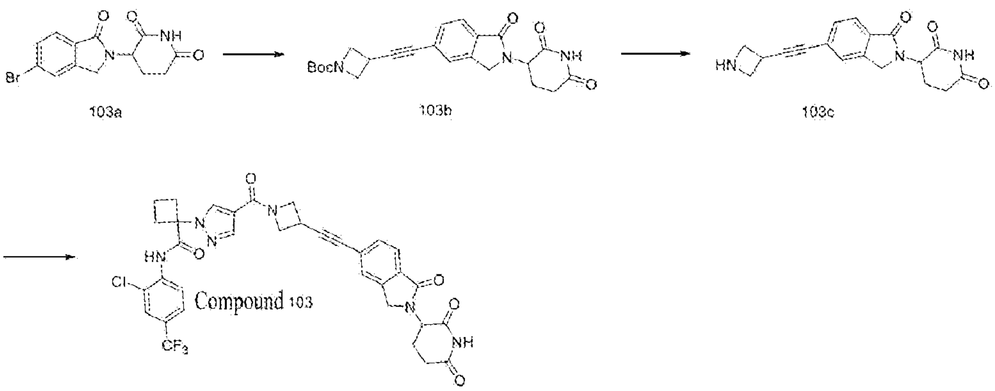

103a

103b

103c

Compound 103

Step 1: tert-butyl 3-((2-(2,6-dioxopiperidin-3-yl)-1-oxoisoindolin-5-yl)ethynyl) azetidine-1-carboxylate (103b)

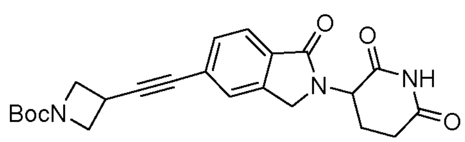

103a (360 mg, 1.11 mmol) was placed in a sealed tube, 10 mL of DMF was added, tert-butyl 3-ethynylazetidine-1-carboxylate (200 mg, 1.10 mmol), CuI (21 mg, 0.11 mmol), $PdCl_2(PPh_3)_2$ (78 mg, 0.11 mmol) and DIPEA (290 mg, 2.24 mmol) were added, nitrogen replacement was performed three times, and the mixture was reacted at 65°C for 3 h. The reaction was cooled to room temperature, 100 mL of water was added, the mixture was extracted with ethyl acetate (30 mL $\times$ 3), the organic phase was dried over anhydrous sodium sulfate, and concentrated under reduced pressure, and the crude product was separated and purified with silica gel column chromatography (DCM/MeOH (v/v) = 20 : 1) to afford 103b (170 mg, yield: 37%).

LCMS m/z = 424.1 $[M+1]^+$.

Step 2: 3-(5-(azetidin-3-ylethynyl)-1-oxoisoindolin-2-yl)piperidine-2,6-dione (103c)

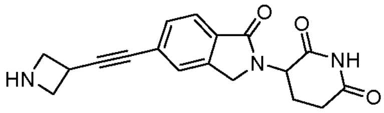

103b (400 mg, 0.94 mmol) was dissolved in DCM (2 mL), and 1 mL of trifluoroacetic acid was added. The mixture was reacted at room temperature for 3 h. The reaction system was concentrated under reduced pressure, and the crude product was separated and purified with reverse phase column chromatography (acetonitrile/water (v/v) = 5 : 95-30 : 70) and freeze-dried to afford 103c (300 mg, yield: 99%).

LCMS m/z = 324.1 $[M+1]^+$.

Step 3: N-(2-chloro-4-(trifluoromethyl)phenyl)-1-(4-(3-((2-(2,6-dioxopiperidin-3-yl)-1-oxoisoindolin-5-yl)ethynyl)azetidine-1-carbonyl)-1H-pyrazol-1-yl)cyclobutane-1-carboxamide (Compound 103)

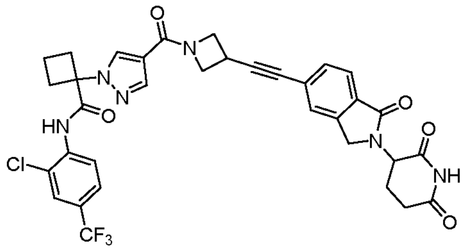

102f (1.0 g, 2.41 mmol) was dissolved in acetonitrile/water ((v/v) = 4 : 1) (20 mL), lithium hydroxide monohydrate (0.3 g, 7.15 mmol) was added, and the mixture was reacted at room temperature for 16 h. The reaction liquid was added to 50 mL of water, the mixture was extracted with 50 mL of ethyl acetate, the pH of the organic phase was adjusted to 6 with 1 mol/L hydrochloric acid, the mixture was extracted with 50 mL of ethyl acetate, the organic phase was dried over anhydrous sodium sulfate, and concentrated under reduced pressure to afford a crude product (0.1 g). The above crude product (36 mg) and 103c (0.03 g, 0.093 mmol) were dissolved in 2 mL of DMF, N-methylimidazole (15 mg, 0.18 mmol) and TCFH (39 mg, 0.14 mmol) were added, and the mixture was reacted at room temperature for 2 h. 20 mL of water was added to the reaction liquid, the mixture was extracted with ethyl acetate (20 mL × 3), dried over anhydrous sodium sulfate, and concentrated under reduced pressure. The crude product was separated and purified with silica gel chromatography column (DCM/MeOH (v/v) = 20 : 1) to afford compound 103 (12 mg, yield: 19%).

$^1H$ NMR (400 MHz, $CDCl_3$) δ 8.80 (s, 1H), 8.55 (d, 1H), 8.04 (s, 1H), 7.94 (s, 1H), 7.91 (s, 1H), 7.86 - 7.81 (m, 1H), 7.62 - 7.57 (m, 1H), 7.56 - 7.48 (m, 3H), 5.26 - 5.16 (m, 1H), 4.85 - 4.15 (m, 6H), 3.84 - 3.70 (m, 1H), 3.19 - 3.02 (m, 2H), 3.01 - 2.70 (m, 4H), 2.45 - 2.17 (m, 3H), 2.16 - 1.96 (m, 1H).

LCMS m/z = 693.2 $[M+1]^+$.

**Example 104: Preparation of compound 104**

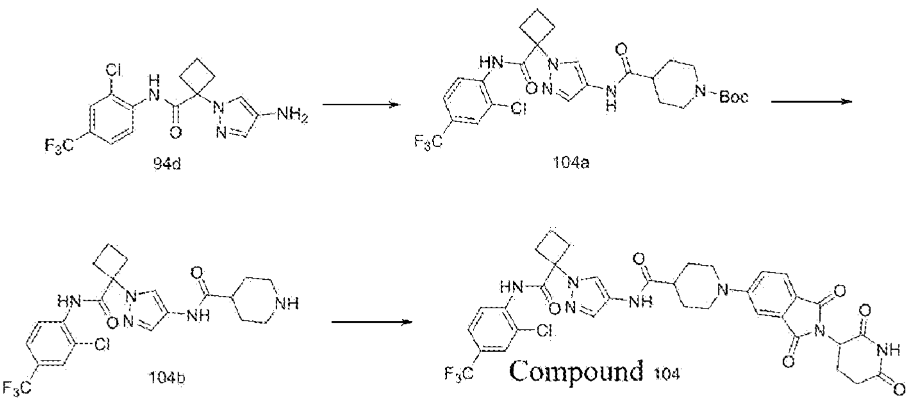


Step 1: tert-butyl 4-((1-(1-((2-chloro-4-(trifluoromethyl)phenyl)carbamoyl) cyclobutyl)-1H- pyrazol-4-yl)carbamoyl)piperidine-1-carboxylate (104a)

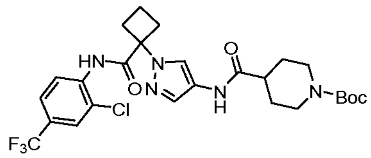

1-(tert-butoxycarbonyl)piperidine-4-carboxylic acid (0.1 g, 0.436 mmol) was dissolved in 20 mL of DCM, 1-chloro-N,N,2-trimethylpropenylamine (87.5 mg, 0.656 mmol) was added, the mixture was reacted at room temperature for 1 h, and then TEA (132.0 mg, 1.304 mmol) and 94d (140 mg, 0.39 mmol) were added in sequence, and the mixture was reacted at room temperature for 12 h. 30 mL of ethyl acetate and 10 mL of water were added to the reaction liquid, the organic phase was separated, washed with 20 mL of saturated sodium chloride solution, dried over anhydrous sodium sulfate, and concentrated under reduced pressure. The crude product was separated and purified with silica gel chromatography column (petroleum ether/ethyl acetate (v/v) = 1 : 1) to afford 104a (0.22 g, yield: 99%).

LCMS m/z = 570.2 $[M+1]^+$.

Step 2: N-(1-(1-((2-chloro-4-(trifluoromethyl)phenyl)carbamoyl)cyclobutyl)-1H-pyrazol-4-yl)piperidine-4-carboxamide (104b) hydrochloride

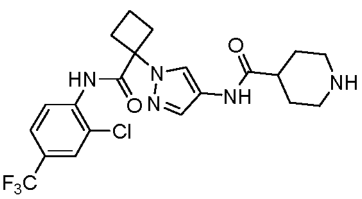

104a (0.22 g, 0.386 mmol) was dissolved in 5 mL of ethyl acetate, 10 mL of a solution of 4 mol/L ethyl acetate hydrogen chloride solution was added, and the mixture was reacted at room temperature for 2 h. The reaction liquid was concentrated under reduced pressure to afford the hydrochloride of crude 104b (170 mg).

LCMS m/z = 470.2 $[M+1]^+$.

Step 3: N-(1-(1-((2-chloro-4-(trifluoromethyl)phenyl)carbamoyl)cyclobutyl)-1H-pyrazol-4-yl)-1-(2-(2,6-dioxopiperidin-3-yl)-1,3-dioxoisoindolin-5-yl)piperidine-4-carboxamide (Compound 104)

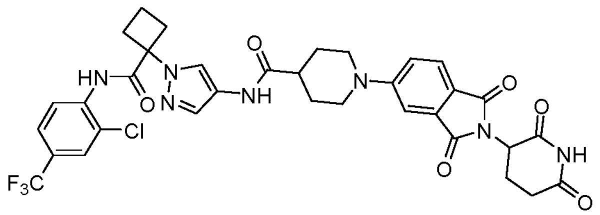

The hydrochloride of crude 104b (0.17 g) was dissolved in 6 mL of dry DMSO, sodium bicarbonate (28.3 mg, 0.337 mmol) was added, the mixture was stirred at room temperature for 20 min, then 1C (93.8 mg, 0.34 mmol) and DIPEA (103 mg, 0.797 mmol) were added in sequence, and the mixture was reacted at 60°C for 2 h. The reaction liquid was cooled to room temperature, 60 mL of water and 20 mL of DCM were added, the organic phase was separated, the aqueous phase was extracted with DCM (20 mL × 3), the organic phases were combined, washed with 50 mL of saturated sodium chloride solution, dried over anhydrous sodium sulfate, and concentrated under reduced pressure. The crude product was separated and purified with prep-TLC (petroleum ether/ethyl acetate (v/v) = 1 : 2) to afford compound 104 (80 mg, yield: 32%).

$^1H$ NMR (400 MHz, $CDCl_3$) δ 8.63 (s, 1H), 8.56 (d, 1H), 8.16 (s, 1H), 8.10 (s, 1H), 7.78 - 7.46 (m, 5H), 7.44 - 7.35 (m, 1H), 7.26 - 7.16 (m, 1H), 5.01 - 4.90 (m, 1H), 4.08 - 3.94 (m, 2H), 3.20 - 2.65 (m, 9H), 2.65 - 2.49 (m, 1H), 2.30 - 1.90 (m, 7H).

LCMS m/z = 726.2 $[M+1]^+$.

**Example 105: Preparation of compound 105**

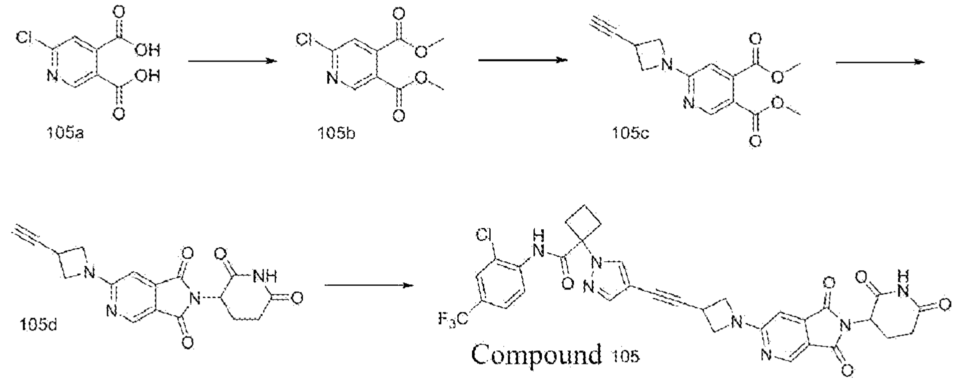

105a

105b

105c

105d

Compound 105

Step 1: dimethyl 6-chloropyridine-3,4-dicarboxylate (105b)

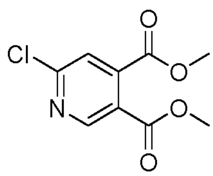

105a (5.0 g, 24.81 mmol) was dissolved in a mixed solvent of 50 mL of methanol and 50 mL of acetonitrile, DIPEA (6.4 g, 49.52 mmol) was added, and trimethylsilyldiazomethane (4.25 g, 37.21 mmol) was slowly added at room temperature, and the mixture was reacted at room temperature for 2 h. 30 mL of water was added to the reaction liquid, the mixture was extracted with ethyl acetate (60 mL × 2), the organic phases were combined, the organic phase was washed with saturated sodium chloride solution (30 mL × 2), dried over anhydrous sodium sulfate, and concentrated under reduced pressure. The crude product was separated and purified with silica gel chromatography column (petroleum ether/ethyl acetate (v/v) = 1 : 1) to afford 105b (1 g, yield: 18%).

Step 2: dimethyl 6-(3-ethynylazetidin-1-yl)pyridine-3,4-dicarboxylate (105c)

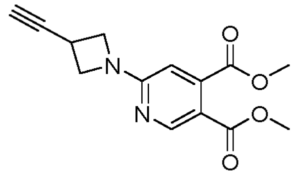

105b (1 g, 4.36 mmol) was dissolved in 10 mL of DMF, 3-ethynylazetidine hydrochloride (765.18 mg, 6.51 mmol) and DIPEA (1.68 g, 13.0 mmol) were added in sequence, and the mixture was reacted at 80°C for 3 h. The reaction liquid was cooled to room temperature, 80 mL of water was added, the mixture was extracted with ethyl acetate (50 mL × 2), the organic phases were combined, the organic phase was washed with saturated sodium chloride solution (20 mL × 2), dried over anhydrous sodium sulfate, and concentrated under reduced pressure. The crude product was separated and purified with silica gel chromatography column (petroleum ether/ethyl acetate (v/v) = 2 : 3) to afford 105c (0.3 g, yield: 25%).

LCMS m/z = 275.3 $[M+1]^+$.

Step 3: 2-(2,6-dioxopiperidin-3-yl)-6-(3-ethynylazetidin-1-yl)-1H-pyrrolo[3,4-c]pyridine-1,3 (2H)-dione (105d)

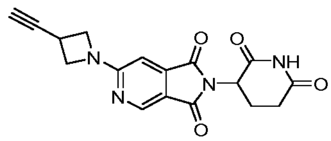

105c (0.3 g, 1.09 mmol) was dissolved in a mixed solvent of 10 mL of methanol and 5 mL of water, lithium hydroxide (130 mg, 5.43 mmol) was added, and the mixture was reacted at room temperature for 4 h. 1 mol/L hydrochloric acid was added to the reaction liquid to adjust the pH to 6, the mixture was concentrated under reduced pressure. The crude product was separated and purified with silica gel chromatography column (DCM/MeOH (v/v) = 10 : 1) to afford a crude product (0.24 g). The above crude product (0.24 g) was dissolved in 20 mL of acetic acid, then 3-aminopiperidine-2,6-dione hydrochloride (374.3 mg, 2.27 mmol) was added, and the mixture was reacted at 130°C for 4 h. The reaction liquid was cooled to room temperature, 15 mL of water was added, the mixture was extracted with ethyl acetate (30 mL × 2), the organic phases were combined, the organic phase was washed with saturated sodium chloride solution (20 mL × 2), dried over anhydrous sodium sulfate, and concentrated under reduced pressure. The crude product was separated and purified with silica gel chromatography column (DCM/MeOH (v/v) = 87 : 13) to afford 105d (0.05 g, yield: 16%).

LCMS m/z = 339.3 $[M+1]^+$.

Step 4: N-(2-chloro-4-(trifluoromethyl)phenyl)-1-(4-((1-(2-(2,6-dioxopiperidin-3-yl)-1,3-dioxo-2,3-dihydro-1H-pyrrolo[3,4-c]pyridin-6-yl)azetidin-3-yl)ethynyl)-1H-pyrazol-1-yl)cyclobutane-1-carboxamide (Compound 105)

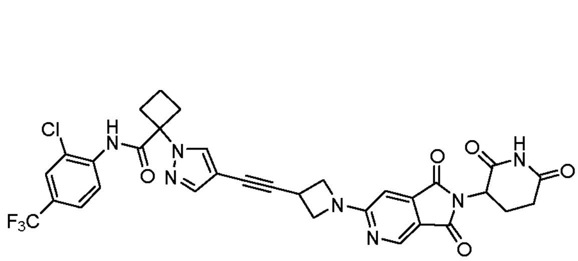

105d (25 mg, 0.074 mmol) was added into a 50 mL single-mouth bottle, 6 mL of dry DMF, 14c (33.82 mg, 0.072 mmol) and TEA (22.1 mg, 0.22 mmol) were added in sequence, nitrogen replacement was performed three times, and $PdCl_2(PPh_3)_2$ (5.2 mg, 0.0074 mmol) and CuI (3 mg, 0.0157 mmol) were added, nitrogen replacement was performed

three times, and the mixture was reacted at 60°C for 2 h. The reaction liquid was cooled to room temperature, 60 mL of saturated ammonium chloride solution was slowly added, a yellow solid was precipitated, filtration was performed, the filter cake was washed with 10 mL of water three times, the filter cake was dissolved in DCM (30 mL), the mixture was dried over anhydrous sodium sulfate and concentrated under reduced pressure, the crude product was separated and purified with silica gel chromatography column (petroleum ether/ethyl acetate (v/v) = 1 : 9) to afford compound 105 (10 mg, yield: 20%).

$^1$H NMR (400 MHz, $CDCl_3$) δ 8.70 (s, 1H), 8.63 (s, 1H), 8.55 (d, 1H), 7.97 (s, 1H), 7.79 (s, 1H), 7.71 (s, 1H), 7.63 - 7.46 (m, 2H), 6.68 (s, 1H), 5.03 - 4.87 (m, 1H), 4.68 - 4.50 (m, 2H), 4.40 - 4.22 (m, 2H), 3.90 - 3.78 (m, 1H), 3.15 - 3.00 (m, 2H), 2.97 - 2.65 (m, 5H), 2.32 - 2.00 (m, 3H).

LCMS m/z = 680.1 [M+1]$^+$.

**Example 106: Preparation of compound 106**

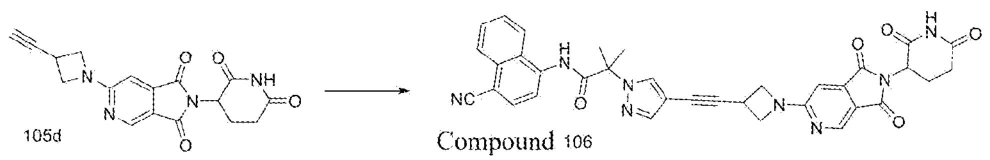

105d

Compound 106

105d (20 mg, 0.059 mmol) was added into a 50 mL single-mouth bottle, 5 mL of dry DMF, 21b (25.4 mg, 0.059 mmol) and TEA (17.8 mg, 0.176 mmol) were added in sequence, nitrogen replacement was performed three times, and $PdCl_2(PPh_3)_2$ (4 mg, 0.0057 mmol) and CuI (2 mg, 0.0105 mmol) were added, nitrogen replacement was performed three times, and the mixture was reacted at 60°C for 2 h. The reaction liquid was cooled to room temperature, 50 mL of saturated ammonium chloride solution was slowly added, a solid was precipitated, filtration was performed, the filter cake was washed with 10 mL of water three times, the filter cake was dissolved in DCM (30 mL), the mixture was dried over anhydrous sodium sulfate and concentrated under reduced pressure, the crude product was separated and purified with silica gel chromatography column (petroleum ether/ethyl acetate (v/v) = 3 : 7) to afford compound 106 (3.5 mg, yield: 9%).

$^1$H NMR (400 MHz, $CDCl_3$) δ 9.91 (s, 1H), 8.63 (s, 1H), 8.35 (d, 1H), 8.26 (d, 1H), 8.05 - 7.78 (m, 5H), 7.78 - 7.60 (m, 2H), 6.67 (s, 1H), 5.03 - 4.85 (m, 1H), 4.70 - 4.45 (m, 2H), 4.40 - 4.20 (m, 2H), 3.94 - 3.74 (m, 1H), 3.00 - 2.62 (m, 3H), 2.22 - 2.08 (m, 1H), 2.01 (s, 6H).

LCMS m/z = 641.2 [M+1]$^+$.

**Example 107: Preparation of compound 107**

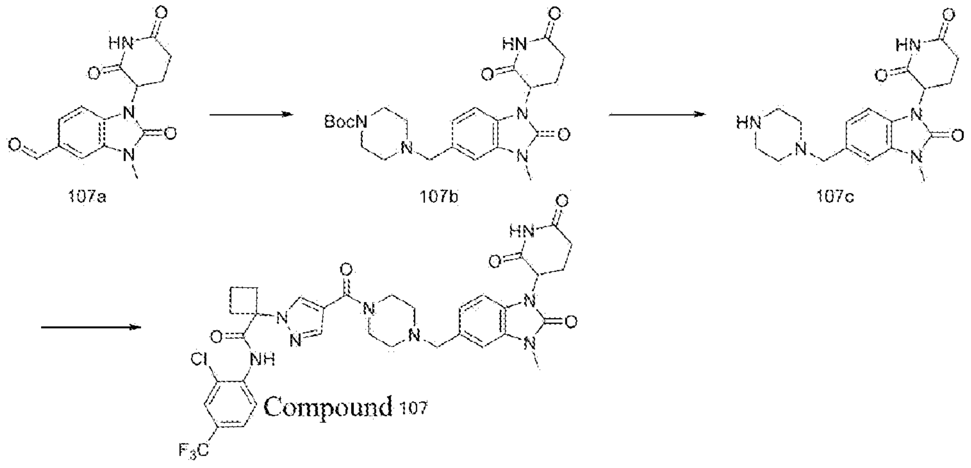

107a

107b

107c

Compound 107

Step 1: tert-butyl 4-((1-(2,6-dioxopiperidin-3-yl)-3-methyl-2-oxo-2,3-dihydro-1H-benzo[d] imidazol-5-yl)methyl)piperazine-1-carboxylate (107b)

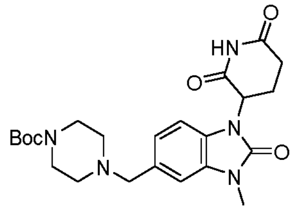

107a (see WO 2020264499 for the synthesis method) (200 mg, 0.70 mmol) was dissolved in 10 mL of DMAc, tert-butyl piperazine-1-carboxylate (200 mg, 1.074 mmol) and 0.1 mL of acetic acid were added, the mixture was stirred at room temperature for 1.0 h, then sodium triacetoxyborohydride (450 mg, 2.12 mmol) was added, and the mixture was reacted at room temperature for 1 h. 20 mL of saturated aqueous sodium bicarbonate solution was slowly added to the reaction liquid, the mixture was extracted with ethyl acetate (10 mL × 3), the organic phase was washed with 50 mL of water, dried over anhydrous sodium sulfate, and concentrated under reduced pressure. The crude product was separated and purified with silica gel column chromatography (DCM/MeOH (v/v) = 20 : 1) to afford 107b (0.20 g, yield: 62%).
LCMS m/z = 458.2 $[M+1]^+$.

Step 2: 3-(3-methyl-2-oxo-5-(piperazin-1-ylmethyl)-2,3-dihydro-1H-benzo[d] imidazol-1-yl)piperidine-2,6-dione (107c)

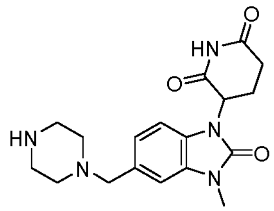

107b (0.2 g, 0.44 mmol) was dissolved in 10 mL of DCM, 6 mL of a solution of 4 mol/L 1,4-dioxane hydrogen chloride solution was added, and the mixture was reacted at room temperature for 3 h. The reaction system was concentrated under reduced pressure. The residue was dissolved in 30 mL of 4 mol/L aqueous NaOH solution and the mixture was extracted with DCM (40 mL × 3). The organic phase was washed with 50 mL of water, dried over anhydrous sodium sulfate, and concentrated under reduced pressure to afford crude 107c (0.15 g).
LCMS m/z = 358.2 $[M+1]^+$.

Step 3: N-(2-chloro-4-(trifluoromethyl)phenyl)-1-(4-(4-((1-(2,6-dioxopiperidin-3-yl)-3-methyl-2-oxo-2,3-dihydro-1H-benzo[d]imidazol-5-yl)methyl)piperazine-1-carbonyl)-1H-pyrazol-1-yl)cyclobutane-1-carboxamide (Compound 107)

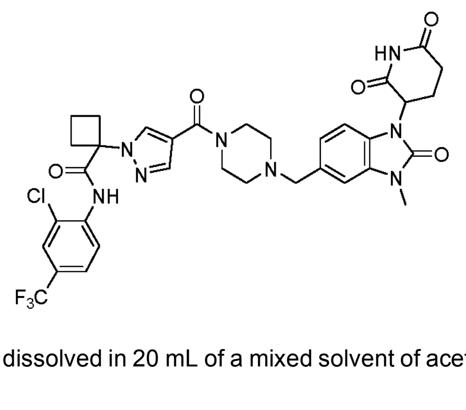

102f (1.0 g, 2.41 mmol) was dissolved in 20 mL of a mixed solvent of acetonitrile and water (v/v) = 4 : 1, lithium

hydroxide monohydrate (0.3 g, 7.15 mmol) was added, and the mixture was reacted at room temperature for 16 h. The reaction liquid was added to 50 mL of water, the mixture was extracted with 50 mL of ethyl acetate, the pH of the organic phase was adjusted to 6 with 1 mol/L hydrochloric acid, the mixture was extracted with 50 mL of ethyl acetate, the organic phase was dried over anhydrous sodium sulfate, and concentrated under reduced pressure to afford crude 1 (0.1 g). Crude 107c (28 mg) was dissolved in 5 mL of DMF, crude 1 (30 mg), TCFH (43 mg, 0.15 mmol) and N-methylimidazole (19 mg, 0.23 mmol) were added, and the mixture was reacted at room temperature for 2 h. The reaction liquid was extracted with ethyl acetate (10 mL × 3), the organic phase was washed with 50 mL of water, dried over anhydrous sodium sulfate, and concentrated under reduced pressure. The crude product was separated and purified with silica gel column chromatography (DCM/MeOH (v/v) = 20 : 1) to afford compound 107 (0.010 g, two-step yield from compound 107b: 17%).

$^1H$ NMR (400 MHz, $CDCl_3$) δ 8.80 (s, 1H), 8.55 (d, 1H), 8.18 (s, 1H), 7.92 (s, 1H), 7.84 (s, 1H), 7.65 - 7.47 (m, 2H), 7.20 - 6.97 (m, 2H), 6.75 (d, 1H), 5.27 - 5.16 (m, 1H), 3.92 - 3.71 (m, 4H), 3.66 (s, 2H), 3.45 (s, 3H), 3.17 - 2.42 (m, 11H), 2.33 - 2.02 (m, 3H).

LCMS m/z = 727.2 $[M+1]^+$.

**Example 108: Preparation of compound 108**

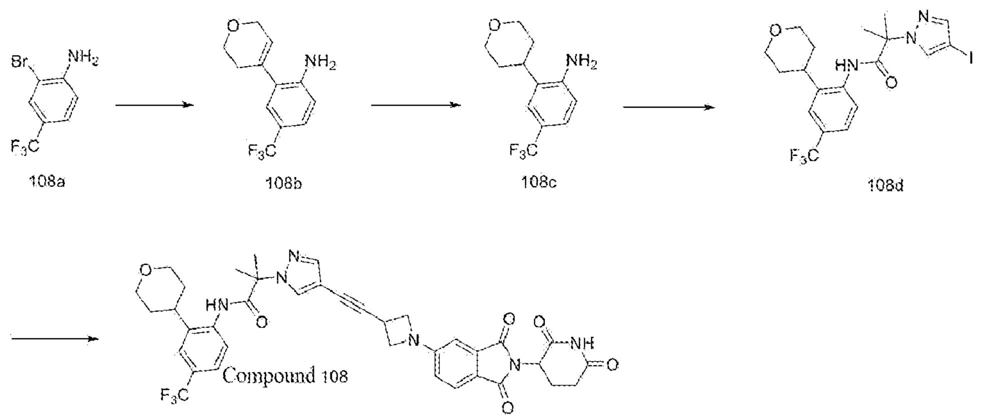

108a 108b 108c 108d

Compound 108

Step 1: 2-(3,6-dihydro-2H-pyran-4-yl)-4-(trifluoromethyl)aniline (108b)

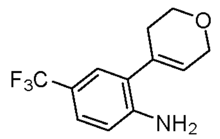

16 mL of dioxane and 0.5 mL of water were added to 3,6-dihydro-2H-pyran-4-boronic acid pinacol ester (1.05 g, 5.0 mmol), 108a (1.0 g, 4.17 mmol) and potassium carbonate (2.31 g, 16.71 mmol), $Pd(dppf)Cl_2$•DCM (0.3 g, 0.37 mmol) was added under nitrogen protection, and the mixture was reacted at 100°C for 6 h. The reaction liquid was cooled to room temperature, 50 mL of water was added, the mixture was extracted with ethyl acetate (50 mL × 3), the organic phases were combined, washed with 50 mL of saturated sodium chloride solution, dried over anhydrous sodium sulfate, and concentrated under reduced pressure. The crude product was separated and purified with silica gel chromatography column (petroleum ether/ethyl acetate (v/v) = 100 : 1-1 : 1) to afford 108b (0.8 g, yield: 79%).

LCMS m/z = 244.1 $[M+1]^+$.

Step 2: 2-(tetrahydro-2H-pyran-4-yl)-4-(trifluoromethyl)aniline (108c)

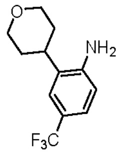

108b (0.8 g, 3.29 mmol) was dissolved in 20 mL of methanol, 0.2 g of 10% palladium on carbon was added, and the mixture was reacted at room temperature for 12 h. The reaction liquid was filtered, and the filtrate was concentrated under reduced pressure to afford crude 108c (0.7 g).

LCMS m/z = 246.1 [M+1] $^+$.

Step 3: 2-(4-iodo-1H-pyrazol-1-yl)-2-methyl-N-(2-(tetrahydro-2H-pyran-4-yl)-4-(trifluoromethyl)phenyl)propanamide (108d)

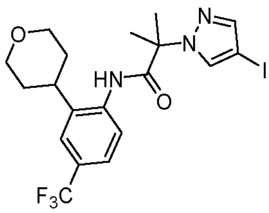

16a-1 (0.5 g, 1.78 mmol) was dissolved in 10 mL of DCM, 1-chloro-N,N,2-trimethylpropenylamine (0.36 g, 2.7 mmol) was added, the mixture was reacted at room temperature for 2 h, and then TEA (0.54 g, 5.34 mmol) and crude 108c (0.44 g) were added in sequence, and the mixture was reacted at room temperature for 12 h. 50 mL of DCM and 50 mL of water were added to the reaction liquid, the organic phase was separated, dried over anhydrous sodium sulfate, and concentrated under reduced pressure. The crude product was separated and purified with silica gel chromatography column (petroleum ether/ethyl acetate (v/v) = 20 : 1-1 : 1) to afford 108d (0.5 g, yield: 55%).

LCMS m/z = 508.0 [M+1] $^+$.

Step 4: 2-(4-((1-(2-(2,6-dioxopiperidin-3-yl)-1,3-dioxoisoindolin-5-yl)azetidin-3-yl)ethynyl)-1H-pyrazol-1-yl)-2-methyl-N-(2-(tetrahydro-2H-pyran-4-yl)-4-(trifluoromethyl)phenyl)propanamide (Compound 108)

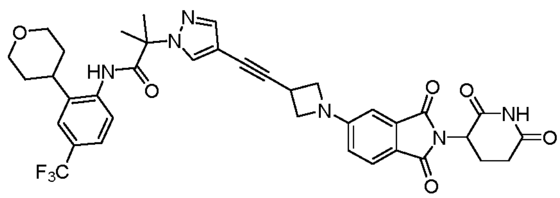

108d (0.48 g, 0.946 mmol), intermediate 1 (0.32 g), TEA (0.57 g, 5.63 mmol), CuI (36 mg, 0.189 mmol) and $PdCl_2(PPh_3)_2$ (66 mg, 0.094 mmol) were added to a reaction bottle, nitrogen replacement was performed three times, 5 mL of DMF was added under nitrogen protection, and the mixture was reacted at 60°C for 3 h. The reaction liquid was cooled to room temperature, 50 mL of water was added, and the mixture was filtered by suction. The filter cake was washed with 10 mL of water. The filter cake was dissolved in 100 mL of DCM, the mixture was dried over anhydrous sodium sulfate, and concentrated under reduced pressure. The crude product was separated and purified with silica gel chromatography column (ethyl acetate/petroleum ether/dichloromethane (v/v) = 2 : 1 : 1) to afford compound 108 (0.1 g, yield: 15%).

$^1H$ NMR (400 MHz, $CDCl_3$) δ 8.72 (s, 1H), 8.10 - 8.01 (m, 1H), 7.95 (s, 1H), 7.88 - 7.76 (m, 2H), 7.68 (d, 1H), 7.51 - 7.41 (m, 2H), 6.86 - 6.78 (m, 1H), 6.56 (dd, 1H), 5.00 - 4.88 (m, 1H), 4.42 - 4.32 (m, 2H), 4.19 - 4.00 (m, 4H), 3.88 - 3.76 (m, 1H), 3.64 - 3.48 (m, 2H), 2.96 - 2.54 (m, 4H), 2.20 - 2.06 (m, 1H), 1.95 (s, 6H), 1.87 - 1.70 (m, 2H), 1.68 - 1.52 (m, 2H).

LCMS m/z = 717.2 [M+1] $^+$.

**Example 109: Preparation of compound 109**

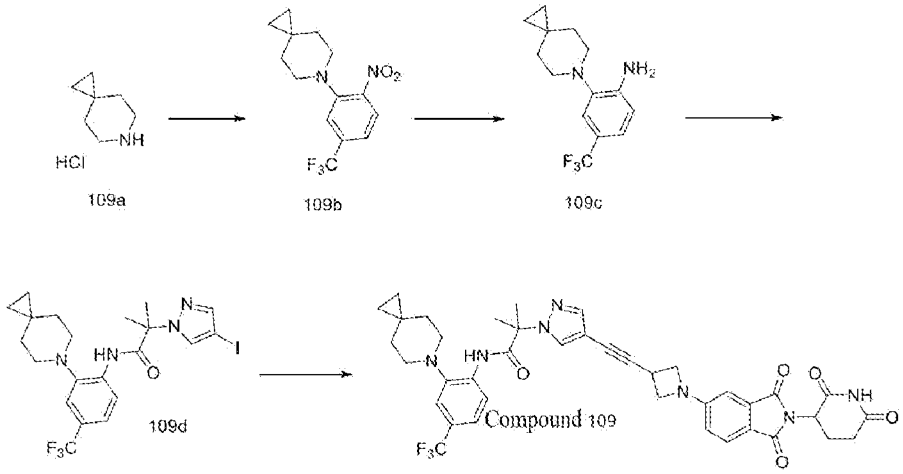

109a 109b 109c

109d Compound 109

Step 1: 6-(2-nitro-5-(trifluoromethyl)phenyl)-6-azaspiro[2.5]octane (109b)

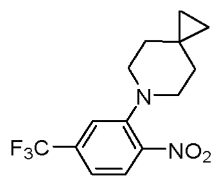

109a (1.0 g, 6.78 mmol) was dissolved in 10 mL of DMF, potassium carbonate (2.81 g, 20.33 mmol) and 3-fluoro-4-nitrotrifluorotoluene (1.42 g, 6.79 mmol) were added at room temperature, and the mixture was reacted at 80°C for 4 h. The reaction liquid was cooled to room temperature and added to 50 mL of water, the mixture was extracted with 50 mL of ethyl acetate, dried over anhydrous sodium sulfate, and concentrated under reduced pressure. The crude product was separated and purified with silica gel chromatography column (petroleum ether/ethyl acetate (v/v) = 100 : 1-20 : 1) to afford 109b (1.3 g, yield: 64%).

LCMS m/z = 301.1 $[M+1]^+$.

Step 2: 2-(6-azaspiro[2.5]octan-6-yl)-4-(trifluoromethyl)aniline (109c)

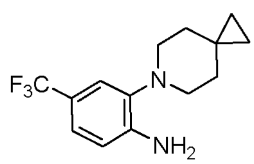

109b (1.3 g, 4.33 mmol) was dissolved in 20 mL of ethanol, 0.2 g of 10% palladium on carbon was added, and the mixture was reacted at room temperature for 12 h under the atmosphere of hydrogen balloon. The reaction liquid was filtered, and the filtrate was concentrated under reduced pressure to afford crude 109c (1.1 g).

LCMS m/z = 271.2 $[M+1]^+$.

Step 3: N-(2-(6-azaspiro[2.5]octan-6-yl)-4-(trifluoromethyl)phenyl)-2-(4-iodo-1H-pyrazol-1-yl)-2-methylpropanamide (109d)

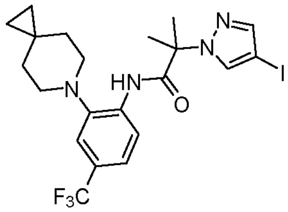

16a-1 (0.5 g, 1.78 mmol) was dissolved in 10 mL of DCM, 1-chloro-N,N,2-trimethylpropenylamine (0.36 g, 2.7 mmol) was added, the mixture was reacted at room temperature for 2 h, and then TEA (0.54 g, 5.34 mmol) and crude 109c (0.48 g) were added in sequence, and the mixture was reacted at room temperature for 12 h. 50 mL of DCM and 50 mL of water were added to the reaction liquid, the organic phase was separated, dried over anhydrous sodium sulfate, and concentrated under reduced pressure. The crude product was separated and purified with silica gel chromatography column (petroleum ether/ethyl acetate (v/v) = 20 : 1) to afford 109d (0.5 g, yield: 53%).

LCMS m/z = 533.0 $[M+1]^+$.

Step 4: N-(2-(6-azaspiro[2.5]octan-6-yl)-4-(trifluoromethyl)phenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 109)

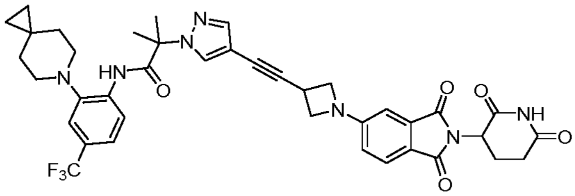

109d (0.5 g, 0.94 mmol), intermediate 1 (0.32 g), TEA (0.57 g, 5.63 mmol), CuI (36 mg, 0.189 mmol) and $PdCl_2(PPh_3)_2$ (66 mg, 0.094 mmol) were added to a reaction bottle, nitrogen replacement was performed three times, 5 mL of DMF was added under nitrogen protection, and the mixture was reacted at 60°C for 3 h. The reaction liquid was cooled to room temperature, 50 mL of water was added, and the mixture was filtered by suction. The filter cake was washed with 10 mL of water. The filter cake was dissolved in 100 mL of DCM, the mixture was dried over anhydrous sodium sulfate, and concentrated under reduced pressure. The crude product was separated and purified with prep-TLC (ethyl acetate/petroleum ether/dichloromethane (v/v) = 2 : 1 : 1) to afford compound 109 (0.05 g, yield: 7%).

$^1H$ NMR (400 MHz, $CDCl_3$) δ 9.56 (br.s, 1H), 8.44 - 8.34 (m, 1H), 7.93 (s, 1H), 7.81 (s, 1H), 7.73 (s, 1H), 7.67 (d, 1H), 7.47 - 7.37 (m, 2H), 6.84 - 6.76 (m, 1H), 6.55 (dd, 1H), 4.99 - 4.88 (m, 1H), 4.42 - 4.29 (m, 2H), 4.13 - 4.00 (m, 2H), 3.89 - 3.75 (m, 1H), 3.02 - 2.62 (m, 7H), 2.20 - 2.06 (m, 1H), 1.95 (s, 6H), 1.80 - 1.40 (m, 4H), 0.40 (s, 4H).

LCMS m/z = 742.7 $[M+1]^+$.

**Example 110: Preparation of the trifluoroacetate of compound 110**

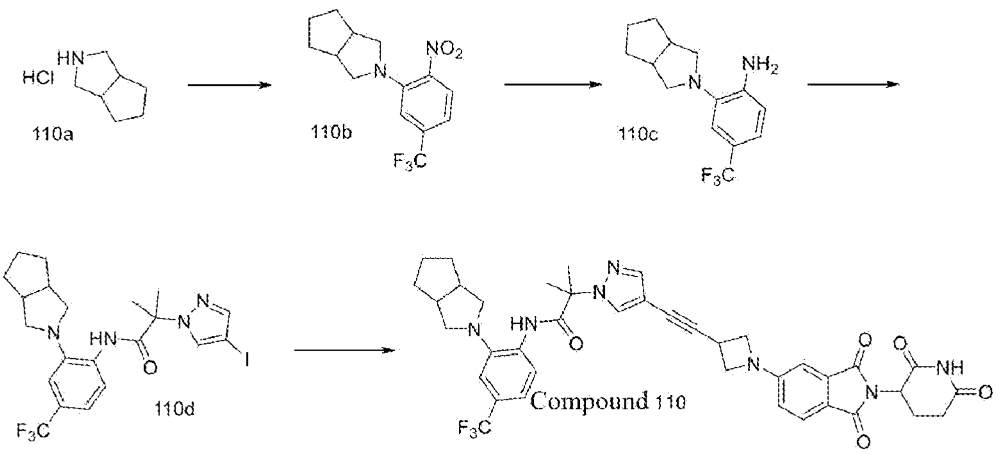

110a 110b 110c 110d Compound 110

Step 1: 2-(2-nitro-5-(trifluoromethyl)phenyl)octahydrocyclopenta[c]pyrrole (110b)

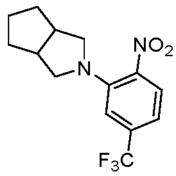

110a (2.0 g, 13.55 mmol) was dissolved in 20 mL of DMF, potassium carbonate (5.63 g, 40.74 mmol) and 2-fluoro-1-nitro-4-(trifluoromethyl)benzene (2.28 g, 10.90 mmol) were added at room temperature, and the mixture was reacted at 80°C for 4 h. The reaction liquid was cooled to room temperature and added to 50 mL of water, the mixture was extracted with 50 mL of ethyl acetate, dried over anhydrous sodium sulfate, and concentrated under reduced pressure. The crude product was separated and purified with silica gel chromatography column (petroleum ether/ethyl acetate (v/v) = 100 : 1-20 : 1) to afford 110b (1.3 g, yield: 40%).

Step 2: 2-(hexahydrocyclopenta[c]pyrrol-2(1H)-yl)-4-(trifluoromethyl)aniline (110c)

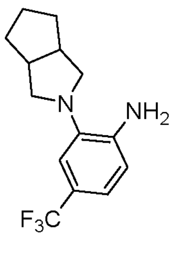

110b (1.3 g, 4.33 mmol) was dissolved in 20 mL of ethanol, 0.2 g of 10% palladium on carbon was added, and the mixture was reacted at room temperature for 12 h. The reaction liquid was filtered, and the filtrate was concentrated under reduced pressure to afford crude 110c (1.1 g).

LCMS m/z = 271.2 [M+1] $^+$.

Step 3: N-(2-(hexahydrocyclopenta[c]pyrrol-2(1H)-yl)-4-(trifluoromethyl) phenyl)-2-(4-iodo-1H-pyrazol-1-yl)-2-methylpropanamide (110d)

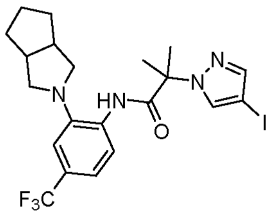

16a-1 (0.5 g, 1.78 mmol) was dissolved in 10 mL of DCM, 1-chloro-N,N,2-trimethylpropenylamine (0.36 g, 2.7 mmol) was added, the mixture was reacted at room temperature for 2 h, and then TEA (1.09 g, 10.77 mmol) and crude 110c (0.48 g) were added in sequence, and the mixture was reacted at room temperature for 12 h. 50 mL of DCM and 50 mL of water were added to the reaction liquid, the organic phase was separated, dried over anhydrous sodium sulfate, and concentrated under reduced pressure. The crude product was separated and purified with silica gel chromatography column (petroleum ether/ethyl acetate (v/v) = 20 : 1) to afford 110d (0.5 g, yield: 53%).

LCMS m/z = 533.1 [M+1] $^+$.

Step 4: 2-(4-((1-(2-(2,6-dioxopiperidin-3-yl)-1,3-dioxoisoindolin-5-yl)azetidin-3-yl)ethynyl)-1H-pyrazol-1-yl)-N-(2-(hexahydrocyclopenta[c]pyrrol-2(1H)-yl)-4-(trifluoromethyl)phenyl)-2-methylpropanamide (Compound 110) trifluoroacetate

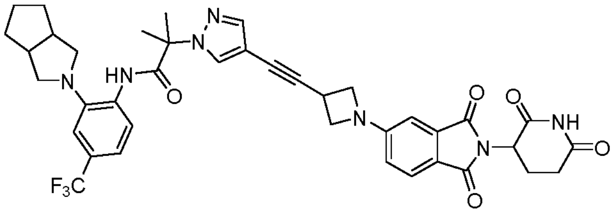

110d (0.5 g, 0.94 mmol), intermediate 1 (0.32 g), TEA (0.57 g, 5.63 mmol), CuI (36 mg, 0.189 mmol) and $PdCl_2(PPh_3)_2$ (66 mg, 0.094 mmol) were added to a reaction bottle, nitrogen replacement was performed three times, 5 mL of DMF was added under nitrogen protection, and the mixture was reacted at 60°C for 3 h. The reaction liquid was cooled to room temperature, 50 mL of water was added, and the mixture was filtered by suction. The filter cake was washed with 10 mL of water. The filter cake was dissolved in 100 mL of DCM, the mixture was dried over anhydrous sodium sulfate, and concentrated under reduced pressure. The crude product was subjected to Pre-HPLC (instrument and preparative column: using Glison GX-281 preparative liquid phase chromatographic instrument, preparative column model: Sunfire C18, 5 μm, inner diameter × length = 30 mm × 150 mm). Preparation method: the crude product was dissolved with methanol and dimethyl sulfoxide, and filtered with a 0.45 μm filter membrane, to prepare into a sample liquid. Mobile phase system: acetonitrile/water (containing 0.1% TFA). Gradient elution method: gradient elution of 5% to 60% acetonitrile (elution time 15 min), and lyophilization was performed to afford the trifluoroacetate of compound 110 (0.1 g).

$^1$H NMR (400 MHz, DMSO-$d_6$) δ 11.06 (s, 1H), 8.66 (s, 1H), 8.38 (s, 1H), 8.16 (d, 1H), 7.82 (s, 1H), 7.68 (d, 1H), 7.43 - 7.33 (m, 2H), 6.92 - 6.85 (m, 1H), 6.72 (dd, 1H), 5.11 - 5.02 (m, 1H), 4.46 - 4.34 (m, 2H), 4.03 - 3.82 (m, 3H), 3.11 - 2.97 (m, 2H), 2.96 - 2.80 (m, 1H), 2.72 - 2.52 (m, 4H), 2.49 - 2.43 (m, 2H), 2.08 - 1.95 (m, 1H), 1.84 (s, 6H), 1.78 - 1.34 (m, 6H).

LCMS m/z = 742.2 [M+1] $^+$.

**Example 111: Preparation of compound 111**

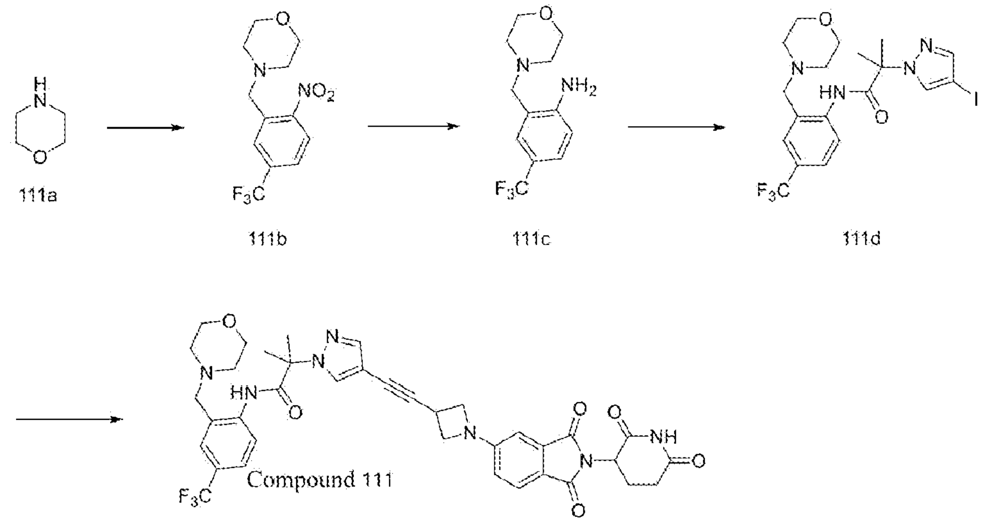


Step 1: 4-(2-nitro-5-(trifluoromethyl)benzyl)morpholine (11 1b)

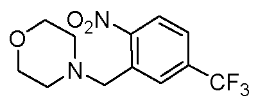

Morpholine (111a) (1.59 g, 18.25 mmol) and 2-nitro-5-trifluoromethylbenzaldehyde (2.0 g, 9.13 mmol) were dissolved in 40 mL of DCE, 1 mL of acetic acid was added, and the mixture was stirred at room temperature for 2 h, then sodium triacetoxyborohydride (5.81g, 27.42 mmol) was added, and the mixture was reacted at room temperature for 12 h. The reaction liquid was added to 100 mL of DCM, and the liquid separation was conducted. The organic phase was washed with 50 mL of saturated aqueous sodium bicarbonate solution, dried over anhydrous sodium sulfate, and concentrated under reduced pressure. The crude product was separated and purified with silica gel chromatography column (petroleum ether/ethyl acetate (v/v) = 100 : 1-1 : 1) to afford 111b (1.5 g, yield: 57%).

LCMS m/z = 291.1 $[M+1]^+$.

Step 2: 2-(morpholinomethyl)-4-(trifluoromethyl)aniline (111c)

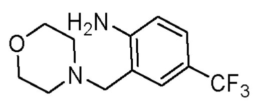

111b (1.5 g, 5.17 mmol) was dissolved in 20 mL of ethanol, 0.2 g of 10% palladium on carbon was added, and the mixture was reacted at room temperature for 4 h. The reaction liquid was filtered and concentrated under reduced pressure to afford crude 111c (1.1 g).

LCMS m/z = 261.3$[M+1]^+$.

Step 3: 2-(4-iodo-1H-pyrazol-1-yl)-2-methyl-N-(2-(morpholinomethyl)-4-(trifluoromethyl)phenyl)propanamide (11 1d)

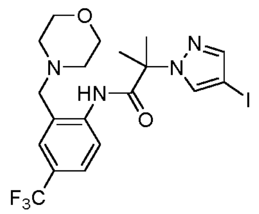

16a-1 (0.5 g, 1.78 mmol) was dissolved in 10 mL of DCM, 1-chloro-N,N,2-trimethylpropenylamine (0.36 g, 2.7 mmol) was added, the mixture was reacted at room temperature for 2 h, and then TEA (1.09 g, 10.77 mmol) and crude 111c (0.47 g) were added in sequence, and the mixture was reacted at room temperature for 12 h. 50 mL of DCM and 50 mL of water were added to the reaction liquid, the organic phase was separated, dried over anhydrous sodium sulfate, and concentrated under reduced pressure. The crude product was separated and purified with silica gel chromatography column (petroleum ether/ethyl acetate (v/v) = 100 : 1-5 : 1) to afford 111d (0.5 g, yield: 54%).

LCMS m/z = 523.1 [M+1] $^+$.

Step 4: 2-(4-((1-(2-(2,6-dioxopiperidin-3-yl)-1,3-dioxoisoindolin-5-yl)azetidin-3-yl)ethynyl)-1H-pyrazol-1-yl)-2-methyl-N-(2-(morpholinomethyl)-4-(trifluoromethyl)phenyl)propanamide (Compound 111)

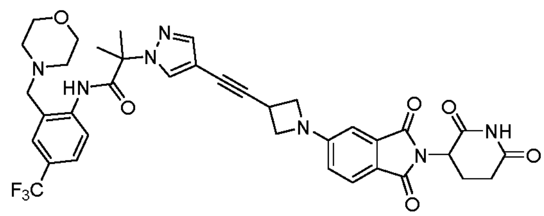

111d (0.49 g, 0.94 mmol), intermediate 1 (0.32 g), TEA (0.57 g, 5.63 mmol), CuI (36 mg, 0.189 mmol) and $PdCl_2(PPh_3)_2$ (66 mg, 0.094 mmol) were added to a reaction bottle, nitrogen replacement was performed three times, 5 mL of DMF was added under nitrogen protection, and the mixture was reacted at 60°C for 3 h. The reaction liquid was cooled to room temperature, 50 mL of water was added, and the mixture was filtered by suction. The filter cake was washed with 10 mL of water. The filter cake was dissolved in 100 mL of DCM, the mixture was dried over anhydrous sodium sulfate, and concentrated under reduced pressure. The crude product was separated and purified with silica gel chromatography column (petroleum ether/ethyl acetate (v/v) = 100 : 1-1 : 5) to afford compound 111 (0.1 g, yield: 15%).

$^1$H NMR (400 MHz, $CDCl_3$) δ 10.27 (s, 1H), 8.34 (d, 1H), 8.08 (s, 1H), 7.74 (s, 1H), 7.71 - 7.61 (m, 2H), 7.59 - 7.51 (m, 1H), 7.39 - 7.29 (m, 1H), 6.80 (d, 1H), 6.55 (dd, 1H), 4.99 - 4.87 (m, 1H), 4.43 - 4.28 (m, 2H), 4.11 - 3.97 (m, 2H), 3.86 - 3.72 (m, 1H), 3.70 - 3.58 (m, 4H), 3.43 (s, 2H), 2.96 - 2.64 (m, 3H), 2.44 - 2.25 (m, 4H), 2.20 - 2.06 (m, 1H), 1.91 (s, 6H).

LCMS m/z = 732.2 [M+1] $^+$.

**Example 112: Preparation of compound 112**

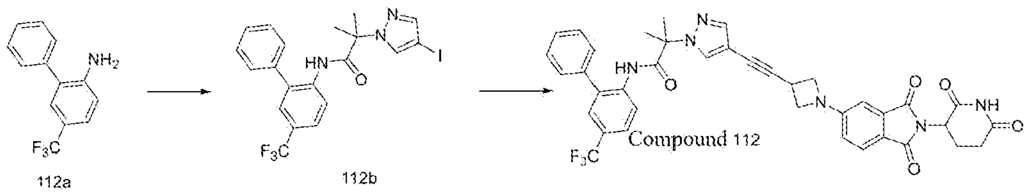


112a 112b

Step 1: 2-(4-iodo-1H-pyrazol-1-yl)-2-methyl-N-(5-(trifluoromethyl)-[1,1'-biphenyl]-2-yl)propanamide (112b)

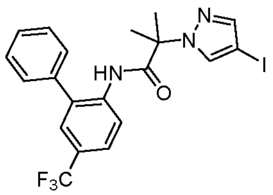

16a-1 (0.5 g, 1.78 mmol) was dissolved in 10 mL of DCM, 1-chloro-N,N,2-trimethylpropenylamine (0.36 g, 2.7 mmol) was added, the mixture was reacted at room temperature for 2 h, and then TEA (1.09 g, 10.77 mmol) and 112a (0.42 g, 1.77 mmol) (see Org. Letts., 2020, 22, 9331-9336 for the synthesis method) were added in sequence, and the mixture was reacted at room temperature for 12 h. 50 mL of DCM and 50 mL of water were added to the reaction liquid, the organic phase was separated, dried over anhydrous sodium sulfate, and concentrated under reduced pressure. The crude product was separated and purified with silica gel chromatography column (petroleum ether/ethyl acetate (v/v) = 20 : 1) to afford 112b (0.5 g, yield: 57%).

LCMS m/z = 500.0 $[M+1]^+$.

Step 2: 2-(4-((1-(2-(2,6-dioxopiperidin-3-yl)-1,3-dioxoisoindolin-5-yl)azetidin-3-yl)ethynyl)-1H-pyrazol-1-yl)-2-methyl-N-(5-(trifluoromethyl)-[1,1'-biphenyl]-2-yl)propanamide (Compound 112)

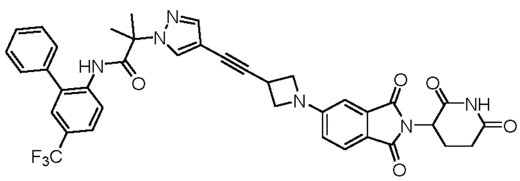

112b (0.47 g, 0.94 mmol), intermediate 1 (0.32 g), TEA (0.57 g, 5.63 mmol), CuI (36 mg, 0.189 mmol) and $PdCl_2(PPh_3)_2$ (66 mg, 0.094 mmol) were added to a reaction bottle, nitrogen replacement was performed three times, 5 mL of DMF was added under nitrogen protection, and the mixture was reacted at 60°C for 3 h. The reaction liquid was cooled to room temperature, 50 mL of water was added, and the mixture was filtered by suction. The filter cake was washed with 10 mL of water. The filter cake was dissolved in 100 mL of DCM, the mixture was dried over anhydrous sodium sulfate, and concentrated under reduced pressure. The crude product was separated and purified with silica gel chromatography column (petroleum ether/dichloromethane/ethyl acetate (v/v) = 1 : 1 : 2) to afford compound 112 (0.1 g, yield: 15%).

$^1H$ NMR (400 MHz, $CDCl_3$) $\delta$ 8.51 (d, 1H), 8.38 (s, 1H), 8.09 - 7.96 (m, 1H), 7.74 - 7.54 (m, 3H), 7.53 - 7.37 (m, 4H), 7.23 - 7.06 (m, 3H), 6.86 - 6.79 (m, 1H), 6.57 (dd, 1H), 5.00 - 4.89 (m, 1H), 4.45 - 4.32 (m, 2H), 4.14 - 4.01 (m, 2H), 3.90 - 3.74 (m, 1H), 3.00 - 2.63 (m, 3H), 2.22 - 2.08 (m, 1H), 1.80 (s, 6H).

LCMS m/z = 709.2 $[M+1]^+$.

**Example 113: Preparation of compound 113**

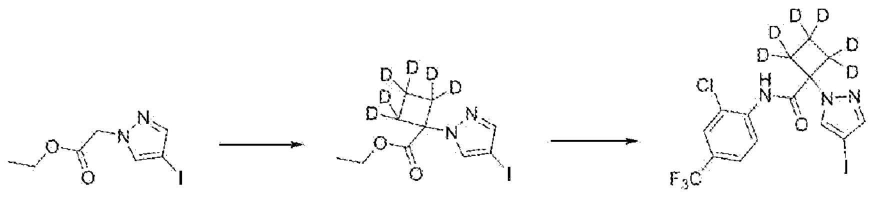

113a 113b 113c

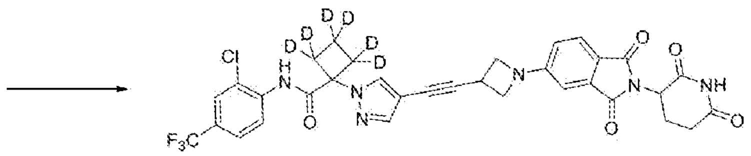

Compound 113

Step 1: ethyl 1-(4-iodo-1H-pyrazol-1-yl)cyclobutane-1-carboxylate-2,2,3,3,4,4-$d_6$ (113b)

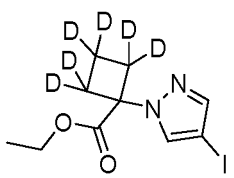

60% sodium hydride (4.0 g) was dispersed in 400 mL of DMSO, the mixture was cooled to 0°C, and a solution of 113a (see WO 2020043008 for the synthesis method) (11.7 g, 41.78 mmol) and 1,3-dibromopropane-1,1,2,2,3,3-$d_6$ (21.5 g, 103.4 mmol) in DMSO (80 mL) was slowly added dropwise. After the dropwise addition was completed, the mixture was warmed to room temperature and reacted for 4 h. 1.5 L of saturated ammonium chloride solution was added to the reaction system, the mixture was extracted with 500 mL of ethyl acetate, the organic phase was dried over anhydrous sodium sulfate, and concentrated under reduced pressure. The crude product was separated and purified with silica gel chromatography column (petroleum ether/ethyl acetate (v/v) = 4 : 1) to afford 113b (6.4 g, yield: 47%).

Step 2: N-(2-chloro-4-(trifluoromethyl)phenyl)-1-(4-iodo-1H-pyrazol-1-yl)cyclobutane-2,2,3,3,4,4-$d_6$-1-carboxamide (113c)

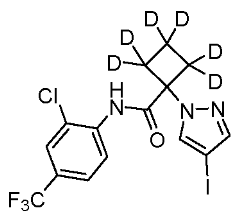

113b (6.4 g, 19.62 mmol) was added to a mixed solvent of 30 mL of THF and 10 mL of water, lithium hydroxide monohydrate (1.68 g, 40.04 mmol) was added, and the mixture was reacted at room temperature for 1 h. 100 mL of water was added to the reaction liquid, the pH was adjusted to 5 with concentrated hydrochloric acid, the mixture was extracted with 100 mL of ethyl acetate, the organic phase was separated, dried over anhydrous sodium sulfate, and concentrated under reduced pressure to afford a crude product (5.0 g). The above crude product (5.0 g) was dissolved in 100 mL of DCM, TCFH (6.8 g, 24.24 mmol) and 11a (3.3 g, 16.87 mmol) were added, the mixture was cooled to 0°C, and N-methylimidazole (5.0 g, 60.89 mmol) was added dropwise at 0°C, and the mixture was reacted at room temperature for 5 h. The reaction liquid was concentrated under reduced pressure, and the crude product was separated and purified

with silica gel chromatography column (petroleum ether/ethyl acetate (v/v) = 4 : 1) to afford 113c (6.6 g, yield: 82%).
LCMS m/z = 476.0 [M+1]$^+$.

Step 3: N-(2-chloro-4-(trifluoromethyl)phenyl)-1-(4-((1-(2-(2,6-dioxopiperidin-3-yl)-1,3-dioxoisoindolin-5-yl)azetidin-3-yl)ethynyl)-1H-pyrazol-1-yl)cyclobutane-2,2,3,3,4,4-$d_6$-1-carboxamide (Compound 113)

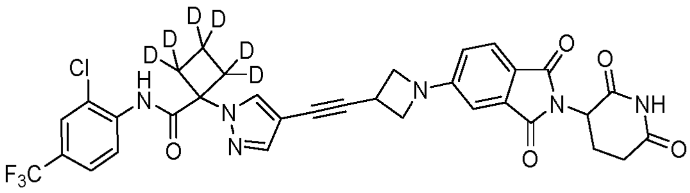

113c (5.5 g, 11.58 mmol) was dissolved in 100 mL of DMF, and intermediate 1 (5.0 g), TEA (3.0 g, 29.65 mmol), CuI (0.19 g, 1.0 mmol) and $PdCl_2(PPh_3)_2$ (0.7 g, 1.0 mmol) were added in sequence, nitrogen replacement was performed three times, and the mixture was reacted at 55°C for 1 h. The reaction liquid was cooled to room temperature, 500 mL of water was added, the solid was precipitated, filtration was performed, the filter cake was washed with 30 mL of water, the filter cake was collected, the filter cake was dissolved with 100 mL of DCM, the mixture was dried over anhydrous sodium sulfate, and concentrated under reduced pressure. The crude product was separated and purified with silica gel chromatography column (petroleum ether/ethyl acetate (v/v) = 1 : 3) to afford compound 113 (5.2 g, yield: 66%).
$^1H$ NMR (400 MHz, $CDCl_3$) δ 8.69 (s, 1H), 8.54 (d, 1H), 8.05 (s, 1H), 7.79 (s, 1H), 7.70 (s, 1H), 7.67 (d, 1H), 7.60 - 7.55 (m, 1H), 7.54 - 7.47 (m, 1H), 6.80 (d, 1H), 6.55 (dd, 1H), 4.98 - 4.89 (m, 1H), 4.42 - 4.30 (m, 2H), 4.12 - 4.02 (m, 2H), 3.87 - 3.74 (m, 1H), 2.96 - 2.65 (m, 3H), 2.19 - 2.04 (m, 1H).
LCMS m/z = 685.2 [M+1] $^+$.

**Example 114: Preparation of compound 114**

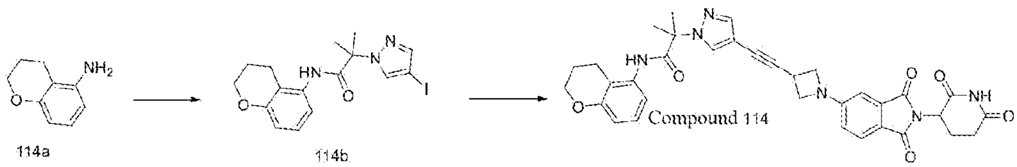

114a

114b

Compound 114

Step 1: N-(chroman-5-yl)-2-(4-iodo-1H-pyrazol-1-yl)-2-methylpropanamide (114b)

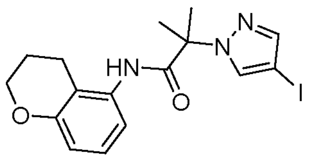

16a-1 (0.5 g, 1.78 mmol) was dissolved in 10 mL of DCM, 1-chloro-N,N,2-trimethylpropenylamine (0.36 g, 2.7 mmol) was added, the mixture was reacted at room temperature for 2 h, and then TEA (1.09 g, 10.77 mmol) and 114a (0.27 g, 1.81 mmol) were added in sequence, and the mixture was reacted at room temperature for 12 h. 50 mL of DCM and 50 mL of water were added to the reaction liquid, the organic phase was separated, dried over anhydrous sodium sulfate, and concentrated under reduced pressure. The crude product was separated and purified with silica gel chromatography column (petroleum ether/ethyl acetate (v/v) = 20 : 1) to afford 114b (0.5g, yield: 68%).
LCMS m/z = 412.0 [M+1] $^+$.

Step 2: N-(chroman-5-yl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 114)

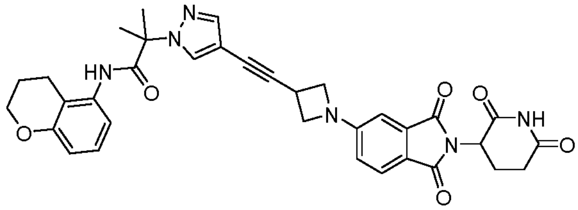

114b (0.39 g, 0.95 mmol), intermediate 1 (0.32 g), TEA (0.57 g, 5.63 mmol), CuI (36 mg, 0.189 mmol) and $PdCl_2(PPh_3)_2$ (66 mg, 0.094 mmol) were added to a reaction bottle, nitrogen replacement was performed three times, 5 mL of DMF was added under nitrogen protection, and the mixture was reacted at 60°C for 3 h. The reaction liquid was cooled to room temperature, 50 mL of water was added, and the mixture was filtered by suction. The filter cake was washed with 10 mL of water. The filter cake was dissolved in 100 mL of DCM, the mixture was dried over anhydrous sodium sulfate, and concentrated under reduced pressure. The crude product was separated and purified with silica gel chromatography column (petroleum ether/dichloromethane/ethyl acetate (v/v) = 1 : 1 : 2) to afford compound 114 (0.1 g, yield: 17%).

$^1H$ NMR (400 MHz, $CDCl_3$) $\delta$ 8.23 (s, 1H), 8.16 - 7.98 (m, 1H), 7.84 - 7.74 (m, 2H), 7.67 (d, 1H), 7.45 - 7.37 (m, 1H), 7.06 (t, 1H), 6.84 - 6.78 (m, 1H), 6.65 - 6.52 (m, 2H), 4.98 - 4.89 (m, 1H), 4.43 - 4.30 (m, 2H), 4.17 - 4.00 (m, 4H), 3.89 - 3.75 (m, 1H), 2.97 - 2.63 (m, 3H), 2.46 - 2.35 (m, 2H), 2.20 - 1.89 (m, 9H).

LCMS m/z = 621.2 $[M+1]^+$.

**Example 115: Preparation of compound 115**

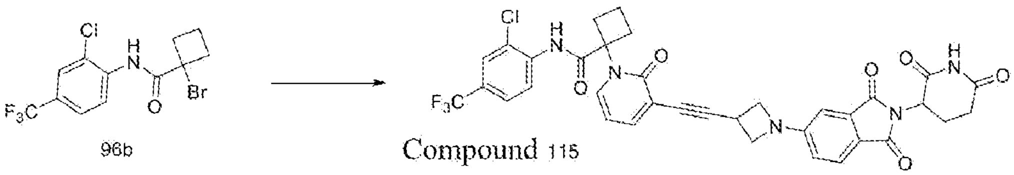

96b

Compound 115

96b (0.32 g, 0.90 mmol), 3-iodopyridin-2(1H)-one (0.1 g, 0.45 mmol) and potassium carbonate (0.25 g, 1.81 mmol) were dissolved in 2 mL of DMF, and the mixture was heated to 80°C and reacted for 5 h. The reaction liquid was cooled to room temperature and added to 50 mL of ethyl acetate, the organic phase was separated, the organic phase was washed with saturated aqueous sodium chloride solution (25 mL × 3), dried over anhydrous sodium sulfate, and concentrated under reduced pressure. The crude product was separated and purified with silica gel chromatography column (petroleum ether/ethyl acetate (v/v) = 100 : 0-1 : 1) to afford a crude product (80 mg). The above crude product (80 mg), crude intermediate 1 (54 mg), TEA (97 mg, 0.96 mmol), CuI (6.1 mg, 0.032 mmol) and $PdCl_2(PPh_3)_2$ (11 mg, 0.016 mmol) were added to a reaction bottle, and 2 mL of DMF was added under nitrogen protection, and the mixture was heated to 50°C and reacted for 0.5 h. The reaction liquid was cooled to room temperature and added to 50 mL of ethyl acetate. The organic phase was washed with saturated aqueous sodium chloride solution (25 mL × 3), dried over anhydrous sodium sulfate, and concentrated under reduced pressure. The crude product was separated and purified with silica gel column chromatography (petroleum ether/dichloromethane/ethyl acetate (v/v) = 1 : 1 : 2) to afford compound 115 (40 mg, yield: 13%).

$^1H$ NMR (400 MHz, $CDCl_3$) $\delta$ 8.90 (s, 1H), 8.49 (d, 1H), 8.02 (s, 1H), 7.72 - 7.36 (m, 5H), 6.83 - 6.76 (m, 1H), 6.54 (dd, 1H), 6.27 (t, 1H), 5.14 - 4.84 (m, 2H), 4.43 - 4.26 (m, 2H), 4.18 - 4.06 (m, 2H), 3.96 - 3.74 (m, 2H), 3.00 - 2.00 (m, 8H).

LCMS m/z = 706.0 $[M+1]^+$.

**Example 116: Preparation of compound 116**

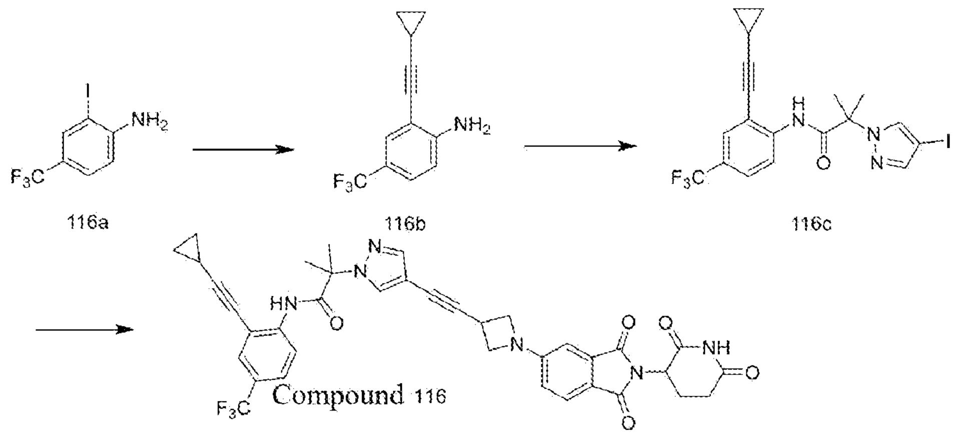

116a 116b 116c

Compound 116

Step 1: 2-(cyclopropylethynyl)-4-(trifluoromethyl)aniline (116b)

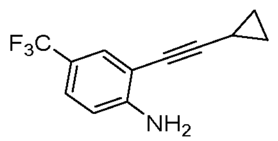

Cyclopropylacetylene (0.55 g, 8.32 mmol) and 116a (1.20 g, 4.18 mmol) were dissolved in 10 mL of DMF, and TEA (0.85 g, 8.40 mmol), $PdCl_2(PPh_3)_2$ (0.29 g, 0.41 mmol) and CuI (0.16 g, 0.84 mmol) were added in sequence under nitrogen protection, and the mixture was reacted at 70°C for 2 h. The reaction liquid was cooled to room temperature, 10 mL of ethyl acetate and 10 mL of water were added, the organic phase was separated, the aqueous phase was extracted with ethyl acetate (10 mL × 2), the organic phases were combined, the organic phase was washed with 10 mL of saturated aqueous sodium chloride solution, dried over anhydrous sodium sulfate, and concentrated under reduced pressure. The crude product was separated and purified with silica gel chromatography column (petroleum ether : ethyl acetate (v/v) = 10 : 1) to afford 116b (0.60 g, yield: 64%).

Step 2: N-(2-(cyclopropylethynyl)-4-(trifluoromethyl)phenyl)-2-(4-iodo-1H-pyrazol-1-yl)-2-methylpropanamide (116c)

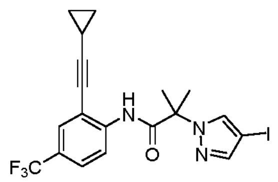

16a-1 (0.70 g, 2.50 mmol) was dissolved in 10 mL of DCM, 1-chloro-N,N,2-trimethylpropenylamine (0.50 g, 3.74 mmol) was added, the mixture was reacted at room temperature for 2 h, and then 116b (0.59 g, 2.62 mmol) and TEA (0.76 g, 7.51 mmol) were added in sequence, and the mixture was reacted at room temperature for 12 h. 10 mL of ethyl acetate and 10 mL of water were added to the reaction liquid, the organic phase was separated, the organic phase was washed with 10 mL of saturated aqueous sodium chloride solution, dried over anhydrous sodium sulfate, and concentrated under reduced pressure. The crude product was separated and purified with silica gel chromatography column (petroleum ether : ethyl acetate (v/v) = 5 : 1) to afford 116c (0.55 g, yield: 45%).

LCMS m/z = 488.0 $[M+1]^+$.

Step 3: N-(2-(cyclopropylethynyl)-4-(trifluoromethyl)phenyl)-2-(4-((1-(2-(2,6-dioxopiperidin-3 -yl)-1,3 -dioxoisoindolin-5 -yl)azetidin-3 -yl)ethynyl)- 1H-pyrazol-1-yl)-2-methylpropanamide (Compound 116)

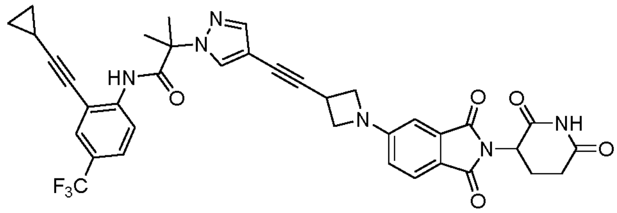

116c (0.55 g, 1.13 mmol) was dissolved in 15 mL of DMF, and intermediate 1 (0.46 g), TEA (0.34 g, 3.36 mmol), CuI (0.043 g, 0.23 mmol) and $PdCl_2(PPh_3)_2$ (0.079 g, 0.11 mmol) were added in sequence, and the mixture was reacted at 60°C for 2 h. The reaction liquid was cooled to room temperature, DCM (30 mL) and water (30 mL) were added, the organic phase was separated, the aqueous phase was extracted with DCM (30 mL $\times$ 3), the organic phases were combined, the organic phase was washed with 10 mL of saturated aqueous sodium chloride solution, dried over anhydrous sodium sulfate, and concentrated under reduced pressure. The crude product was separated and purified with silica gel chromatography column (petroleum ether : ethyl acetate (v/v) = 1 : 1) to afford compound 116 (70 mg, yield: 9%).

$^1H$ NMR (400 MHz, $CDCl_3$) $\delta$ 9.04 (s, 1H), 8.44 (d, 1H), 8.10 - 7.90 (m, 1H), 7.83 - 7.75 (m, 2H), 7.67 (d, 1H), 7.58 - 7.40 (m, 2H), 6.84 - 6.74 (m, 1H), 6.55 (dd, 1H), 5.00 - 4.88 (m, 1H), 4.43 - 4.30 (m, 2H), 4.10 - 3.96 (m, 2H), 3.90 - 3.72 (m, 1H), 3.00 - 2.64 (m, 3H), 2.20 - 2.06 (m, 1H), 1.94 (s, 6H), 1.63 - 1.50 (m, 1H), 1.04 - 0.87 (m, 4H).

LCMS m/z = 697.8 $[M+1]^+$.

**Example 117: Preparation of compound 117**

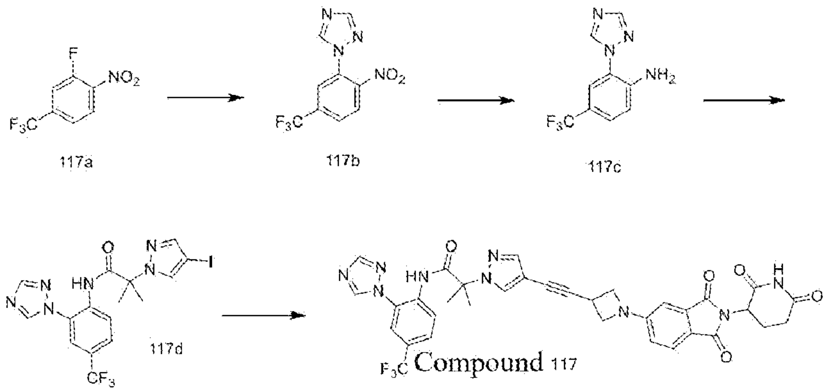

117a

117b

117c

117d

Compound 117

Step 1: 1-(2-nitro-5-(trifluoromethyl)phenyl)-1H-1,2,4-triazole (117b)

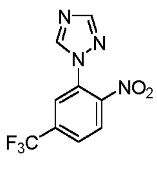

117a (3.0 g, 14.35 mmol), 1,2,4-triazole (1.49 g, 21.52 mmol), and potassium carbonate (3.97 g, 28.73 mmol) were added to 30 mL of DMF and the mixture was placed in a sealed tube and reacted at 80°C for 16 h. The reaction liquid was cooled to room temperature, 100 mL of water was added, the mixture was extracted with 100 mL of ethyl acetate, the organic phases were combined, the organic phase was washed with 50 mL of water, dried over anhydrous sodium sulfate, concentrated under reduced pressure, and separated and purified with silica gel chromatography column (petroleum ether/ethyl acetate (v/v) = 5 : 1) to afford 117b (1.8 g, yield: 49%).

LCMS m/z = 259.0 $[M+1]^+$.

Step 2: 2-(1H-1,2,4-triazol-1-yl)-4-(trifluoromethyl)aniline (117c)

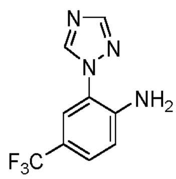

117b (0.8 g, 3.10 mmol) was dissolved in 10 mL of methanol, 0.2 g of 10% palladium on carbon was added, hydrogen replacement was performed three times, and the mixture was reacted at room temperature under the atmosphere of hydrogen balloon for 2 h. The reaction liquid was filtered, and the filtrate was concentrated under reduced pressure. The crude product was separated and purified with silica gel chromatography column (petroleum ether/ethyl acetate (v/v) = 10 : 1) to afford 117c (0.65 g, yield: 92%).

LCMS m/z = 229.0 $[M+1]^+$.

Step 3: N-(2-(1H-1,2,4-triazol-1-yl)-4-(trifluoromethyl)phenyl)-2-(4-iodo-1H-pyrazol-1-yl)-2-methylpropanamide (117d)

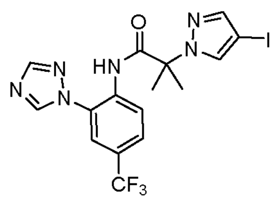

16a-1 (0.96 g, 3.44 mmol) was dissolved in 10 mL of DCM, 1-chloro-N,N,2-trimethylpropenylamine (0.61 g, 4.56 mmol) was added, the mixture was reacted at room temperature for 2 h, and then TEA (0.87 g, 8.60 mmol) and 117c (0.65 g, 2.85 mmol) were added in sequence, and the mixture was reacted at room temperature for 2 h. 150 mL of DCM and 50 mL of saturated aqueous sodium bicarbonate solution were added to the reaction liquid, the organic phase was separated, dried over anhydrous sodium sulfate, and concentrated under reduced pressure. The crude product was separated and purified with silica gel chromatography column (petroleum ether/ethyl acetate (v/v) = 5 : 1) to afford 117d (0.72 g, yield: 52%).

LCMS m/z = 491.4 $[M+1]^+$.

Step 4: N-(2-(1H-1,2,4-triazol-1-yl)-4-(trifluoromethyl)phenyl)-2-(4-((1-(2-(2,6-dioxopiperidin-3 -yl)-1,3 -dioxoisoindolin-5 -yl)azetidin-3 -yl)ethynyl)- 1H-pyrazol-1-yl)-2-methylpropanamide (Compound 117)

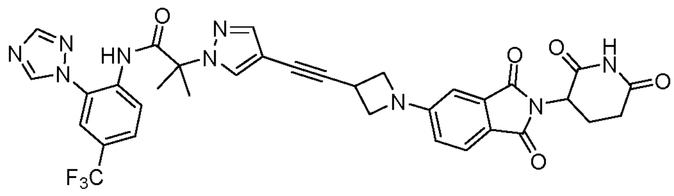

117d (0.3 g, 0.612 mmol), intermediate 1 (0.25 g), TEA (0.19 g, 1.88 mmol), CuI (12 mg, 0.063 mmol) and $PdCl_2(PPh_3)_2$ (43 mg, 0.061 mmol) were added to a reaction bottle, nitrogen replacement was performed three times, 5 mL of DMF was added under nitrogen protection, and the mixture was reacted at 50°C for 1 h. The reaction liquid was cooled to room temperature, 50 mL of water was added, and the mixture was filtered by suction. The filter cake was washed with 10 mL of water. The filter cake was dissolved in 100 mL of DCM, the mixture was dried over anhydrous sodium sulfate, and concentrated under reduced pressure. The crude product was separated and purified with silica gel

column chromatography (petroleum ether/ethyl acetate (v/v) = 1 : 1) to afford compound 117 (0.24 g, yield: 56%).

$^1$H NMR (400 MHz, $CDCl_3$) δ 9.69 (s, 1H), 8.70 (d, 1H), 8.40 (s, 1H), 8.21 - 8.07 (m, 2H), 7.78 - 7.62 (m, 3H), 7.60 - 7.52 (m, 2H), 6.86 - 6.77 (m, 1H), 6.61 - 6.53 (m, 1H), 4.99 - 4.88 (m, 1H), 4.45 - 4.30 (m, 2H), 4.17 - 4.03 (m, 2H), 3.91 - 3.78 (m, 1H), 2.97 - 2.60 (m, 3H), 2.19 - 2.07 (m, 1H), 1.87 (s, 6H).

LCMS m/z = 700.5 [M+1]$^+$.

**Example 118: Preparation of compound 118**

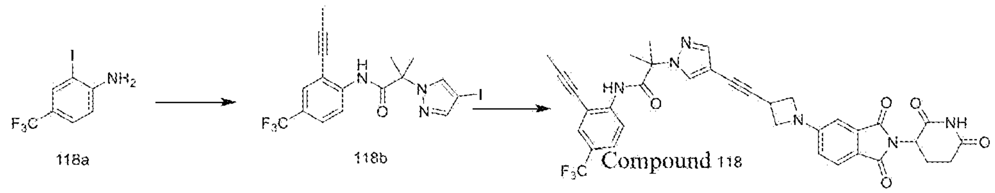


Step 1: 2-(4-iodo-1H-pyrazol-1-yl)-2-methyl-N-(2-(prop-1-yn-1-yl)-4-(trifluoromethyl)phenyl)propanamide (118b)

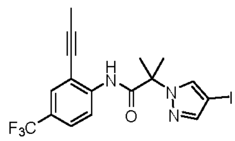

Propargyltrimethylsilane (0.94 g, 8.37 mmol) and 118a (1.20 g, 4.18 mmol) were dissolved in 10 mL of DMF, and TEA (0.85 g, 8.40 mmol), $PdCl_2(PPh_3)_2$ (0.29 g, 0.41 mmol) and CuI (0.16 g, 0.84 mmol) were added in sequence under nitrogen protection, and the mixture was placed in a sealed tube and reacted at 70°C for 2 h. The reaction liquid was cooled to room temperature, 10 mL of ethyl acetate and 10 mL of water were added, the organic phase was separated, the aqueous phase was extracted with ethyl acetate (10 mL × 2), the organic phases were combined, the organic phase was washed with 10 mL of saturated aqueous sodium chloride solution, dried over anhydrous sodium sulfate, and concentrated under reduced pressure. The crude product was separated and purified with silica gel chromatography column (petroleum ether : ethyl acetate (v/v) = 10 : 1) to afford crude 1 (1.00 g). Crude 1 (1.00 g) was dissolved in THF (10 mL), a solution of 1 mol/L TBAF in THF (5.90 mL, 5.90 mmol) was added, and the mixture was reacted at room temperature for 2 h. 10 mL of ethyl acetate and 10 mL of water were added to the reaction liquid, and the organic phase was separated. The organic phase was washed with 10 mL of saturated aqueous sodium chloride solution, dried over anhydrous sodium sulfate, and concentrated under reduced pressure to afford crude 2 (0.70 g). 16a-1 (0.90 g, 3.21 mmol) was dissolved in DCM (10 mL), 1-chloro-N,N,2-trimethylpropenylamine (0.64 g, 4.79 mmol) was added, the mixture was reacted at room temperature for 2 h, and then crude 2 (0.70 g) and TEA (0.97 g, 9.59 mmol) were added in sequence, and the mixture was reacted at room temperature for 12 h. 10 mL of ethyl acetate and 10 mL of water were added to the reaction liquid, the organic phase was separated, the organic phase was washed with 10 mL of saturated aqueous sodium chloride solution, dried over anhydrous sodium sulfate, and concentrated under reduced pressure. The crude product was separated and purified with silica gel chromatography column (petroleum ether : ethyl acetate (v/v) = 5 : 1) to afford 118b (0.28 g, yield: 19%).

LCMS m/z = 462.0 [M+1]$^+$.

Step 2: 2-(4-((1-(2-(2,6-dioxopiperidin-3-yl)-1,3-dioxoisoindolin-5-yl)azetidin-3-yl)ethynyl)-1H-pyrazol-1-yl)-2-methyl-N-(2-(prop-1-yn-1-yl)-4-(trifluoromethyl) phenyl)propanamide (Compound 118)

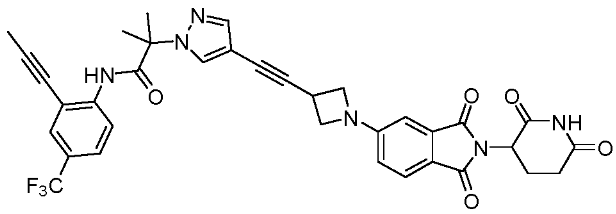

118b (0.28 g, 0.61 mmol) was dissolved in 5 mL of DMF, and intermediate 1 (0.25 g), TEA (0.19 g, 1.88 mmol), CuI (0.023 g, 0.12 mmol) and $PdCl_2(PPh_3)_2$ (0.043 g, 0.061 mmol) were added in sequence, and the mixture was reacted at 60°C for 2 h. The reaction liquid was cooled to room temperature, DCM (30 mL) was added, 30 mL of water was added, the organic phase was separated, the aqueous phase was extracted with DCM (30 mL × 3), the organic phases were combined, and the organic phase was washed with 10 mL of saturated aqueous sodium chloride solution, dried over anhydrous sodium sulfate, and concentrated under reduced pressure. The crude product was separated and purified with silica gel chromatography column (petroleum ether : ethyl acetate (v/v) = 1 : 1) to afford compound 118 (91 mg, yield: 22%).

$^1H$ NMR (400 MHz, $CDCl_3$) δ 8.84 (s, 1H), 8.48 (d, 1H), 7.96 (s, 1H), 7.80 (s, 1H), 7.74 (s, 1H), 7.70 - 7.64 (m, 1H), 7.60 - 7.53 (m, 1H), 7.52 - 7.44 (m, 1H), 6.84 - 6.76 (m, 1H), 6.56 (dd, 1H), 4.99 - 4.89 (m, 1H), 4.43 - 4.30 (m, 2H), 4.10 - 4.00 (m, 2H), 3.88 - 3.74 (m, 1H), 3.00 - 2.62 (m, 3H), 2.20 - 2.06 (m, 4H), 1.94 (s, 6H).

LCMS m/z = 671.5 $[M+1]^+$.

**Example 119: Preparation of compound 119**

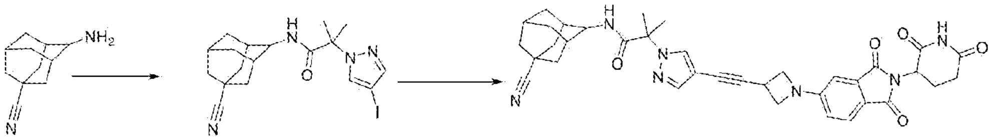

119a 119b Compound 119

Step 1: N-(5-cyanoadamantan-2-yl)-2-(4-iodo-1H-pyrazol-1-yl)-2-methylpropanamide (119b)

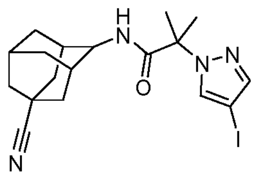

16a-1 (0.5 g, 1.79 mmol) was dissolved in 10 mL of DCM, 1-chloro-N,N,2-trimethylpropenylamine (0.36 g, 2.69 mmol) was added, the mixture was reacted at room temperature for 2 h, and then TEA (1.09 g, 10.77 mmol) and 119a (see US 20090192198 for the synthesis method) (0.32 g, 1.82 mmol) were added in sequence, and the mixture was reacted at room temperature for 12 h. 50 mL of DCM and 50 mL of water were added to the reaction liquid, the organic phase was separated, dried over anhydrous sodium sulfate, and concentrated under reduced pressure. The crude product was separated and purified with silica gel chromatography column (petroleum ether/ethyl acetate (v/v) = 1 : 0-3 : 1) to afford 119b (0.5 g, yield: 64%).

LCMS m/z = 439.0 $[M+1]^+$.

Step 2: N-(5-cyanoadamantan-2-yl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 119)

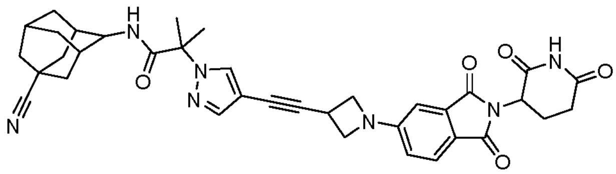

119b (0.41 g, 0.94 mmol), intermediate 1 (0.32 g), TEA (0.57 g, 5.63 mmol), CuI (36 mg, 0.189 mmol) and $PdCl_2(PPh_3)_2$ (66 mg, 0.094 mmol) were added to a reaction bottle, nitrogen replacement was performed three times, 5 mL of DMF was added under nitrogen protection, and the mixture was reacted at 55°C for 1.5 h. The reaction liquid was cooled to room temperature, 50 mL of water was added, and the mixture was filtered by suction. The filter cake was washed with 10 mL of water. The filter cake was dissolved in 100 mL of DCM, the mixture was dried over anhydrous sodium sulfate, and concentrated under reduced pressure. The crude product was separated and purified with silica gel chromatography column (petroleum ether/dichloromethane/ethyl acetate (v/v) = 1 : 1 : 2) to afford compound 119 (0.08 g, yield: 13%).

$^1H$ NMR (400 MHz, $CDCl_3$) δ 7.93 (s, 1H), 7.80 - 7.62 (m, 3H), 6.96 - 6.85 (m, 1H), 6.84 - 6.78 (m, 1H), 6.56 (dd, 1H), 4.99 - 4.89 (m, 1H), 4.41 - 4.30 (m, 2H), 4.12 - 4.01 (m, 2H), 3.95 - 3.74 (m, 2H), 3.00 - 2.64 (m, 3H), 2.20 - 2.05 (m, 5H), 2.04 - 1.98 (m, 2H), 1.98 - 1.89 (m, 3H), 1.83 (s, 6H), 1.56 - 1.49 (m, 4H).

LCMS m/z = 648.4 [M+1] $^+$.

**Example 120: Preparation of compound 120**

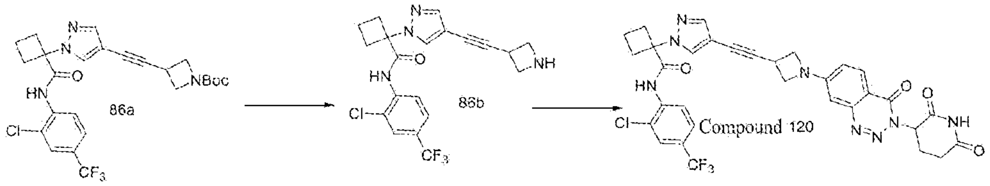


Step 1: 1-(4-(azetidin-3-ylethynyl)-1H-pyrazol-1-yl)-N-(2-chloro-4-(trifluoromethyl)phenyl) cyclobutane-1-carboxamide (86b) trifluoroacetate

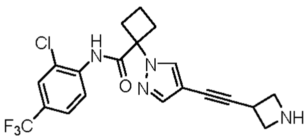

86a (80 mg, 0.153 mmol) was dissolved in 8 mL of DCM, and 2 mL of trifluoroacetic acid was added. The mixture was reacted at room temperature for 1 h. The reaction system was concentrated under reduced pressure to afford the trifluoroacetate of crude 86b (60 mg).

LCMS m/z = 423.2 [M+1] $^+$.

Step 2: N-(2-chloro-4-(trifluoromethyl)phenyl)-1-(4-((1-(3-(2,6-dioxopiperidin-3-yl)-4-oxo-3,4-dihydrobenzo[d][1,2,3]triazin-7-yl)azetidin-3-yl)ethynyl)-1H-pyrazol-1-yl)cyclobutane-1-carboxamide (Compound 120)

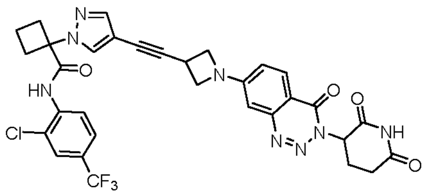

Crude 86b (60 mg) was dissolved in 6 mL of DMSO, sodium bicarbonate (38 mg, 0.45 mmol) was added, the mixture was stirred at room temperature for 20 min, and then 3-(7-fluoro-4-oxobenzo[d][1,2,3]triazin-3(4H)-yl)piperidine-2,6-dione (42 mg, 0.15 mmol) (see WO 2022019597 for the synthesis method) and DIPEA (58 mg, 0.45 mmol) were added in sequence, and the mixture was reacted at 110°C for 16 h. The reaction liquid was cooled to room temperature, 80 mL of water and DCM (30 mL) were added, the organic phase was separated, the aqueous phase was extracted with DCM (20 mL × 3), the organic phases were combined, and the organic phase was washed with 20 mL of saturated sodium chloride solution, dried over anhydrous sodium sulfate, and concentrated under reduced pressure. The crude product was separated and purified with prep-TLC (petroleum ether/ethyl acetate (v/v) = 1 : 3) to afford compound 120 (52 mg, yield: 51%).

$^1$H NMR (400 MHz, $CDCl_3$) δ 8.69 (s, 1H), 8.55 (d, 1H), 8.14 (d, 1H), 7.99 (s, 1H), 7.79 (s, 1H), 7.71 (s, 1H), 7.61 - 7.56 (m, 1H), 7.54 - 7.47 (m, 1H), 6.94 - 6.88 (m, 1H), 6.80 (dd, 1H), 5.84 - 5.72 (m, 1H), 4.46 - 4.35 (m, 2H), 4.18 - 4.07 (m, 2H), 3.93 - 3.77 (m, 1H), 3.19 - 2.68 (m, 7H), 2.47 - 1.97 (m, 3H).

LCMS m/z = 677.1 $[M-1]^-$

**Example 121: Preparation of compound 121**

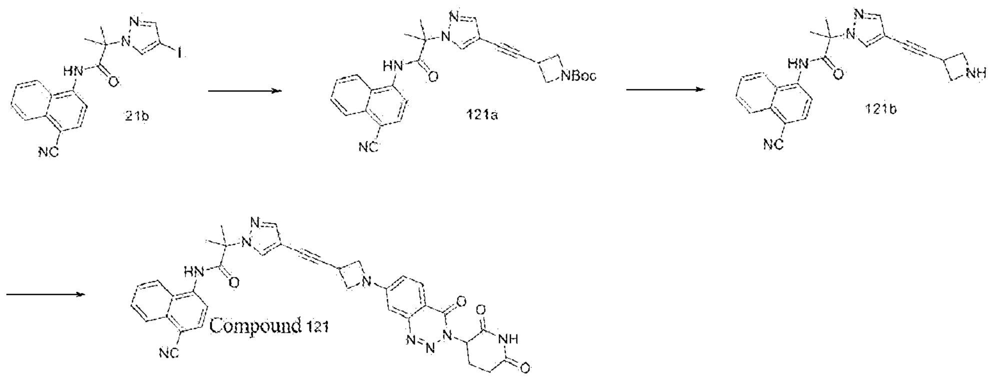

Step 1: tert-butyl 3-((1-(1-((4-cyanonaphthalen-1-yl)amino)-2-methyl-1-oxopropan-2-yl)-1H- pyrazol-4-yl)ethynyl)azetidine-1-carboxylate (121a)

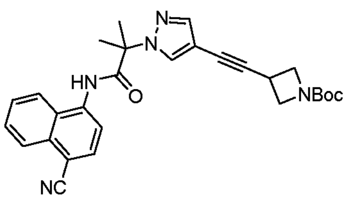

21b (0.5 g, 1.16 mmol) was dissolved in 10 mL of DMF, tert-butyl 3-ethynylazetidine-1-carboxylate (210 mg, 1.16 mmol) and TEA (352 mg, 3.48 mmol) were added in sequence, nitrogen replacement was performed three times, $PdCl_2(PPh_3)_2$ (81 mg, 0.115 mmol) and CuI (11 mg, 0.058 mmol) were added, nitrogen replacement was performed three times, and the mixture was reacted at 50°C for 2 h. The reaction liquid was cooled to room temperature, 50 mL of ethyl acetate and 100 mL of water were added, the organic phase was separated, the organic phase was washed with 50 mL of saturated sodium chloride solution, dried over anhydrous sodium sulfate, and concentrated under reduced

pressure. The crude product was separated and purified with silica gel chromatography column (petroleum ether/ethyl acetate (v/v) = 1 : 2) to afford 121a (0.37 g, yield: 66%).

LCMS m/z = 484.2 [M+1] $^+$.

Step 2; 2-(4-(azetidin-3-ylethynyl)-1H-pyrazol-1-yl)-N-(4-cyanonaphthalen-1-yl)-2-methylpropanamide (121b) trifluoroacetate

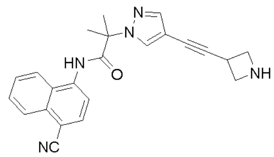

121a (80 mg, 0.166 mmol) was dissolved in 8 mL of DCM, and 2 mL of trifluoroacetic acid was added. The mixture was reacted at room temperature for 1 h. The reaction system was concentrated under reduced pressure to afford the trifluoroacetate of crude 121b (62 mg).

LCMS m/z = 384.2 [M+1] $^+$.

Step 3: N-(4-cyanonaphthalen-1-yl)-2-(4-((1-(3-(2,6-dioxopiperidin-3-yl)-4-oxo-3,4-dihydrobenzo[d][1,2,3]triazin-7-yl)azetidin-3-yl)ethynyl)-1H-pyrazol-1-yl)-2-methylpropanamide (Compound 121)

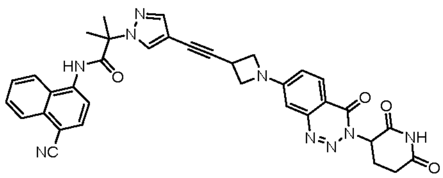

The trifluoroacetate of crude 121b (62 mg) was dissolved in 6 mL of DMSO, sodium bicarbonate (39 mg, 0.46 mmol) was added, the mixture was stirred at room temperature for 20 min, and then 3-(7-fluoro-4-oxobenzo[d][1,2,3]triazin-3(4H)-yl)piperidine-2,6-dione (44 mg, 0.16 mmol) (see WO 2022019597 for the synthesis method) and DIPEA (62 mg, 0.48 mmol) were added in sequence, and the mixture was reacted at 110°C for 16 h. The reaction liquid was cooled to room temperature, 70 mL of water and DCM (30 mL) were added, the organic phase was separated, the aqueous phase was extracted with DCM (20 mL × 3), the organic phases were combined, and the organic phase was washed with 20 mL of saturated sodium chloride solution, dried over anhydrous sodium sulfate, and concentrated under reduced pressure. The crude product was separated and purified with prep-TLC (petroleum ether/ethyl acetate (v/v) = 1 : 4) to afford compound 121 (46 mg, yield: 45%).

$^1$H NMR (400 MHz, $CDCl_3$) δ 9.92 (s, 1H), 8.40 - 8.30 (m, 1H), 8.30 - 8.22 (m, 1H), 8.14 (d, 1H), 8.00 (s, 1H), 7.96 - 7.78 (m, 4H), 7.76 - 7.60 (m, 2H), 6.94 - 6.89 (m, 1H), 6.80 (dd, 1H), 5.84 - 5.70 (m, 1H), 4.49 - 4.37 (m, 2H), 4.18 - 4.06 (m, 2H), 3.93 - 3.78 (m, 1H), 3.05 - 2.72 (m, 3H), 2.44 - 2.32 (m, 1H), 2.01 (s, 6H).

LCMS m/z = 640.2 [M+1] $^+$.

**Example 122: Preparation of compound 122**

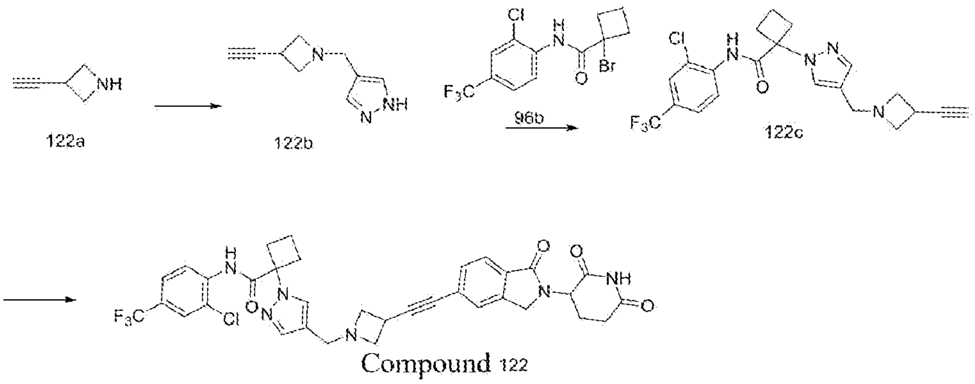


122a 122b 122c

Compound 122

Step 1: 4-((3-ethynylazetidin-1-yl)methyl)-1H-pyrazole (122b)

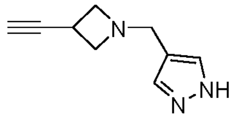

The hydrochloride of 122a (see WO 2022007903 for the synthesis method) (2.00 g, 17.01 mmol), potassium bicarbonate (1.87 g, 18.7 mmol) and 50 mL of THF were added into a reaction flask respectively, the mixture was stirred at room temperature for 1 h, then 1H-pyrazole-4-carbaldehyde (1.81 g, 18.84 mmol) and glacial acetic acid (3.06 g, 51.0 mmol) were added, and the mixture was reacted at room temperature for 2 h. The reaction system was cooled to 0°C and sodium triacetoxyborohydride (7.21 g, 34.02 mmol) was added, the mixture was slowly returned to room temperature and reacted for 16 h. Potassium bicarbonate (15.02 g, 150.2 mmol) was added to the reaction system, then the mixture was dried by adding anhydrous sodium sulfate, filtered through diatomaceous earth pad to remove insoluble matter, the filtrate was concentrated under reduced pressure, and the crude product was separated and purified with silica gel chromatography column (eluted first with ethyl acetate/petroleum ether (v/v) = 1 : 1, then with DCM/MeOH (v/v) = 5:1) to afford 122b (2.50 g, yield: 91%).

LCMS m/z = 162.2 $[M+1]^+$.

Step 2: N-(2-chloro-4-(trifluoromethyl)phenyl)-1-(4-((3-ethynylazetidin-1-yl)methyl)- 1H-pyrazol-1-yl)cyclobutane-1 -carboxamide (122c)

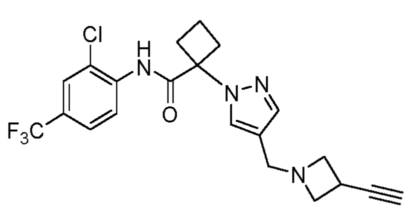

122b (1.00 g, 6.20 mmol), 96b (2.21 g, 6.20 mmol) and cesium carbonate (4.04 g, 12.4 mmol) were added to a reaction flask respectively, 20 mL of acetonitrile was added, and the mixture was reacted at 50°C for 4 h. The reaction liquid was cooled to room temperature and filtered, and the filtrate was concentrated under reduced pressure. The crude product was separated and purified with silica gel chromatography column (eluted with ethyl acetate/petroleum ether (v/v) = 1 : 1 and DCM/MeOH (v/v) = 20 : 1 sequentially) to afford 122c (0.45 g, yield: 17%).

LCMS m/z = 437.1 $[M+1]^+$.

Step 3: N-(2-chloro-4-(trifluoromethyl)phenyl)-1-(4-((3-((2-(2,6-dioxopiperidin-3-yl)-1-oxoisoindolin-5-yl)ethynyl)azetidin-1-yl)methyl)-1H-pyrazol-1-yl)cyclobutane-1-carboxamide (Compound 122)

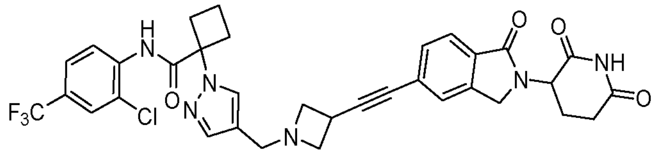

Under nitrogen atmosphere, 3-(5-bromo-1-oxoisoindolin-2-yl)piperidine-2,6-dione (160 mg, 0.50 mmol), 122c (218 mg, 0.50 mmol), Pd(dppf)$Cl_2$•DCM (41 mg, 0.0506 mmol), CuI (9.5 mg, 0.05 mmol) and DIPEA (650 mg, 5.03 mmol) were added into a reaction bottle in sequence, 5 mL of DMF was added, and the mixture was reacted at 80°C for 16 h. The reaction liquid was cooled to room temperature, 20 mL of water was added, the mixture was extracted with ethyl acetate (40 mL × 3), the organic phases were combined, the organic phase was washed with 20 mL of saturated aqueous sodium chloride solution, dried over anhydrous sodium sulfate, and concentrated under reduced pressure. The crude product was separated and purified with silica gel chromatography column (DCM/MeOH (v/v) = 10 : 1). The crude product obtained after purification was further separated and purified with silica gel chromatography column (DCM/MeOH (v/v) = 20 : 1) to afford compound 122 (14 mg, yield: 4%).

$^1$H NMR (400 MHz, $CDCl_3$) δ 8.71 (s, 1H), 8.57 (d, 1H), 8.34 (br.s, 1H), 7.81 (d, 1H), 7.69 (s, 1H), 7.62 - 7.44 (m, 5H), 5.20 (dd, 1H), 4.52 - 4.42 (m, 1H), 4.36 - 4.26 (m, 1H), 3.80 - 3.68 (m, 2H), 3.63 (s, 2H), 3.58 - 3.44 (m, 1H), 3.34 - 3.18 (m, 2H), 3.14 - 3.00 (m, 2H), 2.97 - 2.72 (m, 4H), 2.44 - 2.00 (m, 4H).

LCMS m/z = 679.2 $[M+1]^+$.

**Example 123: Preparation of compound 123**

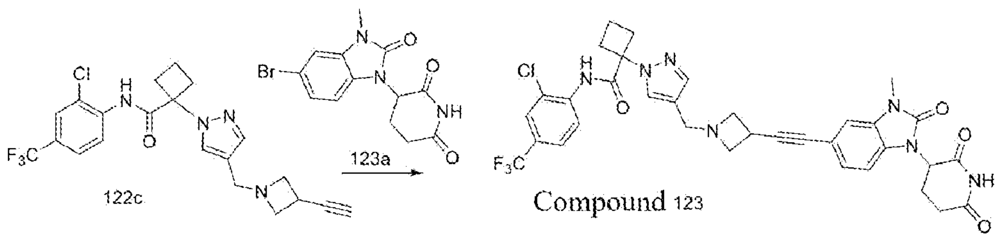


Under nitrogen atmosphere, 123a (85 mg, 0.25 mmol) (see WO 2021188696 for the synthesis method), 122c (218 mg, 0.50 mmol), $PdCl_2(PPh_3)_2$ (70 mg, 0.10 mmol), CuI (19 mg, 0.10 mmol), cesium carbonate (650 mg, 2.00 mmol) and 4 Å molecular sieve (200 mg) were added into a reaction bottle respectively, DMF (5 mL) was added, and the mixture was reacted at 80°C for 5 h. The reaction system was cooled to room temperature and added to 20 mL of water, the mixture was extracted with ethyl acetate (40 mL × 3), the organic phases were combined, the organic phase was washed with 20 mL of saturated sodium chloride solution, dried over anhydrous sodium sulfate, and concentrated under reduced pressure, the crude product was separated and purified with silica gel column chromatography (DCM/MeOH (v/v) = 10 : 1), and the crude product obtained after purification was further separated and purified with silica gel column chromatography (DCM/MeOH (v/v) = 20 : 1) to afford compound 123 (10 mg, yield: 6%).

$^1$H NMR (400 MHz, $CDCl_3$) δ 8.70 (s, 1H), 8.63 - 8.30 (m, 2H), 7.68 (s, 1H), 7.61 - 7.54 (m, 2H), 7.53 - 7.46 (m, 1H), 7.16 - 7.09 (m, 1H), 7.08 - 7.03 (m, 1H), 6.72 (d, 1H), 5.18 (dd, 1H), 3.78 - 3.66 (m, 2H), 3.61 (s, 2H), 3.54 - 3.45 (m, 1H), 3.41 (s, 3H), 3.30 - 3.16 (m, 2H), 3.12 - 2.65 (m, 7H), 2.30 - 2.16 (m, 2H), 2.15 - 2.01 (m, 1H).

LCMS m/z = 694.2 $[M+1]^-$.

**Example 124: Preparation of compound 124**

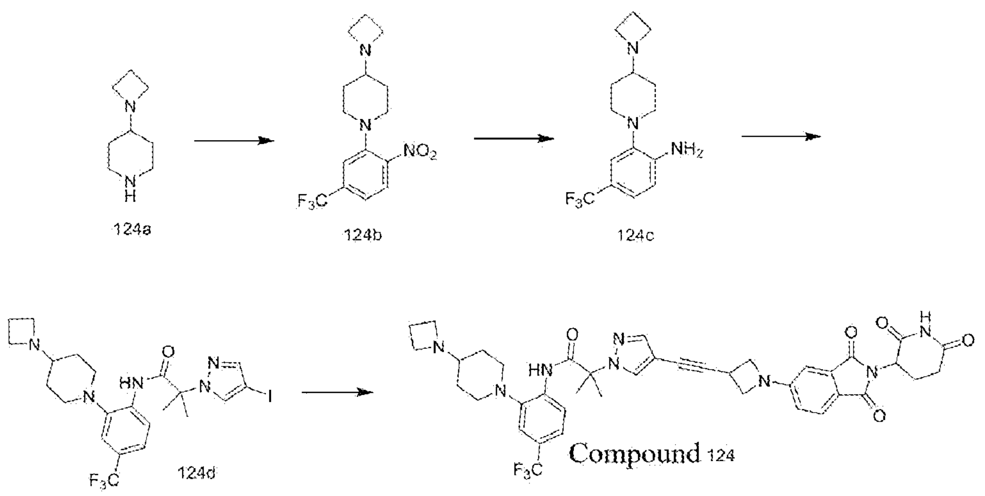

124a 124b 124c

124d Compound 124

Step 1: 4-(azetidin-1-yl)-1-(2-nitro-5-(trifluoromethyl)phenyl)piperidine (124b)

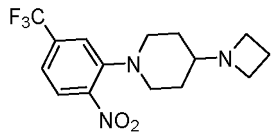

2-fluoro-1-nitro-4-(trifluoromethyl)benzene (0.5 g, 2.39 mmol) and the hydrochloride of 124a (0.56 g) were dissolved in 20 mL of DMF, potassium carbonate (0.82 g, 5.93 mmol) was added, and the mixture was reacted at 80°C for 6 h. The reaction liquid was cooled to room temperature, 50 mL of water was added, the mixture was extracted with 100 mL of ethyl acetate, the organic phases were combined, the organic phase was washed with 50 mL of water, dried over anhydrous sodium sulfate, concentrated under reduced pressure, and the crude product was separated and purified with silica gel chromatography column (DCM/MeOH (v/v) = 15 : 1) to afford 124b (0.4 g, yield: 51%).

LCMS m/z = 330.1 $[M+1]^+$.

Step 2: 2-(4-(azetidin-1-yl)piperidin-1-yl)-4-(trifluoromethyl)aniline (124c)

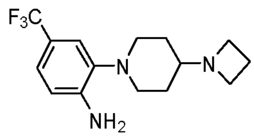

124b (0.2 g, 0.61 mmol) was dissolved in 30 mL of methanol, 0.1 g of 10% palladium on carbon was added, hydrogen replacement was performed three times, and the mixture was reacted at room temperature under the atmosphere of hydrogen balloon for 1 h. The reaction liquid was filtered, and the filtrate was concentrated under reduced pressure to afford crude 124c (0.2 g).

LCMS m/z = 300.3 $[M+1]^+$.

Step 3: N-(2-(4-(azetidin-1-yl)piperidin-1-yl)-4-(trifluoromethyl)phenyl)-2-(4-iodo-1H-pyrazol-1-yl)-2-methylpropanamide (124d)

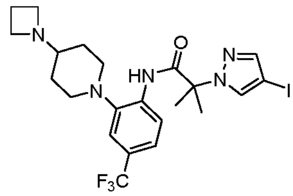

16a-1 (0.19 g, 0.68 mmol) was dissolved in 5 mL of DCM, 1-chloro-N,N,2-trimethylpropenylamine (0.12 g, 0.90 mmol) was added, the mixture was reacted at room temperature for 2 h, and then crude 124c (0.2 g,) and TEA (0.18 g, 1.78 mmol) were added in sequence, and the mixture was reacted at room temperature for 16 h. 80 mL of ethyl acetate and 30 mL of saturated aqueous sodium bicarbonate solution were added to the reaction liquid, the organic phase was separated, the organic phase was washed with 30 mL of saturated aqueous sodium chloride solution, dried over anhydrous sodium sulfate, and concentrated under reduced pressure. The crude product was separated and purified with silica gel chromatography column (DCM/MeOH (v/v) = 15 : 1) to afford 124d (0.07 g, yield: 18%).

Step 4: N-(2-(4-(azetidin-1-yl)piperidin-1-yl)-4-(trifluoromethyl)phenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 124)

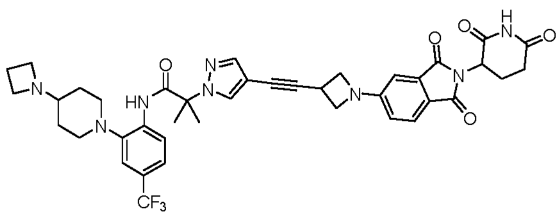

124d (0.07 g, 0.12 mmol) was dissolved in 5 mL of DMF, and intermediate 1 (0.045 g), TEA (0.036 g, 0.36 mmol), CuI (0.003 g, 0.016 mmol) and $PdCl_2(PPh_3)_2$ (0.009 g, 0.013 mmol) were added in sequence, nitrogen replacement was performed three times, and the mixture was reacted at 55°C for 1 h. The reaction liquid was cooled to room temperature, 30 mL of water was added, the solid was precipitated, filtration was performed, the filter cake was washed with 10 mL of water, the filter cake was dissolved with 50 mL of DCM, the mixture was dried over anhydrous sodium sulfate, and concentrated under reduced pressure. The crude product was separated and purified with silica gel chromatography column (DCM/MeOH (v/v) = 10 : 1) to afford compound 124 (0.030 g, yield: 32%).

$^1H$ NMR (400 MHz, $CDCl_3$) δ 9.33 (s, 1H), 8.47 - 8.05 (m, 2H), 7.96 - 7.72 (m, 2H), 7.66 (d, 1H), 7.39 - 7.31 (m, 2H), 6.84 - 6.74 (m, 1H), 6.55 (dd, 1H), 4.99 - 4.87 (m, 1H), 4.43 - 4.27 (m, 2H), 4.13 - 4.00 (m, 2H), 3.90 - 3.71 (m, 1H), 3.70 - 3.06 (m, 4H), 3.05 - 2.53 (m, 7H), 2.50 - 2.05 (m, 4H), 2.00 - 1.50 (m, 10H).

LCMS m/z = 771.2 $[M+1]^+$.

**Example 125: Preparation of compound 125**

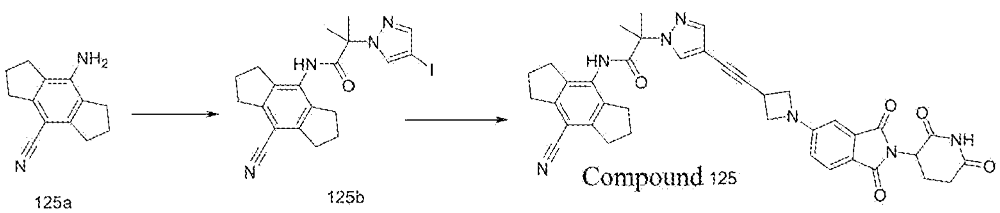
125a 125b Compound 125

Step 1: N-(8-cyano-1,2,3,5,6,7-hexahydro-s-indacen-4-yl)-2-(4-iodo-1H-pyrazol-1-yl)-2-methylpropanamide (125b)

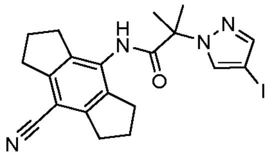

16a-1 (0.5 g, 1.79 mmol) was dissolved in 10 mL of DCM, 1-chloro-N,N,2-trimethylpropenylamine (0.36 g, 2.69 mmol) was added, the mixture was reacted at room temperature for 2 h, and then TEA (1.09 g, 10.77 mmol) and 125a (see WO 2020102576 for the synthesis method) (0.35 g, 1.77 mmol) were added in sequence, and the mixture was reacted at room temperature for 12 h. 50 mL of DCM and 50 mL of water were added to the reaction liquid, the organic phase was separated, dried over anhydrous sodium sulfate, and concentrated under reduced pressure. The crude product was separated and purified with silica gel chromatography column (petroleum ether/ethyl acetate (v/v) = 20 : 1) to afford 125b (0.5 g, yield: 62%).

LCMS m/z = 461.0 $[M+1]^+$.

Step 2: N-(8-cyano-1,2,3,5,6,7-hexahydro-s-indacen-4-yl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 125)

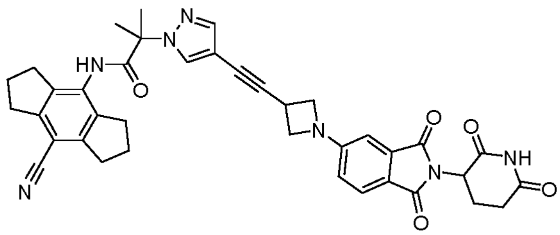

125b (0.43 g, 0.935 mmol), intermediate 1 (0.32 g), TEA (0.57 g, 5.63 mmol), CuI (36 mg, 0.189 mmol) and $PdCl_2(PPh_3)_2$ (66 mg, 0.094 mmol) were added to a reaction bottle, nitrogen replacement was performed three times, 5 mL of DMF was added under nitrogen protection, and the mixture was reacted at 60°C for 3 h. The reaction liquid was cooled to room temperature, 50 mL of water was added, and the mixture was filtered by suction. The filter cake was washed with 10 mL of water. The filter cake was dissolved in 100 mL of DCM, the mixture was dried over anhydrous sodium sulfate, and concentrated under reduced pressure. The crude product was separated and purified with silica gel chromatography column (petroleum ether/dichloromethane/ethyl acetate (v/v) = 1 : 1 : 2) to afford compound 125 (0.07 g, yield: 11%).

$^1H$ NMR (400 MHz, $CDCl_3$) δ 8.20 (s, 1H), 8.02 (s, 1H), 7.85 - 7.73 (m, 2H), 7.67 (d, 1H), 6.86 - 6.76 (m, 1H), 6.62 - 6.50 (m, 1H), 5.00 - 4.86 (m, 1H), 4.45 - 4.27 (m, 2H), 4.15 - 4.00 (m, 2H), 3.90 - 3.71 (m, 1H), 3.14 - 2.60 (m, 11H), 2.20 - 2.04 (m, 5H), 1.93 (s, 6H).

LCMS m/z = 670.7 $[M+1]^+$.

**Example 126: Preparation of compound 126**

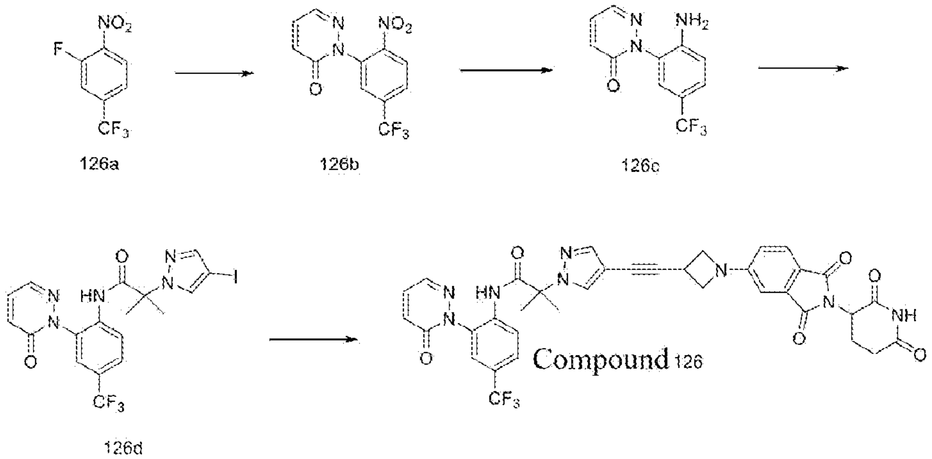

126a 126b 126c

126d Compound 126

Step 1: 2-(2-nitro-5-(trifluoromethyl)phenyl)pyridazin-3(2H)-one (126b)

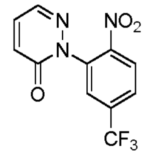

3-pyridazinone (0.46 g, 4.79 mmol) was dissolved in 15 mL of DMF, 60% sodium hydride (83 mg) was added, the mixture was reacted at room temperature for 1 h, then 126a (1.0 g, 4.78 mmol) was added, and the mixture was reacted at room temperature for 1.5 h, then 60 mL of ethyl acetate and 150 mL of water were added, the organic phase was separated and the organic phase was washed with 50 mL of saturated aqueous sodium chloride solution, dried over anhydrous sodium sulfate, and concentrated under reduced pressure. The crude product was separated and purified with silica gel chromatography column (petroleum ether : ethyl acetate (v/v) = 5 : 1) to afford 126b (1.2 g, yield: 88%).

LCMS m/z = 286.1 $[M+1]^+$.

Step 2; 2-(2-amino-5-(trifluoromethyl)phenyl)pyridazin-3(2H)-one (126c)

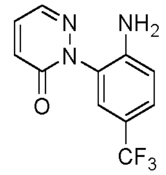

126b (0.3 g, 1.05 mmol) was dissolved in 20 mL of methanol, 60 mg of 10% palladium on carbon was added, hydrogen replacement was performed three times, and the mixture was reacted at room temperature under the atmosphere of hydrogen balloon for 3 h. The reaction liquid was filtered, and the filtrate was concentrated under reduced pressure to afford crude 126c (200 mg).

LCMS m/z = 256.2 $[M+1]^+$.

Step 3: 2-(4-iodo-1H-pyrazol-1-yl)-2-methyl-N-(2-(6-oxopyridazin-1(6H)-yl)-4-(trifluoromethyl)phenyl)propanamide (126d)

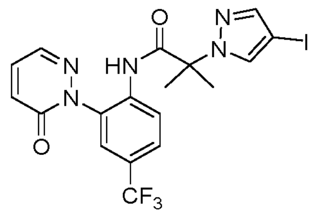

16a-1 (0.23 g, 0.82 mmol) was dissolved in 20 mL of DCM, 1-chloro-N,N,2-trimethylpropenylamine (164 mg, 1.23 mmol) was added, the mixture was reacted at room temperature for 1 h, and then TEA (248 mg, 2.44 mmol) and crude 126c (200 mg) were added in sequence, and the mixture was reacted at room temperature for 12 h. 30 mL of ethyl acetate and 20 mL of water were added to the reaction liquid, the organic phase was separated, the organic phase was washed with 20 mL of saturated aqueous sodium chloride solution, dried over anhydrous sodium sulfate, and concentrated under reduced pressure. The crude product was separated and purified with silica gel chromatography column (petroleum ether : ethyl acetate (v/v) = 4 : 1) to afford 126d (0.26 g, yield: 61%).

LCMS m/z = 518.2 $[M+1]^+$.

Step 4: 2-(4-((1-(2-(2,6-dioxopiperidin-3-yl)-1,3-dioxoisoindolin-5-yl)azetidin-3-yl)ethynyl)-1H-pyrazol-1-yl)-2-methyl-N-(2-(6-oxopyridazin-1(6H)-yl)-4-(trifluoromethyl)phenyl)propanamide (Compound 126)

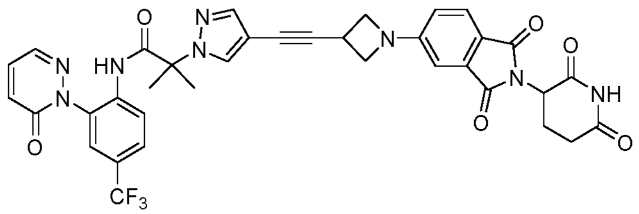

126d (100 mg, 0.19 mmol) was added to 10 mL of dry DMF, intermediate 1 (96 mg) and TEA (57 mg, 0.56 mmol) were added in sequence, nitrogen replacement was performed three times, $PdCl_2(PPh_3)_2$ (14 mg, 0.02 mmol) and CuI (7 mg, 0.037 mmol) were added, nitrogen replacement was performed three times, and the mixture was reacted at 60°C for 2 h. The reaction system was cooled to room temperature, 100 mL of saturated aqueous ammonium chloride solution was slowly added, a yellow solid was precipitated, filtration was performed, the filter cake was washed with 20 mL of water three times, the filter cake was dissolved in 100 mL of DCM, the mixture was dried over anhydrous sodium sulfate and concentrated under reduced pressure, the crude product was separated and purified with silica gel chromatography column (petroleum ether : ethyl acetate (v/v) = 1 : 5) to afford compound 126 (65 mg, yield: 47%).

$^1H$ NMR (400 MHz, $CDCl_3$) δ 8.60 (s, 1H), 8.33 - 8.23 (m, 1H), 8.00 - 7.88 (m, 2H), 7.73 - 7.52 (m, 4H), 7.44 - 7.32 (m, 2H), 7.15 - 7.05 (m, 1H), 6.87 - 6.77 (m, 1H), 6.63 - 6.51 (m, 1H), 4.99 - 4.87 (m, 1H), 4.45 - 4.30 (m, 2H), 4.15 - 4.00 (m, 2H), 3.89 - 3.75 (m, 1H), 2.96 - 2.64 (m, 3H), 2.20 - 2.08 (m, 1H), 1.92 - 1.80 (m, 6H).

LCMS m/z = 727.2 $[M+1]^+$.

**Example 127: Preparation of compound 127**

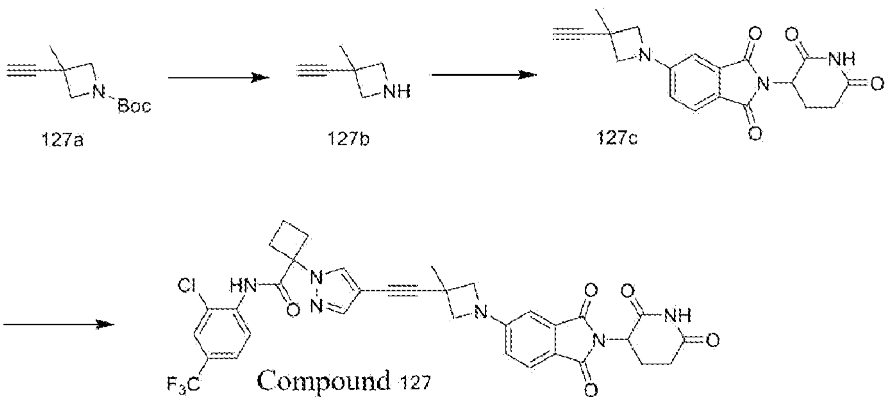

127a 127b 127c

Compound 127

Step 1: 3-ethynyl-3-methylazetidine (127b) hydrochloride

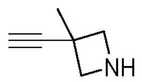

127a (0.7 g, 3.58 mmol) (see WO 2021030711 for the synthesis method) was dissolved in 20 mL of a solution of 4 mol/L ethyl acetate hydrogen chloride solution, and the mixture was reacted at room temperature for 12 h. The reaction liquid was concentrated under reduced pressure to afford the hydrochloride of crude 127b (425 mg).

Step 2: 2-(2,6-dioxopiperidin-3-yl)-5-(3-ethynyl-3-methylazetidin-1-yl)isoindoline-1,3-dione (127c)

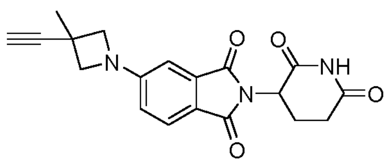

The hydrochloride of crude 127b was dissolved in 5 mL of DMSO, 1C (0.99 g, 3.59 mmol) and DIPEA (2.32 g, 17.95 mmol) were added, and the mixture was reacted at 100°C for 3 h. The reaction liquid was cooled to room temperature and added to 100 mL of water, the mixture was filtered, the filter cake was dissolved in 100 mL of DCM, the mixture was dried over anhydrous sodium sulfate, and concentrated under reduced pressure to afford crude 127c (0.6 g).

LCMS m/z = 352.1 [M+1] $^+$.

Step 3: N-(2-chloro-4-(trifluoromethyl)phenyl)-1-(4-((1-(2-(2,6-dioxopiperidin-3-yl)-1,3-dioxoisoindolin-5-yl)-3-methylazetidin-3-yl)ethynyl)-1H-pyrazol-1-yl)cyclobutane-1-carboxamide (Compound 127)

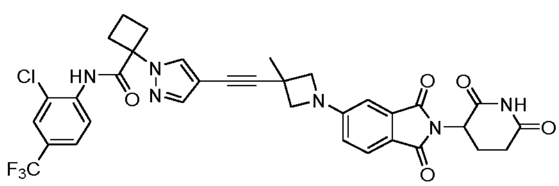

The crude products 127c (0.15 g), 14c (0.21 g, 0.447 mmol), TEA (0.27 g, 2.67 mmol), CuI (17 mg, 0.0893 mmol) and $PdCl_2(PPh_3)_2$ (31 mg, 0.044 mmol) were added to a reaction bottle, 5 mL of DMF was added under nitrogen protection, and the mixture was reacted at 60°C for 3 h. The reaction liquid was cooled to room temperature, 50 mL of

water was added, and the mixture was filtered by suction. The filter cake was washed with 10 mL of water. The filter cake was dissolved in 100 mL of DCM, the mixture was dried over anhydrous sodium sulfate, and concentrated under reduced pressure. The crude product was separated and purified with prep-TLC (ethyl acetate/petroleum ether/dichloromethane (v/v) = 2 : 1 : 1) to afford compound 127 (0.05 g, yield: 16%).

$^1H$ NMR (400 MHz, $CDCl_3$) δ 8.71 (s, 1H), 8.54 (d, 1H), 7.93 (s, 1H), 7.78 (s, 1H), 7.73 - 7.63 (m, 2H), 7.62 - 7.55 (m, 1H), 7.53 - 7.46 (m, 1H), 6.83 - 6.77 (m, 1H), 6.54 (dd, 1H), 4.98 - 4.87 (m, 1H), 4.26 - 4.16 (m, 2H), 4.00 - 3.92 (m, 2H), 3.15 - 3.00 (m, 2H), 2.97 - 2.65 (m, 5H), 2.31 - 2.00 (m, 3H), 1.72 (s, 3H).

LCMS m/z = 693.0 [M+1] $^+$.

**Example 128: Preparation of compound 128**

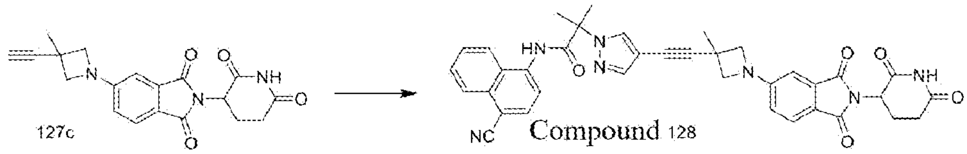
127c
Compound 128

The crude products 127c (0.15 g), 21b (0.19 g, 0.44 mmol), TEA (0.27 g, 2.67 mmol), CuI (17 mg, 0.089 mmol) and $PdCl_2(PPh_3)_2$ (31 mg, 0.044 mmol) were added to a reaction bottle, 5 mL of DMF was added under nitrogen protection, and the mixture was reacted at 60°C for 3 h. The reaction liquid was cooled to room temperature, 50 mL of water was added, and the mixture was filtered by suction. The filter cake was washed with 10 mL of water. The filter cake was dissolved in 100 mL of DCM, the mixture was dried over anhydrous sodium sulfate, and concentrated under reduced pressure. The crude product was separated and purified with prep-TLC (ethyl acetate/petroleum ether/dichloromethane (v/v) = 2 : 1 : 1) to afford **compound 128** (0.06 g, yield: 21%).

$^1H$ NMR (400 MHz, $CDCl_3$) δ 9.92 (s, 1H), 8.39 - 8.31 (m, 1H), 8.30 - 8.21 (m, 1H), 8.00 - 7.80 (m, 5H), 7.75 - 7.62 (m, 3H), 6.84 - 6.75 (m, 1H), 6.54 (dd, 1H), 4.98 - 4.87 (m, 1H), 4.25 - 4.15 (m, 2H), 4.03 - 3.91 (m, 2H), 2.95 - 2.64 (m, 3H), 2.19 - 2.08 (m, 1H), 2.01 (s, 6H), 1.72 (s, 3H).

LCMS m/z = 654.3 [M+1] $^+$.

**Example 129: Preparation of compound 129**

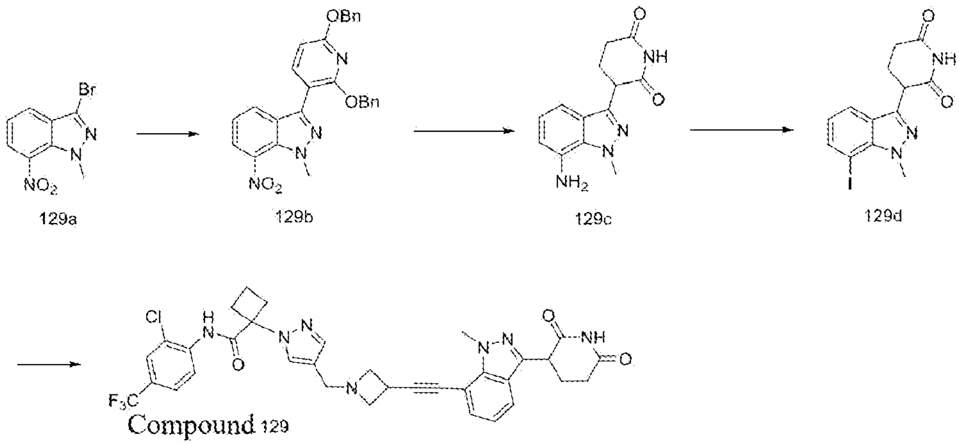
129a 129b 129c 129d
Compound 129

Step 1: 3-(2,6-bis(benzyloxy)pyridin-3-yl)-1-methyl-7-nitro-1H-indazole (129b)

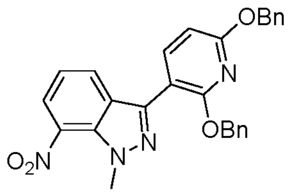

129a (3 g, 11.72 mmol), 2,6-bis(benzyloxy)-3-(4,4,5,5-tetramethyl-1,3,2-dioxaborolan-2-yl)pyridine (see WO 2021262812 for the synthesis method) (7.3 g, 17.5 mmol), 1,4-dioxane (80 mL), water (20 mL), Pd(dppf)$Cl_2$•DCM (1.4 g, 1.72 mmol) and cesium carbonate (11.5 g, 35.3 mmol) were added in sequence to a 50 mL single-mouth bottle under nitrogen protection, nitrogen replacement was performed three times, and the mixture was reacted at 100°C for 12 h. The reaction liquid was cooled to room temperature, poured into 50 mL of water, and the mixture was extracted with ethyl acetate (300 mL × 3). The organic phases were combined, dried over anhydrous sodium sulfate, and concentrated under reduced pressure. The crude product was separated and purified with silica gel chromatography column (ethyl acetate/petroleum ether (v/v) = 10 : 1) to afford 129b (3.3 g, yield: 60%).

LCMS m/z = 467.1 $[M+1]^+$.

Step 2: 3-(7-amino-1-methyl-1H-indazol-3-yl)piperidine-2,6-dione (129c)

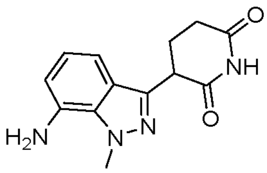

129b (1.6 g, 3.43 mmol), 10% palladium on carbon (0.9 g) and 10% palladium hydroxide/carbon (1.2 g) were dissolved in 20 mL of THF and 20 mL of methanol, hydrogen replacement was performed three times, and the mixture was reacted under the atmosphere of hydrogen balloon at 30°C for 16 h. The reaction liquid was cooled to room temperature, filtered through diatomaceous earth pad, and the filter cake was washed with 50 mL of methanol three times. The organic phases were combined, dried over anhydrous sodium sulfate, and concentrated under reduced pressure to afford crude 129c (650 mg).

LCMS m/z = 259.2 $[M+1]^+$.

Step 3: 3-(7-iodo-1-methyl-1H-indazol-3-yl)piperidine-2,6-dione (129d)

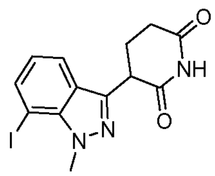

Crude 129c (250 mg), potassium iodide (242 mg, 1.46 mmol), CuI (0.269 g, 1.41 mmol), and iodine (371 mg, 1.46 mmol) were dissolved in acetonitrile (5 mL), and the mixture was cooled to 0°C. Isoamyl nitrite (171 mg, 1.45 mmol) was slowly added, and the mixture was reacted at 30°C for 8 h under nitrogen protection. 10 mL of saturated ammonium chloride solution was added to the reaction liquid, the mixture was extracted with 50 mL of DCM, the organic phase was dried over anhydrous sodium sulfate, and concentrated under reduced pressure. The crude product was separated and purified with silica gel chromatography column (petroleum ether/ethyl acetate (v/v) = 2 : 1-1 : 1) to afford 129d (130 mg, two-step yield from compound 129b: 27%).

LCMS m/z = 370.0 $[M+1]^+$.

Step 4: N-(2-chloro-4-(trifluoromethyl)phenyl)-1-(4-((3-((3-(2,6-dioxopiperidin-3-yl)-1-methyl-1H-indazol-7-yl)ethynyl)azetidin-1-yl)methyl)-1H-pyrazol-1-yl)cyclobutane-1-carboxamide (Compound 129)

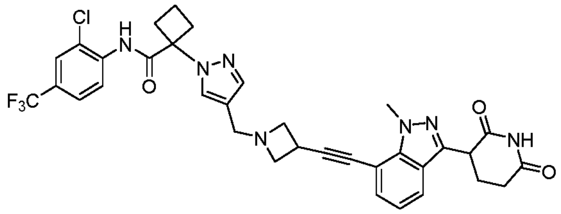

Under nitrogen atmosphere, 129d (0.13 g, 0.35 mmol), 122c (188 mg, 0.43 mmol), $PdCl_2(PPh_3)_2$ (51 mg, 0.073 mmol), CuI (14 mg, 0.0735 mmol) and DIPEA (0.47 g, 3.64 mmol) were added into a reaction bottle in sequence, 5 mL of DMF was added, and the mixture was reacted at 60°C for 5 h. The reaction liquid was cooled to room temperature, 20 mL of water was added, the mixture was filtered, the filter cake was collected, the filter cake was dissolved with 50 mL of DCM, the mixture was dried over anhydrous sodium sulfate, and concentrated under reduced pressure. The crude product was separated and purified with silica gel chromatography column (DCM/MeOH (v/v) = 10 : 1), and the crude product obtained after purification was further separated and purified with silica gel chromatography column (petroleum ether/ethyl acetate (v/v) = 1 : 3) to afford compound 129 (11 mg, yield: 4%).

$^1$H NMR (400 MHz, $CDCl_3$) δ 8.71 (s, 1H), 8.57 (d, 1H), 8.13 (s, 1H), 7.69 (s, 1H), 7.63 (d, 1H), 7.60 - 7.54 (m, 2H), 7.52 - 7.46 (m, 1H), 7.46 - 7.40 (m, 1H), 7.07 (dd, 1H), 4.34 (s, 3H), 4.28 (dd, 1H), 3.82 - 3.69 (m, 2H), 3.65 - 3.51 (m, 3H), 3.31 - 3.17 (m, 2H), 3.13 - 2.92 (m, 3H), 2.86 - 2.62 (m, 3H), 2.57 - 2.43 (m, 1H), 2.43 - 2.17 (m, 2H), 2.15 - 2.02 (m, 1H).

LCMS m/z = 678.2 [M+1] $^+$.

**Example 130: Preparation of compound 130**

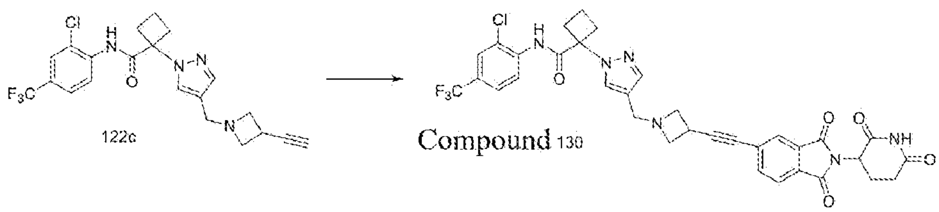

122c

Compound 130

Under nitrogen atmosphere, 5-bromo-2-(2,6-dioxopiperidin-3-yl)isoindoline-1,3-dione (120 mg, 0.36 mmol), 122c (188 mg, 0.43 mmol), $PdCl_2(PPh_3)_2$ (51 mg, 0.073 mmol), CuI (14 mg, 0.0735 mmol) and DIPEA (0.47 g, 3.64 mmol) were added into a reaction bottle in sequence, 5 mL of DMF was added, and the mixture was reacted at 60°C for 5 h. The reaction liquid was cooled to room temperature, 20 mL of water was added, the mixture was filtered, the filter cake was collected, the filter cake was dissolved with 50 mL of DCM, the mixture was dried over anhydrous sodium sulfate, and concentrated under reduced pressure. The crude product was separated and purified with silica gel chromatography column (DCM/MeOH (v/v) = 10 : 1), and the crude product obtained after purification was further separated and purified with silica gel chromatography column (petroleum ether/ethyl acetate (v/v) = 1 : 3) to afford compound 130 (40 mg, yield: 16%).

$^1$H NMR (400 MHz, $CDCl_3$) δ 8.71 (s, 1H), 8.57 (d, 1H), 8.22 (s, 1H), 7.86 (s, 1H), 7.84 - 7.77 (m, 1H), 7.77 - 7.45 (m, 5H), 5.02 - 4.91 (m, 1H), 3.90 - 3.40 (m, 5H), 3.35 - 2.65 (m, 9H), 2.32 - 2.02 (m, 3H).

LCMS m/z = 693.2 [M+1] $^+$.

**Example 131: Preparation of compound 131**

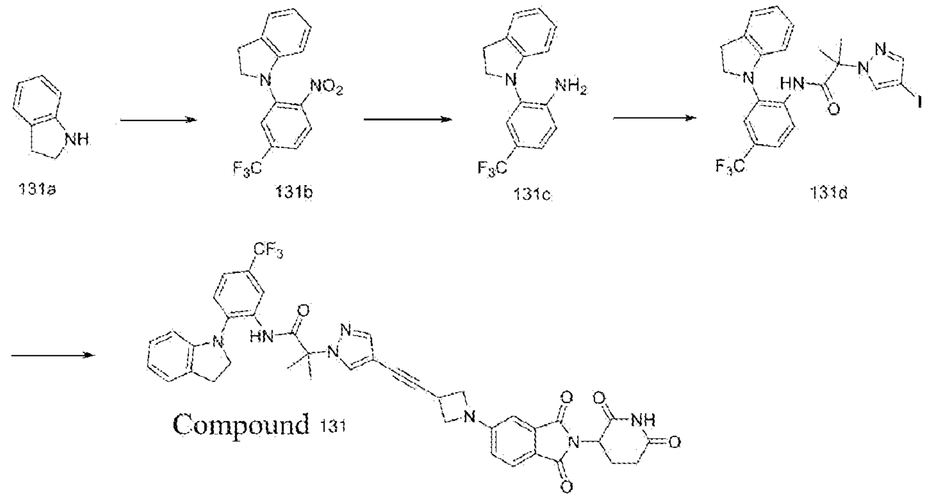

131a 131b 131c 131d

Compound 131

Step 1: 1-(2-nitro-5-(trifluoromethyl)phenyl)indoline (131b)

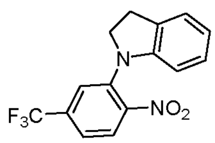

131a (0.88 g, 7.38 mmol), 2-fluoro-1-nitro-4-(trifluoromethyl)benzene (1.0 g, 4.78 mmol) and DIPEA (0.94 g, 7.27 mmol) were added to 10 mL of DMF, and the mixture was reacted at 75°C for 4 h. The reaction liquid was cooled to room temperature and added to 50 mL of water, the mixture was extracted with 50 mL of ethyl acetate, the organic phase was dried over anhydrous sodium sulfate, and concentrated under reduced pressure. The crude product was separated and purified with silica gel chromatography column (petroleum ether/ethyl acetate (v/v) = 100 : 1-20 : 1) to afford 131b (1.0 g, yield: 68%).

LCMS m/z = 309.0 $[M+1]^+$.

Step 2: 2-(indolin-1-yl)-4-(trifluoromethyl)aniline (131c)

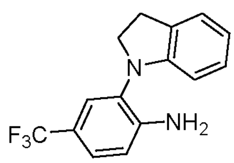

131b (0.8 g, 2.60 mmol) was dissolved in 20 mL of methanol, 0.2 g of 10% palladium on carbon was added, and the mixture was reacted at room temperature for 2 h under the atmosphere of hydrogen balloon. The reaction liquid was filtered, and the filtrate was concentrated under reduced pressure. The crude product was separated and purified with silica gel chromatography column (petroleum ether/ethyl acetate (v/v) = 100 : 1-20 : 1) to afford 131c (0.5 g, yield: 69%).

LCMS m/z = 279.1 $[M+1]^+$.

Step 3: N-(2-(indolin-1-yl)-4-(trifluoromethyl)phenyl)-2-(4-iodo-1H-pyrazol-1-yl)-2-methylpropanamide (131d)

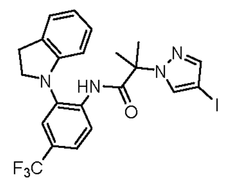

16a-1 (0.5 g, 1.78 mmol) was dissolved in 10 mL of DCM, 1-chloro-N,N,2-trimethylpropenylamine (0.36 g, 2.7 mmol) was added, the mixture was reacted at room temperature for 2 h, and then TEA (1.09 g, 10.77mmol) and 131c (0.5 g, 1.80 mmol) were added in sequence, and the mixture was reacted at room temperature for 12 h. 50 mL of DCM and 50 mL of water were added to the reaction liquid, the organic phase was separated, dried over anhydrous sodium sulfate, and concentrated under reduced pressure. The crude product was separated and purified with silica gel chromatography column (petroleum ether/dichloromethane/ethyl acetate (v/v) = 20 : 1-1 : 1) to afford 131d (0.51 g, yield: 53%).
LCMS m/z = 541.0 [M+1] +.

Step 4: 2-(4-((1-(2-(2,6-dioxopiperidin-3-yl)-1,3-dioxoisoindolin-5-yl)azetidin-3-yl)ethynyl)-1H-pyrazol-1-yl)-N-(2-(indolin-1-yl)-5-(trifluoromethyl)phenyl)-2-methylpropanamide (Compound 131)

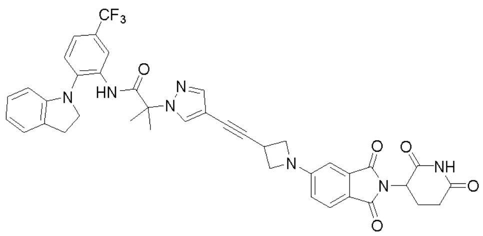

131d (0.51 g, 0.943 mmol), intermediate 1 (0.32 g), TEA (0.57 g, 5.63 mmol), CuI (36 mg, 0.189 mmol) and $PdCl_2(PPh_3)_2$ (66 mg, 0.094 mmol) were added to a reaction bottle, nitrogen replacement was performed three times, 5 mL of DMF was added under nitrogen protection, and the mixture was reacted at 60°C for 3 h. The reaction liquid was cooled to room temperature, 50 mL of water was added, and the mixture was filtered by suction. The filter cake was washed with 10 mL of water. The filter cake was dissolved in 100 mL of DCM, the mixture was dried over anhydrous sodium sulfate, and concentrated under reduced pressure. The crude product was separated and purified with silica gel chromatography column (ethyl acetate/petroleum ether/dichloromethane (v/v) = 2 : 1 : 1) to afford compound 131 (0.09 g, yield: 13%).
$^1H$ NMR (400 MHz, $CDCl_3$) δ 8.76 (s, 1H), 8.54 (d, 1H), 7.93 (s, 1H), 7.69 (d, 1H), 7.55 - 7.41 (m, 3H), 7.32 (s, 1H), 7.20 (d, 1H), 7.01 - 6.93 (m, 1H), 6.86 - 6.76 (m, 2H), 6.58 (dd, 1H), 6.02 (d, 1H), 4.99 - 4.89 (m, 1H), 4.45 - 4.30 (m, 2H), 4.13 - 3.97 (m, 2H), 3.87 - 3.72 (m, 1H), 3.61 (t, 2H), 3.16 (t, 2H), 2.96 - 2.64 (m, 3H), 2.20 - 2.08 (m, 1H), 1.86 (s, 6H).
LCMS m/z = 750.2 [M+1] +.

**Example 132: Preparation of compound 132**

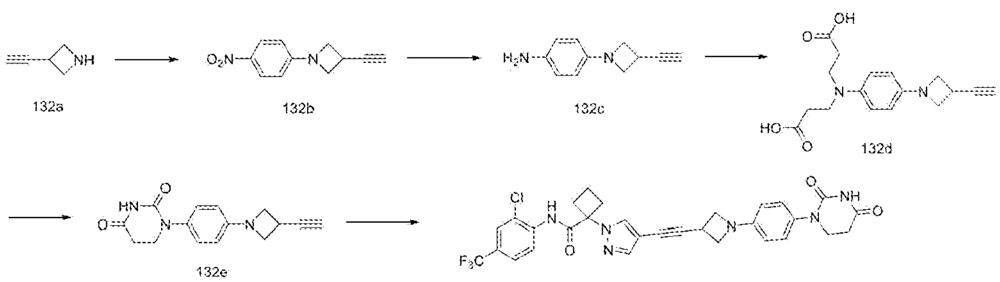
132a 132b 132c 132d 132e

Compound 132

Step 1: 3-ethynyl-1-(4-nitrophenyl)azetidine (132b)

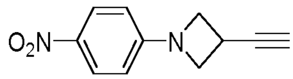

The hydrochloride of 132a (3.0 g, 25.51 mmol) was dissolved in 60 mL of acetonitrile, potassium carbonate (7.1 g, 51.37 mmol) was added, the mixture was stirred at room temperature for 30 min, then 1-fluoro-4-nitrobenzene (3.6 g, 25.5 mmol) was added, and the mixture was reacted at 90°C for 16 h. The reaction system was cooled to room temperature and concentrated under reduced pressure, 300 mL of ethyl acetate and 200 mL of water were added, the organic phase was separated, the organic phase was washed with 50 mL of saturated aqueous sodium chloride solution, dried over anhydrous sodium sulfate, and concentrated under reduced pressure. The crude product was separated and purified with silica gel chromatography column (petroleum ether : ethyl acetate (v/v) = 4 : 1) to afford 132b (4.7 g, yield: 91%).

LCMS m/z = 203.2 $[M+1]^+$.

Step 2: 4-(3-ethynylazetidin-1-yl)aniline (132c)

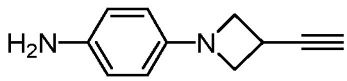

132b (4.7 g, 23.2 mmol) was dissolved in 80 mL of methanol and 20 mL of water, reduced iron powder (6.5 g, 116.1 mmol) and ammonium chloride (6.2 g, 115.9 mmol) were added, and the mixture was reacted at 70°C for 5 h. The reaction system was cooled to room temperature and filtered, and the filtrate was concentrated under reduced pressure, 4000 mL of ethyl acetate was added, the organic phase was washed with water (300 mL × 2), the organic phase was separated, and the organic phase was washed with 200 mL of saturated aqueous sodium chloride solution, dried over anhydrous sodium sulfate, and concentrated under reduced pressure. The crude product was separated and purified with silica gel chromatography column (petroleum ether : ethyl acetate (v/v) = 3 : 1) to afford 132c (3 g, yield: 75%).

LCMS m/z = 173.2 $[M+1]^+$.

Step 3: 3,3'-((4-(3-ethynylazetidin-1-yl)phenyl)azanediyl)dipropionic acid (132d)

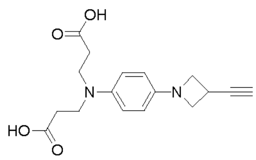

132c (3.0 g, 17.42 mmol) was dissolved in 30 mL of acetic acid, acrylic acid (5 g, 69.39 mmol) was added, and the mixture was reacted at 120°C for 12 h. The reaction system was cooled to room temperature and concentrated under reduced pressure, 200 mL of ethyl acetate was added, the mixture was washed with saturated aqueous sodium carbonate solution (150 mL × 3), then washed with water (100 mL × 2) and saturated aqueous sodium chloride solution (100 mL ×2), dried over anhydrous sodium sulfate, and concentrated under reduced pressure, and the crude product was separated and purified with silica gel chromatography column (petroleum ether : ethyl acetate (v/v) = 1 : 9) to afford 132d (0.6 g, yield: 11%).

LCMS m/z = 317.2 $[M+1]^+$.

Step 4: 1-(4-(3-ethynylazetidin-1-yl)phenyl)dihydropyrimidine-2,4(1H,3H)-dione (132e)

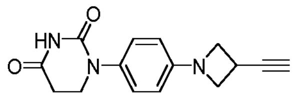

132d (600 mg, 1.9 mmol) was added into a 50 mL single-mouth bottle, 20 mL of acetic acid and urea (456 mg, 7.6 mmol) were added, and the mixture was reacted at 120°C for 12 h. The reaction system was cooled to room temperature and concentrated under reduced pressure, 60 mL of ethyl acetate was added, the mixture was washed with saturated aqueous sodium carbonate solution (50 mL × 3), then washed with water (30 mL × 2) and saturated aqueous sodium chloride solution (30 mL × 2), dried over anhydrous sodium sulfate and concentrated under reduced pressure, and the crude product was separated and purified with silica gel chromatography column (dichloromethane : methanol (v/v) = 9 : 1) to afford 132e (0.11 g, yield: 22%).

LCMS m/z = 270.2 $[M+1]^+$.

Step 5: N-(2-chloro-4-(trifluoromethyl)phenyl)-1-(4-((1-(4-(2,4-dioxotetrahydropyrimidin-1(2H)-yl)phenyl)azetidin-3-yl)ethynyl)-1H-pyrazol-1-yl)cyclobutane-1-carboxamide (Compound 132)

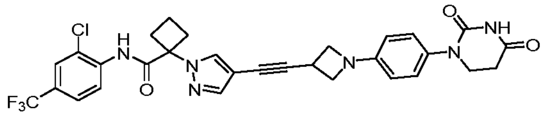

132e (30 mg, 0.11 mmol) was added to 10 mL of dry DMF, then 14c (51 mg, 0.11 mmol) and TEA (33 mg, 0.33 mmol) were added in sequence, nitrogen replacement was performed three times, $PdCl_2(PPh_3)_2$ (14 mg, 0.02 mmol) and CuI (7 mg, 0.037 mmol) were added, nitrogen replacement was performed three times, and the mixture was reacted at 50°C for 2 h. The reaction system was cooled to room temperature, 100 mL of saturated aqueous ammonium chloride solution was slowly added, the mixture was extracted with ethyl acetate (50 mL × 3), the organic phases were combined, and the organic phase was washed with saturated aqueous sodium chloride solution (30 mL × 2), dried over anhydrous sodium sulfate, and concentrated under reduced pressure. The crude product was separated and purified with silica gel chromatography column (petroleum ether : ethyl acetate (v/v) = 1 : 99) to afford compound 132 (10 mg, yield: 15%).

$^1H$ NMR (400 MHz, $CDCl_3$) δ 8.67 (s, 1H), 8.62 - 8.52 (m, 1H), 7.79 - 7.46 (m, 5H), 7.37 - 7.28 (m, 4H), 4.30 - 4.05 (m, 3H), 3.92 - 3.69 (m, 3H), 3.39 - 3.21 (m, 1H), 3.15 - 2.96 (m, 2H), 2.89 - 2.63 (m, 4H), 2.32 - 1.95 (m, 2H).

LCMS m/z = 611.3 $[M+1]^+$.

**Example 133: Preparation of compound 133**

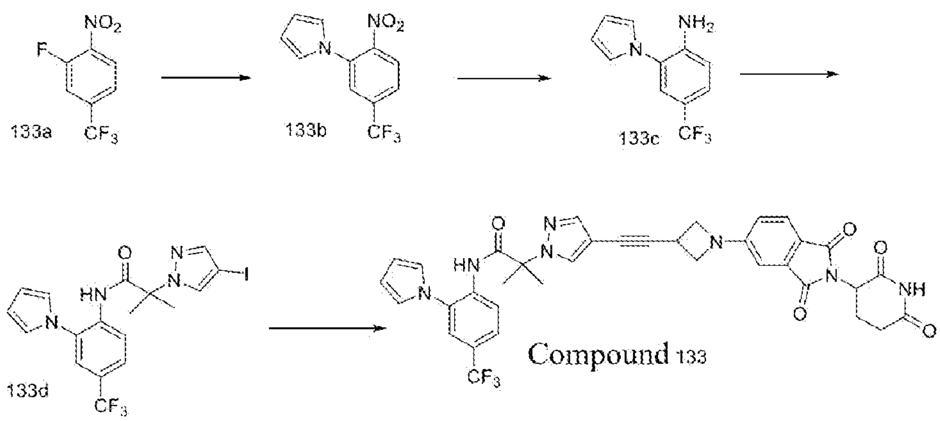
133a 133b 133c 133d Compound 133

Step 1: 1-(2-nitro-5-(trifluoromethyl)phenyl)-1H-pyrrole (133b)

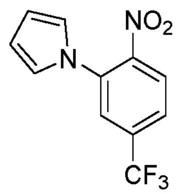

133a (1.0 g, 4.78 mmol) was dissolved in 15 mL of DMF, 1H-pyrrole (482 mg, 7.18 mmol) and cesium carbonate (3.1 g, 9.51 mmol) were added, and the mixture was reacted at room temperature for 16 h. 70 mL of ethyl acetate and 160 mL of water were added to the reaction liquid, the organic phase was separated, the organic phase was washed with 50 mL of saturated aqueous sodium chloride solution, dried over anhydrous sodium sulfate, and concentrated under reduced pressure. The crude product was separated and purified with silica gel chromatography column (petroleum ether : ethyl acetate (v/v) = 7 : 3) to afford 133b (0.35 g, yield: 29%).

LCMS m/z = 257.2 $[M+1]^+$.

Step 2; 2-(1H-pyrrol-1-yl)-4-(trifluoromethyl)aniline (133c)

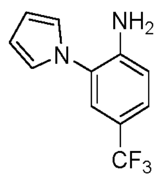

133b (0.35 g, 1.37 mmol) was dissolved in 20 mL of methanol and 5 mL of water, reduced iron powder (384 mg, 6.86 mmol) and ammonium chloride (363 mg, 6.79 mmol) were added, and the mixture was reacted at 70°C for 3 h. The reaction liquid was cooled to room temperature and filtered, the filtrate was concentrated under reduced pressure, 50 mL of ethyl acetate was added, the liquid separation was conducted, and the organic phase was washed with water (30 mL × 2) and 30 mL of saturated aqueous sodium chloride solution in sequence, dried over anhydrous sodium sulfate, and concentrated under reduced pressure. The crude product was separated and purified with silica gel chromatography column (petroleum ether : ethyl acetate (v/v) = 4 : 1) to afford 133c (200 mg, yield: 65%).

LCMS m/z = 227.2 $[M+1]^+$.

Step 3: N-(2-(1H-pyrrol-1-yl)-4-(trifluoromethyl)phenyl)-2-(4-iodo-1H-pyrazol-1-yl)-2-methylpropanamide (133d)

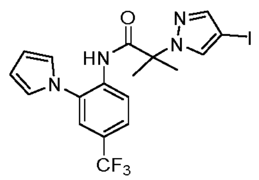

16a-1 (0.23 g, 0.82 mmol) was dissolved in 20 mL of DCM, 1-chloro-N,N,2-trimethylpropenylamine (164 mg, 1.23 mmol) was added, the mixture was reacted at room temperature for 1 h, and then TEA (248 mg, 2.45 mmol) and 133c (190 mg, 0.84 mmol) were added in sequence, and the mixture was reacted at room temperature for 12 h. 30 mL of ethyl acetate and 20 mL of water were added to the reaction liquid, the organic phase was separated, the organic phase was washed with 20 mL of saturated aqueous sodium chloride solution, dried over anhydrous sodium sulfate, and concentrated under reduced pressure. The crude product was separated and purified with silica gel chromatography column (petroleum ether : ethyl acetate (v/v) = 72 : 28) to afford 133d (0.1 g, yield: 25%).

LCMS m/z = 489.2 $[M+1]^+$.

Step 4: N-(2-(1H-pyrrol-1-yl)-4-(trifluoromethyl)phenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 133)

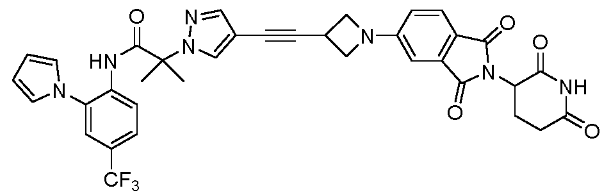

133d (100 mg, 0.2 mmol) was added to 10 mL of dry DMF, intermediate 1 (101 mg) and TEA (61 mg, 0.6 mmol) were added in sequence, nitrogen replacement was performed three times, $PdCl_2(PPh_3)_2$ (14 mg, 0.02 mmol) and CuI (7 mg, 0.037 mmol) were added, nitrogen replacement was performed three times, and the mixture was reacted at 60°C for 2 h. The reaction liquid was cooled to room temperature, 100 mL of saturated aqueous ammonium chloride solution was slowly added, a yellow solid was precipitated, filtration was performed, the filter cake was washed with 20 mL of water three times, the filter cake was dissolved in 100 mL of DCM, the mixture was dried over anhydrous sodium sulfate and concentrated under reduced pressure, the crude product was separated and purified with silica gel chromatography column (petroleum ether : ethyl acetate (v/v) = 13 : 87) to afford compound 133 (60 mg, yield: 43%).

$^1$H NMR (400 MHz, $CDCl_3$) δ 8.64 - 8.48 (m, 2H), 7.90 (s, 1H), 7.72 - 7.57 (m, 3H), 7.50 - 7.35 (m, 2H), 6.86 - 6.76 (m, 1H), 6.66 - 6.50 (m, 3H), 6.42 - 6.36 (m, 2H), 4.99 - 4.88 (m, 1H), 4.44 - 4.30 (m, 2H), 4.15 - 4.00 (m, 2H), 3.90 - 3.75 (m, 1H), 2.98 - 2.65 (m, 3H), 2.20 - 2.07 (m, 1H), 1.82 (s, 6H).

LCMS m/z = 698.2 [M+1]$^+$.

**Example 134: Preparation of compound 134**

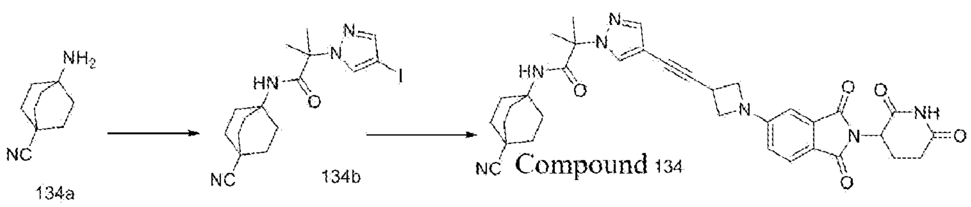
134a
134b
Compound 134

Step 1: N-(4-cyanobicyclo[2.2.2]octan-1-yl)-2-(4-iodo-1H-pyrazol-1-yl)-2-methylpropanamide (134b)

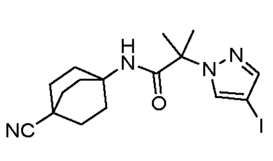

16a-1 (0.5 g, 1.79 mmol) was dissolved in 10 mL of DCM, 1-chloro-N,N,2-trimethylpropenylamine (0.27 g, 2.02 mmol) was added, the mixture was reacted at room temperature for 2 h, and then TEA (1.09 g, 10.77 mmol) and 134a (see US20100267738 for the synthesis method) (0.32 g, 2.13 mmol) were added in sequence, and the mixture was reacted at room temperature for 12 h. 50 mL of DCM and 50 mL of water were added to the reaction liquid, the organic phase was separated, the organic phase was dried over anhydrous sodium sulfate, and concentrated under reduced pressure. The crude product was separated and purified with silica gel chromatography column (petroleum ether/ethyl acetate (v/v) = 100 : 0-3 : 1) to afford 134b (0.5 g, yield: 68%).

LCMS m/z = 413.1 [M+1] $^+$.

Step 2: N-(4-cyanobicyclo[2.2.2]octan-1-yl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 134)

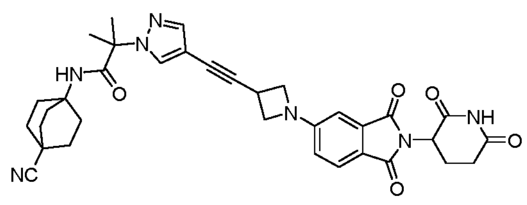

134b (0.39 g, 0.95 mmol), intermediate 1 (0.32 g), TEA (0.57 g, 5.63 mmol), CuI (36 mg, 0.189 mmol) and $PdCl_2(PPh_3)_2$ (66 mg, 0.094 mmol) were added to a reaction bottle in sequence, nitrogen replacement was performed three times, 5 mL of DMF was added under nitrogen protection, and the mixture was reacted at 55°C for 1.5 h. The reaction liquid was cooled to room temperature, 50 mL of water was added, and the mixture was filtered by suction. The filter cake was washed with 10 mL of water. The filter cake was dissolved in 100 mL of DCM, the mixture was dried over anhydrous sodium sulfate, and concentrated under reduced pressure. The crude product was separated and purified with silica gel chromatography column (petroleum ether/dichloromethane/ethyl acetate (v/v) = 1 : 1 : 2) to afford compound 134 (0.08 g, yield: 14%).

$^1$H NMR (400 MHz, $CDCl_3$) $\delta$ 8.07 - 7.91 (m, 1H), 7.75 - 7.62 (m, 3H), 6.86 - 6.76 (m, 1H), 6.56 (dd, 1H), 6.07 (s, 1H), 4.99 - 4.90 (m, 1H), 4.44 - 4.30 (m, 2H), 4.15 - 3.96 (m, 2H), 3.90 - 3.75 (m, 1H), 3.00 - 2.65 (m, 3H), 2.20 - 2.08 (m, 1H), 2.06 - 1.92 (m, 6H), 1.90 - 1.71 (m, 12H).

LCMS m/z = 622.3 [M+1] $^+$.

**Example 135: Preparation of compound 135**

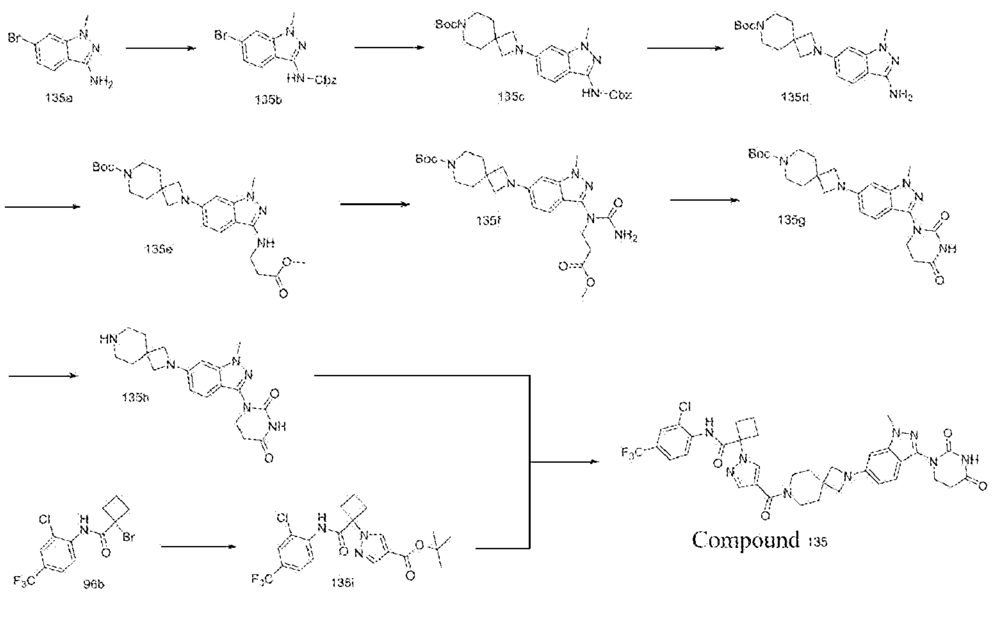


Step 1: Benzyl (6-bromo-1-methyl-1H-indazol-3-yl)carbamate (135b)

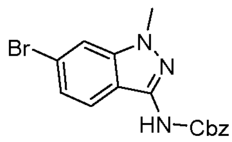

135a (34 g, 150.4 mmol) was dissolved in 600 mL of DCM, the mixture was cooled to 0°C, DIPEA (58.31 g, 451.14 mmol) and benzyl chloroformate (30.79 g, 180.49 mmol) were added in sequence, and the mixture was reacted at room temperature for 16 h. 400 mL of water was added to the reaction liquid, the liquid separation was conducted, the organic phase was dried over anhydrous sodium sulfate, and concentrated under reduced pressure. The crude product was separated and purified with silica gel column chromatography (petroleum ether/ethyl acetate (v/v) = 4 : 1-1 : 1) to afford 135b (30.3 g, yield: 56%).

Step 2: tert-butyl 2-(3-(((benzyloxy)carbonyl)amino)-1-methyl-1H-indazol-6-yl)-2,7-diazaspiro [3.5]nonane-7-carboxylate (135c)

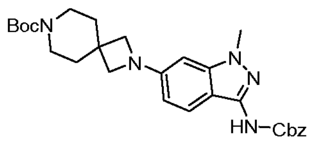

135b (10 g, 27.76 mmol), tert-butyl 2,7-diazaspiro[3.5]nonane-7-carboxylate (9.42 g, 41.64 mmol), cesium carbonate (27.13 g, 83.27 mmol) and RuPhosPdG2 (CAS: 1375325-68-0) (2.16 g, 2.78 mmol) were dissolved in 500 mL of 1,4-dioxane, nitrogen replacement was performed three times, and then the mixture was reacted at 80°C for 2 h under nitrogen atmosphere. The reaction liquid was cooled to room temperature, 400 mL of water was added, the liquid separation was conducted, the organic phase was dried over anhydrous sodium sulfate, and concentrated under reduced pressure. The crude product was separated and purified with silica gel chromatography column (petroleum ether/ethyl acetate (v/v) = 4 : 1-1 : 1) to afford 135c (8.6 g, yield: 61%).

LCMS m/z = 506.3 $[M+1]^+$.

Step 3: tert-butyl 2-(3-amino-1-methyl-1H-indazol-6-yl)-2,7-diazaspiro [3.5]nonane-7-carboxylate (135d)

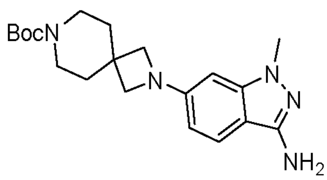

135c (9 g, 17.80 mmol) was dissolved in 100 mL of DCM, 2 g 10% Pd/C was added, and the mixture was reacted at room temperature for 72 h under hydrogen atmosphere. The reaction liquid was filtered, and the filtrate was concentrated under reduced pressure. The crude product was separated and purified with silica gel chromatography column (petroleum ether/ethyl acetate (v/v) = 4 : 1-1 : 2) to afford 135d (4.1 g, yield: 62%).

LCMS m/z = 372.3 $[M+1]^+$.

Step 4: tert-butyl 2-(3-((3-methoxy-3-oxopropyl)amino)-1-methyl-1H-indazol-6-yl)-2,7- diazaspiro[3.5]nonane-7-carboxylate (135e)

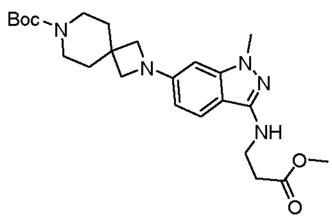

135d (4.1 g, 11.04 mmol), DIPEA (14.27 g, 110.41 mmol) and methyl acrylate (4.75 g, 55.18 mmol) were dissolved in 100 mL of ethanol, the mixture was placed in a sealed tube and reacted at 100°C for 16 h. The reaction system was cooled to room temperature, the reaction liquid was concentrated under reduced pressure, and the crude product was separated and purified with silica gel chromatography column (petroleum ether/ethyl acetate (v/v) = 4 : 1-1 : 1) to afford 135e (3.4 g, yield: 67%).

LCMS m/z = 458.3 $[M+1]^+$.

Step 5: tert-butyl 2-(3-(1-(3-methoxy-3-oxopropyl)ureido)-1-methyl-1H-indazol-6-yl)-2,7- diazaspiro[3.5]nonane-7-carboxylate (135f)

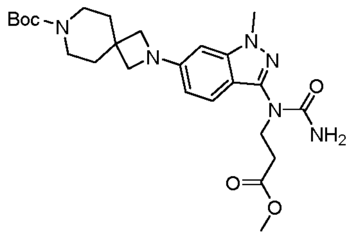

135e (2.5 g, 5.46 mmol) and DIPEA (3.53 g, 27.31 mmol) were dissolved in 50 mL of DCM, triphosgene (4.86 g, 16.38 mmol) was added, and the mixture was reacted at room temperature for 1 h. 5 mL of ammonia (25-28%wt) was added to the reaction liquid and the reaction was continued for 2 h. The reaction liquid was concentrated under reduced pressure, and the crude product was separated and purified with silica gel chromatography column (DCM/MeOH (v/v) = 20 : 1) to afford 135f (1.5 g, yield: 55%).

LCMS m/z = 501.3 $[M+1]^+$.

Step 6: tert-butyl 2-(3-(2,4-dioxotetrahydropyrimidin-1(2H)-yl)-1-methyl-1H-indazol-6-yl)-2,7- diazaspiro[3.5]nonane-7-carboxylate (135g)

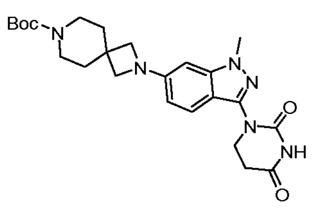

135f (2.5 g, 4.99 mmol) was dissolved in 50 mL of acetonitrile, 1.67 g of a solution of 40% Triton B in methanol was added, and the mixture was reacted at 60°C for 2 h. The reaction liquid was cooled to room temperature and concentrated under reduced pressure, and the crude product was separated and purified with silica gel chromatography column (DCM/MeOH (v/v) = 20 : 1) to afford 135g (1.5 g, yield: 64%).

LCMS m/z = 469.3 $[M+1]^+$.

Step 7: 1-(1-methyl-6-(2,7-diazaspiro[3.5]nonan-2-yl)-1H-indazol-3-yl)dihydropyrimidine-2,4(1H,3H)-dione (135h) trifluoroacetate

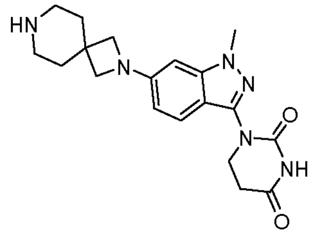

135g (0.5 g, 1.07 mmol) was dissolved in 20 mL of DCM, 10 mL of TFA was added, and the mixture was reacted at room temperature for 2 h. The reaction liquid was concentrated under reduced pressure to afford the trifluoroacetate of crude 135h (0.38 g).

LCMS m/z = 369.3 $[M+1]^+$.

Step 8: tert-butyl 1-(1-((2-chloro-4-(trifluoromethyl)phenyl)carbamoyl) cyclobutyl)-1H- pyrazole-4-carboxylate (135i)

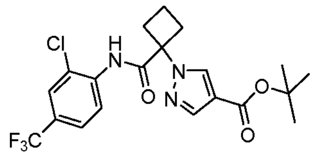

96b (2.0 g, 5.61 mmol) was dissolved in 50 mL of acetonitrile, tert-butyl 1H-pyrazole-4-carboxylate (0.79 g, 4.70 mmol) and cesium carbonate (3.66 g, 11.23 mmol) were added, and the mixture was reacted at 50°C for 12 h. The reaction liquid was cooled to room temperature and filtered, and the filtrate was concentrated under reduced pressure. The crude product was separated and purified with silica gel chromatography column (petroleum ether/ethyl acetate (v/v) = 5 : 1) to afford 135i (1.5 g, yield: 72%).

LCMS m/z = 444.2 $[M+1]^+$.

Step 9: N-(2-chloro-4-(trifluoromethyl)phenyl)-1-(4-(2-(3-(2,4-dioxotetrahydropyrimidin-1(2H)-yl)-1-methyl-1H-indazol-6-yl)-2,7-diazaspiro[3. 5]nonane-7-carbonyl)-1H-pyrazol-1-yl)cyclobutane-1-carboxamide (Compound 135)

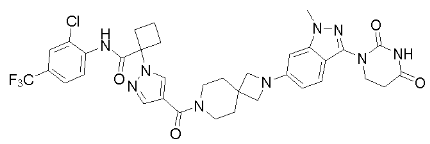

135i (1.0 g, 2.25 mmol) was dissolved in 50 mL of a solution of 4 mol/L 1,4-dioxane hydrogen chloride solution and the mixture was reacted at room temperature for 16 h. The reaction liquid was concentrated under reduced pressure, slurried with 5 mL of MTBE, and filtered, and the filter cake was collected to afford crude 1 (0.4 g). The crude 1 (0.1 g), the trifluoroacetate of crude 135h (0.1 g), N-methylimidazole (0.11 g, 1.34 mmol) and tetramethylchlorourea hexafluorophosphonate (TCFH) (0.22 g, 0.784 mmol) were dissolved in 2 mL of DMF and the mixture was reacted at room temperature for 12 h. 50 mL of water was added to the reaction liquid, the mixture was extracted with ethyl acetate (50 mL × 2), the organic phase was washed with saturated sodium chloride solution (50 mL × 3), dried over anhydrous sodium sulfate, and concentrated under reduced pressure. The crude product was separated and purified with prep-TLC (eluent: DCM/MeOH (v/v) = 20 : 1) to afford compound 135 (0.03 g, yield: 7%).

$^1H$ NMR (400 MHz, $CDCl_3$) δ 8.82 (s, 1H), 8.57 (d, 1H), 7.94 (s, 1H), 7.86 (s, 1H), 7.65 - 7.46 (m, 4H), 6.61 - 6.51 (m, 1H), 6.48 - 6.36 (m, 1H), 4.09 (t, 2H), 3.97 - 3.81 (m, 7H), 3.77 - 3.63 (m, 4H), 3.18 - 3.03 (m, 2H), 2.94 - 2.72

(m, 4H), 2.34 - 1.90 (m, 6H).
LCMS m/z =738.2 [M+1]$^+$.

**Example 136: Preparation of compound 136**

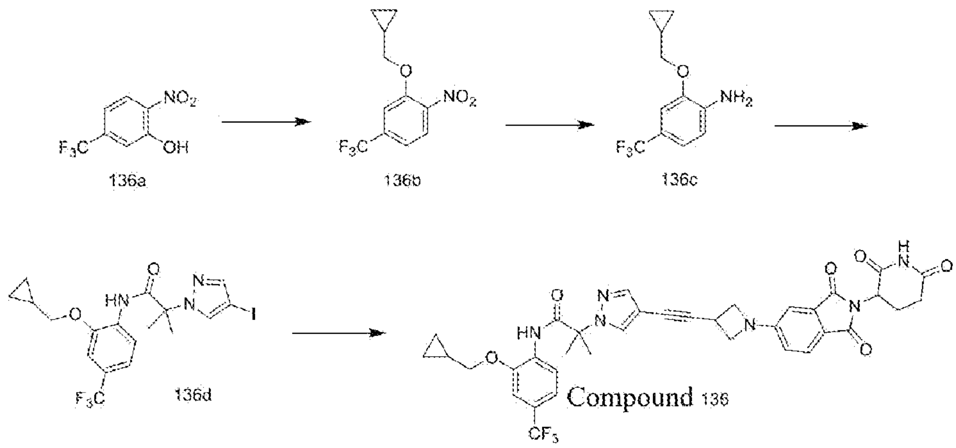


Step 1: 2-(cyclopropylmethoxy)-1-nitro-4-(trifluoromethyl)benzene (136b)

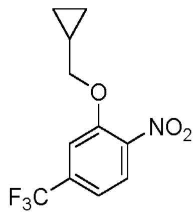

136a (2.07 g, 10.00 mmol), (bromomethyl)cyclopropane (2.03 g, 15.04 mmol) and potassium carbonate (2.77 g, 20.04 mmol) were added to 35 mL of acetonitrile and the mixture was reacted at 55°C for 16 h. The reaction liquid was cooled to room temperature and filtered, and the filtrate was concentrated under reduced pressure. The crude product was separated and purified with silica gel chromatography column (petroleum ether/ethyl acetate (v/v) = 10 : 1) to afford 136b (2.0 g, yield: 77%).

Step 2: 2-(cyclopropylmethoxy)-4-(trifluoromethyl)aniline (136c)

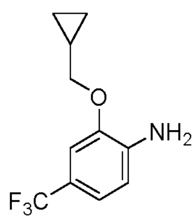

136b (0.5 g, 1.91 mmol) was dissolved in 20 mL of methanol, 0.15 g of 10% palladium on carbon was added, hydrogen replacement was performed three times, and the mixture was reacted at room temperature under the atmosphere of hydrogen balloon for 16 h. The reaction liquid was filtered, and the filtrate was concentrated under reduced pressure to afford crude 136c (0.45 g).
LCMS m/z = 232.1 [M+1]$^+$.

Step 3: N-(2-(cyclopropylmethoxy)-4-(trifluoromethyl)phenyl)-2-(4-iodo-1H-pyrazol-1-yl)-2-methylpropanamide (136d)

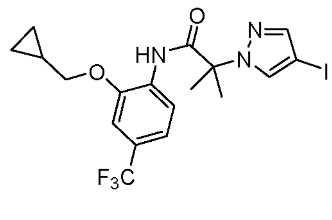

16a-1 (0.38 g, 1.35 mmol) was dissolved in 10 mL of DCM, 1-chloro-N,N,2-trimethylpropenylamine (0.24 g, 1.80 mmol) was added, the mixture was reacted at room temperature for 2 h, and then TEA (0.34 g, 3.36 mmol) and crude 136c (0.26 g) were added in sequence, and the mixture was reacted at room temperature for 16 h. 50 mL of DCM and 50 mL of water were added to the reaction liquid, the organic phase was separated, dried over anhydrous sodium sulfate, and concentrated under reduced pressure. The crude product was separated and purified with silica gel chromatography column (petroleum ether/ethyl acetate (v/v) = 10 : 1) to afford 136d (0.5 g, yield: 75%).

Step 4: N-(2-(cyclopropylmethoxy)-4-(trifluoromethyl)phenyl)-2-(4-((1-(2-(2,6-dioxopiperidin-3 -yl)-1,3 -dioxoisoindolin-5 -yl)azetidin-3 -yl)ethynyl)- 1H-pyrazol-1-yl)-2-methylpropanamide (Compound 136)

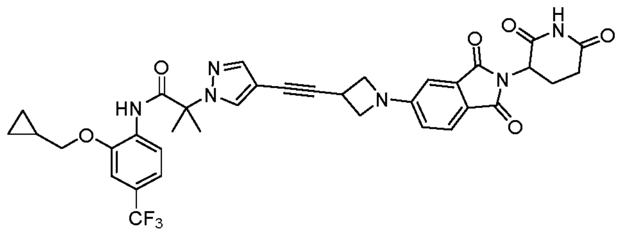

136d (0.2 g, 0.41 mmol), intermediate 1 (0.17 g), TEA (0.12 g, 1.19 mmol), CuI (8 mg, 0.042 mmol) and $PdCl_2(PPh_3)_2$ (29 mg, 0.041 mmol) were added to a reaction bottle, 5 mL of DMF was added under nitrogen protection, and the mixture was reacted at 50°C for 1 h. The reaction liquid was cooled to room temperature, 50 mL of water was added, and the mixture was filtered by suction. The filter cake was washed with 10 mL of water. The filter cake was dissolved in 100 mL of DCM, the mixture was dried over anhydrous sodium sulfate, and concentrated under reduced pressure. The crude product was separated and purified with silica gel column chromatography (petroleum ether/ethyl acetate (v/v) = 1 : 1) to afford compound 136 (0.26 g, yield: 90%).

$^1$H NMR (400 MHz, $CDCl_3$) $\delta$ 8.76 (s, 1H), 8.45 (d, 1H), 8.07 (s, 1H), 7.80 (s, 1H), 7.75 (s, 1H), 7.67 (d, 1H), 7.19 (d, 1H), 6.98 - 6.90 (m, 1H), 6.85 - 6.77 (m, 1H), 6.56 (dd, 1H), 4.98 - 4.87 (m, 1H), 4.43 - 4.30 (m, 2H), 4.10 - 4.00 (m, 2H), 3.88 - 3.72 (m, 3H), 2.95 - 2.65 (m, 3H), 2.18 - 2.07 (m, 1H), 1.95 (s, 6H), 0.94 - 0.80 (m, 1H), 0.78 - 0.65 (m, 2H), 0.40 - 0.31 (m, 2H).

LCMS m/z = 703.2 [M+1] $^+$.

**Example 137: Preparation of compound 137**

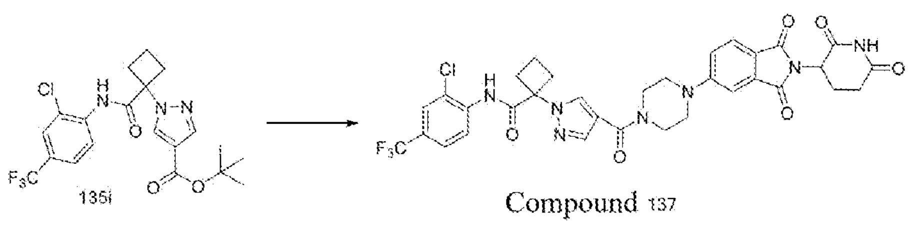

135i (1.0 g, 2.25 mmol) was dissolved in 50 mL of a solution of 4 mol/L 1,4-dioxane hydrogen chloride solution and the mixture was reacted at room temperature for 16 h. The reaction liquid was concentrated under reduced pressure, slurried with 5 mL of MTBE, and filtered, and the filter cake was collected to afford a crude product (0.4 g). The above crude product (80 mg) was dissolved in DCM (10 mL), 1-chloro-N,N,2-trimethylpropenylamine (41.3 mg, 0.309 mmol) was added, the mixture was reacted at room temperature for 1 h, then TEA (62.4 mg, 0.617 mmol) and the hydrochloride of crude 73c (77.8 mg) were added in sequence, and the mixture was reacted at room temperature for 12 h. 20 mL of ethyl acetate and 10 mL of water were added to the reaction liquid, the organic phase was separated, the organic phase was washed with 10 mL of saturated sodium chloride solution, dried over anhydrous sodium sulfate, and concentrated under reduced pressure. The crude product was separated and purified with silica gel chromatography column (petroleum ether/ethyl acetate (v/v) = 12 : 88) to afford compound 137 (50 mg, yield: 16%).

$^1H$ NMR (400 MHz, $CDCl_3$) δ 8.83 (s, 1H), 8.57 (d, 1H), 8.03 - 7.95 (m, 2H), 7.91 (s, 1H), 7.74 (d, 1H), 7.63 - 7.58 (m, 1H), 7.57 - 7.49 (m, 1H), 7.34 - 7.27 (m, 1H), 7.10 (dd, 1H), 5.00 - 4.90 (m, 1H), 4.00 - 3.88 (m, 4H), 3.57 - 3.46 (m, 4H), 3.20 - 3.05 (m, 2H), 2.98 - 2.65 (m, 5H), 2.35 - 2.01 (m, 3H).

LCMS m/z = 712.2 $[M+1]^+$.

**Example 138: Preparation of compound 138**

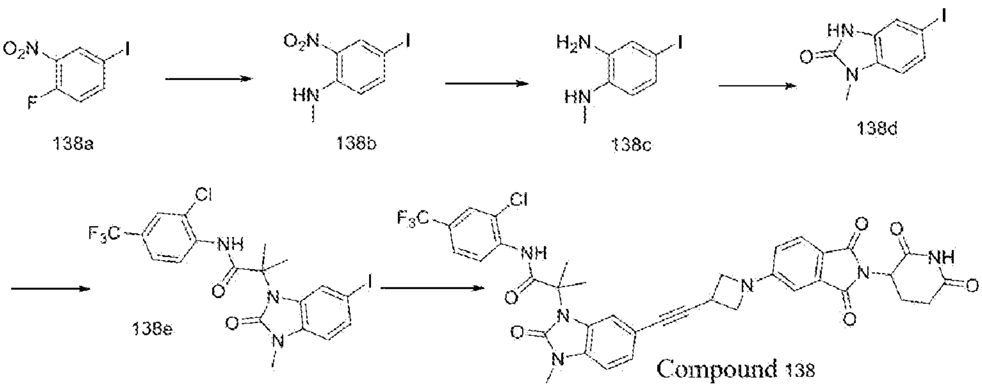

Step 1: 4-iodo-N-methyl-2-nitroaniline (138b)

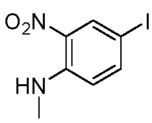

138a (2.0 g, 7.5 mmol) was dissolved in 30 mL of THF, the mixture was cooled to 0°C under ice bath, 20 mL of 40% (wt) aqueous methylamine solution was slowly added dropwise, and the mixture was warmed back to room temperature and reacted for 30 min. 60 mL of ethyl acetate and 50 mL of water were added to the reaction liquid, the organic phase was separated, the organic phase was washed with 30 mL of saturated aqueous sodium chloride solution, dried over anhydrous sodium sulfate, and concentrated under reduced pressure. The crude product was separated and purified with silica gel chromatography column (petroleum ether : ethyl acetate (v/v) = 92 : 8) to afford 138b (1.8 g, yield: 86%).

LCMS m/z = 279.1 $[M+1]^+$.

Step 2; 4-iodo-N1-methylbenzene-1,2-diamine (138c)

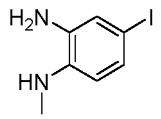

138b (1.8 g, 6.5 mmol) was dissolved in a mixed solvent of 80 mL of methanol and 20 mL of water, then reduced iron powder (1.8 g, 32.1 mmol) and ammonium chloride (1.8 g, 33.7 mmol) were added, and the mixture was reacted at 70°C for 5 h. The reaction liquid was cooled to room temperature and filtered, and the filtrate was concentrated under reduced pressure, 100 mL of ethyl acetate was added, the mixture was washed with water (50 mL × 2), the organic phase was separated, and the organic phase was washed with 30 mL of saturated sodium chloride solution, dried over anhydrous sodium sulfate, and concentrated under reduced pressure. The crude product was separated and purified with silica gel chromatography column (petroleum ether : ethyl acetate (v/v) = 3 : 2) to afford 138c (1.4 g, yield: 87%).
LCMS m/z = 249.1 $[M+1]^+$.

Step 3: 5-iodo-1-methyl-1,3-dihydro-2H-benzo[d]imidazol-2-one (138d)

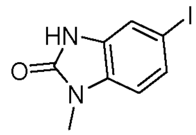

138c (1.4 g, 5.6 mmol) was dissolved in 25 mL of acetonitrile, N,N'-carbonyldiimidazole (1.79 g, 11.0 mmol) was added, and the mixture was reacted at 80°C for 4 h. The reaction liquid was cooled to room temperature and slowly added to 300 mL of water, an off-white solid was precipitated, filtration was performed. The filter cake was washed with 10 mL of petroleum ether twice to afford 138d (1.0 g, yield: 65%).

Step 4: N-(2-chloro-4-(trifluoromethyl)phenyl)-2-(6-iodo-3-methyl-2-oxo-2,3-dihydro-1H-benzo[d]imidazol-1-yl)-2-methylpropanamide (138e)

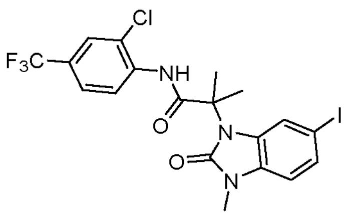

138d (500 mg, 1.82 mmol) was added into a 100 mL single-mouth bottle, 20 mL of acetonitrile, cesium carbonate (1.17 g, 3.6 mmol) and 11b (620 mg, 1.8 mmol) were added in sequence, and the mixture was reacted at 90°C for 16 h. The reaction liquid was cooled to room temperature, 80 mL of ethyl acetate was added, the mixture was washed with water (50 mL × 2) and saturated aqueous sodium chloride solution (30 mL × 2) in sequence, dried over anhydrous sodium sulfate, and concentrated under reduced pressure. The crude product was separated and purified with silica gel chromatography column (petroleum ether : ethyl acetate (v/v) = 1 : 1) to afford 138e (0.11 g, yield: 11%).
LCMS m/z = 538.1 $[M+1]^+$.

Step 5: N-(2-chloro-4-(trifluoromethyl)phenyl)-2-(6-((1-(2-(2,6-dioxopiperidin-3-yl)-1,3-dioxoisoindolin-5-yl)azetidin-3-yl)ethynyl)-3-methyl-2-oxo-2,3-dihydro-1H-benzo[d]imidazol-1-yl)-2-methylpropanamide (Compound 138)

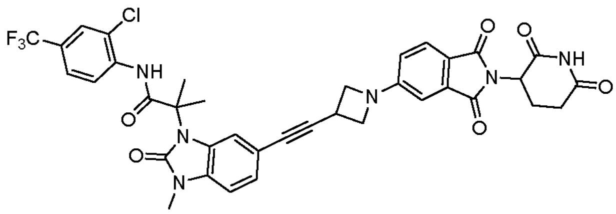

138e (110 mg, 0.205 mmol) was added into a 50 mL single-mouth bottle, 10 mL of dry DMF, intermediate 1 (101 mg) and TEA (33 mg, 0.326 mmol) were added in sequence, nitrogen replacement was performed three times, and $PdCl_2(PPh_3)_2$ (14 mg, 0.02 mmol) and CuI (6 mg, 0.032 mmol) were added, nitrogen replacement was performed three times, and the mixture was reacted at 50°C for 2 h. The reaction system was cooled to room temperature, 100 mL of saturated aqueous ammonium chloride solution was slowly added, the mixture was extracted with ethyl acetate (50 mL × 3), the organic phases were combined, and the organic phase was washed with saturated aqueous sodium chloride solution (30 mL × 2), dried over anhydrous sodium sulfate, and concentrated under reduced pressure. The crude product was separated and purified with silica gel chromatography column (petroleum ether : ethyl acetate (v/v) = 1 : 99) to afford compound 138 (60 mg, yield: 39%).

$^1H$ NMR (400 MHz, DMSO-$d_6$) δ 11.05 (s, 1H), 9.78 (s, 1H), 8.00 - 7.82 (m, 1H), 7.78 - 7.48 (m, 3H), 7.30 - 7.11 (m, 3H), 6.94 - 6.80 (m, 1H), 6.72 (dd, 1H), 5.12 - 5.00 (m, 1H), 4.48 - 4.33 (m, 2H), 4.13 - 4.00 (m, 2H), 3.98 - 3.85 (m, 1H), 3.31 (s, 3H), 2.96 - 2.80 (m, 1H), 2.65 - 2.50 (m, 2H), 2.09 - 1.95 (m, 1H), 1.86 (s, 6H).

LCMS m/z = 747.1 $[M+1]^+$.

**Example 139: Preparation of the trifluoroacetate of compound 139**

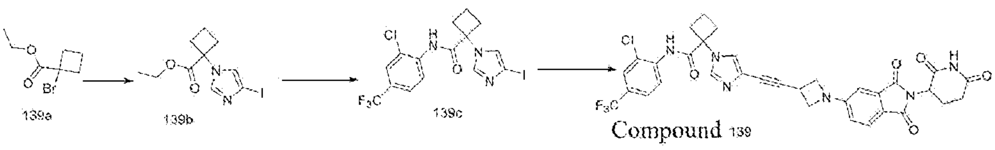

139a 139b 139c Compound 139

Step 1: ethyl 1-(4-iodo-1H-imidazol-1-yl)cyclobutane-1-carboxylate (139b)

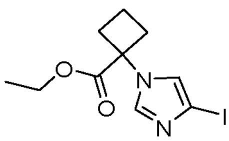

139a (6.0 g, 28.98 mmol) was dissolved in 60 mL of acetonitrile, cesium carbonate (18.88 g, 57.95 mmol) and 4-iodo-1H-imidazole (5.62 g, 28.98 mmol) were added at room temperature, and the mixture was reacted at 75°C for 12 h. The reaction liquid was cooled to room temperature and filtered, and the filtrate was concentrated under reduced pressure, and then separated and purified with silica gel chromatography column (petroleum ether/ethyl acetate (v/v) = 5 : 1-1 : 2) to afford 139b (4.5 g, yield: 49%).

LCMS m/z = 321.0 $[M+1]^+$.

Step 2: N-(2-chloro-4-(trifluoromethyl)phenyl)-1-(4-iodo-1H-imidazol-1-yl)cyclobutane-1-carboxamide (139c)

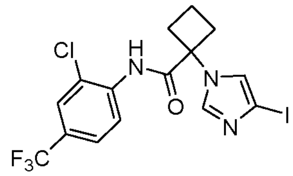

139b (1.0 g, 3.12 mmol) was dissolved in 10 mL of ethanol and 5 mL of water, potassium hydroxide (0.52 g, 9.27 mmol) was added, and the mixture was reacted at room temperature for 12 h. The reaction liquid was filtered, and the filtrate was concentrated under reduced pressure to afford a crude product (0.6 g). The above crude product (0.5 g) was dissolved in 5 mL of DCM, 1-chloro-N,N,2-trimethylpropenylamine (0.34 g, 2.55 mmol) was added, the mixture was reacted at room temperature for 2 h, then TEA (0.52 g, 5.14 mmol) and 3-chloro-4-aminotrifluorotoluene (0.33 g, 1.69 mmol) were added in sequence, and the mixture was reacted at room temperature for 12 h. 50 mL of DCM and 50 mL of water were added to the reaction liquid, the organic phase was separated, dried over anhydrous sodium sulfate, and concentrated under reduced pressure. The crude product was separated and purified with silica gel chromatography column (petroleum ether/ethyl acetate (v/v) = 20 : 1) to afford 139c (0.5 g, yield: 63%).

Step 3: N-(2-chloro-4-(trifluoromethyl)phenyl)-1-(4-((1-(2-(2,6-dioxopiperidin-3-yl)-1,3-dioxoisoindolin-5-yl)azetidin-3-yl)ethynyl)-1H-imidazol-1-yl)cyclobutane-1-carboxamide (Compound 139) trifluoroacetate

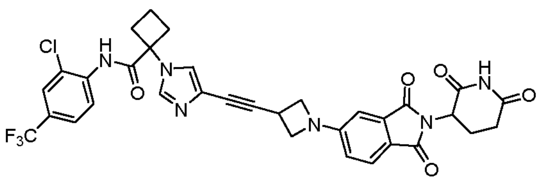

139c (0.5 g, 1.06 mmol), intermediate 1 (0.36 g), TEA (0.64 g, 6.32 mmol), CuI (40 mg, 0.21 mmol) and $PdCl_2(PPh_3)_2$ (74 mg, 0.11 mmol) were added to a reaction bottle, nitrogen replacement was performed three times, 5 mL of DMF was added under nitrogen protection, and the mixture was reacted at 60°C for 3 h. The reaction liquid was cooled to room temperature, 50 mL of water was added, and the mixture was filtered by suction. The filter cake was washed with 10 mL of water. The filter cake was dissolved in 100 mL of DCM, the mixture was dried over anhydrous sodium sulfate, and concentrated under reduced pressure. The crude product was subjected to Pre-HPLC (instrument and preparative column: using Glison GX-281 preparative liquid phase chromatographic instrument, preparative column model: Sunfire C18, 5 μm, inner diameter × length = 30 mm × 150 mm). Preparation method: the crude product was dissolved with methanol and dimethyl sulfoxide, and filtered with a 0.45 μm filter membrane, to prepare into a sample liquid. Mobile phase system: acetonitrile/water (containing 0.1% TFA). Gradient elution method: gradient elution of 5% to 60% acetonitrile (elution time 15 min), and lyophilization was performed to afford the trifluoroacetate of compound 139 (0.05 g).

$^1$H NMR (400 MHz, $CD_3OD$) δ 9.15 - 8.60 (m, 1H), 8.21 - 7.60 (m, 5H), 6.93 - 6.86 (m, 1H), 6.74 (dd, 1H), 5.23 - 5.02 (m, 2H), 4.55 - 4.40 (m, 2H), 4.20 - 3.72 (m, 4H), 2.97 - 2.35 (m, 6H), 2.26 - 2.07 (m, 2H).

LCMS m/z = 679.1 [M+1] $^+$.

**Example 140: Preparation of compound 140**

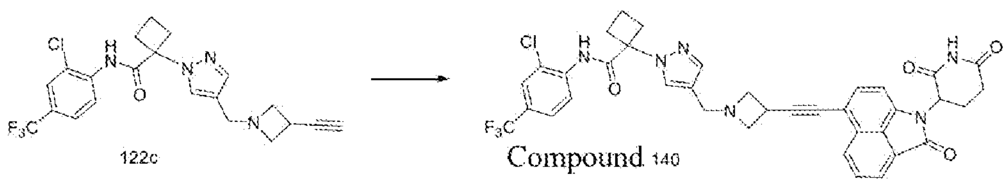

122c

Compound 140

Under nitrogen atmosphere, 3-(6-bromo-2-oxobenzo[cd]indol-1(2H)-yl)piperidine-2,6-dione (120 mg, 0.33 mmol) (referring to WO 2020210630 for the synthesis method), 122c (288 mg, 0.66 mmol), $Pd(dppf)Cl_2$•DCM (54 mg,

0.067 mmol), CuI (13 mg, 0.068 mmol) and DIPEA (0.43 g, 3.33 mmol) were added into a reaction bottle in sequence, 5 mL of DMF was added, and the mixture was reacted at 60°C for 5 h. The reaction liquid was cooled to room temperature, 20 mL of water was added, the mixture was filtered, the filter cake was collected, the filter cake was dissolved with 50 mL of DCM, the mixture was dried over anhydrous sodium sulfate, and concentrated under reduced pressure. The crude product was separated and purified with silica gel chromatography column (DCM/MeOH (v/v) = 10 : 1), and the crude product obtained after purification was further separated and purified with silica gel chromatography column (DCM/MeOH (v/v) = 20 : 1) to afford compound 140 (26 mg, yield: 11%).

$^1$H NMR (400 MHz, $CDCl_3$) δ 8.72 (s, 1H), 8.57 (d, 1H), 8.34 - 8.20 (m, 2H), 8.13 (d, 1H), 7.80 (dd, 1H), 7.71 (s, 1H), 7.66 - 7.44 (m, 4H), 6.70 (d, 1H), 5.36 - 5.24 (m, 1H), 3.95 - 3.51 (m, 5H), 3.45 - 3.17 (m, 2H), 3.14 - 2.64 (m, 7H), 2.36 - 1.98 (m, 3H).

LCMS m/z = 715.2 [M+1] $^+$.

**Example 141: Preparation of compound 141**

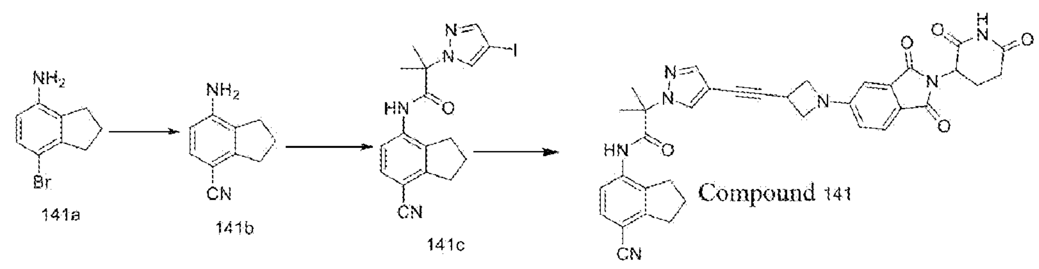


Step 1: 7-amino-2,3-dihydro-1H-indene-4-carbonitrile (141b)

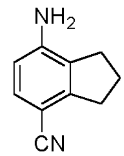

141a (see WO 2020018975 for the synthesis method) (0.84 g, 3.96 mmol) and CuCN (1.07 g, 11.95 mmol) were dissolved in 10 mL of NMP, and the mixture was reacted at 170°C for 5 h. The reaction liquid was cooled to room temperature, 100 mL of water and 100 mL of DCM were added, the mixture was filtered, the liquid separation was conducted for the filtrate, the organic phase was washed with water (50 mL × 3), dried over anhydrous sodium sulfate, and concentrated under reduced pressure, and the crude product was separated and purified with silica gel chromatography column (ethyl acetate/petroleum ether (v/v) = 1 : 100-1 : 3) to afford 141b (0.5 g, yield: 80%).

Step 2: N-(7-cyano-2,3-dihydro-1H-inden-4-yl)-2-(4-iodo-1H-pyrazol-1-yl)-2-methylpropanamide (141c)

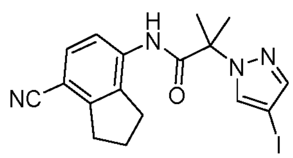

16a-1 (0.5 g, 1.78 mmol) was dissolved in 10 mL of DCM, 1-chloro-N,N,2-trimethylpropenylamine (0.36 g, 2.7 mmol) was added, the mixture was reacted at room temperature for 2 h, and then TEA (1.09 g, 10.77 mmol) and 141b (0.28 g, 1.77 mmol) were added in sequence, and the mixture was reacted at room temperature for 12 h. 50 mL of DCM and 50 mL of water were added to the reaction liquid, the organic phase was separated, dried over anhydrous sodium sulfate, and concentrated under reduced pressure. The crude product was separated and purified with silica gel chromatography column (petroleum ether/ethyl acetate (v/v) = 20 : 1-5 : 1) to afford 141c (0.5 g, yield: 67%).

LCMS m/z = 421.4 $[M+1]^+$.

Step 3: N-(7-cyano-2,3-dihydro-1H-inden-4-yl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 141)

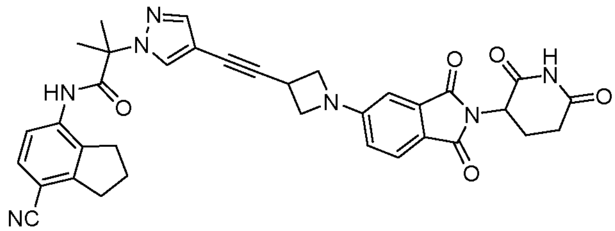

141c (0.4 g, 0.95 mmol), intermediate 1 (0.32 g), TEA (0.57 g, 5.63 mmol), CuI (36 mg, 0.189 mmol) and $PdCl_2(PPh_3)_2$ (66 mg, 0.094 mmol) were added to a reaction bottle, nitrogen replacement was performed three times, 5 mL of DMF was added under nitrogen protection, and the mixture was reacted at 55°C for 1.5 h. The reaction liquid was cooled to room temperature, 50 mL of water was added, and the mixture was filtered by suction. The filter cake was washed with 10 mL of water. The filter cake was dissolved in 100 mL of DCM, the mixture was dried over anhydrous sodium sulfate. The crude product was separated and purified with silica gel chromatography column (petroleum ether/dichloromethane/ethyl acetate (v/v) = 1 : 1 : 2) to afford compound 141 (0.1 g, yield: 17%).

$^1H$ NMR (400 MHz, $CDCl_3$) δ 8.91 (s, 1H), 8.15 - 7.92 (m, 2H), 7.85 - 7.75 (m, 2H), 7.74 - 7.62 (m, 1H), 7.45 - 7.38 (m,1H), 6.85 - 6.76 (m, 1H), 6.55 (dd, 1H), 4.99 - 4.89 (m, 1H), 4.44 - 4.30 (m, 2H), 4.14 - 4.00 (m, 2H), 3.89 - 3.75 (m, 1H), 3.16 - 3.04 (m, 2H), 3.00 - 2.64 (m, 5H), 2.24 - 2.06 (m, 3H), 1.93 (s, 6H).

LCMS m/z = 630.5 $[M+1]^+$.

**Example 142: Preparation of compound 142**

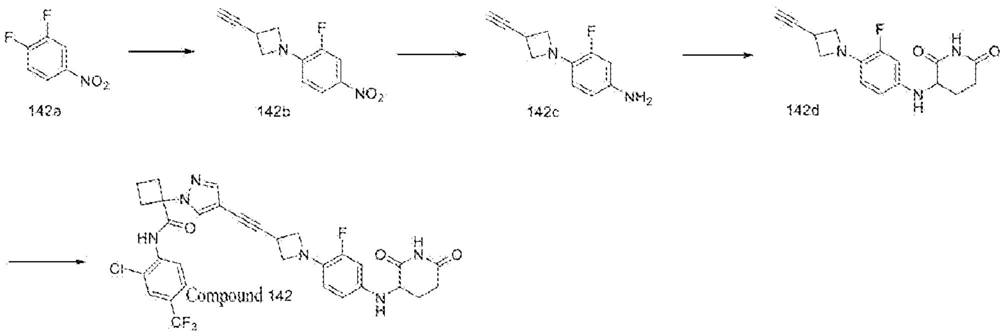

142a 142b 142c 142d

Compound 142

Step 1: 3-ethynyl-1-(2-fluoro-4-nitrophenyl)azetidine (142b)

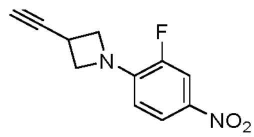

142a (1.0 g, 6.28 mmol) was dissolved in 20 mL of acetonitrile, 3-ethynylazetidine hydrochloride (736 mg, 6.26 mmol) and potassium carbonate (1.7 g, 12.30 mmol) were added in sequence, and the mixture was reacted at 90°C for 2 h. The reaction liquid was cooled to room temperature, 60 mL of ethyl acetate and 200 mL of water were added, the

organic phase was separated, the organic phase was washed with 50 mL of saturated sodium chloride solution, dried over anhydrous sodium sulfate, and concentrated under reduced pressure. The crude product was separated and purified with silica gel chromatography column (petroleum ether/ethyl acetate (v/v) = 4 : 1) to afford 142b (1.2 g, yield: 87%).

Step 2; 4-(3-ethynylazetidin-1-yl)-3-fluoroaniline (142c)

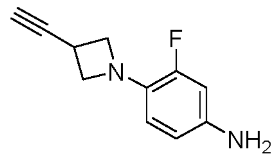

142b (1.2 g, 5.45 mmol) was dissolved in a mixed solvent of 15 mL of methanol and 15 mL of water, reduced iron powder (1.53 g, 27.32 mmol) and ammonium chloride (1.47 g, 27.48 mmol) were added, and the mixture was reacted at 60°C for 2 h. The reaction liquid was cooled to room temperature and filtered to remove insoluble matter, 100 mL of water was added to the filtrate, the mixture was extracted with DCM (60 mL × 3), the organic phases were combined, the organic phase was washed with 50 mL of saturated sodium chloride solution, dried over anhydrous sodium sulfate and concentrated under reduced pressure to afford crude 142c (900 mg).

LCMS m/z = 191.2 $[M+1]^+$.

Step 3: 3-((4-(3-ethynylazetidin-1-yl)-3-fluorophenyl)amino)piperidine-2,6-dione (142d)

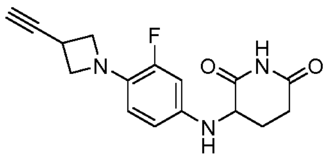

Crude 142c (300 mg) was dissolved in 15 mL of dry DMF, 3-bromopiperidine-2,6-dione (300 mg, 1.56 mmol) and sodium bicarbonate (400 mg, 4.76 mmol) were added in sequence, and the mixture was reacted at 90°C for 3 h. The reaction liquid was cooled to room temperature, 150 mL of water was added, 60 mL of ethyl acetate was added for extraction, the organic phase was separated, the aqueous phase was extracted with ethyl acetate (30 mL × 2), the organic phases were combined, and the organic phase was washed with 50 mL of saturated sodium chloride solution, dried over anhydrous sodium sulfate, and concentrated under reduced pressure. The crude product was separated and purified with silica gel chromatography column (petroleum ether/ethyl acetate (v/v) = 1 : 1) to afford 142d (360 mg, two-step yield from compound 142b: 66%).

LCMS m/z = 302.2 $[M+1]^+$.

Step 4: N-(2-chloro-4-(trifluoromethyl)phenyl)-1-(4-((1-(4-((2,6-dioxopiperidin-3-yl)amino)-2-fluorophenyl)azetidin-3-yl)ethynyl)-1H-pyrazol-1-yl)cyclobutane-1-carboxamide (Compound 142)

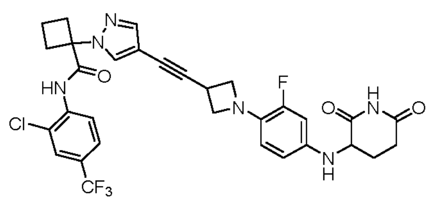

142d (100 mg, 0.33 mmol) was added to 10 mL of dry DMF, 14c (156 mg, 0.332 mmol) and TEA (100 mg, 0.99 mmol) were added in sequence, nitrogen replacement was performed three times, $PdCl_2(PPh_3)_2$ (23 mg, 0.033 mmol) and CuI (13 mg, 0.068 mmol) were added, nitrogen replacement was performed three times, and the mixture was reacted

at 60°C for 2 h. The reaction liquid was cooled to room temperature, 100 mL of saturated ammonium chloride solution was slowly added, a yellow solid was precipitated, filtration was performed, the filter cake was washed with 10 mL of water three times, the filter cake was dissolved in 100 mL of DCM, the mixture was dried over anhydrous sodium sulfate and concentrated under reduced pressure, the crude product was separated and purified with silica gel chromatography column (petroleum ether/ethyl acetate (v/v) = 1 : 4) to afford compound 142 (60 mg, yield: 28%).

$^1$H NMR (400 MHz, $CDCl_3$) δ 8.69 (s, 1H), 8.55 (d, 1H), 7.90 (s, 1H), 7.77 (s, 1H), 7.68 (s, 1H), 7.62 - 7.45 (m, 2H), 7.28 - 7.23 (m, 1H), 6.70 - 6.34 (m, 3H), 4.40 - 4.17 (m, 2H), 4.05 - 3.85 (m, 3H), 3.82 - 3.65 (m, 1H), 3.15 - 2.97 (m, 2H), 2.97 - 2.40 (m, 5H), 2.32 - 2.00 (m, 2H), 1.99 - 1.82 (m, 1H).

LCMS m/z = 643.2 $[M+1]^+$.

**Example 143: Preparation of compound 143**

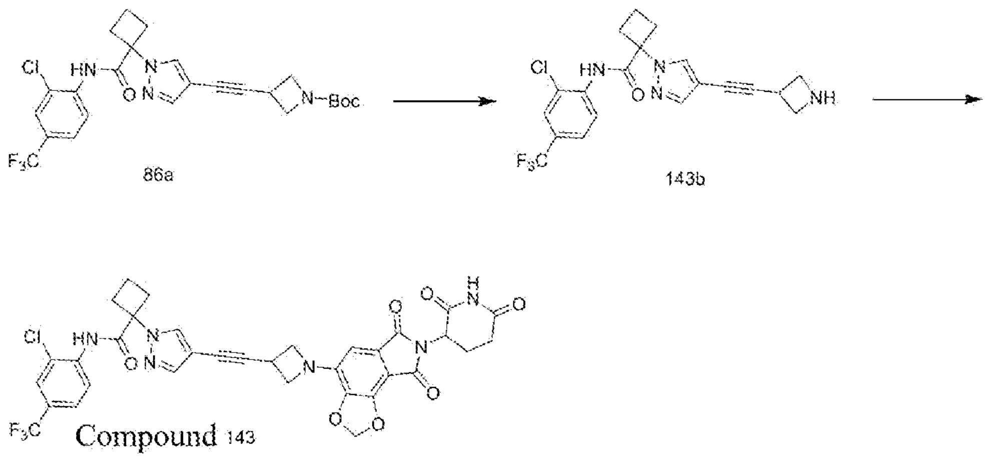

86a

143b

Compound 143

Step 1: 1-(4-(azetidin-3-ylethynyl)-1H-pyrazol-1-yl)-N-(2-chloro-4-(trifluoromethyl)phenyl)cyclobutane-1-carboxamide (143b) 4-methylbenzenesulfonate

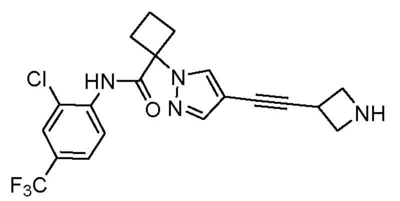

86a (0.15 g, 0.29 mmol) was dissolved in 4 mL of acetonitrile, p-toluenesulfonic acid monohydrate (0.22 g, 1.16 mmol) was added, the mixture was reacted at room temperature for 2 h, and then filtered, and the filter cake was dried under vacuum to afford 4-methylbenzenesulfonate of crude 143b (0.10 g).

LCMS m/z = 423.4 $[M+1]^+$.

Step 2: N-(2-chloro-4-(trifluoromethyl)phenyl)-1-(4-((1-(7-(2,6-dioxopiperidin-3-yl)-6,8-dioxo-7,8-dihydro-6H-[1,3]dioxolo[4,5-e]isoindol-4-yl)azetidin-3-yl)ethynyl)-1H-pyrazol-1-yl)cyclobutane-1-carboxamide (Compound 143)

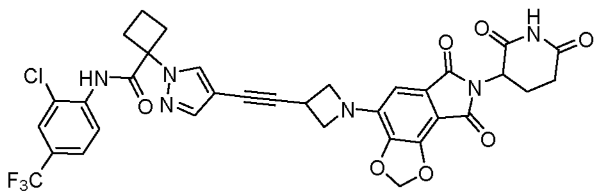

The 4-methylbenzenesulfonate of crude 143b (0.044 g) and 7-(2,6-dioxopiperidin-3-yl)-4-fluoro-6H-[1,3]dioxolo[4,5-e]isoindole-6,8(7H)-dione (0.020 g, 0.062 mmol) (see WO 2021143822 for the synthesis method) were dissolved in 4 mL of DMSO, DIPEA (0.040 g, 0.31 mmol) was added, nitrogen replacement was performed three times, and the mixture was reacted at 80°C for 3 h under nitrogen protection. The reaction liquid was cooled to room temperature, 50 mL of water was added, and the mixture was filtered by suction. The filter cake was washed with 10 mL of water. The filter cake was dissolved in 100 mL of DCM, the mixture was dried over anhydrous sodium sulfate, and concentrated under reduced pressure. The crude product was separated and purified with silica gel column chromatography (petroleum ether/ethyl acetate (v/v) = 1 : 1) to afford compound 143 (0.010 g, yield: 22%).

$^1$H NMR (400 MHz, $CDCl_3$) $\delta$ 8.68 (s, 1H), 8.54 (d, 1H), 7.94 (s, 1H), 7.77 (s, 1H), 7.68 (s, 1H), 7.61 - 7.47 (m, 2H), 6.81 (s, 1H), 6.02 (s, 2H), 4.92 - 4.81 (m, 1H), 4.79 - 4.68 (m, 2H), 4.47 - 4.37 (m, 2H), 3.69 - 3.55 (m, 1H), 3.14 - 3.00 (m, 2H), 2.93 - 2.61 (m, 5H), 2.30 - 1.98 (m, 3H).

LCMS m/z = 723.1 [M+1] $^+$.

**Example 144: Preparation of compound 144**

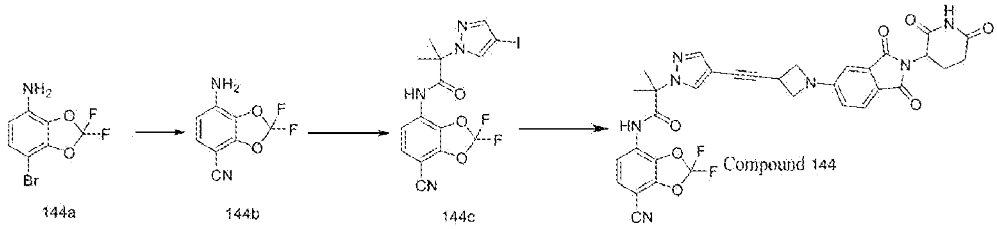

144a 144b 144c Compound 144

Step 1: 7-amino-2,2-difluorobenzo[d][1,3]dioxole-4-carbonitrile (144b)

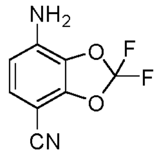

144a (see WO 2018122232 for the synthesis method) (1.0 g, 4.71 mmol) and CuCN (1.07 g, 11.95 mmol) were dissolved in 10 mL of NMP, and the mixture was reacted at 170°C for 5 h. The reaction liquid was cooled to room temperature, 100 mL of water and 100 mL of DCM were added, the mixture was filtered, the liquid separation was conducted for the filtrate, the organic phase was washed with water (50 mL × 3), dried over anhydrous sodium sulfate, and concentrated under reduced pressure, and the crude product was separated and purified with silica gel chromatography column (ethyl acetate/petroleum ether (v/v) = 1 : 100-1 : 3) to afford 144b (0.5 g, yield: 54%).

LCMS m/z = 199.1 [M+1] $^+$.

Step 2: N-(7-cyano-2,2-difluorobenzo[d][1,3]dioxol-4-yl)-2-(4-iodo-1H-pyrazol-1-yl)-2-methylpropanamide (144c)

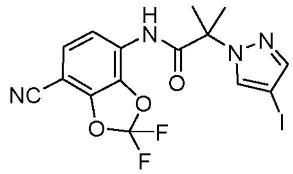

16a-1 (0.5 g, 1.78 mmol) was dissolved in 10 mL of DCM, 1-chloro-N,N,2-trimethylpropenylamine (0.36 g, 2.7 mmol) was added, the mixture was reacted at room temperature for 2 h, and then TEA (1.09 g, 10.74 mmol) and 144b (0.35 g, 1.77 mmol) were added in sequence, and the mixture was reacted at room temperature for 12 h. 50 mL of DCM and 50 mL of water were added to the reaction liquid, the organic phase was separated, dried over anhydrous sodium sulfate, and concentrated under reduced pressure. The crude product was separated and purified with silica gel chromatography column (petroleum ether/ethyl acetate (v/v) = 20 : 1-5 : 1) to afford 144c (0.5 g, yield: 61%).

LCMS m/z = 461.0 $[M+1]^+$.

Step 3: N-(7-cyano-2,2-difluorobenzo[d][1,3]dioxol-4-yl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 144)

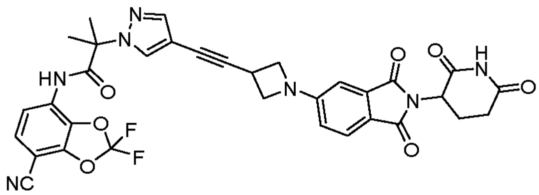

144c (0.43 g, 0.935 mmol), intermediate 1 (0.32 g), TEA (0.57 g, 5.63 mmol), CuI (36 mg, 0.189 mmol) and $PdCl_2(PPh_3)_2$ (66 mg, 0.094 mmol) were added to a reaction bottle, nitrogen replacement was performed three times, 5 mL of DMF was added under nitrogen protection, and the mixture was reacted at 55°C for 1.5 h. The reaction liquid was cooled to room temperature, 50 mL of water was added, and the mixture was filtered by suction. The filter cake was washed with 10 mL of water. The filter cake was dissolved in 100 mL of DCM, the mixture was dried over anhydrous sodium sulfate. The crude product was separated and purified with silica gel chromatography column (petroleum ether/dichloromethane/ethyl acetate (v/v) = 1 : 1 : 2) to afford compound 144 (0.1 g, yield: 16%).

$^1H$ NMR (400 MHz, $CDCl_3$) δ 9.63 (s, 1H), 8.09 (d, 1H), 7.99 (s, 1H), 7.83 (s, 1H), 7.78 (s, 1H), 7.67 (d, 1H), 7.29 (d, 1H), 6.84 - 6.77 (m, 1H), 6.56 (dd, 1H), 4.99 - 4.89 (m, 1H), 4.45 - 4.30 (m, 2H), 4.15 - 4.00 (m, 2H), 3.88 - 3.75 (m, 1H), 3.00 - 2.64 (m, 3H), 2.20 - 2.06 (m, 1H), 1.93 (s, 6H).

LCMS m/z = 670.4 $[M+1]^+$.

**Example 145: Preparation of compound 145**

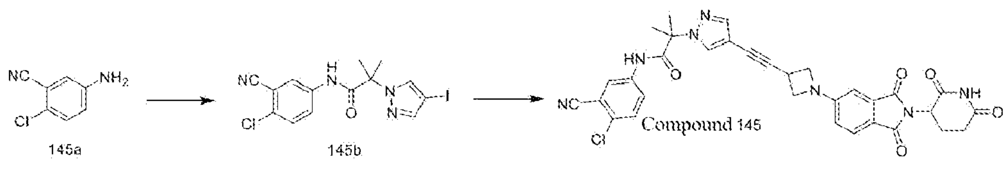


Step 1: N-(4-chloro-3-cyanophenyl)-2-(4-iodo-1H-pyrazol-1-yl)-2-methylpropanamide (145b)

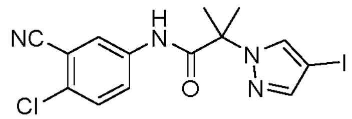

16a-1 (1.0 g, 3.58 mmol) was dissolved in 20 mL of DCM, 1-chloro-N,N,2-trimethylpropenylamine (0.72 g, 5.38 mmol) was added, the mixture was reacted at room temperature for 1 h, and then 145a (0.49 g, 3.21 mmol) and TEA (1.08 g, 10.67 mmol) were added in sequence, and the mixture was reacted at room temperature for 12 h. 10 mL of ethyl acetate and 10 mL of water were added to the reaction liquid, the organic phase was separated, washed with 10 mL of saturated sodium chloride solution, dried over anhydrous sodium sulfate, and concentrated under reduced pressure. The crude product was separated and purified with silica gel chromatography column (petroleum ether : ethyl acetate (v/v) = 5 : 1) to afford 145b (0.37 g, yield: 28%).

Step 2: N-(4-chloro-3-cyanophenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 145)

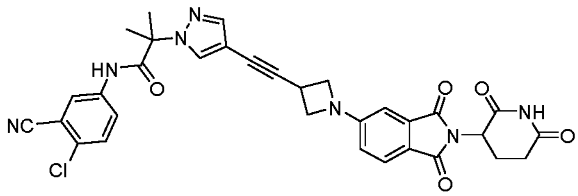

145b (0.37 g, 0.89 mmol) was dissolved in 5 mL of DMF, and intermediate 1 (0.30 g), TEA (0.27 g, 2.67 mmol), CuI (0.034 g, 0.18 mmol) and $PdCl_2(PPh_3)_2$ (0.062 g, 0.088 mmol) were added in sequence, and the mixture was reacted at 60°C for 2 h. The reaction liquid was cooled to room temperature, DCM (30 mL) and 30 mL of water were added, the organic phase was separated, the aqueous phase was extracted with DCM (30 mL $\times$ 3), the organic phases were combined, washed with 30 mL of saturated sodium chloride solution, dried over anhydrous sodium sulfate, and concentrated under reduced pressure. The crude product was separated and purified with silica gel chromatography column (petroleum ether/ethyl acetate (v/v) = 1 : 1) to afford compound 145 (53 mg, yield: 10%).

$^1H$ NMR (400 MHz, $CDCl_3$) $\delta$ 9.08 (s, 1H), 7.95 (s, 1H), 7.88 (d, 1H), 7.82 - 7.76 (m, 2H), 7.67 (d, 1H), 7.57 (dd, 1H), 7.44 - 7.37 (m, 1H), 6.84 - 6.75 (m, 1H), 6.56 (dd, 1H), 4.98 - 4.88 (m, 1H), 4.41 - 4.31 (m, 2H), 4.13 - 4.02 (m, 2H), 3.88 - 3.76 (m, 1H), 3.03 - 2.64 (m, 3H), 2.18 - 2.08 (m, 1H), 1.91 (s, 6H).

LCMS m/z = 622.1 $[M-1]^-$.

**Example 146: Preparation of compound 146**

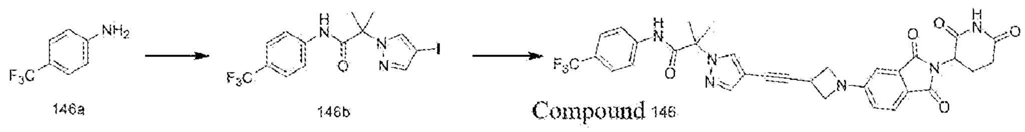


Step 1: 2-(4-iodo-1H-pyrazol-1-yl)-2-methyl-N-(4-(trifluoromethyl)phenyl) propanamide (146b)

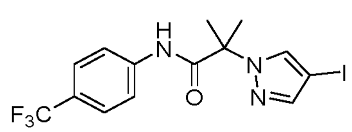

16a-1 (0.99 g, 3.54 mmol) was dissolved in 30 mL of DCM, 1-chloro-N,N,2-trimethylpropenylamine (0.65 g,

4.86 mmol) was added, the mixture was reacted at room temperature for 2 h, and then TEA (1.03 g, 10.18 mmol) and 146a (0.52 g, 3.23 mmol) were added in sequence, and the mixture was reacted at room temperature for 3 h. 100 mL of DCM and 50 mL of saturated aqueous sodium bicarbonate solution were added to the reaction liquid, the organic phase was separated, washed with 80 mL of saturated sodium chloride solution, dried over anhydrous sodium sulfate, and concentrated under reduced pressure. The crude product was separated and purified with silica gel chromatography column (petroleum ether/ethyl acetate (v/v) = 5 : 1) to afford 146b (1.2 g, yield: 88%).

Step 2: 2-(4-((1-(2-(2,6-dioxopiperidin-3-yl)-1,3-dioxoisoindolin-5-yl)azetidin-3-yl)ethynyl)-1H-pyrazol-1-yl)-2-methyl-N-(4-(trifluoromethyl)phenyl)propanamide (Compound 146)

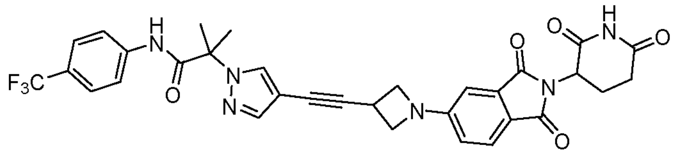

146b (0.21 g, 0.50 mmol) was dissolved in 6 mL of DMF, and intermediate 1 (0.19 g), TEA (0.15g, 1.48 mmol), CuI (10 mg, 0.053 mmol) and $PdCl_2(PPh_3)_2$ (35 mg, 0.05 mmol) were added in sequence, and the mixture was reacted at 55°C for 1 h. The reaction liquid was cooled to room temperature, 50 mL of water was added, and the mixture was filtered by suction. The filter cake was washed with 10 mL of water. The filter cake was dissolved in 100 mL of DCM, the mixture was dried over anhydrous sodium sulfate, and concentrated under reduced pressure. The crude product was separated and purified with silica gel chromatography column (petroleum ether/ethyl acetate (v/v) = 1 : 1) to afford compound 146 (0.12 g, yield: 38%).

$^1$H NMR (400 MHz, $CDCl_3$) δ 8.87 (s, 1H), 8.02 (s, 1H), 7.82 - 7.76 (m, 2H), 7.67 (d, 1H), 7.60 - 7.48 (m, 4H), 6.81 (d, 1H), 6.56 (dd, 1H), 5.00 - 4.86 (m, 1H), 4.43 - 4.30 (m, 2H), 4.13 - 4.00 (m, 2H), 3.90 - 3.73 (m, 1H), 3.00 - 2.60 (m, 3H), 2.23 - 2.05 (m, 1H), 1.92 (s, 6H).

LCMS m/z = 633.2 [M+1] $^+$.

**Example 147: Preparation of compound 147**

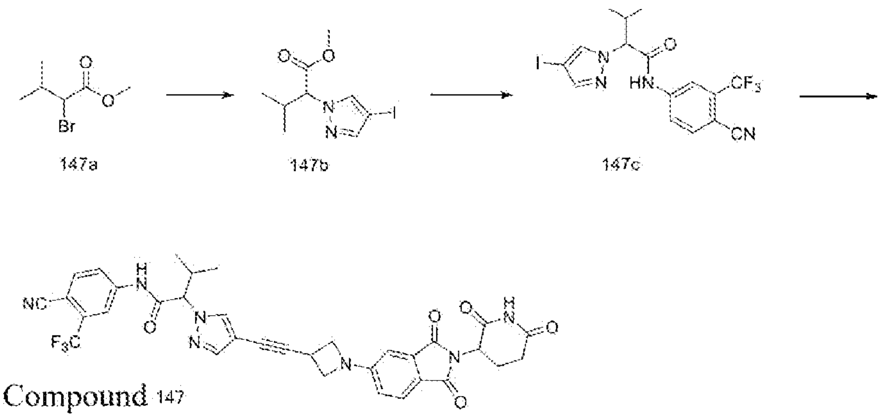
147a 147b 147c

Compound 147

Step 1: methyl 2-(4-iodo-1H-pyrazol-1-yl)-3-methylbutanoate (147b)

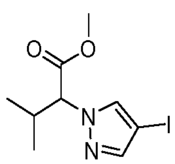

147a (2.0 g, 10.25 mmol) was dissolved in 20 mL of acetonitrile, 4-iodo-1H-pyrazole (1.99 g, 10.26 mmol) and cesium carbonate (4.34 g, 13.32 mmol) were added in sequence, and the mixture was stirred at 80°C for 2 h. The reaction liquid was cooled to room temperature and filtered, and the filtrate was concentrated under reduced pressure. The crude product was separated and purified with silica gel column chromatography (petroleum ether/ethyl acetate (v/v) = 100 : 1-10 : 1) to afford 147b (2.61 g, yield: 83%).

LCMS m/z = 309.0 $[M+1]^+$.

Step 2: N-(4-cyano-3-(trifluoromethyl)phenyl)-2-(4-iodo-1H-pyrazol-1-yl)-3-methylbutanamide (147c)

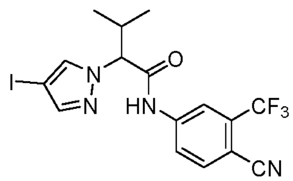

147b (2.60 g, 8.44 mmol) was dissolved in 40 mL of THF and 2 mL of methanol, 10 mL of aqueous solution of lithium hydroxide monohydrate (1.06 g, 25.26 mmol) was added, and the mixture was reacted at room temperature for 16 h. 2 mol/L hydrochloric acid solution was slowly added dropwise to the reaction system until the pH was 2, 20 mL of ethyl acetate was added for extraction, the organic phase was washed with 20 mL of saturated sodium chloride solution, dried over anhydrous sodium sulfate, and concentrated under reduced pressure to afford a crude product (2.4 g). The above crude product (1.0 g) was dissolved in 10 mL of pyridine, and 4-amino-2-(trifluoromethyl)benzonitrile (630 mg, 3.38 mmol) and a solution of 50% 1-propylphosphoric acid cyclic anhydride in ethyl acetate (4.33 g) were added in sequence, the mixture was reacted at 30°C for 16 h. The reaction liquid was cooled to room temperature, 40 mL of ethyl acetate was added, the organic phase was washed with 20 mL of saturated sodium bicarbonate solution and 20 mL of saturated sodium chloride solution, dried over anhydrous sodium sulfate, and concentrated under reduced pressure. The crude product was separated and purified with silica gel column chromatography (petroleum ether/ethyl acetate (v/v) = 1 : 0-4 : 1) to afford 147c (1.5 g, yield: 96%).

LCMS m/z = 463.0 $[M+1]^+$.

Step 3: N-(4-cyano-3-(trifluoromethyl)phenyl)-2-(4-((1-(2-(2,6-dioxopiperidin-3-yl)-1,3-dioxoisoindolin-5-yl)azetidin-3-yl)ethynyl)-1H-pyrazol-1-yl)-3-methylbutanamide (Compound 147)

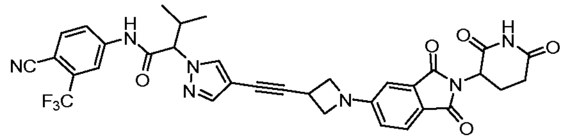

147c (300 mg, 0.65 mmol) was dissolved in 5 mL of DMF, and intermediate 1 (230 mg), CuI (12 mg, 0.063 mmol), $PdCl_2(PPh_3)_2$ (46 mg, 0.066 mmol) and TEA (0.27 mL, 1.94 mmol) were added in sequence, and the mixture was reacted at 55°C for 1 h under nitrogen protection. The reaction liquid was cooled to room temperature, 20 mL of water was added, the solid was precipitated, suction-filtration was performed, the filter cake was dissolved with 50 mL of DCM, the mixture was dried over anhydrous sodium sulfate, and concentrated under reduced pressure. The crude product was separated and purified with silica gel column chromatography (petroleum ether/ethyl acetate (v/v) = 10 : 1-1 : 2) to afford compound 147 (80 mg, yield: 18%).

$^1H$ NMR (400 MHz, $CDCl_3$) δ 10.06 (s, 1H), 8.19 (s, 1H), 8.02 - 7.96 (m, 1H), 7.93 - 7.85 (m, 1H), 7.81 - 7.61 (m, 4H), 6.85 - 6.76 (m, 1H), 6.56 (dd, 1H), 4.99 - 4.89 (m, 1H), 4.52 - 4.30 (m, 3H), 4.13 - 4.01 (m, 2H), 3.89 - 3.73 (m, 1H), 3.00 - 2.51 (m, 4H), 2.23 - 2.07 (m, 1H), 1.10 (d, 3H), 0.82 (d, 3H).

LCMS m/z = 672.2 $[M+1]^+$.

**Example 148: Preparation of compound 148**

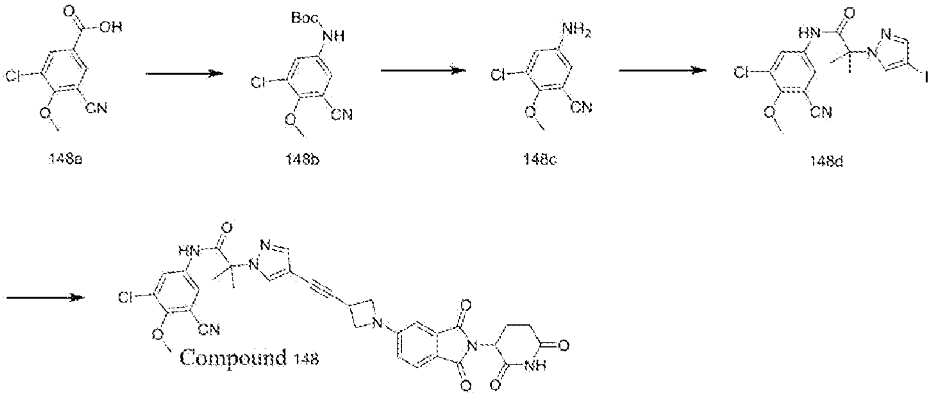

148a 148b 148c 148d

Compound 148

Step 1: tert-butyl N-(3-chloro-5-cyano-4-methoxyphenyl)carbamate (148b)

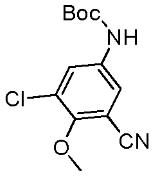

148a (0.4 g, 1.89 mmol) was dissolved in 5 mL of toluene, 5 mL of tert-butanol and TEA (0.54 g, 5.34 mmol) were added, then diphenylphosphoryl azide (0.73 g, 2.65 mmol) was added, and the mixture was reacted at 100°C for 3 h. The reaction liquid was cooled to room temperature, 10 mL of water and 20 mL of ethyl acetate were added, the liquid separation was conducted, the organic layer was washed with 10 mL of saturated sodium chloride solution, dried over anhydrous sodium sulfate, and concentrated under reduced pressure. The crude product obtained was separated and purified with silica gel chromatography column (ethyl acetate/petroleum ether (v/v) = 1 : 9) to afford 148b (0.3 g, yield: 56%).

LCMS m/z = 283.0 $[M+1]^+$.

Step 2: 5-amino-3-chloro-2-methoxybenzonitrile (148c)

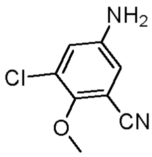

148b (0.3 g, 1.06 mmol) was dissolved in DCM (2 mL), 4 mL of a solution of 2 mol/L ethyl acetate hydrogen chloride solution was added, and the mixture was reacted at room temperature for 2 h. The reaction liquid was filtered, the filter cake was collected, 20 mL of DCM was added to the filter cake, saturated aqueous sodium bicarbonate solution was added dropwise to adjust the pH to 9, the liquid separation was conducted, the organic layer was washed with 5 mL of saturated sodium chloride solution, dried over anhydrous sodium sulfate, and concentrated under reduced pressure to afford crude 148c (0.05 g).

LCMS m/z = 183.1 $[M+1]^+$.

Step 3: N-(3-chloro-5-cyano-4-methoxyphenyl)-2-(4-iodo-1H-pyrazol-1-yl)-2-methylpropanamide (148d)

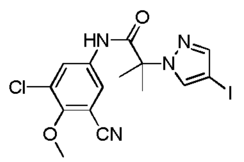

16a-1 (0.280 g, 1.00 mmol) was dissolved in 5 mL of DCM, 1-chloro-N,N,2-trimethylpropenylamine (0.13 g, 0.972 mmol) was added, the mixture was stirred at room temperature for 1 h, and then crude 148c (0.18 g,) and TEA (0.1 g, 0.99 mmol) were added, and the mixture was reacted at room temperature for 1 h. 10 mL of water and 20 mL of DCM were added to the reaction liquid, the liquid separation was conducted, the organic layer was washed with 10 mL of saturated sodium chloride solution, dried over anhydrous sodium sulfate, and concentrated under reduced pressure. The crude product obtained was separated and purified with silica gel chromatography column (ethyl acetate/petroleum ether (v/v) = 1 : 4) to afford 148d (0.16 g, yield: 36%).

LCMS m/z = 445.0 $[M+1]^+$.

Step 4: N-(3-chloro-5-cyano-4-methoxyphenyl)-2-(4-(2-(1-(2-(2,6-dioxopiperidin-3-yl)-1,3-dioxo-2,3-dihydro-1H-isoindol-5-yl)azetidin-3-yl)ethynyl)-1H-pyrazol-1-yl)-2-methylpropanamide (Compound 148)

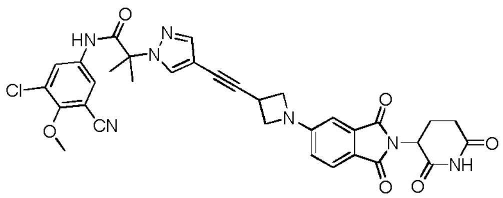

148d (0.15 g, 0.34 mmol) and intermediate 1 (0.11 g) were dissolved in 4 mL of DMF, DIPEA (0.13 g, 1.01 mmol) was added, and CuI (0.01 g, 0.0525 mmol) and $PdCl_2(PPh_3)_2$ (0.036 g, 0.0512 mmol) were added under nitrogen protection, and the mixture was reacted at room temperature for 3 h. 10 mL of water was added to the reaction liquid, the mixture was stirred for 5 min, and filtered by suction, the filter cake was washed with 5 mL of water, the filter cake was collected, the filter cake was dissolved with 50 mL of DCM, the mixture was dried over anhydrous sodium sulfate, concentrated under reduced pressure, and the crude product was separated and purified with silica gel chromatography column (ethyl acetate/petroleum ether (v/v) = 3 : 2) to afford compound 148 (0.07 g, yield: 31%).

$^1H$ NMR (400 MHz, $CDCl_3$) δ 8.99 (s, 1H), 8.10 - 7.95 (m, 1H), 7.84 - 7.57 (m, 5H), 6.84 - 6.78 (m, 1H), 6.56 (dd, 1H), 4.98 - 4.89 (m, 1H), 4.43 - 4.30 (m, 2H), 4.13 - 4.02 (m, 2H), 4.00 (s, 3H), 3.87 - 3.76 (m, 1H), 2.94 - 2.66 (m, 3H), 2.18 - 2.08 (m, 1H), 1.90 (s, 6H).

LCMS m/z = 652.1 $[M-1]^-$.

**Example 149: Preparation of compound 149**

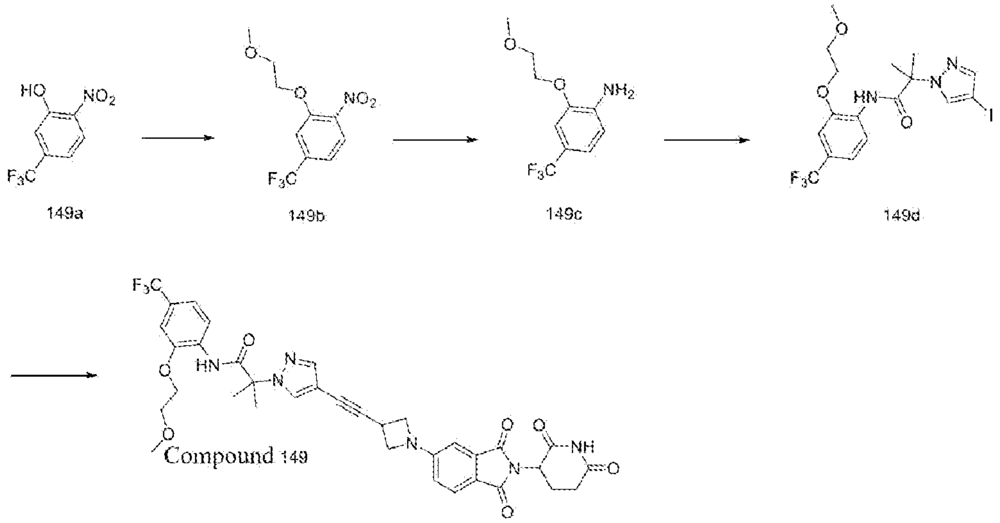

149a 149b 149c 149d

Compound 149

Step 1: 2-(2-methoxyethoxy)-1-nitro-4-(trifluoromethyl)benzene (149b)

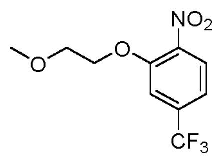

149a (2.0 g, 9.66 mmol), 1-bromo-2-methoxyethane (1.61 g, 11.58 mmol), potassium carbonate (2.67 g, 19.32 mmol) and solid potassium iodide (1.6 g, 9.64 mmol) were added into 20 mL of acetonitrile, the mixture was reacted at reflux for 14 h. The reaction liquid was cooled to room temperature and filtered, and the filtrate was concentrated under reduced pressure. The crude product was separated and purified with silica gel chromatography column (petroleum ether/ethyl acetate (v/v) = 100 : 1-10 : 1) to afford 149b (2.0 g, yield: 78%).

Step 2: 2-(2-methoxyethoxy)-4-(trifluoromethyl)aniline (149c)

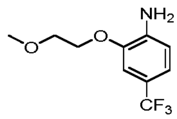

149b (2.0 g, 7.54 mmol) was dissolved in 20 mL of methanol, 0.2 g of 10% palladium on carbon was added, and the mixture was reacted at room temperature for 2 h under the atmosphere of hydrogen balloon. The reaction system was filtered by suction, and the filtrate was concentrated under reduced pressure to afford crude 149c (1.7 g).

LCMS m/z = 236.1 $[M+1]^+$.

Step 3: 2-(4-iodo-1H-pyrazol-1-yl)-N-(2-(2-methoxyethoxy)-4-(trifluoromethyl) phenyl)-2-methylpropanamide (149d)

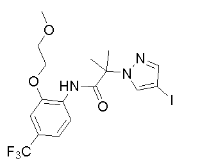

16a-1 (0.5 g, 1.78 mmol) was dissolved in 10 mL of DCM, 1-chloro-N,N,2-trimethylpropenylamine (0.36 g, 2.7 mmol) was added, the mixture was reacted at room temperature for 2 h, and then TEA (1.09 g, 10.77 mmol) and crude 149c (0.42 g) were added in sequence, and the mixture was reacted at room temperature for 12 h. 50 mL of DCM and 50 mL of water were added to the reaction liquid, the organic phase was separated, dried over anhydrous sodium sulfate, and concentrated under reduced pressure. The crude product was separated and purified with silica gel chromatography column (petroleum ether/ethyl acetate (v/v) = 20 : 1-5 : 1) to afford 149d (0.5 g, yield: 56%).

Step 4: 2-(4-((1-(2-(2,6-dioxopiperidin-3-yl)-1,3-dioxoisoindolin-5-yl)azetidin-3-yl)ethynyl)-1H-pyrazol-1-yl)-N-(2-(2-methoxyethoxy)-4-(trifluoromethyl)phenyl)-2-methylpropanamide (Compound 149)

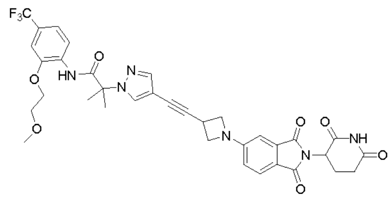

149d (0.47 g, 0.945 mmol), intermediate 1 (0.32 g), TEA (0.57 g, 5.63 mmol), CuI (36 mg, 0.189 mmol) and $PdCl_2(PPh_3)_2$ (66 mg, 0.094 mmol) were added to a reaction bottle, nitrogen replacement was performed three times, 5 mL of DMF was added under nitrogen protection, and the mixture was reacted at 60°C for 3 h. The reaction liquid was cooled to room temperature, 50 mL of water was added, and the mixture was filtered by suction. The filter cake was washed with 10 mL of water. The filter cake was dissolved in 100 mL of DCM, the mixture was dried over anhydrous sodium sulfate, and concentrated under reduced pressure. The crude product was separated and purified with silica gel chromatography column (ethyl acetate/petroleum ether/dichloromethane (v/v) = 2 : 1 : 1) to afford compound 149 (0.07 g, yield: 10%).

$^1H$ NMR (400 MHz, $CDCl_3$) δ 8.91 (s, 1H), 8.44 (d, 1H), 7.94 (s, 1H), 7.81 - 7.73 (m, 2H), 7.67 (d, 1H), 7.21 (d, 1H), 7.06 - 7.01 (m, 1H), 6.85 - 6.77 (m, 1H), 6.60 - 6.50 (m, 1H), 4.98 - 4.88 (m, 1H), 4.42 - 4.30 (m, 2H), 4.19 - 4.10 (m, 2H), 4.10 - 4.01 (m, 2H), 3.89 - 3.70 (m, 3H), 3.47 (s, 3H), 2.97 - 2.63 (m, 3H), 2.21 - 2.07 (m, 1H), 1.93 (s, 6H).

LCMS m/z = 707.3 $[M+1]^+$.

**Example 150: Preparation of compound 150**

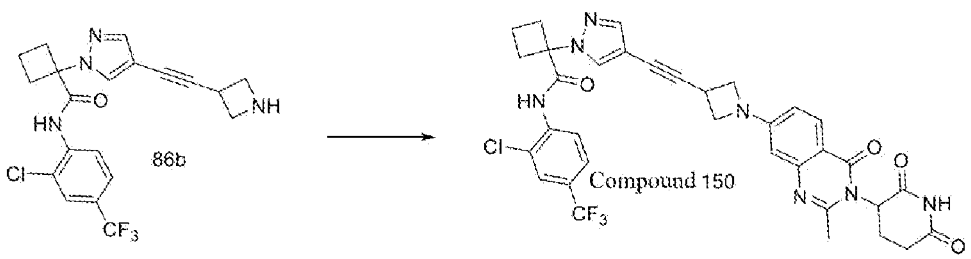

The trifluoroacetate of crude 86b (58 mg) was dissolved in 6 mL of dry DMSO, sodium bicarbonate (35 mg, 0.42 mmol) was added, the mixture was stirred at room temperature for 20 min, 3-(7-fluoro-2-methyl-4-oxoquinazolin-3(4H)-yl)piperidine-2,6-dione (see WO 2009042177 for the synthesis method) (40 mg, 0.138 mmol) and DIPEA (53 mg, 0.41 mmol) were added in sequence, and the mixture was reacted at 110°C for 16 h. The reaction liquid was cooled to room temperature, 60 mL of water was added, DCM (30 mL) was added, the organic phase was separated, the aqueous phase was extracted with DCM (20 mL × 3), the organic phases were combined, and the organic phase was washed with 20 mL of saturated aqueous sodium chloride solution, dried over anhydrous sodium sulfate, and concentrated under reduced pressure. The crude product was separated and purified with prep-TLC (petroleum ether : ethyl acetate (v/v) = 1 : 5) to afford compound 150 (7 mg, yield: 7%).

$^1H$ NMR (400 MHz, $CDCl_3$) δ 8.69 (s, 1H), 8.54 (d, 1H), 8.15 - 7.92 (m, 2H), 7.78 (s, 1H), 7.70 (s, 1H), 7.64 - 7.45 (m, 2H), 6.52 (dd, 1H), 6.48 - 6.42 (m, 1H), 4.75 - 4.60 (m, 1H), 4.40 - 4.25 (m, 2H), 4.13 - 3.96 (m, 2H), 3.86 - 3.70

(m, 1H), 3.15 - 2.56 (m, 10H), 2.30 - 1.95 (m, 3H).

LCMS m/z = 692.2 [M+1] $^+$.

**Example 151: Preparation of compound 151**

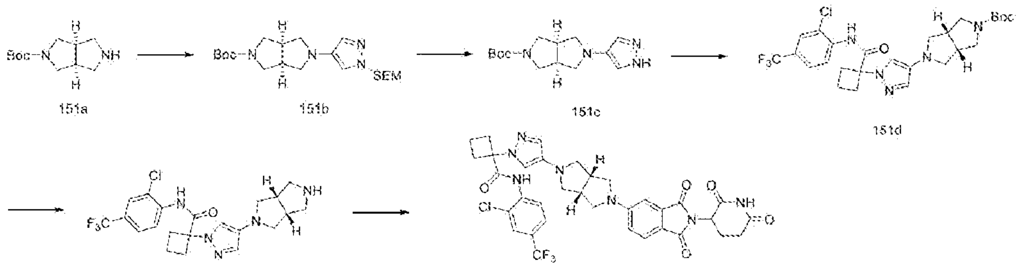

151a 151b 151c 151d 151e Compound 151

Step 1: cis-tert-butyl 5-(1-((2-(trimethylsilyl)ethoxy)methyl)-1H-pyrazol-4-yl)hexahydropyrrolo[3,4-c]pyrrole-2(1H)-carboxylate (151b)

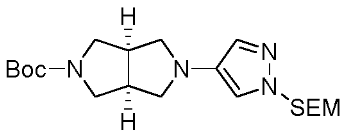

4-iodo-1-((2-(trimethylsilyl)ethoxy)methyl)-1H-pyrazole (1.0 g, 3.08 mmol) and 151a (0.65 g, 3.06 mmol) were dissolved in 25 mL of DMSO, potassium carbonate (1.06 g, 7.67 mmol) was added, nitrogen replacement was performed three times, L-proline (0.11 g, 0.96 mmol) and CuI (0.088 g, 0.462 mmol) were added, nitrogen replacement was performed three times, and the mixture was reacted at 100°C for 7 h. The reaction system was cooled to room temperature, 100 mL of water was added, the mixture was extracted with 100 mL of ethyl acetate. The organic phase was washed with 50 mL of water, dried over anhydrous sodium sulfate and concentrated under reduced pressure. The crude product was separated and purified by silica gel chromatography column (petroleum ether : ethyl acetate (v/v) = 2 : 1) to afford 151b (0.5 g, yield: 40%).

LCMS m/z = 409.7 [M+1]$^+$.

Step 2: cis-tert-butyl 5-(1H-pyrazol-4-yl)hexahydropyrrolo[3,4-c]pyrrole-2(1H)-carboxylate (151c)

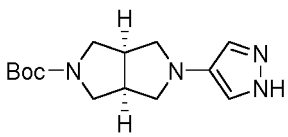

151b (0.55 g, 1.35 mmol) was dissolved in 20 mL of THF, tetrabutylammonium fluoride trihydrate (4.26 g, 13.5 mmol) was added, and the mixture was reacted at 85°C for 5 h. The reaction system was cooled to room temperature, 50 mL of water was slowly added, the mixture was extracted with 100 mL of ethyl acetate, the organic phase was separated, the organic phase was washed with 50 mL of water, dried over anhydrous sodium sulfate, concentrated under reduced pressure, and the crude product was separated and purified with silica gel column chromatography (petroleum ether/ethyl acetate (v/v) = 2 : 1) to afford 151c (0.3 g, yield: 80%).

LCMS m/z = 279.4 [M+1]$^+$.

Step 3: cis-tert-butyl 5-(1-(1-((2-chloro-4-(trifluoromethyl)phenyl)carbamoyl) cyclobutyl)-1H-pyrazol-4-yl)hexahydropyrrolo[3,4-c]pyrrole-2(1H)-carboxylate (151d)

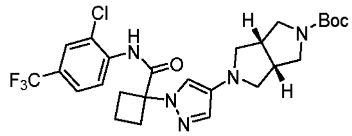

151c (300 mg, 1.08 mmol) and 96b (0.46 g, 1.29 mmol) were dissolved in 15 mL of acetonitrile, potassium carbonate (0.70 g, 5.07 mmol) was added, and the mixture was reacted at 50°C for 6 h. The reaction system was cooled to room temperature and filtered, and the filtrate was concentrated under reduced pressure. The crude product was separated and purified with silica gel chromatography column (petroleum ether/ethyl acetate (v/v) = 4 : 1) to afford 151d (180 mg, yield: 30%).

LC-MS m/z = 554.2 $[M+1]^+$.

Step 4: cis-N-(2-chloro-4-(trifluoromethyl)phenyl)-1-(4-(hexahydropyrrolo[3,4-c]pyrrol-2(1H)-yl)- 1H-pyrazol-1-yl)cyclobutane-1-carboxamide (151e)trifluoroacetate

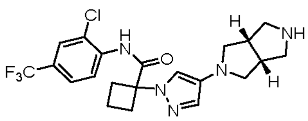

151d (180 mg, 0.325 mmol) was dissolved in 5 mL of DCM, and 2 mL of trifluoroacetic acid was added. The mixture was reacted at room temperature for 2 h. The reaction liquid was concentrated under reduced pressure to afford the trifluoroacetate of crude 151e (220 mg).

LC-MS m/z = 454.2 $[M+1]^+$.

Step 5: cis-N-(2-chloro-4-(trifluoromethyl)phenyl)-1-(4-(5-(2-(2,6-dioxopiperidin-3-yl)-1,3-dioxoisoindolin-5-yl)hexahydropyrrolo[3,4-c]pyrrol-2(1H)-yl)-1H-pyrazol-1-yl)cyclobutane-1-carboxamide (Example 151)

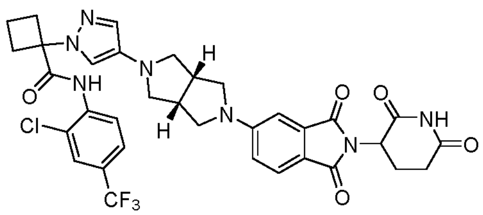

The trifluoroacetate of crude 151e (220 mg) was dissolved in 5 mL of DMSO, sodium bicarbonate (54 mg, 0.64 mmol) was added, the mixture was stirred at room temperature for 10 min, then 0.2 mL of DIPEA and 1C (130 mg, 0.47 mmol) were added, the mixture was reacted at 80°C for 6 h. The reaction liquid was cooled to room temperature, 50 mL of water was added, the mixture was filtered, and the solid was collected. The filter cake was washed with 10 mL of water, the filter cake was dissolved in 100 mL of DCM, the mixture was dried over anhydrous sodium sulfate, and concentrated under reduced pressure. The crude product was separated and purified with silica gel chromatography column (DCM/MeOH (v/v) = 15 : 1) to afford compound 151 (140 mg, yield: 42%).

$^1H$ NMR (400 MHz, $CDCl_3$) δ 8.61 - 8.46 (m, 2H), 8.01 (s, 1H), 7.66 (d, 1H), 7.58 - 7.45 (m, 2H), 7.37 - 7.31 (m, 1H), 7.04 - 6.92 (m, 2H), 6.71 (dd, 1H), 4.98 - 4.86 (m, 1H), 3.79 - 3.68 (m, 2H), 3.47 - 3.28 (m, 4H), 3.28 - 3.10 (m, 4H), 3.08 - 2.94 (m, 2H), 2.93 - 2.64 (m, 5H), 2.30 - 2.00 (m, 3H).

LCMS m/z = 710.2 $[M +1]^+$.

**Example 152: Preparation of compound 152**

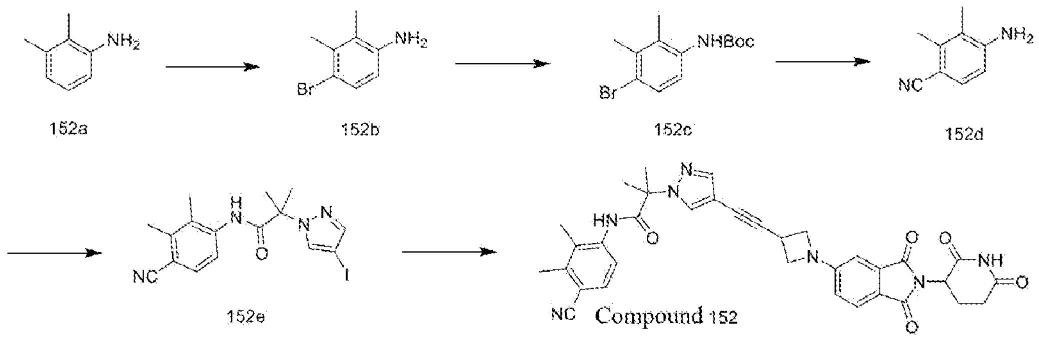

152a 152b 152c 152d 152e Compound 152

Step 1: 4-bromo-2,3-dimethylaniline (152b)

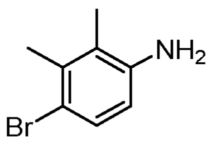

152a (6.00 g, 49.51 mmol) was dissolved in 60 mL of DMF, N-bromosuccinimide (8.81 g, 49.50 mmol) was added, and the mixture was reacted at room temperature for 16 h. 50 mL of ethyl acetate and 80 mL of water were added to the reaction liquid, the liquid separation was conducted, the organic phase was washed with 50 mL of saturated aqueous sodium chloride solution, dried over anhydrous sodium sulfate, and concentrated under reduced pressure. The crude product was separated and purified with silica gel chromatography column (petroleum ether : ethyl acetate (v/v) = 10 : 1) to afford 152b (7.20 g, yield: 73%).

LCMS m/z = 200.1 $[M+1]^+$.

Step 2: tert-butyl (4-bromo-2,3-dimethylphenyl)carbamate (152c)

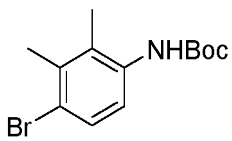

152b (3.00 g, 15.07 mmol) was dissolved in 24 mL of methanol, di-tert-butyl dicarbonate (3.92 g, 17.96 mmol) and sodium carbonate (1.91 g, 18.02 mmol) were added in sequence, and the mixture was reacted at room temperature for 16 h. The reaction liquid was filtered, and the filtrate was concentrated under reduced pressure. The crude product was separated and purified with silica gel chromatography column (petroleum ether : ethyl acetate (v/v) = 10 : 1) to afford 152c (3.2 g, yield: 71%).

Step 3: 4-amino-2,3-dimethylbenzonitrile (152d)

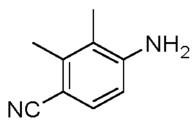

152c (2.7 g, 8.99 mmol) was dissolved in 30 mL of NMP, CuCN (2.43 g, 27.13 mmol) was added, and the mixture was reacted at 170°C for 4 h. The reaction system was cooled to room temperature, filtered with diatomaceous earth, and washed with 50 mL of ethyl acetate, 50 mL of water was added to the filtrate, the organic phase was separated, the organic phase was washed with 50 mL of saturated aqueous sodium chloride solution, dried over anhydrous sodium sulfate, concentrated under reduced pressure, and the crude product was separated and purified with silica gel chromatography column (petroleum ether : ethyl acetate (v/v) = 5 : 1) to afford crude 152d (1.00 g).
LCMS m/z = 147.2 $[M+1]^+$.

Step 4: N-(4-cyano-2,3-dimethylphenyl)-2-(4-iodo-1H-pyrazol-1-yl)-2-methylpropanamide (152e)

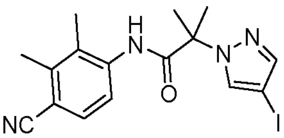

16a-1 (1.15 g, 4.12 mmol) was dissolved in DCM (10 mL), 1-chloro-N,N,2-trimethylpropenylamine (0.69 g, 5.16 mmol) was added, the mixture was reacted at room temperature for 2 h, and then crude 152d (0.50 g) and TEA (1.04 g, 10.28 mmol) were added in sequence, and the mixture was reacted at room temperature for 12 h. 10 mL of ethyl acetate and 10 mL of water were added to the reaction liquid, the organic phase was separated, the organic phase was washed with 10 mL of saturated aqueous sodium chloride solution, dried over anhydrous sodium sulfate, and concentrated under reduced pressure. The crude product was separated and purified with silica gel chromatography column (petroleum ether : ethyl acetate (v/v) = 5 : 1) to afford 152e (0.60 g, yield: 36%).
LCMS m/z = 409.0 $[M+1]^+$.

Step 5: N-(4-cyano-2,3-dimethylphenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 152)

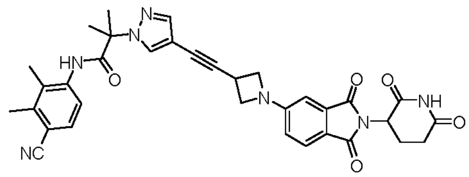

152e (0.60 g, 1.47 mmol) was dissolved in 10 mL of DMF, and intermediate 1 (0.50 g), TEA (0.45 g, 4.45 mmol), CuI (0.056 g, 0.294 mmol) and $PdCl_2(PPh_3)_2$ (0.10 g, 0.14 mmol) were added in sequence, and the mixture was reacted at 60°C for 2 h. The reaction liquid was cooled to room temperature, DCM (30 mL) was added, 30 mL of water was added, the organic phase was separated, the aqueous phase was extracted with DCM (30 mL × 3), the organic phases were combined, and the organic phase was washed with 20 mL of saturated aqueous sodium chloride solution, dried over anhydrous sodium sulfate, and concentrated under reduced pressure. The crude product was separated and purified with silica gel chromatography column (petroleum ether : ethyl acetate (v/v) = 1 : 1) to afford compound 152 (0.22 g, yield: 24%).
$^1H$ NMR (400 MHz, $CDCl_3$) δ 8.80 (s, 1H), 8.03 - 7.92 (m, 2H), 7.85 - 7.75 (m, 2H), 7.67 (d, 1H), 7.44 (d, 1H), 6.85 - 6.77 (m, 1H), 6.56 (dd, 1H), 4.98 - 4.87 (m, 1H), 4.43 - 4.30 (m, 2H), 4.12 - 4.00 (m, 2H), 3.88 - 3.73 (m, 1H), 2.96 - 2.64 (m, 3H), 2.48 (s, 3H), 2.18 - 2.08 (m, 1H), 2.05 (s, 3H), 1.94 (s, 6H).
LCMS m/z = 618.3 $[M+1]^+$.

**Example 153: Preparation of compound 153**

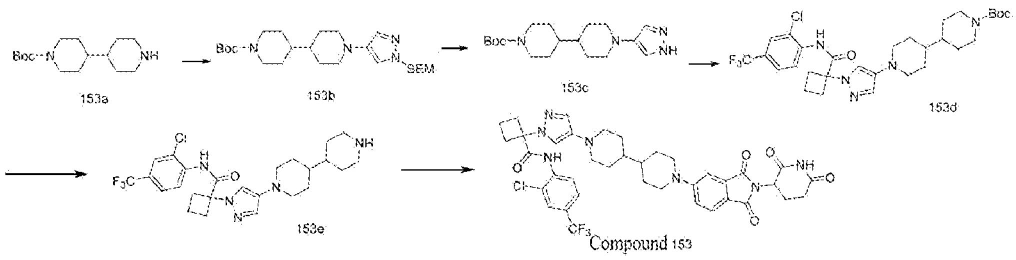


Step 1: tert-butyl 1'-(1-((2-(trimethylsilyl)ethoxy)methyl)-1H-pyrazol-4-yl)-[4,4'-bipiperidine]-1-carboxylate (153b)

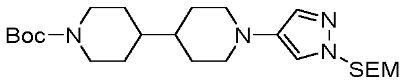

4-iodo-1-((2-(trimethylsilyl)ethoxy)methyl)-1H-pyrazole (1.0 g, 3.08 mmol) and 153a (1.65 g, 6.15 mmol) were dissolved in 25 mL of DMSO, potassium carbonate (1.06 g, 7.67 mmol) was added, nitrogen replacement was performed three times, L-proline (0.11 g, 0.96 mmol) and CuI (0.088 g, 0.462 mmol) were added, nitrogen replacement was performed three times, and the mixture was reacted at 100°C for 7 h. The reaction system was cooled to room temperature, 100 mL of water was added, the mixture was extracted with 100 mL of ethyl acetate. The organic phase was washed with 50 mL of water, dried over anhydrous sodium sulfate and concentrated under reduced pressure. The crude product was separated and purified by silica gel chromatography column (petroleum ether : ethyl acetate (v/v) = 2 : 1) to afford 153b (1.0 g, yield: 70%).

LCMS m/z = 465.7 $[M+1]^+$.

Step 2: tert-butyl 1'-(1H-pyrazol-4-yl)-[4,4'-bipiperidine]-1-carboxylate (153c)

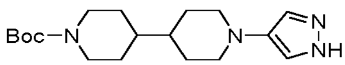

153b (0.5 g, 1.08 mmol) was dissolved in 20 mL of THF, tetrabutylammonium fluoride trihydrate (3.41 g, 10.81 mmol) was added, and the mixture was reacted at 85°C for 5 h. The reaction system was cooled to room temperature, 50 mL of water was slowly added, the mixture was extracted with 100 mL of ethyl acetate, the organic phase was separated, the organic phase was washed with 50 mL of water, dried over anhydrous sodium sulfate, concentrated under reduced pressure, and the crude product was separated and purified with silica gel column chromatography (petroleum ether/ethyl acetate (v/v) = 2 : 1) to afford 153c (0.2 g, yield: 55%).

Step 3: tert-butyl 1'-(1-(1-((2-chloro-4-(trifluoromethyl)phenyl)carbamoyl) cyclobutyl)-1H-pyrazol-4-yl)-[4,4'-bipiperidine]-1-carboxylate (153d)

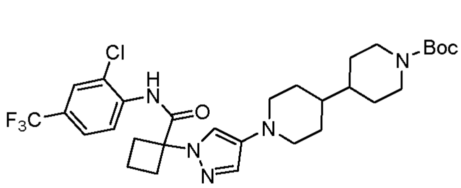

153c (200 mg, 0.60 mmol) and 96b (0.20 g, 0.56 mmol) were dissolved in 15 mL of acetonitrile, potassium carbonate (0.37 g, 2.68 mmol) was added, and the mixture was reacted at 50°C for 6 h. The reaction system was cooled to room temperature and filtered, and the filtrate was concentrated under reduced pressure. The crude product was separated and purified with silica gel chromatography column (petroleum ether/ethyl acetate (v/v) = 4 : 1) to afford 153d (160 mg, yield: 44%).

LC-MS m/z = 610.7 [M+1]+.

Step 4: 1-(4-([4,4'-bipiperidin]-1-yl)-1H-pyrazol-1-yl)-N-(2-chloro-4-(trifluoromethyl)phenyl)cyclobutane-1-carboxamide (153e) trifluoroacetate

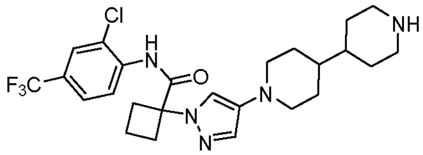

153d (150 mg, 0.25 mmol) was dissolved in 5 mL of DCM, and 2 mL of trifluoroacetic acid was added. The mixture was reacted at room temperature for 2 h. The reaction liquid was concentrated under reduced pressure to afford the trifluoroacetate of crude 153e (160 mg).
LC-MS m/z = 510.2 [M+1]+.

Step 5: N-(2-chloro-4-(trifluoromethyl)phenyl)-1-(4-(1'-(2-(2,6-dioxopiperidin-3-yl)-1,3-dioxoisoindolin-5-yl)-[4,4'-bipiperidin]-1-yl)-1H-pyrazol-1-yl)cyclobutane-1-carboxamide (Compound 153)

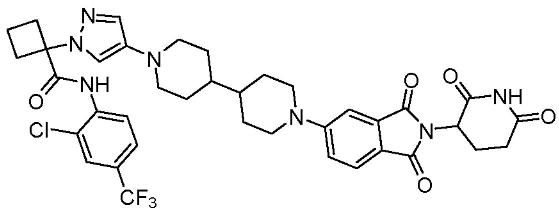

The trifluoroacetate of crude 153e (150 mg) was dissolved in 5 mL of DMSO, sodium bicarbonate (37 mg, 0.44 mmol) was added, the mixture was stirred at room temperature for 10 min, then 0.2 mL of DIPEA and 1C (91 mg, 0.33 mmol) were added, the mixture was reacted at 80°C for 8 h. The reaction liquid was cooled to room temperature, 50 mL of water was added, the mixture was filtered, and the solid was collected. The filter cake was washed with 10 mL of water, the filter cake was dissolved in 100 mL of DCM, the mixture was dried over anhydrous sodium sulfate, and concentrated under reduced pressure. The crude product was separated and purified with silica gel chromatography column (DCM/MeOH (v/v) = 15 : 1) to afford compound 153 (30 mg, yield: 12%).
$^1$H NMR (400 MHz, $CDCl_3$) δ 8.60 - 8.44 (m, 2H), 8.00 (s, 1H), 7.71 - 7.63 (m, 1H), 7.59 - 7.43 (m, 3H), 7.30 - 7.26 (m, 1H), 7.12 - 7.00 (m, 2H), 5.00 - 4.88 (m, 1H), 4.05 - 3.93 (m, 2H), 3.45 - 3.34 (m, 2H), 3.09 - 2.65 (m, 9H), 2.60 - 2.45 (m, 2H), 2.30 - 2.02 (m, 3H), 1.94 - 1.72 (m, 4H), 1.55 - 1.05 (m, 6H).
LCMS m/z = 766.2 [M +1]+.

**Example 154: Preparation of compound 154**

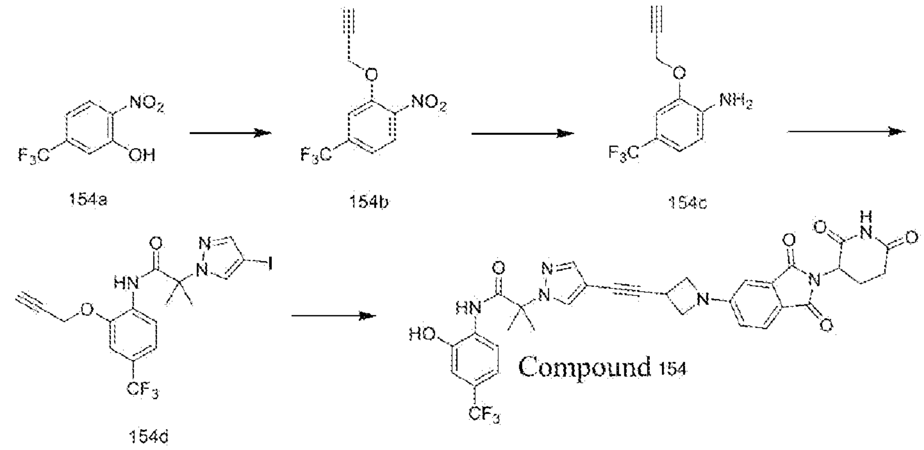

154a 154b 154c

154d Compound 154

Step 1: 1-nitro-2-(prop-2-yn-1-yloxy)-4-(trifluoromethyl)benzene (154b)

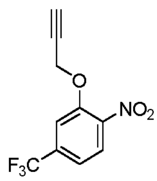

154a (2.07 g, 10.00 mmol), propargyl bromide (2.39 g, 20.09 mmol) and potassium carbonate (2.77 g, 20.04 mmol) were added to 35 mL of acetonitrile and the mixture was reacted at 55°C for 16 h. The reaction system was cooled to room temperature and filtered, and the filtrate was concentrated under reduced pressure. The crude product was separated and purified with silica gel chromatography column (petroleum ether/ethyl acetate (v/v) = 10 : 1) to afford 154b (2.3 g, yield: 94%).

Step 2: 2-(prop-2-yn-1-yloxy)-4-(trifluoromethyl)aniline (154c)

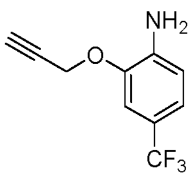

154b (0.5 g, 2.04 mmol) was dissolved in 5 mL of ethanol, 2 mL of saturated aqueous ammonium chloride solution was added, reduced iron powder (0.57 g, 10.18 mmol) was added, and the mixture was reacted at 80°C for 1 h. The reaction system was cooled to room temperature and filtered, 5 mL of water was added to the filtrate, the mixture was extracted with 20 mL of ethyl acetate. The organic phase was washed with 10 mL of water, dried over anhydrous sodium sulfate and concentrated under reduced pressure. The crude product was separated and purified by silica gel chromatography column (petroleum ether/ethyl acetate (v/v) = 10 : 1) to afford 154c (0.36 g, yield: 82%).

LCMS m/z = 215.9 $[M+1]^+$.

Step 3: 2-(4-iodo-1H-pyrazol-1-yl)-2-methyl-N-(2-(prop-2-yn-1-yloxy)-4-(trifluoromethyl)phenyl)propanamide (154d)

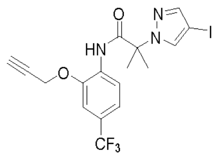

16a-1 (0.56 g, 2.00 mmol) was dissolved in 10 mL of DCM, 1-chloro-N,N,2-trimethylpropenylamine (0.36 g, 2.7 mmol) was added, the mixture was reacted at room temperature for 2 h, and then TEA (0.51 g, 5.04 mmol) and 154c (0.36 g, 1.67 mmol) were added in sequence, and the mixture was reacted at room temperature for 3 h. 50 mL of DCM and 50 mL of saturated aqueous sodium bicarbonate solution were added to the reaction liquid, the organic phase was separated, dried over anhydrous sodium sulfate, and concentrated under reduced pressure. The crude product was separated and purified with silica gel chromatography column (petroleum ether/ethyl acetate (v/v) = 10 : 1) to afford 154d (0.53 g, yield: 67%).

Step 4: 2-(4-((1-(2-(2,6-dioxopiperidin-3-yl)-1,3-dioxoisoindolin-5-yl)azetidin-3-yl)ethynyl)-1H-pyrazol-1-yl)-N-(2-hydroxy-4-(trifluoromethyl)phenyl)-2-methylpropanamide (Compound 154)

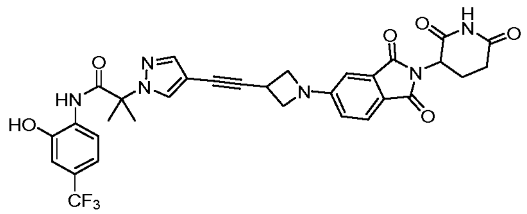

154d (0.2 g, 0.42 mmol), intermediate 1 (0.17 g), TEA (0.12 g, 1.19 mmol), CuI (8 mg, 0.042 mmol) and $PdCl_2(PPh_3)_2$ (29 mg, 0.041 mmol) were added to a reaction bottle, nitrogen replacement was performed three times, 5 mL of DMF was added under nitrogen protection, and the mixture was reacted at 60°C for 1 h. The reaction liquid was cooled to room temperature, 50 mL of water was added, and the mixture was filtered by suction. The filter cake was washed with 10 mL of water. The filter cake was dissolved in 100 mL of DCM, the mixture was dried over anhydrous sodium sulfate, and concentrated under reduced pressure. The crude product was separated and purified with silica gel column chromatography (petroleum ether/ethyl acetate (v/v) = 1 : 1) to afford compound 154 (0.08 g, yield: 29%).

$^1H$ NMR (400 MHz, $CDCl_3$) δ 9.22 (s, 1H), 8.45 (br.s, 1H), 8.10 - 8.00 (m, 1H), 7.81 (s, 2H), 7.72 - 7.62 (m, 1H), 7.25 - 7.02 (m, 3H), 6.85 - 6.75 (m, 1H), 6.55 (dd, 1H), 4.99 - 4.88 (m, 1H), 4.43 - 4.30 (m, 2H), 4.13 - 4.00 (m, 2H), 3.88 - 3.74 (m, 1H), 2.98 - 2.62 (m, 3H), 2.20 - 2.06 (m, 1H), 1.95 (s, 6H).

LCMS m/z = 649.4 $[M+1]^+$.

**Example 155: Preparation of compound 155**

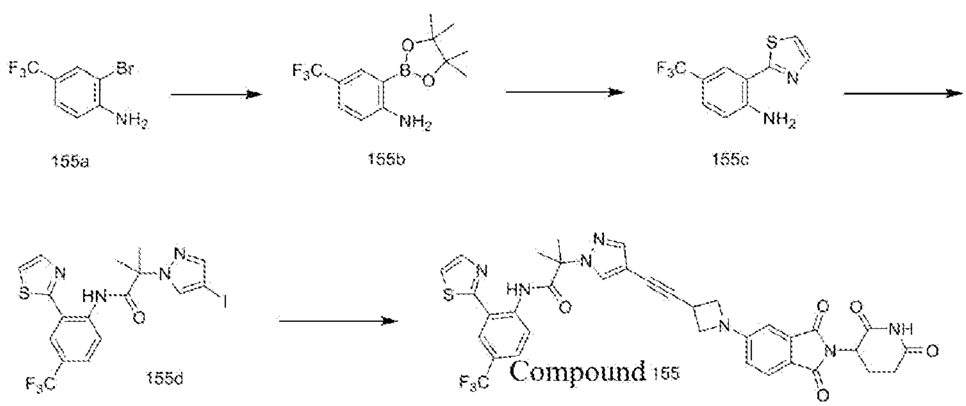
155a 155b 155c 155d Compound 155

Step 1: 2-(4,4,5,5-tetramethyl-1,3,2-dioxaborolan-2-yl)-4-(trifluoromethyl) aniline (155b)

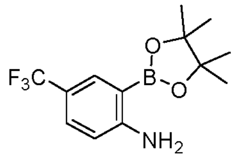

155a (5.0 g, 20.81 mmol) was dissolved in 100 mL of 1,4-dioxane, and bis(pinacolato)diboron (6.88 g, 27.09 mmol), potassium acetate (8.18 g, 83.35 mmol), and Pd(dppf)$Cl_2$·DCM (0.34 g, 0.42 mmol) were added in sequence under nitrogen protection, and the mixture was reacted at 100°C for 16 h. The reaction liquid was cooled to room temperature, 50 mL of ethyl acetate and 80 mL of water were added, the liquid separation was conducted, the aqueous phase was extracted with 50 mL of ethyl acetate, the organic phases were combined, the organic phase was washed with 50 mL of saturated aqueous sodium chloride solution, dried over anhydrous sodium sulfate, and concentrated under reduced pressure. The crude product was separated and purified with silica gel chromatography column (petroleum ether : ethyl acetate (v/v) = 5 : 1) to afford 155b (4.0 g, yield: 67%).

LCMS m/z = 288.0 $[M+1]^+$.

Step 2: 2-(thiazol-2-yl)-4-(trifluoromethyl)aniline (155c)

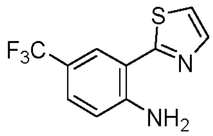

155b (4.00 g, 13.94 mmol) was dissolved in 40 mL of 1,4-dioxane and 10 mL of water, and 2-bromothiazole (3.43 g, 20.91 mmol), sodium carbonate (5.91 g, 55.76 mmol), and Pd(dppf)$Cl_2$•DCM (0.57 g, 0.70 mmol) were added in sequence under nitrogen protection, the mixture was reacted at 100°C for 16 h. The reaction system was cooled to room temperature, 20 mL of ethyl acetate and 40 mL of water were added, the liquid separation was conducted, the aqueous phase was extracted with 20 mL of ethyl acetate, the organic phases were combined, the organic phase was washed with 50 mL of saturated aqueous sodium chloride solution, dried over anhydrous sodium sulfate, and concentrated under reduced pressure. The crude product was separated and purified with silica gel chromatography column (petroleum ether : ethyl acetate (v/v) = 5 : 1) to afford 155c (1.50 g, yield: 44%).

LCMS m/z = 245.2 $[M+1]^+$.

Step 3: 2-(4-iodo-1H-pyrazol-1-yl)-2-methyl-N-(2-(thiazol-2-yl)-4-(trifluoromethyl)phenyl)propanamide (155d)

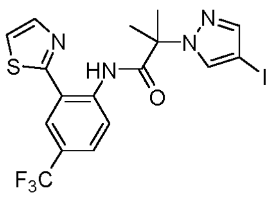

16a-1 (0.70 g, 2.50 mmol) was dissolved in DCM (10 mL), 1-chloro-N,N,2-trimethylpropenylamine (0.50 g, 3.75 mmol) was added, the mixture was reacted at room temperature for 2 h, and then 155c (0.67 g, 2.74 mmol) and TEA (0.76 g, 7.51 mmol) were added in sequence, and the mixture was reacted at room temperature for 12 h. 10 mL of ethyl acetate and 10 mL of water were added to the reaction liquid, the organic phase was separated, the organic phase was washed with 10 mL of saturated aqueous sodium chloride solution, dried over anhydrous sodium sulfate, and concentrated

under reduced pressure. The crude product was separated and purified with silica gel chromatography column (petroleum ether : ethyl acetate (v/v) = 5 : 1) to afford 155d (0.76 g, yield: 60%).

LCMS m/z = 507.0 [M+1] +.

Step 4: 2-(4-((1-(2-(2,6-dioxopiperidin-3-yl)-1,3-dioxoisoindolin-5-yl)azetidin-3-yl)ethynyl)-1H-pyrazol-1-yl)-2-methyl-N-(2-(thiazol-2-yl)-4-(trifluoromethyl) phenyl)propanamide (Compound 155)

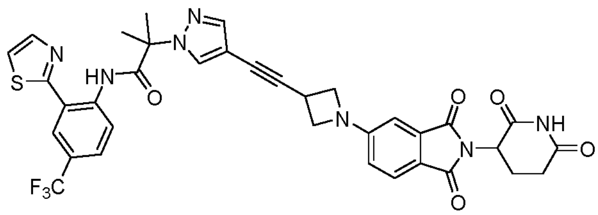

155d (0.66 g, 1.30 mmol) was dissolved in 10 mL of DMF, and intermediate 1 (0.44 g), TEA (0.39 g, 3.85 mmol), CuI (0.050 g, 0.26 mmol) and $PdCl_2(PPh_3)_2$ (0.091 g, 0.13 mmol) were added in sequence, and the mixture was reacted at 60°C for 2 h. The reaction liquid was cooled to room temperature, DCM (30 mL) was added, 30 mL of water was added, the organic phase was separated, the aqueous phase was extracted with DCM (30 mL × 3), the organic phases were combined, and the organic phase was washed with 20 mL of saturated aqueous sodium chloride solution, dried over anhydrous sodium sulfate, and concentrated under reduced pressure. The crude product was separated and purified with silica gel chromatography column (petroleum ether : ethyl acetate (v/v) = 1 : 1) to afford compound 155 (0.45 g, yield: 48%).

$^1H$ NMR (400 MHz, $CDCl_3$) δ 12.25 (s, 1H), 8.93 (d, 1H), 8.00 - 7.90 (m, 2H), 7.86 (s, 1H), 7.75 - 7.57 (m, 4H), 7.35 - 7.30 (m, 1H), 6.86 - 6.79 (m, 1H), 6.57 (dd, 1H), 5.00 - 4.88 (m, 1H), 4.45 - 4.30 (m, 2H), 4.15 - 4.02 (m, 2H), 3.93 - 3.77 (m, 1H), 2.98 - 2.66 (m, 3H), 2.20 - 2.08 (m, 1H), 1.94 (s, 6H).

LCMS m/z = 716.4 [M+1] +.

**Example 156: Preparation of the trifluoroacetate of compound 156**

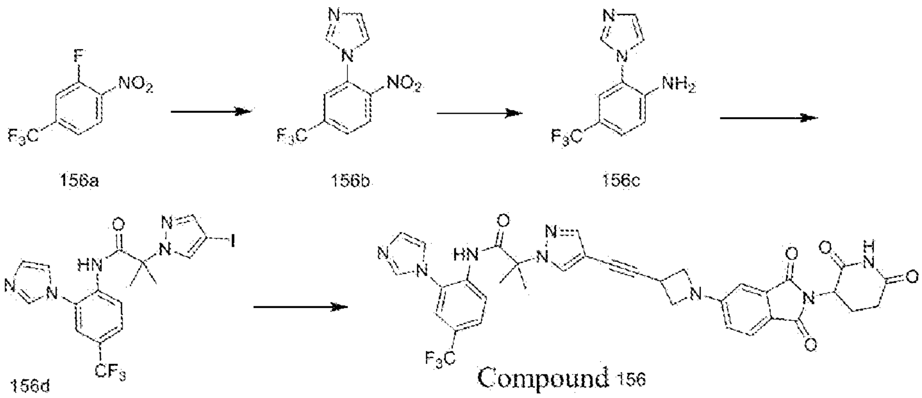

156a 156b 156c 156d Compound 156

Step 1: 1-(2-nitro-5-(trifluoromethyl)phenyl)-1H-imidazole (156b)

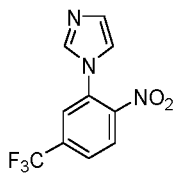

156a (1.0 g, 4.78 mmol), imidazole (0.49 g, 7.20 mmol), and potassium carbonate (1.33 g, 9.62 mmol) were added to 30 mL of DMF and the mixture was placed in a sealed tube and reacted at 80°C for 16 h. The reaction liquid was cooled to room temperature, 100 mL of water was added, the mixture was extracted with 100 mL of ethyl acetate. The organic phase was washed with 50 mL of water, dried over anhydrous sodium sulfate and concentrated under reduced pressure. The crude product was separated and purified by silica gel chromatography column (petroleum ether/ethyl acetate (v/v) = 10 : 1) to afford 156b (0.75 g, yield: 61%).

LCMS m/z = 258.1 [M+1]$^+$.

Step 2: 2-(1H-imidazol-1-yl)-4-(trifluoromethyl)aniline (156c)

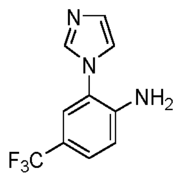

156b (0.5 g, 1.94 mmol) was dissolved in 10 mL of methanol, 0.2 g of 10% palladium on carbon was added, hydrogen replacement was performed three times, and the mixture was reacted at room temperature under the atmosphere of hydrogen balloon for 3.5 h. The reaction liquid was filtered, and the filtrate was concentrated under reduced pressure. The crude product was separated and purified with silica gel chromatography column (petroleum ether/ethyl acetate (v/v) = 10 : 1) to afford 156c (0.45 g, yield: > 99%).

LCMS m/z = 228.1 [M+1] $^+$.

Step 3: N-(2-(1H-imidazol-1-yl)-4-(trifluoromethyl)phenyl)-2-(4-iodo-1H-pyrazol-1-yl)-2-methylpropanamide (156d)

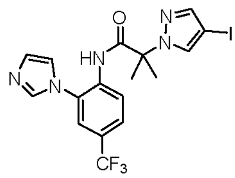

16a-1 (0.67 g, 2.39 mmol) was dissolved in 10 mL of DCM, 1-chloro-N,N,2-trimethylpropenylamine (0.42 g, 3.15 mmol) was added, the mixture was reacted at room temperature for 2 h, and then TEA (1.00 g, 9.88 mmol) and 156c (0.45 g, 1.98 mmol) were added in sequence, and the mixture was reacted at room temperature for 2 h. 80 mL of ethyl acetate and 50 mL of saturated aqueous sodium bicarbonate solution were added to the reaction liquid, the organic phase was separated, dried over anhydrous sodium sulfate, and concentrated under reduced pressure. The crude product was separated and purified with silica gel chromatography column (petroleum ether/ethyl acetate (v/v) = 2 : 1) to afford 156d (0.55 g, yield: 57%).

LCMS m/z = 490.0 [M+1] $^+$.

Step 4: N-(2-(1H-imidazol-1-yl)-4-(trifluoromethyl)phenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 156) trifluoroacetate

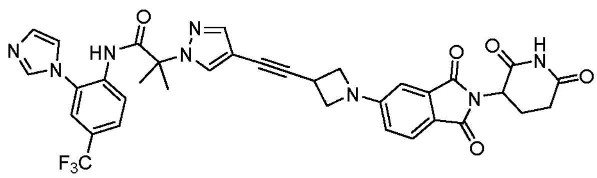

156d (0.23 g, 0.47 mmol), intermediate 1 (0.19 g), TEA (0.14 g, 1.38 mmol), CuI (9 mg, 0.047 mmol) and $PdCl_2(PPh_3)_2$ (33 mg, 0.047 mmol) were subjected to nitrogen replacement three times, and added to 5 mL of DMF in sequence under nitrogen protection, and the mixture was reacted at 50°C for 1 h. The reaction liquid was cooled to room temperature, 50 mL of water was added, and the mixture was filtered by suction. The filter cake was washed with 10 mL of water. The filter cake was dissolved in 100 mL of DCM, the mixture was dried over anhydrous sodium sulfate, and concentrated under reduced pressure. The crude product was separated and purified with silica gel column chromatography (petroleum ether/ethyl acetate (v/v) = 1 : 1) to afford a crude product. The crude product was subjected to Prep-HPLC (instrument and preparative column: using Glison GX-281 preparative liquid phase chromatographic instrument, preparative column model: Sunfire C18, 5 μm, inner diameter × length = 30 mm × 150 mm). Preparation method: the crude product was dissolved with methanol and dimethyl sulfoxide, and filtered with a 0.45 μm filter membrane, to prepare into a sample liquid. Mobile phase system: acetonitrile/water (containing 0.1% TFA). Gradient elution method: gradient elution of 5% to 60% acetonitrile (elution time 15 min), and lyophilization was performed to afford the trifluoroacetate of compound 156 (65 mg).

$^1H$ NMR (400 MHz, DMSO-$d_6$) δ 11.06 (s, 1H), 9.55 - 9.42 (m, 1H), 9.24 - 9.05 (m, 1H), 8.17 (s, 1H), 8.13 - 8.06 (m, 1H), 8.03 - 7.93 (m, 1H), 7.88 - 7.80 (m, 1H), 7.79 - 7.60 (m, 4H), 6.92 - 6.82 (m, 1H), 6.72 (dd, 1H), 5.11 - 5.02 (m, 1H), 4.47 - 4.32 (m, 2H), 4.07 - 3.80 (m, 3H), 2.98 - 2.78 (m, 1H), 2.70 - 2.50 (m, 2H), 2.08 - 1.92 (m, 1H), 1.69 (s, 6H).

LCMS m/z = 699.2 [M+1] $^+$.

**Example 157: Preparation of compound 157**

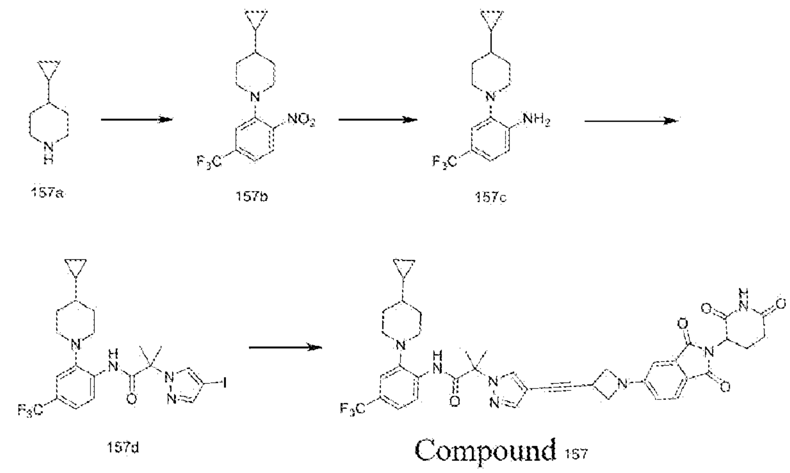

157a 157b 157c

157d Compound 157

Step 1: 4-cyclopropyl-1-(2-nitro-5-(trifluoromethyl)phenyl)piperidine (157b)

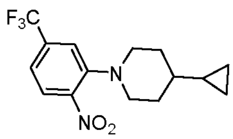

2-fluoro-1-nitro-4-(trifluoromethyl)benzene (0.37 g, 1.77 mmol) and the hydrochloride of 157a (0.31 g, 1.92 mmol) (see Synthesis, 2015, 47, 3963-3971 for the synthesis method) and potassium carbonate (0.62 g, 4.49 mmol) were added to 20 mL of DMF and the mixture was reacted at 80°C for 6 h. The reaction liquid was cooled to room temperature, 50 mL of water was added, the mixture was extracted with 100 mL of ethyl acetate. The organic phase was washed with 50 mL of water, dried over anhydrous sodium sulfate and concentrated under reduced pressure. The crude product was separated and purified by silica gel chromatography column (petroleum ether/ethyl acetate (v/v) = 10 : 1) to afford 157b (0.48 g, yield: 86%).

Step 2: 2-(4-cyclopropylpiperidin-1-yl)-4-(trifluoromethyl)aniline (157c)

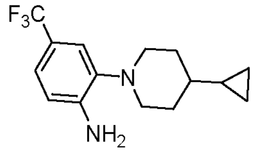

157b (0.25 g, 0.80 mmol) was dissolved in 5 mL of ethanol, 2 mL of saturated aqueous ammonium chloride solution was added, reduced iron powder (0.22 g, 3.93 mmol) was added, and the mixture was reacted at 80°C for 1.5 h. The reaction liquid was cooled to room temperature and filtered, and the filtrate was concentrated under reduced pressure. The crude product was separated and purified with silica gel chromatography column (petroleum ether/ethyl acetate (v/v) = 10 : 1) to afford 157c (0.21 g, yield: 92%).

LCMS m/z = 285.2 $[M+1]^+$.

Step 3: N-(2-(4-cyclopropylpiperidin-1-yl)-4-(trifluoromethyl)phenyl)-2-(4-iodo-1H-pyrazol-1-yl)-2-methylpropanamide (157d)

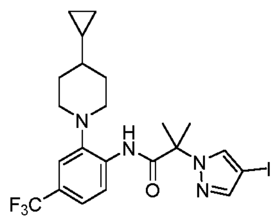

16a-1 (0.24 g, 0.86 mmol) was dissolved in 10 mL of DCM, 1-chloro-N,N,2-trimethylpropenylamine (0.15 g, 1.12 mmol) was added, the mixture was reacted at room temperature for 2 h, and then TEA (0.21 g, 2.10 mmol) and 157c (0.20 g, 0.70 mmol) were added in sequence, and the mixture was reacted at room temperature for 2 h. 80 mL of ethyl acetate and 50 mL of saturated aqueous sodium bicarbonate solution were added to the reaction liquid, the organic phase was separated, dried over anhydrous sodium sulfate, and concentrated under reduced pressure. The crude product was separated and purified with silica gel chromatography column (petroleum ether/ethyl acetate (v/v) = 5 : 1) to afford 157d (0.15 g, yield: 39%).

LCMS m/z = 547.1 $[M+1]^+$.

Step 4: N-(2-(4-cyclopropylpiperidin-1-yl)-4-(trifluoromethyl)phenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 157)

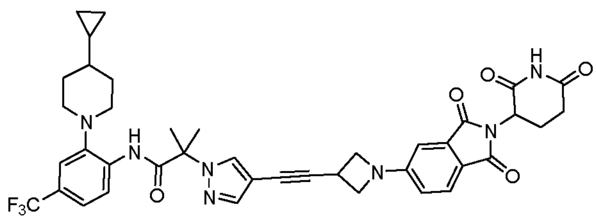

157d (0.15 g, 0.27 mmol), intermediate 1 (0.11 g), TEA (0.082 g, 0.81 mmol), CuI (5 mg, 0.026 mmol) and $PdCl_2(PPh_3)_2$ (19 mg, 0.027 mmol) were added to 5 mL of DMF, nitrogen replacement was performed three times, and the mixture was reacted at 50°C for 1 h. The reaction liquid was cooled to room temperature, 50 mL of water was added, and the mixture was filtered by suction. The filter cake was washed with 10 mL of water. The filter cake was dissolved in 100 mL of DCM, the mixture was dried over anhydrous sodium sulfate, and concentrated under reduced pressure. The crude product was separated and purified with silica gel column chromatography (petroleum ether/ethyl acetate

(v/v) = 1 : 1) to afford compound 157 (110 mg, yield: 54%).

$^1$H NMR (400 MHz, $CDCl_3$) δ 9.32 (s, 1H), 8.44 (d, 1H), 7.98 (s, 1H), 7.80 (s, 1H), 7.75 (s, 1H), 7.67 (d, 1H), 7.42 - 7.28 (m, 2H), 6.84 - 6.76 (m, 1H), 6.54 (dd, 1H), 4.99 - 4.88 (m, 1H), 4.40 - 4.27 (m, 2H), 4.10 - 3.97 (m, 2H), 3.87 - 3.71 (m, 1H), 2.98 - 2.46 (m, 7H), 2.18 - 2.07 (m, 1H), 1.94 (s, 6H), 1.90 - 1.77 (m, 2H), 1.66 - 1.48 (m, 2H), 0.80 - 0.55 (m, 2H), 0.50 - 0.36 (m, 2H), 0.20 - 0.07 (m, 2H).

LCMS m/z = 756.3 [M+1] $^+$.

**Example 158: Preparation of compound 158**

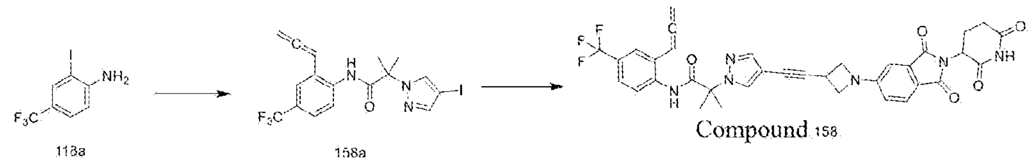

Step 1: 2-(4-iodo-1H-pyrazol-1-yl)-2-methyl-N-(2-(propa-1,2-dien-1-yl)-4-(trifluoromethyl)phenyl)propanamide (158a)

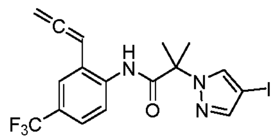

Propargyltrimethylsilane (0.94 g, 8.37 mmol) and 118a (1.20 g, 4.18 mmol) were dissolved in 10 mL of DMF, and TEA (0.85 g, 8.40 mmol), $PdCl_2(PPh_3)_2$ (0.29 g, 0.41 mmol) and CuI (0.16 g, 0.84 mmol) were added in sequence under nitrogen protection, and the mixture was placed in a sealed tube and reacted at 70°C for 2 h. The reaction liquid was cooled to room temperature, 10 mL of ethyl acetate and 10 mL of water were added, the organic phase was separated, the aqueous phase was extracted with ethyl acetate (10 mL × 2), the organic phases were combined, the organic phase was washed with 10 mL of saturated aqueous sodium chloride solution, dried over anhydrous sodium sulfate, and concentrated under reduced pressure. The crude product was separated and purified with silica gel chromatography column (petroleum ether : ethyl acetate (v/v) = 10 : 1) to afford a crude product (1.00 g). The above crude product (1.00 g) was dissolved in THF (10 mL), a solution of 1 mol/L TBAF in THF (5.90 mL, 5.90 mmol) was added, and the mixture was reacted at room temperature for 2 h. 10 mL of ethyl acetate and 10 mL of water were added to the reaction liquid, and the organic phase was separated. The organic phase was washed with 10 mL of saturated aqueous sodium chloride solution, dried over anhydrous sodium sulfate, and concentrated under reduced pressure to afford crude 2 (0.70 g). 16a-1 (0.90 g, 3.21 mmol) was dissolved in DCM (10 mL), 1-chloro-N,N,2-trimethylpropenylamine (0.64 g, 4.79 mmol) was added, the mixture was reacted at room temperature for 2 h, and then the above crude 2 (0.70 g) and TEA (0.97 g, 9.59 mmol) were added in sequence, and the mixture was reacted at room temperature for 12 h. 10 mL of ethyl acetate and 10 mL of water were added to the reaction liquid, the organic phase was separated, the organic phase was washed with 10 mL of saturated aqueous sodium chloride solution, dried over anhydrous sodium sulfate, and concentrated under reduced pressure. The crude product was separated and purified with silica gel chromatography column (petroleum ether : ethyl acetate (v/v) = 5 : 1) to afford 158a (0.33 g, yield: 17%).

LCMS m/z = 462.0 [M+1] $^+$.

Step 2: 2-(4-((1-(2-(2,6-dioxopiperidin-3-yl)-1,3-dioxoisoindolin-5-yl)azetidin-3-yl)ethynyl)-1H-pyrazol-1-yl)-2-methyl-N-(2-(propa-1,2-dien-1-yl)-4-(trifluoromethyl) phenyl)propanamide (Compound 158)

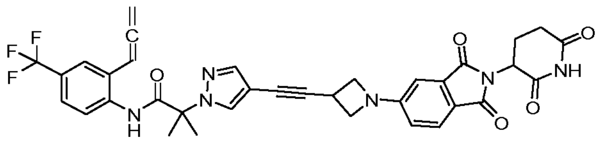

158a (0.33 g, 0.72 mmol) was dissolved in 10 mL of DMF, and intermediate 1 (0.29 g), TEA (0.22 g, 2.17 mmol), CuI (0.027 g, 0.14 mmol) and $PdCl_2(PPh_3)_2$ (0.051 g, 0.073 mmol) were added in sequence, and the mixture was reacted at 60°C for 2 h. The reaction liquid was cooled to room temperature, DCM (30 mL) and water (30 mL) were added, the organic phase was separated, the aqueous phase was extracted with DCM (30 mL × 3), the organic phases were combined, the organic phase was washed with 30 mL of saturated aqueous sodium chloride solution, dried over anhydrous sodium sulfate, and concentrated under reduced pressure. The residue was separated and purified with silica gel chromatography column (petroleum ether : ethyl acetate (v/v) = 1 : 1) to afford a crude product, which was subjected to Prep-HPLC (instrument and preparative column: using SHIMADZU LC-20AP preparative liquid phase chromatographic instrument, preparative column model: Phenomenex C18). Preparation method: the crude product was dissolved with methanol and dimethyl sulfoxide, and filtered with a 0.45 μm filter membrane, to prepare into a sample liquid. Mobile phase system: acetonitrile/water (containing 10 mmol/L ammonium bicarbonate). Gradient elution method: gradient elution of 40% to 70% acetonitrile (elution time: 15 min), lyophilization was performed to afford compound 158 (50 mg, yield: 10%).

$^1H$ NMR (400 MHz, $CDCl_3$) δ 8.87 (s, 1H), 8.16 (s, 1H), 8.06 (d, 1H), 7.84 - 7.76 (m, 2H), 7.66 (d, 1H), 7.58 - 7.52 (m, 1H), 7.48 - 7.38 (m, 1H), 6.84 - 6.76 (m, 1H), 6.55 (dd, 1H), 6.04 (t, 1H), 5.20 (d, 2H), 4.99 - 4.88 (m, 1H), 4.43 - 4.30 (m, 2H), 4.14 - 4.00 (m, 2H), 3.88 - 3.72 (m, 1H), 2.98 - 2.60 (m, 3H), 2.20 - 2.05 (m, 1H), 1.93 (s, 6H).

LCMS m/z = 671.3 [M+1] +.

**Example 159: Preparation of compound 159**

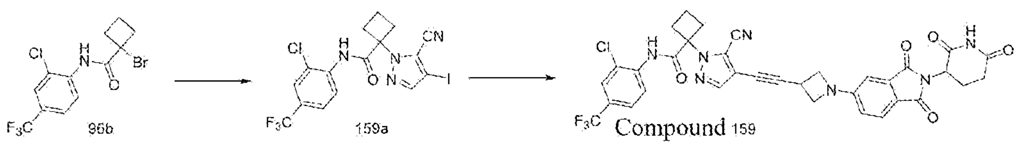

96b

159a

Compound 159

Step 1: N-(2-chloro-4-(trifluoromethyl)phenyl)-1-(5-cyano-4-iodo-1H-pyrazol-1-yl)cyclobutane-1-carboxamide (159a)

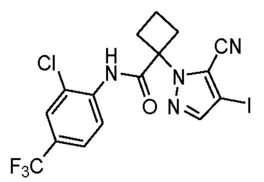

96b (0.5 g, 1.4 mmol) was dissolved in 20 ml of acetonitrile, 4-iodo-1H-pyrazole-5-carbonitrile (366 mg, 1.67 mmol) and cesium carbonate (910 mg, 2.79 mmol) were added, and the mixture was reacted at 90°C for 4 h. The reaction liquid was cooled to room temperature, 60 mL of ethyl acetate and 100 mL of water were added, the organic phase was separated, the organic phase was washed with 50 mL of saturated aqueous sodium chloride solution, dried over anhydrous sodium sulfate, and concentrated under reduced pressure. The crude product was separated and purified with silica gel chromatography column (petroleum ether : ethyl acetate (v/v) = 4 : 1) to afford 159a (120 mg, yield: 17%).

Rf value of compound 159a: 0.7 (developing agent: petroleum ether/ethyl acetate (v/v) = 2 : 1).

LCMS m/z = 495.1 [M+1] +.

Step 2: N-(2-chloro-4-(trifluoromethyl)phenyl)-1-(5-cyano-4-((1-(2-(2,6-dioxopiperidin-3-yl)-1,3-dioxoisoindolin-5-yl)azetidin-3-yl)ethynyl)-1H-pyrazol-1-yl)cyclobutane-1-carboxamide (Compound 159)

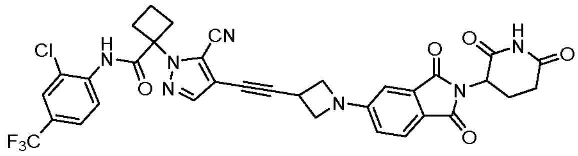

159a (120 mg, 0.24 mmol) was added into a 50 mL single-mouth bottle, 10 mL of dry DMF, intermediate 1 (121 mg) and TEA (73 mg, 0.72 mmol) were added in sequence, nitrogen replacement was performed three times, and $PdCl_2(PPh_3)_2$ (17 mg, 0.024 mmol) and CuI (9 mg, 0.047 mmol) were added, nitrogen replacement was performed three times, and the mixture was reacted at 50°C for 2 h. The reaction liquid was cooled to room temperature, 100 mL of saturated aqueous ammonium chloride solution was slowly added, the mixture was extracted with ethyl acetate (60 mL × 3), the organic phases were combined, and the organic phase was washed with saturated aqueous sodium chloride solution (50 mL × 2), dried over anhydrous sodium sulfate, and concentrated under reduced pressure. The crude product was separated and purified with silica gel chromatography column (petroleum ether : ethyl acetate (v/v) = 13 : 87) to afford a crude product (90 mg). The crude product was prepared by Prep-HPLC (instrument: Waters 2767 preparative liquid phase chromatographic instrument; chromatographic column: SunFire@PrepC18 (19 × 250 nm); mobile phase A: acetonitrile; mobile phase B: water (containing 5 mmol/L ammonium acetate); gradient elution method: gradient elution of 50% to 80% mobile phase A; flow rate: 12 mL/min; column temperature: room temperature; detection wavelength: 210 nm; the sample was dissolved in DMF and the mixture was filtered with a 0.45 μm filter membrane to prepare into a sample liquid; elution time: 18 min), lyophilization was performed to afford compound 159 (25 mg, yield: 15%).

$^1H$ NMR (400 MHz, $CDCl_3$) δ 8.15 - 7.73 (m, 4H), 7.72 - 7.61 (m, 1H), 7.59 - 7.45 (m, 1H), 6.95 (s, 1H), 6.79 (s, 1H), 6.61 - 6.50 (m, 1H), 5.00 - 4.85 (m, 1H), 4.18 - 3.80 (m, 4H), 3.78 - 3.60 (m, 1H), 3.13 - 2.60 (m, 7H), 2.50 - 2.05 (m, 3H).

LCMS m/z = 704.1 $[M+1]^+$.

**Example 160: Preparation of compound 160**

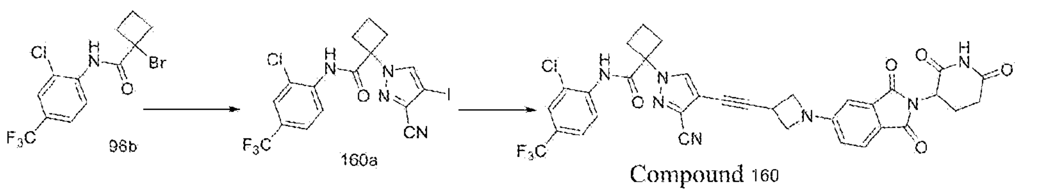

96b

160a

Compound 160

Step 1: N-(2-chloro-4-(trifluoromethyl)phenyl)-1-(3-cyano-4-iodo-1H-pyrazol-1-yl)cyclobutane-1-carboxamide (160a)

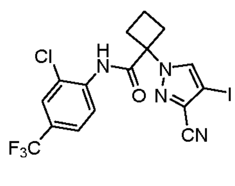

96b (0.5 g, 1.4 mmol) was dissolved in 20 ml of acetonitrile, 4-iodo-1H-pyrazole-5-carbonitrile (366 mg, 1.67 mmol) and cesium carbonate (910 mg, 2.79 mmol) were added, and the mixture was reacted at 90°C for 4 h. The reaction liquid was cooled to room temperature, 60 mL of ethyl acetate and 100 mL of water were added, the organic phase was separated, the organic phase was washed with 50 mL of saturated aqueous sodium chloride solution, dried over anhydrous sodium sulfate, concentrated under reduced pressure, and then separated and purified with silica gel chromatography column (petroleum ether : ethyl acetate (v/v) = 4 : 1) to afford 160a (130 mg, yield: 19%).

Rf value of compound 160a: 0.4 (developing agent: petroleum ether/ethyl acetate (v/v) = 2 : 1).

LCMS m/z = 495.1 $[M+1]^+$.

Step 2: N-(2-chloro-4-(trifluoromethyl)phenyl)-1-(3-cyano-4-((1 -(2-(2,6-dioxopiperidin-3-yl)-1,3-dioxoisoindolin-5-yl)azetidin-3-yl)ethynyl)-1H-pyrazol-1-yl)cyclobutane-1-carboxamide (Compound 160)

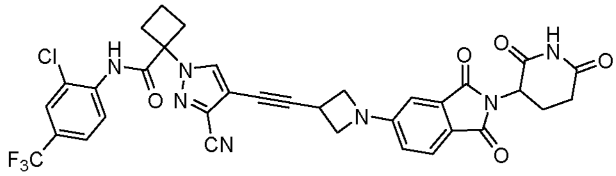

160a (130 mg, 0.263 mmol) was added into a 50 mL single-mouth bottle, 10 mL of dry DMF, intermediate 1 (131 mg) and TEA (73 mg, 0.72 mmol) were added in sequence, nitrogen replacement was performed three times, and $PdCl_2(PPh_3)_2$ (18 mg, 0.026 mmol) and CuI (10 mg, 0.053 mmol) were added, nitrogen replacement was performed three times, and the mixture was reacted at 50°C for 2 h. The reaction liquid was cooled to room temperature, 100 mL of saturated aqueous ammonium chloride solution was slowly added, the mixture was extracted with ethyl acetate (50 mL × 3), the organic phases were combined, and the organic phase was washed with saturated aqueous sodium chloride solution (30 mL × 2), dried over anhydrous sodium sulfate, and concentrated under reduced pressure. The crude product was separated and purified with silica gel chromatography column (petroleum ether : ethyl acetate (v/v) = 12 : 88) to afford compound 160 (75 mg, yield: 41%).

$^1$H NMR (400 MHz, $CDCl_3$) δ 8.57 - 8.45 (m, 2H), 7.96 (s, 1H), 7.78 - 7.58 (m, 3H), 7.56 - 7.49 (m, 1H), 6.86 - 6.78 (m, 1H), 6.56 (dd, 1H), 4.99 - 4.88 (m, 1H), 4.45 - 4.30 (m, 2H), 4.15 - 4.02 (m, 2H), 3.94 - 3.78 (m, 1H), 3.19 - 3.05 (m, 2H), 2.95 - 2.63 (m, 5H), 2.34 - 2.00 (m, 3H).

LCMS m/z = 704.1 $[M+1]^+$.

**Example 161: Preparation of compound 161 and compound 162**

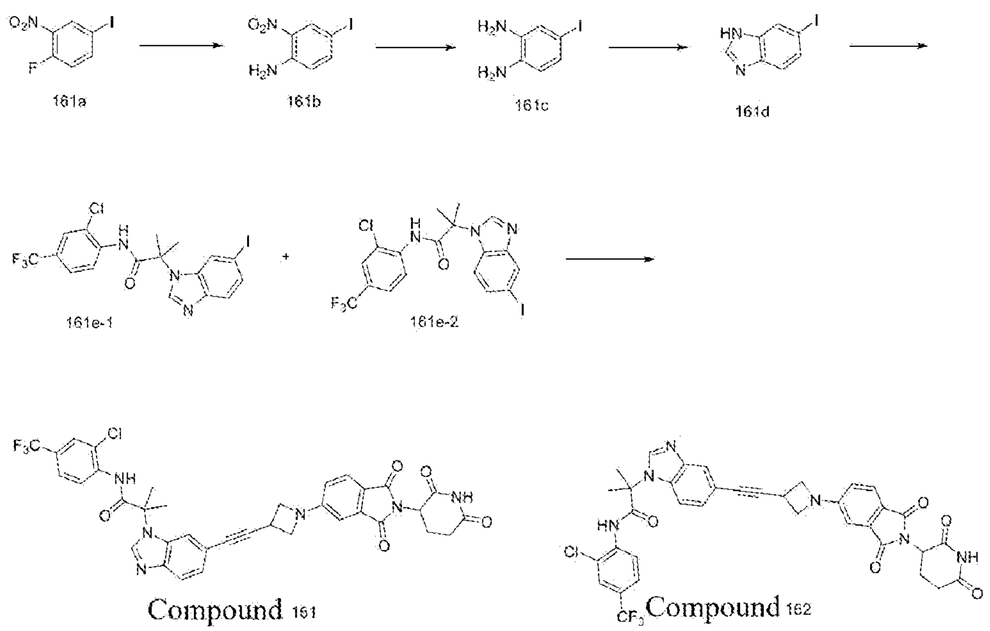

161a 161b 161c 161d

161e-1 161e-2

Compound 161 Compound 162

Step 1: 4-iodo-2-nitroaniline (161b)

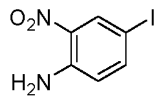

161a (2.0 g, 7.49 mmol) was dissolved in 30 mL of THF, the mixture was cooled to 0°C under ice bath, 30 mL of ammonia (25%wt) was slowly added dropwise, and the mixture was reacted at room temperature for 16 h. 60 mL of ethyl acetate and 50 mL of water were added to the reaction liquid, the organic phase was separated, the organic phase was washed with 50 mL of saturated aqueous sodium chloride solution, dried over anhydrous sodium sulfate, and concentrated under reduced pressure. The crude product was separated and purified with silica gel chromatography column (petroleum ether : ethyl acetate (v/v) = 9 : 1) to afford 161b (1.4 g, yield: 71%).

LCMS m/z = 265.1 [M+1] $^+$.

Step 2: 4-iodobenzene-1,2-diamine (161c)

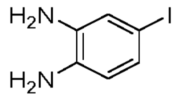

161b (1.4 g, 5.3 mmol) was dissolved in 80 mL of methanol and 20 mL of water, reduced iron powder (1.5 g, 26.8 mmol) and ammonium chloride (1.5 g, 28.0 mmol) were added, and the mixture was reacted at 70°C for 5 h. The reaction liquid was cooled to room temperature and filtered, the filtrate was concentrated under reduced pressure, 100 mL of ethyl acetate was added, the liquid separation was conducted, and the organic phase was washed with water (50 mL × 2) and 50 mL of saturated aqueous sodium chloride solution, dried over anhydrous sodium sulfate, and concentrated under reduced pressure. The crude product was separated and purified with silica gel chromatography column (petroleum ether : ethyl acetate (v/v) = 1 : 1) to afford 161c (1 g, yield: 81%).

LCMS m/z = 235.1 [M+1] $^+$.

Step 3: 6-iodo-1H-benzo[d]imidazole (161d)

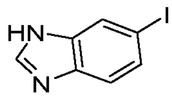

161c (0.5 g, 2.14 mmol) was dissolved in 20 mL of formic acid and the mixture was reacted at 120°C for 4 h. The reaction liquid was cooled to room temperature and concentrated under reduced pressure, 100 mL of ethyl acetate was added, the liquid separation was conducted, the organic phase was washed with saturated aqueous sodium bicarbonate solution (60 mL × 3), and washed with saturated aqueous sodium chloride solution (50 mL × 2), dried over anhydrous sodium sulfate, concentrated under reduced pressure, and the crude product was separated and purified with silica gel chromatography column (petroleum ether : ethyl acetate (v/v) = 1 : 2) to afford 161d (0.49 g, yield :94%).

LCMS m/z = 245.1 [M+1] $^+$.

Step 4: N-(2-chloro-4-(trifluoromethyl)phenyl)-2-(6-iodo-1H-benzo[d]imidazol-1-yl)-2-methylpropanamide (161e-1)

N-(2-chloro-4-(trifluoromethyl)phenyl)-2-(5-iodo-1H-benzo[d]imidazol-1-yl)-2-methylpropanamide (161e-2)

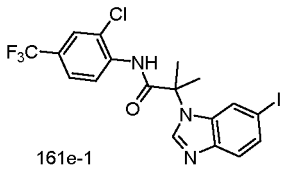

161e-1

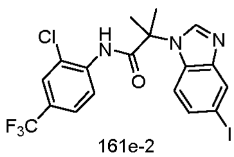

161e-2

161d (490 mg, 2.01 mmol) was added into a 100 mL single-mouth bottle, 20 mL of acetonitrile, cesium carbonate (1.3 g, 4.0 mmol) and 11b (686 mg, 2 mmol) were added in sequence, and the mixture was reacted at 90°C for 16 h. The reaction liquid was cooled to room temperature, 60 mL of ethyl acetate was added, the liquid separation was conducted, the organic phase was washed with water (40 mL × 2), washed with saturated aqueous sodium chloride solution (30 mL × 2), dried over anhydrous sodium sulfate, and concentrated under reduced pressure, and the crude product was separated and purified with silica gel chromatography column (petroleum ether : ethyl acetate (v/v) = 32 : 68) to afford a mixture of 161e-1 and 161e-2 (0.3 g).

Step 5: N-(2-chloro-4-(trifluoromethyl)phenyl)-2-(6-((1-(2-(2,6-dioxopiperidin-3-yl)-1,3-dioxoisoindolin-5-yl)azetidin-3-yl)ethynyl)-1H-benzo[d]imidazol-1-yl)-2-methylpropanamide (Compound 161)

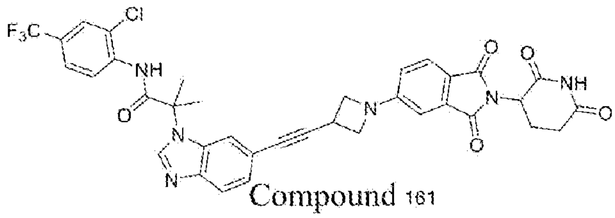

Compound 161

N-(2-chloro-4-(trifluoromethyl)phenyl)-2-(5-((1-(2-(2,6-dioxopiperidin-3-yl)-1,3-dioxoisoindolin-5-yl)azetidin-3-yl)ethynyl)-1H-benzo[d]imidazol-1-yl)-2-methylpropanamide (Compound 162)

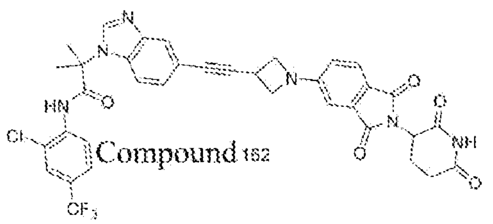

Compound 162

The mixture of 161e-1 and 161e-2 (100 mg) obtained in the previous step was added to 10 mL of dry DMF, intermediate 1 (101 mg) and TEA (33 mg, 0.33 mmol) were added, nitrogen replacement was performed three times, $PdCl_2(PPh_3)_2$ (14 mg, 0.02 mmol) and CuI (6 mg, 0.0315 mmol) were added, nitrogen replacement was performed three times, and the mixture was reacted at 50°C for 2 h. The reaction liquid was cooled to room temperature, 100 mL of saturated aqueous ammonium chloride solution was slowly added, the mixture was extracted with ethyl acetate (50 mL × 3), the organic phase was washed with saturated aqueous sodium chloride solution (30 mL × 2), dried over anhydrous sodium sulfate, and concentrated under reduced pressure. The crude product was separated and purified with silica gel chromatography column (petroleum ether : ethyl acetate (v/v) = 5 : 95) to afford a crude product (70 mg). The crude product was prepared by Prep-HPLC (instrument: Waters 2767 preparative liquid phase chromatographic instrument; chromatographic column: SunFire@PrepC18 (19 × 250 nm); mobile phase A: acetonitrile; mobile phase B: water (containing 5 mmol/L ammonium acetate); gradient elution method: gradient elution of 30% to 75% mobile phase A; flow rate: 12 mL/min; column temperature: room temperature; detection wavelength: 210 nm; the sample was dissolved in DMF and the mixture was filtered with a 0.45 μm filter membrane to prepare into a sample liquid; elution time: 18 min), compound 161 (22 mg) and compound 162 (20 mg) were obtained respectively.

Compound 161:

$^1H$ NMR (400 MHz, $CDCl_3$) δ 8.47 - 8.35 (m, 1H), 8.30 - 8.15 (m, 2H), 7.88 - 7.60 (m, 3H), 7.60 - 7.32 (m, 4H), 6.86 - 6.74 (m, 1H), 6.64 - 6.48 (m, 1H), 4.99 - 4.87 (m, 1H), 4.45 - 4.28 (m, 2H), 4.18 - 4.01 (m, 2H), 3.95 - 3.75 (m, 1H), 2.97 - 2.63 (m, 3H), 2.22 - 1.92 (m, 7H).
LCMS m/z =717.1 $[M+1]^+$.

Compound 162:

$^1H$ NMR (400 MHz, $CDCl_3$) δ 8.50 - 8.37 (m, 1H), 8.24 (s, 1H), 8.14 (s, 1H), 7.95 (s, 1H), 7.76 (s, 1H), 7.67 (d, 1H), 7.59 - 7.42 (m, 2H), 7.37 - 7.29 (m, 1H), 7.26 - 7.20 (m, 1H), 6.86 - 6.75 (m, 1H), 6.56 (dd, 1H), 5.00 - 4.88 (m, 1H), 4.46 - 4.30 (m, 2H), 4.20 - 4.05 (m, 2H), 3.95 - 3.78 (m, 1H), 2.98 - 2.60 (m, 3H), 2.22 - 1.95 (m, 7H).
LCMS m/z =717.2 $[M+1]^+$.

**Example 162: Preparation of compounds 163 and 164**

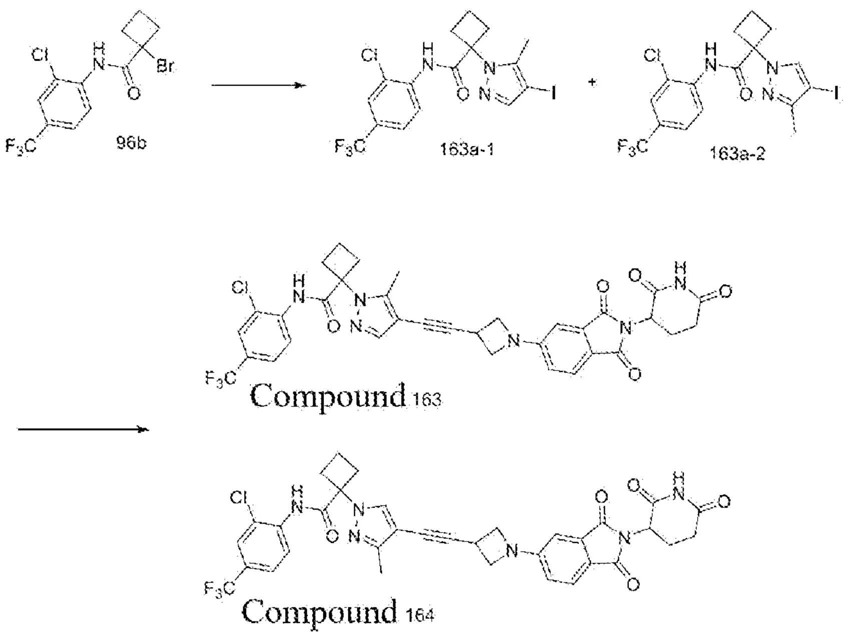

96b

163a-1

163a-2

Compound 163

Compound 164

Step 1: N-(2-chloro-4-(trifluoromethyl)phenyl)-1-(4-iodo-5-methyl-1H-pyrazol-1-yl)cyclobutane-1-carboxamide (163a-1)

N-(2-chloro-4-(trifluoromethyl)phenyl)-1-(4-iodo-3-methyl-1H-pyrazol-1-yl)cyclobutane-1-carboxamide (163a-2)

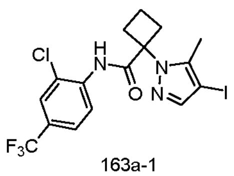

163a-1

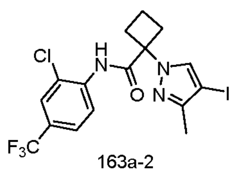

163a-2

96b (0.5 g, 1.4 mmol) was dissolved in 20 mL of acetonitrile, 4-iodo-3-methyl-1H-pyrazole (352 mg, 1.69 mmol) and cesium carbonate (910 mg, 2.79 mmol) were added, and the mixture was reacted at 90°C for 4 h. The reaction liquid was cooled to room temperature, 60 mL of ethyl acetate and 100 mL of water were added, the organic phase was separated, the organic phase was washed with 50 mL of saturated aqueous sodium chloride solution, dried over anhydrous sodium sulfate, and concentrated under reduced pressure. The crude product was separated and purified with

silica gel chromatography column (petroleum ether : ethyl acetate (v/v) = 83 : 17) to afford a mixture of 163a-1 and 163a-2 (210 mg).

Step 2; N-(2-chloro-4-(trifluoromethyl)phenyl)-1-(4-((1-(2-(2,6-dioxopiperidin-3-yl)-1,3-dioxoisoindolin-5-yl)azetidin-3-yl)ethynyl)-5-methyl-1H-pyrazol-1-yl)cyclobutane-1-carboxamide (Compound 163)

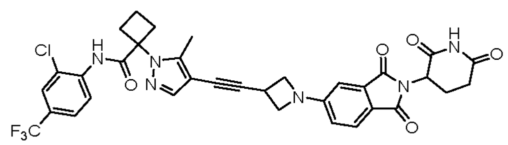

N-(2-chloro-4-(trifluoromethyl)phenyl)-1-(4-((1-(2-(2,6-dioxopiperidin-3-yl)-1 ,3-dioxoisoindolin-5-yl)azetidin-3-yl)ethynyl)-3 -methyl-1H-pyrazol-1-yl)cyclobutane-1-carboxamide (Compound 164)

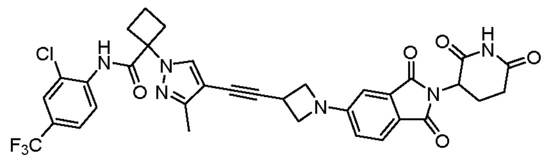

The mixture of 163a-1 and 163a-2 (210 mg) obtained in the previous step was added into a 50 mL single-mouth bottle, and 20 mL of dry DMF, intermediate 1 (217 mg), TEA (130 mg, 1.28 mmol), $PdCl_2(PPh_3)_2$ (30 mg, 0.043 mmol) and CuI (16 mg, 0.084 mmol) were added, nitrogen replacement was performed three times, and the mixture was reacted at 50°C for 2 h. The reaction liquid was cooled to room temperature, 200 mL of saturated aqueous ammonium chloride solution was slowly added, the mixture was extracted with ethyl acetate (80 mL × 3), the organic phase was washed with saturated aqueous sodium chloride solution (60 mL × 2), dried over anhydrous sodium sulfate, and concentrated under reduced pressure. The crude product was separated and purified with silica gel chromatography column (petroleum ether : ethyl acetate (v/v) = 5 : 95) to afford a crude product (120 mg). The crude product was prepared by Prep-HPLC (instrument: Waters 2767 preparative liquid phase chromatographic instrument; chromatographic column: SunFire@PrepC18 (19 × 250 nm); mobile phase A: acetonitrile; mobile phase B: water (containing 5 mmol/L ammonium acetate); Gradient elution method: gradient elution of 50% to 80% mobile phase A; flow rate: 12 mL/min; column temperature: room temperature; detection wavelength: 210 nm; the sample was dissolved in DMF and the mixture was filtered with a 0.45 μm filter membrane to prepare into a sample liquid; elution time: 18 min), compound 163 (40 mg) and compound 164 (25 mg) were obtained respectively.

nuclear magnetic resonance spectrum of compound 163:

$^1$H NMR (400 MHz, $CDCl_3$) δ 8.54 (d, 1H), 8.12 (s, 1H), 7.89 (s, 1H), 7.76 - 7.42 (m, 4H), 6.89 - 6.73 (m, 1H), 6.54 (dd, 1H), 5.00 - 4.85 (m, 1H), 4.45 - 4.25 (m, 2H), 4.15 - 3.96 (m, 2H), 3.92 - 3.72 (m, 1H), 3.14 - 2.55 (m, 7H), 2.40 - 1.94 (m, 6H).

nuclear magnetic resonance spectrum of compound 164:

$^1$H NMR (400 MHz, $CDCl_3$) δ 9.01 (s, 1H), 8.63 - 8.51 (m, 1H), 8.08 (br.s, 1H), 7.72 - 7.44 (m, 4H), 6.86 - 6.74 (m, 1H), 6.62 - 6.48 (m, 1H), 4.99 - 4.87 (m, 1H), 4.45 - 4.27 (m, 2H), 4.13 - 3.97 (m, 2H), 3.93 - 3.75 (m, 1H), 3.12 - 2.62 (m, 7H), 2.37 (s, 3H), 2.29 - 1.98 (m, 3H).

**Example 165: Preparation of compound 165**

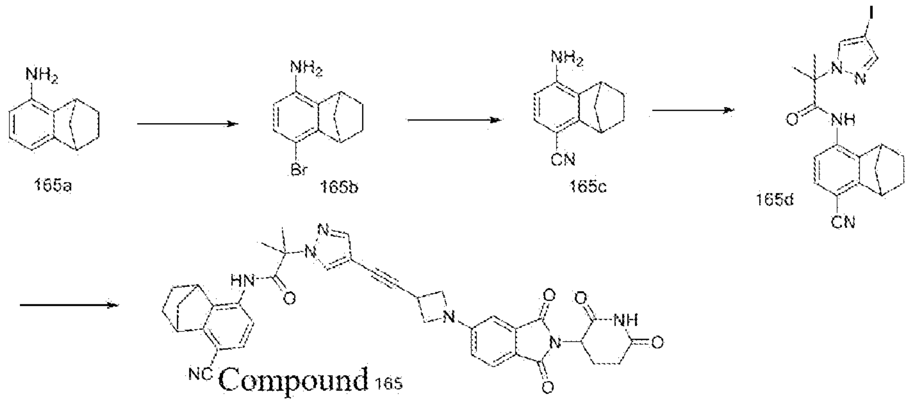

165a 165b 165c 165d

Compound 165

Step 1: 8-bromo-1,2,3,4-tetrahydro-1,4-methanonaphthalen-5-amine (165b)

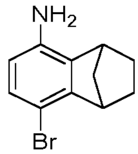

165a (580 mg, 3.64 mmol) (see WO 2021083182 for the synthesis method) was dissolved in 15 mL of DMF, the mixture was cooled to 0°C under ice bath, N-bromosuccinimide (650 mg, 3.65 mmol) was added, and the mixture was reacted at room temperature for 2 h. The reaction liquid was added to 150 mL of water, the mixture was extracted with ethyl acetate (50 mL × 3), the organic phase was washed with saturated aqueous sodium chloride solution (30 mL × 2), dried over anhydrous sodium sulfate, and concentrated under reduced pressure. The crude product was separated and purified with silica gel chromatography column (petroleum ether : ethyl acetate (v/v) = 78 : 22) to afford 165b (0.7 g, yield: 81%).

LCMS m/z = 238.1 $[M+1]^+$.

Step 2: 8-amino-1,2,3,4-tetrahydro-1,4-methanonaphthalene-5-carbonitrile (165c)

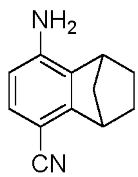

165b (500 mg, 2.11 mmol) was added into a 50 mL single-mouth bottle, 10 mL NMP and cuprous cyanide (562 mg, 6.3 mmol) were added, and the mixture was reacted at 170°C for 5 h. The reaction liquid was cooled to room temperature, 80 mL of ethyl acetate was added, the liquid separation was conducted, the organic phase was washed with saturated aqueous ammonium chloride solution (100 mL × 2), and washed with saturated aqueous sodium chloride solution (60 mL × 2), dried over anhydrous sodium sulfate, concentrated under reduced pressure, and the crude product was separated and purified with silica gel chromatography column (petroleum ether : ethyl acetate (v/v) = 2 : 1) to afford 165c (0.24 g, yield :62%).

LCMS m/z = 185.1 $[M+1]^+$.

Step 3: N-(8-cyano-1,2,3,4-tetrahydro-1,4-methanonaphthalen-5-yl)-2-(4-iodo-1H-pyrazol-1-yl)-2-methylpropanamide (165d)

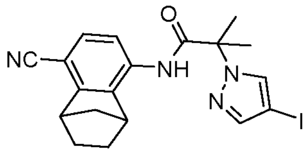

16a-1 (60 mg, 0.214 mmol) was added to a 50 mL single-mouth bottle, 10 mL of dry DCM was added, 1-chloro-N,N,2-trimethylpropenylamine (44 mg, 0.33 mmol) was added, and the mixture was reacted at room temperature for 1 h, then TEA (67 mg, 0.66 mmol) and 165c (40 mg, 0.217 mmol) were added in sequence, and the mixture was reacted at room temperature for 1 h. The reaction liquid was concentrated under reduced pressure, and the crude product was separated and purified with silica gel chromatography column (petroleum ether : ethyl acetate (v/v) = 87 : 13) to afford 165d (85 mg, yield: 89%).

LCMS m/z = 447.2 $[M+1]^+$.

Step 4: N-(8-cyano-1,2,3,4-tetrahydro-1,4-methanonaphthalen-5-yl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 165)

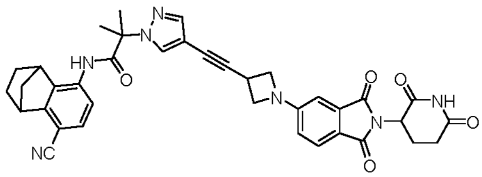

165d (85 mg, 0.19 mmol) was added into a 50 mL single-mouth bottle, 10 mL of dry DMF, intermediate 1 (96 mg) and TEA (57 mg, 0.56 mmol) were added in sequence, nitrogen replacement was performed three times, and $PdCl_2(PPh_3)_2$ (13 mg, 0.019 mmol) and CuI (7 mg, 0.037 mmol) were added, nitrogen replacement was performed three times, and the mixture was reacted at 50°C for 2 h. The reaction system was cooled to room temperature, 100 mL of saturated aqueous ammonium chloride solution was slowly added, the mixture was extracted with ethyl acetate (40 mL × 3), the liquid separation was conducted, and the organic phase was washed with saturated aqueous sodium chloride solution (30 mL × 2), dried over anhydrous sodium sulfate, and concentrated under reduced pressure. The crude product was separated and purified with silica gel chromatography column (petroleum ether : ethyl acetate (v/v) = 1 : 9) to afford compound 165 (70 mg, yield: 56%).

$^1H$ NMR (400 MHz, $CDCl_3$) δ 9.09 (s, 1H), 8.05 - 7.90 (m, 2H), 7.90 - 7.60 (m, 3H), 7.35 - 7.27 (m, 1H), 6.86 - 6.77 (m, 1H), 6.56 (dd, 1H), 5.00 - 4.89 (m, 1H), 4.45 - 4.30 (m, 2H), 4.15 - 4.00 (m, 2H), 3.89 - 3.75 (m, 1H), 3.72 - 3.62 (m, 1H), 3.36 - 3.28 (m, 1H), 2.98 - 2.64 (m, 3H), 2.22 - 1.86 (m, 9H), 1.82 - 1.71 (m, 1H), 1.64 - 1.54 (m, 1H), 1.24 - 1.01 (m, 2H).

LCMS m/z = 656.3 $[M+1]^+$.

**Example 166: Preparation of compound 166**

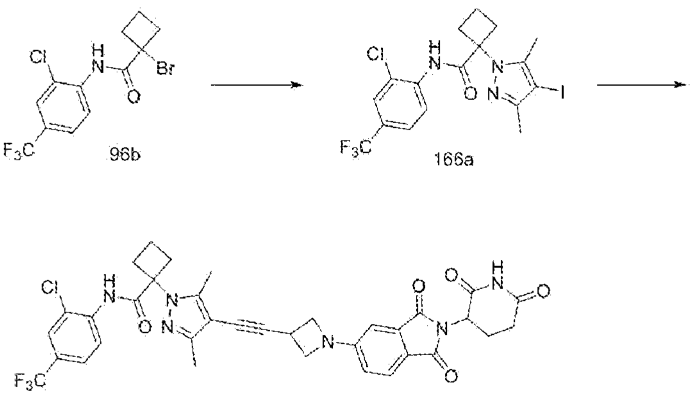

Compound 166

Step 1: N-(2-chloro-4-(trifluoromethyl)phenyl)-1-(4-iodo-3,5-dimethyl-1H-pyrazol-1-yl)cyclobutane-1-carboxamide (166a)

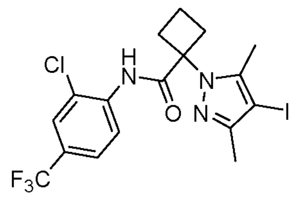

96b (0.5g, 1.40 mmol) was dissolved in 25 mL of acetonitrile, 4-iodo-3,5-dimethyl-1H-pyrazole (406 mg, 1.83 mmol) and cesium carbonate (916 mg, 2.81 mmol) were added, and the mixture was heated to 90°C and reacted for 4 h. The reaction system was cooled to room temperature, 80 mL of ethyl acetate was added, 150 mL purified water was added, the organic phase was separated, the organic phase was washed with 80 mL of saturated aqueous sodium chloride solution, dried over anhydrous sodium sulfate, and concentrated under reduced pressure. The crude product was separated and purified with silica gel chromatography column (petroleum ether : ethyl acetate (v/v) = 87 : 13) to afford 166a (220 mg, yield: 32%).
LCMS m/z = 498.1 [M+1] $^+$.

Step 2: N-(2-chloro-4-(trifluoromethyl)phenyl)-1-(4-((1-(2-(2,6-dioxopiperidin-3-yl)-1,3-dioxoisoindolin-5-yl)azetidin-3-yl)ethynyl)-3,5-dimethyl-1H-pyrazol-1-yl)cyclobutane-1-carboxamide (Compound 166)

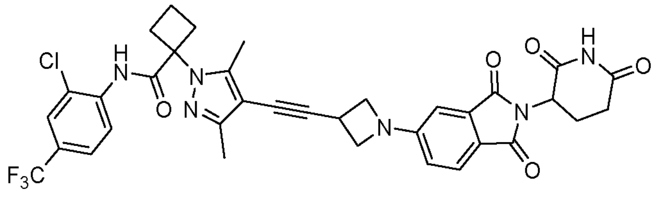

166a (220 mg, 0.44 mmol) was added into a 50 mL single-mouth bottle, 12 mL of dry DMF, crude intermediate 1 (223 mg) and TEA (133 mg, 1.31 mmol) were added, nitrogen replacement was performed three times, and $PdCl_2(PPh_3)_2$ (31 mg, 0.044 mmol) and CuI (17 mg, 0.089 mmol) were added, nitrogen replacement was performed three times, and the mixture was heated to 50°C and reacted for 2 h. The reaction system was cooled to room temperature, 150 mL of saturated aqueous ammonium chloride solution was slowly added, the mixture was extracted with ethyl acetate (60 mL × 3), the organic phases were combined, and the organic phase was washed with saturated aqueous sodium chloride solution (50 mL × 2), dried over anhydrous sodium sulfate, and concentrated under reduced pressure. The

crude product was separated and purified with silica gel chromatography column (petroleum ether : ethyl acetate (v/v) = 18 : 82) to afford a crude product. The crude product was prepared by Prep-HPLC (instrument: Abilene 1290 preparative liquid phase chromatographic instrument; chromatographic column: SunFire@PrepC18 (19 × 250 nm); mobile phase A: water (containing 10 mmol/L ammonium acetate); mobile phase B: acetonitrile; gradient elution: mobile phase A from 50%-98%; flow rate: 15 mL/min; column temperature: room temperature; detection wavelength: 210 nm; the sample was dissolved in DMF and filtered with a 0.45 um filter to prepare a sample solution; elution time: 18 min), compound 166 (80 mg, yield: 26%) was obtained.

$^1H$ NMR (400 MHz, $CDCl_3$) δ 8.55 (d, 1H), 8.12 (s, 1H), 7.94 (s, 1H), 7.67 (d, 1H), 7.59 - 7.45 (m, 2H), 6.84 - 6.76 (m, 1H), 6.55 (dd, 1H), 4.98 - 4.87 (m, 1H), 4.44 - 4.30 (m, 2H), 4.10 - 3.97 (m, 2H), 3.93 - 3.80 (m, 1H), 3.05 - 2.63 (m, 7H), 2.38 - 1.94 (m, 9H).

LCMS m/z = 707.2 $[M+1]^+$.

**Example 167: Preparation of compound 167**

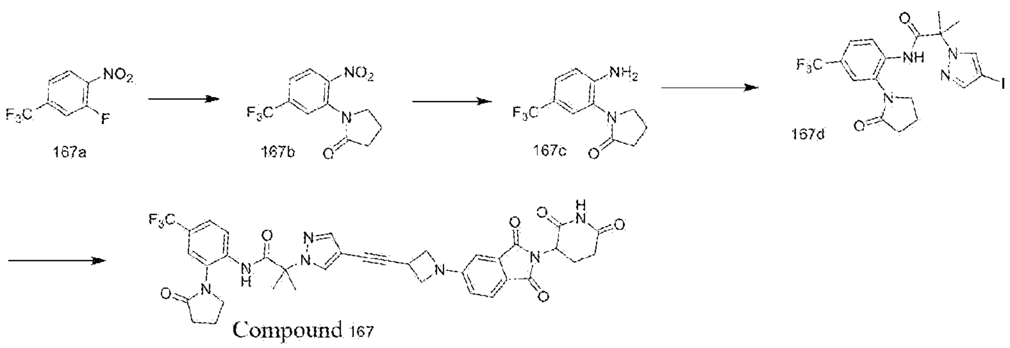

167a 167b 167c 167d

Compound 167

Step 1: 1-(2-nitro-5-(trifluoromethyl)phenyl)pyrrolidin-2-one (167b)

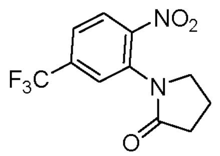

167a (0.42 g, 2.01 mmol), pyrrolidin-2-one (0.35 g, 4.11 mmol) and potassium carbonate (0.56 g, 4.05 mmol) were added to 30 mL of DMF and the mixture was reacted at 80°C for 4 h. The reaction liquid was cooled to room temperature, 100 mL of water was added, the mixture was extracted with 100 mL of ethyl acetate. The organic phase was washed with 50 mL of water, dried over anhydrous sodium sulfate and concentrated under reduced pressure. The crude product was separated and purified by silica gel chromatography column (petroleum ether/ethyl acetate (v/v) = 5 : 1) to afford 167b (0.34 g, yield: 62%).

Step 2: 1-(2-amino-5-(trifluoromethyl)phenyl)pyrrolidin-2-one (167c)

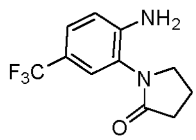

167b (0.3 g, 1.09 mmol) was dissolved in 10 mL of methanol, 0.2 g of 10% palladium on carbon was added, hydrogen replacement was performed three times, and the mixture was reacted at room temperature under the atmosphere of hydrogen balloon for 3.5 h. The reaction liquid was filtered, and the filtrate was concentrated. The crude product

was separated and purified with silica gel chromatography column (petroleum ether/ethyl acetate (v/v) = 10 : 1) to afford 167c (0.26 g, yield: 98%).

LCMS m/z = 245.2 [M+1] +.

Step 3: 2-(4-iodo-1H-pyrazol-1-yl)-2-methyl-N-(2-(2-oxopyrrolidin-1-yl)-4-(trifluoromethyl)phenyl)propanamide (167d)

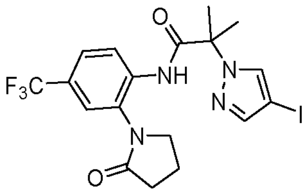

16a-1 (0.34 g, 1.21 mmol) was dissolved in 10 mL of DCM, 1-chloro-N,N,2-trimethylpropenylamine (0.21 g, 1.57 mmol) was added, the mixture was reacted at room temperature for 2 h, and then TEA (0.51 g, 5.0 mmol) and 167c (0.245 g, 1.00 mmol) were added in sequence, and the mixture was reacted at room temperature for 16 h. 150 mL of DCM was added to the reaction liquid, 50 mL of saturated aqueous sodium bicarbonate solution was added, the organic phase was separated, dried over anhydrous sodium sulfate, and concentrated under reduced pressure. The crude product was separated and purified with silica gel chromatography column (petroleum ether/ethyl acetate (v/v) = 5 : 1) to afford 167d (0.4 g, yield: 79%).

LCMS m/z = 507.0 [M+1] +.

Step 4: 2-(4-((1-(2-(2,6-dioxopiperidin-3-yl)-1,3-dioxoisoindolin-5-yl)azetidin-3-yl)ethynyl)-1H-pyrazol-1-yl)-2-methyl-N-(2-(2-oxopyrrolidin-1-yl)-4-(trifluoromethyl) phenyl)propenamide (Compound 167)

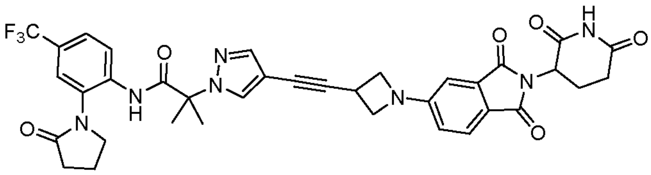

167d (0.2 g, 0.40 mmol), intermediate 1 (0.16 g), TEA (0.12 g, 1.19 mmol), CuI (8 mg, 0.042 mmol) and $PdCl_2(PPh_3)_2$ (29 mg, 0.041 mmol) were added to a reaction bottle, nitrogen replacement was performed three times, 5 mL of DMF was added under nitrogen protection, and the mixture was reacted at 50°C for 1 h. The reaction liquid was cooled to room temperature, 50 mL of water was added, and the mixture was filtered by suction. The filter cake was washed with 10 mL of water. The filter cake was dissolved in 100 mL of DCM, the mixture was dried over anhydrous sodium sulfate, and concentrated under reduced pressure. The crude product was purified with silica gel column chromatography (petroleum ether/ethyl acetate (v/v) = 1 : 1) to afford compound 167 (0.24 g, yield: 84%).

$^1H$ NMR (400 MHz, $CDCl_3$) δ 8.58 (s, 1H), 8.18 (d, 1H), 8.06 (s, 1H), 7.78 (s, 1H), 7.73 - 7.62 (m, 2H), 7.58 - 7.49 (m, 1H), 7.38 (s, 1H), 6.86 - 6.77 (m, 1H), 6.56 (dd, 1H), 4.99 - 4.89 (m, 1H), 4.43 - 4.29 (m, 2H), 4.12 - 4.00 (m, 2H), 3.88 - 3.70 (m, 3H), 2.99 - 2.52 (m, 5H), 2.32 - 2.08 (m, 3H), 1.90 (s, 6H).

LCMS m/z = 716.3 [M+1] +.

**Example 168: Preparation of compound 168**

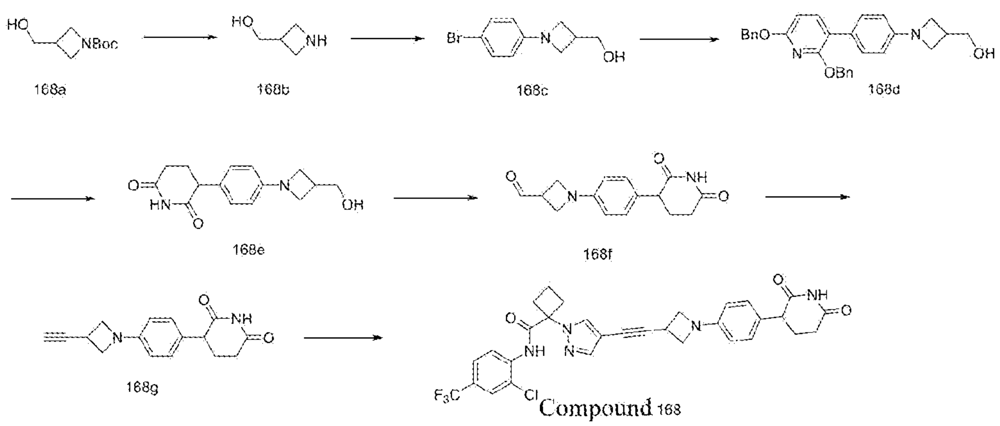


Step 1: Azacyclobutane-3-ylmethanol (168b) trifluoroacetate

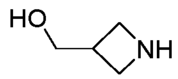

168a (4.0 g, 21.36 mmol) was dissolved in 40 mL of DCM, and 10 mL of trifluoroacetic acid was added. The mixture was reacted at room temperature for 3 h. The reaction liquid was concentrated under reduced pressure to afford the trifluoroacetate of crude 168b (5.0 g).
LCMS m/z = 88.1 $[M+1]^+$.

Step 2: (1-(4-bromophenyl)azetidin-3-yl)methanol (168c)

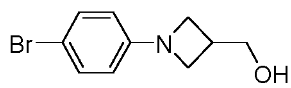

The trifluoroacetate of crude 168b (5 g) was dissolved in 50 mL of DMSO, potassium carbonate (14.7 g, 106.4 mmol) was added, the mixture was stirred at room temperature for 30 min, then 1-bromo-4-iodobenzene (6 g, 21.24 mmol), CuI (810 mg, 4.25 mmol) and L-proline (984 mg, 8.55 mmol) were added in sequence, nitrogen replacement was performed three times, and the mixture was reacted at 90°C for 16 h. The reaction liquid was cooled to room temperature and filtered, the filtrate was added dropwise to 500 mL of water, the mixture was extracted with ethyl acetate (160 mL × 3), the organic phases were combined, and the organic phase was washed with saturated aqueous sodium chloride solution (200 mL × 2), dried over anhydrous sodium sulfate and concentrated under reduced pressure. The crude product was separated and purified with silica gel chromatography column (petroleum ether : ethyl acetate (v/v) = 56 : 44) to afford 168c (3.5 g, two-step yield from compound 168a: 68%).
LCMS m/z = 242.1 $[M+1]^+$.

Step 3: (1-(4-(2,6-bis(benzyloxy)pyridin-3-yl)phenyl)azetidin-3-yl)methanol (168d)

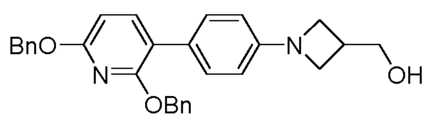

168c (3.5 g, 14.52 mmol) was dissolved in 60 mL of a mixed solvent of 1,4-dioxane and water (v/v) = 4 : 1, and 2,6-bis(benzyloxy)-3-(4,4,5,5-tetramethyl-1,3,2-dioxaborolan-2-yl)pyridine (6 g, 14.38 mmol) (see WO 2021262812 for

the synthesis method), cesium carbonate (9.4 g, 28.85 mmol) and Pd(dppf)$Cl_2$•DCM (587 mg, 0.724 mmol) were added in sequence, nitrogen replacement was performed three times and the mixture was reacted at 100°C for 16 h. The reaction liquid was cooled to room temperature and filtered, and the filtrate was concentrated under reduced pressure. The crude product was separated and purified with silica gel chromatography column (petroleum ether : ethyl acetate (v/v) = 61 : 39) to afford 168d (3.8 g, yield: 58%).

LCMS m/z = 453.2 $[M+1]^+$.

Step 4: 3-(4-(3-(hydroxymethyl)azetidin-1-yl)phenyl)piperidine-2,6-dione (168e)

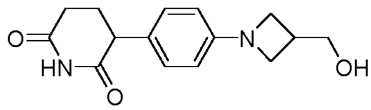

168d (3.8 g, 8.4 mmol) was added into a 100 mL single-mouth bottle, then 50 mL of methanol, 10 mL of DCM and 760 mg of 20% palladium on carbon were added in sequence, hydrogen replacement was performed three times, and the mixture was reacted at 30°C for 4 h. The reaction liquid was filtered, and the filtrate was concentrated under reduced pressure. The crude product was separated and purified with silica gel chromatography column (petroleum ether : ethyl acetate (v/v) = 1 : 4) to afford 168e (1.2 g, yield: 52%).

LCMS m/z = 275.1 $[M+1]^+$.

Step 5: 1-(4-(2,6-dioxopiperidin-3-yl)phenyl)azetidine-3-carbaldehyde (168f)

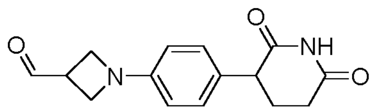

168e (1.2 g, 4.4 mmol) was added into a 100 mL single-mouth bottle, 15 mL of DMSO and 15 mL of THF were added, 2-iodoacylbenzoic acid (1.8 g, 6.43 mmol) was added, and the mixture was reacted at 30°C for 16 h. 200 mL of saturated aqueous sodium carbonate solution and 100 mL of ethyl acetate were added to the reaction liquid, the organic phase was separated, the organic phase was washed with saturated aqueous sodium carbonate solution (80 mL × 2), dried over anhydrous sodium sulfate, concentrated under reduced pressure. The crude product was separated and purified with silica gel chromatography column (petroleum ether : ethyl acetate (v/v) = 39 : 61) to afford 168f (0.4 g, yield: 33%).

LCMS m/z = 273.1 $[M+1]^+$.

Step 6: 3-(4-(3-ethynylazetidin-1-yl)phenyl)piperidine-2,6-dione (168g)

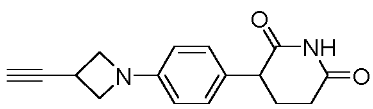

168f (0.4 g, 1.47 mmol) was added into a 100 mL single-mouth bottle, 20 mL of methanol and potassium carbonate (359 mg, 2.6 mmol) were added, the mixture was cooled to 0°C, and dimethyl (1-diazo-2-oxopropyl)phosphonate (432 mg, 2.25 mmol) was slowly added dropwise, and the mixture was reacted at room temperature for 16 h. 150 mL of water and 100 mL of ethyl acetate were added to the reaction liquid, the organic phase was separated, the organic phase was washed with saturated aqueous sodium chloride solution (40 mL × 2), dried over anhydrous sodium sulfate, and concentrated under reduced pressure. The crude product was separated and purified with silica gel chromatography column (petroleum ether : ethyl acetate (v/v) = 3 : 7) to afford a crude product (0.18 g). The above crude product (0.18 g) was dissolved in 30 mL of acetonitrile, 430 mg of a solution of 40% trimethylphenyl hydroxide in methanol

was added, and the mixture was reacted at 60°C for 4 h. The reaction liquid was cooled to room temperature and concentrated under reduced pressure, and the crude product was separated and purified with silica gel chromatography column (petroleum ether : ethyl acetate (v/v) = 27 : 73) to afford 168g (0.1 g, yield: 25%).

LCMS m/z = 269.1 $[M+1]^+$.

Step 7: N-(2-chloro-4-(trifluoromethyl)phenyl)-1-(4-((1-(4-(2,6-dioxopiperidin-3-yl)phenyl)azetidin-3-yl)ethynyl)-1H-pyrazol-1-yl)cyclobutane-1-carboxamide (Compound 168)

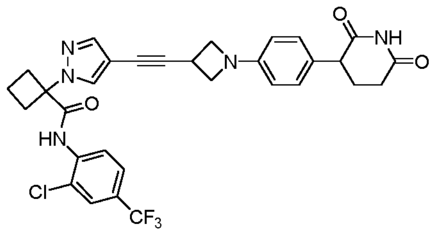

168g (100 mg, 0.37 mmol) was added into a 50 mL single-mouth bottle, 12 mL of dry DMF was added, 14c (189 mg, 0.40 mmol) and TEA (112 mg, 1.11 mmol) were added in sequence, nitrogen replacement was performed three times, and $PdCl_2(PPh_3)_2$ (26 mg, 0.037 mmol) and CuI (14 mg, 0.074 mmol) were added, nitrogen replacement was performed three times, and the mixture was reacted at 50°C for 2 h. The reaction system was cooled to room temperature, 130 mL of saturated aqueous ammonium chloride solution was slowly added, the mixture was extracted with ethyl acetate (70 mL × 3), the organic phases were combined, and the organic phase was washed with saturated aqueous sodium chloride solution (50 mL × 2), dried over anhydrous sodium sulfate, and concentrated under reduced pressure. The crude product was separated and purified with silica gel chromatography column (petroleum ether : ethyl acetate (v/v) = 13 : 87) to afford compound 168 (65 mg, yield: 29%).

$^1H$ NMR (400 MHz, $CDCl_3$) δ 8.69 (s, 1H), 8.54 (d, 1H), 7.98 - 7.84 (m, 1H), 7.82 - 7.45 (m, 4H), 7.25 - 6.97 (m, 2H), 6.55 - 6.33 (m, 2H), 4.35 - 4.15 (m, 2H), 4.05 - 3.82 (m, 2H), 3.79 - 3.65 (m, 2H), 3.17 - 2.98 (m, 2H), 2.86 - 2.52 (m, 4H), 2.35 - 1.98 (m, 4H).

LCMS m/z = 610.2 $[M+1]^+$.

**Example 169: Preparation of compound 169**

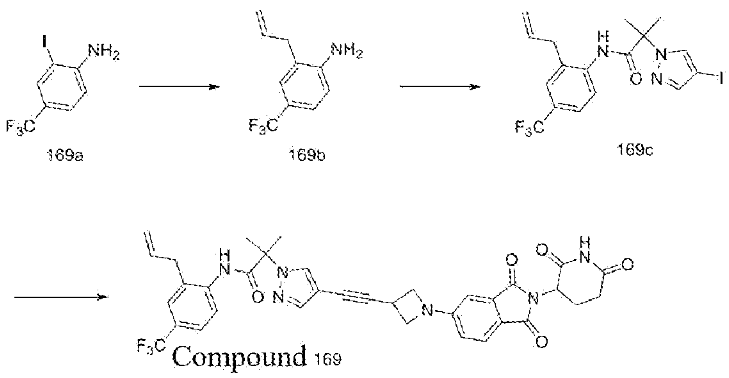

169a 169b 169c

Compound 169

Step 1: 2-allyl-4-(trifluoromethyl)aniline (169b)

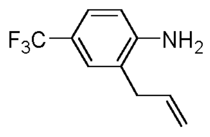

169a (5.74 g, 20.00 mmol) and allylboronic acid pinacol ester (6.71 g, 39.93 mmol) were dissolved in 80 mL of 1,4-dioxane and 25 mL of water, cesium carbonate (13.01 g, 39.93 mmol) was added, nitrogen replacement was performed three times, $Pd(dppf)Cl_2$•DCM (1.63 g, 2.01 mmol) was added, nitrogen replacement was performed three times, and the mixture was reacted at 80°C for 5 h. The reaction liquid was cooled to room temperature, 100 mL of water was added, the mixture was extracted with 100 mL of ethyl acetate. The organic phase was washed with 50 mL of water, dried over anhydrous sodium sulfate and concentrated under reduced pressure. The crude product was separated and purified by silica gel chromatography column (petroleum ether/ethyl acetate (v/v) = 10 : 1) to afford 169b (4.0 g, yield: 99%).

Step 2: N-(2-allyl-4-(trifluoromethyl)phenyl)-2-(4-iodo-1H-pyrazol-1-yl)-2-methylpropanamide (169c)

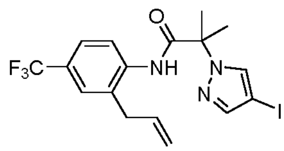

16a-1 (1.67 g, 5.94 mmol) was dissolved in 40 mL of DCM, 1-chloro-N,N,2-trimethylpropenylamine (1.06 g, 7.92 mmol) was added, the mixture was reacted at room temperature for 2 h, and then TEA (2.51 g, 24.80 mmol) and 169b (1.0 g, 4.97 mmol) were added in sequence, and the mixture was reacted at room temperature for 3 h. 150 mL of DCM and 100 mL of saturated aqueous sodium bicarbonate solution were added to the reaction liquid, the organic phase was separated, dried over anhydrous sodium sulfate, and concentrated under reduced pressure. The crude product was separated and purified with silica gel chromatography column (petroleum ether/ethyl acetate (v/v) = 5 : 1) to afford 169c (0.7 g, yield: 30%).

LCMS m/z = 464.0 $[M+1]^+$.

Step 3: N-(2-allyl-4-(trifluoromethyl)phenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 169)

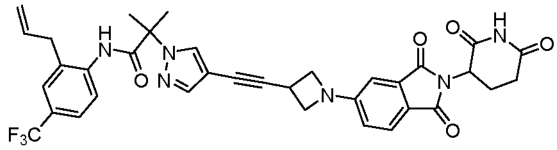

169c (100 mg, 0.22 mmol) was added to 10 mL of dry DMF, intermediate 1 (110 mg) and TEA (67 mg, 0.66 mmol) were added in sequence, nitrogen replacement was performed three times, $PdCl_2(PPh_3)_2$ (15 mg, 0.0214 mmol) and CuI (8 mg, 0.042 mmol) were added, nitrogen replacement was performed three times, and the mixture was reacted at 50°C for 2 h. The reaction liquid was cooled to room temperature, 100 mL of saturated aqueous ammonium chloride solution was slowly added, the mixture was extracted with ethyl acetate (50 mL × 3), the organic phases were combined, and the organic phase was washed with saturated aqueous sodium chloride solution (50 mL × 2), dried over anhydrous sodium sulfate, and concentrated under reduced pressure. The crude product was separated and purified with silica gel chromatography column (petroleum ether : ethyl acetate (v/v) = 12 : 88) to afford compound 169 (75 mg, yield: 51%).

$^1H$ NMR (400 MHz, $CDCl_3$) δ 8.52 (s, 1H), 8.15 (d, 1H), 7.94 (s, 1H), 7.83 - 7.72 (m, 2H), 7.67 (d, 1H), 7.54 - 7.45 (m, 1H), 7.42 - 7.35 (m, 1H), 6.84 - 6.77 (m, 1H), 6.56 (dd, 1H), 5.96 - 5.79 (m, 1H), 5.15 - 5.05 (m, 1H), 5.00 - 4.80 (m, 2H), 4.43 - 4.30 (m, 2H), 4.14 - 4.02 (m, 2H), 3.88 - 3.75 (m, 1H), 3.29 - 3.16 (m, 2H), 2.98 - 2.63 (m, 3H), 2.20 - 2.06 (m, 1H), 1.92 (s, 6H).

LCMS m/z = 673.2 $[M+1]^+$.

**Example 170: Preparation of compound 170**

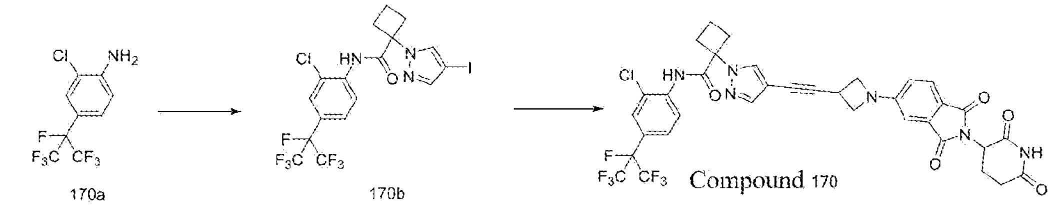

170a 170b Compound 170

Step 1: N-(2-chloro-4-(perfluoropropan-2-yl)phenyl)-1-(4-iodo-1H-pyrazol-1-yl)cyclobutane-1-carboxamide (170b)

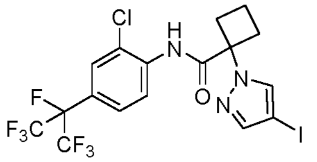

14b (2.30 g, 7.18 mmol) was dissolved in THF (20 mL), 4 mL of water and lithium hydroxide monohydrate (0.6 g, 14.3 mmol) were added, and the mixture was reacted at room temperature for 30 min. 1 mol/L hydrochloric acid was added dropwise to the reaction liquid to adjust the pH to 6, 50 mL of ethyl acetate was added, the liquid separation was conducted, the organic phase was washed with 20 mL of saturated sodium chloride solution, dried over anhydrous sodium sulfate, and concentrated under reduced pressure to afford a crude product (2.0 g). The above crude product (79 mg) was dissolved in 5 mL of DCM, 1-chloro-N,N,2-trimethylpropenylamine (54 mg, 0.40 mmol) was added, the mixture was reacted at room temperature for 1 h, then 0.11 mL of TEA and 170a (80 mg, 0.27 mmol) (see WO 2020126819 for the synthesis method) were added, and the mixture was reacted at room temperature for 16 h. The reaction liquid was concentrated under reduced pressure, and the crude product was separated and purified with silica gel chromatography column (ethyl acetate/petroleum ether (v/v) = 5 : 1) to afford 170b (100 mg, yield: 65%).

Step 2: N-(2-chloro-4-(perfluoropropan-2-yl)phenyl)-1-(4-((1-(2-(2,6-dioxopiperidin-3-yl)-1,3-dioxoisoindolin-5-yl)azetidin-3-yl)ethynyl)-1H-pyrazol-1-yl)cyclobutane-1-carboxamide (Compound 170)

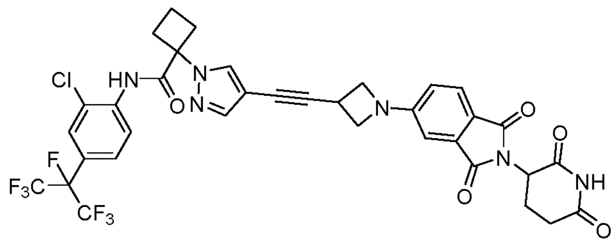

Under nitrogen atmosphere, 170b (100 mg, 0.18 mmol), intermediate 1 (120 mg), $PdCl_2(PPh_3)_2$ (25 mg, 0.036 mmol), CuI (7 mg, 0.037 mmol) and DIPEA (0.12 g, 0.93 mmol) were added into a reaction bottle respectively, 5 mL of DMF was added, and the mixture was reacted at 60°C for 5 h. The reaction system was cooled to room temperature, the reaction liquid was added to 20 mL of water, the mixture was extracted with a mixed solvent of ethyl acetate/methanol (v/v) = 5 : 1 (20 mL × 3), the organic phases were combined, and the organic phase was dried with anhydrous sodium sulfate and concentrated under reduced pressure. The crude product was separated and purified with silica gel chromatography (petroleum ether/ethyl acetate (v/v) = 1 : 2). The obtained crude product was further separated and purified with silica gel column chromatography (petroleum ether/ethyl acetate (v/v) = 1 : 1) to afford compound 170 (15 mg, yield: 11%).

$^1H$ NMR (400 MHz, $CDCl_3$) δ 8.70 (s, 1H), 8.56 (d, 1H), 7.99 (s, 1H), 7.79 (s, 1H), 7.73 - 7.63 (m, 2H), 7.59 -

7.54 (m, 1H), 7.52 - 7.44 (m, 1H), 6.84 - 6.76 (m, 1H), 6.55 (dd, 1H), 4.99 - 4.88 (m, 1H), 4.42 - 4.30 (m, 2H), 4.12 - 4.00 (m, 2H), 3.88 - 3.75 (m, 1H), 3.15 - 3.00 (m, 2H), 2.96 - 2.65 (m, 5H), 2.31 - 2.00 (m, 3H).

LCMS m/z = 778.9 [M+1] $^+$.

**Example 171: Preparation of compound 171**

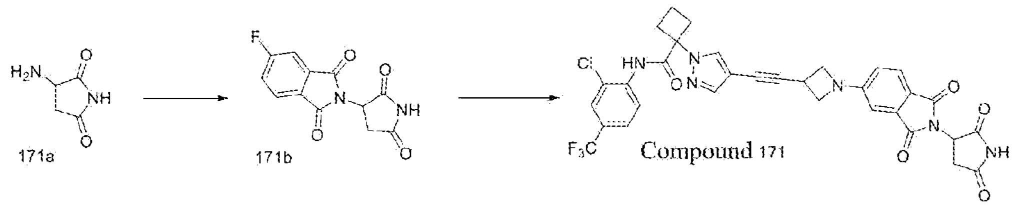


Step 1: 2-(2,5-dioxopyrrolidin-3-yl)-5-fluoroisoindoline-1,3-dione (171b)

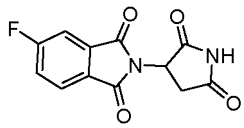

The hydrochloride of 171a (0.8 g, 5.31 mmol) (see WO 2017161119 for the synthesis method), 5-fluoroisobenzofuran-1,3-dione (0.73 g, 4.39 mmol) and potassium acetate (1.43 g, 14.57 mmol) were added to 15 mL of acetic acid in sequence and the mixture was reacted at 90°C for 12 h. The reaction system was cooled to room temperature and added to 50 mL of water, the mixture was extracted with DCM (50 mL × 3), the organic phases were combined, the organic phase was dried over anhydrous sodium sulfate and filtered, and the filtrate was concentrated under reduced pressure, and slurried with 5 mL of ethyl acetate, the mixture was filtered, and the filter cake was dried under vacuum to afford 171b (0.8 g, yield: 70%).

LCMS m/z = 263.1 [M+1] $^+$.

Step 2: N-(2-chloro-4-(trifluoromethyl)phenyl)-1-(4-((1-(2-(2,5-dioxopyrrolidin-3-yl)-1,3-dioxoisoindolin-5-yl)azetidin-3-yl)ethynyl)-1H-pyrazol-1-yl)cyclobutane-1-carboxamide (Compound 171)

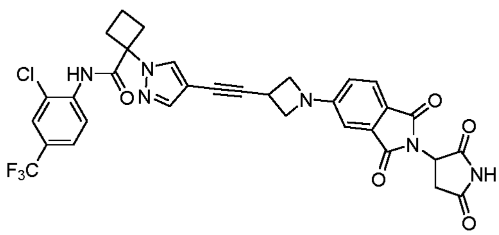

171b (0.19 g, 0.725 mmol), 4-methylbenzenesulfonate of crude 143b (0.3 g) and DIPEA (0.28 g, 2.17 mmol) were dissolved in 5 mL of DMF and the mixture was reacted at 80°C for 5 h. The reaction liquid was cooled to room temperature, 100 mL of ethyl acetate was added, the organic phase was washed with saturated aqueous sodium chloride solution (50 mL × 3), dried over anhydrous sodium sulfate, and concentrated under reduced pressure. The crude product was separated and purified with silica gel chromatography column (petroleum ether/ethyl acetate (v/v) = 1 : 0-1 : 2) to afford compound 171 (0.1 g, yield: 21%).

$^1$H NMR (400 MHz, $CDCl_3$) δ 8.69 (s, 1H), 8.60 - 8.44 (m, 2H), 7.85 - 7.45 (m, 5H), 6.85 - 6.75 (m, 1H), 6.54 (dd, 1H), 5.28 - 5.15 (m, 1H), 4.45 - 4.29 (m, 2H), 4.15 - 4.00 (m, 2H), 3.88 - 3.74 (m, 1H), 3.25 - 2.95 (m, 4H), 2.85 - 2.66 (m, 2H), 2.34 - 2.00 (m, 2H).

LCMS m/z = 665.2 [M+1] +.

**Example 172: Preparation of compound 172**

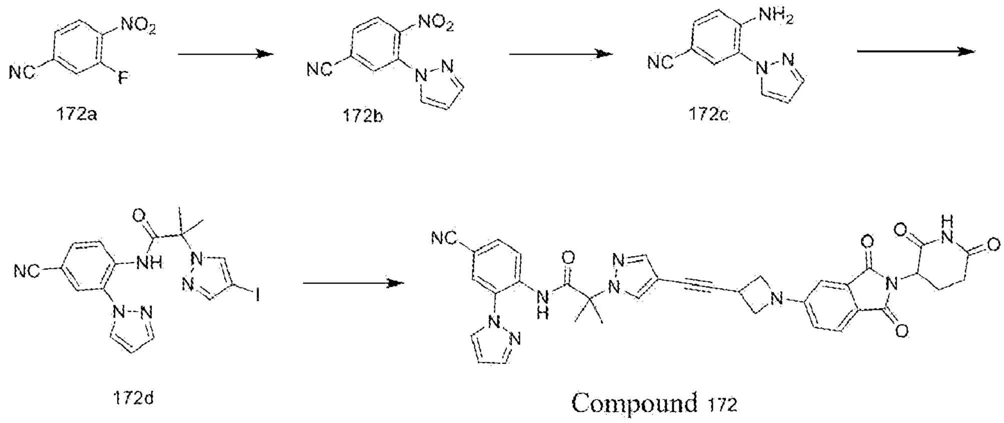

172a 172b 172c

172d

Compound 172

Step 1: 4-nitro-3-(1H-pyrazol-1-yl)benzonitrile (172b)

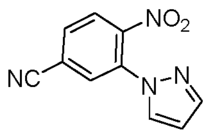

172a (1.66 g, 10.00 mmol), pyrazole (1.02 g, 14.98 mmol) and potassium carbonate (2.77 g, 20.04 mmol) were added to 30 mL of DMF and the mixture was reacted at 80°C for 4 h. The reaction liquid was cooled to room temperature, 100 mL of water was added, the mixture was extracted with 100 mL of ethyl acetate. The organic phase was washed with 50 mL of water, dried over anhydrous sodium sulfate and concentrated under reduced pressure. The crude product was separated and purified by silica gel chromatography column (petroleum ether/ethyl acetate (v/v) = 5 : 1) to afford 172b (1.8 g, yield: 84%).

LCMS m/z = 215.2 [M+1] +.

Step 2: 4-amino-3-(1H-pyrazol-1-yl)benzonitrile (172c)

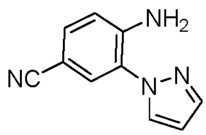

172b (0.9 g, 4.20 mmol) was dissolved in 10 mL of methanol, 0.2 g of 10% palladium on carbon was added, hydrogen replacement was performed three times, and the mixture was reacted at room temperature under the atmosphere of hydrogen balloon for 3.5 h. The reaction liquid was filtered, and the filtrate was concentrated under reduced pressure to afford crude 172c (0.78 g).

LCMS m/z = 185.2 [M+1] +.

Step 3: N-(4-cyano-2-(1H-pyrazol-1-yl)phenyl)-2-(4-iodo-1H-pyrazol-1-yl)-2-methylpropanamide (172d)

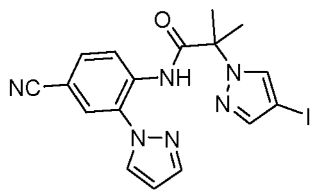

16a-1 (0.49 g, 1.74 mmol) was dissolved in 10 mL of DCM, 1-chloro-N,N,2-trimethylpropenylamine (0.31 g, 2.32 mmol) was added, the mixture was reacted at room temperature for 2 h, and then TEA (0.74 g, 7.31 mmol) and crude 172c (0.27 g) were added in sequence, and the mixture was reacted at room temperature for 16 h. 150 mL of DCM and 50 mL of saturated aqueous sodium bicarbonate solution were added to the reaction liquid, the organic phase was separated, dried over anhydrous sodium sulfate, and concentrated under reduced pressure. The crude product was separated and purified with silica gel chromatography column (petroleum ether/ethyl acetate (v/v) = 5 : 1) to afford 172d (0.25 g, yield: 32%).

Step 4: N-(4-cyano-2-(1H-pyrazol-1-yl)phenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 172)

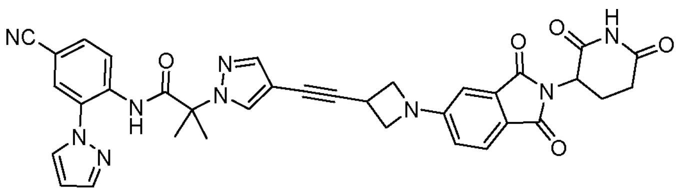

172d (0.22 g, 0.49 mmol), intermediate 1 (0.17 g), TEA (0.15 g, 1.48 mmol), CuI (10 mg, 0.053 mmol) and $PdCl_2(PPh_3)_2$ (34 mg, 0.048 mmol) were added to a reaction bottle, 5 mL of DMF was added under nitrogen protection, and the mixture was reacted at 50°C for 1 h. The reaction liquid was cooled to room temperature, 50 mL of water was added, and the mixture was filtered by suction. The filter cake was washed with 10 mL of water. The filter cake was dissolved in 100 mL of DCM, the mixture was dried over anhydrous sodium sulfate, and concentrated under reduced pressure. The crude product was separated and purified with silica gel column chromatography (petroleum ether/ethyl acetate (v/v) = 1 : 1) to afford compound 172 (0.11 g, yield: 34%).

$^1H$ NMR (400 MHz, $CDCl_3$) δ 10.72 (s, 1H), 8.74 (d, 1H), 8.06 (s, 1H), 7.80 - 7.52 (m, 7H), 6.86 - 6.80 (m, 1H), 6.57 (dd, 1H), 6.53 - 6.46 (m, 1H), 4.99 - 4.89 (m, 1H), 4.45 - 4.32 (m, 2H), 4.14 - 4.03 (m, 2H), 3.92 - 3.78 (m, 1H), 2.96 - 2.65 (m, 3H), 2.20 - 2.06 (m, 1H), 1.88 (s, 6H).

LCMS m/z = 656.6 $[M+1]^+$.

**Example 173: Preparation of compound 173**

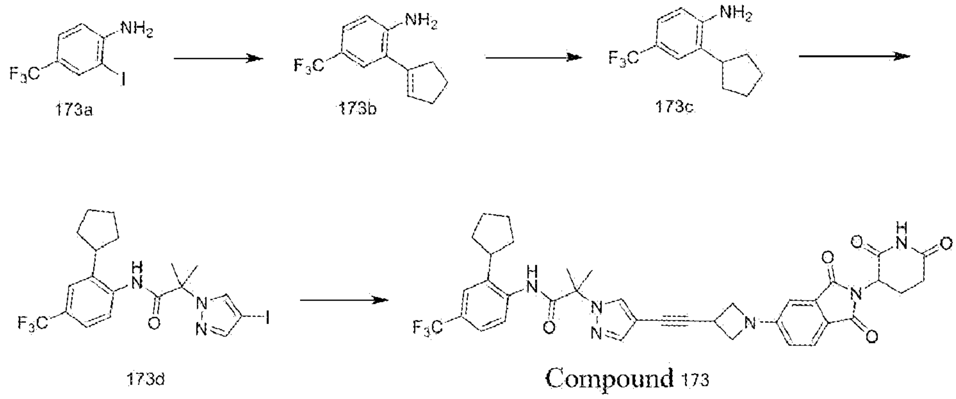

173a 173b 173c

173d Compound 173

Step 1: 2-(cyclopent-1-en-1-yl)-4-(trifluoromethyl)aniline (173b)

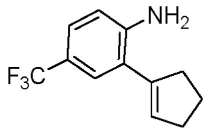

173a (1.0 g, 3.46 mmol), 2-(cyclopent-1-en-1-yl)-4,4,5,5-tetramethyl-1,3,2-dioxaborolane (1.34 g, 6.90 mmol) and cesium carbonate (2.25 g, 6.91 mmol) were added to 25 mL of 1,4-dioxane and 5 mL of water, nitrogen replacement was performed three times, and Pd(dppf)$Cl_2$•DCM (0.28 g, 0.35 mmol) was added, nitrogen replacement was performed three times, and the mixture was reacted at 80°C for 5 h. The reaction liquid was cooled to room temperature, 100 mL of water was added, the mixture was extracted with 100 mL of ethyl acetate. The organic phase was washed with 50 mL of water, dried over anhydrous sodium sulfate and concentrated under reduced pressure. The crude product was separated and purified by silica gel chromatography column (petroleum ether/ethyl acetate (v/v) = 10 : 1) to afford crude 173b (1.1 g).

LCMS m/z = 228.2 $[M+1]^+$.

Step 2: 2-cyclopentyl-4-(trifluoromethyl)aniline (173c)

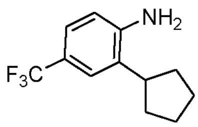

Crude 173b (1.1 g) was dissolved in 10 mL of methanol, 0.2 g of 10% palladium on carbon was added, hydrogen replacement was performed three times, and the mixture was reacted at room temperature under the atmosphere of hydrogen balloon for 16 h. The reaction liquid was filtered, and the filtrate was concentrated under reduced pressure to afford crude 173c (0.8 g).

LCMS m/z = 230.2 $[M+1]^+$.

Step 3: N-(2-cyclopentyl-4-(trifluoromethyl)phenyl)-2-(4-iodo-1H-pyrazol-1-yl)-2-methylpropanamide (173d)

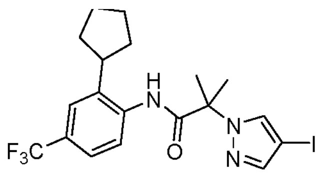

16a-1 (0.34 g, 1.21 mmol) was dissolved in 10 mL of DCM, 1-chloro-N,N,2-trimethylpropenylamine (0.21 g, 1.57 mmol) was added, the mixture was reacted at room temperature for 2 h, and then TEA (0.51 g, 5.04 mmol) and crude 173c (0.23 g) were added in sequence, and the mixture was reacted at room temperature for 16 h. 150 mL of DCM and 50 mL of saturated aqueous sodium bicarbonate solution were added to the reaction liquid, the organic phase was separated, dried over anhydrous sodium sulfate, and concentrated under reduced pressure. The crude product was separated and purified with silica gel chromatography column (petroleum ether/ethyl acetate (v/v) = 5 : 1) to afford 173d (0.45 g, yield: 76%).

Step 4: N-(2-cyclopentyl-4-(trifluoromethyl)phenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 173)

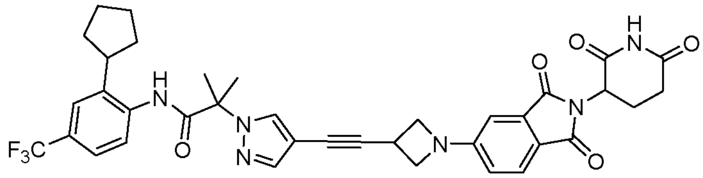

173d (0.20 g, 0.41 mmol), intermediate 1 (0.16 g), TEA (0.12 g, 1.19 mmol), CuI (8 mg, 0.042 mmol) and $PdCl_2(PPh_3)_2$ (28 mg, 0.04 mmol) were added to a reaction bottle, 5 mL of DMF was added under nitrogen protection, and the mixture was reacted at 50°C for 1 h. The reaction liquid was cooled to room temperature, 50 mL of water was added, and the mixture was filtered by suction. The filter cake was washed with 10 mL of water. The filter cake was dissolved in 100 mL of DCM, the mixture was dried over anhydrous sodium sulfate, and concentrated under reduced pressure. The crude product was separated and purified with silica gel column chromatography (petroleum ether/ethyl acetate (v/v) = 1 : 1) to afford compound 173 (0.24 g, yield: 84%).

$^1$H NMR (400 MHz, $CDCl_3$) δ 8.63 (s, 1H), 8.13 (d, 1H), 8.07 (s, 1H), 7.82 (s, 1H), 7.76 (s, 1H), 7.67 (d, 1H), 7.48 - 7.39 (m, 2H), 6.85 - 6.78 (m, 1H), 6.56 (dd, 1H), 4.99 - 4.88 (m, 1H), 4.43 - 4.30 (m, 2H), 4.12 - 4.00 (m, 2H), 3.89 - 3.75 (m, 1H), 2.95 - 2.65 (m, 4H), 2.18 - 2.07 (m, 1H), 2.00 - 1.65 (m, 12H), 1.57 - 1.45 (m, 2H).

LCMS m/z = 701.3 [M+1]$^+$.

**Example 174: Preparation of compound 174**

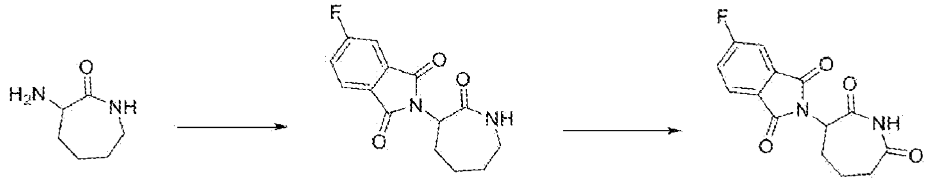

174a 174b 174c

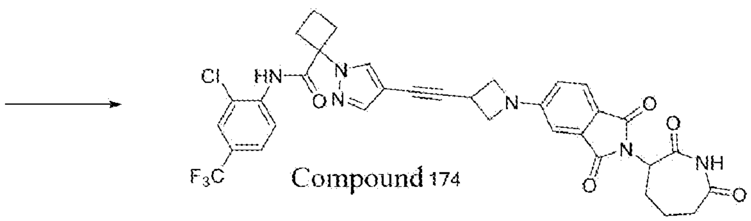

Compound 174

Step 1: 5-fluoro-2-(2-oxoazepan-3-yl)isoindoline-1,3-dione (174b)

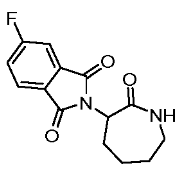

174a (5.25g, 40.96 mmol), 5-fluoroisobenzofuran-1,3-dione (6.8 g, 40.94 mmol) and potassium acetate (10.05 g, 102.4 mmol) were added to 50 mL of acetic acid and the mixture was reacted at 120°C for 12 h. The reaction liquid was cooled to room temperature and filtered, and the filter cake was dissolved in 100 mL of DCM, the mixture was washed with 50 mL of saturated aqueous sodium bicarbonate solution, dried over anhydrous sodium sulfate, and concentrated under reduced pressure to afford 174b (5.0 g, yield: 44%).

Step 2: 2-(2,7-dioxoazepan-3-yl)-5-fluoroisoindoline-1,3-dione (174c)

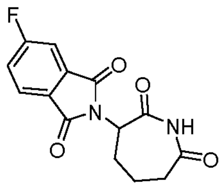

174b (1.0 g, 3.62 mmol) and Dess-Martin oxidant (CAS: 87413-09-0) (4.0 g, 9.43 mmol) were dissolved in 15 mL of acetonitrile, 4 mL of DMSO and 0.08 mL of water, and the mixture was reacted at 90°C for 16 h. The reaction system was cooled to room temperature, 50 mL of ethyl acetate and 50 mL of water were added, the mixture was filtered, the liquid separation was conducted for the filtrate, the organic phase was washed with 50 mL of saturated aqueous sodium bisulfite solution, dried over anhydrous sodium sulfate, concentrated under reduced pressure, and slurried by adding 20 mL of ethyl acetate, the mixture was filtered, and the filter cake was dried under vacuum to afford 174c (0.8 g, yield: 76%).

Step 3: N-(2-chloro-4-(trifluoromethyl)phenyl)-1-(4-((1-(2-(2,7-dioxoazepan-3-yl)-1,3-dioxoisoindolin-5-yl)azetidin-3-yl)ethynyl)-1H-pyrazol-1-yl)cyclobutane-1-carboxamide (Compound 174)

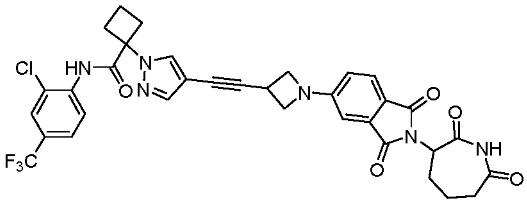

174c (0.07 g, 0.24 mmol), 4-methylbenzenesulfonate of crude 143b (0.1 g) and DIPEA (0.093 g, 0.72 mmol) were dissolved in 2 mL of DMF and the mixture was reacted at 80°C for 5 h. The reaction liquid was cooled to room temperature, 50 mL of ethyl acetate was added, the organic phase was washed with saturated aqueous sodium chloride solution (25 mL × 3), dried over anhydrous sodium sulfate, and concentrated under reduced pressure. The crude product was separated and purified with silica gel chromatography column (petroleum ether/ethyl acetate (v/v) = 1 : 0-1 : 2) to afford compound 174 (0.03 g, yield: 18%).

$^1H$ NMR (400 MHz, $CDCl_3$) δ 8.69 (s, 1H), 8.55 (d, 1H), 7.88 (s, 1H), 7.79 (s, 1H), 7.70 (s, 1H), 7.67 (d, 1H), 7.62 - 7.46 (m, 2H), 6.84 - 6.78 (m, 1H), 6.55 (dd, 1H), 5.25 - 5.10 (m, 1H), 4.45 - 4.27 (m, 2H), 4.15 - 4.00 (m, 2H), 3.90 - 3.72 (m, 1H), 3.20 - 2.65 (m, 7H), 2.34 - 1.98 (m, 5H).

LCMS m/z = 693.2 $[M+1]^+$.

**Example 175: Preparation of compound 175**

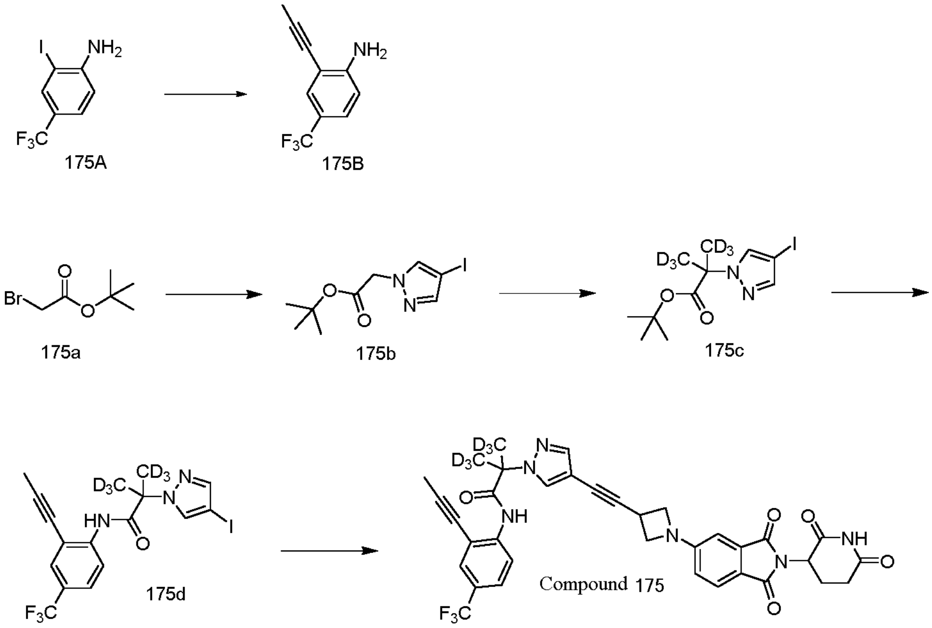
175A 175B
175a 175b 175c
175d Compound 175

Step 1: 2-(prop-1-yn-1-yl)-4-(trifluoromethyl)aniline (175B)

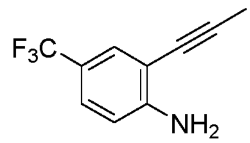

175A (10.00 g, 34.84 mmol) was added to a sealed tube, 100 mL of DMF and 25 mL of methanol were added, and 1-(trimethylsilyl)propyne (7.82 g, 69.67 mmol), CsF (15.88 g, 104.54 mmol), CuI (1.33 g, 6.98 mmol), and $PdCl_2(PPh_3)_2$(2.45 g, 3.49 mmol) were added in sequence under nitrogen protection, and the mixture was reacted at room temperature for 0.5 h. 100 mL of ethyl acetate and 200 mL of purified water were added to the reaction system, the liquid separation was conducted, the aqueous phase was extracted with ethyl acetate (50 mL × 2), the organic phases were combined, the organic phase was washed with 50 mL of saturated aqueous sodium chloride solution, dried over anhydrous sodium sulfate, and concentrated under reduced pressure. The crude product was separated and purified with silica gel chromatography column (petroleum ether : ethyl acetate (v/v) = 10 : 1) to afford 175B (6.50 g, yield: 94%).

LCMS m/z = 200.2 $[M+1]^+$.

Step 2: tert-butyl 2-(4-iodo-1H-pyrazol-1-yl)acetate (175b)

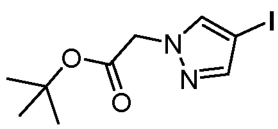

4-iodopyrazole (10.00 g, 51.55 mmol) was dissolved in 100 mL of DMF, 175a (15.08 g, 77.31 mmol) and potassium carbonate (21.37 g, 154.63 mmol) were added in sequence, and the mixture was reacted at 50°C for 3 h. The reaction system was cooled to room temperature, 50 mL of ethyl acetate and 100 mL purified water were added, the liquid separation was conducted, the aqueous phase was extracted with ethyl acetate (30 mL × 2), the organic phase was washed with 30 mL of saturated aqueous sodium chloride solution, dried over anhydrous sodium sulfate, and concentrated under reduced pressure. The crude product was separated and purified with silica gel chromatography column (petroleum ether : ethyl acetate (v/v) = 10 : 1) to afford 175b (15.00 g, yield: 94%).

Step 3: tert-butyl 2-(4-iodo-1H-pyrazol-1-yl)-2-(methyl- *d*$_3$)propanoate-3,3,3-*d*$_3$ (175c)

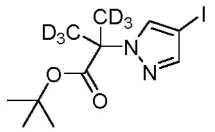

175b (11.60 g, 37.65 mmol) was dissolved in 100 mL of tetrahydrofuran, the temperature of the mixture was lowed to -60°C under nitrogen protection, and a solution of 2 mol/L LDA in THF (47.06 mL, 94.12 mmol) was slowly added dropwise into the above solution and the mixture was stirred at -60°C for 0.5 h, then deuterated methyl iodide (13.64 g, 94.09 mmol) was added dropwise to the reaction system, and the mixture was reacted at room temperature for 2 h. The reaction liquid was poured into 200 mL of aqueous ammonium chloride solution cooled to 0°C, 100 mL of ethyl acetate was added, the liquid separation was conducted, the aqueous phase was extracted with ethyl acetate (50 mL × 2), the organic phase was washed with 50 mL of saturated aqueous sodium chloride solution, dried over anhydrous sodium sulfate, and concentrated under reduced pressure. The crude product was separated and purified with silica gel chromatography column (petroleum ether : ethyl acetate (v/v) = 10 : 1) to afford 175c (4.50 g, yield: 35%).

Step 4: 2-(4-iodo-1H-pyrazol-1-yl)-2-(methyl-*d*$_3$)-N-(2-(prop-1-yn-1-yl)-4-(trifluoromethyl)phenyl)propanamide-*d*$_3$ (175d)

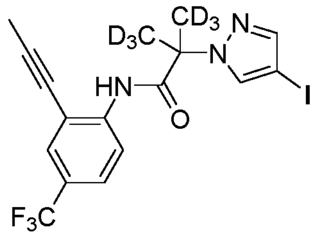

175c (4.50 g, 13.15 mmol) was dissolved in 30 mL of dichloromethane, and 10 mL of trifluoroacetic acid was added. The mixture was reacted at room temperature for 16 h. The reaction system was concentrated under reduced pressure, the residue was dissolved in 30 mL of dichloromethane, and 30 mL of purified water was added for washing. The organic phase was washed with 30 mL of saturated aqueous sodium chloride solution, dried over anhydrous sodium sulfate, and concentrated under reduced pressure. The crude product was separated and purified with silica gel chromatography column (petroleum ether : ethyl acetate (v/v) = 1 : 1) to afford a crude product (3.0 g). The above crude product (0.34 g) was dissolved in 5 mL of dichloromethane, 1-chloro-N,N,2-trimethylpropenylamine (0.24 g, 1.79 mmol) was added, the mixture was reacted at room temperature for 2 h, and then 175B (0.20 g, 1.00 mmol) and triethylamine (0.30 g, 2.96 mmol) were added in sequence, and the mixture was reacted at room temperature for 12 h. 10 mL of ethyl acetate and 10 mL of water were added to the reaction system, the organic phase was separated, the organic phase was washed with 30 mL of saturated aqueous sodium chloride solution, dried over anhydrous sodium sulfate, and concentrated under reduced pressure. The crude product was separated and purified with silica gel chromatography column (petroleum ether : ethyl acetate (v/v) = 5 : 1) to afford 175d (0.23 g, 49%).

LCMS m/z = 468.1 $[M+1]^+$.

Step 5: 2-(4-((1-(2-(2,6-dioxopiperidin-3-yl)-1,3-dioxoisoindolin-5-yl)azetidin-3-yl)ethynyl)-1H-pyrazol-1-yl)-2-(methyl-*d*$_3$)-N-(2-(prop-1-yn-1-yl)-4-(trifluoromethyl) phenyl)propanamide-*d*$_3$ (Compound 175)

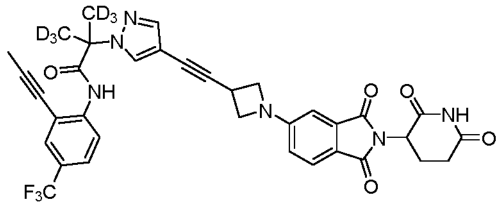

175d (0.23 g, 0.49 mmol) was dissolved in 6 mL of DMF, and crude intermediate 1 (0.20 g), TEA (0.15 g, 1.48 mmol), CuI (0.019 g, 0.1 mmol) and $PdCl_2(PPh_3)_2$ (0.034 g, 0.048 mmol) were added in sequence, and the mixture was reacted at 60°C for 2 h. The reaction system was cooled to room temperature, 15 mL of dichloromethane and 15 mL of purified water were added, the organic phase was separated, the aqueous phase was extracted with dichloromethane (15 mL × 3), the organic phases were combined, and the organic phase was washed with 30 mL of saturated aqueous sodium chloride solution, dried over anhydrous sodium sulfate, and concentrated under reduced pressure. The crude product was separated and purified with silica gel chromatography column (petroleum ether : ethyl acetate (v/v) = 1 : 1) to afford compound 175 (100 mg, yield: 30%).

$^1$H NMR (400 MHz, $CDCl_3$) δ 8.84 (s, 1H), 8.48 (d, 1H), 8.00 (s, 1H), 7.80 (s, 1H), 7.74 (s, 1H), 7.70 - 7.64 (m, 1H), 7.60 - 7.53 (m, 1H), 7.51 - 7.44 (m, 1H), 6.84 - 6.76 (m, 1H), 6.55 (dd, 1H), 4.99 - 4.89 (m, 1H), 4.43 - 4.30 (m, 2H), 4.10 - 4.00 (m, 2H), 3.88 - 3.74 (m, 1H), 2.97 - 2.64 (m, 3H), 2.20 - 2.06 (m, 4H).

LCMS m/z = 677.2 [M+1] $^+$.

**Example 176: Preparation of compound 176**

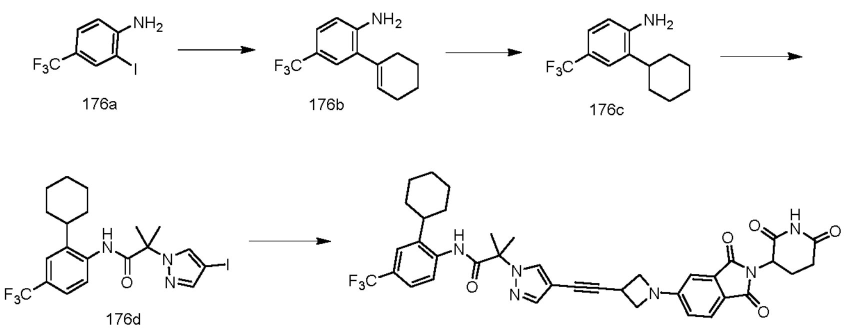

176a 176b 176c

176d

Compound 176

Step 1: 2-(cyclohex-1-en-1-yl)-4-(trifluoromethyl)aniline (176b)

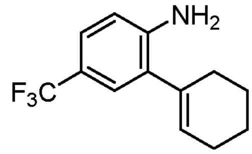

176a (1.0 g, 3.48 mmol), 2-(cyclohex-1-en-1-yl)-4,4,5,5-tetramethyl-1,3,2-dioxaborolane (1.08 g, 5.19 mmol) and cesium carbonate (2.25 g, 6.91 mmol) were added to 25 mL of 1,4-dioxane and 5 mL of water, nitrogen replacement was performed three times, and $Pd(dppf)_2Cl_2 \cdot CH_2Cl_2$ (CAS: 95464-05-4) (0.28 g, 0.35 mmol) was added, nitrogen replacement was performed three times, and the mixture was reacted at 80°C for 5 h. The reaction liquid was cooled to room temperature, 100 mL of water was added, the mixture was extracted with 100 mL of ethyl acetate. The organic phase was washed with 50 mL of water, dried over anhydrous sodium sulfate and concentrated under reduced pressure. The crude product was separated and purified by silica gel chromatography column (petroleum ether/ethyl acetate (v/v) = 10:1) to afford crude 176b (1.0 g).

LCMS m/z = 242.1 [M+1] $^+$.

Step 2: 2-cyclohexyl-4-(trifluoromethyl)aniline (176c)

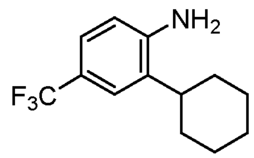

The above crude 176b (1.0 g) was dissolved in 10 mL of methanol, 0.2 g of 10% Pd/C was added, hydrogen replacement was performed three times, and the mixture was reacted at room temperature under the atmosphere of hydrogen balloon for 16 h. The reaction system was filtered, and the filtrate was concentrated under reduced pressure to afford crude 176c (0.8 g).

LCMS m/z = 244.1 $[M+1]^+$.

Step 3: N-(2-cyclohexyl-4-(trifluoromethyl)phenyl)-2-(4-iodo-1H-pyrazol-1-yl)-2-methyl propanamide (176d)

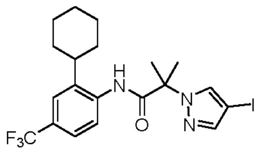

16a-1 (0.34 g, 1.21 mmol) was dissolved in 10 mL of DCM, 1-chloro-N,N,2-trimethylpropenylamine (0.21 g, 1.57 mmol) was added, the mixture was reacted at room temperature for 2 h, and then TEA (0.51 g, 5.04 mmol) and crude 176c (0.24 g) were added in sequence, and the mixture was reacted at room temperature for 16 h. 150 mL of dichloromethane was added to the reaction liquid, 50 mL of saturated aqueous sodium bicarbonate solution was added, the organic phase was separated, dried over anhydrous sodium sulfate, and concentrated under reduced pressure. The crude product was separated and purified with silica gel chromatography column (petroleum ether/ethyl acetate (v/v) = 5 : 1) to afford 176d (0.28 g, yield: 46%).

Step 4: N-(2-cyclohexyl-4-(trifluoromethyl)phenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 176)

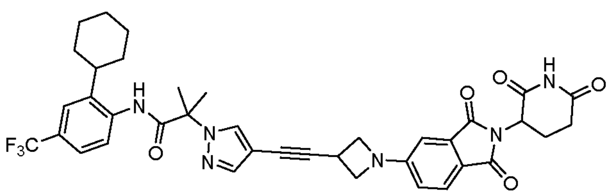

176d (0.28 g, 0.55 mmol), crude intermediate 1 (0.22 g), TEA (0.17 g, 1.68 mmol), CuI (10 mg, 0.053 mmol) and $PdCl_2(PPh_3)_2$ (39 mg, 0.055 mmol) were added to a reaction bottle, 5 mL of DMF was added under nitrogen protection, and the mixture was reacted at 50°C for 1 h. The reaction liquid was cooled to room temperature, 50 mL of water was added, and the mixture was filtered by suction. The filter cake was washed with 10 mL of water. The filter cake was dissolved in 100 mL of DCM, the mixture was dried over anhydrous sodium sulfate, and concentrated under reduced pressure. The crude product was separated and purified with silica gel column chromatography (petroleum ether/ethyl acetate (v/v) = 1 : 1) to afford compound 176 (0.28 g, yield: 71%).

$^1H$ NMR (400 MHz, $CDCl_3$) $\delta$ 8.70 (s, 1H), 8.13 - 8.00 (m, 2H), 7.90 - 7.75 (m, 2H), 7.67 (d, 1H), 7.50 - 7.35 (m, 2H), 6.84 - 6.77 (m, 1H), 6.55 (dd, 1H), 4.99 - 4.89 (m, 1H), 4.44 - 4.30 (m, 2H), 4.13 - 4.00 (m, 2H), 3.89 - 3.75 (m, 1H), 2.95 - 2.65 (m, 3H), 2.43 - 2.28 (m, 1H), 2.18 - 2.07 (m, 1H), 2.03 - 1.65 (m, 11H), 1.55 - 1.20 (m, 5H).

**Example 177: Preparation of compound 177**

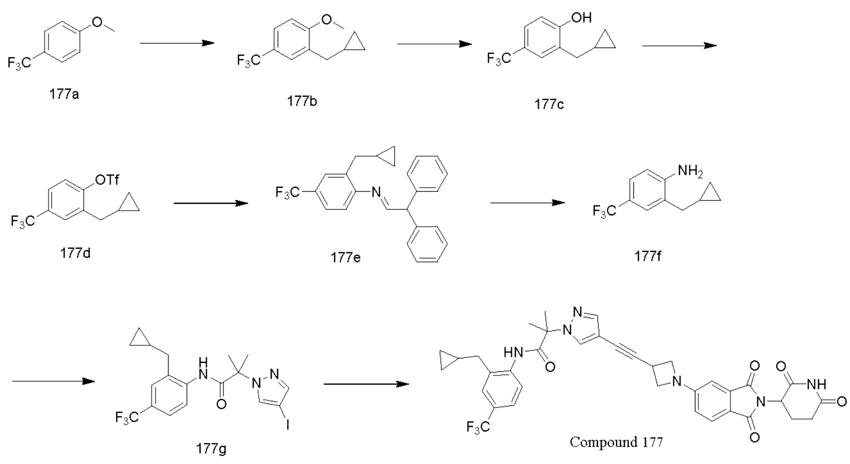

177a 177b 177c
177d 177e 177f
177g Compound 177

Step 1: 2-(cyclopropylmethyl)-1-methoxy-4-(trifluoromethyl)benzene (177b)

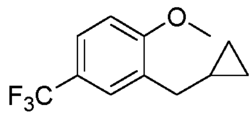

TMEDA (10.56 g, 90.88 mmol) was dissolved in 70 mL of THF, the mixture was cooled to -78°C under nitrogen protection, and a solution of 2.5 mol/L n-BuLi in n-hexane (36.35 mL, 90.88 mmol) was slowly added dropwise to the above solution, the mixture was stirred at -78°C for 15 min, then 40 mL of a solution of 177a (8.00 g, 45.42 mmol) in tetrahydrofuran was added dropwise, and the mixture was warmed to -30°C to -20°C and stirred for 2 h. The reaction system was cooled to -78°C, bromomethylcyclopropane (12.88 g, 95.41 mmol) was added, and the mixture was warmed to room temperature and reacted for 16 h. The reaction liquid was slowly poured into 100 mL of saturated aqueous ammonium chloride solution, the mixture was extracted with ethyl acetate (100 mL × 2), the organic phase was washed with 50 mL of saturated aqueous sodium chloride solution, dried over anhydrous sodium sulfate, and concentrated under reduced pressure. The crude product was separated and purified with silica gel chromatography column (petroleum ether/ethyl acetate (v/v) = 20 : 1) to afford 177b (3.1 g, yield: 30%).

Step 2: 2-(cyclopropylmethyl)-4-(trifluoromethyl)phenol (177c)

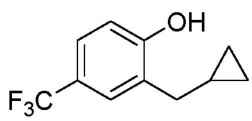

177b (2.5 g, 10.86 mmol) and tetrabutylammonium iodide (8.02 g, 21.71 mmol) were dissolved in 25 mL of dichloromethane, the mixture was cooled to - 78°C under nitrogen protection, and a solution of 1 mol/L $BCl_3$ in DCM (21.72 mL, 21.72 mmol) was slowly added dropwise to the above solution, the mixture was warmed to 0°C and reacted for 0.5 h, and then warmed to room temperature and reacted for 1.5 h. The reaction liquid was poured into 50 mL of ice water, the mixture was extracted with 100 mL of dichloromethane, the organic phase was dried over anhydrous sodium sulfate, and concentrated under reduced pressure. The crude product was separated and purified with silica gel chromatography column (petroleum ether/ethyl acetate (v/v) = 10 : 1) to afford crude 177c (2.3 g).

Step 3: 2-(cyclopropylmethyl)-4-(trifluoromethyl)phenyl trifluoromethanesulfonate (177d)

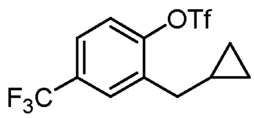

The above crude 177c (2.3 g) and DIPEA (1.65 g, 12.77 mmol) were dissolved in 25 mL of dichloromethane, the mixture was cooled to 0°C under nitrogen protection, and trifluoromethanesulfonic anhydride (3.60 g, 12.76 mmol) was slowly added dropwise, and the mixture was reacted at 0°C-5°C for 0.5 h. 30 mL of water was added to the reaction liquid, the mixture was extracted with 100 mL of dichloromethane, the organic phase was dried over anhydrous sodium sulfate, and concentrated under reduced pressure. The crude product was separated and purified with silica gel chromatography column (petroleum ether/ethyl acetate (v/v) = 20 : 1) to afford 177d (3.0 g, two-step yield from compound 177b: 79%).

Step 4: N-(2-(cyclopropylmethyl)-4-(trifluoromethyl)phenyl)-2,2-diphenylethan-1-imine (177e)

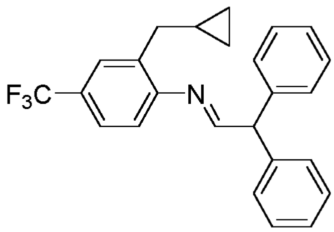

177d (3.00 g, 8.61 mmol) was dissolved in 30 mL of 1,4-dioxane, and benzophenone imine (2.81 g, 15.51 mmol), cesium carbonate (0.45 g, 1.38 mmol), XantPhos (CAS: 161265-03-8) (1.00 g, 1.73 mmol) and $Pd_2(dba)_3$ (CAS: 60748-47-2) (0.79 g, 0.86 mmol) were added in sequence under nitrogen protection, nitrogen replacement was performed three times, and the mixture was reacted at 80°C for 2 h. The reaction liquid was cooled to room temperature, 50 mL of ethyl acetate and 30 mL of purified water were added, the organic phase was separated, the organic phase was washed with 30 mL of saturated aqueous sodium chloride solution, dried over anhydrous sodium sulfate, and concentrated under reduced pressure. The crude product was separated and purified with silica gel chromatography column (petroleum ether/ethyl acetate (v/v) = 10 : 1) to afford 177e (1.10 g, yield: 32%).

Step 5: 2-(cyclopropylmethyl)-4-(trifluoromethyl)aniline (177f)

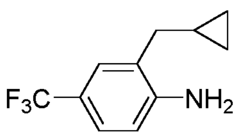

177e (1.10 g, 2.80 mmol) was dissolved in 10 mL of methanol, 3 mL of hydrochloric acid (wt% = 37%) was added, and the mixture was reacted at room temperature for 1 h. 30 mL of ethyl acetate was added to the reaction liquid, the pH was adjusted to 7 with saturated aqueous sodium bicarbonate solution, the organic phase was separated, the organic phase was washed with 50 mL of saturated aqueous sodium chloride solution, dried over anhydrous sodium sulfate, and concentrated under reduced pressure. The crude product was separated and purified with silica gel chromatography column (petroleum ether/ethyl acetate (v/v) = 5 : 1) to afford 177f (0.39 g, yield: 65%).

LCMS m/z = 216.2 $[M+1]^+$.

Step 6: N-(2-(cyclopropylmethyl)-4-(trifluoromethyl)phenyl)-2-(4-iodo-1H-pyrazol-1-yl)-2-methylpropanamide (177g)

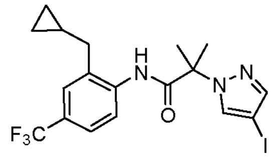

16a-1 (0.78 g, 2.78 mmol) was dissolved in 10 mL of dichloromethane, 1-chloro-N,N,2-trimethylpropenylamine (0.56 g, 4.19 mmol) was added, the mixture was reacted at room temperature for 2 h, and then 177f (0.50 g, 2.32 mmol) and TEA (0.70 g, 6.92 mmol) were added in sequence, and the mixture was reacted at room temperature for 12 h. 10 mL of dichloromethane and 10 mL of water were added to the reaction liquid, the organic phase was separated, the organic phase was washed with 10 mL of saturated aqueous sodium chloride solution, dried over anhydrous sodium sulfate, and concentrated under reduced pressure. The crude product was separated and purified with silica gel chromatography column (petroleum ether/ethyl acetate (v/v) = 5 : 1) to afford 177g (0.50 g, yield: 45%).
LCMS m/z = 478.4 $[M+1]^+$.

Step 7: N-(2-(cyclopropylmethyl)-4-(trifluoromethyl)phenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 177)

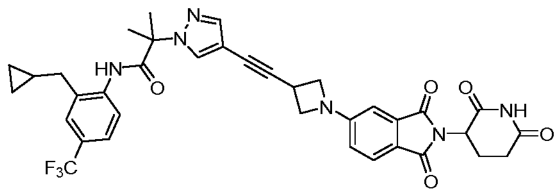

177g (0.50 g, 1.05 mmol) was dissolved in 10 mL of DMF, and crude intermediate 1 (0.39 g), TEA (0.32 g, 3.16 mmol), CuI (0.040 g, 0.21 mmol) and $PdCl_2(PPh_3)_2$ (0.074 g, 0.11 mmol) were added, and the mixture was reacted at 60°C for 2 h. The reaction liquid was cooled to room temperature, 30 mL of dichloromethane and 30 mL of water were added, the organic phase was separated, the aqueous phase was extracted with dichloromethane (30 mL × 3), the organic phases were combined, the organic phase was washed with 30 mL of saturated aqueous sodium chloride solution, dried over anhydrous sodium sulfate, and concentrated under reduced pressure. The crude product was separated and purified with silica gel chromatography column (petroleum ether/ethyl acetate (v/v) = 1 : 1). The crude product was purified with Pre-HPLC (instrument and preparative column: using WATERS 2767 preparative liquid phase chromatographic instrument, preparative column model: Xselect C18, 5 μm, inner diameter × length = 19 mm × 150 mm). Preparation method: the crude product was dissolved with methanol and dimethyl sulfoxide, and filtered with a 0.45 μm filter membrane, to prepare into a sample liquid. Mobile phase system: acetonitrile/water (containing 0.1% TFA). Gradient elution method: gradient elution of 5% to 50% acetonitrile (elution time: 15 min), the obtained preparation solution was adjusted to pH 7 with saturated aqueous sodium bicarbonate solution, the mixture was extracted with dichloromethane (20 mL × 2), and the organic phases were combined, the organic phase was washed with 20 mL of saturated aqueous sodium chloride solution, dried over anhydrous sodium sulfate, and concentrated under reduced pressure to afford compound 177 (0.22 g, yield: 31%).
$^1H$ NMR (400 MHz, $CDCl_3$) δ 8.69 (s, 1H), 8.17 (d, 1H), 7.98 (s, 1H), 7.85 - 7.75 (m, 2H), 7.67 (d, 1H), 7.54 - 7.42 (m, 2H), 6.84 - 6.78 (m, 1H), 6.56 (dd, 1H), 4.99 - 4.88 (m, 1H), 4.44 - 4.30 (m, 2H), 4.14 - 4.00 (m, 2H), 3.90 - 3.74 (m, 1H), 2.95 - 2.64 (m, 3H), 2.37 (d, 2H), 2.17 - 2.08 (m, 1H), 1.95 (s, 6H), 0.92 - 0.82 (m, 1H), 0.60 - 0.50 (m, 2H), 0.18 - 0.07 (m, 2H).
LCMS m/z = 687.3 $[M+1]^+$.

**Example 178: Preparation of compound 178**

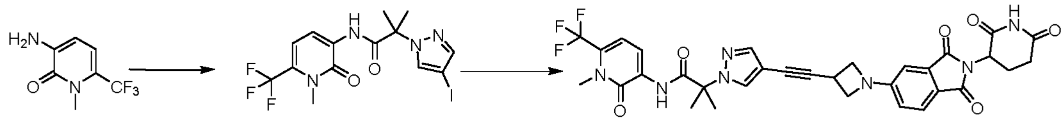

178a 178b Compound 178

Step 1: 2-(4-iodo-1H-pyrazol-1-yl)-2-methyl-N-(1-methyl-2-oxo-6-(trifluoromethyl)-1,2-dihydropyridin-3-yl)propanamide (178b)

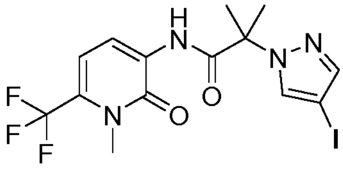

16a-1 (0.054 g, 0.19 mmol) was dissolved in 5 mL of DCM, 1-chloro-N,N,2-trimethylpropenylamine (0.034 g, 0.25 mmol) was added, the mixture was reacted at room temperature for 2 h, and then TEA (0.081 g, 0.80 mmol) and 178a (0.03 g, 0.156 mmol) (see Synthesis, 2011, 7, 1149-1156 for the synthesis method) were added in sequence, and the mixture was reacted at room temperature for 16 h. 50 mL of dichloromethane and 50 mL of saturated aqueous sodium bicarbonate solution were added to the reaction liquid, the organic phase was separated, the organic phase was dried over anhydrous sodium sulfate, and concentrated under reduced pressure. The crude product was separated and purified with silica gel chromatography column (petroleum ether/ethyl acetate (v/v) = 5 : 1) to afford 178b (0.035 g, yield: 49%).

LCMS m/z = 455.0 $[M+1]^+$.

Step 2: 2-(4-((1-(2-(2,6-dioxopiperidin-3-yl)-1,3-dioxoisoindolin-5-yl)azetidin-3-yl)ethynyl)-1H-pyrazol-1-yl)-2-mmthyl-N-(1-methyl-2-oxo-6-(trifluoromethyl)-1,2-dihydropyridin-3-yl)propanamide (Compound 178)

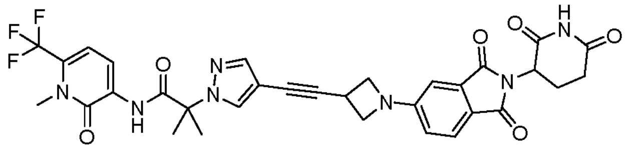

178b (0.03 g, 0.066 mmol), crude intermediate 1 (0.027 g), TEA (0.020 g, 0.198 mmol), CuI (2 mg, 0.0105 mmol) and $PdCl_2(PPh_3)_2$ (5 mg, 0.007 mmol) were added to a reaction bottle, 2 mL of DMF was added under nitrogen protection, and the mixture was reacted at 50°C for 1 h. The reaction liquid was cooled to room temperature, 50 mL of water was added, and the mixture was filtered by suction. The filter cake was washed with 10 mL of water. The filter cake was dissolved in 100 mL of DCM, the mixture was dried over anhydrous sodium sulfate, and concentrated under reduced pressure. The crude product was purified with silica gel column chromatography (petroleum ether/ethyl acetate (v/v) = 1 : 1) to afford compound 178 (0.03 g, yield: 68%).

$^1H$ NMR (400 MHz, $CDCl_3$) δ 9.32 (s, 1H), 8.28 (d, 1H), 7.99 (s, 1H), 7.83 (s, 1H), 7.76 (s, 1H), 7.67 (d, 1H), 6.84 - 6.78 (m, 1H), 6.72 (d, 1H), 6.55 (dd, 1H), 4.98 - 4.89 (m, 1H), 4.40 - 4.30 (m, 2H), 4.10 - 4.02 (m, 2H), 3.87 - 3.72 (m, 1H), 3.70 - 3.60 (m, 3H), 2.97 - 2.63 (m, 3H), 2.19 - 2.07 (m, 1H), 1.92 (s, 6H).

LCMS m/z = 664.7 $[M+1]^+$.

**Example 179: Preparation of compound 179**

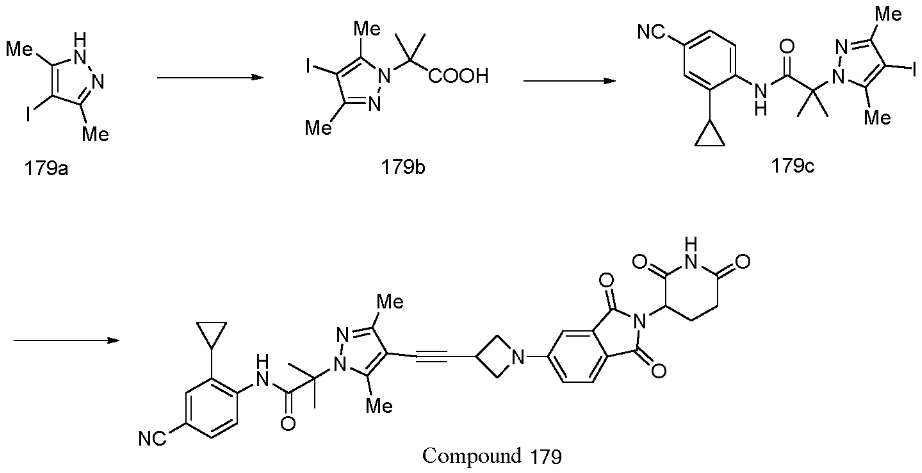

179a

179b

179c

Compound 179

Step 1: 2-(4-iodo-3,5-dimethyl-1H-pyrazol-1-yl)-2-methylpropanoic acid (179b)

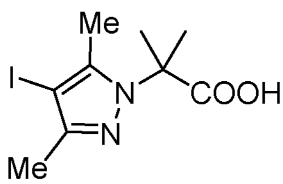

179a (4.0 g, 18.01 mmol), 2-bromo-2-methylpropionic acid (3.00 g, 17.96 mmol) and TEA (5.46 g, 53.96 mmol) were added to a reaction flask respectively, 50 mL of ethyl acetate was added, and the mixture was reacted at 50°C for 3 h. The reaction liquid was cooled to room temperature, 50 mL of water was added, the pH was adjusted to 1 with 1 mol/L hydrochloric acid, the mixture was extracted with ethyl acetate (100 mL × 3), the organic phase was dried with anhydrous sodium sulfate, and concentrated under reduced pressure to afford crude 179b (5.2 g).

Step 2: N-(4-cyano-2-cyclopropylphenyl)-2-(4-iodo-3,5-dimethyl-1H-pyrazol-1-yl)-2-methylpropanamide (179c)

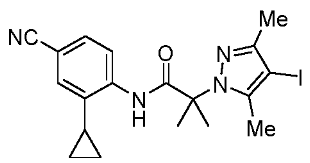

Crude 179b (0.31 g) and 10 mL of dichloromethane were added to a reaction flask respectively, 1-chloro-N,N,2-trimethylpropenylamine (0.20 g, 1.49 mmol) was added, the mixture was stirred at room temperature for 1 h, and then TEA (0.31 g, 3.06 mmol) and 39b (0.19 g, 1.20 mmol) were added, and the mixture was reacted at room temperature for 16 h. The reaction liquid was concentrated under reduced pressure, and the crude product was separated and purified with silica gel chromatography column (ethyl acetate/petroleum ether (v/v) = 10 : 1) to afford 179c (0.20 g, yield: 37%).

Step 3: N-(4-cyano-2-cyclopropylphenyl)-2-(4-((1-(2-(2,6-dioxopiperidin-3-yl)-1,3-dioxoisoindolin-5-yl)azetidin-3-yl)ethynyl)-3,5-dimethyl-1H-pyrazol-1-yl)-2-methylpropanamide (Compound 179)

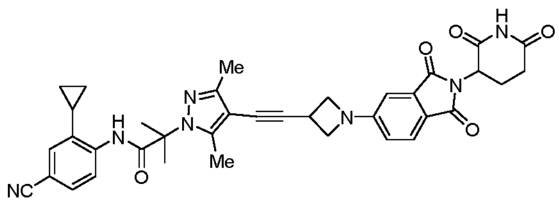

Under nitrogen atmosphere, 179c (0.20 g, 0.45 mmol), crude intermediate 1 (0.24 g), $PdCl_2(PPh_3)_2$ (0.034 g, 0.048 mmol), CuI (0.018 g, 0.095 mmol) and DIPEA (0.19 g, 1.47 mmol) were added into a reaction bottle respectively, 5 mL of DMF was added, and the mixture was reacted at 50°C for 5 h. The reaction system was cooled to room temperature and added to 50 mL of water, the mixture was filtered, the filter cake was dissolved with 20 mL of dichloromethane, the mixture was dried over anhydrous sodium sulfate, and concentrated under reduced pressure. The crude product was separated and purified with silica gel chromatography column (petroleum ether/ethyl acetate (v/v) = 1 : 2), and the crude product obtained was further separated and purified with silica gel column chromatography (petroleum ether/ethyl acetate (v/v) = 1 : 1) to afford compound 179 (0.105 g, yield: 35%).

$^1H$ NMR (400 MHz, $CDCl_3$) δ 8.42 (d, 1H), 8.00 (s, 1H), 7.94 (s, 1H), 7.67 (d, 1H), 7.55 - 7.47 (m, 1H), 7.42 - 7.36 (m, 1H), 6.83 - 6.78 (m, 1H), 6.55 (dd, 1H), 4.97 - 4.88 (m, 1H), 4.44 - 4.32 (m, 2H), 4.07 - 3.97 (m, 2H), 3.92 - 3.78 (m, 1H), 2.96 - 2.64 (m, 3H), 2.29 (d, 6H), 2.18 - 2.08 (m, 1H), 1.93 (s, 6H), 1.36 - 1.22 (m, 1H), 0.84 - 0.74 (m, 2H), 0.50 - 0.40 (m, 2H).

LCMS m/z = 658.8 $[M+1]^+$.

**Example 180: Preparation of compound 180**

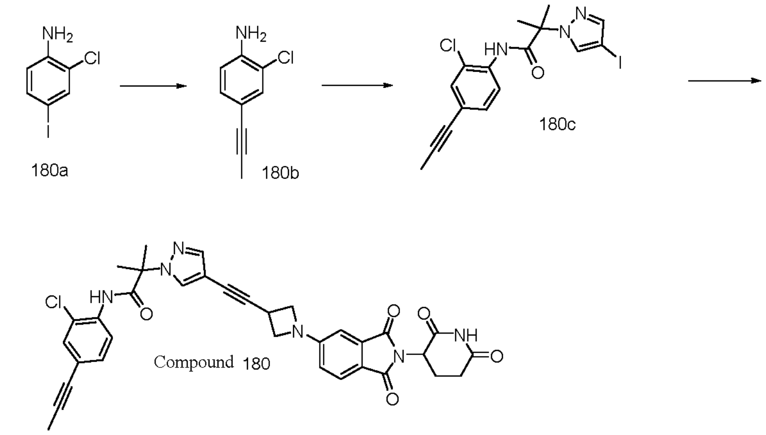

180a

180b

180c

Compound 180

Step 1: 2-chloro-4-(prop-1-yn-1-yl)aniline (180b)

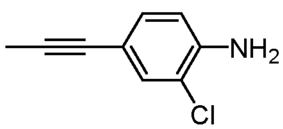

180a (1.0 g, 3.95 mmol), 1-(trimethylsilyl)-1-propyne (0.89 g, 7.93 mmol), CsF (1.8 g, 11.85 mmol), CuI (0.15g, 0.79mmol) and $PdCl_2(PPh_3)_2$ (0.28g, 0.4 mmol) were added into a reaction flask, 10 mL of DMF and 2.5 mL of methanol were added under nitrogen protection, and the mixture was reacted at room temperature for 2 h. 50 mL of ethyl acetate was added to the reaction liquid, the organic phase was washed with saturated aqueous sodium chloride solution (50 mL × 3), dried over anhydrous sodium sulfate, and concentrated under reduced pressure. The crude product was separated and purified with silica gel chromatography column (petroleum ether/ethyl acetate (v/v) = 1 : 0-5 : 1) to afford 180b (0.6 g, yield: 92%).

LCMS m/z = 166.1 $[M+1]^+$.

Step 2: N-(2-chloro-4-(prop-1-yn-1-yl)phenyl)-2-(4-iodo-1H-pyrazol-1-yl)-2-methylpropanamide (180c)

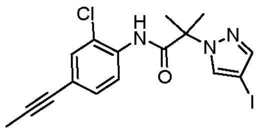

16a-1 (0.13 g, 0.46 mmol) was dissolved in 2 mL of DCM, 1-chloro-N,N,2-trimethylpropenylamine (0.09 g, 0.66 mmol) was added, the mixture was reacted at room temperature for 2 h, and then TEA (0.27 g, 2.67 mmol) and 180b (0.075 g, 0.45 mmol) were added in sequence, and the mixture was reacted at room temperature for 12 h. 50 mL of dichloromethane and 50 mL of water were added to the reaction liquid, the organic phase was separated, dried over anhydrous sodium sulfate, and concentrated under reduced pressure. The crude product was separated and purified with silica gel chromatography column (petroleum ether/ethyl acetate (v/v) = 20 : 1-5 : 1) to afford 180c (0.1 g, yield: 52%).

Step 3: N-(2-chloro-4-(prop-1-yn-1-yl)phenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 180)

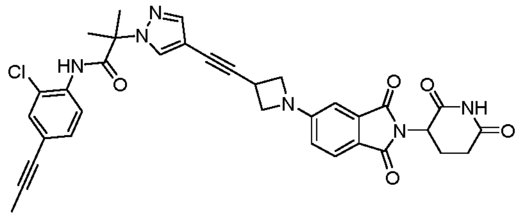

180c (0.1 g, 0.23 mmol), crude intermediate 1 (0.078 g), TEA (0.14 g, 1.38 mmol), CuI (8.8 mg, 0.046 mmol) and $PdCl_2(PPh_3)_2$ (26 mg, 0.037 mmol) were added to 2 mL of DMF under nitrogen protection, and the mixture was reacted at 50°C for 0.5 h. The reaction liquid was cooled to room temperature, 50 mL of water was added, and the mixture was filtered by suction. The filter cake was washed with 10 mL of water. The filter cake was dissolved in 20 mL of DCM, the mixture was dried over anhydrous sodium sulfate. The crude product was separated and purified with silica gel chromatography column (petroleum ether/dichloromethane/ethyl acetate (v/v) = 1 : 1 : 2) to afford compound 180 (0.04 g, yield: 27%).

$^1H$ NMR (400 MHz, $CDCl_3$) δ 8.84 (s, 1H), 8.25 (d, 1H), 8.08 - 7.90 (m, 1H), 7.83 - 7.74 (m, 2H), 7.67 (d, 1H), 7.37 - 7.32 (m, 1H), 7.26 - 7.21 (m, 1H), 6.84 - 6.78 (m, 1H), 6.55 (dd, 1H), 4.98 - 4.88 (m, 1H), 4.40 - 4.30 (m, 2H), 4.13 - 4.01 (m, 2H), 3.88 - 3.74 (m, 1H), 2.96 - 2.64 (m, 3H), 2.20 - 2.07 (m, 1H), 2.02 (s, 3H), 1.94 (s, 6H).

LCMS m/z = 637.2 $[M+1]^+$.

**Example 181: Preparation of compound 181**

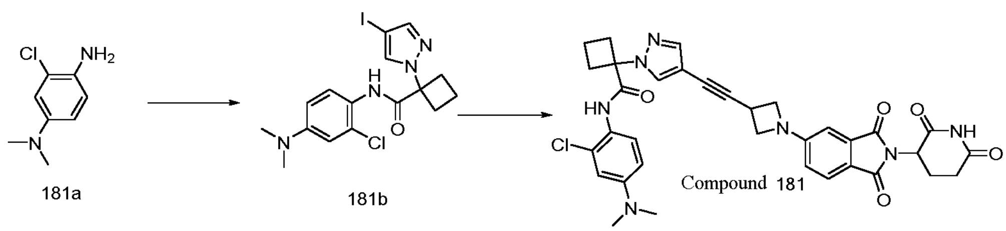
181a
181b
Compound 181

Step 1: N-(2-chloro-4-(dimethylamino)phenyl)-1-(4-iodo-1H-pyrazol-1-yl)cyclobutane-1-carboxamide (181b)

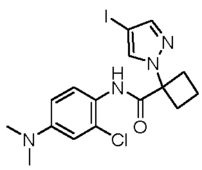

14b (2.30 g, 7.18 mmol) was dissolved in 20 mL of THF, 4 mL of water was added, then lithium hydroxide monohydrate (0.6 g, 14.3 mmol) was added, and the mixture was reacted at room temperature for 30 min. 1 mol/L hydrochloric acid was added dropwise to the reaction liquid to adjust the pH to 6, 50 mL of ethyl acetate was added, the liquid separation was conducted, the organic phase was washed with 20 mL of saturated sodium chloride solution, dried over anhydrous sodium sulfate, and concentrated under reduced pressure to afford a crude product (2.0 g). The above crude product (0.5 g) was dissolved in 30 mL of DCM, 1-chloro-N,N,2-trimethylpropenylamine (0.34 g, 2.52 mmol) was added, the mixture was reacted at room temperature for 2 h, and then TEA (0.52 g, 5.14 mmol) and 181a (0.29 g, 1.70 mmol) (see WO 2008124550 for the synthesis method) were added, and the mixture was reacted at room temperature for 18 h. 20 mL of dichloromethane and 50 mL of saturated aqueous sodium bicarbonate solution were added to the reaction liquid, the organic phase was washed with 100 mL of saturated aqueous sodium chloride solution, dried over anhydrous sodium sulfate, and concentrated under reduced pressure. The crude product was separated and purified with silica gel chromatography column (petroleum ether/ethyl acetate (v/v) = 5 : 1) to afford 181b (0.38 g, yield: 50%).

LCMS m/z = 445.1 $[M+1]^+$.

Step 2: N-(2-chloro-4-(dimethylamino)phenyl)-1-(4-(2-(1-(2-(2,6-dioxopiperidin-3-yl)-1,3-dioxo-2,3-dihydro-1H-isoindol-5-yl)azetidin-3-yl)ethynyl)-1H-pyrazol-1-yl)cyclobutane-1-carboxamide (Compound 181)

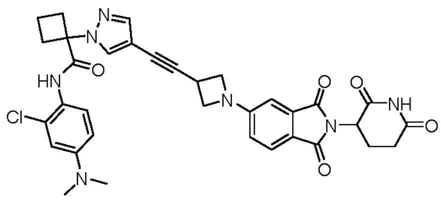

181b (0.35 g, 0.79 mmol) was dissolved in 15 mL of DMF, and crude intermediate 1 (0.40 g), TEA (0.24 g, 2.37 mmol), CuI (30 mg, 0.16 mmol) and $PdCl_2(PPh_3)_2$ (110 mg, 0.16 mmol) were added in sequence, nitrogen replacement was performed three times, and the mixture was reacted at 50°C for 5 h. The reaction liquid was cooled to room temperature, 60 mL of water was added, the solid was precipitated, filtration was performed, the filter cake was washed with 10 mL of water. The filter cake was dissolved in 50 mL of DCM, the mixture was dried over anhydrous sodium sulfate, and concentrated under reduced pressure. The crude product was separated and purified with silica gel chromatography column (petroleum ether/ethyl acetate (v/v) = 1 : 1) to afford compound 181 (0.21 g, yield: 41%).

$^1H$ NMR (400 MHz, $CDCl_3$) δ 8.19 (s, 1H), 8.00 (d, 1H), 7.93 (s, 1H), 7.76 (s, 1H), 7.70 (s, 1H), 7.66 (d, 1H), 6.84 - 6.77 (m, 1H), 6.67 - 6.49 (m, 3H), 4.98 - 4.88 (m, 1H), 4.43 - 4.27 (m, 2H), 4.15 - 4.00 (m, 2H), 3.90 - 3.72 (m, 1H), 3.15 - 3.00 (m, 2H), 2.95 - 2.65 (m, 11H), 2.29 - 1.95 (m, 3H).

LCMS m/z = 654.7 $[M+1]^+$.

**Example 182: Preparation of compound 182**

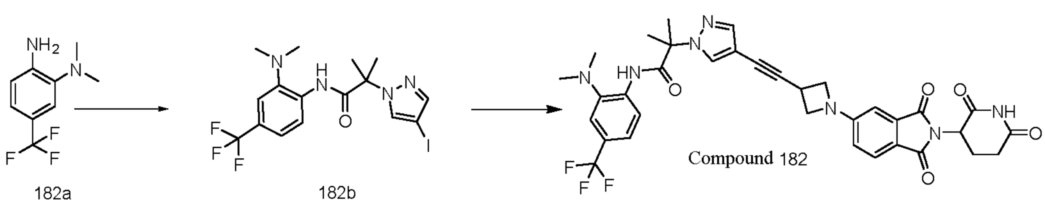

182a 182b Compound 182

Step 1: N-(2-(dimethylamino)-4-(trifluoromethyl)phenyl)-2-(4-iodo-1H-pyrazol-1-yl)-2-methylpropanamide (182b)

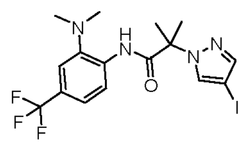

16a-1 (0.5 g, 1.78 mmol) was dissolved in 10 mL of DCM, 1-chloro-N,N,2-trimethylpropenylamine (0.36 g, 2.69 mmol) was added, and the mixture was reacted at room temperature for 2 h. Triethylamine (1.09 g, 10.77 mmol) and 182a (0.37 g, 1.81 mmol) (see WO 2010078408 for the synthesis method) were then added in sequence, and the mixture was reacted at room temperature for 12 h. 50 mL of dichloromethane was added to the reaction liquid, 50 mL of water was added, the organic phase was separated, dried over anhydrous sodium sulfate, and concentrated under reduced pressure. The crude product was separated and purified with silica gel chromatography column (petroleum ether/ethyl acetate (v/v) = 20 : 1-5 : 1) to afford 182b (0.5 g, yield: 60%).
LCMS m/z = 467.1 $[M+1]^+$.

Step 2: N-(2-(dimethylamino)-4-(trifluoromethyl)phenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 182)

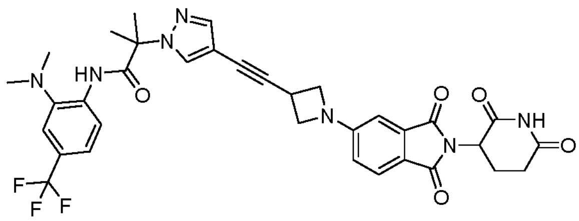

182b (0.44 g, 0.94 mmol), crude intermediate 1 (0.32 g), TEA (0.57 g, 5.63 mmol), CuI (36 mg, 0.19 mmol) and $PdCl_2(PPh_3)_2$ (66 mg, 0.094 mmol) were added to a reaction bottle, 5 mL of DMF was added under nitrogen protection, and the mixture was reacted at 55°C for 1.5 h. The reaction liquid was cooled to room temperature, 50 mL of water was added, and the mixture was filtered by suction. The filter cake was washed with 10 mL of water. The filter cake was dissolved in 100 mL of DCM, the mixture was dried over anhydrous sodium sulfate. The crude product was separated and purified with silica gel chromatography column (petroleum ether/dichloromethane/ethyl acetate (v/v) = 1 : 1 : 2) to afford compound 182 (0.1 g, yield: 16%).
$^1H$ NMR (400 MHz, $CDCl_3$) δ 9.10 (s, 1H), 8.14 (d, 1H), 7.98 (s, 1H), 7.80 (s, 1H), 7.75 (s, 1H), 7.67 (d, 1H), 7.40 - 7.27 (m, 2H), 6.84 - 6.77 (m, 1H), 6.56 (dd, 1H), 4.99 - 4.87 (m, 1H), 4.43 - 4.30 (m, 2H), 4.14 - 4.00 (m, 2H), 3.90 - 3.75 (m, 1H), 2.96 - 2.63 (m, 3H), 2.51 (s, 6H), 2.20 - 2.06 (m, 1H), 1.95 (s, 6H) .
LCMS m/z = 676.3 $[M+1]^+$.

**Example 183: Preparation of compound 183**

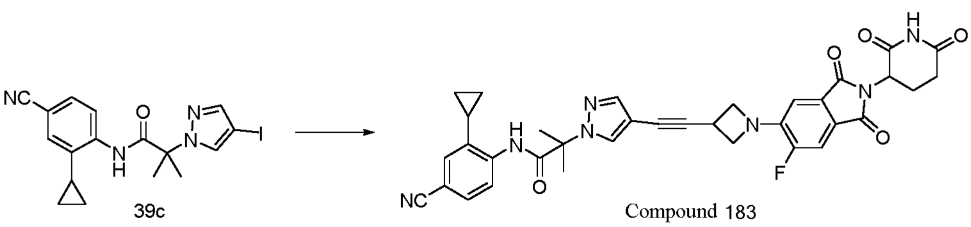

39c

Compound 183

Under nitrogen atmosphere, 39c (0.30 g, 0.71 mmol), crude intermediate 2 (0.38 g), $PdCl_2(PPh_3)_2$ (0.050 g,

0.071 mmol), CuI (0.027 g, 0.14 mmol) and DIPEA (0.28 g, 2.17 mmol) were added into a reaction bottle respectively, 5 mL of DMF was added, and the mixture was reacted at 50°C for 2 h. The reaction system was cooled to room temperature and added to 50 mL of water, the mixture was filtered, the filter cake was collected, the filter cake was dissolved with 50 mL of DCM, the mixture was dried over anhydrous sodium sulfate, and concentrated under reduced pressure. The crude product was separated and purified with silica gel chromatography column (petroleum ether/ethyl acetate (v/v) = 1 : 2), and the crude product obtained was further separated and purified with silica gel column chromatography (petroleum ether/ethyl acetate (v/v) = 1 : 1) to afford compound 183 (0.175 g, yield: 38%).

$^1$H NMR (400 MHz, $CDCl_3$) $\delta$ 9.09 (s, 1H), 8.37 (d, 1H), 7.99 (s, 1H), 7.81 (s, 1H), 7.72 (s, 1H), 7.54 - 7.45 (m, 1H), 7.42 - 7.34 (m, 2H), 6.84 (d, 1H), 4.96 - 4.88 (m, 1H), 4.53 - 4.43 (m, 2H), 4.23 - 4.13 (m, 2H), 3.84 - 3.72 (m, 1H), 2.95 - 2.65 (m, 3H), 2.18 - 2.07 (m, 1H), 1.96 (s, 6H), 1.55 - 1.46 (m, 1H), 1.02 - 0.94 (m, 2H), 0.62-0.53 (m, 2H).

LCMS m/z = 648.3 [M+1]$^+$.

**Example 184: Preparation of compound 184 and compound 185**

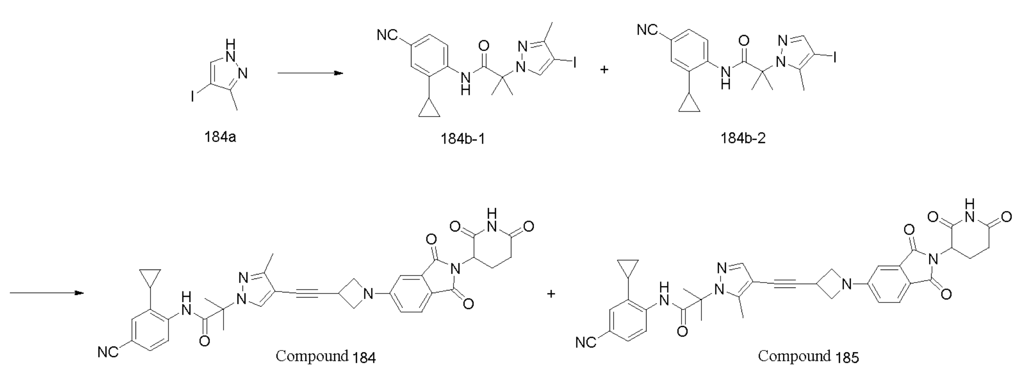

184a 184b-1 184b-2

Compound 184 Compound 185

Step 1: N-(4-cyano-2-cyclopropylphenyl)-2-(4-iodo-3-methyl-1H-pyrazol-1-yl)-2-methylpropanamide (184b-1)

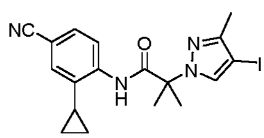

N-(4-cyano-2-cyclopropylphenyl)-2-(4-iodo-5-methyl-1H-pyrazol-1-yl)-2-methylpropanamide (184b-2)

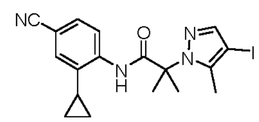

184a (4.00 g, 19.23 mmol), 2-bromo-2-methylpropionic acid (3.20 g, 19.16 mmol), TEA (5.83 g, 57.61 mmol) and 50 mL of ethyl acetate were added to a reaction flask respectively, and the mixture was reacted at 50 °C for 3 h. The reaction system was cooled to room temperature, 1 mol/L hydrochloric acid was added at 0°C to adjust the pH to 2, the mixture was extracted with ethyl acetate (100 mL × 3), the organic phase was dried over anhydrous sodium sulfate, and concentrated under reduced pressure to afford a crude product (5.5 g). The above crude product (0.30 g) and 10 mL of dichloromethane were added to a reaction bottle respectively, 1-chloro-N,N,2-trimethylpropenylamine (0.21 g, 1.57 mmol) was added, the mixture was reacted at room temperature for 1 h, and then triethylamine (0.32 g, 3.16 mmol) and 39b (0.20 g, 1.27 mmol) were added in sequence, and the mixture was reacted at room temperature for 16 h. The reaction liquid was concentrated under reduced pressure, and the crude product was separated and purified with silica gel chromatography column (ethyl acetate/petroleum ether (v/v) = 1 : 10) to afford a mixture of 184b-1 and

184b-2 (0.24 g).

Step 2: N-(4-cyano-2-cyclopropylphenyl)-2-(4-((1-(2-(2,6-dioxopiperidin-3-yl)-1,3-dioxoisoindolin-5-yl)azetidin-3-yl)ethynyl)-3-methyl-1H-pyrazol-1-yl)-2-methylpropanamide (Compound 184)

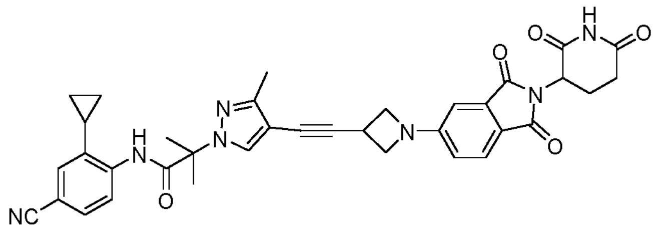

N-(4-cyano-2-cyclopropylphenyl)-2-(4-((1-(2-(2,6-dioxopiperidin-3-yl)-1,3-dioxoisoindolin-5-yl)azetidin-3-yl)ethynyl)-5-methyl-1H-pyrazol-1-yl)-2-methylpropanamide (Compound 185)

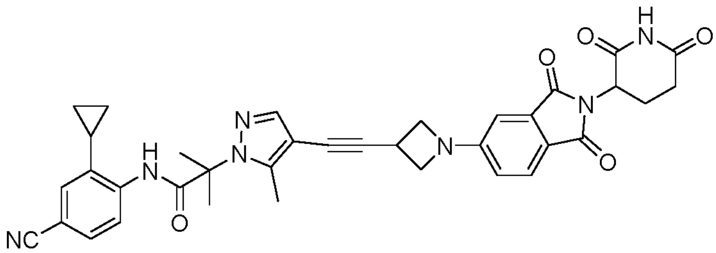

Under nitrogen atmosphere, the mixture of 184b-1 and 184b-2 obtained in the previous step (0.24 g), crude intermediate 1 (0.28 g), $PdCl_2(PPh_3)_2$ (0.039 g, 0.056 mmol), CuI (0.021 g, 0.11 mmol) and DIPEA (0.22 g, 1.70 mmol) were added into a reaction bottle respectively, 5 mL of DMF was added, and the mixture was reacted at 50°C for 2 h. The reaction system was cooled to room temperature and added to 50 mL of water, the mixture was filtered, the filter cake was collected, and the filter cake was subjected to Pre-HPLC (instrument and preparative column: using Waters 2767 preparative liquid phase chromatographic instrument, preparative column model: SunFire@Prep C18, 5 μm, inner diameter × length = 19 mm × 250 mm). Preparation method: the solution of the crude product in DMSO was filtered with a 0.45 μm filter membrane to prepare a sample liquid. Mobile phase system: water (containing 0.1% TFA)/acetonitrile. Gradient elution method: gradient elution of 10% to 50% acetonitrile (elution time: 15 min), lyophilization was performed to afford the trifluoroacetate of compound 184 and the trifluoroacetate of compound 185, respectively. Subsequently, the trifluoroacetates of compounds 184 and 185 were dissolved in 10 mL of ethyl acetate, the mixture was washed by adding 10 mL of ammonia, and the organic phase was dried over anhydrous sodium sulfate and concentrated under reduced pressure to afford compound 184 (50 mg, two-step yield from compound 184a: 7%) and compound 185 (16 mg, two-step yield from compound 184a: 2%), respectively.

Nuclear magnetic resonance spectrum of compound 184:

$^1$H NMR (400 MHz, $CDCl_3$) δ 9.19 (s, 1H), 8.37 (d, 1H), 7.99 (s, 1H), 7.71 (s, 1H), 7.70 - 7.64 (m, 1H), 7.49 (dd, 1H), 7.38 - 7.34 (m, 1H), 6.84 - 6.78 (m, 1H), 6.60 - 6.52 (m, 1H), 4.98 - 4.89 (m, 1H), 4.43 - 4.32 (m, 2H), 4.10 - 4.00 (m, 2H), 3.90 - 3.76 (m, 1H), 2.95 - 2.65 (m, 3H), 2.29 (s, 3H), 2.18 - 2.07 (m, 1H), 1.91 (s, 6H), 1.57 - 1.48 (m, 1H), 1.02 - 0.93 (m, 2H), 0.63 - 0.54 (m, 2H).
LCMS m/z = 644.7 $[M+1]^+$.

Nuclear magnetic resonance spectrum of compound 185:

$^1$H NMR (400 MHz, $CDCl_3$) δ 8.43 (d, 1H), 8.00 (s, 1H), 7.92 (s, 1H), 7.67 (d, 1H), 7.61 (s, 1H), 7.52 (dd, 1H), 7.41 - 7.37 (m, 1H), 6.80 (d, 1H), 6.55 (dd, 1H), 4.97 - 4.89 (m, 1H), 4.42 - 4.30 (m, 2H), 4.09 - 4.00 (m, 2H), 3.89 - 3.77 (m, 1H), 2.95 - 2.65 (m, 3H), 2.33 (s, 3H), 2.17 - 2.08 (m, 1H), 1.95 (s, 6H), 1.36 - 1.22 (m, 1H), 0.92 - 0.76 (m, 2H),

0.48 - 0.40 (m, 2H).
LCMS m/z = 644.8 $[M+1]^+$.

**Example 186: Preparation of compound 186**

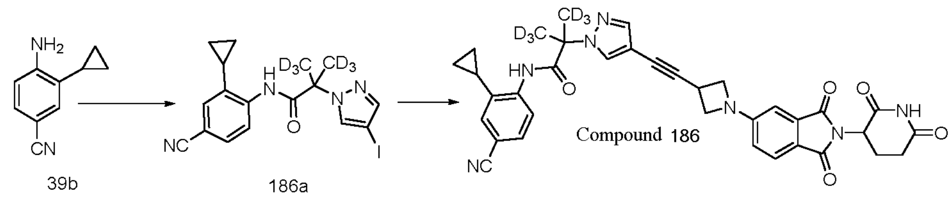

Compound 186

39b

186a

Step 1: N-(4-cyano-2-cyclopropylphenyl)-2-(4-iodo-1H-pyrazol-1-yl)-2,2-(dimethyl-$d_6$)acetamide (186a)

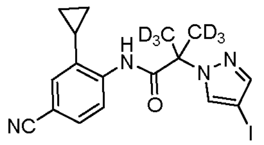

175c (4.50 g, 13.15 mmol) was dissolved in 30 mL of dichloromethane, and 10 mL of trifluoroacetic acid was added. The mixture was reacted at room temperature for 16 h. The reaction system was concentrated under reduced pressure, the residue was dissolved in 30 mL of dichloromethane, and 30 mL of purified water was added for washing. The organic phase was washed with 30 mL of saturated aqueous sodium chloride solution, dried over anhydrous sodium sulfate, and concentrated under reduced pressure. The crude product was separated and purified with silica gel chromatography column (petroleum ether : ethyl acetate (v/v) = 1 : 1) to afford a crude product (3.0 g). The above crude product (0.31 g) was dissolved in 7 mL of DCM, 1-chloro-N,N,2-trimethylpropenylamine (0.22 g, 1.64 mmol) was added, the mixture was reacted at room temperature for 1 h, and then triethylamine (0.33 g, 3.26 mmol) and 39b (0.17 g, 1.07 mmol) were added in sequence, and the mixture was reacted at room temperature for 19 h. 55 mL of dichloromethane was added to the reaction liquid, 60 mL of water was added, the organic phase was separated, dried over anhydrous sodium sulfate, and concentrated under reduced pressure. The crude product was separated and purified with silica gel chromatography column (petroleum ether/ethyl acetate (v/v) = 20 : 1-5 : 1) to afford 186a (0.21 g, yield: 46%).
LCMS m/z = 427.1 $[M+1]^+$.

Step 2: N-(4-cyano-2-cyclopropylphenyl)-2-(4-((1-(2-(2,6-dioxopiperidin-3-yl)-1,3-dioxoisoindolin-5-yl)azetidin-3-yl)ethynyl)-1H-pyrazol-1-yl)-2,2-(dimethyl-$d_6$)acetamide (Compound 186)

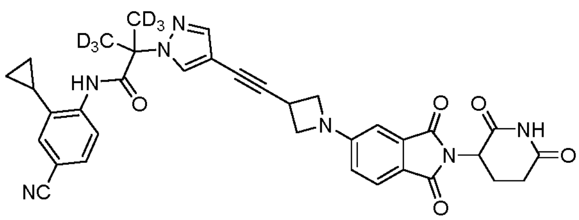

186a (0.1 g, 0.23 mmol), crude intermediate 1 (0.12 g), TEA (0.070 g, 0.69 mmol), CuI (8 mg, 0.042 mmol) and $PdCl_2(PPh_3)_2$ (32 mg, 0.046 mmol) were added to a reaction bottle, 5 mL of DMF was added under nitrogen protection, and the mixture was reacted at 55°C for 4 h. The reaction liquid was cooled to room temperature, 50 mL of water was added, and the mixture was filtered by suction. The filter cake was washed with 10 mL of water. The filter cake was dissolved in 100 mL of DCM, the mixture was dried over anhydrous sodium sulfate. The crude product was separated and purified with silica gel chromatography column (petroleum ether/ethyl acetate (v/v) = 1 : 1.5) to afford

compound 186 (32 mg, yield: 22%).

$^1H$ NMR (400 MHz, $CDCl_3$) δ 9.09 (s, 1H), 8.37 (d, 1H), 8.07 (s, 1H), 7.80 (s, 1H), 7.72 (s, 1H), 7.67 (d, 1H), 7.49 (dd, 1H), 7.40 - 7.34 (m, 1H), 6.81 (d, 1H), 6.55 (dd, 1H), 5.01 - 4.87 (m, 1H), 4.42 - 4.30 (m, 2H), 4.11 - 4.01 (m, 2H), 3.86 - 3.74 (m, 1H), 2.95 - 2.65 (m, 3H), 2.19 - 2.08 (m, 1H), 1.56 - 1.45 (m, 1H), 1.04 - 0.93 (m, 2H), 0.63 - 0.53 (m, 2H) .

LCMS m/z = 636.4 $[M+1]^+$.

**Example 187: Preparation of compound 187**

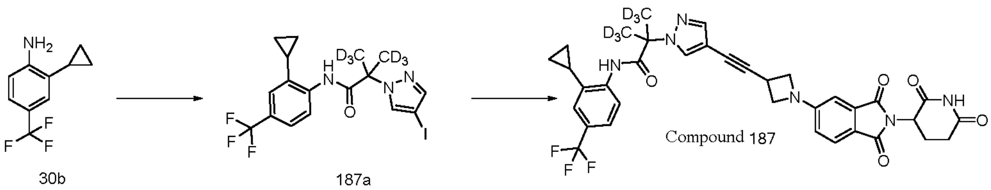

30b

187a

Compound 187

Step 1: N-(2-cyclopropyl-4-(trifluoromethyl)phenyl)-2-(4-iodo-1H-pyrazol-1-yl)-2,2-(dimethyl-$d_6$)acetamide (187a)

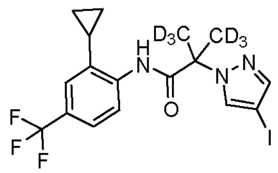

175c (4.50 g, 13.15 mmol) was dissolved in 30 mL of dichloromethane, and 10 mL of trifluoroacetic acid was added. The mixture was reacted at room temperature for 16 h. The reaction system was concentrated under reduced pressure, the residue was dissolved in 30 mL of dichloromethane, and 30 mL of purified water was added for washing. The organic phase was washed with 30 mL of saturated aqueous sodium chloride solution, dried over anhydrous sodium sulfate, and concentrated under reduced pressure. The crude product was separated and purified with silica gel chromatography column (petroleum ether : ethyl acetate (v/v) = 1 : 1) to afford a crude product (3.0 g). The above crude product (0.31 g) was dissolved in 7 mL of DCM, 1-chloro-N,N,2-trimethylpropenylamine (0.22 g, 1.64 mmol) was added, the mixture was reacted at room temperature for 1 h, and then triethylamine (0.33 g, 3.26 mmol) and 30b (0.22 g, 1.09 mmol) were added in sequence, and the mixture was reacted at room temperature for 19 h. 40 mL of dichloromethane was added to the reaction liquid, 70 mL of water was added, the organic phase was separated, dried over anhydrous sodium sulfate, and concentrated under reduced pressure. The crude product was separated and purified with silica gel chromatography column (petroleum ether/ethyl acetate (v/v) = 20 : 1-5 : 1) to afford 187a (0.29 g, yield: 57%).

LCMS m/z = 470.1 $[M+1]^+$.

Step 2: N-(2-cyclopropyl-4-(trifluoromethyl)phenyl)-2-(4-((1-(2-(2,6-dioxopiperidin-3-yl)-1,3-dioxoisoindolin-5-yl)azetidin-3-yl)ethynyl)-1H-pyrazol-1-yl)-2,2-(dimethyl-$d_6$)acetamide (Compound 187)

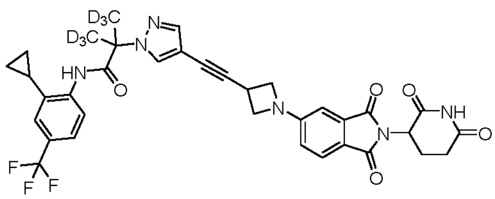

187a (0.14 g, 0.30 mmol), crude intermediate 1 (0.15 g), TEA (0.091 g, 0.9 mmol), CuI (11 mg, 0.06 mmol) and $PdCl_2(PPh_3)_2$ (42 mg, 0.06 mmol) were added to a reaction bottle, 5 mL of DMF was added under nitrogen protection,

and the mixture was reacted at 55°C for 4 h. The reaction liquid was cooled to room temperature, 50 mL of water was added, and the mixture was filtered by suction. The filter cake was washed with 10 mL of water. The filter cake was dissolved in 100 mL of DCM, the mixture was dried over anhydrous sodium sulfate. The crude product was separated and purified with silica gel chromatography column (petroleum ether/ethyl acetate (v/v) = 1 : 1.5) to afford compound 187 (32 mg, yield: 16%).

$^1$H NMR (400 MHz, $CDCl_3$) $\delta$ 8.91 (s, 1H), 8.33 (d, 1H), 8.02 (s, 1H), 7.81 (s, 1H), 7.72 (s, 1H), 7.70 - 7.64 (m, 1H), 7.49 - 7.41 (m, 1H), 7.36 - 7.30 (m, 1H), 6.83 - 6.77 (m, 1H), 6.55 (dd, 1H), 4.98 - 4.88 (m, 1H), 4.43 - 4.30 (m, 2H), 4.11 - 4.00 (m, 2H), 3.90 - 3.75 (m, 1H), 2.96 - 2.62 (m, 3H), 2.19 - 2.08 (m, 1H), 1.59 - 1.47 (m, 1H), 1.00 - 0.90 (m, 2H), 0.62 - 0.54 (m, 2H) .

LCMS m/z = 679.3 $[M+1]^+$.

**Example 188: Preparation of compound 188**

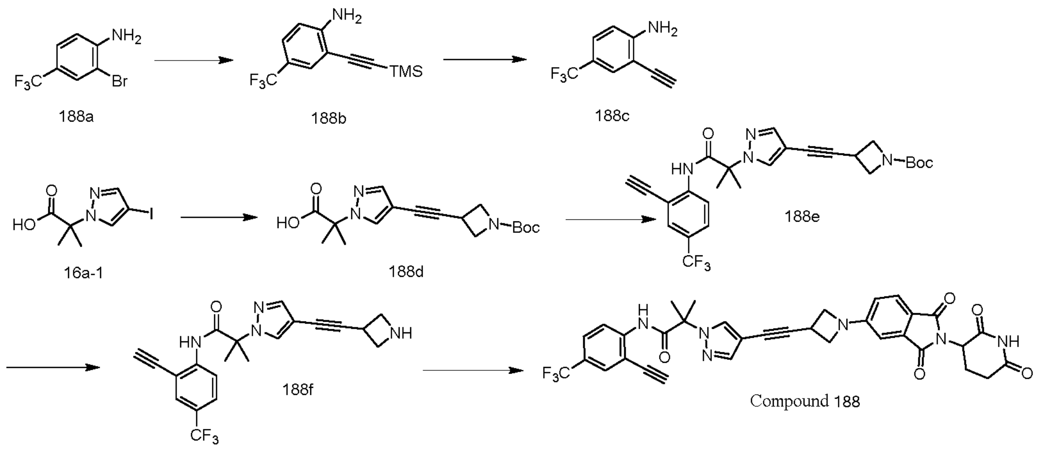

188a 188b 188c 16a-1 188d 188e 188f Compound 188

Step 1: 4-(trifluoromethyl)-2-((trimethylsilyl)ethynyl)aniline (188b)

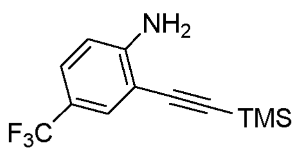

188a (1.0 g, 4.17 mmol), trimethylsilylacetylene (0.82 g, 8.34 mmol), TEA (1.27 g, 12.55 mmol), CuI (79 mg, 0.41 mmol) and $PdCl_2(PPh_3)_2$ (290 mg, 0.41 mmol) were added to a reaction bottle, 30 mL of toluene was added under nitrogen protection, nitrogen replacement was performed three times, and the mixture was reacted at 50°C for 18 h. The reaction liquid was cooled to room temperature, and the reaction system was concentrated under reduced pressure. The crude product was separated and purified with silica gel column chromatography (petroleum ether/ethyl acetate (v/v) = 10 : 1) to afford 188b (0.73 g, yield: 68%).

LCMS m/z = 258.3 $[M+1]^+$.

Step 2: 2-ethynyl-4-(trifluoromethyl)aniline (188c)

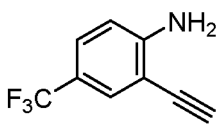

188b (0.4 g, 1.55 mmol) was dissolved in 10 mL of THF, TBAF·$3H_2O$ (0.73 g, 2.31 mmol) was added, and the mixture was stirred at room temperature for 30 min. The reaction system was concentrated under reduced pressure, and the crude product was separated and purified with silica gel column chromatography (petroleum ether/ethyl acetate (v/v) = 5 : 1) to afford 188c (0.22 g, yield: 77%).

Step 3: 2-(4-((1-(tert-butoxycarbonyl)azetidin-3-yl)ethynyl)-1H-pyrazol-1-yl)-2-methylpropanoic acid (188d)

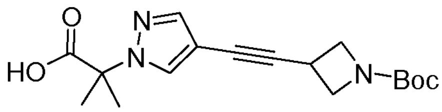

16a-1 (2.8 g, 10.02 mmol), tert-butyl 3-ethynylazetidine-1-carboxylate (2.17 g, 11.97 mmol), TEA (3.04 g, 30.04 mmol), CuI (190 mg, 1.00 mmol) and $PdCl_2(PPh_3)_2$ (700 mg, 1.00 mmol) were added to a reaction flask, and 60 mL of dichloromethane was added under nitrogen protection, nitrogen replacement was performed three times, and the mixture was reacted at room temperature for 18 h. 150 mL of water was added to the reaction system, the pH of the system was adjusted to 2 with 1 mol/L hydrochloric acid, the mixture was extracted with 100 mL of dichloromethane, dried over anhydrous sodium sulfate, and concentrated under reduced pressure. The crude product was separated and purified with silica gel column chromatography (dichloromethane/methanol (v/v) = 20 : 1) to afford 188d (3.3 g, yield: 99%).
LCMS m/z = 334.5 $[M+1]^+$.

Step 4: tert-butyl 3-((1-(1-((2-ethynyl-4-(trifluoromethyl)phenyl)amino)-2-methyl-1-oxopropan- 2-yl)-1H-pyrazol-4-yl)ethynyl)azetidine-1-carboxylate (188e)

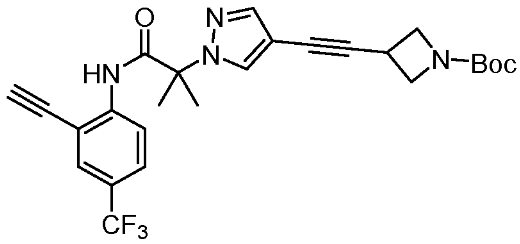

188c (0.185 g, 1.00 mmol) and 188d (0.40 g, 1.20 mmol) were dissolved in 15 mL of DCM, TCFH (0.42 g, 1.50 mmol) was added, and N-methylimidazole (0.33 g, 4.02 mmol) was slowly added dropwise, the mixture was reacted at room temperature for 16 h. The reaction system was concentrated under reduced pressure, and the crude product was separated and purified with silica gel chromatography column (petroleum ether/ethyl acetate (v/v) = 4 : 1) to afford 188e (0.37 g, yield: 74%).

Step 5: 2-(4-(azetidin-3-ylethynyl)-1H-pyrazol-1-yl)-N-(2-ethynyl-4-(trifluoromethyl)phenyl)-2-methylpropanamide (188f) 4-methylbenzenesulfonate

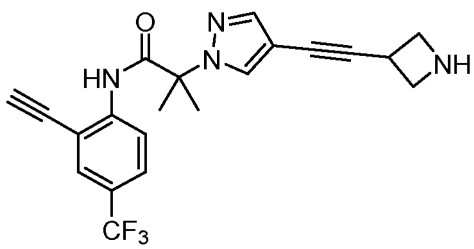

188e (150 mg, 0.3 mmol) was dissolved in 4 mL of acetonitrile, p-toluenesulfonic acid monohydrate (0.22 g,

1.16 mmol) was added, and the mixture was stirred at room temperature for 2 h. The reaction liquid was concentrated under reduced pressure to afford crude 4-methylbenzenesulfonate of 188f (0.36 g).

LC-MS m/z = 401.1 $[M+1]^+$.

Step 6: 2-(4-((1-(2-(2,6-dioxopiperidin-3-yl)-1,3-dioxoisoindolin-5-yl)azetidin-3-yl)ethynyl)-1H-pyrazol-1-yl)-N-(2-ethynyl-4-(trifluoromethyl)phenyl)-2-methylpropanamide (Compound 188)

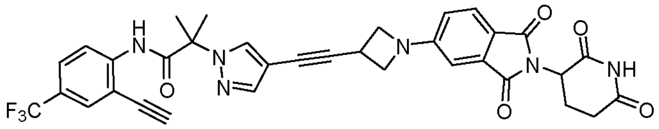

The 4-methylbenzenesulfonate of crude 188f (0.36 g) was dissolved in 5 mL of DMSO, TEA (0.19 g, 1.88 mmol) and 1C (0.17 g, 0.62 mmol) were added, and the mixture was reacted at 80°C for 3 h. The reaction liquid was cooled to room temperature, 40 mL of water was added, the mixture was filtered, the filter cake was collected, the filter cake was washed with 10 mL of water, the filter cake was dissolved with 50 mL of DCM, the mixture was dried over anhydrous sodium sulfate, and concentrated under reduced pressure. The crude product was separated and purified with silica gel chromatography column (petroleum ether/ethyl acetate (v/v) = 1 : 2) to afford compound 188 (30 mg, yield: 7%).

$^1H$ NMR (400 MHz, $CDCl_3$) $\delta$ 8.87 (s, 1H), 8.52 (d, 1H), 8.05 - 7.95 (m, 1H), 7.80 (s, 1H), 7.73 (s, 1H), 7.71 - 7.62 (m, 2H), 7.60 - 7.52 (m, 1H), 6.84 - 6.78 (m, 1H), 6.56 (dd, 1H), 4.98 - 4.89 (m, 1H), 4.42 - 4.30 (m, 2H), 4.12 - 4.00 (m, 2H), 3.87 - 3.75 (m, 1H), 3.45 (s, 1H), 2.98 - 2.65 (m, 3H), 2.18 - 2.08 (m, 1H), 1.95 (s, 6H).

LCMS m/z = 657.2 $[M+1]^+$.

**Example 189: Preparation of compound 189**

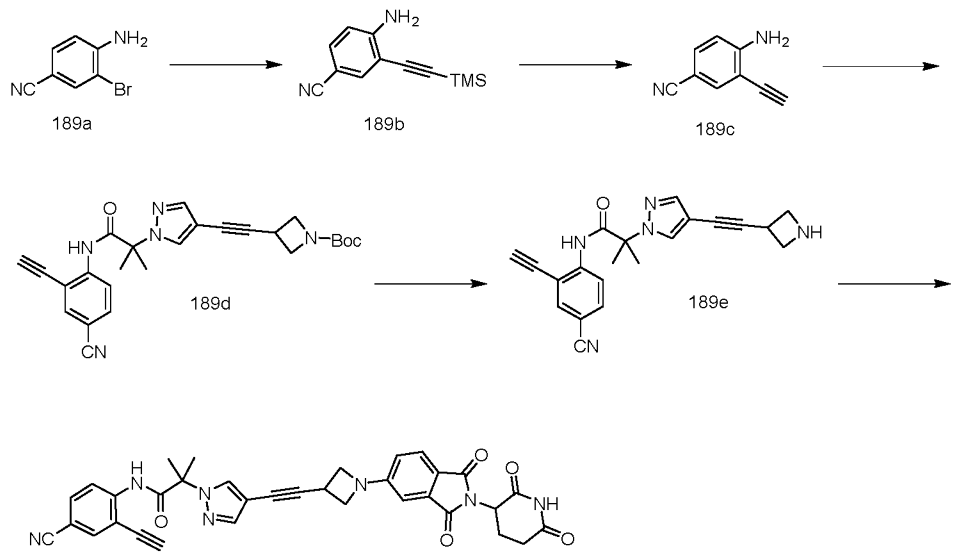
189a
189b
189c
189d
189e

Compound 189

Step 1: 4-amino-3-((trimethylsilyl)ethynyl)benzonitrile (189b)

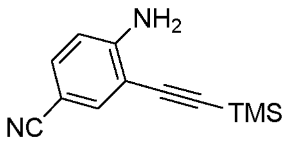

189a (0.82 g, 4.16 mmol), trimethylsilylacetylene (0.82 g, 8.34 mmol), TEA (1.27 g, 12.55 mmol), CuI (79 mg, 0.41 mmol) and $PdCl_2(PPh_3)_2$ (290 mg, 0.41 mmol) were added to a reaction bottle, 30 mL of toluene was added under nitrogen protection, nitrogen replacement was performed three times, and the mixture was reacted at 50°C for 18 h. The reaction liquid was cooled to room temperature, and the reaction system was concentrated under reduced pressure. The crude product was separated and purified with silica gel column chromatography (petroleum ether/ethyl acetate (v/v) = 10 : 1) to afford 189b (0.8 g, yield: 90%).

Step 2: 4-amino-3-ethynylbenzonitrile (189c)

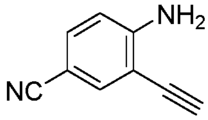

189b (0.4 g, 1.87 mmol) was dissolved in 10 mL of THF, TBAF·$3H_2O$ (0.89 g, 2.82 mmol) was added, and the mixture was stirred at room temperature for 30 min. The reaction system was concentrated under reduced pressure, and the crude product was separated and purified with silica gel column chromatography (petroleum ether/ethyl acetate (v/v) = 5 : 1) to afford 189c (0.17 g, yield: 64%).

Step 3: tert-butyl 3-((1-(1-((4-cyano-2-ethynylphenyl)amino)-2-methyl-1-oxopropan-2-yl)-1H- pyrazol-4-yl)ethynyl)azetidine-1-carboxylate (189d)

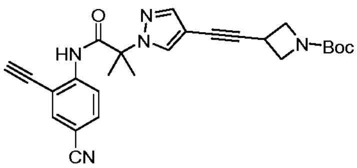

189c (0.17 g, 1.20 mmol) and 188d (0.48 g, 1.44 mmol) were dissolved in 15 mL of DCM, TCFH (0.51 g, 1.82 mmol) was added, and N-methylimidazole (0.39 g, 4.75 mmol) was slowly added dropwise, the mixture was reacted at room temperature for 16 h. The reaction system was concentrated under reduced pressure, and the crude product was separated and purified with silica gel chromatography column (petroleum ether/ethyl acetate (v/v) = 4 : 1) to afford 189d (0.42 g, yield: 76%).

Step 4: 2-(4-(azetidin-3-ylethynyl)-1H-pyrazol-1-yl)-N-(2-ethynyl-4-(trifluoromethyl)phenyl)-2-methylpropanamide (189e) 4-methylbenzenesulfonate

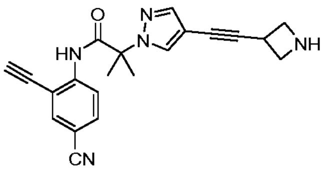

189d (200 mg, 0.437 mmol) was dissolved in 4 mL of acetonitrile, p-toluenesulfonic acid monohydrate (0.33 g,

1.74 mmol) was added, and the mixture was stirred at room temperature for 2 h. The reaction liquid was concentrated under reduced pressure to afford crude 4-methylbenzenesulfonate of 189e (0.55 g).

LC-MS m/z = 358.5 $[M+1]^+$.

Step 5: N-(4-cyano-2-ethynylphenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 189)

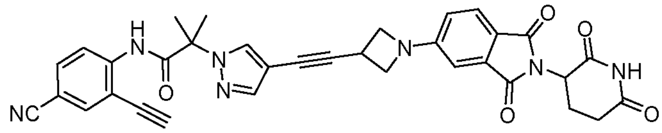

The 4-methylbenzenesulfonate of crude 189e (0.55 g) was dissolved in 5 mL of DMSO, TEA (0.44 g, 4.35 mmol) and 1C (0.24 g, 0.87 mmol) were added, and the mixture was reacted at 80°C for 3 h. The reaction liquid was cooled to room temperature, 40 mL of water was added, the mixture was filtered, the filter cake was collected, the filter cake was washed with 10 mL of water, the filter cake was dissolved with 50 mL of DCM, the mixture was dried over anhydrous sodium sulfate, and concentrated under reduced pressure. The crude product was separated and purified with silica gel chromatography column (petroleum ether/ethyl acetate (v/v) = 1 : 2) to afford compound 189 (60 mg, yield: 11%).

$^1H$ NMR (400 MHz, $CDCl_3$) δ 9.02 (s, 1H), 8.54 (d, 1H), 7.95 (s, 1H), 7.80 (s, 1H), 7.73 (s, 1H), 7.71 - 7.64 (m, 2H), 7.59 (dd, 1H), 6.84 - 6.78 (m, 1H), 6.56 (dd, 1H), 4.98 - 4.89 (m, 1H), 4.43 - 4.30 (m, 2H), 4.13 - 4.00 (m, 2H), 3.88 - 3.75 (m, 1H), 3.48 (s, 1H), 2.98 - 2.65 (m, 3H), 2.20 - 2.05 (m, 1H), 1.94 (s, 6H).

LCMS m/z = 614.2 $[M+1]^+$.

**Example 190: Preparation of compound 190**

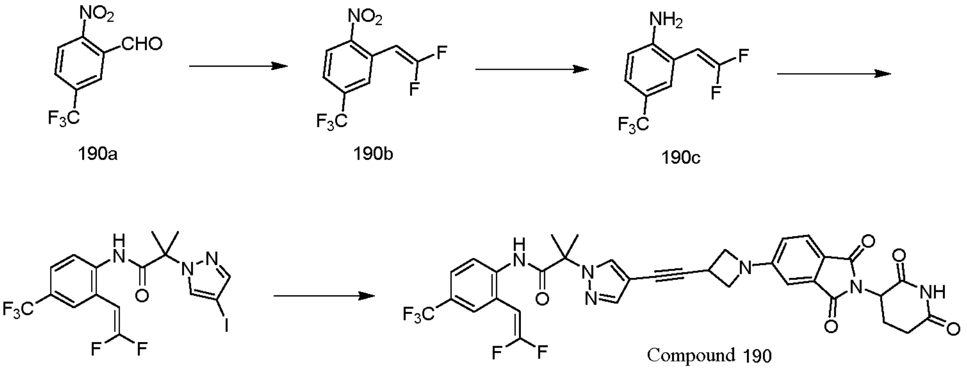

190a 190b 190c

190d Compound 190

Step 1: 2-(2,2-difluorovinyl)-1-nitro-4-(trifluoromethyl)benzene (190b)

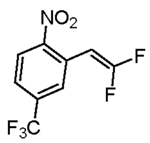

190a (4.38 g, 19.99 mmol), triphenylphosphine (15.73 g, 59.97 mmol) and NaI (5.99 g, 39.96 mmol) were added to 70 mL of acetonitrile, nitrogen replacement was performed three times, the mixture was reacted at 70°C for 30 min, and a solution of methyl 2,2-difluoro-2-(fluorosulfonyl)acetate (6.72 g, 34.98 mmol) in acetonitrile (20 mL) was slowly

added dropwise, nitrogen replacement was performed three times, and the mixture was reacted at 70°C for 3 h. The reaction liquid was cooled to room temperature, 80 mL of water was added, the mixture was extracted with 100 mL of ethyl acetate. The organic phase was washed with 50 mL of water, dried over anhydrous sodium sulfate and concentrated under reduced pressure. The crude product was separated and purified by silica gel chromatography column (petroleum ether) to afford 190b (0.2 g, yield: 4%).

Step 2: 2-(2,2-difluorovinyl)-4-(trifluoromethyl)aniline (190c)

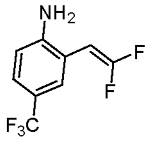

190b (0.2 g, 0.79 mmol) was dissolved in 5 mL of ethanol, 2 mL of saturated aqueous ammonium chloride solution and reduced iron powder (0.44 g, 7.86 mmol) were added, and the mixture was reacted at reflux for 2 h. The reaction system was cooled to room temperature and filtered, the filtrate was extracted with 50 mL of ethyl acetate, and concentrated under reduced pressure. The crude product was separated and purified with silica gel chromatography column (petroleum ether/ethyl acetate (v/v) = 3 : 1) to afford 190c (0.08 g, yield: 45%).
LCMS m/z = 224.2 $[M+1]^+$.

Step 3: N-(2-(2,2-difluorovinyl)-4-(trifluoromethyl)phenyl)-2-(4-iodo-1H-pyrazol-1-yl)-2-methylpropanamide (190d)

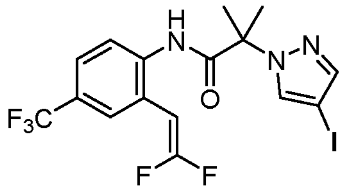

190c (0.08 g, 0.36 mmol) and 16a-1 (0.12 g, 0.43 mmol) were dissolved in 15 mL of DCM, TCFH (0.15 g, 0.53 mmol) was added, and N-methylimidazole (0.12 g, 1.46 mmol) was slowly added dropwise, the mixture was reacted at room temperature for 16 h. The reaction system was concentrated under reduced pressure, and the crude product was separated and purified with silica gel chromatography column (petroleum ether/ethyl acetate (v/v) = 5 : 1) to afford 190d (0.15 g, yield: 86%).
LCMS m/z = 486.4 $[M+1]^+$.

Step 4: N-(2-(2,2-difluorovinyl)-4-(trifluoromethyl)phenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 190)

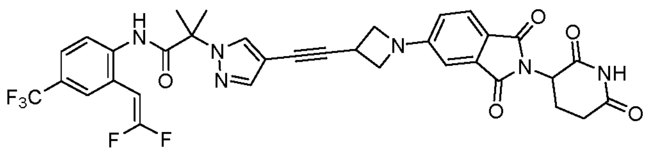

190d (0.15 g, 0.31 mmol), crude intermediate 1 (0.16 g), TEA (0.01 g, 0.099 mmol), CuI (6 mg, 0.031 mmol) and $PdCl_2(PPh_3)_2$ (22 mg, 0.031 mmol) were added to 5 mL of DMF under nitrogen protection, and the mixture was reacted at 50°C for 1 h. The reaction liquid was cooled to room temperature, 50 mL of water was added, and the mixture was filtered by suction. The filter cake was washed with 10 mL of water. The filter cake was dissolved in 100 mL of DCM, the mixture was dried over anhydrous sodium sulfate, and concentrated under reduced pressure. The crude product

was purified with silica gel column chromatography (petroleum ether/ethyl acetate (v/v) = 1 : 1) to afford compound 190 (0.065 g, yield: 30%).

$^1H$ NMR (400 MHz, $CDCl_3$) δ 8.90 (s, 1H), 8.22 (d, 1H), 7.96 (s, 1H), 7.84 - 7.76 (m, 2H), 7.67 (d, 1H), 7.58 - 7.47 (m, 2H), 6.86 - 6.77 (m, 1H), 6.56 (dd, 1H), 5.15 - 4.85 (m, 2H), 4.44 - 4.30 (m, 2H), 4.15 - 4.00 (m, 2H), 3.90 - 3.73 (m, 1H), 2.98 - 2.60 (m, 3H), 2.20 - 2.06 (m, 1H), 1.94 (s, 6H) .

LCMS m/z = 695.2 $[M+1]^+$.

**Example 191: Preparation of compound 191**

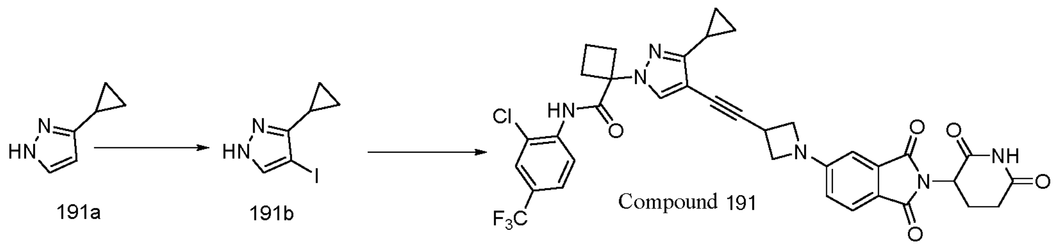


Step 1: 3-cyclopropyl-4-iodo-1H-pyrazole (191b)

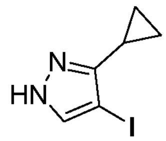

191a (1.5 g, 13.87 mmol) was added to a 100 mL three-mouth flask, 50% concentrated sulfuric acid (40 mL) was added, the mixture was cooled to 0°C, NIS (4.6 g, 20.45 mmol) was slowly added, and the mixture was reacted at room temperature for 16 h. The reaction system was slowly added to 50 mL of saturated aqueous sodium carbonate solution, the mixture was extracted with ethyl acetate (80 mL × 2), and the organic phase was washed with saturated sodium thiosulfate solution (80 mL × 2) and saturated aqueous sodium chloride solution (50 mL × 2), dried over anhydrous sodium sulfate, and concentrated under reduced pressure. The crude product was separated and purified with silica gel chromatography column (petroleum ether : ethyl acetate (v/v) = 81 : 19) to afford 191b (2.335 g, yield: 72%).

LCMS m/z = 235.1 $[M+1]^+$ .

Step 2; N-(2-chloro-4-(trifluoromethyl)phenyl)-1-(3-cyclopropyl-4-((1-(2-(2,6-dioxopiperidin-3-yl)-1,3-dioxoisoindolin-5-yl)azetidin-3-yl)ethynyl)-1H-pyrazol-1-yl)cyclobutane-1-carboxamide (Compound 191)

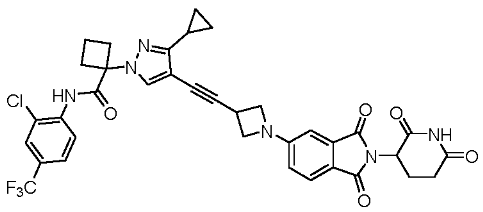

191b (0.8 g, 3.42 mmol) was added into a 100 mL single-mouth bottle, acetonitrile (20 mL) was added, and then ethyl 1-bromocyclobutane-1-carboxylate (698 mg, 3.37 mmol) and cesium carbonate (2.2 g, 6.75 mmol) were added in sequence, and the mixture was reacted at 90°C for 3 h. The reaction system was cooled to room temperature and filtered, and the filtrate was concentrated under reduced pressure. The crude product was separated and purified with silica gel chromatography column (petroleum ether : ethyl acetate (v/v) = 89 : 11) to afford crude 1 (0.9 g). Crude 1 (0.9 g) was dissolved in 15 mL of tetrahydrofuran, 5 mL of water was added, the mixture was cooled to 0°C, lithium hydroxide

monohydrate (315 mg, 7.5 mmol) was added, and the mixture was reacted at room temperature for 2 h. The pH of the reaction system was adjusted to 4 with 0.5 mol/L hydrochloric acid, the mixture was extracted with ethyl acetate (60 mL × 3), the organic phase was washed with 30 mL of saturated aqueous sodium chloride solution (60 mL × 2), dried over anhydrous sodium sulfate, and concentrated under reduced pressure, and the crude product was separated and purified with silica gel chromatography column (dichloromethane : methanol (v/v) = 95 : 5) to afford crude 2 (0.6 g). Crude 2 (300 mg) was added to a 50 mL single-mouth bottle, 15 mL of dichloromethane was added, 1-chloro-N,N,2-trimethylpropenylamine (180 mg, 1.34 mmol) was added, and the mixture was reacted at room temperature for 1 h, then triethylamine (272 mg, 2.69 mmol) and 2-chloro-4-(trifluoromethyl)aniline (195 mg, 0.997 mmol) were added in sequence, and the mixture was reacted at room temperature for 1 h. The reaction liquid was concentrated under reduced pressure, and the crude product was separated and purified with silica gel chromatography column (petroleum ether : ethyl acetate (v/v) = 87 : 13) to afford crude 3 (200 mg). Crude 3 (200 mg) was added to a 50 mL single-mouth bottle, 15 mL of DMF was added, crude intermediate 1 (198 mg) and TEA (118 mg, 1.17 mmol) were added, nitrogen replacement was performed three times, $PdCl_2(PPh_3)_2$ (28 mg, 0.04 mmol) and CuI (15 mg, 0.079 mmol) were added, nitrogen replacement was performed three times, and the mixture was reacted at 50°C for 2 h. The reaction system was cooled to room temperature, 150 mL of saturated aqueous ammonium chloride solution was slowly added, the mixture was extracted with ethyl acetate (80 mL × 3), and the organic phase was washed with saturated aqueous sodium chloride solution (70 mL × 2), dried over anhydrous sodium sulfate, and concentrated under reduced pressure. The crude product was separated and purified with silica gel chromatography column (petroleum ether : ethyl acetate (v/v) = 43 : 57) to afford compound 191 (180 mg, yield: 15%).

$^1H$ NMR (400 MHz, $CDCl_3$) δ 8.81 (s, 1H), 8.54 (d, 1H), 7.98 (s, 1H), 7.74 - 7.46 (m, 4H), 6.85 - 6.77 (m, 1H), 6.55 (dd, 1H), 4.99 - 4.88 (m, 1H), 4.43 - 4.30 (m, 2H), 4.13 - 4.00 (m, 2H), 3.92 - 3.77 (m, 1H), 3.09 - 2.61 (m, 7H), 2.27 - 1.96 (m, 4H), 1.14 - 1.06 (m, 2H), 1.02 - 0.93 (m, 2H).

LCMS m/z = 719.1 $[M+1]^+$.

**Example 192: Preparation of compound 192**

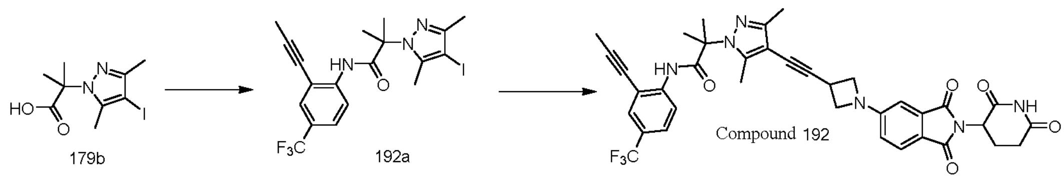

179b 192a Compound 192

Step 1: 2-(4-iodo-3,5-dimethyl-1H-pyrazol-1-yl)-2-methyl-N-(2-(prop-1-yn-1-yl)-4-(trifluoromethyl)phenyl)propanamide (192a)

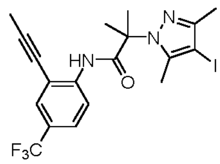

179b (0.34 g, 1.10 mmol) was dissolved in 10 mL of dichloromethane, 1-chloro-N,N,2-trimethylpropenylamine (0.20 g, 1.49 mmol) was added, the mixture was stirred at room temperature for 2 h, and then 175B (0.20 g, 1.00 mmol) and TEA (0.30 g, 2.96 mmol) were added in sequence, and the mixture was reacted at room temperature for 12 h. 10 mL of ethyl acetate and 10 mL purified water were added to the reaction system, the organic phase was separated, the organic phase was washed with 10 mL of saturated aqueous sodium chloride solution, dried over anhydrous sodium sulfate, and concentrated under reduced pressure. The crude product was separated and purified with silica gel chromatography column (petroleum ether : ethyl acetate (v/v) = 5 : 1) to afford 192a (0.26 g, yield: 53%).

Step 2: 2-(4-((1-(2-(2,6-dioxopiperidin-3-yl)-1,3-dioxoisoindolin-5-yl)azetidin-3-yl)ethynyl)-3,5-dimethyl-1H-pyrazol-1-yl)-2-methyl-N-(2-(prop-1-yn-1-yl)-4-(trifluoromethyl)phenyl)propanamide (Compound 192)

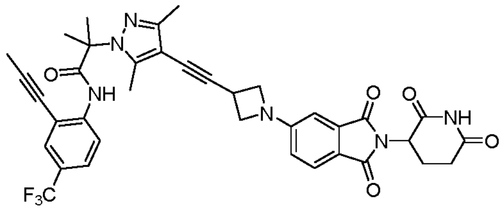

192a (0.26 g, 0.53 mmol) was dissolved in 6 mL of DMF, and crude intermediate 1 (0.21 g), TEA (0.16 g, 1.58 mmol), CuI (0.020 g, 0.105 mmol) and $PdCl_2(PPh_3)_2$ (0.037 g, 0.053 mmol) were added in sequence under nitrogen protection, and the mixture was reacted at 60°C for 2 h. The reaction liquid was cooled to room temperature, 15 mL of dichloromethane and 15 mL of purified water were added, the organic phase was separated, the aqueous phase was extracted with dichloromethane (15 mL × 3), the organic phases were combined, and the organic phase was washed with 15 mL of saturated aqueous sodium chloride solution, dried over anhydrous sodium sulfate, and concentrated under reduced pressure. The crude product was separated and purified with silica gel chromatography column (petroleum ether : ethyl acetate (v/v) = 1 : 1) to afford compound 192 (55 mg, yield: 15%).

$^1$H NMR (400 MHz, $CDCl_3$) δ 8.47 (d, 1H), 8.11 (s, 1H), 7.97 (s, 1H), 7.73 - 7.47 (m, 3H), 6.84 - 6.76 (m, 1H), 6.56 (dd, 1H), 4.99 - 4.88 (m, 1H), 4.45 - 4.32 (m, 2H), 4.10 - 3.97 (m, 2H), 3.94 - 3.80 (m, 1H), 2.99 - 2.64 (m, 3H), 2.30 (s, 3H), 2.26 (s, 3H), 2.17 - 2.07 (m, 1H), 1.96 (s, 3H), 1.88 (s, 6H).

LCMS m/z = 699.2 $[M+1]^+$.

**Example 193: Preparation of compounds 193 and 194**

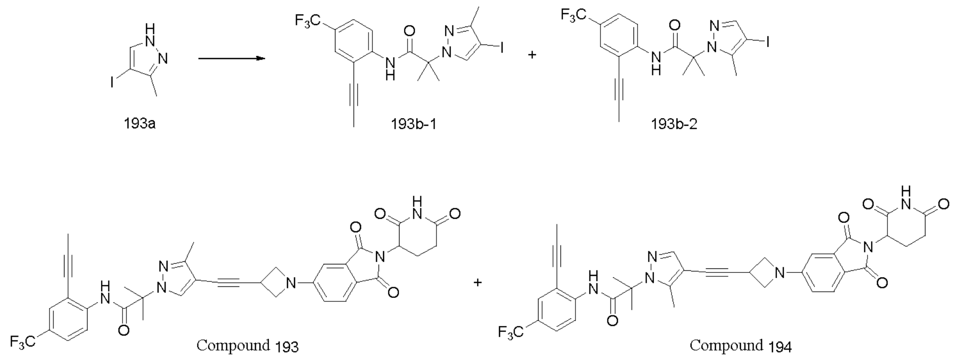

193a

193b-1

193b-2

Compound 193

Compound 194

Step 1: 2-(4-iodo-3-methyl-1H-pyrazol-1-yl)-2-methyl-N-(2-(prop-1-yn-1-yl)-4-(trifluoromethyl)phenyl)propanamide (193b-1)

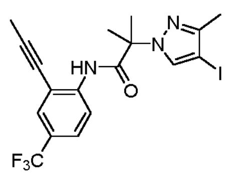

2-(4-iodo-5-methyl-1H-pyrazol-1-yl)-2-methyl-N-(2-(prop-1-yn-1-yl)-4-(trifluoromethyl)phenyl)propanamide (193b-2)

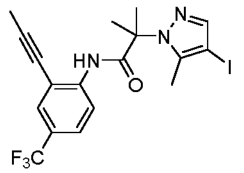

193a (4.00 g, 19.23 mmol), 2-bromo-2-methylpropionic acid (3.20 g, 19.16 mmol), TEA (5.83 g, 57.61 mmol) and 50 mL of ethyl acetate were added to a reaction flask respectively, and the mixture was reacted at 50°C for 3 h. The reaction system was cooled to room temperature, 1 mol/L hydrochloric acid was added at 0°C to adjust the pH to 2, the mixture was extracted with ethyl acetate (100 mL × 3), the organic phase was dried over anhydrous sodium sulfate, and concentrated under reduced pressure to afford a crude product (5.5 g). The above crude product (0.49 g) was dissolved in 10 mL of dichloromethane, 1-chloro-N,N,2-trimethylpropenylamine (0.30 g, 2.24 mmol) was added, the mixture was reacted at room temperature for 2 h, and then 175B (0.30 g, 1.51 mmol) and triethylamine (0.46 g, 4.55 mmol) were added in sequence, and the mixture was reacted at room temperature for 12 h. 10 mL of ethyl acetate and 10 mL of purified water were added to the reaction system, the organic phase was separated, the organic phase was washed with 10 mL of saturated aqueous sodium chloride solution, dried over anhydrous sodium sulfate, and concentrated under reduced pressure. The crude product was separated and purified with silica gel chromatography column (petroleum ether : ethyl acetate (v/v) = 5 : 1) to afford a mixture of 193b-1 and 193b-2 (0.29 g).

Step 2: 2-(4-((1-(2-(2,6-dioxopiperidin-3-yl)-1,3-dioxoisoindolin-5-yl)azetidin-3-yl)ethynyl)-3-methyl-1H-pyrazol-1-yl)-2-methyl-N-(2-(prop-1-yn-1-yl)-4-(trifluoromethyl)phenyl)propanamide (Compound 193)

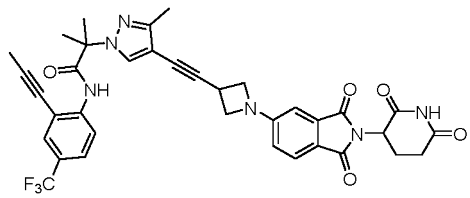

2-(4-((1-(2-(2,6-dioxopiperidin-3-yl)-1,3-dioxoisoindolin-5-yl)azetidin-3-yl)ethynyl)-5-methyl-1H-pyrazol-1-yl)-2-methyl-N-(2-(prop-1-yn-1-yl)-4-(trifluoromethyl)phenyl)propanamide (Compound 194)

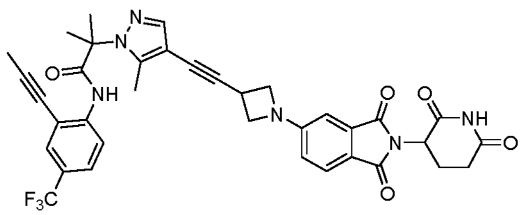

The mixture of crude 193b-1 and 193b-2 (0.29 g) was dissolved in 6 mL of DMF, and crude intermediate 1 (0.25 g), TEA (0.19 g, 1.88 mmol), CuI (0.023 g, 0.12 mmol) and $PdCl_2(PPh_3)_2$ (0.043 g, 0.061 mmol) were added in sequence, and the mixture was reacted at 60°C for 2 h. The reaction system was cooled to room temperature, 15 mL of dichloromethane and 15 mL of purified water were added, the organic phase was separated, the aqueous phase was washed with dichloromethane (15 mL × 3), the organic phases were combined, and the organic phase was washed with 10 mL of saturated aqueous sodium chloride solution, dried over anhydrous sodium sulfate, and concentrated under reduced pressure. The crude product was separated and purified with silica gel chromatography column (petroleum ether : ethyl acetate (v/v) = 1 : 1) to afford a crude product. The crude product was subjected to Pre-HPLC (instrument and preparative column: using Waters 2767 preparative liquid phase chromatographic instrument, preparative column model: SunFire@Prep C18, 5 μm, inner diameter × length = 19 mm × 250 mm). Preparation method: the solution of the crude product in DMF was filtered with a 0.45 μm filter membrane to prepare a sample liquid. Mobile phase system: water (containing 5 mmol/L ammonium acetate)/acetonitrile. Gradient elution method: gradient elution of 40% to 80%

acetonitrile (elution time: 40 min). The preparation liquid was extracted with 50 mL of ethyl acetate. The organic phase was washed with 20 mL of saturated aqueous sodium chloride solution, dried over anhydrous sodium sulfate and concentrated under reduced pressure to afford compound 193 (40 mg, two-step yield from compound 193a: 3%) and compound 194 (20 mg, two-step yield from compound 193a: 2%), respectively.

Nuclear magnetic resonance spectrum of compound 193:

$^1$H NMR (400 MHz, $CDCl_3$) $\delta$ 8.75 (s, 1H), 8.44 (d, 1H), 7.94 (s, 1H), 7.75 - 7.65 (m, 2H), 7.60 - 7.54 (m, 1H), 7.52 - 7.45 (m, 1H), 6.84 - 6.77 (m, 1H), 6.56 (dd, 1H), 4.99 - 4.88 (m, 1H), 4.44 - 4.31 (m, 2H), 4.10 - 3.97 (m, 2H), 3.90 - 3.76 (m, 1H), 2.98 - 2.65 (m, 3H), 2.35 (s, 3H), 2.18 - 2.08 (m, 4H), 1.90 (s, 6H).
LCMS m/z = 685.2 $[M+1]^+$.

Nuclear magnetic resonance spectrum of compound 194:

$^1$H NMR (400 MHz, $CDCl_3$) $\delta$ 8.49 (d, 1H), 8.00 (s, 1H), 7.93 (s, 1H), 7.67 (d, 1H), 7.61 (s, 1H), 7.58 - 7.48 (m, 2H), 6.84 - 6.76 (m, 1H), 6.55 (dd, 1H), 4.98 - 4.89 (m, 1H), 4.42 - 4.32 (m, 2H), 4.10 - 4.00 (m, 2H), 3.90 - 3.76 (m, 1H), 2.99 - 2.65 (m, 3H), 2.31 (s, 3H), 2.18 - 2.06 (m, 1H), 2.00 (s, 3H), 1.92 (s, 6H).
LCMS m/z = 685.2 $[M+1]^+$.

**Example 195: Preparation of compounds 195 and 196**

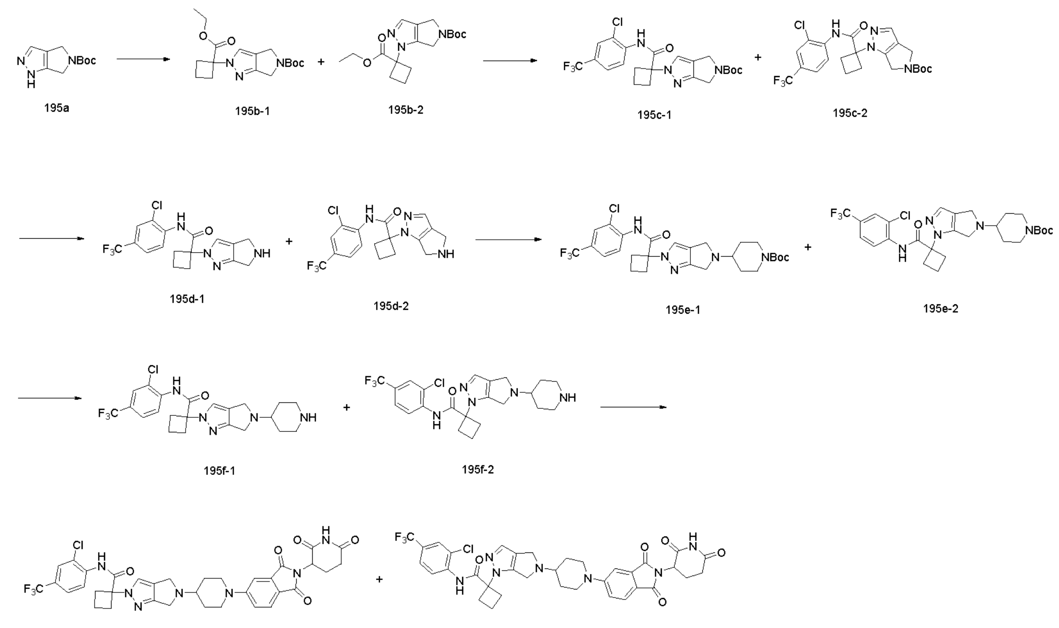

195a, 195b-1, 195b-2, 195c-1, 195c-2, 195d-1, 195d-2, 195e-1, 195e-2, 195f-1, 195f-2, Compound 195, Compound 196

Step 1: tert-butyl 2-(1-(ethoxycarbonyl)cyclobutyl)-2,6-dihydropyrrolo[3,4-c]pyrazole- 5(4H)-carboxylate (195b-1)

tert-butyl 1-(1-(ethoxycarbonyl)cyclobutyl)-4,6-dihydropyrrolo[3,4-c]pyrazole- 5(1H)-carboxylate (195b-2)

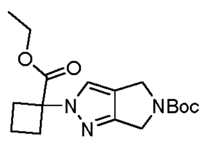

195b-1

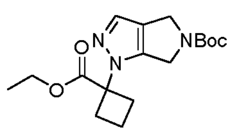

195b-2

195a (10.5 g, 50 mmol) was added to 100 mL of DMF, ethyl 1-bromocyclobutane-1-carboxylate (15.4 g, 74.37 mmol) and cesium carbonate (32.5 g, 0.1 mol) were added, and the mixture was reacted at 90°C for 3 h. The reaction liquid was cooled to room temperature, 200 mL of ethyl acetate was added, the organic phase was washed with 500 mL of purified water, dried over anhydrous sodium sulfate, and concentrated under reduced pressure, and the crude product was separated and purified with silica gel chromatography column (petroleum ether : ethyl acetate (v/v) = 4 : 1) to afford a mixture of 195b-1 and 195b-2 (6.0 g).

Step 2: tert-butyl 2-(1-((2-chloro-4-(trifluoromethyl)phenyl)carbamoyl) cyclobutyl)-2,6-d ihydropyrrolo[3,4-c]pyrazole-5(4H)-carboxylate (195c-1)

tert-butyl 1-(1-((2-chloro-4-(trifluoromethyl)phenyl)carbamoyl)cyclobutyl)-4,6- dihydropyrrolo[3,4-c]pyrazole-5(1H)-carboxylate (195c-2)

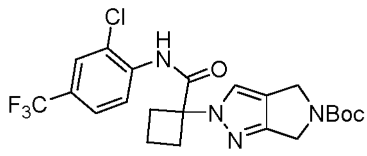

195c-1

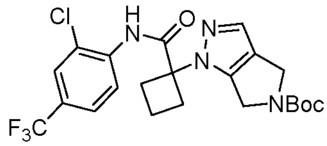

195c-2

The mixture of 195b-1 and 195b-2 (6.0 g) was added to 60 mL of THF, 20 mL of purified water and lithium hydroxide monohydrate (3.4 g, 81 mmol) were added, and the mixture was reacted at room temperature for 3 h. The reaction liquid was concentrated under reduced pressure, the pH of the system was adjusted to 5 with 2 mol/L hydrochloric acid, the mixture was extracted with 100 mL of dichloromethane, the organic phase was dried over anhydrous sodium sulfate and concentrated under reduced pressure to afford a crude product (5.3 g). The above crude product (5.3 g) and 2-chloro-4-(trifluoromethyl)aniline (3.5 g, 18 mmol) were dissolved in 100 mL of DCM, TCFH (7.56 g, 26.94 mmol) was added, and N-methylimidazole (5.6 g, 68.20 mmol) was slowly added dropwise, and the mixture was reacted at room temperature for 16 h. The reaction system was concentrated under reduced pressure, and the crude product was separated and purified with silica gel chromatography column (petroleum ether/ethyl acetate (v/v) = 4 : 1) to afford a mixture of 195c-1 and 195c-2 (6.7 g).

Step 3: N-(2-chloro-4-(trifluoromethyl)phenyl)-1-(5,6-dihydropyrrolo[3,4-c]pyrazol-2(4H)-yl)cyclobutane-1-carboxamide (195d-1)

N-(2-chloro-4-(trifluoromethyl)phenyl)-1-(5,6-dihydropyrrolo[3,4-c]pyrazol-1(4H)-yl)cyclobutane-1-carboxamide (195d-2)

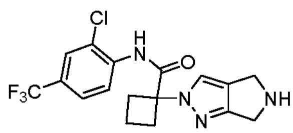

195d-1

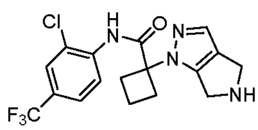

195d-2

The mixture of 195c-1 and 195c-2 (10.0 g) was dissolved in 120 mL of dichloromethane, and 40 mL of trifluoroacetic acid was added. The mixture was reacted at room temperature for 1 h. The reaction system was concentrated under reduced pressure, 200 mL of dichloromethane was added, and the pH was adjusted to 9 with saturated aqueous sodium carbonate solution. The organic phase was dried over anhydrous sodium sulfate and concentrated under reduced pressure to afford a mixture of 195d-1 and 195d-2 (7.2 g).

Step 4: tert-butyl 4-(2-(1-((2-chloro-4-(trifluoromethyl)phenyl)carbamoyl) cyclobutyl)-2,6-dihydropyrrolo[3,4-c]pyrazol-5(4H)-yl)piperidine-1-carboxylate (195e-1)

tert-butyl 4-(1-(1-((2-chloro-4-(trifluoromethyl)phenyl)carbamoyl)cyclobutyl)-4,6-dihydropyrrolo[3,4-c]pyrazol-5(1H)-yl)piperidine-1-carboxylate (195e-2)

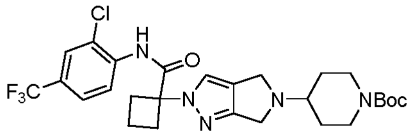

195e-1

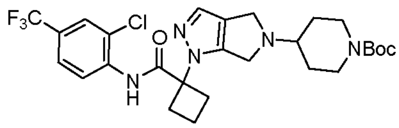

195e-2

The mixture of 195d-1 and 195d-2 (1.5 g) was dissolved in 60 mL of 1,2-dichloroethane, tert-butyl 4-oxopiperidine-1-carboxylate (1.6 g, 8.0 mmol) was added, the mixture was reacted at room temperature for 2 h, then sodium triacetoxyborohydride (1.7 g, 8.0 mmol) was added and the mixture was reacted at room temperature for 15 h. The reaction liquid was concentrated under reduced pressure, and the crude product was separated and purified with silica gel chromatography column (dichloromethane : methanol (v/v) = 10 : 1) to afford a mixture of 195e-1 and 195e-2 (0.9 g).

Step 5: N-(2-chloro-4-(trifluoromethyl)phenyl)-1-(5-(piperidin-4-yl)-5,6-dihydropyrrolo[3,4-c]pyrazol-2(4H)-yl)cyclobutane-1-carboxamide (195f-1)

N-(2-chloro-4-(trifluoromethyl)phenyl)-1-(5-(piperidin-4-yl)-5,6-dihydropyrrolo[3,4-c]pyrazol-1(4H)-yl)cyclobutane-1-carboxamide (195f-2)

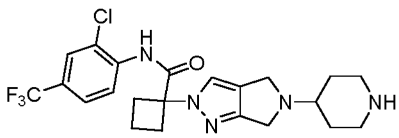

195f-1

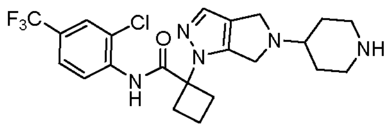

195f-2

In a 50 mL reaction bottle, the mixture of 195e-1 and 195e-2 (0.90 g) was dissolved in 10 mL of dichloromethane, and 10 mL of trifluoroacetic acid was added. The mixture was reacted at 25°C for 2 h. The reaction liquid was concentrated under reduced pressure, and the pH of the residue was adjusted to 9 with 1 mol/L aqueous sodium hydroxide solution, and the mixture was extracted with dichloromethane (50 mL × 3). The organic phase was dried over anhydrous sodium sulfate, and concentrated under reduced pressure to afford a mixture of crude 195f-1 and 195f-2 (0.63 g).

Step 6: N-(2-chloro-4-(trifluoromethyl)phenyl)-1-(5-(1-(2-(2,6-dioxopiperidin-3-yl)-1,3-dioxoisoindolin-5-yl)piperidin-4-yl)-5,6-dihydropyrrolo[3,4-c]pyrazol-2(4H)-yl)cyclobutane-1-carboxamide (Compound 195)

N-(2-chloro-4-(trifluoromethyl)phenyl)-1-(5-(1-(2-(2,6-dioxopiperidin-3-yl)-1,3-dioxoisoindolin-5-yl)piperidin-4-yl)-5,6-dihydropyrrolo[3,4-c]pyrazol-1(4H)-yl)cyclobutane-1-carboxamide (Compound 195)

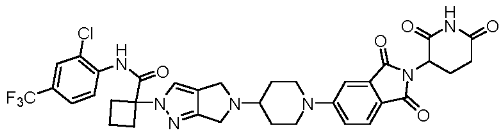

Compound 195

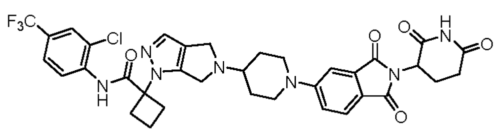

Compound 196

In a 50 mL reaction bottle, the mixture of crude 195f-1 and 195f-2 (0.63 g), 1C (0.45 g, 1.63 mmol), DIPEA (0.3 g, 2.32 mmol) and 30 mL of DMSO were added in sequence, and the mixture was reacted at 90°C for 3 h. The reaction liquid was cooled to room temperature, 200 mL of ethyl acetate was added, the organic phase was washed with water (100 mL × 3) and 50 mL of saturated aqueous sodium chloride solution, dried over anhydrous sodium sulfate, and concentrated under reduced pressure, and the crude product was separated and purified with silica gel chromatography column (dichloromethane/methanol (v/v) = 20 : 1) to afford a mixture of compounds 195 and 196, and the mixture was further purified with Prep-TLC (dichloromethane/methanol (v/v) = 40 : 1) to afford compound 195 (45 mg) and compound 196 (53 mg), respectively.

Nuclear magnetic resonance spectrum of compound 195:

$^1$H NMR (400 MHz, $CD_3OD$) δ 8.48 (d, 1H), 7.75 - 7.55 (m, 4H), 7.41 - 7.34 (m, 1H), 7.24 (dd, 1H), 5.11 - 5.01 (m, 1H), 4.14 - 3.82 (m, 6H), 3.20 - 2.62 (m, 10H), 2.30 - 2.00 (m, 5H), 1.80 - 1.57 (m, 2H).
LCMS m/z = 724.8 $[M+1]^+$.

Nuclear magnetic resonance spectrum of compound 196:

$^1$H NMR (400 MHz, $CD_3OD$) δ 8.46 (d, 1H), 7.75 - 7.55 (m, 3H), 7.50 (s, 1H), 7.38 - 7.30 (m, 1H), 7.21 (dd, 1H), 5.10 - 5.01 (m, 1H), 4.11 - 3.80 (m, 6H), 3.20 - 2.60 (m, 10H), 2.32 - 1.98 (m, 5H), 1.70 - 1.50 (m, 2H).
LCMS m/z = 724.7 $[M+1]^+$.

**Example 197: Preparation of compounds 197 and 198**

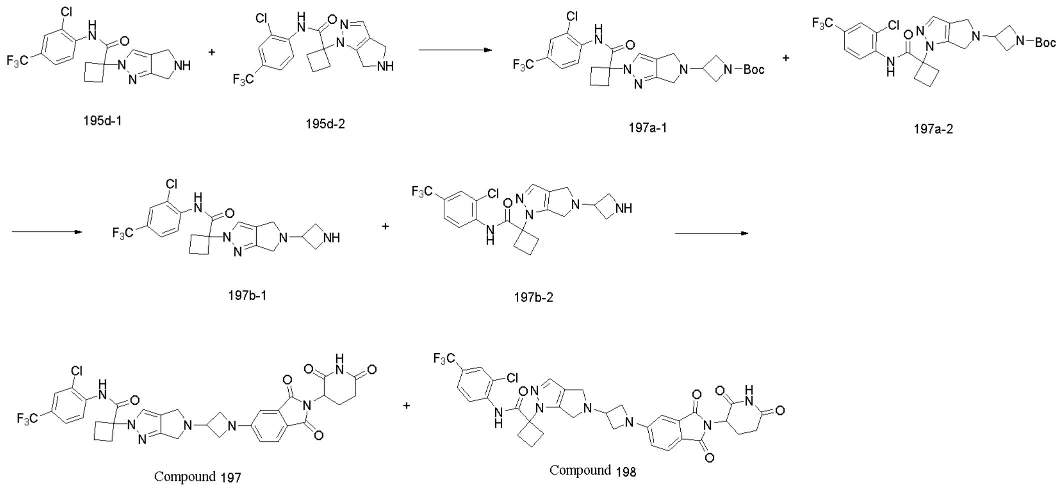

195d-1 195d-2 197a-1 197a-2

197b-1 197b-2

Compound 197 Compound 198

Step 1: tert-butyl 3-(2-(1-((2-chloro-4-(trifluoromethyl)phenyl)carbamoyl) cyclobutyl)-2,6-dihydropyrrolo[3,4-c]pyrazol-5(4H)-yl)azetidine-1-carboxylate(197a-1)

tert-butyl 3-(1-(1-((2-chloro-4-(trifluoromethyl)phenyl)carbamoyl)cyclobutyl)-4,6-dihydropyrrolo[3,4-c]pyrazol-5(1H)-yl)azetidine-1-carboxylate (197a-2)

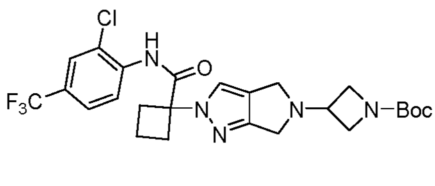

197a-1

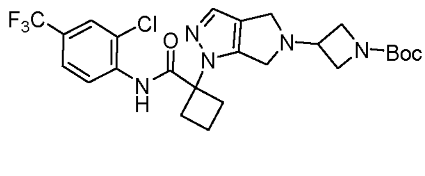

197a-2

The mixture of 195d-1 and 195d-2 (1.5 g) was dissolved in 60 mL of 1,2-dichloroethane, tert-butyl 3-oxoazetidine-1-carboxylate (1.37 g, 8.0 mmol) was added, the mixture was reacted at room temperature for 2 h, then sodium triacetoxyborohydride (1.7 g, 8.0 mmol) was added and the mixture was reacted at room temperature for 15 h. The reaction liquid was concentrated under reduced pressure, and the crude product was separated and purified with silica gel chromatography column (dichloromethane : methanol (v/v) = 10 : 1) to afford a mixture of 197a-1 and 197a-2 (1.5 g).

Step 2: 1-(5-(azetidin-3-yl)-5,6-dihydropyrrolo[3,4-c]pyrazol-2(4H)-yl)-N-(2-chloro-4-(trifluoromethyl)phenyl)cyclobutane-1-carboxamide (197b-1)

1-(5-(azetidin-3-yl)-5, 6-dihydropyrrolo[3,4-c]pyrazol-1 (4H)-yl)-N-(2-chloro-4-(trifluoromethyl)phenyl)cyclobutane-1-carboxamide (197b-2)

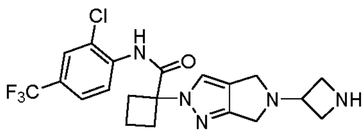

197b-1

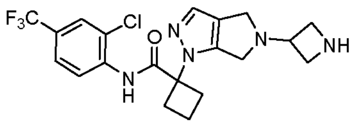

197b-2

In a 50 mL reaction bottle, the mixture of 197a-1 and 197a-2 (1.50 g) was dissolved in 10 mL of dichloromethane, and 10 mL of trifluoroacetic acid was added. The mixture was reacted at 25°C for 2 h. The reaction liquid was concentrated under reduced pressure, and the pH of the residue was adjusted to 9 with 1 mol/L aqueous sodium hydroxide solution, and the mixture was extracted with dichloromethane (50 mL × 3). The organic phase was dried over anhydrous sodium sulfate, and concentrated under reduced pressure to afford a mixture of crude 197b-1 and 197b-2 (1.08 g).

Step 3: N-(2-chloro-4-(trifluoromethyl)phenyl)-1-(5-(1-(2-(2,6-dioxopiperidin-3-yl)-1,3-dioxoisoindolin-5-yl)azetidin-3-yl)-5,6-dihydropyrrolo[3,4-c]pyrazol-2(4H)-yl)cyclobutane-1-carboxamide (Compound 197)

N-(2-chloro-4-(trifluoromethyl)phenyl)-1-(5-(1-(2-(2,6-dioxopiperidin-3-yl)-1,3-dioxoisoindolin-5-yl)azetidin-3-yl)-5,6-dihydropyrrolo[3,4-c]pyrazol-1(4H)-yl)cyclobutane-1-carboxamide (Compound 198)

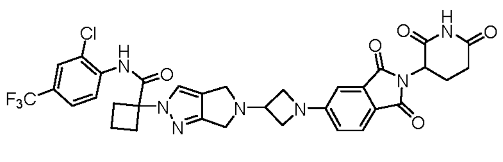

Compound 197

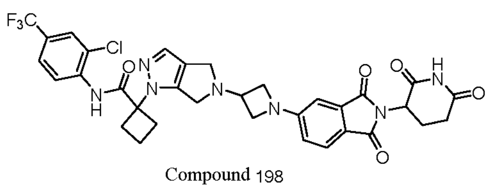

Compound 198

In a 50 mL reaction bottle, the mixture of crude 197b-1 and 197b-2 (1.08 g), 1C (0.82 g, 2.97 mmol), DIPEA (0.64 g, 4.95 mmol) and 30 mL of DMSO were added in sequence, and the mixture was reacted at 90°C for 3 h. The reaction liquid was cooled to room temperature, 200 mL of ethyl acetate was added, the organic phase was washed with water (100 mL × 3) and 50 mL of saturated aqueous sodium chloride solution, dried over anhydrous sodium sulfate, and concentrated under reduced pressure, and the crude product was separated and purified with silica gel chromatography column (dichloromethane/methanol (v/v) = 20 : 1) to afford a mixture of compounds 197 and 198, and the mixture

was further purified with Prep-TLC (dichloromethane/methanol (v/v) = 40 : 1) to afford compound 197 (41 mg) and compound 198 (48 mg), respectively.

Nuclear magnetic resonance spectrum of compound 197:

$^1H$ NMR (400 MHz, $CD_3OD$) δ 8.48 (d, 1H), 7.75 - 7.56 (m, 4H), 6.90 - 6.82 (m, 1H), 6.70 (dd, 1H), 5.10 - 4.99 (m, 1H), 4.30 - 4.17 (m, 2H), 4.13 - 4.01 (m, 3H), 4.00 - 3.87 (m, 4H), 3.10 - 2.62 (m, 7H), 2.28 - 1.99 (m, 3H).
LCMS m/z = 696.7 $[M+1]^+$.

Nuclear magnetic resonance spectrum of compound 198:

$^1H$ NMR (400 MHz, $CD_3OD$) δ 8.45 (d, 1H), 7.75 - 7.56 (m, 3H), 7.51 (s, 1H), 6.88 - 6.79 (m, 1H), 6.66 (dd, 1H), 5.10 - 4.99 (m, 1H), 4.29 - 3.82 (m, 9H), 3.04 - 2.60 (m, 7H), 2.31 - 2.00 (m, 3H).
LCMS m/z = 696.7 $[M+H]^+$.

**Example 199: Preparation of compounds 199 and 200**

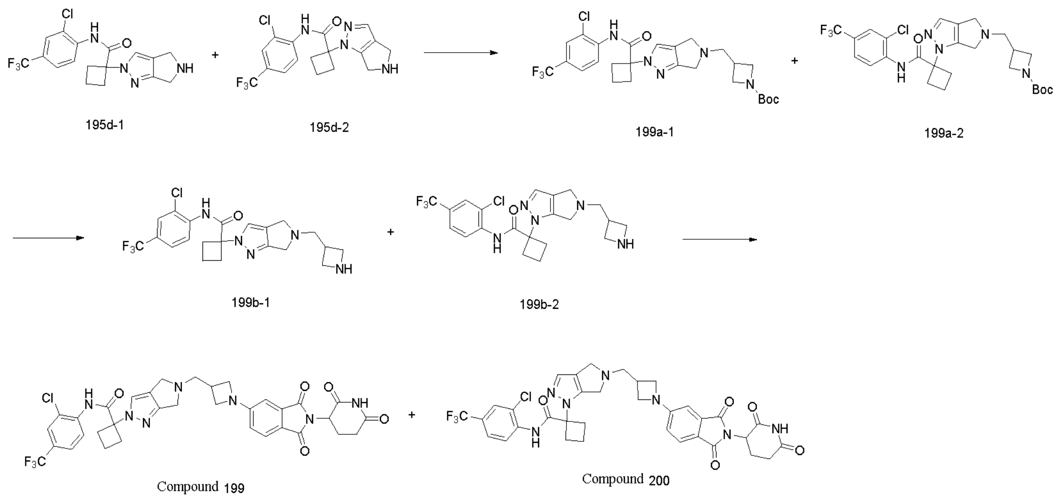

195d-1

195d-2

199a-1

199a-2

199b-1

199b-2

Compound 199

Compound 200

Step 1: tert-butyl 3-((2-(1-((2-chloro-4-(trifluoromethyl)phenyl)carbamoyl) cyclobutyl)-2,6-dihydropyrrolo[3,4-c]pyrazol-5(4H)-yl)methyl)azetidine-1-carboxylate (199a-1)

tert-butyl 3-((1-(1-((2-chloro-4-(trifluoromethyl)phenyl)carbamoyl)cyclobutyl)-4,6- dihydropyrrolo[3,4-c]pyrazol-5(1H)-yl)methyl)azetidine-1-carboxylate (199a-2)

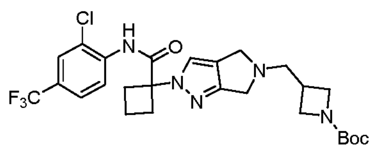

199a-1

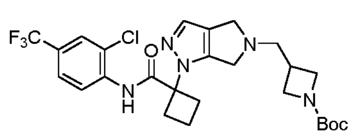

199a-2

The mixture of 195d-1 and 195d-2 (1.5 g, 3.9 mmol) was dissolved in 60 mL of 1,2-dichloroethane, tert-butyl 3-formylazetidine-1-carboxylate (1.5 g, 8.1 mmol) was added, the mixture was reacted at room temperature for 2 h, then sodium triacetoxyborohydride (1.7 g, 8.0 mmol) was added and the mixture was reacted at room temperature for 15 h. The reaction liquid was concentrated under reduced pressure, and the crude product was separated and purified with silica gel chromatography column (dichloromethane : methanol (v/v) = 10 : 1) to afford a mixture of 199a-1 and 199a-2 (0.9 g).

Step 2: 1-(5-(azetidin-3-ylmethyl)-5,6-dihydropyrrolo[3,4-c]pyrazol-2(4H)-yl)-N-(2-chloro-4-(trifluoromethyl)phenyl)cyclobutane-1-carboxamide (199b-1)

1-(5-(azetidin-3-ylmethyl)-5,6-dihydropyrrolo[3,4-c]pyrazol-1(4H)-yl)-N-(2-chloro-4-(trifluoromethyl)phenyl)cyclobutane-1-carboxamide (199b-2)

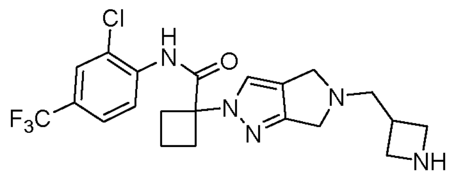

199b-1

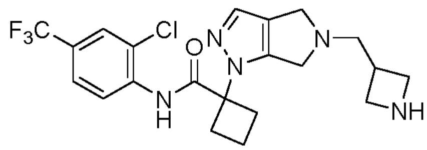

199b-2

In a 50 mL reaction bottle, the mixture of 199a-1 and 199a-2 (0.55 g) was dissolved in 10 mL of dichloromethane, and 10 mL of trifluoroacetic acid was added. The mixture was reacted at 25°C for 2 h. The reaction liquid was concentrated under reduced pressure, and the pH of the residue was adjusted to 9 with 1 mol/L aqueous sodium hydroxide solution, and the mixture was extracted with dichloromethane (30 mL × 3). The organic phase was dried over anhydrous sodium sulfate, and concentrated under reduced pressure to afford a mixture of crude compounds 199b-1 and 199b-2 (0.45 g).

Step 3: N-(2-chloro-4-(trifluoromethyl)phenyl)-1-(5-((1-(2-(2,6-dioxopiperidin-3-yl)-1,3-dioxoisoindolin-5-yl)azetidin-3-yl)methyl)-5,6-dihydropyrrolo[3,4-c]pyrazol-2(4H)-yl)cyclobutane-1-carboxamide (Compound 199)

N-(2-chloro-4-(trifluoromethyl)phenyl)-1-(5-((1-(2-(2,6-dioxopiperidin-3-yl)-1,3-dioxoisoindolin-5-yl)azetidin-3-yl)methyl)-5,6-dihydropyrrolo[3,4-c]pyrazol-1(4H)-yl)cyclobutane-1-carboxamide (Compound 200)

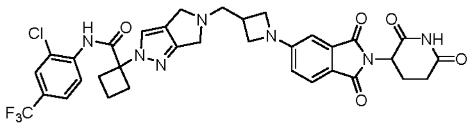

Compound 199

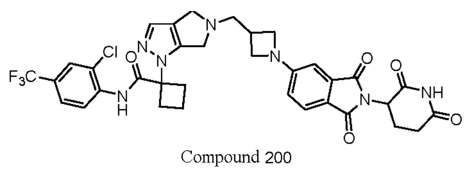

Compound 200

In a 50 mL reaction bottle, the mixture of crude compounds 199b-1 and 199b-2 (0.45 g), 1C (0.33 g, 1.20 mmol), DIPEA (0.26 g, 2.01 mmol) and 10 mL of DMSO were added in sequence, and the mixture was reacted at 90°C for 3 h. The reaction liquid was cooled to room temperature, 100 mL of ethyl acetate was added, the organic phase was washed with water (100 mL × 3) and 50 mL of saturated aqueous sodium chloride solution, dried over anhydrous sodium sulfate, and concentrated under reduced pressure, and the crude product was separated and purified with silica gel chromatography column (dichloromethane/methanol (v/v) = 20 : 1) to afford a mixture of compounds 199 and 200, and the mixture was further purified with Prep-TLC (dichloromethane/methanol (v/v) = 40 : 1) to afford compound 199 (35 mg) and compound 200 (45 mg), respectively.

Nuclear magnetic resonance spectrum of compound 199:

$^1$H NMR (400 MHz, $CD_3OD$) δ 8.48 (d, 1H), 7.77 - 7.54 (m, 4H), 6.86 - 6.78 (m, 1H), 6.70 - 6.60 (m, 1H), 5.10 - 5.00 (m, 1H), 4.29 - 4.15 (m, 2H), 3.97 - 3.75 (m, 6H), 3.23 - 2.60 (m, 10H), 2.28 - 2.00 (m, 3H).
LCMS m/z = 710.7 $[M+1]^+$.

Nuclear magnetic resonance spectrum of compound 200:

$^1$H NMR (400 MHz, $CD_3OD$) δ 8.48 (d, 1H), 7.78 - 7.55 (m, 3H), 7.49 (s, 1H), 6.82 - 6.76 (m, 1H), 6.62 (dd, 1H), 5.09 - 4.99 (m, 1H), 4.25 - 4.07 (m, 2H), 3.96 (s, 2H), 3.83 (s, 2H), 3.81 - 3.72 (m, 2H), 3.18 - 2.60 (m, 10H), 2.30 - 2.02 (m, 3H).
LCMS m/z = 710.8 $[M+1]^+$.

**Example 201: Preparation of compound 201**

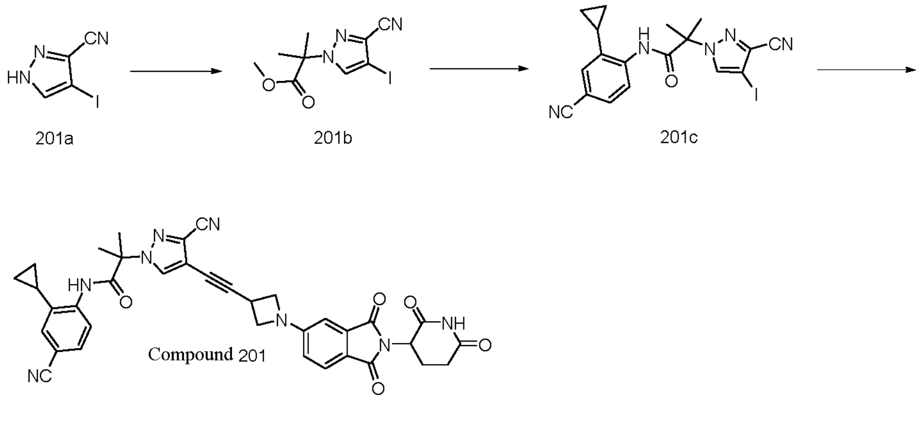

201a

201b

201c

Compound 201

Step 1: methyl 2-(3-cyano-4-iodo-1H-pyrazol-1-yl)-2-methylpropanoate (201b)

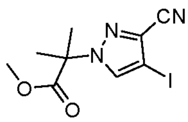

201a (1.5 g, 6.85 mmol) was added into a 100 mL single-mouth bottle, 30 mL of acetonitrile was added, and then methyl 2-bromo-2-methylpropionate (1.47 g, 8.12 mmol) and cesium carbonate (4.4 g, 13.50 mmol) were added in sequence, and the mixture was reacted at 90°C for 16 h. The reaction system was cooled to room temperature and filtered, and the filtrate was concentrated under reduced pressure. The crude product was separated and purified with silica gel chromatography column (petroleum ether : ethyl acetate (v/v) = 87 : 13) to afford 201b (0.7 g, yield: 32%).

$^1$H NMR (400 MHz, $CDCl_3$) $\delta$ 7.69 (s, 1H), 3.74 (s, 3H), 1.86 (s, 6H).

Step 2: N-(4-cyano-2-cyclopropylphenyl)-2-(3-cyano-4-iodo-1H-pyrazol-1-yl)-2-methylpropanamide (201c)

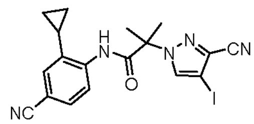

201b (0.7 g, 2.19 mmol) was dissolved in 15 mL of tetrahydrofuran, 5 mL of water was added, the mixture was cooled to 0°C, lithium hydroxide monohydrate (277 mg, 6.6 mmol) was added, and the mixture was reacted at room temperature for 2 h. The pH value of the reaction system was adjusted to 4 with 0.5 mol/L hydrochloric acid, the mixture was extracted with ethyl acetate (60 mL × 3), the organic phase was washed with 50 mL of saturated aqueous sodium chloride solution (60 mL × 2), dried over anhydrous sodium sulfate, and concentrated under reduced pressure to afford a crude product (0.51 g). The above crude product (150 mg, 0.49 mmol) was added to a 50 mL single-mouth bottle, 12 mL of dichloromethane was added, 1-chloro-N,N,2-trimethylpropenylamine (98 mg, 0.73 mmol) was added, and the mixture was reacted at room temperature for 1 h, then triethylamine (148 mg, 1.46 mmol) and 39b (77 mg, 0.49 mmol) were added in sequence, and the mixture was reacted at room temperature for 1 h. The reaction system was concentrated under reduced pressure, and the crude product was separated and purified with silica gel chromatography column (petroleum ether : ethyl acetate (v/v) = 86 : 14) to afford 201c (87 mg, yield: 40%).

LCMS m/z = 446.1 $[M+1]^+$.

Step 3; N-(4-cyano-2-cyclopropylphenyl)-2-(3-cyano-4-((1-(2-(2,6-dioxopiperidin-3-yl)-1,3-dioxoisoindolin-5-yl)azetidin-3-yl)ethynyl)-1H-pyrazol-1-yl)-2-methylpropanamide (Compound 201)

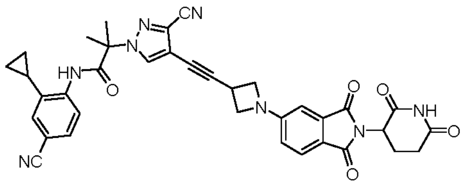

201c (87 mg, 0.195 mmol) was added into a 50 mL single-mouth bottle, 10 mL of DMF was added, crude intermediate 1 (101 mg) and triethylamine (61 mg, 0.6 mmol) were added, nitrogen replacement was performed three times, $PdCl_2(PPh_3)_2$ (14 mg, 0.02 mmol) and CuI (8 mg, 0.042 mmol) were added, nitrogen replacement was performed three times, and the mixture was reacted at 50°C for 2 h. The reaction system was cooled to room temperature, 100 mL of saturated aqueous ammonium chloride solution was slowly added, the mixture was extracted with ethyl acetate (50 mL × 3), and the organic phase was washed with saturated aqueous sodium chloride solution (50 mL × 2), dried over anhydrous sodium sulfate, and concentrated under reduced pressure. The crude product was separated and purified with silica gel chromatography column (petroleum ether : ethyl acetate (v/v) = 24 : 76) to afford compound 201 (65 mg, yield: 51%).

$^1H$ NMR (400 MHz, $CDCl_3$) δ 8.97 (s, 1H), 8.34 (d, 1H), 7.94 (s, 1H), 7.86 (s, 1H), 7.68 (d, 1H), 7.54 - 7.38 (m, 2H), 6.84 - 6.78 (m, 1H), 6.56 (dd, 1H), 4.99 - 4.88 (m, 1H), 4.45 - 4.32 (m, 2H), 4.15 - 4.02 (m, 2H), 3.92 - 3.79 (m, 1H), 3.00 - 2.62 (m, 3H), 2.20 - 2.06 (m, 1H), 2.00 (s, 6H), 1.63 - 1.49 (m, 1H), 1.19 - 1.09 (m, 2H), 0.70 - 0.60 (m, 2H).

LCMS m/z = 653.4 $[M-1]^-$.

**Example 202: Preparation of compound 202**

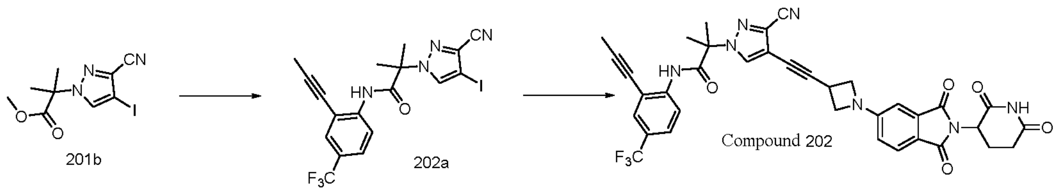


Step 1: 2-(3-cyano-4-iodo-1H-pyrazol-1-yl)-2-methyl-N-(2-(prop-1-yn-1-yl)-4-(trifluoromethyl)phenyl)propanamide (202a)

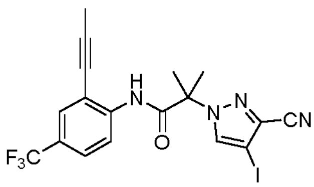

201b (0.7 g, 2.19 mmol) was dissolved in 15 mL of tetrahydrofuran, 5 mL of water was added, the mixture was cooled to 0°C, lithium hydroxide monohydrate (277 mg, 6.6 mmol) was added, and the mixture was reacted at room temperature for 2 h. The pH value of the reaction system was adjusted to 4 with 0.5 mol/L hydrochloric acid, the mixture was extracted with ethyl acetate (60 mL × 3), the organic phase was washed with 50 mL of saturated aqueous sodium chloride solution (60 mL × 2), dried over anhydrous sodium sulfate, and concentrated under reduced pressure to afford a crude product (0.51 g). The above crude product (150 mg) was added to a 50 mL single-mouth bottle, 12 mL of dichloromethane was added, 1-chloro-N,N,2-trimethylpropenylamine (98 mg, 0.73 mmol) was added, and the mixture was reacted at room temperature for 1 h, then triethylamine (148 mg, 1.46 mmol) and 175B (98 mg, 0.49 mmol) were

added in sequence, and the mixture was reacted at room temperature for 1 h. The reaction system was concentrated under reduced pressure, and the crude product was separated and purified with silica gel chromatography column (petroleum ether : ethyl acetate (v/v) = 87 : 13) to afford 202a (231 mg, yield: 22%).

LCMS m/z = 487.1 [M+1]$^+$.

Step 2; 2-(3-cyano-4-((1-(2-(2,6-dioxopiperidin-3-yl)-1,3-dioxoisoindolin-5-yl)azetidin-3-yl)ethynyl)-1H-pyrazol-1-yl)-2-methyl-N-(2-(prop-1-yn-1-yl)-4-(trifluoromethyl)phenyl)propanamide (Compound 202)

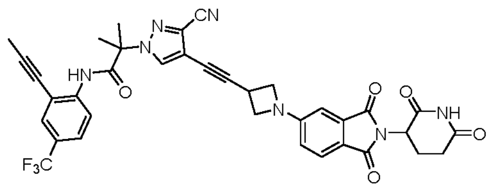

202a (52 mg, 0.11 mmol) was added into a 50 mL single-mouth bottle, 8 mL of DMF was added, crude intermediate 1 (54 mg) and triethylamine (33 mg, 0.33 mmol) were added, nitrogen replacement was performed three times, $PdCl_2(PPh_3)_2$ (8 mg, 0.011 mmol) and CuI (4 mg, 0.021 mmol) were added, nitrogen replacement was performed three times, and the mixture was reacted at 50°C for 2 h. The reaction system was cooled to room temperature, 80 mL of saturated aqueous ammonium chloride solution was slowly added, the mixture was extracted with ethyl acetate (50 mL × 3), and the organic phase was washed with saturated aqueous sodium chloride solution (50 mL × 2), dried over anhydrous sodium sulfate, and concentrated under reduced pressure. The crude product was separated and purified with silica gel chromatography column (petroleum ether : ethyl acetate (v/v) = 24 : 76) to afford compound 202 (38 mg, yield: 50%).

$^1H$ NMR (400 MHz, $CDCl_3$) δ 8.67 (s, 1H), 8.41 (d, 1H), 7.94 (s, 1H), 7.85 (s, 1H), 7.76 - 7.46 (m, 3H), 6.86 - 6.76 (m, 1H), 6.56 (dd, 1H), 4.99 - 4.88 (m, 1H), 4.45 - 4.30 (m, 2H), 4.15 - 4.01 (m, 2H), 3.95 - 3.77 (m, 1H), 2.96 - 2.64 (m, 3H), 2.25 (s, 3H), 2.19 - 2.08 (m, 1H), 1.98 (s, 6H).

**Example 203: Preparation of the trifluoroacetate of compound 203**

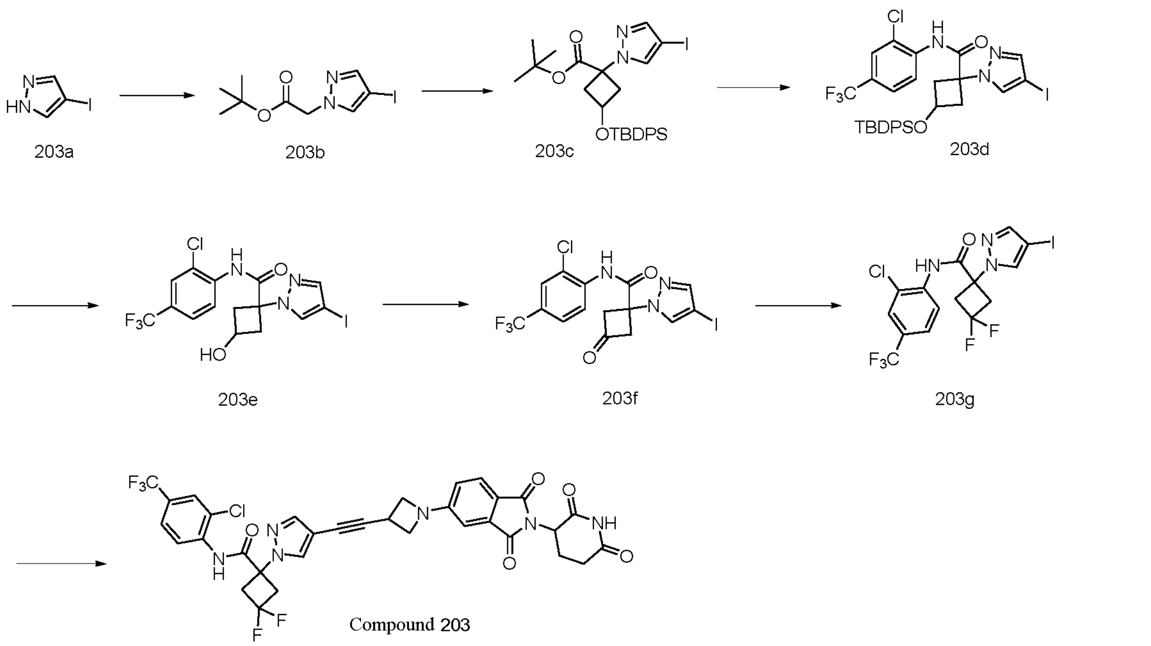
203a 203b 203c 203d 203e 203f 203g Compound 203

Step 1: tert-butyl 2-(4-iodo-1H-pyrazol-1-yl)acetate (203b)

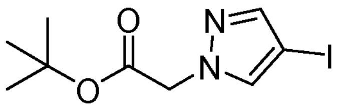

203a (15.6 g, 80.42 mmol) was added to 200 mL of DMF, tert-butyl bromoacetate (17.2 g, 88 mmol) and cesium carbonate (52 g, 0.16 mol) were added, and the mixture was reacted at 25°C for 3 h. 300 mL of ethyl acetate was added to the reaction liquid, and the organic phase was washed with 800 mL of purified water. The organic phase was separated, dried over anhydrous sodium sulfate, and concentrated under reduced pressure. The crude product was separated and purified with silica gel chromatography column (petroleum ether : ethyl acetate (v/v) = 4 : 1) to afford 203b (21.6 g, yield: 87%).

Step 2: tert-butyl 3-((tert-butyldiphenylsilyl)oxy)-1-(4-iodo-1H-pyrazol-1-yl) cyclobutane-1-carboxylate (203c)

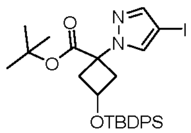

60% sodium hydride (2.45 g) was added to 300 mL of DMSO, the mixture was cooled to 0°C, and 40 mL of tert-butyl ((1,3-dibromoprop-2-yl)oxy)diphenylsilane (20.4 g, 44.7 mmol) (see WO 2013173720 for the synthesis method) and a solution of 203b (10.5 g, 34.08 mmol) in DMSO were added dropwise to the above solution and the mixture was reacted at room temperature for 12 h. 1 L of purified water was added to the reaction system, the mixture was extracted with 300 mL of ethyl acetate, the organic phase was separated, dried over anhydrous sodium sulfate, and concentrated under reduced pressure. The crude product was separated and purified with silica gel chromatography column (petroleum ether : ethyl acetate (v/ v) =4 : 1) to afford 203c (3.5 g, yield: 17%).

LCMS m/z = 603.2 $[M+1]^+$.

Step 3: 3-((tert-butyldiphenylsilyl)oxy)-N-(2-chloro-4-(trifluoromethyl)phenyl)-1-(4-iodo-1H-pyrazol-1-yl)cyclobutane-1-carboxamide (203d)

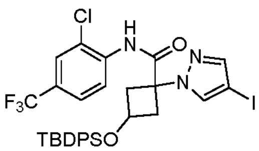

203c (3.5 g, 5.81 mmol) was added to 60 mL of dichloromethane, and 20 mL of trifluoroacetic acid was added. The mixture was reacted at room temperature for 3 h. The reaction liquid was concentrated under reduced pressure, 100 mL of dichloromethane was added, the pH was adjusted to 9 with saturated aqueous sodium carbonate solution, and then the pH was adjusted to 5 with 2 mol/L hydrochloric acid. The organic phase was separated, dried over anhydrous sodium sulfate, and concentrated under reduced pressure to afford a crude product (2.5 g). The above crude product (2.5 g) and 2-chloro-4-(trifluoromethyl)aniline (0.88 g, 4.5 mmol) were dissolved in 100 mL of DCM, TCFH (1.96 g, 7.0 mmol) was added, and N-methylimidazole (1.5 g, 18.27 mmol) was slowly added dropwise, and the mixture was reacted at room temperature for 16 h. The reaction system was concentrated under reduced pressure, and the crude product was separated and purified with silica gel chromatography column (petroleum ether/ethyl acetate (v/v) = 4 : 1) to afford 203d (2.3 g, yield: 71%).

Step 4: N-(2-chloro-4-(trifluoromethyl)phenyl)-3-hydroxy-1-(4-iodo-1H-pyrazol-1-yl)cyclobutane-1-carboxamide (203e)

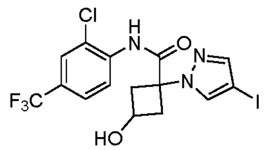

203d (2.3 g, 3.2 mmol) was dissolved in 60 mL of THF, TBAF (2.6 g, 9.94 mmol) was added, and the mixture was reacted at reflux for 5 h. The reaction system was cooled to room temperature and concentrated under reduced pressure, and 50 mL of ethyl acetate was added to the residue. The organic phase was washed with 100 mL of purified water, dried over anhydrous sodium sulfate, and concentrated under reduced pressure to afford 203e (0.6 g, yield: 39.0%).
LCMS m/z = 485.9 $[M+1]^+$.

Step 5: N-(2-chloro-4-(trifluoromethyl)phenyl)-1-(4-iodo-1H-pyrazol-1-yl)-3-oxocyclobutane-1-carboxamide (203f)

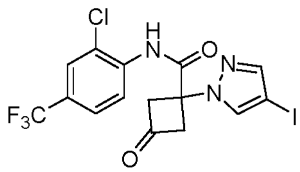

203e (0.5 g, 1.03 mmol) was dissolved in 10 mL of dichloromethane, Dess-Martin oxidant (0.84 g, 1.98 mmol) was added, and the mixture was reacted at room temperature for 2 h. The reaction system was filtered, and the filtrate was concentrated under reduced pressure. The crude product was separated and purified with silica gel chromatography column (petroleum ether : ethyl acetate (v/v) = 2 : 1) to afford 203f (0.4 g, yield: 80%).

Step 6: N-(2-chloro-4-(trifluoromethyl)phenyl)-3,3-difluoro-1-(4-iodo-1H-pyrazol-1-yl)cyclobutane-1-carboxamide (203g)

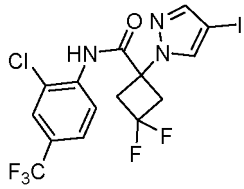

203f (0.1 g, 0.21 mmol) was dissolved in 5 mL of dichloromethane, the mixture was cooled to 0°C, DAST (0.16 g, 0.993 mmol) was added, and the mixture was reacted at room temperature for 16 h. 1 mL of methanol was added to the reaction system, the mixture was concentrated under reduced pressure, and the crude product was separated and purified with silica gel chromatography column (petroleum ether : ethyl acetate (v/v) = 1 : 1) to afford 203g (0.09 g, yield: 85%).

Step 7: N-(2-chloro-4-(trifluoromethyl)phenyl)-1-(4-((1-(2-(2,6-dioxopiperidin-3-yl)-1,3-dioxoisoindolin-5-yl)azetidin-3-yl)ethynyl)-1H-pyrazol-1-yl)-3,3-difluorocyclobutane-1-carboxamide (Compound 203)

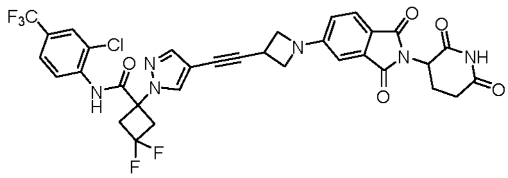

203g (0.09 g, 0.18 mmol), crude intermediate 1 (0.1 g), TEA (0.1 g, 0.989 mmol), CuI (3.6 mg, 0.019 mmol) and $PdCl_2(PPh_3)_2$ (14 mg, 0.02 mmol) were added to a reaction bottle, 5 mL of DMF was added under nitrogen protection, and the mixture was reacted at 75°C for 3 h. The reaction liquid was cooled to room temperature and filtered, and the filtrate was subjected to Prep-HPLC (instrument and preparative column: using Glison GX-281 preparative liquid phase chromatographic instrument, preparative column model: Sunfire C18, 5 μm, inner diameter × length = 30 mm × 150 mm). Preparation method: the crude product was dissolved with methanol and dimethyl sulfoxide, and filtered with a 0.45 μm filter membrane, to prepare into a sample liquid. Mobile phase system: acetonitrile/water (containing 0.1% TFA). Gradient elution method: gradient elution of 5% to 60% acetonitrile (elution time 15 min), and lyophilization was performed to afford the trifluoroacetate of compound 203 (10 mg).

$^1$H NMR (400 MHz, DMSO-$d_6$) δ 11.06 (s, 1H), 9.26 (s, 1H), 8.51 (s, 1H), 8.10 - 8.03 (m, 1H), 7.96 - 7.89 (m, 2H), 7.79 - 7.64 (m, 2H), 6.90 - 6.84 (m, 1H), 6.72 (dd, 1H), 5.12 - 5.00 (m, 1H), 4.44 - 4.34 (m, 2H), 4.08 - 3.82 (m, 3H), 3.78 - 3.45 (m, 4H), 2.96 - 2.78 (m, 1H), 2.73 - 2.46 (m, 2H), 2.09 - 1.93 (m, 1H).

**Example 204: Preparation of compound 204**

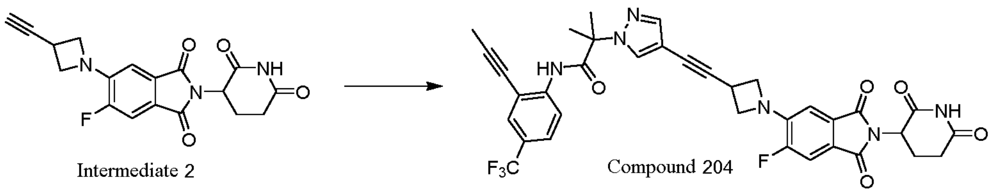


118b (0.50 g, 1.08 mmol) was dissolved in 10 mL of DMF, and crude intermediate 2 (0.46 g), TEA (0.33 g, 3.26 mmol), CuI (0.041 g, 0.22 mmol) and $PdCl_2(PPh_3)_2$ (0.076 g, 0.108 mmol) were added in sequence, and the mixture was reacted at 60°C for 2 h. The reaction system was cooled to room temperature, 30 mL of dichloromethane and 30 mL of purified water were added, the organic phase was separated, the aqueous phase was extracted with dichloromethane (30 mL × 3), the organic phase was washed with 30 mL of saturated aqueous sodium chloride solution, dried over anhydrous sodium sulfate, and concentrated under reduced pressure. The crude product was separated and purified with silica gel chromatography column (petroleum ether : ethyl acetate (v/v) = 1 : 1). 15 mL of methyl tert-butyl ether was added to the obtained crude product. The mixture was stirred at room temperature for 1 h, then filtered, and the filter cake was dried under reduced pressure to afford compound 204 (0.31 g, yield: 42%).

$^1$H NMR (400 MHz, $CDCl_3$) δ 8.84 (s, 1H), 8.48 (d, 1H), 8.09 (s, 1H), 7.81 (s, 1H), 7.74 (s, 1H), 7.61 - 7.44 (m, 2H), 7.33 (d, 1H), 6.84 (d, 1H), 4.97 - 4.87 (m, 1H), 4.55 - 4.40 (m, 2H), 4.25 - 4.10 (m, 2H), 3.85 - 3.72 (m, 1H), 2.96 - 2.62 (m, 3H), 2.20 - 2.08 (m, 4H), 1.95 (s, 6H).

LCMS m/z = 689.7 [M+1]$^+$.

**Example 205: Preparation of compound 205**

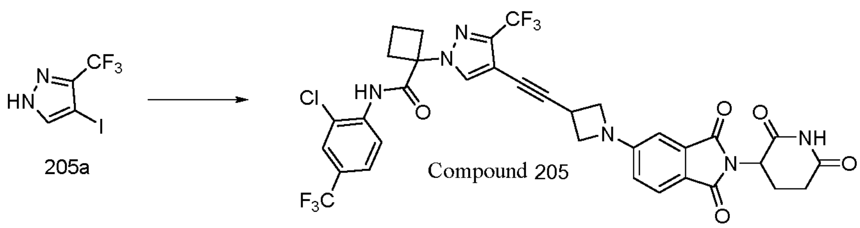

96b (400 mg, 1.12 mmol) was added into a 50 mL single-mouth bottle, 15 mL of acetonitrile was added, and then 205a (295 mg, 1.13 mmol) and cesium carbonate (734 mg, 2.25 mmol) were added in sequence, and the mixture was reacted at 90°C for 16 h. The reaction system was cooled to room temperature and filtered, and the filtrate was concentrated under reduced pressure. The crude product was separated and purified with silica gel chromatography column (petroleum ether : ethyl acetate (v/v) = 88 : 12) to afford a crude product (260 mg). The above crude product (260 mg) was added to a 50 mL single-mouth bottle, 15 mL of DMF was added, crude intermediate 1 (245 mg) and TEA (145 mg, 1.43 mmol) were added, nitrogen replacement was performed three times, $PdCl_2(PPh_3)_2$ (34 mg, 0.048 mmol) and CuI (18 mg, 0.095 mmol) were added, nitrogen replacement was performed three times, and the mixture was reacted at 50°C for 2 h. The reaction system was cooled to room temperature, 160 mL of saturated aqueous ammonium chloride solution was slowly added, the mixture was extracted with ethyl acetate (80 mL × 3), and the organic phase was washed with saturated aqueous sodium chloride solution (80 mL × 2), dried over anhydrous sodium sulfate, and concentrated under reduced pressure. The crude product was separated and purified with silica gel chromatography column (petroleum ether : ethyl acetate (v/v) = 48 : 52) to afford compound 205 (210 mg, yield: 25%).

$^1H$ NMR (400 MHz, $CDCl_3$) δ 8.65 - 8.47 (m, 2H), 8.00 (s, 1H), 7.80 - 7.46 (m, 4H), 6.84 - 6.76 (m, 1H), 6.55 (dd, 1H), 4.98 - 4.88 (m, 1H), 4.43 - 4.30 (m, 2H), 4.11 - 4.00 (m, 2H), 3.90 - 3.75 (m, 1H), 3.19 - 3.02 (m, 2H), 2.95 - 2.65 (m, 5H), 2.35 - 2.04 (m, 3H).

LCMS m/z = 747.1 $[M+1]^+$.

**Example 206: Preparation of compound 206**

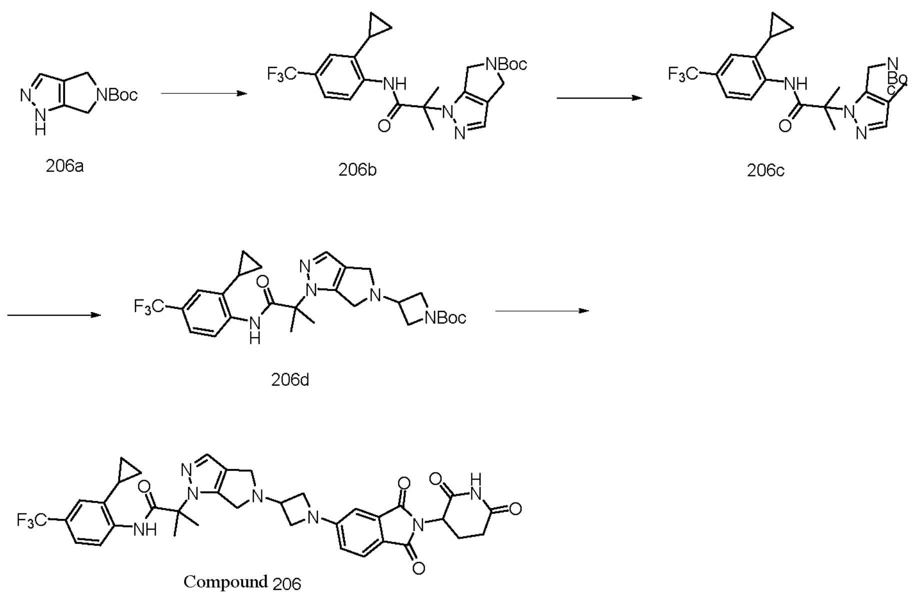

206a 206b 206c

206d

Compound 206

Step 1: tert-butyl 1-(1-((2-cyclopropyl-4-(trifluoromethyl)phenyl)amino)-2-methyl-1-oxopropan-2-yl)-4,6-dihydropyrrolo[3,4-c]pyrazole-5(1H)-carboxylate (206b)

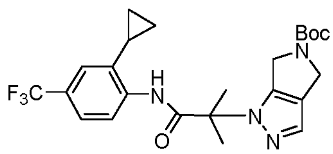

206a (2.1 g, 10.03 mmol) was added to 100 mL of ethyl acetate, 2-bromo-2-methylpropionic acid (2.0 g, 12 mmol) and triethylamine (3.0 g, 29.65 mmol) were added, and the mixture was reacted at 60°C for 3 h. The reaction system was cooled to room temperature, 100 mL of purified water was added, the organic phase was separated, the pH of the aqueous phase was adjusted to 5 with 2 mol/L hydrochloric acid, the mixture was extracted with 200 mL of ethyl acetate, the organic phase was dried over anhydrous sodium sulfate, and concentrated under reduced pressure

to afford a crude product (2.7 g). The above crude product (2.7 g) and 30b (2.0 g, 9.94 mmol) were dissolved in 100 mL of DCM, TCFH (3.8 g, 13.54 mmol) was added, and N-methylimidazole (2.8 g, 34.10 mmol) was slowly added dropwise, the mixture was reacted at room temperature for 16 h. The reaction system was concentrated under reduced pressure, and the crude product was separated and purified with silica gel chromatography column (petroleum ether/ethyl acetate (v/v) = 4 : 1) to afford 206b (1.5 g, yield: 32%).

$^1H$ NMR (400 MHz, $CDCl_3$) δ 9.05 (d, 1H), 8.34 (dd, 1H), 7.49 - 7.29 (m, 3H), 4.70 - 4.52 (m, 2H), 4.45 - 4.31 (m, 2H), 2.00 - 1.90 (m, 6H), 1.60 - 1.44 (m, 10H), 1.04 - 0.92 (m, 2H), 0.64 - 0.56 (m, 2H) .

Step 2: N-(2-cyclopropyl-4-(trifluoromethyl)phenyl)-2-(5,6-dihydropyrrolo[3,4-c]pyrazol-2(4H)-yl)-2-methylpropanamide (206c)

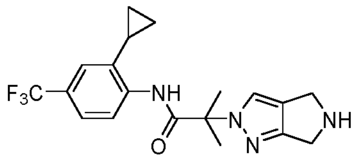

206b (1.5 g, 3.13 mmol) was dissolved in 30 mL of dichloromethane, and 10 mL of trifluoroacetic acid was added. The mixture was reacted at room temperature for 1 h. The reaction system was concentrated under reduced pressure, and 100 mL of dichloromethane was added. The mixture was adjusted to pH 9 with saturated aqueous sodium carbonate solution. The organic phase was separated, dried over anhydrous sodium sulfate, and concentrated under reduced pressure to afford crude 206c (1.1 g).

Step 3: tert-butyl 3-(1-(1-((2-cyclopropyl-4-(trifluoromethyl)phenyl)amino)-2-methyl-1- oxopropan-2-yl)-4,6-dihydropyrrolo[3,4-c]pyrazol-5(1H)-yl)azetidine-1-carboxylate (206d)

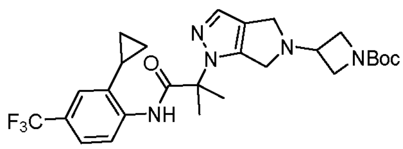

Crude 206c (0.55 g) was dissolved in 60 mL of 1,2-dichloroethane, tert-butyl 3-oxoazetidine-1-carboxylate (0.51 g, 2.98 mmol) was added, the mixture was reacted at room temperature for 2 h, then sodium triacetoxyborohydride (0.63 g, 2.97 mmol) was added and the mixture was reacted at room temperature for 15 h. The reaction liquid was concentrated under reduced pressure, and the crude product was separated and purified with silica gel chromatography column (dichloromethane : methanol (v/v) = 10 : 1) to afford 206d (0.68 g, two-step yield from compound 206b: 81%).

LCMS m/z= 534.6 $[M+1]^+$.

Step 4: N-(2-cyclopropyl-4-(trifluoromethyl)phenyl)-2-(5-(1-(2-(2,6-dioxopiperidin-3-yl)-1,3-dioxoisoindolin-5-yl)azetidin-3-yl)-5,6-dihydropyrrolo[3,4-c]pyrazol-1(4H)-yl)-2-methylpropanamide (Compound 206)

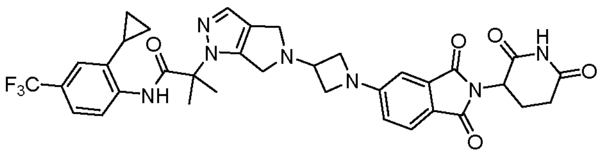

206d (0.68 g, 1.27 mmol) was dissolved in 30 mL of dichloromethane, and 10 mL of trifluoroacetic acid was added. The mixture was reacted at room temperature for 1 h. The reaction system was concentrated under reduced pressure, and 100 mL of dichloromethane was added. The mixture was adjusted to pH 9 with saturated aqueous sodium

carbonate solution. The organic phase was separated, dried over anhydrous sodium sulfate, and concentrated under reduced pressure to afford a crude product. The above crude product was dissolved in 10 mL of DMSO, 1.0 mL of DIPEA and 1C (0.55 g, 2.0 mmol) were added, and the mixture was reacted at 85°C for 3 h. The reaction liquid was cooled to room temperature, 50 mL of purified water was added, the solid was precipitated, filtration was performed, the filter cake was dissolved with 30 mL of dichloromethane, the mixture was dried over anhydrous sodium sulfate, and concentrated under reduced pressure. The crude product was separated and purified with silica gel column chromatography (dichloromethane : methanol (v/v) = 15 : 1) to afford compound 206 (0.54 g, yield: 62%).

$^1H$ NMR (400 MHz, $CDCl_3$) δ 9.10 (s, 1H), 8.34 (d, 1H), 8.23 (s, 1H), 7.70 - 7.62 (m, 1H), 7.50 - 7.29 (m, 3H), 6.85 - 6.76 (m, 1H), 6.54 (dd, 1H), 4.97 - 4.85 (m, 1H), 4.28 - 3.78 (m, 9H), 3.00 - 2.60 (m, 3H), 2.18 - 2.06 (m, 1H), 1.98 - 1.86 (m, 6H), 1.60 - 1.48 (m, 1H), 1.04 - 0.90 (m, 2H), 0.66 - 0.56 (m, 2H).

LCMS m/z = 690.3 $[M+1]^+$.

**Example 207: Preparation of compound 207**

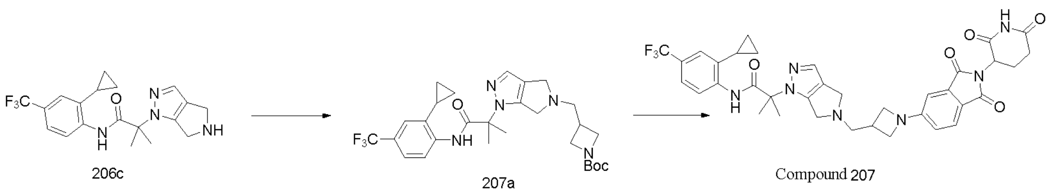


Step 1: tert-butyl 3-((1-(1-((2-cyclopropyl-4-(trifluoromethyl)phenyl)amino)-2-methyl-1-oxopropan-2-yl)-4,6-dihydropyrrolo[3,4-c]pyrazol-5(1H)-yl)methyl)azetidine-1-carboxylate (207a)

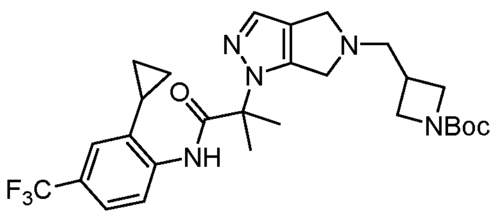

Crude 206c (0.55 g) was dissolved in 60 mL of 1,2-dichloroethane, tert-butyl 3-formylazetidine-1-carboxylate (0.55 g, 2.97 mmol) was added, the mixture was reacted at room temperature for 2 h, then sodium triacetoxyborohydride (0.63 g, 2.97 mmol) was added and the mixture was reacted at room temperature for 15 h. The reaction liquid was concentrated under reduced pressure, and the crude product was separated and purified with silica gel chromatography column (dichloromethane : methanol (v/v) = 10 : 1) to afford 207a (0.5 g, two-step yield from compound 206b: 58%).

Step 2: N-(2-cyclopropyl-4-(trifluoromethyl)phenyl)-2-(5-((1-(2-(2,6-dioxopiperidin-3-yl)-1,3-dioxoisoindolin-5-yl)azetidin-3-yl)methyl)-5,6-dihydropyrrolo[3,4-c]pyrazol-1(4H)-yl)-2-methylpropanamide (Compound 207)

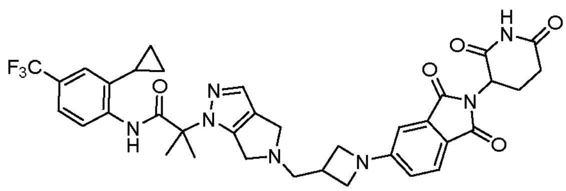

207a (0.5 g, 0.91 mmol) was dissolved in 30 mL of dichloromethane, and 10 mL of trifluoroacetic acid was added. The mixture was reacted at room temperature for 1 h. The reaction liquid was concentrated under reduced

pressure, and 100 mL of dichloromethane was added. The mixture was adjusted to pH 9 with saturated aqueous sodium carbonate solution. The organic phase was separated, dried over anhydrous sodium sulfate, and concentrated under reduced pressure to afford a crude product. The above crude product was dissolved in 10 mL of DMSO, 1.0 mL of DIPEA and 1C (0.55 g, 2.0 mmol) were added, and the mixture was reacted at 85°C for 3 h. The reaction liquid was cooled to room temperature, 50 mL of purified water was added, the solid was precipitated, filtration was performed, the filter cake was dissolved with 30 mL of dichloromethane, the mixture was dried over anhydrous sodium sulfate, and concentrated under reduced pressure. The crude product was separated and purified with silica gel column chromatography (dichloromethane : methanol (v/v) = 15 : 1) to afford **compound** 207 (0.45 g, yield: 70%).

$^1$H NMR (400 MHz, $CDCl_3$) δ 9.38 (br.s, 1H), 9.07 (s, 1H), 8.37 (d, 1H), 7.64 (d, 1H), 7.51 - 7.41 (m, 1H), 7.40 - 7.31 (m, 2H), 6.99 - 6.90 (m, 1H), 6.49 (dd, 1H), 4.89 - 4.76 (m, 1H), 4.24 - 3.65 (m, 8H), 3.20 - 2.90 (m, 3H), 2.89 - 2.65 (m, 2H), 2.60 - 2.40 (m, 1H), 2.12 - 1.86 (m, 7H), 1.60 - 1.44 (m, 1H), 1.13 - 0.87 (m, 2H), 0.68 - 0.54 (m, 2H).

LCMS m/z = 704.3 $[M+1]^+$ .

**Experimental example**

**1. Experiment on inhibiting 22RV1 cell proliferation**

Prostate cancer cells 22RV1 were purchased from ATCC, the cell medium was RPMI 1640+10% FBS, and the cells were cultured in an incubator at 37°C under 5% $CO_2$. On day 1, the cells in the exponential growth phase were collected. With a 1% css-FBS phenol red-free medium, the cell suspension was adjusted to a corresponding concentration, plated at 2000 cells/well and incubated overnight. On day 2, compounds at different concentrations were added, and the plate was placed in the incubator and incubated for further 7 days. After the incubation was completed, according to operation instructions for a CellTiter-Glo kit (Promega, G7573), 50 μL of CellTiter-Glo reagents, which were already pre-melted and equilibrated to room temperature, were added to each well, and the mixtures were uniformly mixed for 2 min using a microplate shaker. The plate was placed at room temperature for 10 min, and then fluorescence signal values were measured using a microplate reader (PHERAstar FSX). The results were processed according to formula (1), the inhibition rate of the compound at different concentrations was calculated, and the $IC_{50}$ value of the compound with an inhibition rate of 50% was calculated using origin9.2 software with DoseResp function, wherein $RLU_{compound}$ was the readout of the treated group, and $RLU_{control}$ was the average value of the DMSO vehicle control group.

$$\text{Inhibition\%} = [1 - RLU_{compound} / RLU_{control}] \times 100\% \quad \text{formula (1)}$$

The results for $IC_{50}$ values on proliferation inhibition of 22RV1 cells were shown in Table 1.

Table 1 $IC_{50}$ values for inhibition of 22RV1 cells by compounds of the present invention

| Serial No. | Compound No. | $IC_{50}$ (μM) | Serial No. | Compound No. | $IC_{50}$ (μM) |
|---|---|---|---|---|---|
| 1 | Compound 3 | < 1 | 47 | Compound 75 | < 50 nM |
| 2 | Compound 6 | < 1 | 48 | Compound 77 | <50nM |
| 3 | Compound 11 | < 50 nM | 49 | Compound 80 | < 1 |
| 4 | Compound 12 | < 50 nM | 50 | Compound 81 | < 1 |
| 5 | Compound 13 | < 1 | 51 | Compound 82 | <50nM |
| 6 | Compound 14 | < 50 nM | 52 | Compound 85 | < 1 |
| 7 | Compound 15 | < 100 nM | 53 | Compound 91 | < 1 |
| 8 | Compound 16 | < 1 | 54 | Compound 112 | < 1 |
| 9 | Compound 17 | < 1 | 55 | Compound 113 | <50nM |
| 10 | Compound 18 | < 1 | 56 | Compound 116 | <50nM |
| 11 | Compound 20 | < 50 nM | 57 | Compound 117 | < 1 |
| 12 | Compound 21 | < 50 nM | 58 | Compound 118 | <50nM |
| 13 | Compound 22 | < 1 | 59 | Compound 133 | < 100 nM |
| 14 | Compound 23 | < 1 | 60 | Trifluoroacetate of compound 139 | < 1 |

(continued)

| Serial No. | Compound No. | $IC_{50}$ (μM) | Serial No. | Compound No. | $IC_{50}$ (μM) |
|---|---|---|---|---|---|
| 15 | Compound 25 | < 1 | 61 | Compound 141 | <50nM |
| 16 | Compound 26 | < 1 | 62 | Compound 152 | <50nM |
| 17 | Compound 27 | < 1 | 63 | Compound 154 | < 1 |
| 18 | Compound 28 | < 50 nM | 64 | Compound 155 | <50nM |
| 19 | Compound 29 | < 100 nM | 65 | Compound 160 | <50nM |
| 20 | Compound 30 | < 50 nM | 66 | Compound 161 | < 1 |
| 21 | Compound 31 | < 100 nM | 67 | Compound 162 | < 1 |
| 22 | Compound 32 | < 1 | 68 | Compound 163 | <50nM |
| 23 | Compound 33 | < 1 | 69 | Compound 164 | <50nM |
| 24 | Compound 34 | < 100 nM | 70 | Compound 165 | < 100 nM |
| 25 | Compound 35 | < 50 nM | 71 | Compound 166 | <50nM |
| 26 | Compound 37 | < 50 nM | 72 | Compound 169 | <50nM |
| 27 | Compound 38 | < 50 nM | 73 | Compound 172 | <50nM |
| 28 | Compound 39 | < 50 nM | 74 | Compound 175 | < 100 nM |
| 29 | Compound 40 | < 100 nM | 75 | Compound 178 | <50nM |
| 30 | Compound 41 | < 50 nM | 76 | Compound 181 | <50nM |
| 31 | Compound 42 | < 1 | 77 | Compound 182 | < 100 nM |
| 32 | Compound 43 | < 1 | 78 | Compound 184 | < 100 nM |
| 33 | Compound 44 | < 1 | 79 | Compound 186 | < 100 nM |
| 34 | Compound 45 | < 50 nM | 80 | Compound 187 | < 100 nM |
| 35 | Compound 46 | < 1 | 81 | Compound 189 | < 100 nM |
| 36 | Compound 47 | < 50 nM | 82 | Compound 190 | < 100 nM |
| 37 | Compound 48 | < 100 nM | 83 | Compound 192 | < 100 nM |
| 38 | Compound 50 | < 100 nM | 84 | Compound 193 | < 100 nM |
| 39 | Compound 51 | < 50 nM | 85 | Compound 194 | < 100 nM |
| 40 | Compound 52 | < 1 | 86 | Compound 197 | < 1 |
| 41 | Compound 56 | < 50 nM | 87 | Compound 201 | < 100 nM |
| 42 | Compound 59 | < 50 nM | 88 | Compound 202 | < 100 nM |
| 43 | Compound 68 | < 50 nM | | | |
| 44 | Compound 69 | < 50 nM | | | |
| 45 | Compound 71 | < 50 nM | | | |
| 46 | Compound 72 | < 50 nM | | | |

Conclusion: the compounds of the present invention had an inhibition effect on prostate cancer cells 22RV1.

**2. Degradation experiment on full-length AR (AR-FL) and AR splice variants (AR-Vs) in 22RV1 cells**

Prostate cancer cells 22RV1 were purchased from ATCC, the cell medium was 1640 + 10% FBS, and the cells were cultured in an incubator at 37°C under 5% $CO_2$. On day 1, the cells in an exponential phase were collected. With a 1% css-FBS phenol red-free medium, the cell suspension was adjusted to a corresponding concentration and plated

into a 6-well plate at 1 mL/well and 100000 cells/well. The next day, a 1% css-FBS phenol-free medium containing the compound to be tested was added, a 1% css-FBS phenol-free medium containing 0.2% DMSO was added to one well as DMSO vehicle control, and the 6-well plate was cultured in an incubator at 37°C under 5% $CO_2$. After 24 h, the cells were digested with pancreatin and collected into a 1.5 mL centrifuge tube. 15 μL of RIPA lysate (containing 1X protease inhibitor cocktail) was added to each well, and the cells were lysed on ice for 15 min and then centrifuged at 12000 g at 4°C for 10 min. The supernatant protein samples were collected, and protein quantification was performed by a BCA method. AR-FL and AR-Vs were detected using fully automatic protein expression quantitative analysis, which involved diluting the protein to be tested to 1 mg/mL. 4 μL of the diluted protein samples were added to 1 μL of 5 × Master Mix (provided in the kit), and the prepared samples were denatured at 95°C for 5 min and placed on ice for use. The primary antibodies, AR (CST, 5153S) and β-actin (CST, 3700), were diluted with Antibody Diluent II (provided in the kit) at a dilution ratio of 1 : 20 and 1 : 200, respectively. The secondary antibody was a mixed goat anti-mouse and goat anti-rabbit secondary antibody in a ratio of 1 : 1, and the color development solution was a mixed solution of Lumino-S and Peroxide in a ratio of 1 : 1. The prepared reagents were successively added to an assay plate according to instructions of the kit and detected on an instrument. Western blot band processing was performed using "Compass for SW", a fully automatic protein expression quantitative analysis software, in which western blot bands were automatically simulated based on signal values. The degradation rate of AR-FL (2) or AR-Vs (3) relative to vehicle control at different compound concentrations was calculated according to formulas (2) and (3), wherein $\text{AR-FL}_{compound}$ was the relative peak area of AR-FL in the treated group, and $\text{AR-FL}_{solvent}$ was the relative peak area of AR-FL in the vehicle control group; $\text{AR-Vs}_{compound}$ was the relative peak area of AR-Vs in the treated group, and $\text{AR-Vs}_{solvent}$ was the relative peak area of AR-Vs in the vehicle control group.

$$\text{Degradation rate of AR-FL} = (1 - \text{AR-FL}_{compound}/\text{AR-FL}_{solvent}) \times 100\% \quad \text{formula (2)}$$

$$\text{Degradation rate of AR-Vs} = (1 - \text{AR-Vs}_{compound}/\text{AR-Vs}_{solvent}) \times 100\% \quad \text{formula (3)}$$

$DC_{50}$ calculation: the compound concentration $DC_{50}$ values at which the degradation rate of AR-FL or AR-Vs was 50% were calculated using OriginPro2015 software and analyzed using DoseResp function according to formula (2) or (3).

Conclusion: Compound 4 had a significant degradation effect on AR-FL in prostate cells 22RV1 at a concentration of 2 μM, and had a significant degradation effect on AR-Vs in prostate cells 22RV1 at a concentration of 2 μM.

**3. Degradation experiment on AR splice variant 7 (AR-V7) in 22RV1 cells**

Prostate cancer cells 22RV1 were purchased from ATCC, the cell medium was 1640 + 10% FBS, and the cells were cultured in an incubator at 37°C under 5% $CO_2$. On day 1, the cells in an exponential phase were collected. With a 1% css-FBS phenol red-free medium, the cell suspension was adjusted to a corresponding concentration and plated into a 6-well plate at 1 mL/well and 300000 cells/well. The next day, a 1% css-FBS phenol-free medium containing the compound to be tested was added, a 1% css-FBS phenol-free medium containing 0.2% DMSO was added to one well as DMSO vehicle control, and the 6-well plate was cultured in an incubator at 37°C under 5% $CO_2$. After 24 h, the cells were digested with pancreatin and collected into a 1.5 mL centrifuge tube. 15 μL of RIPA lysate (containing 1X protease inhibitor cocktail) was added to each well, and the cells were lysed on ice for 15 min and then centrifuged at 12000 g at 4°C for 10 min. The supernatant protein samples were collected, and protein quantification was performed by a BCA method. AR-V7 was detected using fully automatic protein expression quantitative analysis, which involved diluting the protein to be tested to 2 mg/mL. 4 μL of the diluted protein samples were added to 1 μL of 5× Master Mix (provided in the kit), and the prepared samples were denatured at 95°C for 5 min and placed on ice for use. The primary antibodies, AR V7 (CST, 19672S) and β-actin (CST, 3700), were diluted with Antibody Diluent II (provided in the kit) at a dilution ratio of 1 : 10 and 1 : 500, respectively. The secondary antibody was a mixed goat anti-mouse and goat anti-rabbit secondary antibody in a ratio of 1 : 1, and the color development solution was a mixed solution of Lumino-S and Peroxide in a ratio of 1 : 1. The prepared reagents were successively added to an assay plate according to instructions of the kit and detected on an instrument. Western blot band processing was performed using "Compass for SW", a fully automatic protein expression quantitative analysis software, in which western blot bands were automatically simulated based on signal values. The degradation rate of AR-V7 (4) relative to vehicle control at different compound concentrations was calculated according to formula (4), wherein $\text{AR-V7}_{compound}$ was the relative peak area of AR-V7 in the treated group, and $\text{AR-V7}_{solvent}$ was the relative peak area of AR-V7 in the vehicle control group.

$$AR\text{-}V7\% = (1 - AR\text{-}V7_{compound} / AR\text{-}V7_{solvent}) \times 100\% \quad \text{formula (4)}$$

$DC_{50}$ calculation: the compound concentration $DC_{50}$ values at which the degradation rate of AR-V7 was 50% were calculated using Graphpad software and analyzed using log(inhibitor) vs. response - Variable slope (four parameters) function according to formula (4).

The results for $DC_{50}$ on degradation of AR-V7 by the compounds of the present invention were shown in Table 2.

Table 2 $DC_{50}$ on degradation of AR-V7

| Serial No. | Compound | AR-V7 DC50 (μM) |
|---|---|---|
| 1 | Compound 11 | < 0.5 |
| 2 | Compound 14 | < 0.5 |
| 3 | Compound 15 | < 0.5 |
| 4 | Compound 21 | < 0.5 |
| 5 | Compound 28 | < 0.5 |
| 6 | Compound 29 | < 0.5 |
| 7 | Compound 30 | < 0.5 |
| 8 | Compound 39 | < 0.5 |
| 9 | Compound 56 | < 0.5 |
| 10 | Compound 118 | < 0.5 |
| 11 | Compound 155 | < 0.5 |
| 12 | Compound 160 | < 0.5 |
| 13 | Compound 164 | < 0.5 |

Conclusion: the compounds of the present invention had good degradation effect on AR-V7 in prostate cancer cells 22RV1.

**4. Pharmacokinetic test in rats**

**Test objective:** In this experiment, a single dose of each test compound was administered to SD rats intravenously and intragastrically, and the concentrations of the test compounds in plasma of rats were measured to evaluate the pharmacokinetic characteristics of the test compounds in rats.

**Experimental animals:** male SD rats, 200-250 g, 6-8 weeks old, 6 rats/compound, purchased from Chengdu Ddossy Experimental Animals Co., Ltd.

**Experimental method:** on the day of the test, 6 SD rats were randomly grouped according to their body weight; the animals were fasted with water available for 12 to 14 hours one day before the administration of a test compound, and were fed 4 hours after the administration.

Table 3

| Group | Quantity | Administration information | | | | | | |
|---|---|---|---|---|---|---|---|---|
| | Male | Test compound | Administrat ion dosage. (mg/kg) | Administrat ion concentrati on (mg/mL) | Administrati on volume (mL/kg) | Collected samples | Mode of administrati on | Vehicle |
| G1 | 3 | Compound of the present invention | 5 | 0.5 | 10 | Plasma | Oral (intragastric ally) | 5% DMSO+ 5%Solutol+ 30%PEG-400+60% (20%SBE-β-CD) |
| G2 | 3 | Compound of the present invention | 2 | 0.4 | 5 | Plasma | Intravenous injection | 5%DMA+5 %Solutol+9 0% Saline |
| *Dosage is calculated based on free base. | | | | | | | | |

**Sampling:** Before and after the administration, 0.1 mL of blood samples were drawn from the orbits of the animals under isoflurane anesthesia, and placed in an EDTAK2 centrifuge tube. Centrifugation was carried out at 5000 rpm at 4°C for 10 min, and the plasma was collected.

Time points for plasma collection in IV&PO group: 0, 5 min, 15 min, 30 min, 1 h, 2 h, 4 h, 6 h, 8 h, and 24 h.

Before analysis and detection, all samples were stored at -60°C. The samples were analyzed quantitatively by LC-MS/MS.

Table 4 Pharmacokinetic parameters of compounds of the present invention in plasma of rats

| Test compounds | Mode of administration* | $AUC_{0-t}$ (ng/mL·h) | F% |
|---|---|---|---|
| Compound 4 | i.g. (5 mg/kg) | 29429±5590 | 51.2±9.7 |
| Compound 6 | i.g. (5 mg/kg) | 8964±2943 | 47.3±16 |
| Compound 11 | i.g. (5 mg/kg) | 16043±431 | 90.9±2.4 |
| Compound 12 | i.g. (5 mg/kg) | 70521±6979 | 73.0±7.2 |
| Compound 13 | i.g. (5 mg/kg) | 57782±6304 | 77.8±8.5 |
| Compound 15 | i.g. (5 mg/kg) | 10242±1459 | 73.2±10 |
| Compound 20 | i.g. (5 mg/kg) | 15955±5815 | 62.0±23 |
| Compound 21 | i.g. (5 mg/kg) | 15162±2611 | 69.1±12 |
| Compound 29 | i.g. (5 mg/kg) | 48734±7062 | N/A |
| Compound 30 | i.g. (5 mg/kg) | 22516±1513 | 98.1±6.6 |
| Compound 31 | i.g. (5 mg/kg) | 39705±1886 | N/A |
| Compound 33 | i.g. (5 mg/kg) | 9392±916 | 55.3±5.4 |
| Compound 37 | i.g. (5 mg/kg) | 55827±22593 | 69.8±28 |
| Compound 38 | i.g. (5 mg/kg) | 7204±1214 | 43.5±7.3 |
| Compound 47 | i.g. (5 mg/kg) | 11269±3878 | 37.7±13 |
| Compound 118 | i.g. (5 mg/kg) | 13950±816 | 52.4±3.1 |
| Compound 133 | i.g. (5 mg/kg) | 8294±935 | 70.3±7.9 |
| Compound 141 | i.g. (5 mg/kg) | 15658±3196 | 76.1±716 |
| Compound 155 | i.g. (5 mg/kg) | 12043±711 | 37.9±2.2 |
| *Note: i.g. (intragastrical) administration; N/A: not determined | | | |

Conclusion: the compounds of the present invention had a certain degree of oral absorption in rats.

**5. Pharmacokinetic test in mice**

**5.1 Experimental animals:** male ICR mice, 20-25 g, 18 mice/compound. purchased from Chengdu Ddossy Experimental Animals Co., Ltd.

**5.2 Experimental design:** on the day of the test, 18 ICR mice were randomly grouped according to their body weights. The animals were fasted with water available for 12 to 14 hours one day before the administration of a test compound, and were fed 4 hours after the administration.

**Table 5. Administration information**

| Group | Quantity | Administration information | | | | | |
|---|---|---|---|---|---|---|---|
| | Male | Test compound | Administration dosage (mg/kg) | Administration concentration (mg/mL) | Administration volume (mL/kg) | Collected samples | Mode of administration |
| G1 | 9 | Compound of the present invention | 2 | 0.4 | 5 | Plasma | Intravenously |
| G2 | 9 | Compound of the present invention | 5 | 0.5 | 10 | Plasma | Oral administration (gavage) |

Notes: Solvent for intravenous administration: 5%DMA+5%Solutol+90%Saline;
Oral (gavage) administration vehicle: 5%DMSO+5%Solutol+30%PEG400+60% (20%SBE-CD);
*Dosage is calculated based on free base.

Before and after the administration, 0.06 mL of blood was taken from the orbits under isoflurane anesthesia and placed in an EDTAK2 centrifuge tube. The blood was centrifuged at 5000 rpm at 4°C for 10 min, and plasma was collected. The blood collection time points for the intravenous group and intragastric administration group were: 0 min, 5 min, 15 min, 30 min, 1 h, 2 h, 4 h, 6 h, 8 h, and 24 h. Before analysis and detection, all samples were stored at -80°C, and a quantitative analysis of samples was performed using LC-MS/MS.

Conclusion: the compounds of the present invention had a certain degree of oral absorption in mice.

**6. Pharmacokinetic test in beagle dogs**

**Experimental animals:** male beagle dogs, about 8-10 kg, 6 beagle dogs/compound, purchased from Beijing Marshall Biotechnology Co., Ltd.

**Experimental method:** on the day of the test, 6 beagle dogs were randomly grouped according to their body weights. The animals were fasted with water available for 14 to 18 hours one day before the administration of a test compound, and were fed 4 hours after the administration.

**Table 6. Administration information**

| Group | Quantity | Administration information | | | | | |
|---|---|---|---|---|---|---|---|
| | Male | Test compound | Administration dosage (mg/kg) | Administration concentration (mg/mL) | Administration volume (mL/kg) | Collected samples | Mode of administration |
| G1 | 3 | Compound of the present invention | 1 | 1 | 1 | Plasma | Intravenously |
| G2 | 3 | Compound of the present invention | 5 | 1 | 5 | Plasma | Oral administration (gavage) |

Notes: Vehicle for intravenous administration: 5% DMA+5% Solutol+90% Saline;
Oral (gavage) administration vehicle: 5%DMSO+5%Solutol+30%PEG400+60% (20%SBE-CD);
*Dosage is calculated based on free base.

Before and after the administration, 1 ml of blood was taken from the jugular veins or limb veins, and placed in an EDTAK2 centrifuge tube. Centrifugation was carried out at 5000 rpm at 4°C for 10 min, and the plasma was collected. The blood collection time points for the intravenous group and intragastric administration group were: 0 min, 5 min, 15 min, 30 min, 1 h, 2 h, 4 h, 6 h, 8 h, 10 h, 12 h, 24 h, and 48 h. Before analysis and detection, all samples were stored at -80°C. The samples were analyzed quantitatively by LC-MS/MS.

Conclusion: the compounds of the present invention had a certain degree of oral absorption in dogs.

**7. Pharmacokinetic experiment in monkeys**

**Experimental animals:** Male cynomolgus monkeys, 3-5 kg, 3-6 years old, 4 monkeys/compound. Purchased from Suzhou Xishan Biotechnology Inc.

**Experimental method:** on the day of the test, 4 monkeys were randomly grouped according to their body weights. The animals were fasted with water available for 14 to 18 hours one day before the administration of a test compound, and were fed 4 hours after the administration.

**Table 7. Administration information**

| Group | Quantity | Administration information | | | | | |
|---|---|---|---|---|---|---|---|
| | Male | Test compound | Administration dosage (mg/kg) | Administration concentration (mg/mL) | Administration volume (mL/kg) | Collected samples | Mode of administration |
| G1 | 2 | Compound of the present invention | 1 | 1 | 1 | Plasma | Intravenously |
| G2 | 2 | Compound of the present invention | 5 | 1 | 5 | Plasma | Oral administration (gavage) |

Notes: Vehicle for intravenous administration: 5% DMA+5% Solutol+90% Saline;
Oral (gavage) administration vehicle: 5%DMSO+5%Solutol+30%PEG400+60% (20%SBE-CD);
*Dosage is calculated based on free base.

Before and after the administration, 1.0 mL of blood was taken from the limb veins, and placed in an EDTAK2 centrifuge tube. Centrifugation was carried out at 5000 rpm at 4°C for 10 min, and the plasma was collected. The blood collection time points for the intravenous group and intragastric administration group were: 0 min, 5 min, 15 min, 30 min, 1 h, 2 h, 4 h, 6 h, 8 h, 10 h, 12 h, and 24 h. Before analysis and detection, all samples were stored at -80°C, and a quantitative analysis of samples was performed using LC-MS/MS.

Conclusion: the compounds of the present invention had a certain degree of oral absorption in monkeys.

### 8. Test for hERG potassium ion channel

**Test platform:** electrophysiological manual patch-clamp system

**Cell line:** Chinese hamster ovary (CHO) cell lines stably expressing hERG potassium ion channel

**Test method:** In CHO (Chinese Hamster Ovary) cells stably expressing hERG potassium channel, whole cell patch-clamp technique was used to record hERG potassium channel current at room temperature. The glass microelectrode was made of a glass electrode blank (BF150-86-10, Sutter) by a puller. The tip resistance after filling the liquid in the electrode was about 2-5 MΩ. The glass microelectrode can be connected to the patch-clamp amplifier by inserting the glass microelectrode into an amplifier probe. The clamping voltage and data recording were controlled and recorded by the pClamp 10 software through a computer. The sampling frequency was 10 kHz, and the filtering frequency was 2 kHz. After the whole cell records were obtained, the cells were clamped at -80 mV, and the step voltage that induced the hERG potassium current ($I_{\text{hERG}}$) was depolarized from -80 mV to +20 mV for 2 s, then repolarized to -50 mV, and returned to -80 mV after 1 s. This voltage stimulation was given every 10 s, and the administration process was started after the hERG potassium current was confirmed to be stable (at least 1 minute). The compound was administered for at least 1 minute at each test concentration, and at least 2 cells ($n \geq 2$) were tested at each concentration.

**Data processing:** Data analysis processing was carried out by using pClamp 10, GraphPad Prism 5 and Excel software. The inhibition degree of hERG potassium current (peak value of hERG tail current induced at -50 mV) at different compound concentrations was calculated by the following formula:

$$\text{Inhibition\%} = [1 - (I / I\text{o})] \times 100\%$$

wherein, Inhibition% represents the percentage of inhibition of hERG potassium current by the compound, and I and *I*o represent the amplitude of hERG potassium current after and before dosing, respectively.

Compound $IC_{50}$ was calculated using GraphPad Prism 5 software by fitting according to the following equation:

$$Y = \text{Bottom} + (\text{Top-Bottom})/(1+10\text{^}((\text{LogIC}_{50}\text{-X})\text{*HillSlope}))$$

Among the equation, X represents the Log value of the tested concentration of the test sample, Y represents the inhibition percentage at the corresponding concentration, and Bottom and Top represent the minimum and maximum inhibition percentage, respectively.

Conclusion: The compounds of the present invention had no obvious inhibitory effects on hERG potassium channel current.

### 9. Stability test in liver microsomes

This experiment used liver microsomes from five species of humans, dogs, rats and mice as *in vitro* models to evaluate the metabolic stability of the test compound.

At 37°C, 1 μM of the test compound was incubated with microsomal protein and coenzyme NADPH. After the reaction reached a certain time (5 min, 10 min, 20 min, 30 min, and 60 min), ice-cold acetonitrile containing an internal standard was added to terminate the reaction. The LC-MS/MS method was used to detect the concentration of the test compound in the sample, and $T_{1/2}$ was calculated based on the ln value of the residual rate of the drug in the incubation system and the incubation time, and the liver microsome intrinsic clearance rate $CL_{int(mic)}$ and the liver intrinsic clearance rate $CL_{int(Liver)}$ were further calculated.

Conclusion: the compounds of the present invention had good stability in liver microsomes.

**10. CYP450 enzyme inhibition test**

The purpose of this study was to evaluate the effect of the test compound on the activity of five isoenzymes (CYP1A2, CYP2C9, CYP2C19, CYP2D6 and CYP3A4) of human liver microsomal cytochrome P450 (CYP) by using an *in vitro* testing system. The specific probe substrates of CYP450 isoenzymes were incubated with human liver microsomes and test compounds of different concentrations, and reduced nicotinamide adenine dinucleotide phosphate (NADPH) was added to initiate the reaction. After the completion of the reaction, the sample was treated and liquid chromatography-tandem mass spectrometry (LC-MS/MS) was used to quantitatively detect metabolites produced by specific substrates, changes in CYP enzyme activity were determined, and $IC_{50}$ value was calculated to evaluate the inhibitory potential of the test compound on each CYP enzyme subtype.

Conclusion: the compounds of the present invention had no obvious inhibitory effect on the five isoenzymes of human liver microsomal cytochrome P450.

**11. Caco2 permeability test**

The experiment used a monolayer of Caco-2 cells incubated in triplicate in a 96-well Transwell plate. A transport buffer solution (HBSS, 10 mM HEPES, pH 7.4 $\pm$ 0.05) containing the compound of the present invention (2 $\mu$M) or the control compounds of digoxin (10 $\mu$M), nadolol (2 $\mu$M) and metoprolol (2 $\mu$M) was added to the administration end hole on the apical side or the basal side. DMSO-containing transport buffer solution was added to the corresponding receiving end hole. After incubation for 2 h at 37 $\pm$ 1°C, the cell plate was removed and a appropriate amount of samples were taken from the apical side and basal side and transferred to a new 96-well plate. Acetonitrile containing internal standard was then added to precipitate the protein. Samples were analyzed using LC MS/MS and the concentrations of the compounds of the present invention and the control compounds were determined. Concentration data were used to calculate apparent permeability coefficients for transport from the apical side to the basal side, and from the basal side to the apical side of the cell monolayer, and thus to calculate the efflux ratio. The integrity of the cell monolayer after 2 h of incubation was assessed by leakage of Lucifer Yellow.

Conclusion: The compound of the present invention had a certain degree of Caco2 permeability.

**12. Experiment on inhibiting the growth of the mouse 22RV1 subcutaneous transplanted tumor model by the compounds of the present invention**

Cell culture: 22RV1 cells (derived from ATCC) were cultured *in vitro.* The culture conditions included a medium supplemented with 10% fetal calf serum and 10 $\mu$g/mL recombinant insulin at 37°C under 5% $CO_2$. Passaging was performed twice a week. When the cells were maintained in the exponential growth phase, cells were harvested, counted, and inoculated.

Animal: BALB/c nude mice, male, 4-6 weeks old, weighing 14-20 grams, provided by Beijing Vital River Laboratory Animal Technology Co., Ltd.

Tumor inoculation: experimental mice were subcutaneously inoculated with 5 $\times$ $10^6$ 22RV1 cells on the right back, the cells were resuspended in PBS and Matrigel (1 : 1), tumor growth was observed regularly and castration was performed when the tumor grew to an average volume of 80 $mm^3$: the mice were anesthetized with isoflurane, and surgical castration was performed through a midline incision in the scrotum, allowing bilateral access; after each testicle was exposed, the spermatic cord was ligated with 5-0 vicyryl sutures, and then the testicles were removed and the scrotum and skin were sutured with 5-0 vicyryl respectively; after surgery, the animals needed to be observed until they woke up completely. After castration, the tumor might shrink (tumor regression). Grouping and treatment would begin when the tumor re-grew to an average volume of about 100 $mm^3$. The day of grouping was Day 0.

Administration: The compound of the present invention was administered at a certain dose orally (PO), once daily (QD), with 10 mice in each group. All groups were continuously administered for 28 days.

Experiment observation and endpoint: body weights and tumors of the mice were measured 3 times a week. The experiment was terminated after 28-day administration, and all mice were euthanized. The calculation formula of tumor volume was: Tumor volume ($mm^3$) = 1/2 $\times$ (a $\times$ $b^2$) (where a represented the long diameter and b represented the short diameter), and the raw data were measured by a balance and a vernier caliper.

Conclusion: The compounds of the present invention had a certain degree of inhibitory effect on the growth of the mouse 22RV1 subcutaneous transplanted tumor model.

## Claims

**1.** A compound or a stereoisomer, deuterated compound, solvate, prodrug, metabolite, pharmaceutically acceptable

salt or co-crystal thereof, **characterized in that** the compound is selected from a compound as represented by general formula (I),

B-L-K (I)

;

L is selected from a bond or -$C_{1-50}$ hydrocarbyl-, wherein the hydrocarbyl has 0 to 20 methylene units optionally replaced by -Ak- or -Cy-;
each -Ak- is independently selected from -$(CH_2)_q$-, -$(CH_2)_q$-O-, -O-$(CH_2)_q$-, - $(CH_2)_q$-$NR^L$-, -$NR^L$-$(CH_2)_q$-, -$(CH_2)_q$-$NR^L$C(=O)-, -$NR^L(CH_2)_q$C(=O)-, -$(CH_2)_q$-C(=O)$NR^L$-, -C(=O)-, -C(=O)-$(CH_2)_q$-$NR^L$-, -$(C{\equiv}C)_q$-, -CH=CH-, -Si$(R^L)_2$-, - Si(OH)$(R^L)$-, -Si$(OH)_2$-, -P(=O)$(OR^L)$-, -P(=O)$(R^L)$-, -S-, -S(=O)-, -S$(=O)_2$- or a bond, wherein the -$CH_2$- or -CH=CH- is optionally substituted with 0 to 2 substituents selected from H, halogen, OH, CN, $NH_2$, $C_{1-6}$ alkyl, $C_{1-6}$ alkoxy, halogen-substituted $C_{1-6}$ alkyl, hydroxyl-substituted $C_{1-6}$ alkyl or cyano-substituted $C_{1-6}$ alkyl;
each q is independently selected from 0, 1, 2, 3, 4, 5 or 6;
each $R^L$ is independently selected from H, $C_{1-6}$ alkyl, 3- to 7-membered heterocyclyl, 3- to 7-membered cycloalkyl, phenyl or 5- to 6-membered heteroaryl;
each -Cy- is independently selected from a bond, 4- to 8-membered mono-heterocyclic ring group, 4- to 10-membered fused heterocyclic ring group, 5- to 12-membered spiro-heterocyclic ring group, 7- to 10-membered bridged-heterocyclic ring group, 3- to 7-membered monocycloalkyl, 4- to 10-membered fused cycloalkyl, 5- to 12-membered spirocycloalkyl, 7- to 10-membered bridged cycloalkyl, 5- to 10-membered heteroaryl or 6- to 10-membered aryl, the aryl, heteroaryl, cycloalkyl, mono-heterocyclic ring group, fused heterocyclic ring group, spiro-heterocyclic ring group or bridged-heterocyclic ring group is optionally substituted with 0 to 4 substituents selected from H, F, Cl, Br, I, OH, COOH, CN, $NH_2$, =O, $C_{1-4}$ alkyl, halogen-substituted $C_{1-4}$ alkyl, hydroxyl-substituted $C_{1-4}$ alkyl or $C_{1-4}$ alkoxy, and the heteroaryl, mono-heterocyclic ring group, fused heterocyclic ring group, spiro-heterocyclic ring group or bridged-heterocyclic ring group contains 1 to 4 heteroatoms selected from O, S or N, and is optionally substituted with 0, 1 or 2 =O when the heteroatom is selected from S;
B is selected from

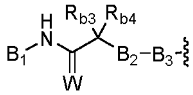;

W is selected from O or S;
$B_1$ is selected from $C_{6-10}$ carbocyclyl or 5- to 10-membered heterocyclyl, the carbocyclyl or heterocyclyl is optionally substituted with 0 to 2 $R^{bb}$, the carbocyclyl or heterocyclyl is optionally substituted with 0 to 4 $R^{b1}$, and the heterocyclyl contains 1 to 4 heteroatoms selected from O, S or N;
$B_2$ is selected from -NHC(=O)- or 5- to 10-membered heterocyclyl, the heterocyclyl is optionally substituted with 0 to 4 $R^{b2}$, and the heterocyclyl contains 1 to 4 heteroatoms selected from O, S or N;
$B_3$ is selected from a bond, phenyl or 5- to 6-membered heteroaryl, and the phenyl and 5- to 6-membered heteroaryl are optionally substituted with 0 to 4 $R^{b2}$,
$R^{b1}$ and $R^{b2}$ are each independently selected from H, F, Cl, Br, I, =O, OH, $NH_2$, CN, $NO_2$, $CF_3$, -C(=O)$NH_2$, -C(=O)NH-$C_{1-4}$ alkyl, -C(=O)N$(C_{1-4}$ alkyl$)_2$, $C_{1-4}$ alkyl, $C_{1-4}$ alkoxy, or $C_{3-6}$ cycloalkyl, and the alkyl, alkoxy or cycloalkyl is optionally substituted with 0 to 4 substituents selected from H, F, Cl, Br, I or OH;
$R^{bb}$ is selected from -T-P$(=O)_2(C_{1-4}$ alkyl$)_2$, -T-S$(=O)_2C_{1-4}$ alkyl, -T-P$(=O)_2(C_{3-6}$ cycloalkyl$)_2$, -T-S$(=O)_2C_{3-6}$ cycloalkyl, -T-C(=O)$NH_2$, -T-C(=O)NH-$C_{1-4}$ alkyl, -T-C(=O)N$(C_{1-4}$ alkyl$)_2$, -T-$C_{1-4}$ alkyl, -T-$C_{2-6}$ alkenyl, -T-$C_{2-6}$ alkynyl, - T- $C_{3-12}$ carbocycle, or -T-3- to 12-membered heterocycle, the alkyl, alkenyl, alkynyl, carbocycle or heterocycle is optionally substituted with 0 to 4 substituents selected from H, F, Cl, Br, I, OH, $NH_2$, =O, halogen-substituted $C_{1-4}$ alkyl, cyano-substituted $C_{1-4}$ alkyl, hydroxyl-substituted $C_{1-4}$ alkyl, $C_{1-4}$ alkyl, $C_{1-4}$ alkoxy, $C_{3-6}$ cycloalkyl or 3- to 7-membered heterocycle, the heterocycle contains 1 to 4 heteroatoms selected from O, S or N;
T is selected from a bond, -C(=O)-, -P$(=O)_2$-, -S$(=O)_2$-, -C(=O)NH-, - P$(=O)_2$NH-, -S$(=O)_2$NH-, -C(=O)N($C_{1-4}$ alkyl)-, -P$(=O)_2$N($C_{1-4}$ alkyl)-, - S$(=O)_2$N($C_{1-4}$ alkyl)-, -NHC(=O)-, -NHP$(=O)_2$-, -NHS$(=O)_2$-, -N($C_{1-4}$ alkyl)C(=O)-, -N($C_{1-4}$ alkyl)P$(=O)_2$-, -N($C_{1-4}$ alkyl)S$(=O)_2$-, O, S, -NH-, -N($C_{1-4}$ alkyl)-, -N($C_{3-6}$ cycloalkyl)-, -N(3- to 10-membered heterocycle)-, -$C_{1-4}$ alkylene-, -O-$C_{1-4}$ alkylene-, -$C_{1-4}$ alkylene-O-, -S-$C_{1-4}$ alkylene-, -NH-$C_{1-4}$ alkylene-, -N($C_{1-4}$ alkyl)-$C_{1-4}$ alkylene-, or -O-$C_{1-4}$ alkylene-O-, and the alkyl or alkylene is optionally substituted with 0 to 4 substituents selected from H, F, Cl, Br, I, OH, =O, $C_{1-4}$ alkyl, $C_{1-4}$ alkoxy, $C_{3-6}$ cycloalkyl or 3- to 7-membered

heterocycle, the heterocycle contains 1 to 4 heteroatoms selected from O, S or N;

alternatively, 2 $R^{bb}$ together with the atom to which they are attached form $C_{4-12}$ carbocycle or 4- to 12-membered heterocycle, the carbocycle or heterocycle is optionally substituted with 0 to 4 substituents selected from H, F, Cl, Br, I, OH, $NH_2$, =O, halogen-substituted $C_{1-4}$ alkyl, cyano-substituted $C_{1-4}$ alkyl, hydroxyl-substituted $C_{1-4}$ alkyl, $C_{1-4}$ alkyl, $C_{1-4}$ alkoxy, $C_{3-6}$ cycloalkyl or 3- to 7-membered heterocycle, and the heterocycle contains 1 to 4 heteroatoms selected from O, S or N;

$R^{b3}$ and $R^{b4}$ are each independently selected from H or $C_{1-6}$ alkyl, and the alkyl is optionally substituted with 0 to 4 substituents selected from H, F, Cl, Br, I, OH, $NH_2$, $C_{1-4}$ alkyl or $C_{1-4}$ alkoxy;

alternatively, $R^{b3}$ and $R^{b4}$ together with the carbon atom to which they are attached form $C_{3-6}$ cycloalkyl or 3- to 8-membered mono-heterocyclic ring, the cycloalkyl or mono-heterocyclic ring is optionally substituted with 0 to 4 substituents selected from H, F, Cl, Br, I, OH, $NH_2$, $C_{1-4}$ alkyl or $C_{1-4}$ alkoxy, and the mono-heterocyclic ring contains 1 to 4 heteroatoms selected from O, S or N;

K is selected from

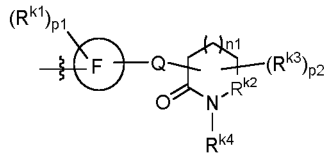 , 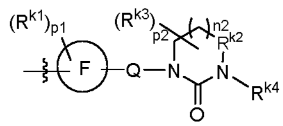 ,

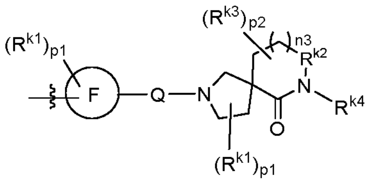 , 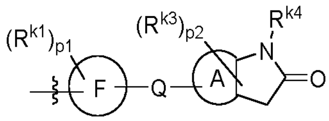 , 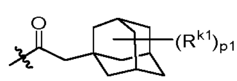 ,

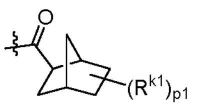 , 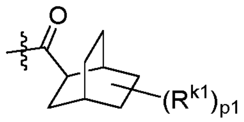 , 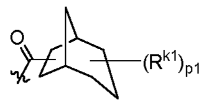 , 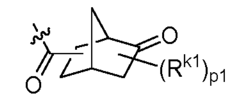 ,

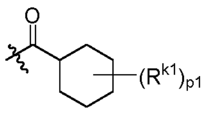 , 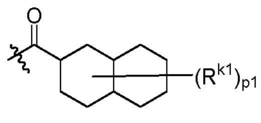 , 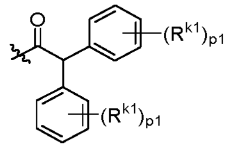 , 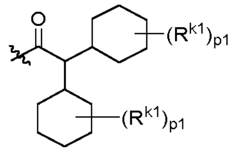 ,

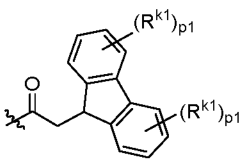 , 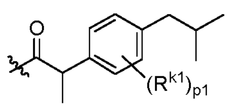 , 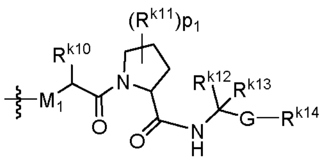 ,

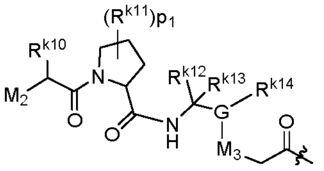 or 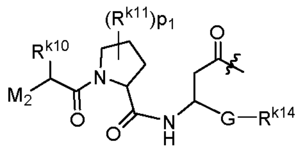 ;

each Q is independently selected from a bond, -O-, -S-, $-CH_2-$, $-NR^q-$, - C(=O)-, $-NR^qC(=O)-$, $-C(=O)NR^q-$ or 3- to 12-membered heterocyclyl, the heterocyclyl is optionally substituted with 0 to 4 substituents selected from

H, F, Cl, Br, I, OH, =O, $NH_2$, CN, COOH, $CONH_2$, $C_{1-4}$ alkyl or $C_{1-4}$ alkoxy, and the heterocyclyl contains 1 to 4 heteroatoms selected from O, S or N;

$R^q$ is selected from H or $C_{1-6}$ alkyl;

A is selected from $C_{3-10}$ carbocyclyl, $C_{6-10}$ aryl, 3- to 10-membered heterocyclyl or 5- to 10-membered heteroaryl, wherein the heterocycle or heteroaryl contains 1 to 4 heteroatoms selected from O, S or N;

each F is independently selected from $C_{3-20}$ carbocyclyl, $C_{6-20}$ aryl, 3- to 20-membered heterocyclyl or 5- to 20-membered heteroaryl, and the heterocyclyl or heteroaryl contains 1 to 4 heteroatoms selected from O, S or N;

each $R^{k2}$ is independently selected from a bond, -C(=O)-, -S(=O)$_2$-, -S(=O)- or -C($R^{k3}$)$_2$-;

each $R^{k1}$ is independently selected from H, F, Cl, Br, I, OH, =O, $NH_2$, CN, COOH, $CONH_2$, $C_{1-6}$ alkyl or $C_{1-6}$ alkoxy, and the alkyl or alkoxy is optionally substituted with 0 to 4 substituents selected from H, F, Cl, Br, I, OH, =O, $NH_2$, CN, COOH, $CONH_2$, $C_{1-4}$ alkyl or $C_{1-4}$ alkoxy;

each $R^{k3}$ is independently selected from H, F, Cl, Br, I, OH, =O, $NH_2$, CN, COOH, $CONH_2$, $C_{1-6}$ alkyl, $C_{1-6}$ alkoxy, $C_{3-8}$ cycloalkyl or 3- to 8-membered heterocyclyl, the alkyl, alkoxy, cycloalkyl or heterocyclyl is optionally substituted with 0 to 4 substituents selected from H, F, Cl, Br, I, OH, =O, $NH_2$, CN, COOH, $CONH_2$, $C_{1-4}$ alkyl or $C_{1-4}$ alkoxy, and the heterocyclyl contains 1 to 4 heteroatoms selected from O, S or N;

alternatively, two $R^{k3}$ together with the carbon atom or ring backbone to which they are directly attached form 3- to 8-membered carbocycle or 3- to 8-membered heterocycle, two $R^{k1}$ together with the carbon atom or ring backbone to which they are directly attached form 3- to 8-membered carbocycle or 3- to 8-membered heterocycle, the carbocycle or heterocycle is optionally substituted with 0 to 4 substituents selected from H, F, Cl, Br, I, OH, =O, $NH_2$, CN, COOH, $CONH_2$, $C_{1-4}$ alkyl or $C_{1-4}$ alkoxy, and the heterocycle contains 1 to 4 heteroatoms selected from O, S or N;

each $R^{k4}$ is independently selected from H, OH, $NH_2$, CN, $CONH_2$, $C_{1-6}$ alkyl, $C_{3-8}$ cycloalkyl or 3- to 8-membered heterocyclyl, the alkyl, cycloalkyl or heterocyclyl is optionally substituted with 0 to 4 substituents selected from H, F, Cl, Br, I, OH, =O, $NH_2$, CN, COOH, $CONH_2$, $C_{1-4}$ alkyl or $C_{1-4}$ alkoxy, and the heterocyclyl contains 1 to 4 heteroatoms selected from O, S or N;

$M_1$ is selected from a bond, -C(=O)NH-, -NHC(=O)-, -$CH_2$-C(=O)NH-, - C(=O)$CH_2$NH-, or 5- to 6-membered heteroaryl, the heteroaryl is optionally substituted with 0 to 4 substituents selected from H, F, Cl, Br, I, OH, =O, $CF_3$, $NH_2$, CN, $C_{1-4}$ alkyl, halogen-substituted $C_{1-4}$ alkyl, hydroxyl-substituted $C_{1-4}$ alkyl, or $C_{1-4}$ alkoxy, and the heteroaryl contains 1 to 4 heteroatoms selected from O, S or N;

$M_2$ is selected from -NHC(=O)-$C_{1-6}$ alkyl, -NHC(=O)-$C_{3-6}$ cycloalkyl or 4- to 10-membered heterocyclyl, the alkyl, cycloalkyl or heterocyclyl is optionally substituted with 0 to 4 substituents selected from H, F, Cl, Br, I, =O, OH, $NH_2$, $C_{1-4}$ alkyl or $C_{1-4}$ alkoxy, and the heterocyclyl contains 1 to 4 heteroatoms selected from O, S or N;

$M_3$ is selected from -NH- or -O-;

$R^{k10}$ is selected from $C_{1-6}$ alkyl, the alkyl is optionally substituted with 0 to 4 substituents selected from H, F, Cl, Br, I, =O, OH, $C_{1-6}$ alkyl or $C_{3-6}$ cycloalkyl;

each $R^{k11}$ is independently selected from H, F, Cl, Br, I, =O, OH, SH, $C_{1-6}$ alkyl, $C_{1-6}$ alkoxy or $C_{1-6}$ alkylthio or -O-C(=O)-$C_{1-6}$ alkyl, and the alkyl, alkoxy or alkylthio is optionally substituted with 0 to 4 substituents selected from H, F, Cl, Br, I, OH, $C_{1-4}$ alkyl or $C_{1-4}$ alkoxy;

$R^{k12}$ and $R^{k13}$ are each independently selected from H, $C_{1-6}$ alkyl or $C_{3-6}$ cycloalkyl, and the alkyl or cycloalkyl is optionally substituted with 0 to 4 substituents selected from H, F, Cl, Br, I, =O, OH, $NH_2$, $C_{1-4}$ alkyl or $C_{1-4}$ alkoxy;

$R^{k14}$ is selected from 5- to 6-membered heteroaryl, the heteroaryl is optionally substituted with 0 to 4 substituents selected from H, F, Cl, Br, I, OH, =O, $CF_3$, CN, $C_{1-4}$ alkyl, halogen-substituted $C_{1-4}$ alkyl, hydroxyl-substituted $C_{1-4}$ alkyl, $C_{1-4}$ alkoxy or $C_{3-6}$ cycloalkyl, and the heteroaryl contains 1 to 4 heteroatoms selected from N, O and S;

G is selected from 6- to 10-membered aryl or 5- to 10-membered heteroaryl, the aryl or heteroaryl is optionally substituted with 0 to 4 substituents selected from H, F, Cl, Br, I, OH, =O, $CF_3$, CN, $C_{1-4}$ alkyl, halogen-substituted $C_{1-4}$ alkyl, hydroxyl-substituted $C_{1-4}$ alkyl, $C_{1-4}$ alkoxy or $C_{3-6}$ cycloalkyl, and the heteroaryl contains 1 to 4 heteroatoms selected from N, O and S;

n1, n2 and n3 are each independently selected from 0, 1, 2 or 3;

each p1 or p2 is independently selected from 0, 1, 2, 3, 4 or 5;

optionally, 1 to 30 H contained in the deuterated compound are replaced by D;

provided that the compound of general formula (I) is not a compound shown in Table A-1.

**2.** The compound or the stereoisomer, deuterated compound, solvate, prodrug, metabolite, pharmaceutically acceptable salt or co-crystal thereof according to claim 1, **characterized in that**,

$B_1$ is selected from $C_{6-10}$ carbocyclyl or 5- to 10-membered heterocyclyl, the carbocyclyl or heterocyclyl is optionally substituted with 0 to 4 $R^{b1}$, and the heterocyclyl contains 1 to 4 heteroatoms selected from O, S or N;

$B_2$ is selected from -NHC(=O)- or 5- to 10-membered heterocyclyl, the heterocyclyl is optionally substituted with

0 to 4 $R^{b2}$, and the heterocyclyl contains 1 to 4 heteroatoms selected from O, S or N;

$B_3$ is selected from a bond, phenyl or 5- to 6-membered heteroaryl, and the phenyl and 5- to 6-membered heteroaryl are optionally substituted with 0 to 4 $R^{b2}$,

$R^{b1}$ and $R^{b2}$ are each independently selected from H, F, Cl, Br, I, =O, OH, $NH_2$, CN, $NO_2$, $CF_3$, -C(=O)$NH_2$, -C(=O)NH-$C_{1-4}$ alkyl, -C(=O)N($C_{1-4}$ alkyl)$_2$, $C_{1-4}$ alkyl, $C_{1-4}$ alkoxy, or $C_{3-6}$ cycloalkyl, and the alkyl, alkoxy or cycloalkyl is optionally substituted with 0 to 4 substituents selected from H, F, Cl, Br, I or OH;

$R^{b3}$ and $R^{b4}$ are each independently selected from H or $C_{1-6}$ alkyl, and the alkyl is optionally substituted with 0 to 4 substituents selected from H, F, Cl, Br, I, OH, $NH_2$, $C_{1-4}$ alkyl or $C_{1-4}$ alkoxy;

alternatively, $R^{b3}$ and $R^{b4}$ together with the carbon atom to which they are attached form $C_{3-6}$ cycloalkyl or 3- to 8-membered mono-heterocyclic ring group, the cycloalkyl or mono-heterocyclic ring group is optionally substituted with 0 to 4 substituents selected from H, F, Cl, Br, I, OH, $NH_2$, $C_{1-4}$ alkyl or $C_{1-4}$ alkoxy, and the mono-heterocyclic ring contains 1 to 4 heteroatoms selected from O, S or N.

**3.** The compound or the stereoisomer, deuterated compound, solvate, prodrug, metabolite, pharmaceutically acceptable salt or co-crystal thereof according to claim 2, **characterized in that**,

L is selected from -Cy1-Ak1-Cy2-Ak2-Cy3-Ak3-Cy4-Ak4-Cy5-Ak5-, -Cy1-Cy2-Cy3-Cy4-Ak1-Ak2-Ak3-Ak4-Ak5-, -Cy1-Ak1-Cy2-Ak2-Cy3-Ak3-Cy4-Ak4-Ak5-, -Ak1-Cy1-Ak2-Cy2-Ak3-Cy3-Ak4-Cy4-Ak5-, -Cy1-Ak1-Cy2-Ak2-Cy3-Cy4-Ak3-Ak4-Ak5-, -Cy1-Ak1-Cy2-Ak2-Ak3-Cy3-Cy4-Ak4-Ak5-, -Cy1-Ak1-Ak2-Ak3-Ak4-Ak5-Cy2-Cy3-Cy4-, -Cy1-Cy2-Ak1-Ak2-Ak3-Ak4-Ak5-Cy3-Cy4-, -Cy1-Cy2-Cy3-Ak1-Ak2-Ak3-Ak4-Ak5-Cy4-, -Cy1-Cy2-Cy3-Cy4-Ak1-Ak2-Ak3-Ak4-Ak5-, -Cy1-Ak1-Cy2-Cy3-Cy4-Ak2-Ak3-Ak4-Ak5-, -Cy1-Cy2-Ak1-Cy3-Cy4-Ak2-Ak3-Ak4-Ak5-, -Cy1-Cy2-Cy3-Ak1-Cy4-Ak2-Ak3-Ak4-Ak5-, - Cy1-Ak1-Ak2-Cy2-Cy3-Cy4-Ak3-Ak4-Ak5-, -Cy1-Cy2-Ak1-Ak2-Cy3-Cy4-Ak3-Ak4-Ak5-, -Cy1-Cy2-Cy3-Ak1-Ak2-Cy4-Ak3-Ak4-Ak5-, -Cy1-Ak1-Ak2-Ak3-Cy2-Cy3-Cy4-Ak4-Ak5-, -Cy1-Cy2-Ak1-Ak2-Ak3-Cy3-Cy4-Ak4-Ak5-, -Cy1-Cy2-Cy3-Ak1-Ak2-Ak3-Cy4-Ak4-Ak5-, -Cy1-Ak1-Ak2-Ak3-Ak4-Cy2-Cy3-Cy4-Ak5-, -Cy1-Cy2-Ak1-Ak2-Ak3-Ak4-Cy3-Cy4-Ak5-, -Cy1-Cy2-Cy3-Ak1-Ak2-Ak3-Ak4-Cy4-Ak5-, -Ak1-Ak2-Ak3-Ak4-Ak5-Cy1-Cy2-Cy3-Cy4-, -Ak1-Cy1-Cy2-Cy3-Cy4-Ak2-Ak3-Ak4-Ak5-, -Ak1-Ak2-Cy1-Cy2-Cy3-Cy4-Ak3-Ak4-Ak5- , -Ak1-Ak2-Ak3-Cy1-Cy2-Cy3-Cy4-Ak4-Ak5-, -Ak1-Ak2-Ak3-Ak4-Cy1-Cy2-Cy3-Cy4-Ak5-, -Ak1-Cy1-Ak2-Ak3-Ak4-Ak5-Cy2-Cy3-Cy4-, -Ak1-Cy1-Cy2-Ak2-Ak3 - Ak4-Ak5 -Cy 3 -Cy4-, -Ak1-Cy1 -Cy2-Cy 3 - Ak2-Ak3 - Ak4-Ak5 -Cy4-, - Ak1-Ak2-Cy1-Ak3-Ak4-Ak5-Cy2-Cy3-Cy4-, -Ak1-Ak2-Cy1-Cy2-Ak3-Ak4-Ak5-Cy3-Cy4-, -Ak1-Ak2-Cy1-Cy2-Cy3-Ak3-Ak4-Ak5-Cy4-, -Ak1-Ak2-Ak3-Cy1-Ak4-Ak5-Cy2-Cy3-Cy4-, -Ak1-Ak2-Ak3-Cy1-Cy2-Ak4-Ak5-Cy3-Cy4-, -Ak1-Ak2-Ak3-Cy1-Cy2-Cy3-Ak4-Ak5-Cy4-, -Ak1-Ak2-Ak3-Ak4-Cy1-Ak5-Cy2-Cy3-Cy4-, -Ak1-Ak2-Ak3-Ak4-Cy1-Cy2-Ak5-Cy3-Cy4-, or -Ak1-Ak2-Ak3-Ak4-Cy1-Cy2-Cy3-Ak5-Cy4-;

Ak1, Ak2, Ak3, Ak4, and Ak5 are each independently selected from -$(CH_2)_q$-, -$(CH_2)_q$-O-, -O-$(CH_2)_q$-, -$(CH_2)_q$-$NR^L$-, -$NR^L$-$(CH_2)_q$-, -$(CH_2)$q-$NR^L$C(=O)-, - $(CH_2)_q$-C(=O)$NR^L$-, -C(=O)-, -C(=O)-$(CH_2)_q$-$NR^L$-, -CH=CH-, -$(C\equiv C)_q$- or a bond, and the -$CH_2$- or -CH=CH- is optionally substituted with 0 to 2 substituents selected from H, halogen, OH, CN, $NH_2$, $C_{1-4}$ alkyl, $C_{1-4}$ alkoxy, halogen-substituted $C_{1-4}$ alkyl, hydroxyl-substituted $C_{1-4}$ alkyl, or cyano-substituted $C_{1-4}$ alkyl;

each Cy1, Cy2, Cy3, Cy4 or Cy5 is independently selected from a bond, 4- to 7-membered mono-heterocyclic ring group, 4- to 10-membered fused heterocyclic ring group, 5- to 12-membered spiro-heterocyclic ring group, 7- to 10-membered bridged-heterocyclic ring group, 3- to 7-membered monocycloalkyl, 4- to 10-membered fused cycloalkyl, 5- to 12-membered spirocycloalkyl, 7- to 10-membered bridged cycloalkyl, 5- to 10-membered heteroaryl or 6- to 10-membered aryl, the aryl, heteroaryl, cycloalkyl, mono-heterocyclic ring group, fused heterocyclic ring group, spiro-heterocyclic ring group or bridged-heterocyclic ring group is optionally substituted with 0 to 4 substituents selected from H, F, Cl, Br, I, OH, COOH, CN, $NH_2$, =O, $C_{1-4}$ alkyl, halogen-substituted $C_{1-4}$ alkyl, hydroxyl-substituted $C_{1-4}$ alkyl or $C_{1-4}$ alkoxy, and the heteroaryl, mono-heterocyclic ring group, fused heterocyclic ring group, spiro-heterocyclic ring group or bridged-heterocyclic ring group contains 1 to 4 heteroatoms selected from O, S or N, and is optionally substituted with 0, 1 or 2 =O when the heteroatom is selected from S;

each q is independently selected from 0, 1, 2, 3 or 4;

each $R^L$ is independently selected from H or $C_{1-6}$ alkyl.

**4.** The compound or the stereoisomer, deuterated compound, solvate, prodrug, metabolite, pharmaceutically acceptable salt or co-crystal thereof according to claim 3, **characterized in that**,

each Cy1, Cy2, Cy3, Cy4 or Cy5 is independently selected from a bond, 4- to 7-membered nitrogen-containing mono-heterocyclic ring group, 4- to 10-membered nitrogen-containing fused heterocyclic ring group, 5- to 12-membered nitrogen-containing spiro-heterocyclic ring group, 7- to 10-membered nitrogen-containing bridged-heterocyclic ring group, 3- to 7-membered monocycloalkyl, 4-to 10-membered fused cycloalkyl, 5- to 12-membered spirocycloalkyl, 7- to 10-membered bridged cycloalky, 5- to 10-membered heteroaryl or 6- to 10-membered aryl, the mono-heterocyclic ring group, fused heterocyclic ring group, bridged-heterocyclic ring group, spiro-heterocyclic ring group, cycloalkyl, aryl or heteroaryl is optionally substituted with 0 to 4 substituents selected from H, F, Cl, Br, I, OH, COOH, CN, $NH_2$, =O, $C_{1-4}$ alkyl, halogen-substituted $C_{1-4}$ alkyl, hydroxyl-substituted $C_{1-4}$ alkyl or $C_{1-4}$ alkoxy, and the mono-heterocyclic ring group, fused heterocyclic ring group, bridged-heterocyclic ring group, spiro-heterocyclic ring group or heteroaryl contains 1 to 4 heteroatoms selected from O, S or N, and is optionally substituted with 0, 1 or 2 =O when the heteroatom is selected from S;

each $R^L$ is independently selected from H or $C_{1-4}$ alkyl;

K is selected from

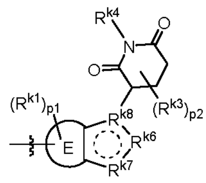 ,

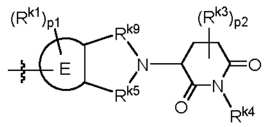 ,

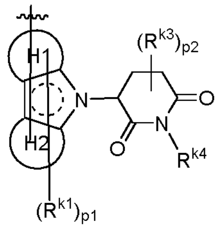 ,

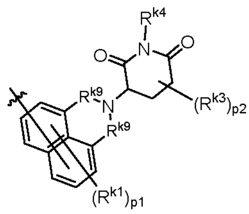 ,

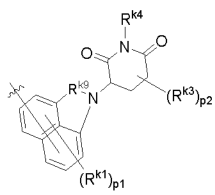 ,

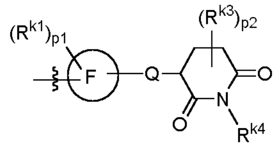 ,

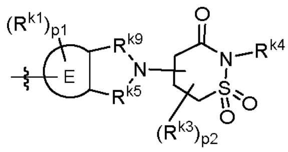 ,

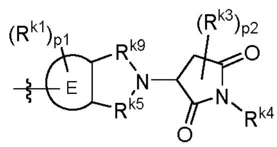 ,

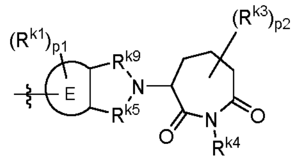 ,

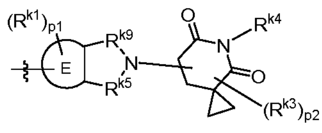 ,

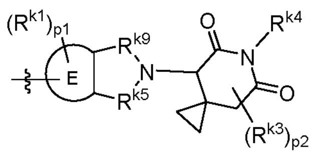 ,

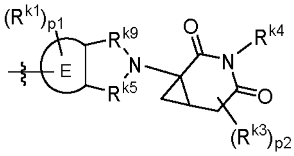 ,

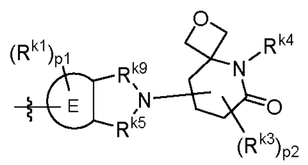 ,

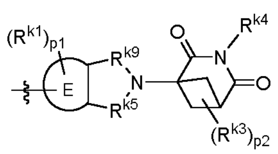 ,

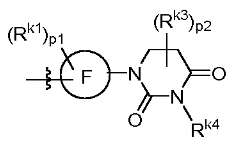 ,

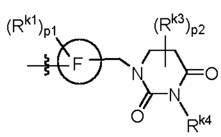 ,

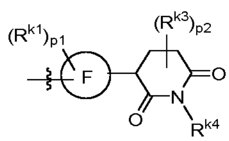 ,

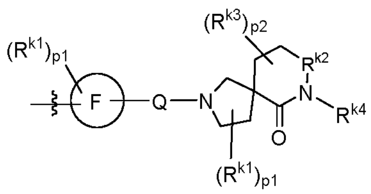 ,

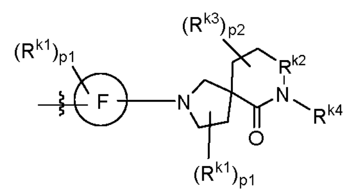 ,

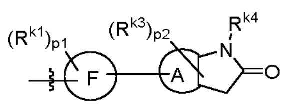 ,

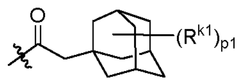,
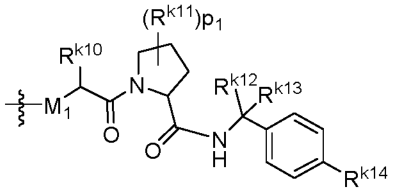,
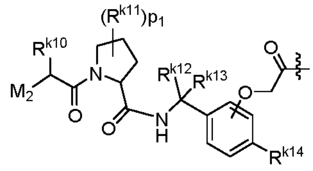,

or

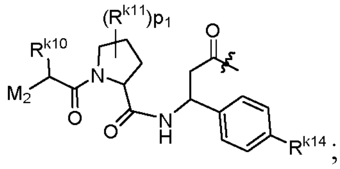;

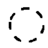

represents a ring selected from an aromatic ring or a non-aromatic ring;

$M_2$ is selected from -NHC(=O)-$C_{1-4}$ alkyl, -NHC(=O)-$C_{3-6}$ cycloalkyl or 4- to 10-membered heterocyclyl, the alkyl, cycloalkyl or heterocyclyl is optionally substituted with 0 to 4 substituents selected from H, F, Cl, Br, I, =O, OH, $NH_2$, $C_{1-4}$ alkyl or $C_{1-4}$ alkoxy, and the heterocyclyl contains 1 to 4 heteroatoms selected from O, S or N;

$R^{k10}$ is selected from $C_{1-4}$ alkyl, and the alkyl is optionally substituted with 0 to 4 substituents selected from H, F, Cl, Br, I, =O, OH, $C_{1-4}$ alkyl or $C_{3-6}$ cycloalkyl;

each $R^{k11}$ is independently selected from H, F, Cl, Br, I, =O, OH, SH, $C_{1-4}$ alkyl, $C_{1-4}$ alkoxy or $C_{1-4}$ alkylthio or -O-C(=O)-$C_{1-4}$ alkyl, and the alkyl, alkoxy or alkylthio is optionally substituted with 0 to 4 substituents selected from H, F, Cl, Br, I, OH, $C_{1-4}$ alkyl or $C_{1-4}$ alkoxy;

$R^{k12}$ and $R^{k13}$ are each independently selected from H, $C_{1-4}$ alkyl or $C_{3-6}$ cycloalkyl, and the alkyl or cycloalkyl is optionally substituted with 0 to 4 substituents selected from H, F, Cl, Br, I, =O, OH, $NH_2$, $C_{1-4}$ alkyl or $C_{1-4}$ alkoxy;

each Q is independently selected from -O-, -S-, -$CH_2$-, -$NR^q$-, -C(=O)-, - $NR^q$C(=O)-, -C(=O)$NR^q$- or 4- to 7-membered heterocyclyl, the heterocyclyl is optionally substituted with 0 to 4 substituents selected from H, F, Cl, Br, I, OH, =O, $NH_2$, CN, COOH, $CONH_2$, $C_{1-4}$ alkyl or $C_{1-4}$ alkoxy, and the heterocyclyl contains 1 to 4 heteroatoms selected from O, S or N;

$R^q$ is selected from H or $C_{1-4}$ alkyl;

$R^{k1}$ and $R^{k3}$ are each independently selected from H, F, Cl, Br, I, OH, =O, $NH_2$, $CF_3$, CN, COOH, $CONH_2$, $C_{1-4}$ alkyl or $C_{1-4}$ alkoxy, and the alkyl or alkoxy is optionally substituted with 0 to 4 substituents selected from H, F, Cl, Br, I, OH, or $NH_2$;

alternatively, two $R^{k3}$ together with the carbon atom or ring backbone to which they are directly attached form 3- to 6-membered carbocycle or 3- to 7-membered heterocycle, two $R^{k1}$ together with the carbon atom or ring backbone to which they are directly attached form 3- to 6-membered carbocycle or 3- to 7-membered heterocycle, the carbocycle or heterocycle is optionally substituted with 0 to 4 substituents selected from H, F, Cl, Br, I, OH, =O, $NH_2$, CN, COOH, $CONH_2$, $C_{1-4}$ alkyl or $C_{1-4}$ alkoxy, and the heterocycle contains 1 to 4 heteroatoms selected from O, S or N;

each $R^{k4}$ is independently selected from H, OH, $NH_2$, $CF_3$, CN or $C_{1-4}$ alkyl;

each $R^{k5}$ is independently selected from C(=O), $CH_2$, $S(=O)_2$,

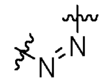,

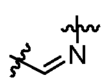, or 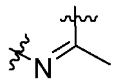;

each $R^{k6}$ is independently selected from C(=O), CH, S(=O), $S(=O)_2$, $CH_2$ or N;

each $R^{k7}$ is independently selected from C(=O), CH, N, $CH_2$, O, S, $N(CH_3)$ or NH;
each $R^{k8}$ is independently selected from C, N or CH;
each $R^{k9}$ is independently selected from C(=O), $CH_2$ or $S(=O)_2$;
each A, H1 or H2 is independently selected from $C_{3-8}$ carbocyclyl, phenyl, 4-to 7-membered heterocyclyl or 5- to 6-membered heteroaryl, and the heterocyclyl or heteroaryl contains 1 to 4 heteroatoms selected from O, S or N;
each E is independently selected from $C_{3-8}$ carbocyclyl, phenyl, 4- to 7-membered heterocyclyl, 8- to 12-membered heterocyclyl, 7- to 12-membered heteroaryl or 5- to 6-membered heteroaryl, and the heterocycle or heteroaryl contains 1 to 4 heteroatoms selected from O, S or N;
each F is independently selected from 3- to 7-membered monocycloalkyl, 4-to 10-membered fused cycloalkyl, 5- to 12-membered spirocycloalkyl, 5- to 10-membered bridged cycloalky, 4- to 7-membered mono-heterocyclic ring group, 4-to 10-membered fused heterocyclic ring group, 5- to 12-membered spiro-heterocyclic ring group, 5- to 10-membered bridged-heterocyclic ring group, $C_{6-14}$ aryl or 5- to 10-membered heteroaryl, and the mono-heterocyclic ring group, fused heterocyclic ring group, spiro-heterocyclic ring group, bridged-heterocyclic ring group or heteroaryl contains 1 to 4 heteroatoms selected from O, S or N.

**5.** The compound or the stereoisomer, deuterated compound, solvate, prodrug, metabolite, pharmaceutically acceptable salt or co-crystal thereof according to claim 4, **characterized in that**,

$R^L$ is selected from H, methyl or ethyl;
each q is independently selected from 0, 1 or 2;
each Cy1, Cy2, Cy3, Cy4 or Cy5 is independently selected from a bond or one of the following substituted or unsubstituted groups: cyclopropyl, cyclobutyl, cyclopentyl, cyclohexyl, azetidinyl, azacyclopentyl, azacyclohexenyl, piperidinyl, morpholinyl, piperazinyl, phenyl, cyclopropyl-fused-cyclopropyl, cyclopropyl-fused-cyclobutyl, cyclopropyl-fused-cyclopentyl, cyclopropyl-fused-cyclohexyl, cyclobutyl-fused-cyclobutyl, cyclobutyl-fused-cyclopentyl, cyclobutyl-fused-cyclohexyl, cyclopentyl-fused-cyclopentyl, cyclopentyl-fused-cyclohexyl, cyclohexyl-fused-cyclohexyl, cyclopropyl-spiro-cyclopropyl, cyclopropyl-spiro-cyclobutyl, cyclopropyl-spiro-cyclopentyl, cyclopropyl-spiro-cyclohexyl, cyclobutyl-spiro-cyclobutyl, cyclobutyl-spiro-cyclopentyl, cyclobutyl-spiro-cyclohexyl, cyclopentyl-spiro-cyclopentyl, cyclopentyl-spiro-cyclohexyl, cyclohexyl-spiro-cyclohexyl, cyclopropyl-fused-azetidinyl, cyclopropyl-fused-azacyclopentyl, cyclopropyl-fused-azacyclohexyl, cyclopropyl-fused-piperidinyl, cyclobutyl-fused-azetidinyl, cyclobutyl-fused-azacyclopentyl, cyclobutyl-fused-azacyclohexyl, cyclobutyl-fused-piperidinyl, cyclopentyl-fused-azetidinyl, cyclopentyl-fused-azacyclopentyl, cyclopentyl-fused-azacyclohexyl, cyclopentyl-fused-piperidinyl, cyclohexyl-fused-azetidinyl, cyclohexyl-fused-azacyclopentyl, cyclohexyl-fused-azacyclohexyl, cyclohexyl-fused-piperidinyl, azetidinyl-fused-azetidinyl, azetidinyl-fused-azacyclopentyl, azetidinyl-fused-azacyclohexyl, azetidinyl-fused-piperidinyl, azacyclopentyl-fused-azetidinyl, azacyclopentyl-fused-azacyclopentyl, azacyclopentyl-fused-azacyclohexyl, azacyclopentyl-fused-piperidinyl, azacyclohexyl-fused-azetidinyl, azacyclohexyl-fused-azacyclopentyl, azacyclohexyl-fused-azacyclohexyl, azacyclohexyl-fused-piperidinyl, cyclobutyl-spiro-azetidinyl, cyclobutyl-spiro-azacyclopentyl, cyclobutyl-spiro-azacyclohexyl, cyclopentyl-spiro-azetidinyl, cyclopentyl-spiro-azacyclopentyl, cyclopentyl-spiro-azacyclohexyl, cyclohexyl-spiro-azetidinyl, cyclohexyl-spiro-azacyclopentyl, cyclohexyl-spiro-azacyclohexyl, azetidinyl-spiro-azetidinyl, azetidinyl-spiro-azacyclopentyl, azetidinyl-spiro-azacyclohexyl, azacyclopentyl-spiro-azetidinyl, azacyclopentyl-spiro-azacyclopentyl, azacyclopentyl-spiro-azacyclohexyl, azacyclohexyl-spiro-azetidinyl, azacyclohexyl-spiro-azacyclopentyl, azacyclohexyl-spiro-azacyclohexyl,

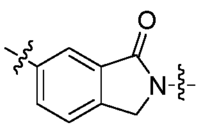, 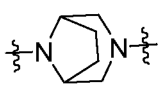, 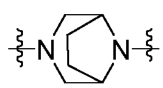,

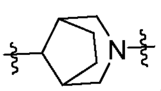, 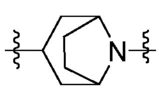, 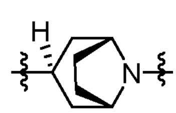, 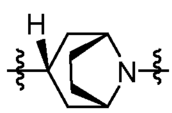, 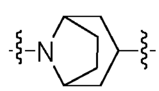, 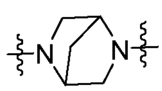,

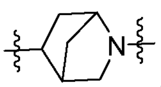, 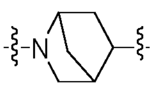, 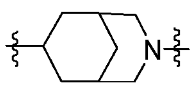, 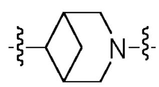, 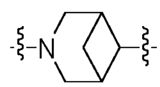, 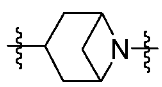,

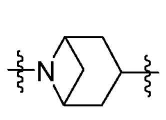, 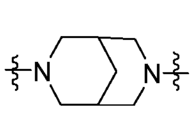, 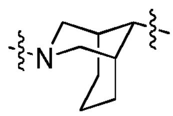, 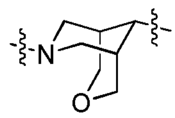, 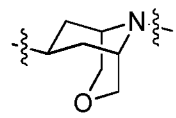,

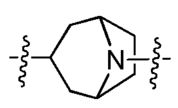, 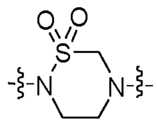 or 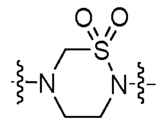,

and when substituted, is optionally substituted with 0 to 4 substituents selected from H, F, Cl, Br, I, OH, $NH_2$, COOH, CN, =O, $C_{1-4}$ alkyl, halogen-substituted $C_{1-4}$ alkyl, hydroxyl-substituted $C_{1-4}$ alkyl or $C_{1-4}$ alkoxy;

$B_1$ is selected from phenyl, naphthyl, thienyl, furyl, pyrrolyl, pyridyl, pyrimidinyl, pyrazinyl, pyridazinyl, quinolinyl, isoquinolinyl, quinazolinyl, 3,4-dihydro-1H-benzopyranyl, or 1,2,3,4-tetrahydroquinolinyl;

alternatively, $B_1$ is selected from benzofuryl, benzothienyl, benzopyrrolyl, benzoxazolyl, benzothiazolyl, benzoimidazolyl, benzopyrazolyl, azacyclopentyl, azacyclohexyl, oxacyclopentyl, oxacyclohexyl, adamantyl, bicyclo[2.2.2]octyl, or 1,2,3,4-tetrahydronaphthyl;

the $B_1$ is optionally substituted with 0 to 4 $R^{b1}$;

$B_2$ is selected from -NHC(=O)-, pyrazolyl, imidazolyl, triazolyl, thiazolyl, oxazolyl, benzopyrrolyl, benzoimidazolyl, pyrazolotetrahydropyrrolyl, or 3-pyridazinonyl, and the pyrazolyl, imidazolyl, thiazolyl, oxazolyl, triazolyl, benzopyrrolyl, benzoimidazolyl, pyrazolotetrahydropyrrolyl, or 3-pyridazinonyl is optionally substituted with 0 to 4 $R^{b2}$,

alternatively, $B_2$ is selected from substituted or unsubstituted benzopyrazolyl, 2-pyridonyl, 1,2,3,4-tetrahydroquinolinyl, 1,2,3,4-tetrahydroisoquinolinyl, azacyclohexyl,

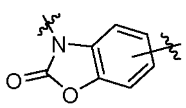, 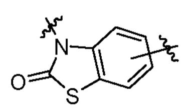, 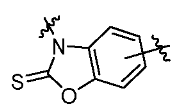, 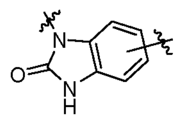,

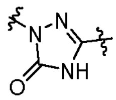 or 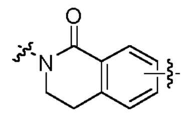,

and when substituted, is optionally substituted with 0 to 4 $R^{b2}$;

$B_3$ is selected from a bond or phenyl, the phenyl is optionally substituted with 0 to 4 $R^{b2}$,

$R^{b1}$ and $R^{b2}$ are each independently selected from H, F, Cl, Br, I, =O, OH, $NH_2$, CN, $NO_2$, $CF_3$, $-C(=O)NH_2$, -C(=O)NH- $CH_3$, $-C(=O)N(CH_3)_2$, methyl, ethyl, propyl, isopropyl, methoxy, ethoxy, cyclopropyl, cyclobutyl, cyclopentyl or cyclohexyl, and the methyl, ethyl, propyl, isopropyl, methoxy, ethoxy, cyclopropyl, cyclobutyl, cyclopentyl or cyclohexyl is optionally substituted with 0 to 4 substituents selected from H, F, Cl, Br, I or OH;

$R^{b3}$ and $R^{b4}$ are each independently selected from H, methyl, ethyl, isopropyl, or methoxymethyl;

alternatively, $R^{b3}$ and $R^{b4}$ together with the carbon atom to which they are attached form cyclopropyl, cyclobutyl, cyclopentyl, cyclohexyl, azetidinyl, azacyclopentyl, azacyclohexyl, oxetanyl, oxacyclopentyl, or oxacyclohexyl, and the cyclopropyl, cyclobutyl, cyclopentyl, cyclohexyl, azetidinyl, azacyclopentyl, azacyclohexyl, oxetanyl, oxacyclopentyl, or oxacyclohexyl is optionally substituted with 0 to 4 substituents selected from H, F, Cl, Br, I, OH, $NH_2$, $C_{1-4}$ alkyl or $C_{1-4}$ alkoxy;

K is selected from

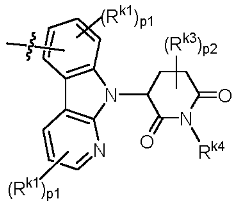, 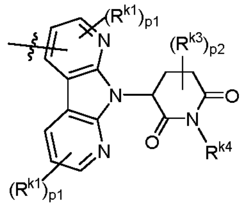, 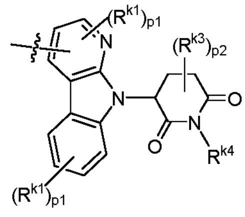,

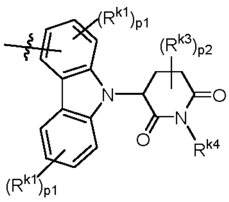 , 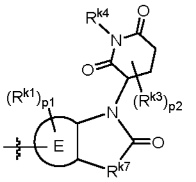 , 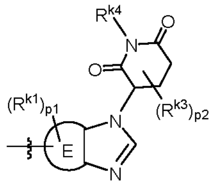 , 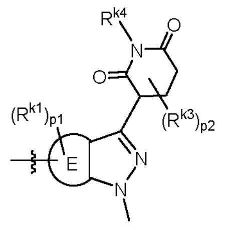 ,

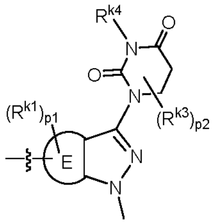 , 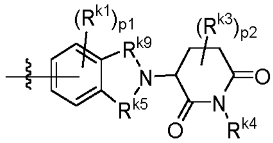 , 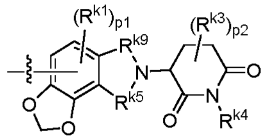 , 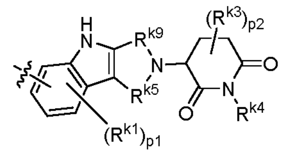

, 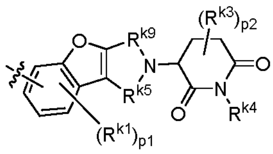 , 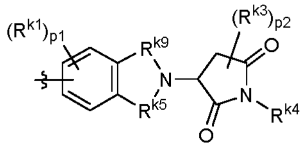 , 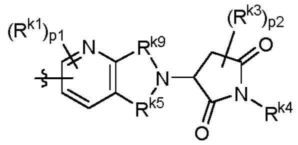 ,

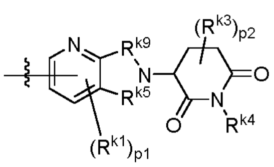 , 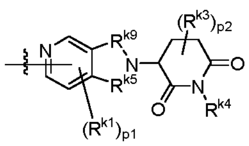 , 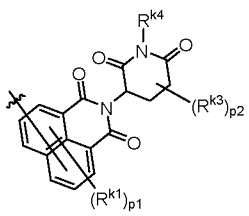 , 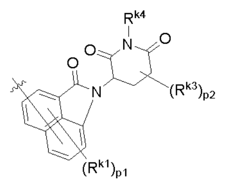 ,

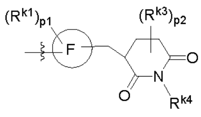 , 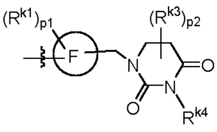 , 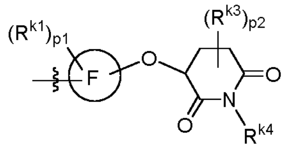 , 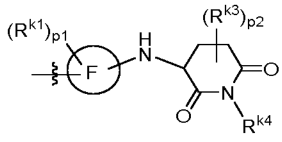 ,

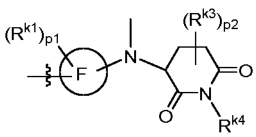 , 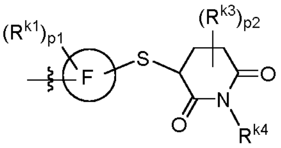 , 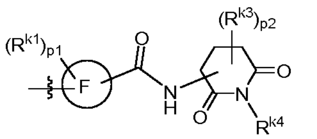 ,

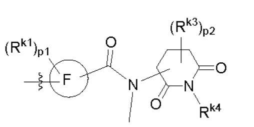 , 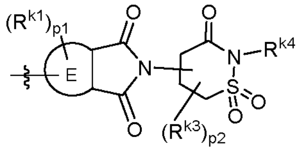 , 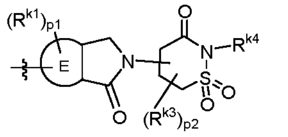 ,

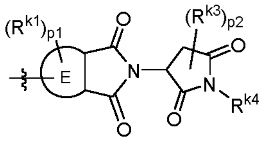, 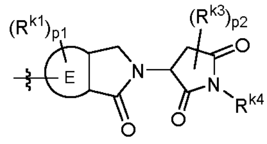, 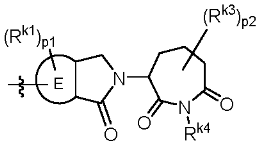, 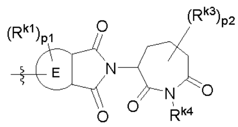

, 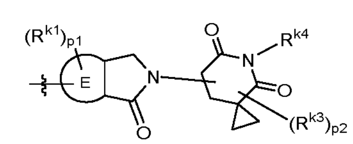, 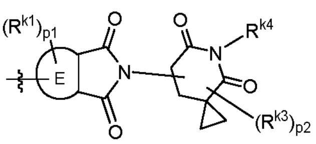, 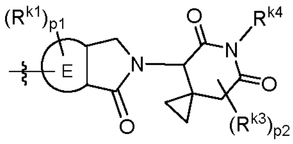,

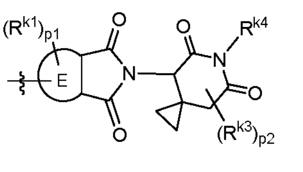, 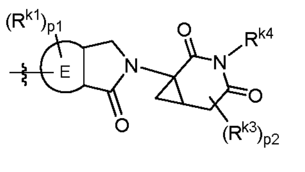, 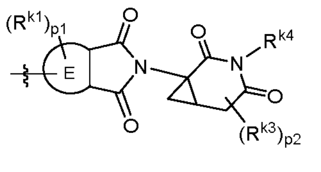,

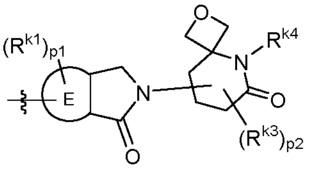, 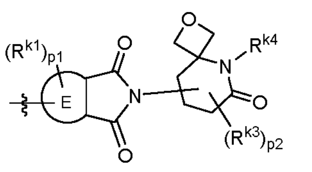, 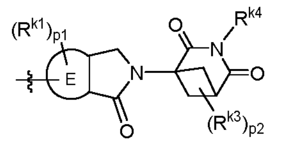,

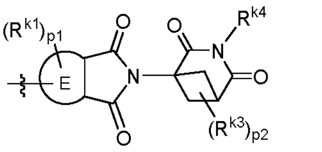, 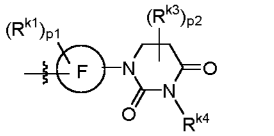, 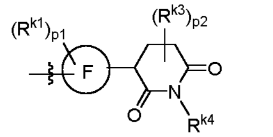,

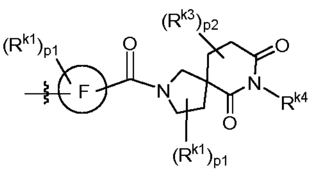, 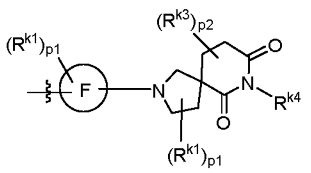, 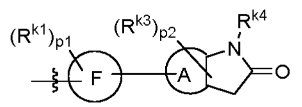,

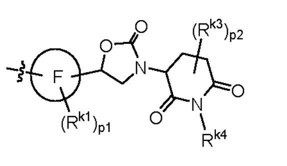, 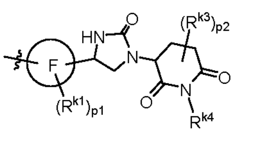, 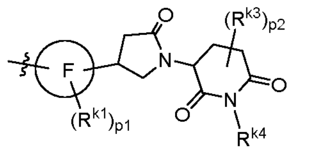,

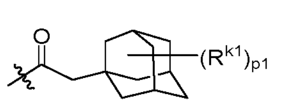, 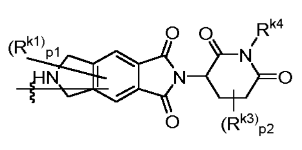, 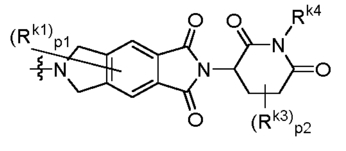,

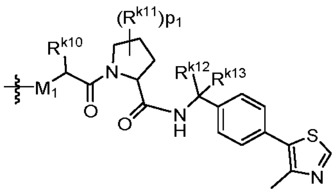 , 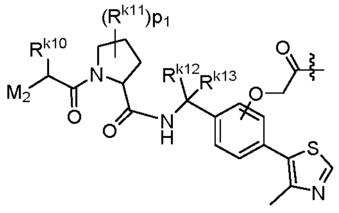 , 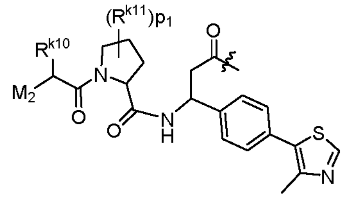 ,

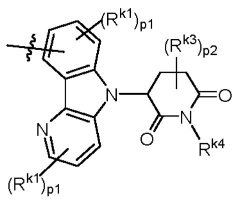 or 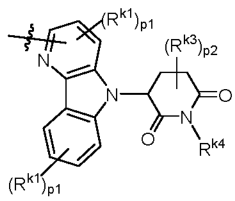 ;

$M_1$ is selected from a bond, -C(=O)NH-, $-CH_2-C(=O)NH-$, $-C(=O)CH_2NH-$, pyrrolyl, pyrazolyl, imidazolyl, triazolyl, oxazolyl, isoxazolyl, furyl, thienyl, or thiazolyl;

$M_2$ is selected from $-NHC(=O)-CH_3$, -NHC(=O)-cyclopropyl, -NHC(=O)-cyclobutyl, azetidinyl, azacyclopentyl, benzoazacyclopentyl, or benzoazacyclohexyl, and the cyclopropyl, cyclobutyl, azetidinyl, azacyclopentyl, benzoazacyclopentyl or benzoazacyclohexyl is optionally substituted with 0 to 4 substituents selected from H, F, Cl, Br, I, =O, OH, $NH_2$, $C_{1-4}$ alkyl or $C_{1-4}$ alkoxy;

$R^{k10}$ is selected from methyl, ethyl, isopropyl, propyl, or tert-butyl, and the methyl, ethyl, isopropyl, propyl, or tert-butyl is optionally substituted with 0 to 4 substituents selected from H, F, Cl, Br, I, =O, OH, $C_{1-4}$ alkyl or $C_{3-6}$ cycloalkyl;

each $R^{k11}$ is independently selected from H, F, Cl, Br, I, =O, OH, SH, methyl, ethyl, isopropyl, propyl, methoxy, ethoxy, propoxy, isopropoxy, methylthio, ethylthio, propylthio or $-O-C(=O)-CH_3$, and the methyl, ethyl, isopropyl, propyl, methoxy, ethoxy, propoxy, isopropoxy, methylthio, ethylthio, or propylthio is optionally substituted with 0 to 4 substituents selected from H, F, Cl, Br, I, OH, $C_{1-4}$ alkyl or $C_{1-4}$ alkoxy;

$R^{k12}$ and $R^{k13}$ are each independently selected from H, methyl, ethyl, isopropyl, propyl, cyclopropyl or cyclobutyl, and the methyl, ethyl, isopropyl, propyl, cyclopropyl or cyclobutyl is optionally substituted with 0 to 4 substituents selected from H, F, Cl, Br, I, =O, OH, $NH_2$, $C_{1-4}$ alkyl or $C_{1-4}$ alkoxy;

each E is independently selected from phenyl, pyridyl, pyridazinyl, pyrazinyl, pyrimidinyl, pyrrolyl, pyrazolyl, imidazolyl, thiazolyl, furyl, thienyl or oxazolyl;

each A is independently selected from phenyl, pyridyl, pyridazinyl, pyrazinyl, pyrimidinyl, pyrrolyl, pyrazolyl, imidazolyl, thiazolyl, furyl, thienyl or oxazolyl;

each F is independently selected from cyclopropyl, cyclobutyl, cyclopentyl, cyclohexyl, bicyclo[1.1.1]pentyl, 6,7-dihydro-5H-cyclopen[c]pyridyl, 2,3-dihydro-1H-indenyl, phenyl, naphthyl, anthracyl, phenanthrenyl, azetidinyl, azacyclopentyl, piperidinyl, morpholinyl, pyridyl, pyrimidinyl, pyridazinyl, pyrazinyl, triazinyl, pyrrolyl, pyrazolyl, imidazolyl, triazolyl, oxazolyl, furyl, thienyl, thiazolyl, 2-pyridonyl, benzoimidazolyl, benzopyrazolyl, benzothiazolyl, benzothienyl, benzofuryl, benzopyrrolyl, benzopyridyl, benzopyrazinyl, benzopyrimidinyl, benzopyridazinyl, benzotriazinyl, pyrrolopyrrolyl, pyrrolopyridyl, pyrrolopyrimidinyl, pyrrolopyridazinyl, pyrrolopyrazinyl, imidazopyrimidinyl, imidazopyridyl, imidazopyrazinyl, imidazopyridazinyl, pyrazolopyridyl, pyrazolopyrimidinyl, pyrazolopyridazinyl, pyrazolopyrazinyl, pyrimidopyridyl, pyrimidopyrazinyl, pyrimidopyridazinyl, pyrimidopyrimidinyl, pyridopyridyl, pyridopyrazinyl, pyridopyridazinyl, pyridazinopyridazinyl, pyridazinopyrazinyl or pyrazinopyrazinyl;

each $R^{k7}$ is independently selected from $CH_2$, O, $N(CH_3)$ or NH;

each p1 or p2 is independently selected from 0, 1 or 2.

**6.** The compound or the stereoisomer, deuterated compound, solvate, prodrug, metabolite, pharmaceutically acceptable salt or co-crystal thereof according to claim 5, **characterized in that**,

each Cy1, Cy2, Cy3, Cy4 or Cy5 is independently selected from a bond or one of the following substituted or unsubstituted groups:

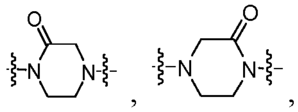

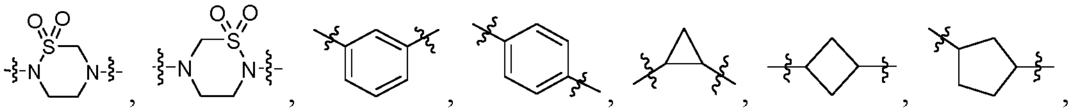

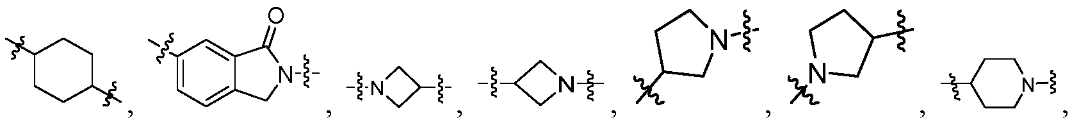

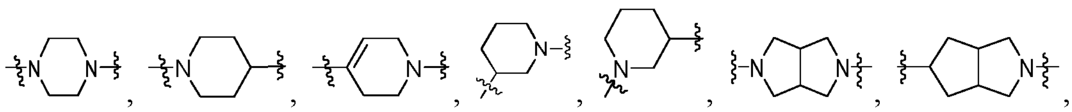

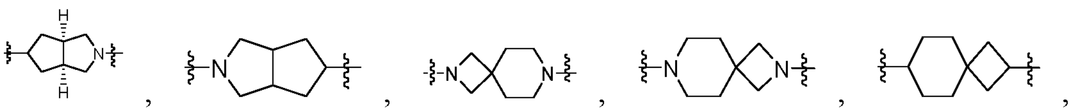

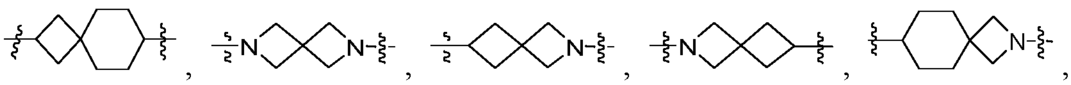

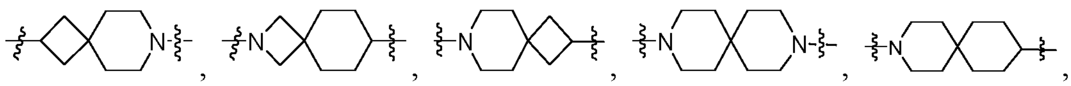

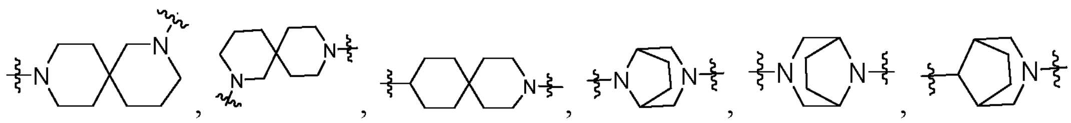

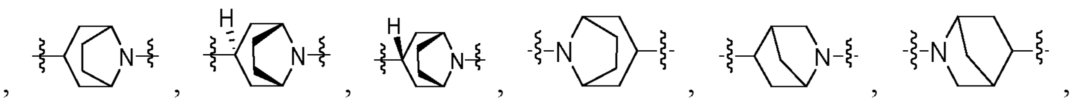

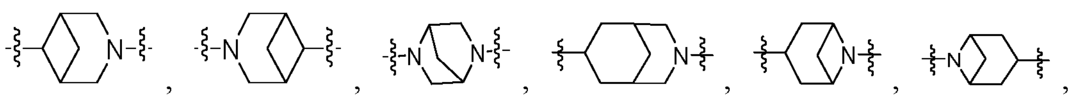

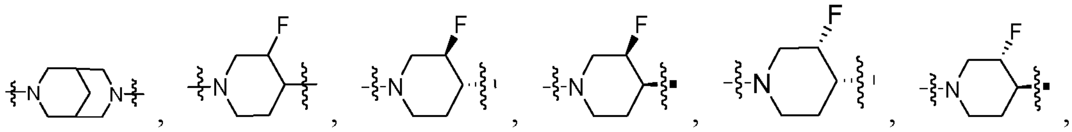

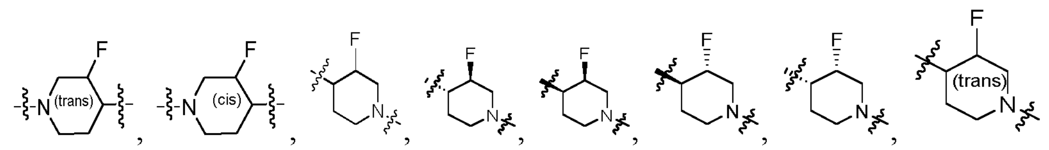

or

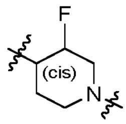

,

and when substituted, is optionally substituted with 0 to 4 substituents selected from H, F, $CF_3$, OH, methyl, methoxy, =O, hydroxymethyl, COOH, CN or $NH_2$;
B is selected from one of the structures shown in Table C-1, Table C-2, Table C-3, Table C-4, Table C-5 and Table C-6;
and K is selected from one of the structural fragments in Table K-1.

**7.** The compound or the stereoisomer, deuterated compound, solvate, prodrug, metabolite, pharmaceutically acceptable salt or co-crystal thereof according to claim 1, **characterized in that**,

$B_1$ is selected from $C_{6-10}$ aryl, 5- to 10-membered heteroaryl, $C_{6-10}$ cycloalkyl, $C_{6-10}$ carbocyclyl, or 5- to 10-membered heterocyclyl, the aryl, heteroaryl, cycloalkyl, carbocyclyl or heterocyclyl is optionally substituted with 1 or 2 $R^{bb}$, the aryl, heteroaryl, cycloalkyl, carbocyclyl or heterocyclyl is optionally substituted with 0 to 3 $R^{b1}$, and the heteroaryl or heterocyclyl contains 1 to 4 heteroatoms selected from O, S or N;
$R^{bb}$ is selected from -T-P(=O)$_2$($C_{1-4}$ alkyl)$_2$, -T-S(=O)$_2$$C_{1-4}$ alkyl, -T-P(=O)$_2$($C_{3-6}$ cycloalkyl)$_2$, -T-S(=O)$_2$$C_{3-6}$ cycloalkyl, -T-C(=O)$NH_2$, -T-C(=O)NH-$C_{1-4}$ alkyl, -T-C(=O)N($C_{1-4}$ alkyl)$_2$, -T-$C_{1-4}$ alkyl, -T-$C_{2-6}$ alkenyl, -T-$C_{2-6}$ alkynyl, - T- $C_{3-12}$ carbocyclyl, or -T-3- to 12-membered heterocyclyl, the alkyl, alkenyl, alkynyl, carbocyclyl or heterocyclyl is optionally substituted with 0 to 4 substituents selected from H, F, Cl, Br, I, OH, $NH_2$, =O, halogen-substituted $C_{1-4}$ alkyl, cyano-substituted $C_{1-4}$ alkyl, hydroxyl-substituted $C_{1-4}$ alkyl, $C_{1-4}$ alkyl, $C_{1-4}$ alkoxy, $C_{3-6}$ cycloalkyl or 3- to 7-membered heterocycle, and the heterocycle contains 1 to 4 heteroatoms selected from O, S or N;
T is selected from a bond, -C(=O)-, -P(=O)$_2$-, -S(=O)$_2$-, -C(=O)NH-, - P(=O)$_2$NH-, -S(=O)$_2$NH-, -C(=O)N($C_{1-4}$ alkyl)-, -P(=O)$_2$N($C_{1-4}$ alkyl)-, - S(=O)$_2$N($C_{1-4}$ alkyl)-, -NHC(=O)-, -NHP(=O)$_2$-, -NHS(=O)$_2$-, -N($C_{1-4}$ alkyl)C(=O)-, -N($C_{1-4}$ alkyl)P(=O)$_2$-, -N($C_{1-4}$ alkyl)S(=O)$_2$-, O, S, NH, N($C_{1-4}$ alkyl), N($C_{3-6}$ cycloalkyl), N(3- to 10-membered heterocycle), $C_{1-4}$ alkylene, -O-$C_{1-4}$ alkylene-, -$C_{1-4}$ alkylene-O-, -S-$C_{1-4}$ alkylene-, -NH-$C_{1-4}$ alkylene-, -N($C_{1-4}$ alkyl)-$C_{1-4}$ alkylene-, or -O-$C_{1-4}$ alkylene-O-, the alkyl or alkylene is optionally substituted with 0 to 4 substituents selected from H, F, Cl, Br, I, OH, =O, $C_{1-4}$ alkyl, $C_{1-4}$ alkoxy, $C_{3-6}$ cycloalkyl or 3- to 7-membered heterocycle, and the heterocycle contains 1 to 4 heteroatoms selected from O, S or N;
alternatively, 2 $R^{bb}$ together with the atom to which they are attached form non-aromatic $C_{4-12}$ carbocycle or 4- to 12-membered heterocycle, the carbocycle or heterocycle is optionally substituted with 0 to 4 substituents selected from H, F, Cl, Br, I, OH, $NH_2$, =O, halogen-substituted $C_{1-4}$ alkyl, cyano-substituted $C_{1-4}$ alkyl, hydroxyl-substituted $C_{1-4}$ alkyl, $C_{1-4}$ alkyl, $C_{1-4}$ alkoxy, $C_{3-6}$ cycloalkyl or 3- to 7-membered heterocycle, and the heterocycle contains 1 to 4 heteroatoms selected from O, S or N.

**8.** The compound or the stereoisomer, deuterated compound, solvate, prodrug, metabolite, pharmaceutically acceptable salt or co-crystal thereof according to claim 7, **characterized in that**,

the definition of L is the same as that described in any one of claims 3, 4, 5 or 6;
and the definition of K is the same as that described in any one of claims 3, 4, 5 or 6.

**9.** The compound or the stereoisomer, deuterated compound, solvate, prodrug, metabolite, pharmaceutically acceptable salt or co-crystal thereof according to claim 8, **characterized in that**,

$B_1$ is selected from phenyl, naphthyl, thienyl, furyl, pyrrolyl, pyridyl, pyrimidinyl, pyrazinyl, pyridazinyl, quinolinyl, isoquinolinyl, quinazolinyl, benzofuryl, benzothienyl, benzopyrrolyl, benzoxazolyl, benzothiazolyl, benzoimidazolyl, benzopyrazolyl, azacyclopentyl, azacyclohexyl, oxacyclopentyl, oxacyclohexyl, adamantyl, bicyclo[2.2.2]octyl,

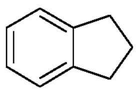, or 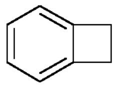,

the $B_1$ is optionally substituted with 1 or 2 $R^{bb}$, and the $B_1$ is optionally substituted with 0 to 3 $R^{b1}$.

$B_2$ is selected from -NHC(=O)- or one of the following substituted or unsubstituted groups: pyrazolyl, imidazolyl, triazolyl, thiazolyl, oxazolyl, benzopyrrolyl, benzoimidazolyl, pyrazolotetrahydropyrrolyl, 3-pyridazinonyl, benzopyrazolyl, 2-pyridonyl, 1,2,3,4-tetrahydroquinolinyl, 1,2,3,4-tetrahydroisoquinolinyl, azacyclohexyl,

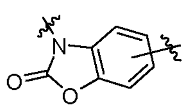, 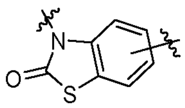,

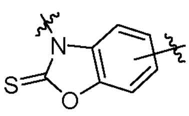, 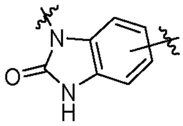, 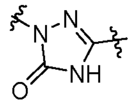, 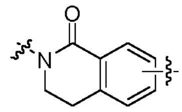, 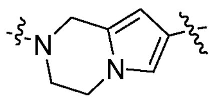,

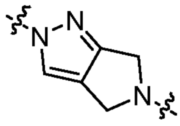, 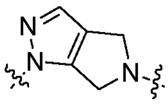, or 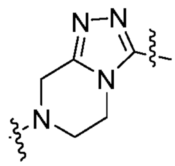,

and when substituted, is optionally substituted with 0 to 4 $R^{b2}$,

$B_3$ is selected from a bond or phenyl, the phenyl is optionally substituted with 0 to 4 $R^{b2}$,

$R^{bb}$ is selected from $-P(=O)_2(CH_3)_2$, $-S(=O)_2CH_3$, $-T-C(=O)NH_2$, $-T-C(=O)NHCH_3$, $-T-C(=O)N(CH_3)_2$, $-T-C(=O)NHCH_2CH_3$, $-T-C(=O)N(CH_2CH_3)_2$, - T-$C_{3-6}$ monocyclic cycloalkyl, -T-phenyl, $-OCH_2CH_2OCH_3$, $-OCH_2CH_2OCH_2CH_3$, -T-5- to 6-membered heteroaryl, -T-4- to 6-membered monocyclic heterocycloalkyl, -T-6- to 10-membered spirocyclic heterocycloalkyl, -T-6- to 10-membered fused cyclic heterocycloalkyl, -T-benzopyrazolyl, -T-benzoimidazolyl, - T-benzopyrrolyl, -T-indolinyl, -T-vinyl, -T-propan-1,2-dien-1-yl, -T-ethynyl, -T-propynyl, or -T-propargyl, the $CH_2$, $CH_3$, cycloalkyl, phenyl, heterocycloalkyl, heteroaryl, benzopyrazolyl, benzoimidazolyl, benzopyrrolyl, indolinyl, vinyl, prop-1,2-dien-1-yl, ethynyl, propynyl, or propargyl is optionally substituted with 0 to 4 substituents selected from H, F, Cl, Br, I, OH, $NH_2$, =O, halogen-substituted $C_{1-4}$ alkyl, cyano-substituted $C_{1-4}$ alkyl, hydroxyl-substituted $C_{1-4}$ alkyl, $C_{1-4}$ alkyl, $C_{1-4}$ alkoxy, $C_{3-6}$ cycloalkyl or 3- to 7-membered heterocycloalkyl, and the heterocycloalkyl contains 1 to 4 heteroatoms selected from O, S or N;

alternatively, $R^{bb}$ is selected from -T-methyl or -T-ethyl, the methyl or ethyl is substituted with 1 to 4 substituents selected from F, Cl, Br, I, OH, $NH_2$, =O, halogen-substituted $C_{1-4}$ alkyl, cyano-substituted $C_{1-4}$ alkyl, hydroxyl-substituted $C_{1-4}$ alkyl, $C_{1-4}$ alkyl, $C_{1-4}$ alkoxy, $C_{3-6}$ cycloalkyl or 3- to 7-membered heterocycloalkyl, and the heterocycloalkyl contains 1 to 4 heteroatoms selected from O, S or N;

T is selected from a bond, -C(=O)-, O, S, NH, $N(CH_3)$, $-CH_2-$, $-CH_2CH_2-$, - $OCH_2O-$, $-OCH_2-$, $-CH_2O-$, $-OCH_2CH_2O-$, $-OCH_2CH_2-$, or $-CH_2CH_2O-$, and the $CH_2$ or $CH_3$ is optionally substituted with 0 to 4 substituents selected from H, F, Cl, Br, I, OH, =O, $C_{1-4}$ alkyl, $C_{1-4}$ alkoxy, $C_{3-6}$ cycloalkyl or 3- to 7-membered heterocycle;

alternatively, 2 $R^{bb}$ together with the atom to which they are attached form non-aromatic $C_{4-12}$ carbocycle or 4- to 12-membered heterocycle, the carbocycle or heterocycle is optionally substituted with 0 to 4 substituents selected from H, F, Cl, Br, I, OH, $NH_2$, =O, halogen-substituted $C_{1-4}$ alkyl, cyano-substituted $C_{1-4}$ alkyl, hydroxyl-substituted $C_{1-4}$ alkyl, $C_{1-4}$ alkyl, $C_{1-4}$ alkoxy, $C_{3-6}$ cycloalkyl or 3- to 7-membered heterocycle, and the heterocycle contains 1 to 4 heteroatoms selected from O, S or N;

$R^{b1}$ and $R^{b2}$ are each independently selected from H, F, Cl, Br, I, =O, OH, $NH_2$, CN, $NO_2$, $CF_3$, $-C(=O)NH_2$, -C(=O)NH- $CH_3$, $-C(=O)N(CH_3)_2$, methyl, ethyl, propyl, isopropyl, methoxy, ethoxy, cyclopropyl, cyclobutyl, cyclopentyl or cyclohexyl, and the methyl, ethyl, propyl, isopropyl, methoxy, ethoxy, cyclopropyl, cyclobutyl, cyclopentyl or cyclohexyl is optionally substituted with 0 to 4 substituents selected from H, F, Cl, Br, I or OH;

$R^{b3}$ and $R^{b4}$ are each independently selected from H, methyl, ethyl, isopropyl, or methoxymethyl;

alternatively, $R^{b3}$ and $R^{b4}$ together with the carbon atom to which they are attached form cyclopropyl, cyclobutyl,

cyclopentyl, cyclohexyl, azetidinyl, azacyclopentyl, azacyclohexyl, oxetanyl, oxacyclopentyl, or oxacyclohexyl, and the cyclopropyl, cyclobutyl, cyclopentyl, cyclohexyl, azetidinyl, azacyclopentyl, azacyclohexyl, oxetanyl, oxacyclopentyl, or oxacyclohexyl is optionally substituted with 0 to 4 substituents selected from H, F, Cl, Br, I, OH, $NH_2$, $C_{1-4}$ alkyl or $C_{1-4}$ alkoxy.

**10.** The compound or the stereoisomer, deuterated compound, solvate, prodrug, metabolite, pharmaceutically acceptable salt or co-crystal thereof according to claim 9, **characterized in that**,

$R^{bb}$ is selected from $-P(=O)_2(CH_3)_2$, $-S(=O)_2CH_3$, $-OCH_2CH_2OCH_3$, $-T-C(=O)NH_2$, $-T-C(=O)NHCH_3$, $-T-C(=O)N(CH_3)$, -T-cyclopropyl, -T-cyclobutyl, - T-cyclopentyl, -T-cyclohexyl, -T-azetidinyl, -T-azacyclopentyl, -T-azacyclohexyl, -T-oxetanyl, -T-oxacyclopentyl, -T-oxacyclohexyl, -T-phenyl, -T-pyridyl, -T-pyrrolyl, -T-thienyl, -T-furyl, -T-pyrazolyl, -T-pyrrolyl, -T-imidazolyl, -T-thiazolyl, -T-oxazolyl, -T-triazolyl, -T-pyridazinyl, -T-pyridazinone, -T-morpholinyl, -T-cyclopropyl-spiro-azacyclohexyl, -T-cyclopentyl-fused-azacyclopentyl, -T-benzopyrazolyl, -T-benzoimidazolyl, -T-benzopyrrolyl, -T-dihydroindolyl, -T-vinyl, -T-prop-1,2-dien-1-yl, -T-ethynyl, -T-propynyl, or -T-propargyl, and the $CH_2$, $CH_3$, cyclopropyl, cyclobutyl, cyclopentyl, cyclohexyl, azetidinyl, azacyclopentyl, azacyclohexyl, oxetanyl, oxacyclopentyl, oxacyclohexyl, phenyl, pyridyl, pyrrolyl, thienyl, furyl, pyrazolyl, pyrrolyl, imidazolyl, thiazolyl, oxazolyl, triazolyl, pyridazinyl, pyridazinone, morpholinyl, cyclopropyl-spiro-azacyclohexyl, cyclopentyl-fused-azacyclopentyl, benzopyrazolyl, benzoimidazolyl, benzopyrrolyl, dihydroindolyl, vinyl, prop-1,2-dien-1-yl, ethynyl, propynyl, or propargyl is optionally substituted with 0 to 4 substituents selected from H, F, Cl, Br, I, OH, =O, $CF_3$, methyl, ethyl, methoxy, ethoxy, cyclopropyl, azetidinyl, azacyclopentyl, piperidinyl or piperazine;

alternatively, $R^{bb}$ is selected from -T-methyl or -T-ethyl, the methyl or ethyl is substituted with 1 to 4 substituents selected from F, Cl, Br, I, OH, =O, $CF_3$, methyl, ethyl, methoxy, ethoxy, or cyclopropyl;

T is selected from a bond, -C(=O)-, O, S, NH, $N(CH_3)$, $-CH_2-$, $-CH_2CH_2-$, $- OCH_2O-$, $-OCH_2-$, $-CH_2O-$, $-OCH_2CH_2O-$, $-OCH_2CH_2-$, or $-CH_2CH_2O-$, and the $CH_2$ or $CH_3$ is optionally substituted with 0 to 4 substituents selected from H, F, Cl, Br, I, OH, =O, methyl, ethyl, methoxy, ethoxy, or cyclopropyl;

alternatively, 2 $R^{bb}$ together with the atom to which they are attached form a ring as shown below:

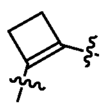 , 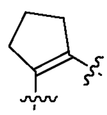 , 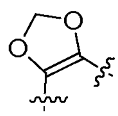 , 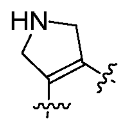 , 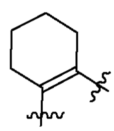 , 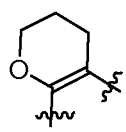 ,

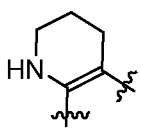 , 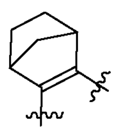 , 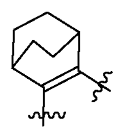 , or 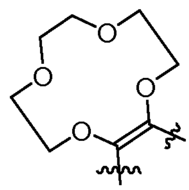 ,

and the ring is optionally substituted with 1 to 4 substituents selected from F, Cl, Br, I, OH, $NH_2$, methyl, ethyl, or cyclopropyl.

**11.** The compound or the stereoisomer, deuterated compound, solvate, prodrug, metabolite, pharmaceutically acceptable salt or co-crystal thereof according to claim 10, **characterized in that**,

B is selected from one of the structures described in claim 6 or one of the structures shown in Table C-7; and the definition of K is the same as that described in claim 6.

**12.** The compound or the stereoisomer, deuterated compound, solvate, prodrug, metabolite, pharmaceutically acceptable salt or co-crystal thereof according to claim 6 or 11, **characterized in that**,

L is selected from a bond, -Ak1-, -Cy1-, -Cy1-Ak1-, -Cy1-Ak1-Ak2-, -Cyl-Ak1-Ak2-Ak3-, -Cy1-Ak1-Ak2-Ak3-Ak4-, -Cy1-Cy2-, -Cy1-Ak1-Cy2-, -Cyl-Cy2-Ak2-, -Cy1-Ak1-Cy2-Ak2-, -Cy1-Ak1-Cy2-Ak2-Ak3-, -Cy1-Ak1-Cy2-Ak2-Ak3-Ak4-, -Cy1-Cy2-Ak2-Ak3-, -Cy1-Cy2-Ak2-Ak3-Ak4-, -Cy1-Ak1-Cy2-Ak2-Ak3-Ak4-, -Cy1-Ak1-Ak2-Cy3-, -Cy1-Ak1-Ak2-Cy3-Ak3-, -Cy1-Cy2-Cy3-, - Cy1-Ak1-Cy2-Cy3-, -Cy1-Cy2-Ak2-Cy3-, -Cy1-Cy2-Cy3-Ak3-,

-Cy1-Ak1-Cy2-Cy3-Ak3-, -Cy1-Cy2-Ak2-Cy3-Ak3-, -Cy1-Ak1-Cy2-Ak2-Cy3-, -Cy1-Ak1-Cy2-Ak2-Cy3-Ak3-, -Cy1-Cy2-Cy3-Ak3-Ak4-, -Cy1-Cy2-Cy3-Ak3-Cy4-, -Cy1-Cy2-Cy3-Cy4-, -Cy1-Ak1-Cy2-Cy3-Cy4-, -Cy1-Cy2-Ak2-Cy3-Cy4-, -Cy1-Cy2-Cy3-Ak3-Cy4-, -Cy1-Cy2-Cy3-Cy4-Ak4-, -Cy1-Ak1-Cy2-Ak2-Cy3-Ak3-Cy4-, -Cyl-Ak1-Cy2-Ak2-Cy3-Cy4-, -Ak1-Cy2-, -Ak1-Cy2-Cy3-, -Ak1-Ak2-Cy3-, -Ak1-Ak2-Cy3-Cy4-, -Ak1-Cy2-Ak2-Cy3-, -Ak1-Cy2-Cy3-Ak3-Cy4-, -Ak1-Cy2-Cy3-Cy4-Ak4-Cy5-, -Ak1-Cy2-Ak2-, -Ak1-Ak2-Ak3-Ak4-, -Ak1-Ak2-Ak3-, -Ak1-Ak2-, -Ak1-Ak2-Ak3-Ak4-Ak5-, -Cy1-Cy2-Cy3-Ak3-Ak4-Ak5-, -Cy1-Cy2-Ak2-Cy3-Ak3-Ak4-Ak5-, -Cy1-Ak1-Cy2-Ak2-Ak3-Ak4-Ak5-, -Cy1-Cy2-Cy3-Cy4-Ak4-Ak5-, -Cy1-Ak1-Ak2-Ak3-Ak4-Ak5-, -Ak1-Cy2-Ak2-Ak3-Ak4-Ak5-, -Ak1-Cy2-Ak2-Ak3-Ak4-, or -Ak1-Cy2-Ak2-Ak3-;

Ak1, Ak2, Ak3, Ak4, and Ak5 are each independently selected from -O-, - $OCH_2$-, -$CH_2O$-, -$OCH_2CH_2$-, -$CH_2CH_2O$-, -CH=CH-, -CH=C(CN)-, -CH=C(F)-, - C(CN)=CH-, -C(F)=CH-, -C=C-, -$C(CH_3)_2$-, -$CH_2$-, -$CH_2CH_2$-, -$CH_2CH_2CH_2$-, - $N(CH_3)$-, -NH-, -$CH_2N(CH_3)$-, -$CH_2NH$-, -$NHCH_2$-, -$CH_2CH_2N(CH_3)$-, - $CH_2CH_2NH$-, -$NHCH_2CH_2$-, -C(=O)-, -C(=O)$CH_2$NH-, -$CH_2$C(=O)NH-, - C(=O)NH- or -NHC(=O)-.

**13.** The compound or the stereoisomer, deuterated compound, solvate, prodrug, metabolite, pharmaceutically acceptable salt or co-crystal thereof according to claim 12, **characterized in that**,
L is selected from a bond or a group shown in Table B-1 or Table B-2, where the left side of the group is connected to B.

**14.** The compound or the stereoisomer, deuterated compound, solvate, prodrug, metabolite, pharmaceutically acceptable salt or co-crystal thereof according to claim 5 or 9, **characterized in that**,

L is selected from a bond, -Ak1-Cy2-, -Ak1-Cy2-Cy3-, -Cy1-Ak1-, -Ak1-Cy2-Ak2-, -Cy1-Cy2-Cy3-, -Cy2-Cy3-, -Cy1-Ak1-Cy2-, -Cy1-Ak1-Cy2-Cy3-, - Cy1-Cy2-Ak2-Cy3-, -Ak1-, -Cy1-, -Ak1-Cy2-Ak2-Ak3-, -Ak1-Cy2-Ak2-Cy3-, - Ak1-Cy2-Ak2-Ak3-Ak4-, or -NHCO-$(CH_2)_{s1}$;
s1 is selected from 0, 1, 2, 3, 4, 5, 6 or 7;
Ak1, Ak2, Ak3, and Ak4 are each independently selected from -C(=O)-, -O-, NH, -CH=CH-, -CH=C(CN)-, -CH=C(F)-, -C(CN)=CH-, -C(F)=CH-, -C=C-, - $C(CH_3)_2$-, -$CH_2$-, -$CH_2CH_2$-, -$CH_2CH_2CH_2$-, or -NHCO-;
each Cy1, Cy2, or Cy3 is independently selected from one of the following substituted or unsubstituted groups:

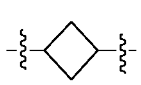 , 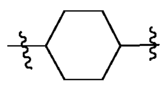 , 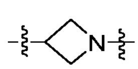 ,

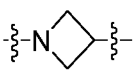 , 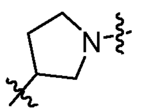 , 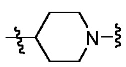 , 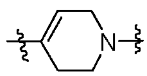 , 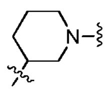 , 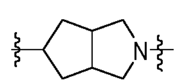 ,

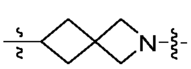 , 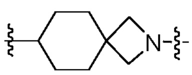 , 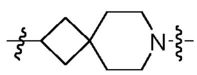 , 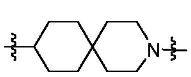 , 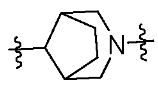 ,

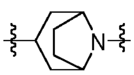 , 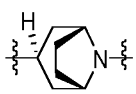 , 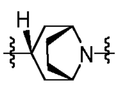 , 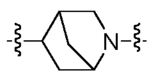 , 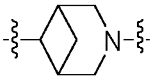 , 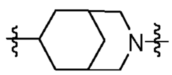 ,

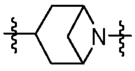 , 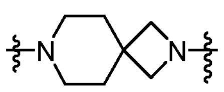 , 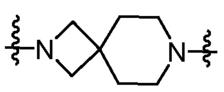 , 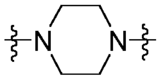 , 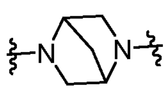 ,

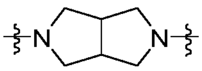 , 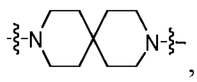 , 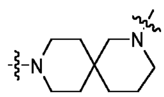 , 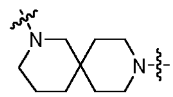 , 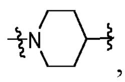 , or 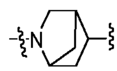 ,

and when substituted, is optionally substituted with 0 to 4 substituents selected from H, F, $CF_3$, OH, methyl, methoxy, =O, hydroxymethyl, COOH, CN or $NH_2$;
preferably, L is selected from a bond or **Table B-2**, where the left side is connected to B.

**15.** The compound or the stereoisomer, deuterated compound, solvate, prodrug, metabolite, pharmaceutically acceptable salt or co-crystal thereof according to claim 5, **characterized in that** the compound of general formula (I) is selected from a compound as represented by general formula (Ia),

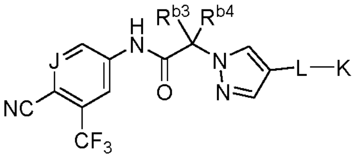

(Ia)

J is selected from N or CH, preferably J is N;
L is selected from -Ak1-Cy2-, -Ak1-Cy2-Cy3-, -Ak1-Cy2-Ak2-, -Cy2-Cy3-, - Ak1- or a bond;
$R^{b3}$ and $R^{b4}$ are each independently selected from methyl or ethyl;
alternatively, $R^{b3}$ and $R^{b4}$ together with the carbon atom to which they are attached form cyclopropyl, cyclobutyl, cyclopentyl, cyclohexyl, azetidinyl, azacyclopentyl, azacyclohexyl, oxetanyl, oxacyclopentyl, or oxacyclohexyl, and the cyclopropyl, cyclobutyl, cyclopentyl, cyclohexyl, azetidinyl, azacyclopentyl, azacyclohexyl, oxetanyl, oxacyclopentyl, or oxacyclohexyl is optionally substituted with 0 to 4 substituents selected from H, F, Cl, Br, I, OH, $NH_2$, $C_{1-4}$ alkyl or $C_{1-4}$ alkoxy;
Ak1 and Ak2 are each independently selected from -CH=CH-, -CH=C(CN)-, -CH=C(F)-, -C(CN)=CH-, -C(F)=CH-, -C=C-, $-C(CH_3)_2-$, $-CH_2-$, $-CH_2CH_2-$, or - $CH_2CH_2CH_2-$;
each Cy1, Cy2, or Cy3 is independently selected from one of the following substituted or unsubstituted groups:

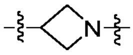, 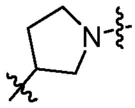, 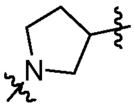, 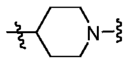,

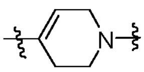, 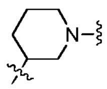, 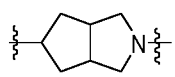, 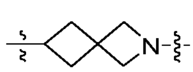, 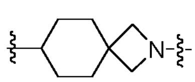,

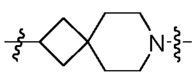, 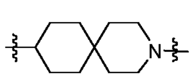, 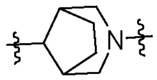, 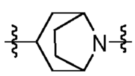, 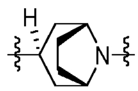, 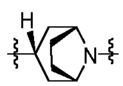,

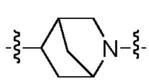, 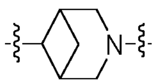, 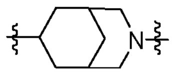, or 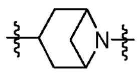,

and when substituted, is optionally substituted with 0 to 4 substituents selected from H, F, $CF_3$, OH, methyl, methoxy, =O, hydroxymethyl, COOH, CN or $NH_2$.

**16.** The compound or the stereoisomer, deuterated compound, solvate, prodrug, metabolite, pharmaceutically acceptable salt or co-crystal thereof according to any one of claims 12-15, **characterized in that**,
K is selected from one of the structural fragments in Table K-2.

**17.** The compound or the stereoisomer, deuterated compound, solvate, prodrug, metabolite, pharmaceutically acceptable salt or co-crystal thereof according to claim 1, **characterized in that** the compound of general formula (I) is selected from a compound as represented by general formula (Ib),

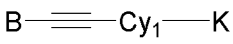

(Ib)

and the definition of B, K or Cy1 is the same as that described in any one of claims 1 to 16.

18. The compound or the stereoisomer, deuterated compound, solvate, prodrug, metabolite, pharmaceutically acceptable salt or co-crystal thereof according to claim 1, **characterized in that** the compound is selected from one of the structures shown in Table E-1.

19. The compound or the stereoisomer, deuterated compound, solvate, prodrug, metabolite, pharmaceutically acceptable salt or co-crystal thereof according to claim 1, **characterized in that** the compound is selected from one of the structures shown in Table E-2.

20. A pharmaceutical composition, **characterized in that** the pharmaceutical composition comprises the compound or the stereoisomer, deuterated compound, solvate, prodrug, metabolite, pharmaceutically acceptable salt or co-crystal thereof according to any one of claims 1-19, and a pharmaceutically acceptable carrier, wherein preferably, the pharmaceutical composition comprises 1-1000 mg of the compound or the stereoisomer, deuterated compound, solvate, prodrug, metabolite, pharmaceutically acceptable salt or co-crystal thereof according to any one of claims 1-19.

21. The compound or the stereoisomer, deuterated compound, solvate, prodrug, metabolite, pharmaceutically acceptable salt or co-crystal thereof according to any one of claims 1-19, or the pharmaceutical composition according to claim 20 for use in the preparation of a drug for the treatment of a disease related to the activity or expression level of AR or AR splice mutants.

22. The compound or the stereoisomer, deuterated compound, solvate, prodrug, metabolite, pharmaceutically acceptable salt or co-crystal thereof according to any one of claims 1-19, or the pharmaceutical composition according to claim 20 for use in the preparation of a drug for the treatment of a disease related to the inhibition or degradation of AR or AR splice mutants.

23. The use according to claim 21 or 22, **characterized in that** the disease is selected from prostate cancer.

24. A method for treating a disease in a mammal, **characterized in that** the method comprises administering to a subject a therapeutically effective amount of the compound or the stereoisomer, deuterated compound, solvate, prodrug, metabolite, pharmaceutically acceptable salt or co-crystal thereof according to any one of claims 1-19, wherein the therapeutically effective amount is preferably 1-1000 mg, and the disease is preferably a disease related to the activity or expression level of AR or AR splice mutants.

## INTERNATIONAL SEARCH REPORT

International application No.
**PCT/CN2022/111743**

**A. CLASSIFICATION OF SUBJECT MATTER**

C07D 401/14(2006.01)i; C07D 417/14(2006.01)i; C07D 471/04(2006.01)i; C07D 471/08(2006.01)i; C07D 471/10(2006.01)i; A61K 31/444(2006.01)i; A61K 31/496(2006.01)i; A61K 31/517(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D A61K A61P

Documentation searched other than minimum documentation to the extent that such documents are included in the fields searched

Electronic data base consulted during the international search (name of data base and, where practicable, search terms used)

CNABS, CNKI, EPODOC, WPI, REGISTRY(STN), CAPLUS(STN): 四川海思科制药有限公司, 雄激素受体, E3泛素连接酶, 前列腺癌, androgen receptor, AR, PROTAC, E3 ubiquitin lipase, prostate cancer

**C. DOCUMENTS CONSIDERED TO BE RELEVANT**

| Category* | Citation of document, with indication, where appropriate, of the relevant passages | Relevant to claim No. |
|---|---|---|
| X | CN 111051298 A (NOVARTIS AG) 21 April 2020 (2020-04-21)<br>description, paragraphs [0065], [0075], and [0480] | 1-4, 7-8, 20-24 |
| X | ACS. "RN 1297716-76-7"<br>*STN Registry,* 20 May 2011 (2011-05-20),<br>pp. 1-6 | 1-5, 7-10, 14, 16 |
| X | ACS. "RN 1280499-56-0"<br>*STN Registry,* 15 April 2011 (2011-04-15),<br>pp. 1-9 | 1-4, 7-8 |
| Y | WO 9822432 A1 (YAMANOUCHI PHARMA CO., LTD. et al.) 28 May 1998 (1998-05-28)<br>abstract, claim 1, and description, tables 7-9, 11, and 14 | 1-24 |
| Y | CN 110963957 A (SHENZHEN ENDUOKAI MEDICAL TECHNOLOGY CO., LTD.) 07 April 2020 (2020-04-07)<br>abstract, and claims 1 and 5 | 1-24 |
| Y | CN 108601764 A (ARVINAS, INC. et al.) 28 September 2018 (2018-09-28)<br>abstract, claims 1 and 16, and description, table 3 | 1-24 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **21 October 2022** | **09 November 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/111743**

**C. DOCUMENTS CONSIDERED TO BE RELEVANT**

| Category* | Citation of document, with indication, where appropriate, of the relevant passages | Relevant to claim No. |
|---|---|---|
| Y | CN 110612294 A (ARVINAS OPERATIONS, INC.) 24 December 2019 (2019-12-24) abstract, and description, paragraphs [0059]-[0060], [0298], and [0704] | 1-24 |
| Y | WO 2019199816 A1 (ARVINAS OPERATIONS, INC.) 17 October 2019 (2019-10-17) abstract, and description, paragraphs [0046] and [0057]-[0132], and table 2 | 1-24 |

Form PCT/ISA/210 (second sheet) (January 2015)

**INTERNATIONAL SEARCH REPORT**

International application No.
**PCT/CN2022/111743**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **24**
because they relate to subject matter not required to be searched by this Authority, namely:

[1] The method of claim 24 is a disease treatment method. However, the present search report was made on the basis of a corresponding pharmaceutical use.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2022/111743**

| Patent document cited in search report | Publication date (day/month/year) | Patent family member(s) | Publication date (day/month/year) |
|---|---|---|---|
| CN 111051298 A | 21 April 2020 | CA 3072694 A1 | 28 February 2019 |
| | | AR 112529 A1 | 06 November 2019 |
| | | AU 2020277231 A1 | 24 December 2020 |
| | | UY 37854 A | 29 March 2019 |
| | | US 2021309638 A1 | 07 October 2021 |
| | | JO P20200042 A1 | 23 February 2019 |
| | | US 2019359594 A1 | 28 November 2019 |
| | | JP 2020531498 A | 05 November 2020 |
| | | SA 520411325 B1 | 13 June 2022 |
| | | AU 2018319577 A1 | 06 February 2020 |
| | | AU 2022231670 A1 | 06 October 2022 |
| | | US 2020231569 A1 | 23 July 2020 |
| | | US 2019062309 A1 | 28 February 2019 |
| | | TW 201920143 A | 01 June 2019 |
| | | ZA 202000208 B | 28 July 2021 |
| | | KR 20200044038 A | 28 April 2020 |
| | | WO 2019038717 A1 | 28 February 2019 |
| | | IL 272748 A | 30 April 2020 |
| | | CR 20200081 A | 14 May 2020 |
| | | BR 112020003373 A2 | 25 August 2020 |
| | | RU 2020111556 A | 23 September 2021 |
| | | PH 12020500125 A1 | 14 September 2020 |
| | | EP 3672949 A1 | 01 July 2020 |
| | | MA 49952 A | 28 April 2021 |
| | | CO 2020001860 A2 | 29 May 2020 |
| | | SG 11202000490 P A | 30 March 2020 |
| | | CU 20200014 A7 | 30 November 2020 |
| | | EA 202090553 A1 | 08 June 2020 |
| | | US 2019367483 A1 | 05 December 2019 |
| | | DO P2020000037 A | 15 August 2020 |
| | | EC SP20013248 A | 29 May 2020 |
| | | CL 2020000427 A1 | 28 August 2020 |
| WO 9822432 A1 | 28 May 1998 | AU 4966497 A | 10 June 1998 |
| | | JP 9822432 A1 | 14 March 2000 |
| CN 110963957 A | 07 April 2020 | WO 2020063407 A1 | 02 April 2020 |
| | | US 2021214311 A1 | 15 July 2021 |
| | | JP 2022501442 A | 06 January 2022 |
| | | EP 3858811 A1 | 04 August 2021 |
| | | KR 20210090615 A | 20 July 2021 |
| CN 108601764 A | 28 September 2018 | CA 2979070 A1 | 22 September 2016 |
| | | AU 2020273338 A1 | 17 December 2020 |
| | | US 2020392131 A1 | 17 December 2020 |
| | | AU 2016232705 A1 | 21 September 2017 |
| | | EP 3270917 A1 | 24 January 2018 |
| | | MX 2017011919 A | 22 May 2018 |
| | | HK 1249058 A1 | 26 October 2018 |
| | | JP 2021073251 A | 13 May 2021 |
| | | RU 2017134730 A | 05 April 2019 |
| | | KR 20180011759 A | 02 February 2018 |
| | | BR 112017019751 A2 | 29 May 2018 |

Form PCT/ISA/210 (patent family annex) (January 2015)

**INTERNATIONAL SEARCH REPORT**
**Information on patent family members**

International application No.
**PCT/CN2022/111743**

| Patent document cited in search report | Publication date (day/month/year) | Patent family member(s) | Publication date (day/month/year) |
|---|---|---|---|
| | | JP 2018512449 A | 17 May 2018 |
| | | WO 2016149668 A1 | 22 September 2016 |
| | | US 2016272639 A1 | 22 September 2016 |
| CN 110612294 A | 24 December 2019 | KR 20190116315 A | 14 October 2019 |
| | | CO 2019009424 A2 | 28 February 2020 |
| | | JP 2020506922 A | 05 March 2020 |
| | | BR 112019015484 A2 | 28 April 2020 |
| | | AU 2022221386 A1 | 15 September 2022 |
| | | EP 3577109 A1 | 11 December 2019 |
| | | CA 3050309 A1 | 09 August 2018 |
| | | IL 268069 A | 26 September 2019 |
| | | RU 2019123462 A | 27 January 2021 |
| | | MX 2019009046 A | 30 October 2019 |
| | | AU 2018215212 A1 | 11 July 2019 |
| | | WO 2018144649 A1 | 09 August 2018 |
| | | US 2018215731 A1 | 02 August 2018 |
| WO 2019199816 A1 | 17 October 2019 | JP 2021521192 A | 26 August 2021 |
| | | RU 2020136948 A | 16 May 2022 |
| | | KR 20210003804 A | 12 January 2021 |
| | | EP 3774772 A1 | 17 February 2021 |
| | | AU 2019251223 A1 | 26 November 2020 |
| | | IL 277934 A | 30 November 2020 |
| | | CA 3095912 A1 | 17 October 2019 |
| | | CN 112262134 A | 22 January 2021 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021194318 A
- WO 2019074962 A
- WO 2020239103 A
- WO 2020063407 A
- WO 2020113233 A
- WO 2020211822 A
- WO 2003096980 A
- WO 2021091575 A
- WO 2021077010 A
- WO 2021023105 A
- WO 2021249534 A
- WO 201875937 A
- WO 2020224656 A
- WO 2018089355 A
- WO 2021066873 A
- WO 2018114786 A
- WO 2020201080 A
- WO 2011029027 A
- WO 2020264499 A
- WO 2020043008 A
- US 20090192198 A
- WO 2022019597 A
- WO 2022007903 A
- WO 2021188696 A
- WO 2020102576 A
- WO 2021030711 A
- WO 2021262812 A
- US 20100267738 A
- WO 2020210630 A
- WO 2020018975 A
- WO 2021143822 A
- WO 2018122232 A
- WO 2009042177 A
- WO 2021083182 A
- WO 2020126819 A
- WO 2017161119 A
- WO 2008124550 A
- WO 2010078408 A
- WO 2013173720 A


### Non-patent literature cited in the description


- *Nat. Commun.,* 2022, vol. 13, 815
- *CHEMICAL ABSTRACTS,* 148893-10-1
- *CHEMICAL ABSTRACTS,* 95464-05-4
- *CHEMICAL ABSTRACTS,* 13965-03-2
- *CHEMICAL ABSTRACTS,* 1445085-55-1
- *CHEMICAL ABSTRACTS,* 1445085-77-7
- *CHEMICAL ABSTRACTS,* 564483-18-7
- *CHEMICAL ABSTRACTS,* 38078-09-0
- *CHEMICAL ABSTRACTS,* 68957-94-8
- *CHEMICAL ABSTRACTS,* 94790-35-9
- *CHEMICAL ABSTRACTS,* 1072-53-3
- *CHEMICAL ABSTRACTS,* 213697-53-1
- *Org. Letts.,* 2020, vol. 22, 9331-9336
- *CHEMICAL ABSTRACTS,* 1375325-68-0
- *Synthesis,* 2015, vol. 47, 3963-3971
- *CHEMICAL ABSTRACTS,* 161265-03-8
- *CHEMICAL ABSTRACTS,* 60748-47-2
- *Synthesis,* 2011, vol. 7, 1149-1156